# EUROPEAN PATENT APPLICATION

(11) **EP 3 540 061 A1**
(43) Date of publication of application: **18.09.2019**
(21) Application number: 19155242.1
(22) Date of filing: 01.04.2015
(51) Int. Cl.: C12N 15/10, C12N 15/113, C12N 15/63

(54) **CRISPR/CAS-RELATED METHODS AND COMPOSITIONS FOR TREATING PRIMARY OPEN ANGLE GLAUCOMA**

(30) Priority: 02.04.2014 US 201461974327 P
(62) Divisional of application: 15716679.4
(71) Applicant: Editas Medicine, Inc., Cambridge, MA 02141 (US)
(72) Inventor: MAEDER, Morgan, L., Jamaica Plain, MA 02130 (US); BUMCROT, David, A., Belmont, MA 02478 (US)
(74) Representative: Donald, Jenny Susan

(57) **Abstract**

CRISPR/CAS-related compositions and methods for treatment of Primary Open Angle Glaucoma (POAG) are disclosed.

## Description

### REFERENCE TO RELATED APPLICATIONS

The present application claims the benefit of U.S. Provisional Application No. 61/974,327, filed April 2, 2014, the contents of which are hereby incorporated by reference in their entirety.

### FIELD OF THE INVENTION

The invention relates to CRISPR/CAS-related methods and components for editing of a target nucleic acid sequence, and applications thereof in connection with Primary Open Angle Glaucoma (POAG).

### BACKGROUND

Glaucoma is the second leading cause of blindness in the world. Primary Open Angle Glaucoma (POAG) is the leading cause of glaucoma, representing more than 50% of glaucoma in the United States (Quigley et al. Investigations in Ophthalmology and Visual Science1997; 38:83-91). POAG affects 3 million subjects in the United States (Glaucoma Research Foundation: www.glaucoma.org; Accessed March 27, 2015). Approximately 1% of subjects ages 40-89 have POAG.

The disease develops due to an imbalance between the production and outflow of aqueous humor within the eye. Aqueous humor (AH) is produced by the ciliary body located in the anterior chamber of the eye. The vast majority (80%) of AH drains through the trabecular meshwork (TM) to the episcleral venous system. The remainder (20%) of AH drains through the interstitium between the iris root and ciliary muscle (Feisal et al., Canadian Family Physician 2005; 51(9): 1229-1237). POAG is likely due to decreased drainage through the trabecular meshwork. Decreased outflow of AH results in increased intraocular pressure (IOP). IOP causes damage to the optic nerve and leads to progressive blindness.

Mutations in the *MYOC* gene have been shown to be a leading genetic cause of POAG. Mutations in *MYOC* have been shown to account for 3% of POAG. Approximately 90,000 individuals in the United States have POAG that is caused by *MYOC* mutations. Many patients with MYOC mutations develop rapidly advancing disease and early-onset POAG, including juvenile-onset POAG.

*MYOC* mutations are inherited in an autosomal dominant fashion. Disease-causing mutations cluster in the olfactomedin domain of exon 3 of the *MYOC* gene. The most common *MYOC* mutation causing severe, early onset disease is a proline to leucine substitution at amino acid position 370 (P370L) (Waryah et al., Gene 2013; 528(2):356-9). The most common *MYOC* mutation is a missense mutation at amino acid position 368 (Q368X). This mutation is associated with less severe disease, termed late-onset POAG.

Treatments that reduce IOP can slow the progression of POAG. Trabeculectomy surgery and eye drops are both effective in in reducing IOP. Eye drops include alpha-adregergic antagonists and beta-adrenergic antagonists. However, POAG is known as a silent cause of blindness, as it is painless and leads to progressive blindness if left untreated. Despite advances in POAG therapies, there remains a need for the treatment and prevention of POAG. A one-time or several dose treatment that reduces IOP and prevents the progression of POAG would be beneficial in the treatment and prevention of POAG.

### SUMMARY OF THE INVENTION

Methods and compositions discussed herein, allow the correction of disorders of the eye, e.g., disorders that affect trabecular meshwork cells, photoreceptor cells and any other cells in the eye, including those of the iris, ciliary body, optic nerve or aqueous humor.

In one aspect, methods and compositions discussed herein, provide for treating or delaying the onset or progression of (POAG). POAG is a common form of glaucoma, characterized by degeneration of the trabecular meshwork, which leads to obstruction of the normal ability of aqueous humor to leave the eye without closure of the space (e.g., the "angle") between the iris and cornea. This obstruction leads to increased intraocular pressure ("IOP"), which can result in progressive visual loss and blindness if not treated appropriately and in a timely fashion. POAG is a progressive ophthalmologic disorder characterized by increased intraocular pressure (IOP).

In one aspect, methods and compositions discussed herein, provide for the correction of the underlying cause of Primary Open Angle Glaucoma (POAG).

Mutations in the *MYOC* gene (also known as *GPOA, JOAG, TIGR, GLC1A, JOAG1* and *myocilin)* have been shown to account for 3% of POAG. Certain mutations in *MYOC* lead to severe, early onset POAG. Mutations in the *MYOC* gene leading to POAG can be described based on the mutated amino acid residue(s) in the MYOC protein. Severe, early-onset POAG can be caused by mutations in the MYOC gene, including mutations in exon 3. Exemplary mutations include, but are not limited to the mutations T377R, 1477, and P370L (Zhuo et al., Molecular Vision 2008; 14:1533-1539).

In an embodiment, the target mutation is at P370, e.g., P370L, in the MYOC gene. In an embodiment, the target mutation is at 1477, e.g., I477N or I477S, in the *MYOC* gene. In an embodiment, the target mutation is at T377, e.g., T377R, in the *MYOC* gene. In an embodiment, the target mutation is at Q368, e.g., Q368stop, in the *MYOC* gene. In an embodiment, the target mutation is a mutational hotspot between amino acid sequence positions 246-252 in the *MYOC* gene. In an embodiment, the target mutation is a mutational hotspot between amino acid sequence positions, e.g., amino acids 368-380, amino acids 368-370 + 377-380, amino acids 364-380, or amino acids 347-380 in the *MYOC* gene. In an embodiment, the target mutation is a mutational hotspot between amino acid sequence positions 423-437 (e.g., amino acids 423-426, amino acids 423-427 and amino acids 423-437) in the *MYOC* gene. In an embodiment, the target mutation is a mutational hotspot between amino acid sequence positions 477-502 in the *MYOC* gene.

"POAG target point position", as used herein, refers to a target position in the *MYOC* gene, typically a single nucleotide, which, if mutated, can result in a mutant protein and give rise to POAG. In an embodiment, the POAG target point position is a position in the *MYOC* gene at which a change can give rise to a mutant protein having a mutation at Q368 (e.g., Q368stop), P370 (e.g., the substitution P370L), T377 (e.g., the substitution T377R), or 1477 (e.g., the substitution I477N or I477S).

"POAG target hotspot position", as used herein, refers to a target position in a region of the *MYOC* gene, which: (1) encodes amino acid sequence positions 246-252, amino acid sequence positions 368-380, amino acid sequence positions 423-437, or amino acid sequence positions 477-502; and (2) when mutated, can give rise to a mutation in one of the aforesaid amino acid sequence regions and give rise to POAG.

While some of the disclosure herein is presented in the context of several specific mutations in the *MYOC* gene, the methods and compositions herein are broadly applicable to any mutation, e.g., a point mutation or a deletion, in the *MYOC* gene that gives rise to POAG.

While not wishing to be bound by theory, it is believed that, in an embodiment, a mutation at a POAG target point position or a POAG target hotspot position is corrected by homology directed repair (HDR), as described herein.

In another aspect, methods and compositions discussed herein may be used to alter the *MYOC* gene to treat or prevent POAG by targeting the *MYOC* gene, e.g., the non-coding or coding regions, e.g., the promoter region, or a transcribed sequence, e.g., intronic or exonic sequence. In an embodiment, coding sequence, e.g., a coding region, e.g., an early coding region, of the *MYOC* gene, is targeted for alteration and knockout of expression.

In another aspect, the methods and compositions discussed herein may be used to alter the *MYOC* gene to treat or prevent POAG by targeting the coding sequence of the *MYOC* gene. In one embodiment, the gene, e.g., the coding sequence of the *MYOC* gene, is targeted to knockout the gene, e.g., to eliminate expression of the gene, e.g., to knockout both alleles of the *MYOC* gene, e.g., by induction of an alteration comprising a deletion or mutation in the *MYOC* gene. In an embodiment, the method provides an alteration that comprises an insertion or deletion, while not wishing to be bound by theory, in an embodiment, a targeted knockout approach is mediated by non-homologous end joining (NHEJ) using a CRISPR/Cas system comprising a Cas9 molecule, e.g., an enzymatically active Cas9 (eaCas9) molecule.

In one embodiment, a coding region, e.g., an early coding region, of the *MYOC* gene is targeted to knockout the *MYOC* gene. In an embodiment, targeting affects both alleles of the *MYOC* gene. In an embodiment, a targeted knockout approach reduces or eliminates expression of functional *MYOC* gene product. In an embodiment, the method provides an alteration that comprises an insertion or deletion.

In another aspect, the methods and compositions discussed herein may be used to alter the *MYOC* gene to treat or prevent POAG by targeting non-coding sequence of the *MYOC* gene, e.g., promoter, an enhancer, an intron, 3'UTR, and/or polyadenylation signal. In one embodiment, the gene, e.g., the non-coding sequence of the *MYOC* gene, is targeted to knockout the gene, e.g., to eliminate expression of the gene, e.g., to knockout both alleles of the *MYOC* gene, e.g., by induction of an alteration comprising a deletion or mutation in the *MYOC* gene. In an embodiment, the method provides an alteration that comprises an insertion or deletion.

"POAG target knockout position", as used herein, refers to a target position in the *MYOC* gene, which if altered by NHEJ-mediated alteration, results in reduction or elimination of expression of a functional *MYOC* gene product. In an embodiment, the position is in the *MYOC* coding region, e.g., an early coding region.

In another aspect, methods and compositions discussed herein may be used to alter the expression of the *MYOC* gene to treat or prevent POAG by targeting the *MYOC* gene, e.g., a promoter region of the *MYOC* gene. In an embodiment, the promoter region of the *MYOC* gene is targeted to knockdown expression of the *MYOC* gene. A targeted knockdown approach reduces or eliminates expression of a mutated *MYOC* gene. As described herein, a targeted knockdown approach is mediated by targeting an enzymatically inactive Cas9 (eiCas9) molecule or an eiCas9 fusion protein (e.g., an eiCas9 fused to a transcription repressor domain or chromatin modifying protein) to alter transcription, e.g., block, reduce, or decrease transcription, of the *MYOC* gene. While not wishing to be bound by theory, in an embodiment, a targeted knockdown approach is mediated by NHEJ using a CRISPR/Cas system comprising a Cas9 molecule, e.g., an enzymatically inactive Cas9 (eiCas9) molecule.

"POAG target knockdown position", as used herein, refers to a position, e.g., in the *MYOC* gene, which if targeted by an eiCas9 molecule or an eiCas9 fusion described herein, results in reduction or elimination of expression of functional *MYOC* gene product. In an embodiment, transcription is reduced or eliminated. In an embodiment, the position is in the *MYOC* promoter sequence. In an embodiment, a position in the promoter sequence of the *MYOC* gene is targeted by an enzymatically inactive Cas9 (eiCas9) molecule or an eiCas9-fusion protein, as described herein.

"POAG target position", as used herein, refers to any of the POAG target point positions, POAG target hotspot positions, POAG target knockout positions and/or POAG target knockdown positions in the *MYOC* gene, as described herein.

In one aspect, disclosed herein is a gRNA molecule, e.g., an isolated or non-naturally occurring gRNA molecule, comprising a targeting domain which is complementary with a target domain from the *MYOC* gene.

In an embodiment, the targeting domain of the gRNA molecule is configured to provide a cleavage event, e.g., a double strand break or a single strand break, sufficiently close to a POAG target position in the *MYOC* gene to allow alteration, e.g., alteration associated with HDR or NHEJ, of a POAG target position in the *MYOC* gene. In an embodiment, the targeting domain is configured such that a cleavage event, e.g., a double strand or single strand break, is positioned within 1, 2, 3, 4, 5, 10, 15, 20, 25, 30, 35, 40, 45, 50, 60, 70, 80, 90, 100, 150, 200, 300, 400, or 500 nucleotides of a POAG target position. The break, e.g., a double strand or single strand break, can be positioned upstream or downstream of a POAG target position in the *MYOC* gene.

In an embodiment, a second gRNA molecule comprising a second targeting domain is configured to provide a cleavage event, e.g., a double strand break or a single strand break, sufficiently close to the POAG target position in the *MYOC* gene, to allow alteration, e.g., alteration associated with HDR or NHEJ, of the POAG target position in the *MYOC* gene, either alone or in combination with the break positioned by said first gRNA molecule. In an embodiment, the targeting domains of the first and second gRNA molecules are configured such that a cleavage event, e.g., a double strand or single strand break, is positioned, independently for each of the gRNA molecules, within 1, 2, 3, 4, 5, 10, 15, 20, 25, 30, 35, 40, 45, 50, 60, 70, 80, 90, 100, 150, 200, 300, 400, or 500 nucleotides of the target position. In an embodiment, the breaks, e.g., double strand or single strand breaks, are positioned on both sides of a nucleotide of a POAG target position in the *MYOC* gene. In an embodiment, the breaks, e.g., double strand or single strand breaks, are positioned on one side, e.g., upstream or downstream, of a nucleotide of a POAG target position in the *MYOC* gene.

In an embodiment, a single strand break is accompanied by an additional single strand break, positioned by a second gRNA molecule, as discussed below. For example, the targeting domains are configured such that a cleavage event, e.g., the two single strand breaks, are positioned within 1, 2, 3, 4, 5, 10, 15, 20, 25, 30, 35, 40, 45, 50, 60, 70, 80, 90, 100, 150, 200, 300, 400, or 500 nucleotides of a POAG target position. In an embodiment, the first and second gRNA molecules are configured such, that when guiding a Cas9 molecule, e.g., a Cas9 nickase, a single strand break will be accompanied by an additional single strand break, positioned by a second gRNA, sufficiently close to one another to result in alteration of a POAG target position in the *MYOC* gene. In an embodiment, the first and second gRNA molecules are configured such that a single strand break positioned by said second gRNA is within 10, 20, 30, 40, or 50 nucleotides of the break positioned by said first gRNA molecule, e.g., when the Cas9 molecule is a nickase. In an embodiment, the two gRNA molecules are configured to position cuts at the same position, or within a few nucleotides of one another, on different strands, e.g., essentially mimicking a double strand break.

In an embodiment, a double strand break can be accompanied by an additional double strand break, positioned by a second gRNA molecule, as is discussed below. For example, the targeting domain of a first gRNA molecule is configured such that a double strand break is positioned upstream of a POAG target position in the *MYOC* gene, e.g., within 1, 2, 3, 4, 5, 10, 15, 20, 25, 30, 35, 40, 45, 50, 60, 70, 80, 90, 100, 150, 200, 300, 400, or 500 nucleotides of the target position; and the targeting domain of a second gRNA molecule is configured such that a double strand break is positioned downstream of a POAG target position in the *MYOC* gene, e.g., within 1, 2, 3, 4, 5, 10, 15, 20, 25, 30, 35, 40, 45, 50, 60, 70, 80, 90, 100, 150, 200, 300, 400, or 500nucleotides of the target position.

In an embodiment, a double strand break can be accompanied by two additional single strand breaks, positioned by a second gRNA molecule and a third gRNA molecule. For example, the targeting domain of a first gRNA molecule is configured such that a double strand break is positioned upstream of a POAG target position in the *MYOC* gene, e.g., within 1, 2, 3, 4, 5, 10, 15, 20, 25, 30, 35, 40, 45, 50, 60, 70, 80, 90, 100, 150, 200, 300, 400, or 500 nucleotides of the target position; and the targeting domains of a second and third gRNA molecule are configured such that two single strand breaks are positioned downstream of a POAG target position in the *MYOC* gene, e.g., within 1, 2, 3, 4, 5, 10, 15, 20, 25, 30, 35, 40, 45, 50, 60, 70, 80, 90, 100, 150, 200, 300, 400, or 500 nucleotides of the target position. In an embodiment, the targeting domain of the first, second and third gRNA molecules are configured such that a cleavage event, e.g., a double strand or single strand break, is positioned, independently for each of the gRNA molecules.

In an embodiment, a first and second single strand breaks can be accompanied by two additional single strand breaks positioned by a third gRNA molecule and a fourth gRNA molecule. For example, the targeting domain of a first and second gRNA molecule are configured such that two single strand breaks are positioned upstream of a POAG target position in the *MYOC* gene, e.g., within 1, 2, 3, 4, 5, 10, 15, 20, 25, 30, 35, 40, 45, 50, 60, 70, 80, 90, 100, 150, 200, 300, 400, or 500 nucleotides of the target position; and the targeting domains of a third and fourth gRNA molecule are configured such that two single strand breaks are positioned downstream of a POAG target position in the *MYOC* gene, e.g., within 1, 2, 3, 4, 5, 10, 15, 20, 25, 30, 35, 40, 45, 50, 60, 70, 80, 90, 100, 150, 200, 300, 400, or 500 nucleotides of the target position.

It is contemplated herein that, in an embodiment, when multiple gRNAs are used to generate (1) two single stranded breaks in close proximity, (2) two double stranded breaks, e.g., flanking a POAG target position, e.g., a mutation (e.g., to remove a piece of DNA, e.g., a insertion mutation) or to create more than one indel in an early coding region, (3) one double stranded break and two paired nicks flanking a POAG target position, e.g., a mutation (e.g., to remove a piece of DNA, e.g., a insertion mutation) or (4) four single stranded breaks, two on each side of a mutation, that they are targeting the same POAG target position. It is further contemplated herein that multiple gRNAs may be used to target more than one POAG target position (e.g., mutation) in the same gene.

In an embodiment, the targeting domain of the first gRNA molecule and the targeting domain of the second gRNA molecules are complementary to opposite strands of the target nucleic acid molecule. In an embodiment, the gRNA molecule and the second gRNA molecule are configured such that the PAMs are oriented outward.

In an embodiment, the targeting domain of a gRNA molecule is configured to avoid unwanted target chromosome elements, such as repeat elements, e.g., Alu repeats, in the target domain. The gRNA molecule may be a first, second, third and/or fourth gRNA molecule, as described herein.

In an embodiment, the targeting domain of a gRNA molecule is configured to position a cleavage event sufficiently far from a preselected nucleotide, e.g., the nucleotide of a coding region, such that the nucleotide is not altered. In an embodiment, the targeting domain of a gRNA molecule is configured to position an intronic cleavage event sufficiently far from an intron/exon border, or naturally occurring splice signal, to avoid alteration of the exonic sequence or unwanted splicing events. The gRNA molecule may be a first, second, third and/or fourth gRNA molecule, as described herein.

In an embodiment, the targeting domain of a gRNA molecule comprises a sequence that is the same as, or differs by no more than 1, 2, 3, 4, or 5 nucleotides from, a targeting domain sequence described herein, e.g., from any one of **Tables 1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E,** or **23A-23B.** In an embodiment, the targeting domain of a gRNA molecule comprises a sequence that is the same as a targeting domain sequence described herein, e.g., from any one of **Tables 1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E, or 23A-23B.**

In an embodiment, when two or more gRNAs are used to position two or more breaks, e.g., two single stranded breaks, in the target nucleic acid sequence, each guide RNA is independently selected from any one of **Tables 1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E, or 23A-23B.**

In an embodiment, a POAG target position, e.g., a mutation in the *MYOC* gene, e.g., a mutation at P370, e.g., a point mutation P370L, is targeted, e.g., for correction. In an embodiment, the targeting domain of a gRNA molecule comprises a sequence that is the same as, or differs by no more than 1, 2, 3, 4, or 5 nucleotides from, a targeting domain sequence from any one of **Tables 1A-1E, 21A-21D, 22A-22E,** or **23A-23B.** In some embodiments, the targeting domain is independently selected from those in **Tables 1A-1E, 21A-21D, 22A-22E,** or **23A-23B.**

In an embodiment, when the POAG target point position is P370L and two gRNAs are used to position two breaks, e.g., two single stranded breaks, in the target nucleic acid sequence, each guide RNA is selected from one of **Tables 1A-1E, 21A-21D, 22A-22E,** or **23A-23B.**

In an embodiment, a POAG target position, e.g., a mutation in the *MYOC* gene, e.g., a mutation at P370, e.g., a point mutation P370L, is targeted, e.g., for correction. In an embodiment, the targeting domain of a gRNA molecule comprises a sequence that is the same as, or differs by no more than 1, 2, 3, 4, or 5 nucleotides from, a targeting domain sequence from any one of **Tables 1A-1E.** In some embodiments, the targeting domain is independently selected from those in **Tables 1A-1E.** For example, in certain embodiments, the targeting domain is independently selected from **Table 1A.**

In an embodiment, when the POAG target point position is P370L and two gRNAs are used to position two breaks, e.g., two single stranded breaks, in the target nucleic acid sequence, each guide RNA is selected from one of **Tables 1A-1E.**

In an embodiment, a POAG target position, e.g., a mutation in the *MYOC* gene, e.g., a mutation at P370, e.g., a point mutation P370L, is targeted, e.g., for correction. In an embodiment, the targeting domain of a gRNA molecule comprises a sequence that is the same as, or differs by no more than 1, 2, 3, 4, or 5 nucleotides from, a targeting domain sequence from any one of **Tables 21A-21D.** In some embodiments, the targeting domain is independently selected from those in **Tables 21A-21D.** For example, in certain embodiments, the targeting domain is independently selected from **Table 21A.**

In an embodiment, when the POAG target point position is P370L and two gRNAs are used to position two breaks, e.g., two single stranded breaks, in the target nucleic acid sequence, each guide RNA is selected from one of **Tables 21A-21D.**

In an embodiment, a POAG target position, e.g., a mutation in the *MYOC* gene, e.g., a mutation at P370, e.g., a point mutation P370L, is targeted, e.g., for correction. In an embodiment, the targeting domain of a gRNA molecule comprises a sequence that is the same as, or differs by no more than 1, 2, 3, 4, or 5 nucleotides from, a targeting domain sequence from any one of **Tables 22A-22E.** In some embodiments, the targeting domain is independently selected from those in **Tables 22A-22E.** For example, in certain embodiments, the targeting domain is independently selected from **Table 22A.**

In an embodiment, when the POAG target point position is P370L and two gRNAs are used to position two breaks, e.g., two single stranded breaks, in the target nucleic acid sequence, each guide RNA is selected from one of **Tables 22A-22E.**

In an embodiment, a POAG target position, e.g., a mutation in the *MYOC* gene, e.g., a mutation at P370, e.g., a point mutation P370L, is targeted, e.g., for correction. In an embodiment, the targeting domain of a gRNA molecule comprises a sequence that is the same as, or differs by no more than 1, 2, 3, 4, or 5 nucleotides from, a targeting domain sequence from any one of **Tables 23A-23B.** In some embodiments, the targeting domain is independently selected from those in **Tables 23A-23B.** For example, in certain embodiments, the targeting domain is independently selected from **Table 23A.**

In an embodiment, when the POAG target point position is P370L and two gRNAs are used to position two breaks, e.g., two single stranded breaks, in the target nucleic acid sequence, each guide RNA is selected from one of **Tables 23A-23B.**

In another embodiment, a POAG target position, e.g., a mutation in the *MYOC* gene, e.g., a mutation at 1477, e.g., a point mutation I477N, is targeted, e.g., for correction. In an embodiment, the targeting domain comprises a sequence that is the same as, or differs by no more than 1, 2, 3, 4, or 5 nucleotides from, a targeting domain sequence from any one of **Tables 2A-2E, 18A-18D, 19A-19E,** or **20A-20D.** In an embodiment, the targeting domain is independently selected from those in **Tables 2A-2E, 18A-18D, 19A-19E,** or **20A-20D.**

In an embodiment, when the POAG target point position is I477N and two gRNAs are used to position two breaks, e.g., two single stranded breaks, in the target nucleic acid sequence, each guide RNA is selected from one of **Tables 2A-2E, 18A-18D, 19A-19E,** or **20A-20D.**

In another embodiment, a POAG target position, e.g., a mutation in the *MYOC* gene, e.g., a mutation at 1477, e.g., a point mutation I477N, is targeted, e.g., for correction. In an embodiment, the targeting domain comprises a sequence that is the same as, or differs by no more than 1, 2, 3, 4, or 5 nucleotides from, a targeting domain sequence from any one of **Tables 2A-2E.** In an embodiment, the targeting domain is independently selected from those in **Tables 2A-2E.** In another embodiment, the targeting domain is independently selected from **Table 2A.** In an embodiment, when the POAG target point position is I477N and two gRNAs are used to position two breaks, e.g., two single stranded breaks, in the target nucleic acid sequence, each guide RNA is selected from one of **Tables 2A-2E.**

In another embodiment, a POAG target position, e.g., a mutation in the *MYOC* gene, e.g., a mutation at 1477, e.g., a point mutation I477N, is targeted, e.g., for correction. In an embodiment, the targeting domain comprises a sequence that is the same as, or differs by no more than 1, 2, 3, 4, or 5 nucleotides from, a targeting domain sequence from any one of **Tables 18A-18D.** In an embodiment, the targeting domain is independently selected from those in **Tables 18A-18D.** In another embodiment the targeting domain is independently selected from **Table 18A.**

In an embodiment, when the POAG target point position is I477N and two gRNAs are used to position two breaks, e.g., two single stranded breaks, in the target nucleic acid sequence, each guide RNA is selected from one of **Tables 18A-18D.**

In another embodiment, a POAG target position, e.g., a mutation in the *MYOC* gene, e.g., a mutation at 1477, e.g., a point mutation I477N, is targeted, e.g., for correction. In an embodiment, the targeting domain comprises a sequence that is the same as, or differs by no more than 1, 2, 3, 4, or 5 nucleotides from, a targeting domain sequence from any one of **Tables 19A-19E.** In an embodiment, the targeting domain is independently selected from those in **Tables 19A-19E.** In another embodiment the targeting domain is independently selected from **Table 19A.**

In an embodiment, when the POAG target point position is I477N and two gRNAs are used to position two breaks, e.g., two single stranded breaks, in the target nucleic acid sequence, each guide RNA is selected from one of **Tables 19A-19E.**

In another embodiment, a POAG target position, e.g., a mutation in the *MYOC* gene, e.g., a mutation at 1477, e.g., a point mutation I477N, is targeted, e.g., for correction. In an embodiment, the targeting domain comprises a sequence that is the same as, or differs by no more than 1, 2, 3, 4, or 5 nucleotides from, a targeting domain sequence from any one of **Tables 20A-20D.** In an embodiment, the targeting domain is independently selected from those in **Tables 20A-20D.** In another embodiment the targeting domain is independently selected from **Table 20A.**

In an embodiment, when the POAG target point position is I477N and two gRNAs are used to position two breaks, e.g., two single stranded breaks, in the target nucleic acid sequence, each guide RNA is selected from one of **Tables 20A-20D.**

In an embodiment, a POAG target position, e.g., a mutation hotspot between amino acids 477-502 is targeted, e.g., for correction. In an embodiment, the targeting domain comprises a sequence that is the same as, or differs by no more than 1, 2, 3, 4, or 5 nucleotides from, a targeting domain sequence from any one of **Tables 3A-3E, 12A-12D, 13A-13E, 14A-14C, 15A-15D, 16A-16E,** or **17A-17B.** In an embodiment, the targeting domain is independently selected from those in **Tables 3A-3E, 12A-12D, 13A-13E, 14A-14C, 15A-15D, 16A-16E,** or **17A-17B.**

In an embodiment, when the POAG target hotspot position is the mutation hotspot between amino acids 477-502 and more than one gRNA is used to position breaks, e.g., two single stranded breaks or two double stranded breaks, or a combination of single strand and double strand breaks, in the target nucleic acid sequence, each guide RNA is selected from one of **Tables 3A-3E, 12A-12D, 13A-13E, 14A-14C, 15A-15D, 16A-16E,** or **17A-17B.** In another embodiment, a POAG target position, e.g., a mutation hotspot between amino acids 477-502 is targeted, e.g., for correction. In an embodiment, the targeting domain comprises a sequence that is the same as, or differs by no more than 1, 2, 3, 4, or 5 nucleotides from, a targeting domain sequence from any one of **Tables 3A-3E.** In an embodiment, the targeting domain is independently selected from those **in Tables 3A-3E.** In another embodiment, the targeting domain is independently selected from **Table 3A.**

In an embodiment, when the POAG target hotspot position is the mutation hotspot between amino acids 477-502 and more than one gRNA is used to position breaks, e.g., two single stranded breaks or two double stranded breaks, or a combination of single strand and double strand breaks, in the target nucleic acid sequence, each guide RNA is selected from one of **Tables 3A-3E.**

In another embodiment, a POAG target position, e.g., a mutation hotspot between amino acids 477-502 is targeted, e.g., for correction. In an embodiment, the targeting domain comprises a sequence that is the same as, or differs by no more than 1, 2, 3, 4, or 5 nucleotides from, a targeting domain sequence from any one of **Tables 12A-12D.** In an embodiment, the targeting domain is independently selected from those in **Tables 12A-12D.** In another embodiment, the targeting domain is independently selected from **Table 12A.**

In an embodiment, when the POAG target hotspot position is the mutation hotspot between amino acids 477-502 and more than one gRNA is used to position breaks, e.g., two single stranded breaks or two double stranded breaks, or a combination of single strand and double strand breaks, in the target nucleic acid sequence, each guide RNA is selected from one of **Tables 12A-12D.**

In another embodiment, a POAG target position, e.g., a mutation hotspot between amino acids 477-502 is targeted, e.g., for correction. In an embodiment, the targeting domain comprises a sequence that is the same as, or differs by no more than 1, 2, 3, 4, or 5 nucleotides from, a targeting domain sequence from any one of **Tables 13A-13E.** In an embodiment, the targeting domain is independently selected from those in **Tables 13A-13E.** In another embodiment, the targeting domain is independently selected from **Table 13A.**

In an embodiment, when the POAG target hotspot position is the mutation hotspot between amino acids 477-502 and more than one gRNA is used to position breaks, e.g., two single stranded breaks or two double stranded breaks, or a combination of single strand and double strand breaks, in the target nucleic acid sequence, each guide RNA is selected from one of **Tables 13A-13E.**

In another embodiment, a POAG target position, e.g., a mutation hotspot between amino acids 477-502 is targeted, e.g., for correction. In an embodiment, the targeting domain comprises a sequence that is the same as, or differs by no more than 1, 2, 3, 4, or 5 nucleotides from, a targeting domain sequence from any one of **Tables 14A-14C.** In an embodiment, the targeting domain is independently selected from those in **Tables 14A-14C.** In another embodiment, the targeting domain is independently selected from **Table 14A.**

In an embodiment, when the POAG target hotspot position is the mutation hotspot between amino acids 477-502 and more than one gRNA is used to position breaks, e.g., two single stranded breaks or two double stranded breaks, or a combination of single strand and double strand breaks, in the target nucleic acid sequence, each guide RNA is selected from one of **Tables 14A-14C.**

In another embodiment, a POAG target position, e.g., a mutation hotspot between amino acids 477-502 is targeted, e.g., for correction. In an embodiment, the targeting domain comprises a sequence that is the same as, or differs by no more than 1, 2, 3, 4, or 5 nucleotides from, a targeting domain sequence from any one of **Tables 15A-15D.** In an embodiment, the targeting domain is independently selected from those in **Tables 15A-15D.** In another embodiment, the targeting domain is independently selected from **Table 15A.**

In an embodiment, when the POAG target hotspot position is the mutation hotspot between amino acids 477-502 and more than one gRNA is used to position breaks, e.g., two single stranded breaks or two double stranded breaks, or a combination of single strand and double strand breaks, in the target nucleic acid sequence, each guide RNA is selected from one of **Tables 15A-15D.**

In another embodiment, a POAG target position, e.g., a mutation hotspot between amino acids 477-502 is targeted, e.g., for correction. In an embodiment, the targeting domain comprises a sequence that is the same as, or differs by no more than 1, 2, 3, 4, or 5 nucleotides from, a targeting domain sequence from any one of **Tables 16A-16E.** In an embodiment, the targeting domain is independently selected from those in **Tables 16A-16E.** In another embodiment, the targeting domain is independently selected from **Table 16A.**

In an embodiment, when the POAG target hotspot position is the mutation hotspot between amino acids 477-502 and more than one gRNA is used to position breaks, e.g., two single stranded breaks or two double stranded breaks, or a combination of single strand and double strand breaks, in the target nucleic acid sequence, each guide RNA is selected from one of **Tables 16A-16E.**

In another embodiment, a POAG target position, e.g., a mutation hotspot between amino acids 477-502 is targeted, e.g., for correction. In an embodiment, the targeting domain comprises a sequence that is the same as, or differs by no more than 1, 2, 3, 4, or 5 nucleotides from, a targeting domain sequence from any one of **Tables 17A-17B.** In an embodiment, the targeting domain is independently selected from those in **Tables 17A-17B.** In another embodiment, the targeting domain is independently selected from **Table 17A.**

In an embodiment, when the POAG target hotspot position is the mutation hotspot between amino acids 477-502 and more than one gRNA is used to position breaks, e.g., two single stranded breaks or two double stranded breaks, or a combination of single strand and double strand breaks, in the target nucleic acid sequence, each guide RNA is selected from one of **Tables 17A-17B.**

In another embodiment, the early coding region of the *MYOC* gene is targeted, e.g., for correction. In an embodiment, the targeting domain comprises a sequence that is the same as, or differs by no more than 1, 2, 3, 4, or 5 nucleotides from, a targeting domain sequence from any one of **Tables 4A-4E, 6A-6E, 7A-7G,** or **8A-8E.** In an embodiment, the targeting domain is independently selected from those in **Tables 4A-4E, 6A-6E, 7A-7G,** or **8A-8E.**

In an embodiment, when the POAG target knockout position is the *MYOC* early coding region and more than one gRNA is used to position breaks, e.g., two single stranded breaks or two double stranded breaks, or a combination of single strand and double strand breaks, e.g., to create one or more indels, in the target nucleic acid sequence, each guide RNA is selected from one of **Tables 4A-4E, 6A-6E, 7A-7G,** or **8A-8E.**

In another embodiment, the early coding region of the *MYOC* gene is targeted, e.g., for correction. In an embodiment, the targeting domain comprises a sequence that is the same as, or differs by no more than 1, 2, 3, 4, or 5 nucleotides from, a targeting domain sequence from any one of **Tables 4A-4E.** In an embodiment, the targeting domain is independently selected from those in **Tables 4A-4E.** In another embodiment, the targeting domain is independently selected from **Table 4A.**

In an embodiment, when the POAG target knockout position is the *MYOC* early coding region and more than one gRNA is used to position breaks, e.g., two single stranded breaks or two double stranded breaks, or a combination of single strand and double strand breaks, e.g., to create one or more indels, in the target nucleic acid sequence, each guide RNA is selected from one of **Tables 4A-4E.**

In another embodiment, the early coding region of the *MYOC* gene is targeted, e.g., for correction. In an embodiment, the targeting domain comprises a sequence that is the same as, or differs by no more than 1, 2, 3, 4, or 5 nucleotides from, a targeting domain sequence from any one of **Tables 6A-6E.** In an embodiment, the targeting domain is independently selected from those in **Tables 6A-6E.** In another embodiment, the targeting domain is independently selected from **Table 6A.**

In an embodiment, when the POAG target knockout position is the *MYOC* early coding region and more than one gRNA is used to position breaks, e.g., two single stranded breaks or two double stranded breaks, or a combination of single strand and double strand breaks, e.g., to create one or more indels, in the target nucleic acid sequence, each guide RNA is selected from one of **Tables 6A-6E.**

In another embodiment, the early coding region of the *MYOC* gene is targeted, e.g., for correction. In an embodiment, the targeting domain comprises a sequence that is the same as, or differs by no more than 1, 2, 3, 4, or 5 nucleotides from, a targeting domain sequence from any one of **Tables 7A-7G.** In an embodiment, the targeting domain is independently selected from those in **Tables 7A-7G.** In another embodiment, the targeting domain is independently selected from **Table 7A.**

In an embodiment, when the POAG target knockout position is the *MYOC* early coding region and more than one gRNA is used to position breaks, e.g., two single stranded breaks or two double stranded breaks, or a combination of single strand and double strand breaks, e.g., to create one or more indels, in the target nucleic acid sequence, each guide RNA is selected from one of **Tables 7A-7G.**

In another embodiment, the early coding region of the *MYOC* gene is targeted, e.g., for correction. In an embodiment, the targeting domain comprises a sequence that is the same as, or differs by no more than 1, 2, 3, 4, or 5 nucleotides from, a targeting domain sequence from any one of **Tables 8A-8E.** In an embodiment, the targeting domain is independently selected from those in **Tables 8A-8E.** In another embodiment, the targeting domain is independently selected from **Table 8A.**

In an embodiment, when the POAG target knockout position is the *MYOC* early coding region and more than one gRNA is used to position breaks, e.g., two single stranded breaks or two double stranded breaks, or a combination of single strand and double strand breaks, e.g., to create one or more indels, in the target nucleic acid sequence, each guide RNA is selected from one of **Tables 8A-8E.**

In an embodiment, the targeting domain of the gRNA molecule is configured to target an enzymatically inactive Cas9 (eiCas9) molecule or an eiCas9 fusion protein (e.g., an eiCas9 fused to a transcription repressor domain), sufficiently close to a POAG target knockdown position to reduce, decrease or repress expression of the *MYOC* gene. In an embodiment, the targeting domain is configured to target the promoter region of the *MYOC* gene to reduce (e.g., block) transcription initiation, binding of one or more transcription enhancers or activators, and/or RNA polymerase. One or more gRNA may be used to target an eiCas9 molecule to the promoter region of the *MYOC* gene.

In an embodiment, when the *MYOC* promoter region is targeted, the targeting domain can comprise a sequence that is the same as, or differs by no more than 1, 2, 3, 4, or 5 nucleotides from, a targeting domain sequence from any one of **Tables 5A-5F, 9A-9E, 10A-10G,** or **11A-11E.** In an embodiment, the targeting domain is independently selected from those in **Tables 5A-5F, 9A-9E, 10A-10G,** or **11A-11E.**

In an embodiment, when the POAG target knockdown position is the *MYOC* promoter region and more than one gRNA is used to position an eiCas9 molecule or an eiCas9-fusion protein (e.g., an eiCas9-transcription repressor domain fusion protein), in the target nucleic acid sequence, each guide RNA is selected from one of **5A-5F, 9A-9E, 10A-10G,** or **11A-11E.**

In an embodiment, when the *MYOC* promoter region is targeted, the targeting domain can comprise a sequence that is the same as, or differs by no more than 1, 2, 3, 4, or 5 nucleotides from, a targeting domain sequence from any one of **Tables 5A-5F.** In an embodiment, the targeting domain is independently selected from those in **Tables 5A-5F.** In another embodiment, the targeting domain is independently selected from **Table 5A.**

In an embodiment, when the POAG target knockdown position is the *MYOC* promoter region and more than one gRNA is used to position an eiCas9 molecule or an eiCas9-fusion protein (e.g., an eiCas9-transcription repressor domain fusion protein), in the target nucleic acid sequence, each guide RNA is selected from one of **Tables 5A-5F.**

In an embodiment, when the *MYOC* promoter region is targeted, the targeting domain can comprise a sequence that is the same as, or differs by no more than 1, 2, 3, 4, or 5 nucleotides from, a targeting domain sequence from any one of **Tables 9A-9E.** In an embodiment, the targeting domain is independently selected from those in **Tables 9A-9E.** In another embodiment, the targeting domain is independently selected from **Table 9A.**

In an embodiment, when the POAG target knockdown position is the *MYOC* promoter region and more than one gRNA is used to position an eiCas9 molecule or an eiCas9-fusion protein (e.g., an eiCas9-transcription repressor domain fusion protein), in the target nucleic acid sequence, each guide RNA is selected from one of **Tables 9A-9E.**

In an embodiment, when the *MYOC* promoter region is targeted, the targeting domain can comprise a sequence that is the same as, or differs by no more than 1, 2, 3, 4, or 5 nucleotides from, a targeting domain sequence from any one of **Tables 10A-10G.** In an embodiment, the targeting domain is independently selected from those in **Tables 10A-10G.** In another embodiment, the targeting domain is independently selected from **Table 10A.**

In an embodiment, when the POAG target knockdown position is the *MYOC* promoter region and more than one gRNA is used to position an eiCas9 molecule or an eiCas9-fusion protein (e.g., an eiCas9-transcription repressor domain fusion protein), in the target nucleic acid sequence, each guide RNA is selected from one of **Tables 10A-10G.**

In an embodiment, when the *MYOC* promoter region is targeted, the targeting domain can comprise a sequence that is the same as, or differs by no more than 1, 2, 3, 4, or 5 nucleotides from, a targeting domain sequence from any one of **Tables 11A-11E.** In an embodiment, the targeting domain is independently selected from those in **Tables 11A-11E.** In another embodiment, the targeting domain is independently selected from **Table 11A.**

In an embodiment, when the POAG target knockdown position is the *MYOC* promoter region and more than one gRNA is used to position an eiCas9 molecule or an eiCas9-fusion protein (e.g., an eiCas9-transcription repressor domain fusion protein), in the target nucleic acid sequence, each guide RNA is selected from one of **Tables 11A-11E.**

In an embodiment, the gRNA, e.g., a gRNA comprising a targeting domain, which is complementary with the *MYOC* gene, is a modular gRNA. In other embodiments, the gRNA is a unimolecular or chimeric gRNA.

In an embodiment, the targeting domain which is complementary with a target domain from the POAG target position in the *MYOC* gene is 16 nucleotides or more in length. In an embodiment, the targeting domain is 16 nucleotides in length. In an embodiment, the targeting domain is 17 nucleotides in length. In another embodiment, the targeting domain is 18 nucleotides in length. In still another embodiment, the targeting domain is 19 nucleotides in length. In still another embodiment, the targeting domain is 20 nucleotides in length. In still another embodiment, the targeting domain is 21 nucleotides in length. In still another embodiment, the targeting domain is 22 nucleotides in length. In still another embodiment, the targeting domain is 23 nucleotides in length. In still another embodiment, the targeting domain is 24 nucleotides in length. In still another embodiment, the targeting domain is 25 nucleotides in length. In still another embodiment, the targeting domain is 26 nucleotides in length.

In an embodiment, the targeting domain comprises 16 nucleotides.

In an embodiment, the targeting domain comprises 17 nucleotides.

In an embodiment, the targeting domain comprises 18 nucleotides.

In an embodiment, the targeting domain comprises 19 nucleotides.

In an embodiment, the targeting domain comprises 20 nucleotides.

In an embodiment, the targeting domain comprises 21 nucleotides.

In an embodiment, the targeting domain comprises 22 nucleotides.

In an embodiment, the targeting domain comprises 23 nucleotides.

In an embodiment, the targeting domain comprises 24 nucleotides.

In an embodiment, the targeting domain comprises 25 nucleotides.

In an embodiment, the targeting domain comprises 26 nucleotides.

A gRNA as described herein may comprise from 5' to 3': a targeting domain (comprising a "core domain", and optionally a "secondary domain"); a first complementarity domain; a linking domain; a second complementarity domain; a proximal domain; and a tail domain. In some embodiments, the proximal domain and tail domain are taken together as a single domain.

In an embodiment, a gRNA comprises a linking domain of no more than 25 nucleotides in length; a proximal and tail domain, that taken together, are at least 20 nucleotides in length; and a targeting domain equal to or greather than 16, 17, 18, 19, 20, 21, 22, 23, 24, 25 or 26nucleotides in length.

In another embodiment, a gRNA comprises a linking domain of no more than 25 nucleotides in length; a proximal and tail domain, that taken together, are at least 25 nucleotides in length; and a targeting domain equal to or greather than 16, 17, 18, 19, 20, 21, 22, 23, 24, 25 or 26nucleotides in length.

In another embodiment, a gRNA comprises a linking domain of no more than 25 nucleotides in length; a proximal and tail domain, that taken together, are at least 30 nucleotides in length; and a targeting domain equal to or greather than 16, 17, 18, 19, 20, 21, 22, 23, 24, 25 or 26nucleotides in length.

In another embodiment, a gRNA comprises a linking domain of no more than 25 nucleotides in length; a proximal and tail domain, that taken together, are at least 40 nucleotides in length; and a targeting domain equal to or greather than 16, 17, 18, 19, 20, 21, 22, 23, 24, 25 or 26nucleotides in length.

A cleavage event, e.g., a double strand or single strand break, is generated by a Cas9 molecule. The Cas9 molecule may be an enzymatically active Cas9 (eaCas9) molecule, e.g., an eaCas9 molecule that forms a double strand break in a target nucleic acid or an eaCas9 molecule forms a single strand break in a target nucleic acid (e.g., a nickase molecule). Alternatively, in an embodiment, the Cas9 molecule may be an enzymatically inactive Cas9 (eiCas9) molecule or a modified eiCas9 molecule, e.g., the eiCas9 molecule is fused to Krüppel-associated box (KRAB) to generate an eiCas9-KRAB fusion protein molecule.

In an embodiment, the eaCas9 molecule catalyzes a double strand break.

In some embodiments, the eaCas9 molecule comprises HNH-like domain cleavage activity but has no, or no significant, N-terminal RuvC-like domain cleavage activity. In an embodiment, the eaCas9 molecule is an HNH-like domain nickase, e.g., the eaCas9 molecule comprises a mutation at D10, e.g., D10A. In another embodiment, the eaCas9 molecule comprises N-terminal RuvC-like domain cleavage activity but has no, or no significant, HNH-like domain cleavage activity. In an embodiment, the eaCas9 molecule is an N-terminal RuvC-like domain nickase, e.g., the eaCas9 molecule comprises a mutation at H840, e.g., H840A. In an embodiment, the eaCas9 molecule is an N-terminal RuvC-like domain nickase, e.g., the eaCas9 molecule comprises a mutation at N863, e.g., an N863A mutation.

In an embodiment, a single strand break is formed in the strand of the target nucleic acid to which the targeting domain of said gRNA is complementary. In another embodiment, a single strand break is formed in the strand of the target nucleic acid other than the strand to which the targeting domain of said gRNA is complementary.

In another aspect, disclosed herein is a nucleic acid, e.g., an isolated or non-naturally occurring nucleic acid, e.g., DNA, that comprises (a) a sequence that encodes a gRNA molecule comprising a targeting domain that is complementary with a POAG target position in the *MYOC* gene as disclosed herein.

In an embodiment, the nucleic acid encodes a gRNA molecule, e.g., a first gRNA molecule, comprising a targeting domain configured to provide a cleavage event, e.g., a double strand break or a single strand break, sufficiently close to a POAG target position in the *MYOC* gene to allow alteration, e.g., alteration associated with HDR or NHEJ, of a POAG target position in the *MYOC* gene.

In an embodiment, the nucleic acid encodes a gRNA molecule, e.g., a first gRNA molecule, comprising a targeting domain configured to target an enzymatically inactive Cas9 (eiCas9) molecule or an eiCas9 fusion protein (e.g., an eiCas9 fused to a transcription repressor domain), sufficiently close to a POAG target knockdown position to reduce, decrease or repress expression of the *MYOC* gene.

In an embodiment, the nucleic acid encodes a gRNA molecule, e.g., the first gRNA molecule, comprising a targeting domain comprising a sequence that is the same as, or differs by no more than 1, 2, 3, 4, or 5 nucleotides from, a targeting domain sequence from any one of **1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E,** or **23A-23B. In** an embodiment, the nucleic acid encodes a gRNA molecule comprising a targeting domain is selected from those in **1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E,** or **23A-23B.**

In an embodiment, the nucleic acid encodes a modular gRNA, e.g., one or more nucleic acids encode a modular gRNA. In another embodiment, the nucleic acid encodes a chimeric gRNA. The nucleic acid may encode a gRNA, e.g., the first gRNA molecule, comprising a targeting domain comprising 16 nucleotides or more in length. In an embodiment, the nucleic acid encodes a gRNA, e.g., the first gRNA molecule, comprising a targeting domain that is 16 nucleotides in length. In another embodiment, the nucleic acid encodes a gRNA, e.g., the first gRNA molecule, comprising a targeting domain that is 17 nucleotides in length. In another embodiment, the nucleic acid encodes a gRNA, e.g., the first gRNA molecule, comprising a targeting domain that is 18 nucleotides in length. In still another embodiment, the nucleic acid encodes a gRNA, e.g., the first gRNA molecule, comprising a targeting domain that is 19 nucleotides in length. In still another embodiment, the nucleic acid encodes a gRNA, e.g., the first gRNA molecule, comprising a targeting domain that is 20 nucleotides in length. In still another embodiment, the nucleic acid encodes a gRNA, e.g., the first gRNA molecule, comprising a targeting domain that is 21 nucleotides in length. In still another embodiment, the nucleic acid encodes a gRNA, e.g., the first gRNA molecule, comprising a targeting domain that is 22 nucleotides in length. In still another embodiment, the nucleic acid encodes a gRNA, e.g., the first gRNA molecule, comprising a targeting domain that is 23 nucleotides in length. In still another embodiment, the nucleic acid encodes a gRNA, e.g., the first gRNA molecule, comprising a targeting domain that is 24 nucleotides in length. In still another embodiment, the nucleic acid encodes a gRNA, e.g., the first gRNA molecule, comprising a targeting domain that is 25 nucleotides in length. In still another embodiment, the nucleic acid encodes a gRNA, e.g., the first gRNA molecule, comprising a targeting domain that is 26 nucleotides in length.

In an embodiment, the targeting domain comprises 16 nucleotides.

In an embodiment, the targeting domain comprises 17 nucleotides.

In an embodiment, the targeting domain comprises 18 nucleotides.

In an embodiment, the targeting domain comprises 19 nucleotides.

In an embodiment, the targeting domain comprises 20 nucleotides.

In an embodiment, the targeting domain comprises 21 nucleotides.

In an embodiment, the targeting domain comprises 22 nucleotides.

In an embodiment, the targeting domain comprises 23 nucleotides.

In an embodiment, the targeting domain comprises 24 nucleotides.

In an embodiment, the targeting domain comprises 25 nucleotides.

In an embodiment, the targeting domain comprises 26 nucleotides.

In an embodiment, a nucleic acid encodes a gRNA comprising from 5' to 3': a targeting domain (comprising a "core domain", and optionally a "secondary domain"); a first complementarity domain; a linking domain; a second complementarity domain; a proximal domain; and a tail domain. In some embodiments, the proximal domain and tail domain are taken together as a single domain.

In an embodiment, a nucleic acid encodes a gRNA e.g., the first gRNA molecule, comprising a linking domain of no more than 25 nucleotides in length; a proximal and tail domain, that taken together, are at least 20 nucleotides in length; and a targeting domain equal to or greater than 16, 17, 18, 19, 20, 21, 22, 23, 24, 25 or 26 nucleotides in length.

In an embodiment, a nucleic acid encodes a gRNA e.g., the first gRNA molecule, comprising a linking domain of no more than 25 nucleotides in length; a proximal and tail domain, that taken together, are at least 25 nucleotides in length; and a targeting domain equal to or greater than 16, 17, 18, 19, 20, 21, 22, 23, 24, 25 or 26 nucleotides in length.

In an embodiment, a nucleic acid encodes a gRNA e.g., the first gRNA molecule, comprising a linking domain of no more than 25 nucleotides in length; a proximal and tail domain, that taken together, are at least 30 nucleotides in length; and a targeting domain equal to or greater than 16, 17, 18, 19, 20, 21, 22, 23, 24, 25 or 26 nucleotides in length.

In an embodiment, a nucleic acid encodes a gRNA comprising e.g., the first gRNA molecule, a linking domain of no more than 25 nucleotides in length; a proximal and tail domain, that taken together, are at least 40 nucleotides in length; and a targeting domain equal to or greater than 16, 17, 18, 19, 20, 21, 22, 23, 24, 25 or 26 nucleotides in length.

In an embodiment, a nucleic acid comprises (a) a sequence that encodes a gRNA molecule e.g., the first gRNA molecule, comprising a targeting domain that is complementary with a target domain in the *MYOC* gene as disclosed herein, and further comprising (b) a sequence that encodes a Cas9 molecule.

The Cas9 molecule may be an enzymatically active Cas9 (eaCas9) molecule, e.g., an eaCas9 molecule that forms a double strand break in a target nucleic acid or an eaCas9 molecule that forms a single strand break in a target nucleic acid (e.g., a nickase molecule). In an embodiment, a single strand break is formed in the strand of the target nucleic acid to which the targeting domain of said gRNA is complementary. In another embodiment, a single strand break is formed in the strand of the target nucleic acid other than the strand to which to which the targeting domain of said gRNA is complementary.

In an embodiment, the eaCas9 molecule catalyzes a double strand break.

In an embodiment, the eaCas9 molecule comprises HNH-like domain cleavage activity but has no, or no significant, N-terminal RuvC-like domain cleavage activity. In another embodiment, the said eaCas9 molecule is an HNH-like domain nickase, e.g., the eaCas9 molecule comprises a mutation at D10, e.g., D10A. In another embodiment, the eaCas9 molecule comprises N-terminal RuvC-like domain cleavage activity but has no, or no significant, HNH-like domain cleavage activity. In another embodiment, the eaCas9 molecule is an N-terminal RuvC-like domain nickase, e.g., the eaCas9 molecule comprises a mutation at H840, e.g., H840A. In another embodiment, the eaCas9 molecule is an N-terminal RuvC-like domain nickase, e.g., the eaCas9 molecule comprises a mutation at N863, e.g., an N863A mutation.

A nucleic acid disclosed herein may comprise (a) a sequence that encodes a gRNA molecule comprising a targeting domain that is complementary with a target domain in the *BCL11A* gene as disclosed herein; (b) a sequence that encodes a Cas9 molecule.

Alternatively, in an embodiment, the Cas9 molecule may be an enzymatically inactive Cas9 (eiCas9) molecule or a modified eiCas9 molecule, e.g., the eiCas9 molecule is fused to Krüppel-associated box (KRAB) to generate an eiCas9-KRAB fusion protein molecule.

A nucleic acid disclosed herein may comprise (a) a sequence that encodes a gRNA molecule comprising a targeting domain that is complementary with a target domain in the *MYOC* gene as disclosed herein; (b) a sequence that encodes a Cas9 molecule; and further may comprise (c)(i) a sequence that encodes a second gRNA molecule described herein having a targeting domain that is complementary to a second target domain of the *MYOC* gene, and optionally, (c)(ii) a sequence that encodes a third gRNA molecule described herein having a targeting domain that is complementary to a third target domain of the *MYOC* gene; and optionally, (c)(iii) a sequence that encodes a fourth gRNA molecule described herein having a targeting domain that is complementary to a fourth target domain of the *MYOC* gene.

In an embodiment, a nucleic acid encodes a second gRNA molecule comprising a targeting domain configured to provide a cleavage event, e.g., a double strand break or a single strand break, sufficiently close to a POAG target position in the *MYOC* gene, to allow alteration, e.g., alteration associated with HDR or NHEJ, of a POAG target position in the *MYOC* gene, either alone or in combination with the break positioned by said first gRNA molecule.

In an embodiment, the nucleic acid encodes a second gRNA molecule comprising a targeting domain configured to target an enzymatically inactive Cas9 (eiCas9) molecule or an eiCas9 fusion protein (e.g., an eiCas9 fused to a transcription repressor domain), sufficiently close to a POAG target knockdown position to reduce, decrease or repress expression of the *MYOC* gene.

In an embodiment, a nucleic acid encodes a third gRNA molecule comprising a targeting domain configured to provide a cleavage event, e.g., a double strand break or a single strand break, sufficiently close to a POAG target position in the *MYOC* gene to allow alteration, e.g., alteration associated with HDR or NHEJ, of a POAG target position in the *MYOC* gene, either alone or in combination with the break positioned by the first and/or second gRNA molecule.

In an embodiment, the nucleic acid encodes a third gRNA molecule comprising a targeting domain configured to target an enzymatically inactive Cas9 (eiCas9) molecule or an eiCas9 fusion protein (e.g., an eiCas9 fused to a transcription repressor domain), sufficiently close to a POAG target knockdown position to reduce, decrease or repress expression of the *BCL11A* gene.

In an embodiment, a nucleic acid encodes a fourth gRNA molecule comprising a targeting domain configured to provide a cleavage event, e.g., a double strand break or a single strand break, sufficiently close to a POAG target position in the *MYOC* gene to allow alteration, e.g., alteration associated with HDR or NHEJ, of a POAG target position in the *MYOC* gene, either alone or in combination with the break positioned by the first gRNA molecule, the second gRNA molecule and/or the third gRNA molecule.

In an embodiment, the nucleic acid encodes a fourth gRNA molecule comprising a targeting domain configured to target an enzymatically inactive Cas9 (eiCas9) or an eiCas9 fusion protein (e.g., an eiCas9 fused to a transcription repressor domain), sufficiently close to a POAG target knockdown position to reduce, decrease or repress expression of the *MYOC* gene.

In an embodiment, the nucleic acid encodes a second gRNA molecule. The second gRNA is selected to target the same POAG target position as the first gRNA molecule. Optionally, the nucleic acid may encode a third gRNA, and further optionally, the nucleic acid may encode a fourth gRNA molecule. The third gRNA molecule and the fourth gRNA molecule are selected to target the same POAG target position as the first and second gRNA molecules.

In an embodiment, the nucleic acid encodes a second gRNA molecule comprising a targeting domain comprising a sequence that is the same as, or differs by no more than 1, 2, 3, 4, or 5 nucleotides from, a targeting domain sequence from one of **Tables 1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E,** or **23A-23B.** In an embodiment, the nucleic acid encodes a second gRNA molecule comprising a targeting domain selected from those in **Tables 1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E,** or **23A-23B.** In an embodiment, when a third or fourth gRNA molecule are present, the third and fourth gRNA molecules may independently comprise a targeting domain comprising a sequence that is the same as, or differs by no more than 1, 2, 3, 4, or 5 nucleotides from, a targeting domain sequence from one of **Tables 1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E,** or **23A-23B.** In a further embodiment, when a third or fourth gRNA molecule are present, the third and fourth gRNA molecules may independently comprise a targeting domain selected from those in **Tables 1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E,** or **23A-23B.**

In an embodiment, the nucleic acid encodes a second gRNA which is a modular gRNA, e.g., wherein one or more nucleic acid molecules encode a modular gRNA. In another embodiment, the nucleic acid encoding a second gRNA is a chimeric gRNA. In another embodiment, when a nucleic acid encodes a third or fourth gRNA, the third and fourth gRNA may be a modular gRNA or a chimeric gRNA. When multiple gRNAs are used, any combination of modular or chimeric gRNAs may be used.

A nucleic acid may encode a second, a third, and/or a fourth gRNA, each independently, comprising a targeting domain comprising 16 nucleotides or more in length. In an embodiment, the nucleic acid encodes a second gRNA comprising a targeting domain that is 16 nucleotides in length. In an embodiment, the nucleic acid encodes a second gRNA comprising a targeting domain that is 17 nucleotides in length. In another embodiment, the nucleic acid encodes a second gRNA comprising a targeting domain that is 18 nucleotides in length. In still another embodiment, the nucleic acid encodes a second gRNA comprising a targeting domain that is 19 nucleotides in length. In still another embodiment, the nucleic acid encodes a second gRNA comprising a targeting domain that is 20 nucleotides in length. In still another embodiment, the nucleic acid encodes a second gRNA comprising a targeting domain that is 21 nucleotides in length. In still another embodiment, the nucleic acid encodes a second gRNA comprising a targeting domain that is 22 nucleotides in length. In still another embodiment, the nucleic acid encodes a second gRNA comprising a targeting domain that is 23 nucleotides in length. In still another embodiment, the nucleic acid encodes a second gRNA comprising a targeting domain that is 24 nucleotides in length. In still another embodiment, the nucleic acid encodes a second gRNA comprising a targeting domain that is 25 nucleotides in length. In still another embodiment, the nucleic acid encodes a second gRNA comprising a targeting domain that is 26 nucleotides in length.

In an embodiment, the targeting domain comprises 16 nucleotides.

In an embodiment, the targeting domain comprises 17 nucleotides.

In an embodiment, the targeting domain comprises 18 nucleotides.

In an embodiment, the targeting domain comprises 19 nucleotides.

In an embodiment, the targeting domain comprises 20 nucleotides.

In an embodiment, the targeting domain comprises 21 nucleotides.

In an embodiment, the targeting domain comprises 22 nucleotides.

In an embodiment, the targeting domain comprises 23 nucleotides.

In an embodiment, the targeting domain comprises 24 nucleotides.

In an embodiment, the targeting domain comprises 25 nucleotides.

In an embodiment, the targeting domain comprises 26 nucleotides.

In an embodiment, a nucleic acid encodes a second, a third, and/or a fourth gRNA, each independently, comprising from 5' to 3': a targeting domain (comprising a "core domain", and optionally a "secondary domain"); a first complementarity domain; a linking domain; a second complementarity domain; a proximal domain; and a tail domain. In some embodiments, the proximal domain and tail domain are taken together as a single domain.

In an embodiment, a nucleic acid encodes a second, a third, and/or a fourth gRNA comprising a linking domain of no more than 25 nucleotides in length; a proximal and tail domain, that taken together, are at least 20 nucleotides in length; and a targeting domain equal to or greater than16, 17, 18, 19, 20, 21, 22, 23, 24, 25 or 26nucleotides in length.

In an embodiment, a nucleic acid encodes a second, a third, and/or a fourth gRNA comprising a linking domain of no more than 25 nucleotides in length; a proximal and tail domain, that taken together, are at least 25 nucleotides in length; and a targeting domain equal to or greater than16, 17, 18, 19, 20, 21, 22, 23, 24, 25 or 26nucleotides in length.

In an embodiment, a nucleic acid encodes a second, a third, and/or a fourth gRNA comprising a linking domain of no more than 25 nucleotides in length; a proximal and tail domain, that taken together, are at least 30 nucleotides in length; and a targeting domain equal to or greater than16, 17, 18, 19, 20, 21, 22, 23, 24, 25 or 26nucleotides in length.

In an embodiment, a nucleic acid encodes a second, a third, and/or a fourth gRNA comprising a linking domain of no more than 25 nucleotides in length; a proximal and tail domain, that taken together, are at least 40 nucleotides in length; and a targeting domain equal to or greater than16, 17, 18, 19, 20, 21, 22, 23, 24, 25 or 26nucleotides in length.

In an embodiment, when the *MYOC* gene is corrected by HDR, the nucleic acid encodes (a) a sequence that encodes a gRNA molecule comprising a targeting domain that is complementary with a target domain in the *MYOC* gene as disclosed herein; (b) a sequence that encodes a Cas9 molecule; optionally, (c)(i) a sequence that encodes a second gRNA molecule described herein having a targeting domain that is complementary to a second target domain of the *MYOC* gene, and further optionally, (c)(ii) a sequence that encodes a third gRNA molecule described herein having a targeting domain that is complementary to a third target domain of the *MYOC* gene; and still further optionally, (c)(iii) a sequence that encodes a fourth gRNA molecule described herein having a targeting domain that is complementary to a fourth target domain of the *MYOC* gene; and further may comprise (d) a template nucleic acid, e.g., a template nucleic acid described herein.

In an embodiment, the template nucleic acid is a single stranded nucleic acid. In another embodiment, the template nucleic acid is a double stranded nucleic acid. In another embodiment, the template nucleic acid comprises a nucleotide sequence, e.g., of one or more nucleotides, that will be added to or will template a change in the target nucleic acid. In another embodiment, the template nucleic acid comprises a nucleotide sequence that may be used to modify the target position. In another embodiment, the template nucleic acid comprises a nucleotide sequence, e.g., of one or more nucleotides, that corresponds to wild type sequence of the target nucleic acid, e.g., of the target position.

The template nucleic acid may comprise a replacement sequence, e.g., a replacement sequence from the **Table 24.** In some embodiments, the template nucleic acid comprises a 5' homology arm, e.g., a 5' homology arm from **Table 24.** In other embodiments, the template nucleic acid comprises a 3' homology arm, e.g., a 3' homology arm from **Table 24.**

In an embodiment, a nucleic acid encodes (a) a sequence that encodes a gRNA molecule comprising a targeting domain that is complementary with a target domain in the *MYOC* gene as disclosed herein, and (b) a sequence that encodes a Cas9 molecule, e.g., a Cas9 molecule described herein. In an embodiment, (a) and (b) are present on the same nucleic acid molecule, e.g., the same vector, e.g., the same viral vector, e.g., the same adeno-associated virus (AAV) vector. In an embodiment, the nucleic acid molecule is an AAV vector. Exemplary AAV vectors that may be used in any of the described compositions and methods include an AAV2 vector, a modified AAV2 vector, an AAV3 vector, a modified AAV3 vector, an AAV6 vector, a modified AAV6 vector, an AAV8 vector and an AAV9 vector.

In another embodiment, (a) is present on a first nucleic acid molecule, e.g. a first vector, e.g., a first viral vector, e.g., a first AAV vector; and (b) is present on a second nucleic acid molecule, e.g., a second vector, e.g., a second vector, e.g., a second AAV vector. The first and second nucleic acid molecules may be AAV vectors.

In another embodiment, a nucleic acid encodes (a) a sequence that encodes a gRNA molecule comprising a targeting domain that is complementary with a target domain in the *MYOC* gene as disclosed herein, and (b) a sequence that encodes a Cas9 molecule, e.g., a Cas9 molecule described herein; and further comprise (c)(i) a sequence that encodes a second gRNA molecule as described herein and optionally, (c)(ii) a sequence that encodes a third gRNA molecule described herein having a targeting domain that is complementary to a third target domain of the *MYOC* gene; and optionally, (c)(iii) a sequence that encodes a fourth gRNA molecule described herein having a targeting domain that is complementary to a fourth target domain of the *MYOC* gene. In some embodiments, the nucleic acid comprises (a), (b) and (c)(i). In an embodiment, the nucleic acid comprises (a), (b), (c)(i) and (c)(ii). In an embodiment, the nucleic acid comprises (a), (b), (c)(i), (c)(ii) and (c)(iii). Each of (a) and (c)(i), (c)(ii) and/or (c)(iii) may be present on the same nucleic acid molecule, e.g., the same vector, e.g., the same viral vector, e.g., the same adeno-associated virus (AAV) vector. In an embodiment, the nucleic acid molecule is an AAV vector.

In another embodiment, (a) and (c)(i) are on different vectors. For example, (a) may be present on a first nucleic acid molecule, e.g. a first vector, e.g., a first viral vector, e.g., a first AAV vector; and (c)(i) may be present on a second nucleic acid molecule, e.g., a second vector, e.g., a second vector, e.g., a second AAV vector. In an embodiment, the first and second nucleic acid molecules are AAV vectors.

In another embodiment, each of (a), (b), and (c)(i) are present on the same nucleic acid molecule, e.g., the same vector, e.g., the same viral vector, e.g., an AAV vector. In an embodiment, the nucleic acid molecule is an AAV vector. In an alternate embodiment, one of (a), (b), and (c)(i) is encoded on a first nucleic acid molecule, e.g., a first vector, e.g., a first viral vector, e.g., a first AAV vector; and a second and third of (a), (b), and (c)(i) is encoded on a second nucleic acid molecule, e.g., a second vector, e.g., a second vector, e.g., a second AAV vector. The first and second nucleic acid molecule may be AAV vectors.

In an embodiment, (a) is present on a first nucleic acid molecule, e.g., a first vector, e.g., a first viral vector, a first AAV vector; and (b) and (c)(i) are present on a second nucleic acid molecule, e.g., a second vector, e.g., a second vector, e.g., a second AAV vector. The first and second nucleic acid molecule may be AAV vectors.

In another embodiment, (b) is present on a first nucleic acid molecule, e.g., a first vector, e.g., a first viral vector, e.g., a first AAV vector; and (a) and (c)(i) are present on a second nucleic acid molecule, e.g., a second vector, e.g., a second vector, e.g., a second AAV vector. The first and second nucleic acid molecule may be AAV vectors.

In another embodiment, (c)(i) is present on a first nucleic acid molecule, e.g., a first vector, e.g., a first viral vector, e.g., a first AAV vector; and (b) and (a) are present on a second nucleic acid molecule, e.g., a second vector, e.g., a second vector, e.g., a second AAV vector. The first and second nucleic acid molecule may be AAV vectors.

In another embodiment, each of (a), (b) and (c)(i) are present on different nucleic acid molecules, e.g., different vectors, e.g., different viral vectors, e.g., different AAV vector. For example, (a) may be on a first nucleic acid molecule, (b) on a second nucleic acid molecule, and (c)(i) on a third nucleic acid molecule. The first, second and third nucleic acid molecule may be AAV vectors.

In another embodiment, when a third and/or fourth gRNA molecule are present, each of (a), (b), (c)(i), (c)(ii) and (c)(iii) may be present on the same nucleic acid molecule, e.g., the same vector, e.g., the same viral vector, e.g., an AAV vector. In an embodiment, the nucleic acid molecule is an AAV vector. In an alternate embodiment, each of (a), (b), (c)(i), (c)(ii) and (c)(iii) may be present on the different nucleic acid molecules, e.g., different vectors, e.g., the different viral vectors, e.g., different AAV vectors. In a further embodiment, each of (a), (b), (c)(i), (c)(ii) and (c)(iii) may be present on more than one nucleic acid molecule, but fewer than five nucleic acid molecules, e.g., AAV vectors.

In another embodiment, when (d) a template nucleic acid is present, each of (a), (b), and (d) may be present on the same nucleic acid molecule, e.g., the same vector, e.g., the same viral vector, e.g., an AAV vector. In an embodiment, the nucleic acid molecule is an AAV vector. In an alternate embodiment, each of (a), (b), and (d) may be present on the different nucleic acid molecules, e.g., different vectors, e.g., the different viral vectors, e.g., different AAV vectors. In a further embodiment, each of (a), (b), and (d) may be present on more than one nucleic acid molecule, but fewer than three nucleic acid molecules, e.g., AAV vectors.

In another embodiment, when (d) a template nucleic acid is present, each of (a), (b), (c)(i) and (d) may be present on the same nucleic acid molecule, e.g., the same vector, e.g., the same viral vector, e.g., an AAV vector. In an embodiment, the nucleic acid molecule is an AAV vector. In an alternate embodiment, each of (a), (b), (c)(i) and (d) may be present on the different nucleic acid molecules, e.g., different vectors, e.g., the different viral vectors, e.g., different AAV vectors. In a further embodiment, each of (a), (b), (c)(i) and (d) may be present on more than one nucleic acid molecule, but fewer than four nucleic acid molecules, e.g., AAV vectors.

In another embodiment, when (d) a template nucleic acid is present, each of (a), (b), (c)(i), (c)(ii) and (d) may be present on the same nucleic acid molecule, e.g., the same vector, e.g., the same viral vector, e.g., an AAV vector. In an embodiment, the nucleic acid molecule is an AAV vector. In an alternate embodiment, each of (a), (b), (c)(i), (c)(ii) and (d) may be present on the different nucleic acid molecules, e.g., different vectors, e.g., the different viral vectors, e.g., different AAV vectors. In a further embodiment, each of (a), (b), (c)(i), (c)(ii) and (d) may be present on more than one nucleic acid molecule, but fewer than five nucleic acid molecules, e.g., AAV vectors.

In another embodiment, when (d) a template nucleic acid is present, each of (a), (b), (c)(i), (c)(ii), (c)(iii) and (d) may be present on the same nucleic acid molecule, e.g., the same vector, e.g., the same viral vector, e.g., an AAV vector. In an embodiment, the nucleic acid molecule is an AAV vector. In an alternate embodiment, each of (a), (b), (c)(i), (c)(ii), (c)(iii) and (d) may be present on the different nucleic acid molecules, e.g., different vectors, e.g., the different viral vectors, e.g., different AAV vectors. In a further embodiment, each of (a), (b), (c)(i), (c)(ii), (c)(iii) and (d) may be present on more than one nucleic acid molecule, but fewer than six nucleic acid molecules, e.g., AAV vectors.

The nucleic acids described herein may comprise a promoter operably linked to the sequence that encodes the gRNA molecule of (a), e.g., a promoter described herein. The nucleic acid may further comprise a second promoter operably linked to the sequence that encodes the second, third and/or fourth gRNA molecule of (c), e.g., a promoter described herein. The promoter and second promoter differ from one another. In some embodiments, the promoter and second promoter are the same.

The nucleic acids described herein may further comprise a promoter operably linked to the sequence that encodes the Cas9 molecule of (b), e.g., a promoter described herein.

In another aspect, disclosed herein is a composition comprising (a) a gRNA molecule comprising a targeting domain that is complementary with a target domain in the *MYOC* gene, as described herein. The composition of (a) may further comprise (b) a Cas9 molecule, e.g., a Cas9 molecule as described herein. A composition of (a) and (b) may further comprise (c) a second, third and/or fourth gRNA molecule, e.g., a second, third and/or fourth gRNA molecule described herein. A composition of (a), (b) and (c) a second, third and/or fourth gRNA molecule, e.g., a second, third and/or fourth gRNA molecule may further comprise (d) a template nucleic acid, e.g., a template nucleic acid described herein. In an embodiment, the composition is a pharmaceutical composition. The compositions described herein, e.g., pharmaceutical compositions described herein, can be used in the treatment or prevention of POAG in a subject, e.g., in accordance with a method disclosed herein..

In another aspect, disclosed herein is a method of altering a cell, e.g., altering the structure, e.g., altering the sequence, of a target nucleic acid of a cell, comprising contacting said cell with: (a) a gRNA that targets the *MYOC* gene, e.g., a gRNA as described herein; (b) a Cas9 molecule, e.g., a Cas9 molecule as described herein; and optionally, (c) a second, third and/or fourth gRNA that targets *MYOC* gene, e.g., a second third and/or fourth gRNA as described herein; and optionally, (d) a template nucleic acid, as described herein.

In an embodiment, the method comprises contacting said cell with (a) and (b).

In an embodiment, the method comprises contacting said cell with (a), (b), and (c).

In an embodiment, the method comprises contacting said cell with (a), (b), (c) and (d).

The gRNA of (a) and optionally (c) may be selected from any of **1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E,** or **23A-23B,** or a gRNA that differs by no more than 1, 2, 3, 4, or 5 nucleotides from, a targeting domain sequence from any of **1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E, or 23A-23B.**

In an embodiment, the method comprises contacting a cell from a subject suffering from or likely to develop POAG. The cell may be from a subject having a mutation at a POAG target position in the *MYOC* gene.

In an embodiment, the cell being contacted in the disclosed method is a target cell from the eye of the subject. The cell may be a trabecular meshwork cell, retinal pigment epithelial cell, a retinal cell, an iris cell, a ciliary body cell and/or the optic nerve. The contacting may be performed *ex vivo* and the contacted cell may be returned to the subject's body after the contacting step. In other embodiments, the contacting step may be performed *in vivo.*

In an embodiment, the method of altering a cell as described herein comprises acquiring knowledge of the presence of a mutation at a POAG target position in said cell, prior to the contacting step. Acquiring knowledge of the presence of a mutation at a POAG target position in the cell may be by sequencing the *MYOC* gene, or a portion of the *MYOC* gene.

In an embodiment, the contacting step of the method comprises contacting the cell with a nucleic acid, e.g., a vector, e.g., an AAV vector, that expresses at least one of (a), (b), and (c). In an embodiment, the contacting step of the method comprises contacting the cell with a nucleic acid, e.g., a vector, e.g., an AAV vector, that expresses each of (a), (b), and (c). In another embodiment, the contacting step of the method comprises delivering to the cell a Cas9 molecule of (b) and a nucleic acid which encodes a gRNA (a) and optionally, a second gRNA (c)(i) (and further optionally, a third gRNA (c)(ii) and/or fourth gRNA (c)(iii).

In an embodiment, the contacting step of the method comprises contacting the cell with a nucleic acid, e.g., a vector, e.g., an AAV vector, that expresses at least one of (a), (b), (c) and (d). In an embodiment, the contacting step of the method comprises contacting the cell with a nucleic acid, e.g., a vector, e.g., an AAV vector, that expresses each of (a), (b), and (c). In another embodiment, the contacting step of the method comprises delivering to the cell a Cas9 molecule of (b), a nucleic acid which encodes a gRNA of (a) and a template nucleic acid of (d), and optionally, a second gRNA (c)(i) (and further optionally, a third gRNA (c)(ii) and/or fourth gRNA (c)(iii).

In an embodiment, contacting comprises contacting the cell with a nucleic acid, e.g., a vector, e.g., an AAV vector, e.g., an AAV2 vector, a modified AAV2 vector, an AAV3 vector, a modified AAV3 vector, an AAV6 vector, a modified AAV6 vector, an AAV8 vector or an AAV9 vector, as described herein.

In an embodiment, contacting comprises delivering to the cell a Cas9 molecule of (b), as a protein or an mRNA, and a nucleic acid which encodes a gRNA of (a) and optionally a second, third and/or fourth gRNA (c).

In an embodiment, contacting comprises delivering to the cell a Cas9 molecule of (b), as a protein or an mRNA, said gRNA of (a), as an RNA, and optionally said second, third and/or fourth gRNA of (c), as an RNA.

In an embodiment, contacting comprises delivering to the cell a gRNA of (a) as an RNA, optionally said second, third and/or fourth gRNA of (c) as an RNA, and a nucleic acid that encodes the Cas9 molecule of (b).

In another aspect, disclosed herein is a method of treating a subject suffering from or likely to develop POAG, e.g., altering the structure, e.g., sequence, of a target nucleic acid of the subject, comprising contacting the subject (or a cell from the subject) with:
(a) a gRNA that targets the *MYOC* gene, e.g., a gRNA disclosed herein;
(b) a Cas9 molecule, e.g., a Cas9 molecule disclosed herein; and
optionally, (c)(i) a second gRNA that targets the *MYOC* gene, e.g., a second gRNA disclosed herein, and
further optionally, (c)(ii) a third gRNA, and still further optionally, (c)(iii) a fourth gRNA that target the MYOC gene, e.g., a third and fourth gRNA disclosed herein.

The method of treating a subject may further comprise contacting the subject (or a cell from the subject) with (d) a template nucleic acid, e.g., a template nucleic acid disclosed herein. A template nucleic acid is used when the method of treating a subject uses HDR to alter the sequence of the target nucleic acid of the subject.

In some embodiments, contacting comprises contacting with (a) and (b).

In some embodiments, contacting comprises contacting with (a), (b), and (c)(i).

In some embodiments, contacting comprises contacting with (a), (b), (c)(i) and (c)(ii).

In some embodiments, contacting comprises contacting with (a), (b), (c)(i), (c)(ii) and (c)(iii).

In some embodiments, contacting comprises contacting with (a), (b), (c)(i) and (d).

In some embodiments, contacting comprises contacting with (a), (b), (c)(i), (c)(ii) and (d).

In some embodiments, contacting comprises contacting with (a), (b), (c)(i), (c)(ii), (c)(iii) and (d).

The gRNA of (a) or (c) (e.g., (c)(i), (c)(ii), or (c)(iii) may be selected from any of **1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E,** or **23A-23B,** or a gRNA that differs by no more than 1, 2, 3, 4, or 5 nucleotides from, a targeting domain sequence from any of **1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E, or 23A-23B.**

In an embodiment, the method comprises acquiring knowledge of the presence of a mutation at a POAG target position in said subject.

In an embodiment, the method comprises acquiring knowledge of the presence of a mutation at a POAG target position in said subject by sequencing the *MYOC* gene or a portion of the *MYOC* gene.

In an embodiment, the method comprises correcting a mutation at a POAG target position.

In an embodiment, the method comprises correcting a mutation at a POAG target position by HDR.

In an embodiment, the method comprises correcting a mutation at a POAG target position by NHEJ.

When the method comprises correcting the mutation at a POAG target position by HDR, a Cas9 of (b), at least one guide RNA (e.g., a guide RNA of (a) and a template nucleic acid of (d) are included in the contacting step.

In an embodiment, a cell of the subject is contacted *ex vivo* with (a), (b), (d) and optionally (c). In an embodiment, said cell is returned to the subject's body.

In an embodiment, a cell of the subject is contacted is *in vivo* with (a), (b) (d) and optionally (c)(i), further optionally (c)(ii), and still further optionally (c)(iii).

In an embodiment, the cell of the subject is contacted *in vivo* by subretinal delivery of (a), (b), (d) and optionally (c)(i), further optionally (c)(ii), and still further optionally (c)(iii).

In an embodiment, the contacting step comprises contacting the subject with a nucleic acid, e.g., a vector, e.g., an AAV vector, described herein, e.g., a nucleic acid that encodes at least one of (a), (b), (d) and optionally (c)(i), further optionally (c)(ii), and still further optionally (c)(iii).

In an embodiment, the contacting step comprises delivering to said subject said Cas9 molecule of (b), as a protein or mRNA, and a nucleic acid which encodes (a), a nucleic acid of (d) and optionally (c)(i), further optionally (c)(ii), and still further optionally (c)(iii).

In an embodiment, the contacting step comprises delivering to the subject the Cas9 molecule of (b), as a protein or mRNA, the gRNA of (a), as an RNA, a nucleic acid of (d) and optionally the second gRNA of (c)(i), further optionally said third gRNA of (c)(ii), and still further optionally said fourth gRNA of (c)(iii), as an RNA.

In an embodiment, the contacting step comprises delivering to the subject the gRNA of (a), as an RNA, optionally said second gRNA of (c)(i), further optionally said third gRNA of (c)(ii), and still further optionally said fourth gRNA of (c)(iii), as an RNA, a nucleic acid that encodes the Cas9 molecule of (b), and a nucleic acid of (d).

When the method comprises (1) correcting the mutation at a POAG target position by NHEJ or (2) knocking down expression of the *MYOC* gene by targeting the promoter region, a Cas9 of (b) and at least one guide RNA (e.g., a guide RNA of (a) are included in the contacting step.

In an embodiment, a cell of the subject is contacted *ex vivo* with (a), (b) and optionally (c)(i), further optionally (c)(ii), and still further optionally (c)(iii). In an embodiment, said cell is returned to the subject's body.

In an embodiment, a cell of the subject is contacted is *in vivo* with (a), (b) and optionally (c)(i), further optionally (c)(ii), and still further optionally (c)(iii). In an embodiment, the cell of the subject is contacted *in vivo* by subretinal delivery of (a), (b) and optionally (c)(i), further optionally (c)(ii), and still further optionally (c)(iii).

In an embodiment, the contacting step comprises contacting the subject with a nucleic acid, e.g., a vector, e.g., an AAV vector, described herein, e.g., a nucleic acid that encodes at least one of (a), (b), and optionally (c)(i), further optionally (c)(ii), and still further optionally (c)(iii).

In an embodiment, the contacting step comprises delivering to said subject said Cas9 molecule of (b), as a protein or mRNA, and a nucleic acid which encodes (a) and optionally (c)(i), further optionally (c)(ii), and still further optionally (c)(iii).

In an embodiment, the contacting step comprises delivering to the subject the Cas9 molecule of (b), as a protein or mRNA, the gRNA of (a), as an RNA, and optionally the second gRNA of (c)(i), further optionally said third gRNA of (c)(ii), and still further optionally said fourth gRNA of (c)(iii), as an RNA.

In an embodiment, the contacting step comprises delivering to the subject the gRNA of (a), as an RNA, optionally said second gRNA of (c)(i), further optionally said third gRNA of (c)(ii), and still further optionally said fourth gRNA of (c)(iii), as an RNA, and a nucleic acid that encodes the Cas9 molecule of (b).

In another aspect, disclosed herein is a reaction mixture comprising a gRNA molecule, a nucleic acid, or a composition described herein, and a cell, e.g., a cell from a subject having, or likely to develop POAG, or a subject having a mutation at a POAG target position

In another aspect, disclosed herein is a kit comprising, (a) a gRNA molecule described herein, or nucleic acid that encodes the gRNA, and one or more of the following:
(b) a Cas9 molecule, e.g., a Cas9 molecule described herein, or a nucleic acid or mRNA that encodes the Cas9;
(c)(i) a second gRNA molecule, e.g., a second gRNA molecule described herein or a nucleic acid that encodes (c)(i);
(c)(ii) a third gRNA molecule, e.g., a second gRNA molecule described herein or a nucleic acid that encodes (c)(ii);
(c)(iii) a fourth gRNA molecule, e.g., a second gRNA molecule described herein or a nucleic acid that encodes (c)(iii);
(d) a template nucleic acid, e.g, a template nucleic acid described herein.

In an embodiment, the kit comprises nucleic acid, e.g., an AAV vector, that encodes one or more of (a), (b), (c)(i), (c)(ii), (c)(iii) and (d).

In another aspect, disclosed herein is non-naturally occurring template nucleic acid described herein.

In yet another aspect, disclosed herein is a gRNA molecule, e.g., a gRNA molecule described herein, for use in treating or preventing POAG in a subject, e.g., in accordance with a method of treating or preventing POAG as described herein.

In an embodiment, the gRNA molecule in used in combination with a Cas9 molecule, e.g., a Cas9 molecule described herein. Additionally or alternatively, in an embodiment, the gRNA molecule is used in combination with a second, third and/or fouth gRNA molecule, e.g., a second, third and/or fouth gRNA molecule described herein.

In still another aspect, disclosed herein is use of a gRNA molecule, e.g., a gRNA molecule described herein, in the manufacture of a medicament for treating or preventing POAG in a subject, e.g., in accordance with a method of treating or preventing POAG as described herein.

In an embodiment, the medicament comprises a Cas9 molecule, e.g., a Cas9 molecule described herein. Additionaly or alternatively, in an embodiment, the medicament comprises a second, third and/or fouth gRNA molecule, e.g., a second, third and/or fouth gRNA molecule described herein.

In an embodiment, the kit further comprises a governing gRNA molecule, or a nucleic acid that encodes a governing gRNA molecule.

In an aspect, the disclosure features a gRNA molecule, referred to herein as a governing gRNA molecule, comprising a targeting domain which is complementary to a target domain on a nucleic acid that encodes a component of the CRISPR/Cas system introduced into a cell or subject. In an embodiment, the governing gRNA molecule targets a nucleic acid that encodes a Cas9 molecule or a nucleic acid that encodes a target gene gRNA molecule. In an embodiment, the governing gRNA comprises a targeting domain that is complementary to a target domain in a sequence that encodes a Cas9 component, e.g., a Cas9 molecule or target gene gRNA molecule. In an embodiment, the target domain is designed with, or has, minimal homology to other nucleic acid sequences in the cell, e.g., to minimize off-target cleavage. For example, the targeting domain on the governing gRNA can be selected to reduce or minimize off-target effects. In an embodiment, a target domain for a governing gRNA can be disposed in the control or coding region of a Cas9 molecule or disposed between a control region and a transcribed region. In an embodiment, a target domain for a governing gRNA can be disposed in the control or coding region of a target gene gRNA molecule or disposed between a control region and a transcribed region for a target gene gRNA. While not wishing to be bound by theory, it is believed that altering, e.g., inactivating, a nucleic acid that encodes a Cas9 molecule or a nucleic acid that encodes a target gene gRNA molecule can be effected by cleavage of the targeted nucleic acid sequence or by binding of a Cas9 molecule/governing gRNA molecule complex to the targeted nucleic acid sequence.

The gRNA molecules and methods, as disclosed herein, can be used in combination with a governing gRNA molecule. The compositions and reaction mixtures, as disclosed herein, can also include a governing gRNA molecule, e.g., a governing gRNA molecule disclosed herein.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In addition, the materials, methods, and examples are illustrative only and not intended to be limiting.

Headings, including numeric and alphabetical headings and subheadings, are for organization and presentation and are not intended to be limiting.

Other features and advantages of the invention will be apparent from the detailed description, drawings, and from the claims.

### BRIEF DESCRIPTION OF THE DRAWING

**Figs. 1A-1I** are representations of several exemplary gRNAs.
**Fig. 1A** depicts a modular gRNA molecule derived in part (or modeled on a sequence in part) from *Streptococcus pyogenes (S. pyogenes)* as a duplexed structure (SEQ ID NOS: 42 and 43, respectively, in order of appearance);
**Fig. 1B** depicts a unimolecular (or chimeric) gRNA molecule derived in part from *S*. *pyogenes* as a duplexed structure (SEQ ID NO: 44);
**Fig. 1C** depicts a unimolecular gRNA molecule derived in part from *S. pyogenes* as a duplexed structure (SEQ ID NO: 45);
**Fig. 1D** depicts a unimolecular gRNA molecule derived in part from *S. pyogenes* as a duplexed structure (SEQ ID NO: 46);
**Fig. 1E** depicts a unimolecular gRNA molecule derived in part from *S. pyogenes* as a duplexed structure (SEQ ID NO: 47);
**Fig. 1F** depicts a modular gRNA molecule derived in part from *Streptococcus thermophilus (S. thermophilus)* as a duplexed structure (SEQ ID NOS: 48 and 49, respectively, in order of appearance);
**Fig. 1G** depicts an alignment of modular gRNA molecules of *S. pyogenes* and *S*. *thermophilus* (SEQ ID NOS: 50-53, respectively, in order of appearance).
**Figs. 1H-1I** depicts additional exemplary structures of unimolecular gRNA molecules. **Fig. 1H** shows an exemplary structure of a unimolecular gRNA molecule derived in part from *S. pyogenes* as a duplexed structure (SEQ ID NO: 45). **Fig. 1I** shows an exemplary structure of a unimolecular gRNA molecule derived in part from *S. aureus* as a duplexed structure (SEQ ID NO: 40).

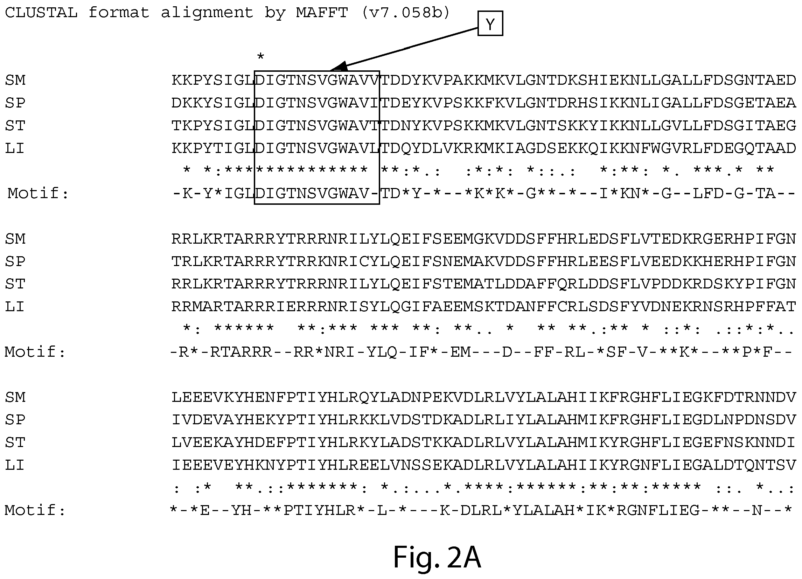

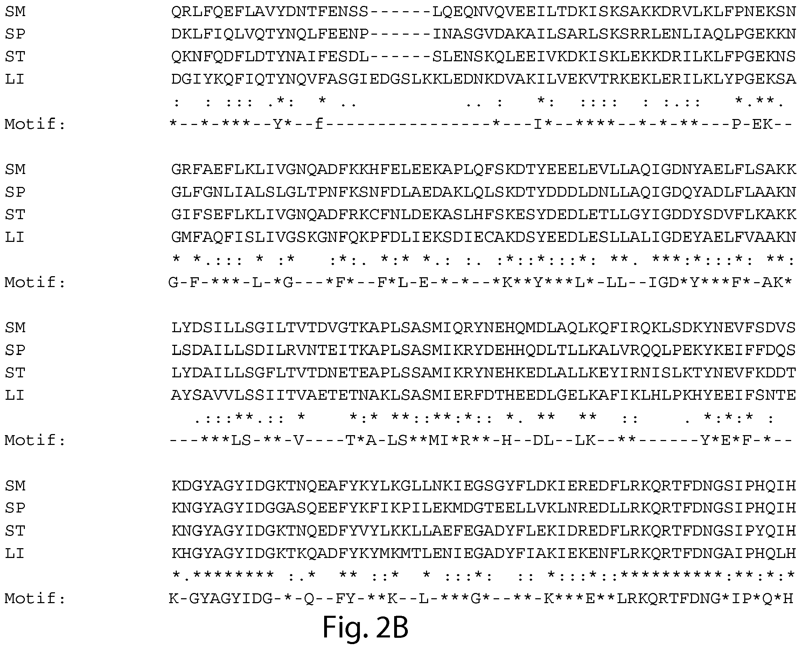

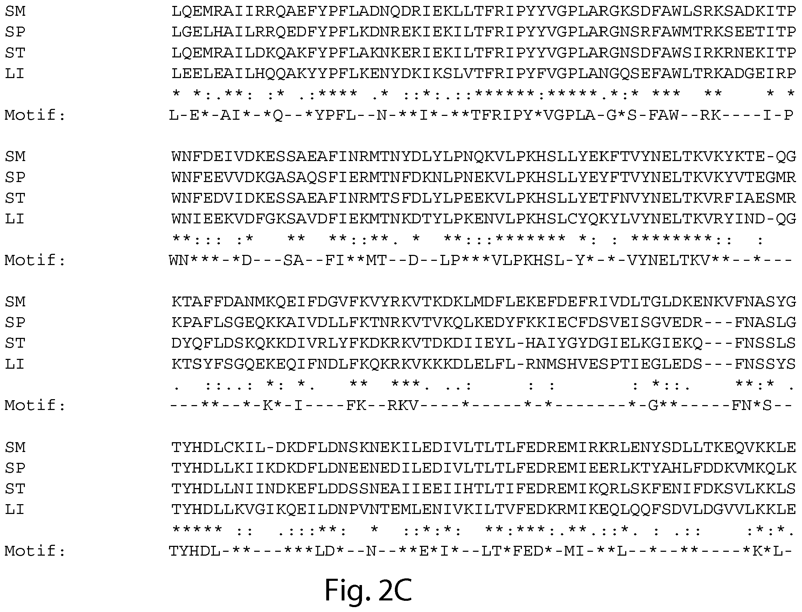

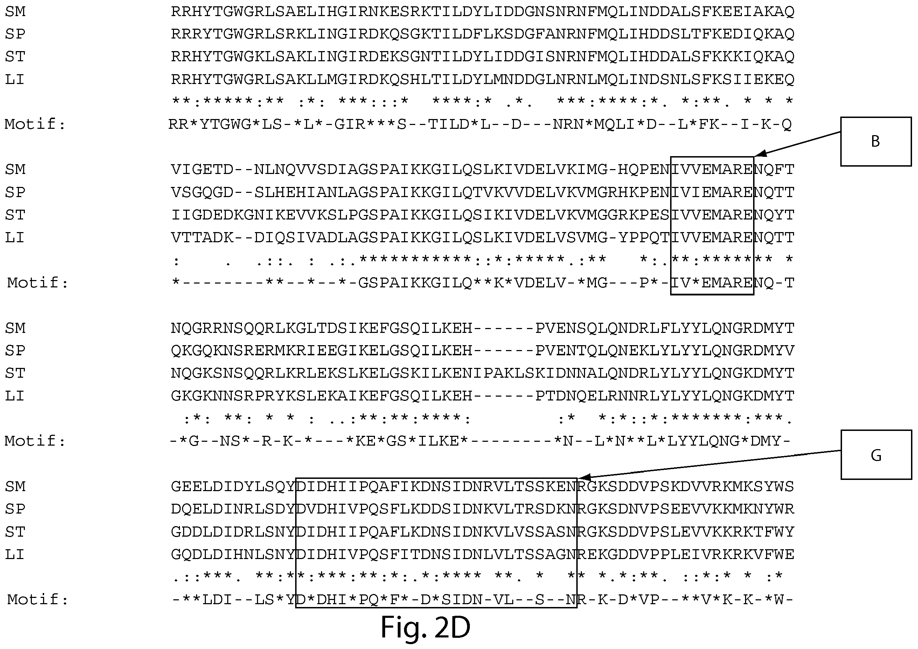

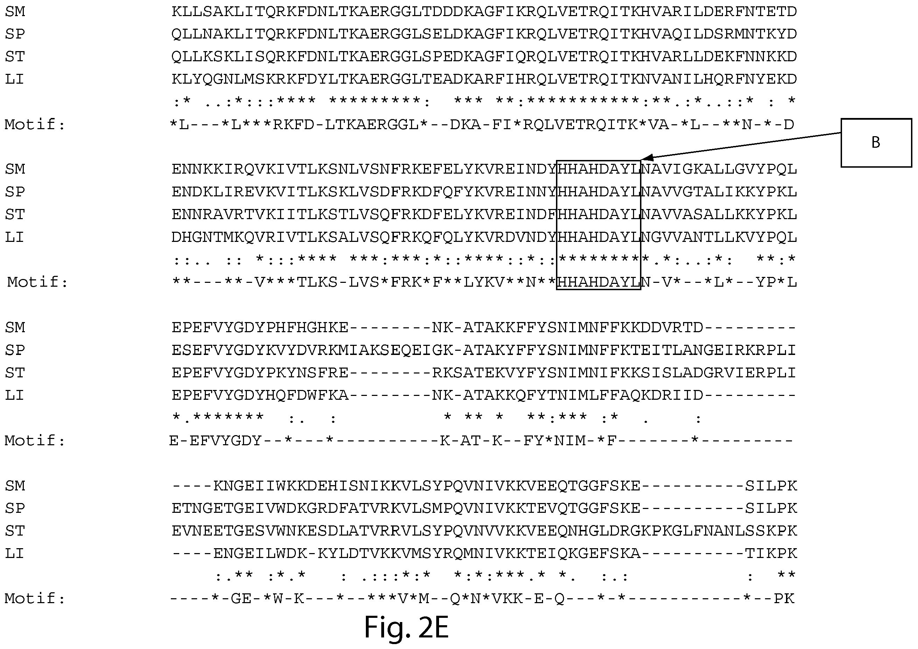

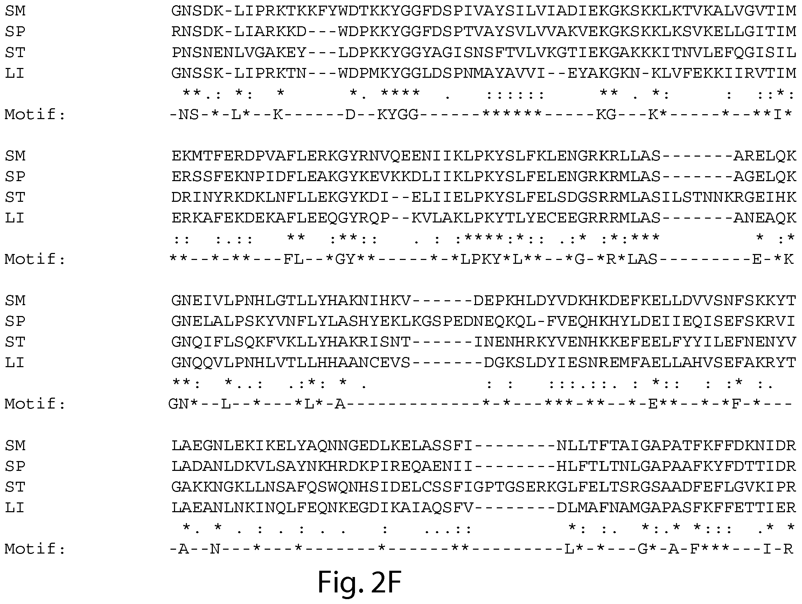

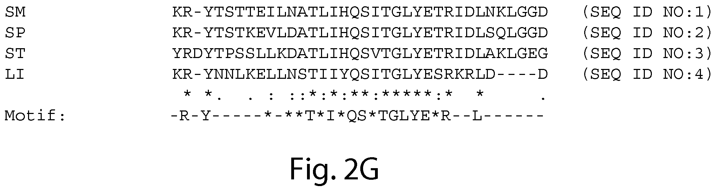

**Figs. 2A-2G** depict an alignment of Cas9 sequences from Chylinski et al. (RNA Biol. 2013; 10(5): 726-737). The N-terminal RuvC-like domain is boxed and indicated with a "Y". The other two RuvC-like domains are boxed and indicated with a "B". The HNH-like domain is boxed and indicated by a "G". Sm: *S. mutans* (SEQ ID NO: 1); Sp: *S. pyogenes* (SEQ ID NO: 2); St: *S. thermophilus* (SEQ ID NO: 3); Li: L. *innocua* (SEQ ID NO: 4). Motif: this is a motif based on the four sequences: residues conserved in all four sequences are indicated by single letter amino acid abbreviation; "*" indicates any amino acid found in the corresponding position of any of the four sequences; and "-" indicates any amino acid, e.g., any of the 20 naturally occurring amino acids, or absent.
**Figs. 3A-3B** show an alignment of the N-terminal RuvC-like domain from the Cas9 molecules disclosed in Chylinski *et al* (SEQ ID NOS: 54-103, respectively, in order of appearance). The last line of **Fig. 3B** identifies 4 highly conserved residues.
**Figs. 4A-4B** show an alignment of the N-terminal RuvC-like domain from the Cas9 molecules disclosed in Chylinski *et al.* with sequence outliers removed (SEQ ID NOS: 104-177, respectively, in order of appearance). The last line of **Fig. 4B** identifies 3 highly conserved residues.
**Figs. 5A-5C** show an alignment of the HNH-like domain from the Cas9 molecules disclosed in Chylinski *et al* (SEQ ID NOS: 178-252, respectively, in order of appearance). The last line of **Fig. 5C** identifies conserved residues.
**Figs. 6A-6B** show an alignment of the HNH-like domain from the Cas9 molecules disclosed in Chylinski *et al.* with sequence outliers removed (SEQ ID NOS: 253-302, respectively, in order of appearance). The last line of **Fig. 6B** identifies 3 highly conserved residues.

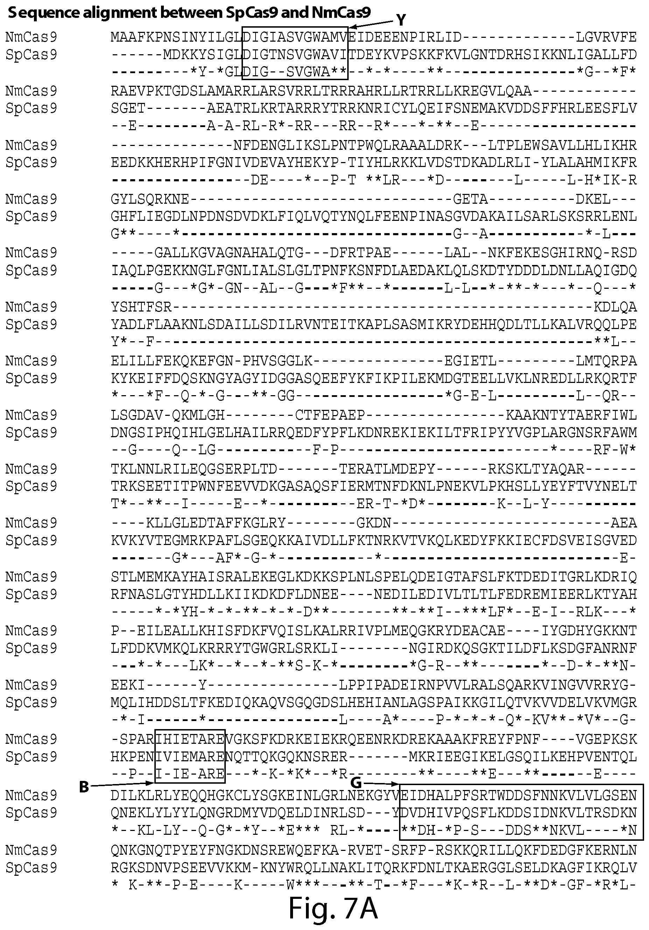

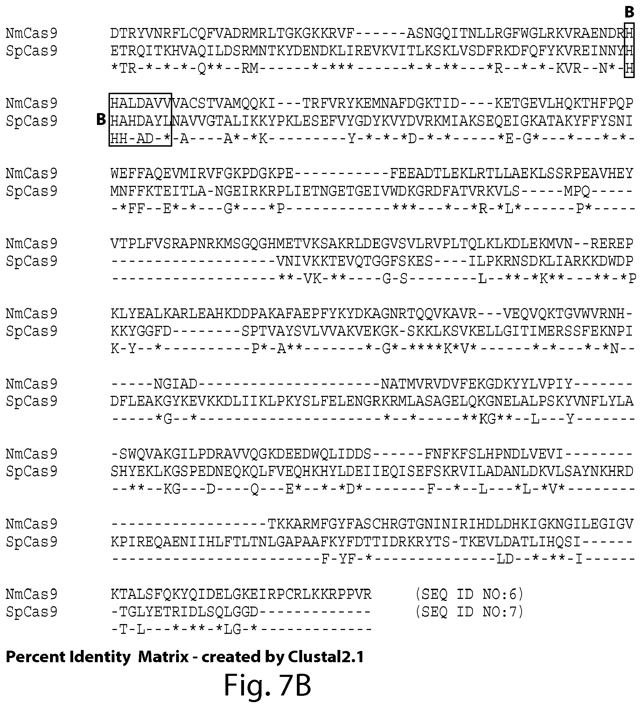

**Figs. 7A-7B** depict an alignment of Cas9 sequences from *S. pyogenes* and *Neisseria meningitidis (N. meningitidis).* The N-terminal RuvC-like domain is boxed and indicated with a "Y". The other two RuvC-like domains are boxed and indicated with a "B". The HNH-like domain is boxed and indicated with a "G". Sp: *S. pyogenes;* Nm: *N. meningitidis.* Motif: this is a motif based on the two sequences: residues conserved in both sequences are indicated by a single amino acid designation; "*" indicates any amino acid found in the corresponding position of any of the two sequences; "-" indicates any amino acid, e.g., any of the 20 naturally occurring amino acids, and "-" indicates any amino acid, e.g., any of the 20 naturally occurring amino acids, or absent.
**Fig. 8** shows a nucleic acid sequence encoding Cas9 of *N. meningitidis* (SEQ ID NO: 303). Sequence indicated by an "R" is an SV40 NLS; sequence indicated as "G" is an HA tag; and sequence indicated by an "O" is a synthetic NLS sequence; the remaining (unmarked) sequence is the open reading frame (ORF).
**Figs. 9A** and **9B** are schematic representations of the domain organization of *S*. *pyogenes* Cas 9. **Fig. 9A** shows the organization of the Cas9 domains, including amino acid positions, in reference to the two lobes of Cas9 (recognition (REC) and nuclease (NUC) lobes). **Fig. 9B** shows the percent homology of each domain across 83 Cas9 orthologs.

### Definitions

"Domain", as used herein, is used to describe segments of a protein or nucleic acid. Unless otherwise indicated, a domain is not required to have any specific functional property.

Calculations of homology or sequence identity between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). The optimal alignment is determined as the best score using the GAP program in the GCG software package with a Blossum 62 scoring matrix with a gap penalty of 12, a gap extend penalty of 4, and a frame shift gap penalty of 5. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences.

"Governing gRNA molecule", as used herein, refers to a gRNA molecule that comprises a targeting domain that is complementary to a target domain on a nucleic acid that comprises a sequence that encodes a component of the CRISPR/Cas system that is introduced into a cell or subject. A governing gRNA does not target an endogenous cell or subject sequence. In an embodiment, a governing gRNA molecule comprises a targeting domain that is complementary with a target sequence on: (a) a nucleic acid that encodes a Cas9 molecule; (b) a nucleic acid that encodes a gRNA which comprises a targeting domain that targets the *MYOC* gene (a target gene gRNA); or on more than one nucleic acid that encodes a CRISPR/Cas component, e.g., both (a) and (b). In an embodiment, a nucleic acid molecule that encodes a CRISPR/Cas component, e.g., that encodes a Cas9 molecule or a target gene gRNA, comprises more than one target domain that is complementary with a governing gRNA targeting domain. While not wishing to be bound by theory, in an embodiment, it is believed that a governing gRNA molecule complexes with a Cas9 molecule and results in Cas9 mediated inactivation of the targeted nucleic acid, e.g., by cleavage or by binding to the nucleic acid, and results in cessation or reduction of the production of a CRISPR/Cas system component. In an embodiment, the Cas9 molecule forms two complexes: a complex comprising a Cas9 molecule with a target gene gRNA, which complex will alter the *MYOC* gene; and a complex comprising a Cas9 molecule with a governing gRNA molecule, which complex will act to prevent further production of a CRISPR/Cas system component, e.g., a Cas9 molecule or a target gene gRNA molecule. In an embodiment, a governing gRNA molecule/Cas9 molecule complex binds to or promotes cleavage of a control region sequence, e.g., a promoter, operably linked to a sequence that encodes a Cas9 molecule, a sequence that encodes a transcribed region, an exon, or an intron, for the Cas9 molecule. In an embodiment, a governing gRNA molecule/Cas9 molecule complex binds to or promotes cleavage of a control region sequence, e.g., a promoter, operably linked to a gRNA molecule, or a sequence that encodes the gRNA molecule. In an embodiment, the governing gRNA, e.g., a Cas9-targeting governing gRNA molecule, or a target gene gRNA-targeting governing gRNA molecule, limits the effect of the Cas9 molecule/target gene gRNA molecule complex-mediated gene targeting. In an embodiment, a governing gRNA places temporal, level of expression, or other limits, on activity of the Cas9 molecule/target gene gRNA molecule complex. In an embodiment, a governing gRNA reduces off-target or other unwanted activity. In an embodiment, a governing gRNA molecule inhibits, e.g., entirely or substantially entirely inhibits, the production of a component of the Cas9 system and thereby limits, or governs, its activity.

"Modulator", as used herein, refers to an entity, e.g., a drug, that can alter the activity (e.g., enzymatic activity, transcriptional activity, or translational activity), amount, distribution, or structure of a subject molecule or genetic sequence. In an embodiment, modulation comprises cleavage, e.g., breaking of a covalent or non-covalent bond, or the forming of a covalent or non-covalent bond, e.g., the attachment of a moiety, to the subject molecule. In an embodiment, a modulator alters the, three dimensional, secondary, tertiary, or quaternary structure, of a subject molecule. A modulator can increase, decrease, initiate, or eliminate a subject activity.

"Large molecule", as used herein, refers to a molecule having a molecular weight of at least 2, 3, 5, 10, 20, 30, 40, 50, 60, 70, 80, 90, or 100 kD. Large molecules include proteins, polypeptides, nucleic acids, biologics, and carbohydrates.

"Polypeptide", as used herein, refers to a polymer of amino acids having less than 100 amino acid residues. In an embodiment, it has less than 50, 20, or 10 amino acid residues.

"Reference molecule", e.g., a reference Cas9 molecule or reference gRNA, as used herein, refers to a molecule to which a subject molecule, e.g., a subject Cas9 molecule of subject gRNA molecule, e.g., a modified or candidate Cas9 molecule is compared. For example, a Cas9 molecule can be characterized as having no more than 10% of the nuclease activity of a reference Cas9 molecule. Examples of reference Cas9 molecules include naturally occurring unmodified Cas9 molecules, e.g., a naturally occurring Cas9 molecule such as a Cas9 molecule of *S. pyogenes, S. aureus* or *S. thermophilus.* In an embodiment, the reference Cas9 molecule is the naturally occurring Cas9 molecule having the closest sequence identity or homology with the Cas9 molecule to which it is being compared. In an embodiment, the reference Cas9 molecule is a sequence, e.g., a naturally occurring or known sequence, which is the parental form on which a change, e.g., a mutation has been made.

"Replacement", or "replaced", as used herein with reference to a modification of a molecule does not require a process limitation but merely indicates that the replacement entity is present.

"Small molecule", as used herein, refers to a compound having a molecular weight less than about 2 kD, e.g., less than about 2 kD, less than about 1.5 kD, less than about 1 kD, or less than about 0.75 kD.

"Subject", as used herein, may mean either a human or non-human animal. The term includes, but is not limited to, mammals (e.g., humans, other primates, pigs, rodents (e.g., mice and rats or hamsters), rabbits, guinea pigs, cows, horses, cats, dogs, sheep, and goats). In an embodiment, the subject is a human. In other embodiments, the subject is poultry.

"Treat", "treating" and "treatment", as used herein, mean the treatment of a disease in a mammal, e.g., in a human, including (a) inhibiting the disease, i.e., arresting or preventing its development; (b) relieving the disease, i.e., causing regression of the disease state; and (c) curing the disease.

"Prevent", "preventing" and "prevention", as used herein, means the prevention of a disease in a mammal, e.g., in a human, including (a) avoiding or precluding the disease; (2) affecting the predisposition toward the disease, e.g., preventing at least one symptom of the disease or to delay onset of at least one symptom of the disease.

"X" as used herein in the context of an amino acid sequence, refers to any amino acid (e.g., any of the twenty natural amino acids) unless otherwise specified.

### Primary Open Angel Glaucoma (POAG)

Glaucoma is the second leading cause of blindness in the world. Primary Open Angle Glaucoma (POAG) is the leading cause of glaucoma and affects approximately 1% of patients ages 40-89.

POAG develops due to an imbalance between the production and outflow of aqueous humor within the eye. Aqueous humor (AH) is produced by the ciliary body located in the posterior chamber. The vast majority (approximately 80%) of AH drains through the trabecular meshwork (TM) to the episcleral venous system. A minority (approximately 20%) of AH drains through the interstitium between the iris root and ciliary muscle (Feisal 2005). POAG is likely due to decreased drainage through the trabecular meshwork; decreased outflow of AH results in increased intraocular pressure (IOP) and IOP causes damage to the optic nerve and leads to progressive blindness.

The etiology of POAG is multi-factorial and complex. However, mutations in the *MYOC* gene (also known as *GLC1A, JOAG1* and *TIGR*) have been shown to be a leading genetic cause of POAG and of juvenile-onset POAG. Mutations in *MYOC* have been shown to account for 3% of POAG. Many patients with *MYOC* mutations develop rapidly advancing disease and/or earlier presentation of POAG, including juvenile-onset POAG.

The *MYOC* gene, also called the trabecular meshwork-induced glucocorticoid receptor (TIGR), encodes myocilin, a 504 amino acid protein encoded by 3 exons. Myocilin is found in the trabecular meshwork and plays a role in cytoskeletal function and in the regulation of IOP.

### Methods to Treat or Prevent POAG

Methods and compositions described herein provide for a therapy, e.g., a one-time therapy, or a multi-dose therapy, that prevents or treats primary open-angle glaucoma (POAG). In an embodiment, a disclosed therapy prevents, inhibits, or reduces the production of mutant myocilin protein in cells of the anterior and posterior chamber of the eye in a subject who has POAG.

While not wishing to be bound by theory, in an embodiment, it is believed that knocking out *MYOC* on ciliary body cells, iris cells, trabecular meshwork cells, retinal cells, e.g. e.g., a rod photoreceptor cell, e.g., a cone photoreceptor cell, e.g., a retinal pigment epithelium cell, e.g., a horizontal cell, e.g., an amacrine cell, e.g., a ganglion cell, will prevent the progression of eye disease in subjects with POAG.

While not wishing to be bound by theory, in an embodiment, it is believed that correction of *MYOC* in ciliary body cells, iris cells, trabecular meshwork cells, retinal cells, e.g. e.g., a rod photoreceptor cell, e.g., a cone photoreceptor cell, e.g., a retinal pigment epithelium cell, e.g., a horizontal cell, e.g., an amacrine cell, e.g., a ganglion cell, will prevent the progression of eye disease in subjects with POAG. Corrected cells will not undergo apoptosis, will not cause inflammation and will produce wild-type, non-aggregating myocilin. In an embodiment, the disease is cured, does not progress or has delayed progression compared to a subject who has not received the therapy.

Myocilin is expressed in the eye, primarily by trabecular meshwork cells and the ciliary body. It is also expressed in the retina. Research indicates that MYOC mutations exert a toxic gain of function effect within trabecular meshwork cells. Mutant myocilin, especially mutants with missense or nonsense mutations in exon 3, e.g., a mutation at T377 (e.g., T377R), a mutation at 1477 (e.g., I477N), or a mutation at P370 (e.g., P370L), may misfold and aggregate in the endoplasmic reticulum (ER). Misfolding and aggregation within the ER elicits the ER stress and unfold protein response, which can lead to apoptosis and inflammation within trabecular meshwork cells. In addition, mutant myocilin protein may aggregate in the trabecular meshwork with other mutant proteins and/or with wild-type myocilin (in heterozygotes). Mutant myocilin aggregates may interfere with the outflow of aqueous humor to the episcleral venous system. Decreased aqueous humor outflow causes increased intraocular pressure, leading to POAG.

The elimination of mutant myocilin production in subjects with a mutation, e.g., a mutation at T377 (e.g., T377R), a mutation at 1477 (e.g., I477N), or a mutation at P370 (e.g., P370L) mutations or other mutant MYOC alleles through knock out of MYOC on ciliary body cells, iris cells, trabecular meshwork cells and retinal cells will prevent the production of the myocilin proteins. Corrected cells will not undergo apoptosis and will not increase inflammation. In an embodiment, POAG does not progress or has delayed progression compared to a subject who has not received the therapy.

Described herein are methods for treating or delaying the onset or progression of POAG caused by mutations in the *MYOC* gene, including but not limited to mutations in exon 3, e.g., a mutation at T377 (e.g.,T377R), a mutation at 1477 (e.g., I477N), or a mutation at P370 (e.g., P370L). The disclosed methods for treating or delaying the onset or progression of POAG alter the *MYOC* gene by genome editing using a gRNA targeting the POAG target position and a Cas9 enzyme. Details on gRNAs targeting the POAG target position and Cas9 enzymes are provided below.

Current treatments to prevent the progression of POAG include treatments that reduce IOP. For example, trabeculectomy surgery and eye drops, including alpha-adregergic antagonists and beta-adrenergic antagonists, are both effective in preventing POAG progression. However, further treatments are needed to reduce IOP and prevent progression of POAG. Disclosed herein are methods that correct the underlying mutations that lead to POAG. Also disclosed herein are methods that knockdown or knockout a *MYOC* gene. Targeted knockdown or knockout of the *MYOC* gene includes targeting one or both alleles of the *MYOC* gene. The disclosed methods may be useful to permanently decrease IOP and prevent the progressive visual loss of POAG. Further, the disclosed methods are more convenient than taking daily eye drops or having surgery.

Disclosed herein are multiple approaches to altering or modifying, i.e., correcting, the *MYOC* gene, using the CRIPSR/Cas system to treat POAG.

In an embodiment, one approach is to repair (i.e., correct) one or more mutations in the *MYOC* gene by HDR. In an embodiment, mutant *MYOC* allele(s) are corrected and restored to wild type state, which preserves myocilin function, restores homeostasis within the TM and preserves IOP, which reverses or prevents progression of POAG.

In another embodiment, the *MYOC* gene is targeted as a targeted knockout or knockdown. A knockout or knockdown of the *MYOC* gene may offer a benefit to subjects with POAG who have a mutation in the *MYOC* gene as well as subjects with POAG without a known *MYOC* mutation. There is evidence that *MYOC* mutations are gain of function mutations leading to altered TM function and the development of IOP. There is further evidence that patients with heterozygous early truncating mutations (Arg46stop) do not develop disease. *MYOC* knock-out mice do not develop POAG and have no detected eye abnormalities. Further, a few patients have been identified who express no myocilin in the eye and have no phenotype. Without wishing to be bound by theory, it is contemplated herein that a knock out or knock down of *MYOC* gene in the eye prevents the development of POAG.

There is also evidence to support a dominant negative effect of certain heterozygous mutations on the wild-type allele (Kuchtey J et al., 2013 Eur J Med Genet.b56(6):292-6. doi: 10.1016/j.ejmg.2013.03.002. Epub 2013 Mar 19). Without wishing to be bound by theory, it is contemplated herein that a knockout of both alleles reverses the dominant negative effect and is beneficial for patients.

Correction of a mutation in the *MYOC* gene or knockdown or knockout of one or both MYOC alleles may be performed prior to disease onset or after disease onset, but preferably early in the disease course.

In an embodiment, treatment is initiated prior to onset of the disease.

In an embodiment, treatment is initiated after onset of the disease, but early in the course of disease progression (e.g., prior to vision loss, a decrease in visual acuity and/or an increase in IOP).

In an embodiment, treatment is initiated after onset of the disease, but prior to a measurable increase in IOP.

In an embodiment, treatment is initiated prior to loss of visual acuity.

In an embodiment, treatment is initiated at onset of loss of visual acuity.

In an embodiment, treatment is initiated after onset of loss of visual acuity.

In an embodiment, treatment is initiated in a subject who has tested positive for a mutation in the MYOC gene, e.g., prior to disease onset or in the earliest stages of disease.

In an embodiment, a subject has a family member that has been diagnosed with POAG. For example, the subject has a family member that has been diagnosed with POAG, and the subject demonstrates a symptom or sign of the disease or has been found to have a mutation in the MYOC gene.

In an embodiment, treatment is initiated in a subject who has no MYOC mutation but has increased intraocular pressure.

In an embodiment, treatment is initiated in a subject at onset of an increase in intraocular pressure.

In an embodiment, treatment is initiated in a subject after onset of an increase in intraocular pressure.

In an embodiment, treatment is initiated in a subject with signs consistent with POAG on ophthalmologic exam, including but not limited to: increased intraocular pressure; cupping of the optic nerve on slit lamp exam, stereobiomicroscopy or ophthalmoscopy; pallor of the optic disk; thinning or notching of the optic disk rim; hemorrhages of the optic disc; vertical cup-to-disk ratio of >0.6 or cup-to-disk asymmetry between eyes of greater than 0.2; peripapillary atrophy.

A subject's vision can evaluated, e.g., prior to treatment, or after treatment, e.g., to monitor the progress of the treatment. In an embodiment, the subject's vision is evaluated prior to treatment, e.g., to determine the need for treatment. In an embodiment, the subject's vision is evaluated after treatment has been initiated, e.g., to access the effectiveness of the treatment. Vision can be evaluated by one or more of: evaluation of increased IOP; evaluating changes in function relative to the contralateral eye, e.g., by utilizing retinal analytical techniques; by evaluating mean, median and distribution of change in best corrected visual acuity (BCVA); evaluation by Optical Coherence Tomography; evaluation of changes in visual field using perimetry; evaluation by full-field electroretinography (ERG); evaluation by slit lamp examination; evaluation of intraocular pressure; evaluation of autofluorescence, evaluation with fundoscopy; evaluation with fundus photography; evaluation with fluorescein angiography (FA); or evaluation of visual field sensitivity (FFST).

In other embodiments, a subject's vision may be assessed by measuring the subject's mobility, e.g., the subject's ability to maneuver in space.

### Methods of Altering MYOC

As disclosed herein, a POAG target position, e.g., *MYOC* gene, can be altered by gene editing, e.g., using CRISPR-Cas9 mediated methods as described herein.

An alteration of the *MYOC* gene can be mediated by any mechanism. Exemplary mechanisms that can be associated with an alteration of the *MYOC* gene include, but are not limited to, non-homologous end joining (e.g., classical or alternative), microhomology-mediated end joining (MMEJ), homology-directed repair (e.g., endogenous donor template mediated), SDSA (synthesis dependent strand annealing), single strand annealing or single strand invasion.

In an embodiment, altering the POAG target position is achieved, e.g., by:
(1) correcting a POAG target position (e.g., a point mutation) in the *MYOC* gene (e.g., HDR-mediated correction with a donor template that corrects the mutation, e.g., the point mutation);
(2) knocking out the *MYOC* gene:
   (a) insertion or deletion (e.g., NHEJ-mediated insertion or deletion) of one or more nucleotides in close proximity to or within an early coding region of the *MYOC* gene, or
   (b) deletion (e.g., NHEJ-mediated deletion) of genomic sequence including a POAG knockout target position of the *MYOC* gene, or
(3) knocking down the *MYOC* gene mediated by enzymatically inactive Cas9 (eiCas9) molecule or an eiCas9-fusion protein by targeting the promoter region of the gene.

All approaches give rise to alteration of the *MYOC* gene. In one embodiment, methods described herein introduce one or more breaks near a POAG target position in at least one allele of the *MYOC* gene. In another embodiment, methods described herein introduce two or more breaks to flank a POAG target position, e.g., POAG knockout target position or a point mutation in the *MYOC* gene. The two or more breaks remove (e.g., delete) genomic sequence including the POAG target position, e.g., POAG knockout target position or point mutation in the *MYOC* gene. In another embodiment, methods described herein comprises knocking down the *MYOC* gene mediated by enzymatically inactive Cas9 (eiCas9) molecule or an eiCas9-fusion protein by targeting the promoter region of a POAG knockdown target position. All methods described herein result in alteration of the *MYOC* gene.

### HDR-mediated repair of MYOC

The methods and compositions described herein introduce one or more breaks near a POAG target position, e.g., Q368 (e.g., Q368stop), P370 (e.g., P370L), T377 (e.g., T377R), 1477 (e.g., I477N or I477S) or the 477-502 mutation hotspot region in the *MYOC* gene. In an embodiment, a mutation (e.g., Q368 (e.g., Q368stop), P370 (e.g., P370L), T377 (e.g., T377R), 1477 (e.g., I477N or I477S) or the 477-502 mutation hotspot region the substitution T377R), or 1477 (e.g., the substitution I477N or I477S) is targeted by cleaving with either one or more nucleases, one or more nickases or any combination thereof to induce HDR with a donor template that corrects the point mutation (e.g., the single nucleotide, e.g., Q368 (e.g., Q368stop), P370 (e.g., P370L), T377 (e.g., T377R), 1477 (e.g., I477N or I477S) or the 477-502 mutation hotspot region. The method can include acquiring knowledge of the mutation carried by the subject, e.g., by sequencing the appropriate portion of the *MYOC* gene.

In an embodiment, guide RNAs were designed to target a mutation (e.g., Q368 (e.g., Q368stop), P370 (e.g., P370L), T377 (e.g., T377R), 1477 (e.g., I477N or I477S) or the 477-502 mutation hotspot region) in the *MYOC* gene. A single gRNA with a Cas9 nuclease or a Cas9 nickase could be used to generate a break (e.g., a single strand break or a double strand break) in close proximity to a mutation (e.g., Q368 (e.g., Q368stop), P370 (e.g., P370L), T377 (e.g., T377R), 1477 (e.g., I477N or I477S) or the 477-502 mutation hotspot region). While not bound by theory, in an embodiment, it is believed that HDR-mediated repair (e.g., with a donor template) of the break (e.g., a single strand break or a double strand break) allow for the correction of the mutation (e.g., Q368 (e.g., Q368stop), P370 (e.g., P370L), T377 (e.g., T377R), 1477 (e.g., I477N or I477S) or the 477-502 mutation hotspot region),which results in restoration of a functional MYOC protein.

In another embodiment, two gRNAs with two Cas9 nickases could be used to generate two single strand breaks in close proximity to a mutation (e.g., Q368 (e.g., Q368stop), P370 (e.g., P370L), T377 (e.g., T377R), 1477 (e.g., I477N or I477S) or the 477-502 mutation hotspot region). While not bound by theory, in an embodiment, it is believed that HDR-mediated repair (e.g., with a donor template) of the breaks (e.g., the two single strand breaks) allow for the correction of the mutation (e.g., Q368 (e.g., Q368stop), P370 (e.g., P370L), T377 (e.g., T377R), 1477 (e.g., I477N or I477S) or the 477-502 mutation hotspot region), which results in restoration of a functional MYOC protein.

In another embodiment, more than two gRNAs may be used in a dual-targeting approach to generate two sets of breaks (e.g., two double strand breaks, one double strand break and a pair of single strand breaks or two pairs of single strand breaks) in close proximity to a mutation (e.g., Q368 (e.g., Q368stop), P370 (e.g., P370L), T377 (e.g., T377R), 1477 (e.g., I477N or I477S) or the 477-502 mutation hotspot region)or delete a genomic sequence containing a mutation (e.g., Q368 (e.g., Q368stop), P370 (e.g., P370L), T377 (e.g., T377R), 1477 (e.g., I477N or I477S) or the 477-502 mutation hotspot region)in the *MYOC* gene. While not bound by theory, in an embodiment, it is believed that HDR-mediated repair (e.g., with a donor template) of the breaks (e.g., two double strand breaks, one double strand break and a pair of single strand breaks or two pairs of single strand breaks) allow for the correction of the mutation (e.g., Q368 (e.g., Q368stop), P370 (e.g., P370L), T377 (e.g., T377R), 1477 (e.g., I477N or I477S) or the 477-502 mutation hotspot region),which results in restoration of a functional MYOC protein.

In an embodiment, a single strand break is introduced (e.g., positioned by one gRNA molecule) at or in close proximity to a POAG target position in the *MYOC* gene. In an embodiment, a single gRNA molecule (e.g., with a Cas9 nickase) is used to create a single strand break at or in close proximity to the POAG target position, e.g., the gRNA is configured such that the single strand break is positioned either upstream (e.g., within 200 bp upstream) or downstream (e.g., within 200 bp downstream) of the POAG target position. In an embodiment, the break is positioned to avoid unwanted target chromosome elements, such as repeat elements, e.g., an *Alu* repeat.

In an embodiment, a double strand break is introduced (e.g., positioned by one gRNA molecule) at or in close proximity to a POAG target position in the *MYOC* gene. In an embodiment, a single gRNA molecule (e.g., with a Cas9 nuclease other than a Cas9 nickase) is used to create a double strand break at or in close proximity to the POAG target position, e.g., the gRNA molecule is configured such that the double strand break is positioned either upstream (e.g., within 200 bp upstream) or downstream of (e.g., within 200 bp downstream) of a POAG target position. In an embodiment, the break is positioned to avoid unwanted target chromosome elements, such as repeat elements, e.g., an *Alu* repeat.

In an embodiment, two single strand breaks are introduced (e.g., positioned by two gRNA molecules) at or in close proximity to a POAG target position in the *MYOC* gene. In an embodiment, two gRNA molecules (e.g., with one or two Cas9 nickcases) are used to create two single strand breaks at or in close proximity to the POAG target position, e.g., the gRNAs molecules are configured such that both of the single strand breaks are positioned upstream (e.g., within 200 bp upstream) or downstream (e.g., within 200 bp downstream) of the POAG target position. In another embodiment, two gRNA molecules (e.g., with two Cas9 nickcases) are used to create two single strand breaks at or in close proximity to the POAG target position, e.g., the gRNAs molecules are configured such that one single strand break is positioned upstream (e.g., within 200 bp upstream) and a second single strand break is positioned downstream (e.g., within 200 bp downstream) of the POAG target position. In an embodiment, the breaks are positioned to avoid unwanted target chromosome elements, such as repeat elements, e.g., an *Alu* repeat.

In an embodiment, two double strand breaks are introduced (e.g., positioned by two gRNA molecules) at or in close proximity to a POAG target position in the *MYOC* gene. In an embodiment, two gRNA molecules (e.g., with one or two Cas9 nucleases that are not Cas9 nickases) are used to create two double strand breaks to flank a POAG target position, e.g., the gRNA molecules are configured such that one double strand break is positioned upstream (e.g., within 200 bp upstream) and a second double strand break is positioned downstream (e.g., within 200 bp downstream) of the POAG target position. In an embodiment, the breaks are positioned to avoid unwanted target chromosome elements, such as repeat elements, e.g., an *Alu* repeat.

In an embodiment, one double strand break and two single strand breaks are introduced (e.g., positioned by three gRNA molecules) at or in close proximity to a POAG target position in the *MYOC* gene. In an embodiment, three gRNA molecules (e.g., with a Cas9 nuclease other than a Cas9 nickase and one or two Cas9 nickases) to create one double strand break and two single strand breaks to flank a POAG target position, e.g., the gRNA molecules are configured such that the double strand break is positioned upstream or downstream of (e.g., within 200 bp upstream or downstream) of the POAG target position, and the two single strand breaks are positioned at the opposite site, e.g., downstream or upstream (within 200 bp downstream or upstream), of the POAG target position. In an embodiment, the breaks are positioned to avoid unwanted target chromosome elements, such as repeat elements, e.g., an *Alu* repeat.

In an embodiment, four single strand breaks are introduced (e.g., positioned by four gRNA molecules) at or in close proximity to a POAG target position in the *MYOC* gene. In an embodiment, four gRNA molecule (e.g., with one or more Cas9 nickases are used to create four single strand breaks to flank a POAG target position in the *MYOC* gene, e.g., the gRNA molecules are configured such that a first and second single strand breaks are positioned upstream (e.g., within 200 bp upstream) of the POAG target position, and a third and a fourth single stranded breaks are positioned downstream (e.g., within 200 bp downstream) of the POAG target position. In an embodiment, the breaks are positioned to avoid unwanted target chromosome elements, such as repeat elements, e.g., *an Alu* repeat.

In an embodiment, two or more (e.g., three or four) gRNA molecules are used with one Cas9 molecule. In another embodiment, when two or more (e.g., three or four) gRNAs are used with two or more Cas9 molecules, at least one Cas9 molecule is from a different species than the other Cas9 molecule(s). For example, when two gRNA molecules are used with two Cas9 molecules, one Cas9 molecule can be from one species and the other Cas9 molecule can be from a different species. Both Cas9 species are used to generate a single or double-strand break, as desired.

### NHEJ-mediated introduction of an indel in close proximity to or within the early coding region of the POAG target knockout position

In an embodiment, the method comprises introducing a NHEJ-mediated insertion or deletion of one more nucleotides in close proximity to the POAG target knockout position (e.g., the early coding region) of the *MYOC* gene. As described herein, in one embodiment, the method comprises the introduction of one or more breaks (e.g., single strand breaks or double strand breaks) sufficiently close to (e.g., either 5' or 3' to) the early coding region of the POAG knockout target position, such that the break-induced indel could be reasonably expected to span the POAG target knockout position (e.g., the early coding region). While not wishing to be bound by theory, it is believed that NHEJ-mediated repair of the break(s) allows for the NHEJ-mediated introduction of an indel in close proximity to within the early coding region of the POAG target knockout position.

In an embodiment, the targeting domain of the gRNA molecule is configured to provide a cleavage event, e.g., a double strand break or a single strand break, sufficiently close to the early coding region in the *MYOC* gene to allow alteration, e.g., alteration associated with NHEJ in the *MYOC* gene. In an embodiment, the targeting domain is configured such that a cleavage event, e.g., a double strand or single strand break, is positioned within 1, 2, 3, 4, 5, 10, 15, 20, 25, 30, 35, 40, 45, 50, 60, 70, 80, 90, 100, 150, 200, 250, 300, 350, 400, 450 or 500 nucleotides of a POAG target knockout position. The break, e.g., a double strand or single strand break, can be positioned upstream or downstream of a POAG target knockout position in the *MYOC* gene.

In an embodiment, a second gRNA molecule comprising a second targeting domain is configured to provide a cleavage event, e.g., a double strand break or a single strand break, sufficiently close to the early coding region in the *MYOC* gene, to allow alteration, e.g., alteration associated with NHEJ in the *MYOC* gene, either alone or in combination with the break positioned by said first gRNA molecule. In an embodiment, the targeting domains of the first and second gRNA molecules are configured such that a cleavage event, e.g., a double strand or single strand break, is positioned, independently for each of the gRNA molecules, within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position. In an embodiment, the breaks, e.g., double strand or single strand breaks, are positioned on both sides of a nucleotide of a POAG target knockout position in the *MYOC* gene. In an embodiment, the breaks, e.g., double strand or single strand breaks, are positioned on one side, e.g., upstream or downstream, of a nucleotide of a POAG target knockout position in the *MYOC* gene.

In an embodiment, a single strand break is accompanied by an additional single strand break, positioned by a second gRNA molecule, as discussed below. For example, the targeting domains are configured such that a cleavage event, e.g., the two single strand breaks, are positioned within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the early coding region in the *MYOC* gene. In an embodiment, the first and second gRNA molecules are configured such, that when guiding a Cas9 nickase, a single strand break will be accompanied by an additional single strand break, positioned by a second gRNA, sufficiently close to one another to result in alteration of the early coding region in the *MYOC* gene. In an embodiment, the first and second gRNA molecules are configured such that a single strand break positioned by said second gRNA is within 10, 20, 30, 40, or 50 nucleotides of the break positioned by said first gRNA molecule, e.g., when the Cas9 molecule is a nickase. In an embodiment, the two gRNA molecules are configured to position cuts at the same position, or within a few nucleotides of one another, on different strands, e.g., essentially mimicking a double strand break.

In an embodiment, a double strand break can be accompanied by an additional double strand break, positioned by a second gRNA molecule, as is discussed below. For example, the targeting domain of a first gRNA molecule is configured such that a double strand break is positioned upstream of the early coding region in the *MYOC* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position; and the targeting domain of a second gRNA molecule is configured such that a double strand break is positioned downstream of the early coding region in the *MYOC* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position.

In an embodiment, a double strand break can be accompanied by two additional single strand breaks, positioned by a second gRNA molecule and a third gRNA molecule. For example, the targeting domain of a first gRNA molecule is configured such that a double strand break is positioned upstream of the early coding region in the *MYOC* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position; and the targeting domains of a second and third gRNA molecule are configured such that two single strand breaks are positioned downstream of the early coding region in the *MYOC* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the target position. In an embodiment, the targeting domain of the first, second and third gRNA molecules are configured such that a cleavage event, e.g., a double strand or single strand break, is positioned, independently for each of the gRNA molecules.

In an embodiment, a first and second single strand breaks can be accompanied by two additional single strand breaks positioned by a third gRNA molecule and a fourth gRNA molecule. For example, the targeting domain of a first and second gRNA molecule are configured such that two single strand breaks are positioned upstream of the early coding region in the *MYOC* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the early coding region in the *MYOC* gene; and the targeting domains of a third and fourth gRNA molecule are configured such that two single strand breaks are positioned downstream of the early coding region in the *MYOC* gene, e.g., within 1, 2, 3, 4, 5, 10, 15, 20, 25, 30, 35, 40, 45, 50, 60, 70, 80, 90, 100, 150, 200, 250, 300, 350, 400, 450 or 500 nucleotides of the early coding region in the *MYOC* gene.

In an embodiment, a single strand break is introduced (e.g., positioned by one gRNA molecule) at or in close proximity to a POAG target position in the *MYOC* gene. In an embodiment, a single gRNA molecule (e.g., with a Cas9 nickase) is used to create a single strand break at or in close proximity to the POAG target position, e.g., the gRNA is configured such that the single strand break is positioned either upstream (e.g., within 500 bp upstream) or downstream (e.g., within 500 bp downstream) of the POAG target position. In an embodiment, the break is positioned to avoid unwanted target chromosome elements, such as repeat elements, e.g., an *Alu* repeat.

In an embodiment, a double strand break is introduced (e.g., positioned by one gRNA molecule) at or in close proximity to a POAG target position in the *MYOC* gene. In an embodiment, a single gRNA molecule (e.g., with a Cas9 nuclease other than a Cas9 nickase) is used to create a double strand break at or in close proximity to the POAG target position, e.g., the gRNA molecule is configured such that the double strand break is positioned either upstream (e.g., within 500 bp upstream) or downstream of (e.g., within 500 bp downstream) of a POAG target position. In an embodiment, the break is positioned to avoid unwanted target chromosome elements, such as repeat elements, e.g., an *Alu* repeat.

In an embodiment, two single strand breaks are introduced (e.g., positioned by two gRNA molecules) at or in close proximity to a POAG target position in the *MYOC* gene. In an embodiment, two gRNA molecules (e.g., with one or two Cas9 nickcases) are used to create two single strand breaks at or in close proximity to the POAG target position, e.g., the gRNAs molecules are configured such that both of the single strand breaks are positioned upstream (e.g., within 500 bp upstream) or downstream (e.g., within 500 bp downstream) of the POAG target position. In another embodiment, two gRNA molecules (e.g., with two Cas9 nickcases) are used to create two single strand breaks at or in close proximity to the POAG target position, e.g., the gRNAs molecules are configured such that one single strand break is positioned upstream (e.g., within 500 bp upstream) and a second single strand break is positioned downstream (e.g., within 500 bp downstream) of the POAG target position. In an embodiment, the breaks are positioned to avoid unwanted target chromosome elements, such as repeat elements, e.g., an *Alu* repeat.

In an embodiment, two double strand breaks are introduced (e.g., positioned by two gRNA molecules) at or in close proximity to a POAG target position in the *MYOC* gene. In an embodiment, two gRNA molecules (e.g., with one or two Cas9 nucleases that are not Cas9 nickases) are used to create two double strand breaks to flank a POAG target position, e.g., the gRNA molecules are configured such that one double strand break is positioned upstream (e.g., within 500 bp upstream) and a second double strand break is positioned downstream (e.g., within 500 bp downstream) of the POAG target position. In an embodiment, the breaks are positioned to avoid unwanted target chromosome elements, such as repeat elements, e.g., an *Alu* repeat.

In an embodiment, one double strand break and two single strand breaks are introduced (e.g., positioned by three gRNA molecules) at or in close proximity to a POAG target position in the *MYOC* gene. In an embodiment, three gRNA molecules (e.g., with a Cas9 nuclease other than a Cas9 nickase and one or two Cas9 nickases) to create one double strand break and two single strand breaks to flank a POAG target position, e.g., the gRNA molecules are configured such that the double strand break is positioned upstream or downstream of (e.g., within 500 bp upstream or downstream) of the POAG target position, and the two single strand breaks are positioned at the opposite site, e.g., downstream or upstream (within 500 bp downstream or upstream), of the POAG target position. In an embodiment, the breaks are positioned to avoid unwanted target chromosome elements, such as repeat elements, e.g., an *Alu* repeat.

### Knocking down the MYOC gene mediated by an enzymatically inactive Cas9 (eiCas9) molecule or an eiCas9-fusion protein by targeting the promoter region of the gene.

A targeted knockdown approach reduces or eliminates expression of functional *MYOC* gene product. As described herein, in an embodiment, a targeted knockdown is mediated by targeting an enzymatically inactive Cas9 (eiCas9) molecule or an eiCas9 fused to a transcription repressor domain or chromatin modifying protein to alter transcription, e.g., to block, reduce, or decrease transcription, of the *MYOC* gene.

Methods and compositions discussed herein may be used to alter the expression of the *MYOC* gene to treat or prevent POAG by targeting a promoter region of the *MYOC* gene. In an embodiment, the promoter region, e.g., at least 2 kb, at least 1.5 kb, at least 1.0 kb, or at least 0.5 kb upstream or downstream of the transcription start site (TSS) is targeted to knockdown expression of the *MYOC* gene. In an embodiment, the methods and compositions discussed herein may be used to knock down the *MYOC* gene to treat or prevent BT by targeting 0.5 kb upstream or downstream of the TSS. A targeted knockdown approach reduces or eliminates expression of functional *MYOC* gene product. As described herein, in an embodiment, a targeted knockdown is mediated by targeting an enzymatically inactive Cas9 (eiCas9) or an eiCas9 fused to a transcription repressor domain or chromatin modifying protein to alter transcription, e.g., to block, reduce, or decrease transcription, of the *MYOC* gene. In an embodiment, one or more eiCas9 molecules may be used to block binding of one or more endogenous transcription factors. In another embodiment, an eiCas9 molecule can be fused to a chromatin modifying protein. Altering chromatin status can result in decreased expression of the target gene. One or more eiCas9 molecules fused to one or more chromatin modifying proteins may be used to alter chromatin status

While some of the disclosure herein is presented in the context of the mutation in the *MYOC* gene that gives rise to an T377 mutant protein (e.g., T377R mutant protein) or a 1477 mutant protein (e.g., I477N mutant protein, e.g., I477S mutant protein) or a P370 mutant protein (e.g., P370L mutant protein), the methods and compositions herein are broadly applicable to any mutation, e.g., a point mutation or a nonsense mutation or a deletion mutation, in the *MYOC* gene that gives rise to POAG.

### I. gRNA Molecules

A gRNA molecule, as that term is used herein, refers to a nucleic acid that promotes the specific targeting or homing of a gRNA molecule/Cas9 molecule complex to a target nucleic acid. gRNA molecules can be unimolecular (having a single RNA molecule), sometimes referred to herein as "chimeric" gRNAs, or modular (comprising more than one, and typically two, separate RNA molecules). A gRNA molecule comprises a number of domains. The gRNA molecule domains are described in more detail below.

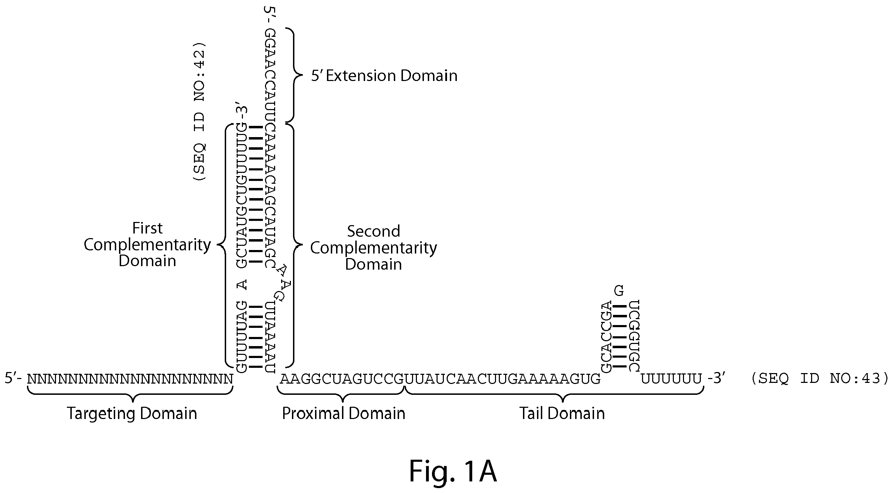

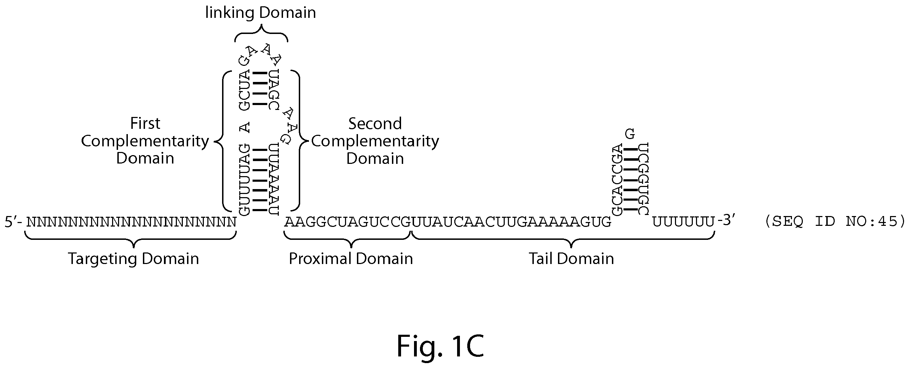

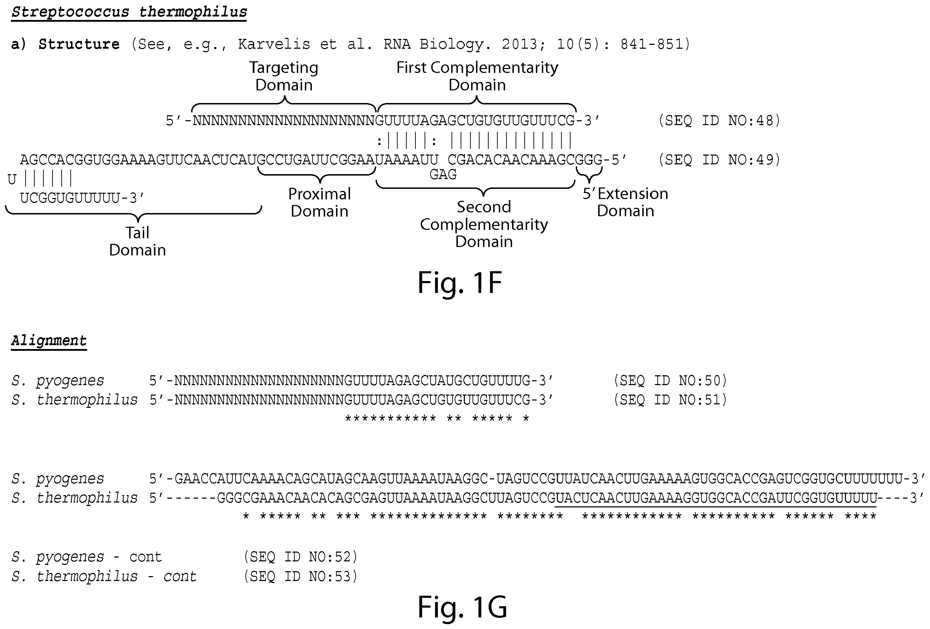

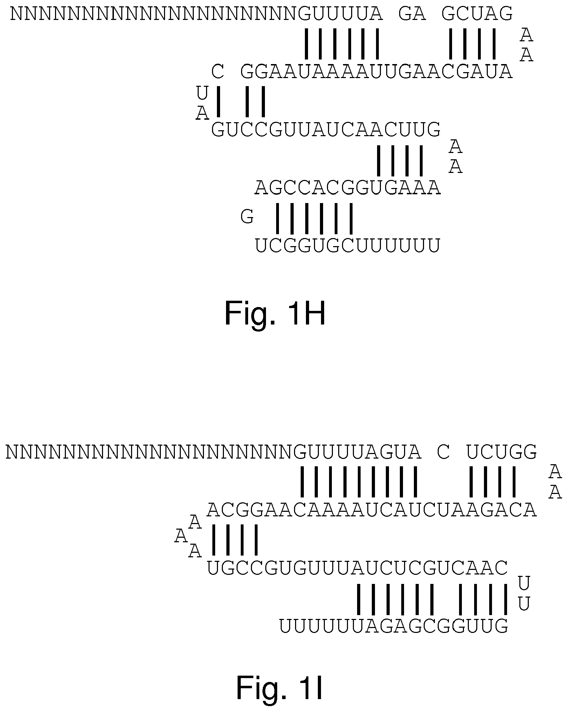

Several exemplary gRNA structures, with domains indicated thereon, are provided in **Figs. 1A-1G****.** While not wishing to be bound by theory, in an embodiment, with regard to the three dimensional form, or intra- or inter-strand interactions of an active form of a gRNA, regions of high complementarity are sometimes shown as duplexes in **Figs. 1A-1G** and other depictions provided herein.

In an embodiment, a unimolecular, or chimeric, gRNA comprises, preferably from 5' to 3':
a targeting domain (which is complementary to a target nucleic acid in the *MYOC* gene, e.g., a targeting domain from any of **1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E,** or **23A-23B);**
a first complementarity domain;
a linking domain;
a second complementarity domain (which is complementary to the first complementarity domain);
a proximal domain; and
optionally, a tail domain.

In an embodiment, a modular gRNA comprises:
a first strand comprising, preferably from 5' to 3';
   a targeting domain (which is complementary to a target nucleic acid in the *MYOC* gene, e.g., a targeting domain from **1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E,** or **23A-23B);** and
   a first complementarity domain; and
a second strand, comprising, preferably from 5' to 3':
   optionally, a 5' extension domain;
   a second complementarity domain;
   a proximal domain; and
   optionally, a tail domain.

The domains are discussed briefly below.

### The Targeting Domain

**Figs. 1A-1G** provide examples of the placement of targeting domains.

The targeting domain comprises a nucleotide sequence that is complementary, e.g., at least 80, 85, 90, or 95% complementary, e.g., fully complementary, to the target sequence on the target nucleic acid. The targeting domain is part of an RNA molecule and will therefore comprise the base uracil (U), while any DNA encoding the gRNA molecule will comprise the base thymine (T). While not wishing to be bound by theory, in an embodiment, it is believed that the complementarity of the targeting domain with the target sequence contributes to specificity of the interaction of the gRNA molecule/Cas9 molecule complex with a target nucleic acid. It is understood that in a targeting domain and target sequence pair, the uracil bases in the targeting domain will pair with the adenine bases in the target sequence. In an embodiment, the target domain itself comprises in the 5' to 3' direction, an optional secondary domain, and a core domain. In an embodiment, the core domain is fully complementary with the target sequence. In an embodiment, the targeting domain is 5 to 50 nucleotides in length. The strand of the target nucleic acid with which the targeting domain is complementary is referred to herein as the complementary strand. Some or all of the nucleotides of the domain can have a modification, e.g., a modification found in Section VIII herein.

In an embodiment, the targeting domain is 16 nucleotides in length.

In an embodiment, the targeting domain is 17 nucleotides in length.

In an embodiment, the targeting domain is 18 nucleotides in length.

In an embodiment, the targeting domain is 19 nucleotides in length.

In an embodiment, the targeting domain is 20 nucleotides in length.

In an embodiment, the targeting domain is 21 nucleotides in length.

In an embodiment, the targeting domain is 22 nucleotides in length.

In an embodiment, the targeting domain is 23 nucleotides in length.

In an embodiment, the targeting domain is 24 nucleotides in length.

In an embodiment, the targeting domain is 25 nucleotides in length.

In an embodiment, the targeting domain is 26 nucleotides in length.

In an embodiment, the targeting domain comprises 16 nucleotides.

In an embodiment, the targeting domain comprises 17 nucleotides.

In an embodiment, the targeting domain comprises 18 nucleotides.

In an embodiment, the targeting domain comprises 19 nucleotides.

In an embodiment, the targeting domain comprises 20 nucleotides.

In an embodiment, the targeting domain comprises 21 nucleotides.

In an embodiment, the targeting domain comprises 22 nucleotides.

In an embodiment, the targeting domain comprises 23 nucleotides.

In an embodiment, the targeting domain comprises 24 nucleotides.

In an embodiment, the targeting domain comprises 25 nucleotides.

In an embodiment, the targeting domain comprises 26 nucleotides.

Targeting domains are discussed in more detail below.

### The First Complementarity Domain

**Figs. 1A-1G** provide examples of first complementarity domains.

The first complementarity domain is complementary with the second complementarity domain, and in an embodiment, has sufficient complementarity to the second complementarity domain to form a duplexed region under at least some physiological conditions. In an embodiment, the first complementarity domain is 5 to 30 nucleotides in length. In an embodiment, the first complementarity domain is 5 to 25 nucleotides in length. In an embodiment, the first complementary domain is 7 to 25 nucleotides in length. In an embodiment, the first complementary domain is 7 to 22 nucleotides in length. In an embodiment, the first complementary domain is 7 to 18 nucleotides in length. In an embodiment, the first complementary domain is 7 to 15 nucleotides in length. In an embodiment, the first complementary domain is 5, 6, 7, 8, 9, 10, 11, 12, 13, 14, 15, 16, 17, 18, 19, 20, 21, 22, 23, 24, or 25 nucleotides in length.

In an embodiment, the first complementarity domain comprises 3 subdomains, which, in the 5' to 3' direction are: a 5' subdomain, a central subdomain, and a 3' subdomain. In an embodiment, the 5' subdomain is 4 to 9, e.g., 4, 5, 6, 7, 8 or 9 nucleotides in length. In an embodiment, the central subdomain is 1, 2, or 3, e.g., 1, nucleotide in length. In an embodiment, the 3' subdomain is 3 to 25, e.g., 4 to 22, 4 to 18, or 4 to 10, or 3, 4, 5, 6, 7, 8, 9, 10, 11, 12, 13, 14, 15, 16, 17, 18, 19, 20, 21, 22, 23, 24, or 25 nucleotides in length.

The first complementarity domain can share homology with, or be derived from, a naturally occurring first complementarity domain. In an embodiment, it has at least 50% homology with a first complementarity domain disclosed herein, e.g., an *S. pyogenes, S. aureus* or *S. thermophilus,* first complementarity domain.

Some or all of the nucleotides of the domain can have a modification, e.g., a modification found in Section VIII herein.

First complementarity domains are discussed in more detail below.

### The Linking Domain

**Figs. 1A-1G** provide examples of linking domains.

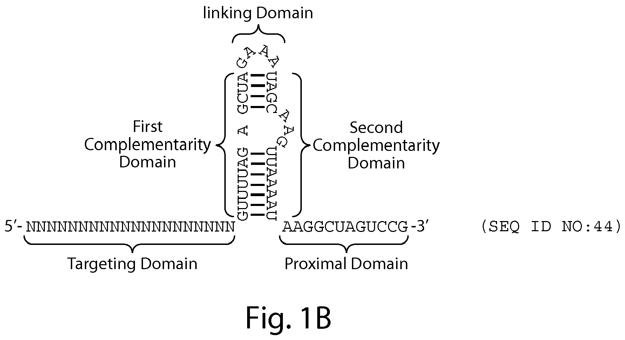

A linking domain serves to link the first complementarity domain with the second complementarity domain of a unimolecular gRNA. The linking domain can link the first and second complementarity domains covalently or non-covalently. In an embodiment, the linkage is covalent. In an embodiment, the linking domain covalently couples the first and second complementarity domains, see, e.g., **Figs. 1B-1E****.** In an embodiment, the linking domain is, or comprises, a covalent bond interposed between the first complementarity domain and the second complementarity domain. Typically the linking domain comprises one or more, e.g., 2, 3, 4, 5, 6, 7, 8, 9, or 10 nucleotides.

In modular gRNA molecules the two molecules are associated by virtue of the hybridization of the complementarity domains see e.g., **Fig. 1A****.**

A wide variety of linking domains are suitable for use in unimolecular gRNA molecules. Linking domains can consist of a covalent bond, or be as short as one or a few nucleotides, e.g., 1, 2, 3, 4, or 5 nucleotides in length. In an embodiment, a linking domain is 2, 3, 4, 5, 6, 7, 8, 9, 10, 15, 20, or 25 or more nucleotides in length. In an embodiment, a linking domain is 2 to 50, 2 to 40, 2 to 30, 2 to 20, 2 to 10, or 2 to 5 nucleotides in length. In an embodiment, a linking domain shares homology with, or is derived from, a naturally occurring sequence, e.g., the sequence of a tracrRNA that is 5' to the second complementarity domain. In an embodiment, the linking domain has at least 50% homology with a linking domain disclosed herein.

Some or all of the nucleotides of the domain can have a modification, e.g., modification found in Section VIII herein.

Linking domains are discussed in more detail below.

### The 5' Extension Domain

In an embodiment, a modular gRNA can comprise additional sequence, 5' to the second complementarity domain, referred to herein as the 5' extension domain, see, e.g., **Fig.** 1A. In an embodiment, the 5' extension domain is, 2 to 10, 2 to 9, 2 to 8, 2 to 7, 2 to 6, 2 to 5, 2 to 4 nucleotides in length. In an embodiment, the 5' extension domain is 2, 3, 4, 5, 6, 7, 8, 9, or 10 or more nucleotides in length.

### The Second Complementarity Domain

**Figs. 1A-1G** provide examples of second complementarity domains.

The second complementarity domain is complementary with the first complementarity domain, and in an embodiment, has sufficient complementarity to the second complementarity domain to form a duplexed region under at least some physiological conditions. In an embodiment, e.g., as shown in **Figs. 1A-1B****,** the second complementarity domain can include sequence that lacks complementarity with the first complementarity domain, e.g., sequence that loops out from the duplexed region.

In an embodiment, the second complementarity domain is 5 to 27 nucleotides in length. In an embodiment, it is longer than the first complementarity region. In an embodiment the second complementary domain is 7 to 27 nucleotides in length. In an embodiment, the second complementary domain is 7 to 25 nucleotides in length. In an embodiment, the second complementary domain is 7 to 20 nucleotides in length. In an embodiment, the second complementary domain is 7 to 17 nucleotides in length. In an embodiment, the complementary domain is 5, 6, 7, 8, 9, 10, 11, 12, 13, 14, 15, 16, 17, 18, 19, 20, 21, 22, 23, 24 or 25 nucleotides in length.

In an embodiment, the second complementarity domain comprises 3 subdomains, which, in the 5' to 3' direction are: a 5' subdomain, a central subdomain, and a 3' subdomain. In an embodiment, the 5' subdomain is 3 to 25, e.g., 4 to 22, 4 to 18, or 4 to 10, or 3, 4, 5, 6, 7, 8, 9, 10, 11, 12, 13, 14, 15, 16, 17, 18, 19, 20, 21, 22, 23, 24, or 25 nucleotides in length. In an embodiment, the central subdomain is 1, 2, 3, 4 or 5, e.g., 3, nucleotides in length. In an embodiment, the 3' subdomain is 4 to 9, e.g., 4, 5, 6, 7, 8 or 9 nucleotides in length.

In an embodiment, the 5' subdomain and the 3' subdomain of the first complementarity domain, are respectively, complementary, e.g., fully complementary, with the 3' subdomain and the 5' subdomain of the second complementarity domain.

The second complementarity domain can share homology with or be derived from a naturally occurring second complementarity domain. In an embodiment, it has at least 50% homology with a second complementarity domain disclosed herein, e.g., an *S. pyogenes, S. aureus* or *S. thermophilus,* first complementarity domain.

Some or all of the nucleotides of the domain can have a modification, e.g., a modification found in Section VIII herein.

### A Proximal domain

**Figs. 1A-1G** provide examples of proximal domains.

In an embodiment, the proximal domain is 5 to 20 nucleotides in length. In an embodiment, the proximal domain can share homology with or be derived from a naturally occurring proximal domain. In an embodiment, it has at least 50% homology with a proximal domain disclosed herein, e.g., an *S. pyogenes, S. aureus* or *S. thermophilus,* proximal domain.

Some or all of the nucleotides of the domain can have a modification, e.g., a modification found in Section VIII herein.

### A Tail Domain

**Figs. 1A-1G** provide examples of tail domains.

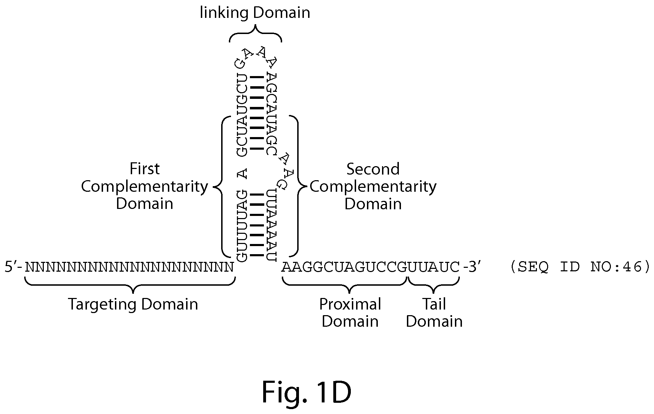

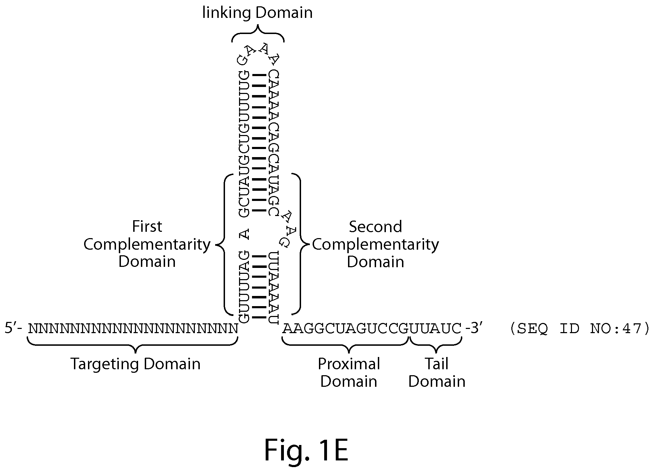

As can be seen by inspection of the tail domains in **Figs. 1A-1E****,** a broad spectrum of tail domains are suitable for use in gRNA molecules. In an embodiment, the tail domain is 0 (absent), 1, 2, 3, 4, 5, 6, 7, 8, 9, or 10 nucleotides in length. In embodiment, the tail domain nucleotides are from or share homology with sequence from the 5' end of a naturally occurring tail domain, see e.g., **Fig. 1D** or **Fig. 1E****.** In an embodiment, the tail domain includes sequences that are complementary to each other and which, under at least some physiological conditions, form a duplexed region.

In an embodiment, the tail domain is absent or is 1 to 50 nucleotides in length. In an embodiment, the tail domain can share homology with or be derived from a naturally occurring proximal tail domain. In an embodiment, it has at least 50% homology with a tail domain disclosed herein, e.g., an *S. pyogenes, S. aureus* or *S. thermophilus,* tail domain.

In an embodiment, the tail domain includes nucleotides at the 3' end that are related to the method of in vitro or in vivo transcription. When a T7 promoter is used for in vitro transcription of the gRNA, these nucleotides may be any nucleotides present before the 3' end of the DNA template. When a U6 promoter is used for in vivo transcription, these nucleotides may be the sequence UUUUUU. When alternate pol-III promoters are used, these nucleotides may be various numbers or uracil bases or may include alternate bases.

The domains of gRNA molecules are described in more detail below.

### The Targeting Domain

The "targeting domain" of the gRNA is complementary to the "target domain" on the target nucleic acid. The strand of the target nucleic acid comprising the nucleotide sequence complementary to the core domain of the gRNA is referred to herein as the "complementary strand" of the target nucleic acid. Guidance on the selection of targeting domains can be found, e.g., in Fu Y et al., Nat Biotechnol 2014 (doi: 10.1038/nbt.2808) and Sternberg SH et al., Nature 2014 (doi: 10.1038/nature13011).

In an embodiment, the targeting domain is 16, 17, 18, 19, 20, 21, 22, 23, 24, 25 or 26 nucleotides in length.

In an embodiment, the targeting domain is 16 nucleotides in length.

In an embodiment, the targeting domain is 17 nucleotides in length.

In an embodiment, the targeting domain is 18 nucleotides in length.

In an embodiment, the targeting domain is 19 nucleotides in length.

In an embodiment, the targeting domain is 20 nucleotides in length.

In an embodiment, the targeting domain is 21 nucleotides in length.

In an embodiment, the targeting domain is 22 nucleotides in length.

In an embodiment, the targeting domain is 23 nucleotides in length.

In an embodiment, the targeting domain is 24 nucleotides in length.

In an embodiment, the targeting domain is 25 nucleotides in length.

In an embodiment, the targeting domain is 26 nucleotides in length.

In an embodiment, the targeting domain comprises 16 nucleotides.

In an embodiment, the targeting domain comprises 17 nucleotides.

In an embodiment, the targeting domain comprises 18 nucleotides.

In an embodiment, the targeting domain comprises 19 nucleotides.

In an embodiment, the targeting domain comprises 20 nucleotides.

In an embodiment, the targeting domain comprises 21 nucleotides.

In an embodiment, the targeting domain comprises 22 nucleotides.

In an embodiment, the targeting domain comprises 23 nucleotides.

In an embodiment, the targeting domain comprises 24 nucleotides.

In an embodiment, the targeting domain comprises 25 nucleotides.

In an embodiment, the targeting domain comprises 26 nucleotides.

In an embodiment, the targeting domain is 10 +/-5, 20+/-5, 30+/-5, 40+/-5, 50+/-5, 60+/-5, 70+/-5, 80+/-5, 90+/-5, or 100+/-5 nucleotides, in length.

In an embodiment, the targeting domain is 20+/-5 nucleotides in length.

In an embodiment, the targeting domain is 20+/-10, 30+/-10, 40+/-10, 50+/-10, 60+/- 10, 70+/-10, 80+/-10, 90+/-10, or 100+/-10 nucleotides, in length.

In an embodiment, the targeting domain is 30+/-10 nucleotides in length.

In an embodiment, the targeting domain is 10 to 100, 10 to 90, 10 to 80, 10 to 70, 10 to 60, 10 to 50, 10 to 40, 10 to 30, 10 to 20 or 10 to 15 nucleotides in length. In another embodiment, the targeting domain is 20 to 100, 20 to 90, 20 to 80, 20 to 70, 20 to 60, 20 to 50, 20 to 40, 20 to 30, or 20 to 25 nucleotides in length.

Typically the targeting domain has full complementarity with the target sequence. In an embodiment, the targeting domain has or includes 1, 2, 3, 4, 5, 6, 7 or 8 nucleotides that are not complementary with the corresponding nucleotide of the targeting domain.

In an embodiment, the target domain includes 1, 2, 3, 4 or 5 nucleotides that are complementary with the corresponding nucleotide of the targeting domain within 5 nucleotides of its 5' end. In an embodiment, the target domain includes 1, 2, 3, 4 or 5 nucleotides that are complementary with the corresponding nucleotide of the targeting domain within 5 nucleotides of its 3' end.

In an embodiment, the target domain includes 1, 2, 3, or 4 nucleotides that are not complementary with the corresponding nucleotide of the targeting domain within 5 nucleotides of its 5' end. In an embodiment, the target domain includes 1, 2, 3, or 4 nucleotides that are not complementary with the corresponding nucleotide of the targeting domain within 5 nucleotides of its 3' end.

In an embodiment, the degree of complementarity, together with other properties of the gRNA, is sufficient to allow targeting of a Cas9 molecule to the target nucleic acid.

In an embodiment, the targeting domain comprises two consecutive nucleotides that are not complementary to the target domain ("non-complementary nucleotides"), e.g., two consecutive noncomplementary nucleotides that are within 5 nucleotides of the 5' end of the targeting domain, within 5 nucleotides of the 3' end of the targeting domain, or more than 5 nucleotides away from one or both ends of the targeting domain.

In an embodiment, no two consecutive nucleotides within 5 nucleotides of the 5' end of the targeting domain, within 5 nucleotides of the 3' end of the targeting domain, or within a region that is more than 5 nucleotides away from one or both ends of the targeting domain, are not complementary to the targeting domain.

In an embodiment, there are no noncomplementary nucleotides within 5 nucleotides of the 5' end of the targeting domain, within 5 nucleotides of the 3' end of the targeting domain, or within a region that is more than 5 nucleotides away from one or both ends of the targeting domain.

In an embodiment, the targeting domain nucleotides do not comprise modifications, e.g., modifications of the type provided in Section VIII. However, in an embodiment, the targeting domain comprises one or more modifications, e.g., modifications that render it less susceptible to degradation or more bio-compatible, e.g., less immunogenic. By way of example, the backbone of the targeting domain can be modified with a phosphorothioate, or other modification(s) from Section VIII. In an embodiment, a nucleotide of the targeting domain can comprise a 2' modification, e.g., a 2-acetylation, e.g., a 2' methylation, or other modification(s) from Section VIII.

In an embodiment, the targeting domain includes 1, 2, 3, 4, 5, 6, 7 or 8 or more modifications. In an embodiment, the targeting domain includes 1, 2, 3, or 4 modifications within 5 nucleotides of its 5' end. In an embodiment, the targeting domain comprises as many as 1, 2, 3, or 4 modifications within 5 nucleotides of its 3' end.

In an embodiment, the targeting domain comprises modifications at two consecutive nucleotides, e.g., two consecutive nucleotides that are within 5 nucleotides of the 5' end of the targeting domain, within 5 nucleotides of the 3' end of the targeting domain, or more than 5 nucleotides away from one or both ends of the targeting domain.

In an embodiment, no two consecutive nucleotides are modified within 5 nucleotides of the 5' end of the targeting domain, within 5 nucleotides of the 3' end of the targeting domain, or within a region that is more than 5 nucleotides away from one or both ends of the targeting domain. In an embodiment, no nucleotide is modified within 5 nucleotides of the 5' end of the targeting domain, within 5 nucleotides of the 3' end of the targeting domain, or within a region that is more than 5 nucleotides away from one or both ends of the targeting domain.

Modifications in the targeting domain can be selected so as to not interfere with targeting efficacy, which can be evaluated by testing a candidate modification in the system described in Section IV. gRNAs having a candidate targeting domain having a selected length, sequence, degree of complementarity, or degree of modification, can be evaluated in a system in Section IV. The candidate targeting domain can be placed, either alone, or with one or more other candidate changes in a gRNA molecule/Cas9 molecule system known to be functional with a selected target and evaluated.

In an embodiment, all of the modified nucleotides are complementary to and capable of hybridizing to corresponding nucleotides present in the target domain. In another embodiment, 1, 2, 3, 4, 5, 6, 7 or 8 or more modified nucleotides are not complementary to or capable of hybridizing to corresponding nucleotides present in the target domain.

In an embodiment, the targeting domain comprises, preferably in the 5'→3' direction: a secondary domain and a core domain. These domains are discussed in more detail below.

### The Core Domain and Secondary Domain of the Targeting Domain

The "core domain" of the targeting domain is complementary to the "core domain target" on the target nucleic acid. In an embodiment, the core domain comprises about 8 to about 13 nucleotides from the 3' end of the targeting domain (e.g., the most 3' 8 to 13 nucleotides of the targeting domain).

In an embodiment, the core domain and targeting domain, are independently, 6 +/-2, 7+/-2, 8+/-2, 9+/-2, 10+/-2, 11+/-2, 12+/-2, 13+/-2, 14+/-2, 15+/-2, or 16+-2, nucleotides in length.

In an embodiment, the core domain and targeting domain, are independently, 10+/-2 nucleotides in length.

In an embodiment, the core domain and targeting domain, are independently, 10+/-4 nucleotides in length.

In an embodiment, the core domain and targeting domain are independently 6, 7, 8, 9, 10, 11, 12, 13, 14, 15, 16, 17, or 18 nucleotides in length.

In an embodiment, the core domain and targeting domain are independently 3 to 20, 4 to 20, 5 to 20, 6 to 20, 7 to 20, 8 to 20, 9 to 20 10 to 20 or 15 to 20 nucleotides in length.

In an embodiment, the core domain and targeting domain are independently 3 to 15, e.g., 6 to 15, 7 to 14, 7 to 13, 6 to 12, 7 to 12, 7 to 11, 7 to 10, 8 to 14, 8 to 13, 8 to 12, 8 to 11, 8 to 10 or 8 to 9 nucleotides in length.

The core domain is complementary with the core domain target. Typically the core domain has exact complementarity with the core domain target. In some embodiments, the core domain can have 1, 2, 3, 4 or 5 nucleotides that are not complementary with the corresponding nucleotide of the core domain. In an embodiment, the degree of complementarity, together with other properties of the gRNA, is sufficient to allow targeting of a Cas9 molecule to the target nucleic acid.

The "secondary domain" of the targeting domain of the gRNA is complementary to the "secondary domain target" of the target nucleic acid.

In an embodiment, the secondary domain is positioned 5' to the core domain.

In an embodiment, the secondary domain is absent or optional.

In an embodiment, if the targeting domain is 26 nucleotides in length and the core domain (counted from the 3' end of the targeting domain) is 8 to 13 nucleotides in length, the secondary domain is 12 to 17 nucleotides in length.

In an embodiment, if the targeting domain is 25 nucleotides in length and the core domain (counted from the 3' end of the targeting domain) is 8 to 13 nucleotides in length, the secondary domain is 12 to 17 nucleotides in length.

In an embodiment, if the targeting domain is 24 nucleotides in length and the core domain (counted from the 3' end of the targeting domain) is 8 to 13 nucleotides in length, the secondary domain is 11 to 16 nucleotides in length.

In an embodiment, if the targeting domain is 23 nucleotides in length and the core domain (counted from the 3' end of the targeting domain) is 8 to 13 nucleotides in length, the secondary domain is 10 to 15 nucleotides in length.

In an embodiment, if the targeting domain is 22 nucleotides in length and the core domain (counted from the 3' end of the targeting domain) is 8 to 13 nucleotides in length, the secondary domain is 9 to 14 nucleotides in length.

In an embodiment, if the targeting domain is 21 nucleotides in length and the core domain (counted from the 3' end of the targeting domain) is 8 to 13 nucleotides in length, the secondary domain is 8 to 13 nucleotides in length.

In an embodiment, if the targeting domain is 20 nucleotides in length and the core domain (counted from the 3' end of the targeting domain) is 8 to 13 nucleotides in length, the secondary domain is 7 to 12 nucleotides in length.

In an embodiment, if the targeting domain is 19 nucleotides in length and the core domain (counted from the 3' end of the targeting domain) is 8 to 13 nucleotides in length, the secondary domain is 6 to 11 nucleotides in length.

In an embodiment, if the targeting domain is 18 nucleotides in length and the core domain (counted from the 3' end of the targeting domain) is 8 to 13 nucleotides in length, the secondary domain is 5 to 10 nucleotides in length.

In an embodiment, if the targeting domain is 17 nucleotides in length and the core domain (counted from the 3' end of the targeting domain) is 8 to 13 nucleotides in length, the secondary domain is 4 to 9 nucleotides in length.

In an embodiment, if the targeting domain is 16 nucleotides in length and the core domain (counted from the 3' end of the targeting domain) is 8 to 13 nucleotides in length, the secondary domain is 3 to 8 nucleotides in length.

In an embodiment, the secondary domain is 0, 1, 2, 3, 4, 5, 6, 7, 8, 9, 10, 11, 12, 13, 14 or 15 nucleotides in length.

The secondary domain is complementary with the secondary domain target. Typically the secondary domain has exact complementarity with the secondary domain target. In an embodiment, the secondary domain can have 1, 2, 3, 4 or 5 nucleotides that are not complementary with the corresponding nucleotide of the secondary domain. In an embodiment, the degree of complementarity, together with other properties of the gRNA, is sufficient to allow targeting of a Cas9 molecule to the target nucleic acid.

In an embodiment, the core domain nucleotides do not comprise modifications, e.g., modifications of the type provided in Section VIII. However, in an embodiment, the core domain comprises one or more modifications, e.g., modifications that render it less susceptible to degradation or more bio-compatible, e.g., less immunogenic. By way of example, the backbone of the core domain can be modified with a phosphorothioate, or other modification(s) from Section VIII. In an embodiment a nucleotide of the core domain can comprise a 2' modification, e.g., a 2-acetylation, e.g., a 2' methylation, or other modification(s) from Section VIII. Typically, a core domain will contain no more than 1, 2, or 3 modifications.

Modifications in the core domain can be selected so as to not interfere with targeting efficacy, which can be evaluated by testing a candidate modification in the system described in Section IV. gRNAs having a candidate core domain having a selected length, sequence, degree of complementarity, or degree of modification, can be evaluated in the system described at Section IV. The candidate core domain can be placed, either alone, or with one or more other candidate changes in a gRNA molecule/Cas9 molecule system known to be functional with a selected target and evaluated.

In an embodiment, the secondary domain nucleotides do not comprise modifications, e.g., modifications of the type provided in Section VIII. However, in an embodiment, the secondary domain comprises one or more modifications, e.g., modifications that render it less susceptible to degradation or more bio-compatible, e.g., less immunogenic. By way of example, the backbone of the secondary domain can be modified with a phosphorothioate, or other modification(s) from Section VIII. In an embodiment a nucleotide of the secondary domain can comprise a 2' modification, e.g., a 2-acetylation, e.g., a 2' methylation, or other modification(s) from Section VIII. Typically, a secondary domain will contain no more than 1, 2, or 3 modifications.

Modifications in the secondary domain can be selected so as to not interfere with targeting efficacy, which can be evaluated by testing a candidate modification in the system described in Section IV. gRNAs having a candidate secondary domain having a selected length, sequence, degree of complementarity, or degree of modification, can be evaluated in the system described at Section IV. The candidate secondary domain can be placed, either alone, or with one or more other candidate changes in a gRNA molecule/Cas9 molecule system known to be functional with a selected target and evaluated.

In an embodiment, (1) the degree of complementarity between the core domain and its target, and (2) the degree of complementarity between the secondary domain and its target, may differ. In an embodiment, (1) may be greater than (2). In an embodiment, (1) may be less than (2). In an embodiment, (1) and (2) are the same, e.g., each may be completely complementary with its target.

In an embodiment, (1) the number of modifications (e.g., modifications from Section VIII) of the nucleotides of the core domain and (2) the number of modification (e.g., modifications from Section VIII) of the nucleotides of the secondary domain, may differ. In an embodiment, (1) may be less than (2). In an embodiment, (1) may be greater than (2). In an embodiment, (1) and (2) may be the same, e.g., each may be free of modifications.

### The First and Second Complementarity Domains

The first complementarity domain is complementary with the second complementarity domain.

Typically the first domain does not have exact complementarity with the second complementarity domain target. In some embodiments, the first complementarity domain can have 1, 2, 3, 4 or 5 nucleotides that are not complementary with the corresponding nucleotide of the second complementarity domain. In an embodiment, 1, 2, 3, 4, 5 or 6, e.g., 3 nucleotides, will not pair in the duplex, and, e.g., form a non-duplexed or looped-out region. In an embodiment, an unpaired, or loop-out, region, e.g., a loop-out of 3 nucleotides, is present on the second complementarity domain. In an embodiment, the unpaired region begins 1, 2, 3, 4, 5, or 6, e.g., 4, nucleotides from the 5' end of the second complementarity domain.

In an embodiment, the degree of complementarity, together with other properties of the gRNA, is sufficient to allow targeting of a Cas9 molecule to the target nucleic acid.

In an embodiment, the first and second complementarity domains are:
independently, 6+/-2, 7+/-2, 8+/-2, 9+/-2, 10+/-2, 11+/-2, 12+/-2, 13+/-2, 14+/-2, 15+/-2, 16+/-2, 17+/-2, 18+/-2, 19+/-2, or 20+/-2, 21+/-2, 22+/-2, 23+/-2, or 24+/-2 nucleotides in length;
independently, 6, 7, 8, 9, 10, 11, 12, 13, 14, 14, 16, 17, 18, 19, 20, 21, 22, 23, 24, 25, or 26, nucleotides in length; or
independently, 5 to 24, 5 to 23, 5 to 22, 5 to 21, 5 to 20, 7 to 18, 9 to 16, or 10 to 14 nucleotides in length.

In an embodiment, the second complementarity domain is longer than the first complementarity domain, e.g., 2, 3, 4, 5, or 6, e.g., 6, nucleotides longer.

In an embodiment, the first and second complementary domains, independently, do not comprise modifications, e.g., modifications of the type provided in Section VIII.

In an embodiment, the first and second complementary domains, independently, comprise one or more modifications, e.g., modifications that the render the domain less susceptible to degradation or more bio-compatible, e.g., less immunogenic. By way of example, the backbone of the domain can be modified with a phosphorothioate, or other modification(s) from Section VIII. In an embodiment a nucleotide of the domain can comprise a 2' modification, e.g., a 2-acetylation, e.g., a 2' methylation, or other modification(s) from Section VIII.

In an embodiment, the first and second complementary domains, independently, include 1, 2, 3, 4, 5, 6, 7 or 8 or more modifications. In an embodiment, the first and second complementary domains, independently, include 1, 2, 3, or 4 modifications within 5 nucleotides of its 5' end. In an embodiment, the first and second complementary domains, independently, include as many as 1, 2, 3, or 4 modifications within 5 nucleotides of its 3' end.

In an embodiment, the first and second complementary domains, independently, include modifications at two consecutive nucleotides, e.g., two consecutive nucleotides that are within 5 nucleotides of the 5' end of the domain, within 5 nucleotides of the 3' end of the domain, or more than 5 nucleotides away from one or both ends of the domain. In an embodiment, the first and second complementary domains, independently, include no two consecutive nucleotides that are modified, within 5 nucleotides of the 5' end of the domain, within 5 nucleotides of the 3' end of the domain, or within a region that is more than 5 nucleotides away from one or both ends of the domain. In an embodiment, the first and second complementary domains, independently, include no nucleotide that is modified within 5 nucleotides of the 5' end of the domain, within 5 nucleotides of the 3' end of the domain, or within a region that is more than 5 nucleotides away from one or both ends of the domain.

Modifications in a complementarity domain can be selected so as to not interfere with targeting efficacy, which can be evaluated by testing a candidate modification in the system described in Section IV. gRNAs having a candidate complementarity domain having a selected length, sequence, degree of complementarity, or degree of modification, can be evaluated in the system described in Section IV. The candidate complementarity domain can be placed, either alone, or with one or more other candidate changes in a gRNA molecule/Cas9 molecule system known to be functional with a selected target and evaluated.

In an embodiment, the first complementarity domain has at least 60, 70, 80, 85%, 90% or 95% homology with, or differs by no more than 1, 2, 3, 4, 5, or 6 nucleotides from, a reference first complementarity domain, e.g., a naturally occurring, e.g., an *S. pyogenes, S. aureus* or *S. thermophilus,* first complementarity domain, or a first complementarity domain described herein, e.g., from **Figs. 1A-1G****.**

In an embodiment, the second complementarity domain has at least 60, 70, 80, 85%, 90%, or 95% homology with, or differs by no more than 1, 2, 3, 4, 5, or 6 nucleotides from, a reference second complementarity domain, e.g., a naturally occurring, e.g., an *S. pyogenes, S. aureus* or *S. thermophilus,* second complementarity domain, or a second complementarity domain described herein, e.g., from **Figs. 1A-1G****.**

The duplexed region formed by first and second complementarity domains is typically 6, 7, 8, 9, 10, 11, 12, 13, 14, 15, 16, 17, 18, 19, 20, 21 or 22 base pairs in length (excluding any looped out or unpaired nucleotides).

In some embodiments, the first and second complementarity domains, when duplexed, comprise 11 paired nucleotides, for example, in the gRNA sequence (one paired strand underlined, one bolded):

NNNNNNNNNNNNNNNNNNNNGUUUUAGAGCUAGAAAUAGCAAGUUAAAAUAA
GGCUAGUCCGUUAUCAACUUGAAAAAGUGGCACCGAGUCGGUGC (SEQ ID NO:
5).

In some embodiments, the first and second complementarity domains, when duplexed, comprise 15 paired nucleotides, for example in the gRNA sequence (one paired strand underlined, one bolded):

NNNNNNNNNNNNNNNNNNNNGUUUUAGAGCUAUGCUGAAAAGCAUAGCAAGU
UAAAAUAAGGCUAGUCCGUUAUCAACUUGAAAAAGUGGCACCGAGUCGGUGC
(SEQ ID NO: 27).

In some embodiments the first and second complementarity domains, when duplexed, comprise 16 paired nucleotides, for example in the gRNA sequence (one paired strand underlined, one bolded):

NNNNNNNNNNNNNNNNNNNNGUUUUAGAGCUAUGCUGGAAACAGCAUAGCAA
GUUAAAAUAAGGCUAGUCCGUUAUCAACUUGAAAAAGUGGCACCGAGUCGGU
GC (SEQ ID NO: 28).

In some embodiments the first and second complementarity domains, when duplexed, comprise 21 paired nucleotides, for example in the gRNA sequence (one paired strand underlined, one bolded):

NNNNNNNNNNNNNNNNNNNNGUUUUAGAGCUAUGCUGUUUUGGAAACAAAAC
AGCAUAGCAAGUUAAAAUAAGGCUAGUCCGUUAUCAACUUGAAAAAGUGGCA
CCGAGUCGGUGC (SEQ ID NO: 29).

In some embodiments, nucleotides are exchanged to remove poly-U tracts, for example in the gRNA sequences (exchanged nucleotides underlined):

NNNNNNNNNNNNNNNNNNNNGUAUUAGAGCUAGAAAUAGCAAGUUAAUAUAA
GGCUAGUCCGUUAUCAACUUGAAAAAGUGGCACCGAGUCGGUGC (SEQ ID NO:
30);

NNNNNNNNNNNNNNNNNNNNGUUUAAGAGCUAGAAAUAGCAAGUUUAAAUAA
GGCUAGUCCGUUAUCAACUUGAAAAAGUGGCACCGAGUCGGUGC (SEQ ID NO:
31); or

NNNNNNNNNNNNNNNNNNNNGUAUUAGAGCUAUGCUGUAUUGGAAACAAUAC
AGCAUAGCAAGUUAAUAUAAGGCUAGUCCGUUAUCAACUUGAAAAAGUGGCA
CCGAGUCGGUGC (SEQ ID NO: 32).

### The 5' Extension Domain

In an embodiment, a modular gRNA can comprise additional sequence, 5' to the second complementarity domain. In an embodiment, the 5' extension domain is 2 to 10, 2 to 9, 2 to 8, 2 to 7, 2 to 6, 2 to 5, or 2 to 4 nucleotides in length. In an embodiment, the 5' extension domain is 2, 3, 4, 5, 6, 7, 8, 9, or 10 or more nucleotides in length.

In an embodiment, the 5' extension domain nucleotides do not comprise modifications, e.g., modifications of the type provided in Section VIII. However, in an embodiment, the 5' extension domain comprises one or more modifications, e.g., modifications that render it less susceptible to degradation or more bio-compatible, e.g., less immunogenic. By way of example, the backbone of the 5' extension domain can be modified with a phosphorothioate, or other modification(s) from Section VIII. In an embodiment, a nucleotide of the 5' extension domain can comprise a 2' modification, e.g., a 2-acetylation, e.g., a 2' methylation, or other modification(s) from Section VIII.

In an embodiment, the 5' extension domain can comprise as many as 1, 2, 3, 4, 5, 6, 7 or 8 modifications. In an embodiment, the 5' extension domain comprises as many as 1, 2, 3, or 4 modifications within 5 nucleotides of its 5' end, e.g., in a modular gRNA molecule. In an embodiment, the 5' extension domain comprises as many as 1, 2, 3, or 4 modifications within 5 nucleotides of its 3' end, e.g., in a modular gRNA molecule.

In an embodiment, the 5' extension domain comprises modifications at two consecutive nucleotides, e.g., two consecutive nucleotides that are within 5 nucleotides of the 5' end of the 5' extension domain, within 5 nucleotides of the 3' end of the 5' extension domain, or more than 5 nucleotides away from one or both ends of the 5' extension domain. In an embodiment, no two consecutive nucleotides are modified within 5 nucleotides of the 5' end of the 5' extension domain, within 5 nucleotides of the 3' end of the 5' extension domain, or within a region that is more than 5 nucleotides away from one or both ends of the 5' extension domain. In an embodiment, no nucleotide is modified within 5 nucleotides of the 5' end of the 5' extension domain, within 5 nucleotides of the 3' end of the 5' extension domain, or within a region that is more than 5 nucleotides away from one or both ends of the 5' extension domain.

Modifications in the 5' extension domain can be selected so as to not interfere with gRNA molecule efficacy, which can be evaluated by testing a candidate modification in the system described in Section IV. gRNAs having a candidate 5' extension domain having a selected length, sequence, degree of complementarity, or degree of modification, can be evaluated in the system described at Section IV. The candidate 5' extension domain can be placed, either alone, or with one or more other candidate changes in a gRNA molecule/Cas9 molecule system known to be functional with a selected target and evaluated.

In an embodiment, the 5' extension domain has at least 60, 70, 80, 85, 90 or 95% homology with, or differs by no more than 1, 2, 3, 4, 5, or 6 nucleotides from, a reference 5' extension domain, e.g., a naturally occurring, e.g., an *S. pyogenes, S. aureus* or *S*. *thermophilus,* 5' extension domain, or a 5' extension domain described herein, e.g., from **Figs. 1A-1G****.**

### The Linking Domain

In a unimolecular gRNA molecule the linking domain is disposed between the first and second complementarity domains. In a modular gRNA molecule, the two molecules are associated with one another by the complementarity domains.

In an embodiment, the linking domain is 10 +/-5, 20+/-5, 30+/-5, 40+/-5, 50+/-5, 60+/-5, 70+/-5, 80+/-5, 90+/-5, or 100+/-5 nucleotides, in length.

In an embodiment, the linking domain is 20+/-10, 30+/-10, 40+/-10, 50+/-10, 60+/- 10, 70+/-10, 80+/-10, 90+/-10, or 100+/-10 nucleotides, in length.

In an embodiment, the linking domain is 10 to 100, 10 to 90, 10 to 80, 10 to 70, 10 to 60, 10 to 50, 10 to 40, 10 to 30, 10 to 20 or 10 to 15 nucleotides in length. In other embodiments, the linking domain is 20 to 100, 20 to 90, 20 to 80, 20 to 70, 20 to 60, 20 to 50, 20 to 40, 20 to 30, or 20 to 25 nucleotides in length.

In an embodiment, the linking domain is 1, 2, 3, 4, 5, 6, 7, 8, 9, 10, 11, 12, 13, 14, 15, 16 17, 18, 19, or 20 nucleotides in length.

In an embodiment, the linking domain is a covalent bond.

In an embodiment, the linking domain comprises a duplexed region, typically adjacent to or within 1, 2, or 3 nucleotides of the 3' end of the first complementarity domain and/or the 5- end of the second complementarity domain. In an embodiment, the duplexed region can be 20+/-10 base pairs in length. In an embodiment, the duplexed region can be 10+/-5, 15+/-5, 20+/-5, or 30+/-5 base pairs in length. In an embodiment, the duplexed region can be 1, 2, 3, 4, 5, 6, 7, 8, 9, 10, 11, 12, 13, 14, or 15 base pairs in length.

Typically the sequences forming the duplexed region have exact complementarity with one another, though in some embodiments as many as 1, 2, 3, 4, 5, 6, 7 or 8 nucleotides are not complementary with the corresponding nucleotides.

In an embodiment, the linking domain nucleotides do not comprise modifications, e.g., modifications of the type provided in Section VIII. However, in an embodiment, the linking domain comprises one or more modifications, e.g., modifications that render it less susceptible to degradation or more bio-compatible, e.g., less immunogenic. By way of example, the backbone of the linking domain can be modified with a phosphorothioate, or other modification(s) from Section VIII. In an embodiment a nucleotide of the linking domain can comprise a 2' modification, e.g., a 2-acetylation, e.g., a 2' methylation, or other modification(s) from Section VIII. In some embodiments, the linking domain can comprise as many as 1, 2, 3, 4, 5, 6, 7 or 8 modifications.

Modifications in a linking domain can be selected so as to not interfere with targeting efficacy, which can be evaluated by testing a candidate modification in the system described in Section IV. gRNAs having a candidate linking domain having a selected length, sequence, degree of complementarity, or degree of modification, can be evaluated a system described in Section IV. A candidate linking domain can be placed, either alone, or with one or more other candidate changes in a gRNA molecule/Cas9 molecule system known to be functional with a selected target and evaluated.

In an embodiment, the linking domain has at least 60, 70, 80, 85, 90 or 95% homology with, or differs by no more than 1, 2, 3, 4, 5 ,or 6 nucleotides from, a reference linking domain, e.g., a linking domain described herein, e.g., from **Figs. 1A-1G****.**

### The Proximal Domain

In an embodiment, the proximal domain is 6+/-2, 7+/-2, 8+/-2, 9+/-2, 10+/-2, 11+/-2, 12+/-2, 13+/-2, 14+/-2, 14+/-2, 16+/-2, 17+/-2, 18+/-2, 19+/-2, or 20+/-2 nucleotides in length.

In an embodiment, the proximal domain is 6, 7, 8, 9, 10, 11, 12, 13, 14, 15, 16, 17, 18, 19, 20, 21, 22, 23, 24, 25 or 26 nucleotides in length.

In an embodiment, the proximal domain is 5 to 20, 7, to 18, 9 to 16, or 10 to 14 nucleotides in length.

In an embodiment, the proximal domain nucleotides do not comprise modifications, e.g., modifications of the type provided in Section VIII. However, in an embodiment, the proximal domain comprises one or more modifications, e.g., modifications that render it less susceptible to degradation or more bio-compatible, e.g., less immunogenic. By way of example, the backbone of the proximal domain can be modified with a phosphorothioate, or other modification(s) from Section VIII. In an embodiment a nucleotide of the proximal domain can comprise a 2' modification, e.g., a 2-acetylation, e.g., a 2' methylation, or other modification(s) from Section VIII.

In an embodiment, the proximal domain can comprise as many as 1, 2, 3, 4, 5, 6, 7 or 8 modifications. In an embodiment, the proximal domain comprises as many as 1, 2, 3, or 4 modifications within 5 nucleotides of its 5' end, e.g., in a modular gRNA molecule. In an embodiment, the target domain comprises as many as 1, 2, 3, or 4 modifications within 5 nucleotides of its 3' end, e.g., in a modular gRNA molecule.

In an embodiment, the proximal domain comprises modifications at two consecutive nucleotides, e.g., two consecutive nucleotides that are within 5 nucleotides of the 5' end of the proximal domain, within 5 nucleotides of the 3' end of the proximal domain, or more than 5 nucleotides away from one or both ends of the proximal domain. In an embodiment, no two consecutive nucleotides are modified within 5 nucleotides of the 5' end of the proximal domain, within 5 nucleotides of the 3' end of the proximal domain, or within a region that is more than 5 nucleotides away from one or both ends of the proximal domain. In an embodiment, no nucleotide is modified within 5 nucleotides of the 5' end of the proximal domain, within 5 nucleotides of the 3' end of the proximal domain, or within a region that is more than 5 nucleotides away from one or both ends of the proximal domain.

Modifications in the proximal domain can be selected so as to not interfere with gRNA molecule efficacy, which can be evaluated by testing a candidate modification in the system described in Section IV. gRNAs having a candidate proximal domain having a selected length, sequence, degree of complementarity, or degree of modification, can be evaluated in the system described at Section IV. The candidate proximal domain can be placed, either alone, or with one or more other candidate changes in a gRNA molecule/Cas9 molecule system known to be functional with a selected target and evaluated.

In an embodiment, the proximal domain has at least 60, 70, 80, 85 90 or 95% homology with, or differs by no more than 1, 2, 3, 4, 5 ,or 6 nucleotides from, a reference proximal domain, e.g., a naturally occurring, e.g., an *S. pyogenes, S. aureus* or *S. thermophilus,* proximal domain, or a proximal domain described herein, e.g., from **Figs. 1A-****1G.**

### The Tail Domain

In an embodiment, the tail domain is 10 +/-5, 20+/-5, 30+/-5, 40+/-5, 50+/-5, 60+/-5, 70+/-5, 80+/-5, 90+/-5, or 100+/-5 nucleotides, in length.

In an embodiment, the tail domain is 20+/-5 nucleotides in length.

In an embodiment, the tail domain is 20+/-10, 30+/-10, 40+/-10, 50+/-10, 60+/-10, 70+/-10, 80+/-10, 90+/-10, or 100+/-10 nucleotides, in length.

In an embodiment, the tail domain is 25+/-10 nucleotides in length.

In an embodiment, the tail domain is 10 to 100, 10 to 90, 10 to 80, 10 to 70, 10 to 60, 10 to 50, 10 to 40, 10 to 30, 10 to 20 or 10 to 15 nucleotides in length.

In other embodiments, the tail domain is 20 to 100, 20 to 90, 20 to 80, 20 to 70, 20 to 60, 20 to 50, 20 to 40, 20 to 30, or 20 to 25 nucleotides in length.

In an embodiment, the tail domain is 1 to 20, 1 to 15, 1 to 10, or 1 to 5 nucleotides in length.

In an embodiment, the tail domain nucleotides do not comprise modifications, e.g., modifications of the type provided in Section VIII. However, in an embodiment, the tail domain comprises one or more modifications, e.g., modifications that render it less susceptible to degradation or more bio-compatible, e.g., less immunogenic. By way of example, the backbone of the tail domain can be modified with a phosphorothioate, or other modification(s) from Section VIII. In an embodiment a nucleotide of the tail domain can comprise a 2' modification, e.g., a 2-acetylation, e.g., a 2' methylation, or other modification(s) from Section VIII.

In some embodiments, the tail domain can have as many as 1, 2, 3, 4, 5, 6, 7 or 8 modifications. In an embodiment, the target domain comprises as many as 1, 2, 3, or 4 modifications within 5 nucleotides of its 5' end. In an embodiment, the target domain comprises as many as 1, 2, 3, or 4 modifications within 5 nucleotides of its 3' end.

In an embodiment, the tail domain comprises a tail duplex domain, which can form a tail duplexed region. In an embodiment, the tail duplexed region can be 3, 4, 5, 6, 7, 8, 9, 10, 11, or 12 base pairs in length. In an embodiment, a further single stranded domain, exists 3' to the tail duplexed domain. In an embodiment, this domain is 3, 4, 5, 6, 7, 8, 9, or 10 nucleotides in length. In an embodiment it is 4 to 6 nucleotides in length.

In an embodiment, the tail domain has at least 60, 70, 80, or 90% homology with, or differs by no more than 1, 2, 3, 4, 5 ,or 6 nucleotides from, a reference tail domain, e.g., a naturally occurring, e.g., an *S. pyogenes, S. aureus* or *S. thermophilus,* tail domain, or a tail domain described herein, e.g., from **Figs. 1A-1G****.**

In an embodiment, the proximal and tail domain, taken together, comprise the following sequences:
AAGGCUAGUCCGUUAUCAACUUGAAAAAGUGGCACCGAGUCGGUGCU (SEQ ID NO: 33), or
AAGGCUAGUCCGUUAUCAACUUGAAAAAGUGGCACCGAGUCGGUGGUGC (SEQ ID NO: 34), or
AAGGCUAGUCCGUUAUCAACUUGAAAAAGUGGCACCGAGUCGGUGCGGAUC (SEQ ID NO: 35), or
AAGGCUAGUCCGUUAUCAACUUGAAAAAGUG (SEQ ID NO: 36), or AAGGCUAGUCCGUUAUCA (SEQ ID NO: 37), or
AAGGCUAGUCCG (SEQ ID NO: 38).

In an embodiment, the tail domain comprises the 3' sequence UUUUUU, e.g., if a U6 promoter is used for transcription.

In an embodiment, the tail domain comprises the 3' sequence UUUU, e.g., if an H1 promoter is used for transcription.

In an embodiment, tail domain comprises variable numbers of 3' Us depending, e.g., on the termination signal of the pol-III promoter used.

In an embodiment, the tail domain comprises variable 3' sequence derived from the DNA template if a T7 promoter is used.

In an embodiment, the tail domain comprises variable 3' sequence derived from the DNA template, e.g., if in vitro transcription is used to generate the RNA molecule.

In an embodiment, the tail domain comprises variable 3' sequence derived from the DNA template, e.g., if a pol-II promoter is used to drive transcription.

Modifications in the tail domain can be selected so as to not interfere with targeting efficacy, which can be evaluated by testing a candidate modification in the system described in Section IV. gRNAs having a candidate tail domain having a selected length, sequence, degree of complementarity, or degree of modification, can be evaluated in the system described in Section IV. The candidate tail domain can be placed, either alone, or with one or more other candidate changes in a gRNA molecule/Cas9 molecule system known to be functional with a selected target and evaluated.

In some embodiments, the tail domain comprises modifications at two consecutive nucleotides, e.g., two consecutive nucleotides that are within 5 nucleotides of the 5' end of the tail domain, within 5 nucleotides of the 3' end of the tail domain, or more than 5 nucleotides away from one or both ends of the tail domain. In an embodiment, no two consecutive nucleotides are modified within 5 nucleotides of the 5' end of the tail domain, within 5 nucleotides of the 3' end of the tail domain, or within a region that is more than 5 nucleotides away from one or both ends of the tail domain. In an embodiment, no nucleotide is modified within 5 nucleotides of the 5' end of the tail domain, within 5 nucleotides of the 3' end of the tail domain, or within a region that is more than 5 nucleotides away from one or both ends of the tail domain.

In an embodiment a gRNA has the following structure:
5' [targeting domain]-[first complementarity domain]-[linking domain]-[second complementarity domain]-[proximal domain]-[tail domain]-3'
wherein, the targeting domain comprises a core domain and optionally a secondary domain, and is 10 to 50 nucleotides in length;
the first complementarity domain is 5 to 25 nucleotides in length and, In an embodiment has at least 50, 60, 70, 80, 85, 90 or 95% homology with a reference first complementarity domain disclosed herein;
the linking domain is 1 to 5 nucleotides in length;
the second complementarity domain is 5 to 27 nucleotides in length and, in an embodiment has at least 50, 60, 70, 80, 85, 90 or 95% homology with a reference second complementarity domain disclosed herein;
the proximal domain is 5 to 20 nucleotides in length and, in an embodiment has at least 50, 60, 70, 80, 85, 90 or 95% homology with a reference proximal domain disclosed herein; and
the tail domain is absent or a nucleotide sequence is 1 to 50 nucleotides in length and, in an embodiment has at least 50, 60, 70, 80, 85, 90 or 95% homology with a reference tail domain disclosed herein.

### Exemplary Chimeric gRNAs

In an embodiment, a unimolecular, or chimeric, gRNA comprises, preferably from 5' to 3':
a targeting domain (which is complementary to a target nucleic acid);
a first complementarity domain, e.g., comprising 15, 16, 17, 18, 19, 20, 21, 22, 23, 24, 25, or 26 nucleotides;
a linking domain;
a second complementarity domain (which is complementary to the first complementarity domain);
a proximal domain; and
a tail domain,
wherein,
   (a) the proximal and tail domain, when taken together, comprise at least 15, 18, 20, 25, 30, 31, 35, 40, 45, 49, 50, or 53 nucleotides;
   (b) there are at least 15, 18, 20, 25, 30, 31, 35, 40, 45, 49, 50, or 53 nucleotides 3' to the last nucleotide of the second complementarity domain; or
   (c) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the sequence from (a), (b), or (c), has at least 60, 75, 80, 85, 90, 95, or 99% homology with the corresponding sequence of a naturally occurring gRNA, or with a gRNA described herein.

In an embodiment, the proximal and tail domain, when taken together, comprise at least 15, 18, 20, 25, 30, 31, 35, 40, 45, 49, 50, or 53 nucleotides.

In an embodiment, there are at least 15, 18, 20, 25, 30, 31, 35, 40, 45, 49, 50, or 53 nucleotides 3' to the last nucleotide of the second complementarity domain.

In an embodiment, there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 16, 17, 18, 19, 20, 21, 22, 23, 24, 25 or 26 nucleotides (e.g., 16, 17, 18, 19, 20, 21, 22, 23, 24, 25 or 26 consecutive nucleotides) having complementarity with the target domain, e.g., the targeting domain is 16, 17, 18, 19, 20, 21, 22, 23, 24, 25 or 26 nucleotides in length.

In an embodiment, the targeting domain comprises, has, or consists of, 16 nucleotides (e.g., 16 consecutive nucleotides) having complementarity with the target domain, e.g., the targeting domain is 16 nucleotides in length.

In an embodiment, the targeting domain comprises, has, or consists of, 17 nucleotides (e.g., 17 consecutive nucleotides) having complementarity with the target domain, e.g., the targeting domain is 17 nucleotides in length.

In an embodiment, the targeting domain comprises, has, or consists of, 18 nucleotides (e.g., 18 consecutive nucleotides) having complementarity with the target domain, e.g., the targeting domain is 18 nucleotides in length.

In an embodiment, the targeting domain comprises, has, or consists of, 19 nucleotides (e.g., 19 consecutive nucleotides) having complementarity with the target domain, e.g., the targeting domain is 19 nucleotides in length.

In an embodiment, the targeting domain comprises, has, or consists of, 20 nucleotides (e.g., 20 consecutive nucleotides) having complementarity with the target domain, e.g., the targeting domain is 20 nucleotides in length.

In an embodiment, the targeting domain comprises, has, or consists of, 21 nucleotides (e.g., 21 consecutive nucleotides) having complementarity with the target domain, e.g., the targeting domain is 21 nucleotides in length.

In an embodiment, the targeting domain comprises, has, or consists of, 22 nucleotides (e.g., 22 consecutive nucleotides) having complementarity with the target domain, e.g., the targeting domain is 22 nucleotides in length.

In an embodiment, the targeting domain comprises, has, or consists of, 23 nucleotides (e.g., 23 consecutive nucleotides) having complementarity with the target domain, e.g., the targeting domain is 23 nucleotides in length.

In an embodiment, the targeting domain comprises, has, or consists of, 24 nucleotides (e.g., 24 consecutive nucleotides) having complementarity with the target domain, e.g., the targeting domain is 24 nucleotides in length.

In an embodiment, the targeting domain comprises, has, or consists of, 25 nucleotides (e.g., 25 consecutive nucleotides) having complementarity with the target domain, e.g., the targeting domain is 25 nucleotides in length.

In an embodiment, the targeting domain comprises, has, or consists of, 26 nucleotides (e.g., 26 consecutive nucleotides) having complementarity with the target domain, e.g., the targeting domain is 26 nucleotides in length.

In an embodiment, the targeting domain comprises, has, or consists of, 16 nucleotides (e.g., 16 consecutive nucleotides) having complementarity with the target domain, e.g., the targeting domain is 16 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 16 nucleotides (e.g., 16 consecutive nucleotides) having complementarity with the target domain, e.g., the targeting domain is 16 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 16 nucleotides (e.g., 16 consecutive nucleotides) having complementarity with the target domain, e.g., the targeting domain is 16 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 17 nucleotides (e.g., 17 consecutive nucleotides) having complementarity with the target domain, e.g., the targeting domain is 17 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 17 nucleotides (e.g., 17 consecutive nucleotides) having complementarity with the target domain, e.g., the targeting domain is 17 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 17 nucleotides (e.g., 17 consecutive nucleotides) having complementarity with the target domain, e.g., the targeting domain is 17 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 18 nucleotides (e.g., 18 consecutive nucleotides) having complementarity with the target domain, e.g., the targeting domain is 18 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 18 nucleotides (e.g., 18 consecutive nucleotides) having complementarity with the target domain, e.g., the targeting domain is 18 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 18 nucleotides (e.g., 18 consecutive nucleotides) having complementarity with the target domain, e.g., the targeting domain is 18 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 19 nucleotides (e.g., 19 consecutive nucleotides) having complementarity with the target domain, e.g., the targeting domain is 19 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 19 nucleotides (e.g., 19 consecutive nucleotides) having complementarity with the target domain, e.g., the targeting domain is 19 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 19 nucleotides (e.g., 19 consecutive nucleotides) having complementarity with the target domain, e.g., the targeting domain is 19 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 20 nucleotides (e.g., 20 consecutive nucleotides) having complementarity with the target domain, e.g., the targeting domain is 20 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 20 nucleotides (e.g., 20 consecutive nucleotides) having complementarity with the target domain, e.g., the targeting domain is 20 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 20 nucleotides (e.g., 20 consecutive nucleotides) having complementarity with the target domain, e.g., the targeting domain is 20 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 21 nucleotides (e.g., 21 consecutive nucleotides) having complementarity with the target domain, e.g., the targeting domain is 21 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 21 nucleotides (e.g., 21 consecutive nucleotides) having complementarity with the target domain, e.g., the targeting domain is 21 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 21 nucleotides (e.g., 21 consecutive nucleotides) having complementarity with the target domain, e.g., the targeting domain is 21 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 22 nucleotides (e.g., 22 consecutive nucleotides) having complementarity with the target domain, e.g., the targeting domain is 22 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 22 nucleotides (e.g., 22 consecutive nucleotides) having complementarity with the target domain, e.g., the targeting domain is 22 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 22 nucleotides (e.g., 22 consecutive nucleotides) having complementarity with the target domain, e.g., the targeting domain is 22 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 23 nucleotides (e.g., 23 consecutive nucleotides) having complementarity with the target domain, e.g., the targeting domain is 23 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 23 nucleotides (e.g., 23 consecutive nucleotides) having complementarity with the target domain, e.g., the targeting domain is 23 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 23 nucleotides (e.g., 23 consecutive nucleotides) having complementarity with the target domain, e.g., the targeting domain is 23 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 24 nucleotides (e.g., 24 consecutive nucleotides) having complementarity with the target domain, e.g., the targeting domain is 24 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 24 nucleotides (e.g., 24 consecutive nucleotides) having complementarity with the target domain, e.g., the targeting domain is 24 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 24 nucleotides (e.g., 24 consecutive nucleotides) having complementarity with the target domain, e.g., the targeting domain is 24 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 25 nucleotides (e.g., 25 consecutive nucleotides) having complementarity with the target domain, e.g., the targeting domain is 25 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 25 nucleotides (e.g., 25 consecutive nucleotides) having complementarity with the target domain, e.g., the targeting domain is 25 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 25 nucleotides (e.g., 25 consecutive nucleotides) having complementarity with the target domain, e.g., the targeting domain is 25 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 26 nucleotides (e.g., 26 consecutive nucleotides) having complementarity with the target domain, e.g., the targeting domain is 26 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 26 nucleotides (e.g., 26 consecutive nucleotides) having complementarity with the target domain, e.g., the targeting domain is 26 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 26 nucleotides (e.g., 26 consecutive nucleotides) having complementarity with the target domain, e.g., the targeting domain is 26 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the unimolecular, or chimeric, gRNA molecule (comprising a targeting domain, a first complementary domain, a linking domain, a second complementary domain, a proximal domain and, optionally, a tail domain) comprises the following sequence in which the targeting domain is depicted as 20 Ns but could be any sequence and range in length from 16 to 26 nucleotides and in which the gRNA sequence is followed by 6 Us, which serve as a termination signal for the U6 promoter, but which could be either absent or fewer in number:

NNNNNNNNNNNNNNNNNNNNGUUUUAGAGCUAGAAAUAGCAAGUUAAA AUAAGGCUAGUCCGUUAUCAACUUGAAAAAGUGGCACCGAGUCGGUGCUUUU UU (SEQ ID NO: 45). In an embodiment, the unimolecular, or chimeric, gRNA molecule is a *S. pyogenes* gRNA molecule.

In some embodiments, the unimolecular, or chimeric, gRNA molecule (comprising a targeting domain, a first complementary domain, a linking domain, a second complementary domain, a proximal domain and, optionally, a tail domain) comprises the following sequence in which the targeting domain is depicted as 20 Ns but could be any sequence and range in length from 16 to 26 nucleotides and in which the gRNA sequence is followed by 6 Us, which serve as a termination signal for the U6 promoter, but which could be either absent or fewer in number:

NNNNNNNNNNNNNNNNNNNNGUUUUAGUACUCUGGAAACAGAAUCUAC UAAAACAAGGCAAAAUGCCGUGUUUAUCUCGUCAACUUGUUGGCGAGAUUUU UU (SEQ ID NO: 40). In an embodiment, the unimolecular, or chimeric, gRNA molecule is a *S. aureus* gRNA molecule.

### Exemplary Modular gRNAs

In an embodiment, a modular gRNA comprises:
a first strand comprising, preferably from 5' to 3';
a targeting domain, e.g., comprising 15, 16, 17, 18, 19, 20, 21, 22, 23, 24, 25, or 26 nucleotides;
a first complementarity domain; and
a second strand, comprising, preferably from 5' to 3':
optionally a 5' extension domain;
a second complementarity domain;
a proximal domain; and
a tail domain,
wherein:
(a) the proximal and tail domain, when taken together, comprise at least 15, 18, 20, 25, 30, 31, 35, 40, 45, 49, 50, or 53 nucleotides;
(b) there are at least 15, 18, 20, 25, 30, 31, 35, 40, 45, 49, 50, or 53 nucleotides 3' to the last nucleotide of the second complementarity domain; or
(c) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the sequence from (a), (b), or (c), has at least 60, 75, 80, 85, 90, 95, or 99% homology with the corresponding sequence of a naturally occurring gRNA, or with a gRNA described herein.

In an embodiment, the proximal and tail domain, when taken together, comprise at least 15, 18, 20, 25, 30, 31, 35, 40, 45, 49, 50, or 53 nucleotides.

In an embodiment, there are at least 15, 18, 20, 25, 30, 31, 35, 40, 45, 49, 50, or 53 nucleotides 3' to the last nucleotide of the second complementarity domain.

In an embodiment, there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 16, 17, 18, 19, 20, 21, 22, 23, 24, 25 or 26 nucleotides (e.g., 16, 17, 18, 19, 20, 21, 22, 23, 24, 25 or 26 consecutive nucleotides) having complementarity with the target domain, e.g., the targeting domain is 16, 17, 18, 19, 20, 21, 22, 23, 24, 25 or 26 nucleotides in length.

In an embodiment, the targeting domain comprises, has, or consists of, 16 nucleotides (e.g., 16 consecutive nucleotides) having complementarity with the target domain, e.g., the targeting domain is 16 nucleotides in length.

In an embodiment, the targeting domain comprises, has, or consists of, 17 nucleotides (e.g., 17 consecutive nucleotides) having complementarity with the target domain, e.g., the targeting domain is 17 nucleotides in length.

In an embodiment, the targeting domain comprises, has, or consists of, 18 nucleotides (e.g., 18 consecutive nucleotides) having complementarity with the target domain, e.g., the targeting domain is 18 nucleotides in length.

In an embodiment, the targeting domain comprises, has, or consists of, 19 nucleotides (e.g., 19 consecutive nucleotides) having complementarity with the target domain, e.g., the targeting domain is 19 nucleotides in length.

In an embodiment, the targeting domain comprises, has, or consists of, 20 nucleotides (e.g., 20 consecutive nucleotides) having complementarity with the target domain, e.g., the targeting domain is 20 nucleotides in length.

In an embodiment, the targeting domain comprises, has, or consists of, 21 nucleotides (e.g., 21 consecutive nucleotides) having complementarity with the target domain, e.g., the targeting domain is 21 nucleotides in length.

In an embodiment, the targeting domain comprises, has, or consists of, 22 nucleotides (e.g., 22 consecutive nucleotides) having complementarity with the target domain, e.g., the targeting domain is 22 nucleotides in length.

In an embodiment, the targeting domain comprises, has, or consists of, 23 nucleotides (e.g., 23 consecutive nucleotides) having complementarity with the target domain, e.g., the targeting domain is 23 nucleotides in length.

In an embodiment, the targeting domain comprises, has, or consists of, 24 nucleotides (e.g., 24 consecutive nucleotides) having complementarity with the target domain, e.g., the targeting domain is 24 nucleotides in length.

In an embodiment, the targeting domain comprises, has, or consists of, 25 nucleotides (e.g., 25 consecutive nucleotides) having complementarity with the target domain, e.g., the targeting domain is 25 nucleotides in length.

In an embodiment, the targeting domain comprises, has, or consists of, 26 nucleotides (e.g., 26 consecutive nucleotides) having complementarity with the target domain, e.g., the targeting domain is 26 nucleotides in length.

In an embodiment, the targeting domain comprises, has, or consists of, 16 nucleotides (e.g., 16 consecutive nucleotides) having complementarity with the target domain, e.g., the targeting domain is 16 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 16 nucleotides (e.g., 16 consecutive nucleotides) having complementarity with the target domain, e.g., the targeting domain is 16 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 16 nucleotides (e.g., 16 consecutive nucleotides) having complementarity with the target domain, e.g., the targeting domain is 16 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 17 nucleotides (e.g., 17 consecutive nucleotides) having complementarity with the target domain, e.g., the targeting domain is 17 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 17 nucleotides (e.g., 17 consecutive nucleotides) having complementarity with the target domain, e.g., the targeting domain is 17 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 17 nucleotides (e.g., 17 consecutive nucleotides) having complementarity with the target domain, e.g., the targeting domain is 17 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 18 nucleotides (e.g., 18 consecutive nucleotides) having complementarity with the target domain, e.g., the targeting domain is 18 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 18 nucleotides (e.g., 18 consecutive nucleotides) having complementarity with the target domain, e.g., the targeting domain is 18 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 18 nucleotides (e.g., 18 consecutive nucleotides) having complementarity with the target domain, e.g., the targeting domain is 18 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 19 nucleotides (e.g., 19 consecutive nucleotides) having complementarity with the target domain, e.g., the targeting domain is 19 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 19 nucleotides (e.g., 19 consecutive nucleotides) having complementarity with the target domain, e.g., the targeting domain is 19 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 19 nucleotides (e.g., 19 consecutive nucleotides) having complementarity with the target domain, e.g., the targeting domain is 19 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 20 nucleotides (e.g., 20 consecutive nucleotides) having complementarity with the target domain, e.g., the targeting domain is 20 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 20 nucleotides (e.g., 20 consecutive nucleotides) having complementarity with the target domain, e.g., the targeting domain is 20 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 20 nucleotides (e.g., 20 consecutive nucleotides) having complementarity with the target domain, e.g., the targeting domain is 20 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 21 nucleotides (e.g., 21 consecutive nucleotides) having complementarity with the target domain, e.g., the targeting domain is 21 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 21 nucleotides (e.g., 21 consecutive nucleotides) having complementarity with the target domain, e.g., the targeting domain is 21 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 21 nucleotides (e.g., 21 consecutive nucleotides) having complementarity with the target domain, e.g., the targeting domain is 21 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 22 nucleotides (e.g., 22 consecutive nucleotides) having complementarity with the target domain, e.g., the targeting domain is 22 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 22 nucleotides (e.g., 22 consecutive nucleotides) having complementarity with the target domain, e.g., the targeting domain is 22 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 22 nucleotides (e.g., 22 consecutive nucleotides) having complementarity with the target domain, e.g., the targeting domain is 22 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 23 nucleotides (e.g., 23 consecutive nucleotides) having complementarity with the target domain, e.g., the targeting domain is 23 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 23 nucleotides (e.g., 23 consecutive nucleotides) having complementarity with the target domain, e.g., the targeting domain is 23 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 23 nucleotides (e.g., 23 consecutive nucleotides) having complementarity with the target domain, e.g., the targeting domain is 23 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 24 nucleotides (e.g., 24 consecutive nucleotides) having complementarity with the target domain, e.g., the targeting domain is 24 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 24 nucleotides (e.g., 24 consecutive nucleotides) having complementarity with the target domain, e.g., the targeting domain is 24 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 24 nucleotides (e.g., 24 consecutive nucleotides) having complementarity with the target domain, e.g., the targeting domain is 24 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 25 nucleotides (e.g., 25 consecutive nucleotides) having complementarity with the target domain, e.g., the targeting domain is 25 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 25 nucleotides (e.g., 25 consecutive nucleotides) having complementarity with the target domain, e.g., the targeting domain is 25 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 25 nucleotides (e.g., 25 consecutive nucleotides) having complementarity with the target domain, e.g., the targeting domain is 25 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

In an embodiment, the targeting domain comprises, has, or consists of, 26 nucleotides (e.g., 26 consecutive nucleotides) having complementarity with the target domain, e.g., the targeting domain is 26 nucleotides in length; and the proximal and tail domain, when taken together, comprise at least 15, 18, 20, 25, 30, 31, 35, 40, 45, 49, 50, or 53 nucleotides.

In an embodiment, the targeting domain comprises, has, or consists of, 26 nucleotides (e.g., 26 consecutive nucleotides) having complementarity with the target domain, e.g., the targeting domain is 26 nucleotides in length; and there are at least 15, 18, 20, 25, 30, 31, 35, 40, 45, 49, 50, or 53 nucleotides 3' to the last nucleotide of the second complementarity domain.

In an embodiment, the targeting domain comprises, has, or consists of, 26 nucleotides (e.g., 26 consecutive nucleotides) having complementarity with the target domain, e.g., the targeting domain is 26 nucleotides in length; and there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain.

### II. Methods for Designing gRNAs

Methods for designing gRNAs are described herein, including methods for selecting, designing and validating target domains. Exemplary targeting domains are also provided herein. Targeting Domains discussed herein can be incorporated into the gRNAs described herein.

Methods for selection and validation of target sequences as well as off-target analyses are described, e.g., in Mali et al., 2013 SCIENCE 339(6121): 823-826; Hsu et al. NAT BIOTECHNOL, 31(9): 827-32; Fu et al., 2014 NAT BIOTECHNOL, doi: 10.1038/nbt.2808. PubMed PMID: 24463574; Heigwer et al., 2014 NAT METHODS 11(2):122-3. doi: 10.1038/nmeth.2812. PubMed PMID: 24481216; Bae et al., 2014 BIOINFORMATICS PubMed PMID: 24463181; Xiao A et al., 2014 BIOINFORMATICS PubMed PMID: 24389662.

For example, a software tool can be used to optimize the choice of gRNA within a user's target sequence, e.g., to minimize total off-target activity across the genome. Off target activity may be other than cleavage. For each possible gRNA choice using *S. pyogenes* Cas9, the tool can identify all off-target sequences (preceding either NAG or NGG PAMs) across the genome that contain up to certain number (e.g., 1, 2, 3, 4, 5, 6, 7, 8, 9, or 10) of mismatched base-pairs. The cleavage efficiency at each off-target sequence can be predicted, e.g., using an experimentally-derived weighting scheme. Each possible gRNA is then ranked according to its total predicted off-target cleavage; the top-ranked gRNAs represent those that are likely to have the greatest on-target and the least off-target cleavage. Other functions, e.g., automated reagent design for CRISPR construction, primer design for the on-target Surveyor assay, and primer design for high-throughput detection and quantification of off-target cleavage via next-gen sequencing, can also be included in the tool. Candidate gRNA molecules can be evaluated by art-known methods or as described in Section IV herein.

The Targeting Domains discussed herein can be incorporated into the gRNAs described herein.

### Strategies to identify gRNAs for S. pyogenes, S. Aureus, and N. meningitidis to knock out the MYOC gene

As an example, three strategies were utilized to identify gRNAs for use with *S*. *pyogenes, S. aureus and N. meningitidis* Cas9 enzymes.

In the first strategy, guide RNAs (gRNAs) for use with the *S. pyogenes* Cas9 (**Tables 4A-4C**) were identified using the publically available web-based ZiFiT server (Fu et al., Improving CRISPR-Cas nuclease specificity using truncated guide RNAs. Nat Biotechnol. 2014 Jan 26. doi: 10.1038/nbt.2808. PubMed PMID: 24463574, for the original references see Sander et al., 2007, NAR 35:W599-605; Sander et al., 2010, NAR 38: W462-8). In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available Repeat-Masker program. RepeatMmasker searches input DNA sequences for repeated elements and regions of low complexity. The output is a detailed annotation of the repeats present in a given query sequence. Following identification, gRNAs for use with a *S*. *pyogenes* Cas9 were ranked into 3 or 4 tiers, as described below.

The gRNAs in tier 1 were selected based on their distance to the target site and their orthogonality in the genome (based on the ZiFiT identification of close matches in the human genome containing an NGG PAM). As an example, for all targets, both 17-mer and 20-mer gRNAs were designed. gRNAs were also selected both for single-gRNA nuclease cutting and for the dual gRNA nickase strategy. Criteria for selecting gRNAs and the determination for which gRNAs can be used for which strategy is based on several considerations:
1. For the dual nickase strategy, gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, it will also often result in indel mutations at the site of only one of the gRNAs. Candidate pair members can be tested for how efficiently they remove the entire sequence versus just causing indel mutations at the site of one gRNA.

In order to find a pair for the dual-nickase strategy it was necessary to either extend the distance from the mutation or remove the requirement for the 5'G. For selection of tier 2 gRNAs, the distance restriction was relaxed in some cases such that a longer sequence was scanned, but the 5'G was required for all gRNAs. Whether or not the distance requirement was relaxed depended on how many sites were found within the original search window. Tier 3 uses the same distance restriction as tier 2, but removes the requirement for a 5'G. Note that tiers are non-inclusive (each gRNA is listed only once).

As discussed above, gRNAs were identified for single-gRNA nuclease cleavage as well as for a dual-gRNA paired "nickase" strategy, as indicated.

gRNAs for use with the *N. meningitidis* (**Tables 4E**) and *S. aureus* (**Tables 4D**) Cas9s were identified manually by scanning genomic DNA sequence for the presence of PAM sequences. These gRNAs were not separated into tiers, but are provided in single lists for each species.

In a second strategy, Guide RNAs (gRNAs) for use with *S. pyogenes, S. aureus and N. meningitidis* Cas9s were identified using a DNA sequence searching algorithm. Guide RNA design was carried out using a custom guide RNA design software based on the public tool cas-offinder (reference:Cas-OFFinder: a fast and versatile algorithm that searches for potential off-target sites of Cas9 RNA-guided endonucleases., Bioinformatics. 2014 Feb 17. Bae S, Park J, Kim JS. PMID:24463181). Said custom guide RNA design software scores guides after calculating their genomewide off-target propensity. Typically matches ranging from perfect matches to 7 mismatches are considered for guides ranging in length from 17 to 24. Once the off-target sites are computationally determined , an aggregate score is calculated for each guide and summarized in a tabular output using a web-interface. In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available RepeatMasker program. RepeatMasker searches input DNA sequences for repeated elements and regions of low complexity. The output is a detailed annotation of the repeats present in a given query sequence.

Following identification, gRNAs were ranked into tiers based on their distance to the target site, their orthogonality and presence of a 5' G (based on identification of close matches in the human genome containing a relavant PAM (e.g., in the case of *S. pyogenes,* a NGG PAM, in the case of *S. aureus,* a NNGRRT or NNGRRV PAM, and in the case of *N. meningitidis,* a NNNNGATT or NNNNGCTT PAM). Orthogonality refers to the number of sequences in the human genome that contain a minimum number of mismatches to the target sequence. A "high level of orthogonality" or "good orthogonality" may, for example, refer to 20-mer gRNAs that have no identical sequences in the human genome besides the intended target, nor any sequences that contain one or two mismatches in the target sequence. Targeting domains with good orthogonality are selected to minimize off-target DNA cleavage.

As an example, for *S. pyogenes* and *N. meningitidis* targets, 17-mer, or 20-mer gRNAs were designed. As another example, for *S. aureus* targets, 18-mer, 19-mer, 20-mer, 21-mer, 22-mer, 23-mer and 24-mer gRNAs were designed. Tarteting domains, disclosed herein, may comprise the 17-mer described in **Tables 6A-6E, 7A-7G or 8A-8E,** e.g., the targeting domains of 18 or more nucleotides may comprise the 17-mer gRNAs described in **Tables 6A-6E, 7A-7G or 8A-8E.** Tarteting domains, disclosed herein, may comprises the 18-mer described in **Tables 6A-6E, 7A-7G or 8A-8E,** e.g., the targeting domains of 19 or more nucleotides may comprise the 18-mer gRNAs described in **Tables 6A-6E, 7A-7G or 8A-8E.** Tarteting domains, disclosed herein, may comprises the 19-mer described in **Tables 6A-6E, 7A-7G or 8A-8E,** e.g., the targeting domains of 20 or more nucleotides may comprise the 19-mer gRNAs described in **Tables 6A-6E, 7A-7G or 8A-8E.** Tarteting domains, disclosed herein, may comprises the 20-mer gRNAs described in **Tables 6A-6E, 7A-7G or 8A-8E,** e.g., the targeting domains of 21 or more nucleotides may comprise the 20-mer gRNAs described in **Tables 6A-6E, 7A-7G or 8A-8E.** Tarteting domains, disclosed herein, may comprises the 21-mer described in **Tables 6A-6E, 7A-7G or 8A-8E,** e.g., the targeting domains of 22 or more nucleotides may comprise the 21-mer gRNAs described in **Tables 6A-6E, 7A-7G or 8A-8E.** Tarteting domains, disclosed herein, may comprises the 22-mer described in **Tables 6A-6E, 7A-7G or 8A-8E,** e.g., the targeting domains of 23 or more nucleotides may comprise the 22-mer gRNAs described in **Tables 6A-6E, 7A-7G or 8A-8E.** Tarteting domains, disclosed herein, may comprises the 23-mer described in **Tables 6A-6E, 7A-7G or 8A-8E,** e.g., the targeting domains of 24 or more nucleotides may comprise the 23-mer gRNAs described in **Tables 6A-6E, 7A-7G or 8A-8E.** Tarteting domains, disclosed herein, may comprises the 24-mer described in **Tables 6A-6E, 7A-7G or 8A-8E,** e.g., the targeting domains of 25 or more nucleotides may comprise the 24-mer gRNAs described in **Tables 6A-6E, 7A-7G or 8A-8E**. gRNAs were identified for both single-gRNA nuclease cleavage and for a dual-gRNA paired "nickase" strategy. Criteria for selecting gRNAs and the determination for which gRNAs can be used for the dual-gRNA paired "nickase" strategy is based on two considerations:
1. gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, cleaving with dual nickase pairs can also result in indel mutations at the site of only one of the gRNAs. Candidate pair members can be tested for how efficiently they remove the entire sequence versus causing indel mutations at the site of one gRNA.

The targeting domains discussed herein can be incorporated into the gRNAs described herein.

gRNAs were identified and ranked into 5 tiers for *S. pyogenes* (**Tables 6A-6E**), and *N. meningitidis* (**Tables 8A-8E**); and 7 tiers for *S. aureus* (**Tables 7A-7G**). For *S. pyogenes,* and *N. meningitidis,* the targeting domain for tier 1 gRNA molecules were selected based on (1) distance to a target site (e.g., start codon), e.g., within 500bp (e.g., downstream) of the target site (e.g., start codon), (2) a high level of orthogonality and (3) the presence of 5'G. The targeting domain for tier 2 gRNA molecules were selected based on (1) distance to a target site (e.g., start codon), e.g., within 500bp (e.g., downstream) of the target site (e.g., start codon) and (2) a high level of orthogonality. The targeting domain for tier 3 gRNA molecules were selected based on (1) distance to a target site (e.g., start codon), e.g., within 500bp (e.g., downstream) of the target site (e.g., start codon) and (2) the presence of 5'G. The targeting domain for tier 4 gRNA molecules were selected based on distance to a target site (e.g., start codon), e.g., within 500bp (e.g., downstream) of the target site (e.g., start codon). The targeting domain for tier 5 gRNA molecules were selected based on distance to the target site (e.g., start codon), e.g., within reminder of the coding sequence, e.g., downstream of the first 500bp of coding sequence (e.g., anywhere from +500 (relative to the start codon) to the stop codon). For *S. aureus,* the targeting domain for tier 1 gRNA molecules were selected based on (1) distance to a target site (e.g., start codon), e.g., within 500bp (e.g., downstream) of the target site (e.g., start codon), (2) a high level of orthogonality, (3) the presence of 5'G and (4) PAM is NNGRRT. The targeting domain for tier 2 gRNA molecules were selected based on (1) distance to a target site (e.g., start codon), e.g., within 500bp (e.g., downstream) of the target site (e.g., start codon), (2) a high level of orthogonality, and (3) PAM is NNGRRT. The targeting domain for tier 3 gRNA molecules were selected based on (1) distance to a target site (e.g., start codon), e.g., within 500bp (e.g., downstream) of the target site (e.g., start codon) and (2) PAM is NNGRRT. The targeting domain for tier 4 gRNA molecules were selected based on (1) distance to a target site (e.g., start codon), e.g., within 500bp (e.g., downstream) of the target site (e.g., start codon) and (2) PAM is NNGRRV. The targeting domain for tier 5 gRNA molecules were selected based on (1) distance to the target site (e.g., start codon), e.g., within reminder of the coding sequence, e.g., downstream of the first 500bp of coding sequence (e.g., anywhere from +500 (relative to the start codon) to the stop codon), (2) the presence of 5'G and (3) PAM is NNGRRT. The targeting domain for tier 6 gRNA molecules were selected based on (1) distance to the target site (e.g., start codon), e.g., within reminder of the coding sequence, e.g., downstream of the first 500bp of coding sequence (e.g., anywhere from +500 (relative to the start codon) to the stop codon) and (2) PAM is NNGRRT. The targeting domain for tier 7 gRNA molecules were selected based on (1) distance to the target site (e.g., start codon), e.g., within reminder of the coding sequence, e.g., downstream of the first 500bp of coding sequence (e.g., anywhere from +500 (relative to the start codon) to the stop codon) and (2) PAM is NNGRRV. Note that tiers are non-inclusive (each gRNA is listed only once for the strategy). In certain instances, no gRNA was identified based on the criteria of the particular tier.

### Strategies to identify gRNAs for S. pyogenes, S. Aureus, and N. meningitidis to knock down the MYOC gene

As an example, three strategies were utilized to identify gRNAs for use with *S*. *pyogenes, S. aureus and N. meningitidis* Cas9 enzymes.

In the first strategy, guide RNAs (gRNAs) for use with the *S. pyogenes* Cas9 (**Tables 5A-5D**) were identified using the publically available web-based ZiFiT server (Fu et al., Improving CRISPR-Cas nuclease specificity using truncated guide RNAs. Nat Biotechnol. 2014 Jan 26. doi: 10.1038/nbt.2808. PubMed PMID: 24463574, for the original references see Sander et al., 2007, NAR 35:W599-605; Sander et al., 2010, NAR 38: W462-8). In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available Repeat-Masker program. RepeatMmasker searches input DNA sequences for repeated elements and regions of low complexity. The output is a detailed annotation of the repeats present in a given query sequence. Following identification, gRNAs for use with a *S*. *pyogenes* Cas9 were ranked into 3 or 4 tiers, as described below.

The gRNAs in tier 1 were selected based on their distance to the target site and their orthogonality in the genome (based on the ZiFiT identification of close matches in the human genome containing an NGG PAM). As an example, for all targets, both 17-mer and 20-mer gRNAs were designed. For selection of tier 2 gRNAs, the distance restriction was relaxed in some cases such that a longer sequence was scanned, but the 5'G was required for all gRNAs. Whether or not the distance requirement was relaxed depended on how many sites were found within the original search window. Tier 3 uses the same distance restriction as tier 2, but removes the requirement for a 5'G. Note that tiers are non-inclusive (each gRNA is listed only once).

gRNAs for use with the *N. meningitidis* (**Tables 5E**) and *S. aureus* (**Tables 5D**) Cas9s were identified manually by scanning genomic DNA sequence for the presence of PAM sequences. These gRNAs were not separated into tiers, but are provided in single lists for each species.

In a second strategy, Guide RNAs (gRNAs) for use with *S. pyogenes, S. aureus and N. meningitidis* Cas9s were identified using a DNA sequence searching algorithm. Guide RNA design was carried out using a custom guide RNA design software based on the public tool cas-offinder (reference:Cas-OFFinder: a fast and versatile algorithm that searches for potential off-target sites of Cas9 RNA-guided endonucleases., Bioinformatics. 2014 Feb 17. Bae S, Park J, Kim JS. PMID:24463181). Said custom guide RNA design software scores guides after calculating their genomewide off-target propensity. Typically matches ranging from perfect matches to 7 mismatches are considered for guides ranging in length from 17 to 24. Once the off-target sites are computationally determined , an aggregate score is calculated for each guide and summarized in a tabular output using a web-interface. In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available RepeatMasker program. RepeatMasker searches input DNA sequences for repeated elements and regions of low complexity. The output is a detailed annotation of the repeats present in a given query sequence.

Following identification, gRNAs were ranked into tiers based on their distance to the target site, their orthogonality and presence of a 5' G (based on identification of close matches in the human genome containing a relavant PAM (e.g., in the case of *S. pyogenes,* a NGG PAM, in the case of *S. aureus,* a NNGRRT or NNGRRV PAM, and in the case of *N. meningitidis,* a NNNNGATT or NNNNGCTT PAM). Orthogonality refers to the number of sequences in the human genome that contain a minimum number of mismatches to the target sequence. A "high level of orthogonality" or "good orthogonality" may, for example, refer to 20-mer gRNAs that have no identical sequences in the human genome besides the intended target, nor any sequences that contain one or two mismatches in the target sequence. Targeting domains with good orthogonality are selected to minimize off-target DNA cleavage.

As an example, for *S. pyogenes* and *N. meningitidis* targets, 17-mer, or 20-mer gRNAs were designed. As another example, for *S. aureus* targets, 18-mer, 19-mer, 20-mer, 21-mer, 22-mer, 23-mer and 24-mer gRNAs were designed. Tarteting domains, disclosed herein, may comprise the 17-mer described in **Tables 9A-9E, 10A-10G or 11A-11E,** e.g., the targeting domains of 18 or more nucleotides may comprise the 17-mer gRNAs described in **Tables 9A-9E, 10A-10G or 11A-11E.** Tarteting domains, disclosed herein, may comprises the 18-mer described in **Tables 9A-9E, 10A-10G or 11A-11E,** e.g., the targeting domains of 19 or more nucleotides may comprise the 18-mer gRNAs described in **Tables 9A-9E, 10A-10G or 11A-11E.** Tarteting domains, disclosed herein, may comprises the 19-mer described in **Tables 9A-9E, 10A-10G or 11A-11E,** e.g., the targeting domains of 20 or more nucleotides may comprise the 19-mer gRNAs described in **Tables 9A-9E, 10A-10G or 11A-11E.** Tarteting domains, disclosed herein, may comprises the 20-mer gRNAs described in **Tables 9A-9E, 10A-10G or 11A-11E,** e.g., the targeting domains of 21 or more nucleotides may comprise the 20-mer gRNAs described in **Tables 9A-9E, 10A-10G or 11A-11E.** Tarteting domains, disclosed herein, may comprises the 21-mer described in **Tables 9A-9E, 10A-10G or 11A-11E,** e.g., the targeting domains of 22 or more nucleotides may comprise the 21-mer gRNAs described in **Tables 9A-9E, 10A-10G or 11A-11E.** Tarteting domains, disclosed herein, may comprises the 22-mer described in **Tables 9A-9E, 10A-10G or 11A-11E,** e.g., the targeting domains of 23 or more nucleotides may comprise the 22-mer gRNAs described in **Tables 9A-9E, 10A-10G or 11A-11E.** Tarteting domains, disclosed herein, may comprises the 23-mer described in **Tables 9A-9E, 10A-10G or 11A-11E,**e.g., the targeting domains of 24 or more nucleotides may comprise the 23-mer gRNAs described in **Tables 9A-9E, 10A-10G or 11A-11E.** Tarteting domains, disclosed herein, may comprises the 24-mer described in **Tables 9A-9E, 10A-10G or 11A-11E,** e.g., the targeting domains of 25 or more nucleotides may comprise the 24-mer gRNAs described in **Tables 9A-9E, 10A-10G or 11A-11E.**

The targeting domains discussed herein can be incorporated into the gRNAs described herein.

gRNAs were identified and ranked into 5 tiers for *S. pyogenes* **(Tables 9A-9E),** and *N. meningitidis* (**Tables 11A-11E**); and 7 tiers for *S. aureus* **(Tables 10A-10G).** For *S*. *pyogenes,* and *N. meningitidis,* the targeting domain for tier 1 gRNA molecules were selected based on (1) distance to a target site, e.g., within 3454-2454bp upstream of transcription start site or 500bp upstream and downstream of transcription start site, (2) a high level of orthogonality and (3) the presence of 5'G. The targeting domain for tier 2 gRNA molecules were selected based on (1) distance to a target site, e.g., within 3454-2454bp upstream of transcription start site or 500bp upstream and downstream of transcription start site and (2) a high level of orthogonality. The targeting domain for tier 3 gRNA molecules were selected based on (1) distance to a target site, e.g., within 3454-2454bp upstream of transcription start site or 500bp upstream and downstream of transcription start site and (2) the presence of 5'G. The targeting domain for tier 4 gRNA molecules were selected based on distance to a target site, e.g., within 3454-2454bp upstream of transcription start site or 500bp upstream and downstream of transcription start site. The targeting domain for tier 5 gRNA molecules were selected based on distance to the target site, e.g., within 2484-903bp upstream of transcription start site or the additional 500bp upstream and downstream of transcription start site (extending to 1kb up and downstream of the transcription start site) . For *S. aureus,* the targeting domain for tier 1 gRNA molecules were selected based on (1) distance to a target site, e.g., within 3454-2454bp upstream of transcription start site or 500bp upstream and downstream of transcription start site, (2) a high level of orthogonality, (3) the presence of 5'G and (4) PAM is NNGRRT. The targeting domain for tier 2 gRNA molecules were selected based on (1) distance to a target site, e.g., within 3454-2454bp upstream of transcription start site or 500bp upstream and downstream of transcription start site, (2) a high level of orthogonality, and (3) PAM is NNGRRT. The targeting domain for tier 3 gRNA molecules were selected based on (1) distance to a target site, e.g., within 3454-2454bp upstream of transcription start site or 500bp upstream and downstream of transcription start site and (2) PAM is NNGRRT. The targeting domain for tier 4 gRNA molecules were selected based on (1) distance to a target site, e.g., within 3454-2454bp upstream of transcription start site or 500bp upstream and downstream of transcription start site and (2) PAM is NNGRRV. The targeting domain for tier 5 gRNA molecules were selected based on (1) distance to the target site, e.g., within 2484-903bp upstream of transcription start site or the additional 500bp upstream and downstream of transcription start site (extending to 1kb up and downstream of the transcription start site), (2) the presence of 5'G and (3) PAM is NNGRRT. The targeting domain for tier 6 gRNA molecules were selected based on (1) distance to the target site, e.g., within 2484-903bp upstream of transcription start site or the additional 500bp upstream and downstream of transcription start site (extending to 1kb up and downstream of the transcription start site) and (2) PAM is NNGRRT. The targeting domain for tier 7 gRNA molecules were selected based on (1) distance to the target site, e.g., within 2484-903bp upstream of transcription start site or the additional 500bp upstream and downstream of transcription start site (extending to 1kb up and downstream of the transcription start site) and (2) PAM is NNGRRV. Note that tiers are non-inclusive (each gRNA is listed only once for the strategy). In certain instances, no gRNA was identified based on the criteria of the particular tier.

### Strategies to identify gRNAs for S. pyogenes, S. Aureus, and N. for the mutational hotspot 477-502 target site in the MYOC gene

As an example, three strategies were utilized to identify gRNAs for use with *S*. *pyogenes, S. aureus and N. meningitidis* Cas9 enzymes.

In the first strategy, guide RNAs (gRNAs) for use with the *S. pyogenes* Cas9 **(Tables 3A-3C)** were identified using the publically available web-based ZiFiT server (Fu et al., Improving CRISPR-Cas nuclease specificity using truncated guide RNAs. Nat Biotechnol. 2014 Jan 26. doi: 10.1038/nbt.2808. PubMed PMID: 24463574, for the original references see Sander et al., 2007, NAR 35:W599-605; Sander et al., 2010, NAR 38: W462-8). In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available Repeat-Masker program. RepeatMmasker searches input DNA sequences for repeated elements and regions of low complexity. The output is a detailed annotation of the repeats present in a given query sequence. Following identification, gRNAs for use with a *S*. *pyogenes* Cas9 were ranked into 3 or 4 tiers, as described below.

The gRNAs in tier 1 were selected based on their distance to the target site and their orthogonality in the genome (based on the ZiFiT identification of close matches in the human genome containing an NGG PAM). As an example, for all targets, both 17-mer and 20-mer gRNAs were designed. gRNAs were also selected both for single-gRNA nuclease cutting and for the dual gRNA nickase strategy. Criteria for selecting gRNAs and the determination for which gRNAs can be used for which strategy is based on several considerations:
1. For the dual nickase strategy, gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, it will also often result in indel mutations at the site of only one of the gRNAs. Candidate pair members can be tested for how efficiently they remove the entire sequence versus just causing indel mutations at the site of one gRNA.

While it can be desirable to have gRNAs start with a 5' G, this requirement was relaxed for some gRNAs in tier 1 in order to identify guides in the correct orientation, within a reasonable distance to the mutation and with a high level of orthogonality. In order to find a pair for the dual-nickase strategy it was necessary to either extend the distance from the mutation or remove the requirement for the 5'G. For selection of tier 2 gRNAs, the distance restriction was relaxed in some cases such that a longer sequence was scanned, but the 5'G was required for all gRNAs. Whether or not the distance requirement was relaxed depended on how many sites were found within the original search window. Tier 3 uses the same distance restriction as tier 2, but removes the requirement for a 5'G. Note that tiers are non-inclusive (each gRNA is listed only once).

As discussed above, gRNAs were identified for single-gRNA nuclease cleavage as well as for a dual-gRNA paired "nickase" strategy, as indicated.

gRNAs for use with the *N. meningitidis* (**Tables 3E**) and *S. aureus* (**Tables 3D**) Cas9s were identified manually by scanning genomic DNA sequence for the presence of PAM sequences. These gRNAs were not separated into tiers, but are provided in single lists for each species.

In a second strategy, Guide RNAs (gRNAs) for use with *S. pyogenes, S. aureus and N. meningitidis* Cas9s were identified using a DNA sequence searching algorithm. Guide RNA design was carried out using a custom guide RNA design software based on the public tool cas-offinder (reference:Cas-OFFinder: a fast and versatile algorithm that searches for potential off-target sites of Cas9 RNA-guided endonucleases., Bioinformatics. 2014 Feb 17. Bae S, Park J, Kim JS. PMID:24463181). Said custom guide RNA design software scores guides after calculating their genomewide off-target propensity. Typically matches ranging from perfect matches to 7 mismatches are considered for guides ranging in length from 17 to 24. Once the off-target sites are computationally determined , an aggregate score is calculated for each guide and summarized in a tabular output using a web-interface. In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available RepeatMasker program. RepeatMasker searches input DNA sequences for repeated elements and regions of low complexity. The output is a detailed annotation of the repeats present in a given query sequence.

Following identification, gRNAs were ranked into tiers based on their distance to the target site, their orthogonality and presence of a 5' G (based on identification of close matches in the human genome containing a relavant PAM (e.g., in the case of *S. pyogenes,* a NGG PAM, in the case of *S. aureus,* a NNGRRT or NNGRRV PAM, and in the case of *N. meningitidis,* a NNNNGATT or NNNNGCTT PAM). Orthogonality refers to the number of sequences in the human genome that contain a minimum number of mismatches to the target sequence. A "high level of orthogonality" or "good orthogonality" may, for example, refer to 20-mer gRNAs that have no identical sequences in the human genome besides the intended target, nor any sequences that contain one or two mismatches in the target sequence. Targeting domains with good orthogonality are selected to minimize off-target DNA cleavage.

As an example, for *S. pyogenes* and *N. meningitidis* targets, 17-mer, or 20-mer gRNAs were designed. As another example, for *S. aureus* targets, 18-mer, 19-mer, 20-mer, 21-mer, 22-mer, 23-mer and 24-mer gRNAs were designed. Targeting domains, disclosed herein, may comprise the 17-mer described in **Tables 12A-12D, 13A-13E, 14A-14C, 15A-15D, 16A-16E, or 17A-17B,** e.g., the targeting domains of 18 or more nucleotides may comprise the 17-mer gRNAs described in **Tables 12A-12D, 13A-13E, 14A-14C, 15A-15D, 16A-16E, or 17A-17B.** Targeting domains, disclosed herein, may comprises the 18-mer described in **Tables 12A-12D, 13A-13E, 14A-14C, 15A-15D, 16A-16E, or 17A-17B,** e.g., the targeting domains of 19 or more nucleotides may comprise the 18-mer gRNAs described in **Tables 12A-12D, 13A-13E, 14A-14C, 15A-15D, 16A-16E, or 17A-17B.** Targeting domains, disclosed herein, may comprises the 19-mer described in **Tables 12A-12D, 13A-13E, 14A-14C, 15A-15D, 16A-16E, or 17A-17B,** e.g., the targeting domains of 20 or more nucleotides may comprise the 19-mer gRNAs described in **Tables 12A-12D, 13A-13E, 14A-14C, 15A-15D, 16A-16E, or 17A-17B.** Targeting domains, disclosed herein, may comprises the 20-mer gRNAs described in **Tables 12A-12D, 13A-13E, 14A-14C, 15A-15D, 16A-16E, or 17A-17B,** e.g., the targeting domains of 21 or more nucleotides may comprise the 20-mer gRNAs described in **Tables 12A-12D, 13A-13E, 14A-14C, 15A-15D, 16A-16E, or 17A-17B.** Targeting domains, disclosed herein, may comprises the 21-mer described in **Tables 12A-12D, 13A-13E, 14A-14C, 15A-15D, 16A-16E, or 17A-17B,** e.g., the targeting domains of 22 or more nucleotides may comprise the 21-mer gRNAs described in **Tables 12A-12D, 13A-13E, 14A-14C, 15A-15D, 16A-16E, or 17A-17B.** Targeting domains, disclosed herein, may comprises the 22-mer described in **Tables 12A-12D, 13A-13E, 14A-14C, 15A-15D, 16A-16E, or 17A-17B,** e.g., the targeting domains of 23 or more nucleotides may comprise the 22-mer gRNAs described in **Tables 12A-12D, 13A-13E, 14A-14C, 15A-15D, 16A-16E, or 17A-17B.** Targeting domains, disclosed herein, may comprises the 23-mer described in **Tables 12A-12D, 13A-13E, 14A-14C, 15A-15D, 16A-16E, or 17A-17B,** e.g., the targeting domains of 24 or more nucleotides may comprise the 23-mer gRNAs described in **Tables 12A-12D, 13A-13E, 14A-14C, 15A-15D, 16A-16E, or 17A-17B.** Targeting domains, disclosed herein, may comprises the 24-mer described in **Tables 12A-12D, 13A-13E, 14A-14C, 15A-15D, 16A-16E, or 17A-17B,** e.g., the targeting domains of 25 or more nucleotides may comprise the 24-mer gRNAs described in **Tables 12A-12D, 13A-13E, 14A-14C, 15A-15D, 16A-16E, or 17A-17B.**

gRNAs were identified for both single-gRNA nuclease cleavage and for a dual-gRNA paired "nickase" strategy. Criteria for selecting gRNAs and the determination for which gRNAs can be used for the dual-gRNA paired "nickase" strategy is based on two considerations:
1. gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, cleaving with dual nickase pairs can also result in indel mutations at the site of only one of the gRNAs. Candidate pair members can be tested for how efficiently they remove the entire sequence versus causing indel mutations at the site of one gRNA.
The targeting domains discussed herein can be incorporated into the gRNAs described herein.

In an embodiment, gRNAs were identified and ranked into 4 tiers for *S. pyogenes* (**Tables 12A-12D**), and *N. meningitidis* (**Tables 14A-14C**); and 5 tiers for *S. aureus* **(Tables 13A-13E**)**.** For *S. pyogenes,* and *N. meningitidis,* the targeting domain for tier 1 gRNA molecules were selected based on (1) distance to a POAG target position, e.g., within 200 bp upstream from the mutational hotspot 477-502 target site, (2) a high level of orthogonality and (3) the presence of 5'G. The targeting domain for tier 2 gRNA molecules were selected based on (1) distance to a POAG target position, e.g., within 200 bp upstream from the mutational hotspot 477-502 target site and (2) a high level of orthogonality. The targeting domain for tier 3 gRNA molecules were selected based on (1) distance to a POAG target position, e.g., within 200 bp upstream from the mutational hotspot 477-502 target site and (2) the presence of 5'G. The targeting domain for tier 4 gRNA molecules were selected based on distance to a POAG target position, e.g., within 200 bp upstream from the mutational hotspot 477-502 target site. For *S. aureus,* the targeting domain for tier 1 gRNA molecules were selected based on (1) distance to a POAG target position, e.g., within 200 bp upstream from the mutational hotspot 477-502 target site, (2) a high level of orthogonality, (3) the presence of 5'G and (4) PAM is NNGRRT. The targeting domain for tier 2 gRNA molecules were selected based on (1) distance to a POAG target position, e.g., within 200 bp upstream from the mutational hotspot 477-502 target site, (2) a high level of orthogonality, and (3) PAM is NNGRRT. The targeting domain for tier 3 gRNA molecules were selected based on (1) distance to a POAG target position, e.g., within 200 bp upstream from the mutational hotspot 477-502 target site, (2) the presence of a 5'G and (2) PAM is NNGRRT. The targeting domain for tier 4 gRNA molecules were selected based on (1) distance to a POAG target position, e.g., within 200 bp upstream from the mutational hotspot 477-502 target site and (2) PAM is NNGRRT. The targeting domain for tier 5 gRNA molecules were selected based on (1) (1) distance to a POAG target position, e.g., within 200 bp upstream from the mutational hotspot 477-502 target site and (2) PAM is NNGRRV. Note that tiers are non-inclusive (each gRNA is listed only once for the strategy). In certain instances, no gRNA was identified based on the criteria of the particular tier.

In another embodiment, gRNAs were identified and ranked into 4 tiers for *S*. *pyogenes* (**Tables 15A-15D**), and *N. meningitidis* (**Tables 17A-17B**); and 5 tiers for *S. aureus* (**Tables 16A-16E**). For *S. pyogenes,* and *N. meningitidis,* the targeting domain for tier 1 gRNA molecules were selected based on (1) distance to a POAG target position, e.g., within 200 bp downstream from the mutational hotspot 477-502 target site, (2) a high level of orthogonality and (3) the presence of 5'G. The targeting domain for tier 2 gRNA molecules were selected based on (1) distance to a POAG target position, e.g., within 200 bp downstream from the mutational hotspot 477-502 target site and (2) a high level of orthogonality. The targeting domain for tier 3 gRNA molecules were selected based on (1) distance to a POAG target position, e.g., within 200 bp downstream from the mutational hotspot 477-502 target site and (2) the presence of 5'G. The targeting domain for tier 4 gRNA molecules were selected based on distance to a POAG target position, e.g., within 200 bp downstream from the mutational hotspot 477-502 target site. For *S. aureus,* the targeting domain for tier 1 gRNA molecules were selected based on (1) distance to a POAG target position, e.g., within 200 bp downstream from the mutational hotspot 477-502 target site, (2) a high level of orthogonality, (3) the presence of 5'G and (4) PAM is NNGRRT. The targeting domain for tier 2 gRNA molecules were selected based on (1) distance to a POAG target position, e.g., within 200 bp downstream from the mutational hotspot 477-502 target site, (2) a high level of orthogonality, and (3) PAM is NNGRRT. The targeting domain for tier 3 gRNA molecules were selected based on (1) distance to a POAG target position, e.g., within 200 bp downstream from the mutational hotspot 477-502 target site, (2) the presence of a 5'G and (2) PAM is NNGRRT. The targeting domain for tier 4 gRNA molecules were selected based on (1) distance to a POAG target position, e.g., within 200 bp downstream from the mutational hotspot 477-502 target site and (2) PAM is NNGRRT. The targeting domain for tier 5 gRNA molecules were selected based on (1) (1) distance to a POAG target position, e.g., within 200 bp downstream from the mutational hotspot 477-502 target site and (2) PAM is NNGRRV. Note that tiers are non-inclusive (each gRNA is listed only once for the strategy). In certain instances, no gRNA was identified based on the criteria of the particular tier.

### Strategies to identify gRNAs for S. pyogenes, S. Aureus, and N. for correcting a mutation (e.g., I477N) in the MYOC gene

As an example, three strategies were utilized to identify gRNAs for use with *S*. *pyogenes, S. aureus and N. meningitidis* Cas9 enzymes.

In the first strategy, guide RNAs (gRNAs) for use with the *S. pyogenes* Cas9 (**Tables 2A-2C**) were identified using the publically available web-based ZiFiT server (Fu et al., Improving CRISPR-Cas nuclease specificity using truncated guide RNAs. Nat Biotechnol. 2014 Jan 26. doi: 10.1038/nbt.2808. PubMed PMID: 24463574, for the original references see Sander et al., 2007, NAR 35:W599-605; Sander et al., 2010, NAR 38: W462-8). In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available Repeat-Masker program. RepeatMmasker searches input DNA sequences for repeated elements and regions of low complexity. The output is a detailed annotation of the repeats present in a given query sequence. Following identification, gRNAs for use with a *S*. *pyogenes* Cas9 were ranked into 3 or 4 tiers, as described below.

The gRNAs in tier 1 were selected based on their distance to the target site and their orthogonality in the genome (based on the ZiFiT identification of close matches in the human genome containing an NGG PAM). As an example, for all targets, both 17-mer and 20-mer gRNAs were designed. gRNAs were also selected both for single-gRNA nuclease cutting and for the dual gRNA nickase strategy. Criteria for selecting gRNAs and the determination for which gRNAs can be used for which strategy is based on several considerations:
1. For the dual nickase strategy, gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, it will also often result in indel mutations at the site of only one of the gRNAs. Candidate pair members can be tested for how efficiently they remove the entire sequence versus just causing indel mutations at the site of one gRNA.

While it can be desirable to have gRNAs start with a 5' G, this requirement was relaxed for some gRNAs in tier 1 in order to identify guides in the correct orientation, within a reasonable distance to the mutation and with a high level of orthogonality. In order to find a pair for the dual-nickase strategy it was necessary to either extend the distance from the mutation or remove the requirement for the 5'G. For selection of tier 2 gRNAs, the distance restriction was relaxed in some cases such that a longer sequence was scanned, but the 5'G was required for all gRNAs. Whether or not the distance requirement was relaxed depended on how many sites were found within the original search window. Tier 3 uses the same distance restriction as tier 2, but removes the requirement for a 5'G. Note that tiers are non-inclusive (each gRNA is listed only once).

As discussed above, gRNAs were identified for single-gRNA nuclease cleavage as well as for a dual-gRNA paired "nickase" strategy, as indicated.

gRNAs for use with the *N. meningitidis* (**Tables 2E**) and *S. aureus* (**Tables 2D**) Cas9s were identified manually by scanning genomic DNA sequence for the presence of PAM sequences. These gRNAs were not separated into tiers, but are provided in single lists for each species.

In a second strategy, Guide RNAs (gRNAs) for use with *S. pyogenes, S. aureus and N. meningitidis* Cas9s were identified using a DNA sequence searching algorithm. Guide RNA design was carried out using a custom guide RNA design software based on the public tool cas-offinder (reference:Cas-OFFinder: a fast and versatile algorithm that searches for potential off-target sites of Cas9 RNA-guided endonucleases., Bioinformatics. 2014 Feb 17. Bae S, Park J, Kim JS. PMID:24463181). Said custom guide RNA design software scores guides after calculating their genomewide off-target propensity. Typically matches ranging from perfect matches to 7 mismatches are considered for guides ranging in length from 17 to 24. Once the off-target sites are computationally determined, an aggregate score is calculated for each guide and summarized in a tabular output using a web-interface. In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available RepeatMasker program. RepeatMasker searches input DNA sequences for repeated elements and regions of low complexity. The output is a detailed annotation of the repeats present in a given query sequence.

Following identification, gRNAs were ranked into tiers based on their distance to the target site, their orthogonality and presence of a 5' G (based on identification of close matches in the human genome containing a relavant PAM (e.g., in the case of *S. pyogenes,* a NGG PAM, in the case of *S. aureus,* a NNGRRT or NNGRRV PAM, and in the case of *N. meningitidis,* a NNNNGATT or NNNNGCTT PAM). Orthogonality refers to the number of sequences in the human genome that contain a minimum number of mismatches to the target sequence. A "high level of orthogonality" or "good orthogonality" may, for example, refer to 20-mer gRNAs that have no identical sequences in the human genome besides the intended target, nor any sequences that contain one or two mismatches in the target sequence. Targeting domains with good orthogonality are selected to minimize off-target DNA cleavage.

As an example, for *S. pyogenes* and *N. meningitidis* targets, 17-mer, or 20-mer gRNAs were designed. As another example, for *S. aureus* targets, 18-mer, 19-mer, 20-mer, 21-mer, 22-mer, 23-mer and 24-mer gRNAs were designed. Targeting domains, disclosed herein, may comprise the 17-mer described in **Tables 18A-18D, 19A-19E, or 20A-20D,** e.g., the targeting domains of 18 or more nucleotides may comprise the 17-mer gRNAs described in **Tables 18A-18D, 19A-19E, or 20A-20D.** Targeting domains, disclosed herein, may comprises the 18-mer described in **Tables 18A-18D, 19A-19E, or 20A-20D,** e.g., the targeting domains of 19 or more nucleotides may comprise the 18-mer gRNAs described in **Tables 18A-18D, 19A-19E, or 20A-20D.** Targeting domains, disclosed herein, may comprises the 19-mer described in **Tables 18A-18D, 19A-19E, or 20A-20D,** e.g., the targeting domains of 20 or more nucleotides may comprise the 19-mer gRNAs described in **Tables 18A-18D, 19A-19E, or 20A-20D.** Targeting domains, disclosed herein, may comprises the 20-mer gRNAs described in **Tables 18A-18D, 19A-19E, or 20A-20D,** e.g., the targeting domains of 21 or more nucleotides may comprise the 20-mer gRNAs described in **Tables 18A-18D, 19A-19E, or 20A-20D.** Targeting domains, disclosed herein, may comprises the 21-mer described in **Tables 18A-18D, 19A-19E, or 20A-20D,** e.g., the targeting domains of 22 or more nucleotides may comprise the 21-mer gRNAs described in **Tables 18A-18D, 19A-19E, or 20A-20D.** Targeting domains, disclosed herein, may comprises the 22-mer described in **Tables 18A-18D, 19A-19E, or 20A-20D,** e.g., the targeting domains of 23 or more nucleotides may comprise the 22-mer gRNAs described in **Tables 18A-18D, 19A-19E, or 20A-20D.** Targeting domains, disclosed herein, may comprises the 23-mer described in **Tables 18A-18D, 19A-19E, or 20A-20D**, e.g., the targeting domains of 24 or more nucleotides may comprise the 23-mer gRNAs described in **Tables 18A-18D, 19A-19E, or 20A-20D.** Targeting domains, disclosed herein, may comprises the 24-mer described in **Tables 18A-18D, 19A-19E, or 20A-20D,** e.g., the targeting domains of 25 or more nucleotides may comprise the 24-mer gRNAs described in **Tables 18A-18D, 19A-19E, or 20A-20D.**

gRNAs were identified for both single-gRNA nuclease cleavage and for a dual-gRNA paired "nickase" strategy. Criteria for selecting gRNAs and the determination for which gRNAs can be used for the dual-gRNA paired "nickase" strategy is based on two considerations:
1. gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, cleaving with dual nickase pairs can also result in indel mutations at the site of only one of the gRNAs. Candidate pair members can be tested for how efficiently they remove the entire sequence versus causing indel mutations at the site of one gRNA.

The targeting domains discussed herein can be incorporated into the gRNAs described herein.

In an embodiment, gRNAs were identified and ranked into 4 tiers for *S. pyogenes* (**Tables 18A-18D**), and *N. meningitidis* (**Tables 20A-20DC**); and 5 tiers for *S. aureus* (**Tables 19A-19D**). For *S. pyogenes,* and *N. meningitidis,* the targeting domain for tier 1 gRNA molecules were selected based on (1) distance to a target site, e.g., within 200 bp from a mutation (e.g., I477N), (2) a high level of orthogonality and (3) the presence of 5'G. The targeting domain for tier 2 gRNA molecules were selected based on (1) distance to a target site, e.g., within 200 bp from a mutation (e.g., I477N) and (2) a high level of orthogonality. The targeting domain for tier 3 gRNA molecules were selected based on (1) distance to a target site, e.g., within 200 bp from a mutation (e.g., I477N) and (2) the presence of 5'G. The targeting domain for tier 4 gRNA molecules were selected based on distance to a target site, e.g., within 200 bp from a mutation (e.g., I477N). For *S. aureus,* the targeting domain for tier 1 gRNA molecules were selected based on (1) distance to a target site, e.g., within 200 bp from a mutation (e.g., I477N), (2) a high level of orthogonality, (3) the presence of 5'G and (4) PAM is NNGRRT. The targeting domain for tier 2 gRNA molecules were selected based on (1) distance to a target site, e.g., within 200 bp from a mutation (e.g., I477N), (2) a high level of orthogonality, and (3) PAM is NNGRRT. The targeting domain for tier 3 gRNA molecules were selected based on (1) distance to a target site, e.g., within 200 bp from a mutation (e.g., I477N), (2) the presence of a 5'G and (2) PAM is NNGRRT. The targeting domain for tier 4 gRNA molecules were selected based on (1) distance to a target site, e.g., within 200 bp from a mutation (e.g., I477N) and (2) PAM is NNGRRT. The targeting domain for tier 5 gRNA molecules were selected based on (1) (1) distance to a target site, e.g., within 200 bp from a mutation (e.g., I477N) and (2) PAM is NNGRRV. Note that tiers are non-inclusive (each gRNA is listed only once for the strategy). In certain instances, no gRNA was identified based on the criteria of the particular tier.

### Strategies to identify gRNAs for S. pyogenes, S. Aureus, and N. for correcting a mutation (e.g., P370L) in the MYOC gene

As an example, three strategies were utilized to identify gRNAs for use with *S*. *pyogenes, S. aureus and N. meningitidis* Cas9 enzymes.

In the first strategy, guide RNAs (gRNAs) for use with the *S. pyogenes* Cas9 (**Tables 1A-1C**) were identified using the publically available web-based ZiFiT server (Fu et al., Improving CRISPR-Cas nuclease specificity using truncated guide RNAs. Nat Biotechnol. 2014 Jan 26. doi: 10.1038/nbt.2808. PubMed PMID: 24463574, for the original references see Sander et al., 2007, NAR 35:W599-605; Sander et al., 2010, NAR 38: W462-8). In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available Repeat-Masker program. RepeatMmasker searches input DNA sequences for repeated elements and regions of low complexity. The output is a detailed annotation of the repeats present in a given query sequence. Following identification, gRNAs for use with a *S*. *pyogenes* Cas9 were ranked into 3 or 4 tiers, as described below.

The gRNAs in tier 1 were selected based on their distance to the target site and their orthogonality in the genome (based on the ZiFiT identification of close matches in the human genome containing an NGG PAM). As an example, for all targets, both 17-mer and 20-mer gRNAs were designed. gRNAs were also selected both for single-gRNA nuclease cutting and for the dual gRNA nickase strategy. Criteria for selecting gRNAs and the determination for which gRNAs can be used for which strategy is based on several considerations:
1. For the dual nickase strategy, gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, it will also often result in indel mutations at the site of only one of the gRNAs. Candidate pair members can be tested for how efficiently they remove the entire sequence versus just causing indel mutations at the site of one gRNA.

While it can be desirable to have gRNAs start with a 5' G, this requirement was relaxed for some gRNAs in tier 1 in order to identify guides in the correct orientation, within a reasonable distance to the mutation and with a high level of orthogonality. In order to find a pair for the dual-nickase strategy it was necessary to either extend the distance from the mutation or remove the requirement for the 5'G. For selection of tier 2 gRNAs, the distance restriction was relaxed in some cases such that a longer sequence was scanned, but the 5'G was required for all gRNAs. Whether or not the distance requirement was relaxed depended on how many sites were found within the original search window. Tier 3 uses the same distance restriction as tier 2, but removes the requirement for a 5'G. Note that tiers are non-inclusive (each gRNA is listed only once).

As discussed above, gRNAs were identified for single-gRNA nuclease cleavage as well as for a dual-gRNA paired "nickase" strategy, as indicated.

gRNAs for use with the *N. meningitidis* (**Tables 1E**) and *S. aureus* (**Tables 1D**) Cas9s were identified manually by scanning genomic DNA sequence for the presence of PAM sequences. These gRNAs were not separated into tiers, but are provided in single lists for each species.

In a second strategy, Guide RNAs (gRNAs) for use with *S. pyogenes, S. aureus and N. meningitidis* Cas9s were identified using a DNA sequence searching algorithm. Guide RNA design was carried out using a custom guide RNA design software based on the public tool cas-offinder (reference:Cas-OFFinder: a fast and versatile algorithm that searches for potential off-target sites of Cas9 RNA-guided endonucleases., Bioinformatics. 2014 Feb 17. Bae S, Park J, Kim JS. PMID:24463181). Said custom guide RNA design software scores guides after calculating their genomewide off-target propensity. Typically matches ranging from perfect matches to 7 mismatches are considered for guides ranging in length from 17 to 24. Once the off-target sites are computationally determined, an aggregate score is calculated for each guide and summarized in a tabular output using a web-interface. In addition to identifying potential gRNA sites adjacent to PAM sequences, the software also identifies all PAM adjacent sequences that differ by 1, 2, 3 or more nucleotides from the selected gRNA sites. Genomic DNA sequence for each gene was obtained from the UCSC Genome browser and sequences were screened for repeat elements using the publically available RepeatMasker program. RepeatMasker searches input DNA sequences for repeated elements and regions of low complexity. The output is a detailed annotation of the repeats present in a given query sequence.

Following identification, gRNAs were ranked into tiers based on their distance to the target site, their orthogonality and presence of a 5' G (based on identification of close matches in the human genome containing a relavant PAM (e.g., in the case of *S. pyogenes,* a NGG PAM, in the case of *S. aureus,* a NNGRRT or NNGRRV PAM, and in the case of *N. meningitidis,* a NNNNGATT or NNNNGCTT PAM). Orthogonality refers to the number of sequences in the human genome that contain a minimum number of mismatches to the target sequence. A "high level of orthogonality" or "good orthogonality" may, for example, refer to 20-mer gRNAs that have no identical sequences in the human genome besides the intended target, nor any sequences that contain one or two mismatches in the target sequence. Targeting domains with good orthogonality are selected to minimize off-target DNA cleavage.

As an example, for *S. pyogenes* and *N. meningitidis* targets, 17-mer, or 20-mer gRNAs were designed. As another example, for *S. aureus* targets, 18-mer, 19-mer, 20-mer, 21-mer, 22-mer, 23-mer and 24-mer gRNAs were designed. Tarteting domains, disclosed herein, may comprise the 17-mer described in **Tables 21A-21D, 22A-22E, or 23A-23B,** e.g., the targeting domains of 18 or more nucleotides may comprise the 17-mer gRNAs described in **Tables 21A-21D, 22A-22E, or 23A-23B.** Tarteting domains, disclosed herein, may comprises the 18-mer described in **Tables 21A-21D, 22A-22E, or 23A-23B,** e.g., the targeting domains of 19 or more nucleotides may comprise the 18-mer gRNAs described in **Tables 21A-21D, 22A-22E, or 23A-23B.** Tarteting domains, disclosed herein, may comprises the 19-mer described in **Tables 21A-21D, 22A-22E, or 23A-23B,** e.g., the targeting domains of 20 or more nucleotides may comprise the 19-mer gRNAs described in **Tables 21A-21D, 22A-22E, or 23A-23B.** Tarteting domains, disclosed herein, may comprises the 20-mer gRNAs described in **Tables 21A-21D, 22A-22E, or 23A-23B,** e.g., the targeting domains of 21 or more nucleotides may comprise the 20-mer gRNAs described in **Tables 21A-21D, 22A-22E, or 23A-23B.** Tarteting domains, disclosed herein, may comprises the 21-mer described in **Tables 21A-21D, 22A-22E, or 23A-23B,** e.g., the targeting domains of 22 or more nucleotides may comprise the 21-mer gRNAs described in **Tables 21A-21D, 22A-22E, or 23A-23B.** Tarteting domains, disclosed herein, may comprises the 22-mer described in **Tables 21A-21D, 22A-22E, or 23A-23B,** e.g., the targeting domains of 23 or more nucleotides may comprise the 22-mer gRNAs described in **Tables 21A-21D, 22A-22E, or 23A-23B.** Tarteting domains, disclosed herein, may comprises the 23-mer described in **Tables 21A-21D, 22A-22E, or 23A-23B**, e.g., the targeting domains of 24 or more nucleotides may comprise the 23-mer gRNAs described in **Tables 21A-21D, 22A-22E, or 23A-23B.** Tarteting domains, disclosed herein, may comprises the 24-mer described in **Tables 21A-21D, 22A-22E, or 23A-23B,** e.g., the targeting domains of 25 or more nucleotides may comprise the 24-mer gRNAs described in **Tables 21A-21D, 22A-22E, or** 23A-23B.gRNAs were identified for both single-gRNA nuclease cleavage and for a dual-gRNA paired "nickase" strategy. Criteria for selecting gRNAs and the determination for which gRNAs can be used for the dual-gRNA paired "nickase" strategy is based on two considerations:
1. gRNA pairs should be oriented on the DNA such that PAMs are facing out and cutting with the D10A Cas9 nickase will result in 5' overhangs.
2. An assumption that cleaving with dual nickase pairs will result in deletion of the entire intervening sequence at a reasonable frequency. However, cleaving with dual nickase pairs can also result in indel mutations at the site of only one of the gRNAs. Candidate pair members can be tested for how efficiently they remove the entire sequence versus causing indel mutations at the site of one gRNA.

The targeting domains discussed herein can be incorporated into the gRNAs described herein.

In an embodiment, gRNAs were identified and ranked into 4 tiers for *S. pyogenes* **(Tables 21A-21D**), and *N. meningitidis* (**Tables 23A-23B**); and 5 tiers for *S. aureus* **(Tables 22A-22E**). For *S. pyogenes,* and *N. meningitidis,* the targeting domain for tier 1 gRNA molecules were selected based on (1) distance to a target site, e.g., within 200 bp from a mutation (e.g., P370L), (2) a high level of orthogonality and (3) the presence of 5'G. The targeting domain for tier 2 gRNA molecules were selected based on (1) distance to a target site, e.g., within 200 bp from a mutation (e.g., P370L) and (2) a high level of orthogonality. The targeting domain for tier 3 gRNA molecules were selected based on (1) distance to a target site, e.g., within 200 bp from a mutation (e.g., P370L) and (2) the presence of 5'G. The targeting domain for tier 4 gRNA molecules were selected based on distance to a target site, e.g., within 200 bp from a mutation (e.g., P370L). For *S. aureus,* the targeting domain for tier 1 gRNA molecules were selected based on (1) distance to a target site, e.g., within 200 bp from a mutation (e.g., P370L), (2) a high level of orthogonality, (3) the presence of 5'G and (4) PAM is NNGRRT. The targeting domain for tier 2 gRNA molecules were selected based on (1) distance to a target site, e.g., within 200 bp from a mutation (e.g., P370L), (2) a high level of orthogonality, and (3) PAM is NNGRRT. The targeting domain for tier 3 gRNA molecules were selected based on (1) distance to a target site, e.g., within 200 bp from a mutation (e.g., P370L), (2) the presence of a 5'G and (2) PAM is NNGRRT. The targeting domain for tier 4 gRNA molecules were selected based on (1) distance to a target site, e.g., within 200 bp from a mutation (e.g., P370L) and (2) PAM is NNGRRT. The targeting domain for tier 5 gRNA molecules were selected based on (1) (1) distance to a target site, e.g., within 200 bp from a mutation (e.g., P370L) and (2) PAM is NNGRRV. Note that tiers are non-inclusive (each gRNA is listed only once for the strategy). In certain instances, no gRNA was identified based on the criteria of the particular tier.

In an embodiment, two or more (e.g., three or four) gRNA molecules are used with one Cas9 molecule. In another embodiment, when two or more (e.g., three or four) gRNAs are used with two or more Cas9 molecules, at least one Cas9 molecule is from a different species than the other Cas9 molecule(s). For example, when two gRNA molecules are used with two Cas9 molecules, one Cas9 molecule can be from one species and the other Cas9 molecule can be from a different species. Both Cas9 species are used to generate a single or double-strand break, as desired.

Any of the targeting domains in the tables described herein can be used with a Cas9 nickase molecule to generate a single strand break.

Any of the targeting domains in the tables described herein can be used with a Cas9 nuclease molecule to generate a double strand break.

When two gRNAs designed for use to target two Cas9 molecules, one Cas9 can be one species, the second Cas9 can be from a different species. Both Cas9 species are used to generate a single or double-strand break, as desired.

It is contemplated herein that any upstream gRNA described herein may be paired with any downstream gRNA described herein. When an upstream gRNA designed for use with one species of Cas9 is paired with a downstream gRNA designed for use from a different species of Cas9, both Cas9 species are used to generate a single or double-strand break, as desired.

### Exemplary Targeting Domains

**Table 1A** provides exemplary targeting domains for the P370L target site selected according to the first tier parameters, and are selected based on the presence of a 5' G (except for MYOC-37, -46, -48, and -50), close proximity and orientation to mutation and orthogonality in the human genome. In an embodiment, the targeting domain is the exact complement of the target domain. Any of the targeting domains in the table can be used with a *S. pyogenes* Cas9 molecule that gives double stranded cleavage. Any of the targeting domains in the table can be used with *S. pyogenes* single-stranded break nucleases (nickases).

In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

In an embodiment, two 20-mer guide RNAs are used to target two *S. pyogenes* Cas9 nucleases or two *S. pyogenes* Cas9 nickases, e.g., MYOC-24 and MYOC-10, MYOC-20 and MYOC-16, or MYOC-24 and MYOC-16 are used. In an embodiment, two 17-mer RNAs are used to target two Cas9 nucleases or two Cas9 nickases, e.g., MYOC-50 and MYOC-32, MYOC-50 and MYOC-37, or MYOC-48 and MYOC-37 are used.

**Table 1A**

| **1st Tier** | selected based on the presence of a 5' G (except for #37, 46, 48, 50), close proximity and orientation to mutation and orthogonality in the human genome | | | |
|---|---|---|---|---|
| **gRNA Name** | **DNA Strand** | **Targeting Domain** | **Target Site Length** | **SEQ ID NO** |
| myoC-8 | - | GGACAGUUCCUGUAUUCUUG | 20 | 387 |
| myoC-10 | - | GUAUUCUUGGGGUGGCUACA | 20 | 388 |
| myoC-16 | - | GGUCAUUUACAGCACCGAUG | 20 | 389 |
| myoC-20 | + | GUGUAGCCACCCCAAGAAUA | 20 | 390 |
| myoC-24 | + | GUCCGUGGUAGCCAGCUCCA | 20 | 391 |
| myoC-27 | - | GAAUACCGAGACAGUGA | 17 | 392 |
| myoC-32 | - | GACAGUUCCUGUAUUCU | 17 | 393 |
| myoC-37 | - | CUACACGGACAUUGACU | 17 | 394 |
| myoC-46 | + | UAGCCACCCCAAGAAUA | 17 | 395 |
| myoC-48 | + | AAUACAGGAACUG UCCG | 17 | 396 |
| myoC-50 | + | CGUGGUAGCCAGCUCCA | 17 | 397 |

**Table 1B** provides exemplary targeting domains for the P370L target site selected according to the second tier parameters and are selected based on the presence of a 5' G and reasonable proximity to mutation. In an embodiment, the targeting domain is the exact complement of the target domain. Any of the targeting domains in the table can be used with a *S. pyogenes* Cas9 molecule that gives double stranded cleavage. Any of the targeting domains in the table can be used with *S. pyogenes* single-stranded break nucleases (nickases). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 1B**

| **2nd Tier** | selected based on the presence of a 5' G and reasonable proximity to mutation | | | |
|---|---|---|---|---|
| **gRNA Name** | **DNA Strand** | **Targeting Domain** | **Target Site Length** | **SEQ ID NO** |
| myoC-1 | - | GCUGAAUACCGAGACAGUGA | 20 | 398 |
| myoC-4 | - | GAGAAGGAAAUCCCUGGAGC | 20 | 399 |
| myoC-13 | - | GACUUGGCUGUGGAUGAAGC | 20 | 400 |
| myoC-28 | - | GACAGUGAAGGCUGAGA | 17 | 401 |
| myoC-38 | - | GGACAUUGACUUGGCUG | 17 | 402 |
| myoC-41 | - | GGAUGAAGCAGGCCUCU | 17 | 403 |
| myoC-44 | + | GGCACCUUUGGCCUCAU | 17 | 404 |

**Table 1C** provides exemplary targeting domains for the P370L target site selected according to the third tier parameters and are selected based on reasonable proximity to mutation. In an embodiment, the targeting domain is the exact complement of the target domain. Any of the targeting domains in the table can be used with a *S. pyogenes* Cas9 molecule that gives double stranded cleavage. Any of the targeting domains in the table can be used with *S. pyogenes* single-stranded break nucleases (nickases). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 1C**

| **3rd Tier** | selected based on reasonable proximity to mutation | | | |
|---|---|---|---|---|
| **gRNA Name** | **DNA Strand** | **Targeting Domain** | **Target Site Length** | **SEQ ID NO** |
| myoC-2 | - | CGAGACAGUGAAGGCUGAGA | 20 | 405 |
| myoC-3 | - | AAGGCUGAGAAGGAAAUCCC | 20 | 406 |
| myoC-5 | - | AUCCCUGGAGCUGGCUACCA | 20 | 407 |
| myoC-6 | - | ACGGACAGUUCCCGUAUUCU | 20 | 408 |
| myoC-7 | - | CGGACAGUUCCCGUAUUCUU | 20 | 409 |
| myoC-9 | - | CAGUUCCCGUAUUCUUGGGG | 20 | 410 |
| myoC-11 | - | UGGCUACACGGACAUUGACU | 20 | 411 |
| myoC-12 | - | CACGGACAUUGACUUGGCUG | 20 | 412 |
| myoC-14 | - | CUGUGGAUGAAGCAGGCCUC | 20 | 413 |
| myoC-15 | - | UGUGGAUGAAGCAGGCCUCU | 20 | 414 |
| myoC-17 | - | UACAGCACCGAUGAGGCCAA | 20 | 415 |
| myoC-18 | + | AAUGGCACCUUUGGCCUCAU | 20 | 416 |
| myoC-19 | + | CGGUGCUGUAAAUGACCCAG | 20 | 417 |
| myoC-21 | + | UGUAGCCACCCCAAGAAUAC | 20 | 418 |
| myoC-22 | + | AAGAAUACGGGAACUGUCCG | 20 | 419 |
| myoC-23 | + | UGUCCGUGGUAGCCAGCUCC | 20 | 420 |
| myoC-25 | + | CUUCUCAGCCUUCACUGUCU | 20 | 421 |
| myoC-26 | + | CUCAUAUCUUAUGACAGUUC | 20 | 422 |
| myoC-29 | - | GCUGAGAAGGAAAUCCC | 17 | 423 |
| myoC-30 | - | AAGGAAAUCCCUGGAGC | 17 | 424 |
| myoC-31 | - | CCUGGAGCUGGCUACCA | 17 | 425 |
| myoC-33 | - | ACAGUUCCCGUAUUCUU | 17 | 426 |
| myoC-34 | - | CAGUUCCCGUAUUCUUG | 17 | 427 |
| myoC-35 | - | UUCCCGUAUUCUUGGGG | 17 | 428 |
| myoC-36 | - | UUCUUGGGGUGGCUACA | 17 | 429 |
| myoC-39 | - | UUGGCUGUGGAUGAAGC | 17 | 430 |
| myoC-40 | - | UGGAUGAAGCAGGCCUC | 17 | 431 |
| myoC-42 | - | CAUUUACAGCACCGAUG | 17 | 432 |
| myoC-43 | - | AGCACCGAUGAGGCCAA | 17 | 433 |
| myoC-45 | + | UGCUGUAAAUGACCCAG | 17 | 434 |
| myoC-47 | + | AGCCACCCCAAGAAUAC | 17 | 435 |
| myoC-49 | + | CCGUGGUAGCCAGCUCC | 17 | 436 |
| myoC-51 | + | CUCAGCCUUCACUGUCU | 17 | 437 |
| myoC-52 | + | AUAUCUUAUGACAGUUC | 17 | 438 |

**Table 1D** provides exemplary targeting domains for the P370L target site selected based on close proximity to mutation. In an embodiment, the targeting domain is the exact complement of the target domain. Any of the targeting domains in the table can be used with a *S. aureus* Cas9 molecule that gives double stranded cleavage. Any of the targeting domains in the table can be used with *S. aureus* single-stranded break nucleases (nickases). In an embodiment, dual targeting is used to create two nicks. In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 1D**

| **gRNA Name** | **DNA Strand** | **Targeting Domain** | **Target Site Length** | **SEQ ID NO** |
|---|---|---|---|---|
| myoC-2904 | - | GUCCAGAACUGUCAUAAGAU | 20 | 1806 |
| myoC-2905 | - | GAACUGUCAUAAGAUAUGAG | 20 | 1807 |
| myoC-2906 | - | CAUAAGAUAUGAGCUGAAUA | 20 | 1808 |
| myoC-2907 | - | AUGAGCUGAAUACCGAGACA | 20 | 1809 |
| myoC-2908 | - | GAAUACCGAGACAGUGAAGG | 20 | 1810 |
| myoC-2909 | - | AUACCGAGACAGUGAAGGCU | 20 | 1811 |
| myoC-2910 | - | CCGAGACAGUGAAGGCUGAG | 20 | 1812 |
| myoC-2 | - | CGAGACAGUGAAGGCUGAGA | 20 | 405 |
| myoC-2912 | - | GAAGGCUGAGAAGGAAAUCC | 20 | 1813 |
| myoC-3 | - | AAGGCUGAGAAGGAAAUCCC | 20 | 406 |
| myoC-2914 | - | AAUCCCUGGAGCUGGCUACC | 20 | 1814 |
| myoC-2915 | - | CACGGACAGUUCCCGUAUUC | 20 | 1815 |
| myoC-6 | - | ACGGACAGUUCCCGUAUUCU | 20 | 408 |
| myoC-2917 | - | CGUAUUCUUGGGGUGGCUAC | 20 | 1816 |
| myoC-2918 | - | ACACGGACAUUGACUUGGCU | 20 | 1817 |
| myoC-2919 | - | GGACAUUGACUUGGCUGUGG | 20 | 1818 |
| myoC-2920 | - | GCUGUGGAUGAAGCAGGCCU | 20 | 1819 |
| myoC-2921 | - | CUGGGUCAUUUACAGCACCG | 20 | 1820 |
| myoC-2922 | + | GCUCAUAUCUUAUGACAGUU | 20 | 1821 |
| myoC-23 | + | UGUCCGUGGUAGCCAGCUCC | 20 | 420 |
| myoC-2924 | + | CUGUCCGUGGUAGCCAGCUC | 20 | 1822 |
| myoC-21 | + | UGUAGCCACCCCAAGAAUAC | 20 | 418 |
| myoC-20 | + | GUGUAGCCACCCCAAGAAUA | 20 | 390 |
| myoC-2927 | + | CGUGUAGCCACCCCAAGAAU | 20 | 1823 |
| myoC-2928 | + | AAUGUCCGUGUAGCCACCCC | 20 | 1824 |
| myoC-2929 | + | CAUCGGUGCUGUAAAUGACC | 20 | 1825 |
| myoC-2930 | - | CAGAACUGUCAUAAGAU | 17 | 1826 |
| myoC-2931 | - | CUGUCAUAAGAUAUGAG | 17 | 1827 |
| myoC-2932 | - | AAGAUAUGAGCUGAAUA | 17 | 1828 |
| myoC-2933 | - | AGCUGAAUACCGAGACA | 17 | 1829 |
| myoC-2934 | - | UACCGAGACAGUGAAGG | 17 | 1830 |
| myoC-2935 | - | CCGAGACAGUGAAGGCU | 17 | 1831 |
| myoC-2936 | - | AGACAGUGAAGGCUGAG | 17 | 1832 |
| myoC-28 | - | GACAGUGAAGGCUGAGA | 17 | 401 |
| myoC-2938 | - | GGCUGAGAAGGAAAUCC | 17 | 1833 |
| myoC-29 | - | GCUGAGAAGGAAAUCCC | 17 | 423 |
| myoC-2940 | - | CCCUGGAGCUGGCUACC | 17 | 1834 |
| myoC-2941 | - | GGACAGUUCCCGUAUUC | 17 | 1835 |
| myoC-544 | - | GACAGUUCCCGUAUUCU | 17 | 881 |
| myoC-2943 | - | AUUCUUGGGGUGGCUAC | 17 | 1836 |
| myoC-2944 | - | CGGACAUUGACUUGGCU | 17 | 1837 |
| myoC-2945 | - | CAUUGACUUGGCUGUGG | 17 | 1838 |
| myoC-2946 | - | GUGGAUGAAGCAGGCCU | 17 | 1839 |
| myoC-2947 | - | GGUCAUUUACAGCACCG | 17 | 1840 |
| myoC-2948 | + | CAUAUCUUAUGACAGUU | 17 | 1841 |
| myoC-49 | + | CCGUGGUAGCCAGCUCC | 17 | 436 |
| myoC-2950 | + | UCCGUGGUAGCCAGCUC | 17 | 1842 |
| myoC-47 | + | AGCCACCCCAAGAAUAC | 17 | 435 |
| myoC-46 | + | UAGCCACCCCAAGAAUA | 17 | 395 |
| myoC-2953 | + | GUAGCCACCCCAAGAAU | 17 | 1843 |
| myoC-2954 | + | GUCCGUGUAGCCACCCC | 17 | 1844 |
| myoC-2955 | + | CGGUGCUGUAAAUGACC | 17 | 1845 |

**Table 1E** provides exemplary targeting domains for the P370L site selected based on close proximity to mutation. In an embodiment, the targeting domain is the exact complement of the target domain. Any of the targeting domains in the table can be used with a *N. meningitidis* Cas9 molecule that gives double stranded cleavage. Any of the targeting domains in the table can be used with *N. meningitidis* single-stranded break nucleases (nickases). In an embodiment, dual targeting is used to create two nicks.

**Table 1E**

| **gRNA Name** | **DNA Strand** | **Targeting Domain** | **Target Site Length** | **SEQ ID NO** |
|---|---|---|---|---|
| myoC-2924 | + | CUGUCCGUGGUAGCCAGCUC | 20 | 1822 |
| myoC-2950 | + | UCCGUGGUAGCCAGCUC | 17 | 1842 |

**Table 2A** provides exemplary targeting domains for the I477N target site selected according to first tier parameters, and are selected based on the presence of a 5' G (except for MYOC-68), close proximity and orientation to mutation and orthogonality in the human genome. In an embodiment, the targeting domain is the exact complement of the target domain. Any of the targeting domains in the table can be used with a *S. pyogenes* Cas9 molecule that gives double stranded cleavage. Any of the targeting domains in the table can be used with *S. pyogenes* single-stranded break nucleases (nickases).

In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

In an embodiment, two 20-mer guide RNAs are used to target two *S. pyogenes* Cas9 nucleases or two *S. pyogenes* Cas9 nickases, e.g., MYOC-68 and MYOC-57 are used. In an embodiment, two 17-mer RNAs are used to target two Cas9 nucleases or two Cas9 nickases, e.g., MYOC-87 and MYOC-74, or MYOC-90 and MYOC-74 are used.

**Table 2A**

| **1st Tier** | selected based on the presence of a 5' G (except for #68), close proximity and orientation to mutation and orthogonality in the human genome | | | |
|---|---|---|---|---|
| **gRNA Name** | **DNA Strand** | **Targeting Domain** | **Target Site Length** | **SEQ ID NO** |
| myoC-53 | - | GUCAACUUUGCUUAUGACAC | 20 | 439 |
| myoC-57 | - | GGAGAAGAAGCUCUUUGCCU | 20 | 440 |
| myoC-60 | + | GACCAUGUUCAAGUUGUCCC | 20 | 441 |
| myoC-63 | + | GCAAAGAGCUUCUUCUCCAG | 20 | 442 |
| myoC-68 | + | AUAGCGGUUCUUGAAUGGGA | 20 | 443 |
| myoC-74 | - | GAAUGACUACAACCCCC | 17 | 444 |
| myoC-78 | + | GGAGGCUUUUCACAUCU | 17 | 445 |
| myoC-87 | + | GCGGUUCUUGAAUGGGA | 17 | 446 |
| myoC-90 | + | GUCAUAAGCAAAGUUGA | 17 | 447 |

**Table 2B** provides exemplary targeting domains for the I477N target site selected according to the second tier parameters and are selected based on the presence of a 5' G and reasonable proximity to mutation. In an embodiment, the targeting domain is the exact complement of the target domain. Any of the targeting domains in the table can be used with a *S. pyogenes* Cas9 molecule that gives double stranded cleavage. Any of the targeting domains in the table can be used with *S. pyogenes* single-stranded break nucleases (nickases). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 2B**

| **2nd Tier** | selected based on the presence of a 5' G and reasonable proximity to mutation | | | |
|---|---|---|---|---|
| **gRNA Name** | **DNA Strand** | **Targeting Domain** | **Target Site Length** | **SEQ ID NO** |
| myoC-62 | + | GGCAAAGAGCUUCUUCUCCA | 20 | 448 |
| myoC-69 | + | GGUUCUUGAAUGGGAUGGUC | 20 | 449 |
| myoC-70 | + | GUUCUUGAAUGGGAUGGUCA | 20 | 450 |
| myoC-73 | - | GCUUAUGACACAGGCAC | 17 | 451 |
| myoC-76 | - | GAAGAAGCUCUUUGCCU | 17 | 452 |

**Table 2C** provides exemplary targeting domains for the I477N target site selected according to the third tier parameters and are selected based on reasonable proximity to mutation. In an embodiment, the targeting domain is the exact complement of the target domain. Any of the targeting domains in the table can be used with a *S. pyogenes* Cas9 molecule that gives double stranded cleavage. Any of the targeting domains in the table can be used with *S. pyogenes* single-stranded break nucleases (nickases). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 2C**

| **3rd Tier** | selected based on reasonable proximity to mutation | | | |
|---|---|---|---|---|
| **gRNA Name** | **DNA Strand** | **Targeting Domain** | **Target Site Length** | **SEQ ID NO** |
| myoC-54 | - | UUUGCUUAUGACACAGGCAC | 20 | 453 |
| myoC-55 | - | CAUGAUUGACUACAACCCCC | 20 | 454 |
| myoC-56 | - | UGGAGAAGAAGCUCUUUGCC | 20 | 455 |
| myoC-58 | - | UGCCUGGGACAACUUGAACA | 20 | 456 |
| myoC-59 | + | CUUGGAGGCUUUUCACAUCU | 20 | 457 |
| myoC-61 | + | AGGCAAAGAGCUUCUUCUCC | 20 | 458 |
| myoC-64 | + | CAAAGAGCUUCUUCUCCAGG | 20 | 459 |
| myoC-65 | + | UCAUGCUGCUGUACUUAUAG | 20 | 460 |
| myoC-66 | + | UACUUAUAGCGGUUCUUGAA | 20 | 461 |
| myoC-67 | + | ACUUAUAGCGGUUCUUGAAU | 20 | 462 |
| myoC-71 | + | UGUGUCAUAAGCAAAGUUGA | 20 | 463 |
| myoC-72 | - | AACUUUGCUUAUGACAC | 17 | 464 |
| myoC-75 | - | AGAAGAAGCUCUUUGCC | 17 | 465 |
| myoC-77 | - | CUGGGACAACUUGAACA | 17 | 466 |
| myoC-79 | + | CAUGUUCAAGUUGUCCC | 17 | 467 |
| myoC-80 | + | CAAAGAGCUUCUUCUCC | 17 | 468 |
| myoC-81 | + | AAAGAGCUUCUUCUCCA | 17 | 469 |
| myoC-82 | + | AAGAGCUUCUUCUCCAG | 17 | 470 |
| myoC-83 | + | AGAGCUUCUUCUCCAGG | 17 | 471 |
| myoC-84 | + | UGCUGCUGUACUUAUAG | 17 | 472 |
| myoC-85 | + | UUAUAGCGGUUCUUGAA | 17 | 473 |
| myoC-86 | + | UAUAGCGGUUCUUGAAU | 17 | 474 |
| myoC-88 | + | UCUUGAAUGGGAUGGUC | 17 | 475 |
| myoC-89 | + | CUUGAAUGGGAUGGUCA | 17 | 476 |

**Table 2D** provides exemplary targeting domains for the I477N target site selected based on close proximity to mutation. In an embodiment, the targeting domain is the exact complement of the target domain. Any of the targeting domains in the table can be used with a *S. aureus* Cas9 molecule that gives double stranded cleavage. Any of the targeting domains in the table can be used with single-stranded break nucleases (nickases). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 2D**

| **gRNA Name** | **DNA Strand** | **Targeting Domain** | **Target Site Length** | **SEQ ID NO** |
|---|---|---|---|---|
| myoC-2956 | - | AGACCCUGACCAUCCCAUUC | 20 | 1846 |
| myoC-2957 | - | GCAUGAUUGACUACAACCCC | 20 | 1847 |
| myoC-55 | - | CAUGAUUGACUACAACCCCC | 20 | 454 |
| myoC-2959 | - | UGAUUGACUACAACCCCCUG | 20 | 1848 |
| myoC-2960 | - | UUGACUACAACCCCCUGGAG | 20 | 1849 |
| myoC-2961 | - | CUGGAGAAGAAGCUCUUUGC | 20 | 1850 |
| myoC-56 | - | UGGAGAAGAAGCUCUUUGCC | 20 | 455 |
| myoC-2963 | - | AGCUCUUUGCCUGGGACAAC | 20 | 1851 |
| myoC-2964 | - | GACAUCAAGCUCUCCAAGAU | 20 | 1852 |
| myoC-2965 | + | AAAGUUGACGGUAGCAUCUG | 20 | 1853 |
| myoC-2966 | + | CGGUUCUUGAAUGGGAUGGU | 20 | 1854 |
| myoC-66 | + | UACUUAUAGCGGUUCUUGAA | 20 | 461 |
| myoC-2968 | + | GUACUUAUAGCGGUUCUUGA | 20 | 1855 |
| myoC-2969 | + | UGCUGUACUUAUAGCGGUUC | 20 | 1856 |
| myoC-62 | + | GGCAAAGAGCUUCUUCUCCA | 20 | 448 |
| myoC-61 | + | AGGCAAAGAGCUUCUUCUCC | 20 | 458 |
| myoC-2972 | + | CAGGCAAAGAGCUUCUUCUC | 20 | 1857 |
| myoC-2973 | + | UGUUCAAGUUGUCCCAGGCA | 20 | 1858 |
| myoC-2974 | + | UGGAGGCUUUUCACAUCUUG | 20 | 1859 |
| myoC-59 | + | CUUGGAGGCUUUUCACAUCU | 20 | 457 |
| myoC-2976 | + | GCUUGGAGGCUUUUCACAUC | 20 | 1860 |
| myoC-2977 | - | CCCUGACCAUCCCAUUC | 17 | 1861 |
| myoC-2978 | - | UGAUUGACUACAACCCC | 17 | 1862 |
| myoC-562 | - | GAUUGACUACAACCCCC | 17 | 886 |
| myoC-2980 | - | UUGACUACAACCCCCUG | 17 | 1863 |
| myoC-2981 | - | ACUACAACCCCCUGGAG | 17 | 1864 |
| myoC-2982 | - | GAGAAGAAGCUCUUUGC | 17 | 1865 |
| myoC-75 | - | AGAAGAAGCUCUUUGCC | 17 | 465 |
| myoC-2984 | - | UCUUUGCCUGGGACAAC | 17 | 1866 |
| myoC-2985 | - | AUCAAGCUCUCCAAGAU | 17 | 1867 |
| myoC-2986 | + | GUUGACGGUAGCAUCUG | 17 | 1868 |
| myoC-2987 | + | UUCUUGAAUGGGAUGGU | 17 | 1869 |
| myoC-85 | + | UUAUAGCGGUUCUUGAA | 17 | 473 |
| myoC-2989 | + | CUUAUAGCGGUUCUUGA | 17 | 1870 |
| myoC-2990 | + | UGUACUUAUAGCGGUUC | 17 | 1871 |
| myoC-81 | + | AAAGAGCUUCUUCUCCA | 17 | 469 |
| myoC-80 | + | CAAAGAGCUUCUUCUCC | 17 | 468 |
| myoC-2993 | + | GCAAAGAGCUUCUUCUC | 17 | 1872 |
| myoC-2994 | + | UCAAGUUGUCCCAGGCA | 17 | 1873 |
| myoC-2995 | + | AGGCUUUUCACAUCUUG | 17 | 1874 |
| myoC-78 | + | GGAGGCUUUUCACAUCU | 17 | 445 |
| myoC-2997 | + | UGGAGGCUUUUCACAUC | 17 | 1875 |

**Table 2E** provides exemplary targeting domains for the I477N target site selected based on close proximity to mutation. In an embodiment, the targeting domain is the exact complement of the target domain. Any of the targeting domains in the table can be used with a *N. meningitidis* Cas9 molecule that gives double stranded cleavage. Any of the targeting domains in the table can be used with single-stranded break nucleases (nickases). In an embodiment, dual targeting is used to create two nicks.

**Table 2E**

| **gRNA Name** | **DNA Strand** | **Targeting Domain** | **Target Site Length** | **SEQ ID NO** |
|---|---|---|---|---|
| myoC-3156 | - | GAACCGCUAUAAGUACAGCA | 20 | 2842 |
| myoC-3157 | - | CCGCUAUAAGUACAGCA | 17 | 2843 |

**Table 3A** provides exemplary targeting domains for the mutational hotspot 477-502 target site selected according to the first tier parameters, and are selected based on the presence of a 5' G (except for MYOC-54 and -1546), close proximity and orientation to mutation and orthogonality in the human genome. In an embodiment, the targeting domain is the exact complement of the target domain. Any of the targeting domains in the table can be used with a *S. pyogenes* Cas9 molecule that gives double stranded cleavage. Any of the targeting domains in the table can be used with *S. pyogenes* single-stranded break nucleases (nickases).

In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

In an embodiment, two 20-mer guide RNAs are used to target two *S. pyogenes* Cas9 nucleases or two *S. pyogenes* Cas9 nickases, e.g., MYOC-1501 and MYOC-54, MYOC-59 and MYOC-1531, MYOC-59 and MYOC-1537, or MYOC-1546 and MYOC-1537 are used. In an embodiment, two 17-mer RNAs are used to target two Cas9 nucleases or two Cas9 nickases, e.g., MYOC-73 and MYOC-1502, or MYOC-1549 and MYOC-78 are used.

For convenience, it is noted that targeting domains for gRNAs MYOC-53, -54, 65-73 and 84-90 are also listed for targeting the I447N mutation. These targeting domains are useful for targeting both a correction of the I447 point mutation and the mutational hotspot 477-502 target site.

**Table 3A**

| **1st Tier** | selected based on the presence of a 5' G (except for #54 and 1546), close proximity and orientation to mutation and orthogonality in the human genome | | | | |
|---|---|---|---|---|---|
| **gRNA Name** | **DNA Strand** | **Targeting Domain** | **Target Site Length** | **Location** | **SEQ ID NO** |
| myoC-53 | - | GUCAACUUUGCUUAUGACAC | 20 | within 100bp upstream of hotspot | 439 |
| myoC-54 | - | UUUGCUUAUGACACAGGCAC | 20 | within 100bp upstream of hotspot | 453 |
| myoC-69 | + | GGUUCUUGAAUGGGAUGGUC | 20 | within 100bp upstream of hotspot | 449 |
| myoC-437 | + | GUUGACGGUAGCAUCUGCUG | 20 | within 100bp upstream of hotspot | 788 |
| myoC-73 | - | GCUUAUGACACAGGCAC | 17 | within 100bp upstream of hotspot | 451 |
| myoC-87 | + | GCGGUUCUUGAAUGGGA | 17 | within 100bp upstream of hotspot | 446 |
| myoC-599 | + | GACGGUAGCAUCUGCUG | 17 | within 100bp upstream of hotspot | 907 |
| myoC-405 | - | GAAAAGCCUCCAAGCUGUAC | 20 | within 100bp downstream of hotspot | 769 |
| myoC-407 | - | GCUGUACAGGCAAUGGCAGA | 20 | within 100bp downstream of hotspot | 771 |
| myoC-413 | - | GAGAUGCUCAGGGCUCCUGG | 20 | within 100bp downstream of hotspot | 777 |
| myoC-423 | + | CCAUUGCCUGUACAGCUUGG | 20 | within 100bp downstream of hotspot | 787 |
| myoC-568 | - | GUACAGGCAAUGGCAGA | 17 | within 100bp downstream of hotspot | 889 |
| myoC-78 | + | GGAGGCUUUUCACAUCU | 17 | within 100bp downstream of hotspot | 445 |

**Table 3B** provides exemplary targeting domains for the mutational hotspot 477-502 target site selected according to the second tier parameters and are selected based on the presence of a 5' G and reasonable proximity to mutation. In an embodiment, the targeting domain is the exact complement of the target domain. Any of the targeting domains in the table can be used with a *S. pyogenes* Cas9 molecule that gives double stranded cleavage. Any of the targeting domains in the table can be used with *S. pyogenes* single-stranded break nucleases (nickases). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 3B**

| **2nd Tier** | selected based on the presence of a 5' G and reasonable proximity to mutation | | | | |
|---|---|---|---|---|---|
| **gRNA Name** | **DNA Strand** | **Targeting Domain** | **Target Site Length** | **Location** | **SEQ ID NO** |
| myoC-70 | + | GUUCUUGAAUGGGAUGGUCA | 20 | within 100bp upstream of hotspot | 450 |
| myoC-90 | + | GUCAUAAGCAAAGUUGA | 17 | within 100bp upstream of hotspot | 447 |
| myoC-398 | - | GCCAAUGCCUUCAUCAUCUG | 20 | 100-200bp upstream of hotspot | 768 |
| myoC-439 | + | GUAGCUGCUGACGGUGUACA | 20 | 100-200bp upstream of hotspot | 790 |
| myoC-441 | + | GCCACAGAUGAUGAAGGCAU | 20 | 100-200bp upstream of hotspot | 792 |
| myoC-445 | + | GUUCGAGUUCCAGAUUCUCU | 20 | 100-200bp upstream of hotspot | 796 |
| myoC-558 | - | GGAACUCGAACAAACCU | 17 | 100-200bp upstream of hotspot | 884 |
| myoC-601 | + | GCUGCUGACGGUGUACA | 17 | 100-200bp upstream of hotspot | 909 |
| myoC-602 | + | GGUGCCACAGAUGAUGA | 17 | 100-200bp upstream of hotspot | 910 |
| myoC-412 | - | GGAGAUGCUCAGGGCUCCUG | 20 | within 100bp downstream of hotspot | 776 |
| myoC-418 | - | GAAGGGAGAGCCAGCCAGCC | 20 | within 100bp downstream of hotspot | 782 |
| myoC-569 | - | GGCAGAAGGAGAUGCUC | 17 | within 100bp downstream of hotspot | 890 |
| myoC-570 | - | GCAGAAGGAGAUGCUCA | 17 | within 100bp downstream of hotspot | 891 |
| myoC-571 | - | GAGAUGCUCAGGGCUCC | 17 | within 100bp downstream of hotspot | 892 |
| myoC-573 | - | GAUGCUCAGGGCUCCUG | 17 | within 100bp downstream of hotspot | 894 |
| myoC-576 | - | GGGCUCCUGGGGGGAGC | 17 | within 100bp downstream of hotspot | 897 |
| myoC-578 | - | GGGGGGAGCAGGCUGAA | 17 | within 100bp downstream of hotspot | 899 |
| myoC-579 | - | GGGAGAGCCAGCCAGCC | 17 | within 100bp downstream of hotspot | 900 |
| myoC-580 | - | GGAGAGCCAGCCAGCCA | 17 | within 100bp downstream of hotspot | 901 |
| myoC-420 | - | GAGCCAGCCAGCCAGGGCCC | 20 | 100-200bp downstream of hotspot | 784 |
| myoC-510 | + | GGUGACCAUGUUCAUCCUUC | 20 | 100-200bp downstream of hotspot | 852 |
| myoC-512 | + | GGAAAGCAGUCAAAGCUGCC | 20 | 100-200bp downstream of hotspot | 854 |
| myoC-513 | + | GAAAGCAGUCAAAGCUGCCU | 20 | 100-200bp downstream of hotspot | 855 |
| myoC-645 | - | GUUUUCAUUAAUCCAGA | 17 | 100-200bp downstream of hotspot | 945 |
| myoC-672 | + | GACCAUGUUCAUCCUUC | 17 | 100-200bp downstream of hotspot | 972 |
| myoC-1591 | + | GCUGCCUGGGCCCUGGC | 17 | 100-200bp downstream of hotspot | 1801 |

**Table 3C** provides exemplary targeting domains for the mutational hotspot 477-502 targeting site selected according to the third tier parameters and are selected based on reasonable proximity to mutation. In an embodiment, the targeting domain is the exact complement of the target domain. Any of the targeting domains in the table can be used with a *S. pyogenes* Cas9 molecule that gives double stranded cleavage. Any of the targeting domains in the table can be used with *S. pyogenes* single-stranded break nucleases (nickases). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 3C**

| **3rd Tier** | selected based on the presence of a 5' G and reasonable proximity to mutation | | | | |
|---|---|---|---|---|---|
| **gRNA Name** | **DNA Strand** | **Targeting Domain** | **Target Site Length** | **Location** | **SEQ ID NO** |
| myoC-65 | + | UCAUGCUGCUGUACUUAUAG | 20 | within 100bp upstream of hotspot | 460 |
| myoC-66 | + | UACUUAUAGCGGUUCUUGAA | 20 | within 100bp upstream of hotspot | 461 |
| myoC-67 | + | ACUUAUAGCGGUUCUUGAAU | 20 | within 100bp upstream of hotspot | 462 |
| myoC-68 | + | AUAGCGGUUCUUGAAUGGGA | 20 | within 100bp upstream of hotspot | 443 |
| myoC-71 | + | UGUGUCAUAAGCAAAGUUGA | 20 | within 100bp upstream of hotspot | 463 |
| myoC-72 | - | AACUUUGCUUAUGACAC | 17 | within 100bp upstream of hotspot | 464 |
| myoC-84 | + | UGCUGCUGUACUUAUAG | 17 | within 100bp upstream of hotspot | 472 |
| myoC-85 | + | UUAUAGCGGUUCUUGAA | 17 | within 100bp upstream of hotspot | 473 |
| myoC-86 | + | UAUAGCGGUUCUUGAAU | 17 | within 100bp upstream of hotspot | 474 |
| myoC-88 | + | UCUUGAAUGGGAUGGUC | 17 | within 100bp upstream of hotspot | 475 |
| myoC-89 | + | CUUGAAUGGGAUGGUCA | 17 | within 100bp upstream of hotspot | 476 |
| myoC-395 | - | CAAACUGAACCCAGAGAAUC | 20 | 100-200bp upstream of hotspot | 765 |
| myoC-396 | - | AUCUGGAACUCGAACAAACC | 20 | 100-200bp upstream of hotspot | 766 |
| myoC-397 | - | UCUGGAACUCGAACAAACCU | 20 | 100-200bp upstream of hotspot | 767 |
| myoC-438 | + | UGCUGAGGUGUAGCUGCUGA | 20 | 100-200bp upstream of hotspot | 789 |
| myoC-440 | + | CAAGGUGCCACAGAUGAUGA | 20 | 100-200bp upstream of hotspot | 791 |
| myoC-442 | + | CAUUGGCGACUGACUGCUUA | 20 | 100-200bp upstream of hotspot | 793 |
| myoC-443 | + | CUUACGGAUGUUUGUCUCCC | 20 | 100-200bp upstream of hotspot | 794 |
| myoC-444 | + | UGUUCGAGUUCCAGAUUCUC | 20 | 100-200bp upstream of hotspot | 795 |
| myoC-446 | + | CAGAUUCUCUGGGUUCAGUU | 20 | 100-200bp upstream of hotspot | 797 |
| myoC-556 | - | ACUGAACCCAGAGAAUC | 17 | 100-200bp upstream of hotspot | 882 |
| myoC-557 | - | UGGAACUCGAACAAACC | 17 | 100-200bp upstream of hotspot | 883 |
| myoC-559 | - | AAUGCCUUCAUCAUCUG | 17 | 100-200bp upstream of hotspot | 885 |
| myoC-600 | + | UGAGGUGUAGCUGCUGA | 17 | 100-200bp upstream of hotspot | 908 |
| myoC-603 | + | ACAGAUGAUGAAGGCAU | 17 | 100-200bp upstream of hotspot | 911 |
| myoC-604 | + | UGGCGACUGACUGCUUA | 17 | 100-200bp upstream of hotspot | 912 |
| myoC-605 | + | ACGGAUGUUUGUCUCCC | 17 | 100-200bp upstream of hotspot | 913 |
| myoC-606 | + | UCGAGUUCCAGAUUCUC | 17 | 100-200bp upstream of hotspot | 914 |
| myoC-607 | + | CGAGUUCCAGAUUCUCU | 17 | 100-200bp upstream of hotspot | 915 |
| myoC-608 | + | AUUCUCUGGGUUCAGUU | 17 | 100-200bp upstream of hotspot | 916 |
| myoC-406 | - | CCUCCAAGCUGUACAGGCAA | 20 | within 100bp downstream of hotspot | 770 |
| myoC-408 | - | AAUGGCAGAAGGAGAUGCUC | 20 | within 100bp downstream of hotspot | 772 |
| myoC-409 | - | AUGGCAGAAGGAGAUGCUCA | 20 | within 100bp downstream of hotspot | 773 |
| myoC-410 | - | AAGGAGAUGCUCAGGGCUCC | 20 | within 100bp downstream of hotspot | 774 |
| myoC-411 | - | AGGAGAUGCUCAGGGCUCCU | 20 | within 100bp downstream of hotspot | 775 |
| myoC-414 | - | AGAUGCUCAGGGCUCCUGGG | 20 | within 100bp downstream of hotspot | 778 |
| myoC-415 | - | UCAGGGCUCCUGGGGGGAGC | 20 | within 100bp downstream of hotspot | 779 |
| myoC-416 | - | UCCUGGGGGGAGCAGGCUGA | 20 | within 100bp downstream of hotspot | 780 |
| myoC-417 | - | CCUGGGGGGAGCAGGCUGAA | 20 | within 100bp downstream of hotspot | 781 |
| myoC-419 | - | AAGGGAGAGCCAGCCAGCCA | 20 | within 100bp downstream of hotspot | 783 |
| myoC-59 | + | CUUGGAGGCUUUUCACAUCU | 20 | within 100bp downstream of hotspot | 457 |
| myoC-421 | + | CCCUUCAGCCUGCUCCCCCC | 20 | within 100bp downstream of hotspot | 785 |
| myoC-422 | + | CUGCCAUUGCCUGUACAGCU | 20 | within 100bp downstream of hotspot | 786 |
| myoC-566 | - | AAGCCUCCAAGCUGUAC | 17 | within 100bp downstream of hotspot | 887 |
| myoC-567 | - | CCAAGCUGUACAGGCAA | 17 | within 100bp downstream of hotspot | 888 |
| myoC-572 | - | AGAUGCUCAGGGCUCCU | 17 | within 100bp downstream of hotspot | 893 |
| myoC-574 | - | AUGCUCAGGGCUCCUGG | 17 | within 100bp downstream of hotspot | 895 |
| myoC-575 | - | UGCUCAGGGCUCCUGGG | 17 | within 100bp downstream of hotspot | 896 |
| myoC-577 | - | UGGGGGGAGCAGGCUGA | 17 | within 100bp downstream of hotspot | 898 |
| myoC-583 | + | UUCAGCCUGCUCCCCCC | 17 | within 100bp downstream of hotspot | 904 |
| myoC-584 | + | CCAUUGCCUGUACAGCU | 17 | within 100bp downstream of hotspot | 905 |
| myoC-585 | + | UUGCCUGUACAGCUUGG | 17 | within 100bp downstream of hotspot | 906 |
| myoC-483 | - | CAAGUUUUCAUUAAUCCAGA | 20 | 100-200bp downstream of hotspot | 825 |
| myoC-484 | - | UUAAUCCAGAAGGAUGAACA | 20 | 100-200bp downstream of hotspot | 826 |
| myoC-485 | - | UGGUCACCAUCUAACUAUUC | 20 | 100-200bp downstream of hotspot | 827 |
| myoC-486 | - | UAUUCAGGAAUUGUAGUCUG | 20 | 100-200bp downstream of hotspot | 828 |
| myoC-487 | - | AUUCAGGAAUUGUAGUCUGA | 20 | 100-200bp downstream of hotspot | 829 |
| myoC-509 | + | ACAAUUCCUGAAUAGUUAGA | 20 | 100-200bp downstream of hotspot | 851 |
| myoC-511 | + | CUUCUGGAUUAAUGAAAACU | 20 | 100-200bp downstream of hotspot | 853 |
| myoC-1576 | + | AGUCAAAGCUGCCUGGGCCC | 20 | 100-200bp downstream of hotspot | 1802 |
| myoC-1577 | + | AAAGCUGCCUGGGCCCUGGC | 20 | 100-200bp downstream of hotspot | 1803 |
| myoC-1578 | + | CUGCCUGGGCCCUGGCUGGC | 20 | 100-200bp downstream of hotspot | 1804 |
| myoC-581 | - | CCAGCCAGCCAGGGCCC | 17 | 100-200bp downstream of hotspot | 902 |
| myoC-646 | - | AUCCAGAAGGAUGAACA | 17 | 100-200bp downstream of hotspot | 946 |
| myoC-647 | - | UCACCAUCUAACUAUUC | 17 | 100-200bp downstream of hotspot | 947 |
| myoC-648 | - | UCAGGAAUUGUAGUCUG | 17 | 100-200bp downstream of hotspot | 948 |
| myoC-649 | - | CAGGAAUUGUAGUCUGA | 17 | 100-200bp downstream of hotspot | 949 |
| myoC-671 | + | AUUCCUGAAUAGUUAGA | 17 | 100-200bp downstream of hotspot | 971 |
| myoC-673 | + | CUGGAUUAAUGAAAACU | 17 | 100-200bp downstream of hotspot | 973 |
| myoC-674 | + | AAGCAGUCAAAGCUGCC | 17 | 100-200bp downstream of hotspot | 974 |
| myoC-675 | + | AGCAGUCAAAGCUGCCU | 17 | 100-200bp downstream of hotspot | 975 |
| myoC-1590 | + | CAAAGCUGCCUGGGCCC | 17 | 100-200bp downstream of hotspot | 1805 |
| myoC-582 | + | CCUGGGCCCUGGCUGGC | 17 | 100-200bp downstream of hotspot | 903 |

**Table 3D** provides exemplary targeting domains for the mutational hotspot 477-502 target site selected based on close proximity to mutation. In an embodiment, the targeting domain is the exact complement of the target domain. Any of the targeting domains in the table can be used with a *S*. *aureus* Cas9 molecule that gives double stranded cleavage. Any of the targeting domains in the table can be used with *S. aureus* single-stranded break nucleases (nickases). In an embodiment, dual targeting is used to create two nicks on opposite DNA strands by using *S. aureus* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 3D**

| **gRNA Name** | **DNA Strand** | **Targeting Domain** | **Target Site Length** | **SEQID NO** |
|---|---|---|---|---|
| myoC-396 | - | AUCUGGAACUCGAACAAACC | 20 | 766 |
| myoC-397 | - | UCUGGAACUCGAACAAACCU | 20 | 767 |
| myoC-2956 | - | AGACCCUGACCAUCCCAUUC | 20 | 1846 |
| myoC-2999 | + | UGUUUGUCUCCCAGGUUUGU | 20 | 2792 |
| myoC-3000 | + | GCAUUGGCGACUGACUGCUU | 20 | 2793 |
| myoC-3001 | + | UGUACAAGGUGCCACAGAUG | 20 | 2794 |
| myoC-2965 | + | AAAGUUGACGGUAGCAUCUG | 20 | 1853 |
| myoC-2966 | + | CGGUUCUUGAAUGGGAUGGU | 20 | 1854 |
| myoC-66 | + | UACUUAUAGCGGUUCUUGAA | 20 | 461 |
| myoC-2968 | + | GUACUUAUAGCGGUUCUUGA | 20 | 1855 |
| myoC-2969 | + | UGCUGUACUUAUAGCGGUUC | 20 | 1856 |
| myoC-3003 | - | CCAAGCUGUACAGGCAAUGG | 20 | 2795 |
| myoC-3004 | - | AGCUGUACAGGCAAUGGCAG | 20 | 2796 |
| myoC-407 | - | GCUGUACAGGCAAUGGCAGA | 20 | 771 |
| myoC-3006 | - | CAAUGGCAGAAGGAGAUGCU | 20 | 2797 |
| myoC-3007 | - | GAAGGAGAUGCUCAGGGCUC | 20 | 2798 |
| myoC-410 | - | AAGGAGAUGCUCAGGGCUCC | 20 | 774 |
| myoC-411 | - | AGGAGAUGCUCAGGGCUCCU | 20 | 775 |
| myoC-412 | - | GGAGAUGCUCAGGGCUCCUG | 20 | 776 |
| myoC-413 | - | GAGAUGCUCAGGGCUCCUGG | 20 | 777 |
| myoC-414 | - | AGAUGCUCAGGGCUCCUGGG | 20 | 778 |
| myoC-3013 | - | GGGCUCCUGGGGGGAGCAGG | 20 | 2799 |
| myoC-3014 | - | CUCCUGGGGGGAGCAGGCUG | 20 | 2800 |
| myoC-416 | - | UCCUGGGGGGAGCAGGCUGA | 20 | 780 |
| myoC-417 | - | CCUGGGGGGAGCAGGCUGAA | 20 | 781 |
| myoC-3017 | - | UGGGGGGAGCAGGCUGAAGG | 20 | 2801 |
| myoC-3018 | - | UGAAGGGAGAGCCAGCCAGC | 20 | 2802 |
| myoC-3019 | - | UUUCCAAGUUUUCAUUAAUC | 20 | 2803 |
| myoC-3020 | - | CCAAGUUUUCAUUAAUCCAG | 20 | 2804 |
| myoC-3021 | - | GUUUUCAUUAAUCCAGAAGG | 20 | 2805 |
| myoC-3022 | - | AUGGUCACCAUCUAACUAUU | 20 | 2806 |
| myoC-485 | - | UGGUCACCAUCUAACUAUUC | 20 | 827 |
| myoC-3024 | - | AACUAUUCAGGAAUUGUAGU | 20 | 2807 |
| myoC-3025 | - | CUAUUCAGGAAUUGUAGUCU | 20 | 2808 |
| myoC-2974 | + | UGGAGGCUUUUCACAUCUUG | 20 | 1859 |
| myoC-59 | + | CUUGGAGGCUUUUCACAUCU | 20 | 457 |
| myoC-2976 | + | GCUUGGAGGCUUUUCACAUC | 20 | 1860 |
| myoC-422 | + | CUGCCAUUGCCUGUACAGCU | 20 | 786 |
| myoC-3030 | + | UCUGCCAUUGCCUGUACAGC | 20 | 2809 |
| myoC-3031 | + | GCCUGCUCCCCCCAGGAGCC | 20 | 2810 |
| myoC-421 | + | CCCUUCAGCCUGCUCCCCCC | 20 | 785 |
| myoC-3033 | + | UCCCUUCAGCCUGCUCCCCC | 20 | 2811 |
| myoC-3034 | + | UGGAAAGCAGUCAAAGCUGC | 20 | 2812 |
| myoC-511 | + | CUUCUGGAUUAAUGAAAACU | 20 | 853 |
| myoC-3036 | + | CCUUCUGGAUUAAUGAAAAC | 20 | 2813 |
| myoC-3037 | + | AUGUUCAUCCUUCUGGAUUA | 20 | 2814 |
| myoC-3038 | + | UGGUGACCAUGUUCAUCCUU | 20 | 2815 |
| myoC-3039 | + | ACGCCCUCAGACUACAAUUC | 20 | 2816 |
| myoC-557 | - | UGGAACUCGAACAAACC | 17 | 883 |
| myoC-558 | - | GGAACUCGAACAAACCU | 17 | 884 |
| myoC-2977 | - | CCCUGACCAUCCCAUUC | 17 | 1861 |
| myoC-3041 | + | UUGUCUCCCAGGUUUGU | 17 | 2817 |
| myoC-3042 | + | UUGGCGACUGACUGCUU | 17 | 2818 |
| myoC-3043 | + | ACAAGGUGCCACAGAUG | 17 | 2819 |
| myoC-2986 | + | GUUGACGGUAGCAUCUG | 17 | 1868 |
| myoC-2987 | + | UUCUUGAAUGGGAUGGU | 17 | 1869 |
| myoC-85 | + | UUAUAGCGGUUCUUGAA | 17 | 473 |
| myoC-2989 | + | CUUAUAGCGGUUCUUGA | 17 | 1870 |
| myoC-2990 | + | UGUACUUAUAGCGGUUC | 17 | 1871 |
| myoC-3045 | - | AGCUGUACAGGCAAUGG | 17 | 2820 |
| myoC-3046 | - | UGUACAGGCAAUGGCAG | 17 | 2821 |
| myoC-568 | - | GUACAGGCAAUGGCAGA | 17 | 889 |
| myoC-3048 | - | UGGCAGAAGGAGAUGCU | 17 | 2822 |
| myoC-3049 | - | GGAGAUGCUCAGGGCUC | 17 | 2823 |
| myoC-571 | - | GAGAUGCUCAGGGCUCC | 17 | 892 |
| myoC-572 | - | AGAUGCUCAGGGCUCCU | 17 | 893 |
| myoC-573 | - | GAUGCUCAGGGCUCCUG | 17 | 894 |
| myoC-574 | - | AUGCUCAGGGCUCCUGG | 17 | 895 |
| myoC-575 | - | UGCUCAGGGCUCCUGGG | 17 | 896 |
| myoC-3055 | - | CUCCUGGGGGGAGCAGG | 17 | 2824 |
| myoC-3056 | - | CUGGGGGGAGCAGGCUG | 17 | 2825 |
| myoC-577 | - | UGGGGGGAGCAGGCUGA | 17 | 898 |
| myoC-578 | - | GGGGGGAGCAGGCUGAA | 17 | 899 |
| myoC-3059 | - | GGGGAGCAGGCUGAAGG | 17 | 2826 |
| myoC-3060 | - | AGGGAGAGCCAGCCAGC | 17 | 2827 |
| myoC-3061 | - | CCAAGUUUUCAUUAAUC | 17 | 2828 |
| myoC-3062 | - | AGUUUUCAUUAAUCCAG | 17 | 2829 |
| myoC-3063 | - | UUCAUUAAUCCAGAAGG | 17 | 2830 |
| myoC-3064 | - | GUCACCAUCUAACUAUU | 17 | 2831 |
| myoC-647 | - | UCACCAUCUAACUAUUC | 17 | 947 |
| myoC-3066 | - | UAUUCAGGAAUUGUAGU | 17 | 2832 |
| myoC-3067 | - | UUCAGGAAUUGUAGUCU | 17 | 2833 |
| myoC-2995 | + | AGGCUUUUCACAUCUUG | 17 | 1874 |
| myoC-78 | + | GGAGGCUUUUCACAUCU | 17 | 445 |
| myoC-2997 | + | UGGAGGCUUUUCACAUC | 17 | 1875 |
| myoC-584 | + | CCAUUGCCUGUACAGCU | 17 | 905 |
| myoC-3072 | + | GCCAUUGCCUGUACAGC | 17 | 2834 |
| myoC-3073 | + | UGCUCCCCCCAGGAGCC | 17 | 2835 |
| myoC-583 | + | UUCAGCCUGCUCCCCCC | 17 | 904 |
| myoC-3075 | + | CUUCAGCCUGCUCCCCC | 17 | 2836 |
| myoC-3076 | + | AAAGCAGUCAAAGCUGC | 17 | 2837 |
| myoC-673 | + | CUGGAUUAAUGAAAACU | 17 | 973 |
| myoC-3078 | + | UCUGGAUUAAUGAAAAC | 17 | 2838 |
| myoC-3079 | + | UUCAUCCUUCUGGAUUA | 17 | 2839 |
| myoC-3080 | + | UGACCAUGUUCAUCCUU | 17 | 2840 |
| myoC-3081 | + | CCCUCAGACUACAAUUC | 17 | 2841 |

**Table 3E** provides exemplary targeting domains for the mutational hotspot 477-502 target site selected based on close proximity to mutation. In an embodiment, the targeting domain is the exact complement of the target domain. Any of the targeting domains in the table can be used with a *N*. *meningitidis* Cas9 molecule that gives double stranded cleavage. Any of the targeting domains in the table can be used with *N. meningitidis* single-stranded break nucleases (nickases). In an embodiment, dual targeting is used to create two nicks.

**Table 3E**

| **gRNA Name** | **DNA Strand** | **Targeting Domain** | **Target Site Length** | **SEQ ID NO** |
|---|---|---|---|---|
| myoC-3091 | + | AUGGUGACCAUGUUCAUCCU | 20 | 2849 |
| myoC-3097 | + | GUGACCAUGUUCAUCCU | 17 | 2855 |

**Table 4A** provides exemplary targeting domains for knocking out the *MYOC* gene selected according to first tier parameters, and are selected based on the presence of a 5' G, close proximity to the start codon (located in exon 1) and orthogonality in the human genome. In an embodiment, the targeting domain is the exact complement of the target domain. Any of the targeting domains in the table can be used with a *S. pyogenes* Cas9 molecule that gives double stranded cleavage. Any of the targeting domains in the table can be used with a *S. pyogenes* Cas9 single-stranded break nucleases (nickases). In an embodiment, dual targeting is used to create two nicks on opposite DNA strands by using *S*. *pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 4A**

| **1st Tier** | selected based on the presence of a 5' G, close proximity to the start codon (gRNAs located in exon1) and orthogonality in the human genome | | | |
|---|---|---|---|---|
| **gRNA Name** | **DNA Strand** | **Targeting Domain** | **Target Site Length** | **SEQ ID NO** |
| myoC-91 | - | GUGCACGUUGCUGCAGCUUU | 20 | 477 |
| myoC-93 | - | GCUUCUGGCCUGCCUGGUGU | 20 | 478 |
| myoC-106 | - | GGAAACCCAAACCAGAGAGU | 20 | 479 |
| myoC-108 | - | GUUGGAAAGCAGCAGCCAGG | 20 | 480 |
| myoC-112 | + | GCACAGCCCGAGCAGUGUCU | 20 | 481 |
| myoC-114 | + | GAACUGACUUGUCUCGGAGG | 20 | 482 |
| myoC-116 | + | GUAGGCAGUCUCCAACUCUC | 20 | 483 |
| myoC-117 | + | GCUGGUCCCGCUCCCGCCUC | 20 | 484 |
| myoC-123 | + | GUCGAGCUUUGGUGGCCUCC | 20 | 485 |
| myoC-124 | + | GGCCUCCAGGUCUAAGCGUU | 20 | 486 |
| myoC-127 | + | GCAUCGGCCACUCUGGUCAU | 20 | 487 |
| myoC-129 | - | GCACGUUGCUGCAGCUU | 17 | 488 |
| myoC-147 | - | GACCCGAGACACUGCUC | 17 | 489 |
| myoC-148 | - | GCUCGGGCUGUGCCACC | 17 | 490 |
| myoC-149 | + | GAGCAGUGUCUCGGGUC | 17 | 491 |
| myoC-152 | + | GAACUGACUUGUCUCGG | 17 | 492 |
| myoC-157 | + | GGUCCAAGGUCAAUUGG | 17 | 493 |
| myoC-160 | + | GGAGCUGAGUCGAGCUU | 17 | 494 |
| myoC-161 | + | GCUGAGUCGAGCUUUGG | 17 | 495 |
| myoC-163 | + | GUUAUGGAUGACUGACA | 17 | 496 |
| myoC-167 | + | GCUGGAUUCAUUGGGAC | 17 | 497 |

**Table 4B** provides exemplary targeting domains for knocking out the *MYOC* gene selected according to the second tier parameters and are selected based on the presence of a 5' G close proximity to the start codon (located in exon 1). In an embodiment, the targeting domain is the exact complement of the target domain. Any of the targeting domains in the table can be used with a *S. pyogenes* Cas9 molecule that gives double stranded cleavage. Any of the targeting domains in the table can be used with a *S. pyogenes* Cas9 single-stranded break nucleases (nickases). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 4B**

| **2nd Tier** | selected based on the presence of a 5' G and close proximity to the start codon (gRNAs located in exon1) | | | |
|---|---|---|---|---|
| **gRNA Name** | **DNA Strand** | **Targeting Domain** | **Target Site Length** | **SEQ ID NO** |
| myoC-92 | - | GCUGCUGCUUCUGGCCUGCC | 20 | 498 |
| myoC-94 | - | GGCCUGCCUGGUGUGGGAUG | 20 | 499 |
| myoC-95 | - | GCCUGCCUGGUGUGGGAUGU | 20 | 500 |
| myoC-96 | - | GGGCCAGGACAGCUCAGCUC | 20 | 501 |
| myoC-97 | - | GGACCAGGCUGCCAGGCCCC | 20 | 502 |
| myoC-98 | - | GGCCCCAGGAGACCCAGGAG | 20 | 503 |
| myoC-99 | - | GACCCAGGAGGGGCUGCAGA | 20 | 504 |
| myoC-100 | - | GGAGGGGCUGCAGAGGGAGC | 20 | 505 |
| myoC-101 | - | GAGGGGCUGCAGAGGGAGCU | 20 | 506 |
| myoC-102 | - | GGGAGCUGGGCACCCUGAGG | 20 | 507 |
| myoC-103 | - | GGAGCUGGGCACCCUGAGGC | 20 | 508 |
| myoC-104 | - | GGGCACCCUGAGGCGGGAGC | 20 | 509 |
| myoC-105 | - | GAGGCGGGAGCGGGACCAGC | 20 | 510 |
| myoC-107 | - | GAGGUUGGAAAGCAGCAGCC | 20 | 511 |
| myoC-109 | - | GCAGCAGCCAGGAGGUAGCA | 20 | 512 |
| myoC-110 | - | GGAGGUAGCAAGGCUGAGAA | 20 | 513 |
| myoC-111 | - | GAGGUAGCAAGGCUGAGAAG | 20 | 514 |
| myoC-113 | + | GCUGCUGCUUUCCAACCUCC | 20 | 515 |
| myoC-115 | + | GUCUCGGAGGAGGUUGCUGU | 20 | 516 |
| myoC-118 | + | GCUCCCUCUGCAGCCCCUCC | 20 | 517 |
| myoC-119 | + | GCAGCCCCUCCUGGGUCUCC | 20 | 518 |
| myoC-120 | + | GGGCCUGGCAGCCUGGUCCA | 20 | 519 |
| myoC-121 | + | GGUCCAAGGUCAAUUGGUGG | 20 | 520 |
| myoC-122 | + | GGAGCUGAGUCGAGCUUUGG | 20 | 521 |
| myoC-125 | | GACAUGGCCUGGCUCUGCUC | 20 | 522 |
| myoC-126 | + | GCAGCUGGAUUCAUUGGGAC | 20 | 523 |
| myoC-128 | + | GGCAGGCCAGAAGCAGCAGC | 20 | 524 |
| myoC-130 | - | GCUGCUUCUGGCCUGCC | 17 | 525 |
| myoC-131 | - | GCCUGGUGUGGGAUGUG | 17 | 526 |
| myoC-132 | - | GACAGCUCAGCUCAGGA | 17 | 527 |
| myoC-133 | - | GCCCCAGGAGACCCAGG | 17 | 528 |
| myoC-134 | - | GGGGCUGCAGAGGGAGC | 17 | 529 |
| myoC-135 | - | GGGCUGCAGAGGGAGCU | 17 | 530 |
| myoC-136 | - | GGGAGCUGGGCACCCUG | 17 | 531 |
| myoC-137 | - | GCUGGGCACCCUGAGGC | 17 | 532 |
| myoC-138 | - | GCACCCUGAGGCGGGAG | 17 | 533 |
| myoC-139 | - | GCGGGAGCGGGACCAGC | 17 | 534 |
| myoC-140 | - | GCAAGAAAAUGAGAAUC | 17 | 535 |
| myoC-141 | - | GAAUCUGGCCAGGAGGU | 17 | 536 |
| myoC-142 | - | GUUGGAAAGCAGCAGCC | 17 | 537 |
| myoC-143 | - | GGAAAGCAGCAGCCAGG | 17 | 538 |
| myoC-144 | - | GCAGCCAGGAGGUAGCA | 17 | 539 |
| myoC-145 | - | GGUAGCAAGGCUGAGAA | 17 | 540 |
| myoC-146 | - | GUAGCAAGGCUGAGAAG | 17 | 541 |
| myoC-150 | + | GCAGUGUCUCGGGUCUG | 17 | 542 |
| myoC-151 | + | GCUGCUUUCCAACCUCC | 17 | 543 |
| myoC-153 | + | GGCAGUCUCCAACUCUC | 17 | 544 |
| myoC-154 | + | GGUCCCGCUCCCGCCUC | 17 | 545 |
| myoC-155 | + | GUCCCGCUCCCGCCUCA | 17 | 546 |
| myoC-156 | + | GCCCCUCCUGGGUCUCC | 17 | 547 |
| myoC-158 | + | GGUGGAGGAGGCUCUCC | 17 | 548 |
| myoC-159 | + | GUGGAGGAGGCUCUCCA | 17 | 549 |
| myoC-162 | + | GAGCUUUGGUGGCCUCC | 17 | 550 |
| myoC-164 | + | GGAUGACUGACAUGGCC | 17 | 551 |
| myoC-165 | + | GCUCUGCUCUGGGCAGC | 17 | 552 |
| myoC-166 | + | GGGCAGCUGGAUUCAUU | 17 | 553 |
| myoC-168 | + | GGGACUGGCCACACUGA | 17 | 554 |

**Table 4C** provides exemplary targeting domains for knocking out the *MYOC* gene selected according to the third tier parameters and are selected to fall within the coding sequence (exon 1, 2 or 3 of the *MYOC* gene). In an embodiment, the targeting domain is the exact complement of the target domain. Any of the targeting domains in the table can be used with a *S. pyogenes* Cas9 molecule that gives double stranded cleavage. Any of the targeting domains in the table can be used with a *S. pyogenes* Cas9 single-stranded break nucleases (nickases). In an embodiment, dual targeting is used to create two nicks on opposite DNA strands by using *S. pyogenes* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 4C**

| **3rd Tier** | Anywhere within coding sequence, does not require 5' G | | | | |
|---|---|---|---|---|---|
| **gRNA Name** | **DNA Strand** | **Targeting Domain** | **Target Site Length** | **Exon** | **SEQ ID NO** |
| myoC-169 | - | UGUGCACGUUGCUGCAGCUU | 20 | 1 | 555 |
| myoC-170 | - | AGCUGUCCAGCUGCUGCUUC | 20 | 1 | 556 |
| myoC-171 | - | UGCUUCUGGCCUGCCUGGUG | 20 | 1 | 557 |
| myoC-172 | - | CCUGCCUGGUGUGGGAUGUG | 20 | 1 | 558 |
| myoC-173 | - | CUGCCUGGUGUGGGAUGUGG | 20 | 1 | 559 |
| myoC-174 | - | UGGUGUGGGAUGUGGGGGCC | 20 | 1 | 560 |
| myoC-175 | - | CAGGACAGCUCAGCUCAGGA | 20 | 1 | 561 |
| myoC-176 | - | AGGAAGGCCAAUGACCAGAG | 20 | 1 | 562 |
| myoC-177 | - | AUGCCAGUAUACCUUCAGUG | 20 | 1 | 563 |
| myoC-178 | - | CAGCUGCCCAGAGCAGAGCC | 20 | 1 | 564 |
| myoC-179 | - | CAGCACCCAACGCUUAGACC | 20 | 1 | 565 |
| myoC-180 | - | CACCCAACGCUUAGACCUGG | 20 | 1 | 566 |
| myoC-181 | - | CAAAGCUCGACUCAGCUCCC | 20 | 1 | 567 |
| myoC-182 | - | CCUCCUCCACCAAUUGACCU | 20 | 1 | 568 |
| myoC-183 | - | CCACCAAUUGACCUUGGACC | 20 | 1 | 569 |
| myoC-184 | - | UGACCUUGGACCAGGCUGCC | 20 | 1 | 570 |
| myoC-185 | - | UGCCAGGCCCCAGGAGACCC | 20 | 1 | 571 |
| myoC-186 | - | CAGGCCCCAGGAGACCCAGG | 20 | 1 | 572 |
| myoC-187 | - | AGGCCCCAGGAGACCCAGGA | 20 | 1 | 573 |
| myoC-188 | - | AGACCCAGGAGGGGCUGCAG | 20 | 1 | 574 |
| myoC-189 | - | AGAGGGAGCUGGGCACCCUG | 20 | 1 | 575 |
| myoC-190 | - | UGGGCACCCUGAGGCGGGAG | 20 | 1 | 576 |
| myoC-191 | - | CCUCCGAGACAAGUCAGUUC | 20 | 1 | 577 |
| myoC-192 | - | CCGAGACAAGUCAGUUCUGG | 20 | 1 | 578 |
| myoC-193 | - | AGGAAGAGAAGAAGCGACUA | 20 | 1 | 579 |
| myoC-194 | - | AAGGCAAGAAAAUGAGAAUC | 20 | 1 | 580 |
| myoC-195 | - | AAGAAAAUGAGAAUCUGGCC | 20 | 1 | 581 |
| myoC-196 | - | AAAAUGAGAAUCUGGCCAGG | 20 | 1 | 582 |
| myoC-197 | - | UGAGAAUCUGGCCAGGAGGU | 20 | 1 | 583 |
| myoC-198 | - | AGGAGGUAGCAAGGCUGAGA | 20 | 1 | 584 |
| myoC-199 | - | CCCAGACCCGAGACACUGCU | 20 | 1 | 585 |
| myoC-200 | - | CCAGACCCGAGACACUGCUC | 20 | 1 | 586 |
| myoC-201 | - | ACUGCUCGGGCUGUGCCACC | 20 | 1 | 587 |
| myoC-202 | + | CACAGCCCGAGCAGUGUCUC | 20 | 1 | 588 |
| myoC-203 | + | CCCGAGCAGUGUCUCGGGUC | 20 | 1 | 589 |
| myoC-204 | + | CCGAGCAGUGUCUCGGGUCU | 20 | 1 | 590 |
| myoC-205 | + | CGAGCAGUGUCUCGGGUCUG | 20 | 1 | 591 |
| myoC-206 | + | UGUCUCGGGUCUGGGGACAC | 20 | 1 | 592 |
| myoC-207 | + | UUCUCAGCCUUGCUACCUCC | 20 | 1 | 593 |
| myoC-208 | + | CCUCCAGAACUGACUUGUCU | 20 | 1 | 594 |
| myoC-209 | + | CCAGAACUGACUUGUCUCGG | 20 | 1 | 595 |
| myoC-210 | + | CAGUCUCCAACUCUCUGGUU | 20 | 1 | 596 |
| myoC-211 | + | AGUCUCCAACUCUCUGGUUU | 20 | 1 | 597 |
| myoC-212 | + | CUCUGGUUUGGGUUUCCAGC | 20 | 1 | 598 |
| myoC-213 | + | CUGGUCCCGCUCCCGCCUCA | 20 | 1 | 599 |
| myoC-214 | + | CUCCCUCUGCAGCCCCUCCU | 20 | 1 | 600 |
| myoC-215 | + | CAGCCCCUCCUGGGUCUCCU | 20 | 1 | 601 |
| myoC-216 | + | AGCCCCUCCUGGGUCUCCUG | 20 | 1 | 602 |
| myoC-217 | + | CUCCUGGGUCUCCUGGGGCC | 20 | 1 | 603 |
| myoC-218 | + | UCUCCUGGGGCCUGGCAGCC | 20 | 1 | 604 |
| myoC-219 | + | CAGCCUGGUCCAAGGUCAAU | 20 | 1 | 605 |
| myoC-220 | + | CCUGGUCCAAGGUCAAUUGG | 20 | 1 | 606 |
| myoC-221 | + | CCAAGGUCAAUUGGUGGAGG | 20 | 1 | 607 |
| myoC-222 | + | AUUGGUGGAGGAGGCUCUCC | 20 | 1 | 608 |
| myoC-223 | + | UUGGUGGAGGAGGCUCUCCA | 20 | 1 | 609 |
| myoC-224 | + | CAGGGAGCUGAGUCGAGCUU | 20 | 1 | 610 |
| myoC-225 | + | UGGCCUCCAGGUCUAAGCGU | 20 | 1 | 611 |
| myoC-226 | + | UGCUGUCUCUCUGUAAGUUA | 20 | 1 | 612 |
| myoC-227 | + | UAAGUUAUGGAUGACUGACA | 20 | 1 | 613 |
| myoC-228 | + | UAUGGAUGACUGACAUGGCC | 20 | 1 | 614 |
| myoC-229 | + | ACAUGGCCUGGCUCUGCUCU | 20 | 1 | 615 |
| myoC-230 | + | CUGGCUCUGCUCUGGGCAGC | 20 | 1 | 616 |
| myoC-231 | + | CUCUGGGCAGCUGGAUUCAU | 20 | 1 | 617 |
| myoC-232 | + | UCUGGGCAGCUGGAUUCAUU | 20 | 1 | 618 |
| myoC-233 | + | AUUGGGACUGGCCACACUGA | 20 | 1 | 619 |
| myoC-234 | + | UGGCCACACUGAAGGUAUAC | 20 | 1 | 620 |
| myoC-235 | + | CACUGAAGGUAUACUGGCAU | 20 | 1 | 621 |
| myoC-236 | + | UAUACUGGCAUCGGCCACUC | 20 | 1 | 622 |
| myoC-237 | + | CUUCCUGAGCUGAGCUGUCC | 20 | 1 | 623 |
| myoC-238 | + | UGGCCCCCACAUCCCACACC | 20 | 1 | 624 |
| myoC-239 | + | CCCCACAUCCCACACCAGGC | 20 | 1 | 625 |
| myoC-240 | + | AGAAGCAGCAGCUGGACAGC | 20 | 1 | 626 |
| myoC-241 | + | AGCUGGACAGCUGGCAUCUC | 20 | 1 | 627 |
| myoC-242 | - | CACGUUGCUGCAGCUUU | 17 | 1 | 628 |
| myoC-243 | - | UGUCCAGCUGCUGCUUC | 17 | 1 | 629 |
| myoC-244 | - | UUCUGGCCUGCCUGGUG | 17 | 1 | 630 |
| myoC-245 | - | UCUGGCCUGCCUGGUGU | 17 | 1 | 631 |
| myoC-246 | - | CUGCCUGGUGUGGGAUG | 17 | 1 | 632 |
| myoC-247 | - | UGCCUGGUGUGGGAUGU | 17 | 1 | 633 |
| myoC-248 | - | CCUGGUGUGGGAUGUGG | 17 | 1 | 634 |
| myoC-249 | - | UGUGGGAUGUGGGGGCC | 17 | 1 | 635 |
| myoC-250 | - | CCAGGACAGCUCAGCUC | 17 | 1 | 636 |
| myoC-251 | - | AAGGCCAAUGACCAGAG | 17 | 1 | 637 |
| myoC-252 | - | CCAGUAUACCUUCAGUG | 17 | 1 | 638 |
| myoC-253 | - | CUGCCCAGAGCAGAGCC | 17 | 1 | 639 |
| myoC-254 | - | CACCCAACGCUUAGACC | 17 | 1 | 640 |
| myoC-255 | - | CCAACGCUUAGACCUGG | 17 | 1 | 641 |
| myoC-256 | - | AGCUCGACUCAGCUCCC | 17 | 1 | 642 |
| myoC-257 | - | CCUCCACCAAUUGACCU | 17 | 1 | 643 |
| myoC-258 | - | CCAAUUGACCUUGGACC | 17 | 1 | 644 |
| myoC-259 | - | CCUUGGACCAGGCUGCC | 17 | 1 | 645 |
| myoC-260 | - | CCAGGCUGCCAGGCCCC | 17 | 1 | 646 |
| myoC-261 | - | CAGGCCCCAGGAGACCC | 17 | 1 | 647 |
| myoC-262 | - | CCCCAGGAGACCCAGGA | 17 | 1 | 648 |
| myoC-263 | - | CCCAGGAGACCCAGGAG | 17 | 1 | 649 |
| myoC-264 | - | CCCAGGAGGGGCUGCAG | 17 | 1 | 650 |
| myoC-265 | - | CCAGGAGGGGCUGCAGA | 17 | 1 | 651 |
| myoC-266 | - | AGCUGGGCACCCUGAGG | 17 | 1 | 652 |
| myoC-267 | - | CACCCUGAGGCGGGAGC | 17 | 1 | 653 |
| myoC-268 | - | AACCCAAACCAGAGAGU | 17 | 1 | 654 |
| myoC-269 | - | CCGAGACAAGUCAGUUC | 17 | 1 | 655 |
| myoC-270 | - | AGACAAGUCAGUUCUGG | 17 | 1 | 656 |
| myoC-271 | - | AAGAGAAGAAGCGACUA | 17 | 1 | 657 |
| myoC-272 | - | AAAAUGAGAAUCUGGCC | 17 | 1 | 658 |
| myoC-273 | - | AUGAGAAUCUGGCCAGG | 17 | 1 | 659 |
| myoC-274 | - | AGGUAGCAAGGCUGAGA | 17 | 1 | 660 |
| myoC-275 | - | AGACCCGAGACACUGCU | 17 | 1 | 661 |
| myoC-276 | + | CAGCCCGAGCAGUGUCU | 17 | 1 | 662 |
| myoC-277 | + | AGCCCGAGCAGUGUCUC | 17 | 1 | 663 |
| myoC-278 | + | AGCAGUGUCUCGGGUCU | 17 | 1 | 664 |
| myoC-279 | + | CUCGGGUCUGGGGACAC | 17 | 1 | 665 |
| myoC-280 | + | UCAGCCUUGCUACCUCC | 17 | 1 | 666 |
| myoC-281 | + | CCAGAACUGACUUGUCU | 17 | 1 | 667 |
| myoC-282 | + | CUGACUUGUCUCGGAGG | 17 | 1 | 668 |
| myoC-283 | + | UCGGAGGAGGUUGCUGU | 17 | 1 | 669 |
| myoC-284 | + | UCUCCAACUCUCUGGUU | 17 | 1 | 670 |
| myoC-285 | + | CUCCAACUCUCUGGUUU | 17 | 1 | 671 |
| myoC-286 | + | UGGUUUGGGUUUCCAGC | 17 | 1 | 672 |
| myoC-287 | + | CCCUCUGCAGCCCCUCC | 17 | 1 | 673 |
| myoC-288 | + | CCUCUGCAGCCCCUCCU | 17 | 1 | 674 |
| myoC-289 | + | CCCCUCCUGGGUCUCCU | 17 | 1 | 675 |
| myoC-290 | + | CCCUCCUGGGUCUCCUG | 17 | 1 | 676 |
| myoC-291 | + | CUGGGUCUCCUGGGGCC | 17 | 1 | 677 |
| myoC-292 | + | CCUGGGGCCUGGCAGCC | 17 | 1 | 678 |
| myoC-293 | + | CCUGGCAGCCUGGUCCA | 17 | 1 | 679 |
| myoC-294 | + | CCUGGUCCAAGGUCAAU | 17 | 1 | 680 |
| myoC-295 | + | CCAAGGUCAAUUGGUGG | 17 | 1 | 681 |
| myoC-296 | + | AGGUCAAUUGGUGGAGG | 17 | 1 | 682 |
| myoC-297 | + | CCUCCAGGUCUAAGCGU | 17 | 1 | 683 |
| myoC-298 | + | CUCCAGGUCUAAGCGUU | 17 | 1 | 684 |
| myoC-299 | + | UGUCUCUCUGUAAGUUA | 17 | 1 | 685 |
| myoC-300 | + | AUGGCCUGGCUCUGCUC | 17 | 1 | 686 |
| myoC-301 | + | UGGCCUGGCUCUGCUCU | 17 | 1 | 687 |
| myoC-302 | + | UGGGCAGCUGGAUUCAU | 17 | 1 | 688 |
| myoC-303 | + | CCACACUGAAGGUAUAC | 17 | 1 | 689 |
| myoC-304 | + | UGAAGGUAUACUGGCAU | 17 | 1 | 690 |
| myoC-305 | + | ACUGGCAUCGGCCACUC | 17 | 1 | 691 |
| myoC-306 | + | UCGGCCACUCUGGUCAU | 17 | 1 | 692 |
| myoC-307 | + | CCUGAGCUGAGCUGUCC | 17 | 1 | 693 |
| myoC-308 | + | CCCCCACAUCCCACACC | 17 | 1 | 694 |
| myoC-309 | + | CACAUCCCACACCAGGC | 17 | 1 | 695 |
| myoC-310 | + | AGGCCAGAAGCAGCAGC | 17 | 1 | 696 |
| myoC-311 | + | AGCAGCAGCUGGACAGC | 17 | 1 | 697 |
| myoC-312 | + | UGGACAGCUGGCAUCUC | 17 | 1 | 698 |
| myoC-313 | - | CUUUUAAUGCAGUUUCUACG | 20 | 2 | 699 |
| myoC-314 | - | UGCAGUUUCUACGUGGAAUU | 20 | 2 | 700 |
| myoC-315 | - | UACGUGGAAUUUGGACACUU | 20 | 2 | 701 |
| myoC-316 | - | UUUGGACACUUUGGCCUUCC | 20 | 2 | 702 |
| myoC-317 | - | UCCUGCUUCCCGAAUUUUGA | 20 | 2 | 703 |
| myoC-318 | - | AUUUUGAAGGAGAGCCCAUC | 20 | 2 | 704 |
| myoC-319 | - | AGAGCCCAUCUGGCUAUCUC | 20 | 2 | 705 |
| myoC-320 | - | CCAUCUGGCUAUCUCAGGAG | 20 | 2 | 706 |
| myoC-321 | - | UGGCUAUCUCAGGAGUGGAG | 20 | 2 | 707 |
| myoC-322 | - | GGCUAUCUCAGGAGUGGAGA | 20 | 2 | 708 |
| myoC-323 | - | AGGAGUGGAGAGGGAGACAC | 20 | 2 | 709 |
| myoC-324 | + | GAAGAAACUUAACUUCAUAC | 20 | 2 | 710 |
| myoC-325 | + | CCACUCCUGAGAUAGCCAGA | 20 | 2 | 711 |
| myoC-326 | + | CACUCCUGAGAUAGCCAGAU | 20 | 2 | 712 |
| myoC-327 | + | AUGGGCUCUCCUUCAAAAUU | 20 | 2 | 713 |
| myoC-328 | + | UGGGCUCUCCUUCAAAAUUC | 20 | 2 | 714 |
| myoC-329 | + | UCCUUCAAAAUUCGGGAAGC | 20 | 2 | 715 |
| myoC-330 | + | AGCAGGAACUUCAGUUAGCU | 20 | 2 | 716 |
| myoC-331 | + | UUAGCUCGGACUUCAGUUCC | 20 | 2 | 717 |
| myoC-332 | + | CUCGGACUUCAGUUCCUGGA | 20 | 2 | 718 |
| myoC-333 | - | UUAAUGCAGUUUCUACG | 17 | 2 | 719 |
| myoC-334 | - | AGUUUCUACGUGGAAUU | 17 | 2 | 720 |
| myoC-335 | - | GUGGAAUUUGGACACUU | 17 | 2 | 721 |
| myoC-336 | - | GGACACUUUGGCCUUCC | 17 | 2 | 722 |
| myoC-337 | - | UGCUUCCCGAAUUUUGA | 17 | 2 | 723 |
| myoC-338 | - | UUGAAGGAGAGCCCAUC | 17 | 2 | 724 |
| myoC-339 | - | GCCCAUCUGGCUAUCUC | 17 | 2 | 725 |
| myoC-340 | - | UCUGGCUAUCUCAGGAG | 17 | 2 | 726 |
| myoC-341 | - | CUAUCUCAGGAGUGGAG | 17 | 2 | 727 |
| myoC-342 | - | UAUCUCAGGAGUGGAGA | 17 | 2 | 728 |
| myoC-343 | - | AGUGGAGAGGGAGACAC | 17 | 2 | 729 |
| myoC-344 | + | GAAACUUAACUUCAUAC | 17 | 2 | 730 |
| myoC-345 | + | CUCCUGAGAUAGCCAGA | 17 | 2 | 731 |
| myoC-346 | + | UCCUGAGAUAGCCAGAU | 17 | 2 | 732 |
| myoC-347 | + | GGCUCUCCUUCAAAAUU | 17 | 2 | 733 |
| myoC-348 | + | GCUCUCCUUCAAAAUUC | 17 | 2 | 734 |
| myoC-349 | + | UUCAAAAUUCGGGAAGC | 17 | 2 | 735 |
| myoC-350 | + | AGGAACUUCAGUUAGCU | 17 | 2 | 736 |
| myoC-351 | + | GCUCGGACUUCAGUUCC | 17 | 2 | 737 |
| myoC-352 | + | GGACUUCAGUUCCUGGA | 17 | 2 | 738 |
| myoC-353 | - | UUUCUGAAUUUACCAGGAUG | 20 | 3 | 739 |
| myoC-354 | - | CAGGAUGUGGAGAACUAGUU | 20 | 3 | 740 |
| myoC-355 | - | AGGAUGUGGAGAACUAGUUU | 20 | 3 | 741 |
| myoC-356 | - | UGUGGAGAACUAGUUUGGGU | 20 | 3 | 742 |
| myoC-357 | - | AGAACAGCAGAAACAAUUAC | 20 | 3 | 743 |
| myoC-358 | - | GAAACAAUUACUGGCAAGUA | 20 | 3 | 744 |
| myoC-359 | - | UUACUGGCAAGUAUGGUGUG | 20 | 3 | 745 |
| myoC-360 | - | GCCCACCUACCCCUACACCC | 20 | 3 | 746 |
| myoC-361 | - | CCUACACCCAGGAGACCACG | 20 | 3 | 747 |
| myoC-362 | - | ACGUGGAGAAUCGACACAGU | 20 | 3 | 748 |
| myoC-363 | - | GAGAAUCGACACAGUUGGCA | 20 | 3 | 749 |
| myoC-364 | - | AGUUGGCACGGAUGUCCGCC | 20 | 3 | 750 |
| myoC-365 | - | CCUCAUCAGCCAGUUUAUGC | 20 | 3 | 751 |
| myoC-366 | - | CUCAUCAGCCAGUUUAUGCA | 20 | 3 | 752 |
| myoC-367 | - | UAUGCAGGGCUACCCUUCUA | 20 | 3 | 753 |
| myoC-368 | - | CUAAGGUUCACAUACUGCCU | 20 | 3 | 754 |
| myoC-369 | - | UCACAUACUGCCUAGGCCAC | 20 | 3 | 755 |
| myoC-370 | - | GCCUAGGCCACUGGAAAGCA | 20 | 3 | 756 |
| myoC-371 | - | CCUAGGCCACUGGAAAGCAC | 20 | 3 | 757 |
| myoC-372 | - | ACUGGAAAGCACGGGUGCUG | 20 | 3 | 758 |
| myoC-373 | - | CACGGGUGCUGUGGUGUACU | 20 | 3 | 759 |
| myoC-374 | - | ACGGGUGCUGUGGUGUACUC | 20 | 3 | 760 |
| myoC-375 | - | CGGGUGCUGUGGUGUACUCG | 20 | 3 | 761 |
| myoC-376 | - | CUCGGGGAGCCUCUAUUUCC | 20 | 3 | 762 |
| myoC-377 | - | UCGGGGAGCCUCUAUUUCCA | 20 | 3 | 763 |
| myoC-1 | - | GCUGAAUACCGAGACAGUGA | 20 | 3 | 398 |
| myoC-2 | - | CGAGACAGUGAAGGCUGAGA | 20 | 3 | 405 |
| myoC-3 | - | AAGGCUGAGAAGGAAAUCCC | 20 | 3 | 406 |
| myoC-4 | - | GAGAAGGAAAUCCCUGGAGC | 20 | 3 | 399 |
| myoC-5 | - | AUCCCUGGAGCUGGCUACCA | 20 | 3 | 407 |
| myoC-6 | - | ACGGACAGUUCCCGUAUUCU | 20 | 3 | 408 |
| myoC-7 | - | CGGACAGUUCCCGUAUUCUU | 20 | 3 | 409 |
| myoC-385 | - | GGACAGUUCCCGUAUUCUUG | 20 | 3 | 764 |
| myoC-9 | - | CAGUUCCCGUAUUCUUGGGG | 20 | 3 | 410 |
| myoC-10 | - | GUAUUCUUGGGGUGGCUACA | 20 | 3 | 388 |
| myoC-11 | - | UGGCUACACGGACAUUGACU | 20 | 3 | 411 |
| myoC-12 | - | CACGGACAUUGACUUGGCUG | 20 | 3 | 412 |
| myoC-13 | - | GACUUGGCUGUGGAUGAAGC | 20 | 3 | 400 |
| myoC-14 | - | CUGUGGAUGAAGCAGGCCUC | 20 | 3 | 413 |
| myoC-15 | - | UGUGGAUGAAGCAGGCCUCU | 20 | 3 | 414 |
| myoC-16 | - | GGUCAUUUACAGCACCGAUG | 20 | 3 | 389 |
| myoC-17 | - | UACAGCACCGAUGAGGCCAA | 20 | 3 | 415 |
| myoC-395 | - | CAAACUGAACCCAGAGAAUC | 20 | 3 | 765 |
| myoC-396 | - | AUCUGGAACUCGAACAAACC | 20 | 3 | 766 |
| myoC-397 | - | UCUGGAACUCGAACAAACCU | 20 | 3 | 767 |
| myoC-398 | - | GCCAAUGCCUUCAUCAUCUG | 20 | 3 | 768 |
| myoC-53 | - | GUCAACUUUGCUUAUGACAC | 20 | 3 | 439 |
| myoC-54 | - | UUUGCUUAUGACACAGGCAC | 20 | 3 | 453 |
| myoC-55 | - | CAUGAUUGACUACAACCCCC | 20 | 3 | 454 |
| myoC-56 | - | UGGAGAAGAAGCUCUUUGCC | 20 | 3 | 455 |
| myoC-57 | - | GGAGAAGAAGCUCUUUGCCU | 20 | 3 | 440 |
| myoC-58 | - | UGCCUGGGACAACUUGAACA | 20 | 3 | 456 |
| myoC-405 | - | GAAAAGCCUCCAAGCUGUAC | 20 | 3 | 769 |
| myoC-406 | - | CCUCCAAGCUGUACAGGCAA | 20 | 3 | 770 |
| myoC-407 | - | GCUGUACAGGCAAUGGCAGA | 20 | 3 | 771 |
| myoC-408 | - | AAUGGCAGAAGGAGAUGCUC | 20 | 3 | 772 |
| myoC-409 | - | AUGGCAGAAGGAGAUGCUCA | 20 | 3 | 773 |
| myoC-410 | - | AAGGAGAUGCUCAGGGCUCC | 20 | 3 | 774 |
| myoC-411 | - | AGGAGAUGCUCAGGGCUCCU | 20 | 3 | 775 |
| myoC-412 | - | GGAGAUGCUCAGGGCUCCUG | 20 | 3 | 776 |
| myoC-413 | - | GAGAUGCUCAGGGCUCCUGG | 20 | 3 | 777 |
| myoC-414 | - | AGAUGCUCAGGGCUCCUGGG | 20 | 3 | 778 |
| myoC-415 | - | UCAGGGCUCCUGGGGGGAGC | 20 | 3 | 779 |
| myoC-416 | - | UCCUGGGGGGAGCAGGCUGA | 20 | 3 | 780 |
| myoC-417 | - | CCUGGGGGGAGCAGGCUGAA | 20 | 3 | 781 |
| myoC-418 | - | GAAGGGAGAGCCAGCCAGCC | 20 | 3 | 782 |
| myoC-419 | - | AAGGGAGAGCCAGCCAGCCA | 20 | 3 | 783 |
| myoC-420 | - | GAGCCAGCCAGCCAGGGCCC | 20 | 3 | 784 |
| myoC-421 | + | CCCUUCAGCCUGCUCCCCCC | 20 | 3 | 785 |
| myoC-422 | + | CUGCCAUUGCCUGUACAGCU | 20 | 3 | 786 |
| myoC-423 | + | CCAUUGCCUGUACAGCUUGG | 20 | 3 | 787 |
| myoC-59 | + | CUUGGAGGCUUUUCACAUCU | 20 | 3 | 457 |
| myoC-60 | + | GACCAUGUUCAAGUUGUCCC | 20 | 3 | 441 |
| myoC-61 | + | AGGCAAAGAGCUUCUUCUCC | 20 | 3 | 458 |
| myoC-62 | + | GGCAAAGAGCUUCUUCUCCA | 20 | 3 | 448 |
| myoC-63 | + | GCAAAGAGCUUCUUCUCCAG | 20 | 3 | 442 |
| myoC-64 | + | CAAAGAGCUUCUUCUCCAGG | 20 | 3 | 459 |
| myoC-65 | + | UCAUGCUGCUGUACUUAUAG | 20 | 3 | 460 |
| myoC-66 | + | UACUUAUAGCGGUUCUUGAA | 20 | 3 | 461 |
| myoC-67 | + | ACUUAUAGCGGUUCUUGAAU | 20 | 3 | 462 |
| myoC-68 | + | AUAGCGGUUCUUGAAUGGGA | 20 | 3 | 443 |
| myoC-69 | + | GGUUCUUGAAUGGGAUGGUC | 20 | 3 | 449 |
| myoC-70 | + | GUUCUUGAAUGGGAUGGUCA | 20 | 3 | 450 |
| myoC-71 | + | UGUGUCAUAAGCAAAGUUGA | 20 | 3 | 463 |
| myoC-437 | + | GUUGACGGUAGCAUCUGCUG | 20 | 3 | 788 |
| myoC-438 | + | UGCUGAGGUGUAGCUGCUGA | 20 | 3 | 789 |
| myoC-439 | + | GUAGCUGCUGACGGUGUACA | 20 | 3 | 790 |
| myoC-440 | + | CAAGGUGCCACAGAUGAUGA | 20 | 3 | 791 |
| myoC-441 | + | GCCACAGAUGAUGAAGGCAU | 20 | 3 | 792 |
| myoC-442 | + | CAUUGGCGACUGACUGCUUA | 20 | 3 | 793 |
| myoC-443 | + | CUUACGGAUGUUUGUCUCCC | 20 | 3 | 794 |
| myoC-444 | + | UGUUCGAGUUCCAGAUUCUC | 20 | 3 | 795 |
| myoC-445 | + | GUUCGAGUUCCAGAUUCUCU | 20 | 3 | 796 |
| myoC-446 | + | CAGAUUCUCUGGGUUCAGUU | 20 | 3 | 797 |
| myoC-447 | + | UCUCUGGGUUCAGUUUGGAG | 20 | 3 | 798 |
| myoC-448 | + | GUUCAGUUUGGAGAGGACAA | 20 | 3 | 799 |
| myoC-449 | + | GGAGAGGACAAUGGCACCUU | 20 | 3 | 800 |
| myoC-18 | + | AAUGGCACCUUUGGCCUCAU | 20 | 3 | 416 |
| myoC-19 | + | CGGUGCUGUAAAUGACCCAG | 20 | 3 | 417 |
| myoC-20 | + | GUGUAGCCACCCCAAGAAUA | 20 | 3 | 390 |
| myoC-21 | + | UGUAGCCACCCCAAGAAUAC | 20 | 3 | 418 |
| myoC-22 | + | AAGAAUACGGGAACUGUCCG | 20 | 3 | 419 |
| myoC-23 | + | UGUCCGUGGUAGCCAGCUCC | 20 | 3 | 420 |
| myoC-24 | + | GUCCGUGGUAGCCAGCUCCA | 20 | 3 | 391 |
| myoC-25 | + | CUUCUCAGCCUUCACUGUCU | 20 | 3 | 421 |
| myoC-26 | + | CUCAUAUCUUAUGACAGUUC | 20 | 3 | 422 |
| myoC-459 | + | CAGUUCUGGACUCAGCGCCC | 20 | 3 | 801 |
| myoC-460 | + | ACUCAGCGCCCUGGAAAUAG | 20 | 3 | 802 |
| myoC-461 | + | ACAGCACCCGUGCUUUCCAG | 20 | 3 | 803 |
| myoC-462 | + | CCCGUGCUUUCCAGUGGCCU | 20 | 3 | 804 |
| myoC-463 | + | GGCAGUAUGUGAACCUUAGA | 20 | 3 | 805 |
| myoC-464 | + | GCAGUAUGUGAACCUUAGAA | 20 | 3 | 806 |
| myoC-465 | + | AAGGGUAGCCCUGCAUAAAC | 20 | 3 | 807 |
| myoC-466 | + | CCUGCAUAAACUGGCUGAUG | 20 | 3 | 808 |
| myoC-467 | + | UGAGGUCAUACUCAAAAACC | 20 | 3 | 809 |
| myoC-468 | + | GGUCAUACUCAAAAACCUGG | 20 | 3 | 810 |
| myoC-469 | + | AACUGUGUCGAUUCUCCACG | 20 | 3 | 811 |
| myoC-470 | + | CGAUUCUCCACGUGGUCUCC | 20 | 3 | 812 |
| myoC-471 | + | GAUUCUCCACGUGGUCUCCU | 20 | 3 | 813 |
| myoC-472 | + | CCACGUGGUCUCCUGGGUGU | 20 | 3 | 814 |
| myoC-473 | + | CACGUGGUCUCCUGGGUGUA | 20 | 3 | 815 |
| myoC-474 | + | ACGUGGUCUCCUGGGUGUAG | 20 | 3 | 816 |
| myoC-475 | + | GGUCUCCUGGGUGUAGGGGU | 20 | 3 | 817 |
| myoC-476 | + | CUCCUGGGUGUAGGGGUAGG | 20 | 3 | 818 |
| myoC-477 | + | UCCUGGGUGUAGGGGUAGGU | 20 | 3 | 819 |
| myoC-478 | + | GGUGUAGGGGUAGGUGGGCU | 20 | 3 | 820 |
| myoC-479 | + | GUGUAGGGGUAGGUGGGCUU | 20 | 3 | 821 |
| myoC-480 | + | UGUAGGGGUAGGUGGGCUUG | 20 | 3 | 822 |
| myoC-481 | + | UCUGCUGUUCUCAGCGUGAG | 20 | 3 | 823 |
| myoC-482 | + | CAAACUAGUUCUCCACAUCC | 20 | 3 | 824 |
| myoC-483 | - | CAAGUUUUCAUUAAUCCAGA | 20 | 3 | 825 |
| myoC-484 | - | UUAAUCCAGAAGGAUGAACA | 20 | 3 | 826 |
| myoC-485 | - | UGGUCACCAUCUAACUAUUC | 20 | 3 | 827 |
| myoC-486 | - | UAUUCAGGAAUUGUAGUCUG | 20 | 3 | 828 |
| myoC-487 | - | AUUCAGGAAUUGUAGUCUGA | 20 | 3 | 829 |
| myoC-488 | - | UUAUCUUCUGUCAGCAUUUA | 20 | 3 | 830 |
| myoC-489 | - | UAUCUUCUGUCAGCAUUUAU | 20 | 3 | 831 |
| myoC-490 | - | GUUCAAGUUUUCUUGUGAUU | 20 | 3 | 832 |
| myoC-491 | - | UUCAAGUUUUCUUGUGAUUU | 20 | 3 | 833 |
| myoC-492 | - | UCAAGUUUUCUUGUGAUUUG | 20 | 3 | 834 |
| myoC-493 | - | GAUUUGGGGCAAAAGCUGUA | 20 | 3 | 835 |
| myoC-494 | - | CAUUGCUCUUGCAUGUUACA | 20 | 3 | 836 |
| myoC-495 | - | AUAAAAAGCAUAACUUCUAA | 20 | 3 | 837 |
| myoC-496 | - | AGGAAGCAGAAUAGCUCCUC | 20 | 3 | 838 |
| myoC-497 | - | UAAGAUGCAUUUACUACAGU | 20 | 3 | 839 |
| myoC-498 | - | UGCUUCAGAUAGAAUACAGU | 20 | 3 | 840 |
| myoC-499 | - | GCUUCAGAUAGAAUACAGUU | 20 | 3 | 841 |
| myoC-500 | + | AAUUUUAUUUCACAAUGUAA | 20 | 3 | 842 |
| myoC-501 | + | AUUUUAUUUCACAAUGUAAA | 20 | 3 | 843 |
| myoC-502 | + | AUCUUACUUAUAUUCGAUGC | 20 | 3 | 844 |
| myoC-503 | + | UUAUAUUCGAUGCUGGCCAG | 20 | 3 | 845 |
| myoC-504 | + | AGAAGUUAUGCUUUUUAUUG | 20 | 3 | 846 |
| myoC-505 | + | AUGCUUUUUAUUGUGGCUUG | 20 | 3 | 847 |
| myoC-506 | + | CAUGUAACAUGCAAGAGCAA | 20 | 3 | 848 |
| myoC-507 | + | AUGCAAGAGCAAUGGUUUUC | 20 | 3 | 849 |
| myoC-508 | + | UAAAUGCUGACAGAAGAUAA | 20 | 3 | 850 |
| myoC-509 | + | ACAAUUCCUGAAUAGUUAGA | 20 | 3 | 851 |
| myoC-510 | + | GGUGACCAUGUUCAUCCUUC | 20 | 3 | 852 |
| myoC-511 | + | CUUCUGGAUUAAUGAAAACU | 20 | 3 | 853 |
| myoC-512 | + | GGAAAGCAGUCAAAGCUGCC | 20 | 3 | 854 |
| myoC-513 | + | GAAAGCAGUCAAAGCUGCCU | 20 | 3 | 855 |
| myoC-514 | - | CUGAAUUUACCAGGAUG | 17 | 3 | 856 |
| myoC-515 | - | GAUGUGGAGAACUAGUU | 17 | 3 | 857 |
| myoC-516 | - | AUGUGGAGAACUAGUUU | 17 | 3 | 858 |
| myoC-517 | - | GGAGAACUAGUUUGGGU | 17 | 3 | 859 |
| myoC-518 | - | ACAGCAGAAACAAUUAC | 17 | 3 | 860 |
| myoC-519 | - | ACAAUUACUGGCAAGUA | 17 | 3 | 861 |
| myoC-520 | - | CUGGCAAGUAUGGUGUG | 17 | 3 | 862 |
| myoC-521 | - | CACCUACCCCUACACCC | 17 | 3 | 863 |
| myoC-522 | - | ACACCCAGGAGACCACG | 17 | 3 | 864 |
| myoC-523 | - | UGGAGAAUCGACACAGU | 17 | 3 | 865 |
| myoC-524 | - | AAUCGACACAGUUGGCA | 17 | 3 | 866 |
| myoC-525 | - | UGGCACGGAUGUCCGCC | 17 | 3 | 867 |
| myoC-526 | - | CAUCAGCCAGUUUAUGC | 17 | 3 | 868 |
| myoC-527 | - | AUCAGCCAGUUUAUGCA | 17 | 3 | 869 |
| myoC-528 | - | GCAGGGCUACCCUUCUA | 17 | 3 | 870 |
| myoC-529 | - | AGGUUCACAUACUGCCU | 17 | 3 | 871 |
| myoC-530 | - | CAUACUGCCUAGGCCAC | 17 | 3 | 872 |
| myoC-531 | - | UAGGCCACUGGAAAGCA | 17 | 3 | 873 |
| myoC-532 | - | AGGCCACUGGAAAGCAC | 17 | 3 | 874 |
| myoC-533 | - | GGAAAGCACGGGUGCUG | 17 | 3 | 875 |
| myoC-534 | - | GGGUGCUGUGGUGUACU | 17 | 3 | 876 |
| myoC-535 | - | GGUGCUGUGGUGUACUC | 17 | 3 | 877 |
| myoC-536 | - | GUGCUGUGGUGUACUCG | 17 | 3 | 878 |
| myoC-537 | - | GGGGAGCCUCUAUUUCC | 17 | 3 | 879 |
| myoC-538 | - | GGGAGCCUCUAUUUCCA | 17 | 3 | 880 |
| myoC-27 | - | GAAUACCGAGACAGUGA | 17 | 3 | 392 |
| myoC-28 | - | GACAGUGAAGGCUGAGA | 17 | 3 | 401 |
| myoC-29 | - | GCUGAGAAGGAAAUCCC | 17 | 3 | 423 |
| myoC-30 | - | AAGGAAAUCCCUGGAGC | 17 | 3 | 424 |
| myoC-31 | - | CCUGGAGCUGGCUACCA | 17 | 3 | 425 |
| myoC-544 | - | GACAGUUCCCGUAUUCU | 17 | 3 | 881 |
| myoC-33 | - | ACAGUUCCCGUAUUCUU | 17 | 3 | 426 |
| myoC-34 | - | CAGUUCCCGUAUUCUUG | 17 | 3 | 427 |
| myoC-35 | - | UUCCCGUAUUCUUGGGG | 17 | 3 | 428 |
| myoC-36 | - | UUCUUGGGGUGGCUACA | 17 | 3 | 429 |
| myoC-37 | - | CUACACGGACAUUGACU | 17 | 3 | 394 |
| myoC-38 | - | GGACAUUGACUUGGCUG | 17 | 3 | 402 |
| myoC-39 | - | UUGGCUGUGGAUGAAGC | 17 | 3 | 430 |
| myoC-40 | - | UGGAUGAAGCAGGCCUC | 17 | 3 | 431 |
| myoC-41 | - | GGAUGAAGCAGGCCUCU | 17 | 3 | 403 |
| myoC-42 | - | CAUUUACAGCACCGAUG | 17 | 3 | 432 |
| myoC-43 | - | AGCACCGAUGAGGCCAA | 17 | 3 | 433 |
| myoC-556 | - | ACUGAACCCAGAGAAUC | 17 | 3 | 882 |
| myoC-557 | - | UGGAACUCGAACAAACC | 17 | 3 | 883 |
| myoC-558 | - | GGAACUCGAACAAACCU | 17 | 3 | 884 |
| myoC-559 | - | AAUGCCUUCAUCAUCUG | 17 | 3 | 885 |
| myoC-72 | - | AACUUUGCUUAUGACAC | 17 | 3 | 464 |
| myoC-73 | - | GCUUAUGACACAGGCAC | 17 | 3 | 451 |
| myoC-562 | - | GAUUGACUACAACCCCC | 17 | 3 | 886 |
| myoC-75 | - | AGAAGAAGCUCUUUGCC | 17 | 3 | 465 |
| myoC-76 | - | GAAGAAGCUCUUUGCCU | 17 | 3 | 452 |
| myoC-77 | - | CUGGGACAACUUGAACA | 17 | 3 | 466 |
| myoC-566 | - | AAGCCUCCAAGCUGUAC | 17 | 3 | 887 |
| myoC-567 | - | CCAAGCUGUACAGGCAA | 17 | 3 | 888 |
| myoC-568 | - | GUACAGGCAAUGGCAGA | 17 | 3 | 889 |
| myoC-569 | - | GGCAGAAGGAGAUGCUC | 17 | 3 | 890 |
| myoC-570 | - | GCAGAAGGAGAUGCUCA | 17 | 3 | 891 |
| myoC-571 | - | GAGAUGCUCAGGGCUCC | 17 | 3 | 892 |
| myoC-572 | - | AGAUGCUCAGGGCUCCU | 17 | 3 | 893 |
| myoC-573 | - | GAUGCUCAGGGCUCCUG | 17 | 3 | 894 |
| myoC-574 | - | AUGCUCAGGGCUCCUGG | 17 | 3 | 895 |
| myoC-575 | - | UGCUCAGGGCUCCUGGG | 17 | 3 | 896 |
| myoC-576 | - | GGGCUCCUGGGGGGAGC | 17 | 3 | 897 |
| myoC-577 | - | UGGGGGGAGCAGGCUGA | 17 | 3 | 898 |
| myoC-578 | - | GGGGGGAGCAGGCUGAA | 17 | 3 | 899 |
| myoC-579 | - | GGGAGAGCCAGCCAGCC | 17 | 3 | 900 |
| myoC-580 | - | GGAGAGCCAGCCAGCCA | 17 | 3 | 901 |
| myoC-581 | - | CCAGCCAGCCAGGGCCC | 17 | 3 | 902 |
| myoC-582 | + | CCUGGGCCCUGGCUGGC | 17 | 3 | 903 |
| myoC-583 | + | UUCAGCCUGCUCCCCCC | 17 | 3 | 904 |
| myoC-584 | + | CCAUUGCCUGUACAGCU | 17 | 3 | 905 |
| myoC-585 | + | UUGCCUGUACAGCUUGG | 17 | 3 | 906 |
| myoC-78 | + | GGAGGCUUUUCACAUCU | 17 | 3 | 445 |
| myoC-79 | + | CAUGUUCAAGUUGUCCC | 17 | 3 | 467 |
| myoC-80 | + | CAAAGAGCUUCUUCUCC | 17 | 3 | 468 |
| myoC-81 | + | AAAGAGCUUCUUCUCCA | 17 | 3 | 469 |
| myoC-82 | + | AAGAGCUUCUUCUCCAG | 17 | 3 | 470 |
| myoC-83 | + | AGAGCUUCUUCUCCAGG | 17 | 3 | 471 |
| myoC-84 | + | UGCUGCUGUACUUAUAG | 17 | 3 | 472 |
| myoC-85 | + | UUAUAGCGGUUCUUGAA | 17 | 3 | 473 |
| myoC-86 | + | UAUAGCGGUUCUUGAAU | 17 | 3 | 474 |
| myoC-87 | + | GCGGUUCUUGAAUGGGA | 17 | 3 | 446 |
| myoC-88 | + | UCUUGAAUGGGAUGGUC | 17 | 3 | 475 |
| myoC-89 | + | CUUGAAUGGGAUGGUCA | 17 | 3 | 476 |
| myoC-90 | + | GUCAUAAGCAAAGUUGA | 17 | 3 | 447 |
| myoC-599 | + | GACGGUAGCAUCUGCUG | 17 | 3 | 907 |
| myoC-600 | + | UGAGGUGUAGCUGCUGA | 17 | 3 | 908 |
| myoC-601 | + | GCUGCUGACGGUGUACA | 17 | 3 | 909 |
| myoC-602 | + | GGUGCCACAGAUGAUGA | 17 | 3 | 910 |
| myoC-603 | + | ACAGAUGAUGAAGGCAU | 17 | 3 | 911 |
| myoC-604 | + | UGGCGACUGACUGCUUA | 17 | 3 | 912 |
| myoC-605 | + | ACGGAUGUUUGUCUCCC | 17 | 3 | 913 |
| myoC-606 | + | UCGAGUUCCAGAUUCUC | 17 | 3 | 914 |
| myoC-607 | + | CGAGUUCCAGAUUCUCU | 17 | 3 | 915 |
| myoC-608 | + | AUUCUCUGGGUUCAGUU | 17 | 3 | 916 |
| myoC-609 | + | CUGGGUUCAGUUUGGAG | 17 | 3 | 917 |
| myoC-610 | + | CAGUUUGGAGAGGACAA | 17 | 3 | 918 |
| myoC-611 | + | GAGGACAAUGGCACCUU | 17 | 3 | 919 |
| myoC-44 | + | GGCACCUUUGGCCUCAU | 17 | 3 | 404 |
| myoC-45 | + | UGCUGUAAAUGACCCAG | 17 | 3 | 434 |
| myoC-46 | + | UAGCCACCCCAAGAAUA | 17 | 3 | 395 |
| myoC-47 | + | AGCCACCCCAAGAAUAC | 17 | 3 | 435 |
| myoC-616 | + | AAUACGGGAACUGUCCG | 17 | 3 | 920 |
| myoC-49 | + | CCGUGGUAGCCAGCUCC | 17 | 3 | 436 |
| myoC-50 | + | CGUGGUAGCCAGCUCCA | 17 | 3 | 397 |
| myoC-51 | + | CUCAGCCUUCACUGUCU | 17 | 3 | 437 |
| myoC-52 | + | AUAUCUUAUGACAGUUC | 17 | 3 | 438 |
| myoC-621 | + | UUCUGGACUCAGCGCCC | 17 | 3 | 921 |
| myoC-622 | + | CAGCGCCCUGGAAAUAG | 17 | 3 | 922 |
| myoC-623 | + | GCACCCGUGCUUUCCAG | 17 | 3 | 923 |
| myoC-624 | + | GUGCUUUCCAGUGGCCU | 17 | 3 | 924 |
| myoC-625 | + | AGUAUGUGAACCUUAGA | 17 | 3 | 925 |
| myoC-626 | + | GUAUGUGAACCUUAGAA | 17 | 3 | 926 |
| myoC-627 | + | GGUAGCCCUGCAUAAAC | 17 | 3 | 927 |
| myoC-628 | + | GCAUAAACUGGCUGAUG | 17 | 3 | 928 |
| myoC-629 | + | GGUCAUACUCAAAAACC | 17 | 3 | 929 |
| myoC-630 | + | CAUACUCAAAAACCUGG | 17 | 3 | 930 |
| myoC-631 | + | UGUGUCGAUUCUCCACG | 17 | 3 | 931 |
| myoC-632 | + | UUCUCCACGUGGUCUCC | 17 | 3 | 932 |
| myoC-633 | + | UCUCCACGUGGUCUCCU | 17 | 3 | 933 |
| myoC-634 | + | CGUGGUCUCCUGGGUGU | 17 | 3 | 934 |
| myoC-635 | + | GUGGUCUCCUGGGUGUA | 17 | 3 | 935 |
| myoC-636 | + | UGGUCUCCUGGGUGUAG | 17 | 3 | 936 |
| myoC-637 | + | CUCCUGGGUGUAGGGGU | 17 | 3 | 937 |
| myoC-638 | + | CUGGGUGUAGGGGUAGG | 17 | 3 | 938 |
| myoC-639 | + | UGGGUGUAGGGGUAGGU | 17 | 3 | 939 |
| myoC-640 | + | GUAGGGGUAGGUGGGCU | 17 | 3 | 940 |
| myoC-641 | + | UAGGGGUAGGUGGGCUU | 17 | 3 | 941 |
| myoC-642 | + | AGGGGUAGGUGGGCUUG | 17 | 3 | 942 |
| myoC-643 | + | GCUGUUCUCAGCGUGAG | 17 | 3 | 943 |
| myoC-644 | + | ACUAGUUCUCCACAUCC | 17 | 3 | 944 |
| myoC-645 | - | GUUUUCAUUAAUCCAGA | 17 | 3 | 945 |
| myoC-646 | - | AUCCAGAAGGAUGAACA | 17 | 3 | 946 |
| myoC-647 | - | UCACCAUCUAACUAUUC | 17 | 3 | 947 |
| myoC-648 | - | UCAGGAAUUGUAGUCUG | 17 | 3 | 948 |
| myoC-649 | - | CAGGAAUUGUAGUCUGA | 17 | 3 | 949 |
| myoC-650 | - | UCUUCUGUCAGCAUUUA | 17 | 3 | 950 |
| myoC-651 | - | CUUCUGUCAGCAUUUAU | 17 | 3 | 951 |
| myoC-652 | - | CAAGUUUUCUUGUGAUU | 17 | 3 | 952 |
| myoC-653 | - | AAGUUUUCUUGUGAUUU | 17 | 3 | 953 |
| myoC-654 | - | AGUUUUCUUGUGAUUUG | 17 | 3 | 954 |
| myoC-655 | - | UUGGGGCAAAAGCUGUA | 17 | 3 | 955 |
| myoC-656 | - | UGCUCUUGCAUGUUACA | 17 | 3 | 956 |
| myoC-657 | - | AAAAGCAUAACUUCUAA | 17 | 3 | 957 |
| myoC-658 | - | AAGCAGAAUAGCUCCUC | 17 | 3 | 958 |
| myoC-659 | - | GAUGCAUUUACUACAGU | 17 | 3 | 959 |
| myoC-660 | - | UUCAGAUAGAAUACAGU | 17 | 3 | 960 |
| myoC-661 | - | UCAGAUAGAAUACAGUU | 17 | 3 | 961 |
| myoC-662 | + | UUUAUUUCACAAUGUAA | 17 | 3 | 962 |
| myoC-663 | + | UUAUUUCACAAUGUAAA | 17 | 3 | 963 |
| myoC-664 | + | UUACUUAUAUUCGAUGC | 17 | 3 | 964 |
| myoC-665 | + | UAUUCGAUGCUGGCCAG | 17 | 3 | 965 |
| myoC-666 | + | AGUUAUGCUUUUUAUUG | 17 | 3 | 966 |
| myoC-667 | + | CUUUUUAUUGUGGCUUG | 17 | 3 | 967 |
| myoC-668 | + | GUAACAUGCAAGAGCAA | 17 | 3 | 968 |
| myoC-669 | + | CAAGAGCAAUGGUUUUC | 17 | 3 | 969 |
| myoC-670 | + | AUGCUGACAGAAGAUAA | 17 | 3 | 970 |
| myoC-671 | + | AUUCCUGAAUAGUUAGA | 17 | 3 | 971 |
| myoC-672 | + | GACCAUGUUCAUCCUUC | 17 | 3 | 972 |
| myoC-673 | + | CUGGAUUAAUGAAAACU | 17 | 3 | 973 |
| myoC-674 | + | AAGCAGUCAAAGCUGCC | 17 | 3 | 974 |
| myoC-675 | + | AGCAGUCAAAGCUGCCU | 17 | 3 | 975 |

**Table 4D** provides exemplary targeting domains for knocking out the *MYOC* gene. In an embodiment, the targeting domain is the exact complement of the target domain. Any of the targeting domains in the table can be used with a *S*. *aureus* Cas9 molecule that gives double stranded cleavage. Any of the targeting domains in the table can be used with a *S*. *aureus* Cas9 single-stranded break nucleases (nickases). In an embodiment, dual targeting is used to create two nicks on opposite DNA strands by using *S*. *aureus* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 4D**

| **gRNA Name** | **DNA Strand** | **Targeting Domain** | **Target Site Length** | **SEQ ID NO** |
|---|---|---|---|---|
| myoC-1592 | - | AGCCUCACCAAGCCUCUGCA | 20 | 1876 |
| myoC-1593 | - | CUGUGCACGUUGCUGCAGCU | 20 | 1877 |
| myoC-1594 | - | ACGUUGCUGCAGCUUUGGGC | 20 | 1878 |
| myoC-1595 | - | CUGCUUCUGGCCUGCCUGGU | 20 | 1879 |
| myoC-171 | - | UGCUUCUGGCCUGCCUGGUG | 20 | 557 |
| myoC-1597 | - | UGGCCUGCCUGGUGUGGGAU | 20 | 1880 |
| myoC-94 | - | GGCCUGCCUGGUGUGGGAUG | 20 | 499 |
| myoC-95 | - | GCCUGCCUGGUGUGGGAUGU | 20 | 500 |
| myoC-1600 | - | CUGGUGUGGGAUGUGGGGGC | 20 | 1881 |
| myoC-1601 | - | GGGGCCAGGACAGCUCAGCU | 20 | 1882 |
| myoC-96 | - | GGGCCAGGACAGCUCAGCUC | 20 | 501 |
| myoC-1603 | - | AGCUCAGGAAGGCCAAUGAC | 20 | 1883 |
| myoC-1604 | - | CUUCAGUGUGGCCAGUCCCA | 20 | 1884 |
| myoC-1605 | - | UCCCAAUGAAUCCAGCUGCC | 20 | 1885 |
| myoC-1606 | - | AUGAAUCCAGCUGCCCAGAG | 20 | 1886 |
| myoC-1607 | - | UGUCAGUCAUCCAUAACUUA | 20 | 1887 |
| myoC-1608 | - | UCAGUCAUCCAUAACUUACA | 20 | 1888 |
| myoC-1609 | - | GCAGCACCCAACGCUUAGAC | 20 | 1889 |
| myoC-179 | - | CAGCACCCAACGCUUAGACC | 20 | 565 |
| myoC-1611 | - | CCAAAGCUCGACUCAGCUCC | 20 | 1890 |
| myoC-181 | - | CAAAGCUCGACUCAGCUCCC | 20 | 567 |
| myoC-1613 | - | AAGCUCGACUCAGCUCCCUG | 20 | 1891 |
| myoC-1614 | - | GCCUCCUCCACCAAUUGACC | 20 | 1892 |
| myoC-1615 | - | UGGACCAGGCUGCCAGGCCC | 20 | 1893 |
| myoC-97 | - | GGACCAGGCUGCCAGGCCCC | 20 | 502 |
| myoC-1617 | - | CUGCCAGGCCCCAGGAGACC | 20 | 1894 |
| myoC-185 | - | UGCCAGGCCCCAGGAGACCC | 20 | 571 |
| myoC-1619 | - | CCAGGCCCCAGGAGACCCAG | 20 | 1895 |
| myoC-186 | - | CAGGCCCCAGGAGACCCAGG | 20 | 572 |
| myoC-1621 | - | AGGAGACCCAGGAGGGGCUG | 20 | 1896 |
| myoC-1622 | - | GAGACCCAGGAGGGGCUGCA | 20 | 1897 |
| myoC-188 | - | AGACCCAGGAGGGGCUGCAG | 20 | 574 |
| myoC-99 | - | GACCCAGGAGGGGCUGCAGA | 20 | 504 |
| myoC-1625 | - | AGGAGGGGCUGCAGAGGGAG | 20 | 1898 |
| myoC-1626 | - | UGCAGAGGGAGCUGGGCACC | 20 | 1899 |
| myoC-1627 | - | AGGGAGCUGGGCACCCUGAG | 20 | 1900 |
| myoC-102 | - | GGGAGCUGGGCACCCUGAGG | 20 | 507 |
| myoC-103 | - | GGAGCUGGGCACCCUGAGGC | 20 | 508 |
| myoC-1630 | - | CUGGGCACCCUGAGGCGGGA | 20 | 1901 |
| myoC-190 | - | UGGGCACCCUGAGGCGGGAG | 20 | 576 |
| myoC-1632 | - | UGAGGCGGGAGCGGGACCAG | 20 | 1902 |
| myoC-105 | - | GAGGCGGGAGCGGGACCAGC | 20 | 510 |
| myoC-1634 | - | GACCAGCUGGAAACCCAAAC | 20 | 1903 |
| myoC-1635 | - | CCAGCUGGAAACCCAAACCA | 20 | 1904 |
| myoC-1636 | - | UGGAAACCCAAACCAGAGAG | 20 | 1905 |
| myoC-106 | - | GGAAACCCAAACCAGAGAGU | 20 | 479 |
| myoC-1638 | - | ACUGCCUACAGCAACCUCCU | 20 | 1906 |
| myoC-1639 | - | UCCUCCGAGACAAGUCAGUU | 20 | 1907 |
| myoC-191 | - | CCUCCGAGACAAGUCAGUUC | 20 | 577 |
| myoC-1641 | - | UCCGAGACAAGUCAGUUCUG | 20 | 1908 |
| myoC-192 | - | CCGAGACAAGUCAGUUCUGG | 20 | 578 |
| myoC-1643 | - | AGACAAGUCAGUUCUGGAGG | 20 | 1909 |
| myoC-1644 | - | ACAAGUCAGUUCUGGAGGAA | 20 | 1910 |
| myoC-1645 | - | AGUCAGUUCUGGAGGAAGAG | 20 | 1911 |
| myoC-1646 | - | AGAGAAGAAGCGACUAAGGC | 20 | 1912 |
| myoC-1647 | - | GAAGCGACUAAGGCAAGAAA | 20 | 1913 |
| myoC-1648 | - | AGCGACUAAGGCAAGAAAAU | 20 | 1914 |
| myoC-1649 | - | CAAGAAAAUGAGAAUCUGGC | 20 | 1915 |
| myoC-195 | - | AAGAAAAUGAGAAUCUGGCC | 20 | 581 |
| myoC-1651 | - | AUGAGAAUCUGGCCAGGAGG | 20 | 1916 |
| myoC-197 | - | UGAGAAUCUGGCCAGGAGGU | 20 | 583 |
| myoC-1653 | - | GGAGGUUGGAAAGCAGCAGC | 20 | 1917 |
| myoC-107 | - | GAGGUUGGAAAGCAGCAGCC | 20 | 511 |
| myoC-1655 | - | GCAGCCAGGAGGUAGCAAGG | 20 | 1918 |
| myoC-1656 | - | AGCCAGGAGGUAGCAAGGCU | 20 | 1919 |
| myoC-1657 | - | CAGGAGGUAGCAAGGCUGAG | 20 | 1920 |
| myoC-198 | - | AGGAGGUAGCAAGGCUGAGA | 20 | 584 |
| myoC-1659 | - | AGGGGCCAGUGUCCCCAGAC | 20 | 1921 |
| myoC-1660 | - | CCCCAGACCCGAGACACUGC | 20 | 1922 |
| myoC-1661 | - | CGGGCUGUGCCACCAGGCUC | 20 | 1923 |
| myoC-1662 | - | GGCUGUGCCACCAGGCUCCA | 20 | 1924 |
| myoC-1663 | + | AACCUCAUUGCAGAGGCUUG | 20 | 1925 |
| myoC-1664 | + | UGCACAGAAGAACCUCAUUG | 20 | 1926 |
| myoC-1665 | + | AGCUGCAGCAACGUGCACAG | 20 | 1927 |
| myoC-1666 | + | CAAAGCUGCAGCAACGUGCA | 20 | 1928 |
| myoC-1667 | + | AGGCAGGCCAGAAGCAGCAG | 20 | 1929 |
| myoC-1668 | + | ACAUCCCACACCAGGCAGGC | 20 | 1930 |
| myoC-1669 | + | UGGUCAUUGGCCUUCCUGAG | 20 | 1931 |
| myoC-1670 | + | CACUCUGGUCAUUGGCCUUC | 20 | 1932 |
| myoC-1671 | + | AUUCAUUGGGACUGGCCACA | 20 | 1933 |
| myoC-231 | + | CUCUGGGCAGCUGGAUUCAU | 20 | 617 |
| myoC-1673 | + | GCUCUGGGCAGCUGGAUUCA | 20 | 1934 |
| myoC-1674 | + | CCUGGCUCUGCUCUGGGCAG | 20 | 1935 |
| myoC-1675 | + | UGACAUGGCCUGGCUCUGCU | 20 | 1936 |
| myoC-1676 | + | CUGCUGUCUCUCUGUAAGUU | 20 | 1937 |
| myoC-1677 | + | GUGGCCUCCAGGUCUAAGCG | 20 | 1938 |
| myoC-1678 | + | AGGCUCUCCAGGGAGCUGAG | 20 | 1939 |
| myoC-1679 | + | GGAGGAGGCUCUCCAGGGAG | 20 | 1940 |
| myoC-223 | + | UUGGUGGAGGAGGCUCUCCA | 20 | 609 |
| myoC-222 | + | AUUGGUGGAGGAGGCUCUCC | 20 | 608 |
| myoC-1682 | + | AAUUGGUGGAGGAGGCUCUC | 20 | 1941 |
| myoC-121 | + | GGUCCAAGGUCAAUUGGUGG | 20 | 520 |
| myoC-1684 | + | UGGUCCAAGGUCAAUUGGUG | 20 | 1942 |
| myoC-220 | + | CCUGGUCCAAGGUCAAUUGG | 20 | 606 |
| myoC-1686 | + | GCCUGGUCCAAGGUCAAUUG | 20 | 1943 |
| myoC-119 | + | GCAGCCCCUCCUGGGUCUCC | 20 | 518 |
| myoC-1688 | + | UGCAGCCCCUCCUGGGUCUC | 20 | 1944 |
| myoC-1689 | + | AGCUCCCUCUGCAGCCCCUC | 20 | 1945 |
| myoC-1690 | + | AGCUGGUCCCGCUCCCGCCU | 20 | 1946 |
| myoC-1691 | + | GCAGUCUCCAACUCUCUGGU | 20 | 1947 |
| myoC-209 | + | CCAGAACUGACUUGUCUCGG | 20 | 595 |
| myoC-1693 | + | UCCAGAACUGACUUGUCUCG | 20 | 1948 |
| myoC-208 | + | CCUCCAGAACUGACUUGUCU | 20 | 594 |
| myoC-1695 | + | UCCUCCAGAACUGACUUGUC | 20 | 1949 |
| myoC-1696 | + | AGUCGCUUCUUCUCUUCCUC | 20 | 1950 |
| myoC-204 | + | CCGAGCAGUGUCUCGGGUCU | 20 | 590 |
| myoC-203 | + | CCCGAGCAGUGUCUCGGGUC | 20 | 589 |
| myoC-1699 | + | GCCCGAGCAGUGUCUCGGGU | 20 | 1951 |
| myoC-1700 | + | GGCACAGCCCGAGCAGUGUC | 20 | 1952 |
| myoC-1701 | + | CUGGAGCCUGGUGGCACAGC | 20 | 1953 |
| myoC-1702 | - | CUCACCAAGCCUCUGCA | 17 | 1954 |
| myoC-1703 | - | UGCACGUUGCUGCAGCU | 17 | 1955 |
| myoC-1704 | - | UUGCUGCAGCUUUGGGC | 17 | 1956 |
| myoC-1705 | - | CUUCUGGCCUGCCUGGU | 17 | 1957 |
| myoC-244 | - | UUCUGGCCUGCCUGGUG | 17 | 630 |
| myoC-1707 | - | CCUGCCUGGUGUGGGAU | 17 | 1958 |
| myoC-246 | - | CUGCCUGGUGUGGGAUG | 17 | 632 |
| myoC-247 | - | UGCCUGGUGUGGGAUGU | 17 | 633 |
| myoC-1710 | - | GUGUGGGAUGUGGGGGC | 17 | 1959 |
| myoC-1711 | - | GCCAGGACAGCUCAGCU | 17 | 1960 |
| myoC-250 | - | CCAGGACAGCUCAGCUC | 17 | 636 |
| myoC-1713 | - | UCAGGAAGGCCAAUGAC | 17 | 1961 |
| myoC-1714 | - | CAGUGUGGCCAGUCCCA | 17 | 1962 |
| myoC-1715 | - | CAAUGAAUCCAGCUGCC | 17 | 1963 |
| myoC-1716 | - | AAUCCAGCUGCCCAGAG | 17 | 1964 |
| myoC-1717 | - | CAGUCAUCCAUAACUUA | 17 | 1965 |
| myoC-1718 | - | GUCAUCCAUAACUUACA | 17 | 1966 |
| myoC-1719 | - | GCACCCAACGCUUAGAC | 17 | 1967 |
| myoC-254 | - | CACCCAACGCUUAGACC | 17 | 640 |
| myoC-1721 | - | AAGCUCGACUCAGCUCC | 17 | 1968 |
| myoC-256 | - | AGCUCGACUCAGCUCCC | 17 | 642 |
| myoC-1723 | - | CUCGACUCAGCUCCCUG | 17 | 1969 |
| myoC-1724 | - | UCCUCCACCAAUUGACC | 17 | 1970 |
| myoC-1725 | - | ACCAGGCUGCCAGGCCC | 17 | 1971 |
| myoC-260 | - | CCAGGCUGCCAGGCCCC | 17 | 646 |
| myoC-1727 | - | CCAGGCCCCAGGAGACC | 17 | 1972 |
| myoC-261 | - | CAGGCCCCAGGAGACCC | 17 | 647 |
| myoC-1729 | - | GGCCCCAGGAGACCCAG | 17 | 1973 |
| myoC-133 | - | GCCCCAGGAGACCCAGG | 17 | 528 |
| myoC-1731 | - | AGACCCAGGAGGGGCUG | 17 | 1974 |
| myoC-1732 | - | ACCCAGGAGGGGCUGCA | 17 | 1975 |
| myoC-264 | - | CCCAGGAGGGGCUGCAG | 17 | 650 |
| myoC-265 | - | CCAGGAGGGGCUGCAGA | 17 | 651 |
| myoC-1735 | - | AGGGGCUGCAGAGGGAG | 17 | 1976 |
| myoC-1736 | - | AGAGGGAGCUGGGCACC | 17 | 1977 |
| myoC-1737 | - | GAGCUGGGCACCCUGAG | 17 | 1978 |
| myoC-266 | - | AGCUGGGCACCCUGAGG | 17 | 652 |
| myoC-137 | - | GCUGGGCACCCUGAGGC | 17 | 532 |
| myoC-1740 | - | GGCACCCUGAGGCGGGA | 17 | 1979 |
| myoC-138 | - | GCACCCUGAGGCGGGAG | 17 | 533 |
| myoC-1742 | - | GGCGGGAGCGGGACCAG | 17 | 1980 |
| myoC-139 | - | GCGGGAGCGGGACCAGC | 17 | 534 |
| myoC-1744 | - | CAGCUGGAAACCCAAAC | 17 | 1981 |
| myoC-1745 | - | GCUGGAAACCCAAACCA | 17 | 1982 |
| myoC-1746 | - | AAACCCAAACCAGAGAG | 17 | 1983 |
| myoC-268 | - | AACCCAAACCAGAGAGU | 17 | 654 |
| myoC-1748 | - | GCCUACAGCAACCUCCU | 17 | 1984 |
| myoC-1749 | - | UCCGAGACAAGUCAGUU | 17 | 1985 |
| myoC-269 | - | CCGAGACAAGUCAGUUC | 17 | 655 |
| myoC-1751 | - | GAGACAAGUCAGUUCUG | 17 | 1986 |
| myoC-270 | - | AGACAAGUCAGUUCUGG | 17 | 656 |
| myoC-1753 | - | CAAGUCAGUUCUGGAGG | 17 | 1987 |
| myoC-1754 | - | AGUCAGUUCUGGAGGAA | 17 | 1988 |
| myoC-1755 | - | CAGUUCUGGAGGAAGAG | 17 | 1989 |
| myoC-1756 | - | GAAGAAGCGACUAAGGC | 17 | 1990 |
| myoC-1757 | - | GCGACUAAGGCAAGAAA | 17 | 1991 |
| myoC-1758 | - | GACUAAGGCAAGAAAAU | 17 | 1992 |
| myoC-1759 | - | GAAAAUGAGAAUCUGGC | 17 | 1993 |
| myoC-272 | - | AAAAUGAGAAUCUGGCC | 17 | 658 |
| myoC-1761 | - | AGAAUCUGGCCAGGAGG | 17 | 1994 |
| myoC-141 | - | GAAUCUGGCCAGGAGGU | 17 | 536 |
| myoC-1763 | - | GGUUGGAAAGCAGCAGC | 17 | 1995 |
| myoC-142 | - | GUUGGAAAGCAGCAGCC | 17 | 537 |
| myoC-1765 | - | GCCAGGAGGUAGCAAGG | 17 | 1996 |
| myoC-1766 | - | CAGGAGGUAGCAAGGCU | 17 | 1997 |
| myoC-1767 | - | GAGGUAGCAAGGCUGAG | 17 | 1998 |
| myoC-274 | - | AGGUAGCAAGGCUGAGA | 17 | 660 |
| myoC-1769 | - | GGCCAGUGUCCCCAGAC | 17 | 1999 |
| myoC-1770 | - | CAGACCCGAGACACUGC | 17 | 2000 |
| myoC-1771 | - | GCUGUGCCACCAGGCUC | 17 | 2001 |
| myoC-1772 | - | UGUGCCACCAGGCUCCA | 17 | 2002 |
| myoC-1773 | + | CUCAUUGCAGAGGCUUG | 17 | 2003 |
| myoC-1774 | + | ACAGAAGAACCUCAUUG | 17 | 2004 |
| myoC-1775 | + | UGCAGCAACGUGCACAG | 17 | 2005 |
| myoC-1776 | + | AGCUGCAGCAACGUGCA | 17 | 2006 |
| myoC-1777 | + | CAGGCCAGAAGCAGCAG | 17 | 2007 |
| myoC-1778 | + | UCCCACACCAGGCAGGC | 17 | 2008 |
| myoC-1779 | + | UCAUUGGCCUUCCUGAG | 17 | 2009 |
| myoC-1780 | + | UCUGGUCAUUGGCCUUC | 17 | 2010 |
| myoC-1781 | + | CAUUGGGACUGGCCACA | 17 | 2011 |
| myoC-302 | + | UGGGCAGCUGGAUUCAU | 17 | 688 |
| myoC-1783 | + | CUGGGCAGCUGGAUUCA | 17 | 2012 |
| myoC-1784 | + | GGCUCUGCUCUGGGCAG | 17 | 2013 |
| myoC-1785 | + | CAUGGCCUGGCUCUGCU | 17 | 2014 |
| myoC-1786 | + | CUGUCUCUCUGUAAGUU | 17 | 2015 |
| myoC-1787 | + | GCCUCCAGGUCUAAGCG | 17 | 2016 |
| myoC-1788 | + | CUCUCCAGGGAGCUGAG | 17 | 2017 |
| myoC-1789 | + | GGAGGCUCUCCAGGGAG | 17 | 2018 |
| myoC-159 | + | GUGGAGGAGGCUCUCCA | 17 | 549 |
| myoC-158 | + | GGUGGAGGAGGCUCUCC | 17 | 548 |
| myoC-1792 | + | UGGUGGAGGAGGCUCUC | 17 | 2019 |
| myoC-295 | + | CCAAGGUCAAUUGGUGG | 17 | 681 |
| myoC-1794 | + | UCCAAGGUCAAUUGGUG | 17 | 2020 |
| myoC-157 | + | GGUCCAAGGUCAAUUGG | 17 | 493 |
| myoC-1796 | + | UGGUCCAAGGUCAAUUG | 17 | 2021 |
| myoC-156 | + | GCCCCUCCUGGGUCUCC | 17 | 547 |
| myoC-1798 | + | AGCCCCUCCUGGGUCUC | 17 | 2022 |
| myoC-1799 | + | UCCCUCUGCAGCCCCUC | 17 | 2023 |
| myoC-1800 | + | UGGUCCCGCUCCCGCCU | 17 | 2024 |
| myoC-1801 | + | GUCUCCAACUCUCUGGU | 17 | 2025 |
| myoC-152 | + | GAACUGACUUGUCUCGG | 17 | 492 |
| myoC-1803 | + | AGAACUGACUUGUCUCG | 17 | 2026 |
| myoC-281 | + | CCAGAACUGACUUGUCU | 17 | 667 |
| myoC-1805 | + | UCCAGAACUGACUUGUC | 17 | 2027 |
| myoC-1806 | + | CGCUUCUUCUCUUCCUC | 17 | 2028 |
| myoC-278 | + | AGCAGUGUCUCGGGUCU | 17 | 664 |
| myoC-149 | + | GAGCAGUGUCUCGGGUC | 17 | 491 |
| myoC-1809 | + | CGAGCAGUGUCUCGGGU | 17 | 2029 |
| myoC-1810 | + | ACAGCCCGAGCAGUGUC | 17 | 2030 |
| myoC-1811 | + | GAGCCUGGUGGCACAGC | 17 | 2031 |

**Table 4E** provides exemplary targeting domains for knocking out the *MYOC* gene. In an embodiment, the targeting domain is the exact complement of the target domain. Any of the targeting domains in the table can be used with an *N. meningitidis* Cas9 molecule that gives double stranded cleavage. Any of the targeting domains in the table can be used with an *N. meningitidis* Cas9 single-stranded break nucleases (nickases). In an embodiment, dual targeting is used to create two nicks on opposite DNA strands by using *N. meningitidis* Cas9 nickases with two targeting domains that are complementary to opposite DNA strands, e.g., a gRNA comprising any minus strand targeting domain may be paired any gRNA comprising a plus strand targeting domain provided that the two gRNAs are oriented on the DNA such that PAMs face outward and the distance between the 5' ends of the gRNAs is 0-50bp.

**Table 4E**

| **gRNA Name** | **DNA Strand** | **Targeting Domain** | **Target Site Length** | **SEQ ID NO** |
|---|---|---|---|---|
| myoC-3082 | + | GCCUGGCUCUGCUCUGGGCA | 20 | 2844 |
| myoC-3083 | + | UGCUGCUUUCCAACCUCCUG | 20 | 2845 |
| myoC-3156 | - | GAACCGCUAUAAGUACAGCA | 20 | 2842 |
| myoC-3087 | - | AUGACAUAGUUCAAGUUUUC | 20 | 2846 |
| myoC-3088 | + | GCGGACAUCCGUGCCAACUG | 20 | 2847 |
| myoC-2924 | + | CUGUCCGUGGUAGCCAGCUC | 20 | 1822 |
| myoC-3090 | + | UCUCCCAGGUUUGUUCGAGU | 20 | 2848 |
| myoC-3091 | + | AUGGUGACCAUGUUCAUCCU | 20 | 2849 |
| myoC-3084 | + | UGGCUCUGCUCUGGGCA | 17 | 2850 |
| myoC-3085 | + | UGCUUUCCAACCUCCUG | 17 | 2851 |
| myoC-3157 | - | CCGCUAUAAGUACAGCA | 17 | 2843 |
| myoC-3093 | - | ACAUAGUUCAAGUUUUC | 17 | 2852 |
| myoC-3094 | + | GACAUCCGUGCCAACUG | 17 | 2853 |
| myoC-2950 | + | UCCGUGGUAGCCAGCUC | 17 | 1842 |
| myoC-3096 | + | CCCAGGUUUGUUCGAGU | 17 | 2854 |
| myoC-3097 | + | GUGACCAUGUUCAUCCU | 17 | 2855 |

**Table 5A** provides exemplary targeting domains for repressing (i.e., knocking down or decreasing) expression of the *MYOC* gene selected according to first tier parameters, and are selected based on the presence of a 5' G, location in the promoter region and orthogonality in the human genome. In an embodiment, the targeting domain is the exact complement of the target domain. Any of the targeting domains in the table can be used with a *S. pyogenes* eiCas9 molecule to cause a steric block at the promoter region to block transcription resulting in the repression of the *MYOC* gene. Alternatively, any of the targeting domains in the table can be used with a *S. pyogenes* eiCas9 fused to a transcriptional repressor to decrease transcription and therefore downregulate gene expression.

**Table 5A**

| **1st Tier** | selected based on the presence of a 5' G, location in promoter region, and orthogonality in the human genome | | | | |
|---|---|---|---|---|---|
| **gRNA Name** | **DNA Strand** | **Targeting Domain** | **Target Site Length** | **Location** | **SEQ ID NO** |
| myoC-696 | - | GCUGCCUCCAUCGUGCCCGG | 20 | 1st 500bp of DNAsel HS region, overlapping transcription factor binding sites | 976 |
| myoC-707 | + | GCUUGGAAGACUCGGGCUUG | 20 | 1st 500bp of DNAsel HS region, overlapping transcription factor binding sites | 977 |
| myoC-706 | + | GGCUUGGAAGACUCGGGCUU | 20 | 1st 500bp of DNAsel HS region, overlapping transcription factor binding sites | 978 |
| myoC-682 | - | GGGAGCCCUGCAAGCACCCG | 20 | 1st 500bp of DNAsel HS region, overlapping transcription factor binding sites | 979 |
| myoC-712 | + | GGGGCCUCCGGGCACGAUGG | 20 | 1st 500bp of DNAsel HS region, overlapping transcription factor binding sites | 980 |
| myoC-694 | - | GUGCGCAGCAUCCCUUAACA | 20 | 1st 500bp of DNAsel HS region, overlapping transcription factor binding sites | 981 |
| myoC-822 | + | GACCCCGGGUGCUUGCA | 17 | 1st 500bp of DNAsel HS region, overlapping transcription factor binding sites | 982 |
| myoC-828 | + | GAGGAAACCUCUGCCGG | 17 | 1st 500bp of DNAsel HS region, overlapping transcription factor binding sites | 983 |
| myoC-812 | + | GAUAACAAAACAACCAG | 17 | 1st 500bp of DNAsel HS region, overlapping transcription factor binding sites | 984 |
| myoC-772 | - | GCCUCCAUCGUGCCCGG | 17 | 1st 500bp of DNAsel HS region, overlapping transcription factor binding sites | 985 |
| myoC-789 | + | GCCUCCGGGCACGAUGG | 17 | 1st 500bp of DNAsel HS region, overlapping transcription factor binding sites | 986 |
| myoC-806 | + | GUCACCUCCACGAAGGU | 17 | 1st 500bp of DNAsel HS region, overlapping transcription factor binding sites | 987 |
| myoC-848 | - | GAAUCUUGCUGGCAGCGUGA | 20 | within 500bp upstream of transcription start site | 988 |
| myoC-839 | - | GAGAUAUAGGAACUAUUAUU | 20 | within 500bp upstream of transcription start site | 989 |
| myoC-857 | - | GCCAGCAAGGCCACCCAUCC | 20 | within 500bp upstream of transcription start site | 990 |
| myoC-838 | - | GGAGAUAUAGGAACUAUUAU | 20 | within 500bp upstream of transcription start site | 991 |
| myoC-871 | + | GGGGAGCCAGCCCUUCAUGG | 20 | within 500bp upstream of transcription start site | 992 |
| myoC-844 | - | GGGGUAUGGGUGCAUAAAUU | 20 | within 500bp upstream of transcription start site | 993 |
| myoC-837 | - | GUAAAACCAGGUGGAGAUAU | 20 | within 500bp upstream of transcription start site | 994 |
| myoC-861 | + | GUGCUGAGAGGUGCCUGGAU | 20 | within 500bp upstream of transcription start site | 995 |
| myoC-907 | - | GAACUAUUAUUGGGGUA | 17 | within 500bp upstream of transcription start site | 996 |
| myoC-931 | + | GAGAGGUUUAUAUAUAC | 17 | within 500bp upstream of transcription start site | 997 |
| myoC-919 | - | GUAUAUAUAAACCUCUC | 17 | within 500bp upstream of transcription start site | 998 |
| myoC-910 | - | GUAUGGGUGCAUAAAUU | 17 | within 500bp upstream of transcription start site | 999 |
| myoC-959 | + | GUCCUUUAAGACGUAGC | 17 | within 500bp upstream of transcription start site | 100 0 |
| myoC-896 | - | GUCUUAAAGGACUUGUU | 17 | within 500bp upstream of transcription start site | 100 1 |
| myoC-955 | + | GUGUGCUGAUUUCAACA | 17 | within 500bp upstream of transcription start site | 100 2 |

**Table 5B** provides exemplary targeting domains for repressing (i.e., knocking down or decreasing) expression of *MYOC* gene selected according to the second tier parameters, and are selected based on the presence of a 5' G, location in the promoter region. In an embodiment, the targeting domain is the exact complement of the target domain. Any of the targeting domains in the table can be used with a *S. pyogenes* eiCas9 molecule to cause a steric block at the promoter region to block transcription resulting in the repression of the *MYOC* gene. Alternatively, any of the targeting domains in the table can be used with a *S. pyogenes* eiCas9 fused to a transcriptional repressor to decrease transcription and therefore downregulate gene expression.

**Table 5B**

| **2nd Tier** | selected based on the presence of a 5' G and location in promoter region | | | | |
|---|---|---|---|---|---|
| **gRNA Name** | **DNA Strand** | **Targeting Domain** | **Target Site Length** | **Location** | **SEQ ID NO** |
| myoC-709 | + | GACUCGGGCUUGGGGGCCUC | 20 | 1st 500bp of DNAsel HS region, overlapping transcription factor binding sites | 1003 |
| myoC-702 | + | GACUGAUGGAGGAGGAGGCU | 20 | 1st 500bp of DNAsel HS region, overlapping transcription factor binding sites | 1004 |
| myoC-679 | - | GAGGUUUCCUCUCCAGCUGG | 20 | 1st 500bp of DNAsel HS region, overlapping transcription factor binding sites | 1005 |
| myoC-676 | - | GCAGAGGUUUCCUCUCCAGC | 20 | 1st 500bp of DNAsel HS region, overlapping transcription factor binding sites | 1006 |
| myoC-741 | + | GCAGGUUGCUCAGGACACCC | 20 | 1st 500bp of DNAsel HS region, overlapping transcription factor binding sites | 1007 |
| myoC-689 | - | GCCAGACACCAGAGACAAAA | 20 | 1st 500bp of DNAsel HS region, overlapping transcription factor binding sites | 1008 |
| myoC-742 | + | GCUCAGGACACCCAGGACCC | 20 | 1st 500bp of DNAsel HS region, overlapping transcription factor binding sites | 1009 |
| myoC-748 | + | GCUGGAGAGGAAACCUCUGC | 20 | 1st 500bp of DNAsel HS region, overlapping transcription factor binding sites | 1010 |
| myoC-701 | + | GCUGUGACUGAUGGAGGAGG | 20 | 1st 500bp of DNAsel HS region, overlapping transcription factor binding sites | 1011 |
| myoC-746 | + | GCUUGCAGGGCUCCCCCAGC | 20 | 1st 500bp of DNAsel HS region, overlapping transcription factor binding sites | 1012 |
| myoC-751 | + | GGAGAGGAAACCUCUGCCGG | 20 | 1st 500bp of DNAsel HS region, overlapping transcription factor binding sites | 1013 |
| myoC-704 | + | GGAGGAGGCUUGGAAGACUC | 20 | 1st 500bp of DNAsel HS region, overlapping transcription factor binding sites | 1014 |
| myoC-718 | + | GGAGGCAGCAGGGGGCGCUA | 20 | 1st 500bp of DNAsel HS region, overlapping transcription factor binding sites | 1015 |
| myoC-716 | + | GGCACGAUGGAGGCAGCAGG | 20 | 1st 500bp of DNAsel HS region, overlapping transcription factor binding sites | 1016 |
| myoC-719 | + | GGCAGCAGGGGGCGCUAGGG | 20 | 1st 500bp of DNAsel HS region, overlapping transcription factor binding sites | 1017 |
| myoC-715 | + | GGGCACGAUGGAGGCAGCAG | 20 | 1st 500bp of DNAsel HS region, overlapping transcription factor binding sites | 1018 |
| myoC-681 | - | GGGGAGCCCUGCAAGCACCC | 20 | 1st 500bp of DNAsel HS region, overlapping transcription factor binding sites | 1019 |
| myoC-680 | - | GGGGGAGCCCUGCAAGCACC | 20 | 1st 500bp of DNAsel HS region, overlapping transcription factor binding sites | 1020 |
| myoC-692 | - | GUGGAGGUGACAGUUUCUCA | 20 | 1st 500bp of DNAsel HS region, overlapping transcription factor binding sites | 1021 |
| myoC-764 | - | GACUCGUUCAUUCAUCC | 17 | 1st 500bp of DNAsel HS region, overlapping transcription factor binding sites | 1022 |
| myoC-826 | + | GAGAGGAAACCUCUGCC | 17 | 1st 500bp of DNAsel HS region, overlapping transcription factor binding sites | 1023 |
| myoC-757 | - | GAGCCCUGCAAGCACCC | 17 | 1st 500bp of DNAsel HS region, overlapping transcription factor binding sites | 1024 |
| myoC-768 | - | GAGGUGACAGUUUCUCA | 17 | 1st 500bp of DNAsel HS region, overlapping transcription factor binding sites | 1025 |
| myoC-752 | - | GAGGUUUCCUCUCCAGC | 17 | 1st 500bp of DNAsel HS region, overlapping transcription factor binding sites | 1026 |
| myoC-759 | - | GCAAGCACCCGGGGUCC | 17 | 1st 500bp of DNAsel HS region, overlapping transcription factor binding sites | 1027 |
| myoC-791 | + | GCACGAUGGAGGCAGCA | 17 | 1st 500bp of DNAsel HS region, overlapping transcription factor binding sites | 1028 |
| myoC-808 | + | GCUCACCAUUUUGUCUC | 17 | 1st 500bp of DNAsel HS region, overlapping transcription factor binding sites | 1029 |
| myoC-771 | - | GCUGCCUCCAUCGUGCC | 17 | 1st 500bp of DNAsel HS region, overlapping transcription factor binding sites | 1030 |
| myoC-777 | + | GCUGUGACUGAUGGAGG | 17 | 1st 500bp of DNAsel HS region, overlapping transcription factor binding sites | 1031 |
| myoC-785 | + | GGAAGACUCGGGCUUGG | 17 | 1st 500bp of DNAsel HS region, overlapping transcription factor binding sites | 1032 |
| myoC-821 | + | GGACCCCGGGUGCUUGC | 17 | 1st 500bp of DNAsel HS region, overlapping transcription factor binding sites | 1033 |
| myoC-825 | + | GGAGAGGAAACCUCUGC | 17 | 1st 500bp of DNAsel HS region, overlapping transcription factor binding sites | 1034 |
| myoC-756 | - | GGAGCCCUGCAAGCACC | 17 | 1st 500bp of DNAsel HS region, overlapping transcription factor binding sites | 1035 |
| myoC-781 | + | GGAGGCUUGGAAGACUC | 17 | 1st 500bp of DNAsel HS region, overlapping transcription factor binding sites | 1036 |
| myoC-799 | + | GGAGGUGGCCUUGUUAA | 17 | 1st 500bp of DNAsel HS region, overlapping transcription factor binding sites | 1037 |
| myoC-790 | + | GGCACGAUGGAGGCAGC | 17 | 1st 500bp of DNAsel HS region, overlapping transcription factor binding sites | 1038 |
| myoC-795 | + | GGCAGCAGGGGGCGCUA | 17 | 1st 500bp of DNAsel HS region, overlapping transcription factor binding sites | 1039 |
| myoC-824 | + | GGCUCCCCCAGCUGGAG | 17 | 1st 500bp of DNAsel HS region, overlapping transcription factor binding sites | 1040 |
| myoC-798 | + | GGGAGGUGGCCUUGUUA | 17 | 1st 500bp of DNAsel HS region, overlapping transcription factor binding sites | 1041 |
| myoC-817 | + | GGGCUGGCAGGUUGCUC | 17 | 1st 500bp of DNAsel HS region, overlapping transcription factor binding sites | 1042 |
| myoC-788 | + | GGGGCCUCCGGGCACGA | 17 | 1st 500bp of DNAsel HS region, overlapping transcription factor binding sites | 1043 |
| myoC-818 | + | GGUUGCUCAGGACACCC | 17 | 1st 500bp of DNAsel HS region, overlapping transcription factor binding sites | 1044 |
| myoC-754 | - | GGUUUCCUCUCCAGCUG | 17 | 1st 500bp of DNAsel HS region, overlapping transcription factor binding sites | 1045 |
| myoC-778 | + | GUGACUGAUGGAGGAGG | 17 | 1st 500bp of DNAsel HS region, overlapping transcription factor binding sites | 1046 |
| myoC-755 | - | GUUUCCUCUCCAGCUGG | 17 | 1st 500bp of DNAsel HS region, overlapping transcription factor binding sites | 1047 |
| myoC-858 | + | GAAAGCUCUGCUGUGCUGAG | 20 | within 500bp upstream of transcription start site | 1048 |
| myoC-863 | + | GCCUGGAUGGGUGGCCUUGC | 20 | within 500bp upstream of transcription start site | 1049 |
| myoC-881 | + | GCUGGGUGGGGCUGUGCACA | 20 | within 500bp upstream of transcription start site | 1050 |
| myoC-880 | + | GGCUGGGUGGGGCUGUGCAC | 20 | within 500bp upstream of transcription start site | 1051 |
| myoC-884 | + | GGGUGGGGCUGUGCACAGGG | 20 | within 500bp upstream of transcription start site | 1052 |
| myoC-877 | + | GGUGGCCACGUGAGGCUGGG | 20 | within 500bp upstream of transcription start site | 1053 |
| myoC-878 | + | GUGGCCACGUGAGGCUGGGU | 20 | within 500bp upstream of transcription start site | 1054 |
| myoC-835 | - | GUGUGUGUGUGUGUAAAACC | 20 | within 500bp upstream of transcription start site | 1055 |
| myoC-937 | + | GAGCCAGCCCUUCAUGG | 17 | within 500bp upstream of transcription start site | 1056 |
| myoC-933 | + | GAGGUUUAUAUAUACUG | 17 | within 500bp upstream of transcription start site | 1057 |
| myoC-904 | - | GAUAUAGGAACUAUUAU | 17 | within 500bp upstream of transcription start site | 1058 |
| myoC-944 | + | GCCACGUGAGGCUGGGU | 17 | within 500bp upstream of transcription start site | 1059 |
| myoC-926 | + | GCUGAGAGGUGCCUGGA | 17 | within 500bp upstream of transcription start site | 1060 |
| myoC-936 | + | GGAGCCAGCCCUUCAUG | 17 | within 500bp upstream of transcription start site | 1061 |
| myoC-958 | + | GGCACUAUGCUAGGAAC | 17 | within 500bp upstream of transcription start site | 1062 |
| myoC-943 | + | GGCCACGUGAGGCUGGG | 17 | within 500bp upstream of transcription start site | 1063 |
| myoC-935 | + | GGGAGCCAGCCCUUCAU | 17 | within 500bp upstream of transcription start site | 1064 |
| myoC-934 | + | GGGGAGCCAGCCCUUCA | 17 | within 500bp upstream of transcription start site | 1065 |
| myoC-947 | + | GGGUGGGGCUGUGCACA | 17 | within 500bp upstream of transcription start site | 1066 |
| myoC-909 | - | GGUAUGGGUGCAUAAAU | 17 | within 500bp upstream of transcription start site | 1067 |
| myoC-942 | + | GGUGGCCACGUGAGGCU | 17 | within 500bp upstream of transcription start site | 1068 |
| myoC-948 | + | GGUGGGGCUGUGCACAG | 17 | within 500bp upstream of transcription start site | 1069 |
| myoC-953 | + | GUACACACACUUACACC | 17 | within 500bp upstream of transcription start site | 1070 |
| myoC-957 | + | GUGCCAGGCACUAUGCU | 17 | within 500bp upstream of transcription start site | 1071 |
| myoC-949 | + | GUGGGGCUGUGCACAGG | 17 | within 500bp upstream of transcription start site | 1072 |
| myoC-902 | - | GUGUGUGUAAAACCAGG | 17 | within 500bp upstream of transcription start site | 1073 |
| myoC-897 | - | GUUCCUAGCAUAGUGCC | 17 | within 500bp upstream of transcription start site | 1074 |
| myoC-952 | + | GUUCCUAUAUCUCCACC | 17 | within 500bp upstream of transcription start site | 1075 |

**Table 5C** provides exemplary targeting domains for repressing (i.e., knocking down or decreasing) expression of *MYOC* gene selected according to the third tier parameters, and are selected based on the location in the promoter region. In an embodiment, the targeting domain is the exact complement of the target domain. Any of the targeting domains in the table can be used with a *S. pyogenes* eiCas9 molecule to cause a steric block at the promoter region to block transcription resulting in the repression of the *MYOC* gene. Alternatively, any of the targeting domains in the table can be used with a *S. pyogenes* eiCas9 fused to a transcriptional repressor to decrease transcription and therefore downregulate gene expression.

**Table 5C**

| **3rd Tier** | selected based on location in promoter region | | | | |
|---|---|---|---|---|---|
| **gRNA Name** | **DNA Strand** | **Targeting Domain** | **Target Site Length** | **Location** | **SEQ ID NO** |
| myoC-738 | + | AAACAACCAGUGGCACGGGC | 20 | 1st 500bp of DNAsel HS region, overlapping transcription factor binding sites | 1076 |
| myoC-737 | + | AACAAAACAACCAGUGGCAC | 20 | 1st 500bp of DNAsel HS region, overlapping transcription factor binding sites | 1077 |
| myoC-739 | + | AACCAGUGGCACGGGCUGGC | 20 | 1st 500bp of DNAsel HS region, overlapping transcription factor binding sites | 1078 |
| myoC-685 | - | AACCUGCCAGCCCGUGCCAC | 20 | 1st 500bp of DNAsel HS region, overlapping transcription factor binding sites | 1079 |
| myoC-734 | + | ACACAGAAAUAGAAAGCAAC | 20 | 1st 500bp of DNAsel HS region, overlapping transcription factor binding sites | 1080 |
| myoC-732 | + | ACCAUUUUGUCUCUGGUGUC | 20 | 1st 500bp of DNAsel HS region, overlapping transcription factor binding sites | 1081 |
| myoC-710 | + | ACUCGGGCUUGGGGGCCUCC | 20 | 1st 500bp of DNAsel HS region, overlapping transcription factor binding sites | 1082 |
| myoC-729 | + | ACUGUCACCUCCACGAAGGU | 20 | 1st 500bp of DNAsel HS region, overlapping transcription factor binding sites | 1083 |
| myoC-728 | + | AGAAACUGUCACCUCCACGA | 20 | 1st 500bp of DNAsel HS region, overlapping transcription factor binding sites | 1084 |
| myoC-678 | - | AGAGGUUUCCUCUCCAGCUG | 20 | 1st 500bp of DNAsel HS region, overlapping transcription factor binding sites | 1085 |
| myoC-727 | + | AGCACUGGGUUUAAGUUGGC | 20 | 1st 500bp of DNAsel HS region, overlapping transcription factor binding sites | 1086 |
| myoC-720 | + | AGCAGGGGGCGCUAGGGAGG | 20 | 1st 500bp of DNAsel HS region, overlapping transcription factor binding sites | 1087 |
| myoC-700 | + | AGCGCUGUGACUGAUGGAGG | 20 | 1st 500bp of DNAsel HS region, overlapping transcription factor binding sites | 1088 |
| myoC-698 | + | AGCUGCAGCGCUGUGACUGA | 20 | 1st 500bp of DNAsel HS region, overlapping transcription factor binding sites | 1089 |
| myoC-703 | + | AGGAGGAGGCUUGGAAGACU | 20 | 1st 500bp of DNAsel HS region, overlapping transcription factor binding sites | 1090 |
| myoC-705 | + | AGGCUUGGAAGACUCGGGCU | 20 | 1st 500bp of DNAsel HS region, overlapping transcription factor binding sites | 1091 |
| myoC-735 | + | AGUGAUAACAAAACAACCAG | 20 | 1st 500bp of DNAsel HS region, overlapping transcription factor binding sites | 1092 |
| myoC-733 | + | AUAAAUUGUCAAUGAAUGCC | 20 | 1st 500bp of DNAsel HS region, overlapping transcription factor binding sites | 1093 |
| myoC-697 | - | AUCAGUCACAGCGCUGCAGC | 20 | 1st 500bp of DNAsel HS region, overlapping transcription factor binding sites | 1094 |
| myoC-725 | + | AUUUCCUUUCUUUCAGCACU | 20 | 1st 500bp of DNAsel HS region, overlapping transcription factor binding sites | 1095 |
| myoC-740 | + | CACGGGCUGGCAGGUUGCUC | 20 | 1st 500bp of DNAsel HS region, overlapping transcription factor binding sites | 1096 |
| myoC-677 | - | CAGAGGUUUCCUCUCCAGCU | 20 | 1st 500bp of DNAsel HS region, overlapping transcription factor binding sites | 1097 |
| myoC-745 | + | CAGGACCCCGGGUGCUUGCA | 20 | 1st 500bp of DNAsel HS region, overlapping transcription factor binding sites | 1098 |
| myoC-747 | + | CAGGGCUCCCCCAGCUGGAG | 20 | 1st 500bp of DNAsel HS region, overlapping transcription factor binding sites | 1099 |
| myoC-690 | - | CAGUCACUGCCCUACCUUCG | 20 | 1st 500bp of DNAsel HS region, overlapping transcription factor binding sites | 1100 |
| myoC-744 | + | CCAGGACCCCGGGUGCUUGC | 20 | 1st 500bp of DNAsel HS region, overlapping transcription factor binding sites | 1101 |
| myoC-713 | + | CCGGGCACGAUGGAGGCAGC | 20 | 1st 500bp of DNAsel HS region, overlapping transcription factor binding sites | 1102 |
| myoC-683 | - | CCUGCAAGCACCCGGGGUCC | 20 | 1st 500bp of DNAsel HS region, overlapping transcription factor binding sites | 1103 |
| myoC-695 | - | CCUGCUGCCUCCAUCGUGCC | 20 | 1st 500bp of DNAsel HS region, overlapping transcription factor binding sites | 1104 |
| myoC-714 | + | CGGGCACGAUGGAGGCAGCA | 20 | 1st 500bp of DNAsel HS region, overlapping transcription factor binding sites | 1105 |
| myoC-721 | + | CUAGGGAGGUGGCCUUGUUA | 20 | 1st 500bp of DNAsel HS region, overlapping transcription factor binding sites | 1106 |
| myoC-743 | + | CUCAGGACACCCAGGACCCC | 20 | 1st 500bp of DNAsel HS region, overlapping transcription factor binding sites | 1107 |
| myoC-684 | - | CUGCAAGCACCCGGGGUCCU | 20 | 1st 500bp of DNAsel HS region, overlapping transcription factor binding sites | 1108 |
| myoC-723 | + | CUGCGCACAAUUCUUCAAGA | 20 | 1st 500bp of DNAsel HS region, overlapping transcription factor binding sites | 1109 |
| myoC-749 | + | CUGGAGAGGAAACCUCUGCC | 20 | 1st 500bp of DNAsel HS region, overlapping transcription factor binding sites | 1110 |
| myoC-730 | + | CUGUCACCUCCACGAAGGUA | 20 | 1st 500bp of DNAsel HS region, overlapping transcription factor binding sites | 1111 |
| myoC-708 | + | CUUGGAAGACUCGGGCUUGG | 20 | 1st 500bp of DNAsel HS region, overlapping transcription factor binding sites | 1112 |
| myoC-693 | - | UAAACCCAGUGCUGAAAGAA | 20 | 1st 500bp of DNAsel HS region, overlapping transcription factor binding sites | 1113 |
| myoC-736 | + | UAACAAAACAACCAGUGGCA | 20 | 1st 500bp of DNAsel HS region, overlapping transcription factor binding sites | 1114 |
| myoC-722 | + | UAGGGAGGUGGCCUUGUUAA | 20 | 1st 500bp of DNAsel HS region, overlapping transcription factor binding sites | 1115 |
| myoC-724 | + | UAUUUCCUUUCUUUCAGCAC | 20 | 1st 500bp of DNAsel HS region, overlapping transcription factor binding sites | 1116 |
| myoC-691 | - | UCACUGCCCUACCUUCGUGG | 20 | 1st 500bp of DNAsel HS region, overlapping transcription factor binding sites | 1117 |
| myoC-699 | + | UGCAGCGCUGUGACUGAUGG | 20 | 1st 500bp of DNAsel HS region, overlapping transcription factor binding sites | 1118 |
| myoC-750 | + | UGGAGAGGAAACCUCUGCCG | 20 | 1st 500bp of DNAsel HS region, overlapping transcription factor binding sites | 1119 |
| myoC-717 | + | UGGAGGCAGCAGGGGGCGCU | 20 | 1st 500bp of DNAsel HS region, overlapping transcription factor binding sites | 1120 |
| myoC-688 | - | UGUGACUCGUUCAUUCAUCC | 20 | 1st 500bp of DNAsel HS region, overlapping transcription factor binding sites | 1121 |
| myoC-687 | - | UGUUUUGUUAUCACUCUCUA | 20 | 1st 500bp of DNAsel HS region, overlapping transcription factor binding sites | 1122 |
| myoC-711 | + | UUGGGGGCCUCCGGGCACGA | 20 | 1st 500bp of DNAsel HS region, overlapping transcription factor binding sites | 1123 |
| myoC-686 | - | UUGUUUUGUUAUCACUCUCU | 20 | 1st 500bp of DNAsel HS region, overlapping transcription factor binding sites | 1124 |
| myoC-726 | + | UUUCAGCACUGGGUUUAAGU | 20 | 1st 500bp of DNAsel HS region, overlapping transcription factor binding sites | 1125 |
| myoC-731 | + | UUUGCUCACCAUUUUGUCUC | 20 | 1st 500bp of DNAsel HS region, overlapping transcription factor binding sites | 1126 |
| myoC-814 | + | AAAACAACCAGUGGCAC | 17 | 1st 500bp of DNAsel HS region, overlapping transcription factor binding sites | 1127 |
| myoC-805 | + | AACUGUCACCUCCACGA | 17 | 1st 500bp of DNAsel HS region, overlapping transcription factor binding sites | 1128 |
| myoC-810 | + | AAUUGUCAAUGAAUGCC | 17 | 1st 500bp of DNAsel HS region, overlapping transcription factor binding sites | 1129 |
| myoC-769 | - | ACCCAGUGCUGAAAGAA | 17 | 1st 500bp of DNAsel HS region, overlapping transcription factor binding sites | 1130 |
| myoC-793 | + | ACGAUGGAGGCAGCAGG | 17 | 1st 500bp of DNAsel HS region, overlapping transcription factor binding sites | 1131 |
| myoC-804 | + | ACUGGGUUUAAGUUGGC | 17 | 1st 500bp of DNAsel HS region, overlapping transcription factor binding sites | 1132 |
| myoC-765 | - | AGACACCAGAGACAAAA | 17 | 1st 500bp of DNAsel HS region, overlapping transcription factor binding sites | 1133 |
| myoC-827 | + | AGAGGAAACCUCUGCCG | 17 | 1st 500bp of DNAsel HS region, overlapping transcription factor binding sites | 1134 |
| myoC-796 | + | AGCAGGGGGCGCUAGGG | 17 | 1st 500bp of DNAsel HS region, overlapping transcription factor binding sites | 1135 |
| myoC-758 | - | AGCCCUGCAAGCACCCG | 17 | 1st 500bp of DNAsel HS region, overlapping transcription factor binding sites | 1136 |
| myoC-776 | + | AGCGCUGUGACUGAUGG | 17 | 1st 500bp of DNAsel HS region, overlapping transcription factor binding sites | 1137 |
| myoC-820 | + | AGGACACCCAGGACCCC | 17 | 1st 500bp of DNAsel HS region, overlapping transcription factor binding sites | 1138 |
| myoC-780 | + | AGGAGGCUUGGAAGACU | 17 | 1st 500bp of DNAsel HS region, overlapping transcription factor binding sites | 1139 |
| myoC-794 | + | AGGCAGCAGGGGGCGCU | 17 | 1st 500bp of DNAsel HS region, overlapping transcription factor binding sites | 1140 |
| myoC-797 | + | AGGGGGCGCUAGGGAGG | 17 | 1st 500bp of DNAsel HS region, overlapping transcription factor binding sites | 1141 |
| myoC-753 | - | AGGUUUCCUCUCCAGCU | 17 | 1st 500bp of DNAsel HS region, overlapping transcription factor binding sites | 1142 |
| myoC-773 | - | AGUCACAGCGCUGCAGC | 17 | 1st 500bp of DNAsel HS region, overlapping transcription factor binding sites | 1143 |
| myoC-809 | + | AUUUUGUCUCUGGUGUC | 17 | 1st 500bp of DNAsel HS region, overlapping transcription factor binding sites | 1144 |
| myoC-813 | + | CAAAACAACCAGUGGCA | 17 | 1st 500bp of DNAsel HS region, overlapping transcription factor binding sites | 1145 |
| myoC-815 | + | CAACCAGUGGCACGGGC | 17 | 1st 500bp of DNAsel HS region, overlapping transcription factor binding sites | 1146 |
| myoC-760 | - | CAAGCACCCGGGGUCCU | 17 | 1st 500bp of DNAsel HS region, overlapping transcription factor binding sites | 1147 |
| myoC-792 | + | CACGAUGGAGGCAGCAG | 17 | 1st 500bp of DNAsel HS region, overlapping transcription factor binding sites | 1148 |
| myoC-811 | + | CAGAAAUAGAAAGCAAC | 17 | 1st 500bp of DNAsel HS region, overlapping transcription factor binding sites | 1149 |
| myoC-803 | + | CAGCACUGGGUUUAAGU | 17 | 1st 500bp of DNAsel HS region, overlapping transcription factor binding sites | 1150 |
| myoC-819 | + | CAGGACACCCAGGACCC | 17 | 1st 500bp of DNAsel HS region, overlapping transcription factor binding sites | 1151 |
| myoC-816 | + | CAGUGGCACGGGCUGGC | 17 | 1st 500bp of DNAsel HS region, overlapping transcription factor binding sites | 1152 |
| myoC-800 | + | CGCACAAUUCUUCAAGA | 17 | 1st 500bp of DNAsel HS region, overlapping transcription factor binding sites | 1153 |
| myoC-770 | - | CGCAGCAUCCCUUAACA | 17 | 1st 500bp of DNAsel HS region, overlapping transcription factor binding sites | 1154 |
| myoC-787 | + | CGGGCUUGGGGGCCUCC | 17 | 1st 500bp of DNAsel HS region, overlapping transcription factor binding sites | 1155 |
| myoC-761 | - | CUGCCAGCCCGUGCCAC | 17 | 1st 500bp of DNAsel HS region, overlapping transcription factor binding sites | 1156 |
| myoC-767 | - | CUGCCCUACCUUCGUGG | 17 | 1st 500bp of DNAsel HS region, overlapping transcription factor binding sites | 1157 |
| myoC-782 | + | CUUGGAAGACUCGGGCU | 17 | 1st 500bp of DNAsel HS region, overlapping transcription factor binding sites | 1158 |
| myoC-807 | + | UCACCUCCACGAAGGUA | 17 | 1st 500bp of DNAsel HS region, overlapping transcription factor binding sites | 1159 |
| myoC-766 | - | UCACUGCCCUACCUUCG | 17 | 1st 500bp of DNAsel HS region, overlapping transcription factor binding sites | 1160 |
| myoC-802 | + | UCCUUUCUUUCAGCACU | 17 | 1st 500bp of DNAsel HS region, overlapping transcription factor binding sites | 1161 |
| myoC-786 | + | UCGGGCUUGGGGGCCUC | 17 | 1st 500bp of DNAsel HS region, overlapping transcription factor binding sites | 1162 |
| myoC-779 | + | UGAUGGAGGAGGAGGCU | 17 | 1st 500bp of DNAsel HS region, overlapping transcription factor binding sites | 1163 |
| myoC-775 | + | UGCAGCGCUGUGACUGA | 17 | 1st 500bp of DNAsel HS region, overlapping transcription factor binding sites | 1164 |
| myoC-823 | + | UGCAGGGCUCCCCCAGC | 17 | 1st 500bp of DNAsel HS region, overlapping transcription factor binding sites | 1165 |
| myoC-784 | + | UGGAAGACUCGGGCUUG | 17 | 1st 500bp of DNAsel HS region, overlapping transcription factor binding sites | 1166 |
| myoC-774 | + | UUCACGGGAAGCGAGGC | 17 | 1st 500bp of DNAsel HS region, overlapping transcription factor binding sites | 1167 |
| myoC-801 | + | UUCCUUUCUUUCAGCAC | 17 | 1st 500bp of DNAsel HS region, overlapping transcription factor binding sites | 1168 |
| myoC-783 | + | UUGGAAGACUCGGGCUU | 17 | 1st 500bp of DNAsel HS region, overlapping transcription factor binding sites | 1169 |
| myoC-763 | - | UUUGUUAUCACUCUCUA | 17 | 1st 500bp of DNAsel HS region, overlapping transcription factor binding sites | 1170 |
| myoC-762 | - | UUUUGUUAUCACUCUCU | 17 | 1st 500bp of DNAsel HS region, overlapping transcription factor binding sites | 1171 |
| myoC-874 | + | AAGACAGAGGUGGCCACGUG | 20 | within 500bp upstream of transcription start site | 1172 |
| myoC-894 | + | AAGUCCUUUAAGACGUAGCA | 20 | within 500bp upstream of transcription start site | 1173 |
| myoC-834 | - | AAUCAGCACACCAGUAGUCC | 20 | within 500bp upstream of transcription start site | 1174 |
| myoC-850 | - | ACCUCUGUCUUCCCCCAUGA | 20 | within 500bp upstream of transcription start site | 1175 |
| myoC-846 | - | ACUCCAAACAGACUUCUGGA | 20 | within 500bp upstream of transcription start site | 1176 |
| myoC-868 | + | ACUGGGGAGCCAGCCCUUCA | 20 | within 500bp upstream of transcription start site | 1177 |
| myoC-891 | + | ACUGUGCCAGGCACUAUGCU | 20 | within 500bp upstream of transcription start site | 1178 |
| myoC-845 | - | AGAAACUCCAAACAGACUUC | 20 | within 500bp upstream of transcription start site | 1179 |
| myoC-867 | + | AGAGAGGUUUAUAUAUACUG | 20 | within 500bp upstream of transcription start site | 1180 |
| myoC-876 | + | AGAGGUGGCCACGUGAGGCU | 20 | within 500bp upstream of transcription start site | 1181 |
| myoC-840 | - | AGAUAUAGGAACUAUUAUUG | 20 | within 500bp upstream of transcription start site | 1182 |
| myoC-856 | - | AGCUCGGGCAUGAGCCAGCA | 20 | within 500bp upstream of transcription start site | 1183 |
| myoC-842 | - | AGGAACUAUUAUUGGGGUAU | 20 | within 500bp upstream of transcription start site | 1184 |
| myoC-886 | + | AUAGUUCCUAUAUCUCCACC | 20 | within 500bp upstream of transcription start site | 1185 |
| myoC-854 | - | AUAUAAACCUCUCUGGAGCU | 20 | within 500bp upstream of transcription start site | 1186 |
| myoC-893 | + | CAAGUCCUUUAAGACGUAGC | 20 | within 500bp upstream of transcription start site | 1187 |
| myoC-833 | - | CAAUGAGUUUGCAGAGUGAA | 20 | within 500bp upstream of transcription start site | 1188 |
| myoC-888 | + | CACACUUACACCAGGACUAC | 20 | within 500bp upstream of transcription start site | 1189 |
| myoC-887 | + | CACGUACACACACUUACACC | 20 | within 500bp upstream of transcription start site | 1190 |
| myoC-866 | + | CAGAGAGGUUUAUAUAUACU | 20 | within 500bp upstream of transcription start site | 1191 |
| myoC-875 | + | CAGAGGUGGCCACGUGAGGC | 20 | within 500bp upstream of transcription start site | 1192 |
| myoC-849 | - | CAGCCCCACCCAGCCUCACG | 20 | within 500bp upstream of transcription start site | 1193 |
| myoC-832 | - | CAUAGUGCCUGGCACAGUGC | 20 | within 500bp upstream of transcription start site | 1194 |
| myoC-865 | + | CCAGAGAGGUUUAUAUAUAC | 20 | within 500bp upstream of transcription start site | 1195 |
| myoC-892 | + | CCAGGCACUAUGCUAGGAAC | 20 | within 500bp upstream of transcription start site | 1196 |
| myoC-853 | - | CCAGUAUAUAUAAACCUCUC | 20 | within 500bp upstream of transcription start site | 1197 |
| myoC-831 | - | CCAGUUCCUAGCAUAGUGCC | 20 | within 500bp upstream of transcription start site | 1198 |
| myoC-872 | + | CCCUUCAUGGGGGAAGACAG | 20 | within 500bp upstream of transcription start site | 1199 |
| myoC-851 | - | CCUCUGUCUUCCCCCAUGAA | 20 | within 500bp upstream of transcription start site | 1200 |
| myoC-864 | + | CUCAUGCCCGAGCUCCAGAG | 20 | within 500bp upstream of transcription start site | 1201 |
| myoC-862 | + | CUGAGAGGUGCCUGGAUGGG | 20 | within 500bp upstream of transcription start site | 1202 |
| myoC-859 | + | CUGCUGUGCUGAGAGGUGCC | 20 | within 500bp upstream of transcription start site | 1203 |
| myoC-869 | + | CUGGGGAGCCAGCCCUUCAU | 20 | within 500bp upstream of transcription start site | 1204 |
| myoC-882 | + | CUGGGUGGGGCUGUGCACAG | 20 | within 500bp upstream of transcription start site | 1205 |
| myoC-889 | + | CUGGUGUGCUGAUUUCAACA | 20 | within 500bp upstream of transcription start site | 1206 |
| myoC-829 | - | CUGUCCCUGCUACGUCUUAA | 20 | within 500bp upstream of transcription start site | 1207 |
| myoC-885 | + | UAACCUUCCAGAAGUCUGUU | 20 | within 500bp upstream of transcription start site | 1208 |
| myoC-830 | - | UACGUCUUAAAGGACUUGUU | 20 | within 500bp upstream of transcription start site | 1209 |
| myoC-841 | - | UAGGAACUAUUAUUGGGGUA | 20 | within 500bp upstream of transcription start site | 1210 |
| myoC-855 | - | UAUAAACCUCUCUGGAGCUC | 20 | within 500bp upstream of transcription start site | 1211 |
| myoC-879 | + | UGGCCACGUGAGGCUGGGUG | 20 | within 500bp upstream of transcription start site | 1212 |
| myoC-870 | + | UGGGGAGCCAGCCCUUCAUG | 20 | within 500bp upstream of transcription start site | 1213 |
| myoC-843 | - | UGGGGUAUGGGUGCAUAAAU | 20 | within 500bp upstream of transcription start site | 1214 |
| myoC-883 | + | UGGGUGGGGCUGUGCACAGG | 20 | within 500bp upstream of transcription start site | 1215 |
| myoC-852 | - | UGUCUUCCCCCAUGAAGGGC | 20 | within 500bp upstream of transcription start site | 1216 |
| myoC-860 | + | UGUGCUGAGAGGUGCCUGGA | 20 | within 500bp upstream of transcription start site | 1217 |
| myoC-836 | - | UGUGUGUGUGUAAAACCAGG | 20 | within 500bp upstream of transcription start site | 1218 |
| myoC-847 | - | UUAUUUUCUAAGAAUCUUGC | 20 | within 500bp upstream of transcription start site | 1219 |
| myoC-873 | + | UUCAUGGGGGAAGACAGAGG | 20 | within 500bp upstream of transcription start site | 1220 |
| myoC-890 | + | UUGAGAACCUGCACUGUGCC | 20 | within 500bp upstream of transcription start site | 1221 |
| myoC-903 | - | AAACCAGGUGGAGAUAU | 17 | within 500bp upstream of transcription start site | 1222 |
| myoC-921 | - | AAACCUCUCUGGAGCUC | 17 | within 500bp upstream of transcription start site | 1223 |
| myoC-908 | - | AACUAUUAUUGGGGUAU | 17 | within 500bp upstream of transcription start site | 1224 |
| myoC-911 | - | AACUCCAAACAGACUUC | 17 | within 500bp upstream of transcription start site | 1225 |
| myoC-940 | + | ACAGAGGUGGCCACGUG | 17 | within 500bp upstream of transcription start site | 1226 |
| myoC-954 | + | ACU UACACCAGGACUAC | 17 | within 500bp upstream of transcription start site | 1227 |
| myoC-956 | + | AGAACCUGCACUGUGCC | 17 | within 500bp upstream of transcription start site | 1228 |
| myoC-928 | + | AGAGGUGCCUGGAUGGG | 17 | within 500bp upstream of transcription start site | 1229 |
| myoC-932 | + | AGAGGUUUAUAUAUACU | 17 | within 500bp upstream of transcription start site | 1230 |
| myoC-923 | - | AGCAAGGCCACCCAUCC | 17 | within 500bp upstream of transcription start site | 1231 |
| myoC-924 | + | AGCUCUGCUGUGCUGAG | 17 | within 500bp upstream of transcription start site | 1232 |
| myoC-941 | + | AGGUGGCCACGUGAGGC | 17 | within 500bp upstream of transcription start site | 1233 |
| myoC-898 | - | AGUGCCUGGCACAGUGC | 17 | within 500bp upstream of transcription start site | 1234 |
| myoC-905 | - | AUAUAGGAACUAUUAUU | 17 | within 500bp upstream of transcription start site | 1235 |
| myoC-930 | + | AUGCCCGAGCUCCAGAG | 17 | within 500bp upstream of transcription start site | 1236 |
| myoC-939 | + | AUGGGGGAAGACAGAGG | 17 | within 500bp upstream of transcription start site | 1237 |
| myoC-900 | - | CAGCACACCAGUAGUCC | 17 | within 500bp upstream of transcription start site | 1238 |
| myoC-912 | - | CCAAACAGACUUCUGGA | 17 | within 500bp upstream of transcription start site | 1239 |
| myoC-945 | + | CCACGUGAGGCUGGGUG | 17 | within 500bp upstream of transcription start site | 1240 |
| myoC-915 | - | CCCCACCCAGCCUCACG | 17 | within 500bp upstream of transcription start site | 1241 |
| myoC-951 | + | CCUUCCAGAAGUCUGUU | 17 | within 500bp upstream of transcription start site | 1242 |
| myoC-927 | + | CUGAGAGGUGCCUGGAU | 17 | within 500bp upstream of transcription start site | 1243 |
| myoC-917 | - | CUGUCUUCCCCCAUGAA | 17 | within 500bp upstream of transcription start site | 1244 |
| myoC-925 | + | CUGUGCUGAGAGGUGCC | 17 | within 500bp upstream of transcription start site | 1245 |
| myoC-918 | - | CUUCCCCCAUGAAGGGC | 17 | within 500bp upstream of transcription start site | 1246 |
| myoC-920 | - | UAAACCUCUCUGGAGCU | 17 | within 500bp upstream of transcription start site | 1247 |
| myoC-906 | - | UAUAGGAACUAUUAUUG | 17 | within 500bp upstream of transcription start site | 1248 |
| myoC-895 | - | UCCCUGCUACGUCUUAA | 17 | within 500bp upstream of transcription start site | 1249 |
| myoC-960 | + | UCCUUUAAGACGUAGCA | 17 | within 500bp upstream of transcription start site | 1250 |
| myoC-922 | - | UCGGGCAUGAGCCAGCA | 17 | within 500bp upstream of transcription start site | 1251 |
| myoC-916 | - | UCUGUCUUCCCCCAUGA | 17 | within 500bp upstream of transcription start site | 1252 |
| myoC-914 | - | UCUUGCUGGCAGCGUGA | 17 | within 500bp upstream of transcription start site | 1253 |
| myoC-899 | - | UGAGUUUGCAGAGUGAA | 17 | within 500bp upstream of transcription start site | 1254 |
| myoC-929 | + | UGGAUGGGUGGCCUUGC | 17 | within 500bp upstream of transcription start site | 1255 |
| myoC-950 | + | UGGGGCUGUGCACAGGG | 17 | within 500bp upstream of transcription start site | 1256 |
| myoC-946 | + | UGGGUGGGGCUGUGCAC | 17 | within 500bp upstream of transcription start site | 1257 |
| myoC-901 | - | UGUGUGUGUGUAAAACC | 17 | within 500bp upstream of transcription start site | 1258 |
| myoC-938 | + | UUCAUGGGGGAAGACAG | 17 | within 500bp upstream of transcription start site | 1259 |
| myoC-913 | - | UUUUCUAAGAAUCUUGC | 17 | within 500bp upstream of transcription start site | 1260 |

**Table 5D** provides exemplary targeting domains for repressing (i.e., knocking down or decreasing) expression of the *MYOC* gene selected according to the fourth tier parameters, and are selected based on the location in the promoter region that are not described in Tables 5A-C. In an embodiment, the targeting domain is the exact complement of the target domain. Any of the targeting domains in the table can be used with a *S. pyogenes* eiCas9 molecule to cause a steric block at the promoter region to block transcription resulting in the repression of the *MYOC* gene. Alternatively, any of the targeting domains in the table can be used with a *S. pyogenes* eiCas9 fused to a transcriptional repressor to decrease transcription and therefore downregulate gene expression.

**Table 5D**

| **4th Tier** | located in promoter region but not in regions described above | | | |
|---|---|---|---|---|
| **gRNA Name** | **DNA Strand** | **Targeting Domain** | **Target Site Length** | **SEQ ID NO** |
| myoC-961 | - | CAUCUGAGCUGGAGACUCCU | 20 | 1261 |
| myoC-962 | - | GCUGGAGACUCCUUGGCUCC | 20 | 1262 |
| myoC-963 | - | CUUGGCUCCAGGCUCCAGAA | 20 | 1263 |
| myoC-964 | - | UCCAGGCUCCAGAAAGGAAA | 20 | 1264 |
| myoC-965 | - | GCUCCAGAAAGGAAAUGGAG | 20 | 1265 |
| myoC-966 | - | CUCCAGAAAGGAAAUGGAGA | 20 | 1266 |
| myoC-967 | - | GGAGAGGGAAACUAGUCUAA | 20 | 1267 |
| myoC-968 | - | AACUAGUCUAACGGAGAAUC | 20 | 1268 |
| myoC-969 | - | UAGUCUAACGGAGAAUCUGG | 20 | 1269 |
| myoC-970 | - | AGUCUAACGGAGAAUCUGGA | 20 | 1270 |
| myoC-971 | - | GUCUAACGGAGAAUCUGGAG | 20 | 1271 |
| myoC-972 | - | AGGGGACAGUGUUUCCUCAG | 20 | 1272 |
| myoC-973 | - | GGGGACAGUGUUUCCUCAGA | 20 | 1273 |
| myoC-974 | - | CAGUGUUUCCUCAGAGGGAA | 20 | 1274 |
| myoC-975 | - | AGUGUUUCCUCAGAGGGAAA | 20 | 1275 |
| myoC-976 | - | GUGUUUCCUCAGAGGGAAAG | 20 | 1276 |
| myoC-977 | - | GGAAAGGGGCCUCCACGUCC | 20 | 1277 |
| myoC-978 | - | UCCACGUCCAGGAGAAUUCC | 20 | 1278 |
| myoC-979 | - | ACGUCCAGGAGAAUUCCAGG | 20 | 1279 |
| myoC-980 | - | UCCAGGAGAAUUCCAGGAGG | 20 | 1280 |
| myoC-981 | - | CCAGGAGAAUUCCAGGAGGU | 20 | 1281 |
| myoC-982 | - | CAGGAGAAUUCCAGGAGGUG | 20 | 1282 |
| myoC-983 | - | UUCCAGGAGGUGGGGACUGC | 20 | 1283 |
| myoC-984 | - | UCCAGGAGGUGGGGACUGCA | 20 | 1284 |
| myoC-985 | - | GAGGUGGGGACUGCAGGGAG | 20 | 1285 |
| myoC-986 | - | AGGUGGGGACUGCAGGGAGU | 20 | 1286 |
| myoC-987 | - | GGUGGGGACUGCAGGGAGUG | 20 | 1287 |
| myoC-988 | - | ACUGCAGGGAGUGGGGACGC | 20 | 1288 |
| myoC-989 | - | CUGCAGGGAGUGGGGACGCU | 20 | 1289 |
| myoC-990 | - | UGCAGGGAGUGGGGACGCUG | 20 | 1290 |
| myoC-991 | - | GUGGGGACGCUGGGGCUGAG | 20 | 1291 |
| myoC-992 | - | UGGGGACGCUGGGGCUGAGC | 20 | 1292 |
| myoC-993 | - | GGGGCUGAGCGGGUGCUGAA | 20 | 1293 |
| myoC-994 | - | CUGAGCGGGUGCUGAAAGGC | 20 | 1294 |
| myoC-995 | - | GCGGGUGCUGAAAGGCAGGA | 20 | 1295 |
| myoC-996 | - | UGAAAGGCAGGAAGGUGAAA | 20 | 1296 |
| myoC-997 | - | GAAAGGCAGGAAGGUGAAAA | 20 | 1297 |
| myoC-998 | - | GCAGGAAGGUGAAAAGGGCA | 20 | 1298 |
| myoC-999 | - | CAGAUGUUCAGUGUUGUUCA | 20 | 1299 |
| myoC-1000 | - | AGAUGUUCAGUGUUGUUCAC | 20 | 1300 |
| myoC-1001 | - | GAUGUUCAGUGUUGUUCACG | 20 | 1301 |
| myoC-1002 | - | UUCAGUGUUGUUCACGGGGC | 20 | 1302 |
| myoC-1003 | - | UCAGUGUUGUUCACGGGGCU | 20 | 1303 |
| myoC-1004 | - | CUUUUUAUCUUUUCUCUGCU | 20 | 1304 |
| myoC-1005 | - | UUUAUCUUUUCUCUGCUUGG | 20 | 1305 |
| myoC-1006 | - | AGAAGAAGUCUAUUUCAUGA | 20 | 1306 |
| myoC-1007 | - | GAAGAAGUCUAUUUCAUGAA | 20 | 1307 |
| myoC-1008 | - | AAGUCAGCUGUUAAAAUUCC | 20 | 1308 |
| myoC-1009 | - | AGUCAGCUGUUAAAAUUCCA | 20 | 1309 |
| myoC-1010 | - | UUAAAAUUCCAGGGUGUGCA | 20 | 1310 |
| myoC-1011 | - | UAAAAUUCCAGGGUGUGCAU | 20 | 1311 |
| myoC-1012 | - | GCAUGGGUUUUCCUUCACGA | 20 | 1312 |
| myoC-1013 | - | UCACGAAGGCCUUUAUUUAA | 20 | 1313 |
| myoC-1014 | - | CACGAAGGCCUUUAUUUAAU | 20 | 1314 |
| myoC-1015 | - | CCUUUAUUUAAUGGGAAUAU | 20 | 1315 |
| myoC-1016 | - | AGGAAGCGAGCUCAUUUCCU | 20 | 1316 |
| myoC-1017 | - | UUUCCUAGGCCGUUAAUUCA | 20 | 1317 |
| myoC-1018 | - | UAAUUCACGGAAGAAGUGAC | 20 | 1318 |
| myoC-1019 | - | GUCUUUUCUUUCAUGUCUUC | 20 | 1319 |
| myoC-1020 | - | UCUUUUCUUUCAUGUCUUCU | 20 | 1320 |
| myoC-1021 | - | UGGGCAACUACUCAGCCCUG | 20 | 1321 |
| myoC-1022 | - | GCAACUACUCAGCCCUGUGG | 20 | 1322 |
| myoC-1023 | - | ACUCAGCCCUGUGGUGGACU | 20 | 1323 |
| myoC-1024 | - | UGGACUUGGCUUAUGCAAGA | 20 | 1324 |
| myoC-1025 | - | UGCAAGACGGUCGAAAACCU | 20 | 1325 |
| myoC-1026 | - | CGGUCGAAAACCUUGGAAUC | 20 | 1326 |
| myoC-1027 | - | AACCUUGGAAUCAGGAGACU | 20 | 1327 |
| myoC-1028 | - | AGGAGACUCGGUUUUCUUUC | 20 | 1328 |
| myoC-1029 | - | UUUCUUUCUGGUUCUGCCAU | 20 | 1329 |
| myoC-1030 | - | UUUCUGGUUCUGCCAUUGGU | 20 | 1330 |
| myoC-1031 | - | AUUGGUUGGCUGUGCGACCG | 20 | 1331 |
| myoC-1032 | - | UUGGUUGGCUGUGCGACCGU | 20 | 1332 |
| myoC-1033 | - | GGCAAGUGUCUCUCCUUCCC | 20 | 1333 |
| myoC-1034 | - | GCAAGUGUCUCUCCUUCCCU | 20 | 1334 |
| myoC-1035 | - | CUUCCCUGUGAUUCUCUGUG | 20 | 1335 |
| myoC-1036 | - | UUCCCUGUGAUUCUCUGUGA | 20 | 1336 |
| myoC-1037 | - | UCCCUGUGAUUCUCUGUGAG | 20 | 1337 |
| myoC-1038 | - | CCCUGUGAUUCUCUGUGAGG | 20 | 1338 |
| myoC-1039 | - | CCUGUGAUUCUCUGUGAGGG | 20 | 1339 |
| myoC-1040 | - | CUGUGAGGGGGGAUGUUGAG | 20 | 1340 |
| myoC-1041 | - | UGUGAGGGGGGAUGUUGAGA | 20 | 1341 |
| myoC-1042 | - | GUGAGGGGGGAUGUUGAGAG | 20 | 1342 |
| myoC-1043 | - | GGGGGGAUGUUGAGAGGGGA | 20 | 1343 |
| myoC-1044 | - | GGGAUGUUGAGAGGGGAAGG | 20 | 1344 |
| myoC-1045 | - | AGAGGGGAAGGAGGCAGAGC | 20 | 1345 |
| myoC-1046 | - | AGCUGGAGCAGCUGAGCCAC | 20 | 1346 |
| myoC-1047 | - | GCUGGAGCAGCUGAGCCACA | 20 | 1347 |
| myoC-1048 | - | CUGGAGCAGCUGAGCCACAG | 20 | 1348 |
| myoC-1049 | - | GAGCAGCUGAGCCACAGGGG | 20 | 1349 |
| myoC-1050 | - | CAGCUGAGCCACAGGGGAGG | 20 | 1350 |
| myoC-1051 | - | CUGAGCCACAGGGGAGGUGG | 20 | 1351 |
| myoC-1052 | - | UGAGCCACAGGGGAGGUGGA | 20 | 1352 |
| myoC-1053 | - | GAGCCACAGGGGAGGUGGAG | 20 | 1353 |
| myoC-1054 | - | AGCCACAGGGGAGGUGGAGG | 20 | 1354 |
| myoC-1055 | - | CAGGGGAGGUGGAGGGGGAC | 20 | 1355 |
| myoC-1056 | - | GGAGGUGGAGGGGGACAGGA | 20 | 1356 |
| myoC-1057 | - | GUGGAGGGGGACAGGAAGGC | 20 | 1357 |
| myoC-1058 | - | ACAGGAAGGCAGGCAGAAGC | 20 | 1358 |
| myoC-1059 | - | CAGGAAGGCAGGCAGAAGCU | 20 | 1359 |
| myoC-1060 | - | CACUGAUCACGUCAGACUCC | 20 | 1360 |
| myoC-1061 | - | ACCGAGAGCCACAAUGCUUC | 20 | 1361 |
| myoC-1062 | - | CCUUCCCUAAGCAUAGACAA | 20 | 1362 |
| myoC-1063 | - | AAAAGAAUGCAGAGACUAAC | 20 | 1363 |
| myoC-1064 | - | AGAAUGCAGAGACUAACUGG | 20 | 1364 |
| myoC-1065 | - | AACUGGUGGUAGCUUUUGCC | 20 | 1365 |
| myoC-1066 | - | UUUGCCUGGCAUUCAAAAAC | 20 | 1366 |
| myoC-1067 | - | UUGCCUGGCAUUCAAAAACU | 20 | 1367 |
| myoC-1068 | - | AAAAACUGGGCCAGAGCAAG | 20 | 1368 |
| myoC-1069 | + | CUGGCAUUUUCCACUUGCUC | 20 | 1369 |
| myoC-1070 | + | UGGCCCAGUUUUUGAAUGCC | 20 | 1370 |
| myoC-1071 | + | GUUAGUCUCUGCAUUCUUUU | 20 | 1371 |
| myoC-1072 | + | UCUGCAUUCUUUUUGGUUAU | 20 | 1372 |
| myoC-1073 | + | AAAUGCCAUUGUCUAUGCUU | 20 | 1373 |
| myoC-1074 | + | AAUGCCAUUGUCUAUGCUUA | 20 | 1374 |
| myoC-1075 | + | CCAUUGUCUAUGCUUAGGGA | 20 | 1375 |
| myoC-1076 | + | AUGCUUAGGGAAGGAAAAUG | 20 | 1376 |
| myoC-1077 | + | GGGAAGGAAAAUGUGGCUGU | 20 | 1377 |
| myoC-1078 | + | GGAAGGAAAAUGUGGCUGUU | 20 | 1378 |
| myoC-1079 | + | UGAGCUUUCCUGAAGCAUUG | 20 | 1379 |
| myoC-1080 | + | UCCUGAAGCAUUGUGGCUCU | 20 | 1380 |
| myoC-1081 | + | AGCAUUGUGGCUCUCGGUCC | 20 | 1381 |
| myoC-1082 | + | GGAGUCUGACGUGAUCAGUG | 20 | 1382 |
| myoC-1083 | + | ACGUGAUCAGUGAGGACUGA | 20 | 1383 |
| myoC-1084 | + | GUCCCCCUCCACCUCCCCUG | 20 | 1384 |
| myoC-1085 | + | CCCCCCUCACAGAGAAUCAC | 20 | 1385 |
| myoC-1086 | + | CCCCCUCACAGAGAAUCACA | 20 | 1386 |
| myoC-1087 | + | CACAGAACACGAGAGCUGCA | 20 | 1387 |
| myoC-1088 | + | ACAGAACACGAGAGCUGCAA | 20 | 1388 |
| myoC-1089 | + | CUUUAUAGCAGAGAAGACUA | 20 | 1389 |
| myoC-1090 | + | AGCAGAGAAGACUAUGGCCC | 20 | 1390 |
| myoC-1091 | + | GCAGAGAAGACUAUGGCCCA | 20 | 1391 |
| myoC-1092 | + | AGAAGACUAUGGCCCAGGGA | 20 | 1392 |
| myoC-1093 | + | GAAGGAGAGACACUUGCCCA | 20 | 1393 |
| myoC-1094 | + | ACGGUCGCACAGCCAACCAA | 20 | 1394 |
| myoC-1095 | + | AACCGAGUCUCCUGAUUCCA | 20 | 1395 |
| myoC-1096 | + | GCAUAAGCCAAGUCCACCAC | 20 | 1396 |
| myoC-1097 | + | CAUAAGCCAAGUCCACCACA | 20 | 1397 |
| myoC-1098 | + | UCACUUCUUCCGUGAAUUAA | 20 | 1398 |
| myoC-1099 | + | CUUCCGUGAAUUAACGGCCU | 20 | 1399 |
| myoC-1100 | + | CCUAUAUUCCCAUUAAAUAA | 20 | 1400 |
| myoC-1101 | + | UUAAAUAAAGGCCUUCGUGA | 20 | 1401 |
| myoC-1102 | + | AGGAAAACCCAUGCACACCC | 20 | 1402 |
| myoC-1103 | + | CAAGCAGAGAAAAGAUAAAA | 20 | 1403 |
| myoC-1104 | + | GAAAAGAUAAAAAGGCUCAC | 20 | 1404 |
| myoC-1105 | + | AAAAGGCUCACAGGAAGCAA | 20 | 1405 |
| myoC-1106 | + | CGUGAACAACACUGAACAUC | 20 | 1406 |
| myoC-1107 | + | GUGAACAACACUGAACAUCU | 20 | 1407 |
| myoC-1108 | + | UCCCUGCAGUCCCCACCUCC | 20 | 1408 |
| myoC-1109 | + | CCCACCUCCUGGAAUUCUCC | 20 | 1409 |
| myoC-1110 | + | UCCUGGAAUUCUCCUGGACG | 20 | 1410 |
| myoC-1111 | + | UGGAAUUCUCCUGGACGUGG | 20 | 1411 |
| myoC-1112 | + | UGGAGGCCCCUUUCCCUCUG | 20 | 1412 |
| myoC-1113 | + | UUCCCUCUCCAUUUCCUUUC | 20 | 1413 |
| myoC-1114 | + | UCCAUUUCCUUUCUGGAGCC | 20 | 1414 |
| myoC-1115 | + | CUUUCUGGAGCCUGGAGCCA | 20 | 1415 |
| myoC-1116 | + | GUCUCCAGCUCAGAUGCACC | 20 | 1416 |
| myoC-1117 | - | AGCAGUGACUGCUGACAGCA | 20 | 1417 |
| myoC-1118 | - | CACGGAGUGACCUGCAGCGC | 20 | 1418 |
| myoC-1119 | - | ACGGAGUGACCUGCAGCGCA | 20 | 1419 |
| myoC-1120 | - | CGGAGUGACCUGCAGCGCAG | 20 | 1420 |
| myoC-1121 | - | AGUGACCUGCAGCGCAGGGG | 20 | 1421 |
| myoC-1122 | - | GGGGAGGAGAAGAAAAAGAG | 20 | 1422 |
| myoC-1123 | - | GGGAGGAGAAGAAAAAGAGA | 20 | 1423 |
| myoC-1124 | - | AAGAAAGACAGAUUCAUUCA | 20 | 1424 |
| myoC-1125 | - | AGAAAGACAGAUUCAUUCAA | 20 | 1425 |
| myoC-1126 | - | ACAGAUUCAUUCAAGGGCAG | 20 | 1426 |
| myoC-1127 | - | CAGAUUCAUUCAAGGGCAGU | 20 | 1427 |
| myoC-1128 | - | GGGCAGUGGGAAUUGACCAC | 20 | 1428 |
| myoC-1129 | - | GGCAGUGGGAAUUGACCACA | 20 | 1429 |
| myoC-1130 | - | GAUUAUAGUCCACGUGAUCC | 20 | 1430 |
| myoC-1131 | - | AUUAUAGUCCACGUGAUCCU | 20 | 1431 |
| myoC-1132 | - | UCCACGUGAUCCUGGGUUCU | 20 | 1432 |
| myoC-1133 | - | ACGUGAUCCUGGGUUCUAGG | 20 | 1433 |
| myoC-1134 | - | GAUCCUGGGUUCUAGGAGGC | 20 | 1434 |
| myoC-1135 | - | AUCCUGGGUUCUAGGAGGCA | 20 | 1435 |
| myoC-1136 | - | UAGGAGGCAGGGCUAUAUUG | 20 | 1436 |
| myoC-1137 | - | AGGAGGCAGGGCUAUAUUGU | 20 | 1437 |
| myoC-1138 | - | GGAGGCAGGGCUAUAUUGUG | 20 | 1438 |
| myoC-1139 | - | GAGGCAGGGCUAUAUUGUGG | 20 | 1439 |
| myoC-1140 | - | AGGCAGGGCUAUAUUGUGGG | 20 | 1440 |
| myoC-1141 | - | GGGGGGAAAAAAUCAGUUCA | 20 | 1441 |
| myoC-1142 | - | GGGGGAAAAAAUCAGUUCAA | 20 | 1442 |
| myoC-1143 | - | AAAAUCAGUUCAAGGGAAGU | 20 | 1443 |
| myoC-1144 | - | AAAUCAGUUCAAGGGAAGUC | 20 | 1444 |
| myoC-1145 | - | GUAAUUCUGAGCAAGUCACA | 20 | 1445 |
| myoC-1146 | - | AAGUCACAAGGUAGUAACUG | 20 | 1446 |
| myoC-1147 | - | UUACUUAGUUUCUCCUUAUU | 20 | 1447 |
| myoC-1148 | - | UUAGGAACUCUUUUUCUCUG | 20 | 1448 |
| myoC-1149 | - | UCUGUGGAGUUAGCAGCACA | 20 | 1449 |
| myoC-1150 | - | CUGUGGAGUUAGCAGCACAA | 20 | 1450 |
| myoC-1151 | - | GCAAUCCCGUUUCUUUUAAC | 20 | 1451 |
| myoC-1152 | - | AGCCAAACAGAUUCAAGCCU | 20 | 1452 |
| myoC-1153 | - | GGUCUUGCUGACUAUAUGAU | 20 | 1453 |
| myoC-1154 | - | AAAAUGAGACUAGUACCCUU | 20 | 1454 |
| myoC-1155 | - | UUUGUAAAUGUCUCAAGUUC | 20 | 1455 |
| myoC-1156 | - | CAAACUGUGUUUCUCCACUC | 20 | 1456 |
| myoC-1157 | - | ACUGUGUUUCUCCACUCUGG | 20 | 1457 |
| myoC-1158 | - | ACUCUGGAGGUGAGUCUGCC | 20 | 1458 |
| myoC-1159 | - | CUCUGGAGGUGAGUCUGCCA | 20 | 1459 |
| myoC-1160 | - | GUGAGUCUGCCAGGGCAGUU | 20 | 1460 |
| myoC-1161 | - | ACAAGUAUUGACACUGUUGU | 20 | 1461 |
| myoC-1162 | - | AACAACAUAAAGUUGCUCAA | 20 | 1462 |
| myoC-1163 | - | AAGGCAAUCAUUAUUUCAAG | 20 | 1463 |
| myoC-1164 | - | AAAGUUACUUCUGACAGUUU | 20 | 1464 |
| myoC-1165 | - | GACAGUUUUGGUAUAUUUAU | 20 | 1465 |
| myoC-1166 | - | UGCUUUUUGUUUUUUCUCUU | 20 | 1466 |
| myoC-1167 | - | GCUUUUUGUUUUUUCUCUUU | 20 | 1467 |
| myoC-1168 | - | UGGGUUUAUUAAUGUAAAGC | 20 | 1468 |
| myoC-1169 | - | GGGUUUAUUAAUGUAAAGCA | 20 | 1469 |
| myoC-1170 | - | AAAGCCUGUGAAUUUGAAUG | 20 | 1470 |
| myoC-1171 | - | AUAGAGCCAUAAACUCAAAG | 20 | 1471 |
| myoC-1172 | + | UUAUUACCACUUUGAGUUUA | 20 | 1472 |
| myoC-1173 | + | GUUUAUGGCUCUAUUCGCAA | 20 | 1473 |
| myoC-1174 | + | AAAUGUUAAAUUUAGUUAGA | 20 | 1474 |
| myoC-1175 | + | UGUUAAAUUUAGUUAGAAGG | 20 | 1475 |
| myoC-1176 | + | UUUUCCUCAUUCAAAUUCAC | 20 | 1476 |
| myoC-1177 | + | AUUCAAAUUCACAGGCUUUC | 20 | 1477 |
| myoC-1178 | + | UCACAGGCUUUCUGGACUGU | 20 | 1478 |
| myoC-1179 | + | GAGAAAAAACAAAAAGCAAA | 20 | 1479 |
| myoC-1180 | + | UAAAUAUUUCCAAACUGCCC | 20 | 1480 |
| myoC-1181 | + | UGGCAGACUCACCUCCAGAG | 20 | 1481 |
| myoC-1182 | + | AGAUUCUAUUCUUAUUUGAU | 20 | 1482 |
| myoC-1183 | + | GAACUUGAGACAUUUACAAA | 20 | 1483 |
| myoC-1184 | + | AACUUGAGACAUUUACAAAU | 20 | 1484 |
| myoC-1185 | + | GUUUGUUUACAGCUGACCAA | 20 | 1485 |
| myoC-1186 | + | UUUGUUUACAGCUGACCAAA | 20 | 1486 |
| myoC-1187 | + | UCAUAUAGUCAGCAAGACCU | 20 | 1487 |
| myoC-1188 | + | GACCUAGGCUUGAAUCUGUU | 20 | 1488 |
| myoC-1189 | + | AUCUGUUUGGCUUUACUCUU | 20 | 1489 |
| myoC-1190 | + | UUUCUUCCUGUUAAAAGAAA | 20 | 1490 |
| myoC-1191 | + | UUCUUCCUGUUAAAAGAAAC | 20 | 1491 |
| myoC-1192 | + | GAGAAAAAGAGUUCCUAAUA | 20 | 1492 |
| myoC-1193 | + | CAGAAUUACUCAGCUUGUAA | 20 | 1493 |
| myoC-1194 | + | AAAAUAUAGUAUUAGAAAUC | 20 | 1494 |
| myoC-1195 | + | AGCCCUGCCUCCUAGAACCC | 20 | 1495 |
| myoC-1196 | + | UCCUAGAACCCAGGAUCACG | 20 | 1496 |
| myoC-1197 | + | CACGUGGACUAUAAUCCCUG | 20 | 1497 |
| myoC-1198 | + | CUUCUCCUCCCCUGCGCUGC | 20 | 1498 |
| myoC-1199 | + | GCAGUCACUGCUGAGCUGCG | 20 | 1499 |
| myoC-1200 | + | CAGUCACUGCUGAGCUGCGU | 20 | 1500 |
| myoC-1201 | + | AGUCACUGCUGAGCUGCGUG | 20 | 1501 |
| myoC-1202 | + | UGCUGAGCUGCGUGGGGUGC | 20 | 1502 |
| myoC-1203 | + | AGCUGCGUGGGGUGCUGGUC | 20 | 1503 |
| myoC-1204 | + | GCUGCGUGGGGUGCUGGUCA | 20 | 1504 |
| myoC-1205 | - | UUUGAAAUUAGACCUCCUGC | 20 | 1505 |
| myoC-1206 | - | UUCCCCAGAUUUCACCAAUG | 20 | 1506 |
| myoC-1207 | - | GAUUUCACCAAUGAGGUUCU | 20 | 1507 |
| myoC-1208 | - | CAGAGUAAGAACUGAUUUAG | 20 | 1508 |
| myoC-1209 | - | UUAGAGGCUAACAUUGACAU | 20 | 1509 |
| myoC-1210 | - | GGGAAAUCUGCCGCUUCUAU | 20 | 1510 |
| myoC-1211 | - | UUCUAUAGGAAUGCUCUCCC | 20 | 1511 |
| myoC-1212 | - | GGAAUGCUCUCCCUGGAGCC | 20 | 1512 |
| myoC-1213 | - | UGCUCUCCCUGGAGCCUGGU | 20 | 1513 |
| myoC-1214 | - | GCUCUCCCUGGAGCCUGGUA | 20 | 1514 |
| myoC-1215 | - | AGGGUGCUGUCCUUGUGUUC | 20 | 1515 |
| myoC-1216 | + | CACAAGGACAGCACCCUACC | 20 | 1516 |
| myoC-1217 | + | ACAGCACCCUACCAGGCUCC | 20 | 1517 |
| myoC-1218 | + | CAGCACCCUACCAGGCUCCA | 20 | 1518 |
| myoC-1219 | + | GGAGAGCAUUCCUAUAGAAG | 20 | 1519 |
| myoC-1220 | + | UUAAAACAACUGUGUAUCUU | 20 | 1520 |
| myoC-1221 | + | UAAAACAACUGUGUAUCUUU | 20 | 1521 |
| myoC-1222 | + | UAAUUUCAGUCUUGCAUCUC | 20 | 1522 |
| myoC-1223 | + | GUGCAUGCCAAGAACCUCAU | 20 | 1523 |
| myoC-1224 | + | AGAACCUCAUUGGUGAAAUC | 20 | 1524 |
| myoC-1225 | + | GAACCUCAUUGGUGAAAUCU | 20 | 1525 |
| myoC-1226 | + | AACCUCAUUGGUGAAAUCUG | 20 | 1526 |
| myoC-1227 | + | AUAUAAAAUAUAGAUUACAA | 20 | 1527 |
| myoC-1228 | + | UGUUAAAAACAAGAUCCAGC | 20 | 1528 |
| myoC-1229 | + | UAAAAACAAGAUCCAGCAGG | 20 | 1529 |
| myoC-1230 | + | AAAAUGUCUGUGAUUUCUAU | 20 | 1530 |
| myoC-1231 | - | CUGAGCUGGAGACUCCU | 17 | 1531 |
| myoC-1232 | - | GGAGACUCCUUGGCUCC | 17 | 1532 |
| myoC-1233 | - | GGCUCCAGGCUCCAGAA | 17 | 1533 |
| myoC-1234 | - | AGGCUCCAGAAAGGAAA | 17 | 1534 |
| myoC-1235 | - | CCAGAAAGGAAAUGGAG | 17 | 1535 |
| myoC-1236 | - | CAGAAAGGAAAUGGAGA | 17 | 1536 |
| myoC-1237 | - | GAGGGAAACUAGUCUAA | 17 | 1537 |
| myoC-1238 | - | UAGUCUAACGGAGAAUC | 17 | 1538 |
| myoC-1239 | - | UCUAACGGAGAAUCUGG | 17 | 1539 |
| myoC-1240 | - | CUAACGGAGAAUCUGGA | 17 | 1540 |
| myoC-1241 | - | UAACGGAGAAUCUGGAG | 17 | 1541 |
| myoC-1242 | - | GGACAGUGUUUCCUCAG | 17 | 1542 |
| myoC-1243 | - | GACAGUGUUUCCUCAGA | 17 | 1543 |
| myoC-1244 | - | UGUUUCCUCAGAGGGAA | 17 | 1544 |
| myoC-1245 | - | GUUUCCUCAGAGGGAAA | 17 | 1545 |
| myoC-1246 | - | UUUCCUCAGAGGGAAAG | 17 | 1546 |
| myoC-1247 | - | AAGGGGCCUCCACGUCC | 17 | 1547 |
| myoC-1248 | - | ACGUCCAGGAGAAUUCC | 17 | 1548 |
| myoC-1249 | - | UCCAGGAGAAUUCCAGG | 17 | 1549 |
| myoC-1250 | - | AGGAGAAUUCCAGGAGG | 17 | 1550 |
| myoC-1251 | - | GGAGAAUUCCAGGAGGU | 17 | 1551 |
| myoC-1252 | - | GAGAAUUCCAGGAGGUG | 17 | 1552 |
| myoC-1253 | - | CAGGAGGUGGGGACUGC | 17 | 1553 |
| myoC-1254 | - | AGGAGGUGGGGACUGCA | 17 | 1554 |
| myoC-1255 | - | GUGGGGACUGCAGGGAG | 17 | 1555 |
| myoC-1256 | - | UGGGGACUGCAGGGAGU | 17 | 1556 |
| myoC-1257 | - | GGGGACUGCAGGGAGUG | 17 | 1557 |
| myoC-1258 | - | GCAGGGAGUGGGGACGC | 17 | 1558 |
| myoC-1259 | - | CAGGGAGUGGGGACGCU | 17 | 1559 |
| myoC-1260 | - | AGGGAGUGGGGACGCUG | 17 | 1560 |
| myoC-1261 | - | GGGACGCUGGGGCUGAG | 17 | 1561 |
| myoC-1262 | - | GGACGCUGGGGCUGAGC | 17 | 1562 |
| myoC-1263 | - | GCUGAGCGGGUGCUGAA | 17 | 1563 |
| myoC-1264 | - | AGCGGGUGCUGAAAGGC | 17 | 1564 |
| myoC-1265 | - | GGUGCUGAAAGGCAGGA | 17 | 1565 |
| myoC-1266 | - | AAGGCAGGAAGGUGAAA | 17 | 1566 |
| myoC-1267 | - | AGGCAGGAAGGUGAAAA | 17 | 1567 |
| myoC-1268 | - | GGAAGGUGAAAAGGGCA | 17 | 1568 |
| myoC-1269 | - | AUGUUCAGUGUUGUUCA | 17 | 1569 |
| myoC-1270 | - | UGUUCAGUGUUGUUCAC | 17 | 1570 |
| myoC-1271 | - | GUUCAGUGUUGUUCACG | 17 | 1571 |
| myoC-1272 | - | AGUGUUGUUCACGGGGC | 17 | 1572 |
| myoC-1273 | - | GUGUUGUUCACGGGGCU | 17 | 1573 |
| myoC-1274 | - | UUUAUCUUUUCUCUGCU | 17 | 1574 |
| myoC-1275 | - | AUCUUUUCUCUGCUUGG | 17 | 1575 |
| myoC-1276 | - | AGAAGUCUAUUUCAUGA | 17 | 1576 |
| myoC-1277 | - | GAAGUCUAUUUCAUGAA | 17 | 1577 |
| myoC-1278 | - | UCAGCUGUUAAAAUUCC | 17 | 1578 |
| myoC-1279 | - | CAGCUGUUAAAAUUCCA | 17 | 1579 |
| myoC-1280 | - | AAAUUCCAGGGUGUGCA | 17 | 1580 |
| myoC-1281 | - | AAUUCCAGGGUGUGCAU | 17 | 1581 |
| myoC-1282 | - | UGGGUUUUCCUUCACGA | 17 | 1582 |
| myoC-1283 | - | CGAAGGCCUUUAUUUAA | 17 | 1583 |
| myoC-1284 | - | GAAGGCCUUUAUUUAAU | 17 | 1584 |
| myoC-1285 | - | UUAUUUAAUGGGAAUAU | 17 | 1585 |
| myoC-1286 | - | AAGCGAGCUCAUUUCCU | 17 | 1586 |
| myoC-1287 | - | CCUAGGCCGUUAAUUCA | 17 | 1587 |
| myoC-1288 | - | UUCACGGAAGAAGUGAC | 17 | 1588 |
| myoC-1289 | - | UUUUCUUUCAUGUCUUC | 17 | 1589 |
| myoC-1290 | - | UUUCUUUCAUGUCUUCU | 17 | 1590 |
| myoC-1291 | - | GCAACUACUCAGCCCUG | 17 | 1591 |
| myoC-1292 | - | ACUACUCAGCCCUGUGG | 17 | 1592 |
| myoC-1293 | - | CAGCCCUGUGGUGGACU | 17 | 1593 |
| myoC-1294 | - | ACUUGGCUUAUGCAAGA | 17 | 1594 |
| myoC-1295 | - | AAGACGGUCGAAAACCU | 17 | 1595 |
| myoC-1296 | - | UCGAAAACCU UGGAAUC | 17 | 1596 |
| myoC-1297 | - | CUUGGAAUCAGGAGACU | 17 | 1597 |
| myoC-1298 | - | AGACUCGGUUUUCUUUC | 17 | 1598 |
| myoC-1299 | - | CUUUCUGGUUCUGCCAU | 17 | 1599 |
| myoC-1300 | - | CUGGUUCUGCCAUUGGU | 17 | 1600 |
| myoC-1301 | - | GGUUGGCUGUGCGACCG | 17 | 1601 |
| myoC-1302 | - | GUUGGCUGUGCGACCGU | 17 | 1602 |
| myoC-1303 | - | AAGUGUCUCUCCUUCCC | 17 | 1603 |
| myoC-1304 | - | AGUGUCUCUCCUUCCCU | 17 | 1604 |
| myoC-1305 | - | CCCUGUGAUUCUCUGUG | 17 | 1605 |
| myoC-1306 | - | CCUGUGAUUCUCUGUGA | 17 | 1606 |
| myoC-1307 | - | CUGUGAUUCUCUGUGAG | 17 | 1607 |
| myoC-1308 | - | UGUGAUUCUCUGUGAGG | 17 | 1608 |
| myoC-1309 | - | GUGAUUCUCUGUGAGGG | 17 | 1609 |
| myoC-1310 | - | UGAGGGGGGAUGUUGAG | 17 | 1610 |
| myoC-1311 | - | GAGGGGGGAUGUUGAGA | 17 | 1611 |
| myoC-1312 | - | AGGGGGGAUGUUGAGAG | 17 | 1612 |
| myoC-1313 | - | GGGAUGUUGAGAGGGGA | 17 | 1613 |
| myoC-1314 | - | AUGUUGAGAGGGGAAGG | 17 | 1614 |
| myoC-1315 | - | GGGGAAGGAGGCAGAGC | 17 | 1615 |
| myoC-1316 | - | UGGAGCAGCUGAGCCAC | 17 | 1616 |
| myoC-1317 | - | GGAGCAGCUGAGCCACA | 17 | 1617 |
| myoC-1318 | - | GAGCAGCUGAGCCACAG | 17 | 1618 |
| myoC-1319 | - | CAGCUGAGCCACAGGGG | 17 | 1619 |
| myoC-1320 | - | CUGAGCCACAGGGGAGG | 17 | 1620 |
| myoC-1321 | - | AGCCACAGGGGAGGUGG | 17 | 1621 |
| myoC-1322 | - | GCCACAGGGGAGGUGGA | 17 | 1622 |
| myoC-1323 | - | CCACAGGGGAGGUGGAG | 17 | 1623 |
| myoC-1324 | - | CACAGGGGAGGUGGAGG | 17 | 1624 |
| myoC-1325 | - | GGGAGGUGGAGGGGGAC | 17 | 1625 |
| myoC-1326 | - | GGUGGAGGGGGACAGGA | 17 | 1626 |
| myoC-1327 | - | GAGGGGGACAGGAAGGC | 17 | 1627 |
| myoC-1328 | - | GGAAGGCAGGCAGAAGC | 17 | 1628 |
| myoC-1329 | - | GAAGGCAGGCAGAAGCU | 17 | 1629 |
| myoC-1330 | - | UGAUCACGUCAGACUCC | 17 | 1630 |
| myoC-1331 | - | GAGAGCCACAAUGCUUC | 17 | 1631 |
| myoC-1332 | - | UCCCUAAGCAUAGACAA | 17 | 1632 |
| myoC-1333 | - | AGAAUGCAGAGACUAAC | 17 | 1633 |
| myoC-1334 | - | AUGCAGAGACUAACUGG | 17 | 1634 |
| myoC-1335 | - | UGGUGGUAGCUUUUGCC | 17 | 1635 |
| myoC-1336 | - | GCCUGGCAUUCAAAAAC | 17 | 1636 |
| myoC-1337 | - | CCUGGCAUUCAAAAACU | 17 | 1637 |
| myoC-1338 | - | AACUGGGCCAGAGCAAG | 17 | 1638 |
| myoC-1339 | + | GCAUUUUCCACUUGCUC | 17 | 1639 |
| myoC-1340 | + | CCCAGUUUUUGAAUGCC | 17 | 1640 |
| myoC-1341 | + | AGUCUCUGCAUUCUUUU | 17 | 1641 |
| myoC-1342 | + | GCAUUCUUUUUGGUUAU | 17 | 1642 |
| myoC-1343 | + | UGCCAUUGUCUAUGCUU | 17 | 1643 |
| myoC-1344 | + | GCCAUUGUCUAUGCUUA | 17 | 1644 |
| myoC-1345 | + | UUGUCUAUGCUUAGGGA | 17 | 1645 |
| myoC-1346 | + | CUUAGGGAAGGAAAAUG | 17 | 1646 |
| myoC-1347 | + | AAGGAAAAUGUGGCUGU | 17 | 1647 |
| myoC-1348 | + | AGGAAAAUGUGGCUGUU | 17 | 1648 |
| myoC-1349 | + | GCUUUCCUGAAGCAUUG | 17 | 1649 |
| myoC-1350 | + | UGAAGCAUUGUGGCUCU | 17 | 1650 |
| myoC-1351 | + | AUUGUGGCUCUCGGUCC | 17 | 1651 |
| myoC-1352 | + | GUCUGACGUGAUCAGUG | 17 | 1652 |
| myoC-1353 | + | UGAUCAGUGAGGACUGA | 17 | 1653 |
| myoC-1354 | + | CCCCUCCACCUCCCCUG | 17 | 1654 |
| myoC-1355 | + | CCCUCACAGAGAAUCAC | 17 | 1655 |
| myoC-1356 | + | CCUCACAGAGAAUCACA | 17 | 1656 |
| myoC-1357 | + | AGAACACGAGAGCUGCA | 17 | 1657 |
| myoC-1358 | + | GAACACGAGAGCUGCAA | 17 | 1658 |
| myoC-1359 | + | UAUAGCAGAGAAGACUA | 17 | 1659 |
| myoC-1360 | + | AGAGAAGACUAUGGCCC | 17 | 1660 |
| myoC-1361 | + | GAGAAGACUAUGGCCCA | 17 | 1661 |
| myoC-1362 | + | AGACUAUGGCCCAGGGA | 17 | 1662 |
| myoC-1363 | + | GGAGAGACACUUGCCCA | 17 | 1663 |
| myoC-1364 | + | GUCGCACAGCCAACCAA | 17 | 1664 |
| myoC-1365 | + | CGAGUCUCCUGAUUCCA | 17 | 1665 |
| myoC-1366 | + | UAAGCCAAGUCCACCAC | 17 | 1666 |
| myoC-1367 | + | AAGCCAAGUCCACCACA | 17 | 1667 |
| myoC-1368 | + | CUUCUUCCGUGAAUUAA | 17 | 1668 |
| myoC-1369 | + | CCGUGAAUUAACGGCCU | 17 | 1669 |
| myoC-1370 | + | AUAUUCCCAUUAAAUAA | 17 | 1670 |
| myoC-1371 | + | AAUAAAGGCCUUCGUGA | 17 | 1671 |
| myoC-1372 | + | AAAACCCAUGCACACCC | 17 | 1672 |
| myoC-1373 | + | GCAGAGAAAAGAUAAAA | 17 | 1673 |
| myoC-1374 | + | AAGAUAAAAAGGCUCAC | 17 | 1674 |
| myoC-1375 | + | AGGCUCACAGGAAGCAA | 17 | 1675 |
| myoC-1376 | + | GAACAACACUGAACAUC | 17 | 1676 |
| myoC-1377 | + | AACAACACUGAACAUCU | 17 | 1677 |
| myoC-1378 | + | CUGCAGUCCCCACCUCC | 17 | 1678 |
| myoC-1379 | + | ACCUCCUGGAAUUCUCC | 17 | 1679 |
| myoC-1380 | + | UGGAAUUCUCCUGGACG | 17 | 1680 |
| myoC-1381 | + | AAUUCUCCUGGACGUGG | 17 | 1681 |
| myoC-1382 | + | AGGCCCCUUUCCCUCUG | 17 | 1682 |
| myoC-1383 | + | CCUCUCCAUUUCCUUUC | 17 | 1683 |
| myoC-1384 | + | AUUUCCUUUCUGGAGCC | 17 | 1684 |
| myoC-1385 | + | UCUGGAGCCUGGAGCCA | 17 | 1685 |
| myoC-1386 | + | UCCAGCUCAGAUGCACC | 17 | 1686 |
| myoC-1387 | - | AGUGACUGCUGACAGCA | 17 | 1687 |
| myoC-1388 | - | GGAGUGACCUGCAGCGC | 17 | 1688 |
| myoC-1389 | - | GAGUGACCUGCAGCGCA | 17 | 1689 |
| myoC-1390 | - | AGUGACCUGCAGCGCAG | 17 | 1690 |
| myoC-1391 | - | GACCUGCAGCGCAGGGG | 17 | 1691 |
| myoC-1392 | - | GAGGAGAAGAAAAAGAG | 17 | 1692 |
| myoC-1393 | - | AGGAGAAGAAAAAGAGA | 17 | 1693 |
| myoC-1394 | - | AAAGACAGAUUCAUUCA | 17 | 1694 |
| myoC-1395 | - | AAGACAGAUUCAUUCAA | 17 | 1695 |
| myoC-1396 | - | GAUUCAUUCAAGGGCAG | 17 | 1696 |
| myoC-1397 | - | AUUCAUUCAAGGGCAGU | 17 | 1697 |
| myoC-1398 | - | CAGUGGGAAUUGACCAC | 17 | 1698 |
| myoC-1399 | - | AGUGGGAAUUGACCACA | 17 | 1699 |
| myoC-1400 | - | UAUAGUCCACGUGAUCC | 17 | 1700 |
| myoC-1401 | - | AUAGUCCACGUGAUCCU | 17 | 1701 |
| myoC-1402 | - | ACGUGAUCCUGGGUUCU | 17 | 1702 |
| myoC-1403 | - | UGAUCCUGGGUUCUAGG | 17 | 1703 |
| myoC-1404 | - | CCUGGGUUCUAGGAGGC | 17 | 1704 |
| myoC-1405 | - | CUGGGUUCUAGGAGGCA | 17 | 1705 |
| myoC-1406 | - | GAGGCAGGGCUAUAUUG | 17 | 1706 |
| myoC-1407 | - | AGGCAGGGCUAUAUUGU | 17 | 1707 |
| myoC-1408 | - | GGCAGGGCUAUAUUGUG | 17 | 1708 |
| myoC-1409 | - | GCAGGGCUAUAUUGUGG | 17 | 1709 |
| myoC-1410 | - | CAGGGCUAUAUUGUGGG | 17 | 1710 |
| myoC-1411 | - | GGGAAAAAAUCAGUUCA | 17 | 1711 |
| myoC-1412 | - | GGAAAAAAUCAGUUCAA | 17 | 1712 |
| myoC-1413 | - | AUCAGUUCAAGGGAAGU | 17 | 1713 |
| myoC-1414 | - | UCAGUUCAAGGGAAGUC | 17 | 1714 |
| myoC-1415 | - | AUUCUGAGCAAGUCACA | 17 | 1715 |
| myoC-1416 | - | UCACAAGGUAGUAACUG | 17 | 1716 |
| myoC-1417 | - | CUUAGUUUCUCCUUAUU | 17 | 1717 |
| myoC-1418 | - | GGAACUCUUUUUCUCUG | 17 | 1718 |
| myoC-1419 | - | GUGGAGUUAGCAGCACA | 17 | 1719 |
| myoC-1420 | - | UGGAGUUAGCAGCACAA | 17 | 1720 |
| myoC-1421 | - | AUCCCGUUUCUUUUAAC | 17 | 1721 |
| myoC-1422 | - | CAAACAGAUUCAAGCCU | 17 | 1722 |
| myoC-1423 | - | CUUGCUGACUAUAUGAU | 17 | 1723 |
| myoC-1424 | - | AUGAGACUAGUACCCUU | 17 | 1724 |
| myoC-1425 | - | GUAAAUGUCUCAAGUUC | 17 | 1725 |
| myoC-1426 | - | ACUGUGUUUCUCCACUC | 17 | 1726 |
| myoC-1427 | - | GUGUUUCUCCACUCUGG | 17 | 1727 |
| myoC-1428 | - | CUGGAGGUGAGUCUGCC | 17 | 1728 |
| myoC-1429 | - | UGGAGGUGAGUCUGCCA | 17 | 1729 |
| myoC-1430 | - | AGUCUGCCAGGGCAGUU | 17 | 1730 |
| myoC-1431 | - | AGUAUUGACACUGUUGU | 17 | 1731 |
| myoC-1432 | - | AACAUAAAGUUGCUCAA | 17 | 1732 |
| myoC-1433 | - | GCAAUCAUUAUUUCAAG | 17 | 1733 |
| myoC-1434 | - | GUUACUUCUGACAGUUU | 17 | 1734 |
| myoC-1435 | - | AGUUUUGGUAUAUUUAU | 17 | 1735 |
| myoC-1436 | - | UUUUUGUUUUUUCUCUU | 17 | 1736 |
| myoC-1437 | - | UUUUGUUUUUUCUCUUU | 17 | 1737 |
| myoC-1438 | - | GUUUAUUAAUGUAAAGC | 17 | 1738 |
| myoC-1439 | - | UUUAUUAAUGUAAAGCA | 17 | 1739 |
| myoC-1440 | - | GCCUGUGAAUUUGAAUG | 17 | 1740 |
| myoC-1441 | - | GAG CCAU AAACU CAAAG | 17 | 1741 |
| myoC-1442 | + | UUACCACUUUGAGUUUA | 17 | 1742 |
| myoC-1443 | + | UAUGGCUCUAUUCGCAA | 17 | 1743 |
| myoC-1444 | + | UGUUAAAUUUAGUUAGA | 17 | 1744 |
| myoC-1445 | + | UAAAUUUAGUUAGAAGG | 17 | 1745 |
| myoC-1446 | + | UCCUCAUUCAAAUUCAC | 17 | 1746 |
| myoC-1447 | + | CAAAUUCACAGGCUUUC | 17 | 1747 |
| myoC-1448 | + | CAGGCUUUCUGGACUGU | 17 | 1748 |
| myoC-1449 | + | AAAAAACAAAAAGCAAA | 17 | 1749 |
| myoC-1450 | + | AUAUUUCCAAACUGCCC | 17 | 1750 |
| myoC-1451 | + | CAGACUCACCUCCAGAG | 17 | 1751 |
| myoC-1452 | + | UUCUAUUCUUAUUUGAU | 17 | 1752 |
| myoC-1453 | + | CUUGAGACAUUUACAAA | 17 | 1753 |
| myoC-1454 | + | UUGAGACAUUUACAAAU | 17 | 1754 |
| myoC-1455 | + | UGUUUACAGCUGACCAA | 17 | 1755 |
| myoC-1456 | + | GUUUACAGCUGACCAAA | 17 | 1756 |
| myoC-1457 | + | UAUAGUCAGCAAGACCU | 17 | 1757 |
| myoC-1458 | + | CUAGGCUUGAAUCUGUU | 17 | 1758 |
| myoC-1459 | + | UGUUUGGCUUUACUCUU | 17 | 1759 |
| myoC-1460 | + | CUUCCUGUUAAAAGAAA | 17 | 1760 |
| myoC-1461 | + | UUCCUGUUAAAAGAAAC | 17 | 1761 |
| myoC-1462 | + | AAAAAGAGUUCCUAAUA | 17 | 1762 |
| myoC-1463 | + | AAUUACUCAGCUUGUAA | 17 | 1763 |
| myoC-1464 | + | AUAUAGUAUUAGAAAUC | 17 | 1764 |
| myoC-1465 | + | CCUGCCUCCUAGAACCC | 17 | 1765 |
| myoC-1466 | + | UAGAACCCAGGAUCACG | 17 | 1766 |
| myoC-1467 | + | GUGGACUAUAAUCCCUG | 17 | 1767 |
| myoC-1468 | + | CUCCUCCCCUGCGCUGC | 17 | 1768 |
| myoC-1469 | + | GUCACUGCUGAGCUGCG | 17 | 1769 |
| myoC-1470 | + | UCACUGCUGAGCUGCGU | 17 | 1770 |
| myoC-1471 | + | CACUGCUGAGCUGCGUG | 17 | 1771 |
| myoC-1472 | + | UGAGCUGCGUGGGGUGC | 17 | 1772 |
| myoC-1473 | + | UGCGUGGGGUGCUGGUC | 17 | 1773 |
| myoC-1474 | + | GCGUGGGGUGCUGGUCA | 17 | 1774 |
| myoC-1475 | - | GAAAUUAGACCUCCUGC | 17 | 1775 |
| myoC-1476 | - | CCCAGAUUUCACCAAUG | 17 | 1776 |
| myoC-1477 | - | UUCACCAAUGAGGUUCU | 17 | 1777 |
| myoC-1478 | - | AGUAAGAACUGAUUUAG | 17 | 1778 |
| myoC-1479 | - | GAGGCUAACAUUGACAU | 17 | 1779 |
| myoC-1480 | - | AAAUCUGCCGCUUCUAU | 17 | 1780 |
| myoC-1481 | - | UAUAGGAAUGCUCUCCC | 17 | 1781 |
| myoC-1482 | - | AUGCUCUCCCUGGAGCC | 17 | 1782 |
| myoC-1483 | - | UCUCCCUGGAGCCUGGU | 17 | 1783 |
| myoC-1484 | - | CUCCCUGGAGCCUGGUA | 17 | 1784 |
| myoC-1485 | - | GUGCUGUCCUUGUGUUC | 17 | 1785 |
| myoC-1486 | + | AAGGACAGCACCCUACC | 17 | 1786 |
| myoC-1487 | + | GCACCCUACCAGGCUCC | 17 | 1787 |
| myoC-1488 | + | CACCCUACCAGGCUCCA | 17 | 1788 |
| myoC-1489 | + | GAGCAUUCCUAUAGAAG | 17 | 1789 |
| myoC-1490 | + | AAACAACUGUGUAUCUU | 17 | 1790 |
| myoC-1491 | + | AACAACUGUGUAUCUUU | 17 | 1791 |
| myoC-1492 | + | UUUCAGUCUUGCAUCUC | 17 | 1792 |
| myoC-1493 | + | CAUGCCAAGAACCUCAU | 17 | 1793 |
| myoC-1494 | + | ACCUCAUUGGUGAAAUC | 17 | 1794 |
| myoC-1495 | + | CCUCAUUGGUGAAAUCU | 17 | 1795 |
| myoC-1496 | + | CUCAUUGGUGAAAUCUG | 17 | 1796 |
| myoC-1497 | + | UAAAAUAUAGAUUACAA | 17 | 1797 |
| myoC-1498 | + | UAAAAACAAGAUCCAGC | 17 | 1798 |
| myoC-1499 | + | AAACAAGAUCCAGCAGG | 17 | 1799 |
| myoC-1500 | + | AUGUCUGUGAUUUCUAU | 17 | 1800 |

**Table 5E** provides exemplary targeting domains for repressing (i.e., knocking down or decreasing) expression of the *MYOC* gene. Any of the targeting domains in the table can be used with a *S*. *aureus* eiCas9 molecule to cause a steric block in the promoter region to block transcription elongation resulting in the repression of the *MYOC* gene. Any of the targeting domains in the table can be used with a *S*. *aureus* eiCas9 fused to a transcriptional repressor to decrease transcription and therefore downregulate gene expression.

**Table 5E**

| **gRNA Name** | **DNA Strand** | **Targeting Domain** | **Target Site Length** | **SEQ ID NO** |
|---|---|---|---|---|
| myoC-1812 | - | GGCAGAGGUUUCCUCUCCAG | 20 | 2032 |
| myoC-676 | - | GCAGAGGUUUCCUCUCCAGC | 20 | 1006 |
| myoC-677 | - | CAGAGGUUUCCUCUCCAGCU | 20 | 1097 |
| myoC-678 | - | AGAGGUUUCCUCUCCAGCUG | 20 | 1085 |
| myoC-679 | - | GAGGUUUCCUCUCCAGCUGG | 20 | 1005 |
| myoC-1817 | - | UGGGGGAGCCCUGCAAGCAC | 20 | 2033 |
| myoC-680 | - | GGGGGAGCCCUGCAAGCACC | 20 | 1020 |
| myoC-1819 | - | CCCUGCAAGCACCCGGGGUC | 20 | 2034 |
| myoC-1820 | - | CACCCGGGGUCCUGGGUGUC | 20 | 2035 |
| myoC-1821 | - | GUUGUUUUGUUAUCACUCUC | 20 | 2036 |
| myoC-686 | - | UUGUUUUGUUAUCACUCUCU | 20 | 1124 |
| myoC-1823 | - | AGGCAUUCAUUGACAAUUUA | 20 | 2037 |
| myoC-1824 | - | UACUUAUAUCUGCCAGACAC | 20 | 2038 |
| myoC-1825 | - | CAGACACCAGAGACAAAAUG | 20 | 2039 |
| myoC-1826 | - | GCAGUCACUGCCCUACCUUC | 20 | 2040 |
| myoC-690 | - | CAGUCACUGCCCUACCUUCG | 20 | 1100 |
| myoC-1828 | - | CGUGGAGGUGACAGUUUCUC | 20 | 2041 |
| myoC-692 | - | GUGGAGGUGACAGUUUCUCA | 20 | 1021 |
| myoC-1830 | - | AGUUUCUCAUGGAAGACGUG | 20 | 2042 |
| myoC-1831 | - | UUCUCAUGGAAGACGUGCAG | 20 | 2043 |
| myoC-1832 | - | CAGCCAACUUAAACCCAGUG | 20 | 2044 |
| myoC-1833 | - | CAACUUAAACCCAGUGCUGA | 20 | 2045 |
| myoC-1834 | - | UUAAACCCAGUGCUGAAAGA | 20 | 2046 |
| myoC-693 | - | UAAACCCAGUGCUGAAAGAA | 20 | 1113 |
| myoC-1836 | - | GAAAGGAAAUAAACACCAUC | 20 | 2047 |
| myoC-1837 | - | AGGAAAUAAACACCAUCUUG | 20 | 2048 |
| myoC-1838 | - | CCCUGCUGCCUCCAUCGUGC | 20 | 2049 |
| myoC-695 | - | CCUGCUGCCUCCAUCGUGCC | 20 | 1104 |
| myoC-1840 | - | GUGCCCGGAGGCCCCCAAGC | 20 | 2050 |
| myoC-1841 | - | GCUGGCCUGCCUCGCUUCCC | 20 | 2051 |
| myoC-1842 | - | CGUGAAUCGUCCUGGUGCAU | 20 | 2052 |
| myoC-1843 | - | AUCGUCCUGGUGCAUCUGAG | 20 | 2053 |
| myoC-1844 | - | UCGUCCUGGUGCAUCUGAGC | 20 | 2054 |
| myoC-1845 | - | GACUCCUUGGCUCCAGGCUC | 20 | 2055 |
| myoC-1846 | - | CCUUGGCUCCAGGCUCCAGA | 20 | 2056 |
| myoC-963 | - | CUUGGCUCCAGGCUCCAGAA | 20 | 1263 |
| myoC-1848 | - | CUCCAGGCUCCAGAAAGGAA | 20 | 2057 |
| myoC-964 | - | UCCAGGCUCCAGAAAGGAAA | 20 | 1264 |
| myoC-1850 | - | CAGGCUCCAGAAAGGAAAUG | 20 | 2058 |
| myoC-1851 | - | GGCUCCAGAAAGGAAAUGGA | 20 | 2059 |
| myoC-965 | - | GCUCCAGAAAGGAAAUGGAG | 20 | 1265 |
| myoC-966 | - | CUCCAGAAAGGAAAUGGAGA | 20 | 1266 |
| myoC-1854 | - | UGGAGAGGGAAACUAGUCUA | 20 | 2060 |
| myoC-967 | - | GGAGAGGGAAACUAGUCUAA | 20 | 1267 |
| myoC-1856 | - | AGAGGGAAACUAGUCUAACG | 20 | 2061 |
| myoC-1857 | - | AAACUAGUCUAACGGAGAAU | 20 | 2062 |
| myoC-968 | - | AACUAGUCUAACGGAGAAUC | 20 | 1268 |
| myoC-1859 | - | CUAGUCUAACGGAGAAUCUG | 20 | 2063 |
| myoC-969 | - | UAGUCUAACGGAGAAUCUGG | 20 | 1269 |
| myoC-970 | - | AGUCUAACGGAGAAUCUGGA | 20 | 1270 |
| myoC-1862 | - | UGGAGGGGACAGUGUUUCCU | 20 | 2064 |
| myoC-1863 | - | GAGGGGACAGUGUUUCCUCA | 20 | 2065 |
| myoC-972 | - | AGGGGACAGUGUUUCCUCAG | 20 | 1272 |
| myoC-973 | - | GGGGACAGUGUUUCCUCAGA | 20 | 1273 |
| myoC-1866 | - | ACAGUGUUUCCUCAGAGGGA | 20 | 2066 |
| myoC-974 | - | CAGUGUUUCCUCAGAGGGAA | 20 | 1274 |
| myoC-1868 | - | GGGAAAGGGGCCUCCACGUC | 20 | 2067 |
| myoC-977 | - | GGAAAGGGGCCUCCACGUCC | 20 | 1277 |
| myoC-1870 | - | AAAGGGGCCUCCACGUCCAG | 20 | 2068 |
| myoC-1871 | - | CUCCACGUCCAGGAGAAUUC | 20 | 2069 |
| myoC-978 | - | UCCACGUCCAGGAGAAUUCC | 20 | 1278 |
| myoC-1873 | - | GUCCAGGAGAAUUCCAGGAG | 20 | 2070 |
| myoC-980 | - | UCCAGGAGAAUUCCAGGAGG | 20 | 1280 |
| myoC-981 | - | CCAGGAGAAUUCCAGGAGGU | 20 | 1281 |
| myoC-1876 | - | AUUCCAGGAGGUGGGGACUG | 20 | 2071 |
| myoC-983 | - | UUCCAGGAGGUGGGGACUGC | 20 | 1283 |
| myoC-984 | - | UCCAGGAGGUGGGGACUGCA | 20 | 1284 |
| myoC-1879 | - | GGAGGUGGGGACUGCAGGGA | 20 | 2072 |
| myoC-985 | - | GAGGUGGGGACUGCAGGGAG | 20 | 1285 |
| myoC-986 | - | AGGUGGGGACUGCAGGGAGU | 20 | 1286 |
| myoC-1882 | - | GACUGCAGGGAGUGGGGACG | 20 | 2073 |
| myoC-988 | - | ACUGCAGGGAGUGGGGACGC | 20 | 1288 |
| myoC-1884 | - | AGGGAGUGGGGACGCUGGGG | 20 | 2074 |
| myoC-1885 | - | AGUGGGGACGCUGGGGCUGA | 20 | 2075 |
| myoC-1886 | - | ACGCUGGGGCUGAGCGGGUG | 20 | 2076 |
| myoC-1887 | - | GCUGAGCGGGUGCUGAAAGG | 20 | 2077 |
| myoC-994 | - | CUGAGCGGGUGCUGAAAGGC | 20 | 1294 |
| myoC-1889 | - | GGGUGCUGAAAGGCAGGAAG | 20 | 2078 |
| myoC-1890 | - | CUGAAAGGCAGGAAGGUGAA | 20 | 2079 |
| myoC-1891 | - | GGAAGGUGAAAAGGGCAAGG | 20 | 2080 |
| myoC-1892 | - | CCAGAUGUUCAGUGUUGUUC | 20 | 2081 |
| myoC-999 | - | CAGAUGUUCAGUGUUGUUCA | 20 | 1299 |
| myoC-1894 | - | GUUCAGUGUUGUUCACGGGG | 20 | 2082 |
| myoC-1002 | - | UUCAGUGUUGUUCACGGGGC | 20 | 1302 |
| myoC-1003 | - | UCAGUGUUGUUCACGGGGCU | 20 | 1303 |
| myoC-1897 | - | GGAGUUUUCCGUUGCUUCCU | 20 | 2083 |
| myoC-1898 | - | CCUUUUUAUCUUUUCUCUGC | 20 | 2084 |
| myoC-1004 | - | CUUUUUAUCUUUUCUCUGCU | 20 | 1304 |
| myoC-1900 | - | UUUUAUCUUUUCUCUGCUUG | 20 | 2085 |
| myoC-1005 | - | UUUAUCUUUUCUCUGCUUGG | 20 | 1305 |
| myoC-1902 | - | UAUCUUUUCUCUGCUUGGAG | 20 | 2086 |
| myoC-1903 | - | CUUUUCUCUGCUUGGAGGAG | 20 | 2087 |
| myoC-1904 | - | GAGGAGAAGAAGUCUAUUUC | 20 | 2088 |
| myoC-1905 | - | GAGAAGAAGUCUAUUUCAUG | 20 | 2089 |
| myoC-1006 | - | AGAAGAAGUCUAUUUCAUGA | 20 | 1306 |
| myoC-1907 | - | AAAGUCAGCUGUUAAAAUUC | 20 | 2090 |
| myoC-1908 | - | GUUAAAAUUCCAGGGUGUGC | 20 | 2091 |
| myoC-1909 | - | GUGUGCAUGGGUUUUCCUUC | 20 | 2092 |
| myoC-1910 | - | UUCACGAAGGCCUUUAUUUA | 20 | 2093 |
| myoC-1013 | - | UCACGAAGGCCUUUAUUUAA | 20 | 1313 |
| myoC-1014 | - | CACGAAGGCCUUUAUUUAAU | 20 | 1314 |
| myoC-1913 | - | GCCUUUAUUUAAUGGGAAUA | 20 | 2094 |
| myoC-1015 | - | CCUUUAUUUAAUGGGAAUAU | 20 | 1315 |
| myoC-1915 | - | AUUUAAUGGGAAUAUAGGAA | 20 | 2095 |
| myoC-1916 | - | AUUUCCUAGGCCGUUAAUUC | 20 | 2096 |
| myoC-1017 | - | UUUCCUAGGCCGUUAAUUCA | 20 | 1317 |
| myoC-1918 | - | CCUAGGCCGUUAAUUCACGG | 20 | 2097 |
| myoC-1919 | - | UUAAUUCACGGAAGAAGUGA | 20 | 2098 |
| myoC-1018 | - | UAAUUCACGGAAGAAGUGAC | 20 | 1318 |
| myoC-1921 | - | AGUCUUUUCUUUCAUGUCUU | 20 | 2099 |
| myoC-1922 | - | GGCAACUACUCAGCCCUGUG | 20 | 2100 |
| myoC-1923 | - | ACUUGGCUUAUGCAAGACGG | 20 | 2101 |
| myoC-1924 | - | AUGCAAGACGGUCGAAAACC | 20 | 2102 |
| myoC-1025 | - | UGCAAGACGGUCGAAAACCU | 20 | 1325 |
| myoC-1926 | - | ACGGUCGAAAACCUUGGAAU | 20 | 2103 |
| myoC-1026 | - | CGGUCGAAAACCUUGGAAUC | 20 | 1326 |
| myoC-1928 | - | CAUUGGUUGGCUGUGCGACC | 20 | 2104 |
| myoC-1929 | - | GGGCAAGUGUCUCUCCUUCC | 20 | 2105 |
| myoC-1930 | - | CCUUGCAGCUCUCGUGUUCU | 20 | 2106 |
| myoC-1931 | - | ACACUUCCCUGUGAUUCUCU | 20 | 2107 |
| myoC-1932 | - | ACUUCCCUGUGAUUCUCUGU | 20 | 2108 |
| myoC-1035 | - | CUUCCCUGUGAUUCUCUGUG | 20 | 1335 |
| myoC-1036 | - | UUCCCUGUGAUUCUCUGUGA | 20 | 1336 |
| myoC-1037 | - | UCCCUGUGAUUCUCUGUGAG | 20 | 1337 |
| myoC-1038 | - | CCCUGUGAUUCUCUGUGAGG | 20 | 1338 |
| myoC-1937 | - | AUUCUCUGUGAGGGGGGAUG | 20 | 2109 |
| myoC-1938 | - | UCUCUGUGAGGGGGGAUGUU | 20 | 2110 |
| myoC-1939 | - | UCUGUGAGGGGGGAUGUUGA | 20 | 2111 |
| myoC-1040 | - | CUGUGAGGGGGGAUGUUGAG | 20 | 1340 |
| myoC-1041 | - | UGUGAGGGGGGAUGUUGAGA | 20 | 1341 |
| myoC-1042 | - | GUGAGGGGGGAUGUUGAGAG | 20 | 1342 |
| myoC-1943 | - | AGGGGGGAUGUUGAGAGGGG | 20 | 2112 |
| myoC-1043 | - | GGGGGGAUGUUGAGAGGGGA | 20 | 1343 |
| myoC-1945 | - | AUGUUGAGAGGGGAAGGAGG | 20 | 2113 |
| myoC-1946 | - | GAGAGGGGAAGGAGGCAGAG | 20 | 2114 |
| myoC-1045 | - | AGAGGGGAAGGAGGCAGAGC | 20 | 1345 |
| myoC-1948 | - | AGGAGGCAGAGCUGGAGCAG | 20 | 2115 |
| myoC-1949 | - | GAGCUGGAGCAGCUGAGCCA | 20 | 2116 |
| myoC-1046 | - | AGCUGGAGCAGCUGAGCCAC | 20 | 1346 |
| myoC-1047 | - | GCUGGAGCAGCUGAGCCACA | 20 | 1347 |
| myoC-1048 | - | CUGGAGCAGCUGAGCCACAG | 20 | 1348 |
| myoC-1953 | - | GCAGCUGAGCCACAGGGGAG | 20 | 2117 |
| myoC-1050 | - | CAGCUGAGCCACAGGGGAGG | 20 | 1350 |
| myoC-1955 | - | GCUGAGCCACAGGGGAGGUG | 20 | 2118 |
| myoC-1051 | - | CUGAGCCACAGGGGAGGUGG | 20 | 1351 |
| myoC-1052 | - | UGAGCCACAGGGGAGGUGGA | 20 | 1352 |
| myoC-1053 | - | GAGCCACAGGGGAGGUGGAG | 20 | 1353 |
| myoC-1959 | - | ACAGGGGAGGUGGAGGGGGA | 20 | 2119 |
| myoC-1055 | - | CAGGGGAGGUGGAGGGGGAC | 20 | 1355 |
| myoC-1961 | - | GAGGGGGACAGGAAGGCAGG | 20 | 2120 |
| myoC-1962 | - | GACAGGAAGGCAGGCAGAAG | 20 | 2121 |
| myoC-1963 | - | UCACUGAUCACGUCAGACUC | 20 | 2122 |
| myoC-1964 | - | GAUCACGUCAGACUCCAGGA | 20 | 2123 |
| myoC-1965 | - | UCACGUCAGACUCCAGGACC | 20 | 2124 |
| myoC-1966 | - | GACCGAGAGCCACAAUGCUU | 20 | 2125 |
| myoC-1061 | - | ACCGAGAGCCACAAUGCUUC | 20 | 1361 |
| myoC-1968 | - | CAAUGCUUCAGGAAAGCUCA | 20 | 2126 |
| myoC-1969 | - | GGCAUUUGCCAAUAACCAAA | 20 | 2127 |
| myoC-1970 | - | GCCAAUAACCAAAAAGAAUG | 20 | 2128 |
| myoC-1971 | - | UUUUGCCUGGCAUUCAAAAA | 20 | 2129 |
| myoC-1972 | - | CUGGCAUUCAAAAACUGGGC | 20 | 2130 |
| myoC-1973 | - | CAAAAACUGGGCCAGAGCAA | 20 | 2131 |
| myoC-1068 | - | AAAAACUGGGCCAGAGCAAG | 20 | 1368 |
| myoC-1975 | - | CCAGAGCAAGUGGAAAAUGC | 20 | 2132 |
| myoC-1976 | - | CAGCAGUGACUGCUGACAGC | 20 | 2133 |
| myoC-1117 | - | AGCAGUGACUGCUGACAGCA | 20 | 1417 |
| myoC-1978 | - | GCACGGAGUGACCUGCAGCG | 20 | 2134 |
| myoC-1118 | - | CACGGAGUGACCUGCAGCGC | 20 | 1418 |
| myoC-1119 | - | ACGGAGUGACCUGCAGCGCA | 20 | 1419 |
| myoC-1120 | - | CGGAGUGACCUGCAGCGCAG | 20 | 1420 |
| myoC-1982 | - | GAGUGACCUGCAGCGCAGGG | 20 | 2135 |
| myoC-1121 | - | AGUGACCUGCAGCGCAGGGG | 20 | 1421 |
| myoC-1984 | - | UGACCUGCAGCGCAGGGGAG | 20 | 2136 |
| myoC-1985 | - | CCUGCAGCGCAGGGGAGGAG | 20 | 2137 |
| myoC-1986 | - | GCGCAGGGGAGGAGAAGAAA | 20 | 2138 |
| myoC-1987 | - | GCAGGGGAGGAGAAGAAAAA | 20 | 2139 |
| myoC-1988 | - | AGGGGAGGAGAAGAAAAAGA | 20 | 2140 |
| myoC-1122 | - | GGGGAGGAGAAGAAAAAGAG | 20 | 1422 |
| myoC-1990 | - | GAAAAAGAGAGGGAUAGUGU | 20 | 2141 |
| myoC-1991 | - | GAGAGGGAUAGUGUAUGAGC | 20 | 2142 |
| myoC-1992 | - | CAAGAAAGACAGAUUCAUUC | 20 | 2143 |
| myoC-1993 | - | GACAGAUUCAUUCAAGGGCA | 20 | 2144 |
| myoC-1126 | - | ACAGAUUCAUUCAAGGGCAG | 20 | 1426 |
| myoC-1127 | - | CAGAUUCAUUCAAGGGCAGU | 20 | 1427 |
| myoC-1996 | - | AGGGCAGUGGGAAUUGACCA | 20 | 2145 |
| myoC-1128 | - | GGGCAGUGGGAAUUGACCAC | 20 | 1428 |
| myoC-1998 | - | GGAUUAUAGUCCACGUGAUC | 20 | 2146 |
| myoC-1999 | - | GUCCACGUGAUCCUGGGUUC | 20 | 2147 |
| myoC-1132 | - | UCCACGUGAUCCUGGGUUCU | 20 | 1432 |
| myoC-2001 | - | UGAUCCUGGGUUCUAGGAGG | 20 | 2148 |
| myoC-2002 | - | CUAGGAGGCAGGGCUAUAUU | 20 | 2149 |
| myoC-1136 | - | UAGGAGGCAGGGCUAUAUUG | 20 | 1436 |
| myoC-1137 | - | AGGAGGCAGGGCUAUAUUGU | 20 | 1437 |
| myoC-1138 | - | GGAGGCAGGGCUAUAUUGUG | 20 | 1438 |
| myoC-1139 | - | GAGGCAGGGCUAUAUUGUGG | 20 | 1439 |
| myoC-1140 | - | AGGCAGGGCUAUAUUGUGGG | 20 | 1440 |
| myoC-2008 | - | UGGGGGGAAAAAAUCAGUUC | 20 | 2150 |
| myoC-1141 | - | GGGGGGAAAAAAUCAGUUCA | 20 | 1441 |
| myoC-1142 | - | GGGGGAAAAAAUCAGUUCAA | 20 | 1442 |
| myoC-2011 | - | AAAAAUCAGUUCAAGGGAAG | 20 | 2151 |
| myoC-1143 | - | AAAAUCAGUUCAAGGGAAGU | 20 | 1443 |
| myoC-1144 | - | AAAUCAGUUCAAGGGAAGUC | 20 | 1444 |
| myoC-2014 | - | CUAUAUUUUUCCUUUACAAG | 20 | 2152 |
| myoC-2015 | - | CCUUUACAAGCUGAGUAAUU | 20 | 2153 |
| myoC-2016 | - | AGCAAGUCACAAGGUAGUAA | 20 | 2154 |
| myoC-2017 | - | AUUACUUAGUUUCUCCUUAU | 20 | 2155 |
| myoC-1147 | - | UUACUUAGUUUCUCCUUAUU | 20 | 1447 |
| myoC-2019 | - | AUUAGGAACUCUUUUUCUCU | 20 | 2156 |
| myoC-1148 | - | UUAGGAACUCUUUUUCUCUG | 20 | 1448 |
| myoC-2021 | - | CUCUGUGGAGUUAGCAGCAC | 20 | 2157 |
| myoC-2022 | - | GGCAAUCCCGUUUCUUUUAA | 20 | 2158 |
| myoC-1151 | - | GCAAUCCCGUUUCUUUUAAC | 20 | 1451 |
| myoC-2024 | - | AUCCCGUUUCUUUUAACAGG | 20 | 2159 |
| myoC-2025 | - | AACAGGAAGAAAACAUUCCU | 20 | 2160 |
| myoC-2026 | - | CUGACUAUAUGAUUGGUUUU | 20 | 2161 |
| myoC-2027 | - | GCGAUGUUUACUAUCUGAUU | 20 | 2162 |
| myoC-2028 | - | UUUACUAUCUGAUUCAGAAA | 20 | 2163 |
| myoC-2029 | - | CUCAAGUUCAGGCUUAACUG | 20 | 2164 |
| myoC-2030 | - | AACUGCAGAACCAAUCAAAU | 20 | 2165 |
| myoC-2031 | - | CAGAACCAAUCAAAUAAGAA | 20 | 2166 |
| myoC-2032 | - | UCAAAUAAGAAUAGAAUCUU | 20 | 2167 |
| myoC-2033 | - | GCAAACUGUGUUUCUCCACU | 20 | 2168 |
| myoC-1156 | - | CAAACUGUGUUUCUCCACUC | 20 | 1456 |
| myoC-2035 | - | UGUGUUUCUCCACUCUGGAG | 20 | 2169 |
| myoC-2036 | - | CACUCUGGAGGUGAGUCUGC | 20 | 2170 |
| myoC-2037 | - | GGUGAGUCUGCCAGGGCAGU | 20 | 2171 |
| myoC-1160 | - | GUGAGUCUGCCAGGGCAGUU | 20 | 1460 |
| myoC-2039 | - | UUGCUUUUUGUUUUUUCUCU | 20 | 2172 |
| myoC-2040 | - | UUGGGUUUAUUAAUGUAAAG | 20 | 2173 |
| myoC-1168 | - | UGGGUUUAUUAAUGUAAAGC | 20 | 1468 |
| myoC-2042 | - | GGGAUUAUUAACCUACAGUC | 20 | 2174 |
| myoC-2043 | - | ACCUACAGUCCAGAAAGCCU | 20 | 2175 |
| myoC-2044 | - | AGUCCAGAAAGCCUGUGAAU | 20 | 2176 |
| myoC-2045 | - | CAGAAAGCCUGUGAAUUUGA | 20 | 2177 |
| myoC-2046 | - | GAAAGCCUGUGAAUUUGAAU | 20 | 2178 |
| myoC-1170 | - | AAAGCCUGUGAAUUUGAAUG | 20 | 1470 |
| myoC-2048 | - | AUUUAACAUUUUAUUCCAUU | 20 | 2179 |
| myoC-2049 | - | ACAUUUUAUUCCAUUGCGAA | 20 | 2180 |
| myoC-2050 | - | UGUGAUUUUGUCAUUACCAA | 20 | 2181 |
| myoC-2051 | - | UUGUUGCAGAUACGUUGUAA | 20 | 2182 |
| myoC-2052 | - | UAUUUAUACUCAAAACUACU | 20 | 2183 |
| myoC-2053 | - | CUUUGAAAUUAGACCUCCUG | 20 | 2184 |
| myoC-2054 | - | GUAAUCUAUAUUUUAUAUAU | 20 | 2185 |
| myoC-2055 | - | AUAUAUUUGAAAACAUCUUU | 20 | 2186 |
| myoC-2056 | - | AUAUUUGAAAACAUCUUUCU | 20 | 2187 |
| myoC-2057 | - | UUUGAAAACAUCUUUCUGAG | 20 | 2188 |
| myoC-2058 | - | GAGUUCCCCAGAUUUCACCA | 20 | 2189 |
| myoC-2059 | - | GUUCUUGGCAUGCACACACA | 20 | 2190 |
| myoC-2060 | - | GGCAUGCACACACACAGAGU | 20 | 2191 |
| myoC-2061 | - | ACACAGAGUAAGAACUGAUU | 20 | 2192 |
| myoC-2062 | - | GCUAACAUUGACAUUGGUGC | 20 | 2193 |
| myoC-2063 | - | UUGGUGCCUGAGAUGCAAGA | 20 | 2194 |
| myoC-2064 | - | CUGAGAUGCAAGACUGAAAU | 20 | 2195 |
| myoC-2065 | - | AUACACAGUUGUUUUAAAGC | 20 | 2196 |
| myoC-2066 | - | UACACAGUUGUUUUAAAGCU | 20 | 2197 |
| myoC-2067 | - | CAGUUGUUUUAAAGCUAGGG | 20 | 2198 |
| myoC-2068 | - | GUUGUUUUAAAGCUAGGGGU | 20 | 2199 |
| myoC-2069 | - | UUGUUUUAAAGCUAGGGGUG | 20 | 2200 |
| myoC-2070 | - | UGUUUUAAAGCUAGGGGUGA | 20 | 2201 |
| myoC-2071 | - | GUUUUAAAGCUAGGGGUGAG | 20 | 2202 |
| myoC-2072 | - | UUUUAAAGCUAGGGGUGAGG | 20 | 2203 |
| myoC-2073 | - | UUUAAAGCUAGGGGUGAGGG | 20 | 2204 |
| myoC-2074 | - | GGGGAAAUCUGCCGCUUCUA | 20 | 2205 |
| myoC-1210 | - | GGGAAAUCUGCCGCUUCUAU | 20 | 1510 |
| myoC-2076 | - | CUUCUAUAGGAAUGCUCUCC | 20 | 2206 |
| myoC-1211 | - | UUCUAUAGGAAUGCUCUCCC | 20 | 1511 |
| myoC-2078 | - | AUGCUCUCCCUGGAGCCUGG | 20 | 2207 |
| myoC-2079 | - | UCUGUCCCUGCUACGUCUUA | 20 | 2208 |
| myoC-2080 | - | CUACGUCUUAAAGGACUUGU | 20 | 2209 |
| myoC-2081 | - | UGGCACAGUGCAGGUUCUCA | 20 | 2210 |
| myoC-2082 | - | GCAGGUUCUCAAUGAGUUUG | 20 | 2211 |
| myoC-2083 | - | GUUCUCAAUGAGUUUGCAGA | 20 | 2212 |
| myoC-2084 | - | UCAAUGAGUUUGCAGAGUGA | 20 | 2213 |
| myoC-833 | - | CAAUGAGUUUGCAGAGUGAA | 20 | 1188 |
| myoC-2086 | - | GAGUGAAUGGAAAUAUAAAC | 20 | 2214 |
| myoC-2087 | - | AAACUAGAAAUAUAUCCUUG | 20 | 2215 |
| myoC-2088 | - | GUGUGUGUGUGUAAAACCAG | 20 | 2216 |
| myoC-836 | - | UGUGUGUGUGUAAAACCAGG | 20 | 1218 |
| myoC-2090 | - | UGUAAAACCAGGUGGAGAUA | 20 | 2217 |
| myoC-837 | - | GUAAAACCAGGUGGAGAUAU | 20 | 994 |
| myoC-2092 | - | UGGAGAUAUAGGAACUAUUA | 20 | 2218 |
| myoC-838 | - | GGAGAUAUAGGAACUAUUAU | 20 | 991 |
| myoC-2094 | - | AUAGGAACUAUUAUUGGGGU | 20 | 2219 |
| myoC-2095 | - | UUGGGGUAUGGGUGCAUAAA | 20 | 2220 |
| myoC-843 | - | UGGGGUAUGGGUGCAUAAAU | 20 | 1214 |
| myoC-2097 | - | AUUGGGAUGUUCUUUUUAAA | 20 | 2221 |
| myoC-2098 | - | AAGAAACUCCAAACAGACUU | 20 | 2222 |
| myoC-845 | - | AGAAACUCCAAACAGACUUC | 20 | 1179 |
| myoC-2100 | - | CUUCUGGAAGGUUAUUUUCU | 20 | 2223 |
| myoC-2101 | - | CUAAGAAUCUUGCUGGCAGC | 20 | 2224 |
| myoC-2102 | - | GGCCACCUCUGUCUUCCCCC | 20 | 2225 |
| myoC-2103 | - | CACCUCUGUCUUCCCCCAUG | 20 | 2226 |
| myoC-2104 | - | CCCAGUAUAUAUAAACCUCU | 20 | 2227 |
| myoC-853 | - | CCAGUAUAUAUAAACCUCUC | 20 | 1197 |
| myoC-2106 | - | UAUAUAAACCUCUCUGGAGC | 20 | 2228 |
| myoC-2107 | - | AACCUCUCUGGAGCUCGGGC | 20 | 2229 |
| myoC-2108 | - | CCAGGCACCUCUCAGCACAG | 20 | 2230 |
| myoC-2109 | - | CUCAGCACAGCAGAGCUUUC | 20 | 2231 |
| myoC-2110 | - | CAGCACAGCAGAGCUUUCCA | 20 | 2232 |
| myoC-2111 | - | AGCACAGCAGAGCUUUCCAG | 20 | 2233 |
| myoC-749 | + | CUGGAGAGGAAACCUCUGCC | 20 | 1110 |
| myoC-748 | + | GCUGGAGAGGAAACCUCUGC | 20 | 1010 |
| myoC-2114 | + | AGCUGGAGAGGAAACCUCUG | 20 | 2234 |
| myoC-747 | + | CAGGGCUCCCCCAGCUGGAG | 20 | 1099 |
| myoC-2116 | + | GCAGGGCUCCCCCAGCUGGA | 20 | 2235 |
| myoC-2117 | + | UUGCAGGGCUCCCCCAGCUG | 20 | 2236 |
| myoC-746 | + | GCUUGCAGGGCUCCCCCAGC | 20 | 1012 |
| myoC-2119 | + | UGCUUGCAGGGCUCCCCCAG | 20 | 2237 |
| myoC-2120 | + | CCCAGGACCCCGGGUGCUUG | 20 | 2238 |
| myoC-2121 | + | UGCUCAGGACACCCAGGACC | 20 | 2239 |
| myoC-2122 | + | GGCAGGUUGCUCAGGACACC | 20 | 2240 |
| myoC-2123 | + | GCACGGGCUGGCAGGUUGCU | 20 | 2241 |
| myoC-2124 | + | AUAACAAAACAACCAGUGGC | 20 | 2242 |
| myoC-2125 | + | UAGAAAGCAACAGGUCCCUA | 20 | 2243 |
| myoC-2126 | + | AAUAGAAAGCAACAGGUCCC | 20 | 2244 |
| myoC-2127 | + | AUGAACGAGUCACACAGAAA | 20 | 2245 |
| myoC-2128 | + | GGAUGAAUGAACGAGUCACA | 20 | 2246 |
| myoC-2129 | + | AUGAAUGCCUGGAUGAAUGA | 20 | 2247 |
| myoC-2130 | + | GUCAAUGAAUGCCUGGAUGA | 20 | 2248 |
| myoC-2131 | + | AAUUGUCAAUGAAUGCCUGG | 20 | 2249 |
| myoC-2132 | + | AAUAAAUUGUCAAUGAAUGC | 20 | 2250 |
| myoC-2133 | + | AAGUACUCAAUAAAUUGUCA | 20 | 2251 |
| myoC-2134 | + | AACUGUCACCUCCACGAAGG | 20 | 2252 |
| myoC-2135 | + | CAUGAGAAACUGUCACCUCC | 20 | 2253 |
| myoC-2136 | + | UUCUUCUGCACGUCUUCCAU | 20 | 2254 |
| myoC-2137 | + | UUUUCUUCUGCACGUCUUCC | 20 | 2255 |
| myoC-2138 | + | UUAUUUCCUUUCUUUCAGCA | 20 | 2256 |
| myoC-721 | + | CUAGGGAGGUGGCCUUGUUA | 20 | 1106 |
| myoC-2140 | + | GCUAGGGAGGUGGCCUUGUU | 20 | 2257 |
| myoC-718 | + | GGAGGCAGCAGGGGGCGCUA | 20 | 1015 |
| myoC-717 | + | UGGAGGCAGCAGGGGGCGCU | 20 | 1120 |
| myoC-2143 | + | AUGGAGGCAGCAGGGGGCGC | 20 | 2258 |
| myoC-714 | + | CGGGCACGAUGGAGGCAGCA | 20 | 1105 |
| myoC-713 | + | CCGGGCACGAUGGAGGCAGC | 20 | 1102 |
| myoC-2146 | + | UCCGGGCACGAUGGAGGCAG | 20 | 2259 |
| myoC-711 | + | UUGGGGGCCUCCGGGCACGA | 20 | 1123 |
| myoC-2148 | + | CUUGGGGGCCUCCGGGCACG | 20 | 2260 |
| myoC-2149 | + | AGACUCGGGCUUGGGGGCCU | 20 | 2261 |
| myoC-706 | + | GGCUUGGAAGACUCGGGCUU | 20 | 978 |
| myoC-705 | + | AGGCUUGGAAGACUCGGGCU | 20 | 1091 |
| myoC-2152 | + | GAGGCUUGGAAGACUCGGGC | 20 | 2262 |
| myoC-2153 | + | GAGGAGGAGGCUUGGAAGAC | 20 | 2263 |
| myoC-702 | + | GACUGAUGGAGGAGGAGGCU | 20 | 1004 |
| myoC-2155 | + | UGACUGAUGGAGGAGGAGGC | 20 | 2264 |
| myoC-700 | + | AGCGCUGUGACUGAUGGAGG | 20 | 1088 |
| myoC-2157 | + | CAGCGCUGUGACUGAUGGAG | 20 | 2265 |
| myoC-699 | + | UGCAGCGCUGUGACUGAUGG | 20 | 1118 |
| myoC-2159 | + | CUGCAGCGCUGUGACUGAUG | 20 | 2266 |
| myoC-698 | + | AGCUGCAGCGCUGUGACUGA | 20 | 1089 |
| myoC-2161 | + | CAGCUGCAGCGCUGUGACUG | 20 | 2267 |
| myoC-2162 | + | ACCAGGACGAUUCACGGGAA | 20 | 2268 |
| myoC-2163 | + | GAUGCACCAGGACGAUUCAC | 20 | 2269 |
| myoC-2164 | + | AGAUGCACCAGGACGAUUCA | 20 | 2270 |
| myoC-2165 | + | CAGAUGCACCAGGACGAUUC | 20 | 2271 |
| myoC-2166 | + | AGUCUCCAGCUCAGAUGCAC | 20 | 2272 |
| myoC-1115 | + | CUUUCUGGAGCCUGGAGCCA | 20 | 1415 |
| myoC-2168 | + | CCUUUCUGGAGCCUGGAGCC | 20 | 2273 |
| myoC-1114 | + | UCCAUUUCCUUUCUGGAGCC | 20 | 1414 |
| myoC-2170 | + | CUCCAUUUCCUUUCUGGAGC | 20 | 2274 |
| myoC-1113 | + | UUCCCUCUCCAUUUCCUUUC | 20 | 1413 |
| myoC-2172 | + | UUUCCCUCUCCAUUUCCUUU | 20 | 2275 |
| myoC-1112 | + | UGGAGGCCCCUUUCCCUCUG | 20 | 1412 |
| myoC-2174 | + | GUGGAGGCCCCUUUCCCUCU | 20 | 2276 |
| myoC-2175 | + | ACGUGGAGGCCCCUUUCCCU | 20 | 2277 |
| myoC-1110 | + | UCCUGGAAUUCUCCUGGACG | 20 | 1410 |
| myoC-2177 | + | CUCCUGGAAUUCUCCUGGAC | 20 | 2278 |
| myoC-2178 | + | CCCCACCUCCUGGAAUUCUC | 20 | 2279 |
| myoC-1108 | + | UCCCUGCAGUCCCCACCUCC | 20 | 1408 |
| myoC-2180 | + | CUCCCUGCAGUCCCCACCUC | 20 | 2280 |
| myoC-2181 | + | CCGUGAACAACACUGAACAU | 20 | 2281 |
| myoC-2182 | + | UCCCAGCCCCGUGAACAACA | 20 | 2282 |
| myoC-2183 | + | AACGGAAAACUCCCAGCCCC | 20 | 2283 |
| myoC-1105 | + | AAAAGGCUCACAGGAAGCAA | 20 | 1405 |
| myoC-2185 | + | AAAAAGGCUCACAGGAAGCA | 20 | 2284 |
| myoC-1104 | + | GAAAAGAUAAAAAGGCUCAC | 20 | 1404 |
| myoC-2187 | + | AGAAAAGAUAAAAAGGCUCA | 20 | 2285 |
| myoC-2188 | + | GACUUCUUCUCCUCCAAGCA | 20 | 2286 |
| myoC-2189 | + | UAGACUUCUUCUCCUCCAAG | 20 | 2287 |
| myoC-2190 | + | UUAUGAAACUGCAUCCCUUC | 20 | 2288 |
| myoC-2191 | + | GAAUUUUAACAGCUGACUUU | 20 | 2289 |
| myoC-1102 | + | AGGAAAACCCAUGCACACCC | 20 | 1402 |
| myoC-2193 | + | AAGGAAAACCCAUGCACACC | 20 | 2290 |
| myoC-1101 | + | UUAAAUAAAGGCCUUCGUGA | 20 | 1401 |
| myoC-2195 | + | AUUAAAUAAAGGCCUUCGUG | 20 | 2291 |
| myoC-2196 | + | CCCAUUAAAUAAAGGCCUUC | 20 | 2292 |
| myoC-2197 | + | GUGAAUUAACGGCCUAGGAA | 20 | 2293 |
| myoC-1099 | + | CUUCCGUGAAUUAACGGCCU | 20 | 1399 |
| myoC-2199 | + | UCUUCCGUGAAUUAACGGCC | 20 | 2294 |
| myoC-2200 | + | AGACUCCAGUCACUUCUUCC | 20 | 2295 |
| myoC-2201 | + | UAGUUGCCCAGAAGACAUGA | 20 | 2296 |
| myoC-2202 | + | UGAGUAGUUGCCCAGAAGAC | 20 | 2297 |
| myoC-2203 | + | CACAGGGCUGAGUAGUUGCC | 20 | 2298 |
| myoC-2204 | + | AAGCCAAGUCCACCACAGGG | 20 | 2299 |
| myoC-2205 | + | UGCAUAAGCCAAGUCCACCA | 20 | 2300 |
| myoC-2206 | + | UGGCAGAACCAGAAAGAAAA | 20 | 2301 |
| myoC-2207 | + | CAACCAAUGGCAGAACCAGA | 20 | 2302 |
| myoC-2208 | + | CAGCCAACCAAUGGCAGAAC | 20 | 2303 |
| myoC-2209 | + | GUCGCACAGCCAACCAAUGG | 20 | 2304 |
| myoC-2210 | + | AAGACUAUGGCCCAGGGAAG | 20 | 2305 |
| myoC-1092 | + | AGAAGACUAUGGCCCAGGGA | 20 | 1392 |
| myoC-2212 | + | GAGAAGACUAUGGCCCAGGG | 20 | 2306 |
| myoC-1091 | + | GCAGAGAAGACUAUGGCCCA | 20 | 1391 |
| myoC-1090 | + | AGCAGAGAAGACUAUGGCCC | 20 | 1390 |
| myoC-2215 | + | UAGCAGAGAAGACUAUGGCC | 20 | 2307 |
| myoC-2216 | + | CUGCAAGGGUCUUUAUAGCA | 20 | 2308 |
| myoC-2217 | + | AGCUGCAAGGGUCUUUAUAG | 20 | 2309 |
| myoC-2218 | + | UCACAGAACACGAGAGCUGC | 20 | 2310 |
| myoC-2219 | + | GGGAAGUGUUCACAGAACAC | 20 | 2311 |
| myoC-2220 | + | CAGGGAAGUGUUCACAGAAC | 20 | 2312 |
| myoC-2221 | + | GAAUCACAGGGAAGUGUUCA | 20 | 2313 |
| myoC-1086 | + | CCCCCUCACAGAGAAUCACA | 20 | 1386 |
| myoC-1085 | + | CCCCCCUCACAGAGAAUCAC | 20 | 1385 |
| myoC-2224 | + | UCCCCCCUCACAGAGAAUCA | 20 | 2314 |
| myoC-2225 | + | UCUCAACAUCCCCCCUCACA | 20 | 2315 |
| myoC-2226 | + | CCUCUCAACAUCCCCCCUCA | 20 | 2316 |
| myoC-1083 | + | ACGUGAUCAGUGAGGACUGA | 20 | 1383 |
| myoC-2228 | + | GACGUGAUCAGUGAGGACUG | 20 | 2317 |
| myoC-2229 | + | UGGAGUCUGACGUGAUCAGU | 20 | 2318 |
| myoC-2230 | + | CCUGGAGUCUGACGUGAUCA | 20 | 2319 |
| myoC-1081 | + | AGCAUUGUGGCUCUCGGUCC | 20 | 1381 |
| myoC-2232 | + | AAGCAUUGUGGCUCUCGGUC | 20 | 2320 |
| myoC-2233 | + | GUUGGGUUCAUUGAGCUUUC | 20 | 2321 |
| myoC-2234 | + | AAAUGUGGCUGUUGGGUUCA | 20 | 2322 |
| myoC-2235 | + | AGGGAAGGAAAAUGUGGCUG | 20 | 2323 |
| myoC-1075 | + | CCAUUGUCUAUGCUUAGGGA | 20 | 1375 |
| myoC-2237 | + | GCCAUUGUCUAUGCUUAGGG | 20 | 2324 |
| myoC-1074 | + | AAUGCCAUUGUCUAUGCUUA | 20 | 1374 |
| myoC-1073 | + | AAAUGCCAUUGUCUAUGCUU | 20 | 1373 |
| myoC-2240 | + | CAAAUGCCAUUGUCUAUGCU | 20 | 2325 |
| myoC-2241 | + | CACUUGCUCUGGCCCAGUUU | 20 | 2326 |
| myoC-2242 | + | UGCGUGGGGUGCUGGUCAGG | 20 | 2327 |
| myoC-2243 | + | GAGCUGCGUGGGGUGCUGGU | 20 | 2328 |
| myoC-1199 | + | GCAGUCACUGCUGAGCUGCG | 20 | 1499 |
| myoC-2245 | + | AGCAGUCACUGCUGAGCUGC | 20 | 2329 |
| myoC-2246 | + | CGUGCUGUCAGCAGUCACUG | 20 | 2330 |
| myoC-2247 | + | UCAAUUCCCACUGCCCUUGA | 20 | 2331 |
| myoC-2248 | + | GUGGUCAAUUCCCACUGCCC | 20 | 2332 |
| myoC-2249 | + | CUCCUAGAACCCAGGAUCAC | 20 | 2333 |
| myoC-2250 | + | UAGCCCUGCCUCCUAGAACC | 20 | 2334 |
| myoC-2251 | + | CACAAUAUAGCCCUGCCUCC | 20 | 2335 |
| myoC-2252 | + | AUCAGGUCUCCCGACUUCCC | 20 | 2336 |
| myoC-2253 | + | GUAAAGGAAAAAUAUAGUAU | 20 | 2337 |
| myoC-1193 | + | CAGAAUUACUCAGCUUGUAA | 20 | 1493 |
| myoC-2255 | + | UCAGAAUUACUCAGCUUGUA | 20 | 2338 |
| myoC-2256 | + | UUACUACCUUGUGACUUGCU | 20 | 2339 |
| myoC-2257 | + | GAAAAAGAGUUCCUAAUAAG | 20 | 2340 |
| myoC-1192 | + | GAGAAAAAGAGUUCCUAAUA | 20 | 1492 |
| myoC-2259 | + | AGAGAAAAAGAGUUCCUAAU | 20 | 2341 |
| myoC-2260 | + | CUGCUAACUCCACAGAGAAA | 20 | 2342 |
| myoC-2261 | + | CUUGUGCUGCUAACUCCACA | 20 | 2343 |
| myoC-2262 | + | CCCUUGUGCUGCUAACUCCA | 20 | 2344 |
| myoC-1190 | + | UUUCUUCCUGUUAAAAGAAA | 20 | 1490 |
| myoC-2264 | + | UUUUCUUCCUGUUAAAAGAA | 20 | 2345 |
| myoC-2265 | + | GAAUGUUUUCUUCCUGUUAA | 20 | 2346 |
| myoC-1189 | + | AUCUGUUUGGCUUUACUCUU | 20 | 1489 |
| myoC-2267 | + | AAUCUGUUUGGCUUUACUCU | 20 | 2347 |
| myoC-2268 | + | AUAGUCAGCAAGACCUAGGC | 20 | 2348 |
| myoC-2269 | + | GAAUCAGAUAGUAAACAUCG | 20 | 2349 |
| myoC-2270 | + | AAGGGUACUAGUCUCAUUUU | 20 | 2350 |
| myoC-2271 | + | UGUUUGUUUACAGCUGACCA | 20 | 2351 |
| myoC-2272 | + | UGAACUUGAGACAUUUACAA | 20 | 2352 |
| myoC-2273 | + | UUCUGCAGUUAAGCCUGAAC | 20 | 2353 |
| myoC-2274 | + | GAUUGGUUCUGCAGUUAAGC | 20 | 2354 |
| myoC-2275 | + | GCAGACUCACCUCCAGAGUG | 20 | 2355 |
| myoC-1181 | + | UGGCAGACUCACCUCCAGAG | 20 | 1481 |
| myoC-2277 | + | CUGGCAGACUCACCUCCAGA | 20 | 2356 |
| myoC-2278 | + | UGCCCUGGCAGACUCACCUC | 20 | 2357 |
| myoC-2279 | + | CAACAACAGUGUCAAUACUU | 20 | 2358 |
| myoC-2280 | + | ACUUGAAAUAAUGAUUGCCU | 20 | 2359 |
| myoC-2281 | + | CAGAAGUAACUUUAAGCCAC | 20 | 2360 |
| myoC-2282 | + | AAUAAAUAUACCAAAACUGU | 20 | 2361 |
| myoC-2283 | + | UUUACAUUAAUAAACCCAAA | 20 | 2362 |
| myoC-2284 | + | GCUUUACAUUAAUAAACCCA | 20 | 2363 |
| myoC-2285 | + | CAUUCAAAUUCACAGGCUUU | 20 | 2364 |
| myoC-2286 | + | AAUAAAAUGUUAAAUUUAGU | 20 | 2365 |
| myoC-1173 | + | GUUUAUGGCUCUAUUCGCAA | 20 | 1473 |
| myoC-2288 | + | AGUUUAUGGCUCUAUUCGCA | 20 | 2366 |
| myoC-2289 | + | CAGGUACUGUUAUUACCACU | 20 | 2367 |
| myoC-2290 | + | GGUCUAAUUUCAAAGUAGUU | 20 | 2368 |
| myoC-1228 | + | UGUUAAAAACAAGAUCCAGC | 20 | 1528 |
| myoC-2292 | + | AUGUUAAAAACAAGAUCCAG | 20 | 2369 |
| myoC-2293 | + | UACAAAGGAAACAAAUGAUA | 20 | 2370 |
| myoC-1227 | + | AUAUAAAAUAUAGAUUACAA | 20 | 1527 |
| myoC-2295 | + | UAUAUAAAAUAUAGAUUACA | 20 | 2371 |
| myoC-2296 | + | GAAAUCUGGGGAACUCUUCU | 20 | 2372 |
| myoC-1226 | + | AACCUCAUUGGUGAAAUCUG | 20 | 1526 |
| myoC-1225 | + | GAACCUCAUUGGUGAAAUCU | 20 | 1525 |
| myoC-1224 | + | AGAACCUCAUUGGUGAAAUC | 20 | 1524 |
| myoC-2300 | + | AAGAACCUCAUUGGUGAAAU | 20 | 2373 |
| myoC-2301 | + | GCAUGCCAAGAACCUCAUUG | 20 | 2374 |
| myoC-2302 | + | ACUCUGUGUGUGUGCAUGCC | 20 | 2375 |
| myoC-2303 | + | AAACAACUGUGUAUCUUUGG | 20 | 2376 |
| myoC-1221 | + | UAAAACAACUGUGUAUCUUU | 20 | 1521 |
| myoC-1220 | + | UUAAAACAACUGUGUAUCUU | 20 | 1520 |
| myoC-2306 | + | UUUAAAACAACUGUGUAUCU | 20 | 2377 |
| myoC-2307 | + | CUCCAGGGAGAGCAUUCCUA | 20 | 2378 |
| myoC-2308 | + | GCACCCUACCAGGCUCCAGG | 20 | 2379 |
| myoC-1218 | + | CAGCACCCUACCAGGCUCCA | 20 | 1518 |
| myoC-1217 | + | ACAGCACCCUACCAGGCUCC | 20 | 1517 |
| myoC-2311 | + | GACAGCACCCUACCAGGCUC | 20 | 2380 |
| myoC-2312 | + | AUAACAGCCAGCCAGAACAC | 20 | 2381 |
| myoC-2313 | + | AGAGAAAAAUAACAGCCAGC | 20 | 2382 |
| myoC-2314 | + | UUUAAGACGUAGCAGGGACA | 20 | 2383 |
| myoC-2315 | + | CCUUUAAGACGUAGCAGGGA | 20 | 2384 |
| myoC-893 | + | CAAGUCCUUUAAGACGUAGC | 20 | 1187 |
| myoC-2317 | + | ACAAGUCCUUUAAGACGUAG | 20 | 2385 |
| myoC-892 | + | CCAGGCACUAUGCUAGGAAC | 20 | 1196 |
| myoC-2319 | + | GCCAGGCACUAUGCUAGGAA | 20 | 2386 |
| myoC-891 | + | ACUGUGCCAGGCACUAUGCU | 20 | 1178 |
| myoC-2321 | + | CACUGUGCCAGGCACUAUGC | 20 | 2387 |
| myoC-2322 | + | AUUCACUCUGCAAACUCAUU | 20 | 2388 |
| myoC-2323 | + | CCAUUCACUCUGCAAACUCA | 20 | 2389 |
| myoC-2324 | + | ACUGGUGUGCUGAUUUCAAC | 20 | 2390 |
| myoC-2325 | + | ACACGUACACACACUUACAC | 20 | 2391 |
| myoC-2326 | + | GUUUGGAGUUUCUUUUUAAA | 20 | 2392 |
| myoC-885 | + | UAACCUUCCAGAAGUCUGUU | 20 | 1208 |
| myoC-2328 | + | AUAACCUUCCAGAAGUCUGU | 20 | 2393 |
| myoC-2329 | + | AUUCUUAGAAAAUAACCUUC | 20 | 2394 |
| myoC-2330 | + | CACGCUGCCAGCAAGAUUCU | 20 | 2395 |
| myoC-882 | + | CUGGGUGGGGCUGUGCACAG | 20 | 1205 |
| myoC-881 | + | GCUGGGUGGGGCUGUGCACA | 20 | 1050 |
| myoC-880 | + | GGCUGGGUGGGGCUGUGCAC | 20 | 1051 |
| myoC-2334 | + | AGGCUGGGUGGGGCUGUGCA | 20 | 2396 |
| myoC-877 | + | GGUGGCCACGUGAGGCUGGG | 20 | 1053 |
| myoC-2336 | + | AGGUGGCCACGUGAGGCUGG | 20 | 2397 |
| myoC-2337 | + | ACAGAGGUGGCCACGUGAGG | 20 | 2398 |
| myoC-2338 | + | GGGAAGACAGAGGUGGCCAC | 20 | 2399 |
| myoC-2339 | + | CAGCCCUUCAUGGGGGAAGA | 20 | 2400 |
| myoC-871 | + | GGGGAGCCAGCCCUUCAUGG | 20 | 992 |
| myoC-870 | + | UGGGGAGCCAGCCCUUCAUG | 20 | 1213 |
| myoC-869 | + | CUGGGGAGCCAGCCCUUCAU | 20 | 1204 |
| myoC-868 | + | ACUGGGGAGCCAGCCCUUCA | 20 | 1177 |
| myoC-2344 | + | UACUGGGGAGCCAGCCCUUC | 20 | 2401 |
| myoC-867 | + | AGAGAGGUUUAUAUAUACUG | 20 | 1180 |
| myoC-866 | + | CAGAGAGGUUUAUAUAUACU | 20 | 1191 |
| myoC-865 | + | CCAGAGAGGUUUAUAUAUAC | 20 | 1195 |
| myoC-2348 | + | UCCAGAGAGGUUUAUAUAUA | 20 | 2402 |
| myoC-2349 | + | UGGCUCAUGCCCGAGCUCCA | 20 | 2403 |
| myoC-2350 | + | GCUGGCUCAUGCCCGAGCUC | 20 | 2404 |
| myoC-2351 | + | GUGGCCUUGCUGGCUCAUGC | 20 | 2405 |
| myoC-2352 | + | CUGUGCUGAGAGGUGCCUGG | 20 | 2406 |
| myoC-2353 | + | UCUGCUGUGCUGAGAGGUGC | 20 | 2407 |
| myoC-2354 | + | CUGGAAAGCUCUGCUGUGCU | 20 | 2408 |
| myoC-2355 | + | CUCUGGAAAGCUCUGCUGUG | 20 | 2409 |
| myoC-2356 | + | AGGCUUGGUGAGGCUUCCUC | 20 | 2410 |
| myoC-2357 | + | GAGGCUUGGUGAGGCUUCCU | 20 | 2411 |
| myoC-2358 | - | AGAGGUUUCCUCUCCAG | 17 | 2412 |
| myoC-752 | - | GAGGUUUCCUCUCCAGC | 17 | 1026 |
| myoC-753 | - | AGGUUUCCUCUCCAGCU | 17 | 1142 |
| myoC-754 | - | GGUUUCCUCUCCAGCUG | 17 | 1045 |
| myoC-755 | - | GUUUCCUCUCCAGCUGG | 17 | 1047 |
| myoC-2363 | - | GGGAGCCCUGCAAGCAC | 17 | 2413 |
| myoC-756 | - | GGAGCCCUGCAAGCACC | 17 | 1035 |
| myoC-2365 | - | UGCAAGCACCCGGGGUC | 17 | 2414 |
| myoC-2366 | - | CCGGGGUCCUGGGUGUC | 17 | 2415 |
| myoC-2367 | - | GUUUUGUUAUCACUCUC | 17 | 2416 |
| myoC-762 | - | UUUUGUUAUCACUCUCU | 17 | 1171 |
| myoC-2369 | - | CAUUCAUUGACAAUUUA | 17 | 2417 |
| myoC-2370 | - | UUAUAUCUGCCAGACAC | 17 | 2418 |
| myoC-2371 | - | ACACCAGAGACAAAAU G | 17 | 2419 |
| myoC-2372 | - | GUCACUGCCCUACCUUC | 17 | 2420 |
| myoC-766 | - | UCACUGCCCUACCUUCG | 17 | 1160 |
| myoC-2374 | - | GGAGGUGACAGUUUCUC | 17 | 2421 |
| myoC-768 | - | GAGGUGACAGUUUCUCA | 17 | 1025 |
| myoC-2376 | - | UUCUCAUGGAAGACGUG | 17 | 2422 |
| myoC-2377 | - | UCAUGGAAGACGUGCAG | 17 | 2423 |
| myoC-2378 | - | CCAACUUAAACCCAGUG | 17 | 2424 |
| myoC-2379 | - | CUUAAACCCAGUGCUGA | 17 | 2425 |
| myoC-2380 | - | AACCCAGUGCUGAAAGA | 17 | 2426 |
| myoC-769 | - | ACCCAGUGCUGAAAGAA | 17 | 1130 |
| myoC-2382 | - | AGGAAAUAAACACCAUC | 17 | 2427 |
| myoC-2383 | - | AAAUAAACACCAUCUUG | 17 | 2428 |
| myoC-2384 | - | UGCUGCCUCCAUCGUGC | 17 | 2429 |
| myoC-771 | - | GCUGCCUCCAUCGUGCC | 17 | 1030 |
| myoC-2386 | - | CCCGGAGGCCCCCAAGC | 17 | 2430 |
| myoC-2387 | - | GGCCUGCCUCGCUUCCC | 17 | 2431 |
| myoC-2388 | - | GAAUCGUCCUGGUGCAU | 17 | 2432 |
| myoC-2389 | - | GUCCUGGUGCAUCUGAG | 17 | 2433 |
| myoC-2390 | - | UCCUGGUGCAUCUGAGC | 17 | 2434 |
| myoC-2391 | - | UCCUUGGCUCCAGGCUC | 17 | 2435 |
| myoC-2392 | - | UGGCUCCAGGCUCCAGA | 17 | 2436 |
| myoC-1233 | - | GGCUCCAGGCUCCAGAA | 17 | 1533 |
| myoC-2394 | - | CAGGCUCCAGAAAGGAA | 17 | 2437 |
| myoC-1234 | - | AGGCUCCAGAAAGGAAA | 17 | 1534 |
| myoC-2396 | - | GCUCCAGAAAGGAAAUG | 17 | 2438 |
| myoC-2397 | - | UCCAGAAAGGAAAUGGA | 17 | 2439 |
| myoC-1235 | - | CCAGAAAGGAAAUGGAG | 17 | 1535 |
| myoC-1236 | - | CAGAAAGGAAAUGGAGA | 17 | 1536 |
| myoC-2400 | - | AGAGGGAAACUAGUCUA | 17 | 2440 |
| myoC-1237 | - | GAGGGAAACUAGUCUAA | 17 | 1537 |
| myoC-2402 | - | GGGAAACUAGUCUAACG | 17 | 2441 |
| myoC-2403 | - | CUAGUCUAACGGAGAAU | 17 | 2442 |
| myoC-1238 | - | UAGUCUAACGGAGAAUC | 17 | 1538 |
| myoC-2405 | - | GUCUAACGGAGAAUCUG | 17 | 2443 |
| myoC-1239 | - | UCUAACGGAGAAUCUGG | 17 | 1539 |
| myoC-1240 | - | CUAACGGAGAAUCUGGA | 17 | 1540 |
| myoC-2408 | - | AGGGGACAGUGUUUCCU | 17 | 2444 |
| myoC-2409 | - | GGGACAGUGUUUCCUCA | 17 | 2445 |
| myoC-1242 | - | GGACAGUGUUUCCUCAG | 17 | 1542 |
| myoC-1243 | - | GACAGUGUUUCCUCAGA | 17 | 1543 |
| myoC-2412 | - | GUGUUUCCUCAGAGGGA | 17 | 2446 |
| myoC-1244 | - | UGUUUCCUCAGAGGGAA | 17 | 1544 |
| myoC-2414 | - | AAAGGGGCCUCCACGUC | 17 | 2447 |
| myoC-1247 | - | AAGGGGCCUCCACGUCC | 17 | 1547 |
| myoC-2416 | - | GGGGCCUCCACGUCCAG | 17 | 2448 |
| myoC-2417 | - | CACGUCCAGGAGAAUUC | 17 | 2449 |
| myoC-1248 | - | ACGUCCAGGAGAAUUCC | 17 | 1548 |
| myoC-2419 | - | CAGGAGAAUUCCAGGAG | 17 | 2450 |
| myoC-1250 | - | AGGAGAAUUCCAGGAGG | 17 | 1550 |
| myoC-1251 | - | GGAGAAUUCCAGGAGGU | 17 | 1551 |
| myoC-2422 | - | CCAGGAGGUGGGGACUG | 17 | 2451 |
| myoC-1253 | - | CAGGAGGUGGGGACUGC | 17 | 1553 |
| myoC-1254 | - | AGGAGGUGGGGACUGCA | 17 | 1554 |
| myoC-2425 | - | GGUGGGGACUGCAGGGA | 17 | 2452 |
| myoC-1255 | - | GUGGGGACUGCAGGGAG | 17 | 1555 |
| myoC-1256 | - | UGGGGACUGCAGGGAGU | 17 | 1556 |
| myoC-2428 | - | UGCAGGGAGUGGGGACG | 17 | 2453 |
| myoC-1258 | - | GCAGGGAGUGGGGACGC | 17 | 1558 |
| myoC-2430 | - | GAGUGGGGACGCUGGGG | 17 | 2454 |
| myoC-2431 | - | GGGGACGCUGGGGCUGA | 17 | 2455 |
| myoC-2432 | - | CUGGGGCUGAGCGGGUG | 17 | 2456 |
| myoC-2433 | - | GAGCGGGUGCUGAAAGG | 17 | 2457 |
| myoC-1264 | - | AGCGGGUGCUGAAAGGC | 17 | 1564 |
| myoC-2435 | - | UGCUGAAAGGCAGGAAG | 17 | 2458 |
| myoC-2436 | - | AAAGGCAGGAAGGUGAA | 17 | 2459 |
| myoC-2437 | - | AGGUGAAAAGGGCAAGG | 17 | 2460 |
| myoC-2438 | - | GAUGUUCAGUGUUGUUC | 17 | 2461 |
| myoC-1269 | - | AUGUUCAGUGUUGUUCA | 17 | 1569 |
| myoC-2440 | - | CAGUGUUGUUCACGGGG | 17 | 2462 |
| myoC-1272 | - | AGUGUUGUUCACGGGGC | 17 | 1572 |
| myoC-1273 | - | GUGUUGUUCACGGGGCU | 17 | 1573 |
| myoC-2443 | - | GUUUUCCGUUGCUUCCU | 17 | 2463 |
| myoC-2444 | - | UUUUAUCUUUUCUCUGC | 17 | 2464 |
| myoC-1274 | - | UUUAUCUUUUCUCUGCU | 17 | 1574 |
| myoC-2446 | - | UAUCUUUUCUCUGCUUG | 17 | 2465 |
| myoC-1275 | - | AUCUUUUCUCUGCUUGG | 17 | 1575 |
| myoC-2448 | - | CUUUUCUCUGCUUGGAG | 17 | 2466 |
| myoC-2449 | - | UUCUCUGCUUGGAGGAG | 17 | 2467 |
| myoC-2450 | - | GAGAAGAAGUCUAUUUC | 17 | 2468 |
| myoC-2451 | - | AAGAAGUCUAUUUCAUG | 17 | 2469 |
| myoC-1276 | - | AGAAGUCUAUUUCAUGA | 17 | 1576 |
| myoC-2453 | - | GUCAGCUGUUAAAAUUC | 17 | 2470 |
| myoC-2454 | - | AAAAUUCCAGGGUGUGC | 17 | 2471 |
| myoC-2455 | - | UGCAUGGGUUUUCCUUC | 17 | 2472 |
| myoC-2456 | - | ACGAAGGCCUUUAUUUA | 17 | 2473 |
| myoC-1283 | - | CGAAGGCCUUUAUUUAA | 17 | 1583 |
| myoC-1284 | - | GAAGGCCUUUAUUUAAU | 17 | 1584 |
| myoC-2459 | - | UUUAUUUAAUGGGAAUA | 17 | 2474 |
| myoC-1285 | - | UUAUUUAAUGGGAAUAU | 17 | 1585 |
| myoC-2461 | - | UAAUGGGAAUAUAGGAA | 17 | 2475 |
| myoC-2462 | - | UCCUAGGCCGUUAAUUC | 17 | 2476 |
| myoC-1287 | - | CCUAGGCCGUUAAUUCA | 17 | 1587 |
| myoC-2464 | - | AGGCCGUUAAUUCACGG | 17 | 2477 |
| myoC-2465 | - | AUUCACGGAAGAAGUGA | 17 | 2478 |
| myoC-1288 | - | UUCACGGAAGAAGUGAC | 17 | 1588 |
| myoC-2467 | - | CUUUUCUUUCAUGUCUU | 17 | 2479 |
| myoC-2468 | - | AACUACUCAGCCCUGUG | 17 | 2480 |
| myoC-2469 | - | UGGCUUAUGCAAGACGG | 17 | 2481 |
| myoC-2470 | - | CAAGACGGUCGAAAACC | 17 | 2482 |
| myoC-1295 | - | AAGACGGUCGAAAACCU | 17 | 1595 |
| myoC-2472 | - | GUCGAAAACCUUGGAAU | 17 | 2483 |
| myoC-1296 | - | UCGAAAACCUUGGAAUC | 17 | 1596 |
| myoC-2474 | - | UGGUUGGCUGUGCGACC | 17 | 2484 |
| myoC-2475 | - | CAAGUGUCUCUCCUUCC | 17 | 2485 |
| myoC-2476 | - | UGCAGCUCUCGUGUUCU | 17 | 2486 |
| myoC-2477 | - | CUUCCCUGUGAUUCUCU | 17 | 2487 |
| myoC-2478 | - | UCCCUGUGAUUCUCUGU | 17 | 2488 |
| myoC-1305 | - | CCCUGUGAUUCUCUGUG | 17 | 1605 |
| myoC-1306 | - | CCUGUGAUUCUCUGUGA | 17 | 1606 |
| myoC-1307 | - | CUGUGAUUCUCUGUGAG | 17 | 1607 |
| myoC-1308 | - | UGUGAUUCUCUGUGAGG | 17 | 1608 |
| myoC-2483 | - | CUCUGUGAGGGGGGAUG | 17 | 2489 |
| myoC-2484 | - | CUGUGAGGGGGGAUGUU | 17 | 2490 |
| myoC-2485 | - | GUGAGGGGGGAUGUUGA | 17 | 2491 |
| myoC-1310 | - | UGAGGGGGGAUGUUGAG | 17 | 1610 |
| myoC-1311 | - | GAGGGGGGAUGUUGAGA | 17 | 1611 |
| myoC-1312 | - | AGGGGGGAUGUUGAGAG | 17 | 1612 |
| myoC-2489 | - | GGGGAUGUUGAGAGGGG | 17 | 2492 |
| myoC-1313 | - | GGGAUGUUGAGAGGGGA | 17 | 1613 |
| myoC-2491 | - | UUGAGAGGGGAAGGAGG | 17 | 2493 |
| myoC-2492 | - | AGGGGAAGGAGGCAGAG | 17 | 2494 |
| myoC-1315 | - | GGGGAAGGAGGCAGAGC | 17 | 1615 |
| myoC-2494 | - | AGGCAGAGCUGGAGCAG | 17 | 2495 |
| myoC-2495 | - | CUGGAGCAGCUGAGCCA | 17 | 2496 |
| myoC-1316 | - | UGGAGCAGCUGAGCCAC | 17 | 1616 |
| myoC-1317 | - | GGAGCAGCUGAGCCACA | 17 | 1617 |
| myoC-1318 | - | GAGCAGCUGAGCCACAG | 17 | 1618 |
| myoC-2499 | - | GCUGAGCCACAGGGGAG | 17 | 2497 |
| myoC-1320 | - | CUGAGCCACAGGGGAGG | 17 | 1620 |
| myoC-2501 | - | GAGCCACAGGGGAGGUG | 17 | 2498 |
| myoC-1321 | - | AGCCACAGGGGAGGUGG | 17 | 1621 |
| myoC-1322 | - | GCCACAGGGGAGGUGGA | 17 | 1622 |
| myoC-1323 | - | CCACAGGGGAGGUGGAG | 17 | 1623 |
| myoC-2505 | - | GGGGAGGUGGAGGGGGA | 17 | 2499 |
| myoC-1325 | - | GGGAGGUGGAGGGGGAC | 17 | 1625 |
| myoC-2507 | - | GGGGACAGGAAGGCAGG | 17 | 2500 |
| myoC-2508 | - | AGGAAGGCAGGCAGAAG | 17 | 2501 |
| myoC-2509 | - | CUGAUCACGUCAGACUC | 17 | 2502 |
| myoC-2510 | - | CACGUCAGACUCCAGGA | 17 | 2503 |
| myoC-2511 | - | CGUCAGACUCCAGGACC | 17 | 2504 |
| myoC-2512 | - | CGAGAGCCACAAUGCU U | 17 | 2505 |
| myoC-1331 | - | GAGAGCCACAAUGCUUC | 17 | 1631 |
| myoC-2514 | - | UGCUUCAGGAAAGCUCA | 17 | 2506 |
| myoC-2515 | - | AUUUGCCAAUAACCAAA | 17 | 2507 |
| myoC-2516 | - | AAUAACCAAAAAGAAUG | 17 | 2508 |
| myoC-2517 | - | UGCCUGGCAUUCAAAAA | 17 | 2509 |
| myoC-2518 | - | GCAUUCAAAAACUGGGC | 17 | 2510 |
| myoC-2519 | - | AAACUGGGCCAGAGCAA | 17 | 2511 |
| myoC-1338 | - | AACUGGGCCAGAGCAAG | 17 | 1638 |
| myoC-2521 | - | GAGCAAGUGGAAAAUGC | 17 | 2512 |
| myoC-2522 | - | CAGUGACUGCUGACAGC | 17 | 2513 |
| myoC-1387 | - | AGUGACUGCUGACAGCA | 17 | 1687 |
| myoC-2524 | - | CGGAGUGACCUGCAGCG | 17 | 2514 |
| myoC-1388 | - | GGAGUGACCUGCAGCGC | 17 | 1688 |
| myoC-1389 | - | GAGUGACCUGCAGCGCA | 17 | 1689 |
| myoC-1390 | - | AGUGACCUGCAGCGCAG | 17 | 1690 |
| myoC-2528 | - | UGACCUGCAGCGCAGGG | 17 | 2515 |
| myoC-1391 | - | GACCUGCAGCGCAGGGG | 17 | 1691 |
| myoC-2530 | - | CCUGCAGCGCAGGGGAG | 17 | 2516 |
| myoC-2531 | - | GCAGCGCAGGGGAGGAG | 17 | 2517 |
| myoC-2532 | - | CAGGGGAGGAGAAGAAA | 17 | 2518 |
| myoC-2533 | - | GGGGAGGAGAAGAAAAA | 17 | 2519 |
| myoC-2534 | - | GGAGGAGAAGAAAAAGA | 17 | 2520 |
| myoC-1392 | - | GAGGAGAAGAAAAAGAG | 17 | 1692 |
| myoC-2536 | - | AAAGAGAGGGAUAGUGU | 17 | 2521 |
| myoC-2537 | - | AGGGAUAGUGUAUGAGC | 17 | 2522 |
| myoC-2538 | - | GAAAGACAGAUUCAUUC | 17 | 2523 |
| myoC-2539 | - | AGAUUCAUUCAAGGGCA | 17 | 2524 |
| myoC-1396 | - | GAUUCAUUCAAGGGCAG | 17 | 1696 |
| myoC-1397 | - | AUUCAUUCAAGGGCAGU | 17 | 1697 |
| myoC-2542 | - | GCAGUGGGAAUUGACCA | 17 | 2525 |
| myoC-1398 | - | CAGUGGGAAUUGACCAC | 17 | 1698 |
| myoC-2544 | - | UUAUAGUCCACGUGAUC | 17 | 2526 |
| myoC-2545 | - | CACGUGAUCCUGGGUUC | 17 | 2527 |
| myoC-1402 | - | ACGUGAUCCUGGGUUCU | 17 | 1702 |
| myoC-2547 | - | UCCUGGGUUCUAGGAGG | 17 | 2528 |
| myoC-2548 | - | GGAGGCAGGGCUAUAUU | 17 | 2529 |
| myoC-1406 | - | GAGGCAGGGCUAUAUUG | 17 | 1706 |
| myoC-1407 | - | AGGCAGGGCUAUAUUGU | 17 | 1707 |
| myoC-1408 | - | GGCAGGGCUAUAUUGUG | 17 | 1708 |
| myoC-1409 | - | GCAGGGCUAUAUUGUGG | 17 | 1709 |
| myoC-1410 | - | CAGGGCUAUAUUGUGGG | 17 | 1710 |
| myoC-2554 | - | GGGGAAAAAAUCAGUUC | 17 | 2530 |
| myoC-1411 | - | GGGAAAAAAUCAGUUCA | 17 | 1711 |
| myoC-1412 | - | GGAAAAAAUCAGUUCAA | 17 | 1712 |
| myoC-2557 | - | AAUCAGUUCAAGGGAAG | 17 | 2531 |
| myoC-1413 | - | AUCAGUUCAAGGGAAGU | 17 | 1713 |
| myoC-1414 | - | UCAGUUCAAGGGAAGUC | 17 | 1714 |
| myoC-2560 | - | UAUUUUUCCUUUACAAG | 17 | 2532 |
| myoC-2561 | - | UUACAAGCUGAGUAAUU | 17 | 2533 |
| myoC-2562 | - | AAGUCACAAGGUAGUAA | 17 | 2534 |
| myoC-2563 | - | ACUUAGUUUCUCCUUAU | 17 | 2535 |
| myoC-1417 | - | CUUAGUUUCUCCUUAUU | 17 | 1717 |
| myoC-2565 | - | AGGAACUCUUUUUCUCU | 17 | 2536 |
| myoC-1418 | - | GGAACUCUUUUUCUCUG | 17 | 1718 |
| myoC-2567 | - | UGUGGAGUUAGCAGCAC | 17 | 2537 |
| myoC-2568 | - | AAUCCCGUUUCUUUUAA | 17 | 2538 |
| myoC-1421 | - | AUCCCGUUUCUUUUAAC | 17 | 1721 |
| myoC-2570 | - | CCGUUUCUUUUAACAGG | 17 | 2539 |
| myoC-2571 | - | AGGAAGAAAACAUUCCU | 17 | 2540 |
| myoC-2572 | - | ACUAUAUGAUUGGUUUU | 17 | 2541 |
| myoC-2573 | - | AUGUUUACUAUCUGAUU | 17 | 2542 |
| myoC-2574 | - | ACUAUCUGAUUCAGAAA | 17 | 2543 |
| myoC-2575 | - | AAGUUCAGGCUUAACUG | 17 | 2544 |
| myoC-2576 | - | UGCAGAACCAAUCAAAU | 17 | 2545 |
| myoC-2577 | - | AACCAAUCAAAUAAGAA | 17 | 2546 |
| myoC-2578 | - | AAUAAGAAUAGAAUCUU | 17 | 2547 |
| myoC-2579 | - | AACUGUGUUUCUCCACU | 17 | 2548 |
| myoC-1426 | - | ACUGUGUUUCUCCACUC | 17 | 1726 |
| myoC-2581 | - | GUUUCUCCACUCUGGAG | 17 | 2549 |
| myoC-2582 | - | UCUGGAGGUGAGUCUGC | 17 | 2550 |
| myoC-2583 | - | GAGUCUGCCAGGGCAGU | 17 | 2551 |
| myoC-1430 | - | AGUCUGCCAGGGCAGUU | 17 | 1730 |
| myoC-2585 | - | CUUUUUGUUUUUUCUCU | 17 | 2552 |
| myoC-2586 | - | GGUUUAUUAAUGUAAAG | 17 | 2553 |
| myoC-1438 | - | GUUUAUUAAUGUAAAGC | 17 | 1738 |
| myoC-2588 | - | AUUAUUAACCUACAGUC | 17 | 2554 |
| myoC-2589 | - | UACAGUCCAGAAAGCCU | 17 | 2555 |
| myoC-2590 | - | CCAGAAAGCCUGUGAAU | 17 | 2556 |
| myoC-2591 | - | AAAGCCUGUGAAUUUGA | 17 | 2557 |
| myoC-2592 | - | AGCCUGUGAAUUUGAAU | 17 | 2558 |
| myoC-1440 | - | GCCUGUGAAUUUGAAUG | 17 | 1740 |
| myoC-2594 | - | UAACAUUUUAUUCCAUU | 17 | 2559 |
| myoC-2595 | - | UUUUAUUCCAUUGCGAA | 17 | 2560 |
| myoC-2596 | - | GAUUUUGUCAUUACCAA | 17 | 2561 |
| myoC-2597 | - | UUGCAGAUACGUUGUAA | 17 | 2562 |
| myoC-2598 | - | UUAUACUCAAAACUACU | 17 | 2563 |
| myoC-2599 | - | UGAAAUUAGACCUCCUG | 17 | 2564 |
| myoC-2600 | - | AUCUAUAUUUUAUAUAU | 17 | 2565 |
| myoC-2601 | - | UAUUUGAAAACAUCUUU | 17 | 2566 |
| myoC-2602 | - | UUUGAAAACAUCUUUCU | 17 | 2567 |
| myoC-2603 | - | GAAAACAUCUUUCUGAG | 17 | 2568 |
| myoC-2604 | - | UUCCCCAGAUUUCACCA | 17 | 2569 |
| myoC-2605 | - | CU UGGCAUGCACACACA | 17 | 2570 |
| myoC-2606 | - | AUGCACACACACAGAGU | 17 | 2571 |
| myoC-2607 | - | CAGAGUAAGAACUGAUU | 17 | 2572 |
| myoC-2608 | - | AACAUUGACAUUGGUGC | 17 | 2573 |
| myoC-2609 | - | GUGCCUGAGAUGCAAGA | 17 | 2574 |
| myoC-2610 | - | AGAUGCAAGACUGAAAU | 17 | 2575 |
| myoC-2611 | - | CACAGUUGUUUUAAAGC | 17 | 2576 |
| myoC-2612 | - | ACAGUUGUUUUAAAGCU | 17 | 2577 |
| myoC-2613 | - | UUGUUUUAAAGCUAGGG | 17 | 2578 |
| myoC-2614 | - | GUUUUAAAGCUAGGGGU | 17 | 2579 |
| myoC-2615 | - | UUUUAAAGCUAGGGGUG | 17 | 2580 |
| myoC-2616 | - | UUUAAAGCUAGGGGUGA | 17 | 2581 |
| myoC-2617 | - | UUAAAGCUAGGGGUGAG | 17 | 2582 |
| myoC-2618 | - | UAAAGCUAGGGGUGAGG | 17 | 2583 |
| myoC-2619 | - | AAAGCUAGGGGUGAGGG | 17 | 2584 |
| myoC-2620 | - | GAAAUCUGCCGCUUCUA | 17 | 2585 |
| myoC-1480 | - | AAAUCUGCCGCUUCUAU | 17 | 1780 |
| myoC-2622 | - | CUAUAGGAAUGCUCUCC | 17 | 2586 |
| myoC-1481 | - | UAUAGGAAUGCUCUCCC | 17 | 1781 |
| myoC-2624 | - | CUCUCCCUGGAGCCUGG | 17 | 2587 |
| myoC-2625 | - | GUCCCUGCUACGUCUUA | 17 | 2588 |
| myoC-2626 | - | CGUCUUAAAGGACUUGU | 17 | 2589 |
| myoC-2627 | - | CACAGUGCAGGUUCUCA | 17 | 2590 |
| myoC-2628 | - | GGUUCUCAAUGAGUUUG | 17 | 2591 |
| myoC-2629 | - | CUCAAUGAGUUUGCAGA | 17 | 2592 |
| myoC-2630 | - | AUGAGUUUGCAGAGUGA | 17 | 2593 |
| myoC-899 | - | UGAGUUUGCAGAGUGAA | 17 | 1254 |
| myoC-2632 | - | UGAAUGGAAAUAUAAAC | 17 | 2594 |
| myoC-2633 | - | CUAGAAAUAUAUCCUUG | 17 | 2595 |
| myoC-2634 | - | UGUGUGUGUAAAACCAG | 17 | 2596 |
| myoC-902 | - | GUGUGUGUAAAACCAGG | 17 | 1073 |
| myoC-2636 | - | AAAACCAGGUGGAGAUA | 17 | 2597 |
| myoC-903 | - | AAACCAGGUGGAGAUAU | 17 | 1222 |
| myoC-2638 | - | AGAUAUAGGAACUAUUA | 17 | 2598 |
| myoC-904 | - | GAUAUAGGAACUAUUAU | 17 | 1058 |
| myoC-2640 | - | GGAACUAUUAUUGGGGU | 17 | 2599 |
| myoC-2641 | - | GGGUAUGGGUGCAUAAA | 17 | 2600 |
| myoC-909 | - | GGUAUGGGUGCAUAAAU | 17 | 1067 |
| myoC-2643 | - | GGGAUGUUCUUUUUAAA | 17 | 2601 |
| myoC-2644 | - | AAACUCCAAACAGACUU | 17 | 2602 |
| myoC-911 | - | AACUCCAAACAGACUUC | 17 | 1225 |
| myoC-2646 | - | CUGGAAGGUUAUUUUCU | 17 | 2603 |
| myoC-2647 | - | AGAAUCUUGCUGGCAGC | 17 | 2604 |
| myoC-2648 | - | CACCUCUGUCUUCCCCC | 17 | 2605 |
| myoC-2649 | - | CUCUGUCUUCCCCCAUG | 17 | 2606 |
| myoC-2650 | - | AGUAUAUAUAAACCUCU | 17 | 2607 |
| myoC-919 | - | GUAUAUAUAAACCUCUC | 17 | 998 |
| myoC-2652 | - | AUAAACCUCUCUGGAGC | 17 | 2608 |
| myoC-2653 | - | CUCUCUGGAGCUCGGGC | 17 | 2609 |
| myoC-2654 | - | GGCACCUCUCAGCACAG | 17 | 2610 |
| myoC-2655 | - | AGCACAGCAGAGCUUUC | 17 | 2611 |
| myoC-2656 | - | CACAGCAGAGCUUUCCA | 17 | 2612 |
| myoC-2657 | - | ACAGCAGAGCUUUCCAG | 17 | 2613 |
| myoC-826 | + | GAGAGGAAACCUCUGCC | 17 | 1023 |
| myoC-825 | + | GGAGAGGAAACCUCUGC | 17 | 1034 |
| myoC-2660 | + | UGGAGAGGAAACCUCUG | 17 | 2614 |
| myoC-824 | + | GGCUCCCCCAGCUGGAG | 17 | 1040 |
| myoC-2662 | + | GGGCUCCCCCAGCUGGA | 17 | 2615 |
| myoC-2663 | + | CAGGGCUCCCCCAGCUG | 17 | 2616 |
| myoC-823 | + | UGCAGGGCUCCCCCAGC | 17 | 1165 |
| myoC-2665 | + | UUGCAGGGCUCCCCCAG | 17 | 2617 |
| myoC-2666 | + | AGGACCCCGGGUGCUUG | 17 | 2618 |
| myoC-2667 | + | UCAGGACACCCAGGACC | 17 | 2619 |
| myoC-2668 | + | AGGUUGCUCAGGACACC | 17 | 2620 |
| myoC-2669 | + | CGGGCUGGCAGGUUGCU | 17 | 2621 |
| myoC-2670 | + | ACAAAACAACCAGUGGC | 17 | 2622 |
| myoC-2671 | + | AAAGCAACAGGUCCCUA | 17 | 2623 |
| myoC-2672 | + | AGAAAGCAACAGGUCCC | 17 | 2624 |
| myoC-2673 | + | AACGAGUCACACAGAAA | 17 | 2625 |
| myoC-2674 | + | UGAAUGAACGAGUCACA | 17 | 2626 |
| myoC-2675 | + | AAUGCCUGGAUGAAUGA | 17 | 2627 |
| myoC-2676 | + | AAUGAAUGCCUGGAUGA | 17 | 2628 |
| myoC-2677 | + | UGUCAAUGAAUGCCUGG | 17 | 2629 |
| myoC-2678 | + | AAAUUGUCAAUGAAUGC | 17 | 2630 |
| myoC-2679 | + | UACUCAAUAAAUUGUCA | 17 | 2631 |
| myoC-2680 | + | UGUCACCUCCACGAAGG | 17 | 2632 |
| myoC-2681 | + | GAGAAACUGUCACCUCC | 17 | 2633 |
| myoC-2682 | + | UUCUGCACGUCUUCCAU | 17 | 2634 |
| myoC-2683 | + | UCUUCUGCACGUCUUCC | 17 | 2635 |
| myoC-2684 | + | UUUCCUUUCUUUCAGCA | 17 | 2636 |
| myoC-798 | + | GGGAGGUGGCCUUGUUA | 17 | 1041 |
| myoC-2686 | + | AGGGAGGUGGCCUUGUU | 17 | 2637 |
| myoC-795 | + | GGCAGCAGGGGGCGCUA | 17 | 1039 |
| myoC-794 | + | AGGCAGCAGGGGGCGCU | 17 | 1140 |
| myoC-2689 | + | GAGGCAGCAGGGGGCGC | 17 | 2638 |
| myoC-791 | + | GCACGAUGGAGGCAGCA | 17 | 1028 |
| myoC-790 | + | GGCACGAUGGAGGCAGC | 17 | 1038 |
| myoC-2692 | + | GGGCACGAUGGAGGCAG | 17 | 2639 |
| myoC-788 | + | GGGGCCUCCGGGCACGA | 17 | 1043 |
| myoC-2694 | + | GGGGGCCUCCGGGCACG | 17 | 2640 |
| myoC-2695 | + | CUCGGGCUUGGGGGCCU | 17 | 2641 |
| myoC-783 | + | UUGGAAGACUCGGGCUU | 17 | 1169 |
| myoC-782 | + | CUUGGAAGACUCGGGCU | 17 | 1158 |
| myoC-2698 | + | GCUUGGAAGACUCGGGC | 17 | 2642 |
| myoC-2699 | + | GAGGAGGCUUGGAAGAC | 17 | 2643 |
| myoC-779 | + | UGAUGGAGGAGGAGGCU | 17 | 1163 |
| myoC-2701 | + | CUGAUGGAGGAGGAGGC | 17 | 2644 |
| myoC-777 | + | GCUGUGACUGAUGGAGG | 17 | 1031 |
| myoC-2703 | + | CGCUGUGACUGAUGGAG | 17 | 2645 |
| myoC-776 | + | AGCGCUGUGACUGAUGG | 17 | 1137 |
| myoC-2705 | + | CAGCGCUGUGACUGAUG | 17 | 2646 |
| myoC-775 | + | UGCAGCGCUGUGACUGA | 17 | 1164 |
| myoC-2707 | + | CUGCAGCGCUGUGACUG | 17 | 2647 |
| myoC-2708 | + | AGGACGAUUCACGGGAA | 17 | 2648 |
| myoC-2709 | + | GCACCAGGACGAUUCAC | 17 | 2649 |
| myoC-2710 | + | UGCACCAGGACGAUUCA | 17 | 2650 |
| myoC-2711 | + | AUGCACCAGGACGAUUC | 17 | 2651 |
| myoC-2712 | + | CUCCAGCUCAGAUGCAC | 17 | 2652 |
| myoC-1385 | + | UCUGGAGCCUGGAGCCA | 17 | 1685 |
| myoC-2714 | + | UUCUGGAGCCUGGAGCC | 17 | 2653 |
| myoC-1384 | + | AUUUCCUUUCUGGAGCC | 17 | 1684 |
| myoC-2716 | + | CAUUUCCUUUCUGGAGC | 17 | 2654 |
| myoC-1383 | + | CCUCUCCAUUUCCUUUC | 17 | 1683 |
| myoC-2718 | + | CCCUCUCCAUUUCCUUU | 17 | 2655 |
| myoC-1382 | + | AGGCCCCUUUCCCUCUG | 17 | 1682 |
| myoC-2720 | + | GAGGCCCCUUUCCCUCU | 17 | 2656 |
| myoC-2721 | + | UGGAGGCCCCUUUCCCU | 17 | 2657 |
| myoC-1380 | + | UGGAAUUCUCCUGGACG | 17 | 1680 |
| myoC-2723 | + | CUGGAAUUCUCCUGGAC | 17 | 2658 |
| myoC-2724 | + | CACCUCCUGGAAUUCUC | 17 | 2659 |
| myoC-1378 | + | CUGCAGUCCCCACCUCC | 17 | 1678 |
| myoC-2726 | + | CCUGCAGUCCCCACCUC | 17 | 2660 |
| myoC-2727 | + | UGAACAACACUGAACAU | 17 | 2661 |
| myoC-2728 | + | CAGCCCCGUGAACAACA | 17 | 2662 |
| myoC-2729 | + | GGAAAACUCCCAGCCCC | 17 | 2663 |
| myoC-1375 | + | AGGCUCACAGGAAGCAA | 17 | 1675 |
| myoC-2731 | + | AAGGCUCACAGGAAGCA | 17 | 2664 |
| myoC-1374 | + | AAGAUAAAAAGGCUCAC | 17 | 1674 |
| myoC-2733 | + | AAAGAUAAAAAGGCUCA | 17 | 2665 |
| myoC-2734 | + | UUCUUCUCCUCCAAGCA | 17 | 2666 |
| myoC-2735 | + | ACUUCUUCUCCUCCAAG | 17 | 2667 |
| myoC-2736 | + | UGAAACUGCAUCCCUUC | 17 | 2668 |
| myoC-2737 | + | UUUUAACAGCUGACUUU | 17 | 2669 |
| myoC-1372 | + | AAAACCCAUGCACACCC | 17 | 1672 |
| myoC-2739 | + | GAAAACCCAUGCACACC | 17 | 2670 |
| myoC-1371 | + | AAUAAAGGCCUUCGUGA | 17 | 1671 |
| myoC-2741 | + | AAAUAAAGGCCUUCGUG | 17 | 2671 |
| myoC-2742 | + | AUUAAAUAAAGGCCUUC | 17 | 2672 |
| myoC-2743 | + | AAUUAACGGCCUAGGAA | 17 | 2673 |
| myoC-1369 | + | CCGUGAAUUAACGGCCU | 17 | 1669 |
| myoC-2745 | + | UCCGUGAAUUAACGGCC | 17 | 2674 |
| myoC-2746 | + | CUCCAGUCACUUCUUCC | 17 | 2675 |
| myoC-2747 | + | UUGCCCAGAAGACAUGA | 17 | 2676 |
| myoC-2748 | + | GUAGUUGCCCAGAAGAC | 17 | 2677 |
| myoC-2749 | + | AGGGCUGAGUAGUUGCC | 17 | 2678 |
| myoC-2750 | + | CCAAGUCCACCACAGGG | 17 | 2679 |
| myoC-2751 | + | AUAAGCCAAGUCCACCA | 17 | 2680 |
| myoC-2752 | + | CAGAACCAGAAAGAAAA | 17 | 2681 |
| myoC-2753 | + | CCAAUGGCAGAACCAGA | 17 | 2682 |
| myoC-2754 | + | CCAACCAAUGGCAGAAC | 17 | 2683 |
| myoC-2755 | + | GCACAGCCAACCAAUGG | 17 | 2684 |
| myoC-2756 | + | ACUAUGGCCCAGGGAAG | 17 | 2685 |
| myoC-1362 | + | AGACUAUGGCCCAGGGA | 17 | 1662 |
| myoC-2758 | + | AAGACUAUGGCCCAGGG | 17 | 2686 |
| myoC-1361 | + | GAGAAGACUAUGGCCCA | 17 | 1661 |
| myoC-1360 | + | AGAGAAGACUAUGGCCC | 17 | 1660 |
| myoC-2761 | + | CAGAGAAGACUAUGGCC | 17 | 2687 |
| myoC-2762 | + | CAAGGGUCUUUAUAGCA | 17 | 2688 |
| myoC-2763 | + | UGCAAGGGUCUUUAUAG | 17 | 2689 |
| myoC-2764 | + | CAGAACACGAGAGCUGC | 17 | 2690 |
| myoC-2765 | + | AAGUGUUCACAGAACAC | 17 | 2691 |
| myoC-2766 | + | GGAAGUGUUCACAGAAC | 17 | 2692 |
| myoC-2767 | + | UCACAGGGAAGUGUUCA | 17 | 2693 |
| myoC-1356 | + | CCUCACAGAGAAUCACA | 17 | 1656 |
| myoC-1355 | + | CCCUCACAGAGAAUCAC | 17 | 1655 |
| myoC-2770 | + | CCCCUCACAGAGAAUCA | 17 | 2694 |
| myoC-2771 | + | CAACAUCCCCCCUCACA | 17 | 2695 |
| myoC-2772 | + | CUCAACAUCCCCCCUCA | 17 | 2696 |
| myoC-1353 | + | UGAUCAGUGAGGACUGA | 17 | 1653 |
| myoC-2774 | + | GUGAUCAGUGAGGACUG | 17 | 2697 |
| myoC-2775 | + | AGUCUGACGUGAUCAGU | 17 | 2698 |
| myoC-2776 | + | GGAGUCUGACGUGAUCA | 17 | 2699 |
| myoC-1351 | + | AUUGUGGCUCUCGGUCC | 17 | 1651 |
| myoC-2778 | + | CAUUGUGGCUCUCGGUC | 17 | 2700 |
| myoC-2779 | + | GGGUUCAUUGAGCUUUC | 17 | 2701 |
| myoC-2780 | + | UGUGGCUGUUGGGUUCA | 17 | 2702 |
| myoC-2781 | + | GAAGGAAAAUGUGGCUG | 17 | 2703 |
| myoC-1345 | + | UUGUCUAUGCUUAGGGA | 17 | 1645 |
| myoC-2783 | + | AUUGUCUAUGCUUAGGG | 17 | 2704 |
| myoC-1344 | + | GCCAUUGUCUAUGCUUA | 17 | 1644 |
| myoC-1343 | + | UGCCAUUGUCUAUGCUU | 17 | 1643 |
| myoC-2786 | + | AUGCCAUUGUCUAUGCU | 17 | 2705 |
| myoC-2787 | + | UUGCUCUGGCCCAGUUU | 17 | 2706 |
| myoC-2788 | + | GUGGGGUGCUGGUCAGG | 17 | 2707 |
| myoC-2789 | + | CUGCGUGGGGUGCUGGU | 17 | 2708 |
| myoC-1469 | + | GUCACUGCUGAGCUGCG | 17 | 1769 |
| myoC-2791 | + | AGUCACUGCUGAGCUGC | 17 | 2709 |
| myoC-2792 | + | GCUGUCAGCAGUCACUG | 17 | 2710 |
| myoC-2793 | + | AUUCCCACUGCCCUUGA | 17 | 2711 |
| myoC-2794 | + | GUCAAUUCCCACUGCCC | 17 | 2712 |
| myoC-2795 | + | CUAGAACCCAGGAUCAC | 17 | 2713 |
| myoC-2796 | + | CCCUGCCUCCUAGAACC | 17 | 2714 |
| myoC-2797 | + | AAUAUAGCCCUGCCUCC | 17 | 2715 |
| myoC-2798 | + | AGGUCUCCCGACUUCCC | 17 | 2716 |
| myoC-2799 | + | AAGGAAAAAUAUAGUAU | 17 | 2717 |
| myoC-1463 | + | AAUUACUCAGCUUGUAA | 17 | 1763 |
| myoC-2801 | + | GAAUUACUCAGCUUGUA | 17 | 2718 |
| myoC-2802 | + | CUACCUUGUGACUUGCU | 17 | 2719 |
| myoC-2803 | + | AAAGAGUUCCUAAUAAG | 17 | 2720 |
| myoC-1462 | + | AAAAAGAGUUCCUAAUA | 17 | 1762 |
| myoC-2805 | + | GAAAAAGAGUUCCUAAU | 17 | 2721 |
| myoC-2806 | + | CUAACUCCACAGAGAAA | 17 | 2722 |
| myoC-2807 | + | GUGCUGCUAACUCCACA | 17 | 2723 |
| myoC-2808 | + | UUGUGCUGCUAACUCCA | 17 | 2724 |
| myoC-1460 | + | CUUCCUGUUAAAAGAAA | 17 | 1760 |
| myoC-2810 | + | UCUUCCUGUUAAAAGAA | 17 | 2725 |
| myoC-2811 | + | UGUUUUCUUCCUGUUAA | 17 | 2726 |
| myoC-1459 | + | UGUUUGGCUUUACUCUU | 17 | 1759 |
| myoC-2813 | + | CUGUUUGGCUUUACUCU | 17 | 2727 |
| myoC-2814 | + | GUCAGCAAGACCUAGGC | 17 | 2728 |
| myoC-2815 | + | UCAGAUAGUAAACAUCG | 17 | 2729 |
| myoC-2816 | + | GGUACUAGUCUCAUUUU | 17 | 2730 |
| myoC-2817 | + | UUGUUUACAGCUGACCA | 17 | 2731 |
| myoC-2818 | + | ACUUGAGACAUUUACAA | 17 | 2732 |
| myoC-2819 | + | UGCAGUUAAGCCUGAAC | 17 | 2733 |
| myoC-2820 | + | UGGUUCUGCAGUUAAGC | 17 | 2734 |
| myoC-2821 | + | GACUCACCUCCAGAGUG | 17 | 2735 |
| myoC-1451 | + | CAGACUCACCUCCAGAG | 17 | 1751 |
| myoC-2823 | + | GCAGACUCACCUCCAGA | 17 | 2736 |
| myoC-2824 | + | CCUGGCAGACUCACCUC | 17 | 2737 |
| myoC-2825 | + | CAACAGUGUCAAUACUU | 17 | 2738 |
| myoC-2826 | + | UGAAAUAAUGAUUGCCU | 17 | 2739 |
| myoC-2827 | + | AAGUAACUUUAAGCCAC | 17 | 2740 |
| myoC-2828 | + | AAAUAUACCAAAACUGU | 17 | 2741 |
| myoC-2829 | + | ACAUUAAUAAACCCAAA | 17 | 2742 |
| myoC-2830 | + | UUACAUUAAUAAACCCA | 17 | 2743 |
| myoC-2831 | + | UCAAAUUCACAGGCUUU | 17 | 2744 |
| myoC-2832 | + | AAAAUGUUAAAUUUAGU | 17 | 2745 |
| myoC-1443 | + | UAUGGCUCUAUUCGCAA | 17 | 1743 |
| myoC-2834 | + | UUAUGGCUCUAUUCGCA | 17 | 2746 |
| myoC-2835 | + | GUACUGUUAUUACCACU | 17 | 2747 |
| myoC-2836 | + | CUAAUUUCAAAGUAGUU | 17 | 2748 |
| myoC-1498 | + | UAAAAACAAGAUCCAGC | 17 | 1798 |
| myoC-2838 | + | U UAAAAACAAGAUCCAG | 17 | 2749 |
| myoC-2839 | + | AAAGGAAACAAAUGAUA | 17 | 2750 |
| myoC-1497 | + | UAAAAUAUAGAUUACAA | 17 | 1797 |
| myoC-2841 | + | AUAAAAUAUAGAUUACA | 17 | 2751 |
| myoC-2842 | + | AUCUGGGGAACUCUUCU | 17 | 2752 |
| myoC-1496 | + | CUCAUUGGUGAAAUCUG | 17 | 1796 |
| myoC-1495 | + | CCUCAUUGGUGAAAUCU | 17 | 1795 |
| myoC-1494 | + | ACCUCAUUGGUGAAAUC | 17 | 1794 |
| myoC-2846 | + | AACCUCAUUGGUGAAAU | 17 | 2753 |
| myoC-2847 | + | UGCCAAGAACCUCAUUG | 17 | 2754 |
| myoC-2848 | + | CUGUGUGUGUGCAUGCC | 17 | 2755 |
| myoC-2849 | + | CAACUGUGUAUCUUUGG | 17 | 2756 |
| myoC-1491 | + | AACAACUGUGUAUCUUU | 17 | 1791 |
| myoC-1490 | + | AAACAACUGUGUAUCUU | 17 | 1790 |
| myoC-2852 | + | AAAACAACUGUGUAUCU | 17 | 2757 |
| myoC-2853 | + | CAGGGAGAGCAUUCCUA | 17 | 2758 |
| myoC-2854 | + | CCCUACCAGGCUCCAGG | 17 | 2759 |
| myoC-1488 | + | CACCCUACCAGGCUCCA | 17 | 1788 |
| myoC-1487 | + | GCACCCUACCAGGCUCC | 17 | 1787 |
| myoC-2857 | + | AGCACCCUACCAGGCUC | 17 | 2760 |
| myoC-2858 | + | ACAGCCAGCCAGAACAC | 17 | 2761 |
| myoC-2859 | + | GAAAAAUAACAGCCAGC | 17 | 2762 |
| myoC-2860 | + | AAGACGUAGCAGGGACA | 17 | 2763 |
| myoC-2861 | + | UUAAGACGUAGCAGGGA | 17 | 2764 |
| myoC-959 | + | GUCCUUUAAGACGUAGC | 17 | 1000 |
| myoC-2863 | + | AGUCCUUUAAGACGUAG | 17 | 2765 |
| myoC-958 | + | GGCACUAUGCUAGGAAC | 17 | 1062 |
| myoC-2865 | + | AGGCACUAUGCUAGGAA | 17 | 2766 |
| myoC-957 | + | GUGCCAGGCACUAUGCU | 17 | 1071 |
| myoC-2867 | + | UGUGCCAGGCACUAUGC | 17 | 2767 |
| myoC-2868 | + | CACUCUGCAAACUCAUU | 17 | 2768 |
| myoC-2869 | + | UUCACUCUGCAAACUCA | 17 | 2769 |
| myoC-2870 | + | GGUGUGCUGAUUUCAAC | 17 | 2770 |
| myoC-2871 | + | CGUACACACACUUACAC | 17 | 2771 |
| myoC-2872 | + | UGGAGUUUCUUUUUAAA | 17 | 2772 |
| myoC-951 | + | CCUUCCAGAAGUCUGUU | 17 | 1242 |
| myoC-2874 | + | ACCUUCCAGAAGUCUGU | 17 | 2773 |
| myoC-2875 | + | CUUAGAAAAUAACCUUC | 17 | 2774 |
| myoC-2876 | + | GCUGCCAGCAAGAUUCU | 17 | 2775 |
| myoC-948 | + | GGUGGGGCUGUGCACAG | 17 | 1069 |
| myoC-947 | + | GGGUGGGGCUGUGCACA | 17 | 1066 |
| myoC-946 | + | UGGGUGGGGCUGUGCAC | 17 | 1257 |
| myoC-2880 | + | CUGGGUGGGGCUGUGCA | 17 | 2776 |
| myoC-943 | + | GGCCACGUGAGGCUGGG | 17 | 1063 |
| myoC-2882 | + | UGGCCACGUGAGGCUGG | 17 | 2777 |
| myoC-2883 | + | GAGGUGGCCACGUGAGG | 17 | 2778 |
| myoC-2884 | + | AAGACAGAGGUGGCCAC | 17 | 2779 |
| myoC-2885 | + | CCCUUCAUGGGGGAAGA | 17 | 2780 |
| myoC-937 | + | GAGCCAGCCCUUCAUGG | 17 | 1056 |
| myoC-936 | + | GGAGCCAGCCCUUCAUG | 17 | 1061 |
| myoC-935 | + | GGGAGCCAGCCCUUCAU | 17 | 1064 |
| myoC-934 | + | GGGGAGCCAGCCCUUCA | 17 | 1065 |
| myoC-2890 | + | UGGGGAGCCAGCCCUUC | 17 | 2781 |
| myoC-933 | + | GAGGUUUAUAUAUACUG | 17 | 1057 |
| myoC-932 | + | AGAGGUUUAUAUAUACU | 17 | 1230 |
| myoC-931 | + | GAGAGGUUUAUAUAUAC | 17 | 997 |
| myoC-2894 | + | AGAGAGGUUUAUAUAUA | 17 | 2782 |
| myoC-2895 | + | CUCAUGCCCGAGCUCCA | 17 | 2783 |
| myoC-2896 | + | GGCUCAUGCCCGAGCUC | 17 | 2784 |
| myoC-2897 | + | GCCUUGCUGGCUCAUGC | 17 | 2785 |
| myoC-2898 | + | UGCUGAGAGGUGCCUGG | 17 | 2786 |
| myoC-2899 | + | GCUGUGCUGAGAGGUGC | 17 | 2787 |
| myoC-2900 | + | GAAAGCUCUGCUGUGCU | 17 | 2788 |
| myoC-2901 | + | UGGAAAGCUCUGCUGUG | 17 | 2789 |
| myoC-2902 | + | CUUGGUGAGGCUUCCUC | 17 | 2790 |
| myoC-2903 | + | GCUUGGUGAGGCUUCCU | 17 | 2791 |

**Table 5F** provides exemplary targeting domains for repressing (i.e., knocking down or decreasing) expression of the *MYOC* gene. Any of the targeting domains in the table can be used with an *N. meningitidis* eiCas9 molecule to cause a steric block in the promoter region to block transcription elongation resulting in the repression of the *MYOC* gene. Any of the targeting domains in the table can be used with an *N. meningitidis* eiCas9 fused to a transcriptional repressor to decrease transcription and therefore downregulate gene expression.

**Table 5F**

| **gRNA Name** | **DNA Strand** | **Targeting Domain** | **Target Site Length** | **SEQ ID NO** |
|---|---|---|---|---|
| myoC-3098 | - | CGUGUUCUGUGAACACUUCC | 20 | 2856 |
| myoC-1975 | - | CCAGAGCAAGUGGAAAAUGC | 20 | 2132 |
| myoC-3100 | - | GAUAGUGUAUGAGCAAGAAA | 20 | 2857 |
| myoC-1996 | - | AGGGCAGUGGGAAUUGACCA | 20 | 2145 |
| myoC-3102 | - | AGUUCAAGGGAAGUCGGGAG | 20 | 2858 |
| myoC-3103 | - | ACAAGGUAGUAACUGAGGCU | 20 | 2859 |
| myoC-3104 | - | CAUUCCUAAGAGUAAAGCCA | 20 | 2860 |
| myoC-3105 | - | AAGCCUAGGUCUUGCUGACU | 20 | 2861 |
| myoC-3106 | - | UCAUUUCAGCGAUGUUUACU | 20 | 2862 |
| myoC-2040 | - | UUGGGUUUAUUAAUGUAAAG | 20 | 2173 |
| myoC-3108 | - | CAAAGUGGUAAUAACAGUAC | 20 | 2863 |
| myoC-3109 | - | CAUCUUUCUGAGAAGAGUUC | 20 | 2864 |
| myoC-3110 | - | AUGCACACACACAGAGUAAG | 20 | 2865 |
| myoC-3111 | + | UCUCCAGCUCAGAUGCACCA | 20 | 2866 |
| myoC-3112 | + | UCUGAGGAAACACUGUCCCC | 20 | 2867 |
| myoC-3113 | + | ACCAGAAAGAAAACCGAGUC | 20 | 2868 |
| myoC-3114 | + | AGGUCUCCCGACUUCCCUUG | 20 | 2869 |
| myoC-2264 | + | UUUUCUUCCUGUUAAAAGAA | 20 | 2345 |
| myoC-3116 | + | UCAGAUAGUAAACAUCGCUG | 20 | 2870 |
| myoC-3117 | + | GCUCUAAAGAUUCUAUUCUU | 20 | 2871 |
| myoC-3118 | + | UGGAGAAACACAGUUUGCUC | 20 | 2872 |
| myoC-3119 | + | UAACUUUAAGCCACUUGAAA | 20 | 2873 |
| myoC-3120 | + | UGUAAUAUAGUAUAAAAUGU | 20 | 2874 |
| myoC-3121 | + | AGGAAACAAAUGAUAAUGAA | 20 | 2875 |
| myoC-3122 | + | AUGUUUUCAAAUAUAUAAAA | 20 | 2876 |
| myoC-3123 | + | GAGAGCAUUCCUAUAGAAGC | 20 | 2877 |
| myoC-3124 | + | UUACACCAGGACUACUGGUG | 20 | 2878 |
| myoC-3125 | + | GGGUUGCCUUCACGCUGCCA | 20 | 2879 |
| myoC-3126 | - | GUUCUGUGAACACUUCC | 17 | 2880 |
| myoC-2521 | - | GAGCAAGUGGAAAAUGC | 17 | 2512 |
| myoC-3128 | - | AGUGUAUGAGCAAGAAA | 17 | 2881 |
| myoC-2542 | - | GCAGUGGGAAUUGACCA | 17 | 2525 |
| myoC-3130 | - | UCAAGGGAAGUCGGGAG | 17 | 2882 |
| myoC-3131 | - | AGGUAGUAACUGAGGCU | 17 | 2883 |
| myoC-3132 | - | UCCUAAGAGUAAAGCCA | 17 | 2884 |
| myoC-3133 | - | CCUAGGUCUUGCUGACU | 17 | 2885 |
| myoC-3134 | - | UUUCAGCGAUGUUUACU | 17 | 2886 |
| myoC-2586 | - | GGUUUAUUAAUGUAAAG | 17 | 2553 |
| myoC-3136 | - | AGUGGUAAUAACAGUAC | 17 | 2887 |
| myoC-3137 | - | CUUUCUGAGAAGAGUUC | 17 | 2888 |
| myoC-3138 | - | CACACACACAGAGUAAG | 17 | 2889 |
| myoC-3139 | + | CCAGCUCAGAUGCACCA | 17 | 2890 |
| myoC-3140 | + | GAGGAAACACUGUCCCC | 17 | 2891 |
| myoC-3141 | + | AGAAAGAAAACCGAGUC | 17 | 2892 |
| myoC-3142 | + | UCUCCCGACUUCCCUUG | 17 | 2893 |
| myoC-2810 | + | UCUUCCUGUUAAAAGAA | 17 | 2725 |
| myoC-3144 | + | GAUAGUAAACAUCGCUG | 17 | 2894 |
| myoC-3145 | + | CUAAAGAUUCUAUUCUU | 17 | 2895 |
| myoC-3146 | + | AGAAACACAGUUUGCUC | 17 | 2896 |
| myoC-3147 | + | CUUUAAGCCACUUGAAA | 17 | 2897 |
| myoC-3148 | + | AAUAUAGUAUAAAAUGU | 17 | 2898 |
| myoC-3149 | + | AAACAAAUGAUAAUGAA | 17 | 2899 |
| myoC-3150 | + | UUUUCAAAUAUAUAAAA | 17 | 2900 |
| myoC-3151 | + | AGCAUUCCUAUAGAAGC | 17 | 2901 |
| myoC-3152 | + | CACCAGGACUACUGGUG | 17 | 2902 |
| myoC-3153 | + | UUGCCUUCACGCUGCCA | 17 | 2903 |

**Table 6A** provides exemplary targeting domains for knocking out the *MYOC* gene selected according to the first tier parameters. The targeting domains bind within the first 500 bp of the coding sequence (e.g., within 500 bp downstream from the start codon), have a high level of orthogonality and start with a 5'G. It is contemplated herein that in an embodiment the targeting domain hybridizes to the target domain through complementary base pairing. Any of the targeting domains in the Table 1can be used with a *S. pyogeness* Cas9 molecule that generates a double stranded break (Cas9 nuclease) or a single-stranded break (Cas9 nickase).

**Table 6A**

| 1st Tier | | | | |
|---|---|---|---|---|
| gRNA Name | DNA Strand | Targeting Domain | Target Site Length | SEQ ID NO |
| myoC-163 | + | GUUAUGGAUGACUGACA | 17 | 496 |
| myoC-155 | + | GUCCCGCUCCCGCCUCA | 17 | 546 |
| myoC-167 | + | GCUGGAUUCAUUGGGAC | 17 | 497 |
| myoC-139 | - | GCGGGAGCGGGACCAGC | 17 | 534 |
| myoC-138 | - | GCACCCUGAGGCGGGAG | 17 | 533 |
| myoC-152 | + | GAACUGACUUGUCUCGG | 17 | 492 |
| myoC-157 | + | GGUCCAAGGUCAAUUGG | 17 | 493 |
| myoC-161 | + | GCUGAGUCGAGCUUUGG | 17 | 495 |
| myoC-166 | + | GGGCAGCUGGAUUCAUU | 17 | 553 |
| myoC-129 | - | GCACGUUGCUGCAGCUU | 17 | 488 |
| myoC-160 | + | GGAGCUGAGUCGAGCUU | 17 | 494 |
| myoC-126 | + | GCAGCUGGAUUCAUUGGGAC | 20 | 523 |
| myoC-107 | - | GAGGUUGGAAAGCAGCAGCC | 20 | 511 |
| myoC-113 | + | GCUGCUGCUUUCCAACCUCC | 20 | 515 |
| myoC-123 | + | GUCGAGCUUUGGUGGCCUCC | 20 | 485 |
| myoC-105 | - | GAGGCGGGAGCGGGACCAGC | 20 | 510 |
| myoC-104 | - | GGGCACCCUGAGGCGGGAGC | 20 | 509 |
| myoC-117 | + | GCUGGUCCCGCUCCCGCCUC | 20 | 484 |
| myoC-125 | + | GACAUGGCCUGGCUCUGCUC | 20 | 522 |
| myoC-114 | + | GAACUGACUUGUCUCGGAGG | 20 | 482 |
| myoC-121 | + | GGUCCAAGGUCAAUUGGUGG | 20 | 520 |
| myoC-122 | + | GGAGCUGAGUCGAGCUUUGG | 20 | 521 |
| myoC-127 | + | GCAUCGGCCACUCUGGUCAU | 20 | 487 |
| myoC-106 | - | GGAAACCCAAACCAGAGAGU | 20 | 479 |
| myoC-95 | - | GCCUGCCUGGUGUGGGAUGU | 20 | 500 |
| myoC-115 | + | GUCUCGGAGGAGGUUGCUGU | 20 | 516 |
| myoC-93 | - | GCUUCUGGCCUGCCUGGUGU | 20 | 478 |
| myoC-124 | + | GGCCUCCAGGUCUAAGCGUU | 20 | 486 |
| myoC-91 | - | GUGCACGUUGCUGCAGCUUU | 20 | 477 |

**Table 6B** provides exemplary targeting domains for knocking out the *MYOC* gene selected according to the second tier parameters. The targeting domains bind within the first 500 bp of the coding sequence (e.g., within 500 bp downstream from the start codon) and have a high level of orthogonality. It is contemplated herein that in an embodiment the targeting domain hybridizes to the target domain through complementary base pairing. Any of the targeting domains in the Table 1can be used with a *S. pyogeness* Cas9 molecule that generates a double stranded break (Cas9 nuclease) or a single-stranded break (Cas9 nickase).

**Table 6B**

| 2nd Tier | | | | |
|---|---|---|---|---|
| gRNA Name | DNA Strand | Targeting Domain | Target Site Length | SEQ ID NO |
| myoC-271 | - | AAGAGAAGAAGCGACUA | 17 | 657 |
| myoC-303 | + | CCACACUGAAGGUAUAC | 17 | 689 |
| myoC-254 | - | CACCCAACGCUUAGACC | 17 | 640 |
| myoC-258 | - | CCAAUUGACCUUGGACC | 17 | 644 |
| myoC-256 | - | AGCUCGACUCAGCUCCC | 17 | 642 |
| myoC-305 | + | ACUGGCAUCGGCCACUC | 17 | 691 |
| myoC-2902 | + | CUUGGUGAGGCUUCCUC | 17 | 2790 |
| myoC-269 | - | CCGAGACAAGUCAGUUC | 17 | 655 |
| myoC-296 | + | AGGUCAAUUGGUGGAGG | 17 | 682 |
| myoC-255 | - | CCAACGCUUAGACCUGG | 17 | 641 |
| myoC-270 | - | AGACAAGUCAGUUCUGG | 17 | 656 |
| myoC-3158 | - | ACCAAGCCUCUGCAAUG | 17 | 2904 |
| myoC-252 | - | CCAGUAUACCUUCAGUG | 17 | 638 |
| myoC-294 | + | CCUGGUCCAAGGUCAAU | 17 | 680 |
| myoC-304 | + | UGAAGGUAUACUGGCAU | 17 | 690 |
| myoC-306 | + | UCGGCCACUCUGGUCAU | 17 | 692 |
| myoC-257 | - | CCUCCACCAAUUGACCU | 17 | 643 |
| myoC-281 | + | CCAGAACUGACUUGUCU | 17 | 667 |
| myoC-268 | - | AACCCAAACCAGAGAGU | 17 | 654 |
| myoC-297 | + | CCUCCAGGUCUAAGCGU | 17 | 683 |
| myoC-298 | + | CUCCAGGUCUAAGCGUU | 17 | 684 |
| myoC-227 | + | UAAGUUAUGGAUGACUGACA | 20 | 613 |
| myoC-213 | + | CUGGUCCCGCUCCCGCCUCA | 20 | 599 |
| myoC-233 | + | AUUGGGACUGGCCACACUGA | 20 | 619 |
| myoC-226 | + | UGCUGUCUCUCUGUAAGUUA | 20 | 612 |
| myoC-234 | + | UGGCCACACUGAAGGUAUAC | 20 | 620 |
| myoC-179 | - | CAGCACCCAACGCUUAGACC | 20 | 565 |
| myoC-183 | - | CCACCAAUUGACCUUGGACC | 20 | 569 |
| myoC-181 | - | CAAAGCUCGACUCAGCUCCC | 20 | 567 |
| myoC-228 | + | UAUGGAUGACUGACAUGGCC | 20 | 614 |
| myoC-222 | + | AUUGGUGGAGGAGGCUCUCC | 20 | 608 |
| myoC-212 | + | CUCUGGUUUGGGUUUCCAGC | 20 | 598 |
| myoC-239 | + | CCCCACAUCCCACACCAGGC | 20 | 625 |
| myoC-236 | + | UAUACUGGCAUCGGCCACUC | 20 | 622 |
| myoC-2356 | + | AGGCUUGGUGAGGCUUCCUC | 20 | 2410 |
| myoC-241 | + | AGCUGGACAGCUGGCAUCUC | 20 | 627 |
| myoC-170 | - | AGCUGUCCAGCUGCUGCUUC | 20 | 556 |
| myoC-191 | - | CCUCCGAGACAAGUCAGUUC | 20 | 577 |
| myoC-3159 | + | ACAGAAGAACCUCAUUGCAG | 20 | 2905 |
| myoC-190 | - | UGGGCACCCUGAGGCGGGAG | 20 | 576 |
| myoC-221 | + | CCAAGGUCAAUUGGUGGAGG | 20 | 607 |
| myoC-209 | + | CCAGAACUGACUUGUCUCGG | 20 | 595 |
| myoC-180 | - | CACCCAACGCUUAGACCUGG | 20 | 566 |
| myoC-192 | - | CCGAGACAAGUCAGUUCUGG | 20 | 578 |
| myoC-220 | + | CCUGGUCCAAGGUCAAUUGG | 20 | 606 |
| myoC-3160 | - | CUCACCAAGCCUCUGCAAUG | 20 | 2906 |
| myoC-177 | - | AUGCCAGUAUACCUUCAGUG | 20 | 563 |
| myoC-3161 | + | CUCAUUGCAGAGGCUUGGUG | 20 | 2907 |
| myoC-219 | + | CAGCCUGGUCCAAGGUCAAU | 20 | 605 |
| myoC-235 | + | CACUGAAGGUAUACUGGCAU | 20 | 621 |
| myoC-182 | - | CCUCCUCCACCAAUUGACCU | 20 | 568 |
| myoC-3162 | + | AGAACCUCAUUGCAGAGGCU | 20 | 2908 |
| myoC-208 | + | CCUCCAGAACUGACUUGUCU | 20 | 594 |
| myoC-225 | + | UGGCCUCCAGGUCUAAGCGU | 20 | 611 |
| myoC-197 | - | UGAGAAUCUGGCCAGGAGGU | 20 | 583 |
| myoC-232 | + | UCUGGGCAGCUGGAUUCAUU | 20 | 618 |
| myoC-169 | - | UGUGCACGUUGCUGCAGCUU | 20 | 555 |
| myoC-224 | + | CAGGGAGCUGAGUCGAGCUU | 20 | 610 |
| myoC-210 | + | CAGUCUCCAACUCUCUGGUU | 20 | 596 |

**Table 6C** provides exemplary targeting domains for knocking out the *MYOC* gene selected according to the third tier parameters. The targeting domains bind within the first 500 bp of the coding sequence (e.g., within 500 bp downstream from the start codon) and start with a 5'G. It is contemplated herein that in an embodiment the targeting domain hybridizes to the target domain through complementary base pairing. Any of the targeting domains in the Table 1can be used with a *S. pyogeness* Cas9 molecule that generates a double stranded break (Cas9 nuclease) or a single-stranded break (Cas9 nickase).

**Table 6C**

| 3rd Tier | | | | |
|---|---|---|---|---|
| gRNA Name | DNA Strand | Targeting Domain | Target Site Length | SEQ ID NO |
| myoC-159 | + | GUGGAGGAGGCUCUCCA | 17 | 549 |
| myoC-132 | - | GACAGCUCAGCUCAGGA | 17 | 527 |
| myoC-168 | + | GGGACUGGCCACACUGA | 17 | 554 |
| myoC-142 | - | GUUGGAAAGCAGCAGCC | 17 | 537 |
| myoC-164 | + | GGAUGACUGACAUGGCC | 17 | 551 |
| myoC-130 | - | GCUGCUUCUGGCCUGCC | 17 | 525 |
| myoC-151 | + | GCUGCUUUCCAACCUCC | 17 | 543 |
| myoC-162 | + | GAGCUUUGGUGGCCUCC | 17 | 550 |
| myoC-158 | + | GGUGGAGGAGGCUCUCC | 17 | 548 |
| myoC-156 | + | GCCCCUCCUGGGUCUCC | 17 | 547 |
| myoC-165 | + | GCUCUGCUCUGGGCAGC | 17 | 552 |
| myoC-134 | - | GGGGCUGCAGAGGGAGC | 17 | 529 |
| myoC-137 | - | GCUGGGCACCCUGAGGC | 17 | 532 |
| myoC-140 | - | GCAAGAAAAUGAGAAUC | 17 | 535 |
| myoC-154 | + | GGUCCCGCUCCCGCCUC | 17 | 545 |
| myoC-153 | + | GGCAGUCUCCAACUCUC | 17 | 544 |
| myoC-3163 | + | GAAGAACCUCAUUGCAG | 17 | 2909 |
| myoC-133 | - | GCCCCAGGAGACCCAGG | 17 | 528 |
| myoC-143 | - | GGAAAGCAGCAGCCAGG | 17 | 538 |
| myoC-136 | - | GGGAGCUGGGCACCCUG | 17 | 531 |
| myoC-131 | - | GCCUGGUGUGGGAUGUG | 17 | 526 |
| myoC-135 | - | GGGCUGCAGAGGGAGCU | 17 | 530 |
| myoC-141 | - | GAAUCUGGCCAGGAGGU | 17 | 536 |
| myoC-120 | + | GGGCCUGGCAGCCUGGUCCA | 20 | 519 |
| myoC-99 | - | GACCCAGGAGGGGCUGCAGA | 20 | 504 |
| myoC-97 | - | GGACCAGGCUGCCAGGCCCC | 20 | 502 |
| myoC-92 | - | GCUGCUGCUUCUGGCCUGCC | 20 | 498 |
| myoC-118 | + | GCUCCCUCUGCAGCCCCUCC | 20 | 517 |
| myoC-119 | + | GCAGCCCCUCCUGGGUCUCC | 20 | 518 |
| myoC-128 | + | GGCAGGCCAGAAGCAGCAGC | 20 | 524 |
| myoC-100 | - | GGAGGGGCUGCAGAGGGAGC | 20 | 505 |
| myoC-103 | - | GGAGCUGGGCACCCUGAGGC | 20 | 508 |
| myoC-96 | - | GGGCCAGGACAGCUCAGCUC | 20 | 501 |
| myoC-116 | + | GUAGGCAGUCUCCAACUCUC | 20 | 483 |
| myoC-98 | - | GGCCCCAGGAGACCCAGGAG | 20 | 503 |
| myoC-108 | - | GUUGGAAAGCAGCAGCCAGG | 20 | 480 |
| myoC-102 | - | GGGAGCUGGGCACCCUGAGG | 20 | 507 |
| myoC-94 | - | GGCCUGCCUGGUGUGGGAUG | 20 | 499 |
| myoC-101 | - | GAGGGGCUGCAGAGGGAGCU | 20 | 506 |

**Table 6D** provides exemplary targeting domains for knocking out the *MYOC* gene selected according to the fourth tier parameters. The targeting domains bind within the first 500 bp of the coding sequence (e.g., within 500 bp downstream from the start codon). It is contemplated herein that in an embodiment the targeting domain hybridizes to the target domain through complementary base pairing. Any of the targeting domains in the Table 1can be used with a *S. pyogeness* Cas9 molecule that generates a double stranded break (Cas9 nuclease) or a single-stranded break (Cas9 nickase).

**Table 6D**

| 4th Tier | | | | |
|---|---|---|---|---|
| gRNA Name | DNA Strand | Targeting Domain | Target Site Length | SEQ ID NO |
| myoC-293 | + | CCUGGCAGCCUGGUCCA | 17 | 679 |
| myoC-265 | - | CCAGGAGGGGCUGCAGA | 17 | 651 |
| myoC-262 | - | CCCCAGGAGACCCAGGA | 17 | 648 |
| myoC-299 | + | UGUCUCUCUGUAAGUUA | 17 | 685 |
| myoC-308 | + | CCCCCACAUCCCACACC | 17 | 694 |
| myoC-261 | - | CAGGCCCCAGGAGACCC | 17 | 647 |
| myoC-260 | - | CCAGGCUGCCAGGCCCC | 17 | 646 |
| myoC-292 | + | CCUGGGGCCUGGCAGCC | 17 | 678 |
| myoC-253 | - | CUGCCCAGAGCAGAGCC | 17 | 639 |
| myoC-249 | - | UGUGGGAUGUGGGGGCC | 17 | 635 |
| myoC-291 | + | CUGGGUCUCCUGGGGCC | 17 | 677 |
| myoC-272 | - | AAAAUGAGAAUCUGGCC | 17 | 658 |
| myoC-259 | - | CCUUGGACCAGGCUGCC | 17 | 645 |
| myoC-287 | + | CCCUCUGCAGCCCCUCC | 17 | 673 |
| myoC-307 | + | CCUGAGCUGAGCUGUCC | 17 | 693 |
| myoC-311 | + | AGCAGCAGCUGGACAGC | 17 | 697 |
| myoC-286 | + | UGGUUUGGGUUUCCAGC | 17 | 672 |
| myoC-310 | + | AGGCCAGAAGCAGCAGC | 17 | 696 |
| myoC-267 | - | CACCCUGAGGCGGGAGC | 17 | 653 |
| myoC-309 | + | CACAUCCCACACCAGGC | 17 | 695 |
| myoC-250 | - | CCAGGACAGCUCAGCUC | 17 | 636 |
| myoC-300 | + | AUGGCCUGGCUCUGCUC | 17 | 686 |
| myoC-312 | + | UGGACAGCUGGCAUCUC | 17 | 698 |
| myoC-243 | - | UGUCCAGCUGCUGCUUC | 17 | 629 |
| myoC-264 | - | CCCAGGAGGGGCUGCAG | 17 | 650 |
| myoC-251 | - | AAGGCCAAUGACCAGAG | 17 | 637 |
| myoC-263 | - | CCCAGGAGACCCAGGAG | 17 | 649 |
| myoC-273 | - | AUGAGAAUCUGGCCAGG | 17 | 659 |
| myoC-282 | + | CUGACUUGUCUCGGAGG | 17 | 668 |
| myoC-266 | - | AGCUGGGCACCCUGAGG | 17 | 652 |
| myoC-295 | + | CCAAGGUCAAUUGGUGG | 17 | 681 |
| myoC-248 | - | CCUGGUGUGGGAUGUGG | 17 | 634 |
| myoC-246 | - | CUGCCUGGUGUGGGAUG | 17 | 632 |
| myoC-290 | + | CCCUCCUGGGUCUCCUG | 17 | 676 |
| myoC-244 | - | UUCUGGCCUGCCUGGUG | 17 | 630 |
| myoC-3164 | + | AUUGCAGAGGCUUGGUG | 17 | 2910 |
| myoC-302 | + | UGGGCAGCUGGAUUCAU | 17 | 688 |
| myoC-288 | + | CCUCUGCAGCCCCUCCU | 17 | 674 |
| myoC-289 | + | CCCCUCCUGGGUCUCCU | 17 | 675 |
| myoC-3165 | + | ACCUCAUUGCAGAGGCU | 17 | 2911 |
| myoC-301 | + | UGGCCUGGCUCUGCUCU | 17 | 687 |
| myoC-247 | - | UGCCUGGUGUGGGAUGU | 17 | 633 |
| myoC-283 | + | UCGGAGGAGGUUGCUGU | 17 | 669 |
| myoC-245 | - | UCUGGCCUGCCUGGUGU | 17 | 631 |
| myoC-284 | + | UCUCCAACUCUCUGGUU | 17 | 670 |
| myoC-242 | - | CACGUUGCUGCAGCUUU | 17 | 628 |
| myoC-285 | + | CUCCAACUCUCUGGUUU | 17 | 671 |
| myoC-223 | + | UUGGUGGAGGAGGCUCUCCA | 20 | 609 |
| myoC-187 | - | AGGCCCCAGGAGACCCAGGA | 20 | 573 |
| myoC-175 | - | CAGGACAGCUCAGCUCAGGA | 20 | 561 |
| myoC-193 | - | AGGAAGAGAAGAAGCGACUA | 20 | 579 |
| myoC-238 | + | U GGCCCCCACAU CCCACACC | 20 | 624 |
| myoC-185 | - | UGCCAGGCCCCAGGAGACCC | 20 | 571 |
| myoC-218 | + | UCUCCUGGGGCCUGGCAGCC | 20 | 604 |
| myoC-178 | - | CAGCUGCCCAGAGCAGAGCC | 20 | 564 |
| myoC-174 | - | UGGUGUGGGAUGUGGGGGCC | 20 | 560 |
| myoC-217 | + | CUCCUGGGUCUCCUGGGGCC | 20 | 603 |
| myoC-195 | - | AAGAAAAUGAGAAUCUGGCC | 20 | 581 |
| myoC-184 | - | UGACCUUGGACCAGGCUGCC | 20 | 570 |
| myoC-237 | + | CUUCCUGAGCUGAGCUGUCC | 20 | 623 |
| myoC-240 | + | AGAAGCAGCAGCUGGACAGC | 20 | 626 |
| myoC-230 | + | CUGGCUCUGCUCUGGGCAGC | 20 | 616 |
| myoC-194 | - | AAGGCAAGAAAAUGAGAAUC | 20 | 580 |
| myoC-188 | - | AGACCCAGGAGGGGCUGCAG | 20 | 574 |
| myoC-176 | - | AGGAAGGCCAAUGACCAGAG | 20 | 562 |
| myoC-186 | - | CAGGCCCCAGGAGACCCAGG | 20 | 572 |
| myoC-196 | - | AAAAUGAGAAUCUGGCCAGG | 20 | 582 |
| myoC-173 | - | CUGCCUGGUGUGGGAUGUGG | 20 | 559 |
| myoC-189 | - | AGAGGGAGCUGGGCACCCUG | 20 | 575 |
| myoC-216 | + | AGCCCCUCCUGGGUCUCCUG | 20 | 602 |
| myoC-171 | - | UGCUUCUGGCCUGCCUGGUG | 20 | 557 |
| myoC-172 | - | CCUGCCUGGUGUGGGAUGUG | 20 | 558 |
| myoC-231 | + | CUCUGGGCAGCUGGAUUCAU | 20 | 617 |
| myoC-214 | + | CUCCCUCUGCAGCCCCUCCU | 20 | 600 |
| myoC-215 | + | CAGCCCCUCCUGGGUCUCCU | 20 | 601 |
| myoC-229 | + | ACAUGGCCUGGCUCUGCUCU | 20 | 615 |
| myoC-211 | + | AGUCUCCAACUCUCUGGUUU | 20 | 597 |

**Table 6E** provides exemplary targeting domains for knocking out the *MYOC* gene selected according to the fifth tier parameters. The targeting domains fall in the coding sequence of the gene, downstream of the first 500bp of coding sequence (e.g., anywhere from +500 (relative to the start codon) to the stop codon of the gene). It is contemplated herein that in an embodiment the targeting domain hybridizes to the target domain through complementary base pairing. Any of the targeting domains in the Table 1can be used with a *S. pyogeness* Cas9 molecule that generates a double stranded break (Cas9 nuclease) or a single-stranded break (Cas9 nickase).

**Table 6E**

| 5th Tier | | | | |
|---|---|---|---|---|
| gRNA Name | DNA Strand | Targeting Domain | Target Site Length | SEQ ID NO |
| myoC-663 | + | UUAUUUCACAAUGUAAA | 17 | 963 |
| myoC-610 | + | CAGUUUGGAGAGGACAA | 17 | 918 |
| myoC-43 | - | AGCACCGAUGAGGCCAA | 17 | 433 |
| myoC-668 | + | GUAACAUGCAAGAGCAA | 17 | 968 |
| myoC-567 | - | CCAAGCUGUACAGGCAA | 17 | 888 |
| myoC-145 | - | GGUAGCAAGGCUGAGAA | 17 | 540 |
| myoC-626 | + | GUAUGUGAACCUUAGAA | 17 | 926 |
| myoC-578 | - | GGGGGGAGCAGGCUGAA | 17 | 899 |
| myoC-85 | + | UUAUAGCGGUUCUUGAA | 17 | 473 |
| myoC-670 | + | AUGCUGACAGAAGAUAA | 17 | 970 |
| myoC-657 | - | AAAAGCAUAACUUCUAA | 17 | 957 |
| myoC-662 | + | UUUAUUUCACAAUGUAA | 17 | 962 |
| myoC-646 | - | AUCCAGAAGGAUGAACA | 17 | 946 |
| myoC-77 | - | CUGGGACAACUUGAACA | 17 | 466 |
| myoC-36 | - | UUCUUGGGGUGGCUACA | 17 | 429 |
| myoC-601 | + | GCUGCUGACGGUGUACA | 17 | 909 |
| myoC-656 | - | UGCUCUUGCAUGUUACA | 17 | 956 |
| myoC-31 | - | CCUGGAGCUGGCUACCA | 17 | 425 |
| myoC-580 | - | GGAGAGCCAGCCAGCCA | 17 | 901 |
| myoC-50 | + | CGUGGUAGCCAGCUCCA | 17 | 397 |
| myoC-81 | + | AAAGAGCUUCUUCUCCA | 17 | 469 |
| myoC-538 | - | GGGAGCCUCUAUUUCCA | 17 | 880 |
| myoC-531 | - | UAGGCCACUGGAAAGCA | 17 | 873 |
| myoC-144 | - | GCAGCCAGGAGGUAGCA | 17 | 539 |
| myoC-524 | - | AAUCGACACAGUUGGCA | 17 | 866 |
| myoC-527 | - | AUCAGCCAGUUUAUGCA | 17 | 869 |
| myoC-570 | - | GCAGAAGGAGAUGCUCA | 17 | 891 |
| myoC-89 | + | CUUGAAUGGGAUGGUCA | 17 | 476 |
| myoC-3166 | + | GAUUCCCACAAAGUUCA | 17 | 2912 |
| myoC-345 | + | CUCCUGAGAUAGCCAGA | 17 | 731 |
| myoC-645 | - | GUUUUCAUUAAUCCAGA | 17 | 945 |
| myoC-568 | - | GUACAGGCAAUGGCAGA | 17 | 889 |
| myoC-3167 | - | CCACCAGGCUCCAGAGA | 17 | 2913 |
| myoC-342 | - | UAUCUCAGGAGUGGAGA | 17 | 728 |
| myoC-274 | - | AGGUAGCAAGGCUGAGA | 17 | 660 |
| myoC-28 | - | GACAGUGAAGGCUGAGA | 17 | 401 |
| myoC-625 | + | AGUAUGUGAACCUUAGA | 17 | 925 |
| myoC-671 | + | AUUCCUGAAUAGUUAGA | 17 | 971 |
| myoC-87 | + | GCGGUUCUUGAAUGGGA | 17 | 446 |
| myoC-352 | + | GGACUUCAGUUCCUGGA | 17 | 738 |
| myoC-602 | + | GGUGCCACAGAUGAUGA | 17 | 910 |
| myoC-577 | - | UGGGGGGAGCAGGCUGA | 17 | 898 |
| myoC-600 | + | UGAGGUGUAGCUGCUGA | 17 | 908 |
| myoC-649 | - | CAGGAAUUGUAGUCUGA | 17 | 949 |
| myoC-27 | - | GAAUACCGAGACAGUGA | 17 | 392 |
| myoC-90 | + | GUCAUAAGCAAAGUUGA | 17 | 447 |
| myoC-337 | - | UGCUUCCCGAAUUUUGA | 17 | 723 |
| myoC-46 | + | UAGCCACCCCAAGAAUA | 17 | 395 |
| myoC-528 | - | GCAGGGCUACCCUUCUA | 17 | 870 |
| myoC-519 | - | ACAAUUACUGGCAAGUA | 17 | 861 |
| myoC-655 | - | UUGGGGCAAAAGCUGUA | 17 | 955 |
| myoC-635 | + | GUGGUCUCCUGGGUGUA | 17 | 935 |
| myoC-604 | + | UGGCGACUGACUGCUUA | 17 | 912 |
| myoC-650 | - | UCUUCUGUCAGCAUUUA | 17 | 950 |
| myoC-627 | + | GGUAGCCCUGCAUAAAC | 17 | 927 |
| myoC-343 | - | AGUGGAGAGGGAGACAC | 17 | 729 |
| myoC-279 | + | CUCGGGUCUGGGGACAC | 17 | 665 |
| myoC-72 | - | AACUUUGCUUAUGACAC | 17 | 464 |
| myoC-530 | - | CAUACUGCCUAGGCCAC | 17 | 872 |
| myoC-532 | - | AGGCCACUGGAAAGCAC | 17 | 874 |
| myoC-73 | - | GCUUAUGACACAGGCAC | 17 | 451 |
| myoC-47 | + | AGCCACCCCAAGAAUAC | 17 | 435 |
| myoC-344 | + | GAAACUUAACUUCAUAC | 17 | 730 |
| myoC-566 | - | AAGCCUCCAAGCUGUAC | 17 | 887 |
| myoC-518 | - | ACAGCAGAAACAAUUAC | 17 | 860 |
| myoC-629 | + | GGUCAUACUCAAAAACC | 17 | 929 |
| myoC-557 | - | UGGAACUCGAACAAACC | 17 | 883 |
| myoC-148 | - | GCUCGGGCUGUGCCACC | 17 | 490 |
| myoC-3168 | - | UCUUUUCUGAAUUUACC | 17 | 2914 |
| myoC-521 | - | CACCUACCCCUACACCC | 17 | 863 |
| myoC-562 | - | GAUUGACUACAACCCCC | 17 | 886 |
| myoC-583 | + | UUCAGCCUGCUCCCCCC | 17 | 904 |
| myoC-621 | + | UUCUGGACUCAGCGCCC | 17 | 921 |
| myoC-581 | - | CCAGCCAGCCAGGGCCC | 17 | 902 |
| myoC-1590 | + | CAAAGCUGCCUGGGCCC | 17 | 1805 |
| myoC-29 | - | GCUGAGAAGGAAAUCCC | 17 | 423 |
| myoC-605 | + | ACGGAUGUUUGUCUCCC | 17 | 913 |
| myoC-79 | + | CAUGUUCAAGUUGUCCC | 17 | 467 |
| myoC-579 | - | GGGAGAGCCAGCCAGCC | 17 | 900 |
| myoC-142 | - | GUUGGAAAGCAGCAGCC | 17 | 537 |
| myoC-3169 | + | UUACCUUCUCUGGAGCC | 17 | 2915 |
| myoC-525 | - | UGGCACGGAUGUCCGCC | 17 | 867 |
| myoC-674 | + | AAGCAGUCAAAGCUGCC | 17 | 974 |
| myoC-75 | - | AGAAGAAGCUCUUUGCC | 17 | 465 |
| myoC-644 | + | ACUAGUUCUCCACAUCC | 17 | 944 |
| myoC-280 | + | UCAGCCUUGCUACCUCC | 17 | 666 |
| myoC-49 | + | CCGUGGUAGCCAGCUCC | 17 | 436 |
| myoC-571 | - | GAGAUGCUCAGGGCUCC | 17 | 892 |
| myoC-632 | + | UUCUCCACGUGGUCUCC | 17 | 932 |
| myoC-80 | + | CAAAGAGCUUCUUCUCC | 17 | 468 |
| myoC-336 | - | GGACACUUUGGCCUUCC | 17 | 722 |
| myoC-351 | + | GCUCGGACUUCAGUUCC | 17 | 737 |
| myoC-537 | - | GGGGAGCCUCUAUUUCC | 17 | 879 |
| myoC-349 | + | UUCAAAAUUCGGGAAGC | 17 | 735 |
| myoC-39 | - | UUGGCUGUGGAUGAAGC | 17 | 430 |
| myoC-576 | - | GGGCUCCUGGGGGGAGC | 17 | 897 |
| myoC-30 | - | AAGGAAAUCCCUGGAGC | 17 | 424 |
| myoC-1591 | + | GCUGCCUGGGCCCUGGC | 17 | 1801 |
| myoC-582 | + | CCUGGGCCCUGGCUGGC | 17 | 903 |
| myoC-664 | + | UUACUUAUAUUCGAUGC | 17 | 964 |
| myoC-526 | - | CAUCAGCCAGUUUAUGC | 17 | 868 |
| myoC-556 | - | ACUGAACCCAGAGAAUC | 17 | 882 |
| myoC-338 | - | UUGAAGGAGAGCCCAUC | 17 | 724 |
| myoC-535 | - | GGUGCUGUGGUGUACUC | 17 | 877 |
| myoC-40 | - | UGGAUGAAGCAGGCCUC | 17 | 431 |
| myoC-658 | - | AAGCAGAAUAGCUCCUC | 17 | 958 |
| myoC-569 | - | GGCAGAAGGAGAUGCUC | 17 | 890 |
| myoC-147 | - | GACCCGAGACACUGCUC | 17 | 489 |
| myoC-339 | - | GCCCAUCUGGCUAUCUC | 17 | 725 |
| myoC-277 | + | AGCCCGAGCAGUGUCUC | 17 | 663 |
| myoC-606 | + | UCGAGUUCCAGAUUCUC | 17 | 914 |
| myoC-3170 | + | UGCAUUCUUACCUUCUC | 17 | 2916 |
| myoC-149 | + | GAGCAGUGUCUCGGGUC | 17 | 491 |
| myoC-88 | + | UCUUGAAUGGGAUGGUC | 17 | 475 |
| myoC-348 | + | GCUCUCCUUCAAAAUUC | 17 | 734 |
| myoC-647 | - | UCACCAUCUAACUAUUC | 17 | 947 |
| myoC-672 | + | GACCAUGUUCAUCCUUC | 17 | 972 |
| myoC-52 | + | AUAUCUUAUGACAGUUC | 17 | 438 |
| myoC-669 | + | CAAGAGCAAUGGUUUUC | 17 | 969 |
| myoC-146 | - | GUAGCAAGGCUGAGAAG | 17 | 541 |
| myoC-45 | + | UGCUGUAAAUGACCCAG | 17 | 434 |
| myoC-665 | + | UAUUCGAUGCUGGCCAG | 17 | 965 |
| myoC-82 | + | AAGAGCUUCUUCUCCAG | 17 | 470 |
| myoC-623 | + | GCACCCGUGCUUUCCAG | 17 | 923 |
| myoC-3171 | - | AAGGUAAGAAUGCAGAG | 17 | 2917 |
| myoC-340 | - | UCUGGCUAUCUCAGGAG | 17 | 726 |
| myoC-341 | - | CUAUCUCAGGAGUGGAG | 17 | 727 |
| myoC-609 | + | CUGGGUUCAGUUUGGAG | 17 | 917 |
| myoC-643 | + | GCUGUUCUCAGCGUGAG | 17 | 943 |
| myoC-622 | + | CAGCGCCCUGGAAAUAG | 17 | 922 |
| myoC-84 | + | UGCUGCUGUACUUAUAG | 17 | 472 |
| myoC-636 | + | UGGUCUCCUGGGUGUAG | 17 | 936 |
| myoC-522 | - | ACACCCAGGAGACCACG | 17 | 864 |
| myoC-631 | + | UGUGUCGAUUCUCCACG | 17 | 931 |
| myoC-333 | - | UUAAUGCAGUUUCUACG | 17 | 719 |
| myoC-616 | + | AAUACGGGAACUGUCCG | 17 | 920 |
| myoC-536 | - | GUGCUGUGGUGUACUCG | 17 | 878 |
| myoC-143 | - | GGAAAGCAGCAGCCAGG | 17 | 538 |
| myoC-83 | + | AGAGCUUCUUCUCCAGG | 17 | 471 |
| myoC-638 | + | CUGGGUGUAGGGGUAGG | 17 | 938 |
| myoC-35 | - | UUCCCGUAUUCUUGGGG | 17 | 428 |
| myoC-575 | - | UGCUCAGGGCUCCUGGG | 17 | 896 |
| myoC-3172 | - | UAAGAAUGCAGAGUGGG | 17 | 2918 |
| myoC-630 | + | CAUACUCAAAAACCUGG | 17 | 930 |
| myoC-3173 | + | CCUUCUCUGGAGCCUGG | 17 | 2919 |
| myoC-574 | - | AUGCUCAGGGCUCCUGG | 17 | 895 |
| myoC-3174 | - | GUAAGAAUGCAGAGUGG | 17 | 2920 |
| myoC-585 | + | UUGCCUGUACAGCUUGG | 17 | 906 |
| myoC-42 | - | CAUUUACAGCACCGAUG | 17 | 432 |
| myoC-514 | - | CUGAAUUUACCAGGAUG | 17 | 856 |
| myoC-628 | + | GCAUAAACUGGCUGAUG | 17 | 928 |
| myoC-573 | - | GAUGCUCAGGGCUCCUG | 17 | 894 |
| myoC-38 | - | GGACAUUGACUUGGCUG | 17 | 402 |
| myoC-599 | + | GACGGUAGCAUCUGCUG | 17 | 907 |
| myoC-533 | - | GGAAAGCACGGGUGCUG | 17 | 875 |
| myoC-559 | - | AAUGCCUUCAUCAUCUG | 17 | 885 |
| myoC-648 | - | UCAGGAAUUGUAGUCUG | 17 | 948 |
| myoC-150 | + | GCAGUGUCUCGGGUCUG | 17 | 542 |
| myoC-3175 | - | GGUAAGAAUGCAGAGUG | 17 | 2921 |
| myoC-520 | - | CUGGCAAGUAUGGUGUG | 17 | 862 |
| myoC-666 | + | AGUUAUGCUUUUUAUUG | 17 | 966 |
| myoC-642 | + | AGGGGUAGGUGGGCUUG | 17 | 942 |
| myoC-667 | + | CUUUUUAUUGUGGCUUG | 17 | 967 |
| myoC-34 | - | CAGUUCCCGUAUUCUUG | 17 | 427 |
| myoC-654 | - | AGUUUUCUUGUGAUUUG | 17 | 954 |
| myoC-3176 | - | CUCUUCCUUGAACUUUG | 17 | 2922 |
| myoC-86 | + | UAUAGCGGUUCUUGAAU | 17 | 474 |
| myoC-603 | + | ACAGAUGAUGAAGGCAU | 17 | 911 |
| myoC-44 | + | GGCACCUUUGGCCUCAU | 17 | 404 |
| myoC-346 | + | UCCUGAGAUAGCCAGAU | 17 | 732 |
| myoC-651 | - | CUUCUGUCAGCAUUUAU | 17 | 951 |
| myoC-673 | + | CUGGAUUAAUGAAAACU | 17 | 973 |
| myoC-37 | - | CUACACGGACAUUGACU | 17 | 394 |
| myoC-534 | - | GGGUGCUGUGGUGUACU | 17 | 876 |
| myoC-558 | - | GGAACUCGAACAAACCU | 17 | 884 |
| myoC-624 | + | GUGCUUUCCAGUGGCCU | 17 | 924 |
| myoC-529 | - | AGGUUCACAUACUGCCU | 17 | 871 |
| myoC-675 | + | AGCAGUCAAAGCUGCCU | 17 | 975 |
| myoC-76 | - | GAAGAAGCUCUUUGCCU | 17 | 452 |
| myoC-572 | - | AGAUGCUCAGGGCUCCU | 17 | 893 |
| myoC-633 | + | UCUCCACGUGGUCUCCU | 17 | 933 |
| myoC-584 | + | CCAUUGCCUGUACAGCU | 17 | 905 |
| myoC-350 | + | AGGAACUUCAGUUAGCU | 17 | 736 |
| myoC-640 | + | GUAGGGGUAGGUGGGCU | 17 | 940 |
| myoC-275 | - | AGACCCGAGACACUGCU | 17 | 661 |
| myoC-78 | + | GGAGGCUUUUCACAUCU | 17 | 445 |
| myoC-41 | - | GGAUGAAGCAGGCCUCU | 17 | 403 |
| myoC-607 | + | CGAGUUCCAGAUUCUCU | 17 | 915 |
| myoC-278 | + | AGCAGUGUCUCGGGUCU | 17 | 664 |
| myoC-51 | + | CUCAGCCUUCACUGUCU | 17 | 437 |
| myoC-276 | + | CAGCCCGAGCAGUGUCU | 17 | 662 |
| myoC-544 | - | GACAGUUCCCGUAUUCU | 17 | 881 |
| myoC-523 | - | UGGAGAAUCGACACAGU | 17 | 865 |
| myoC-660 | - | UUCAGAUAGAAUACAGU | 17 | 960 |
| myoC-659 | - | GAUGCAUUUACUACAGU | 17 | 959 |
| myoC-3177 | - | AGGUAAGAAUGCAGAGU | 17 | 2923 |
| myoC-3178 | + | UUCAAGGAAGAGAACGU | 17 | 2924 |
| myoC-639 | + | UGGGUGUAGGGGUAGGU | 17 | 939 |
| myoC-637 | + | CUCCUGGGUGUAGGGGU | 17 | 937 |
| myoC-517 | - | GGAGAACUAGUUUGGGU | 17 | 859 |
| myoC-634 | + | CGUGGUCUCCUGGGUGU | 17 | 934 |
| myoC-3179 | - | UCUUCCUUGAACUUUGU | 17 | 2925 |
| myoC-347 | + | GGCUCUCCUUCAAAAUU | 17 | 733 |
| myoC-334 | - | AGUUUCUACGUGGAAUU | 17 | 720 |
| myoC-652 | - | CAAGUUUUCUUGUGAUU | 17 | 952 |
| myoC-335 | - | GUGGAAUUUGGACACUU | 17 | 721 |
| myoC-611 | + | GAGGACAAUGGCACCUU | 17 | 919 |
| myoC-641 | + | UAGGGGUAGGUGGGCUU | 17 | 941 |
| myoC-33 | - | ACAGUUCCCGUAUUCUU | 17 | 426 |
| myoC-661 | - | UCAGAUAGAAUACAGUU | 17 | 961 |
| myoC-608 | + | AUUCUCUGGGUUCAGUU | 17 | 916 |
| myoC-515 | - | GAUGUGGAGAACUAGUU | 17 | 857 |
| myoC-3180 | + | UCAAGGAAGAGAACGUU | 17 | 2926 |
| myoC-653 | - | AAGUUUUCUUGUGAUUU | 17 | 953 |
| myoC-516 | - | AUGUGGAGAACUAGUUU | 17 | 858 |
| myoC-501 | + | AUUUUAUUUCACAAUGUAAA | 20 | 843 |
| myoC-448 | + | GUUCAGUUUGGAGAGGACAA | 20 | 799 |
| myoC-17 | - | UACAGCACCGAUGAGGCCAA | 20 | 415 |
| myoC-506 | + | CAUGUAACAUGCAAGAGCAA | 20 | 848 |
| myoC-406 | - | CCUCCAAGCUGUACAGGCAA | 20 | 770 |
| myoC-110 | - | GGAGGUAGCAAGGCUGAGAA | 20 | 513 |
| myoC-464 | + | GCAGUAUGUGAACCUUAGAA | 20 | 806 |
| myoC-417 | - | CCUGGGGGGAGCAGGCUGAA | 20 | 781 |
| myoC-66 | + | UACUUAUAGCGGUUCUUGAA | 20 | 461 |
| myoC-508 | + | UAAAUGCUGACAGAAGAUAA | 20 | 850 |
| myoC-495 | - | AUAAAAAGCAUAACUUCUAA | 20 | 837 |
| myoC-500 | + | AAUUUUAUUUCACAAUGUAA | 20 | 842 |
| myoC-484 | - | UUAAUCCAGAAGGAUGAACA | 20 | 826 |
| myoC-58 | - | UGCCUGGGACAACUUGAACA | 20 | 456 |
| myoC-10 | - | GUAUUCUUGGGGUGGCUACA | 20 | 388 |
| myoC-439 | + | GUAGCUGCUGACGGUGUACA | 20 | 790 |
| myoC-494 | - | CAUUGCUCUUGCAUGUUACA | 20 | 836 |
| myoC-5 | - | AUCCCUGGAGCUGGCUACCA | 20 | 407 |
| myoC-419 | - | AAGGGAGAGCCAGCCAGCCA | 20 | 783 |
| myoC-24 | + | GUCCGUGGUAGCCAGCUCCA | 20 | 391 |
| myoC-62 | + | GGCAAAGAGCUUCUUCUCCA | 20 | 448 |
| myoC-377 | - | UCGGGGAGCCUCUAUUUCCA | 20 | 763 |
| myoC-370 | - | GCCUAGGCCACUGGAAAGCA | 20 | 756 |
| myoC-109 | - | GCAGCAGCCAGGAGGUAGCA | 20 | 512 |
| myoC-363 | - | GAGAAUCGACACAGUUGGCA | 20 | 749 |
| myoC-366 | - | CUCAUCAGCCAGUUUAUGCA | 20 | 752 |
| myoC-409 | - | AUGGCAGAAGGAGAUGCUCA | 20 | 773 |
| myoC-70 | + | GUUCUUGAAUGGGAUGGUCA | 20 | 450 |
| myoC-325 | + | CCACUCCUGAGAUAGCCAGA | 20 | 711 |
| myoC-483 | - | CAAGUUUUCAUUAAUCCAGA | 20 | 825 |
| myoC-407 | - | GCUGUACAGGCAAUGGCAGA | 20 | 771 |
| myoC-3181 | - | GUGCCACCAGGCUCCAGAGA | 20 | 2927 |
| myoC-322 | - | GGCUAUCUCAGGAGUGGAGA | 20 | 708 |
| myoC-198 | - | AGGAGGUAGCAAGGCUGAGA | 20 | 584 |
| myoC-2 | - | CGAGACAGUGAAGGCUGAGA | 20 | 405 |
| myoC-463 | + | GGCAGUAUGUGAACCUUAGA | 20 | 805 |
| myoC-509 | + | ACAAUUCCUGAAUAGUUAGA | 20 | 851 |
| myoC-68 | + | AUAGCGGUUCUUGAAUGGGA | 20 | 443 |
| myoC-332 | + | CUCGGACUUCAGUUCCUGGA | 20 | 718 |
| myoC-440 | + | CAAGGUGCCACAGAUGAUGA | 20 | 791 |
| myoC-416 | - | UCCUGGGGGGAGCAGGCUGA | 20 | 780 |
| myoC-438 | + | UGCUGAGGUGUAGCUGCUGA | 20 | 789 |
| myoC-487 | - | AUUCAGGAAUUGUAGUCUGA | 20 | 829 |
| myoC-1 | - | GCUGAAUACCGAGACAGUGA | 20 | 398 |
| myoC-71 | + | UGUGUCAUAAGCAAAGUUGA | 20 | 463 |
| myoC-317 | - | UCCUGCUUCCCGAAUUUUGA | 20 | 703 |
| myoC-20 | + | GUGUAGCCACCCCAAGAAUA | 20 | 390 |
| myoC-367 | - | UAUGCAGGGCUACCCUUCUA | 20 | 753 |
| myoC-358 | - | GAAACAAUUACUGGCAAGUA | 20 | 744 |
| myoC-493 | - | GAUUUGGGGCAAAAGCUGUA | 20 | 835 |
| myoC-473 | + | CACGUGGUCUCCUGGGUGUA | 20 | 815 |
| myoC-442 | + | CAUUGGCGACUGACUGCUUA | 20 | 793 |
| myoC-488 | - | UUAUCUUCUGUCAGCAUUUA | 20 | 830 |
| myoC-465 | + | AAGGGUAGCCCUGCAUAAAC | 20 | 807 |
| myoC-323 | - | AGGAGUGGAGAGGGAGACAC | 20 | 709 |
| myoC-206 | + | UGUCUCGGGUCUGGGGACAC | 20 | 592 |
| myoC-53 | - | GUCAACUUUGCUUAUGACAC | 20 | 439 |
| myoC-369 | - | UCACAUACUGCCUAGGCCAC | 20 | 755 |
| myoC-371 | - | CCUAGGCCACUGGAAAGCAC | 20 | 757 |
| myoC-54 | - | UUUGCUUAUGACACAGGCAC | 20 | 453 |
| myoC-21 | + | UGUAGCCACCCCAAGAAUAC | 20 | 418 |
| myoC-324 | + | GAAGAAACUUAACUUCAUAC | 20 | 710 |
| myoC-405 | - | GAAAAGCCUCCAAGCUGUAC | 20 | 769 |
| myoC-357 | - | AGAACAGCAGAAACAAU UAC | 20 | 743 |
| myoC-467 | + | UGAGGUCAUACUCAAAAACC | 20 | 809 |
| myoC-396 | - | AUCUGGAACUCGAACAAACC | 20 | 766 |
| myoC-201 | - | ACUGCUCGGGCUGUGCCACC | 20 | 587 |
| myoC-3182 | - | UUUUCUUUUCUGAAUUUACC | 20 | 2928 |
| myoC-360 | - | GCCCACCUACCCCUACACCC | 20 | 746 |
| myoC-55 | - | CAUGAUUGACUACAACCCCC | 20 | 454 |
| myoC-421 | + | CCCUUCAGCCUGCUCCCCCC | 20 | 785 |
| myoC-459 | + | CAGUUCUGGACUCAGCGCCC | 20 | 801 |
| myoC-420 | - | GAGCCAGCCAGCCAGGGCCC | 20 | 784 |
| myoC-1576 | + | AGUCAAAGCUGCCUGGGCCC | 20 | 1802 |
| myoC-3 | - | AAGGCUGAGAAGGAAAUCCC | 20 | 406 |
| myoC-443 | + | CUUACGGAUGUUUGUCUCCC | 20 | 794 |
| myoC-60 | + | GACCAUGUUCAAGUUGUCCC | 20 | 441 |
| myoC-418 | - | GAAGGGAGAGCCAGCCAGCC | 20 | 782 |
| myoC-107 | - | GAGGUUGGAAAGCAGCAGCC | 20 | 511 |
| myoC-3183 | + | UUCUUACCUUCUCUGGAGCC | 20 | 2929 |
| myoC-364 | - | AGUUGGCACGGAUGUCCGCC | 20 | 750 |
| myoC-512 | + | GGAAAGCAGUCAAAGCUGCC | 20 | 854 |
| myoC-56 | - | UGGAGAAGAAGCUCUUUGCC | 20 | 455 |
| myoC-482 | + | CAAACUAGUUCUCCACAUCC | 20 | 824 |
| myoC-207 | + | UUCUCAGCCUUGCUACCUCC | 20 | 593 |
| myoC-23 | + | UGUCCGUGGUAGCCAGCUCC | 20 | 420 |
| myoC-410 | - | AAGGAGAUGCUCAGGGCUCC | 20 | 774 |
| myoC-470 | + | CGAUUCUCCACGUGGUCUCC | 20 | 812 |
| myoC-61 | + | AGGCAAAGAGCUUCUUCUCC | 20 | 458 |
| myoC-316 | - | UUUGGACACUUUGGCCUUCC | 20 | 702 |
| myoC-331 | + | UUAGCUCGGACUUCAGUUCC | 20 | 717 |
| myoC-376 | - | CUCGGGGAGCCUCUAUUUCC | 20 | 762 |
| myoC-329 | + | UCCUUCAAAAUUCGGGAAGC | 20 | 715 |
| myoC-13 | - | GACUUGGCUGUGGAUGAAGC | 20 | 400 |
| myoC-415 | - | UCAGGGCUCCUGGGGGGAGC | 20 | 779 |
| myoC-4 | - | GAGAAGGAAAUCCCUGGAGC | 20 | 399 |
| myoC-1577 | + | AAAGCUGCCUGGGCCCUGGC | 20 | 1803 |
| myoC-1578 | + | CUGCCUGGGCCCUGGCUGGC | 20 | 1804 |
| myoC-502 | + | AUCUUACUUAUAUUCGAUGC | 20 | 844 |
| myoC-365 | - | CCUCAUCAGCCAGUUUAUGC | 20 | 751 |
| myoC-395 | - | CAAACUGAACCCAGAGAAUC | 20 | 765 |
| myoC-318 | - | AUUUUGAAGGAGAGCCCAUC | 20 | 704 |
| myoC-374 | - | ACGGGUGCUGUGGUGUACUC | 20 | 760 |
| myoC-14 | - | CUGUGGAUGAAGCAGGCCUC | 20 | 413 |
| myoC-496 | - | AGGAAGCAGAAUAGCUCCUC | 20 | 838 |
| myoC-408 | - | AAUGGCAGAAGGAGAUGCUC | 20 | 772 |
| myoC-200 | - | CCAGACCCGAGACACUGCUC | 20 | 586 |
| myoC-319 | - | AGAGCCCAUCUGGCUAUCUC | 20 | 705 |
| myoC-202 | + | CACAGCCCGAGCAGUGUCUC | 20 | 588 |
| myoC-444 | + | UGUUCGAGUUCCAGAUUCUC | 20 | 795 |
| myoC-3184 | + | CUCUGCAUUCUUACCUUCUC | 20 | 2930 |
| myoC-203 | + | CCCGAGCAGUGUCUCGGGUC | 20 | 589 |
| myoC-69 | + | GGUUCUUGAAUGGGAUGGUC | 20 | 449 |
| myoC-328 | + | UGGGCUCUCCUUCAAAAUUC | 20 | 714 |
| myoC-485 | - | UGGUCACCAUCUAACUAUUC | 20 | 827 |
| myoC-510 | + | GGUGACCAUGUUCAUCCUUC | 20 | 852 |
| myoC-26 | + | CUCAUAUCUUAUGACAGUUC | 20 | 422 |
| myoC-507 | + | AUGCAAGAGCAAUGGUUUUC | 20 | 849 |
| myoC-111 | - | GAGGUAGCAAGGCUGAGAAG | 20 | 514 |
| myoC-19 | + | CGGUGCUGUAAAUGACCCAG | 20 | 417 |
| myoC-503 | + | UUAUAUUCGAUGCUGGCCAG | 20 | 845 |
| myoC-63 | + | GCAAAGAGCUUCUUCUCCAG | 20 | 442 |
| myoC-461 | + | ACAGCACCCGUGCUUUCCAG | 20 | 803 |
| myoC-3185 | - | GAGAAGGUAAGAAUGCAGAG | 20 | 2931 |
| myoC-320 | - | CCAUCUGGCUAUCUCAGGAG | 20 | 706 |
| myoC-321 | - | UGGCUAUCUCAGGAGUGGAG | 20 | 707 |
| myoC-447 | + | UCUCUGGGUUCAGUUUGGAG | 20 | 798 |
| myoC-481 | + | UCUGCUGUUCUCAGCGUGAG | 20 | 823 |
| myoC-460 | + | ACUCAGCGCCCUGGAAAUAG | 20 | 802 |
| myoC-65 | + | UCAUGCUGCUGUACUUAUAG | 20 | 460 |
| myoC-474 | + | ACGUGGUCUCCUGGGUGUAG | 20 | 816 |
| myoC-361 | - | CCUACACCCAGGAGACCACG | 20 | 747 |
| myoC-469 | + | AACUGUGUCGAUUCUCCACG | 20 | 811 |
| myoC-313 | - | CUUUUAAUGCAGUUUCUACG | 20 | 699 |
| myoC-22 | + | AAGAAUACGGGAACUGUCCG | 20 | 419 |
| myoC-375 | - | CGGGUGCUGUGGUGUACUCG | 20 | 761 |
| myoC-108 | - | GUUGGAAAGCAGCAGCCAGG | 20 | 480 |
| myoC-64 | + | CAAAGAGCUUCUUCUCCAGG | 20 | 459 |
| myoC-476 | + | CUCCUGGGUGUAGGGGUAGG | 20 | 818 |
| myoC-9 | - | CAGUUCCCGUAUUCUUGGGG | 20 | 410 |
| myoC-414 | - | AGAUGCUCAGGGCUCCUGGG | 20 | 778 |
| myoC-3186 | - | AGGUAAGAAUGCAGAGUGGG | 20 | 2932 |
| myoC-468 | + | GGUCAUACUCAAAAACCUGG | 20 | 810 |
| myoC-3187 | + | UUACCUUCUCUGGAGCCUGG | 20 | 2933 |
| myoC-413 | - | GAGAUGCUCAGGGCUCCUGG | 20 | 777 |
| myoC-3188 | - | AAGGUAAGAAUGCAGAGUGG | 20 | 2934 |
| myoC-423 | + | CCAUUGCCUGUACAGCUUGG | 20 | 787 |
| myoC-16 | - | GGUCAUUUACAGCACCGAUG | 20 | 389 |
| myoC-353 | - | UUUCUGAAUUUACCAGGAUG | 20 | 739 |
| myoC-466 | + | CCUGCAUAAACUGGCUGAUG | 20 | 808 |
| myoC-412 | - | GGAGAUGCUCAGGGCUCCUG | 20 | 776 |
| myoC-12 | - | CACGGACAUUGACUUGGCUG | 20 | 412 |
| myoC-437 | + | GUUGACGGUAGCAUCUGCUG | 20 | 788 |
| myoC-372 | - | ACUGGAAAGCACGGGUGCUG | 20 | 758 |
| myoC-398 | - | GCCAAUGCCUUCAUCAUCUG | 20 | 768 |
| myoC-486 | - | UAUUCAGGAAUUGUAGUCUG | 20 | 828 |
| myoC-205 | + | CGAGCAGUGUCUCGGGUCUG | 20 | 591 |
| myoC-3189 | - | GAAGGUAAGAAUGCAGAGUG | 20 | 2935 |
| myoC-359 | - | UUACUGGCAAGUAUGGUGUG | 20 | 745 |
| myoC-504 | + | AGAAGUUAUGCUUUUUAUUG | 20 | 846 |
| myoC-480 | + | UGUAGGGGUAGGUGGGCUUG | 20 | 822 |
| myoC-505 | + | AUGCUUUUUAUUGUGGCUUG | 20 | 847 |
| myoC-385 | - | GGACAGUUCCCGUAUUCUUG | 20 | 764 |
| myoC-492 | - | UCAAGUUUUCUUGUGAUUUG | 20 | 834 |
| myoC-3190 | - | GUUCUCUUCCUUGAACUUUG | 20 | 2936 |
| myoC-67 | + | ACUUAUAGCGGUUCUUGAAU | 20 | 462 |
| myoC-441 | + | GCCACAGAUGAUGAAGGCAU | 20 | 792 |
| myoC-18 | + | AAUGGCACCUUUGGCCUCAU | 20 | 416 |
| myoC-326 | + | CACUCCUGAGAUAGCCAGAU | 20 | 712 |
| myoC-489 | - | UAUCUUCUGUCAGCAUUUAU | 20 | 831 |
| myoC-511 | + | CUUCUGGAUUAAUGAAAACU | 20 | 853 |
| myoC-11 | - | UGGCUACACGGACAUUGACU | 20 | 411 |
| myoC-373 | - | CACGGGUGCUGUGGUGUACU | 20 | 759 |
| myoC-397 | - | UCUGGAACUCGAACAAACCU | 20 | 767 |
| myoC-462 | + | CCCGUGCUUUCCAGUGGCCU | 20 | 804 |
| myoC-368 | - | CUAAGGUUCACAUACUGCCU | 20 | 754 |
| myoC-513 | + | GAAAGCAGUCAAAGCUGCCU | 20 | 855 |
| myoC-57 | - | GGAGAAGAAGCUCUUUGCCU | 20 | 440 |
| myoC-411 | - | AGGAGAUGCUCAGGGCUCCU | 20 | 775 |
| myoC-471 | + | GAUUCUCCACGUGGUCUCCU | 20 | 813 |
| myoC-422 | + | CUGCCAUUGCCUGUACAGCU | 20 | 786 |
| myoC-330 | + | AGCAGGAACUUCAGUUAGCU | 20 | 716 |
| myoC-478 | + | GGUGUAGGGGUAGGUGGGCU | 20 | 820 |
| myoC-199 | - | CCCAGACCCGAGACACUGCU | 20 | 585 |
| myoC-59 | + | CUUGGAGGCUUUUCACAUCU | 20 | 457 |
| myoC-15 | - | UGUGGAUGAAGCAGGCCUCU | 20 | 414 |
| myoC-445 | + | GUUCGAGUUCCAGAUUCUCU | 20 | 796 |
| myoC-204 | + | CCGAGCAGUGUCUCGGGUCU | 20 | 590 |
| myoC-25 | + | CUUCUCAGCCUUCACUGUCU | 20 | 421 |
| myoC-112 | + | GCACAGCCCGAGCAGUGUCU | 20 | 481 |
| myoC-6 | - | ACGGACAGUUCCCGUAUUCU | 20 | 408 |
| myoC-362 | - | ACGUGGAGAAUCGACACAGU | 20 | 748 |
| myoC-498 | - | UGCUUCAGAUAGAAUACAGU | 20 | 840 |
| myoC-497 | - | UAAGAUGCAUUUACUACAGU | 20 | 839 |
| myoC-3191 | - | AGAAGGUAAGAAUGCAGAGU | 20 | 2937 |
| myoC-3192 | + | AAGUUCAAGGAAGAGAACGU | 20 | 2938 |
| myoC-477 | + | UCCUGGGUGUAGGGGUAGGU | 20 | 819 |
| myoC-475 | + | GGUCUCCUGGGUGUAGGGGU | 20 | 817 |
| myoC-356 | - | UGUGGAGAACUAGUUUGGGU | 20 | 742 |
| myoC-472 | + | CCACGUGGUCUCCUGGGUGU | 20 | 814 |
| myoC-3193 | - | UUCUCUUCCUUGAACUUUGU | 20 | 2939 |
| myoC-327 | + | AUGGGCUCUCCUUCAAAAUU | 20 | 713 |
| myoC-314 | - | UGCAGUUUCUACGUGGAAUU | 20 | 700 |
| myoC-490 | - | GUUCAAGUUUUCUUGUGAUU | 20 | 832 |
| myoC-315 | - | UACGUGGAAUUUGGACACUU | 20 | 701 |
| myoC-449 | + | GGAGAGGACAAUGGCACCUU | 20 | 800 |
| myoC-479 | + | GUGUAGGGGUAGGUGGGCUU | 20 | 821 |
| myoC-7 | - | CGGACAGUUCCCGUAUUCUU | 20 | 409 |
| myoC-499 | - | GCUUCAGAUAGAAUACAGUU | 20 | 841 |
| myoC-446 | + | CAGAUUCUCUGGGUUCAGUU | 20 | 797 |
| myoC-354 | - | CAGGAUGUGGAGAACUAGUU | 20 | 740 |
| myoC-3194 | + | AGUUCAAGGAAGAGAACGUU | 20 | 2940 |
| myoC-491 | - | UUCAAGUUUUCUUGUGAUUU | 20 | 833 |
| myoC-355 | - | AGGAUGUGGAGAACUAGUUU | 20 | 741 |

**Table 7A** provides exemplary targeting domains for knocking out the *MYOC* gene selected according to the first tier parameters. The targeting domains bind within the first 500 bp of the coding sequence (e.g., within 500 bp downstream from the start codon), have a high level of orthogonality, start with a 5'G, and PAM is NNGRRT. It is contemplated herein that in an embodiment the targeting domain hybridizes to the target domain through complementary base pairing. Any of the targeting domains in the Table 1can be used with a *S. aureus* Cas9 molecule that generates a double stranded break (Cas9 nuclease) or a single-stranded break (Cas9 nickase).

**Table 7A**

| 1st Tier | | | | |
|---|---|---|---|---|
| gRNA Name | DNA Strand | Targeting Domain | Target Site Length | SEQ ID NO |
| myoC-3195 | + | GGCCUCCAGGUCUAAGCG | 18 | 2941 |
| myoC-1677 | + | GUGGCCUCCAGGUCUAAGCG | 20 | 1938 |
| myoC-3196 | + | GGUGGCCUCCAGGUCUAAGCG | 21 | 2942 |
| myoC-3197 | + | GCUGGUCCCGCUCCCGCCU | 19 | 2943 |
| myoC-3198 | + | GGCAGUCUCCAACUCUCUGGU | 21 | 2944 |
| myoC-3199 | + | GUAGGCAGUCUCCAACUCUCUGGU | 24 | 2945 |
| myoC-3200 | + | GCUGUCUCUCUGUAAGUU | 18 | 2946 |
| myoC-3201 | + | GCUGCUGUCUCUCUGUAAGUU | 21 | 2947 |
| myoC-3202 | + | GUGCUGCUGUCUCUCUGUAAGUU | 23 | 2948 |
| myoC-3203 | + | GGUGCUGCUGUCUCUCUGUAAGUU | 24 | 2949 |
| myoC-3204 | - | GACCAGCUGGAAACCCAAACCA | 22 | 2950 |
| myoC-3205 | - | GGACCAGCUGGAAACCCAAACCA | 23 | 2951 |
| myoC-3206 | - | GGGACCAGCUGGAAACCCAAACCA | 24 | 2952 |
| myoC-3207 | - | GCUCAGGAAGGCCAAUGAC | 19 | 2953 |
| myoC-3208 | - | GCUCAGCUCAGGAAGGCCAAUGAC | 24 | 2954 |
| myoC-3209 | - | GCUUCUGGCCUGCCUGGUG | 19 | 2955 |
| myoC-3210 | - | GCGACUAAGGCAAGAAAAU | 19 | 2956 |
| myoC-3211 | - | GAAGCGACUAAGGCAAGAAAAU | 22 | 2957 |

**Table 7B** provides exemplary targeting domains for knocking out the *MYOC* gene selected according to the second tier parameters. The targeting domains bind within the first 500 bp of the coding sequence (e.g., within 500 bp downstream from the start codon), have a high level of orthogonality and PAM is NNGRRT. It is contemplated herein that in an embodiment the targeting domain hybridizes to the target domain through complementary base pairing. Any of the targeting domains in the Table 1can be used with a *S. aureus* Cas9 molecule that generates a double stranded break (Cas9 nuclease) or a single-stranded break (Cas9 nickase).

**Table 7B**

| 2nd Tier | | | | |
|---|---|---|---|---|
| gRNA Name | DNA Strand | Targeting Domain | Target Site Length | SEQ ID NO |
| myoC-3212 | + | UGGCCUCCAGGUCUAAGCG | 19 | 2958 |
| myoC-3213 | + | UGGUGGCCUCCAGGUCUAAGCG | 22 | 2959 |
| myoC-3214 | + | UUGGUGGCCUCCAGGUCUAAGCG | 23 | 2960 |
| myoC-3215 | + | UUUGGUGGCCUCCAGGUCUAAGCG | 24 | 2961 |
| myoC-3216 | + | CUGGUCCCGCUCCCGCCU | 18 | 2962 |
| myoC-1690 | + | AGCUGGUCCCGCUCCCGCCU | 20 | 1946 |
| myoC-3217 | + | CAGCUGGUCCCGCUCCCGCCU | 21 | 2963 |
| myoC-3218 | + | CCAGCUGGUCCCGCUCCCGCCU | 22 | 2964 |
| myoC-3219 | + | UCCAGCUGGUCCCGCUCCCGCCU | 23 | 2965 |
| myoC-3220 | + | UUCCAGCUGGUCCCGCUCCCGCCU | 24 | 2966 |
| myoC-3221 | + | AGGCAGUCUCCAACUCUCUGGU | 22 | 2967 |
| myoC-3222 | + | UAGGCAGUCUCCAACUCUCUGGU | 23 | 2968 |
| myoC-3223 | + | UGCUGUCUCUCUGUAAGUU | 19 | 2969 |
| myoC-1676 | + | CUGCUGUCUCUCUGUAAGUU | 20 | 1937 |
| myoC-3224 | + | UGCUGCUGUCUCUCUGUAAGUU | 22 | 2970 |
| myoC-3225 | - | AGCUGGAAACCCAAACCA | 18 | 2971 |
| myoC-3226 | - | CAGCUGGAAACCCAAACCA | 19 | 2972 |
| myoC-1635 | - | CCAGCUGGAAACCCAAACCA | 20 | 1904 |
| myoC-3227 | - | ACCAGCUGGAAACCCAAACCA | 21 | 2973 |
| myoC-3228 | - | UCAGUGUGGCCAGUCCCA | 18 | 2974 |
| myoC-3229 | - | UUCAGUGUGGCCAGUCCCA | 19 | 2975 |
| myoC-1604 | - | CUUCAGUGUGGCCAGUCCCA | 20 | 1884 |
| myoC-3230 | - | CCUUCAGUGUGGCCAGUCCCA | 21 | 2976 |
| myoC-3231 | - | ACCUUCAGUGUGGCCAGUCCCA | 22 | 2977 |
| myoC-3232 | - | UACCUUCAGUGUGGCCAGUCCCA | 23 | 2978 |
| myoC-3233 | - | AUACCUUCAGUGUGGCCAGUCCCA | 24 | 2979 |
| myoC-3234 | - | CUCAGGAAGGCCAAUGAC | 18 | 2980 |
| myoC-1603 | - | AGCUCAGGAAGGCCAAUGAC | 20 | 1883 |
| myoC-3235 | - | CAGCUCAGGAAGGCCAAUGAC | 21 | 2981 |
| myoC-3236 | - | UCAGCUCAGGAAGGCCAAUGAC | 22 | 2982 |
| myoC-3237 | - | CUCAGCUCAGGAAGGCCAAUGAC | 23 | 2983 |
| myoC-3238 | - | CUUCUGGCCUGCCUGGUG | 18 | 2984 |
| myoC-171 | - | UGCUUCUGGCCUGCCUGGUG | 20 | 557 |
| myoC-3239 | - | CGACUAAGGCAAGAAAAU | 18 | 2985 |
| myoC-1648 | - | AGCGACUAAGGCAAGAAAAU | 20 | 1914 |
| myoC-3240 | - | AAGCGACUAAGGCAAGAAAAU | 21 | 2986 |
| myoC-3241 | - | AGAAGCGACUAAGGCAAGAAAAU | 23 | 2987 |
| myoC-3242 | - | AAGAAGCGACUAAGGCAAGAAAAU | 24 | 2988 |

**Table 7C** provides exemplary targeting domains for knocking out the *MYOC* gene selected according to the third tier parameters. The targeting domains bind within the first 500 bp of the coding sequence (e.g., within 500 bp downstream from the start codon), and PAM is NNGRRT. It is contemplated herein that in an embodiment the targeting domain hybridizes to the target domain through complementary base pairing. Any of the targeting domains in the Table can be used with a *S. aureus* Cas9 molecule that generates a double stranded break (Cas9 nuclease) or a single-stranded break (Cas9 nickase).

**Table 7C**

| 3rd Tier | | | | |
|---|---|---|---|---|
| gRNA Name | DNA Strand | Targeting Domain | Target Site Length | SEQ ID NO |
| myoC-3243 | + | CUCCCUCUGCAGCCCCUC | 18 | 2989 |
| myoC-3244 | + | GCUCCCUCUGCAGCCCCUC | 19 | 2990 |
| myoC-1689 | + | AGCUCCCUCUGCAGCCCCUC | 20 | 1945 |
| myoC-3245 | + | CAGCUCCCUCUGCAGCCCCUC | 21 | 2991 |
| myoC-3246 | + | CCAGCUCCCUCUGCAGCCCCUC | 22 | 2992 |
| myoC-3247 | + | CCCAGCUCCCUCUGCAGCCCCUC | 23 | 2993 |
| myoC-3248 | + | GCCCAGCUCCCUCUGCAGCCCCUC | 24 | 2994 |
| myoC-3249 | + | UGGCUCUGCUCUGGGCAG | 18 | 2995 |
| myoC-3250 | + | CUGGCUCUGCUCUGGGCAG | 19 | 2996 |
| myoC-1674 | + | CCUGGCUCUGCUCUGGGCAG | 20 | 1935 |
| myoC-3251 | + | GCCUGGCUCUGCUCUGGGCAG | 21 | 2997 |
| myoC-3252 | + | GGCCUGGCUCUGCUCUGGGCAG | 22 | 2998 |
| myoC-3253 | + | UGGCCUGGCUCUGCUCUGGGCAG | 23 | 2999 |
| myoC-3254 | + | AUGGCCUGGCUCUGCUCUGGGCAG | 24 | 3000 |
| myoC-3255 | + | AGGAGGCUCUCCAGGGAG | 18 | 3001 |
| myoC-3256 | + | GAGGAGGCUCUCCAGGGAG | 19 | 3002 |
| myoC-1679 | + | GGAGGAGGCUCUCCAGGGAG | 20 | 1940 |
| myoC-3257 | + | UGGAGGAGGCUCUCCAGGGAG | 21 | 3003 |
| myoC-3258 | + | GUGGAGGAGGCUCUCCAGGGAG | 22 | 3004 |
| myoC-3259 | + | GGUGGAGGAGGCUCUCCAGGGAG | 23 | 3005 |
| myoC-3260 | + | UGGUGGAGGAGGCUCUCCAGGGAG | 24 | 3006 |
| myoC-3261 | + | AGUCUCCAACUCUCUGGU | 18 | 3007 |
| myoC-3262 | + | CAGUCUCCAACUCUCUGGU | 19 | 3008 |
| myoC-1691 | + | GCAGUCUCCAACUCUCUGGU | 20 | 1947 |
| myoC-3263 | - | CUGCUUCUGGCCUGCCUGGUG | 21 | 3009 |
| myoC-3264 | - | GCUGCUUCUGGCCUGCCUGGUG | 22 | 3010 |
| myoC-3265 | - | UGCUGCUUCUGGCCUGCCUGGUG | 23 | 3011 |
| myoC-3266 | - | CUGCUGCUUCUGGCCUGCCUGGUG | 24 | 3012 |

**Table 7D** provides exemplary targeting domains for knocking out the *MYOC* gene selected according to the fourth tier parameters. The targeting domains bind within the first 500 bp of the coding sequence (e.g., within 500 bp downstream from the start codon), and PAM is NNGRRV. It is contemplated herein that in an embodiment the targeting domain hybridizes to the target domain through complementary base pairing. Any of the targeting domains in the Table can be used with a *S. aureus* Cas9 molecule that generates a double stranded break (Cas9 nuclease) or a single-stranded break (Cas9 nickase).

**Table 7D**

| 4th Tier | | | | |
|---|---|---|---|---|
| gRNA Name | DNA Strand | Targeting Domain | Target Site Length | SEQ ID NO |
| myoC-3267 | + | UCAUUGGGACUGGCCACA | 18 | 3013 |
| myoC-3268 | + | UUCAUUGGGACUGGCCACA | 19 | 3014 |
| myoC-1671 | + | AUUCAUUGGGACUGGCCACA | 20 | 1933 |
| myoC-3269 | + | GAUUCAUUGGGACUGGCCACA | 21 | 3015 |
| myoC-3270 | + | GGAUUCAUUGGGACUGGCCACA | 22 | 3016 |
| myoC-3271 | + | UGGAUUCAUUGGGACUGGCCACA | 23 | 3017 |
| myoC-3272 | + | CUGGAUUCAUUGGGACUGGCCACA | 24 | 3018 |
| myoC-3273 | + | GGUGGAGGAGGCUCUCCA | 18 | 3019 |
| myoC-3274 | + | UGGUGGAGGAGGCUCUCCA | 19 | 3020 |
| myoC-223 | + | UUGGUGGAGGAGGCUCUCCA | 20 | 609 |
| myoC-3275 | + | AUUGGUGGAGGAGGCUCUCCA | 21 | 3021 |
| myoC-3276 | + | AAUUGGUGGAGGAGGCUCUCCA | 22 | 3022 |
| myoC-3277 | + | CAAUUGGUGGAGGAGGCUCUCCA | 23 | 3023 |
| myoC-3278 | + | UCAAUUGGUGGAGGAGGCUCUCCA | 24 | 3024 |
| myoC-3279 | + | AAGCUGCAGCAACGUGCA | 18 | 3025 |
| myoC-3280 | + | AAAGCUGCAGCAACGUGCA | 19 | 3026 |
| myoC-1666 | + | CAAAGCUGCAGCAACGUGCA | 20 | 1928 |
| myoC-3281 | + | CCAAAGCUGCAGCAACGUGCA | 21 | 3027 |
| myoC-3282 | + | CCCAAAGCUGCAGCAACGUGCA | 22 | 3028 |
| myoC-3283 | + | GCCCAAAGCUGCAGCAACGUGCA | 23 | 3029 |
| myoC-3284 | + | GGCCCAAAGCUGCAGCAACGUGCA | 24 | 3030 |
| myoC-3285 | + | UCUGGGCAGCUGGAUUCA | 18 | 3031 |
| myoC-3286 | + | CUCUGGGCAGCUGGAUUCA | 19 | 3032 |
| myoC-1673 | + | GCUCUGGGCAGCUGGAUUCA | 20 | 1934 |
| myoC-3287 | + | UGCUCUGGGCAGCUGGAUUCA | 21 | 3033 |
| myoC-3288 | + | CUGCUCUGGGCAGCUGGAUUCA | 22 | 3034 |
| myoC-3289 | + | UCUGCUCUGGGCAGCUGGAUUCA | 23 | 3035 |
| myoC-3290 | + | CUCUGCUCUGGGCAGCUGGAUUCA | 24 | 3036 |
| myoC-3291 | + | UGGUGGAGGAGGCUCUCC | 18 | 3037 |
| myoC-3292 | + | UUGGUGGAGGAGGCUCUCC | 19 | 3038 |
| myoC-222 | + | AUUGGUGGAGGAGGCUCUCC | 20 | 608 |
| myoC-3293 | + | AAUUGGUGGAGGAGGCUCUCC | 21 | 3039 |
| myoC-3294 | + | CAAUUGGUGGAGGAGGCUCUCC | 22 | 3040 |
| myoC-3295 | + | UCAAUUGGUGGAGGAGGCUCUCC | 23 | 3041 |
| myoC-3296 | + | GUCAAUUGGUGGAGGAGGCUCUCC | 24 | 3042 |
| myoC-3297 | + | AGCCCCUCCUGGGUCUCC | 18 | 3043 |
| myoC-3298 | + | CAGCCCCUCCUGGGUCUCC | 19 | 3044 |
| myoC-119 | + | GCAGCCCCUCCUGGGUCUCC | 20 | 518 |
| myoC-3299 | + | UGCAGCCCCUCCUGGGUCUCC | 21 | 3045 |
| myoC-3300 | + | CUGCAGCCCCUCCUGGGUCUCC | 22 | 3046 |
| myoC-3301 | + | UCUGCAGCCCCUCCUGGGUCUCC | 23 | 3047 |
| myoC-3302 | + | CUCUGCAGCCCCUCCUGGGUCUCC | 24 | 3048 |
| myoC-3303 | + | AUCCCACACCAGGCAGGC | 18 | 3049 |
| myoC-3304 | + | CAUCCCACACCAGGCAGGC | 19 | 3050 |
| myoC-1668 | + | ACAUCCCACACCAGGCAGGC | 20 | 1930 |
| myoC-3305 | + | CACAUCCCACACCAGGCAGGC | 21 | 3051 |
| myoC-3306 | + | CCACAUCCCACACCAGGCAGGC | 22 | 3052 |
| myoC-3307 | + | CCCACAUCCCACACCAGGCAGGC | 23 | 3053 |
| myoC-3308 | + | CCCCACAUCCCACACCAGGCAGGC | 24 | 3054 |
| myoC-3309 | + | GCUUGGUGAGGCUUCCUC | 18 | 3055 |
| myoC-3310 | + | GGCUUGGUGAGGCUUCCUC | 19 | 3056 |
| myoC-2356 | + | AGGCUUGGUGAGGCUUCCUC | 20 | 2410 |
| myoC-3311 | + | GAGGCUUGGUGAGGCUUCCUC | 21 | 3057 |
| myoC-3312 | + | AGAGGCUUGGUGAGGCUUCCUC | 22 | 3058 |
| myoC-3313 | + | CAGAGGCUUGGUGAGGCUUCCUC | 23 | 3059 |
| myoC-3314 | + | GCAGAGGCUUGGUGAGGCUUCCUC | 24 | 3060 |
| myoC-3315 | + | UCGCUUCUUCUCUUCCUC | 18 | 3061 |
| myoC-3316 | + | GUCGCUUCUUCUCUUCCUC | 19 | 3062 |
| myoC-1696 | + | AGUCGCUUCUUCUCUUCCUC | 20 | 1950 |
| myoC-3317 | + | UAGUCGCUUCUUCUCUUCCUC | 21 | 3063 |
| myoC-3318 | + | UUAGUCGCUUCUUCUCUUCCUC | 22 | 3064 |
| myoC-3319 | + | CUUAGUCGCUUCUUCUCUUCCUC | 23 | 3065 |
| myoC-3320 | + | CCUUAGUCGCUUCUUCUCUUCCUC | 24 | 3066 |
| myoC-3321 | + | UUGGUGGAGGAGGCUCUC | 18 | 3067 |
| myoC-3322 | + | AUUGGUGGAGGAGGCUCUC | 19 | 3068 |
| myoC-1682 | + | AAUUGGUGGAGGAGGCUCUC | 20 | 1941 |
| myoC-3323 | + | CAAUUGGUGGAGGAGGCUCUC | 21 | 3069 |
| myoC-3324 | + | UCAAUUGGUGGAGGAGGCUCUC | 22 | 3070 |
| myoC-3325 | + | GUCAAUUGGUGGAGGAGGCUCUC | 23 | 3071 |
| myoC-3326 | + | GGUCAAUUGGUGGAGGAGGCUCUC | 24 | 3072 |
| myoC-3327 | + | CAGCCCCUCCUGGGUCUC | 18 | 3073 |
| myoC-3328 | + | GCAGCCCCUCCUGGGUCUC | 19 | 3074 |
| myoC-1688 | + | UGCAGCCCCUCCUGGGUCUC | 20 | 1944 |
| myoC-3329 | + | CUGCAGCCCCUCCUGGGUCUC | 21 | 3075 |
| myoC-3330 | + | UCUGCAGCCCCUCCUGGGUCUC | 22 | 3076 |
| myoC-3331 | + | CUCUGCAGCCCCUCCUGGGUCUC | 23 | 3077 |
| myoC-3332 | + | CCUCUGCAGCCCCUCCUGGGUCUC | 24 | 3078 |
| myoC-3333 | + | CUCCAGAACUGACUUGUC | 18 | 3079 |
| myoC-3334 | + | CCUCCAGAACUGACUUGUC | 19 | 3080 |
| myoC-1695 | + | UCCUCCAGAACUGACUUGUC | 20 | 1949 |
| myoC-3335 | + | UUCCUCCAGAACUGACUUGUC | 21 | 3081 |
| myoC-3336 | + | CUUCCUCCAGAACUGACUUGUC | 22 | 3082 |
| myoC-3337 | + | UCUUCCUCCAGAACUGACUUGUC | 23 | 3083 |
| myoC-3338 | + | CUCUUCCUCCAGAACUGACUUGUC | 24 | 3084 |
| myoC-3339 | + | CUCUGGUCAUUGGCCUUC | 18 | 3085 |
| myoC-3340 | + | ACUCUGGUCAUUGGCCUUC | 19 | 3086 |
| myoC-1670 | + | CACUCUGGUCAUUGGCCUUC | 20 | 1932 |
| myoC-3341 | + | CCACUCUGGUCAUUGGCCUUC | 21 | 3087 |
| myoC-3342 | + | GCCACUCUGGUCAUUGGCCUUC | 22 | 3088 |
| myoC-3343 | + | GGCCACUCUGGUCAUUGGCCUUC | 23 | 3089 |
| myoC-3344 | + | CGGCCACUCUGGUCAUUGGCCUUC | 24 | 3090 |
| myoC-3345 | + | CUGCAGCAACGUGCACAG | 18 | 3091 |
| myoC-3346 | + | GCUGCAGCAACGUGCACAG | 19 | 3092 |
| myoC-1665 | + | AGCUGCAGCAACGUGCACAG | 20 | 1927 |
| myoC-3347 | + | AAGCUGCAGCAACGUGCACAG | 21 | 3093 |
| myoC-3348 | + | AAAGCUGCAGCAACGUGCACAG | 22 | 3094 |
| myoC-3349 | + | CAAAGCUGCAGCAACGUGCACAG | 23 | 3095 |
| myoC-3350 | + | CCAAAGCUGCAGCAACGUGCACAG | 24 | 3096 |
| myoC-3351 | + | GCAGGCCAGAAGCAGCAG | 18 | 3097 |
| myoC-3352 | + | GGCAGGCCAGAAGCAGCAG | 19 | 3098 |
| myoC-1667 | + | AGGCAGGCCAGAAGCAGCAG | 20 | 1929 |
| myoC-3353 | + | CAGGCAGGCCAGAAGCAGCAG | 21 | 3099 |
| myoC-3354 | + | CCAGGCAGGCCAGAAGCAGCAG | 22 | 3100 |
| myoC-3355 | + | ACCAGGCAGGCCAGAAGCAGCAG | 23 | 3101 |
| myoC-3356 | + | CACCAGGCAGGCCAGAAGCAGCAG | 24 | 3102 |
| myoC-3357 | + | GUCAUUGGCCUUCCUGAG | 18 | 3103 |
| myoC-3358 | + | GGUCAUUGGCCUUCCUGAG | 19 | 3104 |
| myoC-1669 | + | UGGUCAUUGGCCUUCCUGAG | 20 | 1931 |
| myoC-3359 | + | CUGGUCAUUGGCCUUCCUGAG | 21 | 3105 |
| myoC-3360 | + | UCUGGUCAUUGGCCUUCCUGAG | 22 | 3106 |
| myoC-3361 | + | CUCUGGUCAUUGGCCUUCCUGAG | 23 | 3107 |
| myoC-3362 | + | ACUCUGGUCAUUGGCCUUCCUGAG | 24 | 3108 |
| myoC-3363 | + | GCUCUCCAGGGAGCUGAG | 18 | 3109 |
| myoC-3364 | + | GGCUCUCCAGGGAGCUGAG | 19 | 3110 |
| myoC-1678 | + | AGGCUCUCCAGGGAGCUGAG | 20 | 1939 |
| myoC-3365 | + | GAGGCUCUCCAGGGAGCUGAG | 21 | 3111 |
| myoC-3366 | + | GGAGGCUCUCCAGGGAGCUGAG | 22 | 3112 |
| myoC-3367 | + | AGGAGGCUCUCCAGGGAGCUGAG | 23 | 3113 |
| myoC-3368 | + | GAGGAGGCUCUCCAGGGAGCUGAG | 24 | 3114 |
| myoC-3369 | + | CAGAACUGACUUGUCUCG | 18 | 3115 |
| myoC-3370 | + | CCAGAACUGACUUGUCUCG | 19 | 3116 |
| myoC-1693 | + | UCCAGAACUGACUUGUCUCG | 20 | 1948 |
| myoC-3371 | + | CUCCAGAACUGACUUGUCUCG | 21 | 3117 |
| myoC-3372 | + | CCUCCAGAACUGACUUGUCUCG | 22 | 3118 |
| myoC-3373 | + | UCCUCCAGAACUGACUUGUCUCG | 23 | 3119 |
| myoC-3374 | + | UUCCUCCAGAACUGACUUGUCUCG | 24 | 3120 |
| myoC-3375 | + | AGAACUGACUUGUCUCGG | 18 | 3121 |
| myoC-3376 | + | CAGAACUGACUUGUCUCGG | 19 | 3122 |
| myoC-209 | + | CCAGAACUGACUUGUCUCGG | 20 | 595 |
| myoC-3377 | + | UCCAGAACUGACUUGUCUCGG | 21 | 3123 |
| myoC-3378 | + | CUCCAGAACUGACUUGUCUCGG | 22 | 3124 |
| myoC-3379 | + | CCUCCAGAACUGACUUGUCUCGG | 23 | 3125 |
| myoC-3380 | + | UCCUCCAGAACUGACUUGUCUCGG | 24 | 3126 |
| myoC-3381 | + | UCCAAGGUCAAUUGGUGG | 18 | 3127 |
| myoC-3382 | + | GUCCAAGGUCAAUUGGUGG | 19 | 3128 |
| myoC-121 | + | GGUCCAAGGUCAAUUGGUGG | 20 | 520 |
| myoC-3383 | + | UGGUCCAAGGUCAAUUGGUGG | 21 | 3129 |
| myoC-3384 | + | CUGGUCCAAGGUCAAUUGGUGG | 22 | 3130 |
| myoC-3385 | + | CCUGGUCCAAGGUCAAUUGGUGG | 23 | 3131 |
| myoC-3386 | + | GCCUGGUCCAAGGUCAAUUGGUGG | 24 | 3132 |
| myoC-3387 | + | UGGUCCAAGGUCAAUUGG | 18 | 3133 |
| myoC-3388 | + | CUGGUCCAAGGUCAAUUGG | 19 | 3134 |
| myoC-220 | + | CCUGGUCCAAGGUCAAUUGG | 20 | 606 |
| myoC-3389 | + | GCCUGGUCCAAGGUCAAUUGG | 21 | 3135 |
| myoC-3390 | + | AGCCUGGUCCAAGGUCAAUUGG | 22 | 3136 |
| myoC-3391 | + | CAGCCUGGUCCAAGGUCAAUUGG | 23 | 3137 |
| myoC-3392 | + | GCAGCCUGGUCCAAGGUCAAUUGG | 24 | 3138 |
| myoC-3393 | + | GUCCAAGGUCAAUUGGUG | 18 | 3139 |
| myoC-3394 | + | GGUCCAAGGUCAAUUGGUG | 19 | 3140 |
| myoC-1684 | + | UGGUCCAAGGUCAAUUGGUG | 20 | 1942 |
| myoC-3395 | + | CUGGUCCAAGGUCAAUUGGUG | 21 | 3141 |
| myoC-3396 | + | CCUGGUCCAAGGUCAAUUGGUG | 22 | 3142 |
| myoC-3397 | + | GCCUGGUCCAAGGUCAAUUGGUG | 23 | 3143 |
| myoC-3398 | + | AGCCUGGUCCAAGGUCAAUUGGUG | 24 | 3144 |
| myoC-3399 | + | CUGGUCCAAGGUCAAUUG | 18 | 3145 |
| myoC-3400 | + | CCUGGUCCAAGGUCAAUUG | 19 | 3146 |
| myoC-1686 | + | GCCUGGUCCAAGGUCAAUUG | 20 | 1943 |
| myoC-3401 | + | AGCCUGGUCCAAGGUCAAUUG | 21 | 3147 |
| myoC-3402 | + | CAGCCUGGUCCAAGGUCAAUUG | 22 | 3148 |
| myoC-3403 | + | GCAGCCUGGUCCAAGGUCAAUUG | 23 | 3149 |
| myoC-3404 | + | GGCAGCCUGGUCCAAGGUCAAUUG | 24 | 3150 |
| myoC-3405 | + | CACAGAAGAACCUCAUUG | 18 | 3151 |
| myoC-3406 | + | GCACAGAAGAACCUCAU UG | 19 | 3152 |
| myoC-1664 | + | UGCACAGAAGAACCUCAUUG | 20 | 1926 |
| myoC-3407 | + | GUGCACAGAAGAACCUCAUUG | 21 | 3153 |
| myoC-3408 | + | CGUGCACAGAAGAACCUCAUUG | 22 | 3154 |
| myoC-3409 | + | ACGUGCACAGAAGAACCUCAUUG | 23 | 3155 |
| myoC-3410 | + | AACGUGCACAGAAGAACCUCAUUG | 24 | 3156 |
| myoC-3411 | + | CCUCAUUGCAGAGGCUUG | 18 | 3157 |
| myoC-3412 | + | ACCUCAUUGCAGAGGCUUG | 19 | 3158 |
| myoC-1663 | + | AACCUCAUUGCAGAGGCUUG | 20 | 1925 |
| myoC-3413 | + | GAACCUCAUUGCAGAGGCUUG | 21 | 3159 |
| myoC-3414 | + | AGAACCUCAUUGCAGAGGCUUG | 22 | 3160 |
| myoC-3415 | + | AAGAACCUCAUUGCAGAGGCUUG | 23 | 3161 |
| myoC-3416 | + | GAAGAACCUCAUUGCAGAGGCUUG | 24 | 3162 |
| myoC-3417 | + | CUGGGCAGCUGGAUUCAU | 18 | 3163 |
| myoC-3418 | + | UCUGGGCAGCUGGAUUCAU | 19 | 3164 |
| myoC-231 | + | CUCUGGGCAGCUGGAUUCAU | 20 | 617 |
| myoC-3419 | + | GCUCUGGGCAGCUGGAUUCAU | 21 | 3165 |
| myoC-3420 | + | UGCUCUGGGCAGCUGGAUUCAU | 22 | 3166 |
| myoC-3421 | + | CUGCUCUGGGCAGCUGGAUUCAU | 23 | 3167 |
| myoC-3422 | + | UCUGCUCUGGGCAGCUGGAUUCAU | 24 | 3168 |
| myoC-3423 | + | GGCUUGGUGAGGCUUCCU | 18 | 3169 |
| myoC-3424 | + | AGGCUUGGUGAGGCUUCCU | 19 | 3170 |
| myoC-2357 | + | GAGGCUUGGUGAGGCUUCCU | 20 | 2411 |
| myoC-3425 | + | AGAGGCUUGGUGAGGCUUCCU | 21 | 3171 |
| myoC-3426 | + | CAGAGGCUUGGUGAGGCUUCCU | 22 | 3172 |
| myoC-3427 | + | GCAGAGGCUUGGUGAGGCUUCCU | 23 | 3173 |
| myoC-3428 | + | UGCAGAGGCUUGGUGAGGCUUCCU | 24 | 3174 |
| myoC-3429 | + | ACAUGGCCUGGCUCUGCU | 18 | 3175 |
| myoC-3430 | + | GACAUGGCCUGGCUCUGCU | 19 | 3176 |
| myoC-1675 | + | UGACAUGGCCUGGCUCUGCU | 20 | 1936 |
| myoC-3431 | + | CUGACAUGGCCUGGCUCUGCU | 21 | 3177 |
| myoC-3432 | + | ACUGACAUGGCCUGGCUCUGCU | 22 | 3178 |
| myoC-3433 | + | GACUGACAUGGCCUGGCUCUGCU | 23 | 3179 |
| myoC-3434 | + | UGACUGACAUGGCCUGGCUCUGCU | 24 | 3180 |
| myoC-3435 | + | UCCAGAACUGACUUGUCU | 18 | 3181 |
| myoC-3436 | + | CUCCAGAACUGACUUGUCU | 19 | 3182 |
| myoC-208 | + | CCUCCAGAACUGACUUGUCU | 20 | 594 |
| myoC-3437 | + | UCCUCCAGAACUGACUUGUCU | 21 | 3183 |
| myoC-3438 | + | UUCCUCCAGAACUGACUUGUCU | 22 | 3184 |
| myoC-3439 | + | CUUCCUCCAGAACUGACUUGUCU | 23 | 3185 |
| myoC-3440 | + | UCUUCCUCCAGAACUGACUUGUCU | 24 | 3186 |
| myoC-3441 | - | AGCGACUAAGGCAAGAAA | 18 | 3187 |
| myoC-3442 | - | AAGCGACUAAGGCAAGAAA | 19 | 3188 |
| myoC-1647 | - | GAAGCGACUAAGGCAAGAAA | 20 | 1913 |
| myoC-3443 | - | AGAAGCGACUAAGGCAAGAAA | 21 | 3189 |
| myoC-3444 | - | AAGAAGCGACUAAGGCAAGAAA | 22 | 3190 |
| myoC-3445 | - | GAAGAAGCGACUAAGGCAAGAAA | 23 | 3191 |
| myoC-3446 | - | AGAAGAAGCGACUAAGGCAAGAAA | 24 | 3192 |
| myoC-3447 | - | AAGUCAGUUCUGGAGGAA | 18 | 3193 |
| myoC-3448 | - | CAAGUCAGUUCUGGAGGAA | 19 | 3194 |
| myoC-1644 | - | ACAAGUCAGUUCUGGAGGAA | 20 | 1910 |
| myoC-3449 | - | GACAAGUCAGUUCUGGAGGAA | 21 | 3195 |
| myoC-3450 | - | AGACAAGUCAGUUCUGGAGGAA | 22 | 3196 |
| myoC-3451 | - | GAGACAAGUCAGUUCUGGAGGAA | 23 | 3197 |
| myoC-3452 | - | CGAGACAAGUCAGUUCUGGAGGAA | 24 | 3198 |
| myoC-3453 | - | AGUCAUCCAUAACUUACA | 18 | 3199 |
| myoC-3454 | - | CAGUCAUCCAUAACUUACA | 19 | 3200 |
| myoC-1608 | - | UCAGUCAUCCAUAACUUACA | 20 | 1888 |
| myoC-3455 | - | GUCAGUCAUCCAUAACUUACA | 21 | 3201 |
| myoC-3456 | - | UGUCAGUCAUCCAUAACUUACA | 22 | 3202 |
| myoC-3457 | - | AUGUCAGUCAUCCAUAACUUACA | 23 | 3203 |
| myoC-3458 | - | CAUGUCAGUCAUCCAUAACUUACA | 24 | 3204 |
| myoC-3459 | - | GACCCAGGAGGGGCUGCA | 18 | 3205 |
| myoC-3460 | - | AGACCCAGGAGGGGCUGCA | 19 | 3206 |
| myoC-1622 | - | GAGACCCAGGAGGGGCUGCA | 20 | 1897 |
| myoC-3461 | - | GGAGACCCAGGAGGGGCUGCA | 21 | 3207 |
| myoC-3462 | - | AGGAGACCCAGGAGGGGCUGCA | 22 | 3208 |
| myoC-3463 | - | CAGGAGACCCAGGAGGGGCUGCA | 23 | 3209 |
| myoC-3464 | - | CCAGGAGACCCAGGAGGGGCUGCA | 24 | 3210 |
| myoC-3465 | - | CCUCACCAAGCCUCUGCA | 18 | 3211 |
| myoC-3466 | - | GCCUCACCAAGCCUCUGCA | 19 | 3212 |
| myoC-1592 | - | AGCCUCACCAAGCCUCUGCA | 20 | 1876 |
| myoC-3467 | - | AAGCCUCACCAAGCCUCUGCA | 21 | 3213 |
| myoC-3468 | - | GAAGCCUCACCAAGCCUCUGCA | 22 | 3214 |
| myoC-3469 | - | GGAAGCCUCACCAAGCCUCUGCA | 23 | 3215 |
| myoC-3470 | - | AGGAAGCCUCACCAAGCCUCUGCA | 24 | 3216 |
| myoC-3471 | - | CCCAGGAGGGGCUGCAGA | 18 | 3217 |
| myoC-3472 | - | ACCCAGGAGGGGCUGCAGA | 19 | 3218 |
| myoC-99 | - | GACCCAGGAGGGGCUGCAGA | 20 | 504 |
| myoC-3473 | - | AGACCCAGGAGGGGCUGCAGA | 21 | 3219 |
| myoC-3474 | - | GAGACCCAGGAGGGGCUGCAGA | 22 | 3220 |
| myoC-3475 | - | GGAGACCCAGGAGGGGCUGCAGA | 23 | 3221 |
| myoC-3476 | - | AGGAGACCCAGGAGGGGCUGCAGA | 24 | 3222 |
| myoC-3477 | - | GGGCACCCUGAGGCGGGA | 18 | 3223 |
| myoC-3478 | - | UGGGCACCCUGAGGCGGGA | 19 | 3224 |
| myoC-1630 | - | CUGGGCACCCUGAGGCGGGA | 20 | 1901 |
| myoC-3479 | - | GCUGGGCACCCUGAGGCGGGA | 21 | 3225 |
| myoC-3480 | - | AGCUGGGCACCCUGAGGCGGGA | 22 | 3226 |
| myoC-3481 | - | GAGCUGGGCACCCUGAGGCGGGA | 23 | 3227 |
| myoC-3482 | - | GGAGCUGGGCACCCUGAGGCGGGA | 24 | 3228 |
| myoC-3483 | - | UCAGUCAUCCAUAACUUA | 18 | 3229 |
| myoC-3484 | - | GUCAGUCAUCCAUAACUUA | 19 | 3230 |
| myoC-1607 | - | UGUCAGUCAUCCAUAACUUA | 20 | 1887 |
| myoC-3485 | - | AUGUCAGUCAUCCAUAACUUA | 21 | 3231 |
| myoC-3486 | - | CAUGUCAGUCAUCCAUAACUUA | 22 | 3232 |
| myoC-3487 | - | CCAUGUCAGUCAUCCAUAACUUA | 23 | 3233 |
| myoC-3488 | - | GCCAUGUCAGUCAUCCAUAACUUA | 24 | 3234 |
| myoC-3489 | - | CCAGCUGGAAACCCAAAC | 18 | 3235 |
| myoC-3490 | - | ACCAGCUGGAAACCCAAAC | 19 | 3236 |
| myoC-1634 | - | GACCAGCUGGAAACCCAAAC | 20 | 1903 |
| myoC-3491 | - | GGACCAGCUGGAAACCCAAAC | 21 | 3237 |
| myoC-3492 | - | GGGACCAGCUGGAAACCCAAAC | 22 | 3238 |
| myoC-3493 | - | CGGGACCAGCUGGAAACCCAAAC | 23 | 3239 |
| myoC-3494 | - | GCGGGACCAGCUGGAAACCCAAAC | 24 | 3240 |
| myoC-3495 | - | AGCACCCAACGCUUAGAC | 18 | 3241 |
| myoC-3496 | - | CAGCACCCAACGCUUAGAC | 19 | 3242 |
| myoC-1609 | - | GCAGCACCCAACGCUUAGAC | 20 | 1889 |
| myoC-3497 | - | AGCAGCACCCAACGCUUAGAC | 21 | 3243 |
| myoC-3498 | - | CAGCAGCACCCAACGCUUAGAC | 22 | 3244 |
| myoC-3499 | - | ACAGCAGCACCCAACGCUUAGAC | 23 | 3245 |
| myoC-3500 | - | GACAGCAGCACCCAACGCUUAGAC | 24 | 3246 |
| myoC-3501 | - | CAGAGGGAGCUGGGCACC | 18 | 3247 |
| myoC-3502 | - | GCAGAGGGAGCUGGGCACC | 19 | 3248 |
| myoC-1626 | - | UGCAGAGGGAGCUGGGCACC | 20 | 1899 |
| myoC-3503 | - | CUGCAGAGGGAGCUGGGCACC | 21 | 3249 |
| myoC-3504 | - | GCUGCAGAGGGAGCUGGGCACC | 22 | 3250 |
| myoC-3505 | - | GGCUGCAGAGGGAGCUGGGCACC | 23 | 3251 |
| myoC-3506 | - | GGGCUGCAGAGGGAGCUGGGCACC | 24 | 3252 |
| myoC-3507 | - | GCCAGGCCCCAGGAGACC | 18 | 3253 |
| myoC-3508 | - | UGCCAGGCCCCAGGAGACC | 19 | 3254 |
| myoC-1617 | - | CUGCCAGGCCCCAGGAGACC | 20 | 1894 |
| myoC-3509 | - | GCUGCCAGGCCCCAGGAGACC | 21 | 3255 |
| myoC-3510 | - | GGCUGCCAGGCCCCAGGAGACC | 22 | 3256 |
| myoC-3511 | - | AGGCUGCCAGGCCCCAGGAGACC | 23 | 3257 |
| myoC-3512 | - | CAGGCUGCCAGGCCCCAGGAGACC | 24 | 3258 |
| myoC-3513 | - | GCACCCAACGCUUAGACC | 18 | 3259 |
| myoC-3514 | - | AGCACCCAACGCUUAGACC | 19 | 3260 |
| myoC-179 | - | CAGCACCCAACGCUUAGACC | 20 | 565 |
| myoC-3515 | - | GCAGCACCCAACGCUUAGACC | 21 | 3261 |
| myoC-3516 | - | AGCAGCACCCAACGCUUAGACC | 22 | 3262 |
| myoC-3517 | - | CAGCAGCACCCAACGCUUAGACC | 23 | 3263 |
| myoC-3518 | - | ACAGCAGCACCCAACGCUUAGACC | 24 | 3264 |
| myoC-3519 | - | CUCCUCCACCAAUUGACC | 18 | 3265 |
| myoC-3520 | - | CCUCCUCCACCAAUUGACC | 19 | 3266 |
| myoC-1614 | - | GCCUCCUCCACCAAUUGACC | 20 | 1892 |
| myoC-3521 | - | AGCCUCCUCCACCAAUUGACC | 21 | 3267 |
| myoC-3522 | - | GAGCCUCCUCCACCAAUUGACC | 22 | 3268 |
| myoC-3523 | - | AGAGCCUCCUCCACCAAUUGACC | 23 | 3269 |
| myoC-3524 | - | GAGAGCCUCCUCCACCAAUUGACC | 24 | 3270 |
| myoC-3525 | - | CCAGGCCCCAGGAGACCC | 18 | 3271 |
| myoC-3526 | - | GCCAGGCCCCAGGAGACCC | 19 | 3272 |
| myoC-185 | - | UGCCAGGCCCCAGGAGACCC | 20 | 571 |
| myoC-3527 | - | CUGCCAGGCCCCAGGAGACCC | 21 | 3273 |
| myoC-3528 | - | GCUGCCAGGCCCCAGGAGACCC | 22 | 3274 |
| myoC-3529 | - | GGCUGCCAGGCCCCAGGAGACCC | 23 | 3275 |
| myoC-3530 | - | AGGCUGCCAGGCCCCAGGAGACCC | 24 | 3276 |
| myoC-3531 | - | ACCAGGCUGCCAGGCCCC | 18 | 3277 |
| myoC-3532 | - | GACCAGGCUGCCAGGCCCC | 19 | 3278 |
| myoC-97 | - | GGACCAGGCUGCCAGGCCCC | 20 | 502 |
| myoC-3533 | - | UGGACCAGGCUGCCAGGCCCC | 21 | 3279 |
| myoC-3534 | - | UUGGACCAGGCUGCCAGGCCCC | 22 | 3280 |
| myoC-3535 | - | CUUGGACCAGGCUGCCAGGCCCC | 23 | 3281 |
| myoC-3536 | - | CCUUGGACCAGGCUGCCAGGCCCC | 24 | 3282 |
| myoC-3537 | - | GACCAGGCUGCCAGGCCC | 18 | 3283 |
| myoC-3538 | - | GGACCAGGCUGCCAGGCCC | 19 | 3284 |
| myoC-1615 | - | UGGACCAGGCUGCCAGGCCC | 20 | 1893 |
| myoC-3539 | - | UUGGACCAGGCUGCCAGGCCC | 21 | 3285 |
| myoC-3540 | - | CUUGGACCAGGCUGCCAGGCCC | 22 | 3286 |
| myoC-3541 | - | CCUUGGACCAGGCUGCCAGGCCC | 23 | 3287 |
| myoC-3542 | - | ACCUUGGACCAGGCUGCCAGGCCC | 24 | 3288 |
| myoC-3543 | - | AAGCUCGACUCAGCUCCC | 18 | 3289 |
| myoC-3544 | - | AAAGCUCGACUCAGCUCCC | 19 | 3290 |
| myoC-181 | - | CAAAGCUCGACUCAGCUCCC | 20 | 567 |
| myoC-3545 | - | CCAAAGCUCGACUCAGCUCCC | 21 | 3291 |
| myoC-3546 | - | ACCAAAGCUCGACUCAGCUCCC | 22 | 3292 |
| myoC-3547 | - | CACCAAAGCUCGACUCAGCUCCC | 23 | 3293 |
| myoC-3548 | - | CCACCAAAGCUCGACUCAGCUCCC | 24 | 3294 |
| myoC-3549 | - | GGUUGGAAAGCAGCAGCC | 18 | 3295 |
| myoC-3550 | - | AGGUUGGAAAGCAGCAGCC | 19 | 3296 |
| myoC-107 | - | GAGGUUGGAAAGCAGCAGCC | 20 | 511 |
| myoC-3551 | - | GGAGGUUGGAAAGCAGCAGCC | 21 | 3297 |
| myoC-3552 | - | AGGAGGUUGGAAAGCAGCAGCC | 22 | 3298 |
| myoC-3553 | - | CAGGAGGUUGGAAAGCAGCAGCC | 23 | 3299 |
| myoC-3554 | - | CCAGGAGGUUGGAAAGCAGCAGCC | 24 | 3300 |
| myoC-3555 | - | GAAAAUGAGAAUCUGGCC | 18 | 3301 |
| myoC-3556 | - | AGAAAAUGAGAAUCUGGCC | 19 | 3302 |
| myoC-195 | - | AAGAAAAUGAGAAUCUGGCC | 20 | 581 |
| myoC-3557 | - | CAAGAAAAUGAGAAUCUGGCC | 21 | 3303 |
| myoC-3558 | - | GCAAGAAAAUGAGAAUCUGGCC | 22 | 3304 |
| myoC-3559 | - | GGCAAGAAAAUGAGAAUCUGGCC | 23 | 3305 |
| myoC-3560 | - | AGGCAAGAAAAUGAGAAUCUGGCC | 24 | 3306 |
| myoC-3561 | - | CCAAUGAAUCCAGCUGCC | 18 | 3307 |
| myoC-3562 | - | CCCAAUGAAUCCAGCUGCC | 19 | 3308 |
| myoC-1605 | - | UCCCAAUGAAUCCAGCUGCC | 20 | 1885 |
| myoC-3563 | - | GUCCCAAUGAAUCCAGCUGCC | 21 | 3309 |
| myoC-3564 | - | AGUCCCAAUGAAUCCAGCUGCC | 22 | 3310 |
| myoC-3565 | - | CAGUCCCAAUGAAUCCAGCUGCC | 23 | 3311 |
| myoC-3566 | - | CCAGUCCCAAUGAAUCCAGCUGCC | 24 | 3312 |
| myoC-3567 | - | AAAGCUCGACUCAGCUCC | 18 | 3313 |
| myoC-3568 | - | CAAAGCUCGACUCAGCUCC | 19 | 3314 |
| myoC-1611 | - | CCAAAGCUCGACUCAGCUCC | 20 | 1890 |
| myoC-3569 | - | ACCAAAGCUCGACUCAGCUCC | 21 | 3315 |
| myoC-3570 | - | CACCAAAGCUCGACUCAGCUCC | 22 | 3316 |
| myoC-3571 | - | CCACCAAAGCUCGACUCAGCUCC | 23 | 3317 |
| myoC-3572 | - | GCCACCAAAGCUCGACUCAGCUCC | 24 | 3318 |
| myoC-3573 | - | GGCGGGAGCGGGACCAGC | 18 | 3319 |
| myoC-3574 | - | AGGCGGGAGCGGGACCAGC | 19 | 3320 |
| myoC-105 | - | GAGGCGGGAGCGGGACCAGC | 20 | 510 |
| myoC-3575 | - | UGAGGCGGGAGCGGGACCAGC | 21 | 3321 |
| myoC-3576 | - | CUGAGGCGGGAGCGGGACCAGC | 22 | 3322 |
| myoC-3577 | - | CCUGAGGCGGGAGCGGGACCAGC | 23 | 3323 |
| myoC-3578 | - | CCCUGAGGCGGGAGCGGGACCAGC | 24 | 3324 |
| myoC-3579 | - | AGGUUGGAAAGCAGCAGC | 18 | 3325 |
| myoC-3580 | - | GAGGUUGGAAAGCAGCAGC | 19 | 3326 |
| myoC-1653 | - | GGAGGUUGGAAAGCAGCAGC | 20 | 1917 |
| myoC-3581 | - | AGGAGGUUGGAAAGCAGCAGC | 21 | 3327 |
| myoC-3582 | - | CAGGAGGUUGGAAAGCAGCAGC | 22 | 3328 |
| myoC-3583 | - | CCAGGAGGUUGGAAAGCAGCAGC | 23 | 3329 |
| myoC-3584 | - | GCCAGGAGGUUGGAAAGCAGCAGC | 24 | 3330 |
| myoC-3585 | - | AGAAGAAGCGACUAAGGC | 18 | 3331 |
| myoC-3586 | - | GAGAAGAAGCGACUAAGGC | 19 | 3332 |
| myoC-1646 | - | AGAGAAGAAGCGACUAAGGC | 20 | 1912 |
| myoC-3587 | - | AAGAGAAGAAGCGACUAAGGC | 21 | 3333 |
| myoC-3588 | - | GAAGAGAAGAAGCGACUAAGGC | 22 | 3334 |
| myoC-3589 | - | GGAAGAGAAGAAGCGACUAAGGC | 23 | 3335 |
| myoC-3590 | - | AGGAAGAGAAGAAGCGACUAAGGC | 24 | 3336 |
| myoC-3591 | - | AGCUGGGCACCCUGAGGC | 18 | 3337 |
| myoC-3592 | - | GAGCUGGGCACCCUGAGGC | 19 | 3338 |
| myoC-103 | - | GGAGCUGGGCACCCUGAGGC | 20 | 508 |
| myoC-3593 | - | GGGAGCUGGGCACCCUGAGGC | 21 | 3339 |
| myoC-3594 | - | AGGGAGCUGGGCACCCUGAGGC | 22 | 3340 |
| myoC-3595 | - | GAGGGAGCUGGGCACCCUGAGGC | 23 | 3341 |
| myoC-3596 | - | AGAGGGAGCUGGGCACCCUGAGGC | 24 | 3342 |
| myoC-3597 | - | GGUGUGGGAUGUGGGGGC | 18 | 3343 |
| myoC-3598 | - | UGGUGUGGGAUGUGGGGGC | 19 | 3344 |
| myoC-1600 | - | CUGGUGUGGGAUGUGGGGGC | 20 | 1881 |
| myoC-3599 | - | CCUGGUGUGGGAUGUGGGGGC | 21 | 3345 |
| myoC-3600 | - | GCCUGGUGUGGGAUGUGGGGGC | 22 | 3346 |
| myoC-3601 | - | UGCCUGGUGUGGGAUGUGGGGGC | 23 | 3347 |
| myoC-3602 | - | CUGCCUGGUGUGGGAUGUGGGGGC | 24 | 3348 |
| myoC-3603 | - | GUUGCUGCAGCUUUGGGC | 18 | 3349 |
| myoC-3604 | - | CGUUGCUGCAGCUUUGGGC | 19 | 3350 |
| myoC-1594 | - | ACGUUGCUGCAGCUUUGGGC | 20 | 1878 |
| myoC-3605 | - | CACGUUGCUGCAGCUUUGGGC | 21 | 3351 |
| myoC-3606 | - | GCACGUUGCUGCAGCUUUGGGC | 22 | 3352 |
| myoC-3607 | - | UGCACGUUGCUGCAGCUUUGGGC | 23 | 3353 |
| myoC-3608 | - | GUGCACGUUGCUGCAGCUUUGGGC | 24 | 3354 |
| myoC-3609 | - | AGAAAAUGAGAAUCUGGC | 18 | 3355 |
| myoC-3610 | - | AAGAAAAUGAGAAUCUGGC | 19 | 3356 |
| myoC-1649 | - | CAAGAAAAUGAGAAUCUGGC | 20 | 1915 |
| myoC-3611 | - | GCAAGAAAAUGAGAAUCUGGC | 21 | 3357 |
| myoC-3612 | - | GGCAAGAAAAUGAGAAUCUGGC | 22 | 3358 |
| myoC-3613 | - | AGGCAAGAAAAUGAGAAUCUGGC | 23 | 3359 |
| myoC-3614 | - | AAGGCAAGAAAAUGAGAAUCUGGC | 24 | 3360 |
| myoC-3615 | - | GCCAGGACAGCUCAGCUC | 18 | 3361 |
| myoC-3616 | - | GGCCAGGACAGCUCAGCUC | 19 | 3362 |
| myoC-96 | - | GGGCCAGGACAGCUCAGCUC | 20 | 501 |
| myoC-3617 | - | GGGGCCAGGACAGCUCAGCUC | 21 | 3363 |
| myoC-3618 | - | GGGGGCCAGGACAGCUCAGCUC | 22 | 3364 |
| myoC-3619 | - | UGGGGGCCAGGACAGCUCAGCUC | 23 | 3365 |
| myoC-3620 | - | GUGGGGGCCAGGACAGCUCAGCUC | 24 | 3366 |
| myoC-3621 | - | UCCGAGACAAGUCAGUUC | 18 | 3367 |
| myoC-3622 | - | CUCCGAGACAAGUCAGUUC | 19 | 3368 |
| myoC-191 | - | CCUCCGAGACAAGUCAGUUC | 20 | 577 |
| myoC-3623 | - | UCCUCCGAGACAAGUCAGUUC | 21 | 3369 |
| myoC-3624 | - | CUCCUCCGAGACAAGUCAGUUC | 22 | 3370 |
| myoC-3625 | - | CCUCCUCCGAGACAAGUCAGUUC | 23 | 3371 |
| myoC-3626 | - | ACCUCCUCCGAGACAAGUCAGUUC | 24 | 3372 |
| myoC-3627 | - | AGGCGGGAGCGGGACCAG | 18 | 3373 |
| myoC-3628 | - | GAGGCGGGAGCGGGACCAG | 19 | 3374 |
| myoC-1632 | - | UGAGGCGGGAGCGGGACCAG | 20 | 1902 |
| myoC-3629 | - | CUGAGGCGGGAGCGGGACCAG | 21 | 3375 |
| myoC-3630 | - | CCUGAGGCGGGAGCGGGACCAG | 22 | 3376 |
| myoC-3631 | - | CCCUGAGGCGGGAGCGGGACCAG | 23 | 3377 |
| myoC-3632 | - | ACCCUGAGGCGGGAGCGGGACCAG | 24 | 3378 |
| myoC-3633 | - | AGGCCCCAGGAGACCCAG | 18 | 3379 |
| myoC-3634 | - | CAGGCCCCAGGAGACCCAG | 19 | 3380 |
| myoC-1619 | - | CCAGGCCCCAGGAGACCCAG | 20 | 1895 |
| myoC-3635 | - | GCCAGGCCCCAGGAGACCCAG | 21 | 3381 |
| myoC-3636 | - | UGCCAGGCCCCAGGAGACCCAG | 22 | 3382 |
| myoC-3637 | - | CUGCCAGGCCCCAGGAGACCCAG | 23 | 3383 |
| myoC-3638 | - | GCUGCCAGGCCCCAGGAGACCCAG | 24 | 3384 |
| myoC-3639 | - | ACCCAGGAGGGGCUGCAG | 18 | 3385 |
| myoC-3640 | - | GACCCAGGAGGGGCUGCAG | 19 | 3386 |
| myoC-188 | - | AGACCCAGGAGGGGCUGCAG | 20 | 574 |
| myoC-3641 | - | GAGACCCAGGAGGGGCUGCAG | 21 | 3387 |
| myoC-3642 | - | GGAGACCCAGGAGGGGCUGCAG | 22 | 3388 |
| myoC-3643 | - | AGGAGACCCAGGAGGGGCUGCAG | 23 | 3389 |
| myoC-3644 | - | CAGGAGACCCAGGAGGGGCUGCAG | 24 | 3390 |
| myoC-3645 | - | UCAGUUCUGGAGGAAGAG | 18 | 3391 |
| myoC-3646 | - | GUCAGUUCUGGAGGAAGAG | 19 | 3392 |
| myoC-1645 | - | AGUCAGUUCUGGAGGAAGAG | 20 | 1911 |
| myoC-3647 | - | AAGUCAGUUCUGGAGGAAGAG | 21 | 3393 |
| myoC-3648 | - | CAAGUCAGUUCUGGAGGAAGAG | 22 | 3394 |
| myoC-3649 | - | ACAAGUCAGUUCUGGAGGAAGAG | 23 | 3395 |
| myoC-3650 | - | GACAAGUCAGUUCUGGAGGAAGAG | 24 | 3396 |
| myoC-3651 | - | GAAUCCAGCUGCCCAGAG | 18 | 3397 |
| myoC-3652 | - | UGAAUCCAGCUGCCCAGAG | 19 | 3398 |
| myoC-1606 | - | AUGAAUCCAGCUGCCCAGAG | 20 | 1886 |
| myoC-3653 | - | AAUGAAUCCAGCUGCCCAGAG | 21 | 3399 |
| myoC-3654 | - | CAAUGAAUCCAGCUGCCCAGAG | 22 | 3400 |
| myoC-3655 | - | CCAAUGAAUCCAGCUGCCCAGAG | 23 | 3401 |
| myoC-3656 | - | CCCAAUGAAUCCAGCUGCCCAGAG | 24 | 3402 |
| myoC-3657 | - | GAAACCCAAACCAGAGAG | 18 | 3403 |
| myoC-3658 | - | GGAAACCCAAACCAGAGAG | 19 | 3404 |
| myoC-1636 | - | UGGAAACCCAAACCAGAGAG | 20 | 1905 |
| myoC-3659 | - | CUGGAAACCCAAACCAGAGAG | 21 | 3405 |
| myoC-3660 | - | GCUGGAAACCCAAACCAGAGAG | 22 | 3406 |
| myoC-3661 | - | AGCUGGAAACCCAAACCAGAGAG | 23 | 3407 |
| myoC-3662 | - | CAGCUGGAAACCCAAACCAGAGAG | 24 | 3408 |
| myoC-3663 | - | GAGGGGCUGCAGAGGGAG | 18 | 3409 |
| myoC-3664 | - | GGAGGGGCUGCAGAGGGAG | 19 | 3410 |
| myoC-1625 | - | AGGAGGGGCUGCAGAGGGAG | 20 | 1898 |
| myoC-3665 | - | CAGGAGGGGCUGCAGAGGGAG | 21 | 3411 |
| myoC-3666 | - | CCAGGAGGGGCUGCAGAGGGAG | 22 | 3412 |
| myoC-3667 | - | CCCAGGAGGGGCUGCAGAGGGAG | 23 | 3413 |
| myoC-3668 | - | ACCCAGGAGGGGCUGCAGAGGGAG | 24 | 3414 |
| myoC-3669 | - | GGCACCCUGAGGCGGGAG | 18 | 3415 |
| myoC-3670 | - | GGGCACCCUGAGGCGGGAG | 19 | 3416 |
| myoC-190 | - | UGGGCACCCUGAGGCGGGAG | 20 | 576 |
| myoC-3671 | - | CUGGGCACCCUGAGGCGGGAG | 21 | 3417 |
| myoC-3672 | - | GCUGGGCACCCUGAGGCGGGAG | 22 | 3418 |
| myoC-3673 | - | AGCUGGGCACCCUGAGGCGGGAG | 23 | 3419 |
| myoC-3674 | - | GAGCUGGGCACCCUGAGGCGGGAG | 24 | 3420 |
| myoC-3675 | - | GGAGCUGGGCACCCUGAG | 18 | 3421 |
| myoC-3676 | - | GGGAGCUGGGCACCCUGAG | 19 | 3422 |
| myoC-1627 | - | AGGGAGCUGGGCACCCUGAG | 20 | 1900 |
| myoC-3677 | - | GAGGGAGCUGGGCACCCUGAG | 21 | 3423 |
| myoC-3678 | - | AGAGGGAGCUGGGCACCCUGAG | 22 | 3424 |
| myoC-3679 | - | CAGAGGGAGCUGGGCACCCUGAG | 23 | 3425 |
| myoC-3680 | - | GCAGAGGGAGCUGGGCACCCUGAG | 24 | 3426 |
| myoC-3681 | - | GGCCCCAGGAGACCCAGG | 18 | 3427 |
| myoC-3682 | - | AGGCCCCAGGAGACCCAGG | 19 | 3428 |
| myoC-186 | - | CAGGCCCCAGGAGACCCAGG | 20 | 572 |
| myoC-3683 | - | CCAGGCCCCAGGAGACCCAGG | 21 | 3429 |
| myoC-3684 | - | GCCAGGCCCCAGGAGACCCAGG | 22 | 3430 |
| myoC-3685 | - | UGCCAGGCCCCAGGAGACCCAGG | 23 | 3431 |
| myoC-3686 | - | CUGCCAGGCCCCAGGAGACCCAGG | 24 | 3432 |
| myoC-3687 | - | GAGAAUCUGGCCAGGAGG | 18 | 3433 |
| myoC-3688 | - | UGAGAAUCUGGCCAGGAGG | 19 | 3434 |
| myoC-1651 | - | AUGAGAAUCUGGCCAGGAGG | 20 | 1916 |
| myoC-3689 | - | AAUGAGAAUCUGGCCAGGAGG | 21 | 3435 |
| myoC-3690 | - | AAAUGAGAAUCUGGCCAGGAGG | 22 | 3436 |
| myoC-3691 | - | AAAAUGAGAAUCUGGCCAGGAGG | 23 | 3437 |
| myoC-3692 | - | GAAAAUGAGAAUCUGGCCAGGAGG | 24 | 3438 |
| myoC-3693 | - | ACAAGUCAGUUCUGGAGG | 18 | 3439 |
| myoC-3694 | - | GACAAGUCAGUUCUGGAGG | 19 | 3440 |
| myoC-1643 | - | AGACAAGUCAGUUCUGGAGG | 20 | 1909 |
| myoC-3695 | - | GAGACAAGUCAGUUCUGGAGG | 21 | 3441 |
| myoC-3696 | - | CGAGACAAGUCAGUUCUGGAGG | 22 | 3442 |
| myoC-3697 | - | CCGAGACAAGUCAGUUCUGGAGG | 23 | 3443 |
| myoC-3698 | - | UCCGAGACAAGUCAGUUCUGGAGG | 24 | 3444 |
| myoC-3699 | - | GAGCUGGGCACCCUGAGG | 18 | 3445 |
| myoC-3700 | - | GGAGCUGGGCACCCUGAGG | 19 | 3446 |
| myoC-102 | - | GGGAGCUGGGCACCCUGAGG | 20 | 507 |
| myoC-3701 | - | AGGGAGCUGGGCACCCUGAGG | 21 | 3447 |
| myoC-3702 | - | GAGGGAGCUGGGCACCCUGAGG | 22 | 3448 |
| myoC-3703 | - | AGAGGGAGCUGGGCACCCUGAGG | 23 | 3449 |
| myoC-3704 | - | CAGAGGGAGCUGGGCACCCUGAGG | 24 | 3450 |
| myoC-3705 | - | GAGACAAGUCAGUUCUGG | 18 | 3451 |
| myoC-3706 | - | CGAGACAAGUCAGUUCUGG | 19 | 3452 |
| myoC-192 | - | CCGAGACAAGUCAGUUCUGG | 20 | 578 |
| myoC-3707 | - | UCCGAGACAAGUCAGUUCUGG | 21 | 3453 |
| myoC-3708 | - | CUCCGAGACAAGUCAGUUCUGG | 22 | 3454 |
| myoC-3709 | - | CCUCCGAGACAAGUCAGUUCUGG | 23 | 3455 |
| myoC-3710 | - | UCCUCCGAGACAAGUCAGUUCUGG | 24 | 3456 |
| myoC-3711 | - | CCUGCCUGGUGUGGGAUG | 18 | 3457 |
| myoC-3712 | - | GCCUGCCUGGUGUGGGAUG | 19 | 3458 |
| myoC-94 | - | GGCCUGCCUGGUGUGGGAUG | 20 | 499 |
| myoC-3713 | - | UGGCCUGCCUGGUGUGGGAUG | 21 | 3459 |
| myoC-3714 | - | CUGGCCUGCCUGGUGUGGGAUG | 22 | 3460 |
| myoC-3715 | - | UCUGGCCUGCCUGGUGUGGGAUG | 23 | 3461 |
| myoC-3716 | - | UUCUGGCCUGCCUGGUGUGGGAUG | 24 | 3462 |
| myoC-3717 | - | GCUCGACUCAGCUCCCUG | 18 | 3463 |
| myoC-3718 | - | AGCUCGACUCAGCUCCCUG | 19 | 3464 |
| myoC-1613 | - | AAGCUCGACUCAGCUCCCUG | 20 | 1891 |
| myoC-3719 | - | AAAGCUCGACUCAGCUCCCUG | 21 | 3465 |
| myoC-3720 | - | CAAAGCUCGACUCAGCUCCCUG | 22 | 3466 |
| myoC-3721 | - | CCAAAGCUCGACUCAGCUCCCUG | 23 | 3467 |
| myoC-3722 | - | ACCAAAGCUCGACUCAGCUCCCUG | 24 | 3468 |
| myoC-3723 | - | GAGACCCAGGAGGGGCUG | 18 | 3469 |
| myoC-3724 | - | GGAGACCCAGGAGGGGCUG | 19 | 3470 |
| myoC-1621 | - | AGGAGACCCAGGAGGGGCUG | 20 | 1896 |
| myoC-3725 | - | CAGGAGACCCAGGAGGGGCUG | 21 | 3471 |
| myoC-3726 | - | CCAGGAGACCCAGGAGGGGCUG | 22 | 3472 |
| myoC-3727 | - | CCCAGGAGACCCAGGAGGGGCUG | 23 | 3473 |
| myoC-3728 | - | CCCCAGGAGACCCAGGAGGGGCUG | 24 | 3474 |
| myoC-3729 | - | CGAGACAAGUCAGUUCUG | 18 | 3475 |
| myoC-3730 | - | CCGAGACAAGUCAGUUCUG | 19 | 3476 |
| myoC-1641 | - | UCCGAGACAAGUCAGUUCUG | 20 | 1908 |
| myoC-3731 | - | CUCCGAGACAAGUCAGUUCUG | 21 | 3477 |
| myoC-3732 | - | CCUCCGAGACAAGUCAGUUCUG | 22 | 3478 |
| myoC-3733 | - | UCCUCCGAGACAAGUCAGUUCUG | 23 | 3479 |
| myoC-3734 | - | CUCCUCCGAGACAAGUCAGUUCUG | 24 | 3480 |
| myoC-3735 | - | GCCUGCCUGGUGUGGGAU | 18 | 3481 |
| myoC-3736 | - | GGCCUGCCUGGUGUGGGAU | 19 | 3482 |
| myoC-1597 | - | UGGCCUGCCUGGUGUGGGAU | 20 | 1880 |
| myoC-3737 | - | CUGGCCUGCCUGGUGUGGGAU | 21 | 3483 |
| myoC-3738 | - | UCUGGCCUGCCUGGUGUGGGAU | 22 | 3484 |
| myoC-3739 | - | UUCUGGCCUGCCUGGUGUGGGAU | 23 | 3485 |
| myoC-3740 | - | CUUCUGGCCUGCCUGGUGUGGGAU | 24 | 3486 |
| myoC-3741 | - | UGCCUACAGCAACCUCCU | 18 | 3487 |
| myoC-3742 | - | CUGCCUACAGCAACCUCCU | 19 | 3488 |
| myoC-1638 | - | ACUGCCUACAGCAACCUCCU | 20 | 1906 |
| myoC-3743 | - | GACUGCCUACAGCAACCUCCU | 21 | 3489 |
| myoC-3744 | - | AGACUGCCUACAGCAACCUCCU | 22 | 3490 |
| myoC-3745 | - | GAGACUGCCUACAGCAACCUCCU | 23 | 3491 |
| myoC-3746 | - | GGAGACUGCCUACAGCAACCUCCU | 24 | 3492 |
| myoC-3747 | - | GUGCACGUUGCUGCAGCU | 18 | 3493 |
| myoC-3748 | - | UGUGCACGUUGCUGCAGCU | 19 | 3494 |
| myoC-1593 | - | CUGUGCACGUUGCUGCAGCU | 20 | 1877 |
| myoC-3749 | - | UCUGUGCACGUUGCUGCAGCU | 21 | 3495 |
| myoC-3750 | - | UUCUGUGCACGUUGCUGCAGCU | 22 | 3496 |
| myoC-3751 | - | CUUCUGUGCACGUUGCUGCAGCU | 23 | 3497 |
| myoC-3752 | - | UCUUCUGUGCACGUUGCUGCAGCU | 24 | 3498 |
| myoC-3753 | - | GGCCAGGACAGCUCAGCU | 18 | 3499 |
| myoC-3754 | - | GGGCCAGGACAGCUCAGCU | 19 | 3500 |
| myoC-1601 | - | GGGGCCAGGACAGCUCAGCU | 20 | 1882 |
| myoC-3755 | - | GGGGGCCAGGACAGCUCAGCU | 21 | 3501 |
| myoC-3756 | - | UGGGGGCCAGGACAGCUCAGCU | 22 | 3502 |
| myoC-3757 | - | GUGGGGGCCAGGACAGCUCAGCU | 23 | 3503 |
| myoC-3758 | - | UGUGGGGGCCAGGACAGCUCAGCU | 24 | 3504 |
| myoC-3759 | - | AAACCCAAACCAGAGAGU | 18 | 3505 |
| myoC-3760 | - | GAAACCCAAACCAGAGAGU | 19 | 3506 |
| myoC-106 | - | GGAAACCCAAACCAGAGAGU | 20 | 479 |
| myoC-3761 | - | UGGAAACCCAAACCAGAGAGU | 21 | 3507 |
| myoC-3762 | - | CUGGAAACCCAAACCAGAGAGU | 22 | 3508 |
| myoC-3763 | - | GCUGGAAACCCAAACCAGAGAGU | 23 | 3509 |
| myoC-3764 | - | AGCUGGAAACCCAAACCAGAGAGU | 24 | 3510 |
| myoC-3765 | - | AGAAUCUGGCCAGGAGGU | 18 | 3511 |
| myoC-3766 | - | GAGAAUCUGGCCAGGAGGU | 19 | 3512 |
| myoC-197 | - | UGAGAAUCUGGCCAGGAGGU | 20 | 583 |
| myoC-3767 | - | AUGAGAAUCUGGCCAGGAGGU | 21 | 3513 |
| myoC-3768 | - | AAUGAGAAUCUGGCCAGGAGGU | 22 | 3514 |
| myoC-3769 | - | AAAUGAGAAUCUGGCCAGGAGGU | 23 | 3515 |
| myoC-3770 | - | AAAAUGAGAAUCUGGCCAGGAGGU | 24 | 3516 |
| myoC-3771 | - | GCUUCUGGCCUGCCUGGU | 18 | 3517 |
| myoC-3772 | - | UGCUUCUGGCCUGCCUGGU | 19 | 3518 |
| myoC-1595 | - | CUGCUUCUGGCCUGCCUGGU | 20 | 1879 |
| myoC-3773 | - | GCUGCUUCUGGCCUGCCUGGU | 21 | 3519 |
| myoC-3774 | - | UGCUGCUUCUGGCCUGCCUGGU | 22 | 3520 |
| myoC-3775 | - | CUGCUGCUUCUGGCCUGCCUGGU | 23 | 3521 |
| myoC-3776 | - | GCUGCUGCUUCUGGCCUGCCUGGU | 24 | 3522 |
| myoC-3777 | - | CUGCCUGGUGUGGGAUGU | 18 | 3523 |
| myoC-3778 | - | CCUGCCUGGUGUGGGAUGU | 19 | 3524 |
| myoC-95 | - | GCCUGCCUGGUGUGGGAUGU | 20 | 500 |
| myoC-3779 | - | GGCCUGCCUGGUGUGGGAUGU | 21 | 3525 |
| myoC-3780 | - | UGGCCUGCCUGGUGUGGGAUGU | 22 | 3526 |
| myoC-3781 | - | CUGGCCUGCCUGGUGUGGGAUGU | 23 | 3527 |
| myoC-3782 | - | UCUGGCCUGCCUGGUGUGGGAUGU | 24 | 3528 |
| myoC-3783 | - | CUCCGAGACAAGUCAGUU | 18 | 3529 |
| myoC-3784 | - | CCUCCGAGACAAGUCAGUU | 19 | 3530 |
| myoC-1639 | - | UCCUCCGAGACAAGUCAGUU | 20 | 1907 |
| myoC-3785 | - | CUCCUCCGAGACAAGUCAGUU | 21 | 3531 |
| myoC-3786 | - | CCUCCUCCGAGACAAGUCAGUU | 22 | 3532 |
| myoC-3787 | - | ACCUCCUCCGAGACAAGUCAGUU | 23 | 3533 |
| myoC-3788 | - | AACCUCCUCCGAGACAAGUCAGUU | 24 | 3534 |

**Table 7E** provides exemplary targeting domains for knocking out the *MYOC* gene selected according to the fifth tier parameters. The targeting domains fall in the coding sequence of the gene, downstream of the first 500bp of coding sequence (e.g., anywhere from +500 (relative to the start codon) to the stop codon of the gene), start with a 5' G and PAM is NNGRRT. It is contemplated herein that in an embodiment the targeting domain hybridizes to the target domain through complementary base pairing. Any of the targeting domains in the Table 1 can be used with a *S*. *aureus* Cas9 molecule that generates a double stranded break (Cas9 nuclease) or a single-stranded break (Cas9 nickase).

**Table 7E**

| 5th Tier | | | | |
|---|---|---|---|---|
| gRNA Name | DNA Strand | Targeting Domain | Target Site Length | SEQ ID NO |
| myoC-3789 | + | GUACUUAUAGCGGUUCUUGAA | 21 | 3535 |
| myoC-3790 | + | GCUGUACUUAUAGCGGUUCUUGAA | 24 | 3536 |
| myoC-3791 | + | GCAAAGAGCUUCUUCUCCA | 19 | 3537 |
| myoC-62 | + | GGCAAAGAGCUUCUUCUCCA | 20 | 448 |
| myoC-3792 | + | GAAAAUUUUAUUUCACAAUGUA | 22 | 3538 |
| myoC-3793 | + | GUCAAUGUCCGUGUAGCCACCCC | 23 | 3539 |
| myoC-3794 | + | GUCCGUGGUAGCCAGCUCC | 19 | 3540 |
| myoC-3795 | + | GAACUGUCCGUGGUAGCCAGCUCC | 24 | 3541 |
| myoC-3796 | + | GCCCUGGAAAUAGAGGCUCC | 20 | 3542 |
| myoC-3797 | + | GCGCCCUGGAAAUAGAGGCUCC | 22 | 3543 |
| myoC-3798 | + | GAUUCUCCACGUGGUCUC | 18 | 3544 |
| myoC-3799 | + | GUCGAUUCUCCACGUGGUCUC | 21 | 3545 |
| myoC-3800 | + | GUGUCGAUUCUCCACGUGGUCUC | 23 | 3546 |
| myoC-3801 | + | GCACAGCCCGAGCAGUGUC | 19 | 3547 |
| myoC-1700 | + | GGCACAGCCCGAGCAGUGUC | 20 | 1952 |
| myoC-3802 | + | GUGGCACAGCCCGAGCAGUGUC | 22 | 3548 |
| myoC-3803 | + | GGUGGCACAGCCCGAGCAGUGUC | 23 | 3549 |
| myoC-3804 | + | GCCCUCAGACUACAAUUC | 18 | 3550 |
| myoC-3805 | + | GUCUACGCCCUCAGACUACAAUUC | 24 | 3551 |
| myoC-3806 | + | GCUGUACUUAUAGCGGUUC | 19 | 3552 |
| myoC-3807 | + | GCUGCUGUACUUAUAGCGGUUC | 22 | 3553 |
| myoC-3808 | + | GCAGUAUGUGAACCUUAG | 18 | 3554 |
| myoC-3809 | + | GGCAGUAUGUGAACCUUAG | 19 | 3555 |
| myoC-3810 | + | GCCUAGGCAGUAUGUGAACCUUAG | 24 | 3556 |
| myoC-3811 | + | GUGUAGGGGUAGGUGGGCU | 19 | 3557 |
| myoC-478 | + | GGUGUAGGGGUAGGUGGGCU | 20 | 820 |
| myoC-3812 | + | GGGUGUAGGGGUAGGUGGGCU | 21 | 3558 |
| myoC-3813 | + | GUUCGAGUUCCAGAUUCU | 18 | 3559 |
| myoC-3814 | + | GUUUGUUCGAGUUCCAGAUUCU | 22 | 3560 |
| myoC-3815 | + | GGUUUGUUCGAGUUCCAGAUUCU | 23 | 3561 |
| myoC-3816 | + | GUUCUUGAAUGGGAUGGU | 18 | 3562 |
| myoC-3817 | + | GGUUCUUGAAUGGGAUGGU | 19 | 3563 |
| myoC-3818 | + | GCGGUUCUUGAAUGGGAUGGU | 21 | 3564 |
| myoC-3819 | + | GUUUGUCUCCCAGGUUUGU | 19 | 3565 |
| myoC-3820 | + | GAUGUUUGUCUCCCAGGUUUGU | 22 | 3566 |
| myoC-3821 | + | GGAUGUUUGUCUCCCAGGUUUGU | 23 | 3567 |
| myoC-3822 | + | GUGACCAUGUUCAUCCUU | 18 | 3568 |
| myoC-3823 | + | GGUGACCAUGUUCAUCCUU | 19 | 3569 |
| myoC-3824 | + | GAUGGUGACCAUGUUCAUCCUU | 22 | 3570 |
| myoC-3000 | + | GCAUUGGCGACUGACUGCUU | 20 | 2793 |
| myoC-3825 | + | GGCAUUGGCGACUGACUGCUU | 21 | 3571 |
| myoC-3826 | + | GAAGGCAUUGGCGACUGACUGCUU | 24 | 3572 |
| myoC-3827 | - | GUCCUCUCCAAACUGAACCCA | 21 | 3573 |
| myoC-3828 | - | GAAUAGCUCCUCUGGCCAGCA | 21 | 3574 |
| myoC-3829 | - | GCAGAAUAGCUCCUCUGGCCAGCA | 24 | 3575 |
| myoC-3830 | - | GGCUUCUAAUGCUUCAGA | 18 | 3576 |
| myoC-3831 | - | GUUGGCUUCUAAUGCUUCAGA | 21 | 3577 |
| myoC-3832 | - | GUUUUCUUUUCUGAAUUUAC | 20 | 3578 |
| myoC-3833 | - | GCCUAGGCCACUGGAAAGC | 19 | 3579 |
| myoC-3834 | - | GAGAAUCGACACAGUUGGC | 19 | 3580 |
| myoC-3835 | - | GGAGAAUCGACACAGUUGGC | 20 | 3581 |
| myoC-3836 | - | GUGGAGAAUCGACACAGUUGGC | 22 | 3582 |
| myoC-3837 | - | GAGCCCAUCUGGCUAUCUC | 19 | 3583 |
| myoC-3838 | - | GAGAGCCCAUCUGGCUAUCUC | 21 | 3584 |
| myoC-3839 | - | GGAGAGCCCAUCUGGCUAUCUC | 22 | 3585 |
| myoC-3840 | - | GUCACCAUCUAACUAUUC | 18 | 3586 |
| myoC-3841 | - | GGUCACCAUCUAACUAUUC | 19 | 3587 |
| myoC-3842 | - | GCUAACUGAAGUUCCUGCUUC | 21 | 3588 |
| myoC-3843 | - | GAGCUAACUGAAGUUCCUGCUUC | 23 | 3589 |
| myoC-3844 | - | GCAUAACUUCUAAAGGAAG | 19 | 3590 |
| myoC-3845 | - | GCUUCAGAUAGAAUACAG | 18 | 3591 |
| myoC-2905 | - | GAACUGUCAUAAGAUAUGAG | 20 | 1807 |
| myoC-3846 | - | GCCUCUAUUUCCAGGGCG | 18 | 3592 |
| myoC-3847 | - | GAGCCUCUAUUUCCAGGGCG | 20 | 3593 |
| myoC-3848 | - | GGAGCCUCUAUUUCCAGGGCG | 21 | 3594 |
| myoC-3849 | - | GGGAGCCUCUAUUUCCAGGGCG | 22 | 3595 |
| myoC-3850 | - | GGGGAGCCUCUAUUUCCAGGGCG | 23 | 3596 |
| myoC-3851 | - | GCUCCAGAGAAGGUAAGAAUG | 21 | 3597 |
| myoC-3852 | - | GGCUCCAGAGAAGGUAAGAAUG | 22 | 3598 |
| myoC-3853 | - | GAAUGCAGAGUGGGGGGACU | 20 | 3599 |
| myoC-3854 | - | GUAAGAAUGCAGAGUGGGGGGACU | 24 | 3600 |
| myoC-2920 | - | GCUGUGGAUGAAGCAGGCCU | 20 | 1819 |
| myoC-3855 | - | GGCUGUGGAUGAAGCAGGCCU | 21 | 3601 |
| myoC-3856 | - | GCUACACGGACAUUGACUUGGCU | 23 | 3602 |
| myoC-3857 | - | GGCUACACGGACAUUGACUUGGCU | 24 | 3603 |
| myoC-3858 | - | GGACAGUUCCCGUAUUCU | 18 | 3604 |
| myoC-3859 | - | GCCACCAGGCUCCAGAGAAGGU | 22 | 3605 |
| myoC-3860 | - | GUGCCACCAGGCUCCAGAGAAGGU | 24 | 3606 |
| myoC-3861 | - | GUUCUCUUCCUUGAACUUUGU | 21 | 3607 |
| myoC-3862 | - | GCACGGAUGUCCGCCAGGUUU | 21 | 3608 |
| myoC-3863 | - | GGCACGGAUGUCCGCCAGGUUU | 22 | 3609 |

**Table 7F** provides exemplary targeting domains for knocking out the *MYOC* gene selected according to the six tier parameters. The targeting domains fall in the coding sequence of the gene, downstream of the first 500bp of coding sequence (e.g., anywhere from +500 (relative to the start codon) to the stop codon of the gene) and PAM is NNGRRT. It is contemplated herein that in an embodiment the targeting domain hybridizes to the target domain through complementary base pairing. Any of the targeting domains in the Table 1 can be used with a *S*. *aureus* Cas9 molecule that generates a double stranded break (Cas9 nuclease) or a single-stranded break (Cas9 nickase).

**Table 7F**

| 6th Tier | | | | |
|---|---|---|---|---|
| gRNA Name | DNA Strand | Targeting Domain | Target Site Length | SEQ ID NO |
| myoC-3864 | + | CUUAUAGCGGUUCUUGAA | 18 | 3610 |
| myoC-3865 | + | ACUUAUAGCGGUUCUUGAA | 19 | 3611 |
| myoC-66 | + | UACUUAUAGCGGUUCUUGAA | 20 | 461 |
| myoC-3866 | + | UGUACUUAUAGCGGUUCUUGAA | 22 | 3612 |
| myoC-3867 | + | CUGUACUUAUAGCGGUUCUUGAA | 23 | 3613 |
| myoC-3868 | + | CAAAGAGCUUCUUCUCCA | 18 | 3614 |
| myoC-3869 | + | AGGCAAAGAGCUUCUUCUCCA | 21 | 3615 |
| myoC-3870 | + | CAGGCAAAGAGCUUCUUCUCCA | 22 | 3616 |
| myoC-3871 | + | CCAGGCAAAGAGCUUCUUCUCCA | 23 | 3617 |
| myoC-3872 | + | CCCAGGCAAAGAGCUUCUUCUCCA | 24 | 3618 |
| myoC-3873 | + | AAAUGCUGACAGAAGAUA | 18 | 3619 |
| myoC-3874 | + | UAAAUGCUGACAGAAGAUA | 19 | 3620 |
| myoC-3875 | + | AUAAAUGCUGACAGAAGAUA | 20 | 3621 |
| myoC-3876 | + | CAUAAAUGCUGACAGAAGAUA | 21 | 3622 |
| myoC-3877 | + | CCAUAAAUGCUGACAGAAGAUA | 22 | 3623 |
| myoC-3878 | + | CCCAUAAAUGCUGACAGAAGAUA | 23 | 3624 |
| myoC-3879 | + | UCCCAUAAAUGCUGACAGAAGAUA | 24 | 3625 |
| myoC-3880 | + | AUUUUAUUUCACAAUGUA | 18 | 3626 |
| myoC-3881 | + | AAUUUUAUUUCACAAUGUA | 19 | 3627 |
| myoC-3882 | + | AAAUUUUAUUUCACAAUGUA | 20 | 3628 |
| myoC-3883 | + | AAAAUUUUAUUUCACAAUGUA | 21 | 3629 |
| myoC-3884 | + | AGAAAAUUUUAUUUCACAAUGUA | 23 | 3630 |
| myoC-3885 | + | AAGAAAAUUUUAUUUCACAAUGUA | 24 | 3631 |
| myoC-3886 | + | UGUCCGUGUAGCCACCCC | 18 | 3632 |
| myoC-3887 | + | AUGUCCGUGUAGCCACCCC | 19 | 3633 |
| myoC-2928 | + | AAUGUCCGUGUAGCCACCCC | 20 | 1824 |
| myoC-3888 | + | CAAUGUCCGUGUAGCCACCCC | 21 | 3634 |
| myoC-3889 | + | UCAAUGUCCGUGUAGCCACCCC | 22 | 3635 |
| myoC-3890 | + | AGUCAAUGUCCGUGUAGCCACCCC | 24 | 3636 |
| myoC-3891 | + | UCCGUGGUAGCCAGCUCC | 18 | 3637 |
| myoC-23 | + | UGUCCGUGGUAGCCAGCUCC | 20 | 420 |
| myoC-3892 | + | CUGUCCGUGGUAGCCAGCUCC | 21 | 3638 |
| myoC-3893 | + | ACUGUCCGUGGUAGCCAGCUCC | 22 | 3639 |
| myoC-3894 | + | AACUGUCCGUGGUAGCCAGCUCC | 23 | 3640 |
| myoC-3895 | + | CCUGGAAAUAGAGGCUCC | 18 | 3641 |
| myoC-3896 | + | CCCUGGAAAUAGAGGCUCC | 19 | 3642 |
| myoC-3897 | + | CGCCCUGGAAAUAGAGGCUCC | 21 | 3643 |
| myoC-3898 | + | AGCGCCCUGGAAAUAGAGGCUCC | 23 | 3644 |
| myoC-3899 | + | CAGCGCCCUGGAAAUAGAGGCUCC | 24 | 3645 |
| myoC-3900 | + | CGAUUCUCCACGUGGUCUC | 19 | 3646 |
| myoC-3901 | + | UCGAUUCUCCACGUGGUCUC | 20 | 3647 |
| myoC-3902 | + | UGUCGAUUCUCCACGUGGUCUC | 22 | 3648 |
| myoC-3903 | + | UGUGUCGAUUCUCCACGUGGUCUC | 24 | 3649 |
| myoC-3904 | + | CACAGCCCGAGCAGUGUC | 18 | 3650 |
| myoC-3905 | + | UGGCACAGCCCGAGCAGUGUC | 21 | 3651 |
| myoC-3906 | + | UGGUGGCACAGCCCGAGCAGUGUC | 24 | 3652 |
| myoC-3907 | + | CGCCCUCAGACUACAAUUC | 19 | 3653 |
| myoC-3039 | + | ACGCCCUCAGACUACAAUUC | 20 | 2816 |
| myoC-3908 | + | UACGCCCUCAGACUACAAUUC | 21 | 3654 |
| myoC-3909 | + | CUACGCCCUCAGACUACAAUUC | 22 | 3655 |
| myoC-3910 | + | UCUACGCCCUCAGACUACAAUUC | 23 | 3656 |
| myoC-3911 | + | CUGUACUUAUAGCGGUUC | 18 | 3657 |
| myoC-2969 | + | UGCUGUACUUAUAGCGGUUC | 20 | 1856 |
| myoC-3912 | + | CUGCUGUACUUAUAGCGGUUC | 21 | 3658 |
| myoC-3913 | + | UGCUGCUGUACUUAUAGCGGUUC | 23 | 3659 |
| myoC-3914 | + | AUGCUGCUGUACUUAUAGCGGUUC | 24 | 3660 |
| myoC-3915 | + | AGGCAGUAUGUGAACCUUAG | 20 | 3661 |
| myoC-3916 | + | UAGGCAGUAUGUGAACCUUAG | 21 | 3662 |
| myoC-3917 | + | CUAGGCAGUAUGUGAACCUUAG | 22 | 3663 |
| myoC-3918 | + | CCUAGGCAGUAUGUGAACCUUAG | 23 | 3664 |
| myoC-3919 | + | AGUUCAAGGAAGAGAACG | 18 | 3665 |
| myoC-3920 | + | AAGUUCAAGGAAGAGAACG | 19 | 3666 |
| myoC-3921 | + | AAAGUUCAAGGAAGAGAACG | 20 | 3667 |
| myoC-3922 | + | CAAAGUUCAAGGAAGAGAACG | 21 | 3668 |
| myoC-3923 | + | ACAAAGUUCAAGGAAGAGAACG | 22 | 3669 |
| myoC-3924 | + | CACAAAGUUCAAGGAAGAGAACG | 23 | 3670 |
| myoC-3925 | + | CCACAAAGUUCAAGGAAGAGAACG | 24 | 3671 |
| myoC-3926 | + | UGUAGGGGUAGGUGGGCU | 18 | 3672 |
| myoC-3927 | + | UGGGUGUAGGGGUAGGUGGGCU | 22 | 3673 |
| myoC-3928 | + | CUGGGUGUAGGGGUAGGUGGGCU | 23 | 3674 |
| myoC-3929 | + | CCUGGGUGUAGGGGUAGGUGGGCU | 24 | 3675 |
| myoC-3930 | + | UGUUCGAGUUCCAGAUUCU | 19 | 3676 |
| myoC-3931 | + | UUGUUCGAGUUCCAGAUUCU | 20 | 3677 |
| myoC-3932 | + | UUUGUUCGAGUUCCAGAUUCU | 21 | 3678 |
| myoC-3933 | + | AGGUUUGUUCGAGUUCCAGAUUCU | 24 | 3679 |
| myoC-2966 | + | CGGUUCUUGAAUGGGAUGGU | 20 | 1854 |
| myoC-3934 | + | AGCGGUUCUUGAAUGGGAUGGU | 22 | 3680 |
| myoC-3935 | + | UAGCGGUUCUUGAAUGGGAUGGU | 23 | 3681 |
| myoC-3936 | + | AUAGCGGUUCUUGAAUGGGAUGGU | 24 | 3682 |
| myoC-3937 | + | ACGUGGUCUCCUGGGUGU | 18 | 3683 |
| myoC-3938 | + | CACGUGGUCUCCUGGGUGU | 19 | 3684 |
| myoC-472 | + | CCACGUGGUCUCCUGGGUGU | 20 | 814 |
| myoC-3939 | + | UCCACGUGGUCUCCUGGGUGU | 21 | 3685 |
| myoC-3940 | + | CUCCACGUGGUCUCCUGGGUGU | 22 | 3686 |
| myoC-3941 | + | UCUCCACGUGGUCUCCUGGGUGU | 23 | 3687 |
| myoC-3942 | + | UUCUCCACGUGGUCUCCUGGGUGU | 24 | 3688 |
| myoC-3943 | + | UUUGUCUCCCAGGUUUGU | 18 | 3689 |
| myoC-2999 | + | UGUUUGUCUCCCAGGUUUGU | 20 | 2792 |
| myoC-3944 | + | AUGUUUGUCUCCCAGGUUUGU | 21 | 3690 |
| myoC-3945 | + | CGGAUGUUUGUCUCCCAGGUUUGU | 24 | 3691 |
| myoC-3038 | + | UGGUGACCAUGUUCAUCCUU | 20 | 2815 |
| myoC-3946 | + | AUGGUGACCAUGUUCAUCCUU | 21 | 3692 |
| myoC-3947 | + | AGAUGGUGACCAUGUUCAUCCUU | 23 | 3693 |
| myoC-3948 | + | UAGAUGGUGACCAUGUUCAUCCUU | 24 | 3694 |
| myoC-3949 | + | AUUGGCGACUGACUGCUU | 18 | 3695 |
| myoC-3950 | + | CAUUGGCGACUGACUGCUU | 19 | 3696 |
| myoC-3951 | + | AGGCAUUGGCGACUGACUGCUU | 22 | 3697 |
| myoC-3952 | + | AAGGCAUUGGCGACUGACUGCUU | 23 | 3698 |
| myoC-3953 | - | CUCUCCAAACUGAACCCA | 18 | 3699 |
| myoC-3954 | - | CCUCUCCAAACUGAACCCA | 19 | 3700 |
| myoC-3955 | - | UCCUCUCCAAACUGAACCCA | 20 | 3701 |
| myoC-3956 | - | UGUCCUCUCCAAACUGAACCCA | 22 | 3702 |
| myoC-3957 | - | UUGUCCUCUCCAAACUGAACCCA | 23 | 3703 |
| myoC-3958 | - | AUUGUCCUCUCCAAACUGAACCCA | 24 | 3704 |
| myoC-3959 | - | UAGCUCCUCUGGCCAGCA | 18 | 3705 |
| myoC-3960 | - | AUAGCUCCUCUGGCCAGCA | 19 | 3706 |
| myoC-3961 | - | AAUAGCUCCUCUGGCCAGCA | 20 | 3707 |
| myoC-3962 | - | AGAAUAGCUCCUCUGGCCAGCA | 22 | 3708 |
| myoC-3963 | - | CAGAAUAGCUCCUCUGGCCAGCA | 23 | 3709 |
| myoC-3964 | - | UGGCUUCUAAUGCUUCAGA | 19 | 3710 |
| myoC-3965 | - | UUGGCUUCUAAUGCUUCAGA | 20 | 3711 |
| myoC-3966 | - | AGUUGGCUUCUAAUGCUUCAGA | 22 | 3712 |
| myoC-3967 | - | CAGUUGGCUUCUAAUGCUUCAGA | 23 | 3713 |
| myoC-3968 | - | ACAGUUGGCUUCUAAUGCUUCAGA | 24 | 3714 |
| myoC-3969 | - | AUCUUCUGUCAGCAUUUA | 18 | 3715 |
| myoC-3970 | - | UAUCUUCUGUCAGCAUUUA | 19 | 3716 |
| myoC-488 | - | UUAUCUUCUGUCAGCAUUUA | 20 | 830 |
| myoC-3971 | - | UUUAUCUUCUGUCAGCAUUUA | 21 | 3717 |
| myoC-3972 | - | CUUUAUCUUCUGUCAGCAUUUA | 22 | 3718 |
| myoC-3973 | - | CCUUUAUCUUCUGUCAGCAUUUA | 23 | 3719 |
| myoC-3974 | - | UCCUUUAUCUUCUGUCAGCAUUUA | 24 | 3720 |
| myoC-3975 | - | UUUCUUUUCUGAAUUUAC | 18 | 3721 |
| myoC-3976 | - | UUUUCUUUUCUGAAUUUAC | 19 | 3722 |
| myoC-3977 | - | CGUUUUCUUUUCUGAAUUUAC | 21 | 3723 |
| myoC-3978 | - | UCGUUUUCUUUUCUGAAUUUAC | 22 | 3724 |
| myoC-3979 | - | UUCGUUUUCUUUUCUGAAUUUAC | 23 | 3725 |
| myoC-3980 | - | CUUCGUUUUCUUUUCUGAAUUUAC | 24 | 3726 |
| myoC-3981 | - | CCUAGGCCACUGGAAAGC | 18 | 3727 |
| myoC-3982 | - | UGCCUAGGCCACUGGAAAGC | 20 | 3728 |
| myoC-3983 | - | CUGCCUAGGCCACUGGAAAGC | 21 | 3729 |
| myoC-3984 | - | ACUGCCUAGGCCACUGGAAAGC | 22 | 3730 |
| myoC-3985 | - | UACUGCCUAGGCCACUGGAAAGC | 23 | 3731 |
| myoC-3986 | - | AUACUGCCUAGGCCACUGGAAAGC | 24 | 3732 |
| myoC-3987 | - | AGAAUCGACACAGUUGGC | 18 | 3733 |
| myoC-3988 | - | UGGAGAAUCGACACAGUUGGC | 21 | 3734 |
| myoC-3989 | - | CGUGGAGAAUCGACACAGUUGGC | 23 | 3735 |
| myoC-3990 | - | ACGUGGAGAAUCGACACAGUUGGC | 24 | 3736 |
| myoC-3991 | - | AGCCCAUCUGGCUAUCUC | 18 | 3737 |
| myoC-319 | - | AGAGCCCAUCUGGCUAUCUC | 20 | 705 |
| myoC-3992 | - | AGGAGAGCCCAUCUGGCUAUCUC | 23 | 3738 |
| myoC-3993 | - | AAGGAGAGCCCAUCUGGCUAUCUC | 24 | 3739 |
| myoC-485 | - | UGGUCACCAUCUAACUAUUC | 20 | 827 |
| myoC-3994 | - | AUGGUCACCAUCUAACUAUUC | 21 | 3740 |
| myoC-3995 | - | CAUGGUCACCAUCUAACUAUUC | 22 | 3741 |
| myoC-3996 | - | ACAUGGUCACCAUCUAACUAUUC | 23 | 3742 |
| myoC-3997 | - | AACAUGGUCACCAUCUAACUAUUC | 24 | 3743 |
| myoC-3998 | - | AACUGAAGUUCCUGCUUC | 18 | 3744 |
| myoC-3999 | - | UAACUGAAGUUCCUGCUUC | 19 | 3745 |
| myoC-4000 | - | CUAACUGAAGUUCCUGCUUC | 20 | 3746 |
| myoC-4001 | - | AGCUAACUGAAGUUCCUGCUUC | 22 | 3747 |
| myoC-4002 | - | CGAGCUAACUGAAGUUCCUGCUUC | 24 | 3748 |
| myoC-4003 | - | CAUAACUUCUAAAGGAAG | 18 | 3749 |
| myoC-4004 | - | AGCAUAACUUCUAAAGGAAG | 20 | 3750 |
| myoC-4005 | - | AAGCAUAACUUCUAAAGGAAG | 21 | 3751 |
| myoC-4006 | - | AAAGCAUAACUUCUAAAGGAAG | 22 | 3752 |
| myoC-4007 | - | AAAAGCAUAACUUCUAAAGGAAG | 23 | 3753 |
| myoC-4008 | - | AAAAAGCAUAACUUCUAAAGGAAG | 24 | 3754 |
| myoC-4009 | - | UGCUUCAGAUAGAAUACAG | 19 | 3755 |
| myoC-4010 | - | AUGCUUCAGAUAGAAUACAG | 20 | 3756 |
| myoC-4011 | - | AAUGCUUCAGAUAGAAUACAG | 21 | 3757 |
| myoC-4012 | - | UAAUGCUUCAGAUAGAAUACAG | 22 | 3758 |
| myoC-4013 | - | CUAAUGCUUCAGAUAGAAUACAG | 23 | 3759 |
| myoC-4014 | - | UCUAAUGCUUCAGAUAGAAUACAG | 24 | 3760 |
| myoC-4015 | - | AAGUUUUCAUUAAUCCAG | 18 | 3761 |
| myoC-4016 | - | CAAGUUUUCAUUAAUCCAG | 19 | 3762 |
| myoC-3020 | - | CCAAGUUUUCAUUAAUCCAG | 20 | 2804 |
| myoC-4017 | - | UCCAAGUUUUCAUUAAUCCAG | 21 | 3763 |
| myoC-4018 | - | UUCCAAGUUUUCAUUAAUCCAG | 22 | 3764 |
| myoC-4019 | - | UUUCCAAGUUUUCAUUAAUCCAG | 23 | 3765 |
| myoC-4020 | - | CUUUCCAAGUUUUCAUUAAUCCAG | 24 | 3766 |
| myoC-4021 | - | ACUGUCAUAAGAUAUGAG | 18 | 3767 |
| myoC-4022 | - | AACUGUCAUAAGAUAUGAG | 19 | 3768 |
| myoC-4023 | - | AGAACUGUCAUAAGAUAUGAG | 21 | 3769 |
| myoC-4024 | - | CAGAACUGUCAUAAGAUAUGAG | 22 | 3770 |
| myoC-4025 | - | CCAGAACUGUCAUAAGAUAUGAG | 23 | 3771 |
| myoC-4026 | - | UCCAGAACUGUCAUAAGAUAUGAG | 24 | 3772 |
| myoC-4027 | - | UUUAAUGCAGUUUCUACG | 18 | 3773 |
| myoC-4028 | - | UUUUAAUGCAGUUUCUACG | 19 | 3774 |
| myoC-313 | - | CUUUUAAUGCAGUUUCUACG | 20 | 699 |
| myoC-4029 | - | UCUUUUAAUGCAGUUUCUACG | 21 | 3775 |
| myoC-4030 | - | UUCUUUUAAUGCAGUUUCUACG | 22 | 3776 |
| myoC-4031 | - | UUUCUUUUAAUGCAGUUUCUACG | 23 | 3777 |
| myoC-4032 | - | CUUUCUUUUAAUGCAGUUUCUACG | 24 | 3778 |
| myoC-4033 | - | AGCCUCUAUUUCCAGGGCG | 19 | 3779 |
| myoC-4034 | - | CGGGGAGCCUCUAUUUCCAGGGCG | 24 | 3780 |
| myoC-4035 | - | CCAGAGAAGGUAAGAAUG | 18 | 3781 |
| myoC-4036 | - | UCCAGAGAAGGUAAGAAUG | 19 | 3782 |
| myoC-4037 | - | CUCCAGAGAAGGUAAGAAUG | 20 | 3783 |
| myoC-4038 | - | AGGCUCCAGAGAAGGUAAGAAUG | 23 | 3784 |
| myoC-4039 | - | CAGGCUCCAGAGAAGGUAAGAAUG | 24 | 3785 |
| myoC-4040 | - | CACCCAGGAGACCACGUG | 18 | 3786 |
| myoC-4041 | - | ACACCCAGGAGACCACGUG | 19 | 3787 |
| myoC-4042 | - | UACACCCAGGAGACCACGUG | 20 | 3788 |
| myoC-4043 | - | CUACACCCAGGAGACCACGUG | 21 | 3789 |
| myoC-4044 | - | CCUACACCCAGGAGACCACGUG | 22 | 3790 |
| myoC-4045 | - | CCCUACACCCAGGAGACCACGUG | 23 | 3791 |
| myoC-4046 | - | CCCCUACACCCAGGAGACCACGUG | 24 | 3792 |
| myoC-4047 | - | AUGCAGAGUGGGGGGACU | 18 | 3793 |
| myoC-4048 | - | AAUGCAGAGUGGGGGGACU | 19 | 3794 |
| myoC-4049 | - | AGAAUGCAGAGUGGGGGGACU | 21 | 3795 |
| myoC-4050 | - | AAGAAUGCAGAGUGGGGGGACU | 22 | 3796 |
| myoC-4051 | - | UAAGAAUGCAGAGUGGGGGGACU | 23 | 3797 |
| myoC-4052 | - | UGUGGAUGAAGCAGGCCU | 18 | 3798 |
| myoC-4053 | - | CUGUGGAUGAAGCAGGCCU | 19 | 3799 |
| myoC-4054 | - | UGGCUGUGGAUGAAGCAGGCCU | 22 | 3800 |
| myoC-4055 | - | UUGGCUGUGGAUGAAGCAGGCCU | 23 | 3801 |
| myoC-4056 | - | CUUGGCUGUGGAUGAAGCAGGCCU | 24 | 3802 |
| myoC-4057 | - | ACGGACAUUGACUUGGCU | 18 | 3803 |
| myoC-4058 | - | CACGGACAUUGACUUGGCU | 19 | 3804 |
| myoC-2918 | - | ACACGGACAUUGACUUGGCU | 20 | 1817 |
| myoC-4059 | - | UACACGGACAUUGACUUGGCU | 21 | 3805 |
| myoC-4060 | - | CUACACGGACAUUGACUUGGCU | 22 | 3806 |
| myoC-4061 | - | CGGACAGUUCCCGUAUUCU | 19 | 3807 |
| myoC-6 | - | ACGGACAGUUCCCGUAUUCU | 20 | 408 |
| myoC-4062 | - | CACGGACAGUUCCCGUAUUCU | 21 | 3808 |
| myoC-4063 | - | CCACGGACAGUUCCCGUAUUCU | 22 | 3809 |
| myoC-4064 | - | ACCACGGACAGUUCCCGUAUUCU | 23 | 3810 |
| myoC-4065 | - | UACCACGGACAGUUCCCGUAUUCU | 24 | 3811 |
| myoC-4066 | - | AGGAUGUGGAGAACUAGU | 18 | 3812 |
| myoC-4067 | - | CAGGAUGUGGAGAACUAGU | 19 | 3813 |
| myoC-4068 | - | CCAGGAUGUGGAGAACUAGU | 20 | 3814 |
| myoC-4069 | - | ACCAGGAUGUGGAGAACUAGU | 21 | 3815 |
| myoC-4070 | - | UACCAGGAUGUGGAGAACUAGU | 22 | 3816 |
| myoC-4071 | - | UUACCAGGAUGUGGAGAACUAGU | 23 | 3817 |
| myoC-4072 | - | UUUACCAGGAUGUGGAGAACUAGU | 24 | 3818 |
| myoC-4073 | - | CCAGGCUCCAGAGAAGGU | 18 | 3819 |
| myoC-4074 | - | ACCAGGCUCCAGAGAAGGU | 19 | 3820 |
| myoC-4075 | - | CACCAGGCUCCAGAGAAGGU | 20 | 3821 |
| myoC-4076 | - | CCACCAGGCUCCAGAGAAGGU | 21 | 3822 |
| myoC-4077 | - | UGCCACCAGGCUCCAGAGAAGGU | 23 | 3823 |
| myoC-4078 | - | UACUGGCAAGUAUGGUGU | 18 | 3824 |
| myoC-4079 | - | UUACUGGCAAGUAUGGUGU | 19 | 3825 |
| myoC-4080 | - | AUUACUGGCAAGUAUGGUGU | 20 | 3826 |
| myoC-4081 | - | AAUUACUGGCAAGUAUGGUGU | 21 | 3827 |
| myoC-4082 | - | CAAUUACUGGCAAGUAUGGUGU | 22 | 3828 |
| myoC-4083 | - | ACAAUUACUGGCAAGUAUGGUGU | 23 | 3829 |
| myoC-4084 | - | AACAAUUACUGGCAAGUAUGGUGU | 24 | 3830 |
| myoC-4085 | - | CUCUUCCUUGAACUUUGU | 18 | 3831 |
| myoC-4086 | - | UCUCUUCCUUGAACUUUGU | 19 | 3832 |
| myoC-3193 | - | UUCUCUUCCUUGAACUUUGU | 20 | 2939 |
| myoC-4087 | - | CGUUCUCUUCCUUGAACUUUGU | 22 | 3833 |
| myoC-4088 | - | ACGUUCUCUUCCUUGAACUUUGU | 23 | 3834 |
| myoC-4089 | - | AACGUUCUCUUCCUUGAACUUUGU | 24 | 3835 |
| myoC-4090 | - | CGGAUGUCCGCCAGGUUU | 18 | 3836 |
| myoC-4091 | - | ACGGAUGUCCGCCAGGUUU | 19 | 3837 |
| myoC-4092 | - | CACGGAUGUCCGCCAGGUUU | 20 | 3838 |
| myoC-4093 | - | UGGCACGGAUGUCCGCCAGGUUU | 23 | 3839 |
| myoC-4094 | - | UUGGCACGGAUGUCCGCCAGGUUU | 24 | 3840 |

**Table 7G** provides exemplary targeting domains for knocking out the *MYOC* gene selected according to the seven tier parameters. The targeting domains fall in the coding sequence of the gene, downstream of the first 500bp of coding sequence (e.g., anywhere from +500 (relative to the start codon) to the stop codon of the gene) and PAM is NNGRRV. It is contemplated herein that in an embodiment the targeting domain hybridizes to the target domain through complementary base pairing. Any of the targeting domains in the Table 1 can be used with a *S*. *aureus* Cas9 molecule that generates a double stranded break (Cas9 nuclease) or a single-stranded break (Cas9 nickase).

**Table 7G**

| 7th Tier | | | | |
|---|---|---|---|---|
| gRNA Name | DNA Strand | Targeting Domain | Target Site Length | SEQ ID NO |
| myoC-4095 | + | GUAAAUUCAGAAAAGAAA | 18 | 3841 |
| myoC-4096 | + | GGUAAAUUCAGAAAAGAAA | 19 | 3842 |
| myoC-4097 | + | UGGUAAAUUCAGAAAAGAAA | 20 | 3843 |
| myoC-4098 | + | CUGGUAAAUUCAGAAAAGAAA | 21 | 3844 |
| myoC-4099 | + | CCUGGUAAAUUCAGAAAAGAAA | 22 | 3845 |
| myoC-4100 | + | UCCUGGUAAAUUCAGAAAAGAAA | 23 | 3846 |
| myoC-4101 | + | AUCCUGGUAAAUUCAGAAAAGAAA | 24 | 3847 |
| myoC-4102 | + | GACUCAGCGCCCUGGAAA | 18 | 3848 |
| myoC-4103 | + | GGACUCAGCGCCCUGGAAA | 19 | 3849 |
| myoC-4104 | + | UGGACUCAGCGCCCUGGAAA | 20 | 3850 |
| myoC-4105 | + | CUGGACUCAGCGCCCUGGAAA | 21 | 3851 |
| myoC-4106 | + | UCUGGACUCAGCGCCCUGGAAA | 22 | 3852 |
| myoC-4107 | + | UUCUGGACUCAGCGCCCUGGAAA | 23 | 3853 |
| myoC-4108 | + | GUUCUGGACUCAGCGCCCUGGAAA | 24 | 3854 |
| myoC-4109 | + | AUCCUGGUAAAUUCAGAA | 18 | 3855 |
| myoC-4110 | + | CAUCCUGGUAAAUUCAGAA | 19 | 3856 |
| myoC-4111 | + | ACAUCCUGGUAAAUUCAGAA | 20 | 3857 |
| myoC-4112 | + | CACAUCCUGGUAAAUUCAGAA | 21 | 3858 |
| myoC-4113 | + | CCACAUCCUGGUAAAUUCAGAA | 22 | 3859 |
| myoC-4114 | + | UCCACAUCCUGGUAAAUUCAGAA | 23 | 3860 |
| myoC-4115 | + | CUCCACAUCCUGGUAAAUUCAGAA | 24 | 3861 |
| myoC-4116 | + | CCCACAAAGUUCAAGGAA | 18 | 3862 |
| myoC-4117 | + | UCCCACAAAGUUCAAGGAA | 19 | 3863 |
| myoC-4118 | + | UUCCCACAAAGUUCAAGGAA | 20 | 3864 |
| myoC-4119 | + | ACGUAGAAACUGCAUUAA | 18 | 3865 |
| myoC-4120 | + | CACGUAGAAACUGCAUUAA | 19 | 3866 |
| myoC-4121 | + | CCACGUAGAAACUGCAUUAA | 20 | 3867 |
| myoC-4122 | + | UCCACGUAGAAACUGCAUUAA | 21 | 3868 |
| myoC-4123 | + | UUCCACGUAGAAACUGCAUUAA | 22 | 3869 |
| myoC-4124 | + | AUUCCACGUAGAAACUGCAUUAA | 23 | 3870 |
| myoC-4125 | + | AAUUCCACGUAGAAACUGCAUUAA | 24 | 3871 |
| myoC-4126 | + | UAUAUUCGAUGCUGGCCA | 18 | 3872 |
| myoC-4127 | + | UUAUAUUCGAUGCUGGCCA | 19 | 3873 |
| myoC-4128 | + | CUUAUAUUCGAUGCUGGCCA | 20 | 3874 |
| myoC-4129 | + | ACUUAUAUUCGAUGCUGGCCA | 21 | 3875 |
| myoC-4130 | + | UACUUAUAUUCGAUGCUGGCCA | 22 | 3876 |
| myoC-4131 | + | UUACUUAUAUUCGAUGCUGGCCA | 23 | 3877 |
| myoC-4132 | + | CUUACUUAUAUUCGAUGCUGGCCA | 24 | 3878 |
| myoC-4133 | + | UUCAAGUUGUCCCAGGCA | 18 | 3879 |
| myoC-4134 | + | GUUCAAGUUGUCCCAGGCA | 19 | 3880 |
| myoC-2973 | + | UGUUCAAGUUGUCCCAGGCA | 20 | 1858 |
| myoC-4135 | + | AUGUUCAAGUUGUCCCAGGCA | 21 | 3881 |
| myoC-4136 | + | CAUGUUCAAGUUGUCCCAGGCA | 22 | 3882 |
| myoC-4137 | + | CCAUGUUCAAGUUGUCCCAGGCA | 23 | 3883 |
| myoC-4138 | + | ACCAUGUUCAAGUUGUCCCAGGCA | 24 | 3884 |
| myoC-4139 | + | AGAAACUGCAUUAAAAGA | 18 | 3885 |
| myoC-4140 | + | UAGAAACUGCAUUAAAAGA | 19 | 3886 |
| myoC-4141 | + | GUAGAAACUGCAUUAAAAGA | 20 | 3887 |
| myoC-4142 | + | CGUAGAAACUGCAUUAAAAGA | 21 | 3888 |
| myoC-4143 | + | ACGUAGAAACU GCAU UAAAAGA | 22 | 3889 |
| myoC-4144 | + | CACGUAGAAACUGCAUUAAAAGA | 23 | 3890 |
| myoC-4145 | + | CCACGUAGAAACUGCAUUAAAAGA | 24 | 3891 |
| myoC-4146 | + | CUCUGGGUUCAGUUUGGA | 18 | 3892 |
| myoC-4147 | + | UCUCUGGGUUCAGUUUGGA | 19 | 3893 |
| myoC-4148 | + | UUCUCUGGGUUCAGUUUGGA | 20 | 3894 |
| myoC-4149 | + | AUUCUCUGGGUUCAGUUUGGA | 21 | 3895 |
| myoC-4150 | + | GAUUCUCUGGGUUCAGUUUGGA | 22 | 3896 |
| myoC-4151 | + | AGAUUCUCUGGGUUCAGUUUGGA | 23 | 3897 |
| myoC-4152 | + | CAGAUUCUCUGGGUUCAGUUUGGA | 24 | 3898 |
| myoC-4153 | + | ACAUCCCAUAAAUGCUGA | 18 | 3899 |
| myoC-4154 | + | AACAUCCCAUAAAUGCUGA | 19 | 3900 |
| myoC-4155 | + | AAACAUCCCAUAAAUGCUGA | 20 | 3901 |
| myoC-4156 | + | UAAACAUCCCAUAAAUGCUGA | 21 | 3902 |
| myoC-4157 | + | UUAAACAUCCCAUAAAUGCUGA | 22 | 3903 |
| myoC-4158 | + | AUUAAACAUCCCAUAAAUGCUGA | 23 | 3904 |
| myoC-4159 | + | CAUUAAACAUCCCAUAAAUGCUGA | 24 | 3905 |
| myoC-4160 | + | ACUUAUAGCGGUUCUUGA | 18 | 3906 |
| myoC-4161 | + | UACUUAUAGCGGUUCUUGA | 19 | 3907 |
| myoC-2968 | + | GUACUUAUAGCGGUUCUUGA | 20 | 1855 |
| myoC-4162 | + | UGUACUUAUAGCGGUUCUUGA | 21 | 3908 |
| myoC-4163 | + | CUGUACUUAUAGCGGUUCUUGA | 22 | 3909 |
| myoC-4164 | + | GCUGUACUUAUAGCGGUUCUUGA | 23 | 3910 |
| myoC-4165 | + | UGCUGUACUUAUAGCGGUUCUUGA | 24 | 3911 |
| myoC-4166 | + | GUAGCCACCCCAAGAAUA | 18 | 3912 |
| myoC-4167 | + | UGUAGCCACCCCAAGAAUA | 19 | 3913 |
| myoC-20 | + | GUGUAGCCACCCCAAGAAUA | 20 | 390 |
| myoC-4168 | + | CGUGUAGCCACCCCAAGAAUA | 21 | 3914 |
| myoC-4169 | + | CCGUGUAGCCACCCCAAGAAUA | 22 | 3915 |
| myoC-4170 | + | UCCGUGUAGCCACCCCAAGAAUA | 23 | 3916 |
| myoC-4171 | + | GUCCGUGUAGCCACCCCAAGAAUA | 24 | 3917 |
| myoC-4172 | + | GUUCAUCCUUCUGGAUUA | 18 | 3918 |
| myoC-4173 | + | UGUUCAUCCUUCUGGAUUA | 19 | 3919 |
| myoC-3037 | + | AUGUUCAUCCUUCUGGAUUA | 20 | 2814 |
| myoC-4174 | + | CAUGUUCAUCCUUCUGGAUUA | 21 | 3920 |
| myoC-4175 | + | CCAUGUUCAUCCUUCUGGAUUA | 22 | 3921 |
| myoC-4176 | + | ACCAUGUUCAUCCUUCUGGAUUA | 23 | 3922 |
| myoC-4177 | + | GACCAUGUUCAUCCUUCUGGAUUA | 24 | 3923 |
| myoC-4178 | + | CCCAAAUCACAAGAAAAC | 18 | 3924 |
| myoC-4179 | + | CCCCAAAUCACAAGAAAAC | 19 | 3925 |
| myoC-4180 | + | GCCCCAAAUCACAAGAAAAC | 20 | 3926 |
| myoC-4181 | + | UGCCCCAAAUCACAAGAAAAC | 21 | 3927 |
| myoC-4182 | + | UUGCCCCAAAUCACAAGAAAAC | 22 | 3928 |
| myoC-4183 | + | UUUGCCCCAAAUCACAAGAAAAC | 23 | 3929 |
| myoC-4184 | + | UUUUGCCCCAAAUCACAAGAAAAC | 24 | 3930 |
| myoC-4185 | + | UUCUGGAUUAAUGAAAAC | 18 | 3931 |
| myoC-4186 | + | CUUCUGGAUUAAUGAAAAC | 19 | 3932 |
| myoC-3036 | + | CCUUCUGGAUUAAUGAAAAC | 20 | 2813 |
| myoC-4187 | + | UCCUUCUGGAUUAAUGAAAAC | 21 | 3933 |
| myoC-4188 | + | AUCCUUCUGGAUUAAUGAAAAC | 22 | 3934 |
| myoC-4189 | + | CAUCCUUCUGGAUUAAUGAAAAC | 23 | 3935 |
| myoC-4190 | + | UCAUCCUUCUGGAUUAAUGAAAAC | 24 | 3936 |
| myoC-4191 | + | GCUUUUGCCCCAAAUCAC | 18 | 3937 |
| myoC-4192 | + | AGCUUUUGCCCCAAAUCAC | 19 | 3938 |
| myoC-4193 | + | CAGCUUUUGCCCCAAAUCAC | 20 | 3939 |
| myoC-4194 | + | ACAGCUUUUGCCCCAAAUCAC | 21 | 3940 |
| myoC-4195 | + | UACAGCUUUUGCCCCAAAUCAC | 22 | 3941 |
| myoC-4196 | + | UUACAGCUUUUGCCCCAAAUCAC | 23 | 3942 |
| myoC-4197 | + | CUUACAGCUUUUGCCCCAAAUCAC | 24 | 3943 |
| myoC-4198 | + | UAGCCACCCCAAGAAUAC | 18 | 3944 |
| myoC-4199 | + | GUAGCCACCCCAAGAAUAC | 19 | 3945 |
| myoC-21 | + | UGUAGCCACCCCAAGAAUAC | 20 | 418 |
| myoC-4200 | + | GUGUAGCCACCCCAAGAAUAC | 21 | 3946 |
| myoC-4201 | + | CGUGUAGCCACCCCAAGAAUAC | 22 | 3947 |
| myoC-4202 | + | CCGUGUAGCCACCCCAAGAAUAC | 23 | 3948 |
| myoC-4203 | + | UCCGUGUAGCCACCCCAAGAAUAC | 24 | 3949 |
| myoC-4204 | + | UCGGUGCUGUAAAUGACC | 18 | 3950 |
| myoC-4205 | + | AUCGGUGCUGUAAAUGACC | 19 | 3951 |
| myoC-2929 | + | CAUCGGUGCUGUAAAUGACC | 20 | 1825 |
| myoC-4206 | + | UCAUCGGUGCUGUAAAUGACC | 21 | 3952 |
| myoC-4207 | + | CUCAUCGGUGCUGUAAAUGACC | 22 | 3953 |
| myoC-4208 | + | CCUCAUCGGUGCUGUAAAUGACC | 23 | 3954 |
| myoC-4209 | + | GCCUCAUCGGUGCUGUAAAUGACC | 24 | 3955 |
| myoC-4210 | + | CUUCAGCCUGCUCCCCCC | 18 | 3956 |
| myoC-4211 | + | CCUUCAGCCUGCUCCCCCC | 19 | 3957 |
| myoC-421 | + | CCCUUCAGCCUGCUCCCCCC | 20 | 785 |
| myoC-4212 | + | UCCCUUCAGCCUGCUCCCCCC | 21 | 3958 |
| myoC-4213 | + | CUCCCUUCAGCCUGCUCCCCCC | 22 | 3959 |
| myoC-4214 | + | UCUCCCUUCAGCCUGCUCCCCCC | 23 | 3960 |
| myoC-4215 | + | CUCUCCCUUCAGCCUGCUCCCCCC | 24 | 3961 |
| myoC-4216 | + | CCUUCAGCCUGCUCCCCC | 18 | 3962 |
| myoC-4217 | + | CCCUUCAGCCUGCUCCCCC | 19 | 3963 |
| myoC-3033 | + | UCCCUUCAGCCUGCUCCCCC | 20 | 2811 |
| myoC-4218 | + | CUCCCUUCAGCCUGCUCCCCC | 21 | 3964 |
| myoC-4219 | + | UCUCCCUUCAGCCUGCUCCCCC | 22 | 3965 |
| myoC-4220 | + | CUCUCCCUUCAGCCUGCUCCCCC | 23 | 3966 |
| myoC-4221 | + | GCUCUCCCUUCAGCCUGCUCCCCC | 24 | 3967 |
| myoC-4222 | + | GUUCUGGACUCAGCGCCC | 18 | 3968 |
| myoC-4223 | + | AGUUCUGGACUCAGCGCCC | 19 | 3969 |
| myoC-459 | + | CAGUUCUGGACUCAGCGCCC | 20 | 801 |
| myoC-4224 | + | ACAGUUCUGGACUCAGCGCCC | 21 | 3970 |
| myoC-4225 | + | GACAGUUCUGGACUCAGCGCCC | 22 | 3971 |
| myoC-4226 | + | UGACAGUUCUGGACUCAGCGCCC | 23 | 3972 |
| myoC-4227 | + | AUGACAGUUCUGGACUCAGCGCCC | 24 | 3973 |
| myoC-4228 | + | CUGCUCCCCCCAGGAGCC | 18 | 3974 |
| myoC-4229 | + | CCUGCUCCCCCCAGGAGCC | 19 | 3975 |
| myoC-3031 | + | GCCUGCUCCCCCCAGGAGCC | 20 | 2810 |
| myoC-4230 | + | AGCCUGCUCCCCCCAGGAGCC | 21 | 3976 |
| myoC-4231 | + | CAGCCUGCUCCCCCCAGGAGCC | 22 | 3977 |
| myoC-4232 | + | UCAGCCUGCUCCCCCCAGGAGCC | 23 | 3978 |
| myoC-4233 | + | UUCAGCCUGCUCCCCCCAGGAGCC | 24 | 3979 |
| myoC-4234 | + | AGUUCUGGACUCAGCGCC | 18 | 3980 |
| myoC-4235 | + | CAGUUCUGGACUCAGCGCC | 19 | 3981 |
| myoC-4236 | + | ACAGUUCUGGACUCAGCGCC | 20 | 3982 |
| myoC-4237 | + | GACAGUUCUGGACUCAGCGCC | 21 | 3983 |
| myoC-4238 | + | UGACAGUUCUGGACUCAGCGCC | 22 | 3984 |
| myoC-4239 | + | AUGACAGUUCUGGACUCAGCGCC | 23 | 3985 |
| myoC-4240 | + | UAUGACAGUUCUGGACUCAGCGCC | 24 | 3986 |
| myoC-4241 | + | GCAAAGAGCUUCUUCUCC | 18 | 3987 |
| myoC-4242 | + | GGCAAAGAGCUUCUUCUCC | 19 | 3988 |
| myoC-61 | + | AGGCAAAGAGCUUCUUCUCC | 20 | 458 |
| myoC-4243 | + | CAGGCAAAGAGCUUCUUCUCC | 21 | 3989 |
| myoC-4244 | + | CCAGGCAAAGAGCUUCUUCUCC | 22 | 3990 |
| myoC-4245 | + | CCCAGGCAAAGAGCUUCUUCUCC | 23 | 3991 |
| myoC-4246 | + | UCCCAGGCAAAGAGCUUCUUCUCC | 24 | 3992 |
| myoC-4247 | + | AGCUCGGACUUCAGUUCC | 18 | 3993 |
| myoC-4248 | + | UAGCUCGGACUUCAGUUCC | 19 | 3994 |
| myoC-331 | + | UUAGCUCGGACUUCAGUUCC | 20 | 717 |
| myoC-4249 | + | GUUAGCUCGGACUUCAGUUCC | 21 | 3995 |
| myoC-4250 | + | AGUUAGCUCGGACUUCAGUUCC | 22 | 3996 |
| myoC-4251 | + | CAGUUAGCUCGGACUUCAGUUCC | 23 | 3997 |
| myoC-4252 | + | UCAGUUAGCUCGGACUUCAGUUCC | 24 | 3998 |
| myoC-4253 | + | CUUCAAAAUUCGGGAAGC | 18 | 3999 |
| myoC-4254 | + | CCUUCAAAAUUCGGGAAGC | 19 | 4000 |
| myoC-329 | + | UCCUUCAAAAUUCGGGAAGC | 20 | 715 |
| myoC-4255 | + | CUCCUUCAAAAUUCGGGAAGC | 21 | 4001 |
| myoC-4256 | + | UCUCCUUCAAAAUUCGGGAAGC | 22 | 4002 |
| myoC-4257 | + | CUCUCCUUCAAAAUUCGGGAAGC | 23 | 4003 |
| myoC-4258 | + | GCUCUCCUUCAAAAUUCGGGAAGC | 24 | 4004 |
| myoC-4259 | + | GGAGCCUGGUGGCACAGC | 18 | 4005 |
| myoC-4260 | + | UGGAGCCUGGUGGCACAGC | 19 | 4006 |
| myoC-1701 | + | CUGGAGCCUGGUGGCACAGC | 20 | 1953 |
| myoC-4261 | + | UCUGGAGCCUGGUGGCACAGC | 21 | 4007 |
| myoC-4262 | + | CUCUGGAGCCUGGUGGCACAGC | 22 | 4008 |
| myoC-4263 | + | UCUCUGGAGCCUGGUGGCACAGC | 23 | 4009 |
| myoC-4264 | + | UUCUCUGGAGCCUGGUGGCACAGC | 24 | 4010 |
| myoC-4265 | + | UGCCAUUGCCUGUACAGC | 18 | 4011 |
| myoC-4266 | + | CUGCCAUUGCCUGUACAGC | 19 | 4012 |
| myoC-3030 | + | UCUGCCAUUGCCUGUACAGC | 20 | 2809 |
| myoC-4267 | + | UUCUGCCAUUGCCUGUACAGC | 21 | 4013 |
| myoC-4268 | + | CUUCUGCCAUUGCCUGUACAGC | 22 | 4014 |
| myoC-4269 | + | CCUUCUGCCAUUGCCUGUACAGC | 23 | 4015 |
| myoC-4270 | + | UCCUUCUGCCAUUGCCUGUACAGC | 24 | 4016 |
| myoC-4271 | + | GUUUCUGCUGUUCUCAGC | 18 | 4017 |
| myoC-4272 | + | UGUUUCUGCUGUUCUCAGC | 19 | 4018 |
| myoC-4273 | + | UUGUUUCUGCUGUUCUCAGC | 20 | 4019 |
| myoC-4274 | + | AUUGUUUCUGCUGUUCUCAGC | 21 | 4020 |
| myoC-4275 | + | AAUUGUUUCUGCUGUUCUCAGC | 22 | 4021 |
| myoC-4276 | + | UAAUUGUUUCUGCUGUUCUCAGC | 23 | 4022 |
| myoC-4277 | + | GUAAUUGUUUCUGCUGUUCUCAGC | 24 | 4023 |
| myoC-4278 | + | GCAGGAACUUCAGUUAGC | 18 | 4024 |
| myoC-4279 | + | AGCAGGAACUUCAGUUAGC | 19 | 4025 |
| myoC-4280 | + | AAGCAGGAACUUCAGUUAGC | 20 | 4026 |
| myoC-4281 | + | GAAGCAGGAACUUCAGUUAGC | 21 | 4027 |
| myoC-4282 | + | GGAAGCAGGAACUUCAGUUAGC | 22 | 4028 |
| myoC-4283 | + | GGGAAGCAGGAACUUCAGUUAGC | 23 | 4029 |
| myoC-4284 | + | CGGGAAGCAGGAACUUCAGUUAGC | 24 | 4030 |
| myoC-4285 | + | GUGUAGGGGUAGGUGGGC | 18 | 4031 |
| myoC-4286 | + | GGUGUAGGGGUAGGUGGGC | 19 | 4032 |
| myoC-4287 | + | GGGUGUAGGGGUAGGUGGGC | 20 | 4033 |
| myoC-4288 | + | UGGGUGUAGGGGUAGGUGGGC | 21 | 4034 |
| myoC-4289 | + | CUGGGUGUAGGGGUAGGUGGGC | 22 | 4035 |
| myoC-4290 | + | CCUGGGUGUAGGGGUAGGUGGGC | 23 | 4036 |
| myoC-4291 | + | UCCUGGGUGUAGGGGUAGGUGGGC | 24 | 4037 |
| myoC-4292 | + | CUUAUAUUCGAUGCUGGC | 18 | 4038 |
| myoC-4293 | + | ACUUAUAUUCGAUGCUGGC | 19 | 4039 |
| myoC-4294 | + | UACUUAUAUUCGAUGCUGGC | 20 | 4040 |
| myoC-4295 | + | UUACUUAUAUUCGAUGCUGGC | 21 | 4041 |
| myoC-4296 | + | CUUACUUAUAUUCGAUGCUGGC | 22 | 4042 |
| myoC-4297 | + | UCUUACUUAUAUUCGAUGCUGGC | 23 | 4043 |
| myoC-4298 | + | AUCUUACUUAUAUUCGAUGCUGGC | 24 | 4044 |
| myoC-4299 | + | GGUAACCAUGUAACAUGC | 18 | 4045 |
| myoC-4300 | + | UGGUAACCAUGUAACAUGC | 19 | 4046 |
| myoC-4301 | + | GUGGUAACCAUGUAACAUGC | 20 | 4047 |
| myoC-4302 | + | UGUGGUAACCAUGUAACAUGC | 21 | 4048 |
| myoC-4303 | + | UUGUGGUAACCAUGUAACAUGC | 22 | 4049 |
| myoC-4304 | + | CUUGUGGUAACCAUGUAACAUGC | 23 | 4050 |
| myoC-4305 | + | GCUUGUGGUAACCAUGUAACAUGC | 24 | 4051 |
| myoC-4306 | + | GAAAGCAGUCAAAGCUGC | 18 | 4052 |
| myoC-4307 | + | GGAAAGCAGUCAAAGCUGC | 19 | 4053 |
| myoC-3034 | + | UGGAAAGCAGUCAAAGCUGC | 20 | 2812 |
| myoC-4308 | + | UUGGAAAGCAGUCAAAGCUGC | 21 | 4054 |
| myoC-4309 | + | CUUGGAAAGCAGUCAAAGCUGC | 22 | 4055 |
| myoC-4310 | + | ACUUGGAAAGCAGUCAAAGCUGC | 23 | 4056 |
| myoC-4311 | + | AACUUGGAAAGCAGUCAAAGCUGC | 24 | 4057 |
| myoC-4312 | + | UUGGAGGCUUUUCACAUC | 18 | 4058 |
| myoC-4313 | + | CUUGGAGGCUUUUCACAUC | 19 | 4059 |
| myoC-2976 | + | GCUUGGAGGCUUUUCACAUC | 20 | 1860 |
| myoC-4314 | + | AGCUUGGAGGCUUUUCACAUC | 21 | 4060 |
| myoC-4315 | + | CAGCUUGGAGGCUUUUCACAUC | 22 | 4061 |
| myoC-4316 | + | ACAGCUUGGAGGCUUUUCACAUC | 23 | 4062 |
| myoC-4317 | + | UACAGCUUGGAGGCUUUUCACAUC | 24 | 4063 |
| myoC-4318 | + | GUGUCUCCCUCUCCACUC | 18 | 4064 |
| myoC-4319 | + | GGUGUCUCCCUCUCCACUC | 19 | 4065 |
| myoC-4320 | + | CGGUGUCUCCCUCUCCACUC | 20 | 4066 |
| myoC-4321 | + | CCGGUGUCUCCCUCUCCACUC | 21 | 4067 |
| myoC-4322 | + | ACCGGUGUCUCCCUCUCCACUC | 22 | 4068 |
| myoC-4323 | + | UACCGGUGUCUCCCUCUCCACUC | 23 | 4069 |
| myoC-4324 | + | AUACCGGUGUCUCCCUCUCCACUC | 24 | 4070 |
| myoC-4325 | + | GUCCGUGGUAGCCAGCUC | 18 | 4071 |
| myoC-4326 | + | UGUCCGUGGUAGCCAGCUC | 19 | 4072 |
| myoC-2924 | + | CUGUCCGUGGUAGCCAGCUC | 20 | 1822 |
| myoC-4327 | + | ACUGUCCGUGGUAGCCAGCUC | 21 | 4073 |
| myoC-4328 | + | AACUGUCCGUGGUAGCCAGCUC | 22 | 4074 |
| myoC-4329 | + | GAACUGUCCGUGGUAGCCAGCUC | 23 | 4075 |
| myoC-4330 | + | GGAACUGUCCGUGGUAGCCAGCUC | 24 | 4076 |
| myoC-4331 | + | CUGCAUUCUUACCUUCUC | 18 | 4077 |
| myoC-4332 | + | UCUGCAUUCUUACCUUCUC | 19 | 4078 |
| myoC-3184 | + | CUCUGCAUUCUUACCUUCUC | 20 | 2930 |
| myoC-4333 | + | ACUCUGCAUUCUUACCUUCUC | 21 | 4079 |
| myoC-4334 | + | CACUCUGCAUUCUUACCUUCUC | 22 | 4080 |
| myoC-4335 | + | CCACUCUGCAUUCUUACCUUCUC | 23 | 4081 |
| myoC-4336 | + | CCCACUCUGCAUUCUUACCUUCUC | 24 | 4082 |
| myoC-4337 | + | GGCAAAGAGCUUCUUCUC | 18 | 4083 |
| myoC-4338 | + | AGGCAAAGAGCUUCUUCUC | 19 | 4084 |
| myoC-2972 | + | CAGGCAAAGAGCUUCUUCUC | 20 | 1857 |
| myoC-4339 | + | CCAGGCAAAGAGCUUCUUCUC | 21 | 4085 |
| myoC-4340 | + | CCCAGGCAAAGAGCUUCUUCUC | 22 | 4086 |
| myoC-4341 | + | UCCCAGGCAAAGAGCUUCUUCUC | 23 | 4087 |
| myoC-4342 | + | GUCCCAGGCAAAGAGCUUCUUCUC | 24 | 4088 |
| myoC-4343 | + | CGAGCAGUGUCUCGGGUC | 18 | 4089 |
| myoC-4344 | + | CCGAGCAGUGUCUCGGGUC | 19 | 4090 |
| myoC-203 | + | CCCGAGCAGUGUCUCGGGUC | 20 | 589 |
| myoC-4345 | + | GCCCGAGCAGUGUCUCGGGUC | 21 | 4091 |
| myoC-4346 | + | AGCCCGAGCAGUGUCUCGGGUC | 22 | 4092 |
| myoC-4347 | + | CAGCCCGAGCAGUGUCUCGGGUC | 23 | 4093 |
| myoC-4348 | + | ACAGCCCGAGCAGUGUCUCGGGUC | 24 | 4094 |
| myoC-4349 | + | GGCUCUCCUUCAAAAUUC | 18 | 4095 |
| myoC-4350 | + | GGGCUCUCCUUCAAAAUUC | 19 | 4096 |
| myoC-328 | + | UGGGCUCUCCUUCAAAAUUC | 20 | 714 |
| myoC-4351 | + | AUGGGCUCUCCUUCAAAAUUC | 21 | 4097 |
| myoC-4352 | + | GAUGGGCUCUCCUUCAAAAUUC | 22 | 4098 |
| myoC-4353 | + | AGAUGGGCUCUCCUUCAAAAUUC | 23 | 4099 |
| myoC-4354 | + | CAGAUGGGCUCUCCUUCAAAAUUC | 24 | 4100 |
| myoC-4355 | + | UAGCUCGGACUUCAGUUC | 18 | 4101 |
| myoC-4356 | + | UUAGCUCGGACUUCAGUUC | 19 | 4102 |
| myoC-4357 | + | GUUAGCUCGGACUUCAGUUC | 20 | 4103 |
| myoC-4358 | + | AGUUAGCUCGGACUUCAGUUC | 21 | 4104 |
| myoC-4359 | + | CAGUUAGCUCGGACUUCAGUUC | 22 | 4105 |
| myoC-4360 | + | UCAGUUAGCUCGGACUUCAGUUC | 23 | 4106 |
| myoC-4361 | + | UUCAGUUAGCUCGGACUUCAGUUC | 24 | 4107 |
| myoC-4362 | + | GCAAGAGCAAUGGUUUUC | 18 | 4108 |
| myoC-4363 | + | UGCAAGAGCAAUGGUUUUC | 19 | 4109 |
| myoC-507 | + | AUGCAAGAGCAAUGGUUUUC | 20 | 849 |
| myoC-4364 | + | CAUGCAAGAGCAAUGGUUUUC | 21 | 4110 |
| myoC-4365 | + | ACAUGCAAGAGCAAUGGUUUUC | 22 | 4111 |
| myoC-4366 | + | AACAUGCAAGAGCAAUGGUUUUC | 23 | 4112 |
| myoC-4367 | + | UAACAUGCAAGAGCAAUGGUUUUC | 24 | 4113 |
| myoC-4368 | + | CCUUCAAAAUUCGGGAAG | 18 | 4114 |
| myoC-4369 | + | UCCUUCAAAAUUCGGGAAG | 19 | 4115 |
| myoC-4370 | + | CUCCUUCAAAAUUCGGGAAG | 20 | 4116 |
| myoC-4371 | + | UCUCCUUCAAAAUUCGGGAAG | 21 | 4117 |
| myoC-4372 | + | CUCUCCUUCAAAAUUCGGGAAG | 22 | 4118 |
| myoC-4373 | + | GCUCUCCUUCAAAAUUCGGGAAG | 23 | 4119 |
| myoC-4374 | + | GGCUCUCCUUCAAAAUUCGGGAAG | 24 | 4120 |
| myoC-4375 | + | CACUCCUGAGAUAGCCAG | 18 | 4121 |
| myoC-4376 | + | CCACUCCUGAGAUAGCCAG | 19 | 4122 |
| myoC-4377 | + | UCCACUCCUGAGAUAGCCAG | 20 | 4123 |
| myoC-4378 | + | CUCCACUCCUGAGAUAGCCAG | 21 | 4124 |
| myoC-4379 | + | UCUCCACUCCUGAGAUAGCCAG | 22 | 4125 |
| myoC-4380 | + | CUCUCCACUCCUGAGAUAGCCAG | 23 | 4126 |
| myoC-4381 | + | CCUCUCCACUCCUGAGAUAGCCAG | 24 | 4127 |
| myoC-4382 | + | AUAUUCGAUGCUGGCCAG | 18 | 4128 |
| myoC-4383 | + | UAUAUUCGAUGCUGGCCAG | 19 | 4129 |
| myoC-503 | + | UUAUAUUCGAUGCUGGCCAG | 20 | 845 |
| myoC-4384 | + | CUUAUAUUCGAUGCUGGCCAG | 21 | 4130 |
| myoC-4385 | + | ACUUAUAUUCGAUGCUGGCCAG | 22 | 4131 |
| myoC-4386 | + | UACUUAUAUUCGAUGCUGGCCAG | 23 | 4132 |
| myoC-4387 | + | UUACUUAUAUUCGAUGCUGGCCAG | 24 | 4133 |
| myoC-4388 | + | UCCUGGGUGUAGGGGUAG | 18 | 4134 |
| myoC-4389 | + | CUCCUGGGUGUAGGGGUAG | 19 | 4135 |
| myoC-4390 | + | UCUCCUGGGUGUAGGGGUAG | 20 | 4136 |
| myoC-4391 | + | GUCUCCUGGGUGUAGGGGUAG | 21 | 4137 |
| myoC-4392 | + | GGUCUCCUGGGUGUAGGGGUAG | 22 | 4138 |
| myoC-4393 | + | UGGUCUCCUGGGUGUAGGGGUAG | 23 | 4139 |
| myoC-4394 | + | GUGGUCUCCUGGGUGUAGGGGUAG | 24 | 4140 |
| myoC-4395 | + | AAGUGUCCAAAUUCCACG | 18 | 4141 |
| myoC-4396 | + | AAAGUGUCCAAAUUCCACG | 19 | 4142 |
| myoC-4397 | + | CAAAGUGUCCAAAUUCCACG | 20 | 4143 |
| myoC-4398 | + | CCAAAGUGUCCAAAUUCCACG | 21 | 4144 |
| myoC-4399 | + | GCCAAAGUGUCCAAAUUCCACG | 22 | 4145 |
| myoC-4400 | + | GGCCAAAGUGUCCAAAUUCCACG | 23 | 4146 |
| myoC-4401 | + | AGGCCAAAGUGUCCAAAUUCCACG | 24 | 4147 |
| myoC-4402 | + | UUCCCACAAAGUUCAAGG | 18 | 4148 |
| myoC-4403 | + | AUUCCCACAAAGUUCAAGG | 19 | 4149 |
| myoC-4404 | + | GAUUCCCACAAAGUUCAAGG | 20 | 4150 |
| myoC-4405 | + | AGAGCAAUGGUUUUCAGG | 18 | 4151 |
| myoC-4406 | + | AAGAGCAAUGGUUUUCAGG | 19 | 4152 |
| myoC-4407 | + | CAAGAGCAAUGGUUUUCAGG | 20 | 4153 |
| myoC-4408 | + | GCAAGAGCAAUGGUUUUCAGG | 21 | 4154 |
| myoC-4409 | + | UGCAAGAGCAAUGGUUUUCAGG | 22 | 4155 |
| myoC-4410 | + | AUGCAAGAGCAAUGGUUUUCAGG | 23 | 4156 |
| myoC-4411 | + | CAUGCAAGAGCAAUGGUUUUCAGG | 24 | 4157 |
| myoC-4412 | + | UACAAGGUGCCACAGAUG | 18 | 4158 |
| myoC-4413 | + | GUACAAGGUGCCACAGAUG | 19 | 4159 |
| myoC-3001 | + | UGUACAAGGUGCCACAGAUG | 20 | 2794 |
| myoC-4414 | + | GUGUACAAGGUGCCACAGAUG | 21 | 4160 |
| myoC-4415 | + | GGUGUACAAGGUGCCACAGAUG | 22 | 4161 |
| myoC-4416 | + | CGGUGUACAAGGUGCCACAGAUG | 23 | 4162 |
| myoC-4417 | + | ACGGUGUACAAGGUGCCACAGAUG | 24 | 4163 |
| myoC-4418 | + | GUCAUACUCAAAAACCUG | 18 | 4164 |
| myoC-4419 | + | GGUCAUACUCAAAAACCUG | 19 | 4165 |
| myoC-4420 | + | AGGUCAUACUCAAAAACCUG | 20 | 4166 |
| myoC-4421 | + | GAGGUCAUACUCAAAAACCUG | 21 | 4167 |
| myoC-4422 | + | UGAGGUCAUACUCAAAAACCUG | 22 | 4168 |
| myoC-4423 | + | AUGAGGUCAUACUCAAAAACCUG | 23 | 4169 |
| myoC-4424 | + | GAUGAGGUCAUACUCAAAAACCUG | 24 | 4170 |
| myoC-4425 | + | CCCUGCAUAAACUGGCUG | 18 | 4171 |
| myoC-4426 | + | GCCCUGCAUAAACUGGCUG | 19 | 4172 |
| myoC-4427 | + | AGCCCUGCAUAAACUGGCUG | 20 | 4173 |
| myoC-4428 | + | UAGCCCUGCAUAAACUGGCUG | 21 | 4174 |
| myoC-4429 | + | GUAGCCCUGCAUAAACUGGCUG | 22 | 4175 |
| myoC-4430 | + | GGUAGCCCUGCAUAAACUGGCUG | 23 | 4176 |
| myoC-4431 | + | GGGUAGCCCUGCAUAAACUGGCUG | 24 | 4177 |
| myoC-4432 | + | AGUUGACGGUAGCAUCUG | 18 | 4178 |
| myoC-4433 | + | AAGUUGACGGUAGCAUCUG | 19 | 4179 |
| myoC-2965 | + | AAAGUUGACGGUAGCAUCUG | 20 | 1853 |
| myoC-4434 | + | CAAAGUUGACGGUAGCAUCUG | 21 | 4180 |
| myoC-4435 | + | GCAAAGUUGACGGUAGCAUCUG | 22 | 4181 |
| myoC-4436 | + | AGCAAAGUUGACGGUAGCAUCUG | 23 | 4182 |
| myoC-4437 | + | AAGCAAAGUUGACGGUAGCAUCUG | 24 | 4183 |
| myoC-4438 | + | CACGUGGUCUCCUGGGUG | 18 | 4184 |
| myoC-4439 | + | CCACGUGGUCUCCUGGGUG | 19 | 4185 |
| myoC-4440 | + | UCCACGUGGUCUCCUGGGUG | 20 | 4186 |
| myoC-4441 | + | CUCCACGUGGUCUCCUGGGUG | 21 | 4187 |
| myoC-4442 | + | UCUCCACGUGGUCUCCUGGGUG | 22 | 4188 |
| myoC-4443 | + | UUCUCCACGUGGUCUCCUGGGUG | 23 | 4189 |
| myoC-4444 | + | AUUCUCCACGUGGUCUCCUGGGUG | 24 | 4190 |
| myoC-4445 | + | GAGGCUUUUCACAUCUUG | 18 | 4191 |
| myoC-4446 | + | GGAGGCUUUUCACAUCUUG | 19 | 4192 |
| myoC-2974 | + | UGGAGGCUUUUCACAUCUUG | 20 | 1859 |
| myoC-4447 | + | UUGGAGGCUUUUCACAUCUUG | 21 | 4193 |
| myoC-4448 | + | CUUGGAGGCUUUUCACAUCUUG | 22 | 4194 |
| myoC-4449 | + | GCUUGGAGGCUUUUCACAUCUUG | 23 | 4195 |
| myoC-4450 | + | AGCUUGGAGGCUUUUCACAUCUUG | 24 | 4196 |
| myoC-4451 | + | UUCUCUGGGUUCAGUUUG | 18 | 4197 |
| myoC-4452 | + | AUUCUCUGGGUUCAGUUUG | 19 | 4198 |
| myoC-4453 | + | GAUUCUCUGGGUUCAGUUUG | 20 | 4199 |
| myoC-4454 | + | AGAUUCUCUGGGUUCAGUUUG | 21 | 4200 |
| myoC-4455 | + | CAGAUUCUCUGGGUUCAGUUUG | 22 | 4201 |
| myoC-4456 | + | CCAGAUUCUCUGGGUUCAGUUUG | 23 | 4202 |
| myoC-4457 | + | UCCAGAUUCUCUGGGUUCAGUUUG | 24 | 4203 |
| myoC-4458 | + | UGGGCUCUCCUUCAAAAU | 18 | 4204 |
| myoC-4459 | + | AUGGGCUCUCCUUCAAAAU | 19 | 4205 |
| myoC-4460 | + | GAUGGGCUCUCCUUCAAAAU | 20 | 4206 |
| myoC-4461 | + | AGAUGGGCUCUCCUUCAAAAU | 21 | 4207 |
| myoC-4462 | + | CAGAUGGGCUCUCCUUCAAAAU | 22 | 4208 |
| myoC-4463 | + | CCAGAUGGGCUCUCCUUCAAAAU | 23 | 4209 |
| myoC-4464 | + | GCCAGAUGGGCUCUCCUUCAAAAU | 24 | 4210 |
| myoC-4465 | + | UGUAGCCACCCCAAGAAU | 18 | 4211 |
| myoC-4466 | + | GUGUAGCCACCCCAAGAAU | 19 | 4212 |
| myoC-2927 | + | CGUGUAGCCACCCCAAGAAU | 20 | 1823 |
| myoC-4467 | + | CCGUGUAGCCACCCCAAGAAU | 21 | 4213 |
| myoC-4468 | + | UCCGUGUAGCCACCCCAAGAAU | 22 | 4214 |
| myoC-4469 | + | GUCCGUGUAGCCACCCCAAGAAU | 23 | 4215 |
| myoC-4470 | + | UGUCCGUGUAGCCACCCCAAGAAU | 24 | 4216 |
| myoC-4471 | + | GUAUUCUAUCUGAAGCAU | 18 | 4217 |
| myoC-4472 | + | UGUAUUCUAUCUGAAGCAU | 19 | 4218 |
| myoC-4473 | + | CUGUAUUCUAUCUGAAGCAU | 20 | 4219 |
| myoC-4474 | + | ACUGUAUUCUAUCUGAAGCAU | 21 | 4220 |
| myoC-4475 | + | AACUGUAUUCUAUCUGAAGCAU | 22 | 4221 |
| myoC-4476 | + | CAACUGUAUUCUAUCUGAAGCAU | 23 | 4222 |
| myoC-4477 | + | CCAACUGUAUUCUAUCUGAAGCAU | 24 | 4223 |
| myoC-4478 | + | GACCCAACUGUAUUCUAU | 18 | 4224 |
| myoC-4479 | + | AGACCCAACUGUAUUCUAU | 19 | 4225 |
| myoC-4480 | + | GAGACCCAACUGUAUUCUAU | 20 | 4226 |
| myoC-4481 | + | UGAGACCCAACUGUAUUCUAU | 21 | 4227 |
| myoC-4482 | + | GUGAGACCCAACUGUAUUCUAU | 22 | 4228 |
| myoC-4483 | + | UGUGAGACCCAACUGUAUUCUAU | 23 | 4229 |
| myoC-4484 | + | AUGUGAGACCCAACUGUAUUCUAU | 24 | 4230 |
| myoC-4485 | + | CAGUGGCCUAGGCAGUAU | 18 | 4231 |
| myoC-4486 | + | CCAGUGGCCUAGGCAGUAU | 19 | 4232 |
| myoC-4487 | + | UCCAGUGGCCUAGGCAGUAU | 20 | 4233 |
| myoC-4488 | + | UUCCAGUGGCCUAGGCAGUAU | 21 | 4234 |
| myoC-4489 | + | UUUCCAGUGGCCUAGGCAGUAU | 22 | 4235 |
| myoC-4490 | + | CUUUCCAGUGGCCUAGGCAGUAU | 23 | 4236 |
| myoC-4491 | + | GCUUUCCAGUGGCCUAGGCAGUAU | 24 | 4237 |
| myoC-4492 | + | AUAAAGGAUAUUUAUUAU | 18 | 4238 |
| myoC-4493 | + | GAUAAAGGAUAUUUAUUAU | 19 | 4239 |
| myoC-4494 | + | AGAUAAAGGAUAUUUAUUAU | 20 | 4240 |
| myoC-4495 | + | AAGAUAAAGGAUAUUUAUUAU | 21 | 4241 |
| myoC-4496 | + | GAAGAUAAAGGAUAUUUAUUAU | 22 | 4242 |
| myoC-4497 | + | AGAAGAUAAAGGAUAUUUAUUAU | 23 | 4243 |
| myoC-4498 | + | CAGAAGAUAAAGGAUAUUUAUUAU | 24 | 4244 |
| myoC-4499 | + | CACAAUGUAAAGGGUUAU | 18 | 4245 |
| myoC-4500 | + | UCACAAUGUAAAGGGUUAU | 19 | 4246 |
| myoC-4501 | + | UUCACAAUGUAAAGGGUUAU | 20 | 4247 |
| myoC-4502 | + | UUUCACAAUGUAAAGGGUUAU | 21 | 4248 |
| myoC-4503 | + | AUUUCACAAUGUAAAGGGUUAU | 22 | 4249 |
| myoC-4504 | + | UAUUUCACAAUGUAAAGGGUUAU | 23 | 4250 |
| myoC-4505 | + | UUAUUUCACAAUGUAAAGGGUUAU | 24 | 4251 |
| myoC-4506 | + | UCUGGAUUAAUGAAAACU | 18 | 4252 |
| myoC-4507 | + | UUCUGGAUUAAUGAAAACU | 19 | 4253 |
| myoC-511 | + | CUUCUGGAUUAAUGAAAACU | 20 | 853 |
| myoC-4508 | + | CCUUCUGGAUUAAUGAAAACU | 21 | 4254 |
| myoC-4509 | + | UCCUUCUGGAUUAAUGAAAACU | 22 | 4255 |
| myoC-4510 | + | AUCCUUCUGGAUUAAUGAAAACU | 23 | 4256 |
| myoC-4511 | + | CAUCCUUCUGGAUUAAUGAAAACU | 24 | 4257 |
| myoC-4512 | + | UAGGCAGUAUGUGAACCU | 18 | 4258 |
| myoC-4513 | + | CUAGGCAGUAUGUGAACCU | 19 | 4259 |
| myoC-4514 | + | CCUAGGCAGUAUGUGAACCU | 20 | 4260 |
| myoC-4515 | + | GCCUAGGCAGUAUGUGAACCU | 21 | 4261 |
| myoC-4516 | + | GGCCUAGGCAGUAUGUGAACCU | 22 | 4262 |
| myoC-4517 | + | UGGCCUAGGCAGUAUGUGAACCU | 23 | 4263 |
| myoC-4518 | + | GUGGCCUAGGCAGUAUGUGAACCU | 24 | 4264 |
| myoC-4519 | + | GCCAUUGCCUGUACAGCU | 18 | 4265 |
| myoC-4520 | + | UGCCAUUGCCUGUACAGCU | 19 | 4266 |
| myoC-422 | + | CUGCCAUUGCCUGUACAGCU | 20 | 786 |
| myoC-4521 | + | UCUGCCAUUGCCUGUACAGCU | 21 | 4267 |
| myoC-4522 | + | UUCUGCCAUUGCCUGUACAGCU | 22 | 4268 |
| myoC-4523 | + | CUUCUGCCAUUGCCUGUACAGCU | 23 | 4269 |
| myoC-4524 | + | CCUUCUGCCAUUGCCUGUACAGCU | 24 | 4270 |
| myoC-4525 | + | UGGAGGCUUUUCACAUCU | 18 | 4271 |
| myoC-4526 | + | UUGGAGGCUUUUCACAUCU | 19 | 4272 |
| myoC-59 | + | CUUGGAGGCUUUUCACAUCU | 20 | 457 |
| myoC-4527 | + | GCUUGGAGGCUUUUCACAUCU | 21 | 4273 |
| myoC-4528 | + | AGCUUGGAGGCUUUUCACAUCU | 22 | 4274 |
| myoC-4529 | + | CAGCUUGGAGGCUUUUCACAUCU | 23 | 4275 |
| myoC-4530 | + | ACAGCUUGGAGGCUUUUCACAUCU | 24 | 4276 |
| myoC-4531 | + | GAGCAGUGUCUCGGGUCU | 18 | 4277 |
| myoC-4532 | + | CGAGCAGUGUCUCGGGUCU | 19 | 4278 |
| myoC-204 | + | CCGAGCAGUGUCUCGGGUCU | 20 | 590 |
| myoC-4533 | + | CCCGAGCAGUGUCUCGGGUCU | 21 | 4279 |
| myoC-4534 | + | GCCCGAGCAGUGUCUCGGGUCU | 22 | 4280 |
| myoC-4535 | + | AGCCCGAGCAGUGUCUCGGGUCU | 23 | 4281 |
| myoC-4536 | + | CAGCCCGAGCAGUGUCUCGGGUCU | 24 | 4282 |
| myoC-4537 | + | UCUGCAUUCUUACCUUCU | 18 | 4283 |
| myoC-4538 | + | CUCUGCAUUCUUACCUUCU | 19 | 4284 |
| myoC-4539 | + | ACUCUGCAUUCUUACCUUCU | 20 | 4285 |
| myoC-4540 | + | CACUCUGCAUUCUUACCUUCU | 21 | 4286 |
| myoC-4541 | + | CCACUCUGCAUUCUUACCUUCU | 22 | 4287 |
| myoC-4542 | + | CCCACUCUGCAUUCUUACCUUCU | 23 | 4288 |
| myoC-4543 | + | CCCCACUCUGCAUUCUUACCUUCU | 24 | 4289 |
| myoC-4544 | + | AGAUUCUCUGGGUUCAGU | 18 | 4290 |
| myoC-4545 | + | CAGAUUCUCUGGGUUCAGU | 19 | 4291 |
| myoC-4546 | + | CCAGAUUCUCUGGGUUCAGU | 20 | 4292 |
| myoC-4547 | + | UCCAGAUUCUCUGGGUUCAGU | 21 | 4293 |
| myoC-4548 | + | UUCCAGAUUCUCUGGGUUCAGU | 22 | 4294 |
| myoC-4549 | + | GUUCCAGAUUCUCUGGGUUCAGU | 23 | 4295 |
| myoC-4550 | + | AGUUCCAGAUUCUCUGGGUUCAGU | 24 | 4296 |
| myoC-4551 | + | UUCUGCUGUUCUCAGCGU | 18 | 4297 |
| myoC-4552 | + | UUUCUGCUGUUCUCAGCGU | 19 | 4298 |
| myoC-4553 | + | GUUUCUGCUGUUCUCAGCGU | 20 | 4299 |
| myoC-4554 | + | UGUUUCUGCUGUUCUCAGCGU | 21 | 4300 |
| myoC-4555 | + | UUGUUUCUGCUGUUCUCAGCGU | 22 | 4301 |
| myoC-4556 | + | AUUGUUUCUGCUGUUCUCAGCGU | 23 | 4302 |
| myoC-4557 | + | AAUUGUUUCUGCUGUUCUCAGCGU | 24 | 4303 |
| myoC-4558 | + | CCGAGCAGUGUCUCGGGU | 18 | 4304 |
| myoC-4559 | + | CCCGAGCAGUGUCUCGGGU | 19 | 4305 |
| myoC-1699 | + | GCCCGAGCAGUGUCUCGGGU | 20 | 1951 |
| myoC-4560 | + | AGCCCGAGCAGUGUCUCGGGU | 21 | 4306 |
| myoC-4561 | + | CAGCCCGAGCAGUGUCUCGGGU | 22 | 4307 |
| myoC-4562 | + | ACAGCCCGAGCAGUGUCUCGGGU | 23 | 4308 |
| myoC-4563 | + | CACAGCCCGAGCAGUGUCUCGGGU | 24 | 4309 |
| myoC-4564 | + | AGGAAGAGAACGUUGGGU | 18 | 4310 |
| myoC-4565 | + | AAGGAAGAGAACGUUGGGU | 19 | 4311 |
| myoC-4566 | + | CAAGGAAGAGAACGUUGGGU | 20 | 4312 |
| myoC-4567 | + | UCAAGGAAGAGAACGUUGGGU | 21 | 4313 |
| myoC-4568 | + | UUCAAGGAAGAGAACGUUGGGU | 22 | 4314 |
| myoC-4569 | + | GUUCAAGGAAGAGAACGUUGGGU | 23 | 4315 |
| myoC-4570 | + | AGUUCAAGGAAGAGAACGUUGGGU | 24 | 4316 |
| myoC-4571 | + | GGGCUCUCCUUCAAAAUU | 18 | 4317 |
| myoC-4572 | + | UGGGCUCUCCUUCAAAAUU | 19 | 4318 |
| myoC-327 | + | AUGGGCUCUCCUUCAAAAUU | 20 | 713 |
| myoC-4573 | + | GAUGGGCUCUCCUUCAAAAUU | 21 | 4319 |
| myoC-4574 | + | AGAUGGGCUCUCCUUCAAAAUU | 22 | 4320 |
| myoC-4575 | + | CAGAUGGGCUCUCCUUCAAAAUU | 23 | 4321 |
| myoC-4576 | + | CCAGAUGGGCUCUCCUUCAAAAUU | 24 | 4322 |
| myoC-4577 | + | UCCACAUCCUGGUAAAUU | 18 | 4323 |
| myoC-4578 | + | CUCCACAUCCUGGUAAAUU | 19 | 4324 |
| myoC-4579 | + | UCUCCACAUCCUGGUAAAUU | 20 | 4325 |
| myoC-4580 | + | UUCUCCACAUCCUGGUAAAUU | 21 | 4326 |
| myoC-4581 | + | GUUCUCCACAUCCUGGUAAAUU | 22 | 4327 |
| myoC-4582 | + | AGUUCUCCACAUCCUGGUAAAUU | 23 | 4328 |
| myoC-4583 | + | UAGUUCUCCACAUCCUGGUAAAUU | 24 | 4329 |
| myoC-4584 | + | GAGCUAUUCUGCUUCCUU | 18 | 4330 |
| myoC-4585 | + | GGAGCUAUUCUGCUUCCUU | 19 | 4331 |
| myoC-4586 | + | AGGAGCUAUUCUGCUUCCUU | 20 | 4332 |
| myoC-4587 | + | GAGGAGCUAUUCUGCUUCCUU | 21 | 4333 |
| myoC-4588 | + | AGAGGAGCUAUUCUGCUUCCUU | 22 | 4334 |
| myoC-4589 | + | CAGAGGAGCUAUUCUGCUUCCUU | 23 | 4335 |
| myoC-4590 | + | CCAGAGGAGCUAUUCUGCUUCCUU | 24 | 4336 |
| myoC-4591 | + | UCAUAUCUUAUGACAGUU | 18 | 4337 |
| myoC-4592 | + | CUCAUAUCUUAUGACAGUU | 19 | 4338 |
| myoC-2922 | + | GCUCAUAUCUUAUGACAGUU | 20 | 1821 |
| myoC-4593 | + | AGCUCAUAUCUUAUGACAGUU | 21 | 4339 |
| myoC-4594 | + | CAGCUCAUAUCUUAUGACAGUU | 22 | 4340 |
| myoC-4595 | + | UCAGCUCAUAUCUUAUGACAGUU | 23 | 4341 |
| myoC-4596 | + | UUCAGCUCAUAUCUUAUGACAGUU | 24 | 4342 |
| myoC-4597 | + | GAUUCUCUGGGUUCAGUU | 18 | 4343 |
| myoC-4598 | + | AGAUUCUCUGGGUUCAGUU | 19 | 4344 |
| myoC-446 | + | CAGAUUCUCUGGGUUCAGUU | 20 | 797 |
| myoC-4599 | + | CCAGAUUCUCUGGGUUCAGUU | 21 | 4345 |
| myoC-4600 | + | UCCAGAUUCUCUGGGUUCAGUU | 22 | 4346 |
| myoC-4601 | + | UUCCAGAUUCUCUGGGUUCAGUU | 23 | 4347 |
| myoC-4602 | + | GUUCCAGAUUCUCUGGGUUCAGUU | 24 | 4348 |
| myoC-4603 | + | UGCAAGAGCAAUGGUUUU | 18 | 4349 |
| myoC-4604 | + | AUGCAAGAGCAAUGGUUUU | 19 | 4350 |
| myoC-4605 | + | CAUGCAAGAGCAAUGGUUUU | 20 | 4351 |
| myoC-4606 | + | ACAUGCAAGAGCAAUGGUUUU | 21 | 4352 |
| myoC-4607 | + | AACAUGCAAGAGCAAUGGUUUU | 22 | 4353 |
| myoC-4608 | + | UAACAUGCAAGAGCAAUGGUUUU | 23 | 4354 |
| myoC-4609 | + | GUAACAUGCAAGAGCAAUGGUUUU | 24 | 4355 |
| myoC-4610 | - | GCCAUUGUCCUCUCCAAA | 18 | 4356 |
| myoC-4611 | - | UGCCAUUGUCCUCUCCAAA | 19 | 4357 |
| myoC-4612 | - | GUGCCAUUGUCCUCUCCAAA | 20 | 4358 |
| myoC-4613 | - | GGUGCCAUUGUCCUCUCCAAA | 21 | 4359 |
| myoC-4614 | - | AGGUGCCAUUGUCCUCUCCAAA | 22 | 4360 |
| myoC-4615 | - | AAGGUGCCAUUGUCCUCUCCAAA | 23 | 4361 |
| myoC-4616 | - | AAAGGUGCCAUUGUCCUCUCCAAA | 24 | 4362 |
| myoC-4617 | - | ACUUUGGCCUUCCAGGAA | 18 | 4363 |
| myoC-4618 | - | CACUUUGGCCUUCCAGGAA | 19 | 4364 |
| myoC-4619 | - | ACACUUUGGCCUUCCAGGAA | 20 | 4365 |
| myoC-4620 | - | GACACUUUGGCCUUCCAGGAA | 21 | 4366 |
| myoC-4621 | - | GGACACUUUGGCCUUCCAGGAA | 22 | 4367 |
| myoC-4622 | - | UGGACACUUUGGCCUUCCAGGAA | 23 | 4368 |
| myoC-4623 | - | UUGGACACUUUGGCCUUCCAGGAA | 24 | 4369 |
| myoC-4624 | - | UGGGGGGAGCAGGCUGAA | 18 | 4370 |
| myoC-4625 | - | CUGGGGGGAGCAGGCUGAA | 19 | 4371 |
| myoC-417 | - | CCUGGGGGGAGCAGGCUGAA | 20 | 781 |
| myoC-4626 | - | UCCUGGGGGGAGCAGGCUGAA | 21 | 4372 |
| myoC-4627 | - | CUCCUGGGGGGAGCAGGCUGAA | 22 | 4373 |
| myoC-4628 | - | GCUCCUGGGGGGAGCAGGCUGAA | 23 | 4374 |
| myoC-4629 | - | GGCUCCUGGGGGGAGCAGGCUGAA | 24 | 4375 |
| myoC-4630 | - | AACUGAAGUCCGAGCUAA | 18 | 4376 |
| myoC-4631 | - | GAACUGAAGUCCGAGCUAA | 19 | 4377 |
| myoC-4632 | - | GGAACUGAAGUCCGAGCUAA | 20 | 4378 |
| myoC-4633 | - | AGGAACUGAAGUCCGAGCUAA | 21 | 4379 |
| myoC-4634 | - | CAGGAACUGAAGUCCGAGCUAA | 22 | 4380 |
| myoC-4635 | - | CCAGGAACUGAAGUCCGAGCUAA | 23 | 4381 |
| myoC-4636 | - | UCCAGGAACUGAAGUCCGAGCUAA | 24 | 4382 |
| myoC-4637 | - | AAAAAGCAUAACUUCUAA | 18 | 4383 |
| myoC-4638 | - | UAAAAAGCAUAACUUCUAA | 19 | 4384 |
| myoC-495 | - | AUAAAAAGCAUAACUUCUAA | 20 | 837 |
| myoC-4639 | - | AAUAAAAAGCAUAACUUCUAA | 21 | 4385 |
| myoC-4640 | - | CAAUAAAAAGCAUAACUUCUAA | 22 | 4386 |
| myoC-4641 | - | ACAAUAAAAAGCAUAACUUCUAA | 23 | 4387 |
| myoC-4642 | - | CACAAUAAAAAGCAUAACUUCUAA | 24 | 4388 |
| myoC-4643 | - | GAGCUGAAUACCGAGACA | 18 | 4389 |
| myoC-4644 | - | UGAGCUGAAUACCGAGACA | 19 | 4390 |
| myoC-2907 | - | AUGAGCUGAAUACCGAGACA | 20 | 1809 |
| myoC-4645 | - | UAUGAGCUGAAUACCGAGACA | 21 | 4391 |
| myoC-4646 | - | AUAUGAGCUGAAUACCGAGACA | 22 | 4392 |
| myoC-4647 | - | GAUAUGAGCUGAAUACCGAGACA | 23 | 4393 |
| myoC-4648 | - | AGAUAUGAGCUGAAUACCGAGACA | 24 | 4394 |
| myoC-4649 | - | CACAUACUGCCUAGGCCA | 18 | 4395 |
| myoC-4650 | - | UCACAUACUGCCUAGGCCA | 19 | 4396 |
| myoC-4651 | - | UUCACAUACUGCCUAGGCCA | 20 | 4397 |
| myoC-4652 | - | GUUCACAUACUGCCUAGGCCA | 21 | 4398 |
| myoC-4653 | - | GGUUCACAUACUGCCUAGGCCA | 22 | 4399 |
| myoC-4654 | - | AGGUUCACAUACUGCCUAGGCCA | 23 | 4400 |
| myoC-4655 | - | AAGGUUCACAUACUGCCUAGGCCA | 24 | 4401 |
| myoC-4656 | - | CUGUGCCACCAGGCUCCA | 18 | 4402 |
| myoC-4657 | - | GCUGUGCCACCAGGCUCCA | 19 | 4403 |
| myoC-1662 | - | GGCUGUGCCACCAGGCUCCA | 20 | 1924 |
| myoC-4658 | - | GGGCUGUGCCACCAGGCUCCA | 21 | 4404 |
| myoC-4659 | - | CGGGCUGUGCCACCAGGCUCCA | 22 | 4405 |
| myoC-4660 | - | UCGGGCUGUGCCACCAGGCUCCA | 23 | 4406 |
| myoC-4661 | - | CUCGGGCUGUGCCACCAGGCUCCA | 24 | 4407 |
| myoC-4662 | - | UGUACAGGCAAUGGCAGA | 18 | 4408 |
| myoC-4663 | - | CUGUACAGGCAAUGGCAGA | 19 | 4409 |
| myoC-407 | - | GCUGUACAGGCAAUGGCAGA | 20 | 771 |
| myoC-4664 | - | AGCUGUACAGGCAAUGGCAGA | 21 | 4410 |
| myoC-4665 | - | AAGCUGUACAGGCAAUGGCAGA | 22 | 4411 |
| myoC-4666 | - | CAAGCUGUACAGGCAAUGGCAGA | 23 | 4412 |
| myoC-4667 | - | CCAAGCUGUACAGGCAAUGGCAGA | 24 | 4413 |
| myoC-4668 | - | AGAAGGUAAGAAUGCAGA | 18 | 4414 |
| myoC-4669 | - | GAGAAGGUAAGAAUGCAGA | 19 | 4415 |
| myoC-4670 | - | AGAGAAGGUAAGAAUGCAGA | 20 | 4416 |
| myoC-4671 | - | CAGAGAAGGUAAGAAUGCAGA | 21 | 4417 |
| myoC-4672 | - | CCAGAGAAGGUAAGAAUGCAGA | 22 | 4418 |
| myoC-4673 | - | UCCAGAGAAGGUAAGAAUGCAGA | 23 | 4419 |
| myoC-4674 | - | CUCCAGAGAAGGUAAGAAUGCAGA | 24 | 4420 |
| myoC-4675 | - | CUAUCUCAGGAGUGGAGA | 18 | 4421 |
| myoC-4676 | - | GCUAUCUCAGGAGUGGAGA | 19 | 4422 |
| myoC-322 | - | GGCUAUCUCAGGAGUGGAGA | 20 | 708 |
| myoC-4677 | - | UGGCUAUCUCAGGAGUGGAGA | 21 | 4423 |
| myoC-4678 | - | CUGGCUAUCUCAGGAGUGGAGA | 22 | 4424 |
| myoC-4679 | - | UCUGGCUAUCUCAGGAGUGGAGA | 23 | 4425 |
| myoC-4680 | - | AUCUGGCUAUCUCAGGAGUGGAGA | 24 | 4426 |
| myoC-4681 | - | GAGGUAGCAAGGCUGAGA | 18 | 4427 |
| myoC-4682 | - | GGAGGUAGCAAGGCUGAGA | 19 | 4428 |
| myoC-198 | - | AGGAGGUAGCAAGGCUGAGA | 20 | 584 |
| myoC-4683 | - | CAGGAGGUAGCAAGGCUGAGA | 21 | 4429 |
| myoC-4684 | - | CCAGGAGGUAGCAAGGCUGAGA | 22 | 4430 |
| myoC-4685 | - | GCCAGGAGGUAGCAAGGCUGAGA | 23 | 4431 |
| myoC-4686 | - | AGCCAGGAGGUAGCAAGGCUGAGA | 24 | 4432 |
| myoC-4687 | - | AGACAGUGAAGGCUGAGA | 18 | 4433 |
| myoC-4688 | - | GAGACAGUGAAGGCUGAGA | 19 | 4434 |
| myoC-2 | - | CGAGACAGUGAAGGCUGAGA | 20 | 405 |
| myoC-4689 | - | CCGAGACAGUGAAGGCUGAGA | 21 | 4435 |
| myoC-4690 | - | ACCGAGACAGUGAAGGCUGAGA | 22 | 4436 |
| myoC-4691 | - | UACCGAGACAGUGAAGGCUGAGA | 23 | 4437 |
| myoC-4692 | - | AUACCGAGACAGUGAAGGCUGAGA | 24 | 4438 |
| myoC-4693 | - | CAUCUGGCUAUCUCAGGA | 18 | 4439 |
| myoC-4694 | - | CCAUCUGGCUAUCUCAGGA | 19 | 4440 |
| myoC-4695 | - | CCCAUCUGGCUAUCUCAGGA | 20 | 4441 |
| myoC-4696 | - | GCCCAUCUGGCUAUCUCAGGA | 21 | 4442 |
| myoC-4697 | - | AGCCCAUCUGGCUAUCUCAGGA | 22 | 4443 |
| myoC-4698 | - | GAGCCCAUCUGGCUAUCUCAGGA | 23 | 4444 |
| myoC-4699 | - | AGAGCCCAUCUGGCUAUCUCAGGA | 24 | 4445 |
| myoC-4700 | - | GGCUAUCUCAGGAGUGGA | 18 | 4446 |
| myoC-4701 | - | UGGCUAUCUCAGGAGUGGA | 19 | 4447 |
| myoC-4702 | - | CUGGCUAUCUCAGGAGUGGA | 20 | 4448 |
| myoC-4703 | - | UCUGGCUAUCUCAGGAGUGGA | 21 | 4449 |
| myoC-4704 | - | AUCUGGCUAUCUCAGGAGUGGA | 22 | 4450 |
| myoC-4705 | - | CAUCUGGCUAUCUCAGGAGUGGA | 23 | 4451 |
| myoC-4706 | - | CCAUCUGGCUAUCUCAGGAGUGGA | 24 | 4452 |
| myoC-4707 | - | CUGGGGGGAGCAGGCUGA | 18 | 4453 |
| myoC-4708 | - | CCUGGGGGGAGCAGGCUGA | 19 | 4454 |
| myoC-416 | - | UCCUGGGGGGAGCAGGCUGA | 20 | 780 |
| myoC-4709 | - | CUCCUGGGGGGAGCAGGCUGA | 21 | 4455 |
| myoC-4710 | - | GCUCCUGGGGGGAGCAGGCUGA | 22 | 4456 |
| myoC-4711 | - | GGCUCCUGGGGGGAGCAGGCUGA | 23 | 4457 |
| myoC-4712 | - | GGGCUCCUGGGGGGAGCAGGCUGA | 24 | 4458 |
| myoC-4713 | - | CUGCUUCCCGAAUUUUGA | 18 | 4459 |
| myoC-4714 | - | CCUGCUUCCCGAAUUUUGA | 19 | 4460 |
| myoC-317 | - | UCCUGCUUCCCGAAUUUUGA | 20 | 703 |
| myoC-4715 | - | UUCCUGCUUCCCGAAUUUUGA | 21 | 4461 |
| myoC-4716 | - | GUUCCUGCUUCCCGAAUUUUGA | 22 | 4462 |
| myoC-4717 | - | AGUUCCUGCUUCCCGAAUUUUGA | 23 | 4463 |
| myoC-4718 | - | AAGUUCCUGCUUCCCGAAUUUUGA | 24 | 4464 |
| myoC-4719 | - | UAAGAUAUGAGCUGAAUA | 18 | 4465 |
| myoC-4720 | - | AUAAGAUAUGAGCUGAAUA | 19 | 4466 |
| myoC-2906 | - | CAUAAGAUAUGAGCUGAAUA | 20 | 1808 |
| myoC-4721 | - | UCAUAAGAUAUGAGCUGAAUA | 21 | 4467 |
| myoC-4722 | - | GUCAUAAGAUAUGAGCUGAAUA | 22 | 4468 |
| myoC-4723 | - | UGUCAUAAGAUAUGAGCUGAAUA | 23 | 4469 |
| myoC-4724 | - | CUGUCAUAAGAUAUGAGCUGAAUA | 24 | 4470 |
| myoC-4725 | - | UAAAAAGCAUAACUUCUA | 18 | 4471 |
| myoC-4726 | - | AUAAAAAGCAUAACUUCUA | 19 | 4472 |
| myoC-4727 | - | AAUAAAAAGCAUAACUUCUA | 20 | 4473 |
| myoC-4728 | - | CAAUAAAAAGCAUAACUUCUA | 21 | 4474 |
| myoC-4729 | - | ACAAUAAAAAGCAUAACUUCUA | 22 | 4475 |
| myoC-4730 | - | CACAAUAAAAAGCAUAACUUCUA | 23 | 4476 |
| myoC-4731 | - | CCACAAUAAAAAGCAUAACUUCUA | 24 | 4477 |
| myoC-4732 | - | UCUGGAACUCGAACAAAC | 18 | 4478 |
| myoC-4733 | - | AUCUGGAACUCGAACAAAC | 19 | 4479 |
| myoC-4734 | - | AAUCUGGAACUCGAACAAAC | 20 | 4480 |
| myoC-4735 | - | GAAUCUGGAACUCGAACAAAC | 21 | 4481 |
| myoC-4736 | - | AGAAUCUGGAACUCGAACAAAC | 22 | 4482 |
| myoC-4737 | - | GAGAAUCUGGAACUCGAACAAAC | 23 | 4483 |
| myoC-4738 | - | AGAGAAUCUGGAACUCGAACAAAC | 24 | 4484 |
| myoC-4739 | - | CUCUUUGCCUGGGACAAC | 18 | 4485 |
| myoC-4740 | - | GCUCUUUGCCUGGGACAAC | 19 | 4486 |
| myoC-2963 | - | AGCUCUUUGCCUGGGACAAC | 20 | 1851 |
| myoC-4741 | - | AAGCUCUUUGCCUGGGACAAC | 21 | 4487 |
| myoC-4742 | - | GAAGCUCUUUGCCUGGGACAAC | 22 | 4488 |
| myoC-4743 | - | AGAAGCUCUUUGCCUGGGACAAC | 23 | 4489 |
| myoC-4744 | - | AAGAAGCUCUUUGCCUGGGACAAC | 24 | 4490 |
| myoC-4745 | - | ACCCAGAGAAUCUGGAAC | 18 | 4491 |
| myoC-4746 | - | AACCCAGAGAAUCUGGAAC | 19 | 4492 |
| myoC-4747 | - | GAACCCAGAGAAUCUGGAAC | 20 | 4493 |
| myoC-4748 | - | UGAACCCAGAGAAUCUGGAAC | 21 | 4494 |
| myoC-4749 | - | CUGAACCCAGAGAAUCUGGAAC | 22 | 4495 |
| myoC-4750 | - | ACUGAACCCAGAGAAUCUGGAAC | 23 | 4496 |
| myoC-4751 | - | AACUGAACCCAGAGAAUCUGGAAC | 24 | 4497 |
| myoC-4752 | - | CUACACCCAGGAGACCAC | 18 | 4498 |
| myoC-4753 | - | CCUACACCCAGGAGACCAC | 19 | 4499 |
| myoC-4754 | - | CCCUACACCCAGGAGACCAC | 20 | 4500 |
| myoC-4755 | - | CCCCUACACCCAGGAGACCAC | 21 | 4501 |
| myoC-4756 | - | ACCCCUACACCCAGGAGACCAC | 22 | 4502 |
| myoC-4757 | - | UACCCCUACACCCAGGAGACCAC | 23 | 4503 |
| myoC-4758 | - | CUACCCCUACACCCAGGAGACCAC | 24 | 4504 |
| myoC-4759 | - | ACAUACUGCCUAGGCCAC | 18 | 4505 |
| myoC-4760 | - | CACAUACUGCCUAGGCCAC | 19 | 4506 |
| myoC-369 | - | UCACAUACUGCCUAGGCCAC | 20 | 755 |
| myoC-4761 | - | UUCACAUACUGCCUAGGCCAC | 21 | 4507 |
| myoC-4762 | - | GUUCACAUACUGCCUAGGCCAC | 22 | 4508 |
| myoC-4763 | - | GGUUCACAUACUGCCUAGGCCAC | 23 | 4509 |
| myoC-4764 | - | AGGUUCACAUACUGCCUAGGCCAC | 24 | 4510 |
| myoC-4765 | - | GGGCCAGUGUCCCCAGAC | 18 | 4511 |
| myoC-4766 | - | GGGGCCAGUGUCCCCAGAC | 19 | 4512 |
| myoC-1659 | - | AGGGGCCAGUGUCCCCAGAC | 20 | 1921 |
| myoC-4767 | - | AAGGGGCCAGUGUCCCCAGAC | 21 | 4513 |
| myoC-4768 | - | GAAGGGGCCAGUGUCCCCAGAC | 22 | 4514 |
| myoC-4769 | - | AGAAGGGGCCAGUGUCCCCAGAC | 23 | 4515 |
| myoC-4770 | - | GAGAAGGGGCCAGUGUCCCCAGAC | 24 | 4516 |
| myoC-4771 | - | UAUUCUUGGGGUGGCUAC | 18 | 4517 |
| myoC-4772 | - | GUAUUCUUGGGGUGGCUAC | 19 | 4518 |
| myoC-2917 | - | CGUAUUCUUGGGGUGGCUAC | 20 | 1816 |
| myoC-4773 | - | CCGUAUUCUUGGGGUGGCUAC | 21 | 4519 |
| myoC-4774 | - | CCCGUAUUCUUGGGGUGGCUAC | 22 | 4520 |
| myoC-4775 | - | UCCCGUAUUCUUGGGGUGGCUAC | 23 | 4521 |
| myoC-4776 | - | UUCCCGUAUUCUUGGGGUGGCUAC | 24 | 4522 |
| myoC-4777 | - | UUUUAAUGCAGUUUCUAC | 18 | 4523 |
| myoC-4778 | - | CUUUUAAUGCAGUUUCUAC | 19 | 4524 |
| myoC-4779 | - | UCUUUUAAUGCAGUUUCUAC | 20 | 4525 |
| myoC-4780 | - | UUCUUUUAAUGCAGUUUCUAC | 21 | 4526 |
| myoC-4781 | - | UUUCUUUUAAUGCAGUUUCUAC | 22 | 4527 |
| myoC-4782 | - | CUUUCUUUUAAUGCAGUUUCUAC | 23 | 4528 |
| myoC-4783 | - | UCUUUCUUUUAAUGCAGUUUCUAC | 24 | 4529 |
| myoC-4784 | - | ACGGGUGCUGUGGUGUAC | 18 | 4530 |
| myoC-4785 | - | CACGGGUGCUGUGGUGUAC | 19 | 4531 |
| myoC-4786 | - | GCACGGGUGCUGUGGUGUAC | 20 | 4532 |
| myoC-4787 | - | AGCACGGGUGCUGUGGUGUAC | 21 | 4533 |
| myoC-4788 | - | AAGCACGGGUGCUGUGGUGUAC | 22 | 4534 |
| myoC-4789 | - | AAAGCACGGGUGCUGUGGUGUAC | 23 | 4535 |
| myoC-4790 | - | GAAAGCACGGGUGCUGUGGUGUAC | 24 | 4536 |
| myoC-4791 | - | CUGGAACUCGAACAAACC | 18 | 4537 |
| myoC-4792 | - | UCUGGAACUCGAACAAACC | 19 | 4538 |
| myoC-396 | - | AUCUGGAACUCGAACAAACC | 20 | 766 |
| myoC-4793 | - | AAUCUGGAACUCGAACAAACC | 21 | 4539 |
| myoC-4794 | - | GAAUCUGGAACUCGAACAAACC | 22 | 4540 |
| myoC-4795 | - | AGAAUCUGGAACUCGAACAAACC | 23 | 4541 |
| myoC-4796 | - | GAGAAUCUGGAACUCGAACAAACC | 24 | 4542 |
| myoC-4797 | - | UCCUCUCCAAACUGAACC | 18 | 4543 |
| myoC-4798 | - | GUCCUCUCCAAACUGAACC | 19 | 4544 |
| myoC-4799 | - | UGUCCUCUCCAAACUGAACC | 20 | 4545 |
| myoC-4800 | - | UUGUCCUCUCCAAACUGAACC | 21 | 4546 |
| myoC-4801 | - | AUUGUCCUCUCCAAACUGAACC | 22 | 4547 |
| myoC-4802 | - | CAUUGUCCUCUCCAAACUGAACC | 23 | 4548 |
| myoC-4803 | - | CCAUUGUCCUCUCCAAACUGAACC | 24 | 4549 |
| myoC-4804 | - | CCCACCUACCCCUACACC | 18 | 4550 |
| myoC-4805 | - | GCCCACCUACCCCUACACC | 19 | 4551 |
| myoC-4806 | - | AGCCCACCUACCCCUACACC | 20 | 4552 |
| myoC-4807 | - | AAGCCCACCUACCCCU ACACC | 21 | 4553 |
| myoC-4808 | - | CAAGCCCACCUACCCCUACACC | 22 | 4554 |
| myoC-4809 | - | CCAAGCCCACCUACCCCUACACC | 23 | 4555 |
| myoC-4810 | - | CCCAAGCCCACCUACCCCUACACC | 24 | 4556 |
| myoC-4811 | - | UCCCUGGAGCUGGCUACC | 18 | 4557 |
| myoC-4812 | - | AUCCCUGGAGCUGGCUACC | 19 | 4558 |
| myoC-2914 | - | AAUCCCUGGAGCUGGCUACC | 20 | 1814 |
| myoC-4813 | - | AAAUCCCUGGAGCUGGCUACC | 21 | 4559 |
| myoC-4814 | - | GAAAUCCCUGGAGCUGGCUACC | 22 | 4560 |
| myoC-4815 | - | GGAAAUCCCUGGAGCUGGCUACC | 23 | 4561 |
| myoC-4816 | - | AGGAAAUCCCUGGAGCUGGCUACC | 24 | 4562 |
| myoC-4817 | - | CCACCUACCCCUACACCC | 18 | 4563 |
| myoC-4818 | - | CCCACCUACCCCUACACCC | 19 | 4564 |
| myoC-360 | - | GCCCACCUACCCCUACACCC | 20 | 746 |
| myoC-4819 | - | AGCCCACCUACCCCUACACCC | 21 | 4565 |
| myoC-4820 | - | AAGCCCACCUACCCCUACACCC | 22 | 4566 |
| myoC-4821 | - | CAAGCCCACCUACCCCUACACCC | 23 | 4567 |
| myoC-4822 | - | CCAAG CCCACCUACCCCUACACCC | 24 | 4568 |
| myoC-4823 | - | AUGAUUGACUACAACCCC | 18 | 4569 |
| myoC-4824 | - | CAUGAUUGACUACAACCCC | 19 | 4570 |
| myoC-2957 | - | GCAUGAUUGACUACAACCCC | 20 | 1847 |
| myoC-4825 | - | AGCAUGAUUGACUACAACCCC | 21 | 4571 |
| myoC-4826 | - | CAGCAUGAUUGACUACAACCCC | 22 | 4572 |
| myoC-4827 | - | GCAGCAUGAUUGACUACAACCCC | 23 | 4573 |
| myoC-4828 | - | AGCAGCAUGAUUGACUACAACCCC | 24 | 4574 |
| myoC-4829 | - | UGAUUGACUACAACCCCC | 18 | 4575 |
| myoC-4830 | - | AUGAUUGACUACAACCCCC | 19 | 4576 |
| myoC-55 | - | CAUGAUUGACUACAACCCCC | 20 | 454 |
| myoC-4831 | - | GCAUGAUUGACUACAACCCCC | 21 | 4577 |
| myoC-4832 | - | AGCAUGAUUGACUACAACCCCC | 22 | 4578 |
| myoC-4833 | - | CAGCAUGAUUGACUACAACCCCC | 23 | 4579 |
| myoC-4834 | - | GCAGCAUGAUUGACUACAACCCCC | 24 | 4580 |
| myoC-4835 | - | GGCUGAGAAGGAAAUCCC | 18 | 4581 |
| myoC-4836 | - | AGGCUGAGAAGGAAAUCCC | 19 | 4582 |
| myoC-3 | - | AAGGCUGAGAAGGAAAUCCC | 20 | 406 |
| myoC-4837 | - | GAAGGCUGAGAAGGAAAUCCC | 21 | 4583 |
| myoC-4838 | - | UGAAGGCUGAGAAGGAAAUCCC | 22 | 4584 |
| myoC-4839 | - | GUGAAGGCUGAGAAGGAAAUCCC | 23 | 4585 |
| myoC-4840 | - | AGUGAAGGCUGAGAAGGAAAUCCC | 24 | 4586 |
| myoC-3549 | - | GGUUGGAAAGCAGCAGCC | 18 | 3295 |
| myoC-3550 | - | AGGUUGGAAAGCAGCAGCC | 19 | 3296 |
| myoC-107 | - | GAGGUUGGAAAGCAGCAGCC | 20 | 511 |
| myoC-4841 | - | GAGAAGAAGCUCUUUGCC | 18 | 4587 |
| myoC-4842 | - | GGAGAAGAAGCUCUUUGCC | 19 | 4588 |
| myoC-56 | - | UGGAGAAGAAGCUCUUUGCC | 20 | 455 |
| myoC-4843 | - | CUGGAGAAGAAGCUCUUUGCC | 21 | 4589 |
| myoC-4844 | - | CCUGGAGAAGAAGCUCUUUGCC | 22 | 4590 |
| myoC-4845 | - | CCCUGGAGAAGAAGCUCUUUGCC | 23 | 4591 |
| myoC-4846 | - | CCCCUGGAGAAGAAGCUCUUUGCC | 24 | 4592 |
| myoC-4847 | - | AGGCUGAGAAGGAAAUCC | 18 | 4593 |
| myoC-4848 | - | AAGGCUGAGAAGGAAAUCC | 19 | 4594 |
| myoC-2912 | - | GAAGGCUGAGAAGGAAAUCC | 20 | 1813 |
| myoC-4849 | - | UGAAGGCUGAGAAGGAAAUCC | 21 | 4595 |
| myoC-4850 | - | GUGAAGGCUGAGAAGGAAAUCC | 22 | 4596 |
| myoC-4851 | - | AGUGAAGGCUGAGAAGGAAAUCC | 23 | 4597 |
| myoC-4852 | - | CAGUGAAGGCUGAGAAGGAAAUCC | 24 | 4598 |
| myoC-4853 | - | GGAGAUGCUCAGGGCUCC | 18 | 4599 |
| myoC-4854 | - | AGGAGAUGCUCAGGGCUCC | 19 | 4600 |
| myoC-410 | - | AAGGAGAUGCUCAGGGCUCC | 20 | 774 |
| myoC-4855 | - | GAAGGAGAUGCUCAGGGCUCC | 21 | 4601 |
| myoC-4856 | - | AGAAGGAGAUGCUCAGGGCUCC | 22 | 4602 |
| myoC-4857 | - | CAGAAGGAGAUGCUCAGGGCUCC | 23 | 4603 |
| myoC-4858 | - | GCAGAAGGAGAUGCUCAGGGCUCC | 24 | 4604 |
| myoC-4859 | - | UGGACACUUUGGCCUUCC | 18 | 4605 |
| myoC-4860 | - | UUGGACACUUUGGCCUUCC | 19 | 4606 |
| myoC-316 | - | UUUGGACACUUUGGCCUUCC | 20 | 702 |
| myoC-4861 | - | AUUUGGACACUUUGGCCUUCC | 21 | 4607 |
| myoC-4862 | - | AAUUUGGACACUUUGGCCUUCC | 22 | 4608 |
| myoC-4863 | - | GAAUUUGGACACUUUGGCCUUCC | 23 | 4609 |
| myoC-4864 | - | GGAAUUUGGACACUUUGGCCUUCC | 24 | 4610 |
| myoC-4865 | - | UACCCAACGUUCUCUUCC | 18 | 4611 |
| myoC-4866 | - | UUACCCAACGUUCUCUUCC | 19 | 4612 |
| myoC-4867 | - | CUUACCCAACGUUCUCUUCC | 20 | 4613 |
| myoC-4868 | - | UCUUACCCAACGUUCUCUUCC | 21 | 4614 |
| myoC-4869 | - | UUCUUACCCAACGUUCUCUUCC | 22 | 4615 |
| myoC-4870 | - | UUUCUUACCCAACGUUCUCUUCC | 23 | 4616 |
| myoC-4871 | - | UUUUCUUACCCAACGUUCUCUUCC | 24 | 4617 |
| myoC-4872 | - | AAGGGAGAGCCAGCCAGC | 18 | 4618 |
| myoC-4873 | - | GAAGGGAGAGCCAGCCAGC | 19 | 4619 |
| myoC-3018 | - | UGAAGGGAGAGCCAGCCAGC | 20 | 2802 |
| myoC-4874 | - | CUGAAGGGAGAGCCAGCCAGC | 21 | 4620 |
| myoC-4875 | - | GCUGAAGGGAGAGCCAGCCAGC | 22 | 4621 |
| myoC-4876 | - | GGCUGAAGGGAGAGCCAGCCAGC | 23 | 4622 |
| myoC-4877 | - | AGGCUGAAGGGAGAGCCAGCCAGC | 24 | 4623 |
| myoC-3579 | - | AGGUUGGAAAGCAGCAGC | 18 | 3325 |
| myoC-3580 | - | GAGGUUGGAAAGCAGCAGC | 19 | 3326 |
| myoC-1653 | - | GGAGGUUGGAAAGCAGCAGC | 20 | 1917 |
| myoC-4878 | - | CCAGACCCGAGACACUGC | 18 | 4624 |
| myoC-4879 | - | CCCAGACCCGAGACACUGC | 19 | 4625 |
| myoC-1660 | - | CCCCAGACCCGAGACACUGC | 20 | 1922 |
| myoC-4880 | - | UCCCCAGACCCGAGACACUGC | 21 | 4626 |
| myoC-4881 | - | GUCCCCAGACCCGAGACACUGC | 22 | 4627 |
| myoC-4882 | - | UGUCCCCAGACCCGAGACACUGC | 23 | 4628 |
| myoC-4883 | - | GUGUCCCCAGACCCGAGACACUGC | 24 | 4629 |
| myoC-4884 | - | GGAGAAGAAGCUCUUUGC | 18 | 4630 |
| myoC-4885 | - | UGGAGAAGAAGCUCUUUGC | 19 | 4631 |
| myoC-2961 | - | CUGGAGAAGAAGCUCUUUGC | 20 | 1850 |
| myoC-4886 | - | CCUGGAGAAGAAGCUCUUUGC | 21 | 4632 |
| myoC-4887 | - | CCCUGGAGAAGAAGCUCUUUGC | 22 | 4633 |
| myoC-4888 | - | CCCCUGGAGAAGAAGCUCUUUGC | 23 | 4634 |
| myoC-4889 | - | CCCCCUGGAGAAGAAGCUCUUUGC | 24 | 4635 |
| myoC-4890 | - | AACUGAACCCAGAGAAUC | 18 | 4636 |
| myoC-4891 | - | AAACUGAACCCAGAGAAUC | 19 | 4637 |
| myoC-395 | - | CAAACUGAACCCAGAGAAUC | 20 | 765 |
| myoC-4892 | - | CCAAACUGAACCCAGAGAAUC | 21 | 4638 |
| myoC-4893 | - | UCCAAACUGAACCCAGAGAAUC | 22 | 4639 |
| myoC-4894 | - | CUCCAAACUGAACCCAGAGAAUC | 23 | 4640 |
| myoC-4895 | - | UCUCCAAACUGAACCCAGAGAAUC | 24 | 4641 |
| myoC-4896 | - | UCCAAGUUUUCAUUAAUC | 18 | 4642 |
| myoC-4897 | - | UUCCAAGUUUUCAUUAAUC | 19 | 4643 |
| myoC-3019 | - | UUUCCAAGUUUUCAUUAAUC | 20 | 2803 |
| myoC-4898 | - | CUUUCCAAGUUUUCAUUAAUC | 21 | 4644 |
| myoC-4899 | - | GCUUUCCAAGUUUUCAUUAAUC | 22 | 4645 |
| myoC-4900 | - | UGCUUUCCAAGUUUUCAUUAAUC | 23 | 4646 |
| myoC-4901 | - | CUGCUUUCCAAGUUUUCAUUAAUC | 24 | 4647 |
| myoC-4902 | - | GGGUGCUGUGGUGUACUC | 18 | 4648 |
| myoC-4903 | - | CGGGUGCUGUGGUGUACUC | 19 | 4649 |
| myoC-374 | - | ACGGGUGCUGUGGUGUACUC | 20 | 760 |
| myoC-4904 | - | CACGGGUGCUGUGGUGUACUC | 21 | 4650 |
| myoC-4905 | - | GCACGGGUGCUGUGGUGUACUC | 22 | 4651 |
| myoC-4906 | - | AGCACGGGUGCUGUGGUGUACUC | 23 | 4652 |
| myoC-4907 | - | AAGCACGGGUGCUGUGGUGUACUC | 24 | 4653 |
| myoC-4908 | - | GGCUGUGCCACCAGGCUC | 18 | 4654 |
| myoC-4909 | - | GGGCUGUGCCACCAGGCUC | 19 | 4655 |
| myoC-1661 | - | CGGGCUGUGCCACCAGGCUC | 20 | 1923 |
| myoC-4910 | - | UCGGGCUGUGCCACCAGGCUC | 21 | 4656 |
| myoC-4911 | - | CUCGGGCUGUGCCACCAGGCUC | 22 | 4657 |
| myoC-4912 | - | GCUCGGGCUGUGCCACCAGGCUC | 23 | 4658 |
| myoC-4913 | - | UGCUCGGGCUGUGCCACCAGGCUC | 24 | 4659 |
| myoC-4914 | - | AGGAGAUGCUCAGGGCUC | 18 | 4660 |
| myoC-4915 | - | AAGGAGAUGCUCAGGGCUC | 19 | 4661 |
| myoC-3007 | - | GAAGGAGAUGCUCAGGGCUC | 20 | 2798 |
| myoC-4916 | - | AGAAGGAGAUGCUCAGGGCUC | 21 | 4662 |
| myoC-4917 | - | CAGAAGGAGAUGCUCAGGGCUC | 22 | 4663 |
| myoC-4918 | - | GCAGAAGGAGAUGCUCAGGGCUC | 23 | 4664 |
| myoC-4919 | - | GGCAGAAGGAGAUGCUCAGGGCUC | 24 | 4665 |
| myoC-4920 | - | UUUCCAGGGCGCUGAGUC | 18 | 4666 |
| myoC-4921 | - | AUUUCCAGGGCGCUGAGUC | 19 | 4667 |
| myoC-4922 | - | UAUUUCCAGGGCGCUGAGUC | 20 | 4668 |
| myoC-4923 | - | CUAUUUCCAGGGCGCUGAGUC | 21 | 4669 |
| myoC-4924 | - | UCUAUUUCCAGGGCGCUGAGUC | 22 | 4670 |
| myoC-4925 | - | CUCUAUUUCCAGGGCGCUGAGUC | 23 | 4671 |
| myoC-4926 | - | CCUCUAUUUCCAGGGCGCUGAGUC | 24 | 4672 |
| myoC-4927 | - | ACCCUGACCAUCCCAUUC | 18 | 4673 |
| myoC-4928 | - | GACCCUGACCAUCCCAU UC | 19 | 4674 |
| myoC-2956 | - | AGACCCUGACCAUCCCAUUC | 20 | 1846 |
| myoC-4929 | - | AAGACCCUGACCAUCCCAUUC | 21 | 4675 |
| myoC-4930 | - | CAAGACCCUGACCAUCCCAUUC | 22 | 4676 |
| myoC-4931 | - | GCAAGACCCUGACCAUCCCAUUC | 23 | 4677 |
| myoC-4932 | - | AGCAAGACCCUGACCAUCCCAUUC | 24 | 4678 |
| myoC-4933 | - | CGGACAGUUCCCGUAUUC | 18 | 4679 |
| myoC-4934 | - | ACGGACAGUUCCCGUAUUC | 19 | 4680 |
| myoC-2915 | - | CACGGACAGUUCCCGUAUUC | 20 | 1815 |
| myoC-4935 | - | CCACGGACAGUUCCCGUAUUC | 21 | 4681 |
| myoC-4936 | - | ACCACGGACAGUUCCCGUAUUC | 22 | 4682 |
| myoC-4937 | - | UACCACGGACAGUUCCCGUAUUC | 23 | 4683 |
| myoC-4938 | - | CUACCACGGACAGUUCCCGUAUUC | 24 | 4684 |
| myoC-4939 | - | UUGGACACUUUGGCCUUC | 18 | 4685 |
| myoC-4940 | - | UUUGGACACUUUGGCCUUC | 19 | 4686 |
| myoC-4941 | - | AUUUGGACACUUUGGCCUUC | 20 | 4687 |
| myoC-4942 | - | AAUUUGGACACUUUGGCCUUC | 21 | 4688 |
| myoC-4943 | - | GAAUUUGGACACUUUGGCCUUC | 22 | 4689 |
| myoC-4944 | - | GGAAUUUGGACACUUUGGCCUUC | 23 | 4690 |
| myoC-4945 | - | UGGAAUUUGGACACUUUGGCCUUC | 24 | 4691 |
| myoC-4946 | - | AGGCAUAAUAGUUUCUUC | 18 | 4692 |
| myoC-4947 | - | AAGGCAUAAUAGUUUCUUC | 19 | 4693 |
| myoC-4948 | - | UAAGGCAUAAUAGUUUCUUC | 20 | 4694 |
| myoC-4949 | - | GUAAGGCAUAAUAGUUUCUUC | 21 | 4695 |
| myoC-4950 | - | UGUAAGGCAUAAUAGUUUCUUC | 22 | 4696 |
| myoC-4951 | - | CUGUAAGGCAUAAUAGUUUCUUC | 23 | 4697 |
| myoC-4952 | - | GCUGUAAGGCAUAAUAGUUUCUUC | 24 | 4698 |
| myoC-4953 | - | UCGGGGAGCCUCUAUUUC | 18 | 4699 |
| myoC-4954 | - | CUCGGGGAGCCUCUAUUUC | 19 | 4700 |
| myoC-4955 | - | ACUCGGGGAGCCUCUAUUUC | 20 | 4701 |
| myoC-4956 | - | UACUCGGGGAGCCUCUAUUUC | 21 | 4702 |
| myoC-4957 | - | GUACUCGGGGAGCCUCUAUUUC | 22 | 4703 |
| myoC-4958 | - | UGUACUCGGGGAGCCUCUAUUUC | 23 | 4704 |
| myoC-4959 | - | GUGUACUCGGGGAGCCUCUAUUUC | 24 | 4705 |
| myoC-4960 | - | GCUUCCCGAAUUUUGAAG | 18 | 4706 |
| myoC-4961 | - | UGCUUCCCGAAUUUUGAAG | 19 | 4707 |
| myoC-4962 | - | CUGCUUCCCGAAUUUUGAAG | 20 | 4708 |
| myoC-4963 | - | CCUGCUUCCCGAAUUUUGAAG | 21 | 4709 |
| myoC-4964 | - | UCCUGCUUCCCGAAUUUUGAAG | 22 | 4710 |
| myoC-4965 | - | UUCCUGCUUCCCGAAUUUUGAAG | 23 | 4711 |
| myoC-4966 | - | GUUCCUGCUUCCCGAAUUUUGAAG | 24 | 4712 |
| myoC-4967 | - | CUCUCACGCUGAGAACAG | 18 | 4713 |
| myoC-4968 | - | CCUCUCACGCUGAGAACAG | 19 | 4714 |
| myoC-4969 | - | GCCUCUCACGCUGAGAACAG | 20 | 4715 |
| myoC-4970 | - | AGCCUCUCACGCUGAGAACAG | 21 | 4716 |
| myoC-4971 | - | GAGCCUCUCACGCUGAGAACAG | 22 | 4717 |
| myoC-4972 | - | AGAGCCUCUCACGCUGAGAACAG | 23 | 4718 |
| myoC-4973 | - | GAGAGCCUCUCACGCUGAGAACAG | 24 | 4719 |
| myoC-4974 | - | CUGUACAGGCAAUGGCAG | 18 | 4720 |
| myoC-4975 | - | GCUGUACAGGCAAUGGCAG | 19 | 4721 |
| myoC-3004 | - | AGCUGUACAGGCAAUGGCAG | 20 | 2796 |
| myoC-4976 | - | AAGCUGUACAGGCAAUGGCAG | 21 | 4722 |
| myoC-4977 | - | CAAGCUGUACAGGCAAUGGCAG | 22 | 4723 |
| myoC-4978 | - | CCAAGCUGUACAGGCAAUGGCAG | 23 | 4724 |
| myoC-4979 | - | UCCAAGCUGUACAGGCAAUGGCAG | 24 | 4725 |
| myoC-4980 | - | GAAGGUAAGAAUGCAGAG | 18 | 4726 |
| myoC-4981 | - | AGAAGGUAAGAAUGCAGAG | 19 | 4727 |
| myoC-3185 | - | GAGAAGGUAAGAAUGCAGAG | 20 | 2931 |
| myoC-4982 | - | AGAGAAGGUAAGAAUGCAGAG | 21 | 4728 |
| myoC-4983 | - | CAGAGAAGGUAAGAAUGCAGAG | 22 | 4729 |
| myoC-4984 | - | CCAGAGAAGGUAAGAAUGCAGAG | 23 | 4730 |
| myoC-4985 | - | UCCAGAGAAGGUAAGAAUGCAGAG | 24 | 4731 |
| myoC-4986 | - | AUCUGGCUAUCUCAGGAG | 18 | 4732 |
| myoC-4987 | - | CAUCUGGCUAUCUCAGGAG | 19 | 4733 |
| myoC-320 | - | CCAUCUGGCUAUCUCAGGAG | 20 | 706 |
| myoC-4988 | - | CCCAUCUGGCUAUCUCAGGAG | 21 | 4734 |
| myoC-4989 | - | GCCCAUCUGGCUAUCUCAGGAG | 22 | 4735 |
| myoC-4990 | - | AGCCCAUCUGGCUAUCUCAGGAG | 23 | 4736 |
| myoC-4991 | - | GAGCCCAUCUGGCUAUCUCAGGAG | 24 | 4737 |
| myoC-4992 | - | GACUACAACCCCCUGGAG | 18 | 4738 |
| myoC-4993 | - | UGACUACAACCCCCUGGAG | 19 | 4739 |
| myoC-2960 | - | UUGACUACAACCCCCUGGAG | 20 | 1849 |
| myoC-4994 | - | AUUGACUACAACCCCCUGGAG | 21 | 4740 |
| myoC-4995 | - | GAUUGACUACAACCCCCUGGAG | 22 | 4741 |
| myoC-4996 | - | UGAUUGACUACAACCCCCUGGAG | 23 | 4742 |
| myoC-4997 | - | AUGAUUGACUACAACCCCCUGGAG | 24 | 4743 |
| myoC-4998 | - | GCUAUCUCAGGAGUGGAG | 18 | 4744 |
| myoC-4999 | - | GGCUAUCUCAGGAGUGGAG | 19 | 4745 |
| myoC-321 | - | UGGCUAUCUCAGGAGUGGAG | 20 | 707 |
| myoC-5000 | - | CUGGCUAUCUCAGGAGUGGAG | 21 | 4746 |
| myoC-5001 | - | UCUGGCUAUCUCAGGAGUGGAG | 22 | 4747 |
| myoC-5002 | - | AUCUGGCUAUCUCAGGAGUGGAG | 23 | 4748 |
| myoC-5003 | - | CAUCUGGCUAUCUCAGGAGUGGAG | 24 | 4749 |
| myoC-5004 | - | GGAGGUAGCAAGGCUGAG | 18 | 4750 |
| myoC-5005 | - | AGGAGGUAGCAAGGCUGAG | 19 | 4751 |
| myoC-1657 | - | CAGGAGGUAGCAAGGCUGAG | 20 | 1920 |
| myoC-5006 | - | CCAGGAGGUAGCAAGGCUGAG | 21 | 4752 |
| myoC-5007 | - | GCCAGGAGGUAGCAAGGCUGAG | 22 | 4753 |
| myoC-5008 | - | AGCCAGGAGGUAGCAAGGCUGAG | 23 | 4754 |
| myoC-5009 | - | CAGCCAGGAGGUAGCAAGGCUGAG | 24 | 4755 |
| myoC-5010 | - | GAGACAGUGAAGGCUGAG | 18 | 4756 |
| myoC-5011 | - | CGAGACAGUGAAGGCUGAG | 19 | 4757 |
| myoC-2910 | - | CCGAGACAGUGAAGGCUGAG | 20 | 1812 |
| myoC-5012 | - | ACCGAGACAGUGAAGGCUGAG | 21 | 4758 |
| myoC-5013 | - | UACCGAGACAGUGAAGGCUGAG | 22 | 4759 |
| myoC-5014 | - | AUACCGAGACAGUGAAGGCUGAG | 23 | 4760 |
| myoC-5015 | - | AAUACCGAGACAGUGAAGGCUGAG | 24 | 4761 |
| myoC-5016 | - | GAGAACUAGUUUGGGUAG | 18 | 4762 |
| myoC-5017 | - | GGAGAACUAGUUUGGGUAG | 19 | 4763 |
| myoC-5018 | - | UGGAGAACUAGUUUGGGUAG | 20 | 4764 |
| myoC-5019 | - | GUGGAGAACUAGUUUGGGUAG | 21 | 4765 |
| myoC-5020 | - | UGUGGAGAACUAGUUUGGGUAG | 22 | 4766 |
| myoC-5021 | - | AUGUGGAGAACUAGUUUGGGUAG | 23 | 4767 |
| myoC-5022 | - | GAUGUGGAGAACUAGUUUGGGUAG | 24 | 4768 |
| myoC-5023 | - | UACACCCAGGAGACCACG | 18 | 4769 |
| myoC-5024 | - | CUACACCCAGGAGACCACG | 19 | 4770 |
| myoC-361 | - | CCUACACCCAGGAGACCACG | 20 | 747 |
| myoC-5025 | - | CCCUACACCCAGGAGACCACG | 21 | 4771 |
| myoC-5026 | - | CCCCUACACCCAGGAGACCACG | 22 | 4772 |
| myoC-5027 | - | ACCCCUACACCCAGGAGACCACG | 23 | 4773 |
| myoC-5028 | - | UACCCCUACACCCAGGAGACCACG | 24 | 4774 |
| myoC-5029 | - | GUAGGAGAGCCUCUCACG | 18 | 4775 |
| myoC-5030 | - | GGUAGGAGAGCCUCUCACG | 19 | 4776 |
| myoC-5031 | - | GGGUAGGAGAGCCUCUCACG | 20 | 4777 |
| myoC-5032 | - | UGGGUAGGAGAGCCUCUCACG | 21 | 4778 |
| myoC-5033 | - | UUGGGUAGGAGAGCCUCUCACG | 22 | 4779 |
| myoC-5034 | - | UUUGGGUAGGAGAGCCUCUCACG | 23 | 4780 |
| myoC-5035 | - | GUUUGGGUAGGAGAGCCUCUCACG | 24 | 4781 |
| myoC-5036 | - | GGGUCAUUUACAGCACCG | 18 | 4782 |
| myoC-5037 | - | UGGGUCAUUUACAGCACCG | 19 | 4783 |
| myoC-2921 | - | CUGGGUCAUUUACAGCACCG | 20 | 1820 |
| myoC-5038 | - | UCUGGGUCAUUUACAGCACCG | 21 | 4784 |
| myoC-5039 | - | CUCUGGGUCAUUUACAGCACCG | 22 | 4785 |
| myoC-5040 | - | CCUCUGGGUCAUUUACAGCACCG | 23 | 4786 |
| myoC-5041 | - | GCCUCUGGGUCAUUUACAGCACCG | 24 | 4787 |
| myoC-5042 | - | GGUGCUGUGGUGUACUCG | 18 | 4788 |
| myoC-5043 | - | GGGUGCUGUGGUGUACUCG | 19 | 4789 |
| myoC-375 | - | CGGGUGCUGUGGUGUACUCG | 20 | 761 |
| myoC-5044 | - | ACGGGUGCUGUGGUGUACUCG | 21 | 4790 |
| myoC-5045 | - | CACGGGUGCUGUGGUGUACUCG | 22 | 4791 |
| myoC-5046 | - | GCACGGGUGCUGUGGUGUACUCG | 23 | 4792 |
| myoC-5047 | - | AGCACGGGUGCUGUGGUGUACUCG | 24 | 4793 |
| myoC-5048 | - | AGCCAGGAGGUAGCAAGG | 18 | 4794 |
| myoC-5049 | - | CAGCCAGGAGGUAGCAAGG | 19 | 4795 |
| myoC-1655 | - | GCAGCCAGGAGGUAGCAAGG | 20 | 1918 |
| myoC-5050 | - | AGCAGCCAGGAGGUAGCAAGG | 21 | 4796 |
| myoC-5051 | - | CAGCAGCCAGGAGGUAGCAAGG | 22 | 4797 |
| myoC-5052 | - | GCAGCAGCCAGGAGGUAGCAAGG | 23 | 4798 |
| myoC-5053 | - | AGCAGCAGCCAGGAGGUAGCAAGG | 24 | 4799 |
| myoC-5054 | - | UUUCAUUAAUCCAGAAGG | 18 | 4800 |
| myoC-5055 | - | UUUUCAUUAAUCCAGAAGG | 19 | 4801 |
| myoC-3021 | - | GUUUUCAUUAAUCCAGAAGG | 20 | 2805 |
| myoC-5056 | - | AGUUUUCAUUAAUCCAGAAGG | 21 | 4802 |
| myoC-5057 | - | AAGUUUUCAUUAAUCCAGAAGG | 22 | 4803 |
| myoC-5058 | - | CAAGUUUUCAUUAAUCCAGAAGG | 23 | 4804 |
| myoC-5059 | - | CCAAGUUUUCAUUAAUCCAGAAGG | 24 | 4805 |
| myoC-5060 | - | GGGGGAGCAGGCUGAAGG | 18 | 4806 |
| myoC-5061 | - | GGGGGGAGCAGGCUGAAGG | 19 | 4807 |
| myoC-3017 | - | UGGGGGGAGCAGGCUGAAGG | 20 | 2801 |
| myoC-5062 | - | CUGGGGGGAGCAGGCUGAAGG | 21 | 4808 |
| myoC-5063 | - | CCUGGGGGGAGCAGGCUGAAGG | 22 | 4809 |
| myoC-5064 | - | UCCUGGGGGGAGCAGGCUGAAGG | 23 | 4810 |
| myoC-5065 | - | CUCCUGGGGGGAGCAGGCUGAAGG | 24 | 4811 |
| myoC-5066 | - | AUACCGAGACAGUGAAGG | 18 | 4812 |
| myoC-5067 | - | AAUACCGAGACAGUGAAGG | 19 | 4813 |
| myoC-2908 | - | GAAUACCGAGACAGUGAAGG | 20 | 1810 |
| myoC-5068 | - | UGAAUACCGAGACAGUGAAGG | 21 | 4814 |
| myoC-5069 | - | CUGAAUACCGAGACAGUGAAGG | 22 | 4815 |
| myoC-5070 | - | GCUGAAUACCGAGACAGUGAAGG | 23 | 4816 |
| myoC-5071 | - | AGCUGAAUACCGAGACAGUGAAGG | 24 | 4817 |
| myoC-5072 | - | GCUCCUGGGGGGAGCAGG | 18 | 4818 |
| myoC-5073 | - | GGCUCCUGGGGGGAGCAGG | 19 | 4819 |
| myoC-3013 | - | GGGCUCCUGGGGGGAGCAGG | 20 | 2799 |
| myoC-5074 | - | AGGGCUCCUGGGGGGAGCAGG | 21 | 4820 |
| myoC-5075 | - | CAGGGCUCCUGGGGGGAGCAGG | 22 | 4821 |
| myoC-5076 | - | UCAGGGCUCCUGGGGGGAGCAGG | 23 | 4822 |
| myoC-5077 | - | CUCAGGGCUCCUGGGGGGAGCAGG | 24 | 4823 |
| myoC-5078 | - | AUGCUCAGGGCUCCUGGG | 18 | 4824 |
| myoC-5079 | - | GAUGCUCAGGGCUCCUGGG | 19 | 4825 |
| myoC-414 | - | AGAUGCUCAGGGCUCCUGGG | 20 | 778 |
| myoC-5080 | - | GAGAUGCUCAGGGCUCCUGGG | 21 | 4826 |
| myoC-5081 | - | GGAGAUGCUCAGGGCUCCUGGG | 22 | 4827 |
| myoC-5082 | - | AGGAGAUGCUCAGGGCUCCUGGG | 23 | 4828 |
| myoC-5083 | - | AAGGAGAUGCUCAGGGCUCCUGGG | 24 | 4829 |
| myoC-5084 | - | GUGGAGAACUAGUUUGGG | 18 | 4830 |
| myoC-5085 | - | UGUGGAGAACUAGUUUGGG | 19 | 4831 |
| myoC-5086 | - | AUGUGGAGAACUAGUUUGGG | 20 | 4832 |
| myoC-5087 | - | GAUGUGGAGAACUAGUUUGGG | 21 | 4833 |
| myoC-5088 | - | GGAUGUGGAGAACUAGUUUGGG | 22 | 4834 |
| myoC-5089 | - | AGGAUGUGGAGAACUAGUUUGGG | 23 | 4835 |
| myoC-5090 | - | CAGGAUGUGGAGAACUAGUUUGGG | 24 | 4836 |
| myoC-5091 | - | AAGCUGUACAGGCAAUGG | 18 | 4837 |
| myoC-5092 | - | CAAGCUGUACAGGCAAUGG | 19 | 4838 |
| myoC-3003 | - | CCAAGCUGUACAGGCAAUGG | 20 | 2795 |
| myoC-5093 | - | UCCAAGCUGUACAGGCAAUGG | 21 | 4839 |
| myoC-5094 | - | CUCCAAGCUGUACAGGCAAUGG | 22 | 4840 |
| myoC-5095 | - | CCUCCAAGCUGUACAGGCAAUGG | 23 | 4841 |
| myoC-5096 | - | GCCUCCAAGCUGUACAGGCAAUGG | 24 | 4842 |
| myoC-5097 | - | GAUGCUCAGGGCUCCUGG | 18 | 4843 |
| myoC-5098 | - | AGAUGCUCAGGGCUCCUGG | 19 | 4844 |
| myoC-413 | - | GAGAUGCUCAGGGCUCCUGG | 20 | 777 |
| myoC-5099 | - | GGAGAUGCUCAGGGCUCCUGG | 21 | 4845 |
| myoC-5100 | - | AGGAGAUGCUCAGGGCUCCUGG | 22 | 4846 |
| myoC-5101 | - | AAGGAGAUGCUCAGGGCUCCUGG | 23 | 4847 |
| myoC-5102 | - | GAAGGAGAUGCUCAGGGCUCCUGG | 24 | 4848 |
| myoC-5103 | - | GGUAAGAAUGCAGAGUGG | 18 | 4849 |
| myoC-5104 | - | AGGUAAGAAUGCAGAGUGG | 19 | 4850 |
| myoC-3188 | - | AAGGUAAGAAUGCAGAGUGG | 20 | 2934 |
| myoC-5105 | - | GAAGGUAAGAAUGCAGAGUGG | 21 | 4851 |
| myoC-5106 | - | AGAAGGUAAGAAUGCAGAGUGG | 22 | 4852 |
| myoC-5107 | - | GAGAAGGUAAGAAUGCAGAGUGG | 23 | 4853 |
| myoC-5108 | - | AGAGAAGGUAAGAAUGCAGAGUGG | 24 | 4854 |
| myoC-5109 | - | ACAUUGACUUGGCUGUGG | 18 | 4855 |
| myoC-5110 | - | GACAUUGACUUGGCUGUGG | 19 | 4856 |
| myoC-2919 | - | GGACAUUGACUUGGCUGUGG | 20 | 1818 |
| myoC-5111 | - | CGGACAUUGACUUGGCUGUGG | 21 | 4857 |
| myoC-5112 | - | ACGGACAUUGACUUGGCUGUGG | 22 | 4858 |
| myoC-5113 | - | CACGGACAUUGACUUGGCUGUGG | 23 | 4859 |
| myoC-5114 | - | ACACGGACAUUGACUUGGCUGUGG | 24 | 4860 |
| myoC-5115 | - | UCUGAAUUUACCAGGAUG | 18 | 4861 |
| myoC-5116 | - | UUCUGAAUUUACCAGGAUG | 19 | 4862 |
| myoC-353 | - | UUUCUGAAUUUACCAGGAUG | 20 | 739 |
| myoC-5117 | - | UUUUCUGAAUUUACCAGGAUG | 21 | 4863 |
| myoC-5118 | - | CUUUUCUGAAUUUACCAGGAUG | 22 | 4864 |
| myoC-5119 | - | UCUUUUCUGAAUUUACCAGGAUG | 23 | 4865 |
| myoC-5120 | - | UUCUUUUCUGAAUUUACCAGGAUG | 24 | 4866 |
| myoC-5121 | - | CUCAUCAGCCAGUUUAUG | 18 | 4867 |
| myoC-5122 | - | CCUCAUCAGCCAGUUUAUG | 19 | 4868 |
| myoC-5123 | - | ACCUCAUCAGCCAGUUUAUG | 20 | 4869 |
| myoC-5124 | - | GACCUCAUCAGCCAGUUUAUG | 21 | 4870 |
| myoC-5125 | - | UGACCUCAUCAGCCAGUUUAUG | 22 | 4871 |
| myoC-5126 | - | AUGACCUCAUCAGCCAGUUUAUG | 23 | 4872 |
| myoC-5127 | - | UAUGACCUCAUCAGCCAGUUUAUG | 24 | 4873 |
| myoC-5128 | - | AUUGACUACAACCCCCUG | 18 | 4874 |
| myoC-5129 | - | GAUUGACUACAACCCCCUG | 19 | 4875 |
| myoC-2959 | - | UGAUUGACUACAACCCCCUG | 20 | 1848 |
| myoC-5130 | - | AUGAUUGACUACAACCCCCUG | 21 | 4876 |
| myoC-5131 | - | CAUGAUUGACUACAACCCCCUG | 22 | 4877 |
| myoC-5132 | - | GCAUGAUUGACUACAACCCCCUG | 23 | 4878 |
| myoC-5133 | - | AGCAUGAUUGACUACAACCCCCUG | 24 | 4879 |
| myoC-5134 | - | AGAUGCUCAGGGCUCCUG | 18 | 4880 |
| myoC-5135 | - | GAGAUGCUCAGGGCUCCUG | 19 | 4881 |
| myoC-412 | - | GGAGAUGCUCAGGGCUCCUG | 20 | 776 |
| myoC-5136 | - | AGGAGAUGCUCAGGGCUCCUG | 21 | 4882 |
| myoC-5137 | - | AAGGAGAUGCUCAGGGCUCCUG | 22 | 4883 |
| myoC-5138 | - | GAAGGAGAUGCUCAGGGCUCCUG | 23 | 4884 |
| myoC-5139 | - | AGAAGGAGAUGCUCAGGGCUCCUG | 24 | 4885 |
| myoC-5140 | - | CCUGGGGGGAGCAGGCUG | 18 | 4886 |
| myoC-5141 | - | UCCUGGGGGGAGCAGGCUG | 19 | 4887 |
| myoC-3014 | - | CUCCUGGGGGGAGCAGGCUG | 20 | 2800 |
| myoC-5142 | - | GCUCCUGGGGGGAGCAGGCUG | 21 | 4888 |
| myoC-5143 | - | GGCUCCUGGGGGGAGCAGGCUG | 22 | 4889 |
| myoC-5144 | - | GGGCUCCUGGGGGGAGCAGGCUG | 23 | 4890 |
| myoC-5145 | - | AGGGCUCCUGGGGGGAGCAGGCUG | 24 | 4891 |
| myoC-5146 | - | AGGUAAGAAUGCAGAGUG | 18 | 4892 |
| myoC-5147 | - | AAGGUAAGAAUGCAGAGUG | 19 | 4893 |
| myoC-3189 | - | GAAGGUAAGAAUGCAGAGUG | 20 | 2935 |
| myoC-5148 | - | AGAAGGUAAGAAUGCAGAGUG | 21 | 4894 |
| myoC-5149 | - | GAGAAGGUAAGAAUGCAGAGUG | 22 | 4895 |
| myoC-5150 | - | AGAGAAGGUAAGAAUGCAGAGUG | 23 | 4896 |
| myoC-5151 | - | CAGAGAAGGUAAGAAUGCAGAGUG | 24 | 4897 |
| myoC-5152 | - | CUGGCUAUCUCAGGAGUG | 18 | 4898 |
| myoC-5153 | - | UCUGGCUAUCUCAGGAGUG | 19 | 4899 |
| myoC-5154 | - | AUCUGGCUAUCUCAGGAGUG | 20 | 4900 |
| myoC-5155 | - | CAUCUGGCUAUCUCAGGAGUG | 21 | 4901 |
| myoC-5156 | - | CCAUCUGGCUAUCUCAGGAGUG | 22 | 4902 |
| myoC-5157 | - | CCCAUCUGGCUAUCUCAGGAGUG | 23 | 4903 |
| myoC-5158 | - | GCCCAUCUGGCUAUCUCAGGAGUG | 24 | 4904 |
| myoC-5159 | - | UGAAUUUACCAGGAUGUG | 18 | 4905 |
| myoC-5160 | - | CUGAAUUUACCAGGAUGUG | 19 | 4906 |
| myoC-5161 | - | UCUGAAUUUACCAGGAUGUG | 20 | 4907 |
| myoC-5162 | - | UUCUGAAUUUACCAGGAUGUG | 21 | 4908 |
| myoC-5163 | - | UUUCUGAAUUUACCAGGAUGUG | 22 | 4909 |
| myoC-5164 | - | UUUUCUGAAUUUACCAGGAUGUG | 23 | 4910 |
| myoC-5165 | - | CUUUUCUGAAUUUACCAGGAUGUG | 24 | 4911 |
| myoC-5166 | - | UCUCUUCCUUGAACUUUG | 18 | 4912 |
| myoC-5167 | - | UUCUCUUCCUUGAACUUUG | 19 | 4913 |
| myoC-3190 | - | GUUCUCUUCCUUGAACUUUG | 20 | 2936 |
| myoC-5168 | - | CGUUCUCUUCCUUGAACUUUG | 21 | 4914 |
| myoC-5169 | - | ACGUUCUCUUCCUUGAACUUUG | 22 | 4915 |
| myoC-5170 | - | AACGUUCUCUUCCUUGAACUUUG | 23 | 4916 |
| myoC-5171 | - | CAACGUUCUCUUCCUUGAACUUUG | 24 | 4917 |
| myoC-5172 | - | CCUGCUUCCCGAAUUUUG | 18 | 4918 |
| myoC-5173 | - | UCCUGCUUCCCGAAUUUUG | 19 | 4919 |
| myoC-5174 | - | UUCCUGCUUCCCGAAUUUUG | 20 | 4920 |
| myoC-5175 | - | GUUCCUGCUUCCCGAAUUUUG | 21 | 4921 |
| myoC-5176 | - | AGUUCCUGCUUCCCGAAUUUUG | 22 | 4922 |
| myoC-5177 | - | AAGUUCCUGCUUCCCGAAUUUUG | 23 | 4923 |
| myoC-5178 | - | GAAGUUCCUGCUUCCCGAAUUUUG | 24 | 4924 |
| myoC-5179 | - | AAACUGAACCCAGAGAAU | 18 | 4925 |
| myoC-5180 | - | CAAACUGAACCCAGAGAAU | 19 | 4926 |
| myoC-5181 | - | CCAAACUGAACCCAGAGAAU | 20 | 4927 |
| myoC-5182 | - | UCCAAACUGAACCCAGAGAAU | 21 | 4928 |
| myoC-5183 | - | CUCCAAACUGAACCCAGAGAAU | 22 | 4929 |
| myoC-5184 | - | UCUCCAAACUGAACCCAGAGAAU | 23 | 4930 |
| myoC-5185 | - | CUCUCCAAACUGAACCCAGAGAAU | 24 | 4931 |
| myoC-5186 | - | GCAGUUUCUACGUGGAAU | 18 | 4932 |
| myoC-5187 | - | UGCAGUUUCUACGUGGAAU | 19 | 4933 |
| myoC-5188 | - | AUGCAGUUUCUACGUGGAAU | 20 | 4934 |
| myoC-5189 | - | AAUGCAGUUUCUACGUGGAAU | 21 | 4935 |
| myoC-5190 | - | UAAUGCAGUUUCUACGUGGAAU | 22 | 4936 |
| myoC-5191 | - | UUAAUGCAGUUUCUACGUGGAAU | 23 | 4937 |
| myoC-5192 | - | UUUAAUGCAGUUUCUACGUGGAAU | 24 | 4938 |
| myoC-5193 | - | CAUCAAGCUCUCCAAGAU | 18 | 4939 |
| myoC-5194 | - | ACAUCAAGCUCUCCAAGAU | 19 | 4940 |
| myoC-2964 | - | GACAUCAAGCUCUCCAAGAU | 20 | 1852 |
| myoC-5195 | - | UGACAUCAAGCUCUCCAAGAU | 21 | 4941 |
| myoC-5196 | - | AUGACAUCAAGCUCUCCAAGAU | 22 | 4942 |
| myoC-5197 | - | UAUGACAUCAAGCUCUCCAAGAU | 23 | 4943 |
| myoC-5198 | - | UUAUGACAUCAAGCUCUCCAAGAU | 24 | 4944 |
| myoC-5199 | - | CCAGAACUGUCAUAAGAU | 18 | 4945 |
| myoC-5200 | - | UCCAGAACUGUCAUAAGAU | 19 | 4946 |
| myoC-2904 | - | GUCCAGAACUGUCAUAAGAU | 20 | 1806 |
| myoC-5201 | - | AGUCCAGAACUGUCAUAAGAU | 21 | 4947 |
| myoC-5202 | - | GAGUCCAGAACUGUCAUAAGAU | 22 | 4948 |
| myoC-5203 | - | UGAGUCCAGAACUGUCAUAAGAU | 23 | 4949 |
| myoC-5204 | - | CUGAGUCCAGAACUGUCAUAAGAU | 24 | 4950 |
| myoC-5205 | - | UUCUGAAUUUACCAGGAU | 18 | 4951 |
| myoC-5206 | - | UUUCUGAAUUUACCAGGAU | 19 | 4952 |
| myoC-5207 | - | UUUUCUGAAUUUACCAGGAU | 20 | 4953 |
| myoC-5208 | - | CUUUUCUGAAUUUACCAGGAU | 21 | 4954 |
| myoC-5209 | - | UCUUUUCUGAAUUUACCAGGAU | 22 | 4955 |
| myoC-5210 | - | UUCUUUUCUGAAUUUACCAGGAU | 23 | 4956 |
| myoC-5211 | - | UUUCUUUUCUGAAUUUACCAGGAU | 24 | 4957 |
| myoC-5212 | - | CAAGUAUGGUGUGUGGAU | 18 | 4958 |
| myoC-5213 | - | GCAAGUAUGGUGUGUGGAU | 19 | 4959 |
| myoC-5214 | - | GGCAAGUAUGGUGUGUGGAU | 20 | 4960 |
| myoC-5215 | - | UGGCAAGUAUGGUGUGUGGAU | 21 | 4961 |
| myoC-5216 | - | CUGGCAAGUAUGGUGUGUGGAU | 22 | 4962 |
| myoC-5217 | - | ACUGGCAAGUAUGGUGUGUGGAU | 23 | 4963 |
| myoC-5218 | - | UACUGGCAAGUAUGGUGUGUGGAU | 24 | 4964 |
| myoC-5219 | - | UUCAAGUUUUCUUGUGAU | 18 | 4965 |
| myoC-5220 | - | GUUCAAGUUUUCUUGUGAU | 19 | 4966 |
| myoC-5221 | - | AGUUCAAGUUUUCUUGUGAU | 20 | 4967 |
| myoC-5222 | - | UAGUUCAAGUUUUCUUGUGAU | 21 | 4968 |
| myoC-5223 | - | AUAGUUCAAGUUUUCUUGUGAU | 22 | 4969 |
| myoC-5224 | - | CAUAGUUCAAGUUUUCUUGUGAU | 23 | 4970 |
| myoC-5225 | - | ACAUAGUUCAAGUUUUCUUGUGAU | 24 | 4971 |
| myoC-5226 | - | CGGGUGCUGUGGUGUACU | 18 | 4972 |
| myoC-5227 | - | ACGGGUGCUGUGGUGUACU | 19 | 4973 |
| myoC-373 | - | CACGGGUGCUGUGGUGUACU | 20 | 759 |
| myoC-5228 | - | GCACGGGUGCUGUGGUGUACU | 21 | 4974 |
| myoC-5229 | - | AGCACGGGUGCUGUGGUGUACU | 22 | 4975 |
| myoC-5230 | - | AAGCACGGGUGCUGUGGUGUACU | 23 | 4976 |
| myoC-5231 | - | AAAGCACGGGUGCUGUGGUGUACU | 24 | 4977 |
| myoC-5232 | - | UGGAACUCGAACAAACCU | 18 | 4978 |
| myoC-5233 | - | CUGGAACUCGAACAAACCU | 19 | 4979 |
| myoC-397 | - | UCUGGAACUCGAACAAACCU | 20 | 767 |
| myoC-5234 | - | AUCUGGAACUCGAACAAACCU | 21 | 4980 |
| myoC-5235 | - | AAUCUGGAACUCGAACAAACCU | 22 | 4981 |
| myoC-5236 | - | GAAUCUGGAACUCGAACAAACCU | 23 | 4982 |
| myoC-5237 | - | AGAAUCUGGAACUCGAACAAACCU | 24 | 4983 |
| myoC-5238 | - | GAGAUGCUCAGGGCUCCU | 18 | 4984 |
| myoC-5239 | - | GGAGAUGCUCAGGGCUCCU | 19 | 4985 |
| myoC-411 | - | AGGAGAUGCUCAGGGCUCCU | 20 | 775 |
| myoC-5240 | - | AAGGAGAUGCUCAGGGCUCCU | 21 | 4986 |
| myoC-5241 | - | GAAGGAGAUGCUCAGGGCUCCU | 22 | 4987 |
| myoC-5242 | - | AGAAGGAGAUGCUCAGGGCUCCU | 23 | 4988 |
| myoC-5243 | - | CAGAAGGAGAUGCUCAGGGCUCCU | 24 | 4989 |
| myoC-5244 | - | AGGAGAGCCUCUCACGCU | 18 | 4990 |
| myoC-5245 | - | UAGGAGAGCCUCUCACGCU | 19 | 4991 |
| myoC-5246 | - | GUAGGAGAGCCUCUCACGCU | 20 | 4992 |
| myoC-5247 | - | GGUAGGAGAGCCUCUCACGCU | 21 | 4993 |
| myoC-5248 | - | GGGUAGGAGAGCCUCUCACGCU | 22 | 4994 |
| myoC-5249 | - | UGGGUAGGAGAGCCUCUCACGCU | 23 | 4995 |
| myoC-5250 | - | UUGGGUAGGAGAGCCUCUCACGCU | 24 | 4996 |
| myoC-5251 | - | CCAGGAGGUAGCAAGGCU | 18 | 4997 |
| myoC-5252 | - | GCCAGGAGGUAGCAAGGCU | 19 | 4998 |
| myoC-1656 | - | AGCCAGGAGGUAGCAAGGCU | 20 | 1919 |
| myoC-5253 | - | CAGCCAGGAGGUAGCAAGGCU | 21 | 4999 |
| myoC-5254 | - | GCAGCCAGGAGGUAGCAAGGCU | 22 | 5000 |
| myoC-5255 | - | AGCAGCCAGGAGGUAGCAAGGCU | 23 | 5001 |
| myoC-5256 | - | CAGCAGCCAGGAGGUAGCAAGGCU | 24 | 5002 |
| myoC-5257 | - | ACCGAGACAGUGAAGGCU | 18 | 5003 |
| myoC-5258 | - | UACCGAGACAGUGAAGGCU | 19 | 5004 |
| myoC-2909 | - | AUACCGAGACAGUGAAGGCU | 20 | 1811 |
| myoC-5259 | - | AAUACCGAGACAGUGAAGGCU | 21 | 5005 |
| myoC-5260 | - | GAAUACCGAGACAGUGAAGGCU | 22 | 5006 |
| myoC-5261 | - | UGAAUACCGAGACAGUGAAGGCU | 23 | 5007 |
| myoC-5262 | - | CUGAAUACCGAGACAGUGAAGGCU | 24 | 5008 |
| myoC-5263 | - | AUGGCAGAAGGAGAUGCU | 18 | 5009 |
| myoC-5264 | - | AAUGGCAGAAGGAGAUGCU | 19 | 5010 |
| myoC-3006 | - | CAAUGGCAGAAGGAGAUGCU | 20 | 2797 |
| myoC-5265 | - | GCAAUGGCAGAAGGAGAUGCU | 21 | 5011 |
| myoC-5266 | - | GGCAAUGGCAGAAGGAGAUGCU | 22 | 5012 |
| myoC-5267 | - | AGGCAAUGGCAGAAGGAGAUGCU | 23 | 5013 |
| myoC-5268 | - | CAGGCAAUGGCAGAAGGAGAUGCU | 24 | 5014 |
| myoC-5269 | - | GAGCCCAUCUGGCUAUCU | 18 | 5015 |
| myoC-5270 | - | AGAGCCCAUCUGGCUAUCU | 19 | 5016 |
| myoC-5271 | - | GAGAGCCCAUCUGGCUAUCU | 20 | 5017 |
| myoC-5272 | - | GGAGAGCCCAUCUGGCUAUCU | 21 | 5018 |
| myoC-5273 | - | AGGAGAGCCCAUCUGGCUAUCU | 22 | 5019 |
| myoC-5274 | - | AAGGAGAGCCCAUCUGGCUAUCU | 23 | 5020 |
| myoC-5275 | - | GAAGGAGAGCCCAUCUGGCUAUCU | 24 | 5021 |
| myoC-5276 | - | AUUCAGGAAUUGUAGUCU | 18 | 5022 |
| myoC-5277 | - | UAUUCAGGAAUUGUAGUCU | 19 | 5023 |
| myoC-3025 | - | CUAUUCAGGAAUUGUAGUCU | 20 | 2808 |
| myoC-5278 | - | ACUAUUCAGGAAUUGUAGUCU | 21 | 5024 |
| myoC-5279 | - | AACUAUUCAGGAAUUGUAGUCU | 22 | 5025 |
| myoC-5280 | - | UAACUAUUCAGGAAUUGUAGUCU | 23 | 5026 |
| myoC-5281 | - | CUAACUAUUCAGGAAUUGUAGUCU | 24 | 5027 |
| myoC-5282 | - | CCUUCCAGGAACUGAAGU | 18 | 5028 |
| myoC-5283 | - | GCCUUCCAGGAACUGAAGU | 19 | 5029 |
| myoC-5284 | - | GGCCUUCCAGGAACUGAAGU | 20 | 5030 |
| myoC-5285 | - | UGGCCUUCCAGGAACUGAAGU | 21 | 5031 |
| myoC-5286 | - | UUGGCCUUCCAGGAACUGAAGU | 22 | 5032 |
| myoC-5287 | - | UUUGGCCUUCCAGGAACUGAAGU | 23 | 5033 |
| myoC-5288 | - | CUUUGGCCUUCCAGGAACUGAAGU | 24 | 5034 |
| myoC-5289 | - | AAGGUAAGAAUGCAGAGU | 18 | 5035 |
| myoC-5290 | - | GAAGGUAAGAAUGCAGAGU | 19 | 5036 |
| myoC-3191 | - | AGAAGGUAAGAAUGCAGAGU | 20 | 2937 |
| myoC-5291 | - | GAGAAGGUAAGAAUGCAGAGU | 21 | 5037 |
| myoC-5292 | - | AGAGAAGGUAAGAAUGCAGAGU | 22 | 5038 |
| myoC-5293 | - | CAGAGAAGGUAAGAAUGCAGAGU | 23 | 5039 |
| myoC-5294 | - | CCAGAGAAGGUAAGAAUGCAGAGU | 24 | 5040 |
| myoC-5295 | - | CUAUUCAGGAAUUGUAGU | 18 | 5041 |
| myoC-5296 | - | ACUAUUCAGGAAUUGUAGU | 19 | 5042 |
| myoC-3024 | - | AACUAUUCAGGAAUUGUAGU | 20 | 2807 |
| myoC-5297 | - | UAACUAUUCAGGAAUUGUAGU | 21 | 5043 |
| myoC-5298 | - | CUAACUAUUCAGGAAUUGUAGU | 22 | 5044 |
| myoC-5299 | - | UCUAACUAUUCAGGAAUUGUAGU | 23 | 5045 |
| myoC-5300 | - | AUCUAACUAUUCAGGAAUUGUAGU | 24 | 5046 |
| myoC-5301 | - | GGAGAGGGAGACACCGGU | 18 | 5047 |
| myoC-5302 | - | UGGAGAGGGAGACACCGGU | 19 | 5048 |
| myoC-5303 | - | GUGGAGAGGGAGACACCGGU | 20 | 5049 |
| myoC-5304 | - | AGUGGAGAGGGAGACACCGGU | 21 | 5050 |
| myoC-5305 | - | GAGUGGAGAGGGAGACACCGGU | 22 | 5051 |
| myoC-5306 | - | GGAGUGGAGAGGGAGACACCGGU | 23 | 5052 |
| myoC-5307 | - | AGGAGUGGAGAGGGAGACACCGGU | 24 | 5053 |
| myoC-5308 | - | UGGAGAACUAGUUUGGGU | 18 | 5054 |
| myoC-5309 | - | GUGGAGAACUAGUUUGGGU | 19 | 5055 |
| myoC-356 | - | UGUGGAGAACUAGUUUGGGU | 20 | 742 |
| myoC-5310 | - | AUGUGGAGAACUAGUUUGGGU | 21 | 5056 |
| myoC-5311 | - | GAUGUGGAGAACUAGUUUGGGU | 22 | 5057 |
| myoC-5312 | - | GGAUGUGGAGAACUAGUUUGGGU | 23 | 5058 |
| myoC-5313 | - | AGGAUGUGGAGAACUAGUUUGGGU | 24 | 5059 |
| myoC-5314 | - | GUUCCUGCUUCCCGAAUU | 18 | 5060 |
| myoC-5315 | - | AGUUCCUGCUUCCCGAAUU | 19 | 5061 |
| myoC-5316 | - | AAGUUCCUGCUUCCCGAAUU | 20 | 5062 |
| myoC-5317 | - | GAAGUUCCUGCUUCCCGAAUU | 21 | 5063 |
| myoC-5318 | - | UGAAGUUCCUGCUUCCCGAAUU | 22 | 5064 |
| myoC-5319 | - | CUGAAGUUCCUGCUUCCCGAAUU | 23 | 5065 |
| myoC-5320 | - | ACUGAAGUUCCUGCUUCCCGAAUU | 24 | 5066 |
| myoC-5321 | - | CACAUAACCCUUUACAUU | 18 | 5067 |
| myoC-5322 | - | UCACAUAACCCUUUACAUU | 19 | 5068 |
| myoC-5323 | - | CUCACAUAACCCUUUACAUU | 20 | 5069 |
| myoC-5324 | - | UCUCACAUAACCCUUUACAUU | 21 | 5070 |
| myoC-5325 | - | GUCUCACAUAACCCUUUACAUU | 22 | 5071 |
| myoC-5326 | - | GGUCUCACAUAACCCUUUACAUU | 23 | 5072 |
| myoC-5327 | - | GGGUCUCACAUAACCCUUUACAUU | 24 | 5073 |
| myoC-5328 | - | UCAAGUUUUCUUGUGAUU | 18 | 5074 |
| myoC-5329 | - | UUCAAGUUUUCUUGUGAUU | 19 | 5075 |
| myoC-490 | - | GUUCAAGUUUUCUUGUGAUU | 20 | 832 |
| myoC-5330 | - | AGUUCAAGUUUUCUUGUGAUU | 21 | 5076 |
| myoC-5331 | - | UAGUUCAAGUUUUCUUGUGAUU | 22 | 5077 |
| myoC-5332 | - | AUAGUUCAAGUUUUCUUGUGAUU | 23 | 5078 |
| myoC-5333 | - | CAUAGUUCAAGUUUUCUUGUGAUU | 24 | 5079 |
| myoC-5334 | - | GGUCACCAUCUAACUAUU | 18 | 5080 |
| myoC-5335 | - | UGGUCACCAUCUAACUAUU | 19 | 5081 |
| myoC-3022 | - | AUGGUCACCAUCUAACUAUU | 20 | 2806 |
| myoC-5336 | - | CAUGGUCACCAUCUAACUAUU | 21 | 5082 |
| myoC-5337 | - | ACAUGGUCACCAUCUAACUAUU | 22 | 5083 |
| myoC-5338 | - | AACAUGGUCACCAUCUAACUAUU | 23 | 5084 |
| myoC-5339 | - | GAACAUGGUCACCAUCUAACUAUU | 24 | 5085 |
| myoC-5340 | - | UAUCUUCUGUCAGCAUUU | 18 | 5086 |
| myoC-5341 | - | UUAUCUUCUGUCAGCAUUU | 19 | 5087 |
| myoC-5342 | - | UUUAUCUUCUGUCAGCAUUU | 20 | 5088 |
| myoC-5343 | - | CUUUAUCUUCUGUCAGCAUUU | 21 | 5089 |
| myoC-5344 | - | CCUUUAUCUUCUGUCAGCAUUU | 22 | 5090 |
| myoC-5345 | - | UCCUUUAUCUUCUGUCAGCAUUU | 23 | 5091 |
| myoC-5346 | - | AUCCUUUAUCUUCUGUCAGCAUUU | 24 | 5092 |
| myoC-5347 | - | UUCUCUUCCUUGAACUUU | 18 | 5093 |
| myoC-5348 | - | GUUCUCUUCCUUGAACUUU | 19 | 5094 |
| myoC-5349 | - | CGUUCUCUUCCUUGAACUUU | 20 | 5095 |
| myoC-5350 | - | ACGUUCUCUUCCUUGAACUUU | 21 | 5096 |
| myoC-5351 | - | AACGUUCUCUUCCUUGAACUUU | 22 | 5097 |
| myoC-5352 | - | CAACGUUCUCUUCCUUGAACUUU | 23 | 5098 |
| myoC-5353 | - | CCAACGUUCUCUUCCUUGAACUUU | 24 | 5099 |

**Table 8A** provides exemplary targeting domains for knocking out the *MYOC* gene selected according to the first tier parameters. The targeting domains bind within the first 500 bp of the coding sequence (e.g., within 500 bp downstream from the start codon), have a high level of orthogonality and start with a 5'G. It is contemplated herein that in an embodiment the targeting domain hybridizes to the target domain through complementary base pairing. Any of the targeting domains in the Table 1can be used with a N. *meningitidis* Cas9 molecule that generates a double stranded break (Cas9 nuclease) or a single-stranded break (Cas9 nickase).

**Table 8A**

| 1st Tier | | | | |
|---|---|---|---|---|
| gRNA Name | DNA Strand | Targeting Domain | Target Site Length | SEQ ID NO |
| myoC-5354 | - | GAUGCCAGCUGUCCAGC | 17 | 5100 |
| myoC-3082 | + | GCCUGGCUCUGCUCUGGGCA | 20 | 2844 |
| myoC-5355 | + | GCACAGAAGAACCUCAUUGC | 20 | 5101 |
| myoC-5356 | - | GGUUCUUCUGUGCACGUUGC | 20 | 5102 |

**Table 8B** provides exemplary targeting domains for knocking out the *MYOC* gene selected according to the second tier parameters. The targeting domains bind within the first 500 bp of the coding sequence (e.g., within 500 bp downstream from the start codon) and have a high level of orthogonality. It is contemplated herein that in an embodiment the targeting domain hybridizes to the target domain through complementary base pairing. Any of the targeting domains in the Table 1can be used with a N. *meningitidis* Cas9 molecule that generates a double stranded break (Cas9 nuclease) or a single-stranded break (Cas9 nickase).

**Table 8B**

| 2nd Tier | | | | |
|---|---|---|---|---|
| gRNA Name | DNA Strand | Targeting Domain | Target Site Length | SEQ ID NO |
| myoC-5357 | - | AGAGAGACAGCAGCACC | 17 | 5103 |
| myoC-5358 | + | CAGAAGAACCUCAUUGC | 17 | 5104 |
| myoC-5359 | - | UCUUCUGUGCACGUUGC | 17 | 5105 |
| myoC-5360 | + | UCAUUGCAGAGGCUUGG | 17 | 5106 |
| myoC-3085 | + | UGCUUUCCAACCUCCUG | 17 | 2851 |
| myoC-5361 | - | UACAGAGAGACAGCAGCACC | 20 | 5107 |
| myoC-5362 | + | ACCUCAUUGCAGAGGCUUGG | 20 | 5108 |
| myoC-3083 | + | UGCUGCUUUCCAACCUCCUG | 20 | 2845 |

**Table 8C** provides exemplary targeting domains for knocking out the *MYOC* gene selected according to the third tier parameters. The targeting domains bind within the first 500 bp of the coding sequence (e.g., within 500 bp downstream from the start codon) and start with a 5'G. It is contemplated herein that in an embodiment the targeting domain hybridizes to the target domain through complementary base pairing. Any of the targeting domains in the Table 1can be used with a N. *meningitidis* Cas9 molecule that generates a double stranded break (Cas9 nuclease) or a single-stranded break (Cas9 nickase).

**Table 8C**

| 3rd Tier | | | | |
|---|---|---|---|---|
| gRNA Name | DNA Strand | Targeting Domain | Target Site Length | SEQ ID NO |
| myoC-5363 | + | GAUUCUCAUUUUCUUGCCUU | 20 | 5109 |

**Table 8D** provides exemplary targeting domains for knocking out the *MYOC* gene selected according to the fourth tier parameters. The targeting domains bind within the first 500 bp of the coding sequence (e.g., within 500 bp downstream from the start codon). It is contemplated herein that in an embodiment the targeting domain hybridizes to the target domain through complementary base pairing. Any of the targeting domains in the Table 1can be used with a *N*. *meningitidis* Cas9 molecule that generates a double stranded break (Cas9 nuclease) or a single-stranded break (Cas9 nickase).

**Table 8D**

| 4th Tier | | | | |
|---|---|---|---|---|
| gRNA Name | DNA Strand | Targeting Domain | Target Site Length | SEQ ID NO |
| myoC-3084 | + | UGGCUCUGCUCUGGGCA | 17 | 2850 |
| myoC-1788 | + | CUCUCCAGGGAGCUGAG | 17 | 2017 |
| myoC-5364 | + | UCUCAUUUUCUUGCCUU | 17 | 5110 |
| myoC-5365 | - | UGAGAUGCCAGCUGUCCAGC | 20 | 5111 |
| myoC-1678 | + | AGGCUCUCCAGGGAGCUGAG | 20 | 1939 |

**Table 8E** provides exemplary targeting domains for knocking out the *MYOC* gene selected according to the fifth tier parameters. The targeting domains fall in the coding sequence of the gene, downstream of the first 500bp of coding sequence (e.g., anywhere from +500 (relative to the start codon) to the stop codon of the gene). It is contemplated herein that in an embodiment the targeting domain hybridizes to the target domain through complementary base pairing. Any of the targeting domains in the Table 1can be used with a *N. meningitidis* Cas9 molecule that generates a double stranded break (Cas9 nuclease) or a single-stranded break (Cas9 nickase).

**Table 8E**

| 5th Tier | | | | |
|---|---|---|---|---|
| gRNA Name | DNA Strand | Targeting Domain | Target Site Length | SEQ ID NO |
| myoC-5366 | - | GCAGAUGCUACCGUCAA | 17 | 5112 |
| myoC-5367 | - | AAGAUGCAUUUACUACA | 17 | 5113 |
| myoC-5368 | - | CAGCCAGCCAGGGCCCA | 17 | 5114 |
| myoC-3157 | - | CCGCUAUAAGUACAGCA | 17 | 2843 |
| myoC-2994 | + | UCAAGUUGUCCCAGGCA | 17 | 1873 |
| myoC-5369 | + | GCUGGCCAGAGGAGCUA | 17 | 5115 |
| myoC-5370 | + | CGAGUACACCACAGCAC | 17 | 5116 |
| myoC-5371 | + | CCUUGCUACCUCCUGGC | 17 | 5117 |
| myoC-2950 | + | UCCGUGGUAGCCAGCUC | 17 | 1842 |
| myoC-5372 | - | UUACUACAGUUGGCUUC | 17 | 5118 |
| myoC-3093 | - | ACAUAGUUCAAGUUUUC | 17 | 2852 |
| myoC-5373 | + | UCUGCUUCCUUUAGAAG | 17 | 5119 |
| myoC-5374 | + | CUGUAAAUGACCCAGAG | 17 | 5120 |
| myoC-5375 | + | CCUGGGUGUAGGGGUAG | 17 | 5121 |
| myoC-3094 | + | GACAUCCGUGCCAACUG | 17 | 2853 |
| myoC-5376 | + | CCUUCUGCCAUUGCCUG | 17 | 5122 |
| myoC-2995 | + | AGGCUUUUCACAUCUUG | 17 | 1874 |
| myoC-5377 | + | GAAGUUAUGCUUUUUAU | 17 | 5123 |
| myoC-5378 | + | UGAAGGCAUUGGCGACU | 17 | 5124 |
| myoC-5379 | + | AAGAAACUAUUAUGCCU | 17 | 5125 |
| myoC-3097 | + | GUGACCAUGUUCAUCCU | 17 | 2855 |
| myoC-5380 | - | UCCGAGCUAACUGAAGU | 17 | 5126 |
| myoC-3096 | + | CCCAGGUUUGUUCGAGU | 17 | 2854 |
| myoC-5381 | + | CAUUGCCUGUACAGCUU | 17 | 5127 |
| myoC-5382 | - | AGGGCCCAGGCAGCUUU | 17 | 5128 |
| myoC-5383 | - | UCAGCAGAUGCUACCGUCAA | 20 | 5129 |
| myoC-5384 | - | AGUAAGAUGCAUUUACUACA | 20 | 5130 |
| myoC-5385 | - | AGCCAGCCAGCCAGGGCCCA | 20 | 5131 |
| myoC-3156 | - | GAACCGCUAUAAGUACAGCA | 20 | 2842 |
| myoC-2973 | + | UGUUCAAGUUGUCCCAGGCA | 20 | 1858 |
| myoC-5386 | + | GAUGCUGGCCAGAGGAGCUA | 20 | 5132 |
| myoC-5387 | + | CCCCGAGUACACCACAGCAC | 20 | 5133 |
| myoC-5388 | + | CAGCCUUGCUACCUCCUGGC | 20 | 5134 |
| myoC-2924 | + | CUGUCCGUGGUAGCCAGCUC | 20 | 1822 |
| myoC-5389 | - | CAUUUACUACAGUUGGCUUC | 20 | 5135 |
| myoC-3087 | - | AUGACAUAGUUCAAGUUUUC | 20 | 2846 |
| myoC-5390 | + | UAUUCUGCUUCCUUUAGAAG | 20 | 5136 |
| myoC-5391 | + | GUGCUGUAAAUGACCCAGAG | 20 | 5137 |
| myoC-4390 | + | UCUCCUGGGUGUAGGGGUAG | 20 | 4136 |
| myoC-3088 | + | GCGGACAUCCGUGCCAACUG | 20 | 2847 |
| myoC-5392 | + | UCUCCUUCUGCCAUUGCCUG | 20 | 5138 |
| myoC-2974 | + | UGGAGGCUUUUCACAUCUUG | 20 | 1859 |
| myoC-5393 | + | UUAGAAGUUAUGCUUUUUAU | 20 | 5139 |
| myoC-5394 | + | UGAUGAAGGCAUUGGCGACU | 20 | 5140 |
| myoC-5395 | + | AGGAAGAAACUAUUAUGCCU | 20 | 5141 |
| myoC-3091 | + | AUGGUGACCAUGUUCAUCCU | 20 | 2849 |
| myoC-5396 | - | AAGUCCGAGCUAACUGAAGU | 20 | 5142 |
| myoC-3090 | + | UCUCCCAGGUUUGUUCGAGU | 20 | 2848 |
| myoC-5397 | + | UGCCAUUGCCUGUACAGCUU | 20 | 5143 |
| myoC-5398 | - | GCCAGGGCCCAGGCAGCUUU | 20 | 5144 |

**Table 9A** provides exemplary targeting domains for knocking down the *MYOC* gene selected according to the first tier parameters. The targeting domains bind within 3454-2454bp upstream of transcription start site or 500bp upstream and downstream of transcription start site, have a high level of orthogonality and start with a 5'G. It is contemplated herein that in an embodiment the targeting domain hybridizes to the target domain through complementary base pairing. Any of the targeting domains in the table can be used with a *S. pyogenes* eiCas9 molecule or eiCas9 fusion protein (e.g., an eiCas9 fused to a transcription repressor domain) to alter the *MYOC* gene (e.g., reduce or eliminate *MYOC* gene expression, *MYOC* protein function, or the level of *MYOC* protein). One or more gRNA may be used to target an eiCas9 to the promoter region of the *MYOC* gene.

**Table 9A**

| 1st Tier | | | | |
|---|---|---|---|---|
| gRNA Name | DNA Strand | Targeting Domain | Target Site Length | Seq ID |
| myoC-1263 | - | GCUGAGCGGGUGCUGAA | 17 | 1563 |
| myoC-1237 | - | GAGGGAAACUAGUCUAA | 17 | 1537 |
| myoC-955 | + | GUGUGCUGAUUUCAACA | 17 | 1002 |
| myoC-163 | + | GUUAUGGAUGACUGACA | 17 | 496 |
| myoC-791 | + | GCACGAUGGAGGCAGCA | 17 | 1028 |
| myoC-822 | + | GACCCCGGGUGCUUGCA | 17 | 982 |
| myoC-155 | + | GUCCCGCUCCCGCCUCA | 17 | 546 |
| myoC-788 | + | GGGGCCUCCGGGCACGA | 17 | 1043 |
| myoC-798 | + | GGGAGGUGGCCUUGUUA | 17 | 1041 |
| myoC-2709 | + | GCACCAGGACGAUUCAC | 17 | 2649 |
| myoC-167 | + | GCUGGAUUCAUUGGGAC | 17 | 497 |
| myoC-931 | + | GAGAGGUUUAUAUAUAC | 17 | 997 |
| myoC-818 | + | GGUUGCUCAGGACACCC | 17 | 1044 |
| myoC-764 | - | GACUCGUUCAUUCAUCC | 17 | 1022 |
| myoC-139 | - | GCGGGAGCGGGACCAGC | 17 | 534 |
| myoC-959 | + | GUCCUUUAAGACGUAGC | 17 | 1000 |
| myoC-821 | + | GGACCCCGGGUGCUUGC | 17 | 1033 |
| myoC-919 | - | GUAUAUAUAAACCUCUC | 17 | 998 |
| myoC-138 | - | GCACCCUGAGGCGGGAG | 17 | 533 |
| myoC-1271 | - | GUUCAGUGUUGUUCACG | 17 | 1571 |
| myoC-772 | - | GCCUCCAUCGUGCCCGG | 17 | 985 |
| myoC-828 | + | GAGGAAACCUCUGCCGG | 17 | 983 |
| myoC-152 | + | GAACUGACUUGUCUCGG | 17 | 492 |
| myoC-937 | + | GAGCCAGCCCUUCAUGG | 17 | 1056 |
| myoC-789 | + | GCCUCCGGGCACGAUGG | 17 | 986 |
| myoC-157 | + | GGUCCAAGGUCAAUUGG | 17 | 493 |
| myoC-785 | + | GGAAGACUCGGGCUUGG | 17 | 1032 |
| myoC-161 | + | GCUGAGUCGAGCUUUGG | 17 | 495 |
| myoC-909 | - | GGUAUGGGUGCAUAAAU | 17 | 1067 |
| myoC-1273 | - | GUGUUGUUCACGGGGCU | 17 | 1573 |
| myoC-806 | + | GUCACCUCCACGAAGGU | 17 | 987 |
| myoC-910 | - | GUAUGGGUGCAUAAAUU | 17 | 999 |
| myoC-166 | + | GGGCAGCUGGAUUCAUU | 17 | 553 |
| myoC-129 | - | GCACGUUGCUGCAGCUU | 17 | 488 |
| myoC-160 | + | GGAGCUGAGUCGAGCUU | 17 | 494 |
| myoC-967 | - | GGAGAGGGAAACUAGUCUAA | 20 | 1267 |
| myoC-694 | - | GUGCGCAGCAUCCCUUAACA | 20 | 981 |
| myoC-692 | - | GUGGAGGUGACAGUUUCUCA | 20 | 1021 |
| myoC-973 | - | GGGGACAGUGUUUCCUCAGA | 20 | 1273 |
| myoC-1012 | - | GCAUGGGUUUUCCUUCACGA | 20 | 1312 |
| myoC-995 | - | GCGGGUGCUGAAAGGCAGGA | 20 | 1295 |
| myoC-848 | - | GAAUCUUGCUGGCAGCGUGA | 20 | 988 |
| myoC-2163 | + | GAUGCACCAGGACGAUUCAC | 20 | 2269 |
| myoC-126 | + | GCAGCUGGAUUCAUUGGGAC | 20 | 523 |
| myoC-680 | - | GGGGGAGCCCUGCAAGCACC | 20 | 1020 |
| myoC-1116 | + | GUCUCCAGCUCAGAUGCACC | 20 | 1416 |
| myoC-741 | + | GCAGGUUGCUCAGGACACCC | 20 | 1007 |
| myoC-681 | - | GGGGAGCCCUGCAAGCACCC | 20 | 1019 |
| myoC-857 | - | GCCAGCAAGGCCACCCAUCC | 20 | 990 |
| myoC-123 | + | GUCGAGCUUUGGUGGCCUCC | 20 | 485 |
| myoC-977 | - | GGAAAGGGGCCUCCACGUCC | 20 | 1277 |
| myoC-105 | - | GAGGCGGGAGCGGGACCAGC | 20 | 510 |
| myoC-104 | - | GGGCACCCUGAGGCGGGAGC | 20 | 509 |
| myoC-117 | + | GCUGGUCCCGCUCCCGCCUC | 20 | 484 |
| myoC-709 | + | GACUCGGGCUUGGGGGCCUC | 20 | 1003 |
| myoC-125 | + | GACAUGGCCUGGCUCUGCUC | 20 | 522 |
| myoC-965 | - | GCUCCAGAAAGGAAAUGGAG | 20 | 1265 |
| myoC-971 | - | GUCUAACGGAGAAUCUGGAG | 20 | 1271 |
| myoC-1001 | - | GAUGUUCAGUGUUGUUCACG | 20 | 1301 |
| myoC-682 | - | GGGAGCCCUGCAAGCACCCG | 20 | 979 |
| myoC-114 | + | GAACUGACUUGUCUCGGAGG | 20 | 482 |
| myoC-696 | - | GCUGCCUCCAUCGUGCCCGG | 20 | 976 |
| myoC-751 | + | GGAGAGGAAACCUCUGCCGG | 20 | 1013 |
| myoC-719 | + | GGCAGCAGGGGGCGCUAGGG | 20 | 1017 |
| myoC-871 | + | GGGGAGCCAGCCCUUCAUGG | 20 | 992 |
| myoC-712 | + | GGGGCCUCCGGGCACGAUGG | 20 | 980 |
| myoC-679 | - | GAGGUUUCCUCUCCAGCUGG | 20 | 1005 |
| myoC-121 | + | GGUCCAAGGUCAAUUGGUGG | 20 | 520 |
| myoC-122 | + | GGAGCUGAGUCGAGCUUUGG | 20 | 521 |
| myoC-707 | + | GCUUGGAAGACUCGGGCUUG | 20 | 977 |
| myoC-127 | + | GCAUCGGCCACUCUGGUCAU | 20 | 487 |
| myoC-861 | + | GUGCUGAGAGGUGCCUGGAU | 20 | 995 |
| myoC-837 | - | GUAAAACCAGGUGGAGAUAU | 20 | 994 |
| myoC-838 | - | GGAGAUAUAGGAACUAUUAU | 20 | 991 |
| myoC-1107 | + | GUGAACAACACUGAACAUCU | 20 | 1407 |
| myoC-106 | - | GGAAACCCAAACCAGAGAGU | 20 | 479 |
| myoC-878 | + | GUGGCCACGUGAGGCUGGGU | 20 | 1054 |
| myoC-95 | - | GCCUGCCUGGUGUGGGAUGU | 20 | 500 |
| myoC-115 | + | GUCUCGGAGGAGGUUGCUGU | 20 | 516 |
| myoC-93 | - | GCUUCUGGCCUGCCUGGUGU | 20 | 478 |
| myoC-844 | - | GGGGUAUGGGUGCAUAAAUU | 20 | 993 |
| myoC-839 | - | GAGAUAUAGGAACUAUUAUU | 20 | 989 |
| myoC-706 | + | GGCUUGGAAGACUCGGGCUU | 20 | 978 |
| myoC-124 | + | GGCCUCCAGGUCUAAGCGUU | 20 | 486 |
| myoC-91 | - | GUGCACGUUGCUGCAGCUUU | 20 | 477 |

**Table 9B** provides exemplary targeting domains for knocking down the *MYOC* gene selected according to the second tier parameters. The targeting domains bind within 3454-2454bp upstream of transcription start site or 500bp upstream and downstream of transcription start site and have a high level of orthogonality. It is contemplated herein that in an embodiment the targeting domain hybridizes to the target domain through complementary base pairing. Any of the targeting domains in the table can be used with a S. pyogenes eiCas9 molecule or eiCas9 fusion protein (e.g., an eiCas9 fused to a transcription repressor domain) to alter the MYOC gene (e.g., reduce or eliminate MYOC gene expression, MYOC protein function, or the level of MYOC protein). One or more gRNA may be used to target an eiCas9 to the promoter region of the MYOC gene.

**Table 9B**

| 2nd Tier | | | | |
|---|---|---|---|---|
| gRNA Name | DNA Strand | Targeting Domain | Target Site Length | Seq ID |
| myoC-1368 | + | CUUCUUCCGUGAAUUAA | 17 | 1668 |
| myoC-895 | - | UCCCUGCUACGUCUUAA | 17 | 1249 |
| myoC-1283 | - | CGAAGGCCUUUAUUUAA | 17 | 1583 |
| myoC-770 | - | CGCAGCAUCCCUUAACA | 17 | 1154 |
| myoC-960 | + | UCCUUUAAGACGUAGCA | 17 | 1250 |
| myoC-813 | + | CAAAACAACCAGUGGCA | 17 | 1145 |
| myoC-1287 | - | CCUAGGCCGUUAAUUCA | 17 | 1587 |
| myoC-2710 | + | UGCACCAGGACGAUUCA | 17 | 2650 |
| myoC-800 | + | CGCACAAUUCUUCAAGA | 17 | 1153 |
| myoC-805 | + | AACUGUCACCUCCACGA | 17 | 1128 |
| myoC-1282 | - | UGGGUUUUCCUUCACGA | 17 | 1582 |
| myoC-1240 | - | CUAACGGAGAAUCUGGA | 17 | 1540 |
| myoC-775 | + | UGCAGCGCUGUGACUGA | 17 | 1164 |
| myoC-914 | - | UCUUGCUGGCAGCGUGA | 17 | 1253 |
| myoC-1371 | + | AAUAAAGGCCUUCGUGA | 17 | 1671 |
| myoC-271 | - | AAGAGAAGAAGCGACUA | 17 | 657 |
| myoC-807 | + | UCACCUCCACGAAGGUA | 17 | 1159 |
| myoC-761 | - | CUGCCAGCCCGUGCCAC | 17 | 1156 |
| myoC-1270 | - | UGUUCAGUGUUGUUCAC | 17 | 1570 |
| myoC-303 | + | CCACACUGAAGGUAUAC | 17 | 689 |
| myoC-954 | + | ACUUACACCAGGACUAC | 17 | 1227 |
| myoC-1386 | + | UCCAGCUCAGAUGCACC | 17 | 1686 |
| myoC-254 | - | CACCCAACGCUUAGACC | 17 | 640 |
| myoC-258 | - | CCAAUUGACCUUGGACC | 17 | 644 |
| myoC-1486 | + | AAGGACAGCACCCUACC | 17 | 1786 |
| myoC-256 | - | AGCUCGACUCAGCUCCC | 17 | 642 |
| myoC-923 | - | AGCAAGGCCACCCAUCC | 17 | 1231 |
| myoC-1247 | - | AAGGGGCCUCCACGUCC | 17 | 1547 |
| myoC-5399 | - | CUUCCCGUGAAUCGUCC | 17 | 5145 |
| myoC-1248 | - | ACGUCCAGGAGAAUUCC | 17 | 1548 |
| myoC-773 | - | AGUCACAGCGCUGCAGC | 17 | 1143 |
| myoC-2390 | - | UCCUGGUGCAUCUGAGC | 17 | 2434 |
| myoC-1264 | - | AGCGGGUGCUGAAAGGC | 17 | 1564 |
| myoC-774 | + | UUCACGGGAAGCGAGGC | 17 | 1167 |
| myoC-815 | + | CAACCAGUGGCACGGGC | 17 | 1146 |
| myoC-1272 | - | AGUGUUGUUCACGGGGC | 17 | 1572 |
| myoC-5400 | - | UGUCCUUGUGUUCUGGC | 17 | 5146 |
| myoC-804 | + | ACUGGGUUUAAGUUGGC | 17 | 1132 |
| myoC-929 | + | UGGAUGGGUGGCCUUGC | 17 | 1255 |
| myoC-1238 | - | UAGUCUAACGGAGAAUC | 17 | 1538 |
| myoC-305 | + | ACUGGCAUCGGCCACUC | 17 | 691 |
| myoC-2902 | + | CUUGGUGAGGCUUCCUC | 17 | 2790 |
| myoC-269 | - | CCGAGACAAGUCAGUUC | 17 | 655 |
| myoC-5401 | - | CUUGAAGCCCCCGGCAG | 17 | 5147 |
| myoC-930 | + | AUGCCCGAGCUCCAGAG | 17 | 1236 |
| myoC-1241 | - | UAACGGAGAAUCUGGAG | 17 | 1541 |
| myoC-1380 | + | UGGAAUUCUCCUGGACG | 17 | 1680 |
| myoC-827 | + | AGAGGAAACCUCUGCCG | 17 | 1134 |
| myoC-5402 | + | ACGAUUCACGGGAAGCG | 17 | 5148 |
| myoC-766 | - | UCACUGCCCUACCUUCG | 17 | 1160 |
| myoC-296 | + | AGGUCAAUUGGUGGAGG | 17 | 682 |
| myoC-776 | + | AGCGCUGUGACUGAUGG | 17 | 1137 |
| myoC-255 | - | CCAACGCUUAGACCUGG | 17 | 641 |
| myoC-1239 | - | UCUAACGGAGAAUCUGG | 17 | 1539 |
| myoC-270 | - | AGACAAGUCAGUUCUGG | 17 | 656 |
| myoC-1381 | + | AAUUCUCCUGGACGUGG | 17 | 1681 |
| myoC-767 | - | CUGCCCUACCUUCGUGG | 17 | 1157 |
| myoC-3158 | - | ACCAAGCCUCUGCAAUG | 17 | 2904 |
| myoC-252 | - | CCAGUAUACCUUCAGUG | 17 | 638 |
| myoC-294 | + | CCUGGUCCAAGGUCAAU | 17 | 680 |
| myoC-304 | + | UGAAGGUAUACUGGCAU | 17 | 690 |
| myoC-1281 | - | AAUUCCAGGGUGUGCAU | 17 | 1581 |
| myoC-306 | + | UCGGCCACUCUGGUCAU | 17 | 692 |
| myoC-257 | - | CCUCCACCAAUUGACCU | 17 | 643 |
| myoC-1369 | + | CCGUGAAUUAACGGCCU | 17 | 1669 |
| myoC-782 | + | CUUGGAAGACUCGGGCU | 17 | 1158 |
| myoC-281 | + | CCAGAACUGACUUGUCU | 17 | 667 |
| myoC-803 | + | CAGCACUGGGUUUAAGU | 17 | 1150 |
| myoC-268 | - | AACCCAAACCAGAGAGU | 17 | 654 |
| myoC-297 | + | CCUCCAGGUCUAAGCGU | 17 | 683 |
| myoC-783 | + | UUGGAAGACUCGGGCUU | 17 | 1169 |
| myoC-298 | + | CUCCAGGUCUAAGCGUU | 17 | 684 |
| myoC-951 | + | CCUUCCAGAAGUCUGUU | 17 | 1242 |
| myoC-975 | - | AGUGUUUCCUCAGAGGGAAA | 20 | 1275 |
| myoC-974 | - | CAGUGUUUCCUCAGAGGGAA | 20 | 1274 |
| myoC-1098 | + | UCACUUCUUCCGUGAAUUAA | 20 | 1398 |
| myoC-829 | - | CUGUCCCUGCUACGUCUUAA | 20 | 1207 |
| myoC-722 | + | UAGGGAGGUGGCCUUGUUAA | 20 | 1115 |
| myoC-1013 | - | UCACGAAGGCCUUUAUUUAA | 20 | 1313 |
| myoC-889 | + | CUGGUGUGCUGAUUUCAACA | 20 | 1206 |
| myoC-227 | + | UAAGUUAUGGAUGACUGACA | 20 | 613 |
| myoC-1009 | - | AGUCAGCUGUUAAAAUUCCA | 20 | 1309 |
| myoC-856 | - | AGCUCGGGCAUGAGCCAGCA | 20 | 1183 |
| myoC-714 | + | CGGGCACGAUGGAGGCAGCA | 20 | 1105 |
| myoC-894 | + | AAGUCCUUUAAGACGUAGCA | 20 | 1173 |
| myoC-736 | + | UAACAAAACAACCAGUGGCA | 20 | 1114 |
| myoC-1010 | - | UUAAAAUUCCAGGGUGUGCA | 20 | 1310 |
| myoC-745 | + | CAGGACCCCGGGUGCUUGCA | 20 | 1098 |
| myoC-213 | + | CUGGUCCCGCUCCCGCCUCA | 20 | 599 |
| myoC-1017 | - | UUUCCUAGGCCGUUAAUUCA | 20 | 1317 |
| myoC-2164 | + | AGAUGCACCAGGACGAU UCA | 20 | 2270 |
| myoC-868 | + | ACUGGGGAGCCAGCCCUUCA | 20 | 1177 |
| myoC-999 | - | CAGAUGUUCAGUGUUGUUCA | 20 | 1299 |
| myoC-723 | + | CUGCGCACAAUUCUUCAAGA | 20 | 1109 |
| myoC-728 | + | AGAAACUGUCACCUCCACGA | 20 | 1084 |
| myoC-711 | + | UUGGGGGCCUCCGGGCACGA | 20 | 1123 |
| myoC-970 | - | AGUCUAACGGAGAAUCUGGA | 20 | 1270 |
| myoC-846 | - | ACUCCAAACAGACUUCUGGA | 20 | 1176 |
| myoC-1006 | - | AGAAGAAGUCUAUUUCAUGA | 20 | 1306 |
| myoC-233 | + | AUUGGGACUGGCCACACUGA | 20 | 619 |
| myoC-698 | + | AGCUGCAGCGCUGUGACUGA | 20 | 1089 |
| myoC-1101 | + | UUAAAUAAAGGCCUUCGUGA | 20 | 1401 |
| myoC-730 | + | CUGUCACCUCCACGAAGGUA | 20 | 1111 |
| myoC-841 | - | UAGGAACUAUUAUUGGGGUA | 20 | 1210 |
| myoC-226 | + | UGCUGUCUCUCUGUAAGUUA | 20 | 612 |
| myoC-721 | + | CUAGGGAGGUGGCCUUGUUA | 20 | 1106 |
| myoC-685 | - | AACCUGCCAGCCCGUGCCAC | 20 | 1079 |
| myoC-737 | + | AACAAAACAACCAGUGGCAC | 20 | 1077 |
| myoC-1000 | - | AGAUGUUCAGUGUUGUUCAC | 20 | 1300 |
| myoC-1018 | - | UAAUUCACGGAAGAAGUGAC | 20 | 1318 |
| myoC-865 | + | CCAGAGAGGUUUAUAUAUAC | 20 | 1195 |
| myoC-234 | + | UGGCCACACUGAAGGUAUAC | 20 | 620 |
| myoC-888 | + | CACACUUACACCAGGACUAC | 20 | 1189 |
| myoC-886 | + | AUAGUUCCUAUAUCUCCACC | 20 | 1185 |
| myoC-179 | - | CAGCACCCAACGCUUAGACC | 20 | 565 |
| myoC-183 | - | CCACCAAUUGACCUUGGACC | 20 | 569 |
| myoC-1216 | + | CACAAGGACAGCACCCUACC | 20 | 1516 |
| myoC-1102 | + | AGGAAAACCCAUGCACACCC | 20 | 1402 |
| myoC-181 | - | CAAAGCUCGACUCAGCUCCC | 20 | 567 |
| myoC-1114 | + | UCCAUUUCCUUUCUGGAGCC | 20 | 1414 |
| myoC-228 | + | UAUGGAUGACUGACAUGGCC | 20 | 614 |
| myoC-859 | + | CUGCUGUGCUGAGAGGUGCC | 20 | 1203 |
| myoC-688 | - | UGUGACUCGUUCAUUCAUCC | 20 | 1121 |
| myoC-710 | + | ACUCGGGCUUGGGGGCCUCC | 20 | 1082 |
| myoC-222 | + | AUUGGUGGAGGAGGCUCUCC | 20 | 608 |
| myoC-1109 | + | CCCACCUCCUGGAAUUCUCC | 20 | 1409 |
| myoC-5403 | - | UCGCUUCCCGUGAAUCGUCC | 20 | 5149 |
| myoC-683 | - | CCUGCAAGCACCCGGGGUCC | 20 | 1103 |
| myoC-978 | - | UCCACGUCCAGGAGAAUUCC | 20 | 1278 |
| myoC-212 | + | CUCUGGUUUGGGUUUCCAGC | 20 | 598 |
| myoC-713 | + | CCGGGCACGAUGGAGGCAGC | 20 | 1102 |
| myoC-697 | - | AUCAGUCACAGCGCUGCAGC | 20 | 1094 |
| myoC-1844 | - | UCGUCCUGGUGCAUCUGAGC | 20 | 2054 |
| myoC-893 | + | CAAGUCCUUUAAGACGUAGC | 20 | 1187 |
| myoC-994 | - | CUGAGCGGGUGCUGAAAGGC | 20 | 1294 |
| myoC-239 | + | CCCCACAUCCCACACCAGGC | 20 | 625 |
| myoC-5404 | + | CGAUUCACGGGAAGCGAGGC | 20 | 5150 |
| myoC-875 | + | CAGAGGUGGCCACGUGAGGC | 20 | 1192 |
| myoC-738 | + | AAACAACCAGUGGCACGGGC | 20 | 1076 |
| myoC-1002 | - | UUCAGUGUUGUUCACGGGGC | 20 | 1302 |
| myoC-739 | + | AACCAGUGGCACGGGCUGGC | 20 | 1078 |
| myoC-727 | + | AGCACUGGGUUUAAGUUGGC | 20 | 1086 |
| myoC-744 | + | CCAGGACCCCGGGUGCUUGC | 20 | 1101 |
| myoC-968 | - | AACUAGUCUAACGGAGAAUC | 20 | 1268 |
| myoC-1106 | + | CGUGAACAACACUGAACAUC | 20 | 1406 |
| myoC-236 | + | UAUACUGGCAUCGGCCACUC | 20 | 622 |
| myoC-2356 | + | AGGCUUGGUGAGGCUUCCUC | 20 | 2410 |
| myoC-855 | - | UAUAAACCUCUCUGGAGCUC | 20 | 1211 |
| myoC-740 | + | CACGGGCUGGCAGGUUGCUC | 20 | 1096 |
| myoC-241 | + | AGCUGGACAGCUGGCAUCUC | 20 | 627 |
| myoC-853 | - | CCAGUAUAUAUAAACCUCUC | 20 | 1197 |
| myoC-732 | + | ACCAUUUUGUCUCUGGUGUC | 20 | 1081 |
| myoC-170 | - | AGCUGUCCAGCUGCUGCUUC | 20 | 556 |
| myoC-191 | - | CCUCCGAGACAAGUCAGUUC | 20 | 577 |
| myoC-1215 | - | AGGGUGCUGUCCUUGUGUUC | 20 | 1515 |
| myoC-735 | + | AGUGAUAACAAAACAACCAG | 20 | 1092 |
| myoC-3159 | + | ACAGAAGAACCUCAUUGCAG | 20 | 2905 |
| myoC-972 | - | AGGGGACAGUGUUUCCUCAG | 20 | 1272 |
| myoC-864 | + | CUCAUGCCCGAGCUCCAGAG | 20 | 1201 |
| myoC-190 | - | UGGGCACCCUGAGGCGGGAG | 20 | 576 |
| myoC-1110 | + | UCCUGGAAUUCUCCUGGACG | 20 | 1410 |
| myoC-750 | + | UGGAGAGGAAACCUCUGCCG | 20 | 1119 |
| myoC-5405 | + | AGGACGAUUCACGGGAAGCG | 20 | 5151 |
| myoC-690 | - | CAGUCACUGCCCUACCUUCG | 20 | 1100 |
| myoC-979 | - | ACGUCCAGGAGAAUUCCAGG | 20 | 1279 |
| myoC-980 | - | UCCAGGAGAAUUCCAGGAGG | 20 | 1280 |
| myoC-720 | + | AGCAGGGGGCGCUAGGGAGG | 20 | 1087 |
| myoC-700 | + | AGCGCUGUGACUGAUGGAGG | 20 | 1088 |
| myoC-221 | + | CCAAGGUCAAUUGGUGGAGG | 20 | 607 |
| myoC-209 | + | CCAGAACUGACUUGUCUCGG | 20 | 595 |
| myoC-699 | + | UGCAGCGCUGUGACUGAUGG | 20 | 1118 |
| myoC-180 | - | CACCCAACGCUUAGACCUGG | 20 | 566 |
| myoC-969 | - | UAGUCUAACGGAGAAUCUGG | 20 | 1269 |
| myoC-192 | - | CCGAGACAAGUCAGUUCUGG | 20 | 578 |
| myoC-1111 | + | UGGAAUUCUCCUGGACGUGG | 20 | 1411 |
| myoC-691 | - | UCACUGCCCUACCUUCGUGG | 20 | 1117 |
| myoC-220 | + | CCUGGUCCAAGGUCAAUUGG | 20 | 606 |
| myoC-708 | + | CUUGGAAGACUCGGGCUUGG | 20 | 1112 |
| myoC-3160 | - | CUCACCAAGCCUCUGCAAUG | 20 | 2906 |
| myoC-867 | + | AGAGAGGUUUAUAUAUACUG | 20 | 1180 |
| myoC-177 | - | AUGCCAGUAUACCUUCAGUG | 20 | 563 |
| myoC-3161 | + | CUCAUUGCAGAGGCUUGGUG | 20 | 2907 |
| myoC-840 | - | AGAUAUAGGAACUAUUAUUG | 20 | 1182 |
| myoC-843 | - | UGGGGUAUGGGUGCAUAAAU | 20 | 1214 |
| myoC-219 | + | CAGCCUGGUCCAAGGUCAAU | 20 | 605 |
| myoC-1014 | - | CACGAAGGCCUUUAUUUAAU | 20 | 1314 |
| myoC-235 | + | CACUGAAGGUAUACUGGCAU | 20 | 621 |
| myoC-1011 | - | UAAAAUUCCAGGGUGUGCAU | 20 | 1311 |
| myoC-842 | - | AGGAACUAUUAUUGGGGUAU | 20 | 1184 |
| myoC-866 | + | CAGAGAGGUUUAUAUAUACU | 20 | 1191 |
| myoC-182 | - | CCUCCUCCACCAAUUGACCU | 20 | 568 |
| myoC-1099 | + | CUUCCGUGAAUUAACGGCCU | 20 | 1399 |
| myoC-961 | - | CAUCUGAGCUGGAGACUCCU | 20 | 1261 |
| myoC-684 | - | CUGCAAGCACCCGGGGUCCU | 20 | 1108 |
| myoC-1016 | - | AGGAAGCGAGCUCAUUUCCU | 20 | 1316 |
| myoC-854 | - | AUAUAAACCUCUCUGGAGCU | 20 | 1186 |
| myoC-3162 | + | AGAACCUCAUUGCAGAGGCU | 20 | 2908 |
| myoC-876 | + | AGAGGUGGCCACGUGAGGCU | 20 | 1181 |
| myoC-705 | + | AGGCUUGGAAGACUCGGGCU | 20 | 1091 |
| myoC-1003 | - | UCAGUGUUGUUCACGGGGCU | 20 | 1303 |
| myoC-208 | + | CCUCCAGAACUGACUUGUCU | 20 | 594 |
| myoC-726 | + | UUUCAGCACUGGGUUUAAGU | 20 | 1125 |
| myoC-225 | + | UGGCCUCCAGGUCUAAGCGU | 20 | 611 |
| myoC-729 | + | ACUGUCACCUCCACGAAGGU | 20 | 1083 |
| myoC-981 | - | CCAGGAGAAUUCCAGGAGGU | 20 | 1281 |
| myoC-232 | + | UCUGGGCAGCUGGAUUCAUU | 20 | 618 |
| myoC-169 | - | UGUGCACGUUGCUGCAGCUU | 20 | 555 |
| myoC-224 | + | CAGGGAGCUGAGUCGAGCUU | 20 | 610 |
| myoC-210 | + | CAGUCUCCAACUCUCUGGUU | 20 | 596 |
| myoC-885 | + | UAACCUUCCAGAAGUCUGUU | 20 | 1208 |
| myoC-830 | - | UACGUCUUAAAGGACUUGUU | 20 | 1209 |

**Table 9C** provides exemplary targeting domains for knocking down the *MYOC* gene selected according to the third tier parameters. The targeting domains bind within 3454-2454bp upstream of transcription start site or 500bp upstream and downstream of transcription start site and start with a 5'G. It is contemplated herein that in an embodiment the targeting domain hybridizes to the target domain through complementary base pairing. Any of the targeting domains in the table can be used with a S. pyogenes eiCas9 molecule or eiCas9 fusion protein (e.g., an eiCas9 fused to a transcription repressor domain) to alter the MYOC gene (e.g., reduce or eliminate MYOC gene expression, MYOC protein function, or the level of MYOC protein). One or more gRNA may be used to target an eiCas9 to the promoter region of the MYOC gene.

**Table 9C**

| 3rd Tier | | | | |
|---|---|---|---|---|
| gRNA Name | DNA Strand | Targeting Domain | Target Site Length | Seq ID |
| myoC-1373 | + | GCAGAGAAAAGAUAAAA | 17 | 1673 |
| myoC-1245 | - | GUUUCCUCAGAGGGAAA | 17 | 1545 |
| myoC-1233 | - | GGCUCCAGGCUCCAGAA | 17 | 1533 |
| myoC-1277 | - | GAAGUCUAUUUCAUGAA | 17 | 1577 |
| myoC-799 | + | GGAGGUGGCCUUGUUAA | 17 | 1037 |
| myoC-947 | + | GGGUGGGGCUGUGCACA | 17 | 1066 |
| myoC-159 | + | GUGGAGGAGGCUCUCCA | 17 | 549 |
| myoC-1268 | - | GGAAGGUGAAAAGGGCA | 17 | 1568 |
| myoC-768 | - | GAGGUGACAGUUUCUCA | 17 | 1025 |
| myoC-934 | + | GGGGAGCCAGCCCUUCA | 17 | 1065 |
| myoC-1243 | - | GACAGUGUUUCCUCAGA | 17 | 1543 |
| myoC-1265 | - | GGUGCUGAAAGGCAGGA | 17 | 1565 |
| myoC-132 | - | GACAGCUCAGCUCAGGA | 17 | 527 |
| myoC-926 | + | GCUGAGAGGUGCCUGGA | 17 | 1060 |
| myoC-168 | + | GGGACUGGCCACACUGA | 17 | 554 |
| myoC-795 | + | GGCAGCAGGGGGCGCUA | 17 | 1039 |
| myoC-907 | - | GAACUAUUAUUGGGGUA | 17 | 996 |
| myoC-958 | + | GGCACUAUGCUAGGAAC | 17 | 1062 |
| myoC-953 | + | GUACACACACUUACACC | 17 | 1070 |
| myoC-952 | + | GUUCCUAUAUCUCCACC | 17 | 1075 |
| myoC-756 | - | GGAGCCCUGCAAGCACC | 17 | 1035 |
| myoC-757 | - | GAGCCCUGCAAGCACCC | 17 | 1024 |
| myoC-164 | + | GGAUGACUGACAUGGCC | 17 | 551 |
| myoC-130 | - | GCUGCUUCUGGCCUGCC | 17 | 525 |
| myoC-826 | + | GAGAGGAAACCUCUGCC | 17 | 1023 |
| myoC-897 | - | GUUCCUAGCAUAGUGCC | 17 | 1074 |
| myoC-771 | - | GCUGCCUCCAUCGUGCC | 17 | 1030 |
| myoC-162 | + | GAGCUUUGGUGGCCUCC | 17 | 550 |
| myoC-1232 | - | GGAGACUCCUUGGCUCC | 17 | 1532 |
| myoC-158 | + | GGUGGAGGAGGCUCUCC | 17 | 548 |
| myoC-156 | + | GCCCCUCCUGGGUCUCC | 17 | 547 |
| myoC-759 | - | GCAAGCACCCGGGGUCC | 17 | 1027 |
| myoC-752 | - | GAGGUUUCCUCUCCAGC | 17 | 1026 |
| myoC-790 | + | GGCACGAUGGAGGCAGC | 17 | 1038 |
| myoC-165 | + | GCUCUGCUCUGGGCAGC | 17 | 552 |
| myoC-134 | - | GGGGCUGCAGAGGGAGC | 17 | 529 |
| myoC-1262 | - | GGACGCUGGGGCUGAGC | 17 | 1562 |
| myoC-1258 | - | GCAGGGAGUGGGGACGC | 17 | 1558 |
| myoC-137 | - | GCUGGGCACCCUGAGGC | 17 | 532 |
| myoC-825 | + | GGAGAGGAAACCUCUGC | 17 | 1034 |
| myoC-140 | - | GCAAGAAAAUGAGAAUC | 17 | 535 |
| myoC-1376 | + | GAACAACACUGAACAUC | 17 | 1676 |
| myoC-781 | + | GGAGGCUUGGAAGACUC | 17 | 1036 |
| myoC-154 | + | GGUCCCGCUCCCGCCUC | 17 | 545 |
| myoC-817 | + | GGGCUGGCAGGUUGCUC | 17 | 1042 |
| myoC-153 | + | GGCAGUCUCCAACUCUC | 17 | 544 |
| myoC-808 | + | GCUCACCAUUUUGUCUC | 17 | 1029 |
| myoC-1485 | - | GUGCUGUCCUUGUGUUC | 17 | 1785 |
| myoC-948 | + | GGUGGGGCUGUGCACAG | 17 | 1069 |
| myoC-812 | + | GAUAACAAAACAACCAG | 17 | 984 |
| myoC-3163 | + | GAAGAACCUCAUUGCAG | 17 | 2909 |
| myoC-1242 | - | GGACAGUGUUUCCUCAG | 17 | 1542 |
| myoC-1255 | - | GUGGGGACUGCAGGGAG | 17 | 1555 |
| myoC-824 | + | GGCUCCCCCAGCUGGAG | 17 | 1040 |
| myoC-1261 | - | GGGACGCUGGGGCUGAG | 17 | 1561 |
| myoC-949 | + | GUGGGGCUGUGCACAGG | 17 | 1072 |
| myoC-902 | - | GUGUGUGUAAAACCAGG | 17 | 1073 |
| myoC-133 | - | GCCCCAGGAGACCCAGG | 17 | 528 |
| myoC-778 | + | GUGACUGAUGGAGGAGG | 17 | 1046 |
| myoC-777 | + | GCUGUGACUGAUGGAGG | 17 | 1031 |
| myoC-943 | + | GGCCACGUGAGGCUGGG | 17 | 1063 |
| myoC-755 | - | GUUUCCUCUCCAGCUGG | 17 | 1047 |
| myoC-936 | + | GGAGCCAGCCCUUCAUG | 17 | 1061 |
| myoC-933 | + | GAGGUUUAUAUAUACUG | 17 | 1057 |
| myoC-136 | - | GGGAGCUGGGCACCCUG | 17 | 531 |
| myoC-754 | - | GGUUUCCUCUCCAGCUG | 17 | 1045 |
| myoC-1257 | - | GGGGACUGCAGGGAGUG | 17 | 1557 |
| myoC-1252 | - | GAGAAUUCCAGGAGGUG | 17 | 1552 |
| myoC-131 | - | GCCUGGUGUGGGAUGUG | 17 | 526 |
| myoC-1284 | - | GAAGGCCUUUAUUUAAU | 17 | 1584 |
| myoC-935 | + | GGGAGCCAGCCCUUCAU | 17 | 1064 |
| myoC-904 | - | GAUAUAGGAACUAUUAU | 17 | 1058 |
| myoC-135 | - | GGGCUGCAGAGGGAGCU | 17 | 530 |
| myoC-942 | + | GGUGGCCACGUGAGGCU | 17 | 1068 |
| myoC-957 | + | GUGCCAGGCACUAUGCU | 17 | 1071 |
| myoC-1251 | - | GGAGAAUUCCAGGAGGU | 17 | 1551 |
| myoC-944 | + | GCCACGUGAGGCUGGGU | 17 | 1059 |
| myoC-896 | - | GUCUUAAAGGACUUGUU | 17 | 1001 |
| myoC-689 | - | GCCAGACACCAGAGACAAAA | 20 | 1008 |
| myoC-997 | - | GAAAGGCAGGAAGGUGAAAA | 20 | 1297 |
| myoC-1007 | - | GAAGAAGUCUAUUUCAUGAA | 20 | 1307 |
| myoC-993 | - | GGGGCUGAGCGGGUGCUGAA | 20 | 1293 |
| myoC-881 | + | GCUGGGUGGGGCUGUGCACA | 20 | 1050 |
| myoC-120 | + | GGGCCUGGCAGCCUGGUCCA | 20 | 519 |
| myoC-998 | - | GCAGGAAGGUGAAAAGGGCA | 20 | 1298 |
| myoC-99 | - | GACCCAGGAGGGGCUGCAGA | 20 | 504 |
| myoC-718 | + | GGAGGCAGCAGGGGGCGCUA | 20 | 1015 |
| myoC-880 | + | GGCUGGGUGGGGCUGUGCAC | 20 | 1051 |
| myoC-1104 | + | GAAAAGAUAAAAAGGCUCAC | 20 | 1404 |
| myoC-835 | - | GUGUGUGUGUGUGUAAAACC | 20 | 1055 |
| myoC-742 | + | GCUCAGGACACCCAGGACCC | 20 | 1009 |
| myoC-97 | - | GGACCAGGCUGCCAGGCCCC | 20 | 502 |
| myoC-92 | - | GCUGCUGCUUCUGGCCUGCC | 20 | 498 |
| myoC-118 | + | GCUCCCUCUGCAGCCCCUCC | 20 | 517 |
| myoC-962 | - | GCUGGAGACUCCUUGGCUCC | 20 | 1262 |
| myoC-119 | + | GCAGCCCCUCCUGGGUCUCC | 20 | 518 |
| myoC-746 | + | GCUUGCAGGGCUCCCCCAGC | 20 | 1012 |
| myoC-676 | - | GCAGAGGUUUCCUCUCCAGC | 20 | 1006 |
| myoC-128 | + | GGCAGGCCAGAAGCAGCAGC | 20 | 524 |
| myoC-100 | - | GGAGGGGCUGCAGAGGGAGC | 20 | 505 |
| myoC-103 | - | GGAGCUGGGCACCCUGAGGC | 20 | 508 |
| myoC-748 | + | GCUGGAGAGGAAACCUCUGC | 20 | 1010 |
| myoC-863 | + | GCCUGGAUGGGUGGCCUUGC | 20 | 1049 |
| myoC-704 | + | GGAGGAGGCUUGGAAGACUC | 20 | 1014 |
| myoC-96 | - | GGGCCAGGACAGCUCAGCUC | 20 | 501 |
| myoC-116 | + | GUAGGCAGUCUCCAACUCUC | 20 | 483 |
| myoC-1019 | - | GUCUUUUCUUUCAUGUCUUC | 20 | 1319 |
| myoC-976 | - | GUGUUUCCUCAGAGGGAAAG | 20 | 1276 |
| myoC-715 | + | GGGCACGAUGGAGGCAGCAG | 20 | 1018 |
| myoC-98 | - | GGCCCCAGGAGACCCAGGAG | 20 | 503 |
| myoC-985 | - | GAGGUGGGGACUGCAGGGAG | 20 | 1285 |
| myoC-991 | - | GUGGGGACGCUGGGGCUGAG | 20 | 1291 |
| myoC-858 | + | GAAAGCUCUGCUGUGCUGAG | 20 | 1048 |
| myoC-716 | + | GGCACGAUGGAGGCAGCAGG | 20 | 1016 |
| myoC-701 | + | GCUGUGACUGAUGGAGGAGG | 20 | 1011 |
| myoC-102 | - | GGGAGCUGGGCACCCUGAGG | 20 | 507 |
| myoC-884 | + | GGGUGGGGCUGUGCACAGGG | 20 | 1052 |
| myoC-877 | + | GGUGGCCACGUGAGGCUGGG | 20 | 1053 |
| myoC-94 | - | GGCCUGCCUGGUGUGGGAUG | 20 | 499 |
| myoC-987 | - | GGUGGGGACUGCAGGGAGUG | 20 | 1287 |
| myoC-101 | - | GAGGGGCUGCAGAGGGAGCU | 20 | 506 |
| myoC-702 | + | GACUGAUGGAGGAGGAGGCU | 20 | 1004 |

**Table 9D** provides exemplary targeting domains for knocking down the *MYOC* gene selected according to the fourth tier parameters. The targeting domains bind within 3454-2454bp upstream of transcription start site or 500bp upstream and downstream of transcription start site. It is contemplated herein that in an embodiment the targeting domain hybridizes to the target domain through complementary base pairing. Any of the targeting domains in the table can be used with a S. pyogenes eiCas9 molecule or eiCas9 fusion protein (e.g., an eiCas9 fused to a transcription repressor domain) to alter the MYOC gene (e.g., reduce or eliminate MYOC gene expression, MYOC protein function, or the level of MYOC protein). One or more gRNA may be used to target an eiCas9 to the promoter region of the MYOC gene.

**Table 9D**

| 4th Tier | | | | |
|---|---|---|---|---|
| gRNA Name | DNA Strand | Targeting Domain | Target Site Length | Seq ID |
| myoC-765 | - | AGACACCAGAGACAAAA | 17 | 1133 |
| myoC-1267 | - | AGGCAGGAAGGUGAAAA | 17 | 1567 |
| myoC-1234 | - | AGGCUCCAGAAAGGAAA | 17 | 1534 |
| myoC-1266 | - | AAGGCAGGAAGGUGAAA | 17 | 1566 |
| myoC-1375 | + | AGGCUCACAGGAAGCAA | 17 | 1675 |
| myoC-769 | - | ACCCAGUGCUGAAAGAA | 17 | 1130 |
| myoC-1244 | - | UGUUUCCUCAGAGGGAA | 17 | 1544 |
| myoC-917 | - | CUGUCUUCCCCCAUGAA | 17 | 1244 |
| myoC-899 | - | UGAGUUUGCAGAGUGAA | 17 | 1254 |
| myoC-1370 | + | AUAUUCCCAUUAAAUAA | 17 | 1670 |
| myoC-5406 | + | CAGCCAGCCAGAACACA | 17 | 5152 |
| myoC-1385 | + | UCUGGAGCCUGGAGCCA | 17 | 1685 |
| myoC-293 | + | CCUGGCAGCCUGGUCCA | 17 | 679 |
| myoC-1279 | - | CAGCUGUUAAAAUUCCA | 17 | 1579 |
| myoC-922 | - | UCGGGCAUGAGCCAGCA | 17 | 1251 |
| myoC-1254 | - | AGGAGGUGGGGACUGCA | 17 | 1554 |
| myoC-1280 | - | AAAUUCCAGGGUGUGCA | 17 | 1580 |
| myoC-1269 | - | AUGUUCAGUGUUGUUCA | 17 | 1569 |
| myoC-265 | - | CCAGGAGGGGCUGCAGA | 17 | 651 |
| myoC-1236 | - | CAGAAAGGAAAUGGAGA | 17 | 1536 |
| myoC-262 | - | CCCCAGGAGACCCAGGA | 17 | 648 |
| myoC-912 | - | CCAAACAGACUUCUGGA | 17 | 1239 |
| myoC-916 | - | UCUGUCUUCCCCCAUGA | 17 | 1252 |
| myoC-1276 | - | AGAAGUCUAUUUCAUGA | 17 | 1576 |
| myoC-763 | - | UUUGUUAUCACUCUCUA | 17 | 1170 |
| myoC-299 | + | UGUCUCUCUGUAAGUUA | 17 | 685 |
| myoC-811 | + | CAGAAAUAGAAAGCAAC | 17 | 1149 |
| myoC-801 | + | UUCCUUUCUUUCAGCAC | 17 | 1168 |
| myoC-814 | + | AAAACAACCAGUGGCAC | 17 | 1127 |
| myoC-946 | + | UGGGUGGGGCUGUGCAC | 17 | 1257 |
| myoC-1374 | + | AAGAUAAAAAGGCUCAC | 17 | 1674 |
| myoC-1288 | - | UUCACGGAAGAAGUGAC | 17 | 1588 |
| myoC-901 | - | UGUGUGUGUGUAAAACC | 17 | 1258 |
| myoC-308 | + | CCCCCACAUCCCACACC | 17 | 694 |
| myoC-1372 | + | AAAACCCAU GCACACCC | 17 | 1672 |
| myoC-261 | - | CAGGCCCCAGGAGACCC | 17 | 647 |
| myoC-819 | + | CAGGACACCCAGGACCC | 17 | 1151 |
| myoC-820 | + | AGGACACCCAGGACCCC | 17 | 1138 |
| myoC-260 | - | CCAGGCUGCCAGGCCCC | 17 | 646 |
| myoC-292 | + | CCUGGGGCCUGGCAGCC | 17 | 678 |
| myoC-253 | - | CUGCCCAGAGCAGAGCC | 17 | 639 |
| myoC-1384 | + | AUUUCCUUUCUGGAGCC | 17 | 1684 |
| myoC-249 | - | UGUGGGAUGUGGGGGCC | 17 | 635 |
| myoC-291 | + | CUGGGUCUCCUGGGGCC | 17 | 677 |
| myoC-272 | - | AAAAUGAGAAUCUGGCC | 17 | 658 |
| myoC-810 | + | AAUUGUCAAUGAAUGCC | 17 | 1129 |
| myoC-259 | - | CCUUGGACCAGGCUGCC | 17 | 645 |
| myoC-925 | + | CUGUGCUGAGAGGUGCC | 17 | 1245 |
| myoC-956 | + | AGAACCUGCACUG UGCC | 17 | 1228 |
| myoC-1378 | + | CUGCAGUCCCCACCUCC | 17 | 1678 |
| myoC-287 | + | CCCUCUGCAGCCCCUCC | 17 | 673 |
| myoC-787 | + | CGGGCUUGGGGGCCUCC | 17 | 1155 |
| myoC-1379 | + | ACCUCCUGGAAUUCUCC | 17 | 1679 |
| myoC-900 | - | CAGCACACCAGUAGUCC | 17 | 1238 |
| myoC-307 | + | CCUGAGCUGAGCUGUCC | 17 | 693 |
| myoC-1278 | - | UCAGCUGUUAAAAUUCC | 17 | 1578 |
| myoC-311 | + | AGCAGCAGCUGGACAGC | 17 | 697 |
| myoC-823 | + | UGCAGGGCUCCCCCAGC | 17 | 1165 |
| myoC-286 | + | UGGUUUGGGUUUCCAGC | 17 | 672 |
| myoC-310 | + | AGGCCAGAAGCAGCAGC | 17 | 696 |
| myoC-267 | - | CACCCUGAGGCGGGAGC | 17 | 653 |
| myoC-309 | + | CACAUCCCACACCAGGC | 17 | 695 |
| myoC-941 | + | AGGUGGCCACGUGAGGC | 17 | 1233 |
| myoC-918 | - | CUUCCCCCAUGAAGGGC | 17 | 1246 |
| myoC-816 | + | CAGUGGCACGGGCUGGC | 17 | 1152 |
| myoC-1253 | - | CAGGAGGUGGGGACUGC | 17 | 1553 |
| myoC-898 | - | AGUGCCUGGCACAGUGC | 17 | 1234 |
| myoC-913 | - | UUUUCUAAGAAUCUUGC | 17 | 1260 |
| myoC-786 | + | UCGGGCUUGGGGGCCUC | 17 | 1162 |
| myoC-250 | - | CCAGGACAGCUCAGCUC | 17 | 636 |
| myoC-921 | - | AAACCUCUCUGGAGCUC | 17 | 1223 |
| myoC-300 | + | AUGGCCUGGCUCUGCUC | 17 | 686 |
| myoC-312 | + | UGGACAGCUGGCAUCUC | 17 | 698 |
| myoC-809 | + | AUUUUGUCUCUGGUGUC | 17 | 1144 |
| myoC-911 | - | AACUCCAAACAGACUUC | 17 | 1225 |
| myoC-243 | - | UGUCCAGCUGCUGCUUC | 17 | 629 |
| myoC-1289 | - | UUUUCUUUCAUGUCUUC | 17 | 1589 |
| myoC-1383 | + | CCUCUCCAUUUCCUUUC | 17 | 1683 |
| myoC-1246 | - | UUUCCUCAGAGGGAAAG | 17 | 1546 |
| myoC-938 | + | UUCAUGGGGGAAGACAG | 17 | 1259 |
| myoC-2657 | - | ACAGCAGAGCUUUCCAG | 17 | 2613 |
| myoC-792 | + | CACGAUGGAGGCAGCAG | 17 | 1148 |
| myoC-264 | - | CCCAGGAGGGGCUGCAG | 17 | 650 |
| myoC-251 | - | AAGGCCAAUGACCAGAG | 17 | 637 |
| myoC-263 | - | CCCAGGAGACCCAGGAG | 17 | 649 |
| myoC-1235 | - | CCAGAAAGGAAAUGGAG | 17 | 1535 |
| myoC-924 | + | AGCUCUGCUGUGCUGAG | 17 | 1232 |
| myoC-915 | - | CCCCACCCAGCCUCACG | 17 | 1241 |
| myoC-758 | - | AGCCCUGCAAGCACCCG | 17 | 1136 |
| myoC-273 | - | AUGAGAAUCUGGCCAGG | 17 | 659 |
| myoC-1249 | - | UCCAGGAGAAUUCCAGG | 17 | 1549 |
| myoC-793 | + | ACGAUGGAGGCAGCAGG | 17 | 1131 |
| myoC-939 | + | AUGGGGGAAGACAGAGG | 17 | 1237 |
| myoC-1250 | - | AGGAGAAUUCCAGGAGG | 17 | 1550 |
| myoC-282 | + | CUGACUUGUCUCGGAGG | 17 | 668 |
| myoC-797 | + | AGGGGGCGCUAGGGAGG | 17 | 1141 |
| myoC-266 | - | AGCUGGGCACCCUGAGG | 17 | 652 |
| myoC-950 | + | UGGGGCUGUGCACAGGG | 17 | 1256 |
| myoC-796 | + | AGCAGGGGGCGCUAGGG | 17 | 1135 |
| myoC-928 | + | AGAGGUGCCUGGAUGGG | 17 | 1229 |
| myoC-295 | + | CCAAGGUCAAUUGGUGG | 17 | 681 |
| myoC-248 | - | CCUGGUGUGGGAUGUGG | 17 | 634 |
| myoC-1275 | - | AUCUUUUCUCUGCUUGG | 17 | 1575 |
| myoC-246 | - | CUGCCUGGUGUGGGAUG | 17 | 632 |
| myoC-290 | + | CCCUCCUGGGUCUCCUG | 17 | 676 |
| myoC-1260 | - | AGGGAGUGGGGACGCUG | 17 | 1560 |
| myoC-1382 | + | AGGCCCCUUUCCCUCUG | 17 | 1682 |
| myoC-940 | + | ACAGAGGUGGCCACGUG | 17 | 1226 |
| myoC-945 | + | CCACGUGAGGCUGGGUG | 17 | 1240 |
| myoC-244 | - | UUCUGGCCUGCCUGGUG | 17 | 630 |
| myoC-3164 | + | AUUGCAGAGGCUUGGUG | 17 | 2910 |
| myoC-906 | - | UAUAGGAACUAUUAUUG | 17 | 1248 |
| myoC-784 | + | UGGAAGACUCGGGCUUG | 17 | 1166 |
| myoC-302 | + | UGGGCAGCUGGAUUCAU | 17 | 688 |
| myoC-927 | + | CUGAGAGGUGCCUGGAU | 17 | 1243 |
| myoC-1285 | - | UUAUUUAAUGGGAAUAU | 17 | 1585 |
| myoC-903 | - | AAACCAGGUGGAGAUAU | 17 | 1222 |
| myoC-908 | - | AACUAUUAUUGGGGUAU | 17 | 1224 |
| myoC-802 | + | UCCUUUCUUUCAGCACU | 17 | 1161 |
| myoC-780 | + | AGGAGGCUUGGAAGACU | 17 | 1139 |
| myoC-932 | + | AGAGGUUUAUAUAUACU | 17 | 1230 |
| myoC-1231 | - | CUGAGCUGGAGACUCCU | 17 | 1531 |
| myoC-288 | + | CCUCUGCAGCCCCUCCU | 17 | 674 |
| myoC-289 | + | CCCCUCCUGGGUCUCCU | 17 | 675 |
| myoC-760 | - | CAAGCACCCGGGGUCCU | 17 | 1147 |
| myoC-1286 | - | AAGCGAGCUCAUUUCCU | 17 | 1586 |
| myoC-753 | - | AGGUUUCCUCUCCAGCU | 17 | 1142 |
| myoC-920 | - | UAAACCUCUCUGGAGCU | 17 | 1247 |
| myoC-1259 | - | CAGGGAGUGGGGACGCU | 17 | 1559 |
| myoC-794 | + | AGGCAGCAGGGGGCGCU | 17 | 1140 |
| myoC-3165 | + | ACCUCAUUGCAGAGGCU | 17 | 2911 |
| myoC-779 | + | UGAUGGAGGAGGAGGCU | 17 | 1163 |
| myoC-1274 | - | UUUAUCUUUUCUCUGCU | 17 | 1574 |
| myoC-1377 | + | AACAACACUGAACAUCU | 17 | 1677 |
| myoC-301 | + | UGGCCUGGCUCUGCUCU | 17 | 687 |
| myoC-762 | - | UUUUGUUAUCACUCUCU | 17 | 1171 |
| myoC-1290 | - | UUUCUUUCAUGUCUUCU | 17 | 1590 |
| myoC-1256 | - | UGGGGACUGCAGGGAGU | 17 | 1556 |
| myoC-247 | - | UGCCUGGUGUGGGAUGU | 17 | 633 |
| myoC-283 | + | UCGGAGGAGGUUGCUGU | 17 | 669 |
| myoC-245 | - | UCUGGCCUGCCUGGUGU | 17 | 631 |
| myoC-905 | - | AUAUAGGAACUAUUAUU | 17 | 1235 |
| myoC-284 | + | UCUCCAACUCUCUGGUU | 17 | 670 |
| myoC-242 | - | CACGUUGCUGCAGCUUU | 17 | 628 |
| myoC-285 | + | CUCCAACUCUCUGGUUU | 17 | 671 |
| myoC-1103 | + | CAAGCAGAGAAAAGAUAAAA | 20 | 1403 |
| myoC-964 | - | UCCAGGCUCCAGAAAGGAAA | 20 | 1264 |
| myoC-996 | - | UGAAAGGCAGGAAGGUGAAA | 20 | 1296 |
| myoC-1105 | + | AAAAGGCUCACAGGAAGCAA | 20 | 1405 |
| myoC-693 | - | UAAACCCAGUGCUGAAAGAA | 20 | 1113 |
| myoC-963 | - | CUUGGCUCCAGGCUCCAGAA | 20 | 1263 |
| myoC-851 | - | CCUCUGUCUUCCCCCAUGAA | 20 | 1200 |
| myoC-833 | - | CAAUGAGUUUGCAGAGUGAA | 20 | 1188 |
| myoC-1100 | + | CCUAUAUUCCCAUUAAAUAA | 20 | 1400 |
| myoC-5407 | + | UAACAGCCAGCCAGAACACA | 20 | 5153 |
| myoC-1115 | + | CUUUCUGGAGCCUGGAGCCA | 20 | 1415 |
| myoC-223 | + | UUGGUGGAGGAGGCUCUCCA | 20 | 609 |
| myoC-984 | - | UCCAGGAGGUGGGGACUGCA | 20 | 1284 |
| myoC-966 | - | CUCCAGAAAGGAAAUGGAGA | 20 | 1266 |
| myoC-187 | - | AGGCCCCAGGAGACCCAGGA | 20 | 573 |
| myoC-175 | - | CAGGACAGCUCAGCUCAGGA | 20 | 561 |
| myoC-860 | + | UGUGCUGAGAGGUGCCUGGA | 20 | 1217 |
| myoC-850 | - | ACCUCUGUCUUCCCCCAUGA | 20 | 1175 |
| myoC-193 | - | AGGAAGAGAAGAAGCGACUA | 20 | 579 |
| myoC-687 | - | UGUUUUGUUAUCACUCUCUA | 20 | 1122 |
| myoC-734 | + | ACACAGAAAUAGAAAGCAAC | 20 | 1080 |
| myoC-892 | + | CCAGGCACUAUGCUAGGAAC | 20 | 1196 |
| myoC-724 | + | UAUUUCCUUUCUUUCAGCAC | 20 | 1116 |
| myoC-238 | + | UGGCCCCCACAUCCCACACC | 20 | 624 |
| myoC-887 | + | CACGUACACACACUUACACC | 20 | 1190 |
| myoC-185 | - | UGCCAGGCCCCAGGAGACCC | 20 | 571 |
| myoC-743 | + | CUCAGGACACCCAGGACCCC | 20 | 1107 |
| myoC-218 | + | UCUCCUGGGGCCUGGCAGCC | 20 | 604 |
| myoC-178 | - | CAGCUGCCCAGAGCAGAGCC | 20 | 564 |
| myoC-174 | - | UGGUGUGGGAUGUGGGGGCC | 20 | 560 |
| myoC-217 | + | CUCCUGGGUCUCCUGGGGCC | 20 | 603 |
| myoC-195 | - | AAGAAAAUGAGAAUCUGGCC | 20 | 581 |
| myoC-733 | + | AUAAAUUGUCAAUGAAUGCC | 20 | 1093 |
| myoC-184 | - | UGACCUUGGACCAGGCUGCC | 20 | 570 |
| myoC-749 | + | CUGGAGAGGAAACCUCUGCC | 20 | 1110 |
| myoC-831 | - | CCAGUUCCUAGCAUAGUGCC | 20 | 1198 |
| myoC-695 | - | CCUGCUGCCUCCAUCGUGCC | 20 | 1104 |
| myoC-890 | + | UUGAGAACCUGCACUGUGCC | 20 | 1221 |
| myoC-1108 | + | UCCCUGCAGUCCCCACCUCC | 20 | 1408 |
| myoC-834 | - | AAUCAGCACACCAGUAGUCC | 20 | 1174 |
| myoC-237 | + | CUUCCUGAGCUGAGCUGUCC | 20 | 623 |
| myoC-1008 | - | AAGUCAGCUGUUAAAAUUCC | 20 | 1308 |
| myoC-240 | + | AGAAGCAGCAGCUGGACAGC | 20 | 626 |
| myoC-230 | + | CUGGCUCUGCUCUGGGCAGC | 20 | 616 |
| myoC-992 | - | UGGGGACGCUGGGGCUGAGC | 20 | 1292 |
| myoC-988 | - | ACUGCAGGGAGUGGGGACGC | 20 | 1288 |
| myoC-852 | - | UGUCUUCCCCCAUGAAGGGC | 20 | 1216 |
| myoC-5408 | - | UGCUGUCCUUGUGUUCUGGC | 20 | 5154 |
| myoC-983 | - | UUCCAGGAGGUGGGGACUGC | 20 | 1283 |
| myoC-832 | - | CAUAGUGCCUGGCACAGUGC | 20 | 1194 |
| myoC-847 | - | UUAUUUUCUAAGAAUCUUGC | 20 | 1219 |
| myoC-194 | - | AAGGCAAGAAAAUGAGAAUC | 20 | 580 |
| myoC-731 | + | UUUGCUCACCAUUUUGUCUC | 20 | 1126 |
| myoC-845 | - | AGAAACUCCAAACAGACUUC | 20 | 1179 |
| myoC-1113 | + | UUCCCUCUCCAUUUCCUUUC | 20 | 1413 |
| myoC-882 | + | CUGGGUGGGGCUGUGCACAG | 20 | 1205 |
| myoC-872 | + | CCCUUCAUGGGGGAAGACAG | 20 | 1199 |
| myoC-2111 | - | AGCACAGCAGAGCUUUCCAG | 20 | 2233 |
| myoC-188 | - | AGACCCAGGAGGGGCUGCAG | 20 | 574 |
| myoC-176 | - | AGGAAGGCCAAUGACCAGAG | 20 | 562 |
| myoC-747 | + | CAGGGCUCCCCCAGCUGGAG | 20 | 1099 |
| myoC-849 | - | CAGCCCCACCCAGCCUCACG | 20 | 1193 |
| myoC-883 | + | UGGGUGGGGCUGUGCACAGG | 20 | 1215 |
| myoC-836 | - | UGUGUGUGUGUAAAACCAGG | 20 | 1218 |
| myoC-186 | - | CAGGCCCCAGGAGACCCAGG | 20 | 572 |
| myoC-196 | - | AAAAUGAGAAUCUGGCCAGG | 20 | 582 |
| myoC-873 | + | UUCAUGGGGGAAGACAGAGG | 20 | 1220 |
| myoC-862 | + | CUGAGAGGUGCCUGGAUGGG | 20 | 1202 |
| myoC-173 | - | CUGCCUGGUGUGGGAUGUGG | 20 | 559 |
| myoC-1005 | - | UUUAUCUUUUCUCUGCUUGG | 20 | 1305 |
| myoC-870 | + | UGGGGAGCCAGCCCUUCAUG | 20 | 1213 |
| myoC-189 | - | AGAGGGAGCUGGGCACCCUG | 20 | 575 |
| myoC-216 | + | AGCCCCUCCUGGGUCUCCUG | 20 | 602 |
| myoC-678 | - | AGAGGUUUCCUCUCCAGCUG | 20 | 1085 |
| myoC-990 | - | UGCAGGGAGUGGGGACGCUG | 20 | 1290 |
| myoC-1112 | + | UGGAGGCCCCUUUCCCUCUG | 20 | 1412 |
| myoC-874 | + | AAGACAGAGGUGGCCACGUG | 20 | 1172 |
| myoC-982 | - | CAGGAGAAUUCCAGGAGGUG | 20 | 1282 |
| myoC-879 | + | UGGCCACGUGAGGCUGGGUG | 20 | 1212 |
| myoC-171 | - | UGCUUCUGGCCUGCCUGGUG | 20 | 557 |
| myoC-172 | - | CCUGCCUGGUGUGGGAUGUG | 20 | 558 |
| myoC-231 | + | CUCUGGGCAGCUGGAUUCAU | 20 | 617 |
| myoC-869 | + | CUGGGGAGCCAGCCCUUCAU | 20 | 1204 |
| myoC-1015 | - | CCUUUAUUUAAUGGGAAUAU | 20 | 1315 |
| myoC-725 | + | AUUUCCUUUCUUUCAGCACU | 20 | 1095 |
| myoC-703 | + | AGGAGGAGGCUUGGAAGACU | 20 | 1090 |
| myoC-214 | + | CUCCCUCUGCAGCCCCUCCU | 20 | 600 |
| myoC-215 | + | CAGCCCCUCCUGGGUCUCCU | 20 | 601 |
| myoC-677 | - | CAGAGGUUUCCUCUCCAGCU | 20 | 1097 |
| myoC-989 | - | CUGCAGGGAGUGGGGACGCU | 20 | 1289 |
| myoC-717 | + | UGGAGGCAGCAGGGGGCGCU | 20 | 1120 |
| myoC-891 | + | ACUGUGCCAGGCACUAUGCU | 20 | 1178 |
| myoC-1004 | - | CUUUUUAUCUUUUCUCUGCU | 20 | 1304 |
| myoC-229 | + | ACAUGGCCUGGCUCUGCUCU | 20 | 615 |
| myoC-686 | - | UUGUUUUGUUAUCACUCUCU | 20 | 1124 |
| myoC-1020 | - | UCUUUUCUUUCAUGUCUUCU | 20 | 1320 |
| myoC-986 | - | AGGUGGGGACUGCAGGGAGU | 20 | 1286 |
| myoC-211 | + | AGUCUCCAACUCUCUGGUUU | 20 | 597 |

**Table 9E** provides exemplary targeting domains for knocking down the *MYOC* gene selected according to the fifth tier parameters. The targeting domains bind within 2484-903bp upstream of transcription start site or the additional 500bp upstream and downstream of transcription start site (extending to lkb up and downstream of the transcription start site). It is contemplated herein that in an embodiment the targeting domain hybridizes to the target domain through complementary base pairing. Any of the targeting domains in the table can be used with a S. pyogenes eiCas9 molecule or eiCas9 fusion protein (e.g., an eiCas9 fused to a transcription repressor domain) to alter the MYOC gene (e.g., reduce or eliminate MYOC gene expression, MYOC protein function, or the level of MYOC protein). One or more gRNA may be used to target an eiCas9 to the promoter region of the MYOC gene.

**Table 9E**

| 5th Tier | | | | |
|---|---|---|---|---|
| gRNA Name | DNA Strand | Targeting Domain | Target Site Length | Seq ID |
| myoC-5409 | + | GAGCAAAGGUUCAAAAA | 17 | 5155 |
| myoC-5410 | + | AGGAUAGUUUUUCAAAA | 17 | 5156 |
| myoC-1453 | + | CUUGAGACAUUUACAAA | 17 | 1753 |
| myoC-1456 | + | GUUUACAGCUGACCAAA | 17 | 1756 |
| myoC-1449 | + | AAAAAACAAAAAGCAAA | 17 | 1749 |
| myoC-5411 | - | UCACAGUCCAUAGCAAA | 17 | 5157 |
| myoC-5412 | + | GUCAUUUUAACAUCAAA | 17 | 5158 |
| myoC-5413 | + | AAGGAUAGUUUUUCAAA | 17 | 5159 |
| myoC-1460 | + | CUUCCUGUUAAAAGAAA | 17 | 1760 |
| myoC-5414 | + | GCAGUCUCUAGGAGAAA | 17 | 5160 |
| myoC-5415 | - | GCAAAAGGAGAAAUAAA | 17 | 5161 |
| myoC-1420 | - | UGGAGUUAGCAGCACAA | 17 | 1720 |
| myoC-1332 | - | UCCCUAAGCAUAGACAA | 17 | 1632 |
| myoC-1497 | + | UAAAAUAUAGAUUACAA | 17 | 1797 |
| myoC-1364 | + | GUCGCACAGCCAACCAA | 17 | 1664 |
| myoC-1455 | + | UGUUUACAGCUGACCAA | 17 | 1755 |
| myoC-5416 | + | AAUAACAAUCUGAGCAA | 17 | 5162 |
| myoC-1443 | + | UAUGGCUCUAUUCGCAA | 17 | 1743 |
| myoC-1358 | + | GAACACGAGAGCUGCAA | 17 | 1658 |
| myoC-1432 | - | AACAUAAAGUUGCUCAA | 17 | 1732 |
| myoC-1395 | - | AAGACAGAUUCAUUCAA | 17 | 1695 |
| myoC-1412 | - | GGAAAAAAUCAGUUCAA | 17 | 1712 |
| myoC-5417 | + | UGCAGUCUCUAGGAGAA | 17 | 5163 |
| myoC-145 | - | GGUAGCAAGGCUGAGAA | 17 | 540 |
| myoC-1463 | + | AAUUACUCAGCUUGUAA | 17 | 1763 |
| myoC-1367 | + | AAGCCAAGUCCACCACA | 17 | 1667 |
| myoC-1399 | - | AGUGGGAAUUGACCACA | 17 | 1699 |
| myoC-1317 | - | GGAGCAGCUGAGCCACA | 17 | 1617 |
| myoC-1419 | - | GUGGAGUUAGCAGCACA | 17 | 1719 |
| myoC-1356 | + | CCUCACAGAGAAUCACA | 17 | 1656 |
| myoC-1415 | - | AUUCUGAGCAAGUCACA | 17 | 1715 |
| myoC-5418 | + | GACUGUGAAAACUGACA | 17 | 5164 |
| myoC-1361 | + | GAGAAGACUAUGGCCCA | 17 | 1661 |
| myoC-1363 | + | GGAGAGACACUUGCCCA | 17 | 1663 |
| myoC-1429 | - | UGGAGGUGAGUCUGCCA | 17 | 1729 |
| myoC-1488 | + | CACCCUACCAGGCUCCA | 17 | 1788 |
| myoC-1365 | + | CGAGUCUCCUGAUUCCA | 17 | 1665 |
| myoC-1439 | - | UUUAUUAAUGUAAAGCA | 17 | 1739 |
| myoC-1387 | - | AGUGACUGCUGACAGCA | 17 | 1687 |
| myoC-144 | - | GCAGCCAGGAGGUAGCA | 17 | 539 |
| myoC-1389 | - | GAGUGACCUGCAGCGCA | 17 | 1689 |
| myoC-5419 | + | AGGAGAAAGGGCAGGCA | 17 | 5165 |
| myoC-1405 | - | CUGGGUUCUAGGAGGCA | 17 | 1705 |
| myoC-1357 | + | AGAACACGAGAGCUGCA | 17 | 1657 |
| myoC-1474 | + | GCGUGGGGUGCUGGUCA | 17 | 1774 |
| myoC-1394 | - | AAAGACAGAUUCAUUCA | 17 | 1694 |
| myoC-1411 | - | GGGAAAAAAUCAGUUCA | 17 | 1711 |
| myoC-1294 | - | ACUUGGCUUAUGCAAGA | 17 | 1594 |
| myoC-1393 | - | AGGAGAAGAAAAAGAGA | 17 | 1693 |
| myoC-3167 | - | CCACCAGGCUCCAGAGA | 17 | 2913 |
| myoC-274 | - | AGGUAGCAAGGCUGAGA | 17 | 660 |
| myoC-1311 | - | GAGGGGGGAUGUUGAGA | 17 | 1611 |
| myoC-1444 | + | UGUUAAAUUUAGUUAGA | 17 | 1744 |
| myoC-1326 | - | GGUGGAGGGGGACAGGA | 17 | 1626 |
| myoC-1362 | + | AGACUAUGGCCCAGGGA | 17 | 1662 |
| myoC-1345 | + | UUGUCUAUGCUUAGGGA | 17 | 1645 |
| myoC-1313 | - | GGGAUGUUGAGAGGGGA | 17 | 1613 |
| myoC-5420 | + | GUGAAAACUGACAUGGA | 17 | 5166 |
| myoC-1322 | - | GCCACAGGGGAGGUGGA | 17 | 1622 |
| myoC-1353 | + | UGAUCAGUGAGGACUGA | 17 | 1653 |
| myoC-2616 | - | UUUAAAGCUAGGGGUGA | 17 | 2581 |
| myoC-1306 | - | CCUGUGAUUCUCUGUGA | 17 | 1606 |
| myoC-5421 | + | UUACUAGUAAUACUUGA | 17 | 5167 |
| myoC-1462 | + | AAAAAGAGUUCCUAAUA | 17 | 1762 |
| myoC-5422 | - | GAGUUCAGCAGGUGAUA | 17 | 5168 |
| myoC-1359 | + | UAUAGCAGAGAAGACUA | 17 | 1659 |
| myoC-271 | - | AAGAGAAGAAGCGACUA | 17 | 657 |
| myoC-5423 | - | CAGUUGUUUUAAAGCUA | 17 | 5169 |
| myoC-5424 | + | UAUUUCUCCUUUUGCUA | 17 | 5170 |
| myoC-1484 | - | CUCCCUGGAGCCUGGUA | 17 | 1784 |
| myoC-5425 | - | ACAAGACAGAUGAAUUA | 17 | 5171 |
| myoC-1344 | + | GCCAUUGUCUAUGCUUA | 17 | 1644 |
| myoC-1442 | + | UUACCACUUUGAGUUUA | 17 | 1742 |
| myoC-1336 | - | GCCUGGCAUUCAAAAAC | 17 | 1636 |
| myoC-1461 | + | UUCCUGUUAAAAGAAAC | 17 | 1761 |
| myoC-1333 | - | AGAAUGCAGAGACUAAC | 17 | 1633 |
| myoC-1421 | - | AUCCCGUUUCUUUUAAC | 17 | 1721 |
| myoC-279 | + | CUCGGGUCUGGGGACAC | 17 | 665 |
| myoC-1366 | + | UAAGCCAAGUCCACCAC | 17 | 1666 |
| myoC-1398 | - | CAGUGGGAAUUGACCAC | 17 | 1698 |
| myoC-1316 | - | UGGAGCAGCUGAGCCAC | 17 | 1616 |
| myoC-5426 | + | GGUAAUGACAAAAUCAC | 17 | 5172 |
| myoC-1355 | + | CCCUCACAGAGAAUCAC | 17 | 1655 |
| myoC-1446 | + | UCCUCAUUCAAAUUCAC | 17 | 1746 |
| myoC-5427 | - | AGGAGAAAUAAAAGGAC | 17 | 5173 |
| myoC-1325 | - | GGGAGGUGGAGGGGGAC | 17 | 1625 |
| myoC-5428 | - | UCGUAGUGACCUGCUAC | 17 | 5174 |
| myoC-148 | - | GCUCGGGCUGUGCCACC | 17 | 490 |
| myoC-5429 | + | UGCAGACACAUCUCACC | 17 | 5175 |
| myoC-5430 | - | GGAGAAAUAAAAGGACC | 17 | 5176 |
| myoC-1486 | + | AAGGACAGCACCCUACC | 17 | 1786 |
| myoC-1465 | + | CCUGCCUCCUAGAACCC | 17 | 1765 |
| myoC-1360 | + | AGAGAAGACUAUGGCCC | 17 | 1660 |
| myoC-1450 | + | AUAUUUCCAAACUGCCC | 17 | 1750 |
| myoC-1481 | - | UAUAGGAAUGCUCUCCC | 17 | 1781 |
| myoC-1303 | - | AAGUGUCUCUCCUUCCC | 17 | 1603 |
| myoC-142 | - | GUUGGAAAGCAGCAGCC | 17 | 537 |
| myoC-1482 | - | AUGCUCUCCCUGGAGCC | 17 | 1782 |
| myoC-3169 | + | UUACCUUCUCUGGAGCC | 17 | 2915 |
| myoC-5431 | + | GGGCAGGCAGGGAGGCC | 17 | 5177 |
| myoC-272 | - | AAAAUGAGAAUCUGGCC | 17 | 658 |
| myoC-1340 | + | CCCAGUUUUUGAAUGCC | 17 | 1640 |
| myoC-1428 | - | CUGGAGGUGAGUCUGCC | 17 | 1728 |
| myoC-1335 | - | UGGUGGUAGCUUUUGCC | 17 | 1635 |
| myoC-1400 | - | UAUAGUCCACGUGAUCC | 17 | 1700 |
| myoC-1330 | - | UGAUCACGUCAGACUCC | 17 | 1630 |
| myoC-151 | + | GCUGCUUUCCAACCUCC | 17 | 543 |
| myoC-280 | + | UCAGCCUUGCUACCUCC | 17 | 666 |
| myoC-5432 | - | CCUGCUACAGGCGCUCC | 17 | 5178 |
| myoC-1487 | + | GCACCCUACCAGGCUCC | 17 | 1787 |
| myoC-1351 | + | AUUGUGGCUCUCGGUCC | 17 | 1651 |
| myoC-1438 | - | GUUUAUUAAUGUAAAGC | 17 | 1738 |
| myoC-1328 | - | GGAAGGCAGGCAGAAGC | 17 | 1628 |
| myoC-1498 | + | UAAAAACAAGAUCCAGC | 17 | 1798 |
| myoC-5433 | - | GGGACUCUGAGUUCAGC | 17 | 5179 |
| myoC-1315 | - | GGGGAAGGAGGCAGAGC | 17 | 1615 |
| myoC-5434 | + | CCUGGAGCGCCUGUAGC | 17 | 5180 |
| myoC-1388 | - | GGAGUGACCUGCAGCGC | 17 | 1688 |
| myoC-1327 | - | GAGGGGGACAGGAAGGC | 17 | 1627 |
| myoC-5435 | + | UAGGAGAAAGGGCAGGC | 17 | 5181 |
| myoC-1404 | - | CCUGGGUUCUAGGAGGC | 17 | 1704 |
| myoC-5436 | + | UCUCUAGGAGAAAGGGC | 17 | 5182 |
| myoC-1475 | - | GAAAUUAGACCUCCUGC | 17 | 1775 |
| myoC-1468 | + | CUCCUCCCCUGCGCUGC | 17 | 1768 |
| myoC-1472 | + | UGAGCUGCGUGGGGUGC | 17 | 1772 |
| myoC-1464 | + | AUAUAGUAUUAGAAAUC | 17 | 1764 |
| myoC-1494 | + | ACCUCAUUGGUGAAAUC | 17 | 1794 |
| myoC-140 | - | GCAAGAAAAUGAGAAUC | 17 | 535 |
| myoC-1296 | - | UCGAAAACCUUGGAAUC | 17 | 1596 |
| myoC-1426 | - | ACUGUGUUUCUCCACUC | 17 | 1726 |
| myoC-147 | - | GACCCGAGACACUGCUC | 17 | 489 |
| myoC-1339 | + | GCAUUUUCCACUUGCUC | 17 | 1639 |
| myoC-5437 | + | AAAAGUUUAACAAUCUC | 17 | 5183 |
| myoC-1492 | + | UUUCAGUCUUGCAUCUC | 17 | 1792 |
| myoC-5438 | + | AUCUAAAUGAAGCUCUC | 17 | 5184 |
| myoC-277 | + | AGCCCGAGCAGUGUCUC | 17 | 663 |
| myoC-3170 | + | UGCAUUCUUACCUUCUC | 17 | 2916 |
| myoC-1414 | - | UCAGUUCAAGGGAAGUC | 17 | 1714 |
| myoC-149 | + | GAGCAGUGUCUCGGGUC | 17 | 491 |
| myoC-1473 | + | UGCGUGGGGUGCUGGUC | 17 | 1773 |
| myoC-1331 | - | GAGAGCCACAAUGCUUC | 17 | 1631 |
| myoC-1425 | - | GUAAAUGUCUCAAGUUC | 17 | 1725 |
| myoC-1485 | - | GUGCUGUCCUUGUGUUC | 17 | 1785 |
| myoC-1447 | + | CAAAUUCACAGGCUUUC | 17 | 1747 |
| myoC-1298 | - | AGACUCGGUUUUCUUUC | 17 | 1598 |
| myoC-1441 | - | GAGCCAUAAACUCAAAG | 17 | 1741 |
| myoC-1338 | - | AACUGGGCCAGAGCAAG | 17 | 1638 |
| myoC-1433 | - | GCAAUCAUUAUUUCAAG | 17 | 1733 |
| myoC-146 | - | GUAGCAAGGCUGAGAAG | 17 | 541 |
| myoC-1489 | + | GAGCAUUCCUAUAGAAG | 17 | 1789 |
| myoC-1318 | - | GAGCAGCUGAGCCACAG | 17 | 1618 |
| myoC-1390 | - | AGUGACCUGCAGCGCAG | 17 | 1690 |
| myoC-1396 | - | GAUUCAUUCAAGGGCAG | 17 | 1696 |
| myoC-1392 | - | GAGGAGAAGAAAAAGAG | 17 | 1692 |
| myoC-1451 | + | CAGACUCACCUCCAGAG | 17 | 1751 |
| myoC-3171 | - | AAGGUAAGAAUGCAGAG | 17 | 2917 |
| myoC-1312 | - | AGGGGGGAUGUUGAGAG | 17 | 1612 |
| myoC-5439 | + | UGAAAACUGACAUGGAG | 17 | 5185 |
| myoC-1323 | - | CCACAGGGGAGGUGGAG | 17 | 1623 |
| myoC-2617 | - | UUAAAGCUAGGGGUGAG | 17 | 2582 |
| myoC-1307 | - | CUGUGAUUCUCUGUGAG | 17 | 1607 |
| myoC-1310 | - | UGAGGGGGGAUGUUGAG | 17 | 1610 |
| myoC-5440 | - | AGUUGUUUUAAAGCUAG | 17 | 5186 |
| myoC-5441 | - | AGCUUCAUUUAGAUUAG | 17 | 5187 |
| myoC-1478 | - | AGUAAGAACUGAUUUAG | 17 | 1778 |
| myoC-1466 | + | UAGAACCCAGGAUCACG | 17 | 1766 |
| myoC-1301 | - | GGUUGGCUGUGCGACCG | 17 | 1601 |
| myoC-1469 | + | GUCACUGCUGAGCUGCG | 17 | 1769 |
| myoC-1445 | + | UAAAUUUAGUUAGAAGG | 17 | 1745 |
| myoC-1314 | - | AUGUUGAGAGGGGAAGG | 17 | 1614 |
| myoC-143 | - | GGAAAGCAGCAGCCAGG | 17 | 538 |
| myoC-273 | - | AUGAGAAUCUGGCCAGG | 17 | 659 |
| myoC-1499 | + | AAACAAGAUCCAGCAGG | 17 | 1799 |
| myoC-1320 | - | CUGAGCCACAGGGGAGG | 17 | 1620 |
| myoC-1324 | - | CACAGGGGAGGUGGAGG | 17 | 1624 |
| myoC-2618 | - | UAAAGCUAGGGGUGAGG | 17 | 2583 |
| myoC-1308 | - | UGUGAUUCUCUGUGAGG | 17 | 1608 |
| myoC-1403 | - | UGAUCCUGGGUUCUAGG | 17 | 1703 |
| myoC-5442 | + | AGAAAGGGCAGGCAGGG | 17 | 5188 |
| myoC-2619 | - | AAAGCUAGGGGUGAGGG | 17 | 2584 |
| myoC-1309 | - | GUGAUUCUCUGUGAGGG | 17 | 1609 |
| myoC-1319 | - | CAGCUGAGCCACAGGGG | 17 | 1619 |
| myoC-1391 | - | GACCUGCAGCGCAGGGG | 17 | 1691 |
| myoC-3172 | - | UAAGAAUGCAGAGUGGG | 17 | 2918 |
| myoC-1410 | - | CAGGGCUAUAUUGUGGG | 17 | 1710 |
| myoC-5443 | + | UGUGAAAACUGACAUGG | 17 | 5189 |
| myoC-1334 | - | AUGCAGAGACUAACUGG | 17 | 1634 |
| myoC-3173 | + | CCUUCUCUGGAGCCUGG | 17 | 2919 |
| myoC-1427 | - | GUGUUUCUCCACUCUGG | 17 | 1727 |
| myoC-3174 | - | GUAAGAAUGCAGAGUGG | 17 | 2920 |
| myoC-1321 | - | AGCCACAGGGGAGGUGG | 17 | 1621 |
| myoC-1292 | - | ACUACUCAGCCCUGUGG | 17 | 1592 |
| myoC-1409 | - | GCAGGGCUAUAUUGUGG | 17 | 1709 |
| myoC-1346 | + | CUUAGGGAAGGAAAAUG | 17 | 1646 |
| myoC-1476 | - | CCCAGAUUUCACCAAUG | 17 | 1776 |
| myoC-1440 | - | GCCUGUGAAUUUGAAUG | 17 | 1740 |
| myoC-1416 | - | UCACAAGGUAGUAACUG | 17 | 1716 |
| myoC-1354 | + | CCCCUCCACCUCCCCUG | 17 | 1654 |
| myoC-1291 | - | GCAACUACUCAGCCCUG | 17 | 1591 |
| myoC-1467 | + | GUGGACUAUAAUCCCUG | 17 | 1767 |
| myoC-1496 | + | CUCAUUGGUGAAAUCUG | 17 | 1796 |
| myoC-1418 | - | GGAACUCUUUUUCUCUG | 17 | 1718 |
| myoC-150 | + | GCAGUGUCUCGGGUCUG | 17 | 542 |
| myoC-1352 | + | GUCUGACGUGAUCAGUG | 17 | 1652 |
| myoC-3175 | - | GGUAAGAAUGCAGAGUG | 17 | 2921 |
| myoC-1471 | + | CACUGCUGAGCUGCGUG | 17 | 1771 |
| myoC-2615 | - | UUUUAAAGCUAGGGGUG | 17 | 2580 |
| myoC-1305 | - | CCCUGUGAUUCUCUGUG | 17 | 1605 |
| myoC-1408 | - | GGCAGGGCUAUAUUGUG | 17 | 1708 |
| myoC-1349 | + | GCUUUCCUGAAGCAUUG | 17 | 1649 |
| myoC-1406 | - | GAGGCAGGGCUAUAUUG | 17 | 1706 |
| myoC-1454 | + | UUGAGACAUUUACAAAU | 17 | 1754 |
| myoC-1479 | - | GAGGCUAACAUUGACAU | 17 | 1779 |
| myoC-1299 | - | CUUUCUGGUUCUGCCAU | 17 | 1599 |
| myoC-1493 | + | CAUGCCAAGAACCUCAU | 17 | 1793 |
| myoC-1423 | - | CUUGCUGACUAUAUGAU | 17 | 1723 |
| myoC-1452 | + | UUCUAUUCUUAUUUGAU | 17 | 1752 |
| myoC-1480 | - | AAAUCUGCCGCUUCUAU | 17 | 1780 |
| myoC-1500 | + | AUGUCUGUGAUUUCUAU | 17 | 1800 |
| myoC-1342 | + | GCAUUCUUUUUGGUUAU | 17 | 1642 |
| myoC-1435 | - | AGUUUUGGUAUAUUUAU | 17 | 1735 |
| myoC-1337 | - | CCUGGCAUUCAAAAACU | 17 | 1637 |
| myoC-1297 | - | CUUGGAAUCAGGAGACU | 17 | 1597 |
| myoC-1293 | - | CAGCCCUGUGGUGGACU | 17 | 1593 |
| myoC-1295 | - | AAGACGGUCGAAAACCU | 17 | 1595 |
| myoC-1457 | + | UAUAGUCAGCAAGACCU | 17 | 1757 |
| myoC-1304 | - | AGUGUCUCUCCUUCCCU | 17 | 1604 |
| myoC-1422 | - | CAAACAGAUUCAAGCCU | 17 | 1722 |
| myoC-1401 | - | AUAGUCCACGUGAUCCU | 17 | 1701 |
| myoC-2612 | - | ACAGUUGUUUUAAAGCU | 17 | 2577 |
| myoC-1329 | - | GAAGGCAGGCAGAAGCU | 17 | 1629 |
| myoC-275 | - | AGACCCGAGACACUGCU | 17 | 661 |
| myoC-1495 | + | CCUCAUUGGUGAAAUCU | 17 | 1795 |
| myoC-1350 | + | UGAAGCAUUGUGGCUCU | 17 | 1650 |
| myoC-5444 | + | CUAGCUGUGCAGUCUCU | 17 | 5190 |
| myoC-278 | + | AGCAGUGUCUCGGGUCU | 17 | 664 |
| myoC-276 | + | CAGCCCGAGCAGUGUCU | 17 | 662 |
| myoC-1290 | - | UUUCUUUCAUGUCUUCU | 17 | 1590 |
| myoC-1477 | - | UUCACCAAUGAGGUUCU | 17 | 1777 |
| myoC-1402 | - | ACGUGAUCCUGGGUUCU | 17 | 1702 |
| myoC-1413 | - | AUCAGUUCAAGGGAAGU | 17 | 1713 |
| myoC-1397 | - | AUUCAUUCAAGGGCAGU | 17 | 1697 |
| myoC-3177 | - | AGGUAAGAAUGCAGAGU | 17 | 2923 |
| myoC-1302 | - | GUUGGCUGUGCGACCGU | 17 | 1602 |
| myoC-1470 | + | UCACUGCUGAGCUGCGU | 17 | 1770 |
| myoC-141 | - | GAAUCUGGCCAGGAGGU | 17 | 536 |
| myoC-1483 | - | UCUCCCUGGAGCCUGGU | 17 | 1783 |
| myoC-1300 | - | CUGGUUCUGCCAUUGGU | 17 | 1600 |
| myoC-1448 | + | CAGGCUUUCUGGACUGU | 17 | 1748 |
| myoC-1347 | + | AAGGAAAAUGUGGCUGU | 17 | 1647 |
| myoC-1407 | - | AGGCAGGGCUAUAUUGU | 17 | 1707 |
| myoC-1431 | - | AGUAUUGACACUGUUGU | 17 | 1731 |
| myoC-1417 | - | CUUAGUUUCUCCUUAUU | 17 | 1717 |
| myoC-1424 | - | AUGAGACUAGUACCCUU | 17 | 1724 |
| myoC-1343 | + | UGCCAUUGUCUAUGCUU | 17 | 1643 |
| myoC-1490 | + | AAACAACUGUGUAUCUU | 17 | 1790 |
| myoC-1459 | + | UGUUUGGCUUUACUCUU | 17 | 1759 |
| myoC-1436 | - | UUUUUGUUUUUUCUCUU | 17 | 1736 |
| myoC-1430 | - | AGUCUGCCAGGGCAGUU | 17 | 1730 |
| myoC-1348 | + | AGGAAAAUGUGGCUGUU | 17 | 1648 |
| myoC-1458 | + | CUAGGCUUGAAUCUGUU | 17 | 1758 |
| myoC-1491 | + | AACAACUGUGUAUCUUU | 17 | 1791 |
| myoC-1437 | - | UUUUGUUUUUUCUCUUU | 17 | 1737 |
| myoC-1434 | - | GUUACUUCUGACAGUUU | 17 | 1734 |
| myoC-1341 | + | AGUCUCUGCAUUCUUUU | 17 | 1641 |
| myoC-5445 | + | UCUGAGCAAAGGUUCAAAAA | 20 | 5191 |
| myoC-5446 | + | AAAAGGAUAGUUUUUCAAAA | 20 | 5192 |
| myoC-1183 | + | GAACUUGAGACAUUUACAAA | 20 | 1483 |
| myoC-1186 | + | UUUGUUUACAGCUGACCAAA | 20 | 1486 |
| myoC-1179 | + | GAGAAAAAACAAAAAGCAAA | 20 | 1479 |
| myoC-5447 | - | UUUUCACAGUCCAUAGCAAA | 20 | 5193 |
| myoC-5448 | + | AAGGUCAUUUUAACAUCAAA | 20 | 5194 |
| myoC-5449 | + | AAAAAGGAUAGUUUUUCAAA | 20 | 5195 |
| myoC-1190 | + | UUUCUUCCUGUUAAAAGAAA | 20 | 1490 |
| myoC-5450 | + | UGUGCAGUCUCUAGGAGAAA | 20 | 5196 |
| myoC-5451 | - | AUAGCAAAAGGAGAAAUAAA | 20 | 5197 |
| myoC-1150 | - | CUGUGGAGUUAGCAGCACAA | 20 | 1450 |
| myoC-1062 | - | CCUUCCCUAAGCAUAGACAA | 20 | 1362 |
| myoC-1227 | + | AUAUAAAAUAUAGAUUACAA | 20 | 1527 |
| myoC-1094 | + | ACGGUCGCACAGCCAACCAA | 20 | 1394 |
| myoC-1185 | + | GUUUGUUUACAGCUGACCAA | 20 | 1485 |
| myoC-5452 | + | ACAAAUAACAAUCUGAGCAA | 20 | 5198 |
| myoC-1173 | + | GUUUAUGGCUCUAUUCGCAA | 20 | 1473 |
| myoC-1088 | + | ACAGAACACGAGAGCUGCAA | 20 | 1388 |
| myoC-1162 | - | AACAACAUAAAGUUGCUCAA | 20 | 1462 |
| myoC-1125 | - | AGAAAGACAGAUUCAUUCAA | 20 | 1425 |
| myoC-1142 | - | GGGGGAAAAAAUCAGUUCAA | 20 | 1442 |
| myoC-5453 | + | CUGUGCAGUCUCUAGGAGAA | 20 | 5199 |
| myoC-110 | - | GGAGGUAGCAAGGCUGAGAA | 20 | 513 |
| myoC-1193 | + | CAGAAUUACUCAGCUUGUAA | 20 | 1493 |
| myoC-1097 | + | CAUAAGCCAAGUCCACCACA | 20 | 1397 |
| myoC-1129 | - | GGCAGUGGGAAUUGACCACA | 20 | 1429 |
| myoC-1047 | - | GCUGGAGCAGCUGAGCCACA | 20 | 1347 |
| myoC-1149 | - | UCUGUGGAGUUAGCAGCACA | 20 | 1449 |
| myoC-1086 | + | CCCCCUCACAGAGAAUCACA | 20 | 1386 |
| myoC-1145 | - | GUAAUUCUGAGCAAGUCACA | 20 | 1445 |
| myoC-5454 | + | AUGGACUGUGAAAACUGACA | 20 | 5200 |
| myoC-1091 | + | GCAGAGAAGACUAUGGCCCA | 20 | 1391 |
| myoC-1093 | + | GAAGGAGAGACACUUGCCCA | 20 | 1393 |
| myoC-1159 | - | CUCUGGAGGUGAGUCUGCCA | 20 | 1459 |
| myoC-1218 | + | CAGCACCCUACCAGGCUCCA | 20 | 1518 |
| myoC-1095 | + | AACCGAGUCUCCUGAUUCCA | 20 | 1395 |
| myoC-1169 | - | GGGUUUAUUAAUGUAAAGCA | 20 | 1469 |
| myoC-1117 | - | AGCAGUGACUGCUGACAGCA | 20 | 1417 |
| myoC-109 | - | GCAGCAGCCAGGAGGUAGCA | 20 | 512 |
| myoC-1119 | - | ACGGAGUGACCUGCAGCGCA | 20 | 1419 |
| myoC-5455 | + | UCUAGGAGAAAGGGCAGGCA | 20 | 5201 |
| myoC-1135 | - | AUCCUGGGUUCUAGGAGGCA | 20 | 1435 |
| myoC-1087 | + | CACAGAACACGAGAGCUGCA | 20 | 1387 |
| myoC-1204 | + | GCUGCGUGGGGUGCUGGUCA | 20 | 1504 |
| myoC-1124 | - | AAGAAAGACAGAUUCAUUCA | 20 | 1424 |
| myoC-1141 | - | GGGGGGAAAAAAUCAGUUCA | 20 | 1441 |
| myoC-1024 | - | UGGACUUGGCUUAUGCAAGA | 20 | 1324 |
| myoC-1123 | - | GGGAGGAGAAGAAAAAGAGA | 20 | 1423 |
| myoC-3181 | - | GUGCCACCAGGCUCCAGAGA | 20 | 2927 |
| myoC-198 | - | AGGAGGUAGCAAGGCUGAGA | 20 | 584 |
| myoC-1041 | - | UGUGAGGGGGGAUGUUGAGA | 20 | 1341 |
| myoC-1174 | + | AAAUGUUAAAUUUAGUUAGA | 20 | 1474 |
| myoC-1056 | - | GGAGGUGGAGGGGGACAGGA | 20 | 1356 |
| myoC-1092 | + | AGAAGACUAUGGCCCAGGGA | 20 | 1392 |
| myoC-1075 | + | CCAUUGUCUAUGCUUAGGGA | 20 | 1375 |
| myoC-1043 | - | GGGGGGAUGUUGAGAGGGGA | 20 | 1343 |
| myoC-5456 | + | ACUGUGAAAACUGACAUGGA | 20 | 5202 |
| myoC-1052 | - | UGAGCCACAGGGGAGGUGGA | 20 | 1352 |
| myoC-1083 | + | ACGUGAUCAGUGAGGACUGA | 20 | 1383 |
| myoC-2070 | - | UGUUUUAAAGCUAGGGGUGA | 20 | 2201 |
| myoC-1036 | - | UUCCCUGUGAUUCUCUGUGA | 20 | 1336 |
| myoC-5457 | + | AAAUUACUAGUAAUACUUGA | 20 | 5203 |
| myoC-1192 | + | GAGAAAAAGAGUUCCUAAUA | 20 | 1492 |
| myoC-5458 | - | UCUGAGUUCAGCAGGUGAUA | 20 | 5204 |
| myoC-1089 | + | CUUUAUAGCAGAGAAGACUA | 20 | 1389 |
| myoC-193 | - | AGGAAGAGAAGAAGCGACUA | 20 | 579 |
| myoC-5459 | - | ACACAGUUGUUUUAAAGCUA | 20 | 5205 |
| myoC-5460 | + | UUUUAUUUCUCCUUUUGCUA | 20 | 5206 |
| myoC-1214 | - | GCUCUCCCUGGAGCCUGGUA | 20 | 1514 |
| myoC-5461 | - | AGCACAAGACAGAUGAAUUA | 20 | 5207 |
| myoC-1074 | + | AAUGCCAUUGUCUAUGCUUA | 20 | 1374 |
| myoC-1172 | + | UUAUUACCACUUUGAGUUUA | 20 | 1472 |
| myoC-1066 | - | UUUGCCUGGCAUUCAAAAAC | 20 | 1366 |
| myoC-1191 | + | UUCUUCCUGUUAAAAGAAAC | 20 | 1491 |
| myoC-1063 | - | AAAAGAAUGCAGAGACUAAC | 20 | 1363 |
| myoC-1151 | - | GCAAUCCCGUUUCUUUUAAC | 20 | 1451 |
| myoC-206 | + | UGUCUCGGGUCUGGGGACAC | 20 | 592 |
| myoC-1096 | + | GCAUAAGCCAAGUCCACCAC | 20 | 1396 |
| myoC-1128 | - | GGGCAGUGGGAAUUGACCAC | 20 | 1428 |
| myoC-1046 | - | AGCUGGAGCAGCUGAGCCAC | 20 | 1346 |
| myoC-5462 | + | AUUGGUAAUGACAAAAUCAC | 20 | 5208 |
| myoC-1085 | + | CCCCCCUCACAGAGAAUCAC | 20 | 1385 |
| myoC-1176 | + | UUUUCCUCAUUCAAAUUCAC | 20 | 1476 |
| myoC-5463 | - | AAAAGGAGAAAUAAAAGGAC | 20 | 5209 |
| myoC-1055 | - | CAGGGGAGGUGGAGGGGGAC | 20 | 1355 |
| myoC-5464 | - | GGCUCGUAGUGACCUGCUAC | 20 | 5210 |
| myoC-201 | - | ACUGCUCGGGCUGUGCCACC | 20 | 587 |
| myoC-5465 | + | AUAUGCAGACACAUCUCACC | 20 | 5211 |
| myoC-5466 | - | AAAGGAGAAAUAAAAGGACC | 20 | 5212 |
| myoC-1216 | + | CACAAGGACAGCACCCUACC | 20 | 1516 |
| myoC-1195 | + | AGCCCUGCCUCCUAGAACCC | 20 | 1495 |
| myoC-1090 | + | AGCAGAGAAGACUAUGGCCC | 20 | 1390 |
| myoC-1180 | + | UAAAUAUUUCCAAACUGCCC | 20 | 1480 |
| myoC-1211 | - | UUCUAUAGGAAUGCUCUCCC | 20 | 1511 |
| myoC-1033 | - | GGCAAGUGUCUCUCCUUCCC | 20 | 1333 |
| myoC-107 | - | GAGGUUGGAAAGCAGCAGCC | 20 | 511 |
| myoC-1212 | - | GGAAUGCUCUCCCUGGAGCC | 20 | 1512 |
| myoC-3183 | + | UUCUUACCUUCUCUGGAGCC | 20 | 2929 |
| myoC-5467 | + | AAAGGGCAGGCAGGGAGGCC | 20 | 5213 |
| myoC-195 | - | AAGAAAAUGAGAAUCUGGCC | 20 | 581 |
| myoC-1070 | + | UGGCCCAGUUUUUGAAUGCC | 20 | 1370 |
| myoC-1158 | - | ACUCUGGAGGUGAGUCUGCC | 20 | 1458 |
| myoC-1065 | - | AACUGGUGGUAGCUUUUGCC | 20 | 1365 |
| myoC-1130 | - | GAUUAUAGUCCACGUGAUCC | 20 | 1430 |
| myoC-1060 | - | CACUGAUCACGUCAGACUCC | 20 | 1360 |
| myoC-113 | + | GCUGCUGCUUUCCAACCUCC | 20 | 515 |
| myoC-207 | + | UUCUCAGCCUUGCUACCUCC | 20 | 593 |
| myoC-5468 | - | UGACCUGCUACAGGCGCUCC | 20 | 5214 |
| myoC-1217 | + | ACAGCACCCUACCAGGCUCC | 20 | 1517 |
| myoC-1081 | + | AGCAUUGUGGCUCUCGGUCC | 20 | 1381 |
| myoC-1168 | - | UGGGUUUAUUAAUGUAAAGC | 20 | 1468 |
| myoC-1058 | - | ACAGGAAGGCAGGCAGAAGC | 20 | 1358 |
| myoC-1228 | + | UGUUAAAAACAAGAUCCAGC | 20 | 1528 |
| myoC-5469 | - | GGGGGGACUCUGAGUUCAGC | 20 | 5215 |
| myoC-1045 | - | AGAGGGGAAGGAGGCAGAGC | 20 | 1345 |
| myoC-5470 | + | AGGCCUGGAGCGCCUGUAGC | 20 | 5216 |
| myoC-1118 | - | CACGGAGUGACCUGCAGCGC | 20 | 1418 |
| myoC-1057 | - | GUGGAGGGGGACAGGAAGGC | 20 | 1357 |
| myoC-5471 | + | CUCUAGGAGAAAGGGCAGGC | 20 | 5217 |
| myoC-1134 | - | GAUCCUGGGUUCUAGGAGGC | 20 | 1434 |
| myoC-5472 | + | CAGUCUCUAGGAGAAAGGGC | 20 | 5218 |
| myoC-1205 | - | UUUGAAAUUAGACCUCCUGC | 20 | 1505 |
| myoC-1198 | + | CUUCUCCUCCCCUGCGCUGC | 20 | 1498 |
| myoC-1202 | + | UGCUGAGCUGCGUGGGGUGC | 20 | 1502 |
| myoC-1194 | + | AAAAUAUAGUAUUAGAAAUC | 20 | 1494 |
| myoC-1224 | + | AGAACCUCAUUGGUGAAAUC | 20 | 1524 |
| myoC-194 | - | AAGGCAAGAAAAUGAGAAUC | 20 | 580 |
| myoC-1026 | - | CGGUCGAAAACCUUGGAAUC | 20 | 1326 |
| myoC-1156 | - | CAAACUGUGUUUCUCCACUC | 20 | 1456 |
| myoC-200 | - | CCAGACCCGAGACACUGCUC | 20 | 586 |
| myoC-1069 | + | CUGGCAUUUUCCACUUGCUC | 20 | 1369 |
| myoC-5473 | + | GUGAAAAGUUUAACAAUCUC | 20 | 5219 |
| myoC-1222 | + | UAAUUUCAGUCUUGCAUCUC | 20 | 1522 |
| myoC-5474 | + | CUAAUCUAAAUGAAGCUCUC | 20 | 5220 |
| myoC-202 | + | CACAGCCCGAGCAGUGUCUC | 20 | 588 |
| myoC-3184 | + | CUCUGCAUUCUUACCUUCUC | 20 | 2930 |
| myoC-1144 | - | AAAUCAGUUCAAGGGAAGUC | 20 | 1444 |
| myoC-203 | + | CCCGAGCAGUGUCUCGGGUC | 20 | 589 |
| myoC-1203 | + | AGCUGCGUGGGGUGCUGGUC | 20 | 1503 |
| myoC-1061 | - | ACCGAGAGCCACAAUGCUUC | 20 | 1361 |
| myoC-1155 | - | UUUGUAAAUGUCUCAAGUUC | 20 | 1455 |
| myoC-1215 | - | AGGGUGCUGUCCUUGUGUUC | 20 | 1515 |
| myoC-1177 | + | AUUCAAAUUCACAGGCUUUC | 20 | 1477 |
| myoC-1028 | - | AGGAGACUCGGUUUUCUUUC | 20 | 1328 |
| myoC-1171 | - | AUAGAGCCAUAAACUCAAAG | 20 | 1471 |
| myoC-1068 | - | AAAAACUGGGCCAGAGCAAG | 20 | 1368 |
| myoC-1163 | - | AAGGCAAUCAUUAUUUCAAG | 20 | 1463 |
| myoC-111 | - | GAGGUAGCAAGGCUGAGAAG | 20 | 514 |
| myoC-1219 | + | GGAGAGCAUUCCUAUAGAAG | 20 | 1519 |
| myoC-1048 | - | CUGGAGCAGCUGAGCCACAG | 20 | 1348 |
| myoC-1120 | - | CGGAG UGACCUGCAGCGCAG | 20 | 1420 |
| myoC-1126 | - | ACAGAUUCAUUCAAGGGCAG | 20 | 1426 |
| myoC-1122 | - | GGGGAGGAGAAGAAAAAGAG | 20 | 1422 |
| myoC-1181 | + | UGGCAGACUCACCUCCAGAG | 20 | 1481 |
| myoC-3185 | - | GAGAAGGUAAGAAUGCAGAG | 20 | 2931 |
| myoC-1042 | - | GUGAGGGGGGAUGUUGAGAG | 20 | 1342 |
| myoC-5475 | + | CUGUGAAAACUGACAUGGAG | 20 | 5221 |
| myoC-1053 | - | GAGCCACAGGGGAGGUGGAG | 20 | 1353 |
| myoC-2071 | - | GUUUUAAAGCUAGGGGUGAG | 20 | 2202 |
| myoC-1037 | - | UCCCUGUGAUUCUCUGUGAG | 20 | 1337 |
| myoC-1040 | - | CUGUGAGGGGGGAUGUUGAG | 20 | 1340 |
| myoC-5476 | - | CACAGUUGUUUUAAAGCUAG | 20 | 5222 |
| myoC-5477 | - | GAGAGCUUCAUUUAGAUUAG | 20 | 5223 |
| myoC-1208 | - | CAGAGUAAGAACUGAUUUAG | 20 | 1508 |
| myoC-1196 | + | UCCUAGAACCCAGGAUCACG | 20 | 1496 |
| myoC-1031 | - | AUUGGUUGGCUGUGCGACCG | 20 | 1331 |
| myoC-1199 | + | GCAGUCACUGCUGAGCUGCG | 20 | 1499 |
| myoC-1175 | + | UGUUAAAUUUAGUUAGAAGG | 20 | 1475 |
| myoC-1044 | - | GGGAUGUUGAGAGGGGAAGG | 20 | 1344 |
| myoC-108 | - | GUUGGAAAGCAGCAGCCAGG | 20 | 480 |
| myoC-196 | - | AAAAUGAGAAUCUGGCCAGG | 20 | 582 |
| myoC-1229 | + | UAAAAACAAGAUCCAGCAGG | 20 | 1529 |
| myoC-1050 | - | CAGCUGAGCCACAGGGGAGG | 20 | 1350 |
| myoC-1054 | - | AGCCACAGGGGAGGUGGAGG | 20 | 1354 |
| myoC-2072 | - | UUUUAAAGCUAGGGGUGAGG | 20 | 2203 |
| myoC-1038 | - | CCCUGUGAUUCUCUGUGAGG | 20 | 1338 |
| myoC-1133 | - | ACGUGAUCCUGGGUUCUAGG | 20 | 1433 |
| myoC-5478 | + | AGGAGAAAGGGCAGGCAGGG | 20 | 5224 |
| myoC-2073 | - | UUUAAAGCUAGGGGUGAGGG | 20 | 2204 |
| myoC-1039 | - | CCUGUGAUUCUCUGUGAGGG | 20 | 1339 |
| myoC-1049 | - | GAGCAGCUGAGCCACAGGGG | 20 | 1349 |
| myoC-1121 | - | AGUGACCUGCAGCGCAGGGG | 20 | 1421 |
| myoC-3186 | - | AGGUAAGAAUGCAGAGUGGG | 20 | 2932 |
| myoC-1140 | - | AGGCAGGGCUAUAUUGUGGG | 20 | 1440 |
| myoC-5479 | + | GACUGUGAAAACUGACAUGG | 20 | 5225 |
| myoC-1064 | - | AGAAUGCAGAGACUAACUGG | 20 | 1364 |
| myoC-3187 | + | UUACCUUCUCUGGAGCCUGG | 20 | 2933 |
| myoC-1157 | - | ACUGUGUUUCUCCACUCUGG | 20 | 1457 |
| myoC-3188 | - | AAGGUAAGAAUGCAGAGUGG | 20 | 2934 |
| myoC-1051 | - | CUGAGCCACAGGGGAGGUGG | 20 | 1351 |
| myoC-1022 | - | GCAACUACUCAGCCCUGUGG | 20 | 1322 |
| myoC-1139 | - | GAGGCAGGGCUAUAUUGUGG | 20 | 1439 |
| myoC-1076 | + | AUGCUUAGGGAAGGAAAAUG | 20 | 1376 |
| myoC-1206 | - | UUCCCCAGAUUUCACCAAUG | 20 | 1506 |
| myoC-1170 | - | AAAGCCUGUGAAUUUGAAUG | 20 | 1470 |
| myoC-1146 | - | AAGUCACAAGGUAGUAACUG | 20 | 1446 |
| myoC-1084 | + | GUCCCCCUCCACCUCCCCUG | 20 | 1384 |
| myoC-1021 | - | UGGGCAACUACUCAGCCCUG | 20 | 1321 |
| myoC-1197 | + | CACGUGGACUAUAAUCCCUG | 20 | 1497 |
| myoC-1226 | + | AACCUCAUUGGUGAAAUCUG | 20 | 1526 |
| myoC-1148 | - | UUAGGAACUCUUUUUCUCUG | 20 | 1448 |
| myoC-205 | + | CGAGCAGUGUCUCGGGUCUG | 20 | 591 |
| myoC-1082 | + | GGAGUCUGACGUGAUCAGUG | 20 | 1382 |
| myoC-3189 | - | GAAGGUAAGAAUGCAGAGUG | 20 | 2935 |
| myoC-1201 | + | AGUCACUGCUGAGCUGCGUG | 20 | 1501 |
| myoC-2069 | - | UUGUUUUAAAGCUAGGGGUG | 20 | 2200 |
| myoC-1035 | - | CUUCCCUGUGAUUCUCUGUG | 20 | 1335 |
| myoC-1138 | - | GGAGGCAGGGCUAUAUUGUG | 20 | 1438 |
| myoC-1079 | + | UGAGCUUUCCUGAAGCAUUG | 20 | 1379 |
| myoC-1136 | - | UAGGAGGCAGGGCUAUAUUG | 20 | 1436 |
| myoC-1184 | + | AACUUGAGACAUUUACAAAU | 20 | 1484 |
| myoC-1209 | - | UUAGAGGCUAACAUUGACAU | 20 | 1509 |
| myoC-1029 | - | UUUCUUUCUGGUUCUGCCAU | 20 | 1329 |
| myoC-1223 | + | GUGCAUGCCAAGAACCUCAU | 20 | 1523 |
| myoC-1153 | - | GGUCUUGCUGACUAUAUGAU | 20 | 1453 |
| myoC-1182 | + | AGAUUCUAUUCUUAUUUGAU | 20 | 1482 |
| myoC-1210 | - | GGGAAAUCUGCCGCUUCUAU | 20 | 1510 |
| myoC-1230 | + | AAAAUGUCUGUGAUUUCUAU | 20 | 1530 |
| myoC-1072 | + | UCUGCAUUCUUUUUGGUUAU | 20 | 1372 |
| myoC-1165 | - | GACAGUUUUGGUAUAUUUAU | 20 | 1465 |
| myoC-1067 | - | U UGCCUGGCAU UCAAAAACU | 20 | 1367 |
| myoC-1027 | - | AACCUUGGAAUCAGGAGACU | 20 | 1327 |
| myoC-1023 | - | ACUCAGCCCUGUGGUGGACU | 20 | 1323 |
| myoC-1025 | - | UGCAAGACGGUCGAAAACCU | 20 | 1325 |
| myoC-1187 | + | UCAUAUAGUCAGCAAGACCU | 20 | 1487 |
| myoC-1034 | - | GCAAGUGUCUCUCCUUCCCU | 20 | 1334 |
| myoC-1152 | - | AGCCAAACAGAUUCAAGCCU | 20 | 1452 |
| myoC-1131 | - | AUUAUAGUCCACGUGAUCCU | 20 | 1431 |
| myoC-2066 | - | UACACAGUUGUUUUAAAGCU | 20 | 2197 |
| myoC-1059 | - | CAGGAAGGCAGGCAGAAGCU | 20 | 1359 |
| myoC-199 | - | CCCAGACCCGAGACACUGCU | 20 | 585 |
| myoC-1225 | + | GAACCUCAUUGGUGAAAUCU | 20 | 1525 |
| myoC-1080 | + | UCCUGAAGCAUUGUGGCUCU | 20 | 1380 |
| myoC-5480 | + | GUGCUAGCUGUGCAGUCUCU | 20 | 5226 |
| myoC-204 | + | CCGAGCAGUGUCUCGGGUCU | 20 | 590 |
| myoC-112 | + | GCACAGCCCGAGCAGUGUCU | 20 | 481 |
| myoC-1207 | - | GAUUUCACCAAUGAGGUUCU | 20 | 1507 |
| myoC-1132 | - | UCCACGUGAUCCUGGGUUCU | 20 | 1432 |
| myoC-1143 | - | AAAAUCAGUUCAAGGGAAGU | 20 | 1443 |
| myoC-1127 | - | CAGAUUCAUUCAAGGGCAGU | 20 | 1427 |
| myoC-3191 | - | AGAAGGUAAGAAUGCAGAGU | 20 | 2937 |
| myoC-1032 | - | UUGGUUGGCUGUGCGACCGU | 20 | 1332 |
| myoC-1200 | + | CAGUCACUGCUGAGCUGCGU | 20 | 1500 |
| myoC-197 | - | UGAGAAUCUGGCCAGGAGGU | 20 | 583 |
| myoC-1213 | - | UGCUCUCCCUGGAGCCUGGU | 20 | 1513 |
| myoC-1030 | - | UUUCUGGUUCUGCCAUUGGU | 20 | 1330 |
| myoC-1178 | + | UCACAGGCUUUCUGGACUGU | 20 | 1478 |
| myoC-1077 | + | GGGAAGGAAAAUGUGGCUGU | 20 | 1377 |
| myoC-1137 | - | AGGAGGCAGGGCUAUAUUGU | 20 | 1437 |
| myoC-1161 | - | ACAAGUAUUGACACUGUUGU | 20 | 1461 |
| myoC-1147 | - | UUACUUAGUUUCUCCUUAUU | 20 | 1447 |
| myoC-1154 | - | AAAAUGAGACUAGUACCCUU | 20 | 1454 |
| myoC-1073 | + | AAAUGCCAUUGUCUAUGCUU | 20 | 1373 |
| myoC-1220 | + | UUAAAACAACUGUGUAUCUU | 20 | 1520 |
| myoC-1189 | + | AUCUGUUUGGCUUUACUCUU | 20 | 1489 |
| myoC-1166 | - | UGCUUUUUGUUUUUUCUCUU | 20 | 1466 |
| myoC-1160 | - | GUGAGUCUGCCAGGGCAGUU | 20 | 1460 |
| myoC-1078 | + | GGAAGGAAAAUGUGGCUGUU | 20 | 1378 |
| myoC-1188 | + | GACCUAGGCUUGAAUCUGUU | 20 | 1488 |
| myoC-1221 | + | UAAAACAACUGUGUAUCUUU | 20 | 1521 |
| myoC-1167 | - | GCUUUUUGUUUUUUCUCUUU | 20 | 1467 |
| myoC-1164 | - | AAAGUUACUUCUGACAGUUU | 20 | 1464 |
| myoC-1071 | + | GUUAGUCUCUGCAUUCUUUU | 20 | 1371 |

**Table 10A** provides exemplary targeting domains for knocking down the *MYOC* gene selected according to the first tier parameters. The targeting domains bind within 3454-2454bp upstream of transcription start site or 500bp upstream and downstream of transcription start site, have a high level of orthogonality, start with a 5'G, and PAM is NNGRRT. It is contemplated herein that in an embodiment the targeting domain hybridizes to the target domain through complementary base pairing. Any of the targeting domains in the table can be used with a S. aureus eiCas9 molecule or eiCas9 fusion protein (e.g., an eiCas9 fused to a transcription repressor domain) to alter the MYOC gene (e.g., reduce or eliminate MYOC gene expression, MYOC protein function, or the level of MYOC protein). One or more gRNA may be used to target an eiCas9 to the promoter region of the MYOC gene.

**Table 10A**

| 1st Tier | | | | |
|---|---|---|---|---|
| gRNA Name | DNA Strand | Targeting Domain | Target Site Length | Seq ID |
| myoC-5481 | + | GAAAGCAACAGGUCCCUA | 18 | 5227 |
| myoC-5482 | + | GAAAUAGAAAGCAACAGGUCCCUA | 24 | 5228 |
| myoC-5483 | + | GCUAGGGAGGUGGCCUUGUUA | 21 | 5229 |
| myoC-5484 | + | GCGCUAGGGAGGUGGCCUUGUUA | 23 | 5230 |
| myoC-5485 | + | GGCGCUAGGGAGGUGGCCUUGUUA | 24 | 5231 |
| myoC-5486 | + | GACUACUGGUGUGCUGAUUUCAAC | 24 | 5232 |
| myoC-5487 | + | GUUGCUCAGGACACCCAGGACC | 22 | 5233 |
| myoC-5488 | + | GGUUGCUCAGGACACCCAGGACC | 23 | 5234 |
| myoC-5489 | + | GAAAACCCAUGCACACCC | 18 | 5235 |
| myoC-5490 | + | GGAAAACCCAUGCACACCC | 19 | 5236 |
| myoC-5491 | + | GAAGGAAAACCCAUGCACACCC | 22 | 5237 |
| myoC-5492 | + | GUGAAGGAAAACCCAUGCACACCC | 24 | 5238 |
| myoC-5493 | + | GACUCCAGUCACUUCUUCC | 19 | 5239 |
| myoC-5494 | + | GAAAAGACUCCAGUCACUUCUUCC | 24 | 5240 |
| myoC-5495 | + | GCUCUGCUGUGCUGAGAGGUGC | 22 | 5241 |
| myoC-3195 | + | GGCCUCCAGGUCUAAGCG | 18 | 2941 |
| myoC-1677 | + | GUGGCCUCCAGGUCUAAGCG | 20 | 1938 |
| myoC-3196 | + | GGUGGCCUCCAGGUCUAAGCG | 21 | 2942 |
| myoC-5496 | + | GACAGAGGUGGCCACGUGAGG | 21 | 5242 |
| myoC-5497 | + | GAAGACAGAGGUGGCCACGUGAGG | 24 | 5243 |
| myoC-5498 | + | GUGCUGAGAGGUGCCUGG | 18 | 5244 |
| myoC-5499 | + | GCUGUGCUGAGAGGUGCCUGG | 21 | 5245 |
| myoC-3197 | + | GCUGGUCCCGCUCCCGCCU | 19 | 2943 |
| myoC-3198 | + | GGCAGUCUCCAACUCUCUGGU | 21 | 2944 |
| myoC-3199 | + | GUAGGCAGUCUCCAACUCUCUGGU | 24 | 2945 |
| myoC-3200 | + | GCUGUCUCUCUGUAAGUU | 18 | 2946 |
| myoC-3201 | + | GCUGCUGUCUCUCUGUAAGUU | 21 | 2947 |
| myoC-3202 | + | GUGCUGCUGUCUCUCUGUAAGUU | 23 | 2948 |
| myoC-3203 | + | GGUGCUGCUGUCUCUCUGUAAGUU | 24 | 2949 |
| myoC-3204 | - | GACCAGCUGGAAACCCAAACCA | 22 | 2950 |
| myoC-3205 | - | GGACCAGCUGGAAACCCAAACCA | 23 | 2951 |
| myoC-3206 | - | GGGACCAGCUGGAAACCCAAACCA | 24 | 2952 |
| myoC-2083 | - | GUUCUCAAUGAGUUUGCAGA | 20 | 2212 |
| myoC-5500 | - | GGUUCUCAAUGAGUUUGCAGA | 21 | 5246 |
| myoC-5501 | - | GCAGGUUCUCAAUGAGUUUGCAGA | 24 | 5247 |
| myoC-5502 | - | GAAGAAGUCUAUUUCAUGA | 19 | 5248 |
| myoC-5503 | - | GAGAAGAAGUCUAUUUCAUGA | 21 | 5249 |
| myoC-5504 | - | GGAGAAGAAGUCUAUUUCAUGA | 22 | 5250 |
| myoC-5505 | - | GAGGAGAAGAAGUCUAUUUCAUGA | 24 | 5251 |
| myoC-5506 | - | GUGGGGACGCUGGGGCUGA | 19 | 5252 |
| myoC-5507 | - | GAGUGGGGACGCUGGGGCUGA | 21 | 5253 |
| myoC-5508 | - | GGAGUGGGGACGCUGGGGCUGA | 22 | 5254 |
| myoC-5509 | - | GGGAGUGGGGACGCUGGGGCUGA | 23 | 5255 |
| myoC-5510 | - | GCAUUCAUUGACAAUUUA | 18 | 5256 |
| myoC-5511 | - | GGCAUUCAUUGACAAUUUA | 19 | 5257 |
| myoC-3207 | - | GCUCAGGAAGGCCAAUGAC | 19 | 2953 |
| myoC-3208 | - | GCUCAGCUCAGGAAGGCCAAUGAC | 24 | 2954 |
| myoC-5512 | - | GUUAAUUCACGGAAGAAGUGAC | 22 | 5258 |
| myoC-5513 | - | GGGAGCCCUGCAAGCACC | 18 | 5259 |
| myoC-5514 | - | GGGGAGCCCUGCAAGCACC | 19 | 5260 |
| myoC-680 | - | GGGGGAGCCCUGCAAGCACC | 20 | 1020 |
| myoC-5515 | - | GCUGGGGGAGCCCUGCAAGCACC | 23 | 5261 |
| myoC-1841 | - | GCUGGCCUGCCUCGCUUCCC | 20 | 2051 |
| myoC-5516 | - | GCAGCUGGCCUGCCUCGCUUCCC | 23 | 5262 |
| myoC-5517 | - | GCCCGGAGGCCCCCAAGC | 18 | 5263 |
| myoC-1840 | - | GUGCCCGGAGGCCCCCAAGC | 20 | 2050 |
| myoC-1908 | - | GUUAAAAUUCCAGGGUGUGC | 20 | 2091 |
| myoC-5518 | - | GCUGUUAAAAUUCCAGGGUGUGC | 23 | 5264 |
| myoC-5519 | - | GCCCUGCAAGCACCCGGGGUC | 21 | 5265 |
| myoC-5520 | - | GAGCCCUGCAAGCACCCGGGGUC | 23 | 5266 |
| myoC-5521 | - | GGAGCCCUGCAAGCACCCGGGGUC | 24 | 5267 |
| myoC-5522 | - | GAAAGGGGCCUCCACGUCCAG | 21 | 5268 |
| myoC-5523 | - | GGAAAGGGGCCUCCACGUCCAG | 22 | 5269 |
| myoC-5524 | - | GGGAAAGGGGCCUCCACGUCCAG | 23 | 5270 |
| myoC-5525 | - | GAGGGAAACUAGUCUAACG | 19 | 5271 |
| myoC-5526 | - | GAGAGGGAAACUAGUCUAACG | 21 | 5272 |
| myoC-5527 | - | GGAGAGGGAAACUAGUCUAACG | 22 | 5273 |
| myoC-3209 | - | GCUUCUGGCCUGCCUGGUG | 19 | 2955 |
| myoC-5528 | - | GAAAUAAACACCAUCUUG | 18 | 5274 |
| myoC-5529 | - | GGAAAUAAACACCAUCUUG | 19 | 5275 |
| myoC-5530 | - | GAAAGGAAAUAAACACCAUCUUG | 23 | 5276 |
| myoC-2082 | - | GCAGGUUCUCAAUGAGUUUG | 20 | 2211 |
| myoC-5531 | - | GUGCAGGUUCUCAAUGAGUUUG | 22 | 5277 |
| myoC-3210 | - | GCGACUAAGGCAAGAAAAU | 19 | 2956 |
| myoC-3211 | - | GAAGCGACUAAGGCAAGAAAAU | 22 | 2957 |
| myoC-5532 | - | GGGUAUGGGUGCAUAAAU | 18 | 5278 |
| myoC-5533 | - | GGGGUAUGGGUGCAUAAAU | 19 | 5279 |
| myoC-5534 | - | GAGAUAUAGGAACUAUUAU | 19 | 5280 |
| myoC-838 | - | GGAGAUAUAGGAACUAUUAU | 20 | 991 |
| myoC-5535 | - | GUGGAGAUAUAGGAACUAUUAU | 22 | 5281 |
| myoC-5536 | - | GGUGGAGAUAUAGGAACUAUUAU | 23 | 5282 |
| myoC-5537 | - | GUUCAGUGUUGUUCACGGGGCU | 22 | 5283 |
| myoC-5538 | - | GACUUCUGGAAGGUUAUUUUCU | 22 | 5284 |
| myoC-5539 | - | GAUAUAGGAACUAUUAUUGGGGU | 23 | 5285 |
| myoC-5540 | - | GCUACGUCUUAAAGGACUUGU | 21 | 5286 |

**Table 10B** provides exemplary targeting domains for knocking down the *MYOC* gene selected according to the second tier parameters. The targeting domains bind within 3454-2454bp upstream of transcription start site or 500bp upstream and downstream of transcription start site, have a high level of orthogonality and PAM is NNGRRT. It is contemplated herein that in an embodiment the targeting domain hybridizes to the target domain through complementary base pairing. Any of the targeting domains in the table can be used with a S. aureus eiCas9 molecule or eiCas9 fusion protein (e.g., an eiCas9 fused to a transcription repressor domain) to alter the MYOC gene (e.g., reduce or eliminate MYOC gene expression, MYOC protein function, or the level of MYOC protein). One or more gRNA may be used to target an eiCas9 to the promoter region of the MYOC gene.

**Table 10B**

| 2nd Tier | | | | |
|---|---|---|---|---|
| gRNA Name | DNA Strand | Targeting Domain | Target Site Length | Seq ID |
| myoC-5541 | + | UUCUGGAGCCUGGAGCCA | 18 | 5287 |
| myoC-5542 | + | UUUCUGGAGCCUGGAGCCA | 19 | 5288 |
| myoC-1115 | + | CUUUCUGGAGCCUGGAGCCA | 20 | 1415 |
| myoC-5543 | + | CCUUUCUGGAGCCUGGAGCCA | 21 | 5289 |
| myoC-5544 | + | UCCUUUCUGGAGCCUGGAGCCA | 22 | 5290 |
| myoC-5545 | + | UUCCUUUCUGGAGCCUGGAGCCA | 23 | 5291 |
| myoC-5546 | + | UUUCCUUUCUGGAGCCUGGAGCCA | 24 | 5292 |
| myoC-5547 | + | AGAAAGCAACAGGUCCCUA | 19 | 5293 |
| myoC-2125 | + | UAGAAAGCAACAGGUCCCUA | 20 | 2243 |
| myoC-5548 | + | AUAGAAAGCAACAGGUCCCUA | 21 | 5294 |
| myoC-5549 | + | AAUAGAAAGCAACAGGUCCCUA | 22 | 5295 |
| myoC-5550 | + | AAAUAGAAAGCAACAGGUCCCUA | 23 | 5296 |
| myoC-5551 | + | AGGGAGGUGGCCUUGUUA | 18 | 5297 |
| myoC-5552 | + | UAGGGAGGUGGCCUUGUUA | 19 | 5298 |
| myoC-721 | + | CUAGGGAGGUGGCCUUGUUA | 20 | 1106 |
| myoC-5553 | + | CGCUAGGGAGGUGGCCUUGUUA | 22 | 5299 |
| myoC-5554 | + | UACUGGUGUGCUGAUUUCAAC | 21 | 5300 |
| myoC-5555 | + | CUACUGGUGUGCUGAUUUCAAC | 22 | 5301 |
| myoC-5556 | + | ACUACUGGUGUGCUGAUUUCAAC | 23 | 5302 |
| myoC-5557 | + | UUGCUCAGGACACCCAGGACC | 21 | 5303 |
| myoC-5558 | + | AGGUUGCUCAGGACACCCAGGACC | 24 | 5304 |
| myoC-1102 | + | AGGAAAACCCAUGCACACCC | 20 | 1402 |
| myoC-5559 | + | AAGGAAAACCCAUGCACACCC | 21 | 5305 |
| myoC-5560 | + | UGAAGGAAAACCCAUGCACACCC | 23 | 5306 |
| myoC-5561 | + | ACUCCAGUCACUUCUUCC | 18 | 5307 |
| myoC-2200 | + | AGACUCCAGUCACUUCUUCC | 20 | 2295 |
| myoC-5562 | + | AAGACUCCAGUCACUUCUUCC | 21 | 5308 |
| myoC-5563 | + | AAAGACUCCAGUCACUUCUUCC | 22 | 5309 |
| myoC-5564 | + | AAAAGACUCCAGUCACUUCUUCC | 23 | 5310 |
| myoC-5565 | + | UGCUGUGCUGAGAGGUGC | 18 | 5311 |
| myoC-5566 | + | CUGCUGUGCUGAGAGGUGC | 19 | 5312 |
| myoC-2353 | + | UCUGCUGUGCUGAGAGGUGC | 20 | 2407 |
| myoC-5567 | + | CUCUGCUGUGCUGAGAGGUGC | 21 | 5313 |
| myoC-5568 | + | AGCUCUGCUGUGCUGAGAGGUGC | 23 | 5314 |
| myoC-5569 | + | AAGCUCUGCUGUGCUGAGAGGUGC | 24 | 5315 |
| myoC-3212 | + | UGGCCUCCAGGUCUAAGCG | 19 | 2958 |
| myoC-3213 | + | UGGUGGCCUCCAGGUCUAAGCG | 22 | 2959 |
| myoC-3214 | + | UUGGUGGCCUCCAGGUCUAAGCG | 23 | 2960 |
| myoC-3215 | + | UUUGGUGGCCUCCAGGUCUAAGCG | 24 | 2961 |
| myoC-5570 | + | AGAGGUGGCCACGUGAGG | 18 | 5316 |
| myoC-5571 | + | CAGAGGUGGCCACGUGAGG | 19 | 5317 |
| myoC-2337 | + | ACAGAGGUGGCCACGUGAGG | 20 | 2398 |
| myoC-5572 | + | AGACAGAGGUGGCCACGUGAGG | 22 | 5318 |
| myoC-5573 | + | AAGACAGAGGUGGCCACGUGAGG | 23 | 5319 |
| myoC-5574 | + | UUGUCAAUGAAUGCCUGG | 18 | 5320 |
| myoC-5575 | + | AUUGUCAAUGAAUGCCUGG | 19 | 5321 |
| myoC-2131 | + | AAUUGUCAAUGAAUGCCUGG | 20 | 2249 |
| myoC-5576 | + | AAAUUGUCAAUGAAUGCCUGG | 21 | 5322 |
| myoC-5577 | + | UAAAUUGUCAAUGAAUGCCUGG | 22 | 5323 |
| myoC-5578 | + | AUAAAUUGUCAAUGAAUGCCUGG | 23 | 5324 |
| myoC-5579 | + | AAUAAAUUGUCAAUGAAUGCCUGG | 24 | 5325 |
| myoC-5580 | + | UGUGCUGAGAGGUGCCUGG | 19 | 5326 |
| myoC-2352 | + | CUGUGCUGAGAGGUGCCUGG | 20 | 2406 |
| myoC-5581 | + | UGCUGUGCUGAGAGGUGCCUGG | 22 | 5327 |
| myoC-5582 | + | CUGCUGUGCUGAGAGGUGCCUGG | 23 | 5328 |
| myoC-5583 | + | UCUGCUGUGCUGAGAGGUGCCUGG | 24 | 5329 |
| myoC-3216 | + | CUGGUCCCGCUCCCGCCU | 18 | 2962 |
| myoC-1690 | + | AGCUGGUCCCGCUCCCGCCU | 20 | 1946 |
| myoC-3217 | + | CAGCUGGUCCCGCUCCCGCCU | 21 | 2963 |
| myoC-3218 | + | CCAGCUGGUCCCGCUCCCGCCU | 22 | 2964 |
| myoC-3219 | + | UCCAGCUGGUCCCGCUCCCGCCU | 23 | 2965 |
| myoC-3220 | + | UUCCAGCUGGUCCCGCUCCCGCCU | 24 | 2966 |
| myoC-3221 | + | AGGCAGUCUCCAACUCUCUGGU | 22 | 2967 |
| myoC-3222 | + | UAGGCAGUCUCCAACUCUCUGGU | 23 | 2968 |
| myoC-3223 | + | UGCUGUCUCUCUGUAAGUU | 19 | 2969 |
| myoC-1676 | + | CUGCUGUCUCUCUGUAAGUU | 20 | 1937 |
| myoC-3224 | + | UGCUGCUGUCUCUCUGUAAGUU | 22 | 2970 |
| myoC-5584 | + | ACCUUCCAGAAGUCUGUU | 18 | 5330 |
| myoC-5585 | + | AACCUUCCAGAAGUCUGUU | 19 | 5331 |
| myoC-885 | + | UAACCUUCCAGAAGUCUGUU | 20 | 1208 |
| myoC-5586 | + | AUAACCUUCCAGAAGUCUGUU | 21 | 5332 |
| myoC-5587 | + | AAUAACCUUCCAGAAGUCUGUU | 22 | 5333 |
| myoC-5588 | + | AAAUAACCUUCCAGAAGUCUGUU | 23 | 5334 |
| myoC-5589 | + | AAAAUAACCUUCCAGAAGUCUGUU | 24 | 5335 |
| myoC-3225 | - | AGCUGGAAACCCAAACCA | 18 | 2971 |
| myoC-3226 | - | CAGCUGGAAACCCAAACCA | 19 | 2972 |
| myoC-1635 | - | CCAGCUGGAAACCCAAACCA | 20 | 1904 |
| myoC-3227 | - | ACCAGCUGGAAACCCAAACCA | 21 | 2973 |
| myoC-3228 | - | UCAGUGUGGCCAGUCCCA | 18 | 2974 |
| myoC-3229 | - | UUCAGUGUGGCCAGUCCCA | 19 | 2975 |
| myoC-1604 | - | CUUCAGUGUGGCCAGUCCCA | 20 | 1884 |
| myoC-3230 | - | CCUUCAGUGUGGCCAGUCCCA | 21 | 2976 |
| myoC-3231 | - | ACCUUCAGUGUGGCCAGUCCCA | 22 | 2977 |
| myoC-3232 | - | UACCUUCAGUGUGGCCAGUCCCA | 23 | 2978 |
| myoC-3233 | - | AUACCUUCAGUGUGGCCAGUCCCA | 24 | 2979 |
| myoC-5590 | - | UCUCAAUGAGUUUGCAGA | 18 | 5336 |
| myoC-5591 | - | UUCUCAAUGAGUUUGCAGA | 19 | 5337 |
| myoC-5592 | - | AGGUUCUCAAUGAGUUUGCAGA | 22 | 5338 |
| myoC-5593 | - | CAGGUUCUCAAUGAGUUUGCAGA | 23 | 5339 |
| myoC-5594 | - | AAGAAGUCUAUUUCAUGA | 18 | 5340 |
| myoC-1006 | - | AGAAGAAGUCUAUUUCAUGA | 20 | 1306 |
| myoC-5595 | - | AGGAGAAGAAGUCUAUUUCAUGA | 23 | 5341 |
| myoC-5596 | - | UGGGGACGCUGGGGCUGA | 18 | 5342 |
| myoC-1885 | - | AGUGGGGACGCUGGGGCUGA | 20 | 2075 |
| myoC-5597 | - | AGGGAGUGGGGACGCUGGGGCUGA | 24 | 5343 |
| myoC-1823 | - | AGGCAUUCAUUGACAAUUUA | 20 | 2037 |
| myoC-3234 | - | CUCAGGAAGGCCAAUGAC | 18 | 2980 |
| myoC-1603 | - | AGCUCAGGAAGGCCAAUGAC | 20 | 1883 |
| myoC-3235 | - | CAGCUCAGGAAGGCCAAUGAC | 21 | 2981 |
| myoC-3236 | - | UCAGCUCAGGAAGGCCAAUGAC | 22 | 2982 |
| myoC-3237 | - | CUCAGCUCAGGAAGGCCAAUGAC | 23 | 2983 |
| myoC-5598 | - | AUUCACGGAAGAAGUGAC | 18 | 5344 |
| myoC-5599 | - | AAUUCACGGAAGAAGUGAC | 19 | 5345 |
| myoC-1018 | - | UAAUUCACGGAAGAAGUGAC | 20 | 1318 |
| myoC-5600 | - | UUAAUUCACGGAAGAAGUGAC | 21 | 5346 |
| myoC-5601 | - | CGUUAAUUCACGGAAGAAGUGAC | 23 | 5347 |
| myoC-5602 | - | CCGUUAAUUCACGGAAGAAGUGAC | 24 | 5348 |
| myoC-5603 | - | UGGGGGAGCCCUGCAAGCACC | 21 | 5349 |
| myoC-5604 | - | CUGGGGGAGCCCUGCAAGCACC | 22 | 5350 |
| myoC-5605 | - | AGCUGGGGGAGCCCUGCAAGCACC | 24 | 5351 |
| myoC-5606 | - | UGGCCUGCCUCGCUUCCC | 18 | 5352 |
| myoC-5607 | - | CUGGCCUGCCUCGCUUCCC | 19 | 5353 |
| myoC-5608 | - | AGCUGGCCUGCCUCGCUUCCC | 21 | 5354 |
| myoC-5609 | - | CAGCUGGCCUGCCUCGCUUCCC | 22 | 5355 |
| myoC-5610 | - | UGCAGCUGGCCUGCCUCGCUUCCC | 24 | 5356 |
| myoC-5611 | - | UGCCCGGAGGCCCCCAAGC | 19 | 5357 |
| myoC-5612 | - | CGUGCCCGGAGGCCCCCAAGC | 21 | 5358 |
| myoC-5613 | - | UCGUGCCCGGAGGCCCCCAAGC | 22 | 5359 |
| myoC-5614 | - | AUCGUGCCCGGAGGCCCCCAAGC | 23 | 5360 |
| myoC-5615 | - | CAUCGUGCCCGGAGGCCCCCAAGC | 24 | 5361 |
| myoC-5616 | - | UAAAAUUCCAGGGUGUGC | 18 | 5362 |
| myoC-5617 | - | UUAAAAUUCCAGGGUGUGC | 19 | 5363 |
| myoC-5618 | - | UGUUAAAAUUCCAGGGUGUGC | 21 | 5364 |
| myoC-5619 | - | CUGUUAAAAUUCCAGGGUGUGC | 22 | 5365 |
| myoC-5620 | - | AGCUGUUAAAAUUCCAGGGUGUGC | 24 | 5366 |
| myoC-5621 | - | CUGCAAGCACCCGGGGUC | 18 | 5367 |
| myoC-5622 | - | CCUGCAAGCACCCGGGGUC | 19 | 5368 |
| myoC-1819 | - | CCCUGCAAGCACCCGGGGUC | 20 | 2034 |
| myoC-5623 | - | AGCCCUGCAAGCACCCGGGGUC | 22 | 5369 |
| myoC-5624 | - | UAAAGUCAGCUGUUAAAAUUC | 21 | 5370 |
| myoC-5625 | - | AUAAAGUCAGCUGUUAAAAUUC | 22 | 5371 |
| myoC-5626 | - | CAUAAAGUCAGCUGUUAAAAUUC | 23 | 5372 |
| myoC-5627 | - | UCAUAAAGUCAGCUGUUAAAAUUC | 24 | 5373 |
| myoC-5628 | - | AGGGGCCUCCACGUCCAG | 18 | 5374 |
| myoC-5629 | - | AAGGGGCCUCCACGUCCAG | 19 | 5375 |
| myoC-1870 | - | AAAGGGGCCUCCACGUCCAG | 20 | 2068 |
| myoC-5630 | - | AGGGAAAGGGGCCUCCACGUCCAG | 24 | 5376 |
| myoC-5631 | - | AGGGAAACUAGUCUAACG | 18 | 5377 |
| myoC-1856 | - | AGAGGGAAACUAGUCUAACG | 20 | 2061 |
| myoC-5632 | - | UGGAGAGGGAAACUAGUCUAACG | 23 | 5378 |
| myoC-5633 | - | AUGGAGAGGGAAACUAGUCUAACG | 24 | 5379 |
| myoC-3238 | - | CUUCUGGCCUGCCUGGUG | 18 | 2984 |
| myoC-171 | - | UGCUUCUGGCCUGCCUGGUG | 20 | 557 |
| myoC-1837 | - | AGGAAAUAAACACCAUCUUG | 20 | 2048 |
| myoC-5634 | - | AAGGAAAUAAACACCAUCUUG | 21 | 5380 |
| myoC-5635 | - | AAAGGAAAUAAACACCAUCUUG | 22 | 5381 |
| myoC-5636 | - | AGAAAGGAAAUAAACACCAUCUUG | 24 | 5382 |
| myoC-5637 | - | AGGUUCUCAAUGAGUUUG | 18 | 5383 |
| myoC-5638 | - | CAGGUUCUCAAUGAGUUUG | 19 | 5384 |
| myoC-5639 | - | UGCAGGUUCUCAAUGAGUUUG | 21 | 5385 |
| myoC-5640 | - | AGUGCAGGUUCUCAAUGAGUUUG | 23 | 5386 |
| myoC-5641 | - | CAGUGCAGGUUCUCAAUGAGUUUG | 24 | 5387 |
| myoC-3239 | - | CGACUAAGGCAAGAAAAU | 18 | 2985 |
| myoC-1648 | - | AGCGACUAAGGCAAGAAAAU | 20 | 1914 |
| myoC-3240 | - | AAGCGACUAAGGCAAGAAAAU | 21 | 2986 |
| myoC-3241 | - | AGAAGCGACUAAGGCAAGAAAAU | 23 | 2987 |
| myoC-3242 | - | AAGAAGCGACUAAGGCAAGAAAAU | 24 | 2988 |
| myoC-843 | - | UGGGGUAUGGGUGCAUAAAU | 20 | 1214 |
| myoC-5642 | - | CGAAGGCCUUUAUUUAAU | 18 | 5388 |
| myoC-5643 | - | ACGAAGGCCUUUAUUUAAU | 19 | 5389 |
| myoC-1014 | - | CACGAAGGCCUUUAUUUAAU | 20 | 1314 |
| myoC-5644 | - | UCACGAAGGCCUUUAUUUAAU | 21 | 5390 |
| myoC-5645 | - | UUCACGAAGGCCUUUAUUUAAU | 22 | 5391 |
| myoC-5646 | - | CUUCACGAAGGCCUUUAUUUAAU | 23 | 5392 |
| myoC-5647 | - | CCUUCACGAAGGCCUUUAUUUAAU | 24 | 5393 |
| myoC-5648 | - | AGAUAUAGGAACUAUUAU | 18 | 5394 |
| myoC-5649 | - | UGGAGAUAUAGGAACUAUUAU | 21 | 5395 |
| myoC-5650 | - | AGGUGGAGAUAUAGGAACUAUUAU | 24 | 5396 |
| myoC-5651 | - | AGUGUUGUUCACGGGGCU | 18 | 5397 |
| myoC-5652 | - | CAGUGUUGUUCACGGGGCU | 19 | 5398 |
| myoC-1003 | - | UCAGUGUUGUUCACGGGGCU | 20 | 1303 |
| myoC-5653 | - | UUCAGUGUUGUUCACGGGGCU | 21 | 5399 |
| myoC-5654 | - | UGUUCAGUGUUGUUCACGGGGCU | 23 | 5400 |
| myoC-5655 | - | AUGUUCAGUGUUGUUCACGGGGCU | 24 | 5401 |
| myoC-5656 | - | UCUGGAAGGUUAUUUUCU | 18 | 5402 |
| myoC-5657 | - | UUCUGGAAGGUUAUUUUCU | 19 | 5403 |
| myoC-2100 | - | CUUCUGGAAGGUUAUUUUCU | 20 | 2223 |
| myoC-5658 | - | ACUUCUGGAAGGUUAUUUUCU | 21 | 5404 |
| myoC-5659 | - | AGACUUCUGGAAGGUUAUUUUCU | 23 | 5405 |
| myoC-5660 | - | CAGACUUCUGGAAGGUUAUUUUCU | 24 | 5406 |
| myoC-5661 | - | AGGAACUAUUAUUGGGGU | 18 | 5407 |
| myoC-5662 | - | UAGGAACUAUUAUUGGGGU | 19 | 5408 |
| myoC-2094 | - | AUAGGAACUAUUAUUGGGGU | 20 | 2219 |
| myoC-5663 | - | UAUAGGAACUAUUAUUGGGGU | 21 | 5409 |
| myoC-5664 | - | AUAUAGGAACUAUUAUUGGGGU | 22 | 5410 |
| myoC-5665 | - | AGAUAUAGGAACUAUUAUUGGGGU | 24 | 5411 |
| myoC-5666 | - | ACGUCUUAAAGGACUUGU | 18 | 5412 |
| myoC-5667 | - | UACGUCUUAAAGGACUUGU | 19 | 5413 |
| myoC-2080 | - | CUACGUCUUAAAGGACUUGU | 20 | 2209 |
| myoC-5668 | - | UGCUACGUCUUAAAGGACUUGU | 22 | 5414 |
| myoC-5669 | - | CUGCUACGUCUUAAAGGACUUGU | 23 | 5415 |
| myoC-5670 | - | CCUGCUACGUCUUAAAGGACUUGU | 24 | 5416 |

**Table 10C** provides exemplary targeting domains for knocking down the *MYOC* gene selected according to the third tier parameters. The targeting domains bind within 3454-2454bp upstream of transcription start site or 500bp upstream and downstream of transcription start site, and PAM is NNGRRT. It is contemplated herein that in an embodiment the targeting domain hybridizes to the target domain through complementary base pairingAny of the targeting domains in the table can be used with a *S*. *aureus* eiCas9 molecule or eiCas9 fusion protein (e.g., an eiCas9 fused to a transcription repressor domain) to alter the MYOC gene (e.g., reduce or eliminate MYOC gene expression, MYOC protein function, or the level of MYOC protein). One or more gRNA may be used to target an eiCas9 to the promoter region of the MYOC gene.

**Table 10C**

| 3rd Tier | | | | |
|---|---|---|---|---|
| gRNA Name | DNA Strand | Targeting Domain | Target Site Length | Seq ID |
| myoC-5671 | + | AUUUCCUUUCUUUCAGCA | 18 | 5417 |
| myoC-5672 | + | UAUUUCCUUUCUUUCAGCA | 19 | 5418 |
| myoC-2138 | + | UUAUUUCCUUUCUUUCAGCA | 20 | 2256 |
| myoC-5673 | + | UUUAUUUCCUUUCUUUCAGCA | 21 | 5419 |
| myoC-5674 | + | GUUUAUUUCCUUUCUUUCAGCA | 22 | 5420 |
| myoC-5675 | + | UGUUUAUUUCCUUUCUUUCAGCA | 23 | 5421 |
| myoC-5676 | + | GUGUUUAUUUCCUUUCUUUCAGCA | 24 | 5422 |
| myoC-5677 | + | GUACUCAAUAAAUUGUCA | 18 | 5423 |
| myoC-5678 | + | AGUACUCAAUAAAUUGUCA | 19 | 5424 |
| myoC-2133 | + | AAGUACUCAAUAAAUUGUCA | 20 | 2251 |
| myoC-5679 | + | UAAGUACUCAAUAAAUUGUCA | 21 | 5425 |
| myoC-5680 | + | AUAAGUACUCAAUAAAUUGUCA | 22 | 5426 |
| myoC-5681 | + | UAUAAGUACUCAAUAAAUUGUCA | 23 | 5427 |
| myoC-5682 | + | AUAUAAGUACUCAAUAAAUUGUCA | 24 | 5428 |
| myoC-5683 | + | GAAUGCCUGGAUGAAUGA | 18 | 5429 |
| myoC-5684 | + | UGAAUGCCUGGAUGAAUGA | 19 | 5430 |
| myoC-2129 | + | AUGAAUGCCUGGAUGAAUGA | 20 | 2247 |
| myoC-5685 | + | AAUGAAUGCCUGGAUGAAUGA | 21 | 5431 |
| myoC-5686 | + | CAAUGAAUGCCUGGAUGAAUGA | 22 | 5432 |
| myoC-5687 | + | UCAAUGAAUGCCUGGAUGAAUGA | 23 | 5433 |
| myoC-5688 | + | GUCAAUGAAUGCCUGGAUGAAUGA | 24 | 5434 |
| myoC-5689 | + | UGGUGUGCUGAUUUCAAC | 18 | 5435 |
| myoC-5690 | + | CUGGUGUGCUGAUUUCAAC | 19 | 5436 |
| myoC-2324 | + | ACUGGUGUGCUGAUUUCAAC | 20 | 2390 |
| myoC-5691 | + | CUCAGGACACCCAGGACC | 18 | 5437 |
| myoC-5692 | + | GCUCAGGACACCCAGGACC | 19 | 5438 |
| myoC-2121 | + | UGCUCAGGACACCCAGGACC | 20 | 2239 |
| myoC-5693 | + | CCUGCAGUCCCCACCUCC | 18 | 5439 |
| myoC-5694 | + | CCCUGCAGUCCCCACCUCC | 19 | 5440 |
| myoC-1108 | + | UCCCUGCAGUCCCCACCUCC | 20 | 1408 |
| myoC-5695 | + | CUCCCUGCAGUCCCCACCUCC | 21 | 5441 |
| myoC-5696 | + | ACUCCCUGCAGUCCCCACCUCC | 22 | 5442 |
| myoC-5697 | + | CACUCCCUGCAGUCCCCACCUCC | 23 | 5443 |
| myoC-5698 | + | CCACUCCCUGCAGUCCCCACCUCC | 24 | 5444 |
| myoC-5699 | + | UAAAUUGUCAAUGAAUGC | 18 | 5445 |
| myoC-5700 | + | AUAAAUUGUCAAUGAAUGC | 19 | 5446 |
| myoC-2132 | + | AAUAAAUUGUCAAUGAAUGC | 20 | 2250 |
| myoC-5701 | + | CAAUAAAUUGUCAAUGAAUGC | 21 | 5447 |
| myoC-5702 | + | UCAAUAAAUUGUCAAUGAAUGC | 22 | 5448 |
| myoC-5703 | + | CUCAAUAAAUUGUCAAUGAAUGC | 23 | 5449 |
| myoC-5704 | + | ACUCAAUAAAUUGUCAAUGAAUGC | 24 | 5450 |
| myoC-3243 | + | CUCCCUCUGCAGCCCCUC | 18 | 2989 |
| myoC-3244 | + | GCUCCCUCUGCAGCCCCUC | 19 | 2990 |
| myoC-1689 | + | AGCUCCCUCUGCAGCCCCUC | 20 | 1945 |
| myoC-3245 | + | CAGCUCCCUCUGCAGCCCCUC | 21 | 2991 |
| myoC-3246 | + | CCAGCUCCCUCUGCAGCCCCUC | 22 | 2992 |
| myoC-3247 | + | CCCAGCUCCCUCUGCAGCCCCUC | 23 | 2993 |
| myoC-3248 | + | GCCCAGCUCCCUCUGCAGCCCCUC | 24 | 2994 |
| myoC-5705 | + | GGGUGGGGCUGUGCACAG | 18 | 5451 |
| myoC-5706 | + | UGGGUGGGGCUGUGCACAG | 19 | 5452 |
| myoC-882 | + | CUGGGUGGGGCUGUGCACAG | 20 | 1205 |
| myoC-5707 | + | GCUGGGUGGGGCUGUGCACAG | 21 | 5453 |
| myoC-5708 | + | GGCUGGGUGGGGCUGUGCACAG | 22 | 5454 |
| myoC-5709 | + | AGGCUGGGUGGGGCUGUGCACAG | 23 | 5455 |
| myoC-5710 | + | GAGGCUGGGUGGGGCUGUGCACAG | 24 | 5456 |
| myoC-3249 | + | UGGCUCUGCUCUGGGCAG | 18 | 2995 |
| myoC-3250 | + | CUGGCUCUGCUCUGGGCAG | 19 | 2996 |
| myoC-1674 | + | CCUGGCUCUGCUCUGGGCAG | 20 | 1935 |
| myoC-3251 | + | GCCUGGCUCUGCUCUGGGCAG | 21 | 2997 |
| myoC-3252 | + | GGCCUGGCUCUGCUCUGGGCAG | 22 | 2998 |
| myoC-3253 | + | UGGCCUGGCUCUGCUCUGGGCAG | 23 | 2999 |
| myoC-3254 | + | AUGGCCUGGCUCUGCUCUGGGCAG | 24 | 3000 |
| myoC-3255 | + | AGGAGGCUCUCCAGGGAG | 18 | 3001 |
| myoC-3256 | + | GAGGAGGCUCUCCAGGGAG | 19 | 3002 |
| myoC-1679 | + | GGAGGAGGCUCUCCAGGGAG | 20 | 1940 |
| myoC-3257 | + | UGGAGGAGGCUCUCCAGGGAG | 21 | 3003 |
| myoC-3258 | + | GUGGAGGAGGCUCUCCAGGGAG | 22 | 3004 |
| myoC-3259 | + | GGUGGAGGAGGCUCUCCAGGGAG | 23 | 3005 |
| myoC-3260 | + | UGGUGGAGGAGGCUCUCCAGGGAG | 24 | 3006 |
| myoC-3261 | + | AGUCUCCAACUCUCUGGU | 18 | 3007 |
| myoC-3262 | + | CAGUCUCCAACUCUCUGGU | 19 | 3008 |
| myoC-1691 | + | GCAGUCUCCAACUCUCUGGU | 20 | 1947 |
| myoC-5711 | - | CAGGAGGUGGGGACUGCA | 18 | 5457 |
| myoC-5712 | - | CCAGGAGGUGGGGACUGCA | 19 | 5458 |
| myoC-984 | - | UCCAGGAGGUGGGGACUGCA | 20 | 1284 |
| myoC-5713 | - | UUCCAGGAGGUGGGGACUGCA | 21 | 5459 |
| myoC-5714 | - | AUUCCAGGAGGUGGGGACUGCA | 22 | 5460 |
| myoC-5715 | - | AAUUCCAGGAGGUGGGGACUGCA | 23 | 5461 |
| myoC-5716 | - | GAAUUCCAGGAGGUGGGGACUGCA | 24 | 5462 |
| myoC-5717 | - | GCACAGUGCAGGUUCUCA | 18 | 5463 |
| myoC-5718 | - | GGCACAGUGCAGGUUCUCA | 19 | 5464 |
| myoC-2081 | - | UGGCACAGUGCAGGUUCUCA | 20 | 2210 |
| myoC-5719 | - | CUGGCACAGUGCAGGUUCUCA | 21 | 5465 |
| myoC-5720 | - | CCUGGCACAGUGCAGGUUCUCA | 22 | 5466 |
| myoC-5721 | - | GCCUGGCACAGUGCAGGUUCUCA | 23 | 5467 |
| myoC-5722 | - | UGCCUGGCACAGUGCAGGUUCUCA | 24 | 5468 |
| myoC-5723 | - | CAGGCAUUCAUUGACAAUUUA | 21 | 5469 |
| myoC-5724 | - | CCAGGCAUUCAUUGACAAUUUA | 22 | 5470 |
| myoC-5725 | - | UCCAGGCAUUCAUUGACAAUUUA | 23 | 5471 |
| myoC-5726 | - | AUCCAGGCAUUCAUUGACAAUUUA | 24 | 5472 |
| myoC-5727 | - | AGUCAGCUGUUAAAAUUC | 18 | 5473 |
| myoC-5728 | - | AAGUCAGCUGUUAAAAUUC | 19 | 5474 |
| myoC-1907 | - | AAAGUCAGCUGUUAAAAUUC | 20 | 2090 |
| myoC-3263 | - | CUGCUUCUGGCCUGCCUGGUG | 21 | 3009 |
| myoC-3264 | - | GCUGCUUCUGGCCUGCCUGGUG | 22 | 3010 |
| myoC-3265 | - | UGCUGCUUCUGGCCUGCCUGGUG | 23 | 3011 |
| myoC-3266 | - | CUGCUGCUUCUGGCCUGCCUGGUG | 24 | 3012 |
| myoC-5729 | - | UUGGGGUAUGGGUGCAUAAAU | 21 | 5475 |
| myoC-5730 | - | AUUGGGGUAUGGGUGCAUAAAU | 22 | 5476 |
| myoC-5731 | - | UAUUGGGGUAUGGGUGCAUAAAU | 23 | 5477 |
| myoC-5732 | - | UUAUUGGGGUAUGGGUGCAUAAAU | 24 | 5478 |

**Table 10D** provides exemplary targeting domains for knocking down the *MYOC* gene selected according to the fourth tier parameters. The targeting domains bind within 3454-2454bp upstream of transcription start site or 500bp upstream and downstream of transcription start site, and PAM is NNGRRV. It is contemplated herein that in an embodiment the targeting domain hybridizes to the target domain through complementary base pairing Any of the targeting domains in the table can be used with a S. aureus eiCas9 molecule or eiCas9 fusion protein (e.g., an eiCas9 fused to a transcription repressor domain) to alter the MYOC gene (e.g., reduce or eliminate MYOC gene expression, MYOC protein function, or the level of MYOC protein). One or more gRNA may be used to target an eiCas9 to the promoter region of the MYOC gene.

**Table 10D**

| 4th Tier | | | | |
|---|---|---|---|---|
| gRNA Name | DNA Strand | Targeting Domain | Target Site Length | Seq ID |
| myoC-5733 | + | GAACGAGUCACACAGAAA | 18 | 5479 |
| myoC-5734 | + | UGAACGAGUCACACAGAAA | 19 | 5480 |
| myoC-2127 | + | AUGAACGAGUCACACAGAAA | 20 | 2245 |
| myoC-5735 | + | AAUGAACGAGUCACACAGAAA | 21 | 5481 |
| myoC-5736 | + | GAAUGAACGAGUCACACAGAAA | 22 | 5482 |
| myoC-5737 | + | UGAAUGAACGAGUCACACAGAAA | 23 | 5483 |
| myoC-5738 | + | AUGAAUGAACGAGUCACACAGAAA | 24 | 5484 |
| myoC-5739 | + | UUGGAGUUUCUUUUUAAA | 18 | 5485 |
| myoC-5740 | + | UUUGGAGUUUCUUUUUAAA | 19 | 5486 |
| myoC-2326 | + | GUUUGGAGUUUCUUUUUAAA | 20 | 2392 |
| myoC-5741 | + | UGUUUGGAGUUUCUUUUUAAA | 21 | 5487 |
| myoC-5742 | + | CUGUUUGGAGUUUCUUUUUAAA | 22 | 5488 |
| myoC-5743 | + | UCUGUUUGGAGUUUCUUUUUAAA | 23 | 5489 |
| myoC-5744 | + | GUCUGUUUGGAGUUUCUUUUUAAA | 24 | 5490 |
| myoC-5745 | + | AAGGCUCACAGGAAGCAA | 18 | 5491 |
| myoC-5746 | + | AAAGGCUCACAGGAAGCAA | 19 | 5492 |
| myoC-1105 | + | AAAAGGCUCACAGGAAGCAA | 20 | 1405 |
| myoC-5747 | + | AAAAAGGCUCACAGGAAGCAA | 21 | 5493 |
| myoC-5748 | + | UAAAAAGGCUCACAGGAAGCAA | 22 | 5494 |
| myoC-5749 | + | AUAAAAAGGCUCACAGGAAGCAA | 23 | 5495 |
| myoC-5750 | + | GAUAAAAAGGCUCACAGGAAGCAA | 24 | 5496 |
| myoC-5751 | + | GAAUUAACGGCCUAGGAA | 18 | 5497 |
| myoC-5752 | + | UGAAUUAACGGCCUAGGAA | 19 | 5498 |
| myoC-2197 | + | GUGAAUUAACGGCCUAGGAA | 20 | 2293 |
| myoC-5753 | + | CGUGAAUUAACGGCCUAGGAA | 21 | 5499 |
| myoC-5754 | + | CCGUGAAUUAACGGCCUAGGAA | 22 | 5500 |
| myoC-5755 | + | UCCGUGAAUUAACGGCCUAGGAA | 23 | 5501 |
| myoC-5756 | + | UUCCGUGAAUUAACGGCCUAGGAA | 24 | 5502 |
| myoC-5757 | + | CAGGCACUAUGCUAGGAA | 18 | 5503 |
| myoC-5758 | + | CCAGGCACUAUGCUAGGAA | 19 | 5504 |
| myoC-2319 | + | GCCAGGCACUAUGCUAGGAA | 20 | 2386 |
| myoC-5759 | + | UGCCAGGCACUAUGCUAGGAA | 21 | 5505 |
| myoC-5760 | + | GUGCCAGGCACUAUGCUAGGAA | 22 | 5506 |
| myoC-5761 | + | UGUGCCAGGCACUAUGCUAGGAA | 23 | 5507 |
| myoC-5762 | + | CUGUGCCAGGCACUAUGCUAGGAA | 24 | 5508 |
| myoC-5763 | + | CAGGACGAUUCACGGGAA | 18 | 5509 |
| myoC-5764 | + | CCAGGACGAUUCACGGGAA | 19 | 5510 |
| myoC-2162 | + | ACCAGGACGAUUCACGGGAA | 20 | 2268 |
| myoC-5765 | + | CACCAGGACGAUUCACGGGAA | 21 | 5511 |
| myoC-5766 | + | GCACCAGGACGAUUCACGGGAA | 22 | 5512 |
| myoC-5767 | + | UGCACCAGGACGAUUCACGGGAA | 23 | 5513 |
| myoC-5768 | + | AUGCACCAGGACGAUUCACGGGAA | 24 | 5514 |
| myoC-5769 | + | CCAGCCCCGUGAACAACA | 18 | 5515 |
| myoC-5770 | + | CCCAGCCCCGUGAACAACA | 19 | 5516 |
| myoC-2182 | + | UCCCAGCCCCGUGAACAACA | 20 | 2282 |
| myoC-5771 | + | CUCCCAGCCCCGUGAACAACA | 21 | 5517 |
| myoC-5772 | + | ACUCCCAGCCCCGUGAACAACA | 22 | 5518 |
| myoC-5773 | + | AACUCCCAGCCCCGUGAACAACA | 23 | 5519 |
| myoC-5774 | + | AAACUCCCAGCCCCGUGAACAACA | 24 | 5520 |
| myoC-3267 | + | UCAUUGGGACUGGCCACA | 18 | 3013 |
| myoC-3268 | + | UUCAUUGGGACUGGCCACA | 19 | 3014 |
| myoC-1671 | + | AUUCAUUGGGACUGGCCACA | 20 | 1933 |
| myoC-3269 | + | GAUUCAUUGGGACUGGCCACA | 21 | 3015 |
| myoC-3270 | + | GGAUUCAUUGGGACUGGCCACA | 22 | 3016 |
| myoC-3271 | + | UGGAUUCAUUGGGACUGGCCACA | 23 | 3017 |
| myoC-3272 | + | CUGGAUUCAUUGGGACUGGCCACA | 24 | 3018 |
| myoC-5775 | + | UGGGUGGGGCUGUGCACA | 18 | 5521 |
| myoC-5776 | + | CUGGGUGGGGCUGUGCACA | 19 | 5522 |
| myoC-881 | + | GCUGGGUGGGGCUGUGCACA | 20 | 1050 |
| myoC-5777 | + | GGCUGGGUGGGGCUGUGCACA | 21 | 5523 |
| myoC-5778 | + | AGGCUGGGUGGGGCUGUGCACA | 22 | 5524 |
| myoC-5779 | + | GAGGCUGGGUGGGGCUGUGCACA | 23 | 5525 |
| myoC-5780 | + | UGAGGCUGGGUGGGGCUGUGCACA | 24 | 5526 |
| myoC-5781 | + | AUGAAUGAACGAGUCACA | 18 | 5527 |
| myoC-5782 | + | GAUGAAUGAACGAGUCACA | 19 | 5528 |
| myoC-2128 | + | GGAUGAAUGAACGAGUCACA | 20 | 2246 |
| myoC-5783 | + | UGGAUGAAUGAACGAGUCACA | 21 | 5529 |
| myoC-5784 | + | CUGGAUGAAUGAACGAGUCACA | 22 | 5530 |
| myoC-5785 | + | CCUGGAUGAAUGAACGAGUCACA | 23 | 5531 |
| myoC-5786 | + | GCCUGGAUGAAUGAACGAGUCACA | 24 | 5532 |
| myoC-5787 | + | UAAGACGUAGCAGGGACA | 18 | 5533 |
| myoC-5788 | + | UUAAGACGUAGCAGGGACA | 19 | 5534 |
| myoC-2314 | + | UUUAAGACGUAGCAGGGACA | 20 | 2383 |
| myoC-5789 | + | CUUUAAGACGUAGCAGGGACA | 21 | 5535 |
| myoC-5790 | + | CCUUUAAGACGUAGCAGGGACA | 22 | 5536 |
| myoC-5791 | + | UCCUUUAAGACGUAGCAGGGACA | 23 | 5537 |
| myoC-5792 | + | GUCCUUUAAGACGUAGCAGGGACA | 24 | 5538 |
| myoC-5793 | + | GCUCAUGCCCGAGCUCCA | 18 | 5539 |
| myoC-5794 | + | GGCUCAUGCCCGAGCUCCA | 19 | 5540 |
| myoC-2349 | + | UGGCUCAUGCCCGAGCUCCA | 20 | 2403 |
| myoC-5795 | + | CUGGCUCAUGCCCGAGCUCCA | 21 | 5541 |
| myoC-5796 | + | GCUGGCUCAUGCCCGAGCUCCA | 22 | 5542 |
| myoC-5797 | + | UGCUGGCUCAUGCCCGAGCUCCA | 23 | 5543 |
| myoC-5798 | + | UUGCUGGCUCAUGCCCGAGCUCCA | 24 | 5544 |
| myoC-3273 | + | GGUGGAGGAGGCUCUCCA | 18 | 3019 |
| myoC-3274 | + | UGGUGGAGGAGGCUCUCCA | 19 | 3020 |
| myoC-223 | + | UUGGUGGAGGAGGCUCUCCA | 20 | 609 |
| myoC-3275 | + | AUUGGUGGAGGAGGCUCUCCA | 21 | 3021 |
| myoC-3276 | + | AAUUGGUGGAGGAGGCUCUCCA | 22 | 3022 |
| myoC-3277 | + | CAAUUGGUGGAGGAGGCUCUCCA | 23 | 3023 |
| myoC-3278 | + | UCAAUUGGUGGAGGAGGCUCUCCA | 24 | 3024 |
| myoC-5799 | + | CUUCUUCUCCUCCAAGCA | 18 | 5545 |
| myoC-5800 | + | ACUUCUUCUCCUCCAAGCA | 19 | 5546 |
| myoC-2188 | + | GACUUCUUCUCCUCCAAGCA | 20 | 2286 |
| myoC-5801 | + | AGACUUCUUCUCCUCCAAGCA | 21 | 5547 |
| myoC-5802 | + | UAGACUUCUUCUCCUCCAAGCA | 22 | 5548 |
| myoC-5803 | + | AUAGACUUCUUCUCCUCCAAGCA | 23 | 5549 |
| myoC-5804 | + | AAUAGACUUCUUCUCCUCCAAGCA | 24 | 5550 |
| myoC-5805 | + | AAAGGCUCACAGGAAGCA | 18 | 5551 |
| myoC-5806 | + | AAAAGGCUCACAGGAAGCA | 19 | 5552 |
| myoC-2185 | + | AAAAAGGCUCACAGGAAGCA | 20 | 2284 |
| myoC-5807 | + | UAAAAAGGCUCACAGGAAGCA | 21 | 5553 |
| myoC-5808 | + | AUAAAAAGGCUCACAGGAAGCA | 22 | 5554 |
| myoC-5809 | + | GAUAAAAAGGCUCACAGGAAGCA | 23 | 5555 |
| myoC-5810 | + | AGAUAAAAAGGCUCACAGGAAGCA | 24 | 5556 |
| myoC-5811 | + | GGCACGAUGGAGGCAGCA | 18 | 5557 |
| myoC-5812 | + | GGGCACGAUGGAGGCAGCA | 19 | 5558 |
| myoC-714 | + | CGGGCACGAUGGAGGCAGCA | 20 | 1105 |
| myoC-5813 | + | CCGGGCACGAUGGAGGCAGCA | 21 | 5559 |
| myoC-5814 | + | UCCGGGCACGAUGGAGGCAGCA | 22 | 5560 |
| myoC-5815 | + | CUCCGGGCACGAUGGAGGCAGCA | 23 | 5561 |
| myoC-5816 | + | CCUCCGGGCACGAUGGAGGCAGCA | 24 | 5562 |
| myoC-3279 | + | AAGCUGCAGCAACGUGCA | 18 | 3025 |
| myoC-3280 | + | AAAGCUGCAGCAACGUGCA | 19 | 3026 |
| myoC-1666 | + | CAAAGCUGCAGCAACGUGCA | 20 | 1928 |
| myoC-3281 | + | CCAAAGCUGCAGCAACGUGCA | 21 | 3027 |
| myoC-3282 | + | CCCAAAGCUGCAGCAACGUGCA | 22 | 3028 |
| myoC-3283 | + | GCCCAAAGCUGCAGCAACGUGCA | 23 | 3029 |
| myoC-3284 | + | GGCCCAAAGCUGCAGCAACGUGCA | 24 | 3030 |
| myoC-5817 | + | GCUGGGUGGGGCUGUGCA | 18 | 5563 |
| myoC-5818 | + | GGCUGGGUGGGGCUGUGCA | 19 | 5564 |
| myoC-2334 | + | AGGCUGGGUGGGGCUGUGCA | 20 | 2396 |
| myoC-5819 | + | GAGGCUGGGUGGGGCUGUGCA | 21 | 5565 |
| myoC-5820 | + | UGAGGCUGGGUGGGGCUGUGCA | 22 | 5566 |
| myoC-5821 | + | GUGAGGCUGGGUGGGGCUGUGCA | 23 | 5567 |
| myoC-5822 | + | CGUGAGGCUGGGUGGGGCUGUGCA | 24 | 5568 |
| myoC-5823 | + | AUUCACUCUGCAAACUCA | 18 | 5569 |
| myoC-5824 | + | CAUUCACUCUGCAAACUCA | 19 | 5570 |
| myoC-2323 | + | CCAUUCACUCUGCAAACUCA | 20 | 2389 |
| myoC-5825 | + | UCCAUUCACUCUGCAAACUCA | 21 | 5571 |
| myoC-5826 | + | UUCCAUUCACUCUGCAAACUCA | 22 | 5572 |
| myoC-5827 | + | UUUCCAUUCACUCUGCAAACUCA | 23 | 5573 |
| myoC-5828 | + | AUUUCCAUUCACUCUGCAAACUCA | 24 | 5574 |
| myoC-5829 | + | AAAAGAUAAAAAGGCUCA | 18 | 5575 |
| myoC-5830 | + | GAAAAGAUAAAAAGGCUCA | 19 | 5576 |
| myoC-2187 | + | AGAAAAGAUAAAAAGGCUCA | 20 | 2285 |
| myoC-5831 | + | GAGAAAAGAUAAAAAGGCUCA | 21 | 5577 |
| myoC-5832 | + | AGAGAAAAGAUAAAAAGGCUCA | 22 | 5578 |
| myoC-5833 | + | CAGAGAAAAGAUAAAAAGGCUCA | 23 | 5579 |
| myoC-5834 | + | GCAGAGAAAAGAUAAAAAGGCUCA | 24 | 5580 |
| myoC-5835 | + | AUGCACCAGGACGAUUCA | 18 | 5581 |
| myoC-5836 | + | GAUGCACCAGGACGAUUCA | 19 | 5582 |
| myoC-2164 | + | AGAUGCACCAGGACGAUUCA | 20 | 2270 |
| myoC-5837 | + | CAGAUGCACCAGGACGAUUCA | 21 | 5583 |
| myoC-5838 | + | UCAGAUGCACCAGGACGAUUCA | 22 | 5584 |
| myoC-5839 | + | CUCAGAUGCACCAGGACGAUUCA | 23 | 5585 |
| myoC-5840 | + | GCUCAGAUGCACCAGGACGAUUCA | 24 | 5586 |
| myoC-3285 | + | UCUGGGCAGCUGGAUUCA | 18 | 3031 |
| myoC-3286 | + | CUCUGGGCAGCUGGAUUCA | 19 | 3032 |
| myoC-1673 | + | GCUCUGGGCAGCUGGAUUCA | 20 | 1934 |
| myoC-3287 | + | UGCUCUGGGCAGCUGGAUUCA | 21 | 3033 |
| myoC-3288 | + | CUGCUCUGGGCAGCUGGAUUCA | 22 | 3034 |
| myoC-3289 | + | UCUGCUCUGGGCAGCUGGAUUCA | 23 | 3035 |
| myoC-3290 | + | CUCUGCUCUGGGCAGCUGGAUUCA | 24 | 3036 |
| myoC-5841 | + | UGGGGAGCCAGCCCUUCA | 18 | 5587 |
| myoC-5842 | + | CUGGGGAGCCAGCCCUUCA | 19 | 5588 |
| myoC-868 | + | ACUGGGGAGCCAGCCCUUCA | 20 | 1177 |
| myoC-5843 | + | UACUGGGGAGCCAGCCCUUCA | 21 | 5589 |
| myoC-5844 | + | AUACUGGGGAGCCAGCCCUUCA | 22 | 5590 |
| myoC-5845 | + | UAUACUGGGGAGCCAGCCCUUCA | 23 | 5591 |
| myoC-5846 | + | AUAUACUGGGGAGCCAGCCCUUCA | 24 | 5592 |
| myoC-5847 | + | GCCCUUCAUGGGGGAAGA | 18 | 5593 |
| myoC-5848 | + | AGCCCUUCAUGGGGGAAGA | 19 | 5594 |
| myoC-2339 | + | CAGCCCUUCAUGGGGGAAGA | 20 | 2400 |
| myoC-5849 | + | CCAGCCCUUCAUGGGGGAAGA | 21 | 5595 |
| myoC-5850 | + | GCCAGCCCUUCAUGGGGGAAGA | 22 | 5596 |
| myoC-5851 | + | AGCCAGCCCUUCAUGGGGGAAGA | 23 | 5597 |
| myoC-5852 | + | GAGCCAGCCCUUCAUGGGGGAAGA | 24 | 5598 |
| myoC-5853 | + | GGGGGCCUCCGGGCACGA | 18 | 5599 |
| myoC-5854 | + | UGGGGGCCUCCGGGCACGA | 19 | 5600 |
| myoC-711 | + | UUGGGGGCCUCCGGGCACGA | 20 | 1123 |
| myoC-5855 | + | CUUGGGGGCCUCCGGGCACGA | 21 | 5601 |
| myoC-5856 | + | GCUUGGGGGCCUCCGGGCACGA | 22 | 5602 |
| myoC-5857 | + | GGCUUGGGGGCCUCCGGGCACGA | 23 | 5603 |
| myoC-5858 | + | GGGCUUGGGGGCCUCCGGGCACGA | 24 | 5604 |
| myoC-5859 | + | UUUAAGACGUAGCAGGGA | 18 | 5605 |
| myoC-5860 | + | CUUUAAGACGUAGCAGGGA | 19 | 5606 |
| myoC-2315 | + | CCUUUAAGACGUAGCAGGGA | 20 | 2384 |
| myoC-5861 | + | UCCUUUAAGACGUAGCAGGGA | 21 | 5607 |
| myoC-5862 | + | GUCCUUUAAGACGUAGCAGGGA | 22 | 5608 |
| myoC-5863 | + | AGUCCUUUAAGACGUAGCAGGGA | 23 | 5609 |
| myoC-5864 | + | AAGUCCUUUAAGACGUAGCAGGGA | 24 | 5610 |
| myoC-5865 | + | AGGGCUCCCCCAGCUGGA | 18 | 5611 |
| myoC-5866 | + | CAGGGCUCCCCCAGCUGGA | 19 | 5612 |
| myoC-2116 | + | GCAGGGCUCCCCCAGCUGGA | 20 | 2235 |
| myoC-5867 | + | UGCAGGGCUCCCCCAGCUGGA | 21 | 5613 |
| myoC-5868 | + | UUGCAGGGCUCCCCCAGCUGGA | 22 | 5614 |
| myoC-5869 | + | CUUGCAGGGCUCCCCCAGCUGGA | 23 | 5615 |
| myoC-5870 | + | GCUUGCAGGGCUCCCCCAGCUGGA | 24 | 5616 |
| myoC-5871 | + | GUUGCCCAGAAGACAUGA | 18 | 5617 |
| myoC-5872 | + | AGUUGCCCAGAAGACAUGA | 19 | 5618 |
| myoC-2201 | + | UAGUUGCCCAGAAGACAUGA | 20 | 2296 |
| myoC-5873 | + | GUAGUUGCCCAGAAGACAUGA | 21 | 5619 |
| myoC-5874 | + | AGUAGUUGCCCAGAAGACAUGA | 22 | 5620 |
| myoC-5875 | + | GAGUAGUUGCCCAGAAGACAUGA | 23 | 5621 |
| myoC-5876 | + | UGAGUAGUUGCCCAGAAGACAUGA | 24 | 5622 |
| myoC-5877 | + | CAAUGAAUGCCUGGAUGA | 18 | 5623 |
| myoC-5878 | + | UCAAUGAAUGCCUGGAUGA | 19 | 5624 |
| myoC-2130 | + | GUCAAUGAAUGCCUGGAUGA | 20 | 2248 |
| myoC-5879 | + | UGUCAAUGAAUGCCUGGAUGA | 21 | 5625 |
| myoC-5880 | + | UUGUCAAUGAAUGCCUGGAUGA | 22 | 5626 |
| myoC-5881 | + | AUUGUCAAUGAAUGCCUGGAUGA | 23 | 5627 |
| myoC-5882 | + | AAUUGUCAAUGAAUGCCUGGAUGA | 24 | 5628 |
| myoC-5883 | + | CUGCAGCGCUGUGACUGA | 18 | 5629 |
| myoC-5884 | + | GCUGCAGCGCUGUGACUGA | 19 | 5630 |
| myoC-698 | + | AGCUGCAGCGCUGUGACUGA | 20 | 1089 |
| myoC-5885 | + | CAGCUGCAGCGCUGUGACUGA | 21 | 5631 |
| myoC-5886 | + | CCAGCUGCAGCGCUGUGACUGA | 22 | 5632 |
| myoC-5887 | + | GCCAGCUGCAGCGCUGUGACUGA | 23 | 5633 |
| myoC-5888 | + | GGCCAGCUGCAGCGCUGUGACUGA | 24 | 5634 |
| myoC-5889 | + | AAAUAAAGGCCUUCGUGA | 18 | 5635 |
| myoC-5890 | + | UAAAUAAAGGCCUUCGUGA | 19 | 5636 |
| myoC-1101 | + | UUAAAUAAAGGCCUUCGUGA | 20 | 1401 |
| myoC-5891 | + | AUUAAAUAAAGGCCUUCGUGA | 21 | 5637 |
| myoC-5892 | + | CAUUAAAUAAAGGCCUUCGUGA | 22 | 5638 |
| myoC-5893 | + | CCAU UAAAUAAAGGCCUUCGUGA | 23 | 5639 |
| myoC-5894 | + | CCCAUUAAAUAAAGGCCUUCGUGA | 24 | 5640 |
| myoC-5895 | + | CAGAGAGGUUUAUAUAUA | 18 | 5641 |
| myoC-5896 | + | CCAGAGAGGUUUAUAUAUA | 19 | 5642 |
| myoC-2348 | + | UCCAGAGAGGUUUAUAUAUA | 20 | 2402 |
| myoC-5897 | + | CUCCAGAGAGGUUUAUAUAUA | 21 | 5643 |
| myoC-5898 | + | GCUCCAGAGAGGUUUAUAUAUA | 22 | 5644 |
| myoC-5899 | + | AGCUCCAGAGAGGUUUAUAUAUA | 23 | 5645 |
| myoC-5900 | + | GAGCUCCAGAGAGGUUUAUAUAUA | 24 | 5646 |
| myoC-5901 | + | AGGCAGCAGGGGGCGCUA | 18 | 5647 |
| myoC-5902 | + | GAGGCAGCAGGGGGCGCUA | 19 | 5648 |
| myoC-718 | + | GGAGGCAGCAGGGGGCGCUA | 20 | 1015 |
| myoC-5903 | + | UGGAGGCAGCAGGGGGCGCUA | 21 | 5649 |
| myoC-5904 | + | AUGGAGGCAGCAGGGGGCGCUA | 22 | 5650 |
| myoC-5905 | + | GAUGGAGGCAGCAGGGGGCGCUA | 23 | 5651 |
| myoC-5906 | + | CGAUGGAGGCAGCAGGGGGCGCUA | 24 | 5652 |
| myoC-5907 | + | AGGCACUAUGCUAGGAAC | 18 | 5653 |
| myoC-5908 | + | CAGGCACUAUGCUAGGAAC | 19 | 5654 |
| myoC-892 | + | CCAGGCACUAUGCUAGGAAC | 20 | 1196 |
| myoC-5909 | + | GCCAGGCACUAUGCUAGGAAC | 21 | 5655 |
| myoC-5910 | + | UGCCAGGCACUAUGCUAGGAAC | 22 | 5656 |
| myoC-5911 | + | GUGCCAGGCACUAUGCUAGGAAC | 23 | 5657 |
| myoC-5912 | + | UGUGCCAGGCACUAUGCUAGGAAC | 24 | 5658 |
| myoC-5913 | + | AACAGCCAGCCAGAACAC | 18 | 5659 |
| myoC-5914 | + | UAACAGCCAGCCAGAACAC | 19 | 5660 |
| myoC-2312 | + | AUAACAGCCAGCCAGAACAC | 20 | 2381 |
| myoC-5915 | + | AAUAACAGCCAGCCAGAACAC | 21 | 5661 |
| myoC-5916 | + | AAAUAACAGCCAGCCAGAACAC | 22 | 5662 |
| myoC-5917 | + | AAAAUAACAGCCAGCCAGAACAC | 23 | 5663 |
| myoC-5918 | + | AAAAAUAACAGCCAGCCAGAACAC | 24 | 5664 |
| myoC-5919 | + | ACGUACACACACUUACAC | 18 | 5665 |
| myoC-5920 | + | CACGUACACACACUUACAC | 19 | 5666 |
| myoC-2325 | + | ACACGUACACACACUUACAC | 20 | 2391 |
| myoC-5921 | + | CACACGUACACACACUUACAC | 21 | 5667 |
| myoC-5922 | + | ACACACGUACACACACUUACAC | 22 | 5668 |
| myoC-5923 | + | CACACACGUACACACACUUACAC | 23 | 5669 |
| myoC-5924 | + | ACACACACGUACACACACUUACAC | 24 | 5670 |
| myoC-5925 | + | GAAGACAGAGGUGGCCAC | 18 | 5671 |
| myoC-5926 | + | GGAAGACAGAGGUGGCCAC | 19 | 5672 |
| myoC-2338 | + | GGGAAGACAGAGGUGGCCAC | 20 | 2399 |
| myoC-5927 | + | GGGGAAGACAGAGGUGGCCAC | 21 | 5673 |
| myoC-5928 | + | GGGGGAAGACAGAGGUGGCCAC | 22 | 5674 |
| myoC-5929 | + | UGGGGGAAGACAGAGGUGGCCAC | 23 | 5675 |
| myoC-5930 | + | AUGGGGGAAGACAGAGGUGGCCAC | 24 | 5676 |
| myoC-5931 | + | UCUCCAGCUCAGAUGCAC | 18 | 5677 |
| myoC-5932 | + | GUCUCCAGCUCAGAUGCAC | 19 | 5678 |
| myoC-2166 | + | AGUCUCCAGCUCAGAUGCAC | 20 | 2272 |
| myoC-5933 | + | GAGUCUCCAGCUCAGAUGCAC | 21 | 5679 |
| myoC-5934 | + | GGAGUCUCCAGCUCAGAUGCAC | 22 | 5680 |
| myoC-5935 | + | AGGAGUCUCCAGCUCAGAUGCAC | 23 | 5681 |
| myoC-5936 | + | AAGGAGUCUCCAGCUCAGAUGCAC | 24 | 5682 |
| myoC-5937 | + | CUGGGUGGGGCUGUGCAC | 18 | 5683 |
| myoC-5938 | + | GCUGGGUGGGGCUGUGCAC | 19 | 5684 |
| myoC-880 | + | GGCUGGGUGGGGCUGUGCAC | 20 | 1051 |
| myoC-5939 | + | AGGCUGGGUGGGGCUGUGCAC | 21 | 5685 |
| myoC-5940 | + | GAGGCUGGGUGGGGCUGUGCAC | 22 | 5686 |
| myoC-5941 | + | UGAGGCUGGGUGGGGCUGUGCAC | 23 | 5687 |
| myoC-5942 | + | GUGAGGCUGGGUGGGGCUGUGCAC | 24 | 5688 |
| myoC-5943 | + | AAAGAUAAAAAGGCUCAC | 18 | 5689 |
| myoC-5944 | + | AAAAGAUAAAAAGGCUCAC | 19 | 5690 |
| myoC-1104 | + | GAAAAGAUAAAAAGGCUCAC | 20 | 1404 |
| myoC-5945 | + | AGAAAAGAUAAAAAGGCUCAC | 21 | 5691 |
| myoC-5946 | + | GAGAAAAGAUAAAAAGGCUCAC | 22 | 5692 |
| myoC-5947 | + | AGAGAAAAGAUAAAAAGGCUCAC | 23 | 5693 |
| myoC-5948 | + | CAGAGAAAAGAUAAAAAGGCUCAC | 24 | 5694 |
| myoC-5949 | + | UGCACCAGGACGAUUCAC | 18 | 5695 |
| myoC-5950 | + | AUGCACCAGGACGAUUCAC | 19 | 5696 |
| myoC-2163 | + | GAUGCACCAGGACGAUUCAC | 20 | 2269 |
| myoC-5951 | + | AGAUGCACCAGGACGAUUCAC | 21 | 5697 |
| myoC-5952 | + | CAGAUGCACCAGGACGAUUCAC | 22 | 5698 |
| myoC-5953 | + | UCAGAUGCACCAGGACGAUUCAC | 23 | 5699 |
| myoC-5954 | + | CUCAGAUGCACCAGGACGAUUCAC | 24 | 5700 |
| myoC-5955 | + | AGUAGUUGCCCAGAAGAC | 18 | 5701 |
| myoC-5956 | + | GAGUAGUUGCCCAGAAGAC | 19 | 5702 |
| myoC-2202 | + | UGAGUAGUUGCCCAGAAGAC | 20 | 2297 |
| myoC-5957 | + | GGAGGAGGCUUGGAAGAC | 18 | 5703 |
| myoC-5958 | + | AGGAGGAGGCUUGGAAGAC | 19 | 5704 |
| myoC-2153 | + | GAGGAGGAGGCUUGGAAGAC | 20 | 2263 |
| myoC-5959 | + | GGAGGAGGAGGCUUGGAAGAC | 21 | 5705 |
| myoC-5960 | + | UGGAGGAGGAGGCUUGGAAGAC | 22 | 5706 |
| myoC-5961 | + | AUGGAGGAGGAGGCUUGGAAGAC | 23 | 5707 |
| myoC-5962 | + | GAUGGAGGAGGAGGCUUGGAAGAC | 24 | 5708 |
| myoC-5963 | + | CCUGGAAUUCUCCUGGAC | 18 | 5709 |
| myoC-5964 | + | UCCUGGAAUUCUCCUGGAC | 19 | 5710 |
| myoC-2177 | + | CUCCUGGAAUUCUCCUGGAC | 20 | 2278 |
| myoC-5965 | + | CCUCCUGGAAUUCUCCUGGAC | 21 | 5711 |
| myoC-5966 | + | ACCUCCUGGAAUUCUCCUGGAC | 22 | 5712 |
| myoC-5967 | + | CACCUCCUGGAAUUCUCCUGGAC | 23 | 5713 |
| myoC-5968 | + | CCACCUCCUGGAAUUCUCCUGGAC | 24 | 5714 |
| myoC-5969 | + | AGAGAGGUUUAUAUAUAC | 18 | 5715 |
| myoC-5970 | + | CAGAGAGGUUUAUAUAUAC | 19 | 5716 |
| myoC-865 | + | CCAGAGAGGUUUAUAUAUAC | 20 | 1195 |
| myoC-5971 | + | UCCAGAGAGGUUUAUAUAUAC | 21 | 5717 |
| myoC-5972 | + | CUCCAGAGAGGUUUAUAUAUAC | 22 | 5718 |
| myoC-5973 | + | GCUCCAGAGAGGUUUAUAUAUAC | 23 | 5719 |
| myoC-5974 | + | AGCUCCAGAGAGGUUUAUAUAUAC | 24 | 5720 |
| myoC-5975 | + | GGAAAACCCAUGCACACC | 18 | 5721 |
| myoC-5976 | + | AGGAAAACCCAUGCACACC | 19 | 5722 |
| myoC-2193 | + | AAGGAAAACCCAUGCACACC | 20 | 2290 |
| myoC-5977 | + | GAAGGAAAACCCAUGCACACC | 21 | 5723 |
| myoC-5978 | + | UGAAGGAAAACCCAUGCACACC | 22 | 5724 |
| myoC-5979 | + | GUGAAGGAAAACCCAUGCACACC | 23 | 5725 |
| myoC-5980 | + | CGUGAAGGAAAACCCAUGCACACC | 24 | 5726 |
| myoC-5981 | + | CAGGUUGCUCAGGACACC | 18 | 5727 |
| myoC-5982 | + | GCAGGUUGCUCAGGACACC | 19 | 5728 |
| myoC-2122 | + | GGCAGGUUGCUCAGGACACC | 20 | 2240 |
| myoC-5983 | + | UGGCAGGUUGCUCAGGACACC | 21 | 5729 |
| myoC-5984 | + | CUGGCAGGUUGCUCAGGACACC | 22 | 5730 |
| myoC-5985 | + | GCUGGCAGGUUGCUCAGGACACC | 23 | 5731 |
| myoC-5986 | + | GGCUGGCAGGUUGCUCAGGACACC | 24 | 5732 |
| myoC-5987 | + | CGGAAAACUCCCAGCCCC | 18 | 5733 |
| myoC-5988 | + | ACGGAAAACUCCCAGCCCC | 19 | 5734 |
| myoC-2183 | + | AACGGAAAACUCCCAGCCCC | 20 | 2283 |
| myoC-5989 | + | CAACGGAAAACUCCCAGCCCC | 21 | 5735 |
| myoC-5990 | + | GCAACGGAAAACUCCCAGCCCC | 22 | 5736 |
| myoC-5991 | + | AGCAACGGAAAACUCCCAGCCCC | 23 | 5737 |
| myoC-5992 | + | AAGCAACGGAAAACUCCCAGCCCC | 24 | 5738 |
| myoC-5993 | + | UAGAAAGCAACAGGUCCC | 18 | 5739 |
| myoC-5994 | + | AUAGAAAGCAACAGGUCCC | 19 | 5740 |
| myoC-2126 | + | AAUAGAAAGCAACAGGUCCC | 20 | 2244 |
| myoC-5995 | + | AAAUAGAAAGCAACAGGUCCC | 21 | 5741 |
| myoC-5996 | + | GAAAUAGAAAGCAACAGGUCCC | 22 | 5742 |
| myoC-5997 | + | AGAAAUAGAAAGCAACAGGUCCC | 23 | 5743 |
| myoC-5998 | + | CAGAAAUAGAAAGCAACAGGUCCC | 24 | 5744 |
| myoC-5999 | + | UUUCUGGAGCCUGGAGCC | 18 | 5745 |
| myoC-6000 | + | CUUUCUGGAGCCUGGAGCC | 19 | 5746 |
| myoC-2168 | + | CCUUUCUGGAGCCUGGAGCC | 20 | 2273 |
| myoC-6001 | + | UCCUUUCUGGAGCCUGGAGCC | 21 | 5747 |
| myoC-6002 | + | UUCCUUUCUGGAGCCUGGAGCC | 22 | 5748 |
| myoC-6003 | + | UUUCCUUUCUGGAGCCUGGAGCC | 23 | 5749 |
| myoC-6004 | + | AUUUCCUUUCUGGAGCCUGGAGCC | 24 | 5750 |
| myoC-6005 | + | CAUUUCCUUUCUGGAGCC | 18 | 5751 |
| myoC-6006 | + | CCAUUUCCUUUCUGGAGCC | 19 | 5752 |
| myoC-1114 | + | UCCAUUUCCUUUCUGGAGCC | 20 | 1414 |
| myoC-6007 | + | CUCCAUUUCCUUUCUGGAGCC | 21 | 5753 |
| myoC-6008 | + | UCUCCAUUUCCUUUCUGGAGCC | 22 | 5754 |
| myoC-6009 | + | CUCUCCAUUUCCUUUCUGGAGCC | 23 | 5755 |
| myoC-6010 | + | CCUCUCCAUUUCCUUUCUGGAGCC | 24 | 5756 |
| myoC-6011 | + | UUCCGUGAAUUAACGGCC | 18 | 5757 |
| myoC-6012 | + | CUUCCGUGAAUUAACGGCC | 19 | 5758 |
| myoC-2199 | + | UCUUCCGUGAAUUAACGGCC | 20 | 2294 |
| myoC-6013 | + | UUCUUCCGUGAAUUAACGGCC | 21 | 5759 |
| myoC-6014 | + | CUUCUUCCGUGAAUUAACGGCC | 22 | 5760 |
| myoC-6015 | + | ACUUCUUCCGUGAAUUAACGGCC | 23 | 5761 |
| myoC-6016 | + | CACUUCUUCCGUGAAUUAACGGCC | 24 | 5762 |
| myoC-6017 | + | GGAGAGGAAACCUCUGCC | 18 | 5763 |
| myoC-6018 | + | UGGAGAGGAAACCUCUGCC | 19 | 5764 |
| myoC-749 | + | CUGGAGAGGAAACCUCUGCC | 20 | 1110 |
| myoC-6019 | + | GCUGGAGAGGAAACCUCUGCC | 21 | 5765 |
| myoC-6020 | + | AGCUGGAGAGGAAACCUCUGCC | 22 | 5766 |
| myoC-6021 | + | CAGCUGGAGAGGAAACCUCUGCC | 23 | 5767 |
| myoC-6022 | + | CCAGCUGGAGAGGAAACCUCUGCC | 24 | 5768 |
| myoC-6023 | + | UGAGAAACUGUCACCUCC | 18 | 5769 |
| myoC-6024 | + | AUGAGAAACUGUCACCUCC | 19 | 5770 |
| myoC-2135 | + | CAUGAGAAACUGUCACCUCC | 20 | 2253 |
| myoC-6025 | + | CCAUGAGAAACUGUCACCUCC | 21 | 5771 |
| myoC-6026 | + | UCCAUGAGAAACUGUCACCUCC | 22 | 5772 |
| myoC-6027 | + | UUCCAUGAGAAACUGUCACCUCC | 23 | 5773 |
| myoC-6028 | + | CUUCCAUGAGAAACUGUCACCUCC | 24 | 5774 |
| myoC-3291 | + | UGGUGGAGGAGGCUCUCC | 18 | 3037 |
| myoC-3292 | + | UUGGUGGAGGAGGCUCUCC | 19 | 3038 |
| myoC-222 | + | AUUGGUGGAGGAGGCUCUCC | 20 | 608 |
| myoC-3293 | + | AAUUGGUGGAGGAGGCUCUCC | 21 | 3039 |
| myoC-3294 | + | CAAUUGGUGGAGGAGGCUCUCC | 22 | 3040 |
| myoC-3295 | + | UCAAUUGGUGGAGGAGGCUCUCC | 23 | 3041 |
| myoC-3296 | + | GUCAAUUGGUGGAGGAGGCUCUCC | 24 | 3042 |
| myoC-3297 | + | AGCCCCUCCUGGGUCUCC | 18 | 3043 |
| myoC-3298 | + | CAGCCCCUCCUGGGUCUCC | 19 | 3044 |
| myoC-119 | + | GCAGCCCCUCCUGGGUCUCC | 20 | 518 |
| myoC-3299 | + | UGCAGCCCCUCCUGGGUCUCC | 21 | 3045 |
| myoC-3300 | + | CUGCAGCCCCUCCUGGGUCUCC | 22 | 3046 |
| myoC-3301 | + | UCUGCAGCCCCUCCUGGGUCUCC | 23 | 3047 |
| myoC-3302 | + | CUCUGCAGCCCCUCCUGGGUCUCC | 24 | 3048 |
| myoC-6029 | + | UUCUUCUGCACGUCUUCC | 18 | 5775 |
| myoC-6030 | + | UUUCUUCUGCACGUCUUCC | 19 | 5776 |
| myoC-2137 | + | UUUUCUUCUGCACGUCUUCC | 20 | 2255 |
| myoC-6031 | + | AUUUUCUUCUGCACGUCUUCC | 21 | 5777 |
| myoC-6032 | + | AAUUUUCUUCUGCACGUCUUCC | 22 | 5778 |
| myoC-6033 | + | UAAUUUUCUUCUGCACGUCUUCC | 23 | 5779 |
| myoC-6034 | + | UUAAUUUUCUUCUGCACGUCUUCC | 24 | 5780 |
| myoC-6035 | + | UUGCAGGGCUCCCCCAGC | 18 | 5781 |
| myoC-6036 | + | CUUGCAGGGCUCCCCCAGC | 19 | 5782 |
| myoC-746 | + | GCUUGCAGGGCUCCCCCAGC | 20 | 1012 |
| myoC-6037 | + | UGCUUGCAGGGCUCCCCCAGC | 21 | 5783 |
| myoC-6038 | + | GUGCUUGCAGGGCUCCCCCAGC | 22 | 5784 |
| myoC-6039 | + | GGUGCUUGCAGGGCUCCCCCAGC | 23 | 5785 |
| myoC-6040 | + | GGGUGCUUGCAGGGCUCCCCCAGC | 24 | 5786 |
| myoC-6041 | + | AGAAAAAUAACAGCCAGC | 18 | 5787 |
| myoC-6042 | + | GAGAAAAAUAACAGCCAGC | 19 | 5788 |
| myoC-2313 | + | AGAGAAAAAUAACAGCCAGC | 20 | 2382 |
| myoC-6043 | + | CAGAGAAAAAUAACAGCCAGC | 21 | 5789 |
| myoC-6044 | + | ACAGAGAAAAAUAACAGCCAGC | 22 | 5790 |
| myoC-6045 | + | GACAGAGAAAAAUAACAGCCAGC | 23 | 5791 |
| myoC-6046 | + | GGACAGAGAAAAAUAACAGCCAGC | 24 | 5792 |
| myoC-6047 | + | GGGCACGAUGGAGGCAGC | 18 | 5793 |
| myoC-6048 | + | CGGGCACGAUGGAGGCAGC | 19 | 5794 |
| myoC-713 | + | CCGGGCACGAUGGAGGCAGC | 20 | 1102 |
| myoC-6049 | + | UCCGGGCACGAUGGAGGCAGC | 21 | 5795 |
| myoC-6050 | + | CUCCGGGCACGAUGGAGGCAGC | 22 | 5796 |
| myoC-6051 | + | CCUCCGGGCACGAUGGAGGCAGC | 23 | 5797 |
| myoC-6052 | + | GCCUCCGGGCACGAUGGAGGCAGC | 24 | 5798 |
| myoC-6053 | + | CCAUUUCCUUUCUGGAGC | 18 | 5799 |
| myoC-6054 | + | UCCAUUUCCUUUCUGGAGC | 19 | 5800 |
| myoC-2170 | + | CUCCAUUUCCUUUCUGGAGC | 20 | 2274 |
| myoC-6055 | + | UCUCCAUUUCCUUUCUGGAGC | 21 | 5801 |
| myoC-6056 | + | CUCUCCAUUUCCUUUCUGGAGC | 22 | 5802 |
| myoC-6057 | + | CCUCUCCAUUUCCUUUCUGGAGC | 23 | 5803 |
| myoC-6058 | + | CCCUCUCCAUUUCCUUUCUGGAGC | 24 | 5804 |
| myoC-6059 | + | AGUCCUUUAAGACGUAGC | 18 | 5805 |
| myoC-6060 | + | AAGUCCUUUAAGACGUAGC | 19 | 5806 |
| myoC-893 | + | CAAGUCCUUUAAGACGUAGC | 20 | 1187 |
| myoC-6061 | + | ACAAGUCCUUUAAGACGUAGC | 21 | 5807 |
| myoC-6062 | + | AACAAGUCCUUUAAGACGUAGC | 22 | 5808 |
| myoC-6063 | + | AAACAAGUCCUUUAAGACGUAGC | 23 | 5809 |
| myoC-6064 | + | CAAACAAGUCCUUUAAGACGUAGC | 24 | 5810 |
| myoC-6065 | + | GGAGGCAGCAGGGGGCGC | 18 | 5811 |
| myoC-6066 | + | UGGAGGCAGCAGGGGGCGC | 19 | 5812 |
| myoC-2143 | + | AUGGAGGCAGCAGGGGGCGC | 20 | 2258 |
| myoC-6067 | + | GAUGGAGGCAGCAGGGGGCGC | 21 | 5813 |
| myoC-6068 | + | CGAUGGAGGCAGCAGGGGGCGC | 22 | 5814 |
| myoC-6069 | + | ACGAUGGAGGCAGCAGGGGGCGC | 23 | 5815 |
| myoC-6070 | + | CACGAUGGAGGCAGCAGGGGGCGC | 24 | 5816 |
| myoC-3303 | + | AUCCCACACCAGGCAGGC | 18 | 3049 |
| myoC-3304 | + | CAUCCCACACCAGGCAGGC | 19 | 3050 |
| myoC-1668 | + | ACAUCCCACACCAGGCAGGC | 20 | 1930 |
| myoC-3305 | + | CACAUCCCACACCAGGCAGGC | 21 | 3051 |
| myoC-3306 | + | CCACAUCCCACACCAGGCAGGC | 22 | 3052 |
| myoC-3307 | + | CCCACAUCCCACACCAGGCAGGC | 23 | 3053 |
| myoC-3308 | + | CCCCACAUCCCACACCAGGCAGGC | 24 | 3054 |
| myoC-6071 | + | ACUGAUGGAGGAGGAGGC | 18 | 5817 |
| myoC-6072 | + | GACUGAUGGAGGAGGAGGC | 19 | 5818 |
| myoC-2155 | + | UGACUGAUGGAGGAGGAGGC | 20 | 2264 |
| myoC-6073 | + | GUGACUGAUGGAGGAGGAGGC | 21 | 5819 |
| myoC-6074 | + | UGUGACUGAUGGAGGAGGAGGC | 22 | 5820 |
| myoC-6075 | + | CUGUGACUGAUGGAGGAGGAGGC | 23 | 5821 |
| myoC-6076 | + | GCUGUGACUGAUGGAGGAGGAGGC | 24 | 5822 |
| myoC-6077 | + | GGCUUGGAAGACUCGGGC | 18 | 5823 |
| myoC-6078 | + | AGGCUUGGAAGACUCGGGC | 19 | 5824 |
| myoC-2152 | + | GAGGCUUGGAAGACUCGGGC | 20 | 2262 |
| myoC-6079 | + | GGAGGCUUGGAAGACUCGGGC | 21 | 5825 |
| myoC-6080 | + | AGGAGGCUUGGAAGACUCGGGC | 22 | 5826 |
| myoC-6081 | + | GAGGAGGCUUGGAAGACUCGGGC | 23 | 5827 |
| myoC-6082 | + | GGAGGAGGCUUGGAAGACUCGGGC | 24 | 5828 |
| myoC-6083 | + | AACAAAACAACCAGUGGC | 18 | 5829 |
| myoC-6084 | + | UAACAAAACAACCAGUGGC | 19 | 5830 |
| myoC-2124 | + | AUAACAAAACAACCAGUGGC | 20 | 2242 |
| myoC-6085 | + | GAUAACAAAACAACCAGUGGC | 21 | 5831 |
| myoC-6086 | + | UGAUAACAAAACAACCAGUGGC | 22 | 5832 |
| myoC-6087 | + | GUGAUAACAAAACAACCAGUGGC | 23 | 5833 |
| myoC-6088 | + | AGUGAUAACAAAACAACCAGUGGC | 24 | 5834 |
| myoC-6089 | + | GGCCUUGCUGGCUCAUGC | 18 | 5835 |
| myoC-6090 | + | UGGCCUUGCUGGCUCAUGC | 19 | 5836 |
| myoC-2351 | + | GUGGCCUUGCUGGCUCAUGC | 20 | 2405 |
| myoC-6091 | + | GGUGGCCUUGCUGGCUCAUGC | 21 | 5837 |
| myoC-6092 | + | GGGUGGCCUUGCUGGCUCAUGC | 22 | 5838 |
| myoC-6093 | + | UGGGUGGCCUUGCUGGCUCAUGC | 23 | 5839 |
| myoC-6094 | + | AUGGGUGGCCUUGCUGGCUCAUGC | 24 | 5840 |
| myoC-6095 | + | CUGUGCCAGGCACUAUGC | 18 | 5841 |
| myoC-6096 | + | ACUGUGCCAGGCACUAUGC | 19 | 5842 |
| myoC-2321 | + | CACUGUGCCAGGCACUAUGC | 20 | 2387 |
| myoC-6097 | + | GCACUGUGCCAGGCACUAUGC | 21 | 5843 |
| myoC-6098 | + | UGCACUGUGCCAGGCACUAUGC | 22 | 5844 |
| myoC-6099 | + | CUGCACUGUGCCAGGCACUAUGC | 23 | 5845 |
| myoC-6100 | + | CCUGCACUGUGCCAGGCACUAUGC | 24 | 5846 |
| myoC-6101 | + | UGGAGAGGAAACCUCUGC | 18 | 5847 |
| myoC-6102 | + | CUGGAGAGGAAACCUCUGC | 19 | 5848 |
| myoC-748 | + | GCUGGAGAGGAAACCUCUGC | 20 | 1010 |
| myoC-6103 | + | AGCUGGAGAGGAAACCUCUGC | 21 | 5849 |
| myoC-6104 | + | CAGCUGGAGAGGAAACCUCUGC | 22 | 5850 |
| myoC-6105 | + | CCAGCUGGAGAGGAAACCUCUGC | 23 | 5851 |
| myoC-6106 | + | CCCAGCUGGAGAGGAAACCUCUGC | 24 | 5852 |
| myoC-6107 | + | CCCUGCAGUCCCCACCUC | 18 | 5853 |
| myoC-6108 | + | UCCCUGCAGUCCCCACCUC | 19 | 5854 |
| myoC-2180 | + | CUCCCUGCAGUCCCCACCUC | 20 | 2280 |
| myoC-6109 | + | ACUCCCUGCAGUCCCCACCUC | 21 | 5855 |
| myoC-6110 | + | CACUCCCUGCAGUCCCCACCUC | 22 | 5856 |
| myoC-6111 | + | CCACUCCCUGCAGUCCCCACCUC | 23 | 5857 |
| myoC-6112 | + | CCCACUCCCUGCAGUCCCCACCUC | 24 | 5858 |
| myoC-3309 | + | GCUUGGUGAGGCUUCCUC | 18 | 3055 |
| myoC-3310 | + | GGCUUGGUGAGGCUUCCUC | 19 | 3056 |
| myoC-2356 | + | AGGCUUGGUGAGGCUUCCUC | 20 | 2410 |
| myoC-3311 | + | GAGGCUUGGUGAGGCUUCCUC | 21 | 3057 |
| myoC-3312 | + | AGAGGCUUGGUGAGGCUUCCUC | 22 | 3058 |
| myoC-3313 | + | CAGAGGCUUGGUGAGGCUUCCUC | 23 | 3059 |
| myoC-3314 | + | GCAGAGGCUUGGUGAGGCUUCCUC | 24 | 3060 |
| myoC-3315 | + | UCGCUUCUUCUCUUCCUC | 18 | 3061 |
| myoC-3316 | + | GUCGCUUCUUCUCUUCCUC | 19 | 3062 |
| myoC-1696 | + | AGUCGCUUCUUCUCUUCCUC | 20 | 1950 |
| myoC-3317 | + | UAGUCGCUUCUUCUCUUCCUC | 21 | 3063 |
| myoC-3318 | + | UUAGUCGCUUCUUCUCUUCCUC | 22 | 3064 |
| myoC-3319 | + | CUUAGUCGCUUCUUCUCUUCCUC | 23 | 3065 |
| myoC-3320 | + | CCUUAGUCGCUUCUUCUCUUCCUC | 24 | 3066 |
| myoC-6113 | + | UGGCUCAUGCCCGAGCUC | 18 | 5859 |
| myoC-6114 | + | CUGGCUCAUGCCCGAGCUC | 19 | 5860 |
| myoC-2350 | + | GCUGGCUCAUGCCCGAGCUC | 20 | 2404 |
| myoC-6115 | + | UGCUGGCUCAUGCCCGAGCUC | 21 | 5861 |
| myoC-6116 | + | UUGCUGGCUCAUGCCCGAGCUC | 22 | 5862 |
| myoC-6117 | + | CUUGCUGGCUCAUGCCCGAGCUC | 23 | 5863 |
| myoC-6118 | + | CCUUGCUGGCUCAUGCCCGAGCUC | 24 | 5864 |
| myoC-3321 | + | UUGGUGGAGGAGGCUCUC | 18 | 3067 |
| myoC-3322 | + | AUUGGUGGAGGAGGCUCUC | 19 | 3068 |
| myoC-1682 | + | AAUUGGUGGAGGAGGCUCUC | 20 | 1941 |
| myoC-3323 | + | CAAUUGGUGGAGGAGGCUCUC | 21 | 3069 |
| myoC-3324 | + | UCAAUUGGUGGAGGAGGCUCUC | 22 | 3070 |
| myoC-3325 | + | GUCAAUUGGUGGAGGAGGCUCUC | 23 | 3071 |
| myoC-3326 | + | GGUCAAUUGGUGGAGGAGGCUCUC | 24 | 3072 |
| myoC-3327 | + | CAGCCCCUCCUGGGUCUC | 18 | 3073 |
| myoC-3328 | + | GCAGCCCCUCCUGGGUCUC | 19 | 3074 |
| myoC-1688 | + | UGCAGCCCCUCCUGGGUCUC | 20 | 1944 |
| myoC-3329 | + | CUGCAGCCCCUCCUGGGUCUC | 21 | 3075 |
| myoC-3330 | + | UCUGCAGCCCCUCCUGGGUCUC | 22 | 3076 |
| myoC-3331 | + | CUCUGCAGCCCCUCCUGGGUCUC | 23 | 3077 |
| myoC-3332 | + | CCUCUGCAGCCCCUCCUGGGUCUC | 24 | 3078 |
| myoC-6119 | + | CCACCUCCUGGAAUUCUC | 18 | 5865 |
| myoC-6120 | + | CCCACCUCCUGGAAUUCUC | 19 | 5866 |
| myoC-2178 | + | CCCCACCUCCUGGAAUUCUC | 20 | 2279 |
| myoC-6121 | + | UCCCCACCUCCUGGAAUUCUC | 21 | 5867 |
| myoC-6122 | + | GUCCCCACCUCCUGGAAUUCUC | 22 | 5868 |
| myoC-6123 | + | AGUCCCCACCUCCUGGAAUUCUC | 23 | 5869 |
| myoC-6124 | + | CAGUCCCCACCUCCUGGAAUUCUC | 24 | 5870 |
| myoC-3333 | + | CUCCAGAACUGACUUGUC | 18 | 3079 |
| myoC-3334 | + | CCUCCAGAACUGACUUGUC | 19 | 3080 |
| myoC-1695 | + | UCCUCCAGAACUGACUUGUC | 20 | 1949 |
| myoC-3335 | + | UUCCUCCAGAACUGACUUGUC | 21 | 3081 |
| myoC-3336 | + | CUUCCUCCAGAACUGACUUGUC | 22 | 3082 |
| myoC-3337 | + | UCUUCCUCCAGAACUGACUUGUC | 23 | 3083 |
| myoC-3338 | + | CUCUUCCUCCAGAACUGACUUGUC | 24 | 3084 |
| myoC-6125 | + | GAUGCACCAGGACGAUUC | 18 | 5871 |
| myoC-6126 | + | AGAUGCACCAGGACGAUUC | 19 | 5872 |
| myoC-2165 | + | CAGAUGCACCAGGACGAUUC | 20 | 2271 |
| myoC-6127 | + | UCAGAUGCACCAGGACGAUUC | 21 | 5873 |
| myoC-6128 | + | CUCAGAUGCACCAGGACGAUUC | 22 | 5874 |
| myoC-6129 | + | GCUCAGAUGCACCAGGACGAUUC | 23 | 5875 |
| myoC-6130 | + | AGCUCAGAUGCACCAGGACGAUUC | 24 | 5876 |
| myoC-6131 | + | UCUUAGAAAAUAACCUUC | 18 | 5877 |
| myoC-6132 | + | UUCUUAGAAAAUAACCUUC | 19 | 5878 |
| myoC-2329 | + | AUUCUUAGAAAAUAACCUUC | 20 | 2394 |
| myoC-6133 | + | GAUUCUUAGAAAAUAACCUUC | 21 | 5879 |
| myoC-6134 | + | AGAUUCUUAGAAAAUAACCUUC | 22 | 5880 |
| myoC-6135 | + | AAGAUUCUUAGAAAAUAACCUUC | 23 | 5881 |
| myoC-6136 | + | CAAGAUUCUUAGAAAAUAACCUUC | 24 | 5882 |
| myoC-6137 | + | CUGGGGAGCCAGCCCUUC | 18 | 5883 |
| myoC-6138 | + | ACUGGGGAGCCAGCCCUUC | 19 | 5884 |
| myoC-2344 | + | UACUGGGGAGCCAGCCCUUC | 20 | 2401 |
| myoC-6139 | + | AUACUGGGGAGCCAGCCCUUC | 21 | 5885 |
| myoC-6140 | + | UAUACUGGGGAGCCAGCCCUUC | 22 | 5886 |
| myoC-6141 | + | AUAUACUGGGGAGCCAGCCCUUC | 23 | 5887 |
| myoC-6142 | + | UAUAUACUGGGGAGCCAGCCCUUC | 24 | 5888 |
| myoC-6143 | + | AUGAAACUGCAUCCCUUC | 18 | 5889 |
| myoC-6144 | + | UAUGAAACUGCAUCCCUUC | 19 | 5890 |
| myoC-2190 | + | UUAUGAAACUGCAUCCCUUC | 20 | 2288 |
| myoC-6145 | + | UUUAUGAAACUGCAUCCCUUC | 21 | 5891 |
| myoC-6146 | + | CUUUAUGAAACUGCAUCCCUUC | 22 | 5892 |
| myoC-6147 | + | ACUUUAUGAAACUGCAUCCCUUC | 23 | 5893 |
| myoC-6148 | + | GACUUUAUGAAACUGCAUCCCUUC | 24 | 5894 |
| myoC-6149 | + | CAUUAAAUAAAGGCCUUC | 18 | 5895 |
| myoC-6150 | + | CCAUUAAAUAAAGGCCUUC | 19 | 5896 |
| myoC-2196 | + | CCCAUUAAAUAAAGGCCUUC | 20 | 2292 |
| myoC-6151 | + | UCCCAUUAAAUAAAGGCCUUC | 21 | 5897 |
| myoC-6152 | + | UUCCCAUUAAAUAAAGGCCUUC | 22 | 5898 |
| myoC-6153 | + | AUUCCCAUUAAAUAAAGGCCUUC | 23 | 5899 |
| myoC-6154 | + | UAUUCCCAUUAAAUAAAGGCCUUC | 24 | 5900 |
| myoC-3339 | + | CUCUGGUCAUUGGCCUUC | 18 | 3085 |
| myoC-3340 | + | ACUCUGGUCAUUGGCCUUC | 19 | 3086 |
| myoC-1670 | + | CACUCUGGUCAUUGGCCUUC | 20 | 1932 |
| myoC-3341 | + | CCACUCUGGUCAUUGGCCUUC | 21 | 3087 |
| myoC-3342 | + | GCCACUCUGGUCAUUGGCCUUC | 22 | 3088 |
| myoC-3343 | + | GGCCACUCUGGUCAUUGGCCUUC | 23 | 3089 |
| myoC-3344 | + | CGGCCACUCUGGUCAUUGGCCUUC | 24 | 3090 |
| myoC-6155 | + | CCCUCUCCAUUUCCUUUC | 18 | 5901 |
| myoC-6156 | + | UCCCUCUCCAUUUCCUUUC | 19 | 5902 |
| myoC-1113 | + | UUCCCUCUCCAUUUCCUUUC | 20 | 1413 |
| myoC-6157 | + | UUUCCCUCUCCAUUUCCUUUC | 21 | 5903 |
| myoC-6158 | + | GUUUCCCUCUCCAUUUCCUUUC | 22 | 5904 |
| myoC-6159 | + | AGUUUCCCUCUCCAUUUCCUUUC | 23 | 5905 |
| myoC-6160 | + | UAGUUUCCCUCUCCAUUUCCUUUC | 24 | 5906 |
| myoC-6161 | + | GACUUCUUCUCCUCCAAG | 18 | 5907 |
| myoC-6162 | + | AGACUUCUUCUCCUCCAAG | 19 | 5908 |
| myoC-2189 | + | UAGACUUCUUCUCCUCCAAG | 20 | 2287 |
| myoC-6163 | + | AUAGACUUCUUCUCCUCCAAG | 21 | 5909 |
| myoC-6164 | + | AAUAGACUUCUUCUCCUCCAAG | 22 | 5910 |
| myoC-6165 | + | AAAUAGACUUCUUCUCCUCCAAG | 23 | 5911 |
| myoC-6166 | + | GAAAUAGACUUCUUCUCCUCCAAG | 24 | 5912 |
| myoC-3345 | + | CUGCAGCAACGUGCACAG | 18 | 3091 |
| myoC-3346 | + | GCUGCAGCAACGUGCACAG | 19 | 3092 |
| myoC-1665 | + | AGCUGCAGCAACGUGCACAG | 20 | 1927 |
| myoC-3347 | + | AAGCUGCAGCAACGUGCACAG | 21 | 3093 |
| myoC-3348 | + | AAAGCUGCAGCAACGUGCACAG | 22 | 3094 |
| myoC-3349 | + | CAAAGCUGCAGCAACGUGCACAG | 23 | 3095 |
| myoC-3350 | + | CCAAAGCUGCAGCAACGUGCACAG | 24 | 3096 |
| myoC-6167 | + | CUUGCAGGGCUCCCCCAG | 18 | 5913 |
| myoC-6168 | + | GCUUGCAGGGCUCCCCCAG | 19 | 5914 |
| myoC-2119 | + | UGCUUGCAGGGCUCCCCCAG | 20 | 2237 |
| myoC-6169 | + | GUGCUUGCAGGGCUCCCCCAG | 21 | 5915 |
| myoC-6170 | + | GGUGCUUGCAGGGCUCCCCCAG | 22 | 5916 |
| myoC-6171 | + | GGGUGCUUGCAGGGCUCCCCCAG | 23 | 5917 |
| myoC-6172 | + | CGGGUGCUUGCAGGGCUCCCCCAG | 24 | 5918 |
| myoC-3351 | + | GCAGGCCAGAAGCAGCAG | 18 | 3097 |
| myoC-3352 | + | GGCAGGCCAGAAGCAGCAG | 19 | 3098 |
| myoC-1667 | + | AGGCAGGCCAGAAGCAGCAG | 20 | 1929 |
| myoC-3353 | + | CAGGCAGGCCAGAAGCAGCAG | 21 | 3099 |
| myoC-3354 | + | CCAGGCAGGCCAGAAGCAGCAG | 22 | 3100 |
| myoC-3355 | + | ACCAGGCAGGCCAGAAGCAGCAG | 23 | 3101 |
| myoC-3356 | + | CACCAGGCAGGCCAGAAGCAGCAG | 24 | 3102 |
| myoC-6173 | + | CGGGCACGAUGGAGGCAG | 18 | 5919 |
| myoC-6174 | + | CCGGGCACGAUGGAGGCAG | 19 | 5920 |
| myoC-2146 | + | UCCGGGCACGAUGGAGGCAG | 20 | 2259 |
| myoC-6175 | + | CUCCGGGCACGAUGGAGGCAG | 21 | 5921 |
| myoC-6176 | + | CCUCCGGGCACGAUGGAGGCAG | 22 | 5922 |
| myoC-6177 | + | GCCUCCGGGCACGAUGGAGGCAG | 23 | 5923 |
| myoC-6178 | + | GGCCUCCGGGCACGAUGGAGGCAG | 24 | 5924 |
| myoC-6179 | + | GCGCUGUGACUGAUGGAG | 18 | 5925 |
| myoC-6180 | + | AGCGCUGUGACUGAUGGAG | 19 | 5926 |
| myoC-2157 | + | CAGCGCUGUGACUGAUGGAG | 20 | 2265 |
| myoC-6181 | + | GCAGCGCUGUGACUGAUGGAG | 21 | 5927 |
| myoC-6182 | + | UGCAGCGCUGUGACUGAUGGAG | 22 | 5928 |
| myoC-6183 | + | CUGCAGCGCUGUGACUGAUGGAG | 23 | 5929 |
| myoC-6184 | + | GCUGCAGCGCUGUGACUGAUGGAG | 24 | 5930 |
| myoC-6185 | + | GGGCUCCCCCAGCUGGAG | 18 | 5931 |
| myoC-6186 | + | AGGGCUCCCCCAGCUGGAG | 19 | 5932 |
| myoC-747 | + | CAGGGCUCCCCCAGCUGGAG | 20 | 1099 |
| myoC-6187 | + | GCAGGGCUCCCCCAGCUGGAG | 21 | 5933 |
| myoC-6188 | + | UGCAGGGCUCCCCCAGCUGGAG | 22 | 5934 |
| myoC-6189 | + | UUGCAGGGCUCCCCCAGCUGGAG | 23 | 5935 |
| myoC-6190 | + | CUUGCAGGGCUCCCCCAGCUGGAG | 24 | 5936 |
| myoC-3357 | + | GUCAUUGGCCUUCCUGAG | 18 | 3103 |
| myoC-3358 | + | GGUCAUUGGCCUUCCUGAG | 19 | 3104 |
| myoC-1669 | + | UGGUCAUUGGCCUUCCUGAG | 20 | 1931 |
| myoC-3359 | + | CUGGUCAUUGGCCUUCCUGAG | 21 | 3105 |
| myoC-3360 | + | UCUGGUCAUUGGCCUUCCUGAG | 22 | 3106 |
| myoC-3361 | + | CUCUGGUCAUUGGCCUUCCUGAG | 23 | 3107 |
| myoC-3362 | + | ACUCUGGUCAUUGGCCUUCCUGAG | 24 | 3108 |
| myoC-3363 | + | GCUCUCCAGGGAGCUGAG | 18 | 3109 |
| myoC-3364 | + | GGCUCUCCAGGGAGCUGAG | 19 | 3110 |
| myoC-1678 | + | AGGCUCUCCAGGGAGCUGAG | 20 | 1939 |
| myoC-3365 | + | GAGGCUCUCCAGGGAGCUGAG | 21 | 3111 |
| myoC-3366 | + | GGAGGCUCUCCAGGGAGCUGAG | 22 | 3112 |
| myoC-3367 | + | AGGAGGCUCUCCAGGGAGCUGAG | 23 | 3113 |
| myoC-3368 | + | GAGGAGGCUCUCCAGGGAGCUGAG | 24 | 3114 |
| myoC-6191 | + | AAGUCCUUUAAGACGUAG | 18 | 5937 |
| myoC-6192 | + | CAAGUCCUUUAAGACGUAG | 19 | 5938 |
| myoC-2317 | + | ACAAGUCCUUUAAGACGUAG | 20 | 2385 |
| myoC-6193 | + | AACAAGUCCUUUAAGACGUAG | 21 | 5939 |
| myoC-6194 | + | AAACAAGUCCUUUAAGACGUAG | 22 | 5940 |
| myoC-6195 | + | CAAACAAGUCCUUUAAGACGUAG | 23 | 5941 |
| myoC-6196 | + | CCAAACAAGUCCUUUAAGACGUAG | 24 | 5942 |
| myoC-6197 | + | UGGGGGCCUCCGGGCACG | 18 | 5943 |
| myoC-6198 | + | UUGGGGGCCUCCGGGCACG | 19 | 5944 |
| myoC-2148 | + | CUUGGGGGCCUCCGGGCACG | 20 | 2260 |
| myoC-6199 | + | GCUUGGGGGCCUCCGGGCACG | 21 | 5945 |
| myoC-6200 | + | GGCUUGGGGGCCUCCGGGCACG | 22 | 5946 |
| myoC-6201 | + | GGGCUUGGGGGCCUCCGGGCACG | 23 | 5947 |
| myoC-6202 | + | CGGGCUUGGGGGCCUCCGGGCACG | 24 | 5948 |
| myoC-6203 | + | CUGGAAUUCUCCUGGACG | 18 | 5949 |
| myoC-6204 | + | CCUGGAAUUCUCCUGGACG | 19 | 5950 |
| myoC-1110 | + | UCCUGGAAUUCUCCUGGACG | 20 | 1410 |
| myoC-6205 | + | CUCCUGGAAUUCUCCUGGACG | 21 | 5951 |
| myoC-6206 | + | CCUCCUGGAAUUCUCCUGGACG | 22 | 5952 |
| myoC-6207 | + | ACCUCCUGGAAUUCUCCUGGACG | 23 | 5953 |
| myoC-6208 | + | CACCUCCUGGAAUUCUCCUGGACG | 24 | 5954 |
| myoC-3369 | + | CAGAACUGACUUGUCUCG | 18 | 3115 |
| myoC-3370 | + | CCAGAACUGACUUGUCUCG | 19 | 3116 |
| myoC-1693 | + | UCCAGAACUGACUUGUCUCG | 20 | 1948 |
| myoC-3371 | + | CUCCAGAACUGACUUGUCUCG | 21 | 3117 |
| myoC-3372 | + | CCUCCAGAACUGACUUGUCUCG | 22 | 3118 |
| myoC-3373 | + | UCCUCCAGAACUGACUUGUCUCG | 23 | 3119 |
| myoC-3374 | + | UUCCUCCAGAACUGACUUGUCUCG | 24 | 3120 |
| myoC-6209 | + | CUGUCACCUCCACGAAGG | 18 | 5955 |
| myoC-6210 | + | ACUGUCACCUCCACGAAGG | 19 | 5956 |
| myoC-2134 | + | AACUGUCACCUCCACGAAGG | 20 | 2252 |
| myoC-6211 | + | AAACUGUCACCUCCACGAAGG | 21 | 5957 |
| myoC-6212 | + | GAAACUGUCACCUCCACGAAGG | 22 | 5958 |
| myoC-6213 | + | AGAAACUGUCACCUCCACGAAGG | 23 | 5959 |
| myoC-6214 | + | GAGAAACUGUCACCUCCACGAAGG | 24 | 5960 |
| myoC-6215 | + | CGCUGUGACUGAUGGAGG | 18 | 5961 |
| myoC-6216 | + | GCGCUGUGACUGAUGGAGG | 19 | 5962 |
| myoC-700 | + | AGCGCUGUGACUGAUGGAGG | 20 | 1088 |
| myoC-6217 | + | CAGCGCUGUGACUGAUGGAGG | 21 | 5963 |
| myoC-6218 | + | GCAGCGCUGUGACUGAUGGAGG | 22 | 5964 |
| myoC-6219 | + | UGCAGCGCUGUGACUGAUGGAGG | 23 | 5965 |
| myoC-6220 | + | CUGCAGCGCUGUGACUGAUGGAGG | 24 | 5966 |
| myoC-3375 | + | AGAACUGACUUGUCUCGG | 18 | 3121 |
| myoC-3376 | + | CAGAACUGACUUGUCUCGG | 19 | 3122 |
| myoC-209 | + | CCAGAACUGACUUGUCUCGG | 20 | 595 |
| myoC-3377 | + | UCCAGAACUGACUUGUCUCGG | 21 | 3123 |
| myoC-3378 | + | CUCCAGAACUGACUUGUCUCGG | 22 | 3124 |
| myoC-3379 | + | CCUCCAGAACUGACUUGUCUCGG | 23 | 3125 |
| myoC-3380 | + | UCCUCCAGAACUGACUUGUCUCGG | 24 | 3126 |
| myoC-6221 | + | UGGCCACGUGAGGCUGGG | 18 | 5967 |
| myoC-6222 | + | GUGGCCACGUGAGGCUGGG | 19 | 5968 |
| myoC-877 | + | GGUGGCCACGUGAGGCUGGG | 20 | 1053 |
| myoC-6223 | + | AGGUGGCCACGUGAGGCUGGG | 21 | 5969 |
| myoC-6224 | + | GAGGUGGCCACGUGAGGCUGGG | 22 | 5970 |
| myoC-6225 | + | AGAGGUGGCCACGUGAGGCUGGG | 23 | 5971 |
| myoC-6226 | + | CAGAGGUGGCCACGUGAGGCUGGG | 24 | 5972 |
| myoC-6227 | + | GGAGCCAGCCCUUCAUGG | 18 | 5973 |
| myoC-6228 | + | GGGAGCCAGCCCUUCAUGG | 19 | 5974 |
| myoC-871 | + | GGGGAGCCAGCCCUUCAUGG | 20 | 992 |
| myoC-6229 | + | UGGGGAGCCAGCCCUUCAUGG | 21 | 5975 |
| myoC-6230 | + | CUGGGGAGCCAGCCCUUCAUGG | 22 | 5976 |
| myoC-6231 | + | ACUGGGGAGCCAGCCCUUCAUGG | 23 | 5977 |
| myoC-6232 | + | UACUGGGGAGCCAGCCCUUCAUGG | 24 | 5978 |
| myoC-6233 | + | CAGCGCUGUGACUGAUGG | 18 | 5979 |
| myoC-6234 | + | GCAGCGCUGUGACUGAUGG | 19 | 5980 |
| myoC-699 | + | UGCAGCGCUGUGACUGAUGG | 20 | 1118 |
| myoC-6235 | + | CUGCAGCGCUGUGACUGAUGG | 21 | 5981 |
| myoC-6236 | + | GCUGCAGCGCUGUGACUGAUGG | 22 | 5982 |
| myoC-6237 | + | AGCUGCAGCGCUGUGACUGAUGG | 23 | 5983 |
| myoC-6238 | + | CAGCUGCAGCGCUGUGACUGAUGG | 24 | 5984 |
| myoC-6239 | + | GUGGCCACGUGAGGCUGG | 18 | 5985 |
| myoC-6240 | + | GGUGGCCACGUGAGGCUGG | 19 | 5986 |
| myoC-2336 | + | AGGUGGCCACGUGAGGCUGG | 20 | 2397 |
| myoC-6241 | + | GAGGUGGCCACGUGAGGCUGG | 21 | 5987 |
| myoC-6242 | + | AGAGGUGGCCACGUGAGGCUGG | 22 | 5988 |
| myoC-6243 | + | CAGAGGUGGCCACGUGAGGCUGG | 23 | 5989 |
| myoC-6244 | + | ACAGAGGUGGCCACGUGAGGCUGG | 24 | 5990 |
| myoC-3381 | + | UCCAAGGUCAAUUGGUGG | 18 | 3127 |
| myoC-3382 | + | GUCCAAGGUCAAUUGGUGG | 19 | 3128 |
| myoC-121 | + | GGUCCAAGGUCAAUUGGUGG | 20 | 520 |
| myoC-3383 | + | UGGUCCAAGGUCAAUUGGUGG | 21 | 3129 |
| myoC-3384 | + | CUGGUCCAAGGUCAAUUGGUGG | 22 | 3130 |
| myoC-3385 | + | CCUGGUCCAAGGUCAAUUGGUGG | 23 | 3131 |
| myoC-3386 | + | GCCUGGUCCAAGGUCAAUUGGUGG | 24 | 3132 |
| myoC-3387 | + | UGGUCCAAGGUCAAUUGG | 18 | 3133 |
| myoC-3388 | + | CUGGUCCAAGGUCAAUUGG | 19 | 3134 |
| myoC-220 | + | CCUGGUCCAAGGUCAAUUGG | 20 | 606 |
| myoC-3389 | + | GCCUGGUCCAAGGUCAAUUGG | 21 | 3135 |
| myoC-3390 | + | AGCCUGGUCCAAGGUCAAUUGG | 22 | 3136 |
| myoC-3391 | + | CAGCCUGGUCCAAGGUCAAUUGG | 23 | 3137 |
| myoC-3392 | + | GCAGCCUGGUCCAAGGUCAAUUGG | 24 | 3138 |
| myoC-6245 | + | GGGAGCCAGCCCUUCAUG | 18 | 5991 |
| myoC-6246 | + | GGGGAGCCAGCCCUUCAUG | 19 | 5992 |
| myoC-870 | + | UGGGGAGCCAGCCCUUCAUG | 20 | 1213 |
| myoC-6247 | + | CUGGGGAGCCAGCCCUUCAUG | 21 | 5993 |
| myoC-6248 | + | ACUGGGGAGCCAGCCCUUCAUG | 22 | 5994 |
| myoC-6249 | + | UACUGGGGAGCCAGCCCUUCAUG | 23 | 5995 |
| myoC-6250 | + | AUACUGGGGAGCCAGCCCUUCAUG | 24 | 5996 |
| myoC-6251 | + | GCAGCGCUGUGACUGAUG | 18 | 5997 |
| myoC-6252 | + | UGCAGCGCUGUGACUGAUG | 19 | 5998 |
| myoC-2159 | + | CUGCAGCGCUGUGACUGAUG | 20 | 2266 |
| myoC-6253 | + | GCUGCAGCGCUGUGACUGAUG | 21 | 5999 |
| myoC-6254 | + | AGCUGCAGCGCUGUGACUGAUG | 22 | 6000 |
| myoC-6255 | + | CAGCUGCAGCGCUGUGACUGAUG | 23 | 6001 |
| myoC-6256 | + | CCAGCUGCAGCGCUGUGACUGAUG | 24 | 6002 |
| myoC-6257 | + | GCUGCAGCGCUGUGACUG | 18 | 6003 |
| myoC-6258 | + | AGCUGCAGCGCUGUGACUG | 19 | 6004 |
| myoC-2161 | + | CAGCUGCAGCGCUGUGACUG | 20 | 2267 |
| myoC-6259 | + | CCAGCUGCAGCGCUGUGACUG | 21 | 6005 |
| myoC-6260 | + | GCCAGCUGCAGCGCUGUGACUG | 22 | 6006 |
| myoC-6261 | + | GGCCAGCUGCAGCGCUGUGACUG | 23 | 6007 |
| myoC-6262 | + | AGGCCAGCUGCAGCGCUGUGACUG | 24 | 6008 |
| myoC-6263 | + | AGAGGUUUAUAUAUACUG | 18 | 6009 |
| myoC-6264 | + | GAGAGGUUUAUAUAUACUG | 19 | 6010 |
| myoC-867 | + | AGAGAGGUUUAUAUAUACUG | 20 | 1180 |
| myoC-6265 | + | CAGAGAGGUUUAUAUAUACUG | 21 | 6011 |
| myoC-6266 | + | CCAGAGAGGUUUAUAUAUACUG | 22 | 6012 |
| myoC-6267 | + | UCCAGAGAGGUUUAUAUAUACUG | 23 | 6013 |
| myoC-6268 | + | CUCCAGAGAGGUUUAUAUAUACUG | 24 | 6014 |
| myoC-6269 | + | GCAGGGCUCCCCCAGCUG | 18 | 6015 |
| myoC-6270 | + | UGCAGGGCUCCCCCAGCUG | 19 | 6016 |
| myoC-2117 | + | UUGCAGGGCUCCCCCAGCUG | 20 | 2236 |
| myoC-6271 | + | CUUGCAGGGCUCCCCCAGCUG | 21 | 6017 |
| myoC-6272 | + | GCUUGCAGGGCUCCCCCAGCUG | 22 | 6018 |
| myoC-6273 | + | UGCUUGCAGGGCUCCCCCAGCUG | 23 | 6019 |
| myoC-6274 | + | GUGCUUGCAGGGCUCCCCCAGCUG | 24 | 6020 |
| myoC-6275 | + | CUGGAGAGGAAACCUCUG | 18 | 6021 |
| myoC-6276 | + | GCUGGAGAGGAAACCUCUG | 19 | 6022 |
| myoC-2114 | + | AGCUGGAGAGGAAACCUCUG | 20 | 2234 |
| myoC-6277 | + | CAGCUGGAGAGGAAACCUCUG | 21 | 6023 |
| myoC-6278 | + | CCAGCUGGAGAGGAAACCUCUG | 22 | 6024 |
| myoC-6279 | + | CCCAGCUGGAGAGGAAACCUCUG | 23 | 6025 |
| myoC-6280 | + | CCCCAGCUGGAGAGGAAACCUCUG | 24 | 6026 |
| myoC-6281 | + | GAGGCCCCUUUCCCUCUG | 18 | 6027 |
| myoC-6282 | + | GGAGGCCCCUUUCCCUCUG | 19 | 6028 |
| myoC-1112 | + | UGGAGGCCCCUUUCCCUCUG | 20 | 1412 |
| myoC-6283 | + | GUGGAGGCCCCUUUCCCUCUG | 21 | 6029 |
| myoC-6284 | + | CGUGGAGGCCCCUUUCCCUCUG | 22 | 6030 |
| myoC-6285 | + | ACGUGGAGGCCCCUUUCCCUCUG | 23 | 6031 |
| myoC-6286 | + | GACGUGGAGGCCCCUUUCCCUCUG | 24 | 6032 |
| myoC-6287 | + | UAAAUAAAGGCCUUCGUG | 18 | 6033 |
| myoC-6288 | + | UUAAAUAAAGGCCUUCGUG | 19 | 6034 |
| myoC-2195 | + | AUUAAAUAAAGGCCUUCGUG | 20 | 2291 |
| myoC-6289 | + | CAUUAAAUAAAGGCCUUCGUG | 21 | 6035 |
| myoC-6290 | + | CCAUUAAAUAAAGGCCUUCGUG | 22 | 6036 |
| myoC-6291 | + | CCCAUUAAAUAAAGGCCUUCGUG | 23 | 6037 |
| myoC-6292 | + | UCCCAUUAAAUAAAGGCCUUCGUG | 24 | 6038 |
| myoC-3393 | + | GUCCAAGGUCAAUUGGUG | 18 | 3139 |
| myoC-3394 | + | GGUCCAAGGUCAAUUGGUG | 19 | 3140 |
| myoC-1684 | + | UGGUCCAAGGUCAAUUGGUG | 20 | 1942 |
| myoC-3395 | + | CUGGUCCAAGGUCAAUUGGUG | 21 | 3141 |
| myoC-3396 | + | CCUGGUCCAAGGUCAAUUGGUG | 22 | 3142 |
| myoC-3397 | + | GCCUGGUCCAAGGUCAAUUGGUG | 23 | 3143 |
| myoC-3398 | + | AGCCUGGUCCAAGGUCAAUUGGUG | 24 | 3144 |
| myoC-6293 | + | CUGGAAAGCUCUGCUGUG | 18 | 6039 |
| myoC-6294 | + | UCUGGAAAGCUCUGCUGUG | 19 | 6040 |
| myoC-2355 | + | CUCUGGAAAGCUCUGCUGUG | 20 | 2409 |
| myoC-6295 | + | CCUCUGGAAAGCUCUGCUGUG | 21 | 6041 |
| myoC-6296 | + | UCCUCUGGAAAGCUCUGCUGUG | 22 | 6042 |
| myoC-6297 | + | UUCCUCUGGAAAGCUCUGCUGUG | 23 | 6043 |
| myoC-6298 | + | CUUCCUCUGGAAAGCUCUGCUGUG | 24 | 6044 |
| myoC-3399 | + | CUGGUCCAAGGUCAAUUG | 18 | 3145 |
| myoC-3400 | + | CCUGGUCCAAGGUCAAUUG | 19 | 3146 |
| myoC-1686 | + | GCCUGGUCCAAGGUCAAUUG | 20 | 1943 |
| myoC-3401 | + | AGCCUGGUCCAAGGUCAAUUG | 21 | 3147 |
| myoC-3402 | + | CAGCCUGGUCCAAGGUCAAUUG | 22 | 3148 |
| myoC-3403 | + | GCAGCCUGGUCCAAGGUCAAUUG | 23 | 3149 |
| myoC-3404 | + | GGCAGCCUGGUCCAAGGUCAAUUG | 24 | 3150 |
| myoC-3405 | + | CACAGAAGAACCUCAUUG | 18 | 3151 |
| myoC-3406 | + | GCACAGAAGAACCUCAUUG | 19 | 3152 |
| myoC-1664 | + | UGCACAGAAGAACCUCAUUG | 20 | 1926 |
| myoC-3407 | + | GUGCACAGAAGAACCUCAUUG | 21 | 3153 |
| myoC-3408 | + | CGUGCACAGAAGAACCUCAUUG | 22 | 3154 |
| myoC-3409 | + | ACGUGCACAGAAGAACCUCAUUG | 23 | 3155 |
| myoC-3410 | + | AACGUGCACAGAAGAACCUCAUUG | 24 | 3156 |
| myoC-3411 | + | CCUCAUUGCAGAGGCUUG | 18 | 3157 |
| myoC-3412 | + | ACCUCAUUGCAGAGGCUUG | 19 | 3158 |
| myoC-1663 | + | AACCUCAUUGCAGAGGCUUG | 20 | 1925 |
| myoC-3413 | + | GAACCUCAUUGCAGAGGCUUG | 21 | 3159 |
| myoC-3414 | + | AGAACCUCAUUGCAGAGGCUUG | 22 | 3160 |
| myoC-3415 | + | AAGAACCUCAUUGCAGAGGCUUG | 23 | 3161 |
| myoC-3416 | + | GAAGAACCUCAUUGCAGAGGCUUG | 24 | 3162 |
| myoC-6299 | + | CAGGACCCCGGGUGCUUG | 18 | 6045 |
| myoC-6300 | + | CCAGGACCCCGGGUGCUUG | 19 | 6046 |
| myoC-2120 | + | CCCAGGACCCCGGGUGCUUG | 20 | 2238 |
| myoC-6301 | + | ACCCAGGACCCCGGGUGCUUG | 21 | 6047 |
| myoC-6302 | + | CACCCAGGACCCCGGGUGCUUG | 22 | 6048 |
| myoC-6303 | + | ACACCCAGGACCCCGGGUGCUUG | 23 | 6049 |
| myoC-6304 | + | GACACCCAGGACCCCGGGUGCUUG | 24 | 6050 |
| myoC-6305 | + | GUGAACAACACUGAACAU | 18 | 6051 |
| myoC-6306 | + | CGUGAACAACACUGAACAU | 19 | 6052 |
| myoC-2181 | + | CCGUGAACAACACUGAACAU | 20 | 2281 |
| myoC-6307 | + | CCCGUGAACAACACUGAACAU | 21 | 6053 |
| myoC-6308 | + | CCCCGUGAACAACACUGAACAU | 22 | 6054 |
| myoC-6309 | + | GCCCCGUGAACAACACUGAACAU | 23 | 6055 |
| myoC-6310 | + | AGCCCCGUGAACAACACUGAACAU | 24 | 6056 |
| myoC-6311 | + | CUUCUGCACGUCUUCCAU | 18 | 6057 |
| myoC-6312 | + | UCUUCUGCACGUCUUCCAU | 19 | 6058 |
| myoC-2136 | + | UUCUUCUGCACGUCUUCCAU | 20 | 2254 |
| myoC-6313 | + | UUUCUUCUGCACGUCUUCCAU | 21 | 6059 |
| myoC-6314 | + | UUUUCUUCUGCACGUCUUCCAU | 22 | 6060 |
| myoC-6315 | + | AUUUUCUUCUGCACGUCUUCCAU | 23 | 6061 |
| myoC-6316 | + | AAUUUUCUUCUGCACGUCUUCCAU | 24 | 6062 |
| myoC-3417 | + | CUGGGCAGCUGGAUUCAU | 18 | 3163 |
| myoC-3418 | + | UCUGGGCAGCUGGAUUCAU | 19 | 3164 |
| myoC-231 | + | CUCUGGGCAGCUGGAUUCAU | 20 | 617 |
| myoC-3419 | + | GCUCUGGGCAGCUGGAUUCAU | 21 | 3165 |
| myoC-3420 | + | UGCUCUGGGCAGCUGGAUUCAU | 22 | 3166 |
| myoC-3421 | + | CUGCUCUGGGCAGCUGGAUUCAU | 23 | 3167 |
| myoC-3422 | + | UCUGCUCUGGGCAGCUGGAUUCAU | 24 | 3168 |
| myoC-6317 | + | GGGGAGCCAGCCCUUCAU | 18 | 6063 |
| myoC-6318 | + | UGGGGAGCCAGCCCUUCAU | 19 | 6064 |
| myoC-869 | + | CUGGGGAGCCAGCCCUUCAU | 20 | 1204 |
| myoC-6319 | + | ACUGGGGAGCCAGCCCUUCAU | 21 | 6065 |
| myoC-6320 | + | UACUGGGGAGCCAGCCCUUCAU | 22 | 6066 |
| myoC-6321 | + | AUACUGGGGAGCCAGCCCUUCAU | 23 | 6067 |
| myoC-6322 | + | UAUACUGGGGAGCCAGCCCUUCAU | 24 | 6068 |
| myoC-6323 | + | GAGAGGUUUAUAUAUACU | 18 | 6069 |
| myoC-6324 | + | AGAGAGGUUUAUAUAUACU | 19 | 6070 |
| myoC-866 | + | CAGAGAGGUUUAUAUAUACU | 20 | 1191 |
| myoC-6325 | + | CCAGAGAGGUUUAUAUAUACU | 21 | 6071 |
| myoC-6326 | + | UCCAGAGAGGUUUAUAUAUACU | 22 | 6072 |
| myoC-6327 | + | CUCCAGAGAGGUUUAUAUAUACU | 23 | 6073 |
| myoC-6328 | + | GCUCCAGAGAGGUUUAUAUAUACU | 24 | 6074 |
| myoC-6329 | + | GUGGAGGCCCCUUUCCCU | 18 | 6075 |
| myoC-6330 | + | CGUGGAGGCCCCUUUCCCU | 19 | 6076 |
| myoC-2175 | + | ACGUGGAGGCCCCUUUCCCU | 20 | 2277 |
| myoC-6331 | + | GACGUGGAGGCCCCUUUCCCU | 21 | 6077 |
| myoC-6332 | + | GGACGUGGAGGCCCCUUUCCCU | 22 | 6078 |
| myoC-6333 | + | UGGACGUGGAGGCCCCUUUCCCU | 23 | 6079 |
| myoC-6334 | + | CUGGACGUGGAGGCCCCUUUCCCU | 24 | 6080 |
| myoC-6335 | + | UCCGUGAAUUAACGGCCU | 18 | 6081 |
| myoC-6336 | + | UUCCGUGAAUUAACGGCCU | 19 | 6082 |
| myoC-1099 | + | CUUCCGUGAAUUAACGGCCU | 20 | 1399 |
| myoC-6337 | + | UCUUCCGUGAAUUAACGGCCU | 21 | 6083 |
| myoC-6338 | + | UUCUUCCGUGAAUUAACGGCCU | 22 | 6084 |
| myoC-6339 | + | CUUCUUCCGUGAAUUAACGGCCU | 23 | 6085 |
| myoC-6340 | + | ACUUCUUCCGUGAAUUAACGGCCU | 24 | 6086 |
| myoC-6341 | + | ACUCGGGCUUGGGGGCCU | 18 | 6087 |
| myoC-6342 | + | GACUCGGGCUUGGGGGCCU | 19 | 6088 |
| myoC-2149 | + | AGACUCGGGCUUGGGGGCCU | 20 | 2261 |
| myoC-6343 | + | AAGACUCGGGCUUGGGGGCCU | 21 | 6089 |
| myoC-6344 | + | GAAGACUCGGGCUUGGGGGCCU | 22 | 6090 |
| myoC-6345 | + | GGAAGACUCGGGCUUGGGGGCCU | 23 | 6091 |
| myoC-6346 | + | UGGAAGACUCGGGCUUGGGGGCCU | 24 | 6092 |
| myoC-3423 | + | GGCUUGGUGAGGCUUCCU | 18 | 3169 |
| myoC-3424 | + | AGGCUUGGUGAGGCUUCCU | 19 | 3170 |
| myoC-2357 | + | GAGGCUUGGUGAGGCUUCCU | 20 | 2411 |
| myoC-3425 | + | AGAGGCUUGGUGAGGCUUCCU | 21 | 3171 |
| myoC-3426 | + | CAGAGGCUUGGUGAGGCUUCCU | 22 | 3172 |
| myoC-3427 | + | GCAGAGGCUUGGUGAGGCUUCCU | 23 | 3173 |
| myoC-3428 | + | UGCAGAGGCUUGGUGAGGCUUCCU | 24 | 3174 |
| myoC-6347 | + | GAGGCAGCAGGGGGCGCU | 18 | 6093 |
| myoC-6348 | + | GGAGGCAGCAGGGGGCGCU | 19 | 6094 |
| myoC-717 | + | UGGAGGCAGCAGGGGGCGCU | 20 | 1120 |
| myoC-6349 | + | AUGGAGGCAGCAGGGGGCGCU | 21 | 6095 |
| myoC-6350 | + | GAUGGAGGCAGCAGGGGGCGCU | 22 | 6096 |
| myoC-6351 | + | CGAUGGAGGCAGCAGGGGGCGCU | 23 | 6097 |
| myoC-6352 | + | ACGAUGGAGGCAGCAGGGGGCGCU | 24 | 6098 |
| myoC-6353 | + | CUGAUGGAGGAGGAGGCU | 18 | 6099 |
| myoC-6354 | + | ACUGAUGGAGGAGGAGGCU | 19 | 6100 |
| myoC-702 | + | GACUGAUGGAGGAGGAGGCU | 20 | 1004 |
| myoC-6355 | + | UGACUGAUGGAGGAGGAGGCU | 21 | 6101 |
| myoC-6356 | + | GUGACUGAUGGAGGAGGAGGCU | 22 | 6102 |
| myoC-6357 | + | UGUGACUGAUGGAGGAGGAGGCU | 23 | 6103 |
| myoC-6358 | + | CUGUGACUGAUGGAGGAGGAGGCU | 24 | 6104 |
| myoC-6359 | + | GCUUGGAAGACUCGGGCU | 18 | 6105 |
| myoC-6360 | + | GGCUUGGAAGACUCGGGCU | 19 | 6106 |
| myoC-705 | + | AGGCUUGGAAGACUCGGGCU | 20 | 1091 |
| myoC-6361 | + | GAGGCUUGGAAGACUCGGGCU | 21 | 6107 |
| myoC-6362 | + | GGAGGCUUGGAAGACUCGGGCU | 22 | 6108 |
| myoC-6363 | + | AGGAGGCUUGGAAGACUCGGGCU | 23 | 6109 |
| myoC-6364 | + | GAGGAGGCUUGGAAGACUCGGGCU | 24 | 6110 |
| myoC-6365 | + | UGUGCCAGGCACUAUGCU | 18 | 6111 |
| myoC-6366 | + | CUGUGCCAGGCACUAUGCU | 19 | 6112 |
| myoC-891 | + | ACUGUGCCAGGCACUAUGCU | 20 | 1178 |
| myoC-6367 | + | CACUGUGCCAGGCACUAUGCU | 21 | 6113 |
| myoC-6368 | + | GCACUGUGCCAGGCACUAUGCU | 22 | 6114 |
| myoC-6369 | + | UGCACUGUGCCAGGCACUAUGCU | 23 | 6115 |
| myoC-6370 | + | CUGCACUGUGCCAGGCACUAUGCU | 24 | 6116 |
| myoC-3429 | + | ACAUGGCCUGGCUCUGCU | 18 | 3175 |
| myoC-3430 | + | GACAUGGCCUGGCUCUGCU | 19 | 3176 |
| myoC-1675 | + | UGACAUGGCCUGGCUCUGCU | 20 | 1936 |
| myoC-3431 | + | CUGACAUGGCCUGGCUCUGCU | 21 | 3177 |
| myoC-3432 | + | ACUGACAUGGCCUGGCUCUGCU | 22 | 3178 |
| myoC-3433 | + | GACUGACAUGGCCUGGCUCUGCU | 23 | 3179 |
| myoC-3434 | + | UGACUGACAUGGCCUGGCUCUGCU | 24 | 3180 |
| myoC-6371 | + | GGAAAGCUCUGCUGUGCU | 18 | 6117 |
| myoC-6372 | + | UGGAAAGCUCUGCUGUGCU | 19 | 6118 |
| myoC-2354 | + | CUGGAAAGCUCUGCUGUGCU | 20 | 2408 |
| myoC-6373 | + | UCUGGAAAGCUCUGCUGUGCU | 21 | 6119 |
| myoC-6374 | + | CUCUGGAAAGCUCUGCUGUGCU | 22 | 6120 |
| myoC-6375 | + | CCUCUGGAAAGCUCUGCUGUGCU | 23 | 6121 |
| myoC-6376 | + | UCCUCUGGAAAGCUCUGCUGUGCU | 24 | 6122 |
| myoC-6377 | + | ACGGGCUGGCAGGUUGCU | 18 | 6123 |
| myoC-6378 | + | CACGGGCUGGCAGGUUGCU | 19 | 6124 |
| myoC-2123 | + | GCACGGGCUGGCAGGUUGCU | 20 | 2241 |
| myoC-6379 | + | GGCACGGGCUGGCAGGUUGCU | 21 | 6125 |
| myoC-6380 | + | UGGCACGGGCUGGCAGGUUGCU | 22 | 6126 |
| myoC-6381 | + | GUGGCACGGGCUGGCAGGUUGCU | 23 | 6127 |
| myoC-6382 | + | AGUGGCACGGGCUGGCAGGUUGCU | 24 | 6128 |
| myoC-6383 | + | GGAGGCCCCUUUCCCUCU | 18 | 6129 |
| myoC-6384 | + | UGGAGGCCCCUUUCCCUCU | 19 | 6130 |
| myoC-2174 | + | GUGGAGGCCCCUUUCCCUCU | 20 | 2276 |
| myoC-6385 | + | CGUGGAGGCCCCUUUCCCUCU | 21 | 6131 |
| myoC-6386 | + | ACGUGGAGGCCCCUUUCCCUCU | 22 | 6132 |
| myoC-6387 | + | GACGUGGAGGCCCCUUUCCCUCU | 23 | 6133 |
| myoC-6388 | + | GGACGUGGAGGCCCCUUUCCCUCU | 24 | 6134 |
| myoC-3435 | + | UCCAGAACUGACUUGUCU | 18 | 3181 |
| myoC-3436 | + | CUCCAGAACUGACUUGUCU | 19 | 3182 |
| myoC-208 | + | CCUCCAGAACUGACUUGUCU | 20 | 594 |
| myoC-3437 | + | UCCUCCAGAACUGACUUGUCU | 21 | 3183 |
| myoC-3438 | + | UUCCUCCAGAACUGACUUGUCU | 22 | 3184 |
| myoC-3439 | + | CUUCCUCCAGAACUGACUUGUCU | 23 | 3185 |
| myoC-3440 | + | UCUUCCUCCAGAACUGACUUGUCU | 24 | 3186 |
| myoC-6389 | + | CGCUGCCAGCAAGAUUCU | 18 | 6135 |
| myoC-6390 | + | ACGCUGCCAGCAAGAUUCU | 19 | 6136 |
| myoC-2330 | + | CACGCUGCCAGCAAGAUUCU | 20 | 2395 |
| myoC-6391 | + | UCACGCUGCCAGCAAGAUUCU | 21 | 6137 |
| myoC-6392 | + | UUCACGCUGCCAGCAAGAUUCU | 22 | 6138 |
| myoC-6393 | + | CUUCACGCUGCCAGCAAGAUUCU | 23 | 6139 |
| myoC-6394 | + | CCUUCACGCUGCCAGCAAGAUUCU | 24 | 6140 |
| myoC-6395 | + | AACCUUCCAGAAGUCUGU | 18 | 6141 |
| myoC-6396 | + | UAACCUUCCAGAAGUCUGU | 19 | 6142 |
| myoC-2328 | + | AUAACCUUCCAGAAGUCUGU | 20 | 2393 |
| myoC-6397 | + | AAUAACCUUCCAGAAGUCUGU | 21 | 6143 |
| myoC-6398 | + | AAAUAACCUUCCAGAAGUCUGU | 22 | 6144 |
| myoC-6399 | + | AAAAUAACCUUCCAGAAGUCUGU | 23 | 6145 |
| myoC-6400 | + | GAAAAUAACCUUCCAGAAGUCUGU | 24 | 6146 |
| myoC-6401 | + | UCACUCUGCAAACUCAUU | 18 | 6147 |
| myoC-6402 | + | UUCACUCUGCAAACUCAUU | 19 | 6148 |
| myoC-2322 | + | AUUCACUCUGCAAACUCAUU | 20 | 2388 |
| myoC-6403 | + | CAUUCACUCUGCAAACUCAUU | 21 | 6149 |
| myoC-6404 | + | CCAUUCACUCUGCAAACUCAUU | 22 | 6150 |
| myoC-6405 | + | UCCAUUCACUCUGCAAACUCAUU | 23 | 6151 |
| myoC-6406 | + | UUCCAUUCACUCUGCAAACUCAUU | 24 | 6152 |
| myoC-6407 | + | CUUGGAAGACUCGGGCUU | 18 | 6153 |
| myoC-6408 | + | GCUUGGAAGACUCGGGCUU | 19 | 6154 |
| myoC-706 | + | GGCUUGGAAGACUCGGGCUU | 20 | 978 |
| myoC-6409 | + | AGGCUUGGAAGACUCGGGCUU | 21 | 6155 |
| myoC-6410 | + | GAGGCUUGGAAGACUCGGGCUU | 22 | 6156 |
| myoC-6411 | + | GGAGGCUUGGAAGACUCGGGCUU | 23 | 6157 |
| myoC-6412 | + | AGGAGGCUUGGAAGACUCGGGCUU | 24 | 6158 |
| myoC-6413 | + | UAGGGAGGUGGCCUUGUU | 18 | 6159 |
| myoC-6414 | + | CUAGGGAGGUGGCCUUGUU | 19 | 6160 |
| myoC-2140 | + | GCUAGGGAGGUGGCCUUGUU | 20 | 2257 |
| myoC-6415 | + | CGCUAGGGAGGUGGCCUUGUU | 21 | 6161 |
| myoC-6416 | + | GCGCUAGGGAGGUGGCCUUGUU | 22 | 6162 |
| myoC-6417 | + | GGCGCUAGGGAGGUGGCCUUGUU | 23 | 6163 |
| myoC-6418 | + | GGGCGCUAGGGAGGUGGCCUUGUU | 24 | 6164 |
| myoC-6419 | + | AUUUUAACAGCUGACUUU | 18 | 6165 |
| myoC-6420 | + | AAUUUUAACAGCUGACUUU | 19 | 6166 |
| myoC-2191 | + | GAAUUUUAACAGCUGACUUU | 20 | 2289 |
| myoC-6421 | + | GGAAUUUUAACAGCUGACUUU | 21 | 6167 |
| myoC-6422 | + | UGGAAUUUUAACAGCUGACUUU | 22 | 6168 |
| myoC-6423 | + | CUGGAAUUUUAACAGCUGACUUU | 23 | 6169 |
| myoC-6424 | + | CCUGGAAUUUUAACAGCUGACUUU | 24 | 6170 |
| myoC-6425 | + | UCCCUCUCCAUUUCCUUU | 18 | 6171 |
| myoC-6426 | + | UUCCCUCUCCAUUUCCUUU | 19 | 6172 |
| myoC-2172 | + | UUUCCCUCUCCAUUUCCUUU | 20 | 2275 |
| myoC-6427 | + | GUUUCCCUCUCCAUUUCCUUU | 21 | 6173 |
| myoC-6428 | + | AGUUUCCCUCUCCAUUUCCUUU | 22 | 6174 |
| myoC-6429 | + | UAGUUUCCCUCUCCAUUUCCUUU | 23 | 6175 |
| myoC-6430 | + | CUAGUUUCCCUCUCCAUUUCCUUU | 24 | 6176 |
| myoC-3441 | - | AGCGACUAAGGCAAGAAA | 18 | 3187 |
| myoC-3442 | - | AAGCGACUAAGGCAAGAAA | 19 | 3188 |
| myoC-1647 | - | GAAGCGACUAAGGCAAGAAA | 20 | 1913 |
| myoC-3443 | - | AGAAGCGACUAAGGCAAGAAA | 21 | 3189 |
| myoC-3444 | - | AAGAAGCGACUAAGGCAAGAAA | 22 | 3190 |
| myoC-3445 | - | GAAGAAGCGACUAAGGCAAGAAA | 23 | 3191 |
| myoC-3446 | - | AGAAGAAGCGACUAAGGCAAGAAA | 24 | 3192 |
| myoC-6431 | - | CAGGCUCCAGAAAGGAAA | 18 | 6177 |
| myoC-6432 | - | CCAGGCUCCAGAAAGGAAA | 19 | 6178 |
| myoC-964 | - | UCCAGGCUCCAGAAAGGAAA | 20 | 1264 |
| myoC-6433 | - | CUCCAGGCUCCAGAAAGGAAA | 21 | 6179 |
| myoC-6434 | - | GCUCCAGGCUCCAGAAAGGAAA | 22 | 6180 |
| myoC-6435 | - | GGCUCCAGGCUCCAGAAAGGAAA | 23 | 6181 |
| myoC-6436 | - | UGGCUCCAGGCUCCAGAAAGGAAA | 24 | 6182 |
| myoC-6437 | - | GGGGUAUGGGUGCAUAAA | 18 | 6183 |
| myoC-6438 | - | UGGGGUAUGGGUGCAUAAA | 19 | 6184 |
| myoC-2095 | - | UUGGGGUAUGGGUGCAUAAA | 20 | 2220 |
| myoC-6439 | - | AUUGGGGUAUGGGUGCAUAAA | 21 | 6185 |
| myoC-6440 | - | UAUUGGGGUAUGGGUGCAUAAA | 22 | 6186 |
| myoC-6441 | - | UUAUUGGGGUAUGGGUGCAUAAA | 23 | 6187 |
| myoC-6442 | - | AUUAUUGGGGUAUGGGUGCAUAAA | 24 | 6188 |
| myoC-6443 | - | UGGGAUGUUCUUUUUAAA | 18 | 6189 |
| myoC-6444 | - | UUGGGAUGUUCUUUUUAAA | 19 | 6190 |
| myoC-2097 | - | AUUGGGAUGUUCUUUUUAAA | 20 | 2221 |
| myoC-6445 | - | AAUUGGGAUGUUCUUUUUAAA | 21 | 6191 |
| myoC-6446 | - | AAAUUGGGAUGUUCUUUUUAAA | 22 | 6192 |
| myoC-6447 | - | UAAAUUGGGAUGUUCUUUUUAAA | 23 | 6193 |
| myoC-6448 | - | AUAAAUUGGGAUGUUCUUUUUAAA | 24 | 6194 |
| myoC-6449 | - | AACCCAGUGCUGAAAGAA | 18 | 6195 |
| myoC-6450 | - | AAACCCAGUGCUGAAAGAA | 19 | 6196 |
| myoC-693 | - | UAAACCCAGUGCUGAAAGAA | 20 | 1113 |
| myoC-6451 | - | UUAAACCCAGUGCUGAAAGAA | 21 | 6197 |
| myoC-6452 | - | CUUAAACCCAGUGCUGAAAGAA | 22 | 6198 |
| myoC-6453 | - | ACUUAAACCCAGUGCUGAAAGAA | 23 | 6199 |
| myoC-6454 | - | AACUUAAACCCAGUGCUGAAAGAA | 24 | 6200 |
| myoC-6455 | - | UGGCUCCAGGCUCCAGAA | 18 | 6201 |
| myoC-6456 | - | UUGGCUCCAGGCUCCAGAA | 19 | 6202 |
| myoC-963 | - | CUUGGCUCCAGGCUCCAGAA | 20 | 1263 |
| myoC-6457 | - | CCUUGGCUCCAGGCUCCAGAA | 21 | 6203 |
| myoC-6458 | - | UCCUUGGCUCCAGGCUCCAGAA | 22 | 6204 |
| myoC-6459 | - | CUCCUUGGCUCCAGGCUCCAGAA | 23 | 6205 |
| myoC-6460 | - | ACUCCUUGGCUCCAGGCUCCAGAA | 24 | 6206 |
| myoC-6461 | - | CCAGGCUCCAGAAAGGAA | 18 | 6207 |
| myoC-6462 | - | UCCAGGCUCCAGAAAGGAA | 19 | 6208 |
| myoC-1848 | - | CUCCAGGCUCCAGAAAGGAA | 20 | 2057 |
| myoC-6463 | - | GCUCCAGGCUCCAGAAAGGAA | 21 | 6209 |
| myoC-6464 | - | GGCUCCAGGCUCCAGAAAGGAA | 22 | 6210 |
| myoC-6465 | - | UGGCUCCAGGCUCCAGAAAGGAA | 23 | 6211 |
| myoC-6466 | - | UUGGCUCCAGGCUCCAGAAAGGAA | 24 | 6212 |
| myoC-3447 | - | AAGUCAGUUCUGGAGGAA | 18 | 3193 |
| myoC-3448 | - | CAAGUCAGUUCUGGAGGAA | 19 | 3194 |
| myoC-1644 | - | ACAAGUCAGUUCUGGAGGAA | 20 | 1910 |
| myoC-3449 | - | GACAAGUCAGUUCUGGAGGAA | 21 | 3195 |
| myoC-3450 | - | AGACAAGUCAGUUCUGGAGGAA | 22 | 3196 |
| myoC-3451 | - | GAGACAAGUCAGUUCUGGAGGAA | 23 | 3197 |
| myoC-3452 | - | CGAGACAAGUCAGUUCUGGAGGAA | 24 | 3198 |
| myoC-6467 | - | UUAAUGGGAAUAUAGGAA | 18 | 6213 |
| myoC-6468 | - | UUUAAUGGGAAUAUAGGAA | 19 | 6214 |
| myoC-1915 | - | AUUUAAUGGGAAUAUAGGAA | 20 | 2095 |
| myoC-6469 | - | UAUUUAAUGGGAAUAUAGGAA | 21 | 6215 |
| myoC-6470 | - | UUAUUUAAUGGGAAUAUAGGAA | 22 | 6216 |
| myoC-6471 | - | UUUAUUUAAUGGGAAUAUAGGAA | 23 | 6217 |
| myoC-6472 | - | CUUUAUUUAAUGGGAAUAUAGGAA | 24 | 6218 |
| myoC-6473 | - | GUGUUUCCUCAGAGGGAA | 18 | 6219 |
| myoC-6474 | - | AGUGUUUCCUCAGAGGGAA | 19 | 6220 |
| myoC-974 | - | CAGUGUUUCCUCAGAGGGAA | 20 | 1274 |
| myoC-6475 | - | ACAGUGUUUCCUCAGAGGGAA | 21 | 6221 |
| myoC-6476 | - | GACAGUGUUUCCUCAGAGGGAA | 22 | 6222 |
| myoC-6477 | - | GGACAGUGUUUCCUCAGAGGGAA | 23 | 6223 |
| myoC-6478 | - | GGGACAGUGUUUCCUCAGAGGGAA | 24 | 6224 |
| myoC-6479 | - | AUGAGUUUGCAGAGUGAA | 18 | 6225 |
| myoC-6480 | - | AAUGAGUUUGCAGAGUGAA | 19 | 6226 |
| myoC-833 | - | CAAUGAGUUUGCAGAGUGAA | 20 | 1188 |
| myoC-6481 | - | UCAAUGAGUUUGCAGAGUGAA | 21 | 6227 |
| myoC-6482 | - | CUCAAUGAGUUUGCAGAGUGAA | 22 | 6228 |
| myoC-6483 | - | UCUCAAUGAGUUUGCAGAGUGAA | 23 | 6229 |
| myoC-6484 | - | UUCUCAAUGAGUUUGCAGAGUGAA | 24 | 6230 |
| myoC-6485 | - | GAAAGGCAGGAAGGUGAA | 18 | 6231 |
| myoC-6486 | - | UGAAAGGCAGGAAGGUGAA | 19 | 6232 |
| myoC-1890 | - | CUGAAAGGCAGGAAGGUGAA | 20 | 2079 |
| myoC-6487 | - | GCUGAAAGGCAGGAAGGUGAA | 21 | 6233 |
| myoC-6488 | - | UGCUGAAAGGCAGGAAGGUGAA | 22 | 6234 |
| myoC-6489 | - | GUGCUGAAAGGCAGGAAGGUGAA | 23 | 6235 |
| myoC-6490 | - | GGUGCUGAAAGGCAGGAAGGUGAA | 24 | 6236 |
| myoC-6491 | - | AGAGGGAAACUAGUCUAA | 18 | 6237 |
| myoC-6492 | - | GAGAGGGAAACUAGUCUAA | 19 | 6238 |
| myoC-967 | - | GGAGAGGGAAACUAGUCUAA | 20 | 1267 |
| myoC-6493 | - | UGGAGAGGGAAACUAGUCUAA | 21 | 6239 |
| myoC-6494 | - | AUGGAGAGGGAAACUAGUCUAA | 22 | 6240 |
| myoC-6495 | - | AAUGGAGAGGGAAACUAGUCUAA | 23 | 6241 |
| myoC-6496 | - | AAAUGGAGAGGGAAACUAGUCUAA | 24 | 6242 |
| myoC-6497 | - | ACGAAGGCCUUUAUUUAA | 18 | 6243 |
| myoC-6498 | - | CACGAAGGCCUUUAUUUAA | 19 | 6244 |
| myoC-1013 | - | UCACGAAGGCCUUUAUUUAA | 20 | 1313 |
| myoC-6499 | - | UUCACGAAGGCCUUUAUUUAA | 21 | 6245 |
| myoC-6500 | - | CUUCACGAAGGCCUUUAUUUAA | 22 | 6246 |
| myoC-6501 | - | CCUUCACGAAGGCCUUUAUUUAA | 23 | 6247 |
| myoC-6502 | - | UCCUUCACGAAGGCCUUUAUUUAA | 24 | 6248 |
| myoC-3453 | - | AGUCAUCCAUAACUUACA | 18 | 3199 |
| myoC-3454 | - | CAGUCAUCCAUAACUUACA | 19 | 3200 |
| myoC-1608 | - | UCAGUCAUCCAUAACUUACA | 20 | 1888 |
| myoC-3455 | - | GUCAGUCAUCCAUAACUUACA | 21 | 3201 |
| myoC-3456 | - | UGUCAGUCAUCCAUAACUUACA | 22 | 3202 |
| myoC-3457 | - | AUGUCAGUCAUCCAUAACUUACA | 23 | 3203 |
| myoC-3458 | - | CAUGUCAGUCAUCCAUAACUUACA | 24 | 3204 |
| myoC-6503 | - | GCACAGCAGAGCUUUCCA | 18 | 6249 |
| myoC-6504 | - | AGCACAGCAGAGCUUUCCA | 19 | 6250 |
| myoC-2110 | - | CAGCACAGCAGAGCUUUCCA | 20 | 2232 |
| myoC-6505 | - | UCAGCACAGCAGAGCUUUCCA | 21 | 6251 |
| myoC-6506 | - | CUCAGCACAGCAGAGCUUUCCA | 22 | 6252 |
| myoC-6507 | - | UCUCAGCACAGCAGAGCUUUCCA | 23 | 6253 |
| myoC-6508 | - | CUCUCAGCACAGCAGAGCUUUCCA | 24 | 6254 |
| myoC-3459 | - | GACCCAGGAGGGGCUGCA | 18 | 3205 |
| myoC-3460 | - | AGACCCAGGAGGGGCUGCA | 19 | 3206 |
| myoC-1622 | - | GAGACCCAGGAGGGGCUGCA | 20 | 1897 |
| myoC-3461 | - | GGAGACCCAGGAGGGGCUGCA | 21 | 3207 |
| myoC-3462 | - | AGGAGACCCAGGAGGGGCUGCA | 22 | 3208 |
| myoC-3463 | - | CAGGAGACCCAGGAGGGGCUGCA | 23 | 3209 |
| myoC-3464 | - | CCAGGAGACCCAGGAGGGGCUGCA | 24 | 3210 |
| myoC-3465 | - | CCUCACCAAGCCUCUGCA | 18 | 3211 |
| myoC-3466 | - | GCCUCACCAAGCCUCUGCA | 19 | 3212 |
| myoC-1592 | - | AGCCUCACCAAGCCUCUGCA | 20 | 1876 |
| myoC-3467 | - | AAGCCUCACCAAGCCUCUGCA | 21 | 3213 |
| myoC-3468 | - | GAAGCCUCACCAAGCCUCUGCA | 22 | 3214 |
| myoC-3469 | - | GGAAGCCUCACCAAGCCUCUGCA | 23 | 3215 |
| myoC-3470 | - | AGGAAGCCUCACCAAGCCUCUGCA | 24 | 3216 |
| myoC-6509 | - | GGGGACAGUGUUUCCUCA | 18 | 6255 |
| myoC-6510 | - | AGGGGACAGUGUUUCCUCA | 19 | 6256 |
| myoC-1863 | - | GAGGGGACAGUGUUUCCUCA | 20 | 2065 |
| myoC-6511 | - | GGAGGGGACAGUGUUUCCUCA | 21 | 6257 |
| myoC-6512 | - | UGGAGGGGACAGUGUUUCCUCA | 22 | 6258 |
| myoC-6513 | - | CUGGAGGGGACAGUGUUUCCUCA | 23 | 6259 |
| myoC-6514 | - | UCUGGAGGGGACAGUGUUUCCUCA | 24 | 6260 |
| myoC-6515 | - | GGAGGUGACAGUUUCUCA | 18 | 6261 |
| myoC-6516 | - | UGGAGGUGACAGUUUCUCA | 19 | 6262 |
| myoC-692 | - | GUGGAGGUGACAGUUUCUCA | 20 | 1021 |
| myoC-6517 | - | CGUGGAGGUGACAGUUUCUCA | 21 | 6263 |
| myoC-6518 | - | UCGUGGAGGUGACAGUUUCUCA | 22 | 6264 |
| myoC-6519 | - | UUCGUGGAGGUGACAGUUUCUCA | 23 | 6265 |
| myoC-6520 | - | CUUCGUGGAGGUGACAGUUUCUCA | 24 | 6266 |
| myoC-6521 | - | UCCUAGGCCGUUAAUUCA | 18 | 6267 |
| myoC-6522 | - | UUCCUAGGCCGUUAAUUCA | 19 | 6268 |
| myoC-1017 | - | UUUCCUAGGCCGUUAAUUCA | 20 | 1317 |
| myoC-6523 | - | AUUUCCUAGGCCGUUAAUUCA | 21 | 6269 |
| myoC-6524 | - | CAUUUCCUAGGCCGUUAAUUCA | 22 | 6270 |
| myoC-6525 | - | UCAUUUCCUAGGCCGUUAAUUCA | 23 | 6271 |
| myoC-6526 | - | CUCAUUUCCUAGGCCGUUAAUUCA | 24 | 6272 |
| myoC-6527 | - | GAUGUUCAGUGUUGUUCA | 18 | 6273 |
| myoC-6528 | - | AGAUGUUCAGUGUUGUUCA | 19 | 6274 |
| myoC-999 | - | CAGAUGUUCAGUGUUGUUCA | 20 | 1299 |
| myoC-6529 | - | CCAGAUGUUCAGUGUUGUUCA | 21 | 6275 |
| myoC-6530 | - | CCCAGAUGUUCAGUGUUGUUCA | 22 | 6276 |
| myoC-6531 | - | GCCCAGAUGUUCAGUGUUGUUCA | 23 | 6277 |
| myoC-6532 | - | UGCCCAGAUGUUCAGUGUUGUUCA | 24 | 6278 |
| myoC-6533 | - | AAACCCAGUGCUGAAAGA | 18 | 6279 |
| myoC-6534 | - | UAAACCCAGUGCUGAAAGA | 19 | 6280 |
| myoC-1834 | - | UUAAACCCAGUGCUGAAAGA | 20 | 2046 |
| myoC-6535 | - | CUUAAACCCAGUGCUGAAAGA | 21 | 6281 |
| myoC-6536 | - | ACUUAAACCCAGUGCUGAAAGA | 22 | 6282 |
| myoC-6537 | - | AACUUAAACCCAGUGCUGAAAGA | 23 | 6283 |
| myoC-6538 | - | CAACU UAAACCCAGUGCUGAAAGA | 24 | 6284 |
| myoC-6539 | - | UUGGCUCCAGGCUCCAGA | 18 | 6285 |
| myoC-6540 | - | CUUGGCUCCAGGCUCCAGA | 19 | 6286 |
| myoC-1846 | - | CCUUGGCUCCAGGCUCCAGA | 20 | 2056 |
| myoC-6541 | - | UCCUUGGCUCCAGGCUCCAGA | 21 | 6287 |
| myoC-6542 | - | CUCCUUGGCUCCAGGCUCCAGA | 22 | 6288 |
| myoC-6543 | - | ACUCCUUGGCUCCAGGCUCCAGA | 23 | 6289 |
| myoC-6544 | - | GACUCCUUGGCUCCAGGCUCCAGA | 24 | 6290 |
| myoC-3471 | - | CCCAGGAGGGGCUGCAGA | 18 | 3217 |
| myoC-3472 | - | ACCCAGGAGGGGCUGCAGA | 19 | 3218 |
| myoC-99 | - | GACCCAGGAGGGGCUGCAGA | 20 | 504 |
| myoC-3473 | - | AGACCCAGGAGGGGCUGCAGA | 21 | 3219 |
| myoC-3474 | - | GAGACCCAGGAGGGGCUGCAGA | 22 | 3220 |
| myoC-3475 | - | GGAGACCCAGGAGGGGCUGCAGA | 23 | 3221 |
| myoC-3476 | - | AGGAGACCCAGGAGGGGCUGCAGA | 24 | 3222 |
| myoC-6545 | - | GGACAGUGUUUCCUCAGA | 18 | 6291 |
| myoC-6546 | - | GGGACAGUGUUUCCUCAGA | 19 | 6292 |
| myoC-973 | - | GGGGACAGUGUUUCCUCAGA | 20 | 1273 |
| myoC-6547 | - | AGGGGACAGUGUUUCCUCAGA | 21 | 6293 |
| myoC-6548 | - | GAGGGGACAGUGUUUCCUCAGA | 22 | 6294 |
| myoC-6549 | - | GGAGGGGACAGUGUUUCCUCAGA | 23 | 6295 |
| myoC-6550 | - | UGGAGGGGACAGUGUUUCCUCAGA | 24 | 6296 |
| myoC-6551 | - | CCAGAAAGGAAAUGGAGA | 18 | 6297 |
| myoC-6552 | - | UCCAGAAAGGAAAUGGAGA | 19 | 6298 |
| myoC-966 | - | CUCCAGAAAGGAAAUGGAGA | 20 | 1266 |
| myoC-6553 | - | GCUCCAGAAAGGAAAUGGAGA | 21 | 6299 |
| myoC-6554 | - | GGCUCCAGAAAGGAAAUGGAGA | 22 | 6300 |
| myoC-6555 | - | AGGCUCCAGAAAGGAAAUGGAGA | 23 | 6301 |
| myoC-6556 | - | CAGGCUCCAGAAAGGAAAUGGAGA | 24 | 6302 |
| myoC-6557 | - | AGGUGGGGACUGCAGGGA | 18 | 6303 |
| myoC-6558 | - | GAGGUGGGGACUGCAGGGA | 19 | 6304 |
| myoC-1879 | - | GGAGGUGGGGACUGCAGGGA | 20 | 2072 |
| myoC-6559 | - | AGGAGGUGGGGACUGCAGGGA | 21 | 6305 |
| myoC-6560 | - | CAGGAGGUGGGGACUGCAGGGA | 22 | 6306 |
| myoC-6561 | - | CCAGGAGGUGGGGACUGCAGGGA | 23 | 6307 |
| myoC-6562 | - | UCCAGGAGGUGGGGACUGCAGGGA | 24 | 6308 |
| myoC-6563 | - | AGUGUUUCCUCAGAGGGA | 18 | 6309 |
| myoC-6564 | - | CAGUGUUUCCUCAGAGGGA | 19 | 6310 |
| myoC-1866 | - | ACAGUGUUUCCUCAGAGGGA | 20 | 2066 |
| myoC-6565 | - | GACAGUGUUUCCUCAGAGGGA | 21 | 6311 |
| myoC-6566 | - | GGACAGUGUUUCCUCAGAGGGA | 22 | 6312 |
| myoC-6567 | - | GGGACAGUGUUUCCUCAGAGGGA | 23 | 6313 |
| myoC-6568 | - | GGGGACAGUGUUUCCUCAGAGGGA | 24 | 6314 |
| myoC-3477 | - | GGGCACCCUGAGGCGGGA | 18 | 3223 |
| myoC-3478 | - | UGGGCACCCUGAGGCGGGA | 19 | 3224 |
| myoC-1630 | - | CUGGGCACCCUGAGGCGGGA | 20 | 1901 |
| myoC-3479 | - | GCUGGGCACCCUGAGGCGGGA | 21 | 3225 |
| myoC-3480 | - | AGCUGGGCACCCUGAGGCGGGA | 22 | 3226 |
| myoC-3481 | - | GAGCUGGGCACCCUGAGGCGGGA | 23 | 3227 |
| myoC-3482 | - | GGAGCUGGGCACCCUGAGGCGGGA | 24 | 3228 |
| myoC-6569 | - | CUCCAGAAAGGAAAUGGA | 18 | 6315 |
| myoC-6570 | - | GCUCCAGAAAGGAAAUGGA | 19 | 6316 |
| myoC-1851 | - | GGCUCCAGAAAGGAAAUGGA | 20 | 2059 |
| myoC-6571 | - | AGGCUCCAGAAAGGAAAUGGA | 21 | 6317 |
| myoC-6572 | - | CAGGCUCCAGAAAGGAAAUGGA | 22 | 6318 |
| myoC-6573 | - | CCAGGCUCCAGAAAGGAAAUGGA | 23 | 6319 |
| myoC-6574 | - | UCCAGGCUCCAGAAAGGAAAUGGA | 24 | 6320 |
| myoC-6575 | - | UCUAACGGAGAAUCUGGA | 18 | 6321 |
| myoC-6576 | - | GUCUAACGGAGAAUCUGGA | 19 | 6322 |
| myoC-970 | - | AGUCUAACGGAGAAUCUGGA | 20 | 1270 |
| myoC-6577 | - | UAGUCUAACGGAGAAUCUGGA | 21 | 6323 |
| myoC-6578 | - | CUAGUCUAACGGAGAAUCUGGA | 22 | 6324 |
| myoC-6579 | - | ACUAGUCUAACGGAGAAUCUGGA | 23 | 6325 |
| myoC-6580 | - | AACUAGUCUAACGGAGAAUCUGGA | 24 | 6326 |
| myoC-6581 | - | ACUUAAACCCAGUGCUGA | 18 | 6327 |
| myoC-6582 | - | AACUUAAACCCAGUGCUGA | 19 | 6328 |
| myoC-1833 | - | CAACUUAAACCCAGUGCUGA | 20 | 2045 |
| myoC-6583 | - | CCAACUUAAACCCAGUGCUGA | 21 | 6329 |
| myoC-6584 | - | GCCAACUUAAACCCAGUGCUGA | 22 | 6330 |
| myoC-6585 | - | AGCCAACUUAAACCCAGUGCUGA | 23 | 6331 |
| myoC-6586 | - | CAGCCAACUUAAACCCAGUGCUGA | 24 | 6332 |
| myoC-6587 | - | AAUUCACGGAAGAAGUGA | 18 | 6333 |
| myoC-6588 | - | UAAUUCACGGAAGAAGUGA | 19 | 6334 |
| myoC-1919 | - | UUAAUUCACGGAAGAAGUGA | 20 | 2098 |
| myoC-6589 | - | GUUAAUUCACGGAAGAAGUGA | 21 | 6335 |
| myoC-6590 | - | CGUUAAUUCACGGAAGAAGUGA | 22 | 6336 |
| myoC-6591 | - | CCGUUAAUUCACGGAAGAAGUGA | 23 | 6337 |
| myoC-6592 | - | GCCGUUAAUUCACGGAAGAAGUGA | 24 | 6338 |
| myoC-6593 | - | AAUGAGUUUGCAGAGUGA | 18 | 6339 |
| myoC-6594 | - | CAAUGAGUUUGCAGAGUGA | 19 | 6340 |
| myoC-2084 | - | UCAAUGAGUUUGCAGAGUGA | 20 | 2213 |
| myoC-6595 | - | CUCAAUGAGUUUGCAGAGUGA | 21 | 6341 |
| myoC-6596 | - | UCUCAAUGAGUUUGCAGAGUGA | 22 | 6342 |
| myoC-6597 | - | UUCUCAAUGAGUUUGCAGAGUGA | 23 | 6343 |
| myoC-6598 | - | GUUCUCAAUGAGUUUGCAGAGUGA | 24 | 6344 |
| myoC-6599 | - | CUUUAUUUAAUGGGAAUA | 18 | 6345 |
| myoC-6600 | - | CCUUUAUUUAAUGGGAAUA | 19 | 6346 |
| myoC-1913 | - | GCCUUUAUUUAAUGGGAAUA | 20 | 2094 |
| myoC-6601 | - | GGCCUUUAUUUAAUGGGAAUA | 21 | 6347 |
| myoC-6602 | - | AGGCCUUUAUUUAAUGGGAAUA | 22 | 6348 |
| myoC-6603 | - | AAGGCCUUUAUUUAAUGGGAAUA | 23 | 6349 |
| myoC-6604 | - | GAAGGCCUUUAUUUAAUGGGAAUA | 24 | 6350 |
| myoC-6605 | - | UAAAACCAGGUGGAGAUA | 18 | 6351 |
| myoC-6606 | - | GUAAAACCAGGUGGAGAUA | 19 | 6352 |
| myoC-2090 | - | UGUAAAACCAGGUGGAGAUA | 20 | 2217 |
| myoC-6607 | - | GUGUAAAACCAGGUGGAGAUA | 21 | 6353 |
| myoC-6608 | - | UGUGUAAAACCAGGUGGAGAUA | 22 | 6354 |
| myoC-6609 | - | GUGUGUAAAACCAGGUGGAGAUA | 23 | 6355 |
| myoC-6610 | - | UGUGUGUAAAACCAGGUGGAGAUA | 24 | 6356 |
| myoC-6611 | - | GAGAGGGAAACUAGUCUA | 18 | 6357 |
| myoC-6612 | - | GGAGAGGGAAACUAGUCUA | 19 | 6358 |
| myoC-1854 | - | UGGAGAGGGAAACUAGUCUA | 20 | 2060 |
| myoC-6613 | - | AUGGAGAGGGAAACUAGUCUA | 21 | 6359 |
| myoC-6614 | - | AAUGGAGAGGGAAACUAGUCUA | 22 | 6360 |
| myoC-6615 | - | AAAUGGAGAGGGAAACUAGUCUA | 23 | 6361 |
| myoC-6616 | - | GAAAUGGAGAGGGAAACUAGUCUA | 24 | 6362 |
| myoC-6617 | - | GAGAUAUAGGAACUAUUA | 18 | 6363 |
| myoC-6618 | - | GGAGAUAUAGGAACUAUUA | 19 | 6364 |
| myoC-2092 | - | UGGAGAUAUAGGAACUAUUA | 20 | 2218 |
| myoC-6619 | - | GUGGAGAUAUAGGAACUAUUA | 21 | 6365 |
| myoC-6620 | - | GGUGGAGAUAUAGGAACUAUUA | 22 | 6366 |
| myoC-6621 | - | AGGUGGAGAUAUAGGAACUAUUA | 23 | 6367 |
| myoC-6622 | - | CAGGUGGAGAUAUAGGAACUAUUA | 24 | 6368 |
| myoC-3483 | - | UCAGUCAUCCAUAACUUA | 18 | 3229 |
| myoC-3484 | - | GUCAGUCAUCCAUAACUUA | 19 | 3230 |
| myoC-1607 | - | UGUCAGUCAUCCAUAACUUA | 20 | 1887 |
| myoC-3485 | - | AUGUCAGUCAUCCAUAACUUA | 21 | 3231 |
| myoC-3486 | - | CAUGUCAGUCAUCCAUAACUUA | 22 | 3232 |
| myoC-3487 | - | CCAUGUCAGUCAUCCAUAACUUA | 23 | 3233 |
| myoC-3488 | - | GCCAUGUCAGUCAUCCAUAACUUA | 24 | 3234 |
| myoC-6623 | - | UGUCCCUGCUACGUCUUA | 18 | 6369 |
| myoC-6624 | - | CUGUCCCUGCUACGUCUUA | 19 | 6370 |
| myoC-2079 | - | UCUGUCCCUGCUACGUCUUA | 20 | 2208 |
| myoC-6625 | - | CUCUGUCCCUGCUACGUCUUA | 21 | 6371 |
| myoC-6626 | - | UCUCUGUCCCUGCUACGUCUUA | 22 | 6372 |
| myoC-6627 | - | UUCUCUGUCCCUGCUACGUCUUA | 23 | 6373 |
| myoC-6628 | - | UUUCUCUGUCCCUGCUACGUCUUA | 24 | 6374 |
| myoC-6629 | - | CACGAAGGCCUUUAUUUA | 18 | 6375 |
| myoC-6630 | - | UCACGAAGGCCUUUAUUUA | 19 | 6376 |
| myoC-1910 | - | UUCACGAAGGCCUUUAUUUA | 20 | 2093 |
| myoC-6631 | - | CUUCACGAAGGCCUUUAUUUA | 21 | 6377 |
| myoC-6632 | - | CCUUCACGAAGGCCUUUAUUUA | 22 | 6378 |
| myoC-6633 | - | UCCUUCACGAAGGCCUUUAUUUA | 23 | 6379 |
| myoC-6634 | - | UUCCUUCACGAAGGCCUUUAUUUA | 24 | 6380 |
| myoC-3489 | - | CCAGCUGGAAACCCAAAC | 18 | 3235 |
| myoC-3490 | - | ACCAGCUGGAAACCCAAAC | 19 | 3236 |
| myoC-1634 | - | GACCAGCUGGAAACCCAAAC | 20 | 1903 |
| myoC-3491 | - | GGACCAGCUGGAAACCCAAAC | 21 | 3237 |
| myoC-3492 | - | GGGACCAGCUGGAAACCCAAAC | 22 | 3238 |
| myoC-3493 | - | CGGGACCAGCUGGAAACCCAAAC | 23 | 3239 |
| myoC-3494 | - | GCGGGACCAGCUGGAAACCCAAAC | 24 | 3240 |
| myoC-6635 | - | GUGAAUGGAAAUAUAAAC | 18 | 6381 |
| myoC-6636 | - | AGUGAAUGGAAAUAUAAAC | 19 | 6382 |
| myoC-2086 | - | GAGUGAAUGGAAAUAUAAAC | 20 | 2214 |
| myoC-6637 | - | AGAGUGAAUGGAAAUAUAAAC | 21 | 6383 |
| myoC-6638 | - | CAGAGUGAAUGGAAAUAUAAAC | 22 | 6384 |
| myoC-6639 | - | GCAGAGUGAAUGGAAAUAUAAAC | 23 | 6385 |
| myoC-6640 | - | UGCAGAGUGAAUGGAAAUAUAAAC | 24 | 6386 |
| myoC-6641 | - | CUUAUAUCUGCCAGACAC | 18 | 6387 |
| myoC-6642 | - | ACUUAUAUCUGCCAGACAC | 19 | 6388 |
| myoC-1824 | - | UACUUAUAUCUGCCAGACAC | 20 | 2038 |
| myoC-6643 | - | GUACUUAUAUCUGCCAGACAC | 21 | 6389 |
| myoC-6644 | - | AGUACUUAUAUCUGCCAGACAC | 22 | 6390 |
| myoC-6645 | - | GAGUACUUAUAUCUGCCAGACAC | 23 | 6391 |
| myoC-6646 | - | UGAGUACUUAUAUCUGCCAGACAC | 24 | 6392 |
| myoC-6647 | - | GGGGAGCCCUGCAAGCAC | 18 | 6393 |
| myoC-6648 | - | GGGGGAGCCCUGCAAGCAC | 19 | 6394 |
| myoC-1817 | - | UGGGGGAGCCCUGCAAGCAC | 20 | 2033 |
| myoC-6649 | - | CUGGGGGAGCCCUGCAAGCAC | 21 | 6395 |
| myoC-6650 | - | GCUGGGGGAGCCCUGCAAGCAC | 22 | 6396 |
| myoC-6651 | - | AGCUGGGGGAGCCCUGCAAGCAC | 23 | 6397 |
| myoC-6652 | - | CAGCUGGGGGAGCCCUGCAAGCAC | 24 | 6398 |
| myoC-3495 | - | AGCACCCAACGCUUAGAC | 18 | 3241 |
| myoC-3496 | - | CAGCACCCAACGCUUAGAC | 19 | 3242 |
| myoC-1609 | - | GCAGCACCCAACGCUUAGAC | 20 | 1889 |
| myoC-3497 | - | AGCAGCACCCAACGCUUAGAC | 21 | 3243 |
| myoC-3498 | - | CAGCAGCACCCAACGCUUAGAC | 22 | 3244 |
| myoC-3499 | - | ACAGCAGCACCCAACGCUUAGAC | 23 | 3245 |
| myoC-3500 | - | GACAGCAGCACCCAACGCUUAGAC | 24 | 3246 |
| myoC-3501 | - | CAGAGGGAGCUGGGCACC | 18 | 3247 |
| myoC-3502 | - | GCAGAGGGAGCUGGGCACC | 19 | 3248 |
| myoC-1626 | - | UGCAGAGGGAGCUGGGCACC | 20 | 1899 |
| myoC-3503 | - | CUGCAGAGGGAGCUGGGCACC | 21 | 3249 |
| myoC-3504 | - | GCUGCAGAGGGAGCUGGGCACC | 22 | 3250 |
| myoC-3505 | - | GGCUGCAGAGGGAGCUGGGCACC | 23 | 3251 |
| myoC-3506 | - | GGGCUGCAGAGGGAGCUGGGCACC | 24 | 3252 |
| myoC-3507 | - | GCCAGGCCCCAGGAGACC | 18 | 3253 |
| myoC-3508 | - | UGCCAGGCCCCAGGAGACC | 19 | 3254 |
| myoC-1617 | - | CUGCCAGGCCCCAGGAGACC | 20 | 1894 |
| myoC-3509 | - | GCUGCCAGGCCCCAGGAGACC | 21 | 3255 |
| myoC-3510 | - | GGCUGCCAGGCCCCAGGAGACC | 22 | 3256 |
| myoC-3511 | - | AGGCUGCCAGGCCCCAGGAGACC | 23 | 3257 |
| myoC-3512 | - | CAGGCUGCCAGGCCCCAGGAGACC | 24 | 3258 |
| myoC-3513 | - | GCACCCAACGCUUAGACC | 18 | 3259 |
| myoC-3514 | - | AGCACCCAACGCUUAGACC | 19 | 3260 |
| myoC-179 | - | CAGCACCCAACGCUUAGACC | 20 | 565 |
| myoC-3515 | - | GCAGCACCCAACGCUUAGACC | 21 | 3261 |
| myoC-3516 | - | AGCAGCACCCAACGCUUAGACC | 22 | 3262 |
| myoC-3517 | - | CAGCAGCACCCAACGCUUAGACC | 23 | 3263 |
| myoC-3518 | - | ACAGCAGCACCCAACGCUUAGACC | 24 | 3264 |
| myoC-3519 | - | CUCCUCCACCAAUUGACC | 18 | 3265 |
| myoC-3520 | - | CCUCCUCCACCAAUUGACC | 19 | 3266 |
| myoC-1614 | - | GCCUCCUCCACCAAUUGACC | 20 | 1892 |
| myoC-3521 | - | AGCCUCCUCCACCAAUUGACC | 21 | 3267 |
| myoC-3522 | - | GAGCCUCCUCCACCAAUUGACC | 22 | 3268 |
| myoC-3523 | - | AGAGCCUCCUCCACCAAUUGACC | 23 | 3269 |
| myoC-3524 | - | GAGAGCCUCCUCCACCAAUUGACC | 24 | 3270 |
| myoC-3525 | - | CCAGGCCCCAGGAGACCC | 18 | 3271 |
| myoC-3526 | - | GCCAGGCCCCAGGAGACCC | 19 | 3272 |
| myoC-185 | - | UGCCAGGCCCCAGGAGACCC | 20 | 571 |
| myoC-3527 | - | CUGCCAGGCCCCAGGAGACCC | 21 | 3273 |
| myoC-3528 | - | GCUGCCAGGCCCCAGGAGACCC | 22 | 3274 |
| myoC-3529 | - | GGCUGCCAGGCCCCAGGAGACCC | 23 | 3275 |
| myoC-3530 | - | AGGCUGCCAGGCCCCAGGAGACCC | 24 | 3276 |
| myoC-6653 | - | CCACCUCUGUCUUCCCCC | 18 | 6399 |
| myoC-6654 | - | GCCACCUCUGUCUUCCCCC | 19 | 6400 |
| myoC-2102 | - | GGCCACCUCUGUCUUCCCCC | 20 | 2225 |
| myoC-6655 | - | UGGCCACCUCUGUCUUCCCCC | 21 | 6401 |
| myoC-6656 | - | GUGGCCACCUCUGUCUUCCCCC | 22 | 6402 |
| myoC-6657 | - | CGUGGCCACCUCUGUCUUCCCCC | 23 | 6403 |
| myoC-6658 | - | ACGUGGCCACCUCUGUCUUCCCCC | 24 | 6404 |
| myoC-3531 | - | ACCAGGCUGCCAGGCCCC | 18 | 3277 |
| myoC-3532 | - | GACCAGGCUGCCAGGCCCC | 19 | 3278 |
| myoC-97 | - | GGACCAGGCUGCCAGGCCCC | 20 | 502 |
| myoC-3533 | - | UGGACCAGGCUGCCAGGCCCC | 21 | 3279 |
| myoC-3534 | - | UUGGACCAGGCUGCCAGGCCCC | 22 | 3280 |
| myoC-3535 | - | CUUGGACCAGGCUGCCAGGCCCC | 23 | 3281 |
| myoC-3536 | - | CCUUGGACCAGGCUGCCAGGCCCC | 24 | 3282 |
| myoC-3537 | - | GACCAGGCUGCCAGGCCC | 18 | 3283 |
| myoC-3538 | - | GGACCAGGCUGCCAGGCCC | 19 | 3284 |
| myoC-1615 | - | UGGACCAGGCUGCCAGGCCC | 20 | 1893 |
| myoC-3539 | - | UUGGACCAGGCUGCCAGGCCC | 21 | 3285 |
| myoC-3540 | - | CUUGGACCAGGCUGCCAGGCCC | 22 | 3286 |
| myoC-3541 | - | CCUUGGACCAGGCUGCCAGGCCC | 23 | 3287 |
| myoC-3542 | - | ACCUUGGACCAGGCUGCCAGGCCC | 24 | 3288 |
| myoC-3543 | - | AAGCUCGACUCAGCUCCC | 18 | 3289 |
| myoC-3544 | - | AAAGCUCGACUCAGCUCCC | 19 | 3290 |
| myoC-181 | - | CAAAGCUCGACUCAGCUCCC | 20 | 567 |
| myoC-3545 | - | CCAAAGCUCGACUCAGCUCCC | 21 | 3291 |
| myoC-3546 | - | ACCAAAGCUCGACUCAGCUCCC | 22 | 3292 |
| myoC-3547 | - | CACCAAAGCUCGACUCAGCUCCC | 23 | 3293 |
| myoC-3548 | - | CCACCAAAGCUCGACUCAGCUCCC | 24 | 3294 |
| myoC-3555 | - | GAAAAUGAGAAUCUGGCC | 18 | 3301 |
| myoC-3556 | - | AGAAAAUGAGAAUCUGGCC | 19 | 3302 |
| myoC-195 | - | AAGAAAAUGAGAAUCUGGCC | 20 | 581 |
| myoC-3557 | - | CAAGAAAAUGAGAAUCUGGCC | 21 | 3303 |
| myoC-3558 | - | GCAAGAAAAUGAGAAUCUGGCC | 22 | 3304 |
| myoC-3559 | - | GGCAAGAAAAUGAGAAUCUGGCC | 23 | 3305 |
| myoC-3560 | - | AGGCAAGAAAAUGAGAAUCUGGCC | 24 | 3306 |
| myoC-3561 | - | CCAAUGAAUCCAGCUGCC | 18 | 3307 |
| myoC-3562 | - | CCCAAUGAAUCCAGCUGCC | 19 | 3308 |
| myoC-1605 | - | UCCCAAUGAAUCCAGCUGCC | 20 | 1885 |
| myoC-3563 | - | GUCCCAAUGAAUCCAGCUGCC | 21 | 3309 |
| myoC-3564 | - | AGUCCCAAUGAAUCCAGCUGCC | 22 | 3310 |
| myoC-3565 | - | CAGUCCCAAUGAAUCCAGCUGCC | 23 | 3311 |
| myoC-3566 | - | CCAGUCCCAAUGAAUCCAGCUGCC | 24 | 3312 |
| myoC-6659 | - | UGCUGCCUCCAUCGUGCC | 18 | 6405 |
| myoC-6660 | - | CUGCUGCCUCCAUCGUGCC | 19 | 6406 |
| myoC-695 | - | CCUGCUGCCUCCAUCGUGCC | 20 | 1104 |
| myoC-6661 | - | CCCUGCUGCCUCCAUCGUGCC | 21 | 6407 |
| myoC-6662 | - | CCCCUGCUGCCUCCAUCGUGCC | 22 | 6408 |
| myoC-6663 | - | CCCCCUGCUGCCUCCAUCGUGCC | 23 | 6409 |
| myoC-6664 | - | GCCCCCUGCUGCCUCCAUCGUGCC | 24 | 6410 |
| myoC-3567 | - | AAAGCUCGACUCAGCUCC | 18 | 3313 |
| myoC-3568 | - | CAAAGCUCGACUCAGCUCC | 19 | 3314 |
| myoC-1611 | - | CCAAAGCUCGACUCAGCUCC | 20 | 1890 |
| myoC-3569 | - | ACCAAAGCUCGACUCAGCUCC | 21 | 3315 |
| myoC-3570 | - | CACCAAAGCUCGACUCAGCUCC | 22 | 3316 |
| myoC-3571 | - | CCACCAAAGCUCGACUCAGCUCC | 23 | 3317 |
| myoC-3572 | - | GCCACCAAAGCUCGACUCAGCUCC | 24 | 3318 |
| myoC-6665 | - | AAAGGGGCCUCCACGUCC | 18 | 6411 |
| myoC-6666 | - | GAAAGGGGCCUCCACGUCC | 19 | 6412 |
| myoC-977 | - | GGAAAGGGGCCUCCACGUCC | 20 | 1277 |
| myoC-6667 | - | GGGAAAGGGGCCUCCACGUCC | 21 | 6413 |
| myoC-6668 | - | AGGGAAAGGGGCCUCCACGUCC | 22 | 6414 |
| myoC-6669 | - | GAGGGAAAGGGGCCUCCACGUCC | 23 | 6415 |
| myoC-6670 | - | AGAGGGAAAGGGGCCUCCACGUCC | 24 | 6416 |
| myoC-6671 | - | CACGUCCAGGAGAAUUCC | 18 | 6417 |
| myoC-6672 | - | CCACGUCCAGGAGAAUUCC | 19 | 6418 |
| myoC-978 | - | UCCACGUCCAGGAGAAUUCC | 20 | 1278 |
| myoC-6673 | - | CUCCACGUCCAGGAGAAUUCC | 21 | 6419 |
| myoC-6674 | - | CCUCCACGUCCAGGAGAAUUCC | 22 | 6420 |
| myoC-6675 | - | GCCUCCACGUCCAGGAGAAUUCC | 23 | 6421 |
| myoC-6676 | - | GGCCUCCACGUCCAGGAGAAUUCC | 24 | 6422 |
| myoC-3573 | - | GGCGGGAGCGGGACCAGC | 18 | 3319 |
| myoC-3574 | - | AGGCGGGAGCGGGACCAGC | 19 | 3320 |
| myoC-105 | - | GAGGCGGGAGCGGGACCAGC | 20 | 510 |
| myoC-3575 | - | UGAGGCGGGAGCGGGACCAGC | 21 | 3321 |
| myoC-3576 | - | CUGAGGCGGGAGCGGGACCAGC | 22 | 3322 |
| myoC-3577 | - | CCUGAGGCGGGAGCGGGACCAGC | 23 | 3323 |
| myoC-3578 | - | CCCUGAGGCGGGAGCGGGACCAGC | 24 | 3324 |
| myoC-6677 | - | AGAGGUUUCCUCUCCAGC | 18 | 6423 |
| myoC-6678 | - | CAGAGGUUUCCUCUCCAGC | 19 | 6424 |
| myoC-676 | - | GCAGAGGUUUCCUCUCCAGC | 20 | 1006 |
| myoC-6679 | - | GGCAGAGGUUUCCUCUCCAGC | 21 | 6425 |
| myoC-6680 | - | CGGCAGAGGUUUCCUCUCCAGC | 22 | 6426 |
| myoC-6681 | - | CCGGCAGAGGUUUCCUCUCCAGC | 23 | 6427 |
| myoC-6682 | - | CCCGGCAGAGGUUUCCUCUCCAGC | 24 | 6428 |
| myoC-6683 | - | AAGAAUCUUGCUGGCAGC | 18 | 6429 |
| myoC-6684 | - | UAAGAAUCUUGCUGGCAGC | 19 | 6430 |
| myoC-2101 | - | CUAAGAAUCUUGCUGGCAGC | 20 | 2224 |
| myoC-6685 | - | UCUAAGAAUCUUGCUGGCAGC | 21 | 6431 |
| myoC-6686 | - | UUCUAAGAAUCUUGCUGGCAGC | 22 | 6432 |
| myoC-6687 | - | UUUCUAAGAAUCUUGCUGGCAGC | 23 | 6433 |
| myoC-6688 | - | UUUUCUAAGAAUCUUGCUGGCAGC | 24 | 6434 |
| myoC-6689 | - | UAUAAACCUCUCUGGAGC | 18 | 6435 |
| myoC-6690 | - | AUAUAAACCUCUCUGGAGC | 19 | 6436 |
| myoC-2106 | - | UAUAUAAACCUCUCUGGAGC | 20 | 2228 |
| myoC-6691 | - | AUAUAUAAACCUCUCUGGAGC | 21 | 6437 |
| myoC-6692 | - | UAUAUAUAAACCUCUCUGGAGC | 22 | 6438 |
| myoC-6693 | - | GUAUAUAUAAACCUCUCUGGAGC | 23 | 6439 |
| myoC-6694 | - | AGUAUAUAUAAACCUCUCUGGAGC | 24 | 6440 |
| myoC-6695 | - | GUCCUGGUGCAUCUGAGC | 18 | 6441 |
| myoC-6696 | - | CGUCCUGGUGCAUCUGAGC | 19 | 6442 |
| myoC-1844 | - | UCGUCCUGGUGCAUCUGAGC | 20 | 2054 |
| myoC-6697 | - | AUCGUCCUGGUGCAUCUGAGC | 21 | 6443 |
| myoC-6698 | - | AAUCGUCCUGGUGCAUCUGAGC | 22 | 6444 |
| myoC-6699 | - | GAAUCGUCCUGGUGCAUCUGAGC | 23 | 6445 |
| myoC-6700 | - | UGAAUCGUCCUGGUGCAUCUGAGC | 24 | 6446 |
| myoC-6701 | - | UGCAGGGAGUGGGGACGC | 18 | 6447 |
| myoC-6702 | - | CUGCAGGGAGUGGGGACGC | 19 | 6448 |
| myoC-988 | - | ACUGCAGGGAGUGGGGACGC | 20 | 1288 |
| myoC-6703 | - | GACUGCAGGGAGUGGGGACGC | 21 | 6449 |
| myoC-6704 | - | GGACUGCAGGGAGUGGGGACGC | 22 | 6450 |
| myoC-6705 | - | GGGACUGCAGGGAGUGGGGACGC | 23 | 6451 |
| myoC-6706 | - | GGGGACUGCAGGGAGUGGGGACGC | 24 | 6452 |
| myoC-6707 | - | GAGCGGGUGCUGAAAGGC | 18 | 6453 |
| myoC-6708 | - | UGAGCGGGUGCUGAAAGGC | 19 | 6454 |
| myoC-994 | - | CUGAGCGGGUGCUGAAAGGC | 20 | 1294 |
| myoC-6709 | - | GCUGAGCGGGUGCUGAAAGGC | 21 | 6455 |
| myoC-6710 | - | GGCUGAGCGGGUGCUGAAAGGC | 22 | 6456 |
| myoC-6711 | - | GGGCUGAGCGGGUGCUGAAAGGC | 23 | 6457 |
| myoC-6712 | - | GGGGCUGAGCGGGUGCUGAAAGGC | 24 | 6458 |
| myoC-3585 | - | AGAAGAAGCGACUAAGGC | 18 | 3331 |
| myoC-3586 | - | GAGAAGAAGCGACUAAGGC | 19 | 3332 |
| myoC-1646 | - | AGAGAAGAAGCGACUAAGGC | 20 | 1912 |
| myoC-3587 | - | AAGAGAAGAAGCGACUAAGGC | 21 | 3333 |
| myoC-3588 | - | GAAGAGAAGAAGCGACUAAGGC | 22 | 3334 |
| myoC-3589 | - | GGAAGAGAAGAAGCGACUAAGGC | 23 | 3335 |
| myoC-3590 | - | AGGAAGAGAAGAAGCGACUAAGGC | 24 | 3336 |
| myoC-3591 | - | AGCUGGGCACCCUGAGGC | 18 | 3337 |
| myoC-3592 | - | GAGCUGGGCACCCUGAGGC | 19 | 3338 |
| myoC-103 | - | GGAGCUGGGCACCCUGAGGC | 20 | 508 |
| myoC-3593 | - | GGGAGCUGGGCACCCUGAGGC | 21 | 3339 |
| myoC-3594 | - | AGGGAGCUGGGCACCCUGAGGC | 22 | 3340 |
| myoC-3595 | - | GAGGGAGCUGGGCACCCUGAGGC | 23 | 3341 |
| myoC-3596 | - | AGAGGGAGCUGGGCACCCUGAGGC | 24 | 3342 |
| myoC-6713 | - | CCUCUCUGGAGCUCGGGC | 18 | 6459 |
| myoC-6714 | - | ACCUCUCUGGAGCUCGGGC | 19 | 6460 |
| myoC-2107 | - | AACCUCUCUGGAGCUCGGGC | 20 | 2229 |
| myoC-6715 | - | AAACCUCUCUGGAGCUCGGGC | 21 | 6461 |
| myoC-6716 | - | UAAACCUCUCUGGAGCUCGGGC | 22 | 6462 |
| myoC-6717 | - | AUAAACCUCUCUGGAGCUCGGGC | 23 | 6463 |
| myoC-6718 | - | UAUAAACCUCUCUGGAGCUCGGGC | 24 | 6464 |
| myoC-6719 | - | CAGUGUUGUUCACGGGGC | 18 | 6465 |
| myoC-6720 | - | UCAGUGUUGUUCACGGGGC | 19 | 6466 |
| myoC-1002 | - | UUCAGUGUUGUUCACGGGGC | 20 | 1302 |
| myoC-6721 | - | GUUCAGUGUUGUUCACGGGGC | 21 | 6467 |
| myoC-6722 | - | UGUUCAGUGUUGUUCACGGGGC | 22 | 6468 |
| myoC-6723 | - | AUGUUCAGUGUUGUUCACGGGGC | 23 | 6469 |
| myoC-6724 | - | GAUGUUCAGUGUUGUUCACGGGGC | 24 | 6470 |
| myoC-3597 | - | GGUGUGGGAUGUGGGGGC | 18 | 3343 |
| myoC-3598 | - | UGGUGUGGGAUGUGGGGGC | 19 | 3344 |
| myoC-1600 | - | CUGGUGUGGGAUGUGGGGGC | 20 | 1881 |
| myoC-3599 | - | CCUGGUGUGGGAUGUGGGGGC | 21 | 3345 |
| myoC-3600 | - | GCCUGGUGUGGGAUGUGGGGGC | 22 | 3346 |
| myoC-3601 | - | UGCCUGGUGUGGGAUGUGGGGGC | 23 | 3347 |
| myoC-3602 | - | CUGCCUGGUGUGGGAUGUGGGGGC | 24 | 3348 |
| myoC-3603 | - | GUUGCUGCAGCUUUGGGC | 18 | 3349 |
| myoC-3604 | - | CGUUGCUGCAGCUUUGGGC | 19 | 3350 |
| myoC-1594 | - | ACGUUGCUGCAGCUUUGGGC | 20 | 1878 |
| myoC-3605 | - | CACGUUGCUGCAGCUUUGGGC | 21 | 3351 |
| myoC-3606 | - | GCACGUUGCUGCAGCUUUGGGC | 22 | 3352 |
| myoC-3607 | - | UGCACGUUGCUGCAGCUUUGGGC | 23 | 3353 |
| myoC-3608 | - | GUGCACGUUGCUGCAGCUUUGGGC | 24 | 3354 |
| myoC-3609 | - | AGAAAAUGAGAAUCUGGC | 18 | 3355 |
| myoC-3610 | - | AAGAAAAUGAGAAUCUGGC | 19 | 3356 |
| myoC-1649 | - | CAAGAAAAUGAGAAUCUGGC | 20 | 1915 |
| myoC-3611 | - | GCAAGAAAAUGAGAAUCUGGC | 21 | 3357 |
| myoC-3612 | - | GGCAAGAAAAUGAGAAUCUGGC | 22 | 3358 |
| myoC-3613 | - | AGGCAAGAAAAUGAGAAUCUGGC | 23 | 3359 |
| myoC-3614 | - | AAGGCAAGAAAAUGAGAAUCUGGC | 24 | 3360 |
| myoC-6725 | - | CCAGGAGGUGGGGACUGC | 18 | 6471 |
| myoC-6726 | - | UCCAGGAGGUGGGGACUGC | 19 | 6472 |
| myoC-983 | - | UUCCAGGAGGUGGGGACUGC | 20 | 1283 |
| myoC-6727 | - | AUUCCAGGAGGUGGGGACUGC | 21 | 6473 |
| myoC-6728 | - | AAUUCCAGGAGGUGGGGACUGC | 22 | 6474 |
| myoC-6729 | - | GAAUUCCAGGAGGUGGGGACUGC | 23 | 6475 |
| myoC-6730 | - | AGAAUUCCAGGAGGUGGGGACUGC | 24 | 6476 |
| myoC-6731 | - | UUUUUAUCUUUUCUCUGC | 18 | 6477 |
| myoC-6732 | - | CUUUUUAUCUUUUCUCUGC | 19 | 6478 |
| myoC-1898 | - | CCUUUUUAUCUUUUCUCUGC | 20 | 2084 |
| myoC-6733 | - | GCCUUUUUAUCUUUUCUCUGC | 21 | 6479 |
| myoC-6734 | - | AGCCUUUUUAUCUUUUCUCUGC | 22 | 6480 |
| myoC-6735 | - | GAGCCUUUUUAUCUUUUCUCUGC | 23 | 6481 |
| myoC-6736 | - | UGAGCCUUUUUAUCUUUUCUCUGC | 24 | 6482 |
| myoC-6737 | - | CUGCUGCCUCCAUCGUGC | 18 | 6483 |
| myoC-6738 | - | CCUGCUGCCUCCAUCGUGC | 19 | 6484 |
| myoC-1838 | - | CCCUGCUGCCUCCAUCGUGC | 20 | 2049 |
| myoC-6739 | - | CCCCUGCUGCCUCCAUCGUGC | 21 | 6485 |
| myoC-6740 | - | CCCCCUGCUGCCUCCAUCGUGC | 22 | 6486 |
| myoC-6741 | - | GCCCCCUGCUGCCUCCAUCGUGC | 23 | 6487 |
| myoC-6742 | - | CGCCCCCUGCUGCCUCCAUCGUGC | 24 | 6488 |
| myoC-6743 | - | CUAGUCUAACGGAGAAUC | 18 | 6489 |
| myoC-6744 | - | ACUAGUCUAACGGAGAAUC | 19 | 6490 |
| myoC-968 | - | AACUAGUCUAACGGAGAAUC | 20 | 1268 |
| myoC-6745 | - | AAACUAGUCUAACGGAGAAUC | 21 | 6491 |
| myoC-6746 | - | GAAACUAGUCUAACGGAGAAUC | 22 | 6492 |
| myoC-6747 | - | GGAAACUAGUCUAACGGAGAAUC | 23 | 6493 |
| myoC-6748 | - | GGGAAACUAGUCUAACGGAGAAUC | 24 | 6494 |
| myoC-6749 | - | AAGGAAAUAAACACCAUC | 18 | 6495 |
| myoC-6750 | - | AAAGGAAAUAAACACCAUC | 19 | 6496 |
| myoC-1836 | - | GAAAGGAAAUAAACACCAUC | 20 | 2047 |
| myoC-6751 | - | AGAAAGGAAAUAAACACCAUC | 21 | 6497 |
| myoC-6752 | - | AAGAAAGGAAAUAAACACCAUC | 22 | 6498 |
| myoC-6753 | - | AAAGAAAGGAAAUAAACACCAUC | 23 | 6499 |
| myoC-6754 | - | GAAAGAAAGGAAAUAAACACCAUC | 24 | 6500 |
| myoC-3615 | - | GCCAGGACAGCUCAGCUC | 18 | 3361 |
| myoC-3616 | - | GGCCAGGACAGCUCAGCUC | 19 | 3362 |
| myoC-96 | - | GGGCCAGGACAGCUCAGCUC | 20 | 501 |
| myoC-3617 | - | GGGGCCAGGACAGCUCAGCUC | 21 | 3363 |
| myoC-3618 | - | GGGGGCCAGGACAGCUCAGCUC | 22 | 3364 |
| myoC-3619 | - | UGGGGGCCAGGACAGCUCAGCUC | 23 | 3365 |
| myoC-3620 | - | GUGGGGGCCAGGACAGCUCAGCUC | 24 | 3366 |
| myoC-6755 | - | CUCCUUGGCUCCAGGCUC | 18 | 6501 |
| myoC-6756 | - | ACUCCUUGGCUCCAGGCUC | 19 | 6502 |
| myoC-1845 | - | GACUCCUUGGCUCCAGGCUC | 20 | 2055 |
| myoC-6757 | - | AGACUCCUUGGCUCCAGGCUC | 21 | 6503 |
| myoC-6758 | - | GAGACUCCUUGGCUCCAGGCUC | 22 | 6504 |
| myoC-6759 | - | GGAGACUCCUUGGCUCCAGGCUC | 23 | 6505 |
| myoC-6760 | - | UGGAGACUCCUUGGCUCCAGGCUC | 24 | 6506 |
| myoC-6761 | - | UGUUUUGUUAUCACUCUC | 18 | 6507 |
| myoC-6762 | - | UUGUUUUGUUAUCACUCUC | 19 | 6508 |
| myoC-1821 | - | GUUGUUUUGUUAUCACUCUC | 20 | 2036 |
| myoC-6763 | - | GGUUGUUUUGUUAUCACUCUC | 21 | 6509 |
| myoC-6764 | - | UGGUUGUUUUGUUAUCACUCUC | 22 | 6510 |
| myoC-6765 | - | CUGGUUGUUUUGUUAUCACUCUC | 23 | 6511 |
| myoC-6766 | - | ACUGGUUGUUUUGUUAUCACUCUC | 24 | 6512 |
| myoC-6767 | - | AGUAUAUAUAAACCUCUC | 18 | 6513 |
| myoC-6768 | - | CAGUAUAUAUAAACCUCUC | 19 | 6514 |
| myoC-853 | - | CCAGUAUAUAUAAACCUCUC | 20 | 1197 |
| myoC-6769 | - | CCCAGUAUAUAUAAACCUCUC | 21 | 6515 |
| myoC-6770 | - | CCCCAGUAUAUAUAAACCUCUC | 22 | 6516 |
| myoC-6771 | - | UCCCCAGUAUAUAUAAACCUCUC | 23 | 6517 |
| myoC-6772 | - | CUCCCCAGUAUAUAUAAACCUCUC | 24 | 6518 |
| myoC-6773 | - | UGGAGGUGACAGUUUCUC | 18 | 6519 |
| myoC-6774 | - | GUGGAGGUGACAGUUUCUC | 19 | 6520 |
| myoC-1828 | - | CGUGGAGGUGACAGUUUCUC | 20 | 2041 |
| myoC-6775 | - | UCGUGGAGGUGACAGUUUCUC | 21 | 6521 |
| myoC-6776 | - | UUCGUGGAGGUGACAGUUUCUC | 22 | 6522 |
| myoC-6777 | - | CUUCGUGGAGGUGACAGUUUCUC | 23 | 6523 |
| myoC-6778 | - | CCUUCGUGGAGGUGACAGUUUCUC | 24 | 6524 |
| myoC-6779 | - | GAAAGGGGCCUCCACGUC | 18 | 6525 |
| myoC-6780 | - | GGAAAGGGGCCUCCACGUC | 19 | 6526 |
| myoC-1868 | - | GGGAAAGGGGCCUCCACGUC | 20 | 2067 |
| myoC-6781 | - | AGGGAAAGGGGCCUCCACGUC | 21 | 6527 |
| myoC-6782 | - | GAGGGAAAGGGGCCUCCACGUC | 22 | 6528 |
| myoC-6783 | - | AGAGGGAAAGGGGCCUCCACGUC | 23 | 6529 |
| myoC-6784 | - | CAGAGGGAAAGGGGCCUCCACGUC | 24 | 6530 |
| myoC-6785 | - | CCCGGGGUCCUGGGUGUC | 18 | 6531 |
| myoC-6786 | - | ACCCGGGGUCCUGGGUGUC | 19 | 6532 |
| myoC-1820 | - | CACCCGGGGUCCUGGGUGUC | 20 | 2035 |
| myoC-6787 | - | GCACCCGGGGUCCUGGGUGUC | 21 | 6533 |
| myoC-6788 | - | AGCACCCGGGGUCCUGGGUGUC | 22 | 6534 |
| myoC-6789 | - | AAGCACCCGGGGUCCUGGGUGUC | 23 | 6535 |
| myoC-6790 | - | CAAGCACCCGGGGUCCUGGGUGUC | 24 | 6536 |
| myoC-6791 | - | CCACGUCCAGGAGAAUUC | 18 | 6537 |
| myoC-6792 | - | UCCACGUCCAGGAGAAUUC | 19 | 6538 |
| myoC-1871 | - | CUCCACGUCCAGGAGAAUUC | 20 | 2069 |
| myoC-6793 | - | CCUCCACGUCCAGGAGAAUUC | 21 | 6539 |
| myoC-6794 | - | GCCUCCACGUCCAGGAGAAUUC | 22 | 6540 |
| myoC-6795 | - | GGCCUCCACGUCCAGGAGAAUUC | 23 | 6541 |
| myoC-6796 | - | GGGCCUCCACGUCCAGGAGAAUUC | 24 | 6542 |
| myoC-6797 | - | UUCCUAGGCCGUUAAUUC | 18 | 6543 |
| myoC-6798 | - | UUUCCUAGGCCGUUAAUUC | 19 | 6544 |
| myoC-1916 | - | AUUUCCUAGGCCGUUAAUUC | 20 | 2096 |
| myoC-6799 | - | CAUUUCCUAGGCCGUUAAUUC | 21 | 6545 |
| myoC-6800 | - | UCAUUUCCUAGGCCGUUAAUUC | 22 | 6546 |
| myoC-6801 | - | CUCAUUUCCUAGGCCGUUAAUUC | 23 | 6547 |
| myoC-6802 | - | GCUCAUUUCCUAGGCCGUUAAUUC | 24 | 6548 |
| myoC-6803 | - | AAACUCCAAACAGACUUC | 18 | 6549 |
| myoC-6804 | - | GAAACUCCAAACAGACUUC | 19 | 6550 |
| myoC-845 | - | AGAAACUCCAAACAGACUUC | 20 | 1179 |
| myoC-6805 | - | AAGAAACUCCAAACAGACUUC | 21 | 6551 |
| myoC-6806 | - | AAAGAAACUCCAAACAGACUUC | 22 | 6552 |
| myoC-6807 | - | AAAAGAAACUCCAAACAGACUUC | 23 | 6553 |
| myoC-6808 | - | AAAAAGAAACUCCAAACAGACUUC | 24 | 6554 |
| myoC-6809 | - | AG UCACUGCCCUACCUUC | 18 | 6555 |
| myoC-6810 | - | CAGUCACUGCCCUACCUUC | 19 | 6556 |
| myoC-1826 | - | GCAGUCACUGCCCUACCUUC | 20 | 2040 |
| myoC-6811 | - | AGCAGUCACUGCCCUACCUUC | 21 | 6557 |
| myoC-6812 | - | AAGCAGUCACUGCCCUACCUUC | 22 | 6558 |
| myoC-6813 | - | AAAGCAGUCACUGCCCUACCUUC | 23 | 6559 |
| myoC-6814 | - | CAAAGCAGUCACUGCCCUACCUUC | 24 | 6560 |
| myoC-6815 | - | GUGCAUGGGUUUUCCUUC | 18 | 6561 |
| myoC-6816 | - | UGUGCAUGGGUUUUCCUUC | 19 | 6562 |
| myoC-1909 | - | GUGUGCAUGGGUUUUCCUUC | 20 | 2092 |
| myoC-6817 | - | GGUGUGCAUGGGUUUUCCUUC | 21 | 6563 |
| myoC-6818 | - | GGGUGUGCAUGGGUUUUCCUUC | 22 | 6564 |
| myoC-6819 | - | AGGGUGUGCAUGGGUUUUCCUUC | 23 | 6565 |
| myoC-6820 | - | CAGGGUGUGCAUGGGUUUUCCUUC | 24 | 6566 |
| myoC-3621 | - | UCCGAGACAAGUCAGUUC | 18 | 3367 |
| myoC-3622 | - | CUCCGAGACAAGUCAGUUC | 19 | 3368 |
| myoC-191 | - | CCUCCGAGACAAGUCAGUUC | 20 | 577 |
| myoC-3623 | - | UCCUCCGAGACAAGUCAGUUC | 21 | 3369 |
| myoC-3624 | - | CUCCUCCGAGACAAGUCAGUUC | 22 | 3370 |
| myoC-3625 | - | CCUCCUCCGAGACAAGUCAGUUC | 23 | 3371 |
| myoC-3626 | - | ACCUCCUCCGAGACAAGUCAGUUC | 24 | 3372 |
| myoC-6821 | - | AGAUGUUCAGUGUUGUUC | 18 | 6567 |
| myoC-6822 | - | CAGAUGUUCAGUGUUGUUC | 19 | 6568 |
| myoC-1892 | - | CCAGAUGUUCAGUGUUGUUC | 20 | 2081 |
| myoC-6823 | - | CCCAGAUGUUCAGUGUUGUUC | 21 | 6569 |
| myoC-6824 | - | GCCCAGAUGUUCAGUGUUGUUC | 22 | 6570 |
| myoC-6825 | - | UGCCCAGAUGUUCAGUGUUGUUC | 23 | 6571 |
| myoC-6826 | - | CUGCCCAGAUGUUCAGUGUUGUUC | 24 | 6572 |
| myoC-6827 | - | GGAGAAGAAGUCUAUUUC | 18 | 6573 |
| myoC-6828 | - | AGGAGAAGAAGUCUAUUUC | 19 | 6574 |
| myoC-1904 | - | GAGGAGAAGAAGUCUAUUUC | 20 | 2088 |
| myoC-6829 | - | GGAGGAGAAGAAGUCUAUUUC | 21 | 6575 |
| myoC-6830 | - | UGGAGGAGAAGAAGUCUAUUUC | 22 | 6576 |
| myoC-6831 | - | UUGGAGGAGAAGAAGUCUAUUUC | 23 | 6577 |
| myoC-6832 | - | CUUGGAGGAGAAGAAGUCUAUUUC | 24 | 6578 |
| myoC-6833 | - | CAGCACAGCAGAGCUUUC | 18 | 6579 |
| myoC-6834 | - | UCAGCACAGCAGAGCUUUC | 19 | 6580 |
| myoC-2109 | - | CUCAGCACAGCAGAGCUUUC | 20 | 2231 |
| myoC-6835 | - | UCUCAGCACAGCAGAGCUUUC | 21 | 6581 |
| myoC-6836 | - | CUCUCAGCACAGCAGAGCUUUC | 22 | 6582 |
| myoC-6837 | - | CCUCUCAGCACAGCAGAGCUUUC | 23 | 6583 |
| myoC-6838 | - | ACCUCUCAGCACAGCAGAGCUUUC | 24 | 6584 |
| myoC-6839 | - | GUGCUGAAAGGCAGGAAG | 18 | 6585 |
| myoC-6840 | - | GGUGCUGAAAGGCAGGAAG | 19 | 6586 |
| myoC-1889 | - | GGGUGCUGAAAGGCAGGAAG | 20 | 2078 |
| myoC-6841 | - | CGGGUGCUGAAAGGCAGGAAG | 21 | 6587 |
| myoC-6842 | - | GCGGGUGCUGAAAGGCAGGAAG | 22 | 6588 |
| myoC-6843 | - | AGCGGGUGCUGAAAGGCAGGAAG | 23 | 6589 |
| myoC-6844 | - | GAGCGGGUGCUGAAAGGCAGGAAG | 24 | 6590 |
| myoC-6845 | - | AGGCACCUCUCAGCACAG | 18 | 6591 |
| myoC-6846 | - | CAGGCACCUCUCAGCACAG | 19 | 6592 |
| myoC-2108 | - | CCAGGCACCUCUCAGCACAG | 20 | 2230 |
| myoC-6847 | - | UCCAGGCACCUCUCAGCACAG | 21 | 6593 |
| myoC-6848 | - | AUCCAGGCACCUCUCAGCACAG | 22 | 6594 |
| myoC-6849 | - | CAUCCAGGCACCUCUCAGCACAG | 23 | 6595 |
| myoC-6850 | - | CCAUCCAGGCACCUCUCAGCACAG | 24 | 6596 |
| myoC-6851 | - | GUGUGUGUGUAAAACCAG | 18 | 6597 |
| myoC-6852 | - | UGUGUGUGUGUAAAACCAG | 19 | 6598 |
| myoC-2088 | - | GUGUGUGUGUGUAAAACCAG | 20 | 2216 |
| myoC-6853 | - | UGUGUGUGUGUGUAAAACCAG | 21 | 6599 |
| myoC-6854 | - | GUGUGUGUGUGUGUAAAACCAG | 22 | 6600 |
| myoC-6855 | - | UGUGUGUGUGUGUGUAAAACCAG | 23 | 6601 |
| myoC-6856 | - | GUGUGUGUGUGUGUGUAAAACCAG | 24 | 6602 |
| myoC-3627 | - | AGGCGGGAGCGGGACCAG | 18 | 3373 |
| myoC-3628 | - | GAGGCGGGAGCGGGACCAG | 19 | 3374 |
| myoC-1632 | - | UGAGGCGGGAGCGGGACCAG | 20 | 1902 |
| myoC-3629 | - | CUGAGGCGGGAGCGGGACCAG | 21 | 3375 |
| myoC-3630 | - | CCUGAGGCGGGAGCGGGACCAG | 22 | 3376 |
| myoC-3631 | - | CCCUGAGGCGGGAGCGGGACCAG | 23 | 3377 |
| myoC-3632 | - | ACCCUGAGGCGGGAGCGGGACCAG | 24 | 3378 |
| myoC-3633 | - | AGGCCCCAGGAGACCCAG | 18 | 3379 |
| myoC-3634 | - | CAGGCCCCAGGAGACCCAG | 19 | 3380 |
| myoC-1619 | - | CCAGGCCCCAGGAGACCCAG | 20 | 1895 |
| myoC-3635 | - | GCCAGGCCCCAGGAGACCCAG | 21 | 3381 |
| myoC-3636 | - | UGCCAGGCCCCAGGAGACCCAG | 22 | 3382 |
| myoC-3637 | - | CUGCCAGGCCCCAGGAGACCCAG | 23 | 3383 |
| myoC-3638 | - | GCUGCCAGGCCCCAGGAGACCCAG | 24 | 3384 |
| myoC-6857 | - | CAGAGGUUUCCUCUCCAG | 18 | 6603 |
| myoC-6858 | - | GCAGAGGUUUCCUCUCCAG | 19 | 6604 |
| myoC-1812 | - | GGCAGAGGUUUCCUCUCCAG | 20 | 2032 |
| myoC-6859 | - | CGGCAGAGGUUUCCUCUCCAG | 21 | 6605 |
| myoC-6860 | - | CCGGCAGAGGUUUCCUCUCCAG | 22 | 6606 |
| myoC-6861 | - | CCCGGCAGAGGUUUCCUCUCCAG | 23 | 6607 |
| myoC-6862 | - | CCCCGGCAGAGGUUUCCUCUCCAG | 24 | 6608 |
| myoC-6863 | - | CACAGCAGAGCUUUCCAG | 18 | 6609 |
| myoC-6864 | - | GCACAGCAGAGCUUUCCAG | 19 | 6610 |
| myoC-2111 | - | AGCACAGCAGAGCUUUCCAG | 20 | 2233 |
| myoC-6865 | - | CAGCACAGCAGAGCUUUCCAG | 21 | 6611 |
| myoC-6866 | - | UCAGCACAGCAGAGCUUUCCAG | 22 | 6612 |
| myoC-6867 | - | CUCAGCACAGCAGAGCUUUCCAG | 23 | 6613 |
| myoC-6868 | - | UCUCAGCACAGCAGAGCUUUCCAG | 24 | 6614 |
| myoC-3639 | - | ACCCAGGAGGGGCUGCAG | 18 | 3385 |
| myoC-3640 | - | GACCCAGGAGGGGCUGCAG | 19 | 3386 |
| myoC-188 | - | AGACCCAGGAGGGGCUGCAG | 20 | 574 |
| myoC-3641 | - | GAGACCCAGGAGGGGCUGCAG | 21 | 3387 |
| myoC-3642 | - | GGAGACCCAGGAGGGGCUGCAG | 22 | 3388 |
| myoC-3643 | - | AGGAGACCCAGGAGGGGCUGCAG | 23 | 3389 |
| myoC-3644 | - | CAGGAGACCCAGGAGGGGCUGCAG | 24 | 3390 |
| myoC-6869 | - | CUCAUGGAAGACGUGCAG | 18 | 6615 |
| myoC-6870 | - | UCUCAUGGAAGACGUGCAG | 19 | 6616 |
| myoC-1831 | - | UUCUCAUGGAAGACGUGCAG | 20 | 2043 |
| myoC-6871 | - | UUUCUCAUGGAAGACGUGCAG | 21 | 6617 |
| myoC-6872 | - | GUUUCUCAUGGAAGACGUGCAG | 22 | 6618 |
| myoC-6873 | - | AGUUUCUCAUGGAAGACGUGCAG | 23 | 6619 |
| myoC-6874 | - | CAGUUUCUCAUGGAAGACGUGCAG | 24 | 6620 |
| myoC-6875 | - | GGGACAGUGUUUCCUCAG | 18 | 6621 |
| myoC-6876 | - | GGGGACAGUGUUUCCUCAG | 19 | 6622 |
| myoC-972 | - | AGGGGACAGUGUUUCCUCAG | 20 | 1272 |
| myoC-6877 | - | GAGGGGACAGUGUUUCCUCAG | 21 | 6623 |
| myoC-6878 | - | GGAGGGGACAGUGUUUCCUCAG | 22 | 6624 |
| myoC-6879 | - | UGGAGGGGACAGUGUUUCCUCAG | 23 | 6625 |
| myoC-6880 | - | CUGGAGGGGACAGUGUUUCCUCAG | 24 | 6626 |
| myoC-3645 | - | UCAGUUCUGGAGGAAGAG | 18 | 3391 |
| myoC-3646 | - | GUCAGUUCUGGAGGAAGAG | 19 | 3392 |
| myoC-1645 | - | AGUCAGUUCUGGAGGAAGAG | 20 | 1911 |
| myoC-3647 | - | AAGUCAGUUCUGGAGGAAGAG | 21 | 3393 |
| myoC-3648 | - | CAAGUCAGUUCUGGAGGAAGAG | 22 | 3394 |
| myoC-3649 | - | ACAAGUCAGUUCUGGAGGAAGAG | 23 | 3395 |
| myoC-3650 | - | GACAAGUCAGUUCUGGAGGAAGAG | 24 | 3396 |
| myoC-3651 | - | GAAUCCAGCUGCCCAGAG | 18 | 3397 |
| myoC-3652 | - | UGAAUCCAGCUGCCCAGAG | 19 | 3398 |
| myoC-1606 | - | AUGAAUCCAGCUGCCCAGAG | 20 | 1886 |
| myoC-3653 | - | AAUGAAUCCAGCUGCCCAGAG | 21 | 3399 |
| myoC-3654 | - | CAAUGAAUCCAGCUGCCCAGAG | 22 | 3400 |
| myoC-3655 | - | CCAAUGAAUCCAGCUGCCCAGAG | 23 | 3401 |
| myoC-3656 | - | CCCAAUGAAUCCAGCUGCCCAGAG | 24 | 3402 |
| myoC-3657 | - | GAAACCCAAACCAGAGAG | 18 | 3403 |
| myoC-3658 | - | GGAAACCCAAACCAGAGAG | 19 | 3404 |
| myoC-1636 | - | UGGAAACCCAAACCAGAGAG | 20 | 1905 |
| myoC-3659 | - | CUGGAAACCCAAACCAGAGAG | 21 | 3405 |
| myoC-3660 | - | GCUGGAAACCCAAACCAGAGAG | 22 | 3406 |
| myoC-3661 | - | AGCUGGAAACCCAAACCAGAGAG | 23 | 3407 |
| myoC-3662 | - | CAGCUGGAAACCCAAACCAGAGAG | 24 | 3408 |
| myoC-6881 | - | CCAGGAGAAUUCCAGGAG | 18 | 6627 |
| myoC-6882 | - | UCCAGGAGAAUUCCAGGAG | 19 | 6628 |
| myoC-1873 | - | GUCCAGGAGAAUUCCAGGAG | 20 | 2070 |
| myoC-6883 | - | CGUCCAGGAGAAUUCCAGGAG | 21 | 6629 |
| myoC-6884 | - | ACGUCCAGGAGAAUUCCAGGAG | 22 | 6630 |
| myoC-6885 | - | CACGUCCAGGAGAAUUCCAGGAG | 23 | 6631 |
| myoC-6886 | - | CCACGUCCAGGAGAAUUCCAGGAG | 24 | 6632 |
| myoC-6887 | - | UUUCUCUGCUUGGAGGAG | 18 | 6633 |
| myoC-6888 | - | UUUUCUCUGCUUGGAGGAG | 19 | 6634 |
| myoC-1903 | - | CUUUUCUCUGCUUGGAGGAG | 20 | 2087 |
| myoC-6889 | - | UCUUUUCUCUGCUUGGAGGAG | 21 | 6635 |
| myoC-6890 | - | AUCUUUUCUCUGCUUGGAGGAG | 22 | 6636 |
| myoC-6891 | - | UAUCUUUUCUCUGCUUGGAGGAG | 23 | 6637 |
| myoC-6892 | - | UUAUCUUUUCUCUGCUUGGAGGAG | 24 | 6638 |
| myoC-6893 | - | GGUGGGGACUGCAGGGAG | 18 | 6639 |
| myoC-6894 | - | AGGUGGGGACUGCAGGGAG | 19 | 6640 |
| myoC-985 | - | GAGGUGGGGACUGCAGGGAG | 20 | 1285 |
| myoC-6895 | - | GGAGGUGGGGACUGCAGGGAG | 21 | 6641 |
| myoC-6896 | - | AGGAGGUGGGGACUGCAGGGAG | 22 | 6642 |
| myoC-6897 | - | CAGGAGGUGGGGACUGCAGGGAG | 23 | 6643 |
| myoC-6898 | - | CCAGGAGGUGGGGACUGCAGGGAG | 24 | 6644 |
| myoC-3663 | - | GAGGGGCUGCAGAGGGAG | 18 | 3409 |
| myoC-3664 | - | GGAGGGGCUGCAGAGGGAG | 19 | 3410 |
| myoC-1625 | - | AGGAGGGGCUGCAGAGGGAG | 20 | 1898 |
| myoC-3665 | - | CAGGAGGGGCUGCAGAGGGAG | 21 | 3411 |
| myoC-3666 | - | CCAGGAGGGGCUGCAGAGGGAG | 22 | 3412 |
| myoC-3667 | - | CCCAGGAGGGGCUGCAGAGGGAG | 23 | 3413 |
| myoC-3668 | - | ACCCAGGAGGGGCUGCAGAGGGAG | 24 | 3414 |
| myoC-3669 | - | GGCACCCUGAGGCGGGAG | 18 | 3415 |
| myoC-3670 | - | GGGCACCCUGAGGCGGGAG | 19 | 3416 |
| myoC-190 | - | UGGGCACCCUGAGGCGGGAG | 20 | 576 |
| myoC-3671 | - | CUGGGCACCCUGAGGCGGGAG | 21 | 3417 |
| myoC-3672 | - | GCUGGGCACCCUGAGGCGGGAG | 22 | 3418 |
| myoC-3673 | - | AGCUGGGCACCCUGAGGCGGGAG | 23 | 3419 |
| myoC-3674 | - | GAGCUGGGCACCCUGAGGCGGGAG | 24 | 3420 |
| myoC-6899 | - | UCCAGAAAGGAAAUGGAG | 18 | 6645 |
| myoC-6900 | - | CUCCAGAAAGGAAAUGGAG | 19 | 6646 |
| myoC-965 | - | GCUCCAGAAAGGAAAUGGAG | 20 | 1265 |
| myoC-6901 | - | GGCUCCAGAAAGGAAAUGGAG | 21 | 6647 |
| myoC-6902 | - | AGGCUCCAGAAAGGAAAUGGAG | 22 | 6648 |
| myoC-6903 | - | CAGGCUCCAGAAAGGAAAUGGAG | 23 | 6649 |
| myoC-6904 | - | CCAGGCUCCAGAAAGGAAAUGGAG | 24 | 6650 |
| myoC-6905 | - | UCUUUUCUCUGCUUGGAG | 18 | 6651 |
| myoC-6906 | - | AUCUUUUCUCUGCUUGGAG | 19 | 6652 |
| myoC-1902 | - | UAUCUUUUCUCUGCUUGGAG | 20 | 2086 |
| myoC-6907 | - | UUAUCUUUUCUCUGCUUGGAG | 21 | 6653 |
| myoC-6908 | - | UUUAUCUUUUCUCUGCUUGGAG | 22 | 6654 |
| myoC-6909 | - | UUUUAUCUUUUCUCUGCUUGGAG | 23 | 6655 |
| myoC-6910 | - | UUUUUAUCUUUUCUCUGCUUGGAG | 24 | 6656 |
| myoC-3675 | - | GGAGCUGGGCACCCUGAG | 18 | 3421 |
| myoC-3676 | - | GGGAGCUGGGCACCCUGAG | 19 | 3422 |
| myoC-1627 | - | AGGGAGCUGGGCACCCUGAG | 20 | 1900 |
| myoC-3677 | - | GAGGGAGCUGGGCACCCUGAG | 21 | 3423 |
| myoC-3678 | - | AGAGGGAGCUGGGCACCCUGAG | 22 | 3424 |
| myoC-3679 | - | CAGAGGGAGCUGGGCACCCUGAG | 23 | 3425 |
| myoC-3680 | - | GCAGAGGGAGCUGGGCACCCUGAG | 24 | 3426 |
| myoC-6911 | - | CGUCCUGGUGCAUCUGAG | 18 | 6657 |
| myoC-6912 | - | UCGUCCUGGUGCAUCUGAG | 19 | 6658 |
| myoC-1843 | - | AUCGUCCUGGUGCAUCUGAG | 20 | 2053 |
| myoC-6913 | - | AAUCGUCCUGGUGCAUCUGAG | 21 | 6659 |
| myoC-6914 | - | GAAUCGUCCUGGUGCAUCUGAG | 22 | 6660 |
| myoC-6915 | - | UGAAUCGUCCUGGUGCAUCUGAG | 23 | 6661 |
| myoC-6916 | - | GUGAAUCGUCCUGGUGCAUCUGAG | 24 | 6662 |
| myoC-6917 | - | CUGCAGGGAGUGGGGACG | 18 | 6663 |
| myoC-6918 | - | ACUGCAGGGAGUGGGGACG | 19 | 6664 |
| myoC-1882 | - | GACUGCAGGGAGUGGGGACG | 20 | 2073 |
| myoC-6919 | - | GGACUGCAGGGAGUGGGGACG | 21 | 6665 |
| myoC-6920 | - | GGGACUGCAGGGAGUGGGGACG | 22 | 6666 |
| myoC-6921 | - | GGGGACUGCAGGGAGUGGGGACG | 23 | 6667 |
| myoC-6922 | - | UGGGGACUGCAGGGAGUGGGGACG | 24 | 6668 |
| myoC-6923 | - | GUCACUGCCCUACCUUCG | 18 | 6669 |
| myoC-6924 | - | AGUCACUGCCCUACCUUCG | 19 | 6670 |
| myoC-690 | - | CAGUCACUGCCCUACCUUCG | 20 | 1100 |
| myoC-6925 | - | GCAGUCACUGCCCUACCUUCG | 21 | 6671 |
| myoC-6926 | - | AGCAGUCACUGCCCUACCUUCG | 22 | 6672 |
| myoC-6927 | - | AAGCAGUCACUGCCCUACCUUCG | 23 | 6673 |
| myoC-6928 | - | AAAGCAGUCACUGCCCUACCUUCG | 24 | 6674 |
| myoC-6929 | - | UGAGCGGGUGCUGAAAGG | 18 | 6675 |
| myoC-6930 | - | CUGAGCGGGUGCUGAAAGG | 19 | 6676 |
| myoC-1887 | - | GCUGAGCGGGUGCUGAAAGG | 20 | 2077 |
| myoC-6931 | - | GGCUGAGCGGGUGCUGAAAGG | 21 | 6677 |
| myoC-6932 | - | GGGCUGAGCGGGUGCUGAAAGG | 22 | 6678 |
| myoC-6933 | - | GGGGCUGAGCGGGUGCUGAAAGG | 23 | 6679 |
| myoC-6934 | - | UGGGGCUGAGCGGGUGCUGAAAGG | 24 | 6680 |
| myoC-6935 | - | AAGGUGAAAAGGGCAAGG | 18 | 6681 |
| myoC-6936 | - | GAAGGUGAAAAGGGCAAGG | 19 | 6682 |
| myoC-1891 | - | GGAAGGUGAAAAGGGCAAGG | 20 | 2080 |
| myoC-6937 | - | AGGAAGGUGAAAAGGGCAAGG | 21 | 6683 |
| myoC-6938 | - | CAGGAAGGUGAAAAGGGCAAGG | 22 | 6684 |
| myoC-6939 | - | GCAGGAAGGUGAAAAGGGCAAGG | 23 | 6685 |
| myoC-6940 | - | GGCAGGAAGGUGAAAAGGGCAAGG | 24 | 6686 |
| myoC-6941 | - | UGUGUGUGUAAAACCAGG | 18 | 6687 |
| myoC-6942 | - | GUGUGUGUGUAAAACCAGG | 19 | 6688 |
| myoC-836 | - | UGUGUGUGUGUAAAACCAGG | 20 | 1218 |
| myoC-6943 | - | GUGUGUGUGUGUAAAACCAGG | 21 | 6689 |
| myoC-6944 | - | UGUGUGUGUGUGUAAAACCAGG | 22 | 6690 |
| myoC-6945 | - | GUGUGUGUGUGUGUAAAACCAGG | 23 | 6691 |
| myoC-6946 | - | UGUGUGUGUGUGUGUAAAACCAGG | 24 | 6692 |
| myoC-3681 | - | GGCCCCAGGAGACCCAGG | 18 | 3427 |
| myoC-3682 | - | AGGCCCCAGGAGACCCAGG | 19 | 3428 |
| myoC-186 | - | CAGGCCCCAGGAGACCCAGG | 20 | 572 |
| myoC-3683 | - | CCAGGCCCCAGGAGACCCAGG | 21 | 3429 |
| myoC-3684 | - | GCCAGGCCCCAGGAGACCCAGG | 22 | 3430 |
| myoC-3685 | - | UGCCAGGCCCCAGGAGACCCAGG | 23 | 3431 |
| myoC-3686 | - | CUGCCAGGCCCCAGGAGACCCAGG | 24 | 3432 |
| myoC-6947 | - | CAGGAGAAUUCCAGGAGG | 18 | 6693 |
| myoC-6948 | - | CCAGGAGAAUUCCAGGAGG | 19 | 6694 |
| myoC-980 | - | UCCAGGAGAAUUCCAGGAGG | 20 | 1280 |
| myoC-6949 | - | GUCCAGGAGAAUUCCAGGAGG | 21 | 6695 |
| myoC-6950 | - | CGUCCAGGAGAAUUCCAGGAGG | 22 | 6696 |
| myoC-6951 | - | ACGUCCAGGAGAAUUCCAGGAGG | 23 | 6697 |
| myoC-6952 | - | CACGUCCAGGAGAAUUCCAGGAGG | 24 | 6698 |
| myoC-3693 | - | ACAAGUCAGUUCUGGAGG | 18 | 3439 |
| myoC-3694 | - | GACAAGUCAGUUCUGGAGG | 19 | 3440 |
| myoC-1643 | - | AGACAAGUCAGUUCUGGAGG | 20 | 1909 |
| myoC-3695 | - | GAGACAAGUCAGUUCUGGAGG | 21 | 3441 |
| myoC-3696 | - | CGAGACAAGUCAGUUCUGGAGG | 22 | 3442 |
| myoC-3697 | - | CCGAGACAAGUCAGUUCUGGAGG | 23 | 3443 |
| myoC-3698 | - | UCCGAGACAAGUCAGUUCUGGAGG | 24 | 3444 |
| myoC-3699 | - | GAGCUGGGCACCCUGAGG | 18 | 3445 |
| myoC-3700 | - | GGAGCUGGGCACCCUGAGG | 19 | 3446 |
| myoC-102 | - | GGGAGCUGGGCACCCUGAGG | 20 | 507 |
| myoC-3701 | - | AGGGAGCUGGGCACCCUGAGG | 21 | 3447 |
| myoC-3702 | - | GAGGGAGCUGGGCACCCUGAGG | 22 | 3448 |
| myoC-3703 | - | AGAGGGAGCUGGGCACCCUGAGG | 23 | 3449 |
| myoC-3704 | - | CAGAGGGAGCUGGGCACCCUGAGG | 24 | 3450 |
| myoC-6953 | - | UAGGCCGUUAAUUCACGG | 18 | 6699 |
| myoC-6954 | - | CUAGGCCGUUAAUUCACGG | 19 | 6700 |
| myoC-1918 | - | CCUAGGCCGUUAAUUCACGG | 20 | 2097 |
| myoC-6955 | - | UCCUAGGCCGUUAAUUCACGG | 21 | 6701 |
| myoC-6956 | - | UUCCUAGGCCGUUAAUUCACGG | 22 | 6702 |
| myoC-6957 | - | UUUCCUAGGCCGUUAAUUCACGG | 23 | 6703 |
| myoC-6958 | - | AUUUCCUAGGCCGUUAAUUCACGG | 24 | 6704 |
| myoC-6959 | - | UCAGUGUUGUUCACGGGG | 18 | 6705 |
| myoC-6960 | - | UUCAGUGUUGUUCACGGGG | 19 | 6706 |
| myoC-1894 | - | GUUCAGUGUUGUUCACGGGG | 20 | 2082 |
| myoC-6961 | - | UGUUCAGUGUUGUUCACGGGG | 21 | 6707 |
| myoC-6962 | - | AUGUUCAGUGUUGUUCACGGGG | 22 | 6708 |
| myoC-6963 | - | GAUGUUCAGUGUUGUUCACGGGG | 23 | 6709 |
| myoC-6964 | - | AGAUGUUCAGUGUUGUUCACGGGG | 24 | 6710 |
| myoC-6965 | - | GGAGUGGGGACGCUGGGG | 18 | 6711 |
| myoC-6966 | - | GGGAGUGGGGACGCUGGGG | 19 | 6712 |
| myoC-1884 | - | AGGGAGUGGGGACGCUGGGG | 20 | 2074 |
| myoC-6967 | - | CAGGGAGUGGGGACGCUGGGG | 21 | 6713 |
| myoC-6968 | - | GCAGGGAGUGGGGACGCUGGGG | 22 | 6714 |
| myoC-6969 | - | UGCAGGGAGUGGGGACGCUGGGG | 23 | 6715 |
| myoC-6970 | - | CUGCAGGGAGUGGGGACGCUGGGG | 24 | 6716 |
| myoC-6971 | - | GGUUUCCUCUCCAGCUGG | 18 | 6717 |
| myoC-6972 | - | AGGUUUCCUCUCCAGCUGG | 19 | 6718 |
| myoC-679 | - | GAGGUUUCCUCUCCAGCUGG | 20 | 1005 |
| myoC-6973 | - | AGAGGUUUCCUCUCCAGCUGG | 21 | 6719 |
| myoC-6974 | - | CAGAGGUUUCCUCUCCAGCUGG | 22 | 6720 |
| myoC-6975 | - | GCAGAGGUUUCCUCUCCAGCUGG | 23 | 6721 |
| myoC-6976 | - | GGCAGAGGUUUCCUCUCCAGCUGG | 24 | 6722 |
| myoC-6977 | - | GUCUAACGGAGAAUCUGG | 18 | 6723 |
| myoC-6978 | - | AGUCUAACGGAGAAUCUGG | 19 | 6724 |
| myoC-969 | - | UAGUCUAACGGAGAAUCUGG | 20 | 1269 |
| myoC-6979 | - | CUAGUCUAACGGAGAAUCUGG | 21 | 6725 |
| myoC-6980 | - | ACUAGUCUAACGGAGAAUCUGG | 22 | 6726 |
| myoC-6981 | - | AACUAGUCUAACGGAGAAUCUGG | 23 | 6727 |
| myoC-6982 | - | AAACUAGUCUAACGGAGAAUCUGG | 24 | 6728 |
| myoC-3705 | - | GAGACAAGUCAGUUCUGG | 18 | 3451 |
| myoC-3706 | - | CGAGACAAGUCAGUUCUGG | 19 | 3452 |
| myoC-192 | - | CCGAGACAAGUCAGUUCUGG | 20 | 578 |
| myoC-3707 | - | UCCGAGACAAGUCAGUUCUGG | 21 | 3453 |
| myoC-3708 | - | CUCCGAGACAAGUCAGUUCUGG | 22 | 3454 |
| myoC-3709 | - | CCUCCGAGACAAGUCAGUUCUGG | 23 | 3455 |
| myoC-3710 | - | UCCUCCGAGACAAGUCAGUUCUGG | 24 | 3456 |
| myoC-6983 | - | UAUCUUUUCUCUGCUUGG | 18 | 6729 |
| myoC-6984 | - | UUAUCUUUUCUCUGCUUGG | 19 | 6730 |
| myoC-1005 | - | UUUAUCUUUUCUCUGCUUGG | 20 | 1305 |
| myoC-6985 | - | UUUUAUCUUUUCUCUGCUUGG | 21 | 6731 |
| myoC-6986 | - | UUUUUAUCUUUUCUCUGCUUGG | 22 | 6732 |
| myoC-6987 | - | CUUUUUAUCUUUUCUCUGCUUGG | 23 | 6733 |
| myoC-6988 | - | CCUUUUUAUCUUUUCUCUGCUUGG | 24 | 6734 |
| myoC-6989 | - | GACACCAGAGACAAAAUG | 18 | 6735 |
| myoC-6990 | - | AGACACCAGAGACAAAAUG | 19 | 6736 |
| myoC-1825 | - | CAGACACCAGAGACAAAAUG | 20 | 2039 |
| myoC-6991 | - | CCAGACACCAGAGACAAAAUG | 21 | 6737 |
| myoC-6992 | - | GCCAGACACCAGAGACAAAAUG | 22 | 6738 |
| myoC-6993 | - | UGCCAGACACCAGAGACAAAAUG | 23 | 6739 |
| myoC-6994 | - | CUGCCAGACACCAGAGACAAAAUG | 24 | 6740 |
| myoC-6995 | - | GGCUCCAGAAAGGAAAUG | 18 | 6741 |
| myoC-6996 | - | AGGCUCCAGAAAGGAAAUG | 19 | 6742 |
| myoC-1850 | - | CAGGCUCCAGAAAGGAAAUG | 20 | 2058 |
| myoC-6997 | - | CCAGGCUCCAGAAAGGAAAUG | 21 | 6743 |
| myoC-6998 | - | UCCAGGCUCCAGAAAGGAAAUG | 22 | 6744 |
| myoC-6999 | - | CUCCAGGCUCCAGAAAGGAAAUG | 23 | 6745 |
| myoC-7000 | - | GCUCCAGGCUCCAGAAAGGAAAUG | 24 | 6746 |
| myoC-7001 | - | CCUCUGUCUUCCCCCAUG | 18 | 6747 |
| myoC-7002 | - | ACCUCUGUCUUCCCCCAUG | 19 | 6748 |
| myoC-2103 | - | CACCUCUGUCUUCCCCCAUG | 20 | 2226 |
| myoC-7003 | - | CCACCUCUGUCUUCCCCCAUG | 21 | 6749 |
| myoC-7004 | - | GCCACCUCUGUCUUCCCCCAUG | 22 | 6750 |
| myoC-7005 | - | GGCCACCUCUGUCUUCCCCCAUG | 23 | 6751 |
| myoC-7006 | - | UGGCCACCUCUGUCUUCCCCCAUG | 24 | 6752 |
| myoC-7007 | - | GAAGAAGUCUAUUUCAUG | 18 | 6753 |
| myoC-7008 | - | AGAAGAAGUCUAUUUCAUG | 19 | 6754 |
| myoC-1905 | - | GAGAAGAAGUCUAUUUCAUG | 20 | 2089 |
| myoC-7009 | - | GGAGAAGAAGUCUAUUUCAUG | 21 | 6755 |
| myoC-7010 | - | AGGAGAAGAAGUCUAUUUCAUG | 22 | 6756 |
| myoC-7011 | - | GAGGAGAAGAAGUCUAUUUCAUG | 23 | 6757 |
| myoC-7012 | - | GGAGGAGAAGAAGUCUAUUUCAUG | 24 | 6758 |
| myoC-3711 | - | CCUGCCUGGUGUGGGAUG | 18 | 3457 |
| myoC-3712 | - | GCCUGCCUGGUGUGGGAUG | 19 | 3458 |
| myoC-94 | - | GGCCUGCCUGGUGUGGGAUG | 20 | 499 |
| myoC-3713 | - | UGGCCUGCCUGGUGUGGGAUG | 21 | 3459 |
| myoC-3714 | - | CUGGCCUGCCUGGUGUGGGAUG | 22 | 3460 |
| myoC-3715 | - | UCUGGCCUGCCUGGUGUGGGAUG | 23 | 3461 |
| myoC-3716 | - | UUCUGGCCUGCCUGGUGUGGGAUG | 24 | 3462 |
| myoC-7013 | - | UCCAGGAGGUGGGGACUG | 18 | 6759 |
| myoC-7014 | - | UUCCAGGAGGUGGGGACUG | 19 | 6760 |
| myoC-1876 | - | AUUCCAGGAGGUGGGGACUG | 20 | 2071 |
| myoC-7015 | - | AAUUCCAGGAGGUGGGGACUG | 21 | 6761 |
| myoC-7016 | - | GAAUUCCAGGAGGUGGGGACUG | 22 | 6762 |
| myoC-7017 | - | AGAAUUCCAGGAGGUGGGGACUG | 23 | 6763 |
| myoC-7018 | - | GAGAAUUCCAGGAGGUGGGGACUG | 24 | 6764 |
| myoC-3717 | - | GCUCGACUCAGCUCCCUG | 18 | 3463 |
| myoC-3718 | - | AGCUCGACUCAGCUCCCUG | 19 | 3464 |
| myoC-1613 | - | AAGCUCGACUCAGCUCCCUG | 20 | 1891 |
| myoC-3719 | - | AAAGCUCGACUCAGCUCCCUG | 21 | 3465 |
| myoC-3720 | - | CAAAGCUCGACUCAGCUCCCUG | 22 | 3466 |
| myoC-3721 | - | CCAAAGCUCGACUCAGCUCCCUG | 23 | 3467 |
| myoC-3722 | - | ACCAAAGCUCGACUCAGCUCCCUG | 24 | 3468 |
| myoC-7019 | - | AGGUUUCCUCUCCAGCUG | 18 | 6765 |
| myoC-7020 | - | GAGGUUUCCUCUCCAGCUG | 19 | 6766 |
| myoC-678 | - | AGAGGUUUCCUCUCCAGCUG | 20 | 1085 |
| myoC-7021 | - | CAGAGGUUUCCUCUCCAGCUG | 21 | 6767 |
| myoC-7022 | - | GCAGAGGUUUCCUCUCCAGCUG | 22 | 6768 |
| myoC-7023 | - | GGCAGAGGUUUCCUCUCCAGCUG | 23 | 6769 |
| myoC-7024 | - | CGGCAGAGGUUUCCUCUCCAGCUG | 24 | 6770 |
| myoC-3723 | - | GAGACCCAGGAGGGGCUG | 18 | 3469 |
| myoC-3724 | - | GGAGACCCAGGAGGGGCUG | 19 | 3470 |
| myoC-1621 | - | AGGAGACCCAGGAGGGGCUG | 20 | 1896 |
| myoC-3725 | - | CAGGAGACCCAGGAGGGGCUG | 21 | 3471 |
| myoC-3726 | - | CCAGGAGACCCAGGAGGGGCUG | 22 | 3472 |
| myoC-3727 | - | CCCAGGAGACCCAGGAGGGGCUG | 23 | 3473 |
| myoC-3728 | - | CCCCAGGAGACCCAGGAGGGGCUG | 24 | 3474 |
| myoC-7025 | - | AGUCUAACGGAGAAUCUG | 18 | 6771 |
| myoC-7026 | - | UAGUCUAACGGAGAAUCUG | 19 | 6772 |
| myoC-1859 | - | CUAGUCUAACGGAGAAUCUG | 20 | 2063 |
| myoC-7027 | - | ACUAGUCUAACGGAGAAUCUG | 21 | 6773 |
| myoC-7028 | - | AACUAGUCUAACGGAGAAUCUG | 22 | 6774 |
| myoC-7029 | - | AAACUAGUCUAACGGAGAAUCUG | 23 | 6775 |
| myoC-7030 | - | GAAACUAGUCUAACGGAGAAUCUG | 24 | 6776 |
| myoC-3729 | - | CGAGACAAGUCAGUUCUG | 18 | 3475 |
| myoC-3730 | - | CCGAGACAAGUCAGUUCUG | 19 | 3476 |
| myoC-1641 | - | UCCGAGACAAGUCAGUUCUG | 20 | 1908 |
| myoC-3731 | - | CUCCGAGACAAGUCAGUUCUG | 21 | 3477 |
| myoC-3732 | - | CCUCCGAGACAAGUCAGUUCUG | 22 | 3478 |
| myoC-3733 | - | UCCUCCGAGACAAGUCAGUUCUG | 23 | 3479 |
| myoC-3734 | - | CUCCUCCGAGACAAGUCAGUUCUG | 24 | 3480 |
| myoC-7031 | - | GCCAACUUAAACCCAGUG | 18 | 6777 |
| myoC-7032 | - | AGCCAACUUAAACCCAGUG | 19 | 6778 |
| myoC-1832 | - | CAGCCAACUUAAACCCAGUG | 20 | 2044 |
| myoC-7033 | - | CCAGCCAACUUAAACCCAGUG | 21 | 6779 |
| myoC-7034 | - | GCCAGCCAACUUAAACCCAGUG | 22 | 6780 |
| myoC-7035 | - | AGCCAGCCAACUUAAACCCAGUG | 23 | 6781 |
| myoC-7036 | - | UAGCCAGCCAACUUAAACCCAGUG | 24 | 6782 |
| myoC-7037 | - | UUUCUCAUGGAAGACGUG | 18 | 6783 |
| myoC-7038 | - | GUUUCUCAUGGAAGACGUG | 19 | 6784 |
| myoC-1830 | - | AGUUUCUCAUGGAAGACGUG | 20 | 2042 |
| myoC-7039 | - | CAGUUUCUCAUGGAAGACGUG | 21 | 6785 |
| myoC-7040 | - | ACAGUUUCUCAUGGAAGACGUG | 22 | 6786 |
| myoC-7041 | - | GACAGUUUCUCAUGGAAGACGUG | 23 | 6787 |
| myoC-7042 | - | UGACAGUUUCUCAUGGAAGACGUG | 24 | 6788 |
| myoC-7043 | - | GCUGGGGCUGAGCGGGUG | 18 | 6789 |
| myoC-7044 | - | CGCUGGGGCUGAGCGGGUG | 19 | 6790 |
| myoC-1886 | - | ACGCUGGGGCUGAGCGGGUG | 20 | 2076 |
| myoC-7045 | - | GACGCUGGGGCUGAGCGGGUG | 21 | 6791 |
| myoC-7046 | - | GGACGCUGGGGCUGAGCGGGUG | 22 | 6792 |
| myoC-7047 | - | GGGACGCUGGGGCUGAGCGGGUG | 23 | 6793 |
| myoC-7048 | - | GGGGACGCUGGGGCUGAGCGGGUG | 24 | 6794 |
| myoC-7049 | - | ACUAGAAAUAUAUCCUUG | 18 | 6795 |
| myoC-7050 | - | AACUAGAAAUAUAUCCUUG | 19 | 6796 |
| myoC-2087 | - | AAACUAGAAAUAUAUCCUUG | 20 | 2215 |
| myoC-7051 | - | UAAACUAGAAAUAUAUCCUUG | 21 | 6797 |
| myoC-7052 | - | AUAAACUAGAAAUAUAUCCUUG | 22 | 6798 |
| myoC-7053 | - | UAUAAACUAGAAAUAUAUCCUUG | 23 | 6799 |
| myoC-7054 | - | AUAUAAACUAGAAAUAUAUCCUUG | 24 | 6800 |
| myoC-7055 | - | UUAUCUUUUCUCUGCUUG | 18 | 6801 |
| myoC-7056 | - | UUUAUCUUUUCUCUGCUUG | 19 | 6802 |
| myoC-1900 | - | UUUUAUCUUUUCUCUGCUUG | 20 | 2085 |
| myoC-7057 | - | UUUUUAUCUUUUCUCUGCUUG | 21 | 6803 |
| myoC-7058 | - | CUUUUUAUCUUUUCUCUGCUUG | 22 | 6804 |
| myoC-7059 | - | CCUUUUUAUCUUUUCUCUGCUUG | 23 | 6805 |
| myoC-7060 | - | GCCUUUUUAUCUUUUCUCUGCUUG | 24 | 6806 |
| myoC-7061 | - | ACUAGUCUAACGGAGAAU | 18 | 6807 |
| myoC-7062 | - | AACUAGUCUAACGGAGAAU | 19 | 6808 |
| myoC-1857 | - | AAACUAGUCUAACGGAGAAU | 20 | 2062 |
| myoC-7063 | - | GAAACUAGUCUAACGGAGAAU | 21 | 6809 |
| myoC-7064 | - | GGAAACUAGUCUAACGGAGAAU | 22 | 6810 |
| myoC-7065 | - | GGGAAACUAGUCUAACGGAGAAU | 23 | 6811 |
| myoC-7066 | - | AGGGAAACUAGUCUAACGGAGAAU | 24 | 6812 |
| myoC-7067 | - | UGAAUCGUCCUGGUGCAU | 18 | 6813 |
| myoC-7068 | - | GUGAAUCGUCCUGGUGCAU | 19 | 6814 |
| myoC-1842 | - | CGUGAAUCGUCCUGGUGCAU | 20 | 2052 |
| myoC-7069 | - | CCGUGAAUCGUCCUGGUGCAU | 21 | 6815 |
| myoC-7070 | - | CCCGUGAAUCGUCCUGGUGCAU | 22 | 6816 |
| myoC-7071 | - | UCCCGUGAAUCGUCCUGGUGCAU | 23 | 6817 |
| myoC-7072 | - | UUCCCGUGAAUCGUCCUGGUGCAU | 24 | 6818 |
| myoC-3735 | - | GCCUGCCUGGUGUGGGAU | 18 | 3481 |
| myoC-3736 | - | GGCCUGCCUGGUGUGGGAU | 19 | 3482 |
| myoC-1597 | - | UGGCCUGCCUGGUGUGGGAU | 20 | 1880 |
| myoC-3737 | - | CUGGCCUGCCUGGUGUGGGAU | 21 | 3483 |
| myoC-3738 | - | UCUGGCCUGCCUGGUGUGGGAU | 22 | 3484 |
| myoC-3739 | - | UUCUGGCCUGCCUGGUGUGGGAU | 23 | 3485 |
| myoC-3740 | - | CUUCUGGCCUGCCUGGUGUGGGAU | 24 | 3486 |
| myoC-7073 | - | UUUAUUUAAUGGGAAUAU | 18 | 6819 |
| myoC-7074 | - | CUUUAUUUAAUGGGAAUAU | 19 | 6820 |
| myoC-1015 | - | CCUUUAUUUAAUGGGAAUAU | 20 | 1315 |
| myoC-7075 | - | GCCUUUAUUUAAUGGGAAUAU | 21 | 6821 |
| myoC-7076 | - | GGCCUUUAUUUAAUGGGAAUAU | 22 | 6822 |
| myoC-7077 | - | AGGCCUUUAUUUAAUGGGAAUAU | 23 | 6823 |
| myoC-7078 | - | AAGGCCUUUAUUUAAUGGGAAUAU | 24 | 6824 |
| myoC-7079 | - | AAAACCAGGUGGAGAUAU | 18 | 6825 |
| myoC-7080 | - | UAAAACCAGGUGGAGAUAU | 19 | 6826 |
| myoC-837 | - | GUAAAACCAGGUGGAGAUAU | 20 | 994 |
| myoC-7081 | - | UGUAAAACCAGGUGGAGAUAU | 21 | 6827 |
| myoC-7082 | - | GUGUAAAACCAGGUGGAGAUAU | 22 | 6828 |
| myoC-7083 | - | UGUGUAAAACCAGGUGGAGAUAU | 23 | 6829 |
| myoC-7084 | - | GUGUGUAAAACCAGGUGGAGAUAU | 24 | 6830 |
| myoC-3741 | - | UGCCUACAGCAACCUCCU | 18 | 3487 |
| myoC-3742 | - | CUGCCUACAGCAACCUCCU | 19 | 3488 |
| myoC-1638 | - | ACUGCCUACAGCAACCUCCU | 20 | 1906 |
| myoC-3743 | - | GACUGCCUACAGCAACCUCCU | 21 | 3489 |
| myoC-3744 | - | AGACUGCCUACAGCAACCUCCU | 22 | 3490 |
| myoC-3745 | - | GAGACUGCCUACAGCAACCUCCU | 23 | 3491 |
| myoC-3746 | - | GGAGACUGCCUACAGCAACCUCCU | 24 | 3492 |
| myoC-7085 | - | AGUUUUCCGUUGCUUCCU | 18 | 6831 |
| myoC-7086 | - | GAGUUUUCCGUUGCUUCCU | 19 | 6832 |
| myoC-1897 | - | GGAGUUUUCCGUUGCUUCCU | 20 | 2083 |
| myoC-7087 | - | GGGAGUUUUCCGUUGCUUCCU | 21 | 6833 |
| myoC-7088 | - | UGGGAGUUUUCCGUUGCUUCCU | 22 | 6834 |
| myoC-7089 | - | CUGGGAGUUUUCCGUUGCUUCCU | 23 | 6835 |
| myoC-7090 | - | GCUGGGAGUUUUCCGUUGCUUCCU | 24 | 6836 |
| myoC-7091 | - | GAGGGGACAGUGUUUCCU | 18 | 6837 |
| myoC-7092 | - | GGAGGGGACAGUGUUUCCU | 19 | 6838 |
| myoC-1862 | - | UGGAGGGGACAGUGUUUCCU | 20 | 2064 |
| myoC-7093 | - | CUGGAGGGGACAGUGUUUCCU | 21 | 6839 |
| myoC-7094 | - | UCUGGAGGGGACAGUGUUUCCU | 22 | 6840 |
| myoC-7095 | - | AUCUGGAGGGGACAGUGUUUCCU | 23 | 6841 |
| myoC-7096 | - | AAUCUGGAGGGGACAGUGUUUCCU | 24 | 6842 |
| myoC-7097 | - | GAGGUUUCCUCUCCAGCU | 18 | 6843 |
| myoC-7098 | - | AGAGGUUUCCUCUCCAGCU | 19 | 6844 |
| myoC-677 | - | CAGAGGUUUCCUCUCCAGCU | 20 | 1097 |
| myoC-7099 | - | GCAGAGGUUUCCUCUCCAGCU | 21 | 6845 |
| myoC-7100 | - | GGCAGAGGUUUCCUCUCCAGCU | 22 | 6846 |
| myoC-7101 | - | CGGCAGAGGUUUCCUCUCCAGCU | 23 | 6847 |
| myoC-7102 | - | CCGGCAGAGGUUUCCUCUCCAGCU | 24 | 6848 |
| myoC-3747 | - | GUGCACGUUGCUGCAGCU | 18 | 3493 |
| myoC-3748 | - | UGUGCACGUUGCUGCAGCU | 19 | 3494 |
| myoC-1593 | - | CUGUGCACGUUGCUGCAGCU | 20 | 1877 |
| myoC-3749 | - | UCUGUGCACGUUGCUGCAGCU | 21 | 3495 |
| myoC-3750 | - | UUCUGUGCACGUUGCUGCAGCU | 22 | 3496 |
| myoC-3751 | - | CUUCUGUGCACGUUGCUGCAGCU | 23 | 3497 |
| myoC-3752 | - | UCUUCUGUGCACGUUGCUGCAGCU | 24 | 3498 |
| myoC-3753 | - | GGCCAGGACAGCUCAGCU | 18 | 3499 |
| myoC-3754 | - | GGGCCAGGACAGCUCAGCU | 19 | 3500 |
| myoC-1601 | - | GGGGCCAGGACAGCUCAGCU | 20 | 1882 |
| myoC-3755 | - | GGGGGCCAGGACAGCUCAGCU | 21 | 3501 |
| myoC-3756 | - | UGGGGGCCAGGACAGCUCAGCU | 22 | 3502 |
| myoC-3757 | - | GUGGGGGCCAGGACAGCUCAGCU | 23 | 3503 |
| myoC-3758 | - | UGUGGGGGCCAGGACAGCUCAGCU | 24 | 3504 |
| myoC-7103 | - | UUUUAUCUUUUCUCUGCU | 18 | 6849 |
| myoC-7104 | - | UUUUUAUCUUUUCUCUGCU | 19 | 6850 |
| myoC-1004 | - | CUUUUUAUCUUUUCUCUGCU | 20 | 1304 |
| myoC-7105 | - | CCUUUUUAUCUUUUCUCUGCU | 21 | 6851 |
| myoC-7106 | - | GCCUUUUUAUCUUUUCUCUGCU | 22 | 6852 |
| myoC-7107 | - | AGCCUUUUUAUCUUUUCUCUGCU | 23 | 6853 |
| myoC-7108 | - | GAGCCUUUUUAUCUUUUCUCUGCU | 24 | 6854 |
| myoC-7109 | - | CAGUAUAUAUAAACCUCU | 18 | 6855 |
| myoC-7110 | - | CCAGUAUAUAUAAACCUCU | 19 | 6856 |
| myoC-2104 | - | CCCAGUAUAUAUAAACCUCU | 20 | 2227 |
| myoC-7111 | - | CCCCAGUAUAUAUAAACCUCU | 21 | 6857 |
| myoC-7112 | - | UCCCCAGUAUAUAUAAACCUCU | 22 | 6858 |
| myoC-7113 | - | CUCCCCAGUAUAUAUAAACCUCU | 23 | 6859 |
| myoC-7114 | - | GCUCCCCAGUAUAUAUAAACCUCU | 24 | 6860 |
| myoC-7115 | - | GUUUUGUUAUCACUCUCU | 18 | 6861 |
| myoC-7116 | - | UGUUUUGUUAUCACUCUCU | 19 | 6862 |
| myoC-686 | - | UUGUUUUGUUAUCACUCUCU | 20 | 1124 |
| myoC-7117 | - | GUUGUUUUGUUAUCACUCUCU | 21 | 6863 |
| myoC-7118 | - | GGUUGUUUUGUUAUCACUCUCU | 22 | 6864 |
| myoC-7119 | - | UGGUUGUUUUGUUAUCACUCUCU | 23 | 6865 |
| myoC-7120 | - | CUGGUUGUUUUGUUAUCACUCUCU | 24 | 6866 |
| myoC-3759 | - | AAACCCAAACCAGAGAGU | 18 | 3505 |
| myoC-3760 | - | GAAACCCAAACCAGAGAGU | 19 | 3506 |
| myoC-106 | - | GGAAACCCAAACCAGAGAGU | 20 | 479 |
| myoC-3761 | - | UGGAAACCCAAACCAGAGAGU | 21 | 3507 |
| myoC-3762 | - | CUGGAAACCCAAACCAGAGAGU | 22 | 3508 |
| myoC-3763 | - | GCUGGAAACCCAAACCAGAGAGU | 23 | 3509 |
| myoC-3764 | - | AGCUGGAAACCCAAACCAGAGAGU | 24 | 3510 |
| myoC-7121 | - | GUGGGGACUGCAGGGAGU | 18 | 6867 |
| myoC-7122 | - | GGUGGGGACUGCAGGGAGU | 19 | 6868 |
| myoC-986 | - | AGGUGGGGACUGCAGGGAGU | 20 | 1286 |
| myoC-7123 | - | GAGGUGGGGACUGCAGGGAGU | 21 | 6869 |
| myoC-7124 | - | GGAGGUGGGGACUGCAGGGAGU | 22 | 6870 |
| myoC-7125 | - | AGGAGGUGGGGACUGCAGGGAGU | 23 | 6871 |
| myoC-7126 | - | CAGGAGGUGGGGACUGCAGGGAGU | 24 | 6872 |
| myoC-7127 | - | AGGAGAAUUCCAGGAGGU | 18 | 6873 |
| myoC-7128 | - | CAGGAGAAUUCCAGGAGGU | 19 | 6874 |
| myoC-981 | - | CCAGGAGAAUUCCAGGAGGU | 20 | 1281 |
| myoC-7129 | - | UCCAGGAGAAUUCCAGGAGGU | 21 | 6875 |
| myoC-7130 | - | GUCCAGGAGAAUUCCAGGAGGU | 22 | 6876 |
| myoC-7131 | - | CGUCCAGGAGAAUUCCAGGAGGU | 23 | 6877 |
| myoC-7132 | - | ACGUCCAGGAGAAUUCCAGGAGGU | 24 | 6878 |
| myoC-3771 | - | GCUUCUGGCCUGCCUGGU | 18 | 3517 |
| myoC-3772 | - | UGCUUCUGGCCUGCCUGGU | 19 | 3518 |
| myoC-1595 | - | CUGCUUCUGGCCUGCCUGGU | 20 | 1879 |
| myoC-3773 | - | GCUGCUUCUGGCCUGCCUGGU | 21 | 3519 |
| myoC-3774 | - | UGCUGCUUCUGGCCUGCCUGGU | 22 | 3520 |
| myoC-3775 | - | CUGCUGCUUCUGGCCUGCCUGGU | 23 | 3521 |
| myoC-3776 | - | GCUGCUGCUUCUGGCCUGCCUGGU | 24 | 3522 |
| myoC-3777 | - | CUGCCUGGUGUGGGAUGU | 18 | 3523 |
| myoC-3778 | - | CCUGCCUGGUGUGGGAUGU | 19 | 3524 |
| myoC-95 | - | GCCUGCCUGGUGUGGGAUGU | 20 | 500 |
| myoC-3779 | - | GGCCUGCCUGGUGUGGGAUGU | 21 | 3525 |
| myoC-3780 | - | UGGCCUGCCUGGUGUGGGAUGU | 22 | 3526 |
| myoC-3781 | - | CUGGCCUGCCUGGUGUGGGAUGU | 23 | 3527 |
| myoC-3782 | - | UCUGGCCUGCCUGGUGUGGGAUGU | 24 | 3528 |
| myoC-7133 | - | GAAACUCCAAACAGACUU | 18 | 6879 |
| myoC-7134 | - | AGAAACUCCAAACAGACUU | 19 | 6880 |
| myoC-2098 | - | AAGAAACUCCAAACAGACUU | 20 | 2222 |
| myoC-7135 | - | AAAGAAACUCCAAACAGACUU | 21 | 6881 |
| myoC-7136 | - | AAAAGAAACUCCAAACAGACUU | 22 | 6882 |
| myoC-7137 | - | AAAAAGAAACUCCAAACAGACUU | 23 | 6883 |
| myoC-7138 | - | UAAAAAGAAACUCCAAACAGACUU | 24 | 6884 |
| myoC-7139 | - | UCUUUUCUUUCAUGUCUU | 18 | 6885 |
| myoC-7140 | - | GUCUUUUCUUUCAUGUCUU | 19 | 6886 |
| myoC-1921 | - | AGUCUUUUCUUUCAUGUCUU | 20 | 2099 |
| myoC-7141 | - | GAGUCUUUUCUUUCAUGUCUU | 21 | 6887 |
| myoC-7142 | - | GGAGUCUUUUCUUUCAUGUCUU | 22 | 6888 |
| myoC-7143 | - | UGGAGUCUUUUCUUUCAUGUCUU | 23 | 6889 |
| myoC-7144 | - | CUGGAGUCUUUUCUUUCAUGUCUU | 24 | 6890 |
| myoC-3783 | - | CUCCGAGACAAGUCAGUU | 18 | 3529 |
| myoC-3784 | - | CCUCCGAGACAAGUCAGUU | 19 | 3530 |
| myoC-1639 | - | UCCUCCGAGACAAGUCAGUU | 20 | 1907 |
| myoC-3785 | - | CUCCUCCGAGACAAGUCAGUU | 21 | 3531 |
| myoC-3786 | - | CCUCCUCCGAGACAAGUCAGUU | 22 | 3532 |
| myoC-3787 | - | ACCUCCUCCGAGACAAGUCAGUU | 23 | 3533 |
| myoC-3788 | - | AACCUCCUCCGAGACAAGUCAGUU | 24 | 3534 |

**Table 10E** provides exemplary targeting domains for knocking down the *MYOC* gene selected according to the fifth tier parameters. The targeting domains bind within 2484-903bp upstream of transcription start site or the additional 500bp upstream and downstream of transcription start site (extending to lkb up and downstream of the transcription start site), start with a 5' G and PAM is NNGRRT. It is contemplated herein that in an embodiment the targeting domain hybridizes to the target domain through complementary base pairing. Any of the targeting domains in the table can be used with a S. aureus eiCas9 molecule or eiCas9 fusion protein (e.g., an eiCas9 fused to a transcription repressor domain) to alter the *MYOC* gene (e.g., reduce or eliminate *MYOC* gene expression, *MYOC* protein function, or the level *of MYOC* protein). One or more gRNA may be used to target an eiCas9 to the promoter region of the *MYOC* gene.

**Table 10E**

| 5th Tier | | | | |
|---|---|---|---|---|
| gRNA Name | DNA Strand | Targeting Domain | Target Site Length | Seq ID |
| myoC-7145 | + | GCAGAACCAGAAAGAAAA | 18 | 6891 |
| myoC-7146 | + | GGCAGAACCAGAAAGAAAA | 19 | 6892 |
| myoC-7147 | + | GUUUUCUUCCUGUUAAAAGAAA | 22 | 6893 |
| myoC-7148 | + | GCUAACUCCACAGAGAAA | 18 | 6894 |
| myoC-7149 | + | GCUGCUAACUCCACAGAGAAA | 21 | 6895 |
| myoC-7150 | + | GUGCUGCUAACUCCACAGAGAAA | 23 | 6896 |
| myoC-7151 | + | GAACUUGAGACAUUUACAA | 19 | 6897 |
| myoC-7152 | + | GCCUGAACUUGAGACAUUUACAA | 23 | 6898 |
| myoC-1173 | + | GUUUAUGGCUCUAUUCGCAA | 20 | 1473 |
| myoC-7153 | + | GAGUUUAUGGCUCUAUUCGCAA | 22 | 6899 |
| myoC-7154 | + | GUUUGUUUACAGCUGACCA | 19 | 6900 |
| myoC-7155 | + | GUGUUUGUUUACAGCUGACCA | 21 | 6901 |
| myoC-7156 | + | GGUGUUUGUUUACAGCUGACCA | 22 | 6902 |
| myoC-7157 | + | GGGUGUUUGUUUACAGCUGACCA | 23 | 6903 |
| myoC-7158 | + | GUCAAUUCCCACUGCCCUUGA | 21 | 6904 |
| myoC-7159 | + | GGUCAAUUCCCACUGCCCUUGA | 22 | 6905 |
| myoC-7160 | + | GUGGUCAAUUCCCACUGCCCUUGA | 24 | 6906 |
| myoC-7161 | + | GCCCUGCCUCCUAGAACC | 18 | 6907 |
| myoC-7162 | + | GGUCAAUUCCCACUGCCC | 18 | 6908 |
| myoC-2248 | + | GUGGUCAAUUCCCACUGCCC | 20 | 2332 |
| myoC-7163 | + | GCAUUGUGGCUCUCGGUCC | 19 | 6909 |
| myoC-7164 | + | GAAGCAUUGUGGCUCUCGGUCC | 22 | 6910 |
| myoC-7165 | + | GUUCACAGAACACGAGAGCUGC | 22 | 6911 |
| myoC-7166 | + | GUGUUCACAGAACACGAGAGCUGC | 24 | 6912 |
| myoC-7167 | + | GCCCUGGCAGACUCACCUC | 19 | 6913 |
| myoC-3801 | + | GCACAGCCCGAGCAGUGUC | 19 | 3547 |
| myoC-1700 | + | GGCACAGCCCGAGCAGUGUC | 20 | 1952 |
| myoC-3802 | + | GUGGCACAGCCCGAGCAGUGUC | 22 | 3548 |
| myoC-3803 | + | GGUGGCACAGCCCGAGCAGUGUC | 23 | 3549 |
| myoC-1199 | + | GCAGUCACUGCUGAGCUGCG | 20 | 1499 |
| myoC-7168 | + | GUCAGCAGUCACUGCUGAGCUGCG | 24 | 6914 |
| myoC-7169 | + | GCCAAGUCCACCACAGGG | 18 | 6915 |
| myoC-7170 | + | GCAUAAGCCAAGUCCACCACAGGG | 24 | 6916 |
| myoC-7171 | + | GGAAGGAAAAUGUGGCUG | 18 | 6917 |
| myoC-7172 | + | GGGAAGGAAAAUGUGGCUG | 19 | 6918 |
| myoC-7173 | + | GCUUAGGGAAGGAAAAUGUGGCUG | 24 | 6919 |
| myoC-7174 | + | GCCAUAUCACCUGCUGAACU | 20 | 6920 |
| myoC-7175 | + | GAGCCAUAUCACCUGCUGAACU | 22 | 6921 |
| myoC-7176 | + | GGUACUGUUAUUACCACU | 18 | 6922 |
| myoC-7177 | + | GUUACUACCUUGUGACUUGCU | 21 | 6923 |
| myoC-7178 | + | GCUGCGUGGGGUGCUGGU | 18 | 6924 |
| myoC-2243 | + | GAGCUGCGUGGGGUGCUGGU | 20 | 2328 |
| myoC-7179 | + | GCUGAGCUGCGUGGGGUGCUGGU | 23 | 6925 |
| myoC-7180 | + | GAAUCUGUUUGGCUUUACUCUU | 22 | 6926 |
| myoC-7181 | + | GUCUAAUUUCAAAGUAGUU | 19 | 6927 |
| myoC-2290 | + | GGUCUAAUUUCAAAGUAGUU | 20 | 2368 |
| myoC-7182 | + | GAGGUCUAAUUUCAAAGUAGUU | 22 | 6928 |
| myoC-7183 | + | GGAGGUCUAAUUUCAAAGUAGUU | 23 | 6929 |
| myoC-7184 | + | GGGUACUAGUCUCAUUUU | 18 | 6930 |
| myoC-7185 | - | GCAUUUGCCAAUAACCAAA | 19 | 6931 |
| myoC-1969 | - | GGCAUUUGCCAAUAACCAAA | 20 | 2127 |
| myoC-7186 | - | GAACCAAUCAAAUAAGAA | 18 | 6932 |
| myoC-7187 | - | GCAGAACCAAUCAAAUAAGAA | 21 | 6933 |
| myoC-2059 | - | GUUCUUGGCAUGCACACACA | 20 | 2190 |
| myoC-7188 | - | GGUUCUUGGCAUGCACACACA | 21 | 6934 |
| myoC-7189 | - | GAGGUUCUUGGCAUGCACACACA | 23 | 6935 |
| myoC-7190 | - | GCAGUGACUGCUGACAGCA | 19 | 6936 |
| myoC-7191 | - | GCUCAGCAGUGACUGCUGACAGCA | 24 | 6937 |
| myoC-7192 | - | GCAAAAGGAGAAAUAAAAGGA | 21 | 6938 |
| myoC-7193 | - | GCAGUGGGAAUUGACCAC | 18 | 6939 |
| myoC-7194 | - | GGCAGUGGGAAUUGACCAC | 19 | 6940 |
| myoC-1128 | - | GGGCAGUGGGAAUUGACCAC | 20 | 1428 |
| myoC-7195 | - | GGUUUAUUAAUGUAAAGC | 18 | 6941 |
| myoC-7196 | - | GGGUUUAUUAAUGUAAAGC | 19 | 6942 |
| myoC-7197 | - | GAUUAUAGUCCACGUGAUC | 19 | 6943 |
| myoC-1998 | - | GGAUUAUAGUCCACGUGAUC | 20 | 2146 |
| myoC-7198 | - | GGGAUUAUAGUCCACGUGAUC | 21 | 6944 |
| myoC-1962 | - | GACAGGAAGGCAGGCAGAAG | 20 | 2121 |
| myoC-7199 | - | GGACAGGAAGGCAGGCAGAAG | 21 | 6945 |
| myoC-7200 | - | GGGACAGGAAGGCAGGCAGAAG | 22 | 6946 |
| myoC-7201 | - | GGGGACAGGAAGGCAGGCAGAAG | 23 | 6947 |
| myoC-7202 | - | GGGGGACAGGAAGGCAGGCAGAAG | 24 | 6948 |
| myoC-7203 | - | GCACAGCUAGCACAAGACAG | 20 | 6949 |
| myoC-7204 | - | GACUGCACAGCUAGCACAAGACAG | 24 | 6950 |
| myoC-7205 | - | GGAGGAGAAGAAAAAGAG | 18 | 6951 |
| myoC-7206 | - | GGGAGGAGAAGAAAAAGAG | 19 | 6952 |
| myoC-1122 | - | GGGGAGGAGAAGAAAAAGAG | 20 | 1422 |
| myoC-7207 | - | GCAGGGGAGGAGAAGAAAAAGAG | 23 | 6953 |
| myoC-7208 | - | GUGUUUCUCCACUCUGGAG | 19 | 6954 |
| myoC-7209 | - | GCUCUCCCUGGAGCCUGG | 18 | 6955 |
| myoC-7210 | - | GAAUGCUCUCCCUGGAGCCUGG | 22 | 6956 |
| myoC-7211 | - | GGAAUGCUCUCCCUGGAGCCUGG | 23 | 6957 |
| myoC-3851 | - | GCUCCAGAGAAGGUAAGAAUG | 21 | 3597 |
| myoC-3852 | - | GGCUCCAGAGAAGGUAAGAAUG | 22 | 3598 |
| myoC-3210 | - | GCGACUAAGGCAAGAAAAU | 19 | 2956 |
| myoC-3211 | - | GAAGCGACUAAGGCAAGAAAAU | 22 | 2957 |
| myoC-7212 | - | GCUUAACUGCAGAACCAAUCAAAU | 24 | 6958 |
| myoC-7213 | - | GUCCAGAAAGCCUGUGAAU | 19 | 6959 |
| myoC-7214 | - | GAAAUCUGCCGCUUCUAU | 18 | 6960 |
| myoC-7215 | - | GGAAAUCUGCCGCUUCUAU | 19 | 6961 |
| myoC-1210 | - | GGGAAAUCUGCCGCUUCUAU | 20 | 1510 |
| myoC-7216 | - | GGGGAAAUCUGCCGCUUCUAU | 21 | 6962 |
| myoC-7217 | - | GGGGGAAAUCUGCCGCUUCUAU | 22 | 6963 |
| myoC-7218 | - | GGGGGGAAAUCUGCCGCUUCUAU | 23 | 6964 |
| myoC-3853 | - | GAAUGCAGAGUGGGGGGACU | 20 | 3599 |
| myoC-3854 | - | GUAAGAAUGCAGAGUGGGGGGACU | 24 | 3600 |
| myoC-7219 | - | GCAAGACGGUCGAAAACCU | 19 | 6965 |
| myoC-7220 | - | GAUACACAGUUGUUUUAAAGCU | 22 | 6966 |
| myoC-7221 | - | GCUUUUUGUUUUUUCUCU | 18 | 6967 |
| myoC-7222 | - | GAUUCAUUCAAGGGCAGU | 18 | 6968 |
| myoC-7223 | - | GACAGAUUCAUUCAAGGGCAGU | 22 | 6969 |
| myoC-3859 | - | GCCACCAGGCUCCAGAGAAGGU | 22 | 3605 |
| myoC-3860 | - | GUGCCACCAGGCUCCAGAGAAGGU | 24 | 3606 |
| myoC-7224 | - | GCUUCAUUUAGAUUAGUGGUU | 21 | 6970 |
| myoC-7225 | - | GAGCUUCAUUUAGAUUAGUGGUU | 23 | 6971 |

**Table 10F** provides exemplary targeting domains for knocking down the *MYOC* gene selected according to the six tier parameters. The targeting domains bind within 2484-903bp upstream of transcription start site or the additional 500bp upstream and downstream of transcription start site (extending to lkb up and downstream of the transcription start site) and PAM is NNGRRT. It is contemplated herein that in an embodiment the targeting domain hybridizes to the target domain through complementary base pairing. Any of the targeting domains in the table can be used with a S. aureus eiCas9 molecule or eiCas9 fusion protein (e.g., an eiCas9 fused to a transcription repressor domain) to alter the MYOC gene (e.g., reduce or eliminate MYOC gene expression, MYOC protein function, or the level of MYOC protein). One or more gRNA may be used to target an eiCas9 to the promoter region of the MYOC gene.

**Table 10F**

| 6th Tier | | | | |
|---|---|---|---|---|
| gRNA Name | DNA Strand | Targeting Domain | Target Site Length | Seq ID |
| myoC-7226 | + | CUGAGCAAAGGUUCAAAA | 18 | 6972 |
| myoC-7227 | + | UCUGAGCAAAGGUUCAAAA | 19 | 6973 |
| myoC-7228 | + | AUCUGAGCAAAGGUUCAAAA | 20 | 6974 |
| myoC-7229 | + | AAUCUGAGCAAAGGUUCAAAA | 21 | 6975 |
| myoC-7230 | + | CAAUCUGAGCAAAGGUUCAAAA | 22 | 6976 |
| myoC-7231 | + | ACAAUCUGAGCAAAGGUUCAAAA | 23 | 6977 |
| myoC-7232 | + | AACAAUCUGAGCAAAGGUUCAAAA | 24 | 6978 |
| myoC-2206 | + | UGGCAGAACCAGAAAGAAAA | 20 | 2301 |
| myoC-7233 | + | AUGGCAGAACCAGAAAGAAAA | 21 | 6979 |
| myoC-7234 | + | AAUGGCAGAACCAGAAAGAAAA | 22 | 6980 |
| myoC-7235 | + | CAAUGGCAGAACCAGAAAGAAAA | 23 | 6981 |
| myoC-7236 | + | CCAAUGGCAGAACCAGAAAGAAAA | 24 | 6982 |
| myoC-7237 | + | UCUUCCUGUUAAAAGAAA | 18 | 6983 |
| myoC-7238 | + | UUCUUCCUGUUAAAAGAAA | 19 | 6984 |
| myoC-1190 | + | UUUCUUCCUGUUAAAAGAAA | 20 | 1490 |
| myoC-7239 | + | UUUUCUUCCUGUUAAAAGAAA | 21 | 6985 |
| myoC-7240 | + | UGUUUUCUUCCUGUUAAAAGAAA | 23 | 6986 |
| myoC-7241 | + | AUGUUUUCUUCCUGUUAAAAGAAA | 24 | 6987 |
| myoC-7242 | + | UGCUAACUCCACAGAGAAA | 19 | 6988 |
| myoC-2260 | + | CUGCUAACUCCACAGAGAAA | 20 | 2342 |
| myoC-7243 | + | UGCUGCUAACUCCACAGAGAAA | 22 | 6989 |
| myoC-7244 | + | UGUGCUGCUAACUCCACAGAGAAA | 24 | 6990 |
| myoC-7245 | + | AACUUGAGACAUUUACAA | 18 | 6991 |
| myoC-2272 | + | UGAACUUGAGACAUUUACAA | 20 | 2352 |
| myoC-7246 | + | CUGAACUUGAGACAUUUACAA | 21 | 6992 |
| myoC-7247 | + | CCUGAACUUGAGACAUUUACAA | 22 | 6993 |
| myoC-7248 | + | AGCCUGAACUUGAGACAUUUACAA | 24 | 6994 |
| myoC-7249 | + | UUAUGGCUCUAUUCGCAA | 18 | 6995 |
| myoC-7250 | + | UUUAUGGCUCUAUUCGCAA | 19 | 6996 |
| myoC-7251 | + | AGUUUAUGGCUCUAUUCGCAA | 21 | 6997 |
| myoC-7252 | + | UGAGUUUAUGGCUCUAUUCGCAA | 23 | 6998 |
| myoC-7253 | + | UUGAGUUUAUGGCUCUAUUCGCAA | 24 | 6999 |
| myoC-7254 | + | UCAACAUCCCCCCUCACA | 18 | 7000 |
| myoC-7255 | + | CUCAACAUCCCCCCUCACA | 19 | 7001 |
| myoC-2225 | + | UCUCAACAUCCCCCCUCACA | 20 | 2315 |
| myoC-7256 | + | CUCUCAACAUCCCCCCUCACA | 21 | 7002 |
| myoC-7257 | + | CCUCUCAACAUCCCCCCUCACA | 22 | 7003 |
| myoC-7258 | + | CCCUCUCAACAUCCCCCCUCACA | 23 | 7004 |
| myoC-7259 | + | CCCCUCUCAACAUCCCCCCUCACA | 24 | 7005 |
| myoC-7260 | + | UUUGUUUACAGCUGACCA | 18 | 7006 |
| myoC-2271 | + | UGUUUGUUUACAGCUGACCA | 20 | 2351 |
| myoC-7261 | + | UGGGUGUUUGUUUACAGCUGACCA | 24 | 7007 |
| myoC-7262 | + | AAUUCCCACUGCCCUUGA | 18 | 7008 |
| myoC-7263 | + | CAAUUCCCACUGCCCUUGA | 19 | 7009 |
| myoC-2247 | + | UCAAUUCCCACUGCCCUUGA | 20 | 2331 |
| myoC-7264 | + | UGGUCAAUUCCCACUGCCCUUGA | 23 | 7010 |
| myoC-7265 | + | AGCCCUGCCUCCUAGAACC | 19 | 7011 |
| myoC-2250 | + | UAGCCCUGCCUCCUAGAACC | 20 | 2334 |
| myoC-7266 | + | AUAGCCCUGCCUCCUAGAACC | 21 | 7012 |
| myoC-7267 | + | UAUAGCCCUGCCUCCUAGAACC | 22 | 7013 |
| myoC-7268 | + | AUAUAGCCCUGCCUCCUAGAACC | 23 | 7014 |
| myoC-7269 | + | AAUAUAGCCCUGCCUCCUAGAACC | 24 | 7015 |
| myoC-7270 | + | UGGUCAAUUCCCACUGCCC | 19 | 7016 |
| myoC-7271 | + | UGUGGUCAAUUCCCACUGCCC | 21 | 7017 |
| myoC-7272 | + | CUGUGGUCAAUUCCCACUGCCC | 22 | 7018 |
| myoC-7273 | + | CCUGUGGUCAAUUCCCACUGCCC | 23 | 7019 |
| myoC-7274 | + | CCCUGUGGUCAAUUCCCACUGCCC | 24 | 7020 |
| myoC-7275 | + | CAUUGUGGCUCUCGGUCC | 18 | 7021 |
| myoC-1081 | + | AGCAUUGUGGCUCUCGGUCC | 20 | 1381 |
| myoC-7276 | + | AAGCAUUGUGGCUCUCGGUCC | 21 | 7022 |
| myoC-7277 | + | UGAAGCAUUGUGGCUCUCGGUCC | 23 | 7023 |
| myoC-7278 | + | CUGAAGCAUUGUGGCUCUCGGUCC | 24 | 7024 |
| myoC-7279 | + | AGUCAGCAAGACCUAGGC | 18 | 7025 |
| myoC-7280 | + | UAGUCAGCAAGACCUAGGC | 19 | 7026 |
| myoC-2268 | + | AUAGUCAGCAAGACCUAGGC | 20 | 2348 |
| myoC-7281 | + | UAUAGUCAGCAAGACCUAGGC | 21 | 7027 |
| myoC-7282 | + | AUAUAGUCAGCAAGACCUAGGC | 22 | 7028 |
| myoC-7283 | + | CAUAUAGUCAGCAAGACCUAGGC | 23 | 7029 |
| myoC-7284 | + | UCAUAUAGUCAGCAAGACCUAGGC | 24 | 7030 |
| myoC-7285 | + | ACAGAACACGAGAGCUGC | 18 | 7031 |
| myoC-7286 | + | CACAGAACACGAGAGCUGC | 19 | 7032 |
| myoC-2218 | + | UCACAGAACACGAGAGCUGC | 20 | 2310 |
| myoC-7287 | + | UUCACAGAACACGAGAGCUGC | 21 | 7033 |
| myoC-7288 | + | UGUUCACAGAACACGAGAGCUGC | 23 | 7034 |
| myoC-7289 | + | CCCUGGCAGACUCACCUC | 18 | 7035 |
| myoC-2278 | + | UGCCCUGGCAGACUCACCUC | 20 | 2357 |
| myoC-7290 | + | CUGCCCUGGCAGACUCACCUC | 21 | 7036 |
| myoC-7291 | + | ACUGCCCUGGCAGACUCACCUC | 22 | 7037 |
| myoC-7292 | + | AACUGCCCUGGCAGACUCACCUC | 23 | 7038 |
| myoC-7293 | + | AAACUGCCCUGGCAGACUCACCUC | 24 | 7039 |
| myoC-3904 | + | CACAGCCCGAGCAGUGUC | 18 | 3650 |
| myoC-3905 | + | UGGCACAGCCCGAGCAGUGUC | 21 | 3651 |
| myoC-3906 | + | UGGUGGCACAGCCCGAGCAGUGUC | 24 | 3652 |
| myoC-7294 | + | AGUCACUGCUGAGCUGCG | 18 | 7040 |
| myoC-7295 | + | CAGUCACUGCUGAGCUGCG | 19 | 7041 |
| myoC-7296 | + | AGCAGUCACUGCUGAGCUGCG | 21 | 7042 |
| myoC-7297 | + | CAGCAGUCACUGCUGAGCUGCG | 22 | 7043 |
| myoC-7298 | + | UCAGCAGUCACUGCUGAGCUGCG | 23 | 7044 |
| myoC-7299 | + | AGCCAAGUCCACCACAGGG | 19 | 7045 |
| myoC-2204 | + | AAGCCAAGUCCACCACAGGG | 20 | 2299 |
| myoC-7300 | + | UAAGCCAAGUCCACCACAGGG | 21 | 7046 |
| myoC-7301 | + | AUAAGCCAAGUCCACCACAGGG | 22 | 7047 |
| myoC-7302 | + | CAUAAGCCAAGUCCACCACAGGG | 23 | 7048 |
| myoC-2235 | + | AGGGAAGGAAAAUGUGGCUG | 20 | 2323 |
| myoC-7303 | + | UAGGGAAGGAAAAUGUGGCUG | 21 | 7049 |
| myoC-7304 | + | UUAGGGAAGGAAAAUGUGGCUG | 22 | 7050 |
| myoC-7305 | + | CUUAGGGAAGGAAAAUGUGGCUG | 23 | 7051 |
| myoC-7306 | + | CAUAUCACCUGCUGAACU | 18 | 7052 |
| myoC-7307 | + | CCAUAUCACCUGCUGAACU | 19 | 7053 |
| myoC-7308 | + | AGCCAUAUCACCUGCUGAACU | 21 | 7054 |
| myoC-7309 | + | CGAGCCAUAUCACCUGCUGAACU | 23 | 7055 |
| myoC-7310 | + | ACGAGCCAUAUCACCUGCUGAACU | 24 | 7056 |
| myoC-7311 | + | AGGUACUGUUAUUACCACU | 19 | 7057 |
| myoC-2289 | + | CAGGUACUGUUAUUACCACU | 20 | 2367 |
| myoC-7312 | + | ACAGGUACUGUUAUUACCACU | 21 | 7058 |
| myoC-7313 | + | CACAGGUACUGUUAUUACCACU | 22 | 7059 |
| myoC-7314 | + | UCACAGGUACUGUUAUUACCACU | 23 | 7060 |
| myoC-7315 | + | AUCACAGGUACUGUUAUUACCACU | 24 | 7061 |
| myoC-7316 | + | ACUACCUUGUGACUUGCU | 18 | 7062 |
| myoC-7317 | + | UACUACCUUGUGACUUGCU | 19 | 7063 |
| myoC-2256 | + | UUACUACCUUGUGACUUGCU | 20 | 2339 |
| myoC-7318 | + | AGUUACUACCUUGUGACUUGCU | 22 | 7064 |
| myoC-7319 | + | CAGUUACUACCUUGUGACUUGCU | 23 | 7065 |
| myoC-7320 | + | UCAGUUACUACCUUGUGACUUGCU | 24 | 7066 |
| myoC-7321 | + | AGCUGCGUGGGGUGCUGGU | 19 | 7067 |
| myoC-7322 | + | UGAGCUGCGUGGGGUGCUGGU | 21 | 7068 |
| myoC-7323 | + | CUGAGCUGCGUGGGGUGCUGGU | 22 | 7069 |
| myoC-7324 | + | UGCUGAGCUGCGUGGGGUGCUGGU | 24 | 7070 |
| myoC-7325 | + | CUGUUUGGCUUUACUCUU | 18 | 7071 |
| myoC-7326 | + | UCUGUUUGGCUUUACUCUU | 19 | 7072 |
| myoC-1189 | + | AUCUGUUUGGCUUUACUCUU | 20 | 1489 |
| myoC-7327 | + | AAUCUGUUUGGCUUUACUCUU | 21 | 7073 |
| myoC-7328 | + | UGAAUCUGUUUGGCUUUACUCUU | 23 | 7074 |
| myoC-7329 | + | UUGAAUCUGUUUGGCUUUACUCUU | 24 | 7075 |
| myoC-7330 | + | UCUAAUUUCAAAGUAGUU | 18 | 7076 |
| myoC-7331 | + | AGGUCUAAUUUCAAAGUAGUU | 21 | 7077 |
| myoC-7332 | + | AGGAGGUCUAAUUUCAAAGUAGUU | 24 | 7078 |
| myoC-7333 | + | CUUGCUCUGGCCCAGUUU | 18 | 7079 |
| myoC-7334 | + | ACUUGCUCUGGCCCAGUUU | 19 | 7080 |
| myoC-2241 | + | CACUUGCUCUGGCCCAGUUU | 20 | 2326 |
| myoC-7335 | + | CCACUUGCUCUGGCCCAGUUU | 21 | 7081 |
| myoC-7336 | + | UCCACUUGCUCUGGCCCAGUUU | 22 | 7082 |
| myoC-7337 | + | UUCCACUUGCUCUGGCCCAGUUU | 23 | 7083 |
| myoC-7338 | + | UUUCCACUUGCUCUGGCCCAGUUU | 24 | 7084 |
| myoC-7339 | + | AGGGUACUAGUCUCAUUUU | 19 | 7085 |
| myoC-2270 | + | AAGGGUACUAGUCUCAUUUU | 20 | 2350 |
| myoC-7340 | + | AAAGGGUACUAGUCUCAUUUU | 21 | 7086 |
| myoC-7341 | + | CAAAGGGUACUAGUCUCAUUUU | 22 | 7087 |
| myoC-7342 | + | CCAAAGGGUACUAGUCUCAUUUU | 23 | 7088 |
| myoC-7343 | + | ACCAAAGGGUACUAGUCUCAUUUU | 24 | 7089 |
| myoC-7344 | - | CAUUUGCCAAUAACCAAA | 18 | 7090 |
| myoC-7345 | - | UGGCAUUUGCCAAUAACCAAA | 21 | 7091 |
| myoC-7346 | - | AUGGCAUUUGCCAAUAACCAAA | 22 | 7092 |
| myoC-7347 | - | AAUGGCAUUUGCCAAUAACCAAA | 23 | 7093 |
| myoC-7348 | - | CAAUGGCAUUUGCCAAUAACCAAA | 24 | 7094 |
| myoC-7349 | - | AGAACCAAUCAAAUAAGAA | 19 | 7095 |
| myoC-2031 | - | CAGAACCAAUCAAAUAAGAA | 20 | 2166 |
| myoC-7350 | - | UGCAGAACCAAUCAAAUAAGAA | 22 | 7096 |
| myoC-7351 | - | CUGCAGAACCAAUCAAAUAAGAA | 23 | 7097 |
| myoC-7352 | - | ACUGCAGAACCAAUCAAAUAAGAA | 24 | 7098 |
| myoC-7353 | - | UCUUGGCAUGCACACACA | 18 | 7099 |
| myoC-7354 | - | UUCUUGGCAUGCACACACA | 19 | 7100 |
| myoC-7355 | - | AGGUUCUUGGCAUGCACACACA | 22 | 7101 |
| myoC-7356 | - | UGAGGUUCUUGGCAUGCACACACA | 24 | 7102 |
| myoC-7357 | - | CAGUGACUGCUGACAGCA | 18 | 7103 |
| myoC-1117 | - | AGCAGUGACUGCUGACAGCA | 20 | 1417 |
| myoC-7358 | - | CAGCAGUGACUGCUGACAGCA | 21 | 7104 |
| myoC-7359 | - | UCAGCAGUGACUGCUGACAGCA | 22 | 7105 |
| myoC-7360 | - | CUCAGCAGUGACUGCUGACAGCA | 23 | 7106 |
| myoC-7361 | - | AAAGGAGAAAUAAAAGGA | 18 | 7107 |
| myoC-7362 | - | AAAAGGAGAAAUAAAAGGA | 19 | 7108 |
| myoC-7363 | - | CAAAAGGAGAAAUAAAAGGA | 20 | 7109 |
| myoC-7364 | - | AGCAAAAGGAGAAAUAAAAGGA | 22 | 7110 |
| myoC-7365 | - | UAGCAAAAGGAGAAAUAAAAGGA | 23 | 7111 |
| myoC-7366 | - | AUAGCAAAAGGAGAAAUAAAAGGA | 24 | 7112 |
| myoC-7367 | - | AGGGCAGUGGGAAUUGACCAC | 21 | 7113 |
| myoC-7368 | - | AAGGGCAGUGGGAAUUGACCAC | 22 | 7114 |
| myoC-7369 | - | CAAGGGCAGUGGGAAUUGACCAC | 23 | 7115 |
| myoC-7370 | - | UCAAGGGCAGUGGGAAUUGACCAC | 24 | 7116 |
| myoC-1168 | - | UGGGUUUAUUAAUGUAAAGC | 20 | 1468 |
| myoC-7371 | - | UUGGGUUUAUUAAUGUAAAGC | 21 | 7117 |
| myoC-7372 | - | UUUGGGUUUAUUAAUGUAAAGC | 22 | 7118 |
| myoC-7373 | - | CUUUGGGUUUAUUAAUGUAAAGC | 23 | 7119 |
| myoC-7374 | - | UCUUUGGGUUUAUUAAUGUAAAGC | 24 | 7120 |
| myoC-7375 | - | AUUAUAGUCCACGUGAUC | 18 | 7121 |
| myoC-7376 | - | AGGGAUUAUAGUCCACGUGAUC | 22 | 7122 |
| myoC-7377 | - | CAGGGAUUAUAGUCCACGUGAUC | 23 | 7123 |
| myoC-7378 | - | ACAGGGAUUAUAGUCCACGUGAUC | 24 | 7124 |
| myoC-7379 | - | AUAUUUUUCCUUUACAAG | 18 | 7125 |
| myoC-7380 | - | UAUAUUUUUCCUUUACAAG | 19 | 7126 |
| myoC-2014 | - | CUAUAUUUUUCCUUUACAAG | 20 | 2152 |
| myoC-7381 | - | ACUAUAUUUUUCCUUUACAAG | 21 | 7127 |
| myoC-7382 | - | UACUAUAUUUUUCCUUUACAAG | 22 | 7128 |
| myoC-7383 | - | AUACUAUAUUUUUCCUUUACAAG | 23 | 7129 |
| myoC-7384 | - | AAUACUAUAUUUUUCCUUUACAAG | 24 | 7130 |
| myoC-7385 | - | CAGGAAGGCAGGCAGAAG | 18 | 7131 |
| myoC-7386 | - | ACAGGAAGGCAGGCAGAAG | 19 | 7132 |
| myoC-7387 | - | ACAGCUAGCACAAGACAG | 18 | 7133 |
| myoC-7388 | - | CACAGCUAGCACAAGACAG | 19 | 7134 |
| myoC-7389 | - | UGCACAGCUAGCACAAGACAG | 21 | 7135 |
| myoC-7390 | - | CUGCACAGCUAGCACAAGACAG | 22 | 7136 |
| myoC-7391 | - | ACUGCACAGCUAGCACAAGACAG | 23 | 7137 |
| myoC-7392 | - | AGGGGAGGAGAAGAAAAAGAG | 21 | 7138 |
| myoC-7393 | - | CAGGGGAGGAGAAGAAAAAGAG | 22 | 7139 |
| myoC-7394 | - | CGCAGGGGAGGAGAAGAAAAAGAG | 24 | 7140 |
| myoC-7395 | - | UGUUUCUCCACUCUGGAG | 18 | 7141 |
| myoC-2035 | - | UGUGUUUCUCCACUCUGGAG | 20 | 2169 |
| myoC-7396 | - | CUGUGUUUCUCCACUCUGGAG | 21 | 7142 |
| myoC-7397 | - | ACUGUGUUUCUCCACUCUGGAG | 22 | 7143 |
| myoC-7398 | - | AACUGUGUUUCUCCACUCUGGAG | 23 | 7144 |
| myoC-7399 | - | AAACUGUGUUUCUCCACUCUGGAG | 24 | 7145 |
| myoC-7400 | - | UGAAAACAUCUUUCUGAG | 18 | 7146 |
| myoC-7401 | - | UUGAAAACAUCUUUCUGAG | 19 | 7147 |
| myoC-2057 | - | UUUGAAAACAUCUUUCUGAG | 20 | 2188 |
| myoC-7402 | - | AUUUGAAAACAUCUUUCUGAG | 21 | 7148 |
| myoC-7403 | - | UAUUUGAAAACAUCUUUCUGAG | 22 | 7149 |
| myoC-7404 | - | AUAUUUGAAAACAUCUUUCUGAG | 23 | 7150 |
| myoC-7405 | - | UAUAUUUGAAAACAUCUUUCUGAG | 24 | 7151 |
| myoC-7406 | - | CUGUGAUUCUCUGUGAGG | 18 | 7152 |
| myoC-7407 | - | CCUGUGAUUCUCUGUGAGG | 19 | 7153 |
| myoC-1038 | - | CCCUGUGAUUCUCUGUGAGG | 20 | 1338 |
| myoC-7408 | - | UCCCUGUGAUUCUCUGUGAGG | 21 | 7154 |
| myoC-7409 | - | UUCCCUGUGAUUCUCUGUGAGG | 22 | 7155 |
| myoC-7410 | - | CUUCCCUGUGAUUCUCUGUGAGG | 23 | 7156 |
| myoC-7411 | - | ACUUCCCUGUGAUUCUCUGUGAGG | 24 | 7157 |
| myoC-7412 | - | UGCUCUCCCUGGAGCCUGG | 19 | 7158 |
| myoC-2078 | - | AUGCUCUCCCUGGAGCCUGG | 20 | 2207 |
| myoC-7413 | - | AAUGCUCUCCCUGGAGCCUGG | 21 | 7159 |
| myoC-7414 | - | AGGAAUGCUCUCCCUGGAGCCUGG | 24 | 7160 |
| myoC-4035 | - | CCAGAGAAGGUAAGAAUG | 18 | 3781 |
| myoC-4036 | - | UCCAGAGAAGGUAAGAAUG | 19 | 3782 |
| myoC-4037 | - | CUCCAGAGAAGGUAAGAAUG | 20 | 3783 |
| myoC-4038 | - | AGGCUCCAGAGAAGGUAAGAAUG | 23 | 3784 |
| myoC-4039 | - | CAGGCUCCAGAGAAGGUAAGAAUG | 24 | 3785 |
| myoC-7415 | - | UUGAAAUUAGACCUCCUG | 18 | 7161 |
| myoC-7416 | - | UUUGAAAUUAGACCUCCUG | 19 | 7162 |
| myoC-2053 | - | CUUUGAAAUUAGACCUCCUG | 20 | 2184 |
| myoC-7417 | - | ACUUUGAAAUUAGACCUCCUG | 21 | 7163 |
| myoC-7418 | - | UACUUUGAAAUUAGACCUCCUG | 22 | 7164 |
| myoC-7419 | - | CUACUUUGAAAUUAGACCUCCUG | 23 | 7165 |
| myoC-7420 | - | ACUACUUUGAAAUUAGACCUCCUG | 24 | 7166 |
| myoC-7421 | - | AGGAACUCUUUUUCUCUG | 18 | 7167 |
| myoC-7422 | - | UAGGAACUCUUUUUCUCUG | 19 | 7168 |
| myoC-1148 | - | UUAGGAACUCUUUUUCUCUG | 20 | 1448 |
| myoC-7423 | - | AUUAGGAACUCUUUUUCUCUG | 21 | 7169 |
| myoC-7424 | - | UAUUAGGAACUCUUUUUCUCUG | 22 | 7170 |
| myoC-7425 | - | UUAUUAGGAACUCUUUUUCUCUG | 23 | 7171 |
| myoC-7426 | - | CUUAUUAGGAACUCUUUUUCUCUG | 24 | 7172 |
| myoC-3239 | - | CGACUAAGGCAAGAAAAU | 18 | 2985 |
| myoC-1648 | - | AGCGACUAAGGCAAGAAAAU | 20 | 1914 |
| myoC-3240 | - | AAGCGACUAAGGCAAGAAAAU | 21 | 2986 |
| myoC-3241 | - | AGAAGCGACUAAGGCAAGAAAAU | 23 | 2987 |
| myoC-3242 | - | AAGAAGCGACUAAGGCAAGAAAAU | 24 | 2988 |
| myoC-7427 | - | CUGCAGAACCAAUCAAAU | 18 | 7173 |
| myoC-7428 | - | ACUGCAGAACCAAUCAAAU | 19 | 7174 |
| myoC-2030 | - | AACUGCAGAACCAAUCAAAU | 20 | 2165 |
| myoC-7429 | - | UAACUGCAGAACCAAUCAAAU | 21 | 7175 |
| myoC-7430 | - | UUAACUGCAGAACCAAUCAAAU | 22 | 7176 |
| myoC-7431 | - | CUUAACUGCAGAACCAAUCAAAU | 23 | 7177 |
| myoC-7432 | - | UCCAGAAAGCCUGUGAAU | 18 | 7178 |
| myoC-2044 | - | AGUCCAGAAAGCCUGUGAAU | 20 | 2176 |
| myoC-7433 | - | CAGUCCAGAAAGCCUGUGAAU | 21 | 7179 |
| myoC-7434 | - | ACAGUCCAGAAAGCCUGUGAAU | 22 | 7180 |
| myoC-7435 | - | UACAGUCCAGAAAGCCUGUGAAU | 23 | 7181 |
| myoC-7436 | - | CUACAGUCCAGAAAGCCUGUGAAU | 24 | 7182 |
| myoC-7437 | - | AGGGGGGAAAUCUGCCGCUUCUAU | 24 | 7183 |
| myoC-4047 | - | AUGCAGAGUGGGGGGACU | 18 | 3793 |
| myoC-4048 | - | AAUGCAGAGUGGGGGGACU | 19 | 3794 |
| myoC-4049 | - | AGAAUGCAGAGUGGGGGGACU | 21 | 3795 |
| myoC-4050 | - | AAGAAUGCAGAGUGGGGGGACU | 22 | 3796 |
| myoC-4051 | - | UAAGAAUGCAGAGUGGGGGGACU | 23 | 3797 |
| myoC-7438 | - | CAAGACGGUCGAAAACCU | 18 | 7184 |
| myoC-1025 | - | UGCAAGACGGUCGAAAACCU | 20 | 1325 |
| myoC-7439 | - | AUGCAAGACGGUCGAAAACCU | 21 | 7185 |
| myoC-7440 | - | UAUGCAAGACGGUCGAAAACCU | 22 | 7186 |
| myoC-7441 | - | UUAUGCAAGACGGUCGAAAACCU | 23 | 7187 |
| myoC-7442 | - | CUUAUGCAAGACGGUCGAAAACCU | 24 | 7188 |
| myoC-7443 | - | CUACAGUCCAGAAAGCCU | 18 | 7189 |
| myoC-7444 | - | CCUACAGUCCAGAAAGCCU | 19 | 7190 |
| myoC-2043 | - | ACCUACAGUCCAGAAAGCCU | 20 | 2175 |
| myoC-7445 | - | AACCUACAGUCCAGAAAGCCU | 21 | 7191 |
| myoC-7446 | - | UAACCUACAGUCCAGAAAGCCU | 22 | 7192 |
| myoC-7447 | - | UUAACCUACAGUCCAGAAAGCCU | 23 | 7193 |
| myoC-7448 | - | AUUAACCUACAGUCCAGAAAGCCU | 24 | 7194 |
| myoC-7449 | - | CAGGAAGAAAACAUUCCU | 18 | 7195 |
| myoC-7450 | - | ACAGGAAGAAAACAUUCCU | 19 | 7196 |
| myoC-2025 | - | AACAGGAAGAAAACAUUCCU | 20 | 2160 |
| myoC-7451 | - | UAACAGGAAGAAAACAUUCCU | 21 | 7197 |
| myoC-7452 | - | UUAACAGGAAGAAAACAUUCCU | 22 | 7198 |
| myoC-7453 | - | UUUAACAGGAAGAAAACAUUCCU | 23 | 7199 |
| myoC-7454 | - | UUUUAACAGGAAGAAAACAUUCCU | 24 | 7200 |
| myoC-7455 | - | CACAGUUGUUUUAAAGCU | 18 | 7201 |
| myoC-7456 | - | ACACAGUUGUUUUAAAGCU | 19 | 7202 |
| myoC-2066 | - | UACACAGUUGUUUUAAAGCU | 20 | 2197 |
| myoC-7457 | - | AUACACAGUUGUUUUAAAGCU | 21 | 7203 |
| myoC-7458 | - | AGAUACACAGUUGUUUUAAAGCU | 23 | 7204 |
| myoC-7459 | - | AAGAUACACAGUUGUUUUAAAGCU | 24 | 7205 |
| myoC-7460 | - | UGCUUUUUGUUUUUUCUCU | 19 | 7206 |
| myoC-2039 | - | UUGCUUUUUGUUUUUUCUCU | 20 | 2172 |
| myoC-7461 | - | UUUGCUUUUUGUUUUUUCUCU | 21 | 7207 |
| myoC-7462 | - | AUUUGCUUUUUGUUUUUUCUCU | 22 | 7208 |
| myoC-7463 | - | CAUUUGCUUUUUGUUUUUUCUCU | 23 | 7209 |
| myoC-7464 | - | CCAUUUGCUUUUUGUUUUUUCUCU | 24 | 7210 |
| myoC-7465 | - | AGAUUCAUUCAAGGGCAGU | 19 | 7211 |
| myoC-1127 | - | CAGAUUCAUUCAAGGGCAGU | 20 | 1427 |
| myoC-7466 | - | ACAGAUUCAUUCAAGGGCAGU | 21 | 7212 |
| myoC-7467 | - | AGACAGAUUCAUUCAAGGGCAGU | 23 | 7213 |
| myoC-7468 | - | AAGACAGAUUCAUUCAAGGGCAGU | 24 | 7214 |
| myoC-4073 | - | CCAGGCUCCAGAGAAGGU | 18 | 3819 |
| myoC-4074 | - | ACCAGGCUCCAGAGAAGGU | 19 | 3820 |
| myoC-4075 | - | CACCAGGCUCCAGAGAAGGU | 20 | 3821 |
| myoC-4076 | - | CCACCAGGCUCCAGAGAAGGU | 21 | 3822 |
| myoC-4077 | - | UGCCACCAGGCUCCAGAGAAGGU | 23 | 3823 |
| myoC-7469 | - | UUAACAUUUUAUUCCAUU | 18 | 7215 |
| myoC-7470 | - | UUUAACAUUUUAUUCCAUU | 19 | 7216 |
| myoC-2048 | - | AUUUAACAUUUUAUUCCAUU | 20 | 2179 |
| myoC-7471 | - | AAUUUAACAUUUUAUUCCAUU | 21 | 7217 |
| myoC-7472 | - | AAAUUUAACAUUUUAUUCCAUU | 22 | 7218 |
| myoC-7473 | - | UAAAUUUAACAUUUUAUUCCAUU | 23 | 7219 |
| myoC-7474 | - | CUAAAUUUAACAUUUUAUUCCAUU | 24 | 7220 |
| myoC-7475 | - | UCAUUUAGAUUAGUGGUU | 18 | 7221 |
| myoC-7476 | - | UUCAUUUAGAUUAGUGGUU | 19 | 7222 |
| myoC-7477 | - | CUUCAUUUAGAUUAGUGGUU | 20 | 7223 |
| myoC-7478 | - | AGCUUCAUUUAGAUUAGUGGUU | 22 | 7224 |
| myoC-7479 | - | AGAGCUUCAUUUAGAUUAGUGGUU | 24 | 7225 |

**Table 10G** provides exemplary targeting domains for knocking down the *MYOC* gene selected according to the seven tier parameters. The targeting domains bind within 2484-903bp upstream of transcription start site or the additional 500bp upstream and downstream of transcription start site (extending to lkb up and downstream of the transcription start site) and PAM is NNGRRV. It is contemplated herein that in an embodiment the targeting domain hybridizes to the target domain through complementary base pairing. Any of the targeting domains in the table can be used with a S. aureus eiCas9 molecule or eiCas9 fusion protein (e.g., an eiCas9 fused to a transcription repressor domain) to alter the MYOC gene (e.g., reduce or eliminate MYOC gene expression, MYOC protein function, or the level of MYOC protein). One or more gRNA may be used to target an eiCas9 to the promoter region of the MYOC gene.

**Table 10G**

| 7th Tier | | | | |
|---|---|---|---|---|
| gRNA Name | DNA Strand | Targeting Domain | Target Site Length | Seq ID |
| myoC-7480 | + | UACAUUAAUAAACCCAAA | 18 | 7226 |
| myoC-7481 | + | UUACAUUAAUAAACCCAAA | 19 | 7227 |
| myoC-2283 | + | UUUACAUUAAUAAACCCAAA | 20 | 2362 |
| myoC-7482 | + | CUUUACAUUAAUAAACCCAAA | 21 | 7228 |
| myoC-7483 | + | GCUUUACAUUAAUAAACCCAAA | 22 | 7229 |
| myoC-7484 | + | UGCUUUACAUUAAUAAACCCAAA | 23 | 7230 |
| myoC-7485 | + | CUGCUUUACAUUAAUAAACCCAAA | 24 | 7231 |
| myoC-7486 | + | AAAGGAUAGUUUUUCAAA | 18 | 7232 |
| myoC-7487 | + | AAAAGGAUAGUUUUUCAAA | 19 | 7233 |
| myoC-5449 | + | AAAAAGGAUAGUUUUUCAAA | 20 | 5195 |
| myoC-7488 | + | AAAAAAGGAUAGUUUUUCAAA | 21 | 7234 |
| myoC-7489 | + | CAAAAAAGGAUAGUUUUUCAAA | 22 | 7235 |
| myoC-7490 | + | UCAAAAAAGGAUAGUUUUUCAAA | 23 | 7236 |
| myoC-7491 | + | UUCAAAAAAGGAUAGUUUUUCAAA | 24 | 7237 |
| myoC-7492 | + | AUAAAAUAUAGAUUACAA | 18 | 7238 |
| myoC-7493 | + | UAUAAAAUAUAGAUUACAA | 19 | 7239 |
| myoC-1227 | + | AUAUAAAAUAUAGAUUACAA | 20 | 1527 |
| myoC-7494 | + | UAUAUAAAAUAUAGAUUACAA | 21 | 7240 |
| myoC-7495 | + | AUAUAUAAAAUAUAGAUUACAA | 22 | 7241 |
| myoC-7496 | + | AAUAUAUAAAAUAUAGAUUACAA | 23 | 7242 |
| myoC-7497 | + | AAAUAUAUAAAAUAUAGAUUACAA | 24 | 7243 |
| myoC-7498 | + | AAAAGGAUAGUUUUUCAA | 18 | 7244 |
| myoC-7499 | + | AAAAAGGAUAGUUUUUCAA | 19 | 7245 |
| myoC-7500 | + | AAAAAAGGAUAGUUUUUCAA | 20 | 7246 |
| myoC-7501 | + | CAAAAAAGGAUAGUUUUUCAA | 21 | 7247 |
| myoC-7502 | + | UCAAAAAAGGAUAGUUUUUCAA | 22 | 7248 |
| myoC-7503 | + | UUCAAAAAAGGAUAGUUUUUCAA | 23 | 7249 |
| myoC-7504 | + | GUUCAAAAAAGGAUAGUUUUUCAA | 24 | 7250 |
| myoC-7505 | + | UUCUUCCUGUUAAAAGAA | 18 | 7251 |
| myoC-7506 | + | UUUCUUCCUGUUAAAAGAA | 19 | 7252 |
| myoC-2264 | + | UUUUCUUCCUGUUAAAAGAA | 20 | 2345 |
| myoC-7507 | + | GUUUUCUUCCUGUUAAAAGAA | 21 | 7253 |
| myoC-7508 | + | UGUUUUCUUCCUGUUAAAAGAA | 22 | 7254 |
| myoC-7509 | + | AUGUUUUCUUCCUGUUAAAAGAA | 23 | 7255 |
| myoC-7510 | + | AAUGUUUUCUUCCUGUUAAAAGAA | 24 | 7256 |
| myoC-7511 | + | UCUGAACCACUAAUCUAA | 18 | 7257 |
| myoC-7512 | + | CUCUGAACCACUAAUCUAA | 19 | 7258 |
| myoC-7513 | + | ACUCUGAACCACUAAUCUAA | 20 | 7259 |
| myoC-7514 | + | AACUCUGAACCACUAAUCUAA | 21 | 7260 |
| myoC-7515 | + | GAACUCUGAACCACUAAUCUAA | 22 | 7261 |
| myoC-7516 | + | AGAACUCUGAACCACUAAUCUAA | 23 | 7262 |
| myoC-7517 | + | AAGAACUCUGAACCACUAAUCUAA | 24 | 7263 |
| myoC-7518 | + | GAAUUACUCAGCUUGUAA | 18 | 7264 |
| myoC-7519 | + | AGAAUUACUCAGCUUGUAA | 19 | 7265 |
| myoC-1193 | + | CAGAAUUACUCAGCUUGUAA | 20 | 1493 |
| myoC-7520 | + | UCAGAAUUACUCAGCUUGUAA | 21 | 7266 |
| myoC-7521 | + | CUCAGAAUUACUCAGCUUGUAA | 22 | 7267 |
| myoC-7522 | + | GCUCAGAAUUACUCAGCUUGUAA | 23 | 7268 |
| myoC-7523 | + | UGCUCAGAAUUACUCAGCUUGUAA | 24 | 7269 |
| myoC-7524 | + | AUGUUUUCUUCCUGUUAA | 18 | 7270 |
| myoC-7525 | + | AAUGUUUUCUUCCUGUUAA | 19 | 7271 |
| myoC-2265 | + | GAAUGUUUUCUUCCUGUUAA | 20 | 2346 |
| myoC-7526 | + | GGAAUGUUUUCUUCCUGUUAA | 21 | 7272 |
| myoC-7527 | + | AGGAAUGUUUUCUUCCUGUUAA | 22 | 7273 |
| myoC-7528 | + | UAGGAAUGUUUUCUUCCUGUUAA | 23 | 7274 |
| myoC-7529 | + | UUAGGAAUGUUUUCUUCCUGUUAA | 24 | 7275 |
| myoC-7530 | + | UGUGCUGCUAACUCCACA | 18 | 7276 |
| myoC-7531 | + | UUGUGCUGCUAACUCCACA | 19 | 7277 |
| myoC-2261 | + | CUUGUGCUGCUAACUCCACA | 20 | 2343 |
| myoC-7532 | + | CCUUGUGCUGCUAACUCCACA | 21 | 7278 |
| myoC-7533 | + | CCCUUGUGCUGCUAACUCCACA | 22 | 7279 |
| myoC-7534 | + | GCCCUUGUGCUGCUAACUCCACA | 23 | 7280 |
| myoC-7535 | + | UGCCCUUGUGCUGCUAACUCCACA | 24 | 7281 |
| myoC-7536 | + | CCCUCACAGAGAAUCACA | 18 | 7282 |
| myoC-7537 | + | CCCCUCACAGAGAAUCACA | 19 | 7283 |
| myoC-1086 | + | CCCCCUCACAGAGAAUCACA | 20 | 1386 |
| myoC-7538 | + | CCCCCCUCACAGAGAAUCACA | 21 | 7284 |
| myoC-7539 | + | UCCCCCCUCACAGAGAAUCACA | 22 | 7285 |
| myoC-7540 | + | AUCCCCCCUCACAGAGAAUCACA | 23 | 7286 |
| myoC-7541 | + | CAUCCCCCCUCACAGAGAAUCACA | 24 | 7287 |
| myoC-7542 | + | GGACUGUGAAAACUGACA | 18 | 7288 |
| myoC-7543 | + | UGGACUGUGAAAACUGACA | 19 | 7289 |
| myoC-5454 | + | AUGGACUGUGAAAACUGACA | 20 | 5200 |
| myoC-7544 | + | UAUGGACUGUGAAAACUGACA | 21 | 7290 |
| myoC-7545 | + | CUAUGGACUGUGAAAACUGACA | 22 | 7291 |
| myoC-7546 | + | GCUAUGGACUGUGAAAACUGACA | 23 | 7292 |
| myoC-7547 | + | UGCUAUGGACUGUGAAAACUGACA | 24 | 7293 |
| myoC-7548 | + | UAUAAAAUAUAGAUUACA | 18 | 7294 |
| myoC-7549 | + | AUAUAAAAUAUAGAUUACA | 19 | 7295 |
| myoC-2295 | + | UAUAUAAAAUAUAGAUUACA | 20 | 2371 |
| myoC-7550 | + | AUAUAUAAAAUAUAGAUUACA | 21 | 7296 |
| myoC-7551 | + | AAUAUAUAAAAUAUAGAUUACA | 22 | 7297 |
| myoC-7552 | + | AAAUAUAUAAAAUAUAGAUUACA | 23 | 7298 |
| myoC-7553 | + | CAAAUAUAUAAAAUAUAGAUUACA | 24 | 7299 |
| myoC-7554 | + | CAUAAGCCAAGUCCACCA | 18 | 7300 |
| myoC-7555 | + | GCAUAAGCCAAGUCCACCA | 19 | 7301 |
| myoC-2205 | + | UGCAUAAGCCAAGUCCACCA | 20 | 2300 |
| myoC-7556 | + | UUGCAUAAGCCAAGUCCACCA | 21 | 7302 |
| myoC-7557 | + | CUUGCAUAAGCCAAGUCCACCA | 22 | 7303 |
| myoC-7558 | + | UCUUGCAUAAGCCAAGUCCACCA | 23 | 7304 |
| myoC-7559 | + | GUCUUGCAUAAGCCAAGUCCACCA | 24 | 7305 |
| myoC-7560 | + | UUUACAUUAAUAAACCCA | 18 | 7306 |
| myoC-7561 | + | CUUUACAUUAAUAAACCCA | 19 | 7307 |
| myoC-2284 | + | GCUUUACAUUAAUAAACCCA | 20 | 2363 |
| myoC-7562 | + | UGCUUUACAUUAAUAAACCCA | 21 | 7308 |
| myoC-7563 | + | CUGCUUUACAUUAAUAAACCCA | 22 | 7309 |
| myoC-7564 | + | CCUGCUUUACAUUAAUAAACCCA | 23 | 7310 |
| myoC-7565 | + | CCCUGCUUUACAUUAAUAAACCCA | 24 | 7311 |
| myoC-7566 | + | AGAGAAGACUAUGGCCCA | 18 | 7312 |
| myoC-7567 | + | CAGAGAAGACUAUGGCCCA | 19 | 7313 |
| myoC-1091 | + | GCAGAGAAGACUAUGGCCCA | 20 | 1391 |
| myoC-7568 | + | AGCAGAGAAGACUAUGGCCCA | 21 | 7314 |
| myoC-7569 | + | UAGCAGAGAAGACUAUGGCCCA | 22 | 7315 |
| myoC-7570 | + | AUAGCAGAGAAGACUAUGGCCCA | 23 | 7316 |
| myoC-7571 | + | UAUAGCAGAGAAGACUAUGGCCCA | 24 | 7317 |
| myoC-7572 | + | CUUGUGCUGCUAACUCCA | 18 | 7318 |
| myoC-7573 | + | CCUUGUGCUGCUAACUCCA | 19 | 7319 |
| myoC-2262 | + | CCCUUGUGCUGCUAACUCCA | 20 | 2344 |
| myoC-7574 | + | GCCCUUGUGCUGCUAACUCCA | 21 | 7320 |
| myoC-7575 | + | UGCCCUUGUGCUGCUAACUCCA | 22 | 7321 |
| myoC-7576 | + | UUGCCCUUGUGCUGCUAACUCCA | 23 | 7322 |
| myoC-7577 | + | AUUGCCCUUGUGCUGCUAACUCCA | 24 | 7323 |
| myoC-7578 | + | GCACCCUACCAGGCUCCA | 18 | 7324 |
| myoC-7579 | + | AGCACCCUACCAGGCUCCA | 19 | 7325 |
| myoC-1218 | + | CAGCACCCUACCAGGCUCCA | 20 | 1518 |
| myoC-7580 | + | ACAGCACCCUACCAGGCUCCA | 21 | 7326 |
| myoC-7581 | + | GACAGCACCCUACCAGGCUCCA | 22 | 7327 |
| myoC-7582 | + | GGACAGCACCCUACCAGGCUCCA | 23 | 7328 |
| myoC-7583 | + | AGGACAGCACCCUACCAGGCUCCA | 24 | 7329 |
| myoC-7584 | + | GCAAGGGUCUUUAUAGCA | 18 | 7330 |
| myoC-7585 | + | UGCAAGGGUCUUUAUAGCA | 19 | 7331 |
| myoC-2216 | + | CUGCAAGGGUCUUUAUAGCA | 20 | 2308 |
| myoC-7586 | + | GCUGCAAGGGUCUUUAUAGCA | 21 | 7332 |
| myoC-7587 | + | AGCUGCAAGGGUCUUUAUAGCA | 22 | 7333 |
| myoC-7588 | + | GAGCUGCAAGGGUCUUUAUAGCA | 23 | 7334 |
| myoC-7589 | + | AGAGCUGCAAGGGUCUUUAUAGCA | 24 | 7335 |
| myoC-7590 | + | UUUAUGGCUCUAUUCGCA | 18 | 7336 |
| myoC-7591 | + | GUUUAUGGCUCUAUUCGCA | 19 | 7337 |
| myoC-2288 | + | AGUUUAUGGCUCUAUUCGCA | 20 | 2366 |
| myoC-7592 | + | GAGUUUAUGGCUCUAUUCGCA | 21 | 7338 |
| myoC-7593 | + | UGAGUUUAUGGCUCUAUUCGCA | 22 | 7339 |
| myoC-7594 | + | UUGAGUUUAUGGCUCUAUUCGCA | 23 | 7340 |
| myoC-7595 | + | UUUGAGUUUAUGGCUCUAUUCGCA | 24 | 7341 |
| myoC-7596 | + | UAGGAGAAAGGGCAGGCA | 18 | 7342 |
| myoC-7597 | + | CUAGGAGAAAGGGCAGGCA | 19 | 7343 |
| myoC-5455 | + | UCUAGGAGAAAGGGCAGGCA | 20 | 5201 |
| myoC-7598 | + | CUCUAGGAGAAAGGGCAGGCA | 21 | 7344 |
| myoC-7599 | + | UCUCUAGGAGAAAGGGCAGGCA | 22 | 7345 |
| myoC-7600 | + | GUCUCUAGGAGAAAGGGCAGGCA | 23 | 7346 |
| myoC-7601 | + | AGUCUCUAGGAGAAAGGGCAGGCA | 24 | 7347 |
| myoC-7602 | + | CCCCCUCACAGAGAAUCA | 18 | 7348 |
| myoC-7603 | + | CCCCCCUCACAGAGAAUCA | 19 | 7349 |
| myoC-2224 | + | UCCCCCCUCACAGAGAAUCA | 20 | 2314 |
| myoC-7604 | + | AUCCCCCCUCACAGAGAAUCA | 21 | 7350 |
| myoC-7605 | + | CAUCCCCCCUCACAGAGAAUCA | 22 | 7351 |
| myoC-7606 | + | ACAUCCCCCCUCACAGAGAAUCA | 23 | 7352 |
| myoC-7607 | + | AACAUCCCCCCUCACAGAGAAUCA | 24 | 7353 |
| myoC-7608 | + | UGGAGUCUGACGUGAUCA | 18 | 7354 |
| myoC-7609 | + | CUGGAGUCUGACGUGAUCA | 19 | 7355 |
| myoC-2230 | + | CCUGGAGUCUGACGUGAUCA | 20 | 2319 |
| myoC-7610 | + | UCCUGGAGUCUGACGUGAUCA | 21 | 7356 |
| myoC-7611 | + | GUCCUGGAGUCUGACGUGAUCA | 22 | 7357 |
| myoC-7612 | + | GGUCCUGGAGUCUGACGUGAUCA | 23 | 7358 |
| myoC-7613 | + | CGGUCCUGGAGUCUGACGUGAUCA | 24 | 7359 |
| myoC-7614 | + | UCUCAACAUCCCCCCUCA | 18 | 7360 |
| myoC-7615 | + | CUCUCAACAUCCCCCCUCA | 19 | 7361 |
| myoC-2226 | + | CCUCUCAACAUCCCCCCUCA | 20 | 2316 |
| myoC-7616 | + | CCCUCUCAACAUCCCCCCUCA | 21 | 7362 |
| myoC-7617 | + | CCCCUCUCAACAUCCCCCCUCA | 22 | 7363 |
| myoC-7618 | + | UCCCCUCUCAACAUCCCCCCUCA | 23 | 7364 |
| myoC-7619 | + | UUCCCCUCUCAACAUCCCCCCUCA | 24 | 7365 |
| myoC-7620 | + | AUGUGGCUGUUGGGUUCA | 18 | 7366 |
| myoC-7621 | + | AAUGUGGCUGUUGGGUUCA | 19 | 7367 |
| myoC-2234 | + | AAAUGUGGCUGUUGGGUUCA | 20 | 2322 |
| myoC-7622 | + | AAAAUGUGGCUGUUGGGUUCA | 21 | 7368 |
| myoC-7623 | + | GAAAAUGUGGCUGUUGGGUUCA | 22 | 7369 |
| myoC-7624 | + | GGAAAAUGUGGCUGUUGGGUUCA | 23 | 7370 |
| myoC-7625 | + | AGGAAAAUGUGGCUGUUGGGUUCA | 24 | 7371 |
| myoC-7626 | + | AUCACAGGGAAGUGUUCA | 18 | 7372 |
| myoC-7627 | + | AAUCACAGGGAAGUGUUCA | 19 | 7373 |
| myoC-2221 | + | GAAUCACAGGGAAGUGUUCA | 20 | 2313 |
| myoC-7628 | + | AGAAUCACAGGGAAGUGUUCA | 21 | 7374 |
| myoC-7629 | + | GAGAAUCACAGGGAAGUGUUCA | 22 | 7375 |
| myoC-7630 | + | AGAGAAUCACAGGGAAGUGUUCA | 23 | 7376 |
| myoC-7631 | + | CAGAGAAUCACAGGGAAGUGUUCA | 24 | 7377 |
| myoC-7632 | + | ACCAAUGGCAGAACCAGA | 18 | 7378 |
| myoC-7633 | + | AACCAAUGGCAGAACCAGA | 19 | 7379 |
| myoC-2207 | + | CAACCAAUGGCAGAACCAGA | 20 | 2302 |
| myoC-7634 | + | CCAACCAAUGGCAGAACCAGA | 21 | 7380 |
| myoC-7635 | + | GCCAACCAAUGGCAGAACCAGA | 22 | 7381 |
| myoC-7636 | + | AGCCAACCAAUGGCAGAACCAGA | 23 | 7382 |
| myoC-7637 | + | CAGCCAACCAAUGGCAGAACCAGA | 24 | 7383 |
| myoC-7638 | + | GGCAGACUCACCUCCAGA | 18 | 7384 |
| myoC-7639 | + | UGGCAGACUCACCUCCAGA | 19 | 7385 |
| myoC-2277 | + | CUGGCAGACUCACCUCCAGA | 20 | 2356 |
| myoC-7640 | + | CCUGGCAGACUCACCUCCAGA | 21 | 7386 |
| myoC-7641 | + | CCCUGGCAGACUCACCUCCAGA | 22 | 7387 |
| myoC-7642 | + | GCCCUGGCAGACUCACCUCCAGA | 23 | 7388 |
| myoC-7643 | + | UGCCCUGGCAGACUCACCUCCAGA | 24 | 7389 |
| myoC-7644 | + | UGUGCAGUCUCUAGGAGA | 18 | 7390 |
| myoC-7645 | + | CUGUGCAGUCUCUAGGAGA | 19 | 7391 |
| myoC-7646 | + | GCUGUGCAGUCUCUAGGAGA | 20 | 7392 |
| myoC-7647 | + | AGCUGUGCAGUCUCUAGGAGA | 21 | 7393 |
| myoC-7648 | + | UAGCUGUGCAGUCUCUAGGAGA | 22 | 7394 |
| myoC-7649 | + | CUAGCUGUGCAGUCUCUAGGAGA | 23 | 7395 |
| myoC-7650 | + | GCUAGCUGUGCAGUCUCUAGGAGA | 24 | 7396 |
| myoC-7651 | + | AAGACUAUGGCCCAGGGA | 18 | 7397 |
| myoC-7652 | + | GAAGACUAUGGCCCAGGGA | 19 | 7398 |
| myoC-1092 | + | AGAAGACUAUGGCCCAGGGA | 20 | 1392 |
| myoC-7653 | + | GAGAAGACUAUGGCCCAGGGA | 21 | 7399 |
| myoC-7654 | + | AGAGAAGACUAUGGCCCAGGGA | 22 | 7400 |
| myoC-7655 | + | CAGAGAAGACUAUGGCCCAGGGA | 23 | 7401 |
| myoC-7656 | + | GCAGAGAAGACUAUGGCCCAGGGA | 24 | 7402 |
| myoC-7657 | + | AUUGUCUAUGCUUAGGGA | 18 | 7403 |
| myoC-7658 | + | CAUUGUCUAUGCUUAGGGA | 19 | 7404 |
| myoC-1075 | + | CCAUUGUCUAUGCUUAGGGA | 20 | 1375 |
| myoC-7659 | + | GCCAUUGUCUAUGCUUAGGGA | 21 | 7405 |
| myoC-7660 | + | UGCCAUUGUCUAUGCUUAGGGA | 22 | 7406 |
| myoC-7661 | + | AUGCCAUUGUCUAUGCUUAGGGA | 23 | 7407 |
| myoC-7662 | + | AAUGCCAUUGUCUAUGCUUAGGGA | 24 | 7408 |
| myoC-5871 | + | GUUGCCCAGAAGACAUGA | 18 | 5617 |
| myoC-5872 | + | AGUUGCCCAGAAGACAUGA | 19 | 5618 |
| myoC-2201 | + | UAGUUGCCCAGAAGACAUGA | 20 | 2296 |
| myoC-5873 | + | GUAGUUGCCCAGAAGACAUGA | 21 | 5619 |
| myoC-5874 | + | AGUAGUUGCCCAGAAGACAUGA | 22 | 5620 |
| myoC-5875 | + | GAGUAGUUGCCCAGAAGACAUGA | 23 | 5621 |
| myoC-5876 | + | UGAGUAGUUGCCCAGAAGACAUGA | 24 | 5622 |
| myoC-7663 | + | GUGAUCAGUGAGGACUGA | 18 | 7409 |
| myoC-7664 | + | CGUGAUCAGUGAGGACUGA | 19 | 7410 |
| myoC-1083 | + | ACGUGAUCAGUGAGGACUGA | 20 | 1383 |
| myoC-7665 | + | GACGUGAUCAGUGAGGACUGA | 21 | 7411 |
| myoC-7666 | + | UGACGUGAUCAGUGAGGACUGA | 22 | 7412 |
| myoC-7667 | + | CUGACGUGAUCAGUGAGGACUGA | 23 | 7413 |
| myoC-7668 | + | UCUGACGUGAUCAGUGAGGACUGA | 24 | 7414 |
| myoC-7669 | + | GAAAAAGAGUUCCUAAUA | 18 | 7415 |
| myoC-7670 | + | AGAAAAAGAGUUCCUAAUA | 19 | 7416 |
| myoC-1192 | + | GAGAAAAAGAGUUCCUAAUA | 20 | 1492 |
| myoC-7671 | + | AGAGAAAAAGAGUUCCUAAUA | 21 | 7417 |
| myoC-7672 | + | CAGAGAAAAAGAGUUCCUAAUA | 22 | 7418 |
| myoC-7673 | + | ACAGAGAAAAAGAGUUCCUAAUA | 23 | 7419 |
| myoC-7674 | + | CACAGAGAAAAAGAGUUCCUAAUA | 24 | 7420 |
| myoC-7675 | + | CAAAGGAAACAAAUGAUA | 18 | 7421 |
| myoC-7676 | + | ACAAAGGAAACAAAUGAUA | 19 | 7422 |
| myoC-2293 | + | UACAAAGGAAACAAAUGAUA | 20 | 2370 |
| myoC-7677 | + | UUACAAAGGAAACAAAUGAUA | 21 | 7423 |
| myoC-7678 | + | AUUACAAAGGAAACAAAUGAUA | 22 | 7424 |
| myoC-7679 | + | GAUUACAAAGGAAACAAAUGAUA | 23 | 7425 |
| myoC-7680 | + | AGAUUACAAAGGAAACAAAUGAUA | 24 | 7426 |
| myoC-7681 | + | CCAGGGAGAGCAUUCCUA | 18 | 7427 |
| myoC-7682 | + | UCCAGGGAGAGCAUUCCUA | 19 | 7428 |
| myoC-2307 | + | CUCCAGGGAGAGCAUUCCUA | 20 | 2378 |
| myoC-7683 | + | GCUCCAGGGAGAGCAUUCCUA | 21 | 7429 |
| myoC-7684 | + | GGCUCCAGGGAGAGCAUUCCUA | 22 | 7430 |
| myoC-7685 | + | AGGCUCCAGGGAGAGCAUUCCUA | 23 | 7431 |
| myoC-7686 | + | CAGGCUCCAGGGAGAGCAUUCCUA | 24 | 7432 |
| myoC-7687 | + | AGAAUUACUCAGCUUGUA | 18 | 7433 |
| myoC-7688 | + | CAGAAUUACUCAGCUUGUA | 19 | 7434 |
| myoC-2255 | + | UCAGAAUUACUCAGCUUGUA | 20 | 2338 |
| myoC-7689 | + | CUCAGAAUUACUCAGCUUGUA | 21 | 7435 |
| myoC-7690 | + | GCUCAGAAUUACUCAGCUUGUA | 22 | 7436 |
| myoC-7691 | + | UGCUCAGAAUUACUCAGCUUGUA | 23 | 7437 |
| myoC-7692 | + | UUGCUCAGAAUUACUCAGCUUGUA | 24 | 7438 |
| myoC-7693 | + | UGCCAUUGUCUAUGCUUA | 18 | 7439 |
| myoC-7694 | + | AUGCCAUUGUCUAUGCUUA | 19 | 7440 |
| myoC-1074 | + | AAUGCCAUUGUCUAUGCUUA | 20 | 1374 |
| myoC-7695 | + | AAAUGCCAUUGUCUAUGCUUA | 21 | 7441 |
| myoC-7696 | + | CAAAUGCCAUUGUCUAUGCUUA | 22 | 7442 |
| myoC-7697 | + | GCAAAUGCCAUUGUCUAUGCUUA | 23 | 7443 |
| myoC-7698 | + | GGCAAAUGCCAUUGUCUAUGCUUA | 24 | 7444 |
| myoC-7699 | + | GGGAAGUGUUCACAGAAC | 18 | 7445 |
| myoC-7700 | + | AGGGAAGUGUUCACAGAAC | 19 | 7446 |
| myoC-2220 | + | CAGGGAAGUGUUCACAGAAC | 20 | 2312 |
| myoC-7701 | + | ACAGGGAAGUGUUCACAGAAC | 21 | 7447 |
| myoC-7702 | + | CACAGGGAAGUGUUCACAGAAC | 22 | 7448 |
| myoC-7703 | + | UCACAGGGAAGUGUUCACAGAAC | 23 | 7449 |
| myoC-7704 | + | AUCACAGGGAAGUGUUCACAGAAC | 24 | 7450 |
| myoC-7705 | + | GCCAACCAAUGGCAGAAC | 18 | 7451 |
| myoC-7706 | + | AGCCAACCAAUGGCAGAAC | 19 | 7452 |
| myoC-2208 | + | CAGCCAACCAAUGGCAGAAC | 20 | 2303 |
| myoC-7707 | + | ACAGCCAACCAAUGGCAGAAC | 21 | 7453 |
| myoC-7708 | + | CACAGCCAACCAAUGGCAGAAC | 22 | 7454 |
| myoC-7709 | + | GCACAGCCAACCAAUGGCAGAAC | 23 | 7455 |
| myoC-7710 | + | CGCACAGCCAACCAAUGGCAGAAC | 24 | 7456 |
| myoC-7711 | + | CUGCAGUUAAGCCUGAAC | 18 | 7457 |
| myoC-7712 | + | UCUGCAGUUAAGCCUGAAC | 19 | 7458 |
| myoC-2273 | + | UUCUGCAGUUAAGCCUGAAC | 20 | 2353 |
| myoC-7713 | + | GUUCUGCAGUUAAGCCUGAAC | 21 | 7459 |
| myoC-7714 | + | GGUUCUGCAGUUAAGCCUGAAC | 22 | 7460 |
| myoC-7715 | + | UGGUUCUGCAGUUAAGCCUGAAC | 23 | 7461 |
| myoC-7716 | + | UUGGUUCUGCAGUUAAGCCUGAAC | 24 | 7462 |
| myoC-7717 | + | GAAGUGUUCACAGAACAC | 18 | 7463 |
| myoC-7718 | + | GGAAGUGUUCACAGAACAC | 19 | 7464 |
| myoC-2219 | + | GGGAAGUGUUCACAGAACAC | 20 | 2311 |
| myoC-7719 | + | AGGGAAGUGUUCACAGAACAC | 21 | 7465 |
| myoC-7720 | + | CAGGGAAGUGUUCACAGAACAC | 22 | 7466 |
| myoC-7721 | + | ACAGGGAAGUGUUCACAGAACAC | 23 | 7467 |
| myoC-7722 | + | CACAGGGAAGUGUUCACAGAACAC | 24 | 7468 |
| myoC-7723 | + | GAAGUAACUUUAAGCCAC | 18 | 7469 |
| myoC-7724 | + | AGAAGUAACUUUAAGCCAC | 19 | 7470 |
| myoC-2281 | + | CAGAAGUAACUUUAAGCCAC | 20 | 2360 |
| myoC-7725 | + | UCAGAAGUAACUUUAAGCCAC | 21 | 7471 |
| myoC-7726 | + | GUCAGAAGUAACUUUAAGCCAC | 22 | 7472 |
| myoC-7727 | + | UGUCAGAAGUAACUUUAAGCCAC | 23 | 7473 |
| myoC-7728 | + | CUGUCAGAAGUAACUUUAAGCCAC | 24 | 7474 |
| myoC-7729 | + | ACUGACAUGGAGGGGCAC | 18 | 7475 |
| myoC-7730 | + | AACUGACAUGGAGGGGCAC | 19 | 7476 |
| myoC-7731 | + | AAACUGACAUGGAGGGGCAC | 20 | 7477 |
| myoC-7732 | + | AAAACUGACAUGGAGGGGCAC | 21 | 7478 |
| myoC-7733 | + | GAAAACUGACAUGGAGGGGCAC | 22 | 7479 |
| myoC-7734 | + | UGAAAACUGACAUGGAGGGGCAC | 23 | 7480 |
| myoC-7735 | + | GUGAAAACUGACAUGGAGGGGCAC | 24 | 7481 |
| myoC-7736 | + | CCCCUCACAGAGAAUCAC | 18 | 7482 |
| myoC-7737 | + | CCCCCUCACAGAGAAUCAC | 19 | 7483 |
| myoC-1085 | + | CCCCCCUCACAGAGAAUCAC | 20 | 1385 |
| myoC-7738 | + | UCCCCCCUCACAGAGAAUCAC | 21 | 7484 |
| myoC-7739 | + | AUCCCCCCUCACAGAGAAUCAC | 22 | 7485 |
| myoC-7740 | + | CAUCCCCCCUCACAGAGAAUCAC | 23 | 7486 |
| myoC-7741 | + | ACAUCCCCCCUCACAGAGAAUCAC | 24 | 7487 |
| myoC-7742 | + | CCUAGAACCCAGGAUCAC | 18 | 7488 |
| myoC-7743 | + | UCCUAGAACCCAGGAUCAC | 19 | 7489 |
| myoC-2249 | + | CUCCUAGAACCCAGGAUCAC | 20 | 2333 |
| myoC-7744 | + | CCUCCUAGAACCCAGGAUCAC | 21 | 7490 |
| myoC-7745 | + | GCCUCCUAGAACCCAGGAUCAC | 22 | 7491 |
| myoC-7746 | + | UGCCUCCUAGAACCCAGGAUCAC | 23 | 7492 |
| myoC-7747 | + | CUGCCUCCUAGAACCCAGGAUCAC | 24 | 7493 |
| myoC-5955 | + | AGUAGUUGCCCAGAAGAC | 18 | 5701 |
| myoC-5956 | + | GAGUAGUUGCCCAGAAGAC | 19 | 5702 |
| myoC-2202 | + | UGAGUAGUUGCCCAGAAGAC | 20 | 2297 |
| myoC-7748 | + | CUGAGUAGUUGCCCAGAAGAC | 21 | 7494 |
| myoC-7749 | + | GCUGAGUAGUUGCCCAGAAGAC | 22 | 7495 |
| myoC-7750 | + | GGCUGAGUAGUUGCCCAGAAGAC | 23 | 7496 |
| myoC-7751 | + | GGGCUGAGUAGUUGCCCAGAAGAC | 24 | 7497 |
| myoC-7752 | + | UGGACUGUGAAAACUGAC | 18 | 7498 |
| myoC-7753 | + | AUGGACUGUGAAAACUGAC | 19 | 7499 |
| myoC-7754 | + | UAUGGACUGUGAAAACUGAC | 20 | 7500 |
| myoC-7755 | + | CUAUGGACUGUGAAAACUGAC | 21 | 7501 |
| myoC-7756 | + | GCUAUGGACUGUGAAAACUGAC | 22 | 7502 |
| myoC-7757 | + | UGCUAUGGACUGUGAAAACUGAC | 23 | 7503 |
| myoC-7758 | + | UUGCUAUGGACUGUGAAAACUGAC | 24 | 7504 |
| myoC-7759 | + | CUAAAUUACUAGUAAUAC | 18 | 7505 |
| myoC-7760 | + | GCUAAAUUACUAGUAAUAC | 19 | 7506 |
| myoC-7761 | + | AGCUAAAUUACUAGUAAUAC | 20 | 7507 |
| myoC-7762 | + | GAGCUAAAUUACUAGUAAUAC | 21 | 7508 |
| myoC-7763 | + | GGAGCUAAAUUACUAGUAAUAC | 22 | 7509 |
| myoC-7764 | + | AGGAGCUAAAUUACUAGUAAUAC | 23 | 7510 |
| myoC-7765 | + | CAGGAGCUAAAUUACUAGUAAUAC | 24 | 7511 |
| myoC-7766 | + | CAGAGAAGACUAUGGCCC | 18 | 7512 |
| myoC-7767 | + | GCAGAGAAGACUAUGGCCC | 19 | 7513 |
| myoC-1090 | + | AGCAGAGAAGACUAUGGCCC | 20 | 1390 |
| myoC-7768 | + | UAGCAGAGAAGACUAUGGCCC | 21 | 7514 |
| myoC-7769 | + | AUAGCAGAGAAGACUAUGGCCC | 22 | 7515 |
| myoC-7770 | + | UAUAGCAGAGAAGACUAUGGCCC | 23 | 7516 |
| myoC-7771 | + | UUAUAGCAGAGAAGACUAUGGCCC | 24 | 7517 |
| myoC-7772 | + | CAGGUCUCCCGACUUCCC | 18 | 7518 |
| myoC-7773 | + | UCAGGUCUCCCGACUUCCC | 19 | 7519 |
| myoC-2252 | + | AUCAGGUCUCCCGACUUCCC | 20 | 2336 |
| myoC-7774 | + | AAUCAGGUCUCCCGACUUCCC | 21 | 7520 |
| myoC-7775 | + | AAAUCAGGUCUCCCGACUUCCC | 22 | 7521 |
| myoC-7776 | + | GAAAUCAGGUCUCCCGACUUCCC | 23 | 7522 |
| myoC-7777 | + | AGAAAUCAGGUCUCCCGACUUCCC | 24 | 7523 |
| myoC-7778 | + | AGGGCAGGCAGGGAGGCC | 18 | 7524 |
| myoC-7779 | + | AAGGGCAGGCAGGGAGGCC | 19 | 7525 |
| myoC-5467 | + | AAAGGGCAGGCAGGGAGGCC | 20 | 5213 |
| myoC-7780 | + | GAAAGGGCAGGCAGGGAGGCC | 21 | 7526 |
| myoC-7781 | + | AGAAAGGGCAGGCAGGGAGGCC | 22 | 7527 |
| myoC-7782 | + | GAGAAAGGGCAGGCAGGGAGGCC | 23 | 7528 |
| myoC-7783 | + | GGAGAAAGGGCAGGCAGGGAGGCC | 24 | 7529 |
| myoC-7784 | + | GCAGAGAAGACUAUGGCC | 18 | 7530 |
| myoC-7785 | + | AGCAGAGAAGACUAUGGCC | 19 | 7531 |
| myoC-2215 | + | UAGCAGAGAAGACUAUGGCC | 20 | 2307 |
| myoC-7786 | + | AUAGCAGAGAAGACUAUGGCC | 21 | 7532 |
| myoC-7787 | + | UAUAGCAGAGAAGACUAUGGCC | 22 | 7533 |
| myoC-7788 | + | UUAUAGCAGAGAAGACUAUGGCC | 23 | 7534 |
| myoC-7789 | + | UUUAUAGCAGAGAAGACUAUGGCC | 24 | 7535 |
| myoC-7790 | + | UCUGUGUGUGUGCAUGCC | 18 | 7536 |
| myoC-7791 | + | CUCUGUGUGUGUGCAUGCC | 19 | 7537 |
| myoC-2302 | + | ACUCUGUGUGUGUGCAUGCC | 20 | 2375 |
| myoC-7792 | + | UACUCUGUGUGUGUGCAUGCC | 21 | 7538 |
| myoC-7793 | + | UUACUCUGUGUGUGUGCAUGCC | 22 | 7539 |
| myoC-7794 | + | CUUACUCUGUGUGUGUGCAUGCC | 23 | 7540 |
| myoC-7795 | + | UCUUACUCUGUGUGUGUGCAUGCC | 24 | 7541 |
| myoC-7796 | + | CAGGGCUGAGUAGUUGCC | 18 | 7542 |
| myoC-7797 | + | ACAGGGCUGAGUAGUUGCC | 19 | 7543 |
| myoC-2203 | + | CACAGGGCUGAGUAGUUGCC | 20 | 2298 |
| myoC-7798 | + | CCACAGGGCUGAGUAGUUGCC | 21 | 7544 |
| myoC-7799 | + | ACCACAGGGCUGAGUAGUUGCC | 22 | 7545 |
| myoC-7800 | + | CACCACAGGGCUGAGUAGUUGCC | 23 | 7546 |
| myoC-7801 | + | CCACCACAGGGCUGAGUAGUUGCC | 24 | 7547 |
| myoC-7802 | + | CAAUAUAGCCCUGCCUCC | 18 | 7548 |
| myoC-7803 | + | ACAAUAUAGCCCUGCCUCC | 19 | 7549 |
| myoC-2251 | + | CACAAUAUAGCCCUGCCUCC | 20 | 2335 |
| myoC-7804 | + | CCACAAUAUAGCCCUGCCUCC | 21 | 7550 |
| myoC-7805 | + | CCCACAAUAUAGCCCUGCCUCC | 22 | 7551 |
| myoC-7806 | + | CCCCACAAUAUAGCCCUGCCUCC | 23 | 7552 |
| myoC-7807 | + | CCCCCACAAUAUAGCCCUGCCUCC | 24 | 7553 |
| myoC-7808 | + | AGCACCCUACCAGGCUCC | 18 | 7554 |
| myoC-7809 | + | CAGCACCCUACCAGGCUCC | 19 | 7555 |
| myoC-1217 | + | ACAGCACCCUACCAGGCUCC | 20 | 1517 |
| myoC-7810 | + | GACAGCACCCUACCAGGCUCC | 21 | 7556 |
| myoC-7811 | + | GGACAGCACCCUACCAGGCUCC | 22 | 7557 |
| myoC-7812 | + | AGGACAGCACCCUACCAGGCUCC | 23 | 7558 |
| myoC-7813 | + | AAGGACAGCACCCUACCAGGCUCC | 24 | 7559 |
| myoC-7814 | + | UUGGUUCUGCAGUUAAGC | 18 | 7560 |
| myoC-7815 | + | AUUGGUUCUGCAGUUAAGC | 19 | 7561 |
| myoC-2274 | + | GAUUGGUUCUGCAGUUAAGC | 20 | 2354 |
| myoC-7816 | + | UGAUUGGUUCUGCAGUUAAGC | 21 | 7562 |
| myoC-7817 | + | UUGAUUGGUUCUGCAGUUAAGC | 22 | 7563 |
| myoC-7818 | + | UUUGAUUGGUUCUGCAGUUAAGC | 23 | 7564 |
| myoC-7819 | + | AUUUGAUUGGUUCUGCAGUUAAGC | 24 | 7565 |
| myoC-4259 | + | GGAGCCUGGUGGCACAGC | 18 | 4005 |
| myoC-4260 | + | UGGAGCCUGGUGGCACAGC | 19 | 4006 |
| myoC-1701 | + | CUGGAGCCUGGUGGCACAGC | 20 | 1953 |
| myoC-4261 | + | UCUGGAGCCUGGUGGCACAGC | 21 | 4007 |
| myoC-4262 | + | CUCUGGAGCCUGGUGGCACAGC | 22 | 4008 |
| myoC-4263 | + | UCUCUGGAGCCUGGUGGCACAGC | 23 | 4009 |
| myoC-4264 | + | UUCUCUGGAGCCUGGUGGCACAGC | 24 | 4010 |
| myoC-7820 | + | UUAAAAACAAGAUCCAGC | 18 | 7566 |
| myoC-7821 | + | GUUAAAAACAAGAUCCAGC | 19 | 7567 |
| myoC-1228 | + | UGUUAAAAACAAGAUCCAGC | 20 | 1528 |
| myoC-7822 | + | AUGUUAAAAACAAGAUCCAGC | 21 | 7568 |
| myoC-7823 | + | UAUGUUAAAAACAAGAUCCAGC | 22 | 7569 |
| myoC-7824 | + | AUAUGUUAAAAACAAGAUCCAGC | 23 | 7570 |
| myoC-7825 | + | AAUAUGUUAAAAACAAGAUCCAGC | 24 | 7571 |
| myoC-7826 | + | CUAGGAGAAAGGGCAGGC | 18 | 7572 |
| myoC-7827 | + | UCUAGGAGAAAGGGCAGGC | 19 | 7573 |
| myoC-5471 | + | CUCUAGGAGAAAGGGCAGGC | 20 | 5217 |
| myoC-7828 | + | UCUCUAGGAGAAAGGGCAGGC | 21 | 7574 |
| myoC-7829 | + | GUCUCUAGGAGAAAGGGCAGGC | 22 | 7575 |
| myoC-7830 | + | AGUCUCUAGGAGAAAGGGCAGGC | 23 | 7576 |
| myoC-7831 | + | CAGUCUCUAGGAGAAAGGGCAGGC | 24 | 7577 |
| myoC-7832 | + | AAGGGCAGGCAGGGAGGC | 18 | 7578 |
| myoC-7833 | + | AAAGGGCAGGCAGGGAGGC | 19 | 7579 |
| myoC-7834 | + | GAAAGGGCAGGCAGGGAGGC | 20 | 7580 |
| myoC-7835 | + | AGAAAGGGCAGGCAGGGAGGC | 21 | 7581 |
| myoC-7836 | + | GAGAAAGGGCAGGCAGGGAGGC | 22 | 7582 |
| myoC-7837 | + | GGAGAAAGGGCAGGCAGGGAGGC | 23 | 7583 |
| myoC-7838 | + | AGGAGAAAGGGCAGGCAGGGAGGC | 24 | 7584 |
| myoC-7839 | + | CAGUCACUGCUGAGCUGC | 18 | 7585 |
| myoC-7840 | + | GCAGUCACUGCUGAGCUGC | 19 | 7586 |
| myoC-2245 | + | AGCAGUCACUGCUGAGCUGC | 20 | 2329 |
| myoC-7841 | + | CAGCAGUCACUGCUGAGCUGC | 21 | 7587 |
| myoC-7842 | + | UCAGCAGUCACUGCUGAGCUGC | 22 | 7588 |
| myoC-7843 | + | GUCAGCAGUCACUGCUGAGCUGC | 23 | 7589 |
| myoC-7844 | + | UGUCAGCAGUCACUGCUGAGCUGC | 24 | 7590 |
| myoC-7845 | + | AACCUCAUUGGUGAAAUC | 18 | 7591 |
| myoC-7846 | + | GAACCUCAUUGGUGAAAUC | 19 | 7592 |
| myoC-1224 | + | AGAACCUCAUUGGUGAAAUC | 20 | 1524 |
| myoC-7847 | + | AAGAACCUCAUUGGUGAAAUC | 21 | 7593 |
| myoC-7848 | + | CAAGAACCUCAUUGGUGAAAUC | 22 | 7594 |
| myoC-7849 | + | CCAAGAACCUCAUUGGUGAAAUC | 23 | 7595 |
| myoC-7850 | + | GCCAAGAACCUCAUUGGUGAAAUC | 24 | 7596 |
| myoC-3315 | + | UCGCUUCUUCUCUUCCUC | 18 | 3061 |
| myoC-3316 | + | GUCGCUUCUUCUCUUCCUC | 19 | 3062 |
| myoC-1696 | + | AGUCGCUUCUUCUCUUCCUC | 20 | 1950 |
| myoC-3317 | + | UAGUCGCUUCUUCUCUUCCUC | 21 | 3063 |
| myoC-3318 | + | UUAGUCGCUUCUUCUCUUCCUC | 22 | 3064 |
| myoC-3319 | + | CUUAGUCGCUUCUUCUCUUCCUC | 23 | 3065 |
| myoC-3320 | + | CCUUAGUCGCUUCUUCUCUUCCUC | 24 | 3066 |
| myoC-7851 | + | CAGCACCCUACCAGGCUC | 18 | 7597 |
| myoC-7852 | + | ACAGCACCCUACCAGGCUC | 19 | 7598 |
| myoC-2311 | + | GACAGCACCCUACCAGGCUC | 20 | 2380 |
| myoC-7853 | + | GGACAGCACCCUACCAGGCUC | 21 | 7599 |
| myoC-7854 | + | AGGACAGCACCCUACCAGGCUC | 22 | 7600 |
| myoC-7855 | + | AAGGACAGCACCCUACCAGGCUC | 23 | 7601 |
| myoC-7856 | + | CAAGGACAGCACCCUACCAGGCUC | 24 | 7602 |
| myoC-7857 | + | AAUCUAAAUGAAGCUCUC | 18 | 7603 |
| myoC-7858 | + | UAAUCUAAAUGAAGCUCUC | 19 | 7604 |
| myoC-5474 | + | CUAAUCUAAAUGAAGCUCUC | 20 | 5220 |
| myoC-7859 | + | ACUAAUCUAAAUGAAGCUCUC | 21 | 7605 |
| myoC-7860 | + | CACUAAUCUAAAUGAAGCUCUC | 22 | 7606 |
| myoC-7861 | + | CCACUAAUCUAAAUGAAGCUCUC | 23 | 7607 |
| myoC-7862 | + | ACCACUAAUCUAAAUGAAGCUCUC | 24 | 7608 |
| myoC-7863 | + | UGCUAGCUGUGCAGUCUC | 18 | 7609 |
| myoC-7864 | + | GUGCUAGCUGUGCAGUCUC | 19 | 7610 |
| myoC-7865 | + | UGUGCUAGCUGUGCAGUCUC | 20 | 7611 |
| myoC-7866 | + | UUGUGCUAGCUGUGCAGUCUC | 21 | 7612 |
| myoC-7867 | + | CUUGUGCUAGCUGUGCAGUCUC | 22 | 7613 |
| myoC-7868 | + | UCUUGUGCUAGCUGUGCAGUCUC | 23 | 7614 |
| myoC-7869 | + | GUCUUGUGCUAGCUGUGCAGUCUC | 24 | 7615 |
| myoC-4331 | + | CUGCAUUCUUACCUUCUC | 18 | 4077 |
| myoC-4332 | + | UCUGCAUUCUUACCUUCUC | 19 | 4078 |
| myoC-3184 | + | CUCUGCAUUCUUACCUUCUC | 20 | 2930 |
| myoC-4333 | + | ACUCUGCAUUCUUACCUUCUC | 21 | 4079 |
| myoC-4334 | + | CACUCUGCAUUCUUACCUUCUC | 22 | 4080 |
| myoC-4335 | + | CCACUCUGCAUUCUUACCUUCUC | 23 | 4081 |
| myoC-4336 | + | CCCACUCUGCAUUCUUACCUUCUC | 24 | 4082 |
| myoC-7870 | + | GCAUUGUGGCUCUCGGUC | 18 | 7616 |
| myoC-7871 | + | AGCAUUGUGGCUCUCGGUC | 19 | 7617 |
| myoC-2232 | + | AAGCAUUGUGGCUCUCGGUC | 20 | 2320 |
| myoC-7872 | + | GAAGCAUUGUGGCUCUCGGUC | 21 | 7618 |
| myoC-7873 | + | UGAAGCAUUGUGGCUCUCGGUC | 22 | 7619 |
| myoC-7874 | + | CUGAAGCAUUGUGGCUCUCGGUC | 23 | 7620 |
| myoC-7875 | + | CCUGAAGCAUUGUGGCUCUCGGUC | 24 | 7621 |
| myoC-4343 | + | CGAGCAGUGUCUCGGGUC | 18 | 4089 |
| myoC-4344 | + | CCGAGCAGUGUCUCGGGUC | 19 | 4090 |
| myoC-203 | + | CCCGAGCAGUGUCUCGGGUC | 20 | 589 |
| myoC-4345 | + | GCCCGAGCAGUGUCUCGGGUC | 21 | 4091 |
| myoC-4346 | + | AGCCCGAGCAGUGUCUCGGGUC | 22 | 4092 |
| myoC-4347 | + | CAGCCCGAGCAGUGUCUCGGGUC | 23 | 4093 |
| myoC-4348 | + | ACAGCCCGAGCAGUGUCUCGGGUC | 24 | 4094 |
| myoC-7876 | + | UGGGUUCAUUGAGCUUUC | 18 | 7622 |
| myoC-7877 | + | UUGGGUUCAUUGAGCUUUC | 19 | 7623 |
| myoC-2233 | + | GUUGGGUUCAUUGAGCUUUC | 20 | 2321 |
| myoC-7878 | + | UGUUGGGUUCAUUGAGCUUUC | 21 | 7624 |
| myoC-7879 | + | CUGUUGGGUUCAUUGAGCUUUC | 22 | 7625 |
| myoC-7880 | + | GCUGUUGGGUUCAUUGAGCUUUC | 23 | 7626 |
| myoC-7881 | + | GGCUGUUGGGUUCAUUGAGCUUUC | 24 | 7627 |
| myoC-7882 | + | GACUAUGGCCCAGGGAAG | 18 | 7628 |
| myoC-7883 | + | AGACUAUGGCCCAGGGAAG | 19 | 7629 |
| myoC-2210 | + | AAGACUAUGGCCCAGGGAAG | 20 | 2305 |
| myoC-7884 | + | GAAGACUAUGGCCCAGGGAAG | 21 | 7630 |
| myoC-7885 | + | AGAAGACUAUGGCCCAGGGAAG | 22 | 7631 |
| myoC-7886 | + | GAGAAGACUAUGGCCCAGGGAAG | 23 | 7632 |
| myoC-7887 | + | AGAGAAGACUAUGGCCCAGGGAAG | 24 | 7633 |
| myoC-7888 | + | AAAAGAGUUCCUAAUAAG | 18 | 7634 |
| myoC-7889 | + | AAAAAGAGUUCCUAAUAAG | 19 | 7635 |
| myoC-2257 | + | GAAAAAGAGUUCCUAAUAAG | 20 | 2340 |
| myoC-7890 | + | AGAAAAAGAGUUCCUAAUAAG | 21 | 7636 |
| myoC-7891 | + | GAGAAAAAGAGUUCCUAAUAAG | 22 | 7637 |
| myoC-7892 | + | AGAGAAAAAGAGUUCCUAAUAAG | 23 | 7638 |
| myoC-7893 | + | CAGAGAAAAAGAGUUCCUAAUAAG | 24 | 7639 |
| myoC-7894 | + | GUUAAAAACAAGAUCCAG | 18 | 7640 |
| myoC-7895 | + | UGUUAAAAACAAGAUCCAG | 19 | 7641 |
| myoC-2292 | + | AUGUUAAAAACAAGAUCCAG | 20 | 2369 |
| myoC-7896 | + | UAUGUUAAAAACAAGAUCCAG | 21 | 7642 |
| myoC-7897 | + | AUAUGUUAAAAACAAGAUCCAG | 22 | 7643 |
| myoC-7898 | + | AAUAUGUUAAAAACAAGAUCCAG | 23 | 7644 |
| myoC-7899 | + | UAAUAUGUUAAAAACAAGAUCCAG | 24 | 7645 |
| myoC-7900 | + | GCAGACUCACCUCCAGAG | 18 | 7646 |
| myoC-7901 | + | GGCAGACUCACCUCCAGAG | 19 | 7647 |
| myoC-1181 | + | UGGCAGACUCACCUCCAGAG | 20 | 1481 |
| myoC-7902 | + | CUGGCAGACUCACCUCCAGAG | 21 | 7648 |
| myoC-7903 | + | CCUGGCAGACUCACCUCCAGAG | 22 | 7649 |
| myoC-7904 | + | CCCUGGCAGACUCACCUCCAGAG | 23 | 7650 |
| myoC-7905 | + | GCCCUGGCAGACUCACCUCCAGAG | 24 | 7651 |
| myoC-7906 | + | CUGCAAGGGUCUUUAUAG | 18 | 7652 |
| myoC-7907 | + | GCUGCAAGGGUCUUUAUAG | 19 | 7653 |
| myoC-2217 | + | AGCUGCAAGGGUCUUUAUAG | 20 | 2309 |
| myoC-7908 | + | GAGCUGCAAGGGUCUUUAUAG | 21 | 7654 |
| myoC-7909 | + | AGAGCUGCAAGGGUCUUUAUAG | 22 | 7655 |
| myoC-7910 | + | GAGAGCUGCAAGGGUCUUUAUAG | 23 | 7656 |
| myoC-7911 | + | CGAGAGCUGCAAGGGUCUUUAUAG | 24 | 7657 |
| myoC-7912 | + | UAGCUGUGCAGUCUCUAG | 18 | 7658 |
| myoC-7913 | + | CUAGCUGUGCAGUCUCUAG | 19 | 7659 |
| myoC-7914 | + | GCUAGCUGUGCAGUCUCUAG | 20 | 7660 |
| myoC-7915 | + | UGCUAGCUGUGCAGUCUCUAG | 21 | 7661 |
| myoC-7916 | + | GUGCUAGCUGUGCAGUCUCUAG | 22 | 7662 |
| myoC-7917 | + | UGUGCUAGCUGUGCAGUCUCUAG | 23 | 7663 |
| myoC-7918 | + | UUGUGCUAGCUGUGCAGUCUCUAG | 24 | 7664 |
| myoC-7919 | + | AUCAGAUAGUAAACAUCG | 18 | 7665 |
| myoC-7920 | + | AAUCAGAUAGUAAACAUCG | 19 | 7666 |
| myoC-2269 | + | GAAUCAGAUAGUAAACAUCG | 20 | 2349 |
| myoC-7921 | + | UGAAUCAGAUAGUAAACAUCG | 21 | 7667 |
| myoC-7922 | + | CUGAAUCAGAUAGUAAACAUCG | 22 | 7668 |
| myoC-7923 | + | UCUGAAUCAGAUAGUAAACAUCG | 23 | 7669 |
| myoC-7924 | + | UUCUGAAUCAGAUAGUAAACAUCG | 24 | 7670 |
| myoC-7925 | + | ACCCUACCAGGCUCCAGG | 18 | 7671 |
| myoC-7926 | + | CACCCUACCAGGCUCCAGG | 19 | 7672 |
| myoC-2308 | + | GCACCCUACCAGGCUCCAGG | 20 | 2379 |
| myoC-7927 | + | AGCACCCUACCAGGCUCCAGG | 21 | 7673 |
| myoC-7928 | + | CAGCACCCUACCAGGCUCCAGG | 22 | 7674 |
| myoC-7929 | + | ACAGCACCCUACCAGGCUCCAGG | 23 | 7675 |
| myoC-7930 | + | GACAGCACCCUACCAGGCUCCAGG | 24 | 7676 |
| myoC-7931 | + | UCUAGGAGAAAGGGCAGG | 18 | 7677 |
| myoC-7932 | + | CUCUAGGAGAAAGGGCAGG | 19 | 7678 |
| myoC-7933 | + | UCUCUAGGAGAAAGGGCAGG | 20 | 7679 |
| myoC-7934 | + | GUCUCUAGGAGAAAGGGCAGG | 21 | 7680 |
| myoC-7935 | + | AGUCUCUAGGAGAAAGGGCAGG | 22 | 7681 |
| myoC-7936 | + | CAGUCUCUAGGAGAAAGGGCAGG | 23 | 7682 |
| myoC-7937 | + | GCAGUCUCUAGGAGAAAGGGCAGG | 24 | 7683 |
| myoC-7938 | + | CGUGGGGUGCUGGUCAGG | 18 | 7684 |
| myoC-7939 | + | GCGUGGGGUGCUGGUCAGG | 19 | 7685 |
| myoC-2242 | + | UGCGUGGGGUGCUGGUCAGG | 20 | 2327 |
| myoC-7940 | + | CUGCGUGGGGUGCUGGUCAGG | 21 | 7686 |
| myoC-7941 | + | GCUGCGUGGGGUGCUGGUCAGG | 22 | 7687 |
| myoC-7942 | + | AGCUGCGUGGGGUGCUGGUCAGG | 23 | 7688 |
| myoC-7943 | + | GAGCUGCGUGGGGUGCUGGUCAGG | 24 | 7689 |
| myoC-7944 | + | GAAGACUAUGGCCCAGGG | 18 | 7690 |
| myoC-7945 | + | AGAAGACUAUGGCCCAGGG | 19 | 7691 |
| myoC-2212 | + | GAGAAGACUAUGGCCCAGGG | 20 | 2306 |
| myoC-7946 | + | AGAGAAGACUAUGGCCCAGGG | 21 | 7692 |
| myoC-7947 | + | CAGAGAAGACUAUGGCCCAGGG | 22 | 7693 |
| myoC-7948 | + | GCAGAGAAGACUAUGGCCCAGGG | 23 | 7694 |
| myoC-7949 | + | AGCAGAGAAGACUAUGGCCCAGGG | 24 | 7695 |
| myoC-7950 | + | CAUUGUCUAUGCUUAGGG | 18 | 7696 |
| myoC-7951 | + | CCAUUGUCUAUGCUUAGGG | 19 | 7697 |
| myoC-2237 | + | GCCAUUGUCUAUGCUUAGGG | 20 | 2324 |
| myoC-7952 | + | UGCCAUUGUCUAUGCUUAGGG | 21 | 7698 |
| myoC-7953 | + | AUGCCAUUGUCUAUGCUUAGGG | 22 | 7699 |
| myoC-7954 | + | AAUGCCAUUGUCUAUGCUUAGGG | 23 | 7700 |
| myoC-7955 | + | AAAUGCCAUUGUCUAUGCUUAGGG | 24 | 7701 |
| myoC-7956 | + | CGCACAGCCAACCAAUGG | 18 | 7702 |
| myoC-7957 | + | UCGCACAGCCAACCAAUGG | 19 | 7703 |
| myoC-2209 | + | GUCGCACAGCCAACCAAUGG | 20 | 2304 |
| myoC-7958 | + | GGUCGCACAGCCAACCAAUGG | 21 | 7704 |
| myoC-7959 | + | CGGUCGCACAGCCAACCAAUGG | 22 | 7705 |
| myoC-7960 | + | ACGGUCGCACAGCCAACCAAUGG | 23 | 7706 |
| myoC-7961 | + | CACGGUCGCACAGCCAACCAAUGG | 24 | 7707 |
| myoC-7962 | + | CUGUGAAAACUGACAUGG | 18 | 7708 |
| myoC-7963 | + | ACUGUGAAAACUGACAUGG | 19 | 7709 |
| myoC-5479 | + | GACUGUGAAAACUGACAUGG | 20 | 5225 |
| myoC-7964 | + | GGACUGUGAAAACUGACAUGG | 21 | 7710 |
| myoC-7965 | + | UGGACUGUGAAAACUGACAUGG | 22 | 7711 |
| myoC-7966 | + | AUGGACUGUGAAAACUGACAUGG | 23 | 7712 |
| myoC-7967 | + | UAUGGACUGUGAAAACUGACAUGG | 24 | 7713 |
| myoC-7968 | + | ACAACUGUGUAUCUUUGG | 18 | 7714 |
| myoC-7969 | + | AACAACUGUGUAUCUUUGG | 19 | 7715 |
| myoC-2303 | + | AAACAACUGUGUAUCUUUGG | 20 | 2376 |
| myoC-7970 | + | AAAACAACUGUGUAUCUUUGG | 21 | 7716 |
| myoC-7971 | + | UAAAACAACUGUGUAUCUUUGG | 22 | 7717 |
| myoC-7972 | + | UUAAAACAACUGUGUAUCUUUGG | 23 | 7718 |
| myoC-7973 | + | UUUAAAACAACUGUGUAUCUUUGG | 24 | 7719 |
| myoC-7974 | + | ACUGUGAAAACUGACAUG | 18 | 7720 |
| myoC-7975 | + | GACUGUGAAAACUGACAUG | 19 | 7721 |
| myoC-7976 | + | GGACUGUGAAAACUGACAUG | 20 | 7722 |
| myoC-7977 | + | UGGACUGUGAAAACUGACAUG | 21 | 7723 |
| myoC-7978 | + | AUGGACUGUGAAAACUGACAUG | 22 | 7724 |
| myoC-7979 | + | UAUGGACUGUGAAAACUGACAUG | 23 | 7725 |
| myoC-7980 | + | CUAUGGACUGUGAAAACUGACAUG | 24 | 7726 |
| myoC-7981 | + | UGCUGUCAGCAGUCACUG | 18 | 7727 |
| myoC-7982 | + | GUGCUGUCAGCAGUCACUG | 19 | 7728 |
| myoC-2246 | + | CGUGCUGUCAGCAGUCACUG | 20 | 2330 |
| myoC-7983 | + | CCGUGCUGUCAGCAGUCACUG | 21 | 7729 |
| myoC-7984 | + | UCCGUGCUGUCAGCAGUCACUG | 22 | 7730 |
| myoC-7985 | + | CUCCGUGCUGUCAGCAGUCACUG | 23 | 7731 |
| myoC-7986 | + | ACUCCGUGCUGUCAGCAGUCACUG | 24 | 7732 |
| myoC-7987 | + | CGUGAUCAGUGAGGACUG | 18 | 7733 |
| myoC-7988 | + | ACGUGAUCAGUGAGGACUG | 19 | 7734 |
| myoC-2228 | + | GACGUGAUCAGUGAGGACUG | 20 | 2317 |
| myoC-7989 | + | UGACGUGAUCAGUGAGGACUG | 21 | 7735 |
| myoC-7990 | + | CUGACGUGAUCAGUGAGGACUG | 22 | 7736 |
| myoC-7991 | + | UCUGACGUGAUCAGUGAGGACUG | 23 | 7737 |
| myoC-7992 | + | GUCUGACGUGAUCAGUGAGGACUG | 24 | 7738 |
| myoC-7993 | + | UACGAGCCAUAUCACCUG | 18 | 7739 |
| myoC-7994 | + | CUACGAGCCAUAUCACCUG | 19 | 7740 |
| myoC-7995 | + | ACUACGAGCCAUAUCACCUG | 20 | 7741 |
| myoC-7996 | + | CACUACGAGCCAUAUCACCUG | 21 | 7742 |
| myoC-7997 | + | UCACUACGAGCCAUAUCACCUG | 22 | 7743 |
| myoC-7998 | + | GUCACUACGAGCCAUAUCACCUG | 23 | 7744 |
| myoC-7999 | + | GGUCACUACGAGCCAUAUCACCUG | 24 | 7745 |
| myoC-8000 | + | CCUCAUUGGUGAAAUCUG | 18 | 7746 |
| myoC-8001 | + | ACCUCAUUGGUGAAAUCUG | 19 | 7747 |
| myoC-1226 | + | AACCUCAUUGGUGAAAUCUG | 20 | 1526 |
| myoC-8002 | + | GAACCUCAUUGGUGAAAUCUG | 21 | 7748 |
| myoC-8003 | + | AGAACCUCAUUGGUGAAAUCUG | 22 | 7749 |
| myoC-8004 | + | AAGAACCUCAUUGGUGAAAUCUG | 23 | 7750 |
| myoC-8005 | + | CAAGAACCUCAUUGGUGAAAUCUG | 24 | 7751 |
| myoC-8006 | + | AGACUCACCUCCAGAGUG | 18 | 7752 |
| myoC-8007 | + | CAGACUCACCUCCAGAGUG | 19 | 7753 |
| myoC-2275 | + | GCAGACUCACCUCCAGAGUG | 20 | 2355 |
| myoC-8008 | + | GGCAGACUCACCUCCAGAGUG | 21 | 7754 |
| myoC-8009 | + | UGGCAGACUCACCUCCAGAGUG | 22 | 7755 |
| myoC-8010 | + | CUGGCAGACUCACCUCCAGAGUG | 23 | 7756 |
| myoC-8011 | + | CCUGGCAGACUCACCUCCAGAGUG | 24 | 7757 |
| myoC-8012 | + | AUGCCAAGAACCUCAUUG | 18 | 7758 |
| myoC-8013 | + | CAUGCCAAGAACCUCAUUG | 19 | 7759 |
| myoC-2301 | + | GCAUGCCAAGAACCUCAUUG | 20 | 2374 |
| myoC-8014 | + | UGCAUGCCAAGAACCUCAUUG | 21 | 7760 |
| myoC-8015 | + | GUGCAUGCCAAGAACCUCAUUG | 22 | 7761 |
| myoC-8016 | + | UGUGCAUGCCAAGAACCUCAUUG | 23 | 7762 |
| myoC-8017 | + | GUGUGCAUGCCAAGAACCUCAUUG | 24 | 7763 |
| myoC-8018 | + | GAACCUCAUUGGUGAAAU | 18 | 7764 |
| myoC-8019 | + | AGAACCUCAUUGGUGAAAU | 19 | 7765 |
| myoC-2300 | + | AAGAACCUCAUUGGUGAAAU | 20 | 2373 |
| myoC-8020 | + | CAAGAACCUCAUUGGUGAAAU | 21 | 7766 |
| myoC-8021 | + | CCAAGAACCUCAUUGGUGAAAU | 22 | 7767 |
| myoC-8022 | + | GCCAAGAACCUCAUUGGUGAAAU | 23 | 7768 |
| myoC-8023 | + | UGCCAAGAACCUCAUUGGUGAAAU | 24 | 7769 |
| myoC-8024 | + | AAAGGUACAAAUAACAAU | 18 | 7770 |
| myoC-8025 | + | AAAAGGUACAAAUAACAAU | 19 | 7771 |
| myoC-8026 | + | CAAAAGGUACAAAUAACAAU | 20 | 7772 |
| myoC-8027 | + | UCAAAAGGUACAAAUAACAAU | 21 | 7773 |
| myoC-8028 | + | AUCAAAAGGUACAAAUAACAAU | 22 | 7774 |
| myoC-8029 | + | CAUCAAAAGGUACAAAUAACAAU | 23 | 7775 |
| myoC-8030 | + | ACAUCAAAAGGUACAAAUAACAAU | 24 | 7776 |
| myoC-8031 | + | AGAAAAAGAGUUCCUAAU | 18 | 7777 |
| myoC-8032 | + | GAGAAAAAGAGUUCCUAAU | 19 | 7778 |
| myoC-2259 | + | AGAGAAAAAGAGUUCCUAAU | 20 | 2341 |
| myoC-8033 | + | CAGAGAAAAAGAGUUCCUAAU | 21 | 7779 |
| myoC-8034 | + | ACAGAGAAAAAGAGUUCCUAAU | 22 | 7780 |
| myoC-8035 | + | CACAGAGAAAAAGAGUUCCUAAU | 23 | 7781 |
| myoC-8036 | + | CCACAGAGAAAAAGAGUUCCUAAU | 24 | 7782 |
| myoC-8037 | + | AAAGGAAAAAUAUAGUAU | 18 | 7783 |
| myoC-8038 | + | UAAAGGAAAAAUAUAGUAU | 19 | 7784 |
| myoC-2253 | + | GUAAAGGAAAAAUAUAGUAU | 20 | 2337 |
| myoC-8039 | + | UGUAAAGGAAAAAUAUAGUAU | 21 | 7785 |
| myoC-8040 | + | UUGUAAAGGAAAAAUAUAGUAU | 22 | 7786 |
| myoC-8041 | + | CUUGUAAAGGAAAAAUAUAGUAU | 23 | 7787 |
| myoC-8042 | + | GCUUGUAAAGGAAAAAUAUAGUAU | 24 | 7788 |
| myoC-8043 | + | UGGAGGGGCACAAGAACU | 18 | 7789 |
| myoC-8044 | + | AUGGAGGGGCACAAGAACU | 19 | 7790 |
| myoC-8045 | + | CAUGGAGGGGCACAAGAACU | 20 | 7791 |
| myoC-8046 | + | ACAUGGAGGGGCACAAGAACU | 21 | 7792 |
| myoC-8047 | + | GACAUGGAGGGGCACAAGAACU | 22 | 7793 |
| myoC-8048 | + | UGACAUGGAGGGGCACAAGAACU | 23 | 7794 |
| myoC-8049 | + | CUGACAUGGAGGGGCACAAGAACU | 24 | 7795 |
| myoC-8050 | + | CGCCUGUAGCAGGUCACU | 18 | 7796 |
| myoC-8051 | + | GCGCCUGUAGCAGGUCACU | 19 | 7797 |
| myoC-8052 | + | AGCGCCUGUAGCAGGUCACU | 20 | 7798 |
| myoC-8053 | + | GAGCGCCUGUAGCAGGUCACU | 21 | 7799 |
| myoC-8054 | + | GGAGCGCCUGUAGCAGGUCACU | 22 | 7800 |
| myoC-8055 | + | UGGAGCGCCUGUAGCAGGUCACU | 23 | 7801 |
| myoC-8056 | + | CUGGAGCGCCUGUAGCAGGUCACU | 24 | 7802 |
| myoC-8057 | + | CUCCUUUUGCUAUGGACU | 18 | 7803 |
| myoC-8058 | + | UCUCCUUUUGCUAUGGACU | 19 | 7804 |
| myoC-8059 | + | UUCUCCUUUUGCUAUGGACU | 20 | 7805 |
| myoC-8060 | + | UUUCUCCUUUUGCUAUGGACU | 21 | 7806 |
| myoC-8061 | + | AUUUCUCCUUUUGCUAUGGACU | 22 | 7807 |
| myoC-8062 | + | UAUUUCUCCUUUUGCUAUGGACU | 23 | 7808 |
| myoC-8063 | + | UUAUUUCUCCUUUUGCUAUGGACU | 24 | 7809 |
| myoC-8064 | + | UUGAAAUAAUGAUUGCCU | 18 | 7810 |
| myoC-8065 | + | CUUGAAAUAAUGAUUGCCU | 19 | 7811 |
| myoC-2280 | + | ACUUGAAAUAAUGAUUGCCU | 20 | 2359 |
| myoC-8066 | + | CACUUGAAAUAAUGAUUGCCU | 21 | 7812 |
| myoC-8067 | + | CCACUUGAAAUAAUGAUUGCCU | 22 | 7813 |
| myoC-8068 | + | GCCACUUGAAAUAAUGAUUGCCU | 23 | 7814 |
| myoC-8069 | + | AGCCACUUGAAAUAAUGAUUGCCU | 24 | 7815 |
| myoC-8070 | + | AAUGCCAUUGUCUAUGCU | 18 | 7816 |
| myoC-8071 | + | AAAUGCCAUUGUCUAUGCU | 19 | 7817 |
| myoC-2240 | + | CAAAUGCCAUUGUCUAUGCU | 20 | 2325 |
| myoC-8072 | + | GCAAAUGCCAUUGUCUAUGCU | 21 | 7818 |
| myoC-8073 | + | GGCAAAUGCCAUUGUCUAUGCU | 22 | 7819 |
| myoC-8074 | + | UGGCAAAUGCCAUUGUCUAUGCU | 23 | 7820 |
| myoC-8075 | + | UUGGCAAAUGCCAUUGUCUAUGCU | 24 | 7821 |
| myoC-8076 | + | UUUAUUUCUCCUUUUGCU | 18 | 7822 |
| myoC-8077 | + | UUUUAUUUCUCCUUUUGCU | 19 | 7823 |
| myoC-8078 | + | CUUUUAUUUCUCCUUUUGCU | 20 | 7824 |
| myoC-8079 | + | CCUUUUAUUUCUCCUUUUGCU | 21 | 7825 |
| myoC-8080 | + | UCCUUUUAUUUCUCCUUUUGCU | 22 | 7826 |
| myoC-8081 | + | GUCCUUUUAUUUCUCCUUUUGCU | 23 | 7827 |
| myoC-8082 | + | GGUCCUUUUAUUUCUCCUUUUGCU | 24 | 7828 |
| myoC-8083 | + | ACCUCAUUGGUGAAAUCU | 18 | 7829 |
| myoC-8084 | + | AACCUCAUUGGUGAAAUCU | 19 | 7830 |
| myoC-1225 | + | GAACCUCAUUGGUGAAAUCU | 20 | 1525 |
| myoC-8085 | + | AGAACCUCAUUGGUGAAAUCU | 21 | 7831 |
| myoC-8086 | + | AAGAACCUCAUUGGUGAAAUCU | 22 | 7832 |
| myoC-8087 | + | CAAGAACCUCAUUGGUGAAAUCU | 23 | 7833 |
| myoC-8088 | + | CCAAGAACCUCAUUGGUGAAAUCU | 24 | 7834 |
| myoC-8089 | + | UAAAACAACUGUGUAUCU | 18 | 7835 |
| myoC-8090 | + | UUAAAACAACUGUGUAUCU | 19 | 7836 |
| myoC-2306 | + | UUUAAAACAACUGUGUAUCU | 20 | 2377 |
| myoC-8091 | + | CUUUAAAACAACUGUGUAUCU | 21 | 7837 |
| myoC-8092 | + | GCUUUAAAACAACUGUGUAUCU | 22 | 7838 |
| myoC-8093 | + | AGCUUUAAAACAACUGUGUAUCU | 23 | 7839 |
| myoC-8094 | + | UAGCUUUAAAACAACUGUGUAUCU | 24 | 7840 |
| myoC-8095 | + | UCUGUUUGGCUUUACUCU | 18 | 7841 |
| myoC-8096 | + | AUCUGUUUGGCUUUACUCU | 19 | 7842 |
| myoC-2267 | + | AAUCUGUUUGGCUUUACUCU | 20 | 2347 |
| myoC-8097 | + | GAAUCUGUUUGGCUUUACUCU | 21 | 7843 |
| myoC-8098 | + | UGAAUCUGUUUGGCUUUACUCU | 22 | 7844 |
| myoC-8099 | + | UUGAAUCUGUUUGGCUUUACUCU | 23 | 7845 |
| myoC-8100 | + | CUUGAAUCUGUUUGGCUUUACUCU | 24 | 7846 |
| myoC-8101 | + | UAAUCUAAAUGAAGCUCU | 18 | 7847 |
| myoC-8102 | + | CUAAUCUAAAUGAAGCUCU | 19 | 7848 |
| myoC-8103 | + | ACUAAUCUAAAUGAAGCUCU | 20 | 7849 |
| myoC-8104 | + | CACUAAUCUAAAUGAAGCUCU | 21 | 7850 |
| myoC-8105 | + | CCACUAAUCUAAAUGAAGCUCU | 22 | 7851 |
| myoC-8106 | + | ACCACUAAUCUAAAUGAAGCUCU | 23 | 7852 |
| myoC-8107 | + | AACCACUAAUCUAAAUGAAGCUCU | 24 | 7853 |
| myoC-8108 | + | GCUAGCUGUGCAGUCUCU | 18 | 7854 |
| myoC-8109 | + | UGCUAGCUGUGCAGUCUCU | 19 | 7855 |
| myoC-5480 | + | GUGCUAGCUGUGCAGUCUCU | 20 | 5226 |
| myoC-8110 | + | UGUGCUAGCUGUGCAGUCUCU | 21 | 7856 |
| myoC-8111 | + | UUGUGCUAGCUGUGCAGUCUCU | 22 | 7857 |
| myoC-8112 | + | CUUGUGCUAGCUGUGCAGUCUCU | 23 | 7858 |
| myoC-8113 | + | UCUUGUGCUAGCUGUGCAGUCUCU | 24 | 7859 |
| myoC-4531 | + | GAGCAGUGUCUCGGGUCU | 18 | 4277 |
| myoC-4532 | + | CGAGCAGUGUCUCGGGUCU | 19 | 4278 |
| myoC-204 | + | CCGAGCAGUGUCUCGGGUCU | 20 | 590 |
| myoC-4533 | + | CCCGAGCAGUGUCUCGGGUCU | 21 | 4279 |
| myoC-4534 | + | GCCCGAGCAGUGUCUCGGGUCU | 22 | 4280 |
| myoC-4535 | + | AGCCCGAGCAGUGUCUCGGGUCU | 23 | 4281 |
| myoC-4536 | + | CAGCCCGAGCAGUGUCUCGGGUCU | 24 | 4282 |
| myoC-4537 | + | UCUGCAUUCUUACCUUCU | 18 | 4283 |
| myoC-4538 | + | CUCUGCAUUCUUACCUUCU | 19 | 4284 |
| myoC-4539 | + | ACUCUGCAUUCUUACCUUCU | 20 | 4285 |
| myoC-4540 | + | CACUCUGCAUUCUUACCUUCU | 21 | 4286 |
| myoC-4541 | + | CCACUCUGCAUUCUUACCUUCU | 22 | 4287 |
| myoC-4542 | + | CCCACUCUGCAUUCUUACCUUCU | 23 | 4288 |
| myoC-4543 | + | CCCCACUCUGCAUUCUUACCUUCU | 24 | 4289 |
| myoC-8114 | + | AAUCUGGGGAACUCUUCU | 18 | 7860 |
| myoC-8115 | + | AAAUCUGGGGAACUCUUCU | 19 | 7861 |
| myoC-2296 | + | GAAAUCUGGGGAACUCUUCU | 20 | 2372 |
| myoC-8116 | + | UGAAAUCUGGGGAACUCUUCU | 21 | 7862 |
| myoC-8117 | + | GUGAAAUCUGGGGAACUCUUCU | 22 | 7863 |
| myoC-8118 | + | GGUGAAAUCUGGGGAACUCUUCU | 23 | 7864 |
| myoC-8119 | + | UGGUGAAAUCUGGGGAACUCUUCU | 24 | 7865 |
| myoC-8120 | + | GAGUCUGACGUGAUCAGU | 18 | 7866 |
| myoC-8121 | + | GGAGUCUGACGUGAUCAGU | 19 | 7867 |
| myoC-2229 | + | UGGAGUCUGACGUGAUCAGU | 20 | 2318 |
| myoC-8122 | + | CUGGAGUCUGACGUGAUCAGU | 21 | 7868 |
| myoC-8123 | + | CCUGGAGUCUGACGUGAUCAGU | 22 | 7869 |
| myoC-8124 | + | UCCUGGAGUCUGACGUGAUCAGU | 23 | 7870 |
| myoC-8125 | + | GUCCUGGAGUCUGACGUGAUCAGU | 24 | 7871 |
| myoC-8126 | + | UAAAAUGUUAAAUUUAGU | 18 | 7872 |
| myoC-8127 | + | AUAAAAUGUUAAAUUUAGU | 19 | 7873 |
| myoC-2286 | + | AAUAAAAUGUUAAAUUUAGU | 20 | 2365 |
| myoC-8128 | + | GAAUAAAAUGUUAAAUUUAGU | 21 | 7874 |
| myoC-8129 | + | GGAAUAAAAUGUUAAAUUUAGU | 22 | 7875 |
| myoC-8130 | + | UGGAAUAAAAUGUUAAAUUUAGU | 23 | 7876 |
| myoC-8131 | + | AUGGAAUAAAAUGUUAAAUUUAGU | 24 | 7877 |
| myoC-4558 | + | CCGAGCAGUGUCUCGGGU | 18 | 4304 |
| myoC-4559 | + | CCCGAGCAGUGUCUCGGGU | 19 | 4305 |
| myoC-1699 | + | GCCCGAGCAGUGUCUCGGGU | 20 | 1951 |
| myoC-4560 | + | AGCCCGAGCAGUGUCUCGGGU | 21 | 4306 |
| myoC-4561 | + | CAGCCCGAGCAGUGUCUCGGGU | 22 | 4307 |
| myoC-4562 | + | ACAGCCCGAGCAGUGUCUCGGGU | 23 | 4308 |
| myoC-4563 | + | CACAGCCCGAGCAGUGUCUCGGGU | 24 | 4309 |
| myoC-8132 | + | UAAAUAUACCAAAACUGU | 18 | 7878 |
| myoC-8133 | + | AUAAAUAUACCAAAACUGU | 19 | 7879 |
| myoC-2282 | + | AAUAAAUAUACCAAAACUGU | 20 | 2361 |
| myoC-8134 | + | CAAUAAAUAUACCAAAACUGU | 21 | 7880 |
| myoC-8135 | + | CCAAUAAAUAUACCAAAACUGU | 22 | 7881 |
| myoC-8136 | + | GCCAAUAAAUAUACCAAAACUGU | 23 | 7882 |
| myoC-8137 | + | AGCCAAUAAAUAUACCAAAACUGU | 24 | 7883 |
| myoC-8138 | + | ACAACAGUGUCAAUACUU | 18 | 7884 |
| myoC-8139 | + | AACAACAGUGUCAAUACUU | 19 | 7885 |
| myoC-2279 | + | CAACAACAGUGUCAAUACUU | 20 | 2358 |
| myoC-8140 | + | CCAACAACAGUGUCAAUACUU | 21 | 7886 |
| myoC-8141 | + | ACCAACAACAGUGUCAAUACUU | 22 | 7887 |
| myoC-8142 | + | UACCAACAACAGUGUCAAUACUU | 23 | 7888 |
| myoC-8143 | + | AUACCAACAACAGUGUCAAUACUU | 24 | 7889 |
| myoC-8144 | + | AUGCCAUUGUCUAUGCUU | 18 | 7890 |
| myoC-8145 | + | AAUGCCAUUGUCUAUGCUU | 19 | 7891 |
| myoC-1073 | + | AAAUGCCAUUGUCUAUGCUU | 20 | 1373 |
| myoC-8146 | + | CAAAUGCCAUUGUCUAUGCUU | 21 | 7892 |
| myoC-8147 | + | GCAAAUGCCAUUGUCUAUGCUU | 22 | 7893 |
| myoC-8148 | + | GGCAAAUGCCAUUGUCUAUGCUU | 23 | 7894 |
| myoC-8149 | + | UGGCAAAUGCCAUUGUCUAUGCUU | 24 | 7895 |
| myoC-8150 | + | AAAACAACUGUGUAUCUU | 18 | 7896 |
| myoC-8151 | + | UAAAACAACUGUGUAUCUU | 19 | 7897 |
| myoC-1220 | + | UUAAAACAACUGUGUAUCUU | 20 | 1520 |
| myoC-8152 | + | UUUAAAACAACUGUGUAUCUU | 21 | 7898 |
| myoC-8153 | + | CUUUAAAACAACUGUGUAUCUU | 22 | 7899 |
| myoC-8154 | + | GCUUUAAAACAACUGUGUAUCUU | 23 | 7900 |
| myoC-8155 | + | AGCUUUAAAACAACUGUGUAUCUU | 24 | 7901 |
| myoC-8156 | + | UUCAAAU UCACAGGCUUU | 18 | 7902 |
| myoC-8157 | + | AUUCAAAUUCACAGGCUUU | 19 | 7903 |
| myoC-2285 | + | CAUUCAAAUUCACAGGCUUU | 20 | 2364 |
| myoC-8158 | + | UCAUUCAAAUUCACAGGCUUU | 21 | 7904 |
| myoC-8159 | + | CUCAUUCAAAUUCACAGGCUUU | 22 | 7905 |
| myoC-8160 | + | CCUCAUUCAAAUUCACAGGCUUU | 23 | 7906 |
| myoC-8161 | + | UCCUCAUUCAAAUUCACAGGCUUU | 24 | 7907 |
| myoC-8162 | + | AAACAACUGUGUAUCUUU | 18 | 7908 |
| myoC-8163 | + | AAAACAACUGUGUAUCUUU | 19 | 7909 |
| myoC-1221 | + | UAAAACAACUGUGUAUCUUU | 20 | 1521 |
| myoC-8164 | + | UUAAAACAACUGUGUAUCUUU | 21 | 7910 |
| myoC-8165 | + | UUUAAAACAACUGUGUAUCUUU | 22 | 7911 |
| myoC-8166 | + | CUUUAAAACAACUGUGUAUCUUU | 23 | 7912 |
| myoC-8167 | + | GCUUUAAAACAACUGUGUAUCUUU | 24 | 7913 |
| myoC-8168 | - | UUGCCUGGCAUUCAAAAA | 18 | 7914 |
| myoC-8169 | - | UUUGCCUGGCAUUCAAAAA | 19 | 7915 |
| myoC-1971 | - | UUUUGCCUGGCAUUCAAAAA | 20 | 2129 |
| myoC-8170 | - | CUUUUGCCUGGCAUUCAAAAA | 21 | 7916 |
| myoC-8171 | - | GCUUUUGCCUGGCAUUCAAAAA | 22 | 7917 |
| myoC-8172 | - | AGCUUUUGCCUGGCAUUCAAAAA | 23 | 7918 |
| myoC-8173 | - | UAGCUUUUGCCUGGCAUUCAAAAA | 24 | 7919 |
| myoC-8174 | - | AGGGGAGGAGAAGAAAAA | 18 | 7920 |
| myoC-8175 | - | CAGGGGAGGAGAAGAAAAA | 19 | 7921 |
| myoC-1987 | - | GCAGGGGAGGAGAAGAAAAA | 20 | 2139 |
| myoC-8176 | - | CGCAGGGGAGGAGAAGAAAAA | 21 | 7922 |
| myoC-8177 | - | GCGCAGGGGAGGAGAAGAAAAA | 22 | 7923 |
| myoC-8178 | - | AGCGCAGGGGAGGAGAAGAAAAA | 23 | 7924 |
| myoC-8179 | - | CAGCGCAGGGGAGGAGAAGAAAAA | 24 | 7925 |
| myoC-8180 | - | UUCACAGUCCAUAGCAAA | 18 | 7926 |
| myoC-8181 | - | UUUCACAGUCCAUAGCAAA | 19 | 7927 |
| myoC-5447 | - | UUUUCACAGUCCAUAGCAAA | 20 | 5193 |
| myoC-8182 | - | GUUUUCACAGUCCAUAGCAAA | 21 | 7928 |
| myoC-8183 | - | AGUUUUCACAGUCCAUAGCAAA | 22 | 7929 |
| myoC-8184 | - | CAGUUUUCACAGUCCAUAGCAAA | 23 | 7930 |
| myoC-8185 | - | UCAGUUUUCACAGUCCAUAGCAAA | 24 | 7931 |
| myoC-3441 | - | AGCGACUAAGGCAAGAAA | 18 | 3187 |
| myoC-3442 | - | AAGCGACUAAGGCAAGAAA | 19 | 3188 |
| myoC-1647 | - | GAAGCGACUAAGGCAAGAAA | 20 | 1913 |
| myoC-3443 | - | AGAAGCGACUAAGGCAAGAAA | 21 | 3189 |
| myoC-3444 | - | AAGAAGCGACUAAGGCAAGAAA | 22 | 3190 |
| myoC-3445 | - | GAAGAAGCGACUAAGGCAAGAAA | 23 | 3191 |
| myoC-3446 | - | AGAAGAAGCGACUAAGGCAAGAAA | 24 | 3192 |
| myoC-8186 | - | GCAGGGGAGGAGAAGAAA | 18 | 7932 |
| myoC-8187 | - | CGCAGGGGAGGAGAAGAAA | 19 | 7933 |
| myoC-1986 | - | GCGCAGGGGAGGAGAAGAAA | 20 | 2138 |
| myoC-8188 | - | AGCGCAGGGGAGGAGAAGAAA | 21 | 7934 |
| myoC-8189 | - | CAGCGCAGGGGAGGAGAAGAAA | 22 | 7935 |
| myoC-8190 | - | GCAGCGCAGGGGAGGAGAAGAAA | 23 | 7936 |
| myoC-8191 | - | UGCAGCGCAGGGGAGGAGAAGAAA | 24 | 7937 |
| myoC-8192 | - | UACUAUCUGAUUCAGAAA | 18 | 7938 |
| myoC-8193 | - | UUACUAUCUGAUUCAGAAA | 19 | 7939 |
| myoC-2028 | - | UUUACUAUCUGAUUCAGAAA | 20 | 2163 |
| myoC-8194 | - | GUUUACUAUCUGAUUCAGAAA | 21 | 7940 |
| myoC-8195 | - | UGUUUACUAUCUGAUUCAGAAA | 22 | 7941 |
| myoC-8196 | - | AUGUUUACUAUCUGAUUCAGAAA | 23 | 7942 |
| myoC-8197 | - | GAUGUUUACUAUCUGAUUCAGAAA | 24 | 7943 |
| myoC-8198 | - | UGAUUUUGUCAUUACCAA | 18 | 7944 |
| myoC-8199 | - | GUGAUUUUGUCAUUACCAA | 19 | 7945 |
| myoC-2050 | - | UGUGAUUUUGUCAUUACCAA | 20 | 2181 |
| myoC-8200 | - | CUGUGAUUUUGUCAUUACCAA | 21 | 7946 |
| myoC-8201 | - | CCUGUGAUUUUGUCAUUACCAA | 22 | 7947 |
| myoC-8202 | - | ACCUGUGAUUUUGUCAUUACCAA | 23 | 7948 |
| myoC-8203 | - | UACCUGUGAUUUUGUCAUUACCAA | 24 | 7949 |
| myoC-8204 | - | AAAACUGGGCCAGAGCAA | 18 | 7950 |
| myoC-8205 | - | AAAAACUGGGCCAGAGCAA | 19 | 7951 |
| myoC-1973 | - | CAAAAACUGGGCCAGAGCAA | 20 | 2131 |
| myoC-8206 | - | UCAAAAACUGGGCCAGAGCAA | 21 | 7952 |
| myoC-8207 | - | UUCAAAAACUGGGCCAGAGCAA | 22 | 7953 |
| myoC-8208 | - | AUUCAAAAACUGGGCCAGAGCAA | 23 | 7954 |
| myoC-8209 | - | CAUUCAAAAACUGGGCCAGAGCAA | 24 | 7955 |
| myoC-8210 | - | UUUCACAGUCCAUAGCAA | 18 | 7956 |
| myoC-8211 | - | UUUUCACAGUCCAUAGCAA | 19 | 7957 |
| myoC-8212 | - | GUUUUCACAGUCCAUAGCAA | 20 | 7958 |
| myoC-8213 | - | AGUUUUCACAGUCCAUAGCAA | 21 | 7959 |
| myoC-8214 | - | CAGUUUUCACAGUCCAUAGCAA | 22 | 7960 |
| myoC-8215 | - | UCAGUUUUCACAGUCCAUAGCAA | 23 | 7961 |
| myoC-8216 | - | GUCAGUUUUCACAGUCCAUAGCAA | 24 | 7962 |
| myoC-8217 | - | GGGAAAAAAUCAGUUCAA | 18 | 7963 |
| myoC-8218 | - | GGGGAAAAAAUCAGUUCAA | 19 | 7964 |
| myoC-1142 | - | GGGGGAAAAAAUCAGUUCAA | 20 | 1442 |
| myoC-8219 | - | GGGGGGAAAAAAUCAGUUCAA | 21 | 7965 |
| myoC-8220 | - | UGGGGGGAAAAAAUCAGUUCAA | 22 | 7966 |
| myoC-8221 | - | GUGGGGGGAAAAAAUCAGUUCAA | 23 | 7967 |
| myoC-8222 | - | UGUGGGGGGAAAAAAUCAGUUCAA | 24 | 7968 |
| myoC-8223 | - | AUUUUAUUCCAUUGCGAA | 18 | 7969 |
| myoC-8224 | - | CAUUUUAUUCCAUUGCGAA | 19 | 7970 |
| myoC-2049 | - | ACAUUUUAUUCCAUUGCGAA | 20 | 2180 |
| myoC-8225 | - | AACAUUUUAUUCCAUUGCGAA | 21 | 7971 |
| myoC-8226 | - | UAACAUUUUAUUCCAUUGCGAA | 22 | 7972 |
| myoC-8227 | - | UUAACAUUUUAUUCCAUUGCGAA | 23 | 7973 |
| myoC-8228 | - | UUUAACAUUUUAUUCCAUUGCGAA | 24 | 7974 |
| myoC-8229 | - | UAGCAAAAGGAGAAAUAA | 18 | 7975 |
| myoC-8230 | - | AUAGCAAAAGGAGAAAUAA | 19 | 7976 |
| myoC-8231 | - | CAUAGCAAAAGGAGAAAUAA | 20 | 7977 |
| myoC-8232 | - | CCAUAGCAAAAGGAGAAAUAA | 21 | 7978 |
| myoC-8233 | - | UCCAUAGCAAAAGGAGAAAUAA | 22 | 7979 |
| myoC-8234 | - | GUCCAUAGCAAAAGGAGAAAUAA | 23 | 7980 |
| myoC-8235 | - | AGUCCAUAGCAAAAGGAGAAAUAA | 24 | 7981 |
| myoC-8236 | - | CAAGUCACAAGGUAGUAA | 18 | 7982 |
| myoC-8237 | - | GCAAGUCACAAGGUAGUAA | 19 | 7983 |
| myoC-2016 | - | AGCAAGUCACAAGGUAGUAA | 20 | 2154 |
| myoC-8238 | - | GAGCAAGUCACAAGGUAGUAA | 21 | 7984 |
| myoC-8239 | - | UGAGCAAGUCACAAGGUAGUAA | 22 | 7985 |
| myoC-8240 | - | CUGAGCAAGUCACAAGGUAGUAA | 23 | 7986 |
| myoC-8241 | - | UCUGAGCAAGUCACAAGGUAGUAA | 24 | 7987 |
| myoC-8242 | - | GUUGCAGAUACGUUGUAA | 18 | 7988 |
| myoC-8243 | - | UGUUGCAGAUACGUUGUAA | 19 | 7989 |
| myoC-2051 | - | UUGUUGCAGAUACGUUGUAA | 20 | 2182 |
| myoC-8244 | - | GUUGUUGCAGAUACGUUGUAA | 21 | 7990 |
| myoC-8245 | - | AGUUGUUGCAGAUACGUUGUAA | 22 | 7991 |
| myoC-8246 | - | CAGUUGUUGCAGAUACGUUGUAA | 23 | 7992 |
| myoC-8247 | - | ACAGUUGUUGCAGAUACGUUGUAA | 24 | 7993 |
| myoC-8248 | - | CAAUCCCGUUUCUUUUAA | 18 | 7994 |
| myoC-8249 | - | GCAAUCCCGUUUCUUUUAA | 19 | 7995 |
| myoC-2022 | - | GGCAAUCCCGUUUCUUUUAA | 20 | 2158 |
| myoC-8250 | - | GGGCAAUCCCGUUUCUUUUAA | 21 | 7996 |
| myoC-8251 | - | AGGGCAAUCCCGUUUCUUUUAA | 22 | 7997 |
| myoC-8252 | - | AAGGGCAAUCCCGUUUCUUUUAA | 23 | 7998 |
| myoC-8253 | - | CAAGGGCAAUCCCGUUUCUUUUAA | 24 | 7999 |
| myoC-8254 | - | UGGAGCAGCUGAGCCACA | 18 | 8000 |
| myoC-8255 | - | CUGGAGCAGCUGAGCCACA | 19 | 8001 |
| myoC-1047 | - | GCUGGAGCAGCUGAGCCACA | 20 | 1347 |
| myoC-8256 | - | AGCUGGAGCAGCUGAGCCACA | 21 | 8002 |
| myoC-8257 | - | GAGCUGGAGCAGCUGAGCCACA | 22 | 8003 |
| myoC-8258 | - | AGAGCUGGAGCAGCUGAGCCACA | 23 | 8004 |
| myoC-8259 | - | CAGAGCUGGAGCAGCUGAGCCACA | 24 | 8005 |
| myoC-8260 | - | GUUCCCCAGAUUUCACCA | 18 | 8006 |
| myoC-8261 | - | AGUUCCCCAGAUUUCACCA | 19 | 8007 |
| myoC-2058 | - | GAGUUCCCCAGAUUUCACCA | 20 | 2189 |
| myoC-8262 | - | AGAGUUCCCCAGAUUUCACCA | 21 | 8008 |
| myoC-8263 | - | AAGAGUUCCCCAGAUUUCACCA | 22 | 8009 |
| myoC-8264 | - | GAAGAGUUCCCCAGAUUUCACCA | 23 | 8010 |
| myoC-8265 | - | AGAAGAGUUCCCCAGAUUUCACCA | 24 | 8011 |
| myoC-8266 | - | GGCAGUGGGAAUUGACCA | 18 | 8012 |
| myoC-8267 | - | GGGCAGUGGGAAUUGACCA | 19 | 8013 |
| myoC-1996 | - | AGGGCAGUGGGAAUUGACCA | 20 | 2145 |
| myoC-8268 | - | AAGGGCAGUGGGAAUUGACCA | 21 | 8014 |
| myoC-8269 | - | CAAGGGCAGUGGGAAUUGACCA | 22 | 8015 |
| myoC-8270 | - | UCAAGGGCAGUGGGAAUUGACCA | 23 | 8016 |
| myoC-8271 | - | UUCAAGGGCAGUGGGAAUUGACCA | 24 | 8017 |
| myoC-8272 | - | GCUGGAGCAGCUGAGCCA | 18 | 8018 |
| myoC-8273 | - | AGCUGGAGCAGCUGAGCCA | 19 | 8019 |
| myoC-1949 | - | GAGCUGGAGCAGCUGAGCCA | 20 | 2116 |
| myoC-8274 | - | AGAGCUGGAGCAGCUGAGCCA | 21 | 8020 |
| myoC-8275 | - | CAGAGCUGGAGCAGCUGAGCCA | 22 | 8021 |
| myoC-8276 | - | GCAGAGCUGGAGCAGCUGAGCCA | 23 | 8022 |
| myoC-8277 | - | GGCAGAGCUGGAGCAGCUGAGCCA | 24 | 8023 |
| myoC-4656 | - | CUGUGCCACCAGGCUCCA | 18 | 4402 |
| myoC-4657 | - | GCUGUGCCACCAGGCUCCA | 19 | 4403 |
| myoC-1662 | - | GGCUGUGCCACCAGGCUCCA | 20 | 1924 |
| myoC-4658 | - | GGGCUGUGCCACCAGGCUCCA | 21 | 4404 |
| myoC-4659 | - | CGGGCUGUGCCACCAGGCUCCA | 22 | 4405 |
| myoC-4660 | - | UCGGGCUGUGCCACCAGGCUCCA | 23 | 4406 |
| myoC-4661 | - | CUCGGGCUGUGCCACCAGGCUCCA | 24 | 4407 |
| myoC-8278 | - | GGAGUGACCUGCAGCGCA | 18 | 8024 |
| myoC-8279 | - | CGGAGUGACCUGCAGCGCA | 19 | 8025 |
| myoC-1119 | - | ACGGAGUGACCUGCAGCGCA | 20 | 1419 |
| myoC-8280 | - | CACGGAGUGACCUGCAGCGCA | 21 | 8026 |
| myoC-8281 | - | GCACGGAGUGACCUGCAGCGCA | 22 | 8027 |
| myoC-8282 | - | AGCACGGAGUGACCUGCAGCGCA | 23 | 8028 |
| myoC-8283 | - | CAGCACGGAGUGACCUGCAGCGCA | 24 | 8029 |
| myoC-8284 | - | CAGAUUCAUUCAAGGGCA | 18 | 8030 |
| myoC-8285 | - | ACAGAUUCAUUCAAGGGCA | 19 | 8031 |
| myoC-1993 | - | GACAGAUUCAUUCAAGGGCA | 20 | 2144 |
| myoC-8286 | - | AGACAGAUUCAUUCAAGGGCA | 21 | 8032 |
| myoC-8287 | - | AAGACAGAUUCAUUCAAGGGCA | 22 | 8033 |
| myoC-8288 | - | AAAGACAGAUUCAUUCAAGGGCA | 23 | 8034 |
| myoC-8289 | - | GAAAGACAGAUUCAUUCAAGGGCA | 24 | 8035 |
| myoC-8290 | - | AUGCUUCAGGAAAGCUCA | 18 | 8036 |
| myoC-8291 | - | AAUGCUUCAGGAAAGCUCA | 19 | 8037 |
| myoC-1968 | - | CAAUGCUUCAGGAAAGCUCA | 20 | 2126 |
| myoC-8292 | - | ACAAUGCUUCAGGAAAGCUCA | 21 | 8038 |
| myoC-8293 | - | CACAAUGCUUCAGGAAAGCUCA | 22 | 8039 |
| myoC-8294 | - | CCACAAUGCUUCAGGAAAGCUCA | 23 | 8040 |
| myoC-8295 | - | GCCACAAUGCUUCAGGAAAGCUCA | 24 | 8041 |
| myoC-8296 | - | GGGGAAAAAAUCAGUUCA | 18 | 8042 |
| myoC-8297 | - | GGGGGAAAAAAUCAGUUCA | 19 | 8043 |
| myoC-1141 | - | GGGGGGAAAAAAUCAGUUCA | 20 | 1441 |
| myoC-8298 | - | UGGGGGGAAAAAAUCAGUUCA | 21 | 8044 |
| myoC-8299 | - | GUGGGGGGAAAAAAUCAGUUCA | 22 | 8045 |
| myoC-8300 | - | UGUGGGGGGAAAAAAUCAGUUCA | 23 | 8046 |
| myoC-8301 | - | UUGUGGGGGGAAAAAAUCAGUUCA | 24 | 8047 |
| myoC-8302 | - | GGGAGGAGAAGAAAAAGA | 18 | 8048 |
| myoC-8303 | - | GGGGAGGAGAAGAAAAAGA | 19 | 8049 |
| myoC-1988 | - | AGGGGAGGAGAAGAAAAAGA | 20 | 2140 |
| myoC-8304 | - | CAGGGGAGGAGAAGAAAAAGA | 21 | 8050 |
| myoC-8305 | - | GCAGGGGAGGAGAAGAAAAAGA | 22 | 8051 |
| myoC-8306 | - | CGCAGGGGAGGAGAAGAAAAAGA | 23 | 8052 |
| myoC-8307 | - | GCGCAGGGGAGGAGAAGAAAAAGA | 24 | 8053 |
| myoC-8308 | - | GGUGCCUGAGAUGCAAGA | 18 | 8054 |
| myoC-8309 | - | UGGUGCCUGAGAUGCAAGA | 19 | 8055 |
| myoC-2063 | - | UUGGUGCCUGAGAUGCAAGA | 20 | 2194 |
| myoC-8310 | - | AUUGGUGCCUGAGAUGCAAGA | 21 | 8056 |
| myoC-8311 | - | CAUUGGUGCCUGAGAUGCAAGA | 22 | 8057 |
| myoC-8312 | - | ACAUUGGUGCCUGAGAUGCAAGA | 23 | 8058 |
| myoC-8313 | - | GACAUUGGUGCCUGAGAUGCAAGA | 24 | 8059 |
| myoC-4668 | - | AGAAGGUAAGAAUGCAGA | 18 | 4414 |
| myoC-4669 | - | GAGAAGGUAAGAAUGCAGA | 19 | 4415 |
| myoC-4670 | - | AGAGAAGGUAAGAAUGCAGA | 20 | 4416 |
| myoC-4671 | - | CAGAGAAGGUAAGAAUGCAGA | 21 | 4417 |
| myoC-4672 | - | CCAGAGAAGGUAAGAAUGCAGA | 22 | 4418 |
| myoC-4673 | - | UCCAGAGAAGGUAAGAAUGCAGA | 23 | 4419 |
| myoC-4674 | - | CUCCAGAGAAGGUAAGAAUGCAGA | 24 | 4420 |
| myoC-4681 | - | GAGGUAGCAAGGCUGAGA | 18 | 4427 |
| myoC-4682 | - | GGAGGUAGCAAGGCUGAGA | 19 | 4428 |
| myoC-198 | - | AGGAGGUAGCAAGGCUGAGA | 20 | 584 |
| myoC-4683 | - | CAGGAGGUAGCAAGGCUGAGA | 21 | 4429 |
| myoC-4684 | - | CCAGGAGGUAGCAAGGCUGAGA | 22 | 4430 |
| myoC-4685 | - | GCCAGGAGGUAGCAAGGCUGAGA | 23 | 4431 |
| myoC-4686 | - | AGCCAGGAGGUAGCAAGGCUGAGA | 24 | 4432 |
| myoC-8314 | - | UGAGGGGGGAUGUUGAGA | 18 | 8060 |
| myoC-8315 | - | GUGAGGGGGGAUGUUGAGA | 19 | 8061 |
| myoC-1041 | - | UGUGAGGGGGGAUGUUGAGA | 20 | 1341 |
| myoC-8316 | - | CUGUGAGGGGGGAUGUUGAGA | 21 | 8062 |
| myoC-8317 | - | UCUGUGAGGGGGGAUGUUGAGA | 22 | 8063 |
| myoC-8318 | - | CUCUGUGAGGGGGGAUGUUGAGA | 23 | 8064 |
| myoC-8319 | - | UCUCUGUGAGGGGGGAUGUUGAGA | 24 | 8065 |
| myoC-8320 | - | UCACGUCAGACUCCAGGA | 18 | 8066 |
| myoC-8321 | - | AUCACGUCAGACUCCAGGA | 19 | 8067 |
| myoC-1964 | - | GAUCACGUCAGACUCCAGGA | 20 | 2123 |
| myoC-8322 | - | UGAUCACGUCAGACUCCAGGA | 21 | 8068 |
| myoC-8323 | - | CUGAUCACGUCAGACUCCAGGA | 22 | 8069 |
| myoC-8324 | - | ACUGAUCACGUCAGACUCCAGGA | 23 | 8070 |
| myoC-8325 | - | CACUGAUCACGUCAGACUCCAGGA | 24 | 8071 |
| myoC-8326 | - | GGGGAUGUUGAGAGGGGA | 18 | 8072 |
| myoC-8327 | - | GGGGGAUGUUGAGAGGGGA | 19 | 8073 |
| myoC-1043 | - | GGGGGGAUGUUGAGAGGGGA | 20 | 1343 |
| myoC-8328 | - | AGGGGGGAUGUUGAGAGGGGA | 21 | 8074 |
| myoC-8329 | - | GAGGGGGGAUGUUGAGAGGGGA | 22 | 8075 |
| myoC-8330 | - | UGAGGGGGGAUGUUGAGAGGGGA | 23 | 8076 |
| myoC-8331 | - | GUGAGGGGGGAUGUUGAGAGGGGA | 24 | 8077 |
| myoC-8332 | - | AGGGGAGGUGGAGGGGGA | 18 | 8078 |
| myoC-8333 | - | CAGGGGAGGUGGAGGGGGA | 19 | 8079 |
| myoC-1959 | - | ACAGGGGAGGUGGAGGGGGA | 20 | 2119 |
| myoC-8334 | - | CACAGGGGAGGUGGAGGGGGA | 21 | 8080 |
| myoC-8335 | - | CCACAGGGGAGGUGGAGGGGGA | 22 | 8081 |
| myoC-8336 | - | GCCACAGGGGAGGUGGAGGGGGA | 23 | 8082 |
| myoC-8337 | - | AGCCACAGGGGAGGUGGAGGGGGA | 24 | 8083 |
| myoC-8338 | - | AGCCACAGGGGAGGUGGA | 18 | 8084 |
| myoC-8339 | - | GAGCCACAGGGGAGGUGGA | 19 | 8085 |
| myoC-1052 | - | UGAGCCACAGGGGAGGUGGA | 20 | 1352 |
| myoC-8340 | - | CUGAGCCACAGGGGAGGUGGA | 21 | 8086 |
| myoC-8341 | - | GCUGAGCCACAGGGGAGGUGGA | 22 | 8087 |
| myoC-8342 | - | AGCUGAGCCACAGGGGAGGUGGA | 23 | 8088 |
| myoC-8343 | - | CAGCUGAGCCACAGGGGAGGUGGA | 24 | 8089 |
| myoC-8344 | - | UUUUAAAGCUAGGGGUGA | 18 | 8090 |
| myoC-8345 | - | GUUUUAAAGCUAGGGGUGA | 19 | 8091 |
| myoC-2070 | - | UGUUUUAAAGCUAGGGGUGA | 20 | 2201 |
| myoC-8346 | - | UUGUUUUAAAGCUAGGGGUGA | 21 | 8092 |
| myoC-8347 | - | GUUGUUUUAAAGCUAGGGGUGA | 22 | 8093 |
| myoC-8348 | - | AGUUGUUUUAAAGCUAGGGGUGA | 23 | 8094 |
| myoC-8349 | - | CAGUUGUUUUAAAGCUAGGGGUGA | 24 | 8095 |
| myoC-8350 | - | CCCUGUGAUUCUCUGUGA | 18 | 8096 |
| myoC-8351 | - | UCCCUGUGAUUCUCUGUGA | 19 | 8097 |
| myoC-1036 | - | UUCCCUGUGAUUCUCUGUGA | 20 | 1336 |
| myoC-8352 | - | CUUCCCUGUGAUUCUCUGUGA | 21 | 8098 |
| myoC-8353 | - | ACUUCCCUGUGAUUCUCUGUGA | 22 | 8099 |
| myoC-8354 | - | CACUUCCCUGUGAUUCUCUGUGA | 23 | 8100 |
| myoC-8355 | - | ACACUUCCCUGUGAUUCUCUGUGA | 24 | 8101 |
| myoC-8356 | - | UGUGAGGGGGGAUGUUGA | 18 | 8102 |
| myoC-8357 | - | CUGUGAGGGGGGAUGUUGA | 19 | 8103 |
| myoC-1939 | - | UCUGUGAGGGGGGAUGUUGA | 20 | 2111 |
| myoC-8358 | - | CUCUGUGAGGGGGGAUGUUGA | 21 | 8104 |
| myoC-8359 | - | UCUCUGUGAGGGGGGAUGUUGA | 22 | 8105 |
| myoC-8360 | - | UUCUCUGUGAGGGGGGAUGUUGA | 23 | 8106 |
| myoC-8361 | - | AUUCUCUGUGAGGGGGGAUGUUGA | 24 | 8107 |
| myoC-8362 | - | GAAAGCCUGUGAAUUUGA | 18 | 8108 |
| myoC-8363 | - | AGAAAGCCUGUGAAUUUGA | 19 | 8109 |
| myoC-2045 | - | CAGAAAGCCUGUGAAUUUGA | 20 | 2177 |
| myoC-8364 | - | CCAGAAAGCCUGUGAAUUUGA | 21 | 8110 |
| myoC-8365 | - | UCCAGAAAGCCUGUGAAUUUGA | 22 | 8111 |
| myoC-8366 | - | GUCCAGAAAGCCUGUGAAUUUGA | 23 | 8112 |
| myoC-8367 | - | AGUCCAGAAAGCCUGUGAAUUUGA | 24 | 8113 |
| myoC-8368 | - | GGAAAUCUGCCGCUUCUA | 18 | 8114 |
| myoC-8369 | - | GGGAAAUCUGCCGCUUCUA | 19 | 8115 |
| myoC-2074 | - | GGGGAAAUCUGCCGCUUCUA | 20 | 2205 |
| myoC-8370 | - | GGGGGAAAUCUGCCGCUUCUA | 21 | 8116 |
| myoC-8371 | - | GGGGGGAAAUCUGCCGCUUCUA | 22 | 8117 |
| myoC-8372 | - | AGGGGGGAAAUCUGCCGCUUCUA | 23 | 8118 |
| myoC-8373 | - | GAGGGGGGAAAUCUGCCGCUUCUA | 24 | 8119 |
| myoC-8374 | - | CACAAGACAGAUGAAUUA | 18 | 8120 |
| myoC-8375 | - | GCACAAGACAGAUGAAUUA | 19 | 8121 |
| myoC-5461 | - | AGCACAAGACAGAUGAAUUA | 20 | 5207 |
| myoC-8376 | - | UAGCACAAGACAGAUGAAUUA | 21 | 8122 |
| myoC-8377 | - | CUAGCACAAGACAGAUGAAUUA | 22 | 8123 |
| myoC-8378 | - | GCUAGCACAAGACAGAUGAAUUA | 23 | 8124 |
| myoC-8379 | - | AGCUAGCACAAGACAGAUGAAUUA | 24 | 8125 |
| myoC-8380 | - | AAUCCCGUUUCUUUUAAC | 18 | 8126 |
| myoC-8381 | - | CAAUCCCGUUUCUUUUAAC | 19 | 8127 |
| myoC-1151 | - | GCAAUCCCGUUUCUUUUAAC | 20 | 1451 |
| myoC-8382 | - | GGCAAUCCCGUUUCUUUUAAC | 21 | 8128 |
| myoC-8383 | - | GGGCAAUCCCGUUUCUUUUAAC | 22 | 8129 |
| myoC-8384 | - | AGGGCAAUCCCGUUUCUUUUAAC | 23 | 8130 |
| myoC-8385 | - | AAGGGCAAUCCCGUUUCUUUUAAC | 24 | 8131 |
| myoC-8386 | - | CUGGAGCAGCUGAGCCAC | 18 | 8132 |
| myoC-8387 | - | GCUGGAGCAGCUGAGCCAC | 19 | 8133 |
| myoC-1046 | - | AGCUGGAGCAGCUGAGCCAC | 20 | 1346 |
| myoC-8388 | - | GAGCUGGAGCAGCUGAGCCAC | 21 | 8134 |
| myoC-8389 | - | AGAGCUGGAGCAGCUGAGCCAC | 22 | 8135 |
| myoC-8390 | - | CAGAGCUGGAGCAGCUGAGCCAC | 23 | 8136 |
| myoC-8391 | - | GCAGAGCUGGAGCAGCUGAGCCAC | 24 | 8137 |
| myoC-8392 | - | CUGUGGAGUUAGCAGCAC | 18 | 8138 |
| myoC-8393 | - | UCUGUGGAGUUAGCAGCAC | 19 | 8139 |
| myoC-2021 | - | CUCUGUGGAGUUAGCAGCAC | 20 | 2157 |
| myoC-8394 | - | UCUCUGUGGAGUUAGCAGCAC | 21 | 8140 |
| myoC-8395 | - | UUCUCUGUGGAGUUAGCAGCAC | 22 | 8141 |
| myoC-8396 | - | UUUCUCUGUGGAGUUAGCAGCAC | 23 | 8142 |
| myoC-8397 | - | UUUUCUCUGUGGAGUUAGCAGCAC | 24 | 8143 |
| myoC-4765 | - | GGGCCAGUGUCCCCAGAC | 18 | 4511 |
| myoC-4766 | - | GGGGCCAGUGUCCCCAGAC | 19 | 4512 |
| myoC-1659 | - | AGGGGCCAGUGUCCCCAGAC | 20 | 1921 |
| myoC-4767 | - | AAGGGGCCAGUGUCCCCAGAC | 21 | 4513 |
| myoC-4768 | - | GAAGGGGCCAGUGUCCCCAGAC | 22 | 4514 |
| myoC-4769 | - | AGAAGGGGCCAGUGUCCCCAGAC | 23 | 4515 |
| myoC-4770 | - | GAGAAGGGGCCAGUGUCCCCAGAC | 24 | 4516 |
| myoC-8398 | - | GGGGAGGUGGAGGGGGAC | 18 | 8144 |
| myoC-8399 | - | AGGGGAGGUGGAGGGGGAC | 19 | 8145 |
| myoC-1055 | - | CAGGGGAGGUGGAGGGGGAC | 20 | 1355 |
| myoC-8400 | - | ACAGGGGAGGUGGAGGGGGAC | 21 | 8146 |
| myoC-8401 | - | CACAGGGGAGGUGGAGGGGGAC | 22 | 8147 |
| myoC-8402 | - | CCACAGGGGAGGUGGAGGGGGAC | 23 | 8148 |
| myoC-8403 | - | GCCACAGGGGAGGUGGAGGGGGAC | 24 | 8149 |
| myoC-8404 | - | GCAAGACGGUCGAAAACC | 18 | 8150 |
| myoC-8405 | - | UGCAAGACGGUCGAAAACC | 19 | 8151 |
| myoC-1924 | - | AUGCAAGACGGUCGAAAACC | 20 | 2102 |
| myoC-8406 | - | UAUGCAAGACGGUCGAAAACC | 21 | 8152 |
| myoC-8407 | - | UUAUGCAAGACGGUCGAAAACC | 22 | 8153 |
| myoC-8408 | - | CUUAUGCAAGACGGUCGAAAACC | 23 | 8154 |
| myoC-8409 | - | GCUUAUGCAAGACGGUCGAAAACC | 24 | 8155 |
| myoC-8410 | - | UUGGUUGGCUGUGCGACC | 18 | 8156 |
| myoC-8411 | - | AUUGGUUGGCUGUGCGACC | 19 | 8157 |
| myoC-1928 | - | CAUUGGUUGGCUGUGCGACC | 20 | 2104 |
| myoC-8412 | - | CCAUUGGUUGGCUGUGCGACC | 21 | 8158 |
| myoC-8413 | - | GCCAUUGGUUGGCUGUGCGACC | 22 | 8159 |
| myoC-8414 | - | UGCCAUUGGUUGGCUGUGCGACC | 23 | 8160 |
| myoC-8415 | - | CUGCCAUUGGUUGGCUGUGCGACC | 24 | 8161 |
| myoC-8416 | - | ACGUCAGACUCCAGGACC | 18 | 8162 |
| myoC-8417 | - | CACGUCAGACUCCAGGACC | 19 | 8163 |
| myoC-1965 | - | UCACGUCAGACUCCAGGACC | 20 | 2124 |
| myoC-8418 | - | AUCACGUCAGACUCCAGGACC | 21 | 8164 |
| myoC-8419 | - | GAUCACGUCAGACUCCAGGACC | 22 | 8165 |
| myoC-8420 | - | UGAUCACGUCAGACUCCAGGACC | 23 | 8166 |
| myoC-8421 | - | CUGAUCACGUCAGACUCCAGGACC | 24 | 8167 |
| myoC-8422 | - | CUAUAGGAAUGCUCUCCC | 18 | 8168 |
| myoC-8423 | - | UCUAUAGGAAUGCUCUCCC | 19 | 8169 |
| myoC-1211 | - | UUCUAUAGGAAUGCUCUCCC | 20 | 1511 |
| myoC-8424 | - | CUUCUAUAGGAAUGCUCUCCC | 21 | 8170 |
| myoC-8425 | - | GCUUCUAUAGGAAUGCUCUCCC | 22 | 8171 |
| myoC-8426 | - | CGCUUCUAUAGGAAUGCUCUCCC | 23 | 8172 |
| myoC-8427 | - | CCGCUUCUAUAGGAAUGCUCUCCC | 24 | 8173 |
| myoC-3549 | - | GGUUGGAAAGCAGCAGCC | 18 | 3295 |
| myoC-3550 | - | AGGUUGGAAAGCAGCAGCC | 19 | 3296 |
| myoC-107 | - | GAGGUUGGAAAGCAGCAGCC | 20 | 511 |
| myoC-3551 | - | GGAGGUUGGAAAGCAGCAGCC | 21 | 3297 |
| myoC-3552 | - | AGGAGGUUGGAAAGCAGCAGCC | 22 | 3298 |
| myoC-3553 | - | CAGGAGGUUGGAAAGCAGCAGCC | 23 | 3299 |
| myoC-3554 | - | CCAGGAGGUUGGAAAGCAGCAGCC | 24 | 3300 |
| myoC-3555 | - | GAAAAUGAGAAUCUGGCC | 18 | 3301 |
| myoC-3556 | - | AGAAAAUGAGAAUCUGGCC | 19 | 3302 |
| myoC-195 | - | AAGAAAAUGAGAAUCUGGCC | 20 | 581 |
| myoC-3557 | - | CAAGAAAAUGAGAAUCUGGCC | 21 | 3303 |
| myoC-3558 | - | GCAAGAAAAUGAGAAUCUGGCC | 22 | 3304 |
| myoC-3559 | - | GGCAAGAAAAUGAGAAUCUGGCC | 23 | 3305 |
| myoC-3560 | - | AGGCAAGAAAAUGAGAAUCUGGCC | 24 | 3306 |
| myoC-8428 | - | UCUAUAGGAAUGCUCUCC | 18 | 8174 |
| myoC-8429 | - | UUCUAUAGGAAUGCUCUCC | 19 | 8175 |
| myoC-2076 | - | CUUCUAUAGGAAUGCUCUCC | 20 | 2206 |
| myoC-8430 | - | GCUUCUAUAGGAAUGCUCUCC | 21 | 8176 |
| myoC-8431 | - | CGCUUCUAUAGGAAUGCUCUCC | 22 | 8177 |
| myoC-8432 | - | CCGCUUCUAUAGGAAUGCUCUCC | 23 | 8178 |
| myoC-8433 | - | GCCGCUUCUAUAGGAAUGCUCUCC | 24 | 8179 |
| myoC-8434 | - | CCUGCCUGCCCUUUCUCC | 18 | 8180 |
| myoC-8435 | - | CCCUGCCUGCCCUUUCUCC | 19 | 8181 |
| myoC-8436 | - | UCCCUGCCUGCCCUUUCUCC | 20 | 8182 |
| myoC-8437 | - | CUCCCUGCCUGCCCUUUCUCC | 21 | 8183 |
| myoC-8438 | - | CCUCCCUGCCUGCCCUUUCUCC | 22 | 8184 |
| myoC-8439 | - | GCCUCCCUGCCUGCCCUUUCUCC | 23 | 8185 |
| myoC-8440 | - | GGCCUCCCUGCCUGCCCUUUCUCC | 24 | 8186 |
| myoC-8441 | - | GCAAGUGUCUCUCCUUCC | 18 | 8187 |
| myoC-8442 | - | GGCAAGUGUCUCUCCUUCC | 19 | 8188 |
| myoC-1929 | - | GGGCAAGUGUCUCUCCUUCC | 20 | 2105 |
| myoC-8443 | - | UGGGCAAGUGUCUCUCCUUCC | 21 | 8189 |
| myoC-8444 | - | GUGGGCAAGUGUCUCUCCUUCC | 22 | 8190 |
| myoC-8445 | - | CGUGGGCAAGUGUCUCUCCUUCC | 23 | 8191 |
| myoC-8446 | - | CCGUGGGCAAGUGUCUCUCCUUCC | 24 | 8192 |
| myoC-8447 | - | ACACAGUUGUUUUAAAGC | 18 | 8193 |
| myoC-8448 | - | UACACAGUUGUUUUAAAGC | 19 | 8194 |
| myoC-2065 | - | AUACACAGUUGUUUUAAAGC | 20 | 2196 |
| myoC-8449 | - | GAUACACAGUUGUUUUAAAGC | 21 | 8195 |
| myoC-8450 | - | AGAUACACAGUUGUUUUAAAGC | 22 | 8196 |
| myoC-8451 | - | AAGAUACACAGUUGUUUUAAAGC | 23 | 8197 |
| myoC-8452 | - | AAAGAUACACAGUUGUUUUAAAGC | 24 | 8198 |
| myoC-8453 | - | GCAGUGACUGCUGACAGC | 18 | 8199 |
| myoC-8454 | - | AGCAGUGACUGCUGACAGC | 19 | 8200 |
| myoC-1976 | - | CAGCAGUGACUGCUGACAGC | 20 | 2133 |
| myoC-8455 | - | UCAGCAGUGACUGCUGACAGC | 21 | 8201 |
| myoC-8456 | - | CUCAGCAGUGACUGCUGACAGC | 22 | 8202 |
| myoC-8457 | - | GCUCAGCAGUGACUGCUGACAGC | 23 | 8203 |
| myoC-8458 | - | AGCUCAGCAGUGACUGCUGACAGC | 24 | 8204 |
| myoC-3579 | - | AGGUUGGAAAGCAGCAGC | 18 | 3325 |
| myoC-3580 | - | GAGGUUGGAAAGCAGCAGC | 19 | 3326 |
| myoC-1653 | - | GGAGGUUGGAAAGCAGCAGC | 20 | 1917 |
| myoC-3581 | - | AGGAGGUUGGAAAGCAGCAGC | 21 | 3327 |
| myoC-3582 | - | CAGGAGGUUGGAAAGCAGCAGC | 22 | 3328 |
| myoC-3583 | - | CCAGGAGGUUGGAAAGCAGCAGC | 23 | 3329 |
| myoC-3584 | - | GCCAGGAGGUUGGAAAGCAGCAGC | 24 | 3330 |
| myoC-8459 | - | AGGGGAAGGAGGCAGAGC | 18 | 8205 |
| myoC-8460 | - | GAGGGGAAGGAGGCAGAGC | 19 | 8206 |
| myoC-1045 | - | AGAGGGGAAGGAGGCAGAGC | 20 | 1345 |
| myoC-8461 | - | GAGAGGGGAAGGAGGCAGAGC | 21 | 8207 |
| myoC-8462 | - | UGAGAGGGGAAGGAGGCAGAGC | 22 | 8208 |
| myoC-8463 | - | UUGAGAGGGGAAGGAGGCAGAGC | 23 | 8209 |
| myoC-8464 | - | GUUGAGAGGGGAAGGAGGCAGAGC | 24 | 8210 |
| myoC-8465 | - | GAGGGAUAGUGUAUGAGC | 18 | 8211 |
| myoC-8466 | - | AGAGGGAUAGUGUAUGAGC | 19 | 8212 |
| myoC-1991 | - | GAGAGGGAUAGUGUAUGAGC | 20 | 2142 |
| myoC-8467 | - | AGAGAGGGAUAGUGUAUGAGC | 21 | 8213 |
| myoC-8468 | - | AAGAGAGGGAUAGUGUAUGAGC | 22 | 8214 |
| myoC-8469 | - | AAAGAGAGGGAUAGUGUAUGAGC | 23 | 8215 |
| myoC-8470 | - | AAAAGAGAGGGAUAGUGUAUGAGC | 24 | 8216 |
| myoC-8471 | - | CGGAGUGACCUGCAGCGC | 18 | 8217 |
| myoC-8472 | - | ACGGAGUGACCUGCAGCGC | 19 | 8218 |
| myoC-1118 | - | CACGGAGUGACCUGCAGCGC | 20 | 1418 |
| myoC-8473 | - | GCACGGAGUGACCUGCAGCGC | 21 | 8219 |
| myoC-8474 | - | AGCACGGAGUGACCUGCAGCGC | 22 | 8220 |
| myoC-8475 | - | CAGCACGGAGUGACCUGCAGCGC | 23 | 8221 |
| myoC-8476 | - | ACAGCACGGAGUGACCUGCAGCGC | 24 | 8222 |
| myoC-3585 | - | AGAAGAAGCGACUAAGGC | 18 | 3331 |
| myoC-3586 | - | GAGAAGAAGCGACUAAGGC | 19 | 3332 |
| myoC-1646 | - | AGAGAAGAAGCGACUAAGGC | 20 | 1912 |
| myoC-3587 | - | AAGAGAAGAAGCGACUAAGGC | 21 | 3333 |
| myoC-3588 | - | GAAGAGAAGAAGCGACUAAGGC | 22 | 3334 |
| myoC-3589 | - | GGAAGAGAAGAAGCGACUAAGGC | 23 | 3335 |
| myoC-3590 | - | AGGAAGAGAAGAAGCGACUAAGGC | 24 | 3336 |
| myoC-8477 | - | GGCAUUCAAAAACUGGGC | 18 | 8223 |
| myoC-8478 | - | UGGCAUUCAAAAACUGGGC | 19 | 8224 |
| myoC-1972 | - | CUGGCAUUCAAAAACUGGGC | 20 | 2130 |
| myoC-8479 | - | CCUGGCAUUCAAAAACUGGGC | 21 | 8225 |
| myoC-8480 | - | GCCUGGCAUUCAAAAACUGGGC | 22 | 8226 |
| myoC-8481 | - | UGCCUGGCAUUCAAAAACUGGGC | 23 | 8227 |
| myoC-8482 | - | UUGCCUGGCAUUCAAAAACUGGGC | 24 | 8228 |
| myoC-3609 | - | AGAAAAUGAGAAUCUGGC | 18 | 3355 |
| myoC-3610 | - | AAGAAAAUGAGAAUCUGGC | 19 | 3356 |
| myoC-1649 | - | CAAGAAAAUGAGAAUCUGGC | 20 | 1915 |
| myoC-3611 | - | GCAAGAAAAUGAGAAUCUGGC | 21 | 3357 |
| myoC-3612 | - | GGCAAGAAAAUGAGAAUCUGGC | 22 | 3358 |
| myoC-3613 | - | AGGCAAGAAAAUGAGAAUCUGGC | 23 | 3359 |
| myoC-3614 | - | AAGGCAAGAAAAUGAGAAUCUGGC | 24 | 3360 |
| myoC-8483 | - | AGAGCAAGUGGAAAAUGC | 18 | 8229 |
| myoC-8484 | - | CAGAGCAAGUGGAAAAUGC | 19 | 8230 |
| myoC-1975 | - | CCAGAGCAAGUGGAAAAUGC | 20 | 2132 |
| myoC-8485 | - | GCCAGAGCAAGUGGAAAAUGC | 21 | 8231 |
| myoC-8486 | - | GGCCAGAGCAAGUGGAAAAUGC | 22 | 8232 |
| myoC-8487 | - | GGGCCAGAGCAAGUGGAAAAUGC | 23 | 8233 |
| myoC-8488 | - | UGGGCCAGAGCAAGUGGAAAAUGC | 24 | 8234 |
| myoC-4878 | - | CCAGACCCGAGACACUGC | 18 | 4624 |
| myoC-4879 | - | CCCAGACCCGAGACACUGC | 19 | 4625 |
| myoC-1660 | - | CCCCAGACCCGAGACACUGC | 20 | 1922 |
| myoC-4880 | - | UCCCCAGACCCGAGACACUGC | 21 | 4626 |
| myoC-4881 | - | GUCCCCAGACCCGAGACACUGC | 22 | 4627 |
| myoC-4882 | - | UGUCCCCAGACCCGAGACACUGC | 23 | 4628 |
| myoC-4883 | - | GUGUCCCCAGACCCGAGACACUGC | 24 | 4629 |
| myoC-8489 | - | CUCUGGAGGUGAGUCUGC | 18 | 8235 |
| myoC-8490 | - | ACUCUGGAGGUGAGUCUGC | 19 | 8236 |
| myoC-2036 | - | CACUCUGGAGGUGAGUCUGC | 20 | 2170 |
| myoC-8491 | - | CCACUCUGGAGGUGAGUCUGC | 21 | 8237 |
| myoC-8492 | - | UCCACUCUGGAGGUGAGUCUGC | 22 | 8238 |
| myoC-8493 | - | CUCCACUCUGGAGGUGAGUCUGC | 23 | 8239 |
| myoC-8494 | - | UCUCCACUCUGGAGGUGAGUCUGC | 24 | 8240 |
| myoC-8495 | - | UAACAUUGACAUUGGUGC | 18 | 8241 |
| myoC-8496 | - | CUAACAUUGACAUUGGUGC | 19 | 8242 |
| myoC-2062 | - | GCUAACAUUGACAUUGGUGC | 20 | 2193 |
| myoC-8497 | - | GGCUAACAUUGACAUUGGUGC | 21 | 8243 |
| myoC-8498 | - | AGGCUAACAUUGACAUUGGUGC | 22 | 8244 |
| myoC-8499 | - | GAGGCUAACAUUGACAUUGGUGC | 23 | 8245 |
| myoC-8500 | - | AGAGGCUAACAUUGACAUUGGUGC | 24 | 8246 |
| myoC-8501 | - | GUCGAAAACCUUGGAAUC | 18 | 8247 |
| myoC-8502 | - | GGUCGAAAACCUUGGAAUC | 19 | 8248 |
| myoC-1026 | - | CGGUCGAAAACCUUGGAAUC | 20 | 1326 |
| myoC-8503 | - | ACGGUCGAAAACCUUGGAAUC | 21 | 8249 |
| myoC-8504 | - | GACGGUCGAAAACCUUGGAAUC | 22 | 8250 |
| myoC-8505 | - | AGACGGUCGAAAACCUUGGAAUC | 23 | 8251 |
| myoC-8506 | - | AAGACGGUCGAAAACCUUGGAAUC | 24 | 8252 |
| myoC-8507 | - | AACUGUGUUUCUCCACUC | 18 | 8253 |
| myoC-8508 | - | AAACUGUGUUUCUCCACUC | 19 | 8254 |
| myoC-1156 | - | CAAACUGUGUUUCUCCACUC | 20 | 1456 |
| myoC-8509 | - | GCAAACUGUGUUUCUCCACUC | 21 | 8255 |
| myoC-8510 | - | AGCAAACUGUGUUUCUCCACUC | 22 | 8256 |
| myoC-8511 | - | GAGCAAACUGUGUUUCUCCACUC | 23 | 8257 |
| myoC-8512 | - | AGAGCAAACUGUGUUUCUCCACUC | 24 | 8258 |
| myoC-8513 | - | ACUGAUCACGUCAGACUC | 18 | 8259 |
| myoC-8514 | - | CACUGAUCACGUCAGACUC | 19 | 8260 |
| myoC-1963 | - | UCACUGAUCACGUCAGACUC | 20 | 2122 |
| myoC-8515 | - | CUCACUGAUCACGUCAGACUC | 21 | 8261 |
| myoC-8516 | - | CCUCACUGAUCACGUCAGACUC | 22 | 8262 |
| myoC-8517 | - | UCCUCACUGAUCACGUCAGACUC | 23 | 8263 |
| myoC-8518 | - | GUCCUCACUGAUCACGUCAGACUC | 24 | 8264 |
| myoC-8519 | - | UUACUAGUAAUUUAGCUC | 18 | 8265 |
| myoC-8520 | - | AUUACUAGUAAUUUAGCUC | 19 | 8266 |
| myoC-8521 | - | UAUUACUAGUAAUUUAGCUC | 20 | 8267 |
| myoC-8522 | - | GUAUUACUAGUAAUUUAGCUC | 21 | 8268 |
| myoC-8523 | - | AGUAUUACUAGUAAUUUAGCUC | 22 | 8269 |
| myoC-8524 | - | AAGUAUUACUAGUAAUUUAGCUC | 23 | 8270 |
| myoC-8525 | - | CAAGUAUUACUAGUAAUUUAGCUC | 24 | 8271 |
| myoC-4908 | - | GGCUGUGCCACCAGGCUC | 18 | 4654 |
| myoC-4909 | - | GGGCUGUGCCACCAGGCUC | 19 | 4655 |
| myoC-1661 | - | CGGGCUGUGCCACCAGGCUC | 20 | 1923 |
| myoC-4910 | - | UCGGGCUGUGCCACCAGGCUC | 21 | 4656 |
| myoC-4911 | - | CUCGGGCUGUGCCACCAGGCUC | 22 | 4657 |
| myoC-4912 | - | GCUCGGGCUGUGCCACCAGGCUC | 23 | 4658 |
| myoC-4913 | - | UGCUCGGGCUGUGCCACCAGGCUC | 24 | 4659 |
| myoC-8526 | - | AUCAGUUCAAGGGAAGUC | 18 | 8272 |
| myoC-8527 | - | AAUCAGUUCAAGGGAAGUC | 19 | 8273 |
| myoC-1144 | - | AAAUCAGUUCAAGGGAAGUC | 20 | 1444 |
| myoC-8528 | - | AAAAUCAGUUCAAGGGAAGUC | 21 | 8274 |
| myoC-8529 | - | AAAAAUCAGUUCAAGGGAAGUC | 22 | 8275 |
| myoC-8530 | - | AAAAAAUCAGUUCAAGGGAAGUC | 23 | 8276 |
| myoC-8531 | - | GAAAAAAUCAGUUCAAGGGAAGUC | 24 | 8277 |
| myoC-8532 | - | GAUUAUUAACCUACAGUC | 18 | 8278 |
| myoC-8533 | - | GGAUUAUUAACCUACAGUC | 19 | 8279 |
| myoC-2042 | - | GGGAUUAUUAACCUACAGUC | 20 | 2174 |
| myoC-8534 | - | AGGGAUUAUUAACCUACAGUC | 21 | 8280 |
| myoC-8535 | - | CAGGGAUUAUUAACCUACAGUC | 22 | 8281 |
| myoC-8536 | - | GCAGGGAUUAUUAACCUACAGUC | 23 | 8282 |
| myoC-8537 | - | AGCAGGGAUUAUUAACCUACAGUC | 24 | 8283 |
| myoC-8538 | - | AGAAAGACAGAUUCAUUC | 18 | 8284 |
| myoC-8539 | - | AAGAAAGACAGAUUCAUUC | 19 | 8285 |
| myoC-1992 | - | CAAGAAAGACAGAUUCAUUC | 20 | 2143 |
| myoC-8540 | - | GCAAGAAAGACAGAUUCAUUC | 21 | 8286 |
| myoC-8541 | - | AGCAAGAAAGACAGAUUCAUUC | 22 | 8287 |
| myoC-8542 | - | GAGCAAGAAAGACAGAUUCAUUC | 23 | 8288 |
| myoC-8543 | - | UGAGCAAGAAAGACAGAUUCAUUC | 24 | 8289 |
| myoC-8544 | - | CGAGAGCCACAAUGCUUC | 18 | 8290 |
| myoC-8545 | - | CCGAGAGCCACAAUGCUUC | 19 | 8291 |
| myoC-1061 | - | ACCGAGAGCCACAAUGCUUC | 20 | 1361 |
| myoC-8546 | - | GACCGAGAGCCACAAUGCUUC | 21 | 8292 |
| myoC-8547 | - | GGACCGAGAGCCACAAUGCUUC | 22 | 8293 |
| myoC-8548 | - | AGGACCGAGAGCCACAAUGCUUC | 23 | 8294 |
| myoC-8549 | - | CAGGACCGAGAGCCACAAUGCUUC | 24 | 8295 |
| myoC-8550 | - | GGGGGAAAAAAUCAGUUC | 18 | 8296 |
| myoC-8551 | - | GGGGGGAAAAAAUCAGUUC | 19 | 8297 |
| myoC-2008 | - | UGGGGGGAAAAAAUCAGUUC | 20 | 2150 |
| myoC-8552 | - | GUGGGGGGAAAAAAUCAGUUC | 21 | 8298 |
| myoC-8553 | - | UGUGGGGGGAAAAAAUCAGUUC | 22 | 8299 |
| myoC-8554 | - | UUGUGGGGGGAAAAAAUCAGUUC | 23 | 8300 |
| myoC-8555 | - | AUUGUGGGGGGAAAAAAUCAGUUC | 24 | 8301 |
| myoC-8556 | - | CCACGUGAUCCUGGGUUC | 18 | 8302 |
| myoC-8557 | - | UCCACGUGAUCCUGGGUUC | 19 | 8303 |
| myoC-1999 | - | GUCCACGUGAUCCUGGGUUC | 20 | 2147 |
| myoC-8558 | - | AGUCCACGUGAUCCUGGGUUC | 21 | 8304 |
| myoC-8559 | - | UAGUCCACGUGAUCCUGGGUUC | 22 | 8305 |
| myoC-8560 | - | AUAGUCCACGUGAUCCUGGGUUC | 23 | 8306 |
| myoC-8561 | - | UAUAGUCCACGUGAUCCUGGGUUC | 24 | 8307 |
| myoC-8562 | - | CACAGUCCAUAGCAAAAG | 18 | 8308 |
| myoC-8563 | - | UCACAGUCCAUAGCAAAAG | 19 | 8309 |
| myoC-8564 | - | UUCACAGUCCAUAGCAAAAG | 20 | 8310 |
| myoC-8565 | - | UUUCACAGUCCAUAGCAAAAG | 21 | 8311 |
| myoC-8566 | - | UUUUCACAGUCCAUAGCAAAAG | 22 | 8312 |
| myoC-8567 | - | GUUUUCACAGUCCAUAGCAAAAG | 23 | 8313 |
| myoC-8568 | - | AGUUUUCACAGUCCAUAGCAAAAG | 24 | 8314 |
| myoC-8569 | - | GGGUUUAUUAAUGUAAAG | 18 | 8315 |
| myoC-8570 | - | UGGGUUUAUUAAUGUAAAG | 19 | 8316 |
| myoC-2040 | - | UUGGGUUUAUUAAUGUAAAG | 20 | 2173 |
| myoC-8571 | - | UUUGGGUUUAUUAAUGUAAAG | 21 | 8317 |
| myoC-8572 | - | CUUUGGGUUUAUUAAUGUAAAG | 22 | 8318 |
| myoC-8573 | - | UCUUUGGGUUUAUUAAUGUAAAG | 23 | 8319 |
| myoC-8574 | - | CUCUUUGGGUUUAUUAAUGUAAAG | 24 | 8320 |
| myoC-8575 | - | AAACUGGGCCAGAGCAAG | 18 | 8321 |
| myoC-8576 | - | AAAACUGGGCCAGAGCAAG | 19 | 8322 |
| myoC-1068 | - | AAAAACUGGGCCAGAGCAAG | 20 | 1368 |
| myoC-8577 | - | CAAAAACUGGGCCAGAGCAAG | 21 | 8323 |
| myoC-8578 | - | UCAAAAACUGGGCCAGAGCAAG | 22 | 8324 |
| myoC-8579 | - | UUCAAAAACUGGGCCAGAGCAAG | 23 | 8325 |
| myoC-8580 | - | AUUCAAAAACUGGGCCAGAGCAAG | 24 | 8326 |
| myoC-8581 | - | AAAUCAGUUCAAGGGAAG | 18 | 8327 |
| myoC-8582 | - | AAAAUCAGUUCAAGGGAAG | 19 | 8328 |
| myoC-2011 | - | AAAAAUCAGUUCAAGGGAAG | 20 | 2151 |
| myoC-8583 | - | AAAAAAUCAGUUCAAGGGAAG | 21 | 8329 |
| myoC-8584 | - | GAAAAAAUCAGUUCAAGGGAAG | 22 | 8330 |
| myoC-8585 | - | GGAAAAAAUCAGUUCAAGGGAAG | 23 | 8331 |
| myoC-8586 | - | GGGAAAAAAUCAGUUCAAGGGAAG | 24 | 8332 |
| myoC-8587 | - | GGAGCAGCUGAGCCACAG | 18 | 8333 |
| myoC-8588 | - | UGGAGCAGCUGAGCCACAG | 19 | 8334 |
| myoC-1048 | - | CUGGAGCAGCUGAGCCACAG | 20 | 1348 |
| myoC-8589 | - | GCUGGAGCAGCUGAGCCACAG | 21 | 8335 |
| myoC-8590 | - | AGCUGGAGCAGCUGAGCCACAG | 22 | 8336 |
| myoC-8591 | - | GAGCUGGAGCAGCUGAGCCACAG | 23 | 8337 |
| myoC-8592 | - | AGAGCUGGAGCAGCUGAGCCACAG | 24 | 8338 |
| myoC-8593 | - | GAGGCAGAGCUGGAGCAG | 18 | 8339 |
| myoC-8594 | - | GGAGGCAGAGCUGGAGCAG | 19 | 8340 |
| myoC-1948 | - | AGGAGGCAGAGCUGGAGCAG | 20 | 2115 |
| myoC-8595 | - | AAGGAGGCAGAGCUGGAGCAG | 21 | 8341 |
| myoC-8596 | - | GAAGGAGGCAGAGCUGGAGCAG | 22 | 8342 |
| myoC-8597 | - | GGAAGGAGGCAGAGCUGGAGCAG | 23 | 8343 |
| myoC-8598 | - | GGGAAGGAGGCAGAGCUGGAGCAG | 24 | 8344 |
| myoC-8599 | - | GUGUCUGCAUAUGAGCAG | 18 | 8345 |
| myoC-8600 | - | UGUGUCUGCAUAUGAGCAG | 19 | 8346 |
| myoC-8601 | - | AUGUGUCUGCAUAUGAGCAG | 20 | 8347 |
| myoC-8602 | - | GAUGUGUCUGCAUAUGAGCAG | 21 | 8348 |
| myoC-8603 | - | AGAUGUGUCUGCAUAUGAGCAG | 22 | 8349 |
| myoC-8604 | - | GAGAUGUGUCUGCAUAUGAGCAG | 23 | 8350 |
| myoC-8605 | - | UGAGAUGUGUCUGCAUAUGAGCAG | 24 | 8351 |
| myoC-8606 | - | GAGUGACCUGCAGCGCAG | 18 | 8352 |
| myoC-8607 | - | GGAGUGACCUGCAGCGCAG | 19 | 8353 |
| myoC-1120 | - | CGGAG UGACCUGCAGCGCAG | 20 | 1420 |
| myoC-8608 | - | ACGGAGUGACCUGCAGCGCAG | 21 | 8354 |
| myoC-8609 | - | CACGGAGUGACCUGCAGCGCAG | 22 | 8355 |
| myoC-8610 | - | GCACGGAGUGACCUGCAGCGCAG | 23 | 8356 |
| myoC-8611 | - | AGCACGGAGUGACCUGCAGCGCAG | 24 | 8357 |
| myoC-8612 | - | AGAUUCAUUCAAGGGCAG | 18 | 8358 |
| myoC-8613 | - | CAGAUUCAUUCAAGGGCAG | 19 | 8359 |
| myoC-1126 | - | ACAGAUUCAUUCAAGGGCAG | 20 | 1426 |
| myoC-8614 | - | GACAGAUUCAUUCAAGGGCAG | 21 | 8360 |
| myoC-8615 | - | AGACAGAUUCAUUCAAGGGCAG | 22 | 8361 |
| myoC-8616 | - | AAGACAGAUUCAUUCAAGGGCAG | 23 | 8362 |
| myoC-8617 | - | AAAGACAGAUUCAUUCAAGGGCAG | 24 | 8363 |
| myoC-8618 | - | GAGGGGAAGGAGGCAGAG | 18 | 8364 |
| myoC-8619 | - | AGAGGGGAAGGAGGCAGAG | 19 | 8365 |
| myoC-1946 | - | GAGAGGGGAAGGAGGCAGAG | 20 | 2114 |
| myoC-8620 | - | UGAGAGGGGAAGGAGGCAGAG | 21 | 8366 |
| myoC-8621 | - | UUGAGAGGGGAAGGAGGCAGAG | 22 | 8367 |
| myoC-8622 | - | GUUGAGAGGGGAAGGAGGCAGAG | 23 | 8368 |
| myoC-8623 | - | UGUUGAGAGGGGAAGGAGGCAGAG | 24 | 8369 |
| myoC-4980 | - | GAAGGUAAGAAUGCAGAG | 18 | 4726 |
| myoC-4981 | - | AGAAGGUAAGAAUGCAGAG | 19 | 4727 |
| myoC-3185 | - | GAGAAGGUAAGAAUGCAGAG | 20 | 2931 |
| myoC-4982 | - | AGAGAAGGUAAGAAUGCAGAG | 21 | 4728 |
| myoC-4983 | - | CAGAGAAGGUAAGAAUGCAGAG | 22 | 4729 |
| myoC-4984 | - | CCAGAGAAGGUAAGAAUGCAGAG | 23 | 4730 |
| myoC-4985 | - | UCCAGAGAAGGUAAGAAUGCAGAG | 24 | 4731 |
| myoC-8624 | - | GAGGGGGGAUGUUGAGAG | 18 | 8370 |
| myoC-8625 | - | UGAGGGGGGAUGUUGAGAG | 19 | 8371 |
| myoC-1042 | - | GUGAGGGGGGAUGUUGAGAG | 20 | 1342 |
| myoC-8626 | - | UGUGAGGGGGGAUGUUGAGAG | 21 | 8372 |
| myoC-8627 | - | CUGUGAGGGGGGAUGUUGAGAG | 22 | 8373 |
| myoC-8628 | - | UCUGUGAGGGGGGAUGUUGAGAG | 23 | 8374 |
| myoC-8629 | - | CUCUGUGAGGGGGGAUGUUGAGAG | 24 | 8375 |
| myoC-8630 | - | UGCAGCGCAGGGGAGGAG | 18 | 8376 |
| myoC-8631 | - | CUGCAGCGCAGGGGAGGAG | 19 | 8377 |
| myoC-1985 | - | CCUGCAGCGCAGGGGAGGAG | 20 | 2137 |
| myoC-8632 | - | ACCUGCAGCGCAGGGGAGGAG | 21 | 8378 |
| myoC-8633 | - | GACCUGCAGCGCAGGGGAGGAG | 22 | 8379 |
| myoC-8634 | - | UGACCUGCAGCGCAGGGGAGGAG | 23 | 8380 |
| myoC-8635 | - | GUGACCUGCAGCGCAGGGGAGGAG | 24 | 8381 |
| myoC-8636 | - | AGCUGAGCCACAGGGGAG | 18 | 8382 |
| myoC-8637 | - | CAGCUGAGCCACAGGGGAG | 19 | 8383 |
| myoC-1953 | - | GCAGCUGAGCCACAGGGGAG | 20 | 2117 |
| myoC-8638 | - | AGCAGCUGAGCCACAGGGGAG | 21 | 8384 |
| myoC-8639 | - | GAGCAGCUGAGCCACAGGGGAG | 22 | 8385 |
| myoC-8640 | - | GGAGCAGCUGAGCCACAGGGGAG | 23 | 8386 |
| myoC-8641 | - | UGGAGCAGCUGAGCCACAGGGGAG | 24 | 8387 |
| myoC-8642 | - | ACCUGCAGCGCAGGGGAG | 18 | 8388 |
| myoC-8643 | - | GACCUGCAGCGCAGGGGAG | 19 | 8389 |
| myoC-1984 | - | UGACCUGCAGCGCAGGGGAG | 20 | 2136 |
| myoC-8644 | - | GUGACCUGCAGCGCAGGGGAG | 21 | 8390 |
| myoC-8645 | - | AGUGACCUGCAGCGCAGGGGAG | 22 | 8391 |
| myoC-8646 | - | GAGUGACCUGCAGCGCAGGGGAG | 23 | 8392 |
| myoC-8647 | - | GGAGUGACCUGCAGCGCAGGGGAG | 24 | 8393 |
| myoC-8648 | - | GCCACAGGGGAGGUGGAG | 18 | 8394 |
| myoC-8649 | - | AGCCACAGGGGAGGUGGAG | 19 | 8395 |
| myoC-1053 | - | GAGCCACAGGGGAGGUGGAG | 20 | 1353 |
| myoC-8650 | - | UGAGCCACAGGGGAGGUGGAG | 21 | 8396 |
| myoC-8651 | - | CUGAGCCACAGGGGAGGUGGAG | 22 | 8397 |
| myoC-8652 | - | GCUGAGCCACAGGGGAGGUGGAG | 23 | 8398 |
| myoC-8653 | - | AGCUGAGCCACAGGGGAGGUGGAG | 24 | 8399 |
| myoC-5004 | - | GGAGGUAGCAAGGCUGAG | 18 | 4750 |
| myoC-5005 | - | AGGAGGUAGCAAGGCUGAG | 19 | 4751 |
| myoC-1657 | - | CAGGAGGUAGCAAGGCUGAG | 20 | 1920 |
| myoC-5006 | - | CCAGGAGGUAGCAAGGCUGAG | 21 | 4752 |
| myoC-5007 | - | GCCAGGAGGUAGCAAGGCUGAG | 22 | 4753 |
| myoC-5008 | - | AGCCAGGAGGUAGCAAGGCUGAG | 23 | 4754 |
| myoC-5009 | - | CAGCCAGGAGGUAGCAAGGCUGAG | 24 | 4755 |
| myoC-8654 | - | UUUAAAGCUAGGGGUGAG | 18 | 8400 |
| myoC-8655 | - | UUUUAAAGCUAGGGGUGAG | 19 | 8401 |
| myoC-2071 | - | GUUUUAAAGCUAGGGGUGAG | 20 | 2202 |
| myoC-8656 | - | UGUUUUAAAGCUAGGGGUGAG | 21 | 8402 |
| myoC-8657 | - | UUGUUUUAAAGCUAGGGGUGAG | 22 | 8403 |
| myoC-8658 | - | GUUGUUUUAAAGCUAGGGGUGAG | 23 | 8404 |
| myoC-8659 | - | AGUUGUUUUAAAGCUAGGGGUGAG | 24 | 8405 |
| myoC-8660 | - | CCUGUGAUUCUCUGUGAG | 18 | 8406 |
| myoC-8661 | - | CCCUGUGAUUCUCUGUGAG | 19 | 8407 |
| myoC-1037 | - | UCCCUGUGAUUCUCUGUGAG | 20 | 1337 |
| myoC-8662 | - | UUCCCUGUGAUUCUCUGUGAG | 21 | 8408 |
| myoC-8663 | - | CUUCCCUGUGAUUCUCUGUGAG | 22 | 8409 |
| myoC-8664 | - | ACUUCCCUGUGAUUCUCUGUGAG | 23 | 8410 |
| myoC-8665 | - | CACUUCCCUGUGAUUCUCUGUGAG | 24 | 8411 |
| myoC-8666 | - | GUGAGGGGGGAUGUUGAG | 18 | 8412 |
| myoC-8667 | - | UGUGAGGGGGGAUGUUGAG | 19 | 8413 |
| myoC-1040 | - | CUGUGAGGGGGGAUGUUGAG | 20 | 1340 |
| myoC-8668 | - | UCUGUGAGGGGGGAUGUUGAG | 21 | 8414 |
| myoC-8669 | - | CUCUGUGAGGGGGGAUGUUGAG | 22 | 8415 |
| myoC-8670 | - | UCUCUGUGAGGGGGGAUGUUGAG | 23 | 8416 |
| myoC-8671 | - | UUCUCUGUGAGGGGGGAUGUUGAG | 24 | 8417 |
| myoC-8672 | - | ACGGAGUGACCUGCAGCG | 18 | 8418 |
| myoC-8673 | - | CACGGAGUGACCUGCAGCG | 19 | 8419 |
| myoC-1978 | - | GCACGGAGUGACCUGCAGCG | 20 | 2134 |
| myoC-8674 | - | AGCACGGAGUGACCUGCAGCG | 21 | 8420 |
| myoC-8675 | - | CAGCACGGAGUGACCUGCAGCG | 22 | 8421 |
| myoC-8676 | - | ACAGCACGGAGUGACCUGCAGCG | 23 | 8422 |
| myoC-8677 | - | GACAGCACGGAGUGACCUGCAGCG | 24 | 8423 |
| myoC-5048 | - | AGCCAGGAGGUAGCAAGG | 18 | 4794 |
| myoC-5049 | - | CAGCCAGGAGGUAGCAAGG | 19 | 4795 |
| myoC-1655 | - | GCAGCCAGGAGGUAGCAAGG | 20 | 1918 |
| myoC-5050 | - | AGCAGCCAGGAGGUAGCAAGG | 21 | 4796 |
| myoC-5051 | - | CAGCAGCCAGGAGGUAGCAAGG | 22 | 4797 |
| myoC-5052 | - | GCAGCAGCCAGGAGGUAGCAAGG | 23 | 4798 |
| myoC-5053 | - | AGCAGCAGCCAGGAGGUAGCAAGG | 24 | 4799 |
| myoC-8678 | - | CCCGUUUCUUUUAACAGG | 18 | 8424 |
| myoC-8679 | - | UCCCGUUUCUUUUAACAGG | 19 | 8425 |
| myoC-2024 | - | AUCCCGUUUCUUUUAACAGG | 20 | 2159 |
| myoC-8680 | - | AAUCCCGUUUCUUUUAACAGG | 21 | 8426 |
| myoC-8681 | - | CAAUCCCGUUUCUUUUAACAGG | 22 | 8427 |
| myoC-8682 | - | GCAAUCCCGUUUCUUUUAACAGG | 23 | 8428 |
| myoC-8683 | - | GGCAAUCCCGUUUCUUUUAACAGG | 24 | 8429 |
| myoC-8684 | - | GGGGGACAGGAAGGCAGG | 18 | 8430 |
| myoC-8685 | - | AGGGGGACAGGAAGGCAGG | 19 | 8431 |
| myoC-1961 | - | GAGGGGGACAGGAAGGCAGG | 20 | 2120 |
| myoC-8686 | - | GGAGGGGGACAGGAAGGCAGG | 21 | 8432 |
| myoC-8687 | - | UGGAGGGGGACAGGAAGGCAGG | 22 | 8433 |
| myoC-8688 | - | GUGGAGGGGGACAGGAAGGCAGG | 23 | 8434 |
| myoC-8689 | - | GGUGGAGGGGGACAGGAAGGCAGG | 24 | 8435 |
| myoC-8690 | - | GUUGAGAGGGGAAGGAGG | 18 | 8436 |
| myoC-8691 | - | UGUUGAGAGGGGAAGGAGG | 19 | 8437 |
| myoC-1945 | - | AUGUUGAGAGGGGAAGGAGG | 20 | 2113 |
| myoC-8692 | - | GAUGUUGAGAGGGGAAGGAGG | 21 | 8438 |
| myoC-8693 | - | GGAUGUUGAGAGGGGAAGGAGG | 22 | 8439 |
| myoC-8694 | - | GGGAUGUUGAGAGGGGAAGGAGG | 23 | 8440 |
| myoC-8695 | - | GGGGAUGUUGAGAGGGGAAGGAGG | 24 | 8441 |
| myoC-3687 | - | GAGAAUCUGGCCAGGAGG | 18 | 3433 |
| myoC-3688 | - | UGAGAAUCUGGCCAGGAGG | 19 | 3434 |
| myoC-1651 | - | AUGAGAAUCUGGCCAGGAGG | 20 | 1916 |
| myoC-3689 | - | AAUGAGAAUCUGGCCAGGAGG | 21 | 3435 |
| myoC-3690 | - | AAAUGAGAAUCUGGCCAGGAGG | 22 | 3436 |
| myoC-3691 | - | AAAAUGAGAAUCUGGCCAGGAGG | 23 | 3437 |
| myoC-3692 | - | GAAAAUGAGAAUCUGGCCAGGAGG | 24 | 3438 |
| myoC-8696 | - | AUCCUGGGUUCUAGGAGG | 18 | 8442 |
| myoC-8697 | - | GAUCCUGGGUUCUAGGAGG | 19 | 8443 |
| myoC-2001 | - | UGAUCCUGGGUUCUAGGAGG | 20 | 2148 |
| myoC-8698 | - | GUGAUCCUGGGUUCUAGGAGG | 21 | 8444 |
| myoC-8699 | - | CGUGAUCCUGGGUUCUAGGAGG | 22 | 8445 |
| myoC-8700 | - | ACGUGAUCCUGGGUUCUAGGAGG | 23 | 8446 |
| myoC-8701 | - | CACGUGAUCCUGGGUUCUAGGAGG | 24 | 8447 |
| myoC-8702 | - | GCUGAGCCACAGGGGAGG | 18 | 8448 |
| myoC-8703 | - | AGCUGAGCCACAGGGGAGG | 19 | 8449 |
| myoC-1050 | - | CAGCUGAGCCACAGGGGAGG | 20 | 1350 |
| myoC-8704 | - | GCAGCUGAGCCACAGGGGAGG | 21 | 8450 |
| myoC-8705 | - | AGCAGCUGAGCCACAGGGGAGG | 22 | 8451 |
| myoC-8706 | - | GAGCAGCUGAGCCACAGGGGAGG | 23 | 8452 |
| myoC-8707 | - | GGAGCAGCUGAGCCACAGGGGAGG | 24 | 8453 |
| myoC-8708 | - | UUAAAGCUAGGGGUGAGG | 18 | 8454 |
| myoC-8709 | - | UUUAAAGCUAGGGGUGAGG | 19 | 8455 |
| myoC-2072 | - | UUUUAAAGCUAGGGGUGAGG | 20 | 2203 |
| myoC-8710 | - | GUUUUAAAGCUAGGGGUGAGG | 21 | 8456 |
| myoC-8711 | - | UGUUUUAAAGCUAGGGGUGAGG | 22 | 8457 |
| myoC-8712 | - | UUGUUUUAAAGCUAGGGGUGAGG | 23 | 8458 |
| myoC-8713 | - | GUUGUUUUAAAGCUAGGGGUGAGG | 24 | 8459 |
| myoC-8714 | - | UUGGCUUAUGCAAGACGG | 18 | 8460 |
| myoC-8715 | - | CUUGGCUUAUGCAAGACGG | 19 | 8461 |
| myoC-1923 | - | ACUUGGCUUAUGCAAGACGG | 20 | 2101 |
| myoC-8716 | - | GACUUGGCUUAUGCAAGACGG | 21 | 8462 |
| myoC-8717 | - | GGACUUGGCUUAUGCAAGACGG | 22 | 8463 |
| myoC-8718 | - | UGGACUUGGCUUAUGCAAGACGG | 23 | 8464 |
| myoC-8719 | - | GUGGACUUGGCUUAUGCAAGACGG | 24 | 8465 |
| myoC-8720 | - | GAGAAAUAAAAGGACCGG | 18 | 8466 |
| myoC-8721 | - | GGAGAAAUAAAAGGACCGG | 19 | 8467 |
| myoC-8722 | - | AGGAGAAAUAAAAGGACCGG | 20 | 8468 |
| myoC-8723 | - | AAGGAGAAAUAAAAGGACCGG | 21 | 8469 |
| myoC-8724 | - | AAAGGAGAAAUAAAAGGACCGG | 22 | 8470 |
| myoC-8725 | - | AAAAGGAGAAAUAAAAGGACCGG | 23 | 8471 |
| myoC-8726 | - | CAAAAGGAGAAAUAAAAGGACCGG | 24 | 8472 |
| myoC-8727 | - | GUGACCUGCAGCGCAGGG | 18 | 8473 |
| myoC-8728 | - | AGUGACCUGCAGCGCAGGG | 19 | 8474 |
| myoC-1982 | - | GAGUGACCUGCAGCGCAGGG | 20 | 2135 |
| myoC-8729 | - | GGAGUGACCUGCAGCGCAGGG | 21 | 8475 |
| myoC-8730 | - | CGGAGUGACCUGCAGCGCAGGG | 22 | 8476 |
| myoC-8731 | - | ACGGAGUGACCUGCAGCGCAGGG | 23 | 8477 |
| myoC-8732 | - | CACGGAGUGACCUGCAGCGCAGGG | 24 | 8478 |
| myoC-8733 | - | UAAAGCUAGGGGUGAGGG | 18 | 8479 |
| myoC-8734 | - | UUAAAGCUAGGGGUGAGGG | 19 | 8480 |
| myoC-2073 | - | UUUAAAGCUAGGGGUGAGGG | 20 | 2204 |
| myoC-8735 | - | UUUUAAAGCUAGGGGUGAGGG | 21 | 8481 |
| myoC-8736 | - | GUUUUAAAGCUAGGGGUGAGGG | 22 | 8482 |
| myoC-8737 | - | UGUUUUAAAGCUAGGGGUGAGGG | 23 | 8483 |
| myoC-8738 | - | UUGUUUUAAAGCUAGGGGUGAGGG | 24 | 8484 |
| myoC-8739 | - | GUUGUUUUAAAGCUAGGG | 18 | 8485 |
| myoC-8740 | - | AGUUGUUUUAAAGCUAGGG | 19 | 8486 |
| myoC-2067 | - | CAGUUGUUUUAAAGCUAGGG | 20 | 2198 |
| myoC-8741 | - | ACAGUUGUUUUAAAGCUAGGG | 21 | 8487 |
| myoC-8742 | - | CACAGUUGUUUUAAAGCUAGGG | 22 | 8488 |
| myoC-8743 | - | ACACAGUUGUUUUAAAGCUAGGG | 23 | 8489 |
| myoC-8744 | - | UACACAGUUGUUUUAAAGCUAGGG | 24 | 8490 |
| myoC-8745 | - | UGACCUGCAGCGCAGGGG | 18 | 8491 |
| myoC-8746 | - | GUGACCUGCAGCGCAGGGG | 19 | 8492 |
| myoC-1121 | - | AGUGACCUGCAGCGCAGGGG | 20 | 1421 |
| myoC-8747 | - | GAGUGACCUGCAGCGCAGGGG | 21 | 8493 |
| myoC-8748 | - | GGAGUGACCUGCAGCGCAGGGG | 22 | 8494 |
| myoC-8749 | - | CGGAGUGACCUGCAGCGCAGGGG | 23 | 8495 |
| myoC-8750 | - | ACGGAGUGACCUGCAGCGCAGGGG | 24 | 8496 |
| myoC-8751 | - | GGGGGAUGUUGAGAGGGG | 18 | 8497 |
| myoC-8752 | - | GGGGGGAUGUUGAGAGGGG | 19 | 8498 |
| myoC-1943 | - | AGGGGGGAUGUUGAGAGGGG | 20 | 2112 |
| myoC-8753 | - | GAGGGGGGAUGUUGAGAGGGG | 21 | 8499 |
| myoC-8754 | - | UGAGGGGGGAUGUUGAGAGGGG | 22 | 8500 |
| myoC-8755 | - | GUGAGGGGGGAUGUUGAGAGGGG | 23 | 8501 |
| myoC-8756 | - | UGUGAGGGGGGAUGUUGAGAGGGG | 24 | 8502 |
| myoC-8757 | - | GCAGGGCUAUAUUGUGGG | 18 | 8503 |
| myoC-8758 | - | GGCAGGGCUAUAUUGUGGG | 19 | 8504 |
| myoC-1140 | - | AGGCAGGGCUAUAUUGUGGG | 20 | 1440 |
| myoC-8759 | - | GAGGCAGGGCUAUAUUGUGGG | 21 | 8505 |
| myoC-8760 | - | GGAGGCAGGGCUAUAUUGUGGG | 22 | 8506 |
| myoC-8761 | - | AGGAGGCAGGGCUAUAUUGUGGG | 23 | 8507 |
| myoC-8762 | - | UAGGAGGCAGGGCUAUAUUGUGGG | 24 | 8508 |
| myoC-5103 | - | GGUAAGAAUGCAGAGUGG | 18 | 4849 |
| myoC-5104 | - | AGGUAAGAAUGCAGAGUGG | 19 | 4850 |
| myoC-3188 | - | AAGGUAAGAAUGCAGAGUGG | 20 | 2934 |
| myoC-5105 | - | GAAGGUAAGAAUGCAGAGUGG | 21 | 4851 |
| myoC-5106 | - | AGAAGGUAAGAAUGCAGAGUGG | 22 | 4852 |
| myoC-5107 | - | GAGAAGGUAAGAAUGCAGAGUGG | 23 | 4853 |
| myoC-5108 | - | AGAGAAGGUAAGAAUGCAGAGUGG | 24 | 4854 |
| myoC-8763 | - | GAGCCACAGGGGAGGUGG | 18 | 8509 |
| myoC-8764 | - | UGAGCCACAGGGGAGGUGG | 19 | 8510 |
| myoC-1051 | - | CUGAGCCACAGGGGAGGUGG | 20 | 1351 |
| myoC-8765 | - | GCUGAGCCACAGGGGAGGUGG | 21 | 8511 |
| myoC-8766 | - | AGCUGAGCCACAGGGGAGGUGG | 22 | 8512 |
| myoC-8767 | - | CAGCUGAGCCACAGGGGAGGUGG | 23 | 8513 |
| myoC-8768 | - | GCAGCUGAGCCACAGGGGAGGUGG | 24 | 8514 |
| myoC-8769 | - | GGCAGGGCUAUAUUGUGG | 18 | 8515 |
| myoC-8770 | - | AGGCAGGGCUAUAUUGUGG | 19 | 8516 |
| myoC-1139 | - | GAGGCAGGGCUAUAUUGUGG | 20 | 1439 |
| myoC-8771 | - | GGAGGCAGGGCUAUAUUGUGG | 21 | 8517 |
| myoC-8772 | - | AGGAGGCAGGGCUAUAUUGUGG | 22 | 8518 |
| myoC-8773 | - | UAGGAGGCAGGGCUAUAUUGUGG | 23 | 8519 |
| myoC-8774 | - | CUAGGAGGCAGGGCUAUAUUGUGG | 24 | 8520 |
| myoC-8775 | - | CAAUAACCAAAAAGAAUG | 18 | 8521 |
| myoC-8776 | - | CCAAUAACCAAAAAGAAUG | 19 | 8522 |
| myoC-1970 | - | GCCAAUAACCAAAAAGAAUG | 20 | 2128 |
| myoC-8777 | - | UGCCAAUAACCAAAAAGAAUG | 21 | 8523 |
| myoC-8778 | - | UUGCCAAUAACCAAAAAGAAUG | 22 | 8524 |
| myoC-8779 | - | UUUGCCAAUAACCAAAAAGAAUG | 23 | 8525 |
| myoC-8780 | - | AUUUGCCAAUAACCAAAAAGAAUG | 24 | 8526 |
| myoC-8781 | - | AGCCUGUGAAUUUGAAUG | 18 | 8527 |
| myoC-8782 | - | AAGCCUGUGAAUUUGAAUG | 19 | 8528 |
| myoC-1170 | - | AAAGCCUGUGAAUUUGAAUG | 20 | 1470 |
| myoC-8783 | - | GAAAGCCUGUGAAUUUGAAUG | 21 | 8529 |
| myoC-8784 | - | AGAAAGCCUGUGAAUUUGAAUG | 22 | 8530 |
| myoC-8785 | - | CAGAAAGCCUGUGAAUUUGAAUG | 23 | 8531 |
| myoC-8786 | - | CCAGAAAGCCUGUGAAUUUGAAUG | 24 | 8532 |
| myoC-8787 | - | UCUCUGUGAGGGGGGAUG | 18 | 8533 |
| myoC-8788 | - | UUCUCUGUGAGGGGGGAUG | 19 | 8534 |
| myoC-1937 | - | AUUCUCUGUGAGGGGGGAUG | 20 | 2109 |
| myoC-8789 | - | GAUUCUCUGUGAGGGGGGAUG | 21 | 8535 |
| myoC-8790 | - | UGAUUCUCUGUGAGGGGGGAUG | 22 | 8536 |
| myoC-8791 | - | GUGAUUCUCUGUGAGGGGGGAUG | 23 | 8537 |
| myoC-8792 | - | UGUGAUUCUCUGUGAGGGGGGAUG | 24 | 8538 |
| myoC-8793 | - | CAAGUUCAGGCUUAACUG | 18 | 8539 |
| myoC-8794 | - | UCAAGUUCAGGCUUAACUG | 19 | 8540 |
| myoC-2029 | - | CUCAAGUUCAGGCUUAACUG | 20 | 2164 |
| myoC-8795 | - | UCUCAAGUUCAGGCUUAACUG | 21 | 8541 |
| myoC-8796 | - | GUCUCAAGUUCAGGCUUAACUG | 22 | 8542 |
| myoC-8797 | - | UGUCUCAAGUUCAGGCUUAACUG | 23 | 8543 |
| myoC-8798 | - | AUGUCUCAAGUUCAGGCUUAACUG | 24 | 8544 |
| myoC-5146 | - | AGGUAAGAAUGCAGAGUG | 18 | 4892 |
| myoC-5147 | - | AAGGUAAGAAUGCAGAGUG | 19 | 4893 |
| myoC-3189 | - | GAAGGUAAGAAUGCAGAGUG | 20 | 2935 |
| myoC-5148 | - | AGAAGGUAAGAAUGCAGAGUG | 21 | 4894 |
| myoC-5149 | - | GAGAAGGUAAGAAUGCAGAGUG | 22 | 4895 |
| myoC-5150 | - | AGAGAAGGUAAGAAUGCAGAGUG | 23 | 4896 |
| myoC-5151 | - | CAGAGAAGGUAAGAAUGCAGAGUG | 24 | 4897 |
| myoC-8799 | - | UGAGCCACAGGGGAGGUG | 18 | 8545 |
| myoC-8800 | - | CUGAGCCACAGGGGAGGUG | 19 | 8546 |
| myoC-1955 | - | GCUGAGCCACAGGGGAGGUG | 20 | 2118 |
| myoC-8801 | - | AGCUGAGCCACAGGGGAGGUG | 21 | 8547 |
| myoC-8802 | - | CAGCUGAGCCACAGGGGAGGUG | 22 | 8548 |
| myoC-8803 | - | GCAGCUGAGCCACAGGGGAGGUG | 23 | 8549 |
| myoC-8804 | - | AGCAGCUGAGCCACAGGGGAGGUG | 24 | 8550 |
| myoC-8805 | - | GUUUUAAAGCUAGGGGUG | 18 | 8551 |
| myoC-8806 | - | UGUUUUAAAGCUAGGGGUG | 19 | 8552 |
| myoC-2069 | - | UUGUUUUAAAGCUAGGGGUG | 20 | 2200 |
| myoC-8807 | - | GUUGUUUUAAAGCUAGGGGUG | 21 | 8553 |
| myoC-8808 | - | AGUUGUUUUAAAGCUAGGGGUG | 22 | 8554 |
| myoC-8809 | - | CAGUUGUUUUAAAGCUAGGGGUG | 23 | 8555 |
| myoC-8810 | - | ACAGUUGUUUUAAAGCUAGGGGUG | 24 | 8556 |
| myoC-8811 | - | CAACUACUCAGCCCUGUG | 18 | 8557 |
| myoC-8812 | - | GCAACUACUCAGCCCUGUG | 19 | 8558 |
| myoC-1922 | - | GGCAACUACUCAGCCCUGUG | 20 | 2100 |
| myoC-8813 | - | GGGCAACUACUCAGCCCUGUG | 21 | 8559 |
| myoC-8814 | - | UGGGCAACUACUCAGCCCUGUG | 22 | 8560 |
| myoC-8815 | - | CUGGGCAACUACUCAGCCCUGUG | 23 | 8561 |
| myoC-8816 | - | UCUGGGCAACUACUCAGCCCUGUG | 24 | 8562 |
| myoC-8817 | - | UCCCUGUGAUUCUCUGUG | 18 | 8563 |
| myoC-8818 | - | UUCCCUGUGAUUCUCUGUG | 19 | 8564 |
| myoC-1035 | - | CUUCCCUGUGAUUCUCUGUG | 20 | 1335 |
| myoC-8819 | - | ACUUCCCUGUGAUUCUCUGUG | 21 | 8565 |
| myoC-8820 | - | CACUUCCCUGUGAUUCUCUGUG | 22 | 8566 |
| myoC-8821 | - | ACACUUCCCUGUGAUUCUCUGUG | 23 | 8567 |
| myoC-8822 | - | AACACUUCCCUGUGAUUCUCUGUG | 24 | 8568 |
| myoC-8823 | - | AGGCAGGGCUAUAUUGUG | 18 | 8569 |
| myoC-8824 | - | GAGGCAGGGCUAUAUUGUG | 19 | 8570 |
| myoC-1138 | - | GGAGGCAGGGCUAUAUUGUG | 20 | 1438 |
| myoC-8825 | - | AGGAGGCAGGGCUAUAUUGUG | 21 | 8571 |
| myoC-8826 | - | UAGGAGGCAGGGCUAUAUUGUG | 22 | 8572 |
| myoC-8827 | - | CUAGGAGGCAGGGCUAUAUUGUG | 23 | 8573 |
| myoC-8828 | - | UCUAGGAGGCAGGGCUAUAUUGUG | 24 | 8574 |
| myoC-8829 | - | GGAGGCAGGGCUAUAUUG | 18 | 8575 |
| myoC-8830 | - | AGGAGGCAGGGCUAUAUUG | 19 | 8576 |
| myoC-1136 | - | UAGGAGGCAGGGCUAUAUUG | 20 | 1436 |
| myoC-8831 | - | CUAGGAGGCAGGGCUAUAUUG | 21 | 8577 |
| myoC-8832 | - | UCUAGGAGGCAGGGCUAUAUUG | 22 | 8578 |
| myoC-8833 | - | UUCUAGGAGGCAGGGCUAUAUUG | 23 | 8579 |
| myoC-8834 | - | GUUCUAGGAGGCAGGGCUAUAUUG | 24 | 8580 |
| myoC-8835 | - | GAGAUGCAAGACUGAAAU | 18 | 8581 |
| myoC-8836 | - | UGAGAUGCAAGACUGAAAU | 19 | 8582 |
| myoC-2064 | - | CUGAGAUGCAAGACUGAAAU | 20 | 2195 |
| myoC-8837 | - | CCUGAGAUGCAAGACUGAAAU | 21 | 8583 |
| myoC-8838 | - | GCCUGAGAUGCAAGACUGAAAU | 22 | 8584 |
| myoC-8839 | - | UGCCUGAGAUGCAAGACUGAAAU | 23 | 8585 |
| myoC-8840 | - | GUGCCUGAGAUGCAAGACUGAAAU | 24 | 8586 |
| myoC-8841 | - | GGUCGAAAACCUUGGAAU | 18 | 8587 |
| myoC-8842 | - | CGGUCGAAAACCUUGGAAU | 19 | 8588 |
| myoC-1926 | - | ACGGUCGAAAACCUUGGAAU | 20 | 2103 |
| myoC-8843 | - | GACGGUCGAAAACCUUGGAAU | 21 | 8589 |
| myoC-8844 | - | AGACGGUCGAAAACCUUGGAAU | 22 | 8590 |
| myoC-8845 | - | AAGACGGUCGAAAACCUUGGAAU | 23 | 8591 |
| myoC-8846 | - | CAAGACGGUCGAAAACCUUGGAAU | 24 | 8592 |
| myoC-8847 | - | AAGCCUGUGAAUUUGAAU | 18 | 8593 |
| myoC-8848 | - | AAAGCCUGUGAAUUUGAAU | 19 | 8594 |
| myoC-2046 | - | GAAAGCCUGUGAAUUUGAAU | 20 | 2178 |
| myoC-8849 | - | AGAAAGCCUGUGAAUUUGAAU | 21 | 8595 |
| myoC-8850 | - | CAGAAAGCCUGUGAAUUUGAAU | 22 | 8596 |
| myoC-8851 | - | CCAGAAAGCCUGUGAAUUUGAAU | 23 | 8597 |
| myoC-8852 | - | UCCAGAAAGCCUGUGAAUUUGAAU | 24 | 8598 |
| myoC-8853 | - | GGUGAGAUGUGUCUGCAU | 18 | 8599 |
| myoC-8854 | - | GGGUGAGAUGUGUCUGCAU | 19 | 8600 |
| myoC-8855 | - | CGGGUGAGAUGUGUCUGCAU | 20 | 8601 |
| myoC-8856 | - | CCGGGUGAGAUGUGUCUGCAU | 21 | 8602 |
| myoC-8857 | - | ACCGGGUGAGAUGUGUCUGCAU | 22 | 8603 |
| myoC-8858 | - | GACCGGGUGAGAUGUGUCUGCAU | 23 | 8604 |
| myoC-8859 | - | GGACCGGGUGAGAUGUGUCUGCAU | 24 | 8605 |
| myoC-8860 | - | AAUCUAUAUUUUAUAUAU | 18 | 8606 |
| myoC-8861 | - | UAAUCUAUAUUUUAUAUAU | 19 | 8607 |
| myoC-2054 | - | GUAAUCUAUAUUUUAUAUAU | 20 | 2185 |
| myoC-8862 | - | UGUAAUCUAUAUUUUAUAUAU | 21 | 8608 |
| myoC-8863 | - | UUGUAAUCUAUAUUUUAUAUAU | 22 | 8609 |
| myoC-8864 | - | UUUGUAAUCUAUAUUUUAUAUAU | 23 | 8610 |
| myoC-8865 | - | CUUUGUAAUCUAUAUUUUAUAUAU | 24 | 8611 |
| myoC-8866 | - | UACUUAGUUUCUCCUUAU | 18 | 8612 |
| myoC-8867 | - | UUACUUAGUUUCUCCUUAU | 19 | 8613 |
| myoC-2017 | - | AUUACUUAGUUUCUCCUUAU | 20 | 2155 |
| myoC-8868 | - | GAUUACUUAGUUUCUCCUUAU | 21 | 8614 |
| myoC-8869 | - | AGAUUACUUAGUUUCUCCUUAU | 22 | 8615 |
| myoC-8870 | - | AAGAUUACUUAGUUUCUCCUUAU | 23 | 8616 |
| myoC-8871 | - | UAAGAUUACUUAGUUUCUCCUUAU | 24 | 8617 |
| myoC-8872 | - | AAACUGUGUUUCUCCACU | 18 | 8618 |
| myoC-8873 | - | CAAACUGUGUUUCUCCACU | 19 | 8619 |
| myoC-2033 | - | GCAAACUGUGUUUCUCCACU | 20 | 2168 |
| myoC-8874 | - | AGCAAACUGUGUUUCUCCACU | 21 | 8620 |
| myoC-8875 | - | GAGCAAACUGUGUUUCUCCACU | 22 | 8621 |
| myoC-8876 | - | AGAGCAAACUGUGUUUCUCCACU | 23 | 8622 |
| myoC-8877 | - | UAGAGCAAACUGUGUUUCUCCACU | 24 | 8623 |
| myoC-8878 | - | UUUAUACUCAAAACUACU | 18 | 8624 |
| myoC-8879 | - | AUUUAUACUCAAAACUACU | 19 | 8625 |
| myoC-2052 | - | UAUUUAUACUCAAAACUACU | 20 | 2183 |
| myoC-8880 | - | AUAUUUAUACUCAAAACUACU | 21 | 8626 |
| myoC-8881 | - | AAUAUUUAUACUCAAAACUACU | 22 | 8627 |
| myoC-8882 | - | AAAUAUUUAUACUCAAAACUACU | 23 | 8628 |
| myoC-8883 | - | GAAAUAUUUAUACUCAAAACUACU | 24 | 8629 |
| myoC-8884 | - | ACUAGUAAUUUAGCUCCU | 18 | 8630 |
| myoC-8885 | - | UACUAGUAAUUUAGCUCCU | 19 | 8631 |
| myoC-8886 | - | UUACUAGUAAUUUAGCUCCU | 20 | 8632 |
| myoC-8887 | - | AUUACUAGUAAUUUAGCUCCU | 21 | 8633 |
| myoC-8888 | - | UAUUACUAGUAAUUUAGCUCCU | 22 | 8634 |
| myoC-8889 | - | GUAUUACUAGUAAUUUAGCUCCU | 23 | 8635 |
| myoC-8890 | - | AGUAUUACUAGUAAUUUAGCUCCU | 24 | 8636 |
| myoC-5251 | - | CCAGGAGGUAGCAAGGCU | 18 | 4997 |
| myoC-5252 | - | GCCAGGAGGUAGCAAGGCU | 19 | 4998 |
| myoC-1656 | - | AGCCAGGAGGUAGCAAGGCU | 20 | 1919 |
| myoC-5253 | - | CAGCCAGGAGGUAGCAAGGCU | 21 | 4999 |
| myoC-5254 | - | GCAGCCAGGAGGUAGCAAGGCU | 22 | 5000 |
| myoC-5255 | - | AGCAGCCAGGAGGUAGCAAGGCU | 23 | 5001 |
| myoC-5256 | - | CAGCAGCCAGGAGGUAGCAAGGCU | 24 | 5002 |
| myoC-8891 | - | ACUUCCCUGUGAUUCUCU | 18 | 8637 |
| myoC-8892 | - | CACUUCCCUGUGAUUCUCU | 19 | 8638 |
| myoC-1931 | - | ACACUUCCCUGUGAUUCUCU | 20 | 2107 |
| myoC-8893 | - | AACACUUCCCUGUGAUUCUCU | 21 | 8639 |
| myoC-8894 | - | GAACACUUCCCUGUGAUUCUCU | 22 | 8640 |
| myoC-8895 | - | UGAACACUUCCCUGUGAUUCUCU | 23 | 8641 |
| myoC-8896 | - | GUGAACACUUCCCUGUGAUUCUCU | 24 | 8642 |
| myoC-8897 | - | UAGGAACUCUUUUUCUCU | 18 | 8643 |
| myoC-8898 | - | UUAGGAACUCUUUUUCUCU | 19 | 8644 |
| myoC-2019 | - | AUUAGGAACUCUUUUUCUCU | 20 | 2156 |
| myoC-8899 | - | UAUUAGGAACUCUUUUUCUCU | 21 | 8645 |
| myoC-8900 | - | UUAUUAGGAACUCUUUUUCUCU | 22 | 8646 |
| myoC-8901 | - | CUUAUUAGGAACUCUUUUUCUCU | 23 | 8647 |
| myoC-8902 | - | CCUUAUUAGGAACUCUUUUUCUCU | 24 | 8648 |
| myoC-8903 | - | CACGUGAUCCUGGGUUCU | 18 | 8649 |
| myoC-8904 | - | CCACGUGAUCCUGGGUUCU | 19 | 8650 |
| myoC-1132 | - | UCCACGUGAUCCUGGGUUCU | 20 | 1432 |
| myoC-8905 | - | GUCCACGUGAUCCUGGGUUCU | 21 | 8651 |
| myoC-8906 | - | AGUCCACGUGAUCCUGGGUUCU | 22 | 8652 |
| myoC-8907 | - | UAGUCCACGUGAUCCUGGGUUCU | 23 | 8653 |
| myoC-8908 | - | AUAGUCCACGUGAUCCUGGGUUCU | 24 | 8654 |
| myoC-8909 | - | UUGCAGCUCUCGUGUUCU | 18 | 8655 |
| myoC-8910 | - | CUUGCAGCUCUCGUGUUCU | 19 | 8656 |
| myoC-1930 | - | CCUUGCAGCUCUCGUGUUCU | 20 | 2106 |
| myoC-8911 | - | CCCUUGCAGCUCUCGUGUUCU | 21 | 8657 |
| myoC-8912 | - | ACCCUUGCAGCUCUCGUGUUCU | 22 | 8658 |
| myoC-8913 | - | GACCCUUGCAGCUCUCGUGUUCU | 23 | 8659 |
| myoC-8914 | - | AGACCCUUGCAGCUCUCGUGUUCU | 24 | 8660 |
| myoC-8915 | - | AUUUGAAAACAUCUUUCU | 18 | 8661 |
| myoC-8916 | - | UAUUUGAAAACAUCUUUCU | 19 | 8662 |
| myoC-2056 | - | AUAUUUGAAAACAUCUUUCU | 20 | 2187 |
| myoC-8917 | - | UAUAUUUGAAAACAUCUUUCU | 21 | 8663 |
| myoC-8918 | - | AUAUAUUUGAAAACAUCUUUCU | 22 | 8664 |
| myoC-8919 | - | UAUAUAUUUGAAAACAUCUUUCU | 23 | 8665 |
| myoC-8920 | - | UUAUAUAUUUGAAAACAUCUUUCU | 24 | 8666 |
| myoC-8921 | - | AAUCAGUUCAAGGGAAGU | 18 | 8667 |
| myoC-8922 | - | AAAUCAGUUCAAGGGAAGU | 19 | 8668 |
| myoC-1143 | - | AAAAUCAGUUCAAGGGAAGU | 20 | 1443 |
| myoC-8923 | - | AAAAAUCAGUUCAAGGGAAGU | 21 | 8669 |
| myoC-8924 | - | AAAAAAUCAGUUCAAGGGAAGU | 22 | 8670 |
| myoC-8925 | - | GAAAAAAUCAGUUCAAGGGAAGU | 23 | 8671 |
| myoC-8926 | - | GGAAAAAAUCAGUUCAAGGGAAGU | 24 | 8672 |
| myoC-8927 | - | UGAGUCUGCCAGGGCAGU | 18 | 8673 |
| myoC-8928 | - | GUGAGUCUGCCAGGGCAGU | 19 | 8674 |
| myoC-2037 | - | GGUGAGUCUGCCAGGGCAGU | 20 | 2171 |
| myoC-8929 | - | AGGUGAGUCUGCCAGGGCAGU | 21 | 8675 |
| myoC-8930 | - | GAGGUGAGUCUGCCAGGGCAGU | 22 | 8676 |
| myoC-8931 | - | GGAGGUGAGUCUGCCAGGGCAGU | 23 | 8677 |
| myoC-8932 | - | UGGAGGUGAGUCUGCCAGGGCAGU | 24 | 8678 |
| myoC-8933 | - | CAUGCACACACACAGAGU | 18 | 8679 |
| myoC-8934 | - | GCAUGCACACACACAGAGU | 19 | 8680 |
| myoC-2060 | - | GGCAUGCACACACACAGAGU | 20 | 2191 |
| myoC-8935 | - | UGGCAUGCACACACACAGAGU | 21 | 8681 |
| myoC-8936 | - | UUGGCAUGCACACACACAGAGU | 22 | 8682 |
| myoC-8937 | - | CUUGGCAUGCACACACACAGAGU | 23 | 8683 |
| myoC-8938 | - | UCUUGGCAUGCACACACACAGAGU | 24 | 8684 |
| myoC-5289 | - | AAGGUAAGAAUGCAGAGU | 18 | 5035 |
| myoC-5290 | - | GAAGGUAAGAAUGCAGAGU | 19 | 5036 |
| myoC-3191 | - | AGAAGGUAAGAAUGCAGAGU | 20 | 2937 |
| myoC-5291 | - | GAGAAGGUAAGAAUGCAGAGU | 21 | 5037 |
| myoC-5292 | - | AGAGAAGGUAAGAAUGCAGAGU | 22 | 5038 |
| myoC-5293 | - | CAGAGAAGGUAAGAAUGCAGAGU | 23 | 5039 |
| myoC-5294 | - | CCAGAGAAGGUAAGAAUGCAGAGU | 24 | 5040 |
| myoC-3765 | - | AGAAUCUGGCCAGGAGGU | 18 | 3511 |
| myoC-3766 | - | GAGAAUCUGGCCAGGAGGU | 19 | 3512 |
| myoC-197 | - | UGAGAAUCUGGCCAGGAGGU | 20 | 583 |
| myoC-3767 | - | AUGAGAAUCUGGCCAGGAGGU | 21 | 3513 |
| myoC-3768 | - | AAUGAGAAUCUGGCCAGGAGGU | 22 | 3514 |
| myoC-3769 | - | AAAUGAGAAUCUGGCCAGGAGGU | 23 | 3515 |
| myoC-3770 | - | AAAAUGAGAAUCUGGCCAGGAGGU | 24 | 3516 |
| myoC-8939 | - | UGUUUUAAAGCUAGGGGU | 18 | 8685 |
| myoC-8940 | - | UUGUUUUAAAGCUAGGGGU | 19 | 8686 |
| myoC-2068 | - | GUUGUUUUAAAGCUAGGGGU | 20 | 2199 |
| myoC-8941 | - | AGUUGUUUUAAAGCUAGGGGU | 21 | 8687 |
| myoC-8942 | - | CAGUUGUUUUAAAGCUAGGGGU | 22 | 8688 |
| myoC-8943 | - | ACAGUUGUUUUAAAGCUAGGGGU | 23 | 8689 |
| myoC-8944 | - | CACAGUUGUUUUAAAGCUAGGGGU | 24 | 8690 |
| myoC-8945 | - | UUCCCUGUGAUUCUCUGU | 18 | 8691 |
| myoC-8946 | - | CUUCCCUGUGAUUCUCUGU | 19 | 8692 |
| myoC-1932 | - | ACUUCCCUGUGAUUCUCUGU | 20 | 2108 |
| myoC-8947 | - | CACUUCCCUGUGAUUCUCUGU | 21 | 8693 |
| myoC-8948 | - | ACACUUCCCUGUGAUUCUCUGU | 22 | 8694 |
| myoC-8949 | - | AACACUUCCCUGUGAUUCUCUGU | 23 | 8695 |
| myoC-8950 | - | GAACACUUCCCUGUGAUUCUCUGU | 24 | 8696 |
| myoC-8951 | - | AAAAGAGAGGGAUAGUGU | 18 | 8697 |
| myoC-8952 | - | AAAAAGAGAGGGAUAGUGU | 19 | 8698 |
| myoC-1990 | - | GAAAAAGAGAGGGAUAGUGU | 20 | 2141 |
| myoC-8953 | - | AGAAAAAGAGAGGGAUAGUGU | 21 | 8699 |
| myoC-8954 | - | AAGAAAAAGAGAGGGAUAGUGU | 22 | 8700 |
| myoC-8955 | - | GAAGAAAAAGAGAGGGAUAGUGU | 23 | 8701 |
| myoC-8956 | - | AGAAGAAAAAGAGAGGGAUAGUGU | 24 | 8702 |
| myoC-8957 | - | GAGGCAGGGCUAUAUUGU | 18 | 8703 |
| myoC-8958 | - | GGAGGCAGGGCUAUAUUGU | 19 | 8704 |
| myoC-1137 | - | AGGAGGCAGGGCUAUAUUGU | 20 | 1437 |
| myoC-8959 | - | UAGGAGGCAGGGCUAUAUUGU | 21 | 8705 |
| myoC-8960 | - | CUAGGAGGCAGGGCUAUAUUGU | 22 | 8706 |
| myoC-8961 | - | UCUAGGAGGCAGGGCUAUAUUGU | 23 | 8707 |
| myoC-8962 | - | UUCUAGGAGGCAGGGCUAUAUUGU | 24 | 8708 |
| myoC-8963 | - | GCACAAGACAGAUGAAUU | 18 | 8709 |
| myoC-8964 | - | AGCACAAGACAGAUGAAUU | 19 | 8710 |
| myoC-8965 | - | UAGCACAAGACAGAUGAAUU | 20 | 8711 |
| myoC-8966 | - | CUAGCACAAGACAGAUGAAUU | 21 | 8712 |
| myoC-8967 | - | GCUAGCACAAGACAGAUGAAUU | 22 | 8713 |
| myoC-8968 | - | AGCUAGCACAAGACAGAUGAAUU | 23 | 8714 |
| myoC-8969 | - | CAGCUAGCACAAGACAGAUGAAUU | 24 | 8715 |
| myoC-8970 | - | UUUACAAGCUGAGUAAUU | 18 | 8716 |
| myoC-8971 | - | CUUUACAAGCUGAGUAAUU | 19 | 8717 |
| myoC-2015 | - | CCUUUACAAGCUGAGUAAUU | 20 | 2153 |
| myoC-8972 | - | UCCUUUACAAGCUGAGUAAUU | 21 | 8718 |
| myoC-8973 | - | UUCCUUUACAAGCUGAGUAAUU | 22 | 8719 |
| myoC-8974 | - | UUUCCUUUACAAGCUGAGUAAUU | 23 | 8720 |
| myoC-8975 | - | UUUUCCUUUACAAGCUGAGUAAUU | 24 | 8721 |
| myoC-8976 | - | ACAGAGUAAGAACUGAUU | 18 | 8722 |
| myoC-8977 | - | CACAGAGUAAGAACUGAUU | 19 | 8723 |
| myoC-2061 | - | ACACAGAGUAAGAACUGAUU | 20 | 2192 |
| myoC-8978 | - | CACACAGAGUAAGAACUGAUU | 21 | 8724 |
| myoC-8979 | - | ACACACAGAGUAAGAACUGAUU | 22 | 8725 |
| myoC-8980 | - | CACACACAGAGUAAGAACUGAUU | 23 | 8726 |
| myoC-8981 | - | ACACACACAGAGUAAGAACUGAUU | 24 | 8727 |
| myoC-8982 | - | GAUGUUUACUAUCUGAUU | 18 | 8728 |
| myoC-8983 | - | CGAUGUUUACUAUCUGAUU | 19 | 8729 |
| myoC-2027 | - | GCGAUGUUUACUAUCUGAUU | 20 | 2162 |
| myoC-8984 | - | AGCGAUGUUUACUAUCUGAUU | 21 | 8730 |
| myoC-8985 | - | CAGCGAUGUUUACUAUCUGAUU | 22 | 8731 |
| myoC-8986 | - | UCAGCGAUGUUUACUAUCUGAUU | 23 | 8732 |
| myoC-8987 | - | UUCAGCGAUGUUUACUAUCUGAUU | 24 | 8733 |
| myoC-8988 | - | AGGAGGCAGGGCUAUAUU | 18 | 8734 |
| myoC-8989 | - | UAGGAGGCAGGGCUAUAUU | 19 | 8735 |
| myoC-2002 | - | CUAGGAGGCAGGGCUAUAUU | 20 | 2149 |
| myoC-8990 | - | UCUAGGAGGCAGGGCUAUAUU | 21 | 8736 |
| myoC-8991 | - | UUCUAGGAGGCAGGGCUAUAUU | 22 | 8737 |
| myoC-8992 | - | GUUCUAGGAGGCAGGGCUAUAUU | 23 | 8738 |
| myoC-8993 | - | GGUUCUAGGAGGCAGGGCUAUAUU | 24 | 8739 |
| myoC-8994 | - | ACUUAGUUUCUCCUUAUU | 18 | 8740 |
| myoC-8995 | - | UACUUAGUUUCUCCUUAUU | 19 | 8741 |
| myoC-1147 | - | UUACUUAGUUUCUCCUUAUU | 20 | 1447 |
| myoC-8996 | - | AUUACUUAGUUUCUCCUUAUU | 21 | 8742 |
| myoC-8997 | - | GAUUACUUAGUUUCUCCUUAUU | 22 | 8743 |
| myoC-8998 | - | AGAUUACUUAGUUUCUCCUUAUU | 23 | 8744 |
| myoC-8999 | - | AAGAUUACUUAGUUUCUCCUUAUU | 24 | 8745 |
| myoC-9000 | - | AGUUGUCAAUUGUCCCUU | 18 | 8746 |
| myoC-9001 | - | AAGUUGUCAAUUGUCCCUU | 19 | 8747 |
| myoC-9002 | - | AAAGUUGUCAAUUGUCCCUU | 20 | 8748 |
| myoC-9003 | - | GAAAGUUGUCAAUUGUCCCUU | 21 | 8749 |
| myoC-9004 | - | AGAAAGUUGUCAAUUGUCCCUU | 22 | 8750 |
| myoC-9005 | - | UAGAAAGUUGUCAAUUGUCCCUU | 23 | 8751 |
| myoC-9006 | - | GUAGAAAGUUGUCAAUUGUCCCUU | 24 | 8752 |
| myoC-9007 | - | CCGAGAGCCACAAUGCUU | 18 | 8753 |
| myoC-9008 | - | ACCGAGAGCCACAAUGCUU | 19 | 8754 |
| myoC-1966 | - | GACCGAGAGCCACAAUGCUU | 20 | 2125 |
| myoC-9009 | - | GGACCGAGAGCCACAAUGCUU | 21 | 8755 |
| myoC-9010 | - | AGGACCGAGAGCCACAAUGCUU | 22 | 8756 |
| myoC-9011 | - | CAGGACCGAGAGCCACAAUGCUU | 23 | 8757 |
| myoC-9012 | - | CCAGGACCGAGAGCCACAAUGCUU | 24 | 8758 |
| myoC-9013 | - | AAAUAAGAAUAGAAUCUU | 18 | 8759 |
| myoC-9014 | - | CAAAUAAGAAUAGAAUCUU | 19 | 8760 |
| myoC-2032 | - | UCAAAUAAGAAUAGAAUCUU | 20 | 2167 |
| myoC-9015 | - | AUCAAAUAAGAAUAGAAUCUU | 21 | 8761 |
| myoC-9016 | - | AAUCAAAUAAGAAUAGAAUCUU | 22 | 8762 |
| myoC-9017 | - | CAAUCAAAUAAGAAUAGAAUCUU | 23 | 8763 |
| myoC-9018 | - | CCAAUCAAAUAAGAAUAGAAUCUU | 24 | 8764 |
| myoC-9019 | - | GAGUCUGCCAGGGCAGUU | 18 | 8765 |
| myoC-9020 | - | UGAGUCUGCCAGGGCAGUU | 19 | 8766 |
| myoC-1160 | - | GUGAGUCUGCCAGGGCAGUU | 20 | 1460 |
| myoC-9021 | - | GGUGAGUCUGCCAGGGCAGUU | 21 | 8767 |
| myoC-9022 | - | AGGUGAGUCUGCCAGGGCAGUU | 22 | 8768 |
| myoC-9023 | - | GAGGUGAGUCUGCCAGGGCAGUU | 23 | 8769 |
| myoC-9024 | - | GGAGGUGAGUCUGCCAGGGCAGUU | 24 | 8770 |
| myoC-9025 | - | UCUGUGAGGGGGGAUGUU | 18 | 8771 |
| myoC-9026 | - | CUCUGUGAGGGGGGAUGUU | 19 | 8772 |
| myoC-1938 | - | UCUCUGUGAGGGGGGAUGUU | 20 | 2110 |
| myoC-9027 | - | UUCUCUGUGAGGGGGGAUGUU | 21 | 8773 |
| myoC-9028 | - | AUUCUCUGUGAGGGGGGAUGUU | 22 | 8774 |
| myoC-9029 | - | GAUUCUCUGUGAGGGGGGAUGUU | 23 | 8775 |
| myoC-9030 | - | UGAUUCUCUGUGAGGGGGGAUGUU | 24 | 8776 |
| myoC-9031 | - | UUAAAAUGACCUUUAUUU | 18 | 8777 |
| myoC-9032 | - | GUUAAAAUGACCUUUAUUU | 19 | 8778 |
| myoC-9033 | - | UGUUAAAAUGACCUUUAUUU | 20 | 8779 |
| myoC-9034 | - | AUGUUAAAAUGACCUUUAUUU | 21 | 8780 |
| myoC-9035 | - | GAUGUUAAAAUGACCUUUAUUU | 22 | 8781 |
| myoC-9036 | - | UGAUGUUAAAAUGACCUUUAUUU | 23 | 8782 |
| myoC-9037 | - | UUGAUGUUAAAAUGACCUUUAUUU | 24 | 8783 |
| myoC-9038 | - | AUAUUUGAAAACAUCUUU | 18 | 8784 |
| myoC-9039 | - | UAUAUUUGAAAACAUCUUU | 19 | 8785 |
| myoC-2055 | - | AUAUAUUUGAAAACAUCUUU | 20 | 2186 |
| myoC-9040 | - | UAUAUAUUUGAAAACAUCUUU | 21 | 8786 |
| myoC-9041 | - | UUAUAUAUUUGAAAACAUCUUU | 22 | 8787 |
| myoC-9042 | - | UUUAUAUAUUUGAAAACAUCUUU | 23 | 8788 |
| myoC-9043 | - | UUUUAUAUAUUUGAAAACAUCUUU | 24 | 8789 |
| myoC-9044 | - | UUGAAAAACUAUCCUUUU | 18 | 8790 |
| myoC-9045 | - | UUUGAAAAACUAUCCUUUU | 19 | 8791 |
| myoC-9046 | - | UUUUGAAAAACUAUCCUUUU | 20 | 8792 |
| myoC-9047 | - | CUUUUGAAAAACUAUCCUUUU | 21 | 8793 |
| myoC-9048 | - | CCUUUUGAAAAACUAUCCUUUU | 22 | 8794 |
| myoC-9049 | - | CCCUUUUGAAAAACUAUCCUUUU | 23 | 8795 |
| myoC-9050 | - | UCCCUUUUGAAAAACUAUCCUUUU | 24 | 8796 |
| myoC-9051 | - | GACUAUAUGAUUGGUUUU | 18 | 8797 |
| myoC-9052 | - | UGACUAUAUGAUUGGUUUU | 19 | 8798 |
| myoC-2026 | - | CUGACUAUAUGAUUGGUUUU | 20 | 2161 |
| myoC-9053 | - | GCUGACUAUAUGAUUGGUUUU | 21 | 8799 |
| myoC-9054 | - | UGCUGACUAUAUGAUUGGUUUU | 22 | 8800 |
| myoC-9055 | - | UUGCUGACUAUAUGAUUGGUUUU | 23 | 8801 |
| myoC-9056 | - | CUUGCUGACUAUAUGAUUGGUUUU | 24 | 8802 |

**Table 11A** provides exemplary targeting domains for knocking down the *MYOC* gene selected according to the first tier parameters. The targeting domains bind within 3454-2454bp upstream of transcription start site or 500bp upstream and downstream of transcription start site, have a high level of orthogonality and start with a 5'G. It is contemplated herein that in an embodiment the targeting domain hybridizes to the target domain through complementary base pairing. Any of the targeting domains in the table can be used with a N. meningitidis eiCas9 molecule or eiCas9 fusion protein (e.g., an eiCas9 fused to a transcription repressor domain) to alter the MYOC gene (e.g., reduce or eliminate MYOC gene expression, MYOC protein function, or the level of MYOC protein). One or more gRNA may be used to target an eiCas9 to the promoter region of the MYOC gene.

**Table 11A**

| 1st Tier | | | | |
|---|---|---|---|---|
| gRNA Name | DNA Strand | Targeting Domain | Target Site Length | Seq ID |
| myoC-2699 | + | GAGGAGGCUUGGAAGAC | 17 | 2643 |
| myoC-3140 | + | GAGGAAACACUGUCCCC | 17 | 2891 |
| myoC-826 | + | GAGAGGAAACCUCUGCC | 17 | 1023 |
| myoC-5354 | - | GAUGCCAGCUGUCCAGC | 17 | 5100 |
| myoC-9057 | - | GCGCUGCAGCUGGCCUG | 17 | 8803 |
| myoC-3125 | + | GGGUUGCCUUCACGCUGCCA | 20 | 2879 |
| myoC-3082 | + | GCCUGGCUCUGCUCUGGGCA | 20 | 2844 |
| myoC-9058 | + | GCGCUGUGACUGAUGGAGGA | 20 | 8804 |
| myoC-2153 | + | GAGGAGGAGGCUUGGAAGAC | 20 | 2263 |
| myoC-9059 | - | GUUAUCACUCUCUAGGGACC | 20 | 8805 |
| myoC-5355 | + | GCACAGAAGAACCUCAUUGC | 20 | 5101 |
| myoC-5356 | - | GGUUCUUCUGUGCACGUUGC | 20 | 5102 |

**Table 11B** provides exemplary targeting domains for knocking down the *MYOC* gene selected according to the second tier parameters. The targeting domains bind within 3454-2454bp upstream of transcription start site or 500bp upstream and downstream of transcription start site and have a high level of orthogonality. It is contemplated herein that in an embodiment the targeting domain hybridizes to the target domain through complementary base pairing. Any of the targeting domains in the table can be used with a N. meningitidis eiCas9 molecule or eiCas9 fusion protein (e.g., an eiCas9 fused to a transcription repressor domain) to alter the MYOC gene (e.g., reduce or eliminate MYOC gene expression, MYOC protein function, or the level of MYOC protein). One or more gRNA may be used to target an eiCas9 to the promoter region of the MYOC gene.

**Table 11B**

| 2nd Tier | | | | |
|---|---|---|---|---|
| gRNA Name | DNA Strand | Targeting Domain | Target Site Length | Seq ID |
| myoC-3153 | + | UUGCCUUCACGCUGCCA | 17 | 2903 |
| myoC-9060 | + | CUGUGACUGAUGGAGGA | 17 | 8806 |
| myoC-9061 | + | AACGGCCUAGGAAAUGA | 17 | 8807 |
| myoC-5357 | - | AGAGAGACAGCAGCACC | 17 | 5103 |
| myoC-9062 | - | AUCACUCUCUAGGGACC | 17 | 8808 |
| myoC-5358 | + | CAGAAGAACCUCAUUGC | 17 | 5104 |
| myoC-5359 | - | UCUUCUGUGCACGUUGC | 17 | 5105 |
| myoC-9063 | - | CGGGGCUGGGAGUUUUC | 17 | 8809 |
| myoC-5360 | + | UCAUUGCAGAGGCUUGG | 17 | 5106 |
| myoC-9064 | + | ACAACACUGAACAUCUG | 17 | 8810 |
| myoC-3152 | + | CACCAGGACUACUGGUG | 17 | 2902 |
| myoC-9065 | + | CACGAAGGUAGGGCAGU | 17 | 8811 |
| myoC-3111 | + | UCUCCAGCUCAGAUGCACCA | 20 | 2866 |
| myoC-9066 | + | AUUAACGGCCUAGGAAAUGA | 20 | 8812 |
| myoC-5361 | - | UACAGAGAGACAGCAGCACC | 20 | 5107 |
| myoC-3112 | + | UCUGAGGAAACACUGUCCCC | 20 | 2867 |
| myoC-749 | + | CUGGAGAGGAAACCUCUGCC | 20 | 1110 |
| myoC-9067 | - | UCACGGGGCUGGGAGUUUUC | 20 | 8813 |
| myoC-2108 | - | CCAGGCACCUCUCAGCACAG | 20 | 2230 |
| myoC-5362 | + | ACCUCAUUGCAGAGGCUUGG | 20 | 5108 |
| myoC-9068 | - | ACAGCGCUGCAGCUGGCCUG | 20 | 8814 |
| myoC-9069 | + | UGAACAACACUGAACAUCUG | 20 | 8815 |
| myoC-3124 | + | UUACACCAGGACUACUGGUG | 20 | 2878 |
| myoC-9070 | + | CUCCACGAAGGUAGGGCAGU | 20 | 8816 |

**Table 11C** provides exemplary targeting domains for knocking down the *MYOC* gene selected according to the third tier parameters. The targeting domains bind within 3454-2454bp upstream of transcription start site or 500bp upstream and downstream of transcription start site and start with a 5'G. It is contemplated herein that in an embodiment the targeting domain hybridizes to the target domain through complementary base pairing. Any of the targeting domains in the table can be used with a N. meningitidis eiCas9 molecule or eiCas9 fusion protein (e.g., an eiCas9 fused to a transcription repressor domain) to alter the MYOC gene (e.g., reduce or eliminate MYOC gene expression, MYOC protein function, or the level of MYOC protein). One or more gRNA may be used to target an eiCas9 to the promoter region of the MYOC gene.

**Table 11C**

| 3rd Tier | | | | |
|---|---|---|---|---|
| gRNA Name | DNA Strand | Targeting Domain | Target Site Length | Seq ID |
| myoC-2654 | - | GGCACCUCUCAGCACAG | 17 | 2610 |
| myoC-9071 | - | GAGCCUUUUUAUCUUUU | 17 | 8817 |
| myoC-5363 | + | GAUUCUCAUUUUCUUGCCUU | 20 | 5109 |

**Table 11D** provides exemplary targeting domains for knocking down the *MYOC* gene selected according to the fourth tier parameters. The targeting domains bind within 3454-2454bp upstream of transcription start site or 500bp upstream and downstream of transcription start site. It is contemplated herein that in an embodiment the targeting domain hybridizes to the target domain through complementary base pairing. Any of the targeting domains in the table can be used with a N. meningitidis eiCas9 molecule or eiCas9 fusion protein (e.g., an eiCas9 fused to a transcription repressor domain) to alter the MYOC gene (e.g., reduce or eliminate MYOC gene expression, MYOC protein function, or the level of MYOC protein). One or more gRNA may be used to target an eiCas9 to the promoter region of the MYOC gene.

**Table 11D**

| 4th Tier | | | | |
|---|---|---|---|---|
| gRNA Name | DNA Strand | Targeting Domain | Target Site Length | Seq ID |
| myoC-3139 | + | CCAGCUCAGAUGCACCA | 17 | 2890 |
| myoC-3084 | + | UGGCUCUGCUCUGGGCA | 17 | 2850 |
| myoC-820 | + | AGGACACCCAGGACCCC | 17 | 1138 |
| myoC-1788 | + | CUCUCCAGGGAGCUGAG | 17 | 2017 |
| myoC-5364 | + | UCUCAUUUUCUUGCCUU | 17 | 5110 |
| myoC-743 | + | CUCAGGACACCCAGGACCCC | 20 | 1107 |
| myoC-5365 | - | UGAGAUGCCAGCUGUCCAGC | 20 | 5111 |
| myoC-1678 | + | AGGCUCUCCAGGGAGCUGAG | 20 | 1939 |
| myoC-9072 | - | UGUGAGCCUUUUUAUCUUUU | 20 | 8818 |

**Table 11E** provides exemplary targeting domains for knocking down the *MYOC* gene selected according to the fifth tier parameters. The targeting domains bind within 2484-903bp upstream of transcription start site or the additional 500bp upstream and downstream of transcription start site (extending to lkb up and downstream of the transcription start site). It is contemplated herein that in an embodiment the targeting domain hybridizes to the target domain through complementary base pairing. Any of the targeting domains in the table can be used with a N. meningitidis eiCas9 molecule or eiCas9 fusion protein (e.g., an eiCas9 fused to a transcription repressor domain) to alter the MYOC gene (e.g., reduce or eliminate MYOC gene expression, MYOC protein function, or the level of MYOC protein). One or more gRNA may be used to target an eiCas9 to the promoter region of the MYOC gene.

**Table 11E**

| 5th Tier | | | | |
|---|---|---|---|---|
| gRNA Name | DNA Strand | Targeting Domain | Target Site Length | Seq ID |
| myoC-3150 | + | UUUUCAAAUAUAUAAAA | 17 | 2900 |
| myoC-3128 | - | AGUGUAUGAGCAAGAAA | 17 | 2881 |
| myoC-3147 | + | CUUUAAGCCACUUGAAA | 17 | 2897 |
| myoC-2810 | + | UCUUCCUGUUAAAAGAA | 17 | 2725 |
| myoC-3149 | + | AAACAAAUGAUAAUGAA | 17 | 2899 |
| myoC-2542 | - | GCAGUGGGAAUUGACCA | 17 | 2525 |
| myoC-3132 | - | UCCUAAGAGUAAAGCCA | 17 | 2884 |
| myoC-9073 | - | UCCAGGACCGAGAGCCA | 17 | 8819 |
| myoC-9074 | + | UGAGGACUGAUGGAGCA | 17 | 8820 |
| myoC-9075 | - | AGCUCCUGAGAGCUUCA | 17 | 8821 |
| myoC-2780 | + | UGUGGCUGUUGGGUUCA | 17 | 2702 |
| myoC-9076 | - | AGGCAAUCAUUAUUUCA | 17 | 8822 |
| myoC-9077 | - | CUCAGCCCUGUGGUGGA | 17 | 8823 |
| myoC-9078 | + | UGACUUGCUCAGAAUUA | 17 | 8824 |
| myoC-9079 | + | CAUAUAGUCAGCAAGAC | 17 | 8825 |
| myoC-3136 | - | AGUGGUAAUAACAGUAC | 17 | 2887 |
| myoC-9080 | + | AGAUUUCCCCCCUCACC | 17 | 8826 |
| myoC-9081 | - | AUUUAUUGGCUAUUGCC | 17 | 8827 |
| myoC-3126 | - | GUUCUGUGAACACUUCC | 17 | 2880 |
| myoC-3151 | + | AGCAUUCCUAUAGAAGC | 17 | 2901 |
| myoC-5371 | + | CCUUGCUACCUCCUGGC | 17 | 5117 |
| myoC-2521 | - | GAGCAAGUGGAAAAUGC | 17 | 2512 |
| myoC-9082 | - | GGGUGAGGGGGGAAAUC | 17 | 8828 |
| myoC-3146 | + | AGAAACACAGUUUGCUC | 17 | 2896 |
| myoC-3141 | + | AGAAAGAAAACCGAGUC | 17 | 2892 |
| myoC-9083 | + | UUUCCUCAUUCAAAUUC | 17 | 8829 |
| myoC-3137 | - | CUUUCUGAGAAGAGUUC | 17 | 2888 |
| myoC-2586 | - | GGUUUAUUAAUGUAAAG | 17 | 2553 |
| myoC-3138 | - | CACACACACAGAGUAAG | 17 | 2889 |
| myoC-3130 | - | UCAAGGGAAGUCGGGAG | 17 | 2882 |
| myoC-9084 | + | AUACUUGAAGGUGAUCG | 17 | 8830 |
| myoC-3085 | + | UGCUUUCCAACCUCCUG | 17 | 2851 |
| myoC-3144 | + | GAUAGUAAACAUCGCUG | 17 | 2894 |
| myoC-9085 | + | ACCUAGGCUUGAAUCUG | 17 | 8831 |
| myoC-3142 | + | UCUCCCGACUUCCCUUG | 17 | 2893 |
| myoC-9086 | - | CCUUUUUUGAACCUUUG | 17 | 8832 |
| myoC-9087 | + | GACUGUAGGUUAAUAAU | 17 | 8833 |
| myoC-3133 | - | CCUAGGUCUUGCUGACU | 17 | 2885 |
| myoC-3134 | - | UUUCAGCGAUGUUUACU | 17 | 2886 |
| myoC-9088 | - | CUAGUAAUUUAGCUCCU | 17 | 8834 |
| myoC-3131 | - | AGGUAGUAACUGAGGCU | 17 | 2883 |
| myoC-9089 | - | UUGUAAAUGUCUCAAGU | 17 | 8835 |
| myoC-9090 | - | UGCAGAGACUAACUGGU | 17 | 8836 |
| myoC-3148 | + | AAUAUAGUAUAAAAUGU | 17 | 2898 |
| myoC-9091 | + | UUGGCAAAUGCCAUUGU | 17 | 8837 |
| myoC-5364 | + | UCUCAUUUUCUUGCCUU | 17 | 5110 |
| myoC-3145 | + | CUAAAGAUUCUAUUCUU | 17 | 2895 |
| myoC-3122 | + | AUGUUUUCAAAUAUAUAAAA | 20 | 2876 |
| myoC-3100 | - | GAUAGUGUAUGAGCAAGAAA | 20 | 2857 |
| myoC-3119 | + | UAACUUUAAGCCACUUGAAA | 20 | 2873 |
| myoC-2264 | + | UUUUCUUCCUGUUAAAAGAA | 20 | 2345 |
| myoC-3121 | + | AGGAAACAAAUGAUAAUGAA | 20 | 2875 |
| myoC-1996 | - | AGGGCAGUGGGAAUUGACCA | 20 | 2145 |
| myoC-3104 | - | CAUUCCUAAGAGUAAAGCCA | 20 | 2860 |
| myoC-9092 | - | GACUCCAGGACCGAGAGCCA | 20 | 8838 |
| myoC-9093 | + | CAGUGAGGACUGAUGGAGCA | 20 | 8839 |
| myoC-9094 | - | UUUAGCUCCUGAGAGCUUCA | 20 | 8840 |
| myoC-2234 | + | AAAUGUGGCUGUUGGGUUCA | 20 | 2322 |
| myoC-9095 | - | CAAAGGCAAUCAUUAUUUCA | 20 | 8841 |
| myoC-9096 | - | CUACUCAGCCCUGUGGUGGA | 20 | 8842 |
| myoC-9097 | + | UUGUGACUUGCUCAGAAUUA | 20 | 8843 |
| myoC-9098 | + | AAUCAUAUAGUCAGCAAGAC | 20 | 8844 |
| myoC-3108 | - | CAAAGUGGUAAUAACAGUAC | 20 | 2863 |
| myoC-9099 | + | GGCAGAUUUCCCCCCUCACC | 20 | 8845 |
| myoC-9100 | - | UAUAUUUAUUGGCUAUUGCC | 20 | 8846 |
| myoC-3098 | - | CGUGUUCUGUGAACACUUCC | 20 | 2856 |
| myoC-3123 | + | GAGAGCAUUCCUAUAGAAGC | 20 | 2877 |
| myoC-5388 | + | CAGCCUUGCUACCUCCUGGC | 20 | 5134 |
| myoC-1975 | - | CCAGAGCAAGUGGAAAAUGC | 20 | 2132 |
| myoC-9101 | - | UAGGGGUGAGGGGGGAAAUC | 20 | 8847 |
| myoC-3118 | + | UGGAGAAACACAGUUUGCUC | 20 | 2872 |
| myoC-3113 | + | ACCAGAAAGAAAACCGAGUC | 20 | 2868 |
| myoC-9102 | + | UUUUUUCCUCAUUCAAAUUC | 20 | 8848 |
| myoC-3109 | - | CAUCUUUCUGAGAAGAGUUC | 20 | 2864 |
| myoC-2040 | - | UUGGGUUUAUUAAUGUAAAG | 20 | 2173 |
| myoC-3110 | - | AUGCACACACACAGAGUAAG | 20 | 2865 |
| myoC-3102 | - | AGUUCAAGGGAAGUCGGGAG | 20 | 2858 |
| myoC-9103 | + | GUAAUACUUGAAGGUGAUCG | 20 | 8849 |
| myoC-3083 | + | UGCUGCUUUCCAACCUCCUG | 20 | 2845 |
| myoC-3116 | + | UCAGAUAGUAAACAUCGCUG | 20 | 2870 |
| myoC-9104 | + | AAGACCUAGGCUUGAAUCUG | 20 | 8850 |
| myoC-3114 | + | AGGUCUCCCGACUUCCCUUG | 20 | 2869 |
| myoC-9105 | - | UAUCCUUUUUUGAACCUUUG | 20 | 8851 |
| myoC-9106 | + | CUGGACUGUAGGUUAAUAAU | 20 | 8852 |
| myoC-3105 | - | AAGCCUAGGUCUUGCUGACU | 20 | 2861 |
| myoC-3106 | - | UCAUUUCAGCGAUGUUUACU | 20 | 2862 |
| myoC-8886 | - | UUACUAGUAAUUUAGCUCCU | 20 | 8632 |
| myoC-3103 | - | ACAAGGUAGUAACUGAGGCU | 20 | 2859 |
| myoC-9107 | - | CAUUUGUAAAUGUCUCAAGU | 20 | 8853 |
| myoC-9108 | - | GAAUGCAGAGACUAACUGGU | 20 | 8854 |
| myoC-3120 | + | UGUAAUAUAGUAUAAAAUGU | 20 | 2874 |
| myoC-9109 | + | UUAUUGGCAAAUGCCAUUGU | 20 | 8855 |
| myoC-5363 | + | GAUUCUCAUUUUCUUGCCUU | 20 | 5109 |
| myoC-3117 | + | GCUCUAAAGAUUCUAUUCUU | 20 | 2871 |

**Table 12A** provides exemplary targeting domains for the mutational hotspot 477-502 target site in the MYOC gene selected according to the first tier parameters. The targeting domains bind within 200 bp upstream from the mutational hotspot 477-502 target site, have a high level of orthogonality and start with a 5'G. It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 12A**

| 1st Tier | | | | |
|---|---|---|---|---|
| gRNA Name | DNA Strand | Targeting Domain | Target Site Length | SEQ ID NO |
| MYOC-hotspot200up-1 | + | GCUGCUGACGGUGUACA | 17 | 909 |
| MYOC-hotspot200up-2 | + | GCGGUUCUUGAAUGGGA | 17 | 446 |
| MYOC-hotspot200up-3 | - | GCUUAUGACACAGGCAC | 17 | 451 |
| MYOC-hotspot200up-4 | + | GACGGUAGCAUCUGCUG | 17 | 907 |
| MYOC-hotspot200up-5 | - | GGAACUCGAACAAACCU | 17 | 884 |
| MYOC-hotspot200up-6 | + | GUAGCUGCUGACGGUGUACA | 20 | 790 |
| MYOC-hotspot200u p-7 | - | GUCAACUUUGCUUAUGACAC | 20 | 439 |
| MYOC-hotspot200up-8 | + | GGUUCUUGAAUGGGAUGGUC | 20 | 449 |
| MYOC-hotspot200up-9 | + | GUUGACGGUAGCAUCUGCUG | 20 | 788 |
| MYOC-hotspot200up-10 | - | GCCAAUGCCUUCAUCAUCUG | 20 | 768 |
| MYOC-hotspot200up-11 | + | GCCACAGAUGAUGAAGGCAU | 20 | 792 |

**Table 12B** provides exemplary targeting domains for the mutational hotspot 477-502 target site in the MYOC gene selected according to the second tier parameters. The targeting domains bind within 200 bp upstream from the mutational hotspot 477-502 target site and have a high level of orthogonality. It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 12B**

| 2nd Tier | | | | |
|---|---|---|---|---|
| gRNA Name | DNA Strand | Targeting Domain | Target Site Length | SEQ ID NO |
| MYOC-hotspot200up-12 | + | UUAUAGCGGUUCUUGAA | 17 | 473 |
| MYOC-hotspot200up-13 | + | UGGCGACUGACUGCUUA | 17 | 912 |
| MYOC-hotspot200up-14 | - | AACUUUGCUUAUGACAC | 17 | 464 |
| MYOC-hotspot200up-15 | - | UGGAACUCGAACAAACC | 17 | 883 |
| MYOC-hotspot200up-16 | + | ACGGAUGUUUGUCUCCC | 17 | 913 |
| MYOC-hotspot200up-17 | + | UCUUGAAUGGGAUGGUC | 17 | 475 |
| MYOC-hotspot200up-18 | + | UGCUGCUGUACUUAUAG | 17 | 472 |
| MYOC-hotspot200up-19 | + | UAUAGCGGUUCUUGAAU | 17 | 474 |
| MYOC-hotspot200up-20 | + | UACUUAUAGCGGUUCUUGAA | 20 | 461 |
| MYOC-hotspot200up-21 | + | AUAGCGGUUCUUGAAUGGGA | 20 | 443 |
| MYOC-hotspot200up-22 | + | CAAGGUGCCACAGAUGAUGA | 20 | 791 |
| MYOC-hotspot200up-23 | + | CAUUGGCGACUGACUGCUUA | 20 | 793 |
| MYOC-hotspot200up-24 | - | UUUGCUUAUGACACAGGCAC | 20 | 453 |
| MYOC-hotspot200up-25 | - | AUCUGGAACUCGAACAAACC | 20 | 766 |
| MYOC-hotspot200up-26 | + | CUUACGGAUGUUUGUCUCCC | 20 | 794 |
| MYOC-hotspot200up-27 | + | ACUUAUAGCGGUUCUUGAAU | 20 | 462 |
| MYOC-hotspot200up-28 | - | UCUGGAACUCGAACAAACCU | 20 | 767 |

**Table 12C** provides exemplary targeting domains for the mutational hotspot 477-502 target site in the MYOC gene selected according to the third tier parameters. The targeting domains bind within 200 bp upstream from the mutational hotspot 477-502 target site and start with a 5'G. It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 12C**

| 3rd Tier | | | | |
|---|---|---|---|---|
| gRNA Name | DNA Strand | Targeting Domain | Target Site Length | SEQ ID NO |
| MYOC-hotspot200up-29 | + | GGUGCCACAGAUGAUGA | 17 | 910 |
| MYOC-hotspot200up-30 | + | GUCAUAAGCAAAGUUGA | 17 | 447 |
| MYOC-hotspot200up-31 | + | GUUCUUGAAUGGGAUGGUCA | 20 | 450 |

**Table 12D** provides exemplary targeting domains for the mutational hotspot 477-502 target site in the MYOC gene selected according to the fourth tier parameters. The targeting domains bind within 200 bp upstream from the mutational hotspot 477-502 target site. It is contemplated herein that in an embodiment the targeting domain hybridizes to the target domain through complementary base pairing. Any of the targeting domains in the table can be used with a *S. pyogenes* Cas9 molecule that generates a double stranded break (Cas9 nuclease) or a single-stranded break (Cas9 nickase).

**Table 12D**

| 4th Tier | | | | |
|---|---|---|---|---|
| gRNA Name | DNA Strand | Targeting Domain | Target Site Length | SEQ ID NO |
| MYOC-hotspot200up-32 | + | CUUGAAUGGGAUGGUCA | 17 | 476 |
| MYOC-hotspot200up-33 | + | UGAGGUGUAGCUGCUGA | 17 | 908 |
| MYOC-hotspot200up-34 | - | AAUGCCUUCAUCAUCUG | 17 | 885 |
| MYOC-hotspot200up-35 | + | ACAGAUGAUGAAGGCAU | 17 | 911 |
| MYOC-hotspot200up-36 | + | UGCUGAGGUGUAGCUGCUGA | 20 | 789 |
| MYOC-hotspot200up-37 | + | UGUGUCAUAAGCAAAGUUGA | 20 | 463 |

**Table 13A** provides exemplary targeting domains for the mutational hotspot 477-502 target site in the MYOC gene selected according to the first tier parameters. The targeting domains bind within 200 bp upstream from the mutational hotspot 477-502 target site, have a high level of orthogonality, start with a 5'G, and PAM is NNGRRT. It is contemplated herein that in an embodiment the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase).

**Table 13A**

| 1st Tier | | | | |
|---|---|---|---|---|
| gRNA Name | DNA Strand | Targeting Domain | Target Site Length | SEQ ID NO |
| MYOC-hotspot200up-38 | + | GUACUUAUAGCGGUUCUUGAA | 21 | 3535 |
| MYOC-hotspot200up-39 | + | GCUGUACUUAUAGCGGUUCUUGAA | 24 | 3536 |
| MYOC-hotspot200up-40 | + | GCUGCUGUACUUAUAGCGGUUC | 22 | 3553 |
| MYOC-hotspot200up-41 | + | GCGGUUCUUGAAUGGGAUGGU | 21 | 3564 |
| MYOC-hotspot200up-42 | + | GAUGUUUGUCUCCCAGGUUUGU | 22 | 3566 |
| MYOC-hotspot200up-43 | + | GGAUGUUUGUCUCCCAGGUUUGU | 23 | 3567 |

**Table 13B** provides exemplary targeting domains for the mutational hotspot 477-502 target site in the MYOC gene selected according to the second tier parameters. The targeting domains bind within 200 bp upstream from the mutational hotspot 477-502 target site, have a high level of orthogonality and PAM is NNGRRT. It is contemplated herein that in an embodiment the targeting domain hybridizes to the target domain through complementary base pairing. Any of the targeting domains in the table can be used with a *S. aureus* Cas9 molecule that generates a double stranded break (Cas9 nuclease) or a single-stranded break (Cas9 nickase).

**Table 13B**

| 2nd Tier | | | | |
|---|---|---|---|---|
| gRNA Name | DNA Stran d | Targeting Domain | Target Site Length | SEQ ID NO |
| MYOC-hotspot200up-44 | + | UGUACUUAUAGCGGUUCUUGAA | 22 | 3612 |
| MYOC-hotspot200up-45 | + | CUGUACUUAUAGCGGUUCUUGAA | 23 | 3613 |
| MYOC-hotspot200up-46 | + | CUGCUGUACUUAUAGCGGUUC | 21 | 3658 |
| MYOC-hotspot200up-47 | + | UGCUGCUGUACUUAUAGCGGUUC | 23 | 3659 |
| MYOC-hotspot200up-48 | + | AUGCUGCUGUACUUAUAGCGGUU C | 24 | 3660 |
| MYOC-hotspot200up-49 | + | AGCGGUUCUUGAAUGGGAUGGU | 22 | 3680 |
| MYOC-hotspot200up-50 | + | UAGCGGUUCUUGAAUGGGAUGGU | 23 | 3681 |
| MYOC-hotspot200up-51 | + | AUAGCGGUUCUUGAAUGGGAUGG U | 24 | 3682 |
| MYOC-hotspot200up-52 | + | AUGUUUGUCUCCCAGGUUUGU | 21 | 3690 |
| MYOC-hotspot200up-53 | + | CGGAUGUUUGUCUCCCAGGUUUG U | 24 | 3691 |

**Table 13C** provides exemplary targeting domains for the mutational hotspot 477-502 target site in the MYOC gene selected according to the third tier parameters. The targeting domains bind within 200 bp upstream from the mutational hotspot 477-502 target site, start with a 5' G and PAM is NNGRRT. It is contemplated herein that in an embodiment the targeting domain hybridizes to the target domain through complementary base pairing. Any of the targeting domains in the Table can be used with a *S. aureus* Cas9 molecule that generates a double stranded break (Cas9 nuclease) or a single-stranded break (Cas9 nickase).

**Table 13C**

| 3rd Tier | | | | |
|---|---|---|---|---|
| gRNA Name | DNA Strand | Targeting Domain | Target Site Length | SEQ ID NO |
| MYOC-hotspot200up-54 | + | GCUGUACUUAUAGCGGUUC | 19 | 3552 |
| MYOC-hotspot200up-55 | + | GUUCUUGAAUGGGAUGGU | 18 | 3562 |
| MYOC-hotspot200up-56 | + | GGUUCUUGAAUGGGAUGGU | 19 | 3563 |
| MYOC-hotspot200up-57 | + | GUUUGUCUCCCAGGUUUGU | 19 | 3565 |
| MYOC-hotspot200up-58 | + | GCAUUGGCGACUGACUGCUU | 20 | 2793 |
| MYOC-hotspot200up-59 | + | GGCAUUGGCGACUGACUGCUU | 21 | 3571 |
| MYOC-hotspot200up-60 | + | GAAGGCAUUGGCGACUGACUGCUU | 24 | 3572 |

**Table 13D** provides exemplary targeting domains for the mutational hotspot 477-502 target site in the MYOC gene selected according to the fourth tier parameters. The targeting domains bind within 200 bp upstream from the mutational hotspot 477-502 target site, and PAM is NNGRRT. It is contemplated herein that in an embodiment the targeting domain hybridizes to the target domain through complementary base pairing. Any of the targeting domains in the Table can be used with a *S. aureus* Cas9 molecule that generates a double stranded break (Cas9 nuclease) or a single-stranded break (Cas9 nickase).

**Table 13D**

| 4th Tier | | | | |
|---|---|---|---|---|
| gRNA Name | DNA Strand | Targeting Domain | Target Site Length | SEQ ID NO |
| MYOC-hotspot200up-61 | + | CUUAUAGCGGUUCUUGAA | 18 | 3610 |
| MYOC-hotspot200up-62 | + | ACUUAUAGCGGUUCUUGAA | 19 | 3611 |
| MYOC-hotspot200up-20 | + | UACUUAUAGCGGUUCUUGAA | 20 | 461 |
| MYOC-hotspot200up-63 | + | CUGUACUUAUAGCGGUUC | 18 | 3657 |
| MYOC-hotspot200up-64 | + | UGCUGUACUUAUAGCGGUUC | 20 | 1856 |
| MYOC-hotspot200up-65 | + | CGGUUCUUGAAUGGGAUGGU | 20 | 1854 |
| MYOC-hotspot200up-66 | + | UUUGUCUCCCAGGUUUGU | 18 | 3689 |
| MYOC-hotspot200up-67 | + | UGUUUGUCUCCCAGGUUUGU | 20 | 2792 |
| MYOC-hotspot200up-68 | + | AUUGGCGACUGACUGCUU | 18 | 3695 |
| MYOC-hotspot200up-69 | + | CAUUGGCGACUGACUGCUU | 19 | 3696 |
| MYOC-hotspot200up-70 | + | AGGCAUUGGCGACUGACUGCUU | 22 | 3697 |
| MYOC-hotspot200up-71 | + | AAGGCAUUGGCGACUGACUGCUU | 23 | 3698 |

**Table 13E** provides exemplary targeting domains for the mutational hotspot 477-502 target site in the MYOC gene selected according to the fifth tier parameters. The targeting domains bind within 200 bp upstream from the mutational hotspot 477-502 target site, and PAM is NNGRRV. It is contemplated herein that in an embodiment the targeting domain hybridizes to the target domain through complementary base pairing. Any of the targeting domains in the Table can be used with a *S. aureus* Cas9 molecule that generates a double stranded break (Cas9 nuclease) or a single-stranded break (Cas9 nickase).

**Table 13E**

| 5th Tier | | | | |
|---|---|---|---|---|
| gRNA Name | DNA Strand | Targeting Domain | Target Site Length | SEQ ID NO |
| MYOC-hotspot200up-72 | + | ACUUAUAGCGGUUCUUGA | 18 | 3906 |
| MYOC-hotspot200up-73 | + | UACUUAUAGCGGUUCUUGA | 19 | 3907 |
| MYOC-hotspot200up-74 | + | GUACUUAUAGCGGUUCUUGA | 20 | 1855 |
| MYOC-hotspot200up-75 | + | UGUACUUAUAGCGGUUCUUGA | 21 | 3908 |
| MYOC-hotspot200up-76 | + | CUGUACUUAUAGCGGUUCUUGA | 22 | 3909 |
| MYOC-hotspot200up-77 | + | GCUGUACUUAUAGCGGUUCUUGA | 23 | 3910 |
| MYOC-hotspot200up-78 | + | UGCUGUACUUAUAGCGGUUCUUGA | 24 | 3911 |
| MYOC-hotspot200up-79 | + | UACAAGGUGCCACAGAUG | 18 | 4158 |
| MYOC-hotspot200up-80 | + | GUACAAGGUGCCACAGAUG | 19 | 4159 |
| MYOC-hotspot200up-81 | + | UGUACAAGGUGCCACAGAUG | 20 | 2794 |
| MYOC-hotspot200up-82 | + | GUGUACAAGGUGCCACAGAUG | 21 | 4160 |
| MYOC-hotspot200up-83 | + | GGUGUACAAGGUGCCACAGAUG | 22 | 4161 |
| MYOC-hotspot200up-84 | + | CGGUGUACAAGGUGCCACAGAUG | 23 | 4162 |
| MYOC-hotspot200up-85 | + | ACGGUGUACAAGGUGCCACAGAUG | 24 | 4163 |
| MYOC-hotspot200up-86 | + | AGUUGACGGUAGCAUCUG | 18 | 4178 |
| MYOC-hotspot200up-87 | + | AAGUUGACGGUAGCAUCUG | 19 | 4179 |
| MYOC-hotspot200up-88 | + | AAAGUUGACGGUAGCAUCUG | 20 | 1853 |
| MYOC-hotspot200up-89 | + | CAAAGUUGACGGUAGCAUCUG | 21 | 4180 |
| MYOC-hotspot200up-90 | + | GCAAAGUUGACGGUAGCAUCUG | 22 | 4181 |
| MYOC-hotspot200up-91 | + | AGCAAAGUUGACGGUAGCAUCUG | 23 | 4182 |
| MYOC-hotspot200up-92 | + | AAGCAAAGUUGACGGUAGCAUCUG | 24 | 4183 |
| MYOC-hotspot200up-93 | - | CUGGAACUCGAACAAACC | 18 | 4537 |
| MYOC-hotspot200up-94 | - | UCUGGAACUCGAACAAACC | 19 | 4538 |
| MYOC-hotspot200up-25 | - | AUCUGGAACUCGAACAAACC | 20 | 766 |
| MYOC-hotspot200up-95 | - | ACCCUGACCAUCCCAUUC | 18 | 4673 |
| MYOC-hotspot200up-96 | - | GACCCUGACCAUCCCAUUC | 19 | 4674 |
| MYOC-hotspot200up-97 | - | AGACCCUGACCAUCCCAUUC | 20 | 1846 |
| MYOC-hotspot200up-98 | - | AAGACCCUGACCAUCCCAUUC | 21 | 4675 |
| MYOC-hotspot200up-99 | - | CAAGACCCUGACCAUCCCAUUC | 22 | 4676 |
| MYOC-hotspot200up-100 | - | GCAAGACCCUGACCAUCCCAUUC | 23 | 4677 |
| MYOC-hotspot200up-101 | - | AGCAAGACCCUGACCAUCCCAUUC | 24 | 4678 |
| MYOC-hotspot200up-102 | - | UGGAACUCGAACAAACCU | 18 | 4978 |
| MYOC-hotspot200up-103 | - | CUGGAACUCGAACAAACCU | 19 | 4979 |
| MYOC-hotspot200up-28 | - | UCUGGAACUCGAACAAACCU | 20 | 767 |

**Table 14A** provides exemplary targeting domains for the mutational hotspot 477-502 target site in the MYOC gene selected according to the second tier parameters. The targeting domains bind within 200 bp upstream from the mutational hotspot 477-502 target site, have a high level of orthogonality and start with a 5'G. It is contemplated herein that in an embodiment the targeting domain hybridizes to the target domain through complementary base pairing. Any of the targeting domains in the Table can be used with a *N*. *meningitidis* Cas9 molecule that generates a double stranded break (Cas9 nuclease) or a single-stranded break (Cas9 nickase).

**Table 14A**

| 2nd Tier | | | | |
|---|---|---|---|---|
| gRNA Name | DNA Strand | Targeting Domain | Target Site Length | SEQ ID NO |
| MYOC-hotspot200up-104 | - | UCAGCAGAUGCUACCGUCAA | 20 | 5129 |

**Table 14B** provides exemplary targeting domains for the mutational hotspot 477-502 target site in the MYOC gene selected according to the third tier parameters. The targeting domains bind within 200 bp upstream from the mutational hotspot 477-502 target site and start with a 5'G. It is contemplated herein that in an embodiment the targeting domain hybridizes to the target domain through complementary base pairing. Any of the targeting domains in the Table can be used with a *N*. *meningitidis* Cas9 molecule that generates a double stranded break (Cas9 nuclease) or a single-stranded break (Cas9 nickase).

**Table 14B**

| 3rd Tier | | | | |
|---|---|---|---|---|
| gRNA Name | DNA Strand | Targeting Domain | Target Site Length | SEQ ID NO |
| MYOC-hotspot200up-105 | - | GCAGAUGCUACCGUCAA | 17 | 5112 |

**Table 14C** provides exemplary targeting domains for the mutational hotspot 477-502 target site in the MYOC gene selected according to the fourth tier parameters. The targeting domains bind within 200 bp upstream from the mutational hotspot 477-502 target site. It is contemplated herein that in an embodiment the targeting domain hybridizes to the target domain through complementary base pairing. Any of the targeting domains in the Table can be used with a *N*. *meningitidis* Cas9 molecule that generates a double stranded break (Cas9 nuclease) or a single-stranded break (Cas9 nickase).

**Table 14C**

| 4th Tier | | | | |
|---|---|---|---|---|
| gRNA Name | DNA Strand | Targeting Domain | Target Site Length | SEQ ID NO |
| MYOC-hotspot200up-106 | + | UGAAGGCAUUGGCGACU | 17 | 5124 |
| MYOC-hotspot200up-107 | + | UGAUGAAGGCAUUGGCGACU | 20 | 5140 |

**Table 15A** provides exemplary targeting domains for the mutational hotspot 477-502 target site in the MYOC gene selected according to the first tier parameters. The targeting domains bind within 200 bp downstream from the mutational hotspot 477-502 target site, have a high level of orthogonality and start with a 5' G. It is contemplated herein that in an embodiment the targeting domain hybridizes to the target domain through complementary base pairing. Any of the targeting domains in the Table can be used with a *S. pyogenes* Cas9 molecule that generates a double stranded break (Cas9 nuclease) or a single-stranded break (Cas9 nickase).

**Table 15A**

| 1st Tier | | | | |
|---|---|---|---|---|
| gRNA Name | DNA Strand | Targeting Domain | Target Site Length | SEQ ID NO |
| MYOC-hotspot200down-1 | - | GCUGUACAGGCAAUGGCAGA | 20 | 771 |
| MYOC-hotspot200down-2 | - | GAAAAGCCUCCAAGCUGUAC | 20 | 769 |
| MYOC-hotspot200down-3 | + | GGUGACCAUGUUCAUCCUUC | 20 | 852 |

**Table 15B** provides exemplary targeting domains for the mutational hotspot 477-502 target site in the MYOC gene selected according to the second tier parameters. The targeting domains bind within 200 bp downstream from the mutational hotspot 477-502 target site and have a high level of orthogonality. It is contemplated herein that in an embodiment the targeting domain hybridizes to the target domain through complementary base pairing. Any of the targeting domains in the Table can be used with a *S. pyogenes* Cas9 molecule that generates a double stranded break (Cas9 nuclease) or a single-stranded break (Cas9 nickase).

**Table 15B**

| 2nd Tier | | | | |
|---|---|---|---|---|
| gRNA Name | DNA Strand | Targeting Domain | Target Site Length | SEQ ID NO |
| MYOC-hotspot200down-4 | + | AUUCCUGAAUAGUUAGA | 17 | 971 |
| MYOC-hotspot200down-5 | - | CAGGAAUUGUAGUCUGA | 17 | 949 |
| MYOC-hotspot200down-6 | - | AAGCCUCCAAGCUGUAC | 17 | 887 |
| MYOC-hotspot200down-7 | - | UCACCAUCUAACUAUUC | 17 | 947 |
| MYOC-hotspot200down-8 | + | UUGCCUGUACAGCUUGG | 17 | 906 |
| MYOC-hotspot200down-9 | - | CCUCCAAGCUGUACAGGCAA | 20 | 770 |
| MYOC-hotspot200down-10 | - | UUAAUCCAGAAGGAUGAACA | 20 | 826 |
| MYOC-hotspot200down-11 | - | CAAGUUUUCAUUAAUCCAGA | 20 | 825 |
| MYOC-hotspot200down-12 | + | ACAAUUCCUGAAUAGUUAGA | 20 | 851 |
| MYOC-hotspot200down-13 | - | AUUCAGGAAUUGUAGUCUGA | 20 | 829 |
| MYOC-hotspot200down-14 | + | CCCUUCAGCCUGCUCCCCCC | 20 | 785 |
| MYOC-hotspot200down-15 | + | AGUCAAAGCUGCCUGGGCCC | 20 | 1802 |
| MYOC-hotspot200down-16 | - | AAGGAGAUGCUCAGGGCUCC | 20 | 774 |
| MYOC-hotspot200down-17 | + | AAAGCUGCCUGGGCCCUGGC | 20 | 1803 |
| MYOC-hotspot200down-18 | - | UGGUCACCAUCUAACUAUUC | 20 | 827 |
| MYOC-hotspot200down-19 | + | CCAUUGCCUGUACAGCUUGG | 20 | 787 |
| MYOC-hotspot200down-20 | + | CUUCUGGAUUAAUGAAAACU | 20 | 853 |
| MYOC-hotspot200down-21 | - | AGGAGAUGCUCAGGGCUCCU | 20 | 775 |
| MYOC-hotspot200down-22 | + | CUGCCAUUGCCUGUACAGCU | 20 | 786 |

**Table 15C** provides exemplary targeting domains for the mutational hotspot 477-502 target site in the MYOC gene selected according to the third tier parameters. The targeting domains bind within 200 bp downstream from the mutational hotspot 477-502 target site and start with a 5'G. It is contemplated herein that in an embodiment the targeting domain hybridizes to the target domain through complementary base pairing. Any of the targeting domains in the Table can be used with a *S. pyogenes* Cas9 molecule that generates a double stranded break (Cas9 nuclease) or a single-stranded break (Cas9 nickase).

**Table 15C**

| 3rd Tier | | | | |
|---|---|---|---|---|
| gRNA Name | DNA Strand | Targeting Domain | Target Site Length | SEQ ID NO |
| MYOC-hotspot200down-23 | - | GGGGGGAGCAGGCUGAA | 17 | 899 |
| MYOC-hotspot200down-24 | - | GGAGAGCCAGCCAGCCA | 17 | 901 |
| MYOC-hotspot200down-25 | - | GCAGAAGGAGAUGCUCA | 17 | 891 |
| MYOC-hotspot200down-26 | - | GUUUUCAUUAAUCCAGA | 17 | 945 |
| MYOC-hotspot200down-27 | - | GUACAGGCAAUGGCAGA | 17 | 889 |
| MYOC-hotspot200down-28 | - | GGGAGAGCCAGCCAGCC | 17 | 900 |
| MYOC-hotspot200down-29 | - | GAGAUGCUCAGGGCUCC | 17 | 892 |
| MYOC-hotspot200down-30 | - | GGGCUCCUGGGGGGAGC | 17 | 897 |
| MYOC-hotspot200down-31 | + | GCUGCCUGGGCCCUGGC | 17 | 1801 |
| MYOC-hotspot200down-32 | - | GGCAGAAGGAGAUGCUC | 17 | 890 |
| MYOC-hotspot200down-33 | + | GACCAUGUUCAUCCUUC | 17 | 972 |
| MYOC-hotspot200down-34 | - | GAUGCUCAGGGCUCCUG | 17 | 894 |
| MYOC-hotspot200down-35 | - | GAGCCAGCCAGCCAGGGCCC | 20 | 784 |
| MYOC-hotspot200down-36 | - | GAAGGGAGAGCCAGCCAGCC | 20 | 782 |
| MYOC-hotspot200down-37 | + | GGAAAGCAGUCAAAGCUGCC | 20 | 854 |
| MYOC-hotspot200down-38 | - | GAGAUGCUCAGGGCUCCUGG | 20 | 777 |
| MYOC-hotspot200down-39 | - | GGAGAUGCUCAGGGCUCCUG | 20 | 776 |
| MYOC-hotspot200down-40 | + | GAAAGCAGUCAAAGCUGCCU | 20 | 855 |

**Table 15D** provides exemplary targeting domains for the mutational hotspot 477-502 target site in the MYOC gene selected according to the fourth tier parameters. The targeting domains bind within 200 bp downstream from the mutational hotspot 477-502 target site. It is contemplated herein that in an embodiment the targeting domain hybridizes to the target domain through complementary base pairing. Any of the targeting domains in the Table can be used with a *S. pyogenes* Cas9 molecule that generates a double stranded break (Cas9 nuclease) or a single-stranded break (Cas9 nickase).

**Table 15D**

| 4th Tier | | | | |
|---|---|---|---|---|
| gRNA Name | DNA Strand | Targeting Domain | Target Site Length | SEQ ID NO |
| MYOC-hotspot200down-41 | - | CCAAGCUGUACAGGCAA | 17 | 888 |
| MYOC-hotspot200down-42 | - | AUCCAGAAGGAUGAACA | 17 | 946 |
| MYOC-hotspot200down-43 | - | UGGGGGGAGCAGGCUGA | 17 | 898 |
| MYOC-hotspot200down-44 | + | UUCAGCCUGCUCCCCCC | 17 | 904 |
| MYOC-hotspot200down-45 | - | CCAGCCAGCCAGGGCCC | 17 | 902 |
| MYOC-hotspot200down-46 | + | CAAAGCUGCCUGGGCCC | 17 | 1805 |
| MYOC-hotspot200down-47 | + | AAGCAGUCAAAGCUGCC | 17 | 974 |
| MYOC-hotspot200down-48 | + | CCUGGGCCCUGGCUGGC | 17 | 903 |
| MYOC-hotspot200down-49 | - | UGCUCAGGGCUCCUGGG | 17 | 896 |
| MYOC-hotspot200down-50 | - | AUGCUCAGGGCUCCUGG | 17 | 895 |
| MYOC-hotspot200down-51 | - | UCAGGAAUUGUAGUCUG | 17 | 948 |
| MYOC-hotspot200down-52 | + | CUGGAUUAAUGAAAACU | 17 | 973 |
| MYOC-hotspot200down-53 | + | AGCAGUCAAAGCUGCCU | 17 | 975 |
| MYOC-hotspot200down-54 | - | AGAUGCUCAGGGCUCCU | 17 | 893 |
| MYOC-hotspot200down-55 | + | CCAUUGCCUGUACAGCU | 17 | 905 |
| MYOC-hotspot200down-56 | - | CCUGGGGGGAGCAGGCUGAA | 20 | 781 |
| MYOC-hotspot200down-57 | - | AAGGGAGAGCCAGCCAGCCA | 20 | 783 |
| MYOC-hotspot200down-58 | - | AUGGCAGAAGGAGAUGCUCA | 20 | 773 |
| MYOC-hotspot200down-59 | - | UCCUGGGGGGAGCAGGCUGA | 20 | 780 |
| MYOC-hotspot200down-60 | - | UCAGGGCUCCUGGGGGGAGC | 20 | 779 |
| MYOC-hotspot200down-61 | + | CUGCCUGGGCCCUGGCUGGC | 20 | 1804 |
| MYOC-hotspot200down-62 | - | AAUGGCAGAAGGAGAUGCUC | 20 | 772 |
| MYOC-hotspot200down-63 | - | AGAUGCUCAGGGCUCCUGGG | 20 | 778 |
| MYOC-hotspot200down-64 | - | UAUUCAGGAAUUGUAGUCUG | 20 | 828 |

**Table 16A** provides exemplary targeting domains for the mutational hotspot 477-502 target site in the MYOC gene selected according to the first tier parameters. The targeting domains bind within 200 bp downstream from the mutational hotspot 477-502 target site, have a high level of orthogonality, start with a 5'G, and PAM is NNGRRT. It is contemplated herein that in an embodiment the targeting domain hybridizes to the target domain through complementary base pairing. Any of the targeting domains in the Table can be used with a *S. aureus* Cas9 molecule that generates a double stranded break (Cas9 nuclease) or a single-stranded break (Cas9 nickase).

**Table 16A**

| 1st Tier | | | | |
|---|---|---|---|---|
| gRNA Name | DNA Stran d | Targeting Domain | Target Site Length | SEQ ID NO |
| MYOC-hotspot200down-65 | + | GUCUACGCCCUCAGACUACAAUU C | 24 | 3551 |
| MYOC-hotspot200down-66 | + | GAUGGUGACCAUGUUCAUCCUU | 22 | 3570 |

**Table 16B** provides exemplary targeting domains for the mutational hotspot 477-502 target site in the MYOC gene selected according to the second tier parameters. The targeting domains bind within 200 bp downstream from the mutational hotspot 477-502 target site, have a high level of orthogonality and PAM is NNGRRT. It is contemplated herein that in an embodiment the targeting domain hybridizes to the target domain through complementary base pairing. Any of the targeting domains in the Table can be used with a *S. aureus* Cas9 molecule that generates a double stranded break (Cas9 nuclease) or a single-stranded break (Cas9 nickase).

**Table 16B**

| 2nd Tier | | | | |
|---|---|---|---|---|
| gRNA Name | DNA Strand | Targeting Domain | Target Site Length | SEQ ID NO |
| MYOC-hotspot200down-67 | + | UACGCCCUCAGACUACAAUUC | 21 | 3654 |
| MYOC-hotspot200down-68 | + | CUACGCCCUCAGACUACAAUUC | 22 | 3655 |
| MYOC-hotspot200down-69 | + | UCUACGCCCUCAGACUACAAUUC | 23 | 3656 |
| MYOC-hotspot200down-70 | + | AUGGUGACCAUGUUCAUCCUU | 21 | 3692 |
| MYOC-hotspot200down-71 | + | AGAUGGUGACCAUGUUCAUCCUU | 23 | 3693 |
| MYOC-hotspot200down-72 | + | UAGAUGGUGACCAUGUUCAUCCUU | 24 | 3694 |
| MYOC-hotspot200down-73 | - | AUGGUCACCAUCUAACUAUUC | 21 | 3740 |
| MYOC-hotspot200down-74 | - | CAUGGUCACCAUCUAACUAUUC | 22 | 3741 |
| MYOC-hotspot200down-75 | - | ACAUGGUCACCAUCUAACUAUUC | 23 | 3742 |
| MYOC-hotspot200down-76 | - | AACAUGGUCACCAUCUAACUAUUC | 24 | 3743 |

**Table 16C** provides exemplary targeting domains for the mutational hotspot 477-502 target site in the MYOC gene selected according to the third tier parameters. The targeting domains bind within 200 bp downstream from the mutational hotspot 477-502 target site, start with a 5' G and PAM is NNGRRT. It is contemplated herein that in an embodiment the targeting domain hybridizes to the target domain through complementary base pairing. Any of the targeting domains in the Table can be used with a *S. aureus* Cas9 molecule that generates a double stranded break (Cas9 nuclease) or a single-stranded break (Cas9 nickase).

**Table 16C**

| 3rd Tier | | | | |
|---|---|---|---|---|
| gRNA Name | DNA Strand | Targeting Domain | Target Site Length | SEQ ID |
| MYOC-hotspot200down-77 | + | GCCCUCAGACUACAAUUC | 18 | 3550 |
| MYOC-hotspot200down-78 | + | GUGACCAUGUUCAUCCUU | 18 | 3568 |
| MYOC-hotspot200down-79 | + | GGUGACCAUGUUCAUCCUU | 19 | 3569 |
| MYOC-hotspot200down-80 | - | GUCACCAUCUAACUAUUC | 18 | 3586 |
| MYOC-hotspot200down-81 | - | GGUCACCAUCUAACUAUUC | 19 | 3587 |

**Table 16D** provides exemplary targeting domains for the mutational hotspot 477-502 target site in the MYOC gene selected according to the fourth tier parameters. The targeting domains bind within 200 bp downstream from the mutational hotspot 477-502 target site, and PAM is NNGRRT. It is contemplated herein that in an embodiment the targeting domain hybridizes to the target domain through complementary base pairing. Any of the targeting domains in the Table can be used with a *S. aureus* Cas9 molecule that generates a double stranded break (Cas9 nuclease) or a single-stranded break (Cas9 nickase).

**Table 16D**

| 4th Tier | | | | |
|---|---|---|---|---|
| gRNA Name | DNA Strand | Targeting Domain | Target Site Length | SEQ ID NO |
| MYOC-hotspot200down-82 | + | CGCCCUCAGACUACAAUUC | 19 | 3653 |
| MYOC-hotspot200down-83 | + | ACGCCCUCAGACUACAAUUC | 20 | 2816 |
| MYOC-hotspot200down-84 | + | UGGUGACCAUGUUCAUCCUU | 20 | 2815 |
| MYOC-hotspot200down-18 | - | UGGUCACCAUCUAACUAUUC | 20 | 827 |
| MYOC-hotspot200down-85 | - | AAGUUUUCAUUAAUCCAG | 18 | 3761 |
| MYOC-hotspot200down-86 | - | CAAGUUUUCAUUAAUCCAG | 19 | 3762 |
| MYOC-hotspot200down-87 | - | CCAAGUUUUCAUUAAUCCAG | 20 | 2804 |
| MYOC-hotspot200down-88 | - | UCCAAGUUUUCAUUAAUCCAG | 21 | 3763 |
| MYOC-hotspot200down-89 | - | UUCCAAGUUUUCAUUAAUCCAG | 22 | 3764 |
| MYOC-hotspot200down-90 | - | UUUCCAAGUUUUCAUUAAUCCAG | 23 | 3765 |
| MYOC-hotspot200down-91 | - | CUUUCCAAGUUUUCAUUAAUCCAG | 24 | 3766 |

**Table 16E** provides exemplary targeting domains for the mutational hotspot 477-502 target site in the MYOC gene selected according to the fifth tier parameters. The targeting domains bind within 200 bp downstream from the mutational hotspot 477-502 target site, and PAM is NNGRRV. It is contemplated herein that in an embodiment the targeting domain hybridizes to the target domain through complementary base pairing. Any of the targeting domains in the Table can be used with a *S. aureus* Cas9 molecule that generates a double stranded break (Cas9 nuclease) or a single-stranded break (Cas9 nickase).

**Table 16E**

| 5th Tier | | | | |
|---|---|---|---|---|
| gRNA Name | DNA Strand | Targeting Domain | Target Site Length | SEQ ID NO |
| MYOC-hotspot200down-92 | + | GUUCAUCCUUCUGGAUUA | 18 | 3918 |
| MYOC-hotspot200down-93 | + | UGUUCAUCCUUCUGGAUUA | 19 | 3919 |
| MYOC-hotspot200down-94 | + | AUGUUCAUCCUUCUGGAUUA | 20 | 2814 |
| MYOC-hotspot200down-95 | + | CAUGUUCAUCCUUCUGGAUUA | 21 | 3920 |
| MYOC-hotspot200down-96 | + | CCAUGUUCAUCCUUCUGGAUUA | 22 | 3921 |
| MYOC-hotspot200down-97 | + | ACCAUGUUCAUCCUUCUGGAUUA | 23 | 3922 |
| MYOC-hotspot200down-98 | + | GACCAUGUUCAUCCUUCUGGAUUA | 24 | 3923 |
| MYOC-hotspot200down-99 | + | UUCUGGAUUAAUGAAAAC | 18 | 3931 |
| MYOC-hotspot200down-100 | + | CUUCUGGAUUAAUGAAAAC | 19 | 3932 |
| MYOC-hotspot200down-101 | + | CCUUCUGGAUUAAUGAAAAC | 20 | 2813 |
| MYOC-hotspot200down-102 | + | UCCUUCUGGAUUAAUGAAAAC | 21 | 3933 |
| MYOC-hotspot200down-103 | + | AUCCUUCUGGAUUAAUGAAAAC | 22 | 3934 |
| MYOC-hotspot200down-104 | + | CAUCCUUCUGGAUUAAUGAAAAC | 23 | 3935 |
| MYOC-hotspot200down-105 | + | UCAUCCUUCUGGAUUAAUGAAAAC | 24 | 3936 |
| MYOC-hotspot200down-106 | + | CUUCAGCCUGCUCCCCCC | 18 | 3956 |
| MYOC-hotspot200down-107 | + | CCUUCAGCCUGCUCCCCCC | 19 | 3957 |
| MYOC-hotspot200down-14 | + | CCCUUCAGCCUGCUCCCCCC | 20 | 785 |
| MYOC-hotspot200down-108 | + | UCCCUUCAGCCUGCUCCCCCC | 21 | 3958 |
| MYOC-hotspot200down-109 | + | CUCCCUUCAGCCUGCUCCCCCC | 22 | 3959 |
| MYOC-hotspot200down-110 | + | UCUCCCUUCAGCCUGCUCCCCCC | 23 | 3960 |
| MYOC-hotspot200down-111 | + | CUCUCCCUUCAGCCUGCUCCCCCC | 24 | 3961 |
| MYOC-hotspot200down-112 | + | CCUUCAGCCUGCUCCCCC | 18 | 3962 |
| MYOC-hotspot200down-113 | + | CCCUUCAGCCUGCUCCCCC | 19 | 3963 |
| MYOC-hotspot200down-114 | + | UCCCUUCAGCCUGCUCCCCC | 20 | 2811 |
| MYOC-hotspot200down-115 | + | CUCCCUUCAGCCUGCUCCCCC | 21 | 3964 |
| MYOC-hotspot200down-116 | + | UCUCCCUUCAGCCUGCUCCCCC | 22 | 3965 |
| MYOC-hotspot200down-117 | + | CUCUCCCUUCAGCCUGCUCCCCC | 23 | 3966 |
| MYOC-hotspot200down-118 | + | GCUCUCCCUUCAGCCUGCUCCCCC | 24 | 3967 |
| MYOC-hotspot200down-119 | + | CUGCUCCCCCCAGGAGCC | 18 | 3974 |
| MYOC-hotspot200down-120 | + | CCUGCUCCCCCCAGGAGCC | 19 | 3975 |
| MYOC-hotspot200down-121 | + | GCCUGCUCCCCCCAGGAGCC | 20 | 2810 |
| MYOC-hotspot200down-122 | + | AGCCUGCUCCCCCCAGGAGCC | 21 | 3976 |
| MYOC-hotspot200down-123 | + | CAGCCUGCUCCCCCCAGGAGCC | 22 | 3977 |
| MYOC-hotspot200down-124 | + | UCAGCCUGCUCCCCCCAGGAGCC | 23 | 3978 |
| MYOC-hotspot200down-125 | + | UUCAGCCUGCUCCCCCCAGGAGCC | 24 | 3979 |
| MYOC-hotspot200down-126 | + | UGCCAUUGCCUGUACAGC | 18 | 4011 |
| MYOC-hotspot200down-127 | + | CUGCCAUUGCCUGUACAGC | 19 | 4012 |
| MYOC-hotspot200down-128 | + | UCUGCCAUUGCCUGUACAGC | 20 | 2809 |
| MYOC-hotspot200down-129 | + | UUCUGCCAUUGCCUGUACAGC | 21 | 4013 |
| MYOC-hotspot200down-130 | + | CUUCUGCCAUUGCCUGUACAGC | 22 | 4014 |
| MYOC-hotspot200down-131 | + | CCUUCUGCCAUUGCCUGUACAGC | 23 | 4015 |
| MYOC-hotspot200down-132 | + | UCCUUCUGCCAUUGCCUGUACAGC | 24 | 4016 |
| MYOC-hotspot200down-133 | + | GAAAGCAGUCAAAGCUGC | 18 | 4052 |
| MYOC-hotspot200down-134 | + | GGAAAGCAGUCAAAGCUGC | 19 | 4053 |
| MYOC-hotspot200down-135 | + | UGGAAAGCAGUCAAAGCUGC | 20 | 2812 |
| MYOC-hotspot200down-136 | + | UUGGAAAGCAGUCAAAGCUGC | 21 | 4054 |
| MYOC-hotspot200down-137 | + | CUUGGAAAGCAGUCAAAGCUGC | 22 | 4055 |
| MYOC-hotspot200down-138 | + | ACUUGGAAAGCAGUCAAAGCUGC | 23 | 4056 |
| MYOC-hotspot200down-139 | + | AACUUGGAAAGCAGUCAAAGCUGC | 24 | 4057 |
| MYOC-hotspot200down-140 | + | UCUGGAUUAAUGAAAACU | 18 | 4252 |
| MYOC-hotspot200down-141 | + | UUCUGGAUUAAUGAAAACU | 19 | 4253 |
| MYOC-hotspot200down-20 | + | CUUCUGGAUUAAUGAAAACU | 20 | 853 |
| MYOC-hotspot200down-142 | + | CCUUCUGGAUUAAUGAAAACU | 21 | 4254 |
| MYOC-hotspot200down-143 | + | UCCUUCUGGAUUAAUGAAAACU | 22 | 4255 |
| MYOC-hotspot200down-144 | + | AUCCUUCUGGAUUAAUGAAAACU | 23 | 4256 |
| MYOC-hotspot200down-145 | + | CAUCCUUCUGGAUUAAUGAAAACU | 24 | 4257 |
| MYOC-hotspot200down-146 | + | GCCAUUGCCUGUACAGCU | 18 | 4265 |
| MYOC-hotspot200down-147 | + | UGCCAUUGCCUGUACAGCU | 19 | 4266 |
| MYOC-hotspot200down-22 | + | CUGCCAUUGCCUGUACAGCU | 20 | 786 |
| MYOC-hotspot200down-148 | + | UCUGCCAUUGCCUGUACAGCU | 21 | 4267 |
| MYOC-hotspot200down-149 | + | UUCUGCCAUUGCCUGUACAGCU | 22 | 4268 |
| MYOC-hotspot200down-150 | + | CUUCUGCCAUUGCCUGUACAGCU | 23 | 4269 |
| MYOC-hotspot200down-151 | + | CCUUCUGCCAUUGCCUGUACAGCU | 24 | 4270 |
| MYOC-hotspot200down-152 | - | UGGGGGGAGCAGGCUGAA | 18 | 4370 |
| MYOC-hotspot200down-153 | - | CUGGGGGGAGCAGGCUGAA | 19 | 4371 |
| MYOC-hotspot200down-56 | - | CCUGGGGGGAGCAGGCUGAA | 20 | 781 |
| MYOC-hotspot200down-154 | - | UCCUGGGGGGAGCAGGCUGAA | 21 | 4372 |
| MYOC-hotspot200down-155 | - | CUCCUGGGGGGAGCAGGCUGAA | 22 | 4373 |
| MYOC-hotspot200down-156 | - | GCUCCUGGGGGGAGCAGGCUGAA | 23 | 4374 |
| MYOC-hotspot200down-157 | - | GGCUCCUGGGGGGAGCAGGCUGAA | 24 | 4375 |
| MYOC-hotspot200down-158 | - | UGUACAGGCAAUGGCAGA | 18 | 4408 |
| MYOC-hotspot200down-159 | - | CUGUACAGGCAAUGGCAGA | 19 | 4409 |
| MYOC-hotspot200down-1 | - | GCUGUACAGGCAAUGGCAGA | 20 | 771 |
| MYOC-hotspot200down-160 | - | AGCUGUACAGGCAAUGGCAGA | 21 | 4410 |
| MYOC-hotspot200down-161 | - | AAGCUGUACAGGCAAUGGCAGA | 22 | 4411 |
| MYOC-hotspot200down-162 | - | CAAGCUGUACAGGCAAUGGCAGA | 23 | 4412 |
| MYOC-hotspot200down-163 | - | CCAAGCUGUACAGGCAAUGGCAGA | 24 | 4413 |
| MYOC-hotspot200down-164 | - | CUGGGGGGAGCAGGCUGA | 18 | 4453 |
| MYOC-hotspot200down-165 | - | CCUGGGGGGAGCAGGCUGA | 19 | 4454 |
| MYOC-hotspot200down-59 | - | UCCUGGGGGGAGCAGGCUGA | 20 | 780 |
| MYOC-hotspot200down-166 | - | CUCCUGGGGGGAGCAGGCUGA | 21 | 4455 |
| MYOC-hotspot200down-167 | - | GCUCCUGGGGGGAGCAGGCUGA | 22 | 4456 |
| MYOC-hotspot200down-168 | - | GGCUCCUGGGGGGAGCAGGCUGA | 23 | 4457 |
| MYOC-hotspot200down-169 | - | GGGCUCCUGGGGGGAGCAGGCUGA | 24 | 4458 |
| MYOC-hotspot200down-170 | - | GGAGAUGCUCAGGGCUCC | 18 | 4599 |
| MYOC-hotspot200down-171 | - | AGGAGAUGCUCAGGGCUCC | 19 | 4600 |
| MYOC-hotspot200down-16 | - | AAGGAGAUGCUCAGGGCUCC | 20 | 774 |
| MYOC-hotspot200down-172 | - | GAAGGAGAUGCUCAGGGCUCC | 21 | 4601 |
| MYOC-hotspot200down-173 | - | AGAAGGAGAUGCUCAGGGCUCC | 22 | 4602 |
| MYOC-hotspot200down-174 | - | CAGAAGGAGAUGCUCAGGGCUCC | 23 | 4603 |
| MYOC-hotspot200down-175 | - | GCAGAAGGAGAUGCUCAGGGCUCC | 24 | 4604 |
| MYOC-hotspot200down-176 | - | AAGGGAGAGCCAGCCAGC | 18 | 4618 |
| MYOC-hotspot200down-177 | - | GAAGGGAGAGCCAGCCAGC | 19 | 4619 |
| MYOC-hotspot200down-178 | - | UGAAGGGAGAGCCAGCCAGC | 20 | 2802 |
| MYOC-hotspot200down-179 | - | CUGAAGGGAGAGCCAGCCAGC | 21 | 4620 |
| MYOC-hotspot200down-180 | - | GCUGAAGGGAGAGCCAGCCAGC | 22 | 4621 |
| MYOC-hotspot200down-181 | - | GGCUGAAGGGAGAGCCAGCCAGC | 23 | 4622 |
| MYOC-hotspot200down-182 | - | AGGCUGAAGGGAGAGCCAGCCAGC | 24 | 4623 |
| MYOC-hotspot200down-183 | - | UCCAAGUUUUCAUUAAUC | 18 | 4642 |
| MYOC-hotspot200down-184 | - | UUCCAAGUUUUCAUUAAUC | 19 | 4643 |
| MYOC-hotspot200down-185 | - | UUUCCAAGUUUUCAUUAAUC | 20 | 2803 |
| MYOC-hotspot200down-186 | - | CUUUCCAAGUUUUCAUUAAUC | 21 | 4644 |
| MYOC-hotspot200down-187 | - | GCUUUCCAAGUUUUCAUUAAUC | 22 | 4645 |
| MYOC-hotspot200down-188 | - | UGCUUUCCAAGUUUUCAUUAAUC | 23 | 4646 |
| MYOC-hotspot200down-189 | - | CUGCUUUCCAAGUUUUCAUUAAUC | 24 | 4647 |
| MYOC-hotspot200down-190 | - | AGGAGAUGCUCAGGGCUC | 18 | 4660 |
| MYOC-hotspot200down-191 | - | AAGGAGAUGCUCAGGGCUC | 19 | 4661 |
| MYOC-hotspot200down-192 | - | GAAGGAGAUGCUCAGGGCUC | 20 | 2798 |
| MYOC-hotspot200down-193 | - | AGAAGGAGAUGCUCAGGGCUC | 21 | 4662 |
| MYOC-hotspot200down-194 | - | CAGAAGGAGAUGCUCAGGGCUC | 22 | 4663 |
| MYOC-hotspot200down-195 | - | GCAGAAGGAGAUGCUCAGGGCUC | 23 | 4664 |
| MYOC-hotspot200down-196 | - | GGCAGAAGGAGAUGCUCAGGGCUC | 24 | 4665 |
| MYOC-hotspot200down-197 | - | CUGUACAGGCAAUGGCAG | 18 | 4720 |
| MYOC-hotspot200down-198 | - | GCUGUACAGGCAAUGGCAG | 19 | 4721 |
| MYOC-hotspot200down-199 | - | AGCUGUACAGGCAAUGGCAG | 20 | 2796 |
| MYOC-hotspot200down-200 | - | AAGCUG UACAGGCAAUGGCAG | 21 | 4722 |
| MYOC-hotspot200down-201 | - | CAAGCUGUACAGGCAAUGGCAG | 22 | 4723 |
| MYOC-hotspot200down-202 | - | CCAAGCUGUACAGGCAAUGGCAG | 23 | 4724 |
| MYOC-hotspot200down-203 | - | UCCAAGCUGUACAGGCAAUGGCAG | 24 | 4725 |
| MYOC-hotspot200down-204 | - | UUUCAUUAAUCCAGAAGG | 18 | 4800 |
| MYOC-hotspot200down-205 | - | UUUUCAUUAAUCCAGAAGG | 19 | 4801 |
| MYOC-hotspot200down-206 | - | GUUUUCAUUAAUCCAGAAGG | 20 | 2805 |
| MYOC-hotspot200down-207 | - | AGUUUUCAUUAAUCCAGAAGG | 21 | 4802 |
| MYOC-hotspot200down-208 | - | AAGUUUUCAUUAAUCCAGAAGG | 22 | 4803 |
| MYOC-hotspot200down-209 | - | CAAGUUUUCAUUAAUCCAGAAGG | 23 | 4804 |
| MYOC-hotspot200down-210 | - | CCAAGUUUUCAUUAAUCCAGAAGG | 24 | 4805 |
| MYOC-hotspot200down-211 | - | GGGGGAGCAGGCUGAAGG | 18 | 4806 |
| MYOC-hotspot200down-212 | - | GGGGGGAGCAGGCUGAAGG | 19 | 4807 |
| MYOC-hotspot200down-213 | - | UGGGGGGAGCAGGCUGAAGG | 20 | 2801 |
| MYOC-hotspot200down-214 | - | CUGGGGGGAGCAGGCUGAAGG | 21 | 4808 |
| MYOC-hotspot200down-215 | - | CCUGGGGGGAGCAGGCUGAAGG | 22 | 4809 |
| MYOC-hotspot200down-216 | - | UCCUGGGGGGAGCAGGCUGAAGG | 23 | 4810 |
| MYOC-hotspot200down-217 | - | CUCCUGGGGGGAGCAGGCUGAAGG | 24 | 4811 |
| MYOC-hotspot200down-218 | - | GCUCCUGGGGGGAGCAGG | 18 | 4818 |
| MYOC-hotspot200down-219 | - | GGCUCCUGGGGGGAGCAGG | 19 | 4819 |
| MYOC-hotspot200down-220 | - | GGGCUCCUGGGGGGAGCAGG | 20 | 2799 |
| MYOC-hotspot200down-221 | - | AGGGCUCCUGGGGGGAGCAGG | 21 | 4820 |
| MYOC-hotspot200down-222 | - | CAGGGCUCCUGGGGGGAGCAGG | 22 | 4821 |
| MYOC-hotspot200down-223 | - | UCAGGGCUCCUGGGGGGAGCAGG | 23 | 4822 |
| MYOC-hotspot200down-224 | - | CUCAGGGCUCCUGGGGGGAGCAGG | 24 | 4823 |
| MYOC-hotspot200down-225 | - | AUGCUCAGGGCUCCUGGG | 18 | 4824 |
| MYOC-hotspot200down-226 | - | GAUGCUCAGGGCUCCUGGG | 19 | 4825 |
| MYOC-hotspot200down-63 | - | AGAUGCUCAGGGCUCCUGGG | 20 | 778 |
| MYOC-hotspot200down-227 | - | GAGAUGCUCAGGGCUCCUGGG | 21 | 4826 |
| MYOC-hotspot200down-228 | - | GGAGAUGCUCAGGGCUCCUGGG | 22 | 4827 |
| MYOC-hotspot200down-229 | - | AGGAGAUGCUCAGGGCUCCUGGG | 23 | 4828 |
| MYOC-hotspot200down-230 | - | AAGGAGAUGCUCAGGGCUCCUGGG | 24 | 4829 |
| MYOC-hotspot200down-231 | - | AAGCUGUACAGGCAAUGG | 18 | 4837 |
| MYOC-hotspot200down-232 | - | CAAGCUGUACAGGCAAUGG | 19 | 4838 |
| MYOC-hotspot200down-233 | - | CCAAGCUGUACAGGCAAUGG | 20 | 2795 |
| MYOC-hotspot200down-234 | - | UCCAAGCUGUACAGGCAAUGG | 21 | 4839 |
| MYOC-hotspot200down-235 | - | CUCCAAGCUGUACAGGCAAUGG | 22 | 4840 |
| MYOC-hotspot200down-236 | - | CCUCCAAGCUGUACAGGCAAUGG | 23 | 4841 |
| MYOC-hotspot200down-237 | - | GCCUCCAAGCUGUACAGGCAAUGG | 24 | 4842 |
| MYOC-hotspot200down-238 | - | GAUGCUCAGGGCUCCUGG | 18 | 4843 |
| MYOC-hotspot200down-239 | - | AGAUGCUCAGGGCUCCUGG | 19 | 4844 |
| MYOC-hotspot200down-38 | - | GAGAUGCUCAGGGCUCCUGG | 20 | 777 |
| MYOC-hotspot200down-240 | - | GGAGAUGCUCAGGGCUCCUGG | 21 | 4845 |
| MYOC-hotspot200down-241 | - | AGGAGAUGCUCAGGGCUCCUGG | 22 | 4846 |
| MYOC-hotspot200down-242 | - | AAGGAGAUGCUCAGGGCUCCUGG | 23 | 4847 |
| MYOC-hotspot200down-243 | - | GAAGGAGAUGCUCAGGGCUCCUGG | 24 | 4848 |
| MYOC-hotspot200down-244 | - | AGAUGCUCAGGGCUCCUG | 18 | 4880 |
| MYOC-hotspot200down-245 | - | GAGAUGCUCAGGGCUCCUG | 19 | 4881 |
| MYOC-hotspot200down-39 | - | GGAGAUGCUCAGGGCUCCUG | 20 | 776 |
| MYOC-hotspot200down-246 | - | AGGAGAUGCUCAGGGCUCCUG | 21 | 4882 |
| MYOC-hotspot200down-247 | - | AAGGAGAUGCUCAGGGCUCCUG | 22 | 4883 |
| MYOC-hotspot200down-248 | - | GAAGGAGAUGCUCAGGGCUCCUG | 23 | 4884 |
| MYOC-hotspot200down-249 | - | AGAAGGAGAUGCUCAGGGCUCCUG | 24 | 4885 |
| MYOC-hotspot200down-250 | - | CCUGGGGGGAGCAGGCUG | 18 | 4886 |
| MYOC-hotspot200down-251 | - | UCCUGGGGGGAGCAGGCUG | 19 | 4887 |
| MYOC-hotspot200down-252 | - | CUCCUGGGGGGAGCAGGCUG | 20 | 2800 |
| MYOC-hotspot200down-253 | - | GCUCCUGGGGGGAGCAGGCUG | 21 | 4888 |
| MYOC-hotspot200down-254 | - | GGCUCCUGGGGGGAGCAGGCUG | 22 | 4889 |
| MYOC-hotspot200down-255 | - | GGGCUCCUGGGGGGAGCAGGCUG | 23 | 4890 |
| MYOC-hotspot200down-256 | - | AGGGCUCCUGGGGGGAGCAGGCUG | 24 | 4891 |
| MYOC-hotspot200down-257 | - | GAGAUGCUCAGGGCUCCU | 18 | 4984 |
| MYOC-hotspot200down-258 | - | GGAGAUGCUCAGGGCUCCU | 19 | 4985 |
| MYOC-hotspot200down-21 | - | AGGAGAUGCUCAGGGCUCCU | 20 | 775 |
| MYOC-hotspot200down-259 | - | AAGGAGAUGCUCAGGGCUCCU | 21 | 4986 |
| MYOC-hotspot200down-260 | - | GAAGGAGAUGCUCAGGGCUCCU | 22 | 4987 |
| MYOC-hotspot200down-261 | - | AGAAGGAGAUGCUCAGGGCUCCU | 23 | 4988 |
| MYOC-hotspot200down-262 | - | CAGAAGGAGAUGCUCAGGGCUCCU | 24 | 4989 |
| MYOC-hotspot200down-263 | - | AUGGCAGAAGGAGAUGCU | 18 | 5009 |
| MYOC-hotspot200down-264 | - | AAUGGCAGAAGGAGAUGCU | 19 | 5010 |
| MYOC-hotspot200down-265 | - | CAAUGGCAGAAGGAGAUGCU | 20 | 2797 |
| MYOC-hotspot200down-266 | - | GCAAUGGCAGAAGGAGAUGCU | 21 | 5011 |
| MYOC-hotspot200down-267 | - | GGCAAUGGCAGAAGGAGAUGCU | 22 | 5012 |
| MYOC-hotspot200down-268 | - | AGGCAAUGGCAGAAGGAGAUGCU | 23 | 5013 |
| MYOC-hotspot200down-269 | - | CAGGCAAUGGCAGAAGGAGAUGCU | 24 | 5014 |
| MYOC-hotspot200down-270 | - | AUUCAGGAAUUGUAGUCU | 18 | 5022 |
| MYOC-hotspot200down-271 | - | UAUUCAGGAAUUGUAGUCU | 19 | 5023 |
| MYOC-hotspot200down-272 | - | CUAUUCAGGAAUUGUAGUCU | 20 | 2808 |
| MYOC-hotspot200down-273 | - | ACUAUUCAGGAAUUGUAGUCU | 21 | 5024 |
| MYOC-hotspot200down-274 | - | AACUAUUCAGGAAUUGUAGUCU | 22 | 5025 |
| MYOC-hotspot200down-275 | - | UAACUAUUCAGGAAUUGUAGUCU | 23 | 5026 |
| MYOC-hotspot200down-276 | - | CUAACUAUUCAGGAAUUGUAGUCU | 24 | 5027 |
| MYOC-hotspot200down-277 | - | CUAUUCAGGAAUUGUAGU | 18 | 5041 |
| MYOC-hotspot200down-278 | - | ACUAUUCAGGAAUUGUAGU | 19 | 5042 |
| MYOC-hotspot200down-279 | - | AACUAUUCAGGAAUUGUAGU | 20 | 2807 |
| MYOC-hotspot200down-280 | - | UAACUAUUCAGGAAUUGUAGU | 21 | 5043 |
| MYOC-hotspot200down-281 | - | CUAACUAUUCAGGAAUUGUAGU | 22 | 5044 |
| MYOC-hotspot200down-282 | - | UCUAACUAUUCAGGAAUUGUAGU | 23 | 5045 |
| MYOC-hotspot200down-283 | - | AUCUAACUAUUCAGGAAUUGUAGU | 24 | 5046 |
| MYOC-hotspot200down-284 | - | GGUCACCAUCUAACUAUU | 18 | 5080 |
| MYOC-hotspot200down-285 | - | UGGUCACCAUCUAACUAUU | 19 | 5081 |
| MYOC-hotspot200down-286 | - | AUGGUCACCAUCUAACUAUU | 20 | 2806 |
| MYOC-hotspot200down-287 | - | CAUGGUCACCAUCUAACUAUU | 21 | 5082 |
| MYOC-hotspot200down-288 | - | ACAUGGUCACCAUCUAACUAUU | 22 | 5083 |
| MYOC-hotspot200down-289 | - | AACAUGGUCACCAUCUAACUAUU | 23 | 5084 |
| MYOC-hotspot200down-290 | - | GAACAUGGUCACCAUCUAACUAUU | 24 | 5085 |

**Table 17A** provides exemplary targeting domains for the mutational hotspot 477-502 target site in the MYOC gene selected according to the third tier parameters. The targeting domains bind within 200 bp downstream from the mutational hotspot 477-502 target site and start with a 5'G. It is contemplated herein that in an embodiment the targeting domain hybridizes to the target domain through complementary base pairing. Any of the targeting domains in the Table can be used with a *N*. *meningitidis* Cas9 molecule that generates a double stranded break (Cas9 nuclease) or a single-stranded break (Cas9 nickase).

**Table 17A**

| 3rd Tier | | | | |
|---|---|---|---|---|
| gRNA Name | DNA Strand | Targeting Domain | Target Site Length | SEQ ID NO |
| MYOC-hotspot200down-291 | + | GUGACCAUGUUCAUCCU | 17 | 2855 |
| MYOC-hotspot200down-292 | - | GCCAGGGCCCAGGCAGCUUU | 20 | 5144 |

**Table 17B** provides exemplary targeting domains for the mutational hotspot 477-502 target site in the MYOC gene selected according to the fourth tier parameters. The targeting domains bind within 200 bp downstream from the mutational hotspot 477-502 target site. It is contemplated herein that in an embodiment the targeting domain hybridizes to the target domain through complementary base pairing. Any of the targeting domains in the Table can be used with a *N*. *meningitidis* Cas9 molecule that generates a double stranded break (Cas9 nuclease) or a single-stranded break (Cas9 nickase).

**Table 17B**

| 4th Tier | | | | |
|---|---|---|---|---|
| gRNA Name | DNA Strand | Targeting Domain | Target Site Length | SEQ ID NO |
| MYOC-hotspot200down-293 | - | CAGCCAGCCAGGGCCCA | 17 | 5114 |
| MYOC-hotspot200down-294 | + | CCUUCUGCCAUUGCCUG | 17 | 5122 |
| MYOC-hotspot200down-295 | + | CAUUGCCUGUACAGCUU | 17 | 5127 |
| MYOC-hotspot200down-296 | - | AGGGCCCAGGCAGCUUU | 17 | 5128 |
| MYOC-hotspot200down-297 | - | AGCCAGCCAGCCAGGGCCCA | 20 | 5131 |
| MYOC-hotspot200down-298 | + | UCUCCUUCUGCCAUUGCCUG | 20 | 5138 |
| MYOC-hotspot200down-299 | + | AUGGUGACCAUGUUCAUCCU | 20 | 2849 |
| MYOC-hotspot200down-300 | + | UGCCAUUGCCUGUACAGCUU | 20 | 5143 |

**Table 18A** provides exemplary targeting domains for correcting a mutation (e.g., I477N) in the MYOC gene selected according to the first tier parameters. The targeting domains bind within 200 bp from a mutation (e.g., I477N), have a high level of orthogonality and start with a 5'G. It is contemplated herein that in an embodiment the targeting domain hybridizes to the target domain through complementary base pairing. Any of the targeting domains in the Table can be used with a *S. pyogenes* Cas9 molecule that generates a double stranded break (Cas9 nuclease) or a single-stranded break (Cas9 nickase).

**Table 18A**

| 1st Tier | | | | |
|---|---|---|---|---|
| gRNA Name | DNA Strand | Targeting Domain | Target Site Length | SEQ ID NO |
| MYOC-I477N-1 | + | GCUGCUGACGGUGUACA | 17 | 909 |
| MYOC-I477N-2 | + | GCGGUUCUUGAAUGGGA | 17 | 446 |
| MYOC-I477N-3 | - | GCUUAUGACACAGGCAC | 17 | 451 |
| MYOC-I477N-4 | - | GAUUGACUACAACCCCC | 17 | 886 |
| MYOC-I477N-5 | + | GACGGUAGCAUCUGCUG | 17 | 907 |
| MYOC-I477N-6 | - | GGAACUCGAACAAACCU | 17 | 884 |
| MYOC-I477N-7 | + | GGAGGCUUUUCACAUCU | 17 | 445 |
| MYOC-I477N-8 | + | GUAGCUGCUGACGGUGUACA | 20 | 790 |
| MYOC-I477N-9 | + | GGCAAAGAGCUUCUUCUCCA | 20 | 448 |
| MYOC-I477N-10 | - | GCUGUACAGGCAAUGGCAGA | 20 | 771 |
| MYOC-I477N-11 | - | GUCAACUUUGCUUAUGACAC | 20 | 439 |
| MYOC-I477N-12 | - | GAAAAGCCUCCAAGCUGUAC | 20 | 769 |
| MYOC-I477N-13 | + | GACCAUGUUCAAGUUGUCCC | 20 | 441 |
| MYOC-I477N-14 | + | GGUUCUUGAAUGGGAUGGUC | 20 | 449 |
| MYOC-I477N-15 | + | GCAAAGAGCUUCUUCUCCAG | 20 | 442 |
| MYOC-I477N-16 | + | GUUGACGGUAGCAUCUGCUG | 20 | 788 |
| MYOC-I477N-17 | - | GCCAAUGCCUUCAUCAUCUG | 20 | 768 |
| MYOC-I477N-18 | + | GCCACAGAUGAUGAAGGCAU | 20 | 792 |
| MYOC-I477N-19 | - | GGAGAAGAAGCUCUUUGCCU | 20 | 440 |

**Table 18B** provides exemplary targeting domains for correcting a mutation (e.g., I477N) in the MYOC gene selected according to the second tier parameters. The targeting domains bind within 200 bp from a mutation (e.g., I477N) and have a high level of orthogonality. It is contemplated herein that in an embodiment the targeting domain hybridizes to the target domain through complementary base pairing. Any of the targeting domains in the Table can be used with a *S. pyogenes* Cas9 molecule that generates a double stranded break (Cas9 nuclease) or a single-stranded break (Cas9 nickase).

**Table 18B**

| 2nd Tier | | | | |
|---|---|---|---|---|
| gRNA Name | DNA Strand | Targeting Domain | Target Site Length | SEQ ID NO |
| MYOC-I477N-20 | + | UUAUAGCGGUUCUUGAA | 17 | 473 |
| MYOC-I477N-21 | + | UGGCGACUGACUGCUUA | 17 | 912 |
| MYOC-I477N-22 | - | AACUUUGCUUAUGACAC | 17 | 464 |
| MYOC-I477N-23 | - | AAGCCUCCAAGCUGUAC | 17 | 887 |
| MYOC-I477N-24 | - | UGGAACUCGAACAAACC | 17 | 883 |
| MYOC-I477N-25 | + | ACGGAUGUUUGUCUCCC | 17 | 913 |
| MYOC-I477N-26 | + | UCUUGAAUGGGAUGGUC | 17 | 475 |
| MYOC-I477N-27 | + | UGCUGCUGUACUUAUAG | 17 | 472 |
| MYOC-I477N-28 | + | UUGCCUGUACAGCUUGG | 17 | 906 |
| MYOC-I477N-29 | + | UAUAGCGGUUCUUGAAU | 17 | 474 |
| MYOC-I477N-30 | - | CCUCCAAGCUGUACAGGCAA | 20 | 770 |
| MYOC-I477N-31 | + | UACUUAUAGCGGUUCUUGAA | 20 | 461 |
| MYOC-I477N-32 | - | UGCCUGGGACAACUUGAACA | 20 | 456 |
| MYOC-I477N-33 | + | AUAGCGGUUCUUGAAUGGGA | 20 | 443 |
| MYOC-I477N-34 | + | CAAGGUGCCACAGAUGAUGA | 20 | 791 |
| MYOC-I477N-35 | + | CAUUGGCGACUGACUGCUUA | 20 | 793 |
| MYOC-I477N-36 | - | UUUGCUUAUGACACAGGCAC | 20 | 453 |
| MYOC-I477N-37 | - | AUCUGGAACUCGAACAAACC | 20 | 766 |
| MYOC-I477N-38 | - | CAUGAUUGACUACAACCCCC | 20 | 454 |
| MYOC-I477N-39 | + | CCCUUCAGCCUGCUCCCCCC | 20 | 785 |
| MYOC-I477N-40 | + | AGUCAAAGCUGCCUGGGCCC | 20 | 1802 |
| MYOC-I477N-41 | + | CUUACGGAUGUUUGUCUCCC | 20 | 794 |
| MYOC-I477N-42 | - | AAGGAGAUGCUCAGGGCUCC | 20 | 774 |
| MYOC-I477N-43 | + | AAAGCUGCCUGGGCCCUGGC | 20 | 1803 |
| MYOC-I477N-44 | + | UCAUGCUGCUGUACUUAUAG | 20 | 460 |
| MYOC-I477N-45 | + | CCAUUGCCUGUACAGCUUGG | 20 | 787 |
| MYOC-I477N-46 | + | ACUUAUAGCGGUUCUUGAAU | 20 | 462 |
| MYOC-I477N-47 | - | UCUGGAACUCGAACAAACCU | 20 | 767 |
| MYOC-I477N-48 | - | AGGAGAUGCUCAGGGCUCCU | 20 | 775 |
| MYOC-I477N-49 | + | CUGCCAUUGCCUGUACAGCU | 20 | 786 |
| MYOC-I477N-50 | + | CUUGGAGGCUUUUCACAUCU | 20 | 457 |

**Table 18C** provides exemplary targeting domains for correcting a mutation (e.g., I477N) in the MYOC gene selected according to the third tier parameters. The targeting domains bind within 200 bp from a mutation (e.g., I477N) and start with a 5'G. It is contemplated herein that in an embodiment the targeting domain hybridizes to the target domain through complementary base pairing. Any of the targeting domains in the Table can be used with a *S. pyogenes* Cas9 molecule that generates a double stranded break (Cas9 nuclease) or a single-stranded break (Cas9 nickase).

**Table 18C**

| 3rd Tier | | | | |
|---|---|---|---|---|
| gRNA Name | Strand, | Targeting Domain | Target Site Length | SEQ ID NO |
| MYOC-I477N-51 | - | GGGGGGAGCAGGCUGAA | 17 | 899 |
| MYOC-I477N-52 | - | GGAGAGCCAGCCAGCCA | 17 | 901 |
| MYOC-I477N-53 | - | GCAGAAGGAGAUGCUCA | 17 | 891 |
| MYOC-I477N-54 | - | GUACAGGCAAUGGCAGA | 17 | 889 |
| MYOC-I477N-55 | + | GGUGCCACAGAUGAUGA | 17 | 910 |
| MYOC-I477N-56 | + | GUCAUAAGCAAAGUUGA | 17 | 447 |
| MYOC-I477N-57 | - | GGGAGAGCCAGCCAGCC | 17 | 900 |
| MYOC-I477N-58 | - | GAGAUGCUCAGGGCUCC | 17 | 892 |
| MYOC-I477N-59 | - | GGGCUCCUGGGGGGAGC | 17 | 897 |
| MYOC-I477N-60 | + | GCUGCCUGGGCCCUGGC | 17 | 1801 |
| MYOC-I477N-61 | - | GGCAGAAGGAGAUGCUC | 17 | 890 |
| MYOC-I477N-62 | - | GAUGCUCAGGGCUCCUG | 17 | 894 |
| MYOC-I477N-63 | - | GAAGAAGCUCUUUGCCU | 17 | 452 |
| MYOC-I477N-64 | + | GUUCUUGAAUGGGAUGGUCA | 20 | 450 |
| MYOC-I477N-65 | - | GAGCCAGCCAGCCAGGGCCC | 20 | 784 |
| MYOC-I477N-66 | - | GAAGGGAGAGCCAGCCAGCC | 20 | 782 |
| MYOC-I477N-67 | + | GGAAAGCAGUCAAAGCUGCC | 20 | 854 |
| MYOC-I477N-68 | - | GAGAUGCUCAGGGCUCCUGG | 20 | 777 |
| MYOC-I477N-69 | - | GGAGAUGCUCAGGGCUCCUG | 20 | 776 |
| MYOC-I477N-70 | + | GAAAGCAGUCAAAGCUGCCU | 20 | 855 |

**Table 18D** provides exemplary targeting domains for correcting a mutation (e.g., I477N) in the MYOC gene selected according to the fourth tier parameters. The targeting domains bind within 200 bp from a mutation (e.g., I477N). It is contemplated herein that in an embodiment the targeting domain hybridizes to the target domain through complementary base pairing. Any of the targeting domains in the Table can be used with a *S. pyogenes* Cas9 molecule that generates a double stranded break (Cas9 nuclease) or a single-stranded break (Cas9 nickase).

**Table 18D**

| 4th Tier | | | | |
|---|---|---|---|---|
| gRNA Name | DNA Strand | Targeting Domain | Target Site Length | SEQ ID NO |
| MYOC-I477N-71 | - | CCAAGCUGUACAGGCAA | 17 | 888 |
| MYOC-I477N-72 | - | CUGGGACAACUUGAACA | 17 | 466 |
| MYOC-I477N-73 | + | AAAGAGCUUCUUCUCCA | 17 | 469 |
| MYOC-I477N-74 | + | CUUGAAUGGGAUGGUCA | 17 | 476 |
| MYOC-I477N-75 | - | UGGGGGGAGCAGGCUGA | 17 | 898 |
| MYOC-I477N-76 | + | UGAGGUGUAGCUGCUGA | 17 | 908 |
| MYOC-I477N-77 | + | UUCAGCCUGCUCCCCCC | 17 | 904 |
| MYOC-I477N-78 | - | CCAGCCAGCCAGGGCCC | 17 | 902 |
| MYOC-I477N-79 | + | CAAAGCUGCCUGGGCCC | 17 | 1805 |
| MYOC-I477N-80 | + | CAUGUUCAAGUUGUCCC | 17 | 467 |
| MYOC-I477N-81 | + | AAGCAGUCAAAGCUGCC | 17 | 974 |
| MYOC-I477N-82 | - | AGAAGAAGCUCUUUGCC | 17 | 465 |
| MYOC-I477N-83 | + | CAAAGAGCUUCUUCUCC | 17 | 468 |
| MYOC-I477N-84 | + | CCUGGGCCCUGGCUGGC | 17 | 903 |
| MYOC-I477N-85 | + | AAGAGCUUCUUCUCCAG | 17 | 470 |
| MYOC-I477N-86 | + | AGAGCUUCUUCUCCAGG | 17 | 471 |
| MYOC-I477N-87 | - | UGCUCAGGGCUCCUGGG | 17 | 896 |
| MYOC-I477N-88 | - | AUGCUCAGGGCUCCUGG | 17 | 895 |
| MYOC-I477N-89 | - | AAUGCCUUCAUCAUCUG | 17 | 885 |
| MYOC-I477N-90 | + | ACAGAUGAUGAAGGCAU | 17 | 911 |
| MYOC-I477N-91 | + | AGCAGUCAAAGCUGCCU | 17 | 975 |
| MYOC-I477N-92 | - | AGAUGCUCAGGGCUCCU | 17 | 893 |
| MYOC-I477N-93 | + | CCAUUGCCUGUACAGCU | 17 | 905 |
| MYOC-I477N-94 | - | CCUGGGGGGAGCAGGCUGAA | 20 | 781 |
| MYOC-I477N-95 | - | AAGGGAGAGCCAGCCAGCCA | 20 | 783 |
| MYOC-I477N-96 | - | AUGGCAGAAGGAGAUGCUCA | 20 | 773 |
| MYOC-I477N-97 | - | UCCUGGGGGGAGCAGGCUGA | 20 | 780 |
| MYOC-I477N-98 | + | UGCUGAGGUGUAGCUGCUGA | 20 | 789 |
| MYOC-I477N-99 | + | UGUGUCAUAAGCAAAGUUGA | 20 | 463 |
| MYOC-I477N-100 | - | UGGAGAAGAAGCUCUUUGCC | 20 | 455 |
| MYOC-I477N-101 | + | AGGCAAAGAGCUUCUUCUCC | 20 | 458 |
| MYOC-I477N-102 | - | UCAGGGCUCCUGGGGGGAGC | 20 | 779 |
| MYOC-I477N-103 | + | CUGCCUGGGCCCUGGCUGGC | 20 | 1804 |
| MYOC-I477N-104 | - | AAUGGCAGAAGGAGAUGCUC | 20 | 772 |
| MYOC-I477N-105 | + | CAAAGAGCUUCUUCUCCAGG | 20 | 459 |
| MYOC-I477N-106 | - | AGAUGCUCAGGGCUCCUGGG | 20 | 778 |

**Table 19A** provides exemplary targeting domains for correcting a mutation (e.g., I477N) in the MYOC gene selected according to the first tier parameters. The targeting domains bind within 200 bp from a mutation (e.g., I477N), have a high level of orthogonality, start with a 5'G, and PAM is NNGRRT. It is contemplated herein that in an embodiment the targeting domain hybridizes to the target domain through complementary base pairing. Any of the targeting domains in the Table can be used with a *S. aureus* Cas9 molecule that generates a double stranded break (Cas9 nuclease) or a single-stranded break (Cas9 nickase).

**Table 19A**

| 1st Tier | | | | |
|---|---|---|---|---|
| gRNA Name | DNA Strand | Targeting Domain | Target Site Length | SEQ ID NO |
| MYOC-I477N-107 | + | GUACUUAUAGCGGUUCUUGAA | 21 | 3535 |
| MYOC-I477N-108 | + | GCUGUACUUAUAGCGGUUCUUGAA | 24 | 3536 |
| MYOC-I477N-109 | + | GCUGCUGUACUUAUAGCGGUUC | 22 | 3553 |
| MYOC-I477N-110 | + | GCGGUUCUUGAAUGGGAUGGU | 21 | 3564 |
| MYOC-I477N-111 | + | GAUGUUUGUCUCCCAGGUUUGU | 22 | 3566 |
| MYOC-I477N-112 | + | GGAUGUUUGUCUCCCAGGUUUGU | 23 | 3567 |

**Table 19B** provides exemplary targeting domains for correcting a mutation (e.g., I477N) in the MYOC gene selected according to the second tier parameters. The targeting domains bind within 200 bp from a mutation (e.g., I477N), have a high level of orthogonality and PAM is NNGRRT. It is contemplated herein that in an embodiment the targeting domain hybridizes to the target domain through complementary base pairing. Any of the targeting domains in the Table can be used with a *S. aureus* Cas9 molecule that generates a double stranded break (Cas9 nuclease) or a single-stranded break (Cas9 nickase).

**Table 19B**

| 2nd Tier | | | | |
|---|---|---|---|---|
| gRNA Name | DNA Strand | Targeting Domain | Target Site Length | SEQ ID NO |
| MYOC-I477N-113 | + | UGUACUUAUAGCGGUUCUUGAA | 22 | 3612 |
| MYOC-I477N-114 | + | CUGUACUUAUAGCGGUUCUUGAA | 23 | 3613 |
| MYOC-I477N-115 | + | AGGCAAAGAGCUUCUUCUCCA | 21 | 3615 |
| MYOC-I477N-116 | + | CAGGCAAAGAGCUUCUUCUCCA | 22 | 3616 |
| MYOC-I477N-117 | + | CCAGGCAAAGAGCUUCUUCUCCA | 23 | 3617 |
| MYOC-I477N-118 | + | CCCAGGCAAAGAGCUUCUUCUCCA | 24 | 3618 |
| MYOC-I477N-119 | + | CUGCUGUACUUAUAGCGGUUC | 21 | 3658 |
| MYOC-I477N-120 | + | UGCUGCUGUACUUAUAGCGGUUC | 23 | 3659 |
| MYOC-I477N-121 | + | AUGCUGCUGUACUUAUAGCGGUUC | 24 | 3660 |
| MYOC-I477N-122 | + | AGCGGUUCUUGAAUGGGAUGGU | 22 | 3680 |
| MYOC-I477N-123 | + | UAGCGGUUCUUGAAUGGGAUGGU | 23 | 3681 |
| MYOC-I477N-124 | + | AUAGCGGUUCUUGAAUGGGAUGGU | 24 | 3682 |
| MYOC-I477N-125 | + | AUGUUUGUCUCCCAGGUUUGU | 21 | 3690 |
| MYOC-I477N-126 | + | CGGAUGUUUGUCUCCCAGGUUUGU | 24 | 3691 |

**Table 19C** provides exemplary targeting domains for correcting a mutation (e.g., I477N) in the MYOC gene selected according to the third tier parameters. The targeting domains bind within 200 bp from a mutation (e.g., I477N), start with a 5' G and PAM is NNGRRT. It is contemplated herein that in an embodiment the targeting domain hybridizes to the target domain through complementary base pairing. Any of the targeting domains in the Table can be used with a *S. aureus* Cas9 molecule that generates a double stranded break (Cas9 nuclease) or a single-stranded break (Cas9 nickase).

**Table 19C**

| 3rd Tier | | | | |
|---|---|---|---|---|
| gRNA Name | DNA Strand | Targeting Domain | Target Site Length | SEQ ID NO |
| MYOC-I477N-127 | + | GCAAAGAGCUUCUUCUCCA | 19 | 3537 |
| MYOC-I477N-9 | + | GGCAAAGAGCUUCUUCUCCA | 20 | 448 |
| MYOC-I477N-128 | + | GCUGUACUUAUAGCGGUUC | 19 | 3552 |
| MYOC-I477N-129 | + | GUUCUUGAAUGGGAUGGU | 18 | 3562 |
| MYOC-I477N-130 | + | GGUUCUUGAAUGGGAUGGU | 19 | 3563 |
| MYOC-I477N-131 | + | GUUUGUCUCCCAGGUUUGU | 19 | 3565 |
| MYOC-I477N-132 | + | GCAUUGGCGACUGACUGCUU | 20 | 2793 |
| MYOC-I477N-133 | + | GGCAUUGGCGACUGACUGCUU | 21 | 3571 |
| MYOC-I477N-134 | + | GAAGGCAUUGGCGACUGACUGCUU | 24 | 3572 |

**Table 19D** provides exemplary targeting domains for correcting a mutation (e.g., I477N) in the MYOC gene selected according to the fourth tier parameters. The targeting domains bind within 200 bp from a mutation (e.g., I477N), and PAM is NNGRRT. It is contemplated herein that in an embodiment the targeting domain hybridizes to the target domain through complementary base pairing. Any of the targeting domains in the Table can be used with a *S. aureus* Cas9 molecule that generates a double stranded break (Cas9 nuclease) or a single-stranded break (Cas9 nickase).

**Table 19D**

| 4th Tier | | | | |
|---|---|---|---|---|
| gRNA Name | DNA Strand | Targeting Domain | Target Site Length | SEQ ID NO |
| MYOC-I477N-135 | + | CUUAUAGCGGUUCUUGAA | 18 | 3610 |
| MYOC-I477N-136 | + | ACUUAUAGCGGUUCUUGAA | 19 | 3611 |
| MYOC-I477N-31 | + | UACUUAUAGCGGUUCUUGAA | 20 | 461 |
| MYOC-I477N-137 | + | CAAAGAGCUUCUUCUCCA | 18 | 3614 |
| MYOC-I477N-138 | + | CUGUACUUAUAGCGGUUC | 18 | 3657 |
| MYOC-I477N-139 | + | UGCUGUACUUAUAGCGGUUC | 20 | 1856 |
| MYOC-I477N-140 | + | CGGUUCUUGAAUGGGAUGGU | 20 | 1854 |
| MYOC-I477N-141 | + | UUUGUCUCCCAGGUUUGU | 18 | 3689 |
| MYOC-I477N-142 | + | UGUUUGUCUCCCAGGUUUGU | 20 | 2792 |
| MYOC-I477N-143 | + | AUUGGCGACUGACUGCUU | 18 | 3695 |
| MYOC-I477N-144 | + | CAUUGGCGACUGACUGCUU | 19 | 3696 |
| MYOC-I477N-145 | + | AGGCAUUGGCGACUGACUGCUU | 22 | 3697 |
| MYOC-I477N-146 | + | AAGGCAUUGGCGACUGACUGCUU | 23 | 3698 |

**Table 19E** provides exemplary targeting domains for correcting a mutation (e.g., I477N) in the MYOC gene selected according to the fifth tier parameters. The targeting domains bind within 200 bp from a mutation (e.g., I477N), and PAM is NNGRRV. It is contemplated herein that in an embodiment the targeting domain hybridizes to the target domain through complementary base pairing. Any of the targeting domains in the Table can be used with a *S. aureus* Cas9 molecule that generates a double stranded break (Cas9 nuclease) or a single-stranded break (Cas9 nickase).

**Table 19E**

| 5th Tier | | | | |
|---|---|---|---|---|
| gRNA Name | DNA Strand | Targeting Domain | Target Site Length | SEQ ID NO |
| MYOC-I477N-147 | + | UUCAAGUUGUCCCAGGCA | 18 | 3879 |
| MYOC-I477N-148 | + | GUUCAAGUUGUCCCAGGCA | 19 | 3880 |
| MYOC-I477N-149 | + | UGUUCAAGUUGUCCCAGGCA | 20 | 1858 |
| MYQC-I477N-150 | + | AUGUUCAAGUUGUCCCAGGCA | 21 | 3881 |
| MYOC-I477N-151 | + | CAUGUUCAAGUUGUCCCAGGCA | 22 | 3882 |
| MYOC-I477N-152 | + | CCAUGUUCAAGUUGUCCCAGGCA | 23 | 3883 |
| MYOC-I477N-153 | + | ACCAUGUUCAAGUUGUCCCAGGCA | 24 | 3884 |
| MYOC-I477N-154 | + | ACUUAUAGCGGUUCUUGA | 18 | 3906 |
| MYOC-I477N-155 | + | UACUUAUAGCGGUUCUUGA | 19 | 3907 |
| MYOC-I477N-156 | + | GUACUUAUAGCGGUUCUUGA | 20 | 1855 |
| MYOC-I477N-157 | + | UGUACUUAUAGCGGUUCUUGA | 21 | 3908 |
| MYOC-I477N-158 | + | CUGUACUUAUAGCGGUUCUUGA | 22 | 3909 |
| MYOC-I477N-159 | + | GCUGUACUUAUAGCGGUUCUUGA | 23 | 3910 |
| MYOC-I477N-160 | + | UGCUGUACUUAUAGCGGUUCUUGA | 24 | 3911 |
| MYOC-I477N-161 | + | CUUCAGCCUGCUCCCCCC | 18 | 3956 |
| MYOC-I477N-162 | + | CCUUCAGCCUGCUCCCCCC | 19 | 3957 |
| MYOC-I477N-39 | + | CCCUUCAGCCUGCUCCCCCC | 20 | 785 |
| MYOC-I477N-163 | + | UCCCUUCAGCCUGCUCCCCCC | 21 | 3958 |
| MYOC-I477N-164 | + | CUCCCUUCAGCCUGCUCCCCCC | 22 | 3959 |
| MYOC-I477N-165 | + | UCUCCCUUCAGCCUGCUCCCCCC | 23 | 3960 |
| MYOC-I477N-166 | + | CUCUCCCUUCAGCCUGCUCCCCCC | 24 | 3961 |
| MYOC-I477N-167 | + | CCUUCAGCCUGCUCCCCC | 18 | 3962 |
| MYOC-I477N-168 | + | CCCUUCAGCCUGCUCCCCC | 19 | 3963 |
| MYOC-I477N-169 | + | UCCCUUCAGCCUGCUCCCCC | 20 | 2811 |
| MYOC-I477N-170 | + | CUCCCUUCAGCCUGCUCCCCC | 21 | 3964 |
| MYOC-I477N-171 | + | UCUCCCUUCAGCCUGCUCCCCC | 22 | 3965 |
| MYOC-I477N-172 | + | CUCUCCCUUCAGCCUGCUCCCCC | 23 | 3966 |
| MYOC-I477N-173 | + | GCUCUCCCUUCAGCCUGCUCCCCC | 24 | 3967 |
| MYOC-I477N-174 | + | CUGCUCCCCCCAGGAGCC | 18 | 3974 |
| MYOC-I477N-175 | + | CCUGCUCCCCCCAGGAGCC | 19 | 3975 |
| MYOC-I477N-176 | + | GCCUGCUCCCCCCAGGAGCC | 20 | 2810 |
| MYOC-I477N-177 | + | AGCCUGCUCCCCCCAGGAGCC | 21 | 3976 |
| MYOC-I477N-178 | + | CAGCCUGCUCCCCCCAGGAGCC | 22 | 3977 |
| MYOC-I477N-179 | + | UCAGCCUGCUCCCCCCAGGAGCC | 23 | 3978 |
| MYOC-I477N-180 | + | UUCAGCCUGCUCCCCCCAGGAGCC | 24 | 3979 |
| MYOC-I477N-181 | + | GCAAAGAGCUUCUUCUCC | 18 | 3987 |
| MYOC-I477N-182 | + | GGCAAAGAGCUUCUUCUCC | 19 | 3988 |
| MYOC-I477N-101 | + | AGGCAAAGAGCUUCUUCUCC | 20 | 458 |
| MYOC-I477N-183 | + | CAGGCAAAGAGCUUCUUCUCC | 21 | 3989 |
| MYOC-I477N-184 | + | CCAGGCAAAGAGCUUCUUCUCC | 22 | 3990 |
| MYOC-I477N-185 | + | CCCAGGCAAAGAGCUUCUUCUCC | 23 | 3991 |
| MYOC-I477N-186 | + | UCCCAGGCAAAGAGCUUCUUCUCC | 24 | 3992 |
| MYOC-I477N-187 | + | UGCCAUUGCCUGUACAGC | 18 | 4011 |
| MYOC-I477N-188 | + | CUGCCAUUGCCUGUACAGC | 19 | 4012 |
| MYOC-I477N-189 | + | UCUGCCAUUGCCUGUACAGC | 20 | 2809 |
| MYOC-I477N-190 | + | UUCUGCCAUUGCCUGUACAGC | 21 | 4013 |
| MYOC-I477N-191 | + | CUUCUGCCAUUGCCUGUACAGC | 22 | 4014 |
| MYOC-I477N-192 | + | CCUUCUGCCAUUGCCUGUACAGC | 23 | 4015 |
| MYOC-I477N-193 | + | UCCUUCUGCCAUUGCCUGUACAGC | 24 | 4016 |
| MYOC-I477N-194 | + | GAAAGCAGUCAAAGCUGC | 18 | 4052 |
| MYOC-I477N-195 | + | GGAAAGCAGUCAAAGCUGC | 19 | 4053 |
| MYOC-I477N-196 | + | UGGAAAGCAGUCAAAGCUGC | 20 | 2812 |
| MYOC-I477N-197 | + | UUGGAGGCUUUUCACAUC | 18 | 4058 |
| MYOC-I477N-198 | + | CUUGGAGGCUUUUCACAUC | 19 | 4059 |
| MYOC-I477N-199 | + | GCUUGGAGGCUUUUCACAUC | 20 | 1860 |
| MYOC-I477N-200 | + | AGCUUGGAGGCUUUUCACAUC | 21 | 4060 |
| MYOC-I477N-201 | + | CAGCUUGGAGGCUUUUCACAUC | 22 | 4061 |
| MYOC-I477N-202 | + | ACAGCUUGGAGGCUUUUCACAUC | 23 | 4062 |
| MYOC-I477N-203 | + | UACAGCUUGGAGGCUUUUCACAUC | 24 | 4063 |
| MYOC-I477N-204 | + | GGCAAAGAGCUUCUUCUC | 18 | 4083 |
| MYOC-I477N-205 | + | AGGCAAAGAGCUUCUUCUC | 19 | 4084 |
| MYOC-I477N-206 | + | CAGGCAAAGAGCUUCUUCUC | 20 | 1857 |
| MYOC-I477N-207 | + | CCAGGCAAAGAGCUUCUUCUC | 21 | 4085 |
| MYOC-I477N-208 | + | CCCAGGCAAAGAGCUUCUUCUC | 22 | 4086 |
| MYOC-I477N-209 | + | UCCCAGGCAAAGAGCUUCUUCUC | 23 | 4087 |
| MYOC-I477N-210 | + | GUCCCAGGCAAAGAGCUUCUUCUC | 24 | 4088 |
| MYOC-I477N-211 | + | UACAAGGUGCCACAGAUG | 18 | 4158 |
| MYOC-I477N-212 | + | GUACAAGGUGCCACAGAUG | 19 | 4159 |
| MYOC-I477N-213 | + | UGUACAAGGUGCCACAGAUG | 20 | 2794 |
| MYOC-I477N-214 | + | GUGUACAAGGUGCCACAGAUG | 21 | 4160 |
| MYOC-I477N-215 | + | GGUGUACAAGGUGCCACAGAUG | 22 | 4161 |
| MYOC-I477N-216 | + | CGGUGUACAAGGUGCCACAGAUG | 23 | 4162 |
| MYOC-I477N-217 | + | ACGGUGUACAAGGUGCCACAGAUG | 24 | 4163 |
| MYOC-I477N-218 | + | AGUUGACGGUAGCAUCUG | 18 | 4178 |
| MYOC-I477N-219 | + | AAGUUGACGGUAGCAUCUG | 19 | 4179 |
| MYOC-I477N-220 | + | AAAGUUGACGGUAGCAUCUG | 20 | 1853 |
| MYOC-I477N-221 | + | CAAAGUUGACGGUAGCAUCUG | 21 | 4180 |
| MYOC-I477N-222 | + | GCAAAGUUGACGGUAGCAUCUG | 22 | 4181 |
| MYOC-I477N-223 | + | AGCAAAGUUGACGGUAGCAUCUG | 23 | 4182 |
| MYOC-I477N-224 | + | AAGCAAAGUUGACGGUAGCAUCUG | 24 | 4183 |
| MYOC-I477N-225 | + | GAGGCUUUUCACAUCUUG | 18 | 4191 |
| MYOC-I477N-226 | + | GGAGGCUUUUCACAUCUUG | 19 | 4192 |
| MYOC-I477N-227 | + | UGGAGGCUUUUCACAUCUUG | 20 | 1859 |
| MYOC-I477N-228 | + | UUGGAGGCUUUUCACAUCUUG | 21 | 4193 |
| MYOC-I477N-229 | + | CUUGGAGGCUUUUCACAUCUUG | 22 | 4194 |
| MYOC-I477N-230 | + | GCUUGGAGGCUUUUCACAUCUUG | 23 | 4195 |
| MYOC-I477N-231 | + | AGCUUGGAGGCUUUUCACAUCUUG | 24 | 4196 |
| MYOC-I477N-232 | + | GCCAUUGCCUGUACAGCU | 18 | 4265 |
| MYOC-I477N-233 | + | UGCCAUUGCCUGUACAGCU | 19 | 4266 |
| MYOC-I477N-49 | + | CUGCCAUUGCCUGUACAGCU | 20 | 786 |
| MYOC-I477N-234 | + | UCUGCCAUUGCCUGUACAGCU | 21 | 4267 |
| MYOC-I477N-235 | + | UUCUGCCAUUGCCUGUACAGCU | 22 | 4268 |
| MYOC-I477N-236 | + | CUUCUGCCAUUGCCUGUACAGCU | 23 | 4269 |
| MYOC-I477N-237 | + | CCUUCUGCCAUUGCCUGUACAGCU | 24 | 4270 |
| MYOC-I477N-238 | + | UGGAGGCUUUUCACAUCU | 18 | 4271 |
| MYOC-I477N-239 | + | UUGGAGGCUUUUCACAUCU | 19 | 4272 |
| MYOC-I477N-50 | + | CUUGGAGGCUUUUCACAUCU | 20 | 457 |
| MYOC-I477N-240 | + | GCUUGGAGGCUUUUCACAUCU | 21 | 4273 |
| MYOC-I477N-241 | + | AGCUUGGAGGCUUUUCACAUCU | 22 | 4274 |
| MYOC-I477N-242 | + | CAGCUUGGAGGCUUUUCACAUCU | 23 | 4275 |
| MYOC-I477N-243 | + | ACAGCUUGGAGGCUUUUCACAUCU | 24 | 4276 |
| MYOC-I477N-244 | - | UGGGGGGAGCAGGCUGAA | 18 | 4370 |
| MYOC-I477N-245 | - | CUGGGGGGAGCAGGCUGAA | 19 | 4371 |
| MYOC-I477N-94 | - | CCUGGGGGGAGCAGGCUGAA | 20 | 781 |
| MYOC-I477N-246 | - | UCCUGGGGGGAGCAGGCUGAA | 21 | 4372 |
| MYOC-I477N-247 | - | CUCCUGGGGGGAGCAGGCUGAA | 22 | 4373 |
| MYOC-I477N-248 | - | GCUCCUGGGGGGAGCAGGCUGAA | 23 | 4374 |
| MYOC-I477N-249 | - | GGCUCCUGGGGGGAGCAGGCUGAA | 24 | 4375 |
| MYOC-I477N-250 | - | UGUACAGGCAAUGGCAGA | 18 | 4408 |
| MYOC-I477N-251 | - | CUGUACAGGCAAUGGCAGA | 19 | 4409 |
| MYOC-I477N-10 | - | GCUGUACAGGCAAUGGCAGA | 20 | 771 |
| MYOC-I477N-252 | - | AGCUGUACAGGCAAUGGCAGA | 21 | 4410 |
| MYOC-I477N-253 | - | AAGCUGUACAGGCAAUGGCAGA | 22 | 4411 |
| MYOC-I477N-254 | - | CAAGCUGUACAGGCAAUGGCAGA | 23 | 4412 |
| MYOC-I477N-255 | - | CCAAGCUGUACAGGCAAUGGCAGA | 24 | 4413 |
| MYOC-I477N-256 | - | CUGGGGGGAGCAGGCUGA | 18 | 4453 |
| MYOC-I477N-257 | - | CCUGGGGGGAGCAGGCUGA | 19 | 4454 |
| MYOC-I477N-97 | - | UCCUGGGGGGAGCAGGCUGA | 20 | 780 |
| MYOC-I477N-258 | - | CUCCUGGGGGGAGCAGGCUGA | 21 | 4455 |
| MYOC-I477N-259 | - | GCUCCUGGGGGGAGCAGGCUGA | 22 | 4456 |
| MYOC-I477N-260 | - | GGCUCCUGGGGGGAGCAGGCUGA | 23 | 4457 |
| MYOC-I477N-261 | - | GGGCUCCUGGGGGGAGCAGGCUGA | 24 | 4458 |
| MYOC-I477N-262 | - | CUCUUUGCCUGGGACAAC | 18 | 4485 |
| MYOC-I477N-263 | - | GCUCUUUGCCUGGGACAAC | 19 | 4486 |
| MYOC-I477N-264 | - | AGCUCUUUGCCUGGGACAAC | 20 | 1851 |
| MYOC-I477N-265 | - | AAGCUCUUUGCCUGGGACAAC | 21 | 4487 |
| MYOC-I477N-266 | - | GAAGCUCUUUGCCUGGGACAAC | 22 | 4488 |
| MYOC-I477N-267 | - | AGAAGCUCUUUGCCUGGGACAAC | 23 | 4489 |
| MYOC-I477N-268 | - | AAGAAGCUCUUUGCCUGGGACAAC | 24 | 4490 |
| MYOC-I477N-269 | - | CUGGAACUCGAACAAACC | 18 | 4537 |
| MYOC-I477N-270 | - | UCUGGAACUCGAACAAACC | 19 | 4538 |
| MYOC-I477N-37 | - | AUCUGGAACUCGAACAAACC | 20 | 766 |
| MYOC-I477N-271 | - | AUGAUUGACUACAACCCC | 18 | 4569 |
| MYOC-I477N-272 | - | CAUGAUUGACUACAACCCC | 19 | 4570 |
| MYOC-I477N-273 | - | GCAUGAUUGACUACAACCCC | 20 | 1847 |
| MYOC-I477N-274 | - | AGCAUGAUUGACUACAACCCC | 21 | 4571 |
| MYOC-I477N-275 | - | CAGCAUGAUUGACUACAACCCC | 22 | 4572 |
| MYOC-I477N-276 | - | GCAGCAUGAUUGACUACAACCCC | 23 | 4573 |
| MYOC-I477N-277 | - | AGCAGCAUGAUUGACUACAACCCC | 24 | 4574 |
| MYOC-I477N-278 | - | UGAUUGACUACAACCCCC | 18 | 4575 |
| MYOC-I477N-279 | - | AUGAUUGACUACAACCCCC | 19 | 4576 |
| MYOC-I477N-38 | - | CAUGAUUGACUACAACCCCC | 20 | 454 |
| MYOC-I477N-280 | - | GCAUGAUUGACUACAACCCCC | 21 | 4577 |
| MYOC-I477N-281 | - | AGCAUGAUUGACUACAACCCCC | 22 | 4578 |
| MYOC-I477N-282 | - | CAGCAUGAUUGACUACAACCCCC | 23 | 4579 |
| MYOC-I477N-283 | - | GCAGCAUGAUUGACUACAACCCCC | 24 | 4580 |
| MYOC-I477N-284 | - | GAGAAGAAGCUCUUUGCC | 18 | 4587 |
| MYOC-I477N-285 | - | GGAGAAGAAGCUCUUUGCC | 19 | 4588 |
| MYOC-I477N-100 | - | UGGAGAAGAAGCUCUUUGCC | 20 | 455 |
| MYOC-I477N-286 | - | CUGGAGAAGAAGCUCUUUGCC | 21 | 4589 |
| MYOC-I477N-287 | - | CCUGGAGAAGAAGCUCUUUGCC | 22 | 4590 |
| MYOC-I477N-288 | - | CCCUGGAGAAGAAGCUCUUUGCC | 23 | 4591 |
| MYOC-I477N-289 | - | CCCCUGGAGAAGAAGCUCUUUGCC | 24 | 4592 |
| MYOC-I477N-290 | - | GGAGAUGCUCAGGGCUCC | 18 | 4599 |
| MYOC-I477N-291 | - | AGGAGAUGCUCAGGGCUCC | 19 | 4600 |
| MYOC-I477N-42 | - | AAGGAGAUGCUCAGGGCUCC | 20 | 774 |
| MYOC-I477N-292 | - | GAAGGAGAUGCUCAGGGCUCC | 21 | 4601 |
| MYOC-I477N-293 | - | AGAAGGAGAUGCUCAGGGCUCC | 22 | 4602 |
| MYOC-I477N-294 | - | CAGAAGGAGAUGCUCAGGGCUCC | 23 | 4603 |
| MYOC-I477N-295 | - | GCAGAAGGAGAUGCUCAGGGCUCC | 24 | 4604 |
| MYOC-I477N-296 | - | AAGGGAGAGCCAGCCAGC | 18 | 4618 |
| MYOC-I477N-297 | - | GAAGGGAGAGCCAGCCAGC | 19 | 4619 |
| MYOC-I477N-298 | - | UGAAGGGAGAGCCAGCCAGC | 20 | 2802 |
| MYOC-I477N-299 | - | CUGAAGGGAGAGCCAGCCAGC | 21 | 4620 |
| MYOC-I477N-300 | - | GCUGAAGGGAGAGCCAGCCAGC | 22 | 4621 |
| MYQC-I477N-301 | - | GGCUGAAGGGAGAGCCAGCCAGC | 23 | 4622 |
| MYOC-I477N-302 | - | AGGCUGAAGGGAGAGCCAGCCAGC | 24 | 4623 |
| MYOC-I477N-303 | - | GGAGAAGAAGCUCUUUGC | 18 | 4630 |
| MYOC-I477N-304 | - | UGGAGAAGAAGCUCUUUGC | 19 | 4631 |
| MYOC-I477N-305 | - | CUGGAGAAGAAGCUCUUUGC | 20 | 1850 |
| MYOC-I477N-306 | - | CCUGGAGAAGAAGCUCUUUGC | 21 | 4632 |
| MYOC-I477N-307 | - | CCCUGGAGAAGAAGCUCUUUGC | 22 | 4633 |
| MYOC-I477N-308 | - | CCCCUGGAGAAGAAGCUCUUUGC | 23 | 4634 |
| MYOC-I477N-309 | - | CCCCCUGGAGAAGAAGCUCUUUGC | 24 | 4635 |
| MYQC-I477N-310 | - | AGGAGAUGCUCAGGGCUC | 18 | 4660 |
| MYOC-I477N-311 | - | AAGGAGAUGCUCAGGGCUC | 19 | 4661 |
| MYOC-I477N-312 | - | GAAGGAGAUGCUCAGGGCUC | 20 | 2798 |
| MYOC-I477N-313 | - | AGAAGGAGAUGCUCAGGGCUC | 21 | 4662 |
| MYOC-I477N-314 | - | CAGAAGGAGAUGCUCAGGGCUC | 22 | 4663 |
| MYOC-I477N-315 | - | GCAGAAGGAGAUGCUCAGGGCUC | 23 | 4664 |
| MYOC-I477N-316 | - | GGCAGAAGGAGAUGCUCAGGGCUC | 24 | 4665 |
| MYOC-I477N-317 | - | ACCCUGACCAUCCCAUUC | 18 | 4673 |
| MYOC-I477N-318 | - | GACCCUGACCAUCCCAUUC | 19 | 4674 |
| MYOC-I477N-319 | - | AGACCCUGACCAUCCCAUUC | 20 | 1846 |
| MYOC-I477N-320 | - | AAGACCCUGACCAUCCCAUUC | 21 | 4675 |
| MYOC-I477N-321 | - | CAAGACCCUGACCAUCCCAUUC | 22 | 4676 |
| MYOC-I477N-322 | - | GCAAGACCCUGACCAUCCCAUUC | 23 | 4677 |
| MYOC-I477N-323 | - | AGCAAGACCCUGACCAUCCCAUUC | 24 | 4678 |
| MYOC-I477N-324 | - | CUGUACAGGCAAUGGCAG | 18 | 4720 |
| MYOC-I477N-325 | - | GCUGUACAGGCAAUGGCAG | 19 | 4721 |
| MYOC-I477N-326 | - | AGCUGUACAGGCAAUGGCAG | 20 | 2796 |
| MYOC-I477N-327 | - | AAGCUGUACAGGCAAUGGCAG | 21 | 4722 |
| MYOC-I477N-328 | - | CAAGCUGUACAGGCAAUGGCAG | 22 | 4723 |
| MYOC-I477N-329 | - | CCAAGCUGUACAGGCAAUGGCAG | 23 | 4724 |
| MYOC-I477N-330 | - | UCCAAGCUGUACAGGCAAUGGCAG | 24 | 4725 |
| MYOC-I477N-331 | - | GACUACAACCCCCUGGAG | 18 | 4738 |
| MYOC-I477N-332 | - | UGACUACAACCCCCUGGAG | 19 | 4739 |
| MYOC-I477N-333 | - | UUGACUACAACCCCCUGGAG | 20 | 1849 |
| MYOC-I477N-334 | - | AUUGACUACAACCCCCUGGAG | 21 | 4740 |
| MYOC-I477N-335 | - | GAUUGACUACAACCCCCUGGAG | 22 | 4741 |
| MYOC-I477N-336 | - | UGAUUGACUACAACCCCCUGGAG | 23 | 4742 |
| MYOC-I477N-337 | - | AUGAUUGACUACAACCCCCUGGAG | 24 | 4743 |
| MYOC-I477N-338 | - | GGGGGAGCAGGCUGAAGG | 18 | 4806 |
| MYOC-I477N-339 | - | GGGGGGAGCAGGCUGAAGG | 19 | 4807 |
| MYOC-I477N-340 | - | UGGGGGGAGCAGGCUGAAGG | 20 | 2801 |
| MYOC-I477N-341 | - | CUGGGGGGAGCAGGCUGAAGG | 21 | 4808 |
| MYOC-I477N-342 | - | CCUGGGGGGAGCAGGCUGAAGG | 22 | 4809 |
| MYOC-I477N-343 | - | UCCUGGGGGGAGCAGGCUGAAGG | 23 | 4810 |
| MYOC-I477N-344 | - | CUCCUGGGGGGAGCAGGCUGAAGG | 24 | 4811 |
| MYOC-I477N-345 | - | GCUCCUGGGGGGAGCAGG | 18 | 4818 |
| MYOC-I477N-346 | - | GGCUCCUGGGGGGAGCAGG | 19 | 4819 |
| MYOC-I477N-347 | - | GGGCUCCUGGGGGGAGCAGG | 20 | 2799 |
| MYOC-I477N-348 | - | AGGGCUCCUGGGGGGAGCAGG | 21 | 4820 |
| MYOC-I477N-349 | - | CAGGGCUCCUGGGGGGAGCAGG | 22 | 4821 |
| MYOC-I477N-350 | - | UCAGGGCUCCUGGGGGGAGCAGG | 23 | 4822 |
| MYOC-I477N-351 | - | CUCAGGGCUCCUGGGGGGAGCAGG | 24 | 4823 |
| MYOC-I477N-352 | - | AUGCUCAGGGCUCCUGGG | 18 | 4824 |
| MYOC-I477N-353 | - | GAUGCUCAGGGCUCCUGGG | 19 | 4825 |
| MYOC-I477N-106 | - | AGAUGCUCAGGGCUCCUGGG | 20 | 778 |
| MYOC-I477N-354 | - | GAGAUGCUCAGGGCUCCUGGG | 21 | 4826 |
| MYOC-I477N-355 | - | GGAGAUGCUCAGGGCUCCUGGG | 22 | 4827 |
| MYOC-I477N-356 | - | AGGAGAUGCUCAGGGCUCCUGGG | 23 | 4828 |
| MYOC-I477N-357 | - | AAGGAGAUGCUCAGGGCUCCUGGG | 24 | 4829 |
| MYOC-I477N-358 | - | AAGCUGUACAGGCAAUGG | 18 | 4837 |
| MYOC-I477N-359 | - | CAAGCUGUACAGGCAAUGG | 19 | 4838 |
| MYOC-I477N-360 | - | CCAAGCUGUACAGGCAAUGG | 20 | 2795 |
| MYOC-I477N-361 | - | UCCAAGCUGUACAGGCAAUGG | 21 | 4839 |
| MYOC-I477N-362 | - | CUCCAAGCUGUACAGGCAAUGG | 22 | 4840 |
| MYOC-I477N-363 | - | CCUCCAAGCUGUACAGGCAAUGG | 23 | 4841 |
| MYOC-I477N-364 | - | GCCUCCAAGCUGUACAGGCAAUGG | 24 | 4842 |
| MYOC-I477N-365 | - | GAUGCUCAGGGCUCCUGG | 18 | 4843 |
| MYOC-I477N-366 | - | AGAUGCUCAGGGCUCCUGG | 19 | 4844 |
| MYOC-I477N-68 | - | GAGAUGCUCAGGGCUCCUGG | 20 | 777 |
| MYOC-I477N-367 | - | GGAGAUGCUCAGGGCUCCUGG | 21 | 4845 |
| MYOC-I477N-368 | - | AGGAGAUGCUCAGGGCUCCUGG | 22 | 4846 |
| MYOC-I477N-369 | - | AAGGAGAUGCUCAGGGCUCCUGG | 23 | 4847 |
| MYOC-I477N-370 | - | GAAGGAGAUGCUCAGGGCUCCUGG | 24 | 4848 |
| MYOC-I477N-371 | - | AUUGACUACAACCCCCUG | 18 | 4874 |
| MYOC-I477N-372 | - | GAUUGACUACAACCCCCUG | 19 | 4875 |
| MYOC-I477N-373 | - | UGAUUGACUACAACCCCCUG | 20 | 1848 |
| MYOC-I477N-374 | - | AUGAUUGACUACAACCCCCUG | 21 | 4876 |
| MYOC-I477N-375 | - | CAUGAUUGACUACAACCCCCUG | 22 | 4877 |
| MYOC-I477N-376 | - | GCAUGAUUGACUACAACCCCCUG | 23 | 4878 |
| MYOC-I477N-377 | - | AGCAUGAUUGACUACAACCCCCUG | 24 | 4879 |
| MYOC-I477N-378 | - | AGAUGCUCAGGGCUCCUG | 18 | 4880 |
| MYOC-I477N-379 | - | GAGAUGCUCAGGGCUCCUG | 19 | 4881 |
| MYOC-I477N-69 | - | GGAGAUGCUCAGGGCUCCUG | 20 | 776 |
| MYOC-I477N-380 | - | AGGAGAUGCUCAGGGCUCCUG | 21 | 4882 |
| MYOC-I477N-381 | - | AAGGAGAUGCUCAGGGCUCCUG | 22 | 4883 |
| MYOC-I477N-382 | - | GAAGGAGAUGCUCAGGGCUCCUG | 23 | 4884 |
| MYOC-I477N-383 | - | AGAAGGAGAUGCUCAGGGCUCCUG | 24 | 4885 |
| MYOC-I477N-384 | - | CCUGGGGGGAGCAGGCUG | 18 | 4886 |
| MYOC-I477N-385 | - | UCCUGGGGGGAGCAGGCUG | 19 | 4887 |
| MYOC-I477N-386 | - | CUCCUGGGGGGAGCAGGCUG | 20 | 2800 |
| MYOC-I477N-387 | - | GCUCCUGGGGGGAGCAGGCUG | 21 | 4888 |
| MYOC-I477N-388 | - | GGCUCCUGGGGGGAGCAGGCUG | 22 | 4889 |
| MYOC-I477N-389 | - | GGGCUCCUGGGGGGAGCAGGCUG | 23 | 4890 |
| MYOC-I477N-390 | - | AGGGCUCCUGGGGGGAGCAGGCUG | 24 | 4891 |
| MYOC-I477N-391 | - | CAUCAAGCUCUCCAAGAU | 18 | 4939 |
| MYOC-I477N-392 | - | ACAUCAAGCUCUCCAAGAU | 19 | 4940 |
| MYOC-I477N-393 | - | GACAUCAAGCUCUCCAAGAU | 20 | 1852 |
| MYOC-I477N-394 | - | UGACAUCAAGCUCUCCAAGAU | 21 | 4941 |
| MYOC-I477N-395 | - | AUGACAUCAAGCUCUCCAAGAU | 22 | 4942 |
| MYOC-I477N-396 | - | UAUGACAUCAAGCUCUCCAAGAU | 23 | 4943 |
| MYOC-I477N-397 | - | UUAUGACAUCAAGCUCUCCAAGAU | 24 | 4944 |
| MYOC-I477N-398 | - | UGGAACUCGAACAAACCU | 18 | 4978 |
| MYOC-I477N-399 | - | CUGGAACUCGAACAAACCU | 19 | 4979 |
| MYOC-I477N-47 | - | UCUGGAACUCGAACAAACCU | 20 | 767 |
| MYOC-I477N-400 | - | GAGAUGCUCAGGGCUCCU | 18 | 4984 |
| MYQC-I477N-401 | - | GGAGAUGCUCAGGGCUCCU | 19 | 4985 |
| MYOC-I477N-48 | - | AGGAGAUGCUCAGGGCUCCU | 20 | 775 |
| MYOC-I477N-402 | - | AAGGAGAUGCUCAGGGCUCCU | 21 | 4986 |
| MYOC-I477N-403 | - | GAAGGAGAUGCUCAGGGCUCCU | 22 | 4987 |
| MYOC-I477N-404 | - | AGAAGGAGAUGCUCAGGGCUCCU | 23 | 4988 |
| MYOC-I477N-405 | - | CAGAAGGAGAUGCUCAGGGCUCCU | 24 | 4989 |
| MYOC-I477N-406 | - | AUGGCAGAAGGAGAUGCU | 18 | 5009 |
| MYOC-I477N-407 | - | AAUGGCAGAAGGAGAUGCU | 19 | 5010 |
| MYOC-I477N-408 | - | CAAUGGCAGAAGGAGAUGCU | 20 | 2797 |
| MYOC-I477N-409 | - | GCAAUGGCAGAAGGAGAUGCU | 21 | 5011 |
| MYOC-I477N-410 | - | GGCAAUGGCAGAAGGAGAUGCU | 22 | 5012 |
| MYOC-I477N-411 | - | AGGCAAUGGCAGAAGGAGAUGCU | 23 | 5013 |
| MYOC-I477N-412 | - | CAGGCAAUGGCAGAAGGAGAUGCU | 24 | 5014 |

**Table 20A** provides exemplary targeting domains for correcting a mutation (e.g., I477N) in the MYOC gene selected according to the first tier parameters. The targeting domains bind within 200 bp from a mutation (e.g., I477N), have a high level of orthogonality and start with a 5'G. It is contemplated herein that in an embodiment the targeting domain hybridizes to the target domain through complementary base pairing. Any of the targeting domains in the Table can be used with a N. *meningitidis* Cas9 molecule that generates a double stranded break (Cas9 nuclease) or a single-stranded break (Cas9 nickase).

**Table 20A**

| 1st Tier | | | | |
|---|---|---|---|---|
| gRNA Name | DNA Strand | Targeting Domain | Target Site Length | SEQ ID NO |
| MYOC-I477N-413 | - | GAACCGCUAUAAGUACAGCA | 20 | 2842 |

**Table 20B** provides exemplary targeting domains for correcting a mutation (e.g., I477N) in the MYOC gene selected according to the second tier parameters. The targeting domains bind within 200 bp from a mutation (e.g., I477N) and have a high level of orthogonality. It is contemplated herein that in an embodiment the targeting domain hybridizes to the target domain through complementary base pairing. Any of the targeting domains in the Table can be used with a *N. meningitidis* Cas9 molecule that generates a double stranded break (Cas9 nuclease) or a single-stranded break (Cas9 nickase).

**Table 20B**

| 2nd Tier | | | | |
|---|---|---|---|---|
| gRNA Name | DNA Strand | Targeting Domain | Target Site Length | SEQ ID NO |
| MYOC-I477N-414 | - | UCAGCAGAUGCUACCGUCAA | 20 | 5129 |

**Table 20C** provides exemplary targeting domains for correcting a mutation (e.g., I477N) in the MYOC gene selected according to the third tier parameters. The targeting domains bind within 200 bp from a mutation (e.g., I477N) and start with a 5'G. It is contemplated herein that in an embodiment the targeting domain hybridizes to the target domain through complementary base pairing. Any of the targeting domains in the Table can be used with a N. *meningitidis* Cas9 molecule that generates a double stranded break (Cas9 nuclease) or a single-stranded break (Cas9 nickase).

**Table 20C**

| 3rd Tier | | | | |
|---|---|---|---|---|
| gRNA Name | DNA Strand | Targeting Domain | Target Site Length | SEQ ID NO |
| MYOC-I477N-415 | - | GCAGAUGCUACCGUCAA | 17 | 5112 |
| MYOC-I477N-416 | - | GCCAGGGCCCAGGCAGCUUU | 20 | 5144 |

**Table 20D** provides exemplary targeting domains for correcting a mutation (e.g., I477N) in the MYOC gene selected according to the fourth tier parameters. The targeting domains bind within 200 bp from a mutation (e.g., I477N). It is contemplated herein that in an embodiment the targeting domain hybridizes to the target domain through complementary base pairing. Any of the targeting domains in the Table can be used with a *N. meningitidis* Cas9 molecule that generates a double stranded break (Cas9 nuclease) or a single-stranded break (Cas9 nickase).

**Table 20D**

| 4th Tier | | | | |
|---|---|---|---|---|
| gRNA Name | DNA Strand | Targeting Domain | Target Site Length | SEQ ID NO |
| MYOC-I477N-417 | - | CAGCCAGCCAGGGCCCA | 17 | 5114 |
| MYOC-I477N-418 | - | CCGCUAUAAGUACAGCA | 17 | 2843 |
| MYOC-I477N-419 | + | UCAAGUUGUCCCAGGCA | 17 | 1873 |
| MYOC-I477N-420 | + | CCUUCUGCCAUUGCCUG | 17 | 5122 |
| MYOC-I477N-421 | + | AGGCUUUUCACAUCUUG | 17 | 1874 |
| MYOC-I477N-422 | + | UGAAGGCAUUGGCGACU | 17 | 5124 |
| MYOC-I477N-423 | + | CAUUGCCUGUACAGCUU | 17 | 5127 |
| MYOC-I477N-424 | - | AGGGCCCAGGCAGCUUU | 17 | 5128 |
| MYOC-I477N-425 | - | AGCCAGCCAGCCAGGGCCCA | 20 | 5131 |
| MYOC-I477N-149 | + | UGUUCAAGUUGUCCCAGGCA | 20 | 1858 |
| MYOC-I477N-426 | + | UCUCCUUCUGCCAUUGCCUG | 20 | 5138 |
| MYOC-I477N-227 | + | UGGAGGCUUUUCACAUCUUG | 20 | 1859 |
| MYOC-I477N-427 | + | UGAUGAAGGCAUUGGCGACU | 20 | 5140 |
| MYOC-I477N-428 | + | UGCCAUUGCCUGUACAGCUU | 20 | 5143 |

**Table 21A** provides exemplary targeting domains for correcting a mutation (e.g., P370L) in the MYOC gene selected according to the first tier parameters. The targeting domains bind within 200 bp from a mutation (e.g., P370L), have a high level of orthogonality and start with a 5'G. It is contemplated herein that in an embodiment the targeting domain hybridizes to the target domain through complementary base pairing. Any of the targeting domains in the Table can be used with a *S. pyogenes* Cas9 molecule that generates a double stranded break (Cas9 nuclease) or a single-stranded break (Cas9 nickase).

**Table 21A**

| 1st Tier | | | | |
|---|---|---|---|---|
| gRNA Name | DNA Strand | Targeting Domain | Target Site Length | SEQ ID NO |
| MYOC-P370L-1 | - | GGGAGCCUCUAUUUCCA | 17 | 880 |
| MYOC-P370L-2 | - | GAAUACCGAGACAGUGA | 17 | 392 |
| MYOC-P370L-3 | - | GCAGGGCUACCCUUCUA | 17 | 870 |
| MYOC-P370L-4 | + | GGUAGCCCUGCAUAAAC | 17 | 927 |
| MYOC-P370L-5 | - | GGUGCUGUGGUGUACUC | 17 | 877 |
| MYOC-P370L-6 | + | GCACCCGUGCUUUCCAG | 17 | 923 |
| MYOC-P370L-7 | - | GUGCUGUGGUGUACUCG | 17 | 878 |
| MYOC-P370L-8 | - | GGACAUUGACUUGGCUG | 17 | 402 |
| MYOC-P370L-9 | - | GGGUGCUGUGGUGUACU | 17 | 876 |
| MYOC-P370L-10 | - | GGAACUCGAACAAACCU | 17 | 884 |
| MYOC-P370L-11 | - | GACAGUUCCCGUAUUCU | 17 | 881 |
| MYOC-P370L-12 | + | GUUCAGUUUGGAGAGGACAA | 20 | 799 |
| MYOC-P370L-13 | + | GCAGUAUGUGAACCUUAGAA | 20 | 806 |
| MYOC-P370L-14 | - | GUAUUCUUGGGGUGGCUACA | 20 | 388 |
| MYOC-P370L-15 | + | GUCCGUGGUAGCCAGCUCCA | 20 | 391 |
| MYOC-P370L-16 | - | GCCUAGGCCACUGGAAAGCA | 20 | 756 |
| MYOC-P370L-17 | + | GGCAGUAUGUGAACCUUAGA | 20 | 805 |
| MYOC-P370L-18 | - | GCUGAAUACCGAGACAGUGA | 20 | 398 |
| MYOC-P370L-19 | + | GUGUAGCCACCCCAAGAAUA | 20 | 390 |
| MYOC-P370L-20 | - | GACUUGGCUGUGGAUGAAGC | 20 | 400 |
| MYOC-P370L-21 | - | GGUCAUUUACAGCACCGAUG | 20 | 389 |
| MYOC-P370L-22 | - | GCCAAUGCCUUCAUCAUCUG | 20 | 768 |
| MYOC-P370L-23 | - | GGACAGUUCCCGUAUUCUUG | 20 | 764 |
| MYOC-P370L-24 | + | GCCACAGAUGAUGAAGGCAU | 20 | 792 |
| MYOC-P370L-25 | + | GUUCGAGUUCCAGAUUCUCU | 20 | 796 |
| MYOC-P370L-26 | + | GGAGAGGACAAUGGCACCUU | 20 | 800 |

**Table 21B** provides exemplary targeting domains for correcting a mutation (e.g., P370L) in the MYOC gene selected according to the second tier parameters. The targeting domains bind within 200 bp from a mutation (e.g., P370L) and have a high level of orthogonality. It is contemplated herein that in an embodiment the targeting domain hybridizes to the target domain through complementary base pairing. Any of the targeting domains in the Table can be used with a *S. pyogenes* Cas9 molecule that generates a double stranded break (Cas9 nuclease) or a single-stranded break (Cas9 nickase).

**Table 21B**

| 2nd Tier | | | | |
|---|---|---|---|---|
| gRNA Name | DNA Strand | Targeting Domain | Target Site Length | SEQ ID NO |
| MYOC-P370L-27 | - | AGCACCGAUGAGGCCAA | 17 | 433 |
| MYOC-P370L-28 | + | CGUGGUAGCCAGCUCCA | 17 | 397 |
| MYOC-P370L-29 | - | AUCAGCCAGUUUAUGCA | 17 | 869 |
| MYOC-P370L-30 | + | AGUAUGUGAACCUUAGA | 17 | 925 |
| MYOC-P370L-31 | + | UAGCCACCCCAAGAAUA | 17 | 395 |
| MYOC-P370L-32 | + | UGGCGACUGACUGCUUA | 17 | 912 |
| MYOC-P370L-33 | - | CAUACUGCCUAGGCCAC | 17 | 872 |
| MYOC-P370L-34 | + | AGCCACCCCAAGAAUAC | 17 | 435 |
| MYOC-P370L-35 | - | UGGAACUCGAACAAACC | 17 | 883 |
| MYOC-P370L-36 | + | UUCUGGACUCAGCGCCC | 17 | 921 |
| MYOC-P370L-37 | + | ACGGAUGUUUGUCUCCC | 17 | 913 |
| MYOC-P370L-38 | + | CCGUGGUAGCCAGCUCC | 17 | 436 |
| MYOC-P370L-39 | + | UCGAGUUCCAGAUUCUC | 17 | 914 |
| MYOC-P370L-40 | + | AUAUCUUAUGACAGUUC | 17 | 438 |
| MYOC-P370L-41 | + | CAGCGCCCUGGAAAUAG | 17 | 922 |
| MYOC-P370L-42 | + | AAUACGGGAACUGUCCG | 17 | 920 |
| MYOC-P370L-43 | - | UUCCCGUAUUCUUGGGG | 17 | 428 |
| MYOC-P370L-44 | - | CAUUUACAGCACCGAUG | 17 | 432 |
| MYOC-P370L-45 | - | CAGUUCCCGUAUUCUUG | 17 | 427 |
| MYOC-P370L-46 | - | CUACACGGACAUUGACU | 17 | 394 |
| MYOC-P370L-47 | + | CGAGUUCCAGAUUCUCU | 17 | 915 |
| MYOC-P370L-48 | - | ACAGUUCCCGUAUUCUU | 17 | 426 |
| MYOC-P370L-49 | - | UACAGCACCGAUGAGGCCAA | 20 | 415 |
| MYOC-P370L-50 | - | AUCCCUGGAGCUGGCUACCA | 20 | 407 |
| MYOC-P370L-51 | - | UCGGGGAGCCUCUAUUUCCA | 20 | 763 |
| MYOC-P370L-52 | + | CAAGGUGCCACAGAUGAUGA | 20 | 791 |
| MYOC-P370L-53 | - | UAUGCAGGGCUACCCUUCUA | 20 | 753 |
| MYOC-P370L-54 | + | CAUUGGCGACUGACUGCUUA | 20 | 793 |
| MYOC-P370L-55 | + | AAGGGUAGCCCUGCAUAAAC | 20 | 807 |
| MYOC-P370L-56 | - | UCACAUACUGCCUAGGCCAC | 20 | 755 |
| MYOC-P370L-57 | - | CCUAGGCCACUGGAAAGCAC | 20 | 757 |
| MYOC-P370L-58 | + | UGUAGCCACCCCAAGAAUAC | 20 | 418 |
| MYOC-P370L-59 | - | AUCUGGAACUCGAACAAACC | 20 | 766 |
| MYOC-P370L-60 | + | CAGUUCUGGACUCAGCGCCC | 20 | 801 |
| MYOC-P370L-61 | - | AAGGCUGAGAAGGAAAUCCC | 20 | 406 |
| MYOC-P370L-62 | + | CUUACGGAUGUUUGUCUCCC | 20 | 794 |
| MYOC-P370L-63 | + | UGUCCGUGGUAGCCAGCUCC | 20 | 420 |
| MYOC-P370L-64 | - | CUCGGGGAGCCUCUAUUUCC | 20 | 762 |
| MYOC-P370L-65 | - | CAAACUGAACCCAGAGAAUC | 20 | 765 |
| MYOC-P370L-66 | - | ACGGGUGCUGUGGUGUACUC | 20 | 760 |
| MYOC-P370L-67 | + | UGUUCGAGUUCCAGAUUCUC | 20 | 795 |
| MYOC-P370L-68 | + | CUCAUAUCUUAUGACAGUUC | 20 | 422 |
| MYOC-P370L-69 | + | CGGUGCUGUAAAUGACCCAG | 20 | 417 |
| MYOC-P370L-70 | + | ACUCAGCGCCCUGGAAAUAG | 20 | 802 |
| MYOC-P370L-71 | + | AAGAAUACGGGAACUGUCCG | 20 | 419 |
| MYOC-P370L-72 | - | CGGGUGCUGUGGUGUACUCG | 20 | 761 |
| MYOC-P370L-73 | - | CAGUUCCCGUAUUCUUGGGG | 20 | 410 |
| MYOC-P370L-74 | - | CACGGACAUUGACUUGGCUG | 20 | 412 |
| MYOC-P370L-75 | - | ACUGGAAAGCACGGGUGCUG | 20 | 758 |
| MYOC-P370L-76 | + | AAUGGCACCUUUGGCCUCAU | 20 | 416 |
| MYOC-P370L-77 | - | UGGCUACACGGACAUUGACU | 20 | 411 |
| MYOC-P370L-78 | - | CACGGGUGCUGUGGUGUACU | 20 | 759 |
| MYOC-P370L-79 | - | UCUGGAACUCGAACAAACCU | 20 | 767 |
| MYOC-P370L-80 | + | CCCGUGCUUUCCAGUGGCCU | 20 | 804 |
| MYOC-P370L-81 | - | CUAAGGUUCACAUACUGCCU | 20 | 754 |
| MYOC-P370L-82 | - | ACGGACAGUUCCCGUAUUCU | 20 | 408 |
| MYOC-P370L-83 | - | CGGACAGUUCCCGUAUUCUU | 20 | 409 |

**Table 21C** provides exemplary targeting domains for correcting a mutation (e.g., P370L) in the MYOC gene selected according to the third tier parameters. The targeting domains bind within 200 bp from a mutation (e.g., P370L) and start with a 5'G. It is contemplated herein that in an embodiment the targeting domain hybridizes to the target domain through complementary base pairing. Any of the targeting domains in the Table can be used with a *S. pyogenes* Cas9 molecule that generates a double stranded break (Cas9 nuclease) or a single-stranded break (Cas9 nickase).

**Table 21C**

| 3rd Tier | | | | |
|---|---|---|---|---|
| gRNA Name | DNA Strand | Targeting Domain | Target Site Length | SEQ ID NO |
| MYOC-P370L-84 | + | GUAUGUGAACCUUAGAA | 17 | 926 |
| MYOC-P370L-85 | - | GACAGUGAAGGCUGAGA | 17 | 401 |
| MYOC-P370L-86 | + | GGUGCCACAGAUGAUGA | 17 | 910 |
| MYOC-P370L-87 | - | GCUGAGAAGGAAAUCCC | 17 | 423 |
| MYOC-P370L-88 | - | GGGGAGCCUCUAUUUCC | 17 | 879 |
| MYOC-P370L-89 | - | GGAAAGCACGGGUGCUG | 17 | 875 |
| MYOC-P370L-90 | + | GGCACCUUUGGCCUCAU | 17 | 404 |
| MYOC-P370L-91 | + | GUGCUUUCCAGUGGCCU | 17 | 924 |
| MYOC-P370L-92 | - | GGAUGAAGCAGGCCUCU | 17 | 403 |
| MYOC-P370L-93 | + | GAGGACAAUGGCACCUU | 17 | 919 |
| MYOC-P370L-94 | - | GAGAAGGAAAUCCCUGGAGC | 20 | 399 |

**Table 21D** provides exemplary targeting domains for correcting a mutation (e.g., P370L) in the MYOC gene selected according to the fourth tier parameters. The targeting domains bind within 200 bp from a mutation (e.g., P370L). It is contemplated herein that in an embodiment the targeting domain hybridizes to the target domain through complementary base pairing. Any of the targeting domains in the Table can be used with a *S. pyogenes* Cas9 molecule that generates a double stranded break (Cas9 nuclease) or a single-stranded break (Cas9 nickase).

**Table 21D**

| 4th Tier | | | | |
|---|---|---|---|---|
| gRNA Name | DNA Strand | Targeting Domain | Target Site Length | SEQ ID NO |
| MYOC-P370L-95 | + | CAGUUUGGAGAGGACAA | 17 | 918 |
| MYOC-P370L-96 | - | UUCUUGGGGUGGCUACA | 17 | 429 |
| MYOC-P370L-97 | - | CCUGGAGCUGGCUACCA | 17 | 425 |
| MYOC-P370L-98 | - | UAGGCCACUGGAAAGCA | 17 | 873 |
| MYOC-P370L-99 | - | AGGCCACUGGAAAGCAC | 17 | 874 |
| MYOC-P370L-100 | - | UUGGCUGUGGAUGAAGC | 17 | 430 |
| MYOC-P370L-101 | - | AAGGAAAUCCCUGGAGC | 17 | 424 |
| MYOC-P370L-102 | - | CAUCAGCCAGUUUAUGC | 17 | 868 |
| MYOC-P370L-103 | - | ACUGAACCCAGAGAAUC | 17 | 882 |
| MYOC-P370L-104 | - | UGGAUGAAGCAGGCCUC | 17 | 431 |
| MYOC-P370L-105 | + | UGCUGUAAAUGACCCAG | 17 | 434 |
| MYOC-P370L-106 | + | CUGGGUUCAGUUUGGAG | 17 | 917 |
| MYOC-P370L-107 | - | AAUGCCUUCAUCAUCUG | 17 | 885 |
| MYOC-P370L-108 | + | ACAGAUGAUGAAGGCAU | 17 | 911 |
| MYOC-P370L-109 | - | AGGUUCACAUACUGCCU | 17 | 871 |
| MYOC-P370L-110 | + | CUCAGCCUUCACUGUCU | 17 | 437 |
| MYOC-P370L-111 | + | AUUCUCUGGGUUCAGUU | 17 | 916 |
| MYOC-P370L-112 | - | CGAGACAGUGAAGGCUGAGA | 20 | 405 |
| MYOC-P370L-113 | - | CUGUGGAUGAAGCAGGCCUC | 20 | 413 |
| MYOC-P370L-114 | + | ACAGCACCCGUGCUUUCCAG | 20 | 803 |
| MYOC-P370L-115 | + | UCUCUGGGUUCAGUUUGGAG | 20 | 798 |
| MYOC-P370L-116 | - | UGUGGAUGAAGCAGGCCUCU | 20 | 414 |
| MYOC-P370L-117 | + | CUUCUCAGCCUUCACUGUCU | 20 | 421 |
| MYOC-P370L-118 | + | CAGAUUCUCUGGGUUCAGUU | 20 | 797 |

**Table 22A** provides exemplary targeting domains for correcting a mutation (e.g., P370L) in the MYOC gene selected according to the first tier parameters. The targeting domains bind within 200 bp from a mutation (e.g., P370L), have a high level of orthogonality, start with a 5'G, and PAM is NNGRRT. It is contemplated herein that in an embodiment the targeting domain hybridizes to the target domain through complementary base pairing. Any of the targeting domains in the Table can be used with a *S. aureus* Cas9 molecule that generates a double stranded break (Cas9 nuclease) or a single-stranded break (Cas9 nickase).

**Table 22A**

| 1st Tier | | | | |
|---|---|---|---|---|
| gRNA Name | DNA Strand | Targeting Domain | Target Site Length | SEQ ID NO |
| MYOC-P370L-119 | + | GUCAAUGUCCGUGUAGCCACCCC | 23 | 3539 |
| MYOC-P370L-120 | + | GAACUGUCCGUGGUAGCCAGCUCC | 24 | 3541 |
| MYOC-P370L-121 | + | GCGCCCUGGAAAUAGAGGCUCC | 22 | 3543 |
| MYOC-P370L-122 | + | GCCUAGGCAGUAUGUGAACCUUAG | 24 | 3556 |
| MYOC-P370L-123 | + | GUUUGUUCGAGUUCCAGAUUCU | 22 | 3560 |
| MYOC-P370L-124 | + | GGUUUGUUCGAGUUCCAGAUUCU | 23 | 3561 |
| MYOC-P370L-125 | + | GAUGUUUGUCUCCCAGGUUUGU | 22 | 3566 |
| MYOC-P370L-126 | + | GGAUGUUUGUCUCCCAGGUUUGU | 23 | 3567 |
| MYOC-P370L-127 | - | GGAGCCUCUAUUUCCAGGGCG | 21 | 3594 |
| MYOC-P370L-128 | - | GGGAGCCUCUAUUUCCAGGGCG | 22 | 3595 |
| MYOC-P370L-129 | - | GGGGAGCCUCUAUUUCCAGGGCG | 23 | 3596 |
| MYOC-P370L-130 | - | GGCUGUGGAUGAAGCAGGCCU | 21 | 3601 |
| MYOC-P370L-131 | - | GCUACACGGACAUUGACUUGGCU | 23 | 3602 |
| MYOC-P370L-132 | - | GGCUACACGGACAUUGACUUGGCU | 24 | 3603 |

**Table 22B** provides exemplary targeting domains for correcting a mutation (e.g., P370L) in the MYOC gene selected according to the second tier parameters. The targeting domains bind within 200 bp from a mutation (e.g., P370L), have a high level of orthogonality and PAM is NNGRRT. It is contemplated herein that in an embodiment the targeting domain hybridizes to the target domain through complementary base pairing. Any of the targeting domains in the Table can be used with a *S. aureus* Cas9 molecule that generates a double stranded break (Cas9 nuclease) or a single-stranded break (Cas9 nickase).

**Table 22B**

| 2nd Tier | | | | |
|---|---|---|---|---|
| gRNA Name | DNA Strand | Targeting Domain | Target Site Length | SEQ ID NO |
| MYOC-P370L-133 | + | CAAUGUCCGUGUAGCCACCCC | 21 | 3634 |
| MYOC-P370L-134 | + | UCAAUGUCCGUGUAGCCACCCC | 22 | 3635 |
| MYOC-P370L-135 | + | AGUCAAUGUCCGUGUAGCCACCCC | 24 | 3636 |
| MYOC-P370L-136 | + | CUGUCCGUGGUAGCCAGCUCC | 21 | 3638 |
| MYOC-P370L-137 | + | ACUGUCCGUGGUAGCCAGCUCC | 22 | 3639 |
| MYOC-P370L-138 | + | AACUGUCCGUGGUAGCCAGCUCC | 23 | 3640 |
| MYOC-P370L-139 | + | CGCCCUGGAAAUAGAGGCUCC | 21 | 3643 |
| MYOC-P370L-140 | + | AGCGCCCUGGAAAUAGAGGCUCC | 23 | 3644 |
| MYOC-P370L-141 | + | CAGCGCCCUGGAAAUAGAGGCUCC | 24 | 3645 |
| MYOC-P370L-142 | + | UAGGCAGUAUGUGAACCUUAG | 21 | 3662 |
| MYOC-P370L-143 | + | CUAGGCAGUAUGUGAACCUUAG | 22 | 3663 |
| MYOC-P370L-144 | + | CCUAGGCAGUAUGUGAACCUUAG | 23 | 3664 |
| MYOC-P370L-145 | + | UUUGUUCGAGUUCCAGAUUCU | 21 | 3678 |
| MYOC-P370L-146 | + | AGGUUUGUUCGAGUUCCAGAUUCU | 24 | 3679 |
| MYOC-P370L-147 | + | AUGUUUGUCUCCCAGGUUUGU | 21 | 3690 |
| MYOC-P370L-148 | + | CGGAUGUUUGUCUCCCAGGUUUGU | 24 | 3691 |
| MYOC-P370L-149 | - | CUGCCUAGGCCACUGGAAAGC | 21 | 3729 |
| MYOC-P370L-150 | - | ACUGCCUAGGCCACUGGAAAGC | 22 | 3730 |
| MYOC-P370L-151 | - | UACUGCCUAGGCCACUGGAAAGC | 23 | 3731 |
| MYOC-P370L-152 | - | AUACUGCCUAGGCCACUGGAAAGC | 24 | 3732 |
| MYOC-P370L-153 | - | AGAACUGUCAUAAGAUAUGAG | 21 | 3769 |
| MYOC-P370L-154 | - | CAGAACUGUCAUAAGAUAUGAG | 22 | 3770 |
| MYOC-P370L-155 | - | CCAGAACUGUCAUAAGAUAUGAG | 23 | 3771 |
| MYOC-P370L-156 | - | UCCAGAACUGUCAUAAGAUAUGAG | 24 | 3772 |
| MYOC-P370L-157 | - | CGGGGAGCCUCUAUUUCCAGGGCG | 24 | 3780 |
| MYOC-P370L-158 | - | UGGCUGUGGAUGAAGCAGGCCU | 22 | 3800 |
| MYOC-P370L-159 | - | UUGGCUGUGGAUGAAGCAGGCCU | 23 | 3801 |
| MYOC-P370L-160 | - | CUUGGCUGUGGAUGAAGCAGGCCU | 24 | 3802 |
| MYOC-P370L-161 | - | UACACGGACAUUGACUUGGCU | 21 | 3805 |
| MYOC-P370L-162 | - | CUACACGGACAUUGACUUGGCU | 22 | 3806 |
| MYOC-P370L-163 | - | CACGGACAGUUCCCGUAUUCU | 21 | 3808 |
| MYOC-P370L-164 | - | CCACGGACAGUUCCCGUAUUCU | 22 | 3809 |
| MYOC-P370L-165 | - | ACCACGGACAGUUCCCGUAUUCU | 23 | 3810 |
| MYOC-P370L-166 | - | UACCACGGACAGUUCCCGUAUUCU | 24 | 3811 |

**Table 22C** provides exemplary targeting domains for correcting a mutation (e.g., P370L) in the MYOC gene selected according to the third tier parameters. The targeting domains bind within 200 bp from a mutation (e.g., P370L), start with a 5' G and PAM is NNGRRT. It is contemplated herein that in an embodiment the targeting domain hybridizes to the target domain through complementary base pairing. Any of the targeting domains in the Table can be used with a *S*. *aureus* Cas9 molecule that generates a double stranded break (Cas9 nuclease) or a single-stranded break (Cas9 nickase).

**Table 22C**

| 3rd Tier | | | | |
|---|---|---|---|---|
| gRNA Name | DNA Strand | Targeting Domain | Target Site Length | SEQ ID NO |
| MYOC-P370L-167 | + | GUCCGUGGUAGCCAGCUCC | 19 | 3540 |
| MYOC-P370L-168 | + | GCCCUGGAAAUAGAGGCUCC | 20 | 3542 |
| MYOC-P370L-169 | + | GCAGUAUGUGAACCUUAG | 18 | 3554 |
| MYOC-P370L-170 | + | GGCAGUAUGUGAACCUUAG | 19 | 3555 |
| MYOC-P370L-171 | + | GUUCGAGUUCCAGAUUCU | 18 | 3559 |
| MYOC-P370L-172 | + | GUUUGUCUCCCAGGUUUGU | 19 | 3565 |
| MYOC-P370L-173 | + | GCAUUGGCGACUGACUGCUU | 20 | 2793 |
| MYOC-P370L-174 | + | GGCAUUGGCGACUGACUGCUU | 21 | 3571 |
| MYOC-P370L-175 | + | GAAGGCAUUGGCGACUGACUGCUU | 24 | 3572 |
| MYOC-P370L-176 | - | GUCCUCUCCAAACUGAACCCA | 21 | 3573 |
| MYOC-P370L-177 | - | GCCUAGGCCACUGGAAAGC | 19 | 3579 |
| MYOC-P370L-178 | - | GAACUGUCAUAAGAUAUGAG | 20 | 1807 |
| MYOC-P370L-179 | - | GCCUCUAUUUCCAGGGCG | 18 | 3592 |
| MYOC-P370L-180 | - | GAGCCUCUAUUUCCAGGGCG | 20 | 3593 |
| MYOC-P370L-181 | - | GCUGUGGAUGAAGCAGGCCU | 20 | 1819 |
| MYOC-P370L-182 | - | GGACAGUUCCCGUAUUCU | 18 | 3604 |

**Table 22D** provides exemplary targeting domains for correcting a mutation (e.g., P370L) in the MYOC gene selected according to the fourth tier parameters. The targeting domains bind within 200 bp from a mutation (e.g., P370L), and PAM is NNGRRT. It is contemplated herein that in an embodiment the targeting domain hybridizes to the target domain through complementary base pairing. Any of the targeting domains in the Table can be used with a *S*. *aureus* Cas9 molecule that generates a double stranded break (Cas9 nuclease) or a single-stranded break (Cas9 nickase).

**Table 22D**

| 4th Tier | | | | |
|---|---|---|---|---|
| gRNA Name | DNA Strand | Targeting Domain | Target Site Length | SEQ ID NO |
| MYOC-P370L-183 | + | UGUCCGUGUAGCCACCCC | 18 | 3632 |
| MYOC-P370L-184 | + | AUGUCCGUGUAGCCACCCC | 19 | 3633 |
| MYOC-P370L-185 | + | AAUGUCCGUGUAGCCACCCC | 20 | 1824 |
| MYOC-P370L-186 | + | UCCGUGGUAGCCAGCUCC | 18 | 3637 |
| MYOC-P370L-63 | + | UGUCCGUGGUAGCCAGCUCC | 20 | 420 |
| MYOC-P370L-187 | + | CCUGGAAAUAGAGGCUCC | 18 | 3641 |
| MYOC-P370L-188 | + | CCCUGGAAAUAGAGGCUCC | 19 | 3642 |
| MYOC-P370L-189 | + | AGGCAGUAUGUGAACCUUAG | 20 | 3661 |
| MYOC-P370L-190 | + | UGUUCGAGUUCCAGAUUCU | 19 | 3676 |
| MYOC-P370L-191 | + | UUGUUCGAGUUCCAGAUUCU | 20 | 3677 |
| MYOC-P370L-192 | + | UUUGUCUCCCAGGUUUGU | 18 | 3689 |
| MYOC-P370L-193 | + | UGUUUGUCUCCCAGGUUUGU | 20 | 2792 |
| MYOC-P370L-194 | + | AUUGGCGACUGACUGCUU | 18 | 3695 |
| MYOC-P370L-195 | + | CAUUGGCGACUGACUGCUU | 19 | 3696 |
| MYOC-P370L-196 | + | AGGCAUUGGCGACUGACUGCUU | 22 | 3697 |
| MYOC-P370L-197 | + | AAGGCAUUGGCGACUGACUGCUU | 23 | 3698 |
| MYOC-P370L-198 | - | CUCUCCAAACUGAACCCA | 18 | 3699 |
| MYOC-P370L-199 | - | CCUCUCCAAACUGAACCCA | 19 | 3700 |
| MYOC-P370L-200 | - | UCCUCUCCAAACUGAACCCA | 20 | 3701 |
| MYOC-P370L-201 | - | UGUCCUCUCCAAACUGAACCCA | 22 | 3702 |
| MYOC-P370L-202 | - | UUGUCCUCUCCAAACUGAACCCA | 23 | 3703 |
| MYOC-P370L-203 | - | AUUGUCCUCUCCAAACUGAACCCA | 24 | 3704 |
| MYOC-P370L-204 | - | CCUAGGCCACUGGAAAGC | 18 | 3727 |
| MYOC-P370L-205 | - | UGCCUAGGCCACUGGAAAGC | 20 | 3728 |
| MYOC-P370L-206 | - | ACUGUCAUAAGAUAUGAG | 18 | 3767 |
| MYOC-P370L-207 | - | AACUGUCAUAAGAUAUGAG | 19 | 3768 |
| MYOC-P370L-208 | - | AGCCUCUAUUUCCAGGGCG | 19 | 3779 |
| MYOC-P370L-209 | - | UGUGGAUGAAGCAGGCCU | 18 | 3798 |
| MYOC-P370L-210 | - | CUGUGGAUGAAGCAGGCCU | 19 | 3799 |
| MYOC-P370L-211 | - | ACGGACAUUGACUUGGCU | 18 | 3803 |
| MYOC-P370L-212 | - | CACGGACAUUGACUUGGCU | 19 | 3804 |
| MYOC-P370L-213 | - | ACACGGACAUUGACUUGGCU | 20 | 1817 |
| MYOC-P370L-214 | - | CGGACAGUUCCCGUAUUCU | 19 | 3807 |
| MYOC-P370L-82 | - | ACGGACAGUUCCCGUAUUCU | 20 | 408 |

**Table 22E** provides exemplary targeting domains for correcting a mutation (e.g., P370L) in the MYOC gene selected according to the fifth tier parameters. The targeting domains bind within 200 bp from a mutation (e.g., P370L), and PAM is NNGRRV. It is contemplated herein that in an embodiment the targeting domain hybridizes to the target domain through complementary base pairing. Any of the targeting domains in the Table can be used with a *S*. *aureus* Cas9 molecule that generates a double stranded break (Cas9 nuclease) or a single-stranded break (Cas9 nickase).

**Table 22E**

| 5th Tier | | | | |
|---|---|---|---|---|
| gRNA Name | DNA Strand | Targeting Domain | Target Site | SEQ ID NO |
| MYOC-P370L-215 | + | GACUCAGCGCCCUGGAAA | 18 | 3848 |
| MYOC-P370L-216 | + | GGACUCAGCGCCCUGGAAA | 19 | 3849 |
| MYOC-P370L-217 | + | UGGACUCAGCGCCCUGGAAA | 20 | 3850 |
| MYOC-P370L-218 | + | CUGGACUCAGCGCCCUGGAAA | 21 | 3851 |
| MYOC-P370L-219 | + | UCUGGACUCAGCGCCCUGGAAA | 22 | 3852 |
| MYOC-P370L-220 | + | UUCUGGACUCAGCGCCCUGGAAA | 23 | 3853 |
| MYOC-P370L-221 | + | GUUCUGGACUCAGCGCCCUGGAAA | 24 | 3854 |
| MYOC-P370L-222 | + | CUCUGGGUUCAGUUUGGA | 18 | 3892 |
| MYOC-P370L-223 | + | UCUCUGGGUUCAGUUUGGA | 19 | 3893 |
| MYOC-P370L-224 | + | UUCUCUGGGUUCAGUUUGGA | 20 | 3894 |
| MYOC-P370L-225 | + | AUUCUCUGGGUUCAGUUUGGA | 21 | 3895 |
| MYOC-P370L-226 | + | GAUUCUCUGGGUUCAGUUUGGA | 22 | 3896 |
| MYOC-P370L-227 | + | AGAUUCUCUGGGUUCAGUUUGGA | 23 | 3897 |
| MYOC-P370L-228 | + | CAGAUUCUCUGGGUUCAGUUUGGA | 24 | 3898 |
| MYOC-P370L-229 | + | GUAGCCACCCCAAGAAUA | 18 | 3912 |
| MYOC-P370L-230 | + | UGUAGCCACCCCAAGAAUA | 19 | 3913 |
| MYOC-P370L-19 | + | GUGUAGCCACCCCAAGAAUA | 20 | 390 |
| MYOC-P370L-231 | + | CGUGUAGCCACCCCAAGAAUA | 21 | 3914 |
| MYOC-P370L-232 | + | CCGUGUAGCCACCCCAAGAAUA | 22 | 3915 |
| MYOC-P370L-233 | + | UCCGUGUAGCCACCCCAAGAAUA | 23 | 3916 |
| MYOC-P370L-234 | + | GUCCGUGUAGCCACCCCAAGAAUA | 24 | 3917 |
| MYOC-P370L-235 | + | UAGCCACCCCAAGAAUAC | 18 | 3944 |
| MYOC-P370L-236 | + | GUAGCCACCCCAAGAAUAC | 19 | 3945 |
| MYOC-P370L-58 | + | UGUAGCCACCCCAAGAAUAC | 20 | 418 |
| MYOC-P370L-237 | + | GUGUAGCCACCCCAAGAAUAC | 21 | 3946 |
| MYOC-P370L-238 | + | CGUGUAGCCACCCCAAGAAUAC | 22 | 3947 |
| MYOC-P370L-239 | + | CCGUGUAGCCACCCCAAGAAUAC | 23 | 3948 |
| MYOC-P370L-240 | + | UCCGUGUAGCCACCCCAAGAAUAC | 24 | 3949 |
| MYOC-P370L-241 | + | UCGGUGCUGUAAAUGACC | 18 | 3950 |
| MYOC-P370L-242 | + | AUCGGUGCUGUAAAUGACC | 19 | 3951 |
| MYOC-P370L-243 | + | CAUCGGUGCUGUAAAUGACC | 20 | 1825 |
| MYOC-P370L-244 | + | UCAUCGGUGCUGUAAAUGACC | 21 | 3952 |
| MYOC-P370L-245 | + | CUCAUCGGUGCUGUAAAUGACC | 22 | 3953 |
| MYOC-P370L-246 | + | CCUCAUCGGUGCUGUAAAUGACC | 23 | 3954 |
| MYOC-P370L-247 | + | GCCUCAUCGGUGCUGUAAAUGACC | 24 | 3955 |
| MYOC-P370L-248 | + | GUUCUGGACUCAGCGCCC | 18 | 3968 |
| MYOC-P370L-249 | + | AGUUCUGGACUCAGCGCCC | 19 | 3969 |
| MYOC-P370L-60 | + | CAGUUCUGGACUCAGCGCCC | 20 | 801 |
| MYOC-P370L-250 | + | ACAGUUCUGGACUCAGCGCCC | 21 | 3970 |
| MYOC-P370L-251 | + | GACAGUUCUGGACUCAGCGCCC | 22 | 3971 |
| MYOC-P370L-252 | + | UGACAGUUCUGGACUCAGCGCCC | 23 | 3972 |
| MYOC-P370L-253 | + | AUGACAGUUCUGGACUCAGCGCCC | 24 | 3973 |
| MYOC-P370L-254 | + | AGUUCUGGACUCAGCGCC | 18 | 3980 |
| MYOC-P370L-255 | + | CAGUUCUGGACUCAGCGCC | 19 | 3981 |
| MYOC-P370L-256 | + | ACAGUUCUGGACUCAGCGCC | 20 | 3982 |
| MYOC-P370L-257 | + | GACAGUUCUGGACUCAGCGCC | 21 | 3983 |
| MYOC-P370L-258 | + | UGACAGUUCUGGACUCAGCGCC | 22 | 3984 |
| MYOC-P370L-259 | + | AUGACAGUUCUGGACUCAGCGCC | 23 | 3985 |
| MYOC-P370L-260 | + | UAUGACAGUUCUGGACUCAGCGCC | 24 | 3986 |
| MYOC-P370L-261 | + | GUCCGUGGUAGCCAGCUC | 18 | 4071 |
| MYOC-P370L-262 | + | UGUCCGUGGUAGCCAGCUC | 19 | 4072 |
| MYOC-P370L-263 | + | CUGUCCGUGGUAGCCAGCUC | 20 | 1822 |
| MYOC-P370L-264 | + | ACUGUCCGUGGUAGCCAGCUC | 21 | 4073 |
| MYOC-P370L-265 | + | AACUGUCCGUGGUAGCCAGCUC | 22 | 4074 |
| MYOC-P370L-266 | + | GAACUGUCCGUGGUAGCCAGCUC | 23 | 4075 |
| MYOC-P370L-267 | + | GGAACUGUCCGUGGUAGCCAGCUC | 24 | 4076 |
| MYOC-P370L-268 | + | UUCUCUGGGUUCAGUUUG | 18 | 4197 |
| MYOC-P370L-269 | + | AUUCUCUGGGUUCAGUUUG | 19 | 4198 |
| MYOC-P370L-270 | + | GAUUCUCUGGGUUCAGUUUG | 20 | 4199 |
| MYOC-P370L-271 | + | AGAUUCUCUGGGUUCAGUUUG | 21 | 4200 |
| MYOC-P370L-272 | + | CAGAUUCUCUGGGUUCAGUUUG | 22 | 4201 |
| MYOC-P370L-273 | + | CCAGAUUCUCUGGGUUCAGUUUG | 23 | 4202 |
| MYOC-P370L-274 | + | UCCAGAUUCUCUGGGUUCAGUUUG | 24 | 4203 |
| MYOC-P370L-275 | + | UGUAGCCACCCCAAGAAU | 18 | 4211 |
| MYOC-P370L-276 | + | GUGUAGCCACCCCAAGAAU | 19 | 4212 |
| MYOC-P370L-277 | + | CGUGUAGCCACCCCAAGAAU | 20 | 1823 |
| MYOC-P370L-278 | + | CCGUGUAGCCACCCCAAGAAU | 21 | 4213 |
| MYOC-P370L-279 | + | UCCGUGUAGCCACCCCAAGAAU | 22 | 4214 |
| MYOC-P370L-280 | + | GUCCGUGUAGCCACCCCAAGAAU | 23 | 4215 |
| MYOC-P370L-281 | + | UGUCCGUGUAGCCACCCCAAGAAU | 24 | 4216 |
| MYOC-P370L-282 | + | CAGUGGCCUAGGCAGUAU | 18 | 4231 |
| MYOC-P370L-283 | + | CCAGUGGCCUAGGCAGUAU | 19 | 4232 |
| MYOC-P370L-284 | + | UCCAGUGGCCUAGGCAGUAU | 20 | 4233 |
| MYOC-P370L-285 | + | UUCCAGUGGCCUAGGCAGUAU | 21 | 4234 |
| MYOC-P370L-286 | + | UUUCCAGUGGCCUAGGCAGUAU | 22 | 4235 |
| MYOC-P370L-287 | + | CUUUCCAGUGGCCUAGGCAGUAU | 23 | 4236 |
| MYOC-P370L-288 | + | GCUUUCCAGUGGCCUAGGCAGUAU | 24 | 4237 |
| MYOC-P370L-289 | + | UAGGCAGUAUGUGAACCU | 18 | 4258 |
| MYOC-P370L-290 | + | CUAGGCAGUAUGUGAACCU | 19 | 4259 |
| MYOC-P370L-291 | + | CCUAGGCAGUAUGUGAACCU | 20 | 4260 |
| MYOC-P370L-292 | + | GCCUAGGCAGUAUGUGAACCU | 21 | 4261 |
| MYOC-P370L-293 | + | GGCCUAGGCAGUAUGUGAACCU | 22 | 4262 |
| MYOC-P370L-294 | + | UGGCCUAGGCAGUAUGUGAACCU | 23 | 4263 |
| MYOC-P370L-295 | + | GUGGCCUAGGCAGUAUGUGAACCU | 24 | 4264 |
| MYOC-P370L-296 | + | AGAUUCUCUGGGUUCAGU | 18 | 4290 |
| MYOC-P370L-297 | + | CAGAUUCUCUGGGUUCAGU | 19 | 4291 |
| MYOC-P370L-298 | + | CCAGAUUCUCUGGGUUCAGU | 20 | 4292 |
| MYOC-P370L-299 | + | UCCAGAUUCUCUGGGUUCAGU | 21 | 4293 |
| MYOC-P370L-300 | + | UUCCAGAUUCUCUGGGUUCAGU | 22 | 4294 |
| MYOC-P370L-301 | + | GUUCCAGAUUCUCUGGGUUCAGU | 23 | 4295 |
| MYOC-P370L-302 | + | AGUUCCAGAUUCUCUGGGUUCAGU | 24 | 4296 |
| MYOC-P370L-303 | + | UCAUAUCUUAUGACAGUU | 18 | 4337 |
| MYOC-P370L-304 | + | CUCAUAUCUUAUGACAGUU | 19 | 4338 |
| MYOC-P370L-305 | + | GCUCAUAUCUUAUGACAGUU | 20 | 1821 |
| MYOC-P370L-306 | + | AGCUCAUAUCUUAUGACAGUU | 21 | 4339 |
| MYOC-P370L-307 | + | CAGCUCAUAUCUUAUGACAGUU | 22 | 4340 |
| MYOC-P370L-308 | + | UCAGCUCAUAUCUUAUGACAGUU | 23 | 4341 |
| MYOC-P370L-309 | + | UUCAGCUCAUAUCUUAUGACAGUU | 24 | 4342 |
| MYOC-P370L-310 | + | GAUUCUCUGGGUUCAGUU | 18 | 4343 |
| MYOC-P370L-311 | + | AGAUUCUCUGGGUUCAGUU | 19 | 4344 |
| MYOC-P370L-118 | + | CAGAUUCUCUGGGUUCAGUU | 20 | 797 |
| MYOC-P370L-312 | + | CCAGAUUCUCUGGGUUCAGUU | 21 | 4345 |
| MYOC-P370L-313 | + | UCCAGAUUCUCUGGGUUCAGUU | 22 | 4346 |
| MYOC-P370L-314 | + | UUCCAGAUUCUCUGGGUUCAGUU | 23 | 4347 |
| MYOC-P370L-315 | + | GUUCCAGAUUCUCUGGGUUCAGUU | 24 | 4348 |
| MYOC-P370L-316 | - | GCCAUUGUCCUCUCCAAA | 18 | 4356 |
| MYOC-P370L-317 | - | UGCCAUUGUCCUCUCCAAA | 19 | 4357 |
| MYOC-P370L-318 | - | GUGCCAUUGUCCUCUCCAAA | 20 | 4358 |
| MYOC-P370L-319 | - | GGUGCCAUUGUCCUCUCCAAA | 21 | 4359 |
| MYOC-P370L-320 | - | AGGUGCCAUUGUCCUCUCCAAA | 22 | 4360 |
| MYOC-P370L-321 | - | AAGGUGCCAUUGUCCUCUCCAAA | 23 | 4361 |
| MYOC-P370L-322 | - | AAAGGUGCCAUUGUCCUCUCCAAA | 24 | 4362 |
| MYOC-P370L-323 | - | GAGCUGAAUACCGAGACA | 18 | 4389 |
| MYOC-P370L-324 | - | UGAGCUGAAUACCGAGACA | 19 | 4390 |
| MYOC-P370L-325 | - | AUGAGCUGAAUACCGAGACA | 20 | 1809 |
| MYOC-P370L-326 | - | UAUGAGCUGAAUACCGAGACA | 21 | 4391 |
| MYOC-P370L-327 | - | AUAUGAGCUGAAUACCGAGACA | 22 | 4392 |
| MYOC-P370L-328 | - | GAUAUGAGCUGAAUACCGAGACA | 23 | 4393 |
| MYOC-P370L-329 | - | AGAUAUGAGCUGAAUACCGAGACA | 24 | 4394 |
| MYOC-P370L-330 | - | CACAUACUGCCUAGGCCA | 18 | 4395 |
| MYOC-P370L-331 | - | UCACAUACUGCCUAGGCCA | 19 | 4396 |
| MYOC-P370L-332 | - | UUCACAUACUGCCUAGGCCA | 20 | 4397 |
| MYOC-P370L-333 | - | GUUCACAUACUGCCUAGGCCA | 21 | 4398 |
| MYOC-P370L-334 | - | GGUUCACAUACUGCCUAGGCCA | 22 | 4399 |
| MYOC-P370L-335 | - | AGGUUCACAUACUGCCUAGGCCA | 23 | 4400 |
| MYOC-P370L-336 | - | AAGGUUCACAUACUGCCUAGGCCA | 24 | 4401 |
| MYOC-P370L-337 | - | AGACAGUGAAGGCUGAGA | 18 | 4433 |
| MYOC-P370L-338 | - | GAGACAGUGAAGGCUGAGA | 19 | 4434 |
| MYOC-P370L-112 | - | CGAGACAGUGAAGGCUGAGA | 20 | 405 |
| MYOC-P370L-339 | - | CCGAGACAGUGAAGGCUGAGA | 21 | 4435 |
| MYOC-P370L-340 | - | ACCGAGACAGUGAAGGCUGAGA | 22 | 4436 |
| MYOC-P370L-341 | - | UACCGAGACAGUGAAGGCUGAGA | 23 | 4437 |
| MYOC-P370L-342 | - | AUACCGAGACAGUGAAGGCUGAGA | 24 | 4438 |
| MYOC-P370L-343 | - | UAAGAUAUGAGCUGAAUA | 18 | 4465 |
| MYOC-P370L-344 | - | AUAAGAUAUGAGCUGAAUA | 19 | 4466 |
| MYOC-P370L-345 | - | CAUAAGAUAUGAGCUGAAUA | 20 | 1808 |
| MYOC-P370L-346 | - | UCAUAAGAUAUGAGCUGAAUA | 21 | 4467 |
| MYOC-P370L-347 | - | GUCAUAAGAUAUGAGCUGAAUA | 22 | 4468 |
| MYOC-P370L-348 | - | UGUCAUAAGAUAUGAGCUGAAUA | 23 | 4469 |
| MYOC-P370L-349 | - | CUGUCAUAAGAUAUGAGCUGAAUA | 24 | 4470 |
| MYOC-P370L-350 | - | UCUGGAACUCGAACAAAC | 18 | 4478 |
| MYOC-P370L-351 | - | AUCUGGAACUCGAACAAAC | 19 | 4479 |
| MYOC-P370L-352 | - | AAUCUGGAACUCGAACAAAC | 20 | 4480 |
| MYOC-P370L-353 | - | GAAUCUGGAACUCGAACAAAC | 21 | 4481 |
| MYOC-P370L-354 | - | AGAAUCUGGAACUCGAACAAAC | 22 | 4482 |
| MYOC-P370L-355 | - | GAGAAUCUGGAACUCGAACAAAC | 23 | 4483 |
| MYOC-P370L-356 | - | AGAGAAUCUGGAACUCGAACAAAC | 24 | 4484 |
| MYOC-P370L-357 | - | ACCCAGAGAAUCUGGAAC | 18 | 4491 |
| MYOC-P370L-358 | - | AACCCAGAGAAUCUGGAAC | 19 | 4492 |
| MYOC-P370L-359 | - | GAACCCAGAGAAUCUGGAAC | 20 | 4493 |
| MYOC-P370L-360 | - | UGAACCCAGAGAAUCUGGAAC | 21 | 4494 |
| MYOC-P370L-361 | - | CUGAACCCAGAGAAUCUGGAAC | 22 | 4495 |
| MYOC-P370L-362 | - | ACUGAACCCAGAGAAUCUGGAAC | 23 | 4496 |
| MYOC-P370L-363 | - | AACUGAACCCAGAGAAUCUGGAAC | 24 | 4497 |
| MYOC-P370L-364 | - | ACAUACUGCCUAGGCCAC | 18 | 4505 |
| MYOC-P370L-365 | - | CACAUACUGCCUAGGCCAC | 19 | 4506 |
| MYOC-P370L-56 | - | UCACAUACUGCCUAGGCCAC | 20 | 755 |
| MYOC-P370L-366 | - | UUCACAUACUGCCUAGGCCAC | 21 | 4507 |
| MYOC-P370L-367 | - | GUUCACAUACUGCCUAGGCCAC | 22 | 4508 |
| MYOC-P370L-368 | - | GGUUCACAUACUGCCUAGGCCAC | 23 | 4509 |
| MYOC-P370L-369 | - | AGGUUCACAUACUGCCUAGGCCAC | 24 | 4510 |
| MYOC-P370L-370 | - | UAUUCUUGGGGUGGCUAC | 18 | 4517 |
| MYOC-P370L-371 | - | GUAUUCUUGGGGUGGCUAC | 19 | 4518 |
| MYOC-P370L-372 | - | CGUAUUCUUGGGGUGGCUAC | 20 | 1816 |
| MYOC-P370L-373 | - | CCGUAUUCUUGGGGUGGCUAC | 21 | 4519 |
| MYOC-P370L-374 | - | CCCGUAUUCUUGGGGUGGCUAC | 22 | 4520 |
| MYOC-P370L-375 | - | UCCCGUAUUCUUGGGGUGGCUAC | 23 | 4521 |
| MYOC-P370L-376 | - | UUCCCGUAUUCUUGGGGUGGCUAC | 24 | 4522 |
| MYOC-P370L-377 | - | ACGGGUGCUGUGGUGUAC | 18 | 4530 |
| MYOC-P370L-378 | - | CACGGGUGCUGUGGUGUAC | 19 | 4531 |
| MYOC-P370L-379 | - | GCACGGGUGCUGUGGUGUAC | 20 | 4532 |
| MYOC-P370L-380 | - | AGCACGGGUGCUGUGGUGUAC | 21 | 4533 |
| MYOC-P370L-381 | - | AAGCACGGGUGCUGUGGUGUAC | 22 | 4534 |
| MYOC-P370L-382 | - | AAAGCACGGGUGCUGUGGUGUAC | 23 | 4535 |
| MYOC-P370L-383 | - | GAAAGCACGGGUGCUGUGGUGUAC | 24 | 4536 |
| MYOC-P370L-384 | - | CUGGAACUCGAACAAACC | 18 | 4537 |
| MYOC-P370L-385 | - | UCUGGAACUCGAACAAACC | 19 | 4538 |
| MYOC-P370L-59 | - | AUCUGGAACUCGAACAAACC | 20 | 766 |
| MYOC-P370L-386 | - | AAUCUGGAACUCGAACAAACC | 21 | 4539 |
| MYOC-P370L-387 | - | GAAUCUGGAACUCGAACAAACC | 22 | 4540 |
| MYOC-P370L-388 | - | AGAAUCUGGAACUCGAACAAACC | 23 | 4541 |
| MYOC-P370L-389 | - | GAGAAUCUGGAACUCGAACAAACC | 24 | 4542 |
| MYOC-P370L-390 | - | UCCUCUCCAAACUGAACC | 18 | 4543 |
| MYOC-P370L-391 | - | GUCCUCUCCAAACUGAACC | 19 | 4544 |
| MYOC-P370L-392 | - | UGUCCUCUCCAAACUGAACC | 20 | 4545 |
| MYOC-P370L-393 | - | UUGUCCUCUCCAAACUGAACC | 21 | 4546 |
| MYOC-P370L-394 | - | AUUGUCCUCUCCAAACUGAACC | 22 | 4547 |
| MYOC-P370L-395 | - | CAUUGUCCUCUCCAAACUGAACC | 23 | 4548 |
| MYOC-P370L-396 | - | CCAUUGUCCUCUCCAAACUGAACC | 24 | 4549 |
| MYOC-P370L-397 | - | UCCCUGGAGCUGGCUACC | 18 | 4557 |
| MYOC-P370L-398 | - | AUCCCUGGAGCUGGCUACC | 19 | 4558 |
| MYOC-P370L-399 | - | AAUCCCUGGAGCUGGCUACC | 20 | 1814 |
| MYOC-P370L-400 | - | AAAUCCCUGGAGCUGGCUACC | 21 | 4559 |
| MYOC-P370L-401 | - | GAAAUCCCUGGAGCUGGCUACC | 22 | 4560 |
| MYOC-P370L-402 | - | GGAAAUCCCUGGAGCUGGCUACC | 23 | 4561 |
| MYOC-P370L-403 | - | AGGAAAUCCCUGGAGCUGGCUACC | 24 | 4562 |
| MYOC-P370L-404 | - | GGCUGAGAAGGAAAUCCC | 18 | 4581 |
| MYOC-P370L-405 | - | AGGCUGAGAAGGAAAUCCC | 19 | 4582 |
| MYOC-P370L-61 | - | AAGGCUGAGAAGGAAAUCCC | 20 | 406 |
| MYOC-P370L-406 | - | GAAGGCUGAGAAGGAAAUCCC | 21 | 4583 |
| MYOC-P370L-407 | - | UGAAGGCUGAGAAGGAAAUCCC | 22 | 4584 |
| MYOC-P370L-408 | - | GUGAAGGCUGAGAAGGAAAUCCC | 23 | 4585 |
| MYOC-P370L-409 | - | AGUGAAGGCUGAGAAGGAAAUCCC | 24 | 4586 |
| MYOC-P370L-410 | - | AGGCUGAGAAGGAAAUCC | 18 | 4593 |
| MYOC-P370L-411 | - | AAGGCUGAGAAGGAAAUCC | 19 | 4594 |
| MYOC-P370L-412 | - | GAAGGCUGAGAAGGAAAUCC | 20 | 1813 |
| MYOC-P370L-413 | - | UGAAGGCUGAGAAGGAAAUCC | 21 | 4595 |
| MYOC-P370L-414 | - | GUGAAGGCUGAGAAGGAAAUCC | 22 | 4596 |
| MYOC-P370L-415 | - | AGUGAAGGCUGAGAAGGAAAUCC | 23 | 4597 |
| MYOC-P370L-416 | - | CAGUGAAGGCUGAGAAGGAAAUCC | 24 | 4598 |
| MYOC-P370L-417 | - | AACUGAACCCAGAGAAUC | 18 | 4636 |
| MYOC-P370L-418 | - | AAACUGAACCCAGAGAAUC | 19 | 4637 |
| MYOC-P370L-65 | - | CAAACUGAACCCAGAGAAUC | 20 | 765 |
| MYOC-P370L-419 | - | CCAAACUGAACCCAGAGAAUC | 21 | 4638 |
| MYOC-P370L-420 | - | UCCAAACUGAACCCAGAGAAUC | 22 | 4639 |
| MYOC-P370L-421 | - | CUCCAAACUGAACCCAGAGAAUC | 23 | 4640 |
| MYOC-P370L-422 | - | UCUCCAAACUGAACCCAGAGAAUC | 24 | 4641 |
| MYOC-P370L-423 | - | GGGUGCUGUGGUGUACUC | 18 | 4648 |
| MYOC-P370L-424 | - | CGGGUGCUGUGGUGUACUC | 19 | 4649 |
| MYOC-P370L-66 | - | ACGGGUGCUGUGGUGUACUC | 20 | 760 |
| MYOC-P370L-425 | - | CACGGGUGCUGUGGUGUACUC | 21 | 4650 |
| MYOC-P370L-426 | - | GCACGGGUGCUGUGGUGUACUC | 22 | 4651 |
| MYOC-P370L-427 | - | AGCACGGGUGCUGUGGUGUACUC | 23 | 4652 |
| MYOC-P370L-428 | - | AAGCACGGGUGCUGUGGUGUACUC | 24 | 4653 |
| MYOC-P370L-429 | - | UUUCCAGGGCGCUGAGUC | 18 | 4666 |
| MYOC-P370L-430 | - | AUUUCCAGGGCGCUGAGUC | 19 | 4667 |
| MYOC-P370L-431 | - | UAUUUCCAGGGCGCUGAGUC | 20 | 4668 |
| MYOC-P370L-432 | - | CUAUUUCCAGGGCGCUGAGUC | 21 | 4669 |
| MYOC-P370L-433 | - | UCUAUUUCCAGGGCGCUGAGUC | 22 | 4670 |
| MYOC-P370L-434 | - | CUCUAUUUCCAGGGCGCUGAGUC | 23 | 4671 |
| MYOC-P370L-435 | - | CCUCUAUUUCCAGGGCGCUGAGUC | 24 | 4672 |
| MYOC-P370L-436 | - | CGGACAGUUCCCGUAUUC | 18 | 4679 |
| MYOC-P370L-437 | - | ACGGACAGUUCCCGUAUUC | 19 | 4680 |
| MYOC-P370L-438 | - | CACGGACAGUUCCCGUAUUC | 20 | 1815 |
| MYOC-P370L-439 | - | CCACGGACAGUUCCCGUAUUC | 21 | 4681 |
| MYOC-P370L-440 | - | ACCACGGACAGUUCCCGUAUUC | 22 | 4682 |
| MYOC-P370L-441 | - | UACCACGGACAGUUCCCGUAUUC | 23 | 4683 |
| MYOC-P370L-442 | - | CUACCACGGACAGUUCCCGUAUUC | 24 | 4684 |
| MYOC-P370L-443 | - | UCGGGGAGCCUCUAUUUC | 18 | 4699 |
| MYOC-P370L-444 | - | CUCGGGGAGCCUCUAUUUC | 19 | 4700 |
| MYOC-P370L-445 | - | ACUCGGGGAGCCUCUAUUUC | 20 | 4701 |
| MYOC-P370L-446 | - | UACUCGGGGAGCCUCUAUUUC | 21 | 4702 |
| MYOC-P370L-447 | - | GUACUCGGGGAGCCUCUAUUUC | 22 | 4703 |
| MYOC-P370L-448 | - | UGUACUCGGGGAGCCUCUAUUUC | 23 | 4704 |
| MYOC-P370L-449 | - | GUGUACUCGGGGAGCCUCUAUUUC | 24 | 4705 |
| MYOC-P370L-450 | - | GAGACAGUGAAGGCUGAG | 18 | 4756 |
| MYOC-P370L-451 | - | CGAGACAGUGAAGGCUGAG | 19 | 4757 |
| MYOC-P370L-452 | - | CCGAGACAGUGAAGGCUGAG | 20 | 1812 |
| MYOC-P370L-453 | - | ACCGAGACAGUGAAGGCUGAG | 21 | 4758 |
| MYOC-P370L-454 | - | UACCGAGACAGUGAAGGCUGAG | 22 | 4759 |
| MYOC-P370L-455 | - | AUACCGAGACAGUGAAGGCUGAG | 23 | 4760 |
| MYOC-P370L-456 | - | AAUACCGAGACAGUGAAGGCUGAG | 24 | 4761 |
| MYOC-P370L-457 | - | GGGUCAUUUACAGCACCG | 18 | 4782 |
| MYOC-P370L-458 | - | UGGGUCAUUUACAGCACCG | 19 | 4783 |
| MYOC-P370L-459 | - | CUGGGUCAUUUACAGCACCG | 20 | 1820 |
| MYOC-P370L-460 | - | UCUGGGUCAUUUACAGCACCG | 21 | 4784 |
| MYOC-P370L-461 | - | CUCUGGGUCAUUUACAGCACCG | 22 | 4785 |
| MYOC-P370L-462 | - | CCUCUGGGUCAUUUACAGCACCG | 23 | 4786 |
| MYOC-P370L-463 | - | GCCUCUGGGUCAUUUACAGCACCG | 24 | 4787 |
| MYOC-P370L-464 | - | GGUGCUGUGGUGUACUCG | 18 | 4788 |
| MYOC-P370L-465 | - | GGGUGCUGUGGUGUACUCG | 19 | 4789 |
| MYOC-P370L-72 | - | CGGGUGCUGUGGUGUACUCG | 20 | 761 |
| MYOC-P370L-466 | - | ACGGGUGCUGUGGUGUACUCG | 21 | 4790 |
| MYOC-P370L-467 | - | CACGGGUGCUGUGGUGUACUCG | 22 | 4791 |
| MYOC-P370L-468 | - | GCACGGGUGCUGUGGUGUACUCG | 23 | 4792 |
| MYOC-P370L-469 | - | AGCACGGGUGCUGUGGUGUACUCG | 24 | 4793 |
| MYOC-P370L-470 | - | AUACCGAGACAGUGAAGG | 18 | 4812 |
| MYOC-P370L-471 | - | AAUACCGAGACAGUGAAGG | 19 | 4813 |
| MYOC-P370L-472 | - | GAAUACCGAGACAGUGAAGG | 20 | 1810 |
| MYOC-P370L-473 | - | UGAAUACCGAGACAGUGAAGG | 21 | 4814 |
| MYOC-P370L-474 | - | CUGAAUACCGAGACAGUGAAGG | 22 | 4815 |
| MYOC-P370L-475 | - | GCUGAAUACCGAGACAGUGAAGG | 23 | 4816 |
| MYOC-P370L-476 | - | AGCUGAAUACCGAGACAGUGAAGG | 24 | 4817 |
| MYOC-P370L-477 | - | ACAUUGACUUGGCUGUGG | 18 | 4855 |
| MYOC-P370L-478 | - | GACAUUGACUUGGCUGUGG | 19 | 4856 |
| MYOC-P370L-479 | - | GGACAUUGACUUGGCUGUGG | 20 | 1818 |
| MYOC-P370L-480 | - | CGGACAUUGACUUGGCUGUGG | 21 | 4857 |
| MYOC-P370L-481 | - | ACGGACAUUGACUUGGCUGUGG | 22 | 4858 |
| MYOC-P370L-482 | - | CACGGACAUUGACUUGGCUGUGG | 23 | 4859 |
| MYOC-P370L-483 | - | ACACGGACAUUGACUUGGCUGUGG | 24 | 4860 |
| MYOC-P370L-484 | - | AAACUGAACCCAGAGAAU | 18 | 4925 |
| MYOC-P370L-485 | - | CAAACUGAACCCAGAGAAU | 19 | 4926 |
| MYOC-P370L-486 | - | CCAAACUGAACCCAGAGAAU | 20 | 4927 |
| MYOC-P370L-487 | - | UCCAAACUGAACCCAGAGAAU | 21 | 4928 |
| MYOC-P370L-488 | - | CUCCAAACUGAACCCAGAGAAU | 22 | 4929 |
| MYOC-P370L-489 | - | UCUCCAAACUGAACCCAGAGAAU | 23 | 4930 |
| MYOC-P370L-490 | - | CUCUCCAAACUGAACCCAGAGAAU | 24 | 4931 |
| MYOC-P370L-491 | - | CCAGAACUGUCAUAAGAU | 18 | 4945 |
| MYOC-P370L-492 | - | UCCAGAACUGUCAUAAGAU | 19 | 4946 |
| MYOC-P370L-493 | - | GUCCAGAACUGUCAUAAGAU | 20 | 1806 |
| MYOC-P370L-494 | - | AGUCCAGAACUGUCAUAAGAU | 21 | 4947 |
| MYOC-P370L-495 | - | GAGUCCAGAACUGUCAUAAGAU | 22 | 4948 |
| MYOC-P370L-496 | - | UGAGUCCAGAACUGUCAUAAGAU | 23 | 4949 |
| MYOC-P370L-497 | - | CUGAGUCCAGAACUGUCAUAAGAU | 24 | 4950 |
| MYOC-P370L-498 | - | CGGGUGCUGUGGUGUACU | 18 | 4972 |
| MYOC-P370L-499 | - | ACGGGUGCUGUGGUGUACU | 19 | 4973 |
| MYOC-P370L-78 | - | CACGGGUGCUGUGGUGUACU | 20 | 759 |
| MYOC-P370L-500 | - | GCACGGGUGCUGUGGUGUACU | 21 | 4974 |
| MYOC-P370L-501 | - | AGCACGGGUGCUGUGGUGUACU | 22 | 4975 |
| MYOC-P370L-502 | - | AAGCACGGGUGCUGUGGUGUACU | 23 | 4976 |
| MYOC-P370L-503 | - | AAAGCACGGGUGCUGUGGUGUACU | 24 | 4977 |
| MYOC-P370L-504 | - | UGGAACUCGAACAAACCU | 18 | 4978 |
| MYOC-P370L-505 | - | CUGGAACUCGAACAAACCU | 19 | 4979 |
| MYOC-P370L-79 | - | UCUGGAACUCGAACAAACCU | 20 | 767 |
| MYOC-P370L-506 | - | AUCUGGAACUCGAACAAACCU | 21 | 4980 |
| MYOC-P370L-507 | - | AAUCUGGAACUCGAACAAACCU | 22 | 4981 |
| MYOC-P370L-508 | - | GAAUCUGGAACUCGAACAAACCU | 23 | 4982 |
| MYOC-P370L-509 | - | AGAAUCUGGAACUCGAACAAACCU | 24 | 4983 |
| MYOC-P370L-510 | - | ACCGAGACAGUGAAGGCU | 18 | 5003 |
| MYOC-P370L-511 | - | UACCGAGACAGUGAAGGCU | 19 | 5004 |
| MYOC-P370L-512 | - | AUACCGAGACAGUGAAGGCU | 20 | 1811 |
| MYOC-P370L-513 | - | AAUACCGAGACAGUGAAGGCU | 21 | 5005 |
| MYOC-P370L-514 | - | GAAUACCGAGACAGUGAAGGCU | 22 | 5006 |
| MYOC-P370L-515 | - | UGAAUACCGAGACAGUGAAGGCU | 23 | 5007 |
| MYOC-P370L-516 | - | CUGAAUACCGAGACAGUGAAGGCU | 24 | 5008 |

**Table 23A** provides exemplary targeting domains for correcting a mutation (e.g., P370L) in the MYOC gene selected according to the third tier parameters. The targeting domains bind within 200 bp from a mutation (e.g., P370L) and start with a 5'G. It is contemplated herein that in an embodiment the targeting domain hybridizes to the target domain through complementary base pairing. Any of the targeting domains in the Table can be used with a *N. meningitidis* Cas9 molecule that generates a double stranded break (Cas9 nuclease) or a single-stranded break (Cas9 nickase).

**Table 23A**

| 3rd Tier | | | | |
|---|---|---|---|---|
| gRNA Name | DNA Strand | Targeting Domain | Target Site Length | SEQ ID NO |
| MYOC-P370L-517 | + | GUGCUGUAAAUGACCCAGAG | 20 | 5137 |

**Table 23B** provides exemplary targeting domains for correcting a mutation (e.g., P370L) in the MYOC gene selected according to the fourth tier parameters. The targeting domains bind within 200 bp from a mutation (e.g., P370L). It is contemplated herein that in an embodiment the targeting domain hybridizes to the target domain through complementary base pairing. Any of the targeting domains in the Table can be used with a *N*. *meningitidis* Cas9 molecule that generates a double stranded break (Cas9 nuclease) or a single-stranded break (Cas9 nickase).

**Table 23B**

| 4th Tier | | | | |
|---|---|---|---|---|
| gRNA Name | DNA Strand | Targeting Domain | Target Site Length | SEQ ID NO |
| MYOC-P370L-518 | + | CGAGUACACCACAGCAC | 17 | 5116 |
| MYOC-P370L-519 | + | UCCGUGGUAGCCAGCUC | 17 | 1842 |
| MYOC-P370L-520 | + | CUGUAAAUGACCCAGAG | 17 | 5120 |
| MYOC-P370L-521 | + | UGAAGGCAUUGGCGACU | 17 | 5124 |
| MYOC-P370L-522 | + | CCCAGGUUUGUUCGAGU | 17 | 2854 |
| MYOC-P370L-523 | + | CCCCGAGUACACCACAGCAC | 20 | 5133 |
| MYOC-P370L-263 | + | CUGUCCGUGGUAGCCAGCUC | 20 | 1822 |
| MYOC-P370L-524 | + | UGAUGAAGGCAUUGGCGACU | 20 | 5140 |
| MYOC-P370L-525 | + | UCUCCCAGGUUUGUUCGAGU | 20 | 2848 |

### III. Cas9 Molecules

Cas9 molecules of a variety of species can be used in the methods and compositions described herein. While the *S. pyogenes, S. aureus* and *S. thermophilus* Cas9 molecules are the subject of much of the disclosure herein, Cas9 molecules of, derived from, or based on the Cas9 proteins of other species listed herein can be used as well. In other words, while the much of the description herein uses *S. pyogenes* and *S. thermophilus* Cas9 molecules, Cas9 molecules from the other species can replace them, e.g., *Staphylococcus aureus* and *Neisseria meningitidis* Cas9 molecules. Additional Cas9 species include: *Acidovorax avenae, Actinobacillus pleuropneumoniae, Actinobacillus succinogenes, Actinobacillus suis, Actinomyces sp., cycliphilus denitrificans, Aminomonas paucivorans, Bacillus cereus, Bacillus smithii, Bacillus thuringiensis, Bacteroides sp., Blastopirellula marina, Bradyrhizobium sp., Brevibacillus laterosporus, Campylobacter coli, Campylobacter jejuni, Campylobacter lari, Candidatus Puniceispirillum, Clostridium cellulolyticum, Clostridium perfringens, Corynebacterium accolens, Corynebacterium diphtheria, Corynebacterium matruchotii, Dinoroseobacter shibae, Eubacterium dolichum, gamma proteobacterium, Gluconacetobacter diazotrophicus, Haemophilus parainfluenzae, Haemophilus sputorum, Helicobacter canadensis, Helicobacter cinaedi, Helicobacter mustelae, Ilyobacter polytropus, Kingella kingae, Lactobacillus crispatus, Listeria ivanovii, Listeria monocytogenes, Listeriaceae bacterium, Methylocystis sp., Methylosinus trichosporium, Mobiluncus mulieris, Neisseria bacilliformis, Neisseria cinerea, Neisseria flavescens, Neisseria lactamica, Neisseria meningitidis, Neisseria sp., Neisseria wadsworthii, Nitrosomonas sp., Parvibaculum lavamentivorans, Pasteurella multocida, Phascolarctobacterium succinatutens, Ralstonia syzygii, Rhodopseudomonas palustris, Rhodovulum sp., Simonsiella muelleri, Sphingomonas sp., Sporolactobacillus vineae, Staphylococcus lugdunensis, Streptococcus sp., Subdoligranulum sp., Tistrella mobilis, Treponema sp.,* or *Verminephrobacter eiseniae.*

A Cas9 molecule or Cas 9 polypeptide, as that term is used herein, refers to a molecule or polypeptide that can interact with a guide RNA (gRNA) molecule and, in concert with the gRNA molecule, home or localizes to a site which comprises a target domain and PAM sequence.
Cas9 molecule and Cas9 polypeptide, as those terms are used herein, refer to naturally occurring Cas9 molecules and to engineered, altered, or modified Cas9 molecules or Cas9 polypeptides that differ, e.g., by at least one amino acid residue, from a reference sequence, e.g., the most similar naturally occurring Cas9 molecule or a sequence of **Table 25.**

### Cas9 Domains

Crystal structures have been determined for two different naturally occurring bacterial Cas9 molecules (Jinek et al., Science, 343(6176): 1247997, 2014) and for *S. pyogenes* Cas9 with a guide RNA (e.g., a synthetic fusion of crRNA and tracrRNA) (Nishimasu et al., Cell, 156:935-949, 2014; and Anders et al., Nature, 2014, doi: 10.1038/nature13579).

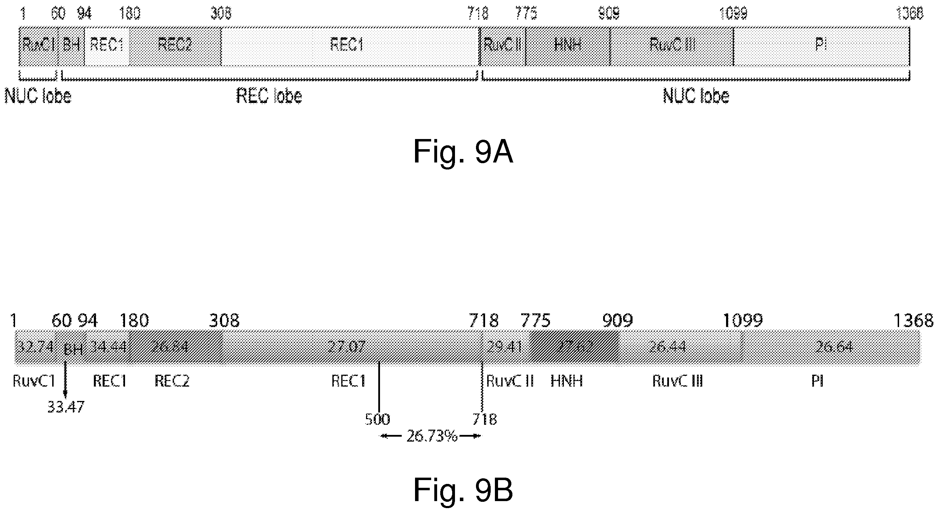

A naturally occurring Cas9 molecule comprises two lobes: a recognition (REC) lobe and a nuclease (NUC) lobe; each of which further comprises domains described herein. **Figs. 9A-9B** provide a schematic of the organization of important Cas9 domains in the primary structure. The domain nomenclature and the numbering of the amino acid residues encompassed by each domain used throughout this disclosure is as described in Nishimasu et al. The numbering of the amino acid residues is with reference to Cas9 from *S. pyogenes.*

The REC lobe comprises the arginine-rich bridge helix (BH), the REC1 domain, and the REC2 domain. The REC lobe does not share structural similarity with other known proteins, indicating that it is a Cas9-specific functional domain. The BH domain is a long α helix and arginine rich region and comprises amino acids 60-93 of the sequence of *S. pyogenes* Cas9. The REC1 domain is important for recognition of the repeat:anti-repeat duplex, e.g., of a gRNA or a tracrRNA, and is therefore critical for Cas9 activity by recognizing the target sequence. The REC1 domain comprises two REC1 motifs at amino acids 94 to 179 and 308 to 717 of the sequence of *S. pyogenes* Cas9. These two REC1 domains, though separated by the REC2 domain in the linear primary structure, assemble in the tertiary structure to form the REC1 domain. The REC2 domain, or parts thereof, may also play a role in the recognition of the repeat: anti-repeat duplex. The REC2 domain comprises amino acids 180-307 of the sequence of *S. pyogenes* Cas9.

The NUC lobe comprises the RuvC domain (also referred to herein as RuvC-like domain), the HNH domain (also referred to herein as HNH-like domain), and the PAM-interacting (PI) domain. The RuvC domain shares structural similarity to retroviral integrase superfamily members and cleaves a single strand, e.g., the non-complementary strand of the target nucleic acid molecule. The RuvC domain is assembled from the three split RuvC motifs (RuvC I, RuvCII, and RuvCIII, which are often commonly referred to in the art as RuvCI domain, or N-terminal RuvC domain, RuvCII domain, and RuvCIII domain) at amino acids 1-59, 718-769, and 909-1098, respectively, of the sequence of *S. pyogenes* Cas9. Similar to the REC1 domain, the three RuvC motifs are linearly separated by other domains in the primary structure, however in the tertiary structure, the three RuvC motifs assemble and form the RuvC domain. The HNH domain shares structural similarity with HNH endonucleases, and cleaves a single strand, e.g., the complementary strand of the target nucleic acid molecule. The HNH domain lies between the RuvC II-III motifs and comprises amino acids 775-908 of the sequence of *S. pyogenes* Cas9. The PI domain interacts with the PAM of the target nucleic acid molecule, and comprises amino acids 1099-1368 of the sequence of *S. pyogenes* Cas9.

### A RuvC-like domain and an HNH-like domain

In an embodiment, a Cas9 molecule or Cas9 polypeptide comprises an HNH-like domain and a RuvC-like domain. In an embodiment, cleavage activity is dependent on a RuvC-like domain and an HNH-like domain. A Cas9 molecule or Cas9 polypeptide, e.g., an eaCas9 molecule or eaCas9 polypeptide, can comprise one or more of the following domains: a RuvC-like domain and an HNH-like domain. In an embodiment, a Cas9 molecule or Cas9 polypeptide is an eaCas9 molecule or eaCas9 polypeptide and the eaCas9 molecule or eaCas9 polypeptide comprises a RuvC-like domain, e.g., a RuvC-like domain described below, and/or an HNH-like domain, e.g., an HNH-like domain described below.

### RuvC-like domains

In an embodiment, a RuvC-like domain cleaves, a single strand, e.g., the non-complementary strand of the target nucleic acid molecule. The Cas9 molecule or Cas9 polypeptide can include more than one RuvC-like domain (e.g., one, two, three or more RuvC-like domains). In an embodiment, a RuvC-like domain is at least 5, 6, 7, 8 amino acids in length but not more than 20, 19, 18, 17, 16 or 15 amino acids in length. In an embodiment, the Cas9 molecule or Cas9 polypeptide comprises an N-terminal RuvC-like domain of about 10 to 20 amino acids, e.g., about 15 amino acids in length.

### N-terminal RuvC-like domains

Some naturally occurring Cas9 molecules comprise more than one RuvC-like domain with cleavage being dependent on the N-terminal RuvC-like domain. Accordingly, Cas9 molecules or Cas9 polypeptide can comprise an N-terminal RuvC-like domain. Exemplary N-terminal RuvC-like domains are described below.

In an embodiment, an eaCas9 molecule or eaCas9 polypeptide comprises an N-terminal RuvC-like domain comprising an amino acid sequence of formula I:
D-X1-G-X2-X3-X4-X5-G-X6-X7-X8-X9 (SEQ ID NO: 8),
wherein,
X1 is selected from I, V, M, L and T (e.g., selected from I, V, and L);
X2 is selected from T, I, V, S, N, Y, E and L (e.g., selected from T, V, and I);
X3 is selected from N, S, G, A, D, T, R, M and F (e.g., A or N);
X4 is selected from S, Y, N and F (e.g., S);
X5 is selected from V, I, L, C, T and F (e.g., selected from V, I and L);
X6 is selected from W, F, V, Y, S and L (e.g., W);
X7 is selected from A, S, C, V and G (e.g., selected from A and S);
X8 is selected from V, I, L, A, M and H (e.g., selected from V, I, M and L); and
X9 is selected from any amino acid or is absent (e.g., selected from T, V, I, L, Δ, F, S, A, Y, M and R, or, e.g., selected from T, V, I, L and Δ).

In an embodiment, the N-terminal RuvC-like domain differs from a sequence of SEQ ID NO:8, by as many as 1 but no more than 2, 3, 4, or 5 residues.

In embodiment, the N-terminal RuvC-like domain is cleavage competent.

In embodiment, the N-terminal RuvC-like domain is cleavage incompetent.

In an embodiment, a eaCas9 molecule or eaCas9 polypeptide comprises an N-terminal RuvC-like domain comprising an amino acid sequence of formula II:
D-X1-G-X2-X3-S-X5-G-X6-X7-X8-X9, (SEQ ID NO: 9),
wherein
X1 is selected from I, V, M, L and T (e.g., selected from I, V, and L);
X2 is selected from T, I, V, S, N, Y, E and L (e.g., selected from T, V, and I);
X3 is selected from N, S, G, A, D, T, R, M and F (e.g., A or N);
X5 is selected from V, I, L, C, T and F (e.g., selected from V, I and L);
X6 is selected from W, F, V, Y, S and L (e.g., W);
X7 is selected from A, S, C, V and G (e.g., selected from A and S);
X8 is selected from V, I, L, A, M and H (e.g., selected from V, I, M and L); and
X9 is selected from any amino acid or is absent (e.g., selected from T, V, I, L, Δ, F, S, A, Y, M and R or selected from e.g., T, V, I, L and Δ).

In an embodiment, the N-terminal RuvC-like domain differs from a sequence of SEQ ID NO:9 by as many as 1 but no more than 2, 3, 4, or 5 residues.

In an embodiment, the N-terminal RuvC-like domain comprises an amino acid sequence of formula III:
D-I-G-X2-X3-S-V-G-W-A-X8-X9 (SEQ ID NO: 10),
wherein
X2 is selected from T, I, V, S, N, Y, E and L (e.g., selected from T, V, and I);
X3 is selected from N, S, G, A, D, T, R, M and F (e.g., A or N);
X8 is selected from V, I, L, A, M and H (e.g., selected from V, I, M and L); and
X9 is selected from any amino acid or is absent (e.g., selected from T, V, I, L, Δ, F, S, A, Y, M and R or selected from e.g., T, V, I, L and Δ).

In an embodiment, the N-terminal RuvC-like domain differs from a sequence of SEQ ID NO:10 by as many as 1 but no more than, 2, 3, 4, or 5 residues.

In an embodiment, the N-terminal RuvC-like domain comprises an amino acid sequence of formula III:
D-I-G-T-N-S-V-G-W-A-V-X (SEQ ID NO: 11),
wherein
X is a non-polar alkyl amino acid or a hydroxyl amino acid, e.g., X is selected from V, I, L and T (e.g., the eaCas9 molecule can comprise an N-terminal RuvC-like domain shown in **Figs. 2A-2G** (is depicted as Y)).

In an embodiment, the N-terminal RuvC-like domain differs from a sequence of SEQ ID NO:11 by as many as 1 but no more than, 2, 3, 4, or 5 residues.

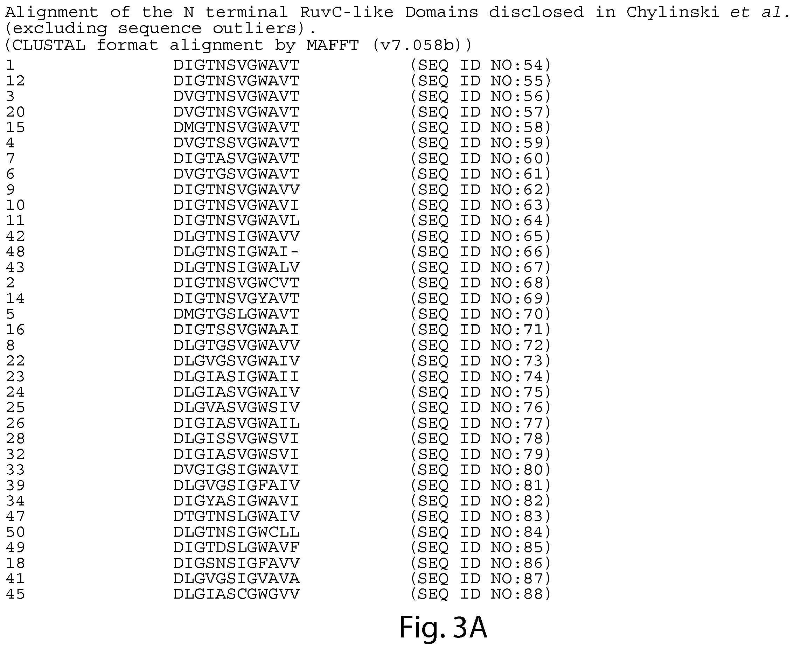

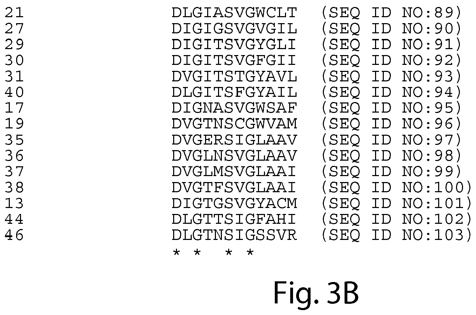

In an embodiment, the N-terminal RuvC-like domain differs from a sequence of an N-terminal RuvC like domain disclosed herein, e.g., in **Figs. 3A-3B** or **Figs. 7A-7B****,** as many as 1 but no more than 2, 3, 4, or 5 residues. In an embodiment, 1, 2, 3 or all of the highly conserved residues identified in **Figs. 3A-3B** or **Figs. 7A-7B** are present.

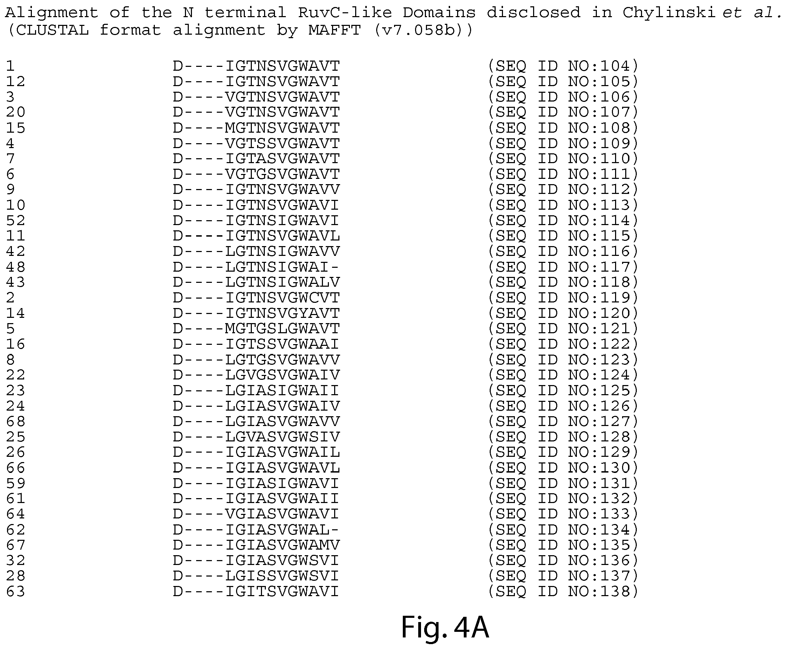

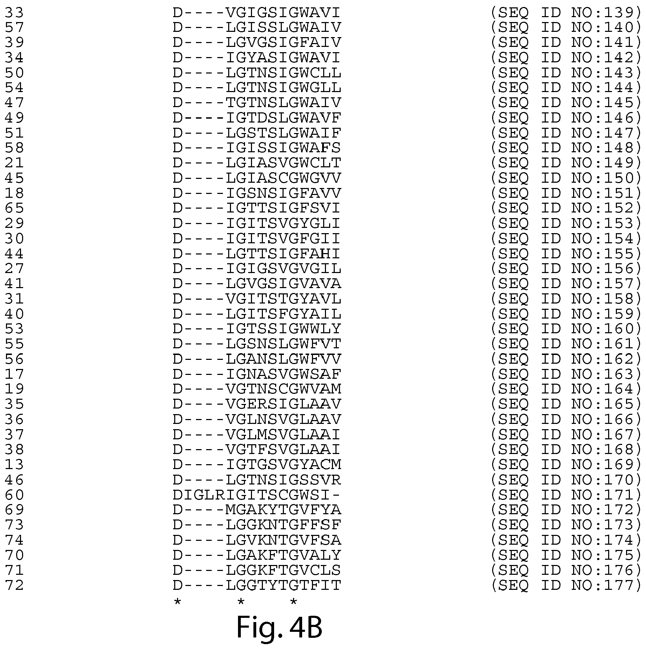

In an embodiment, the N-terminal RuvC-like domain differs from a sequence of an N-terminal RuvC-like domain disclosed herein, e.g., in **Figs. 4A-4B** or **Figs. 7A-7B****,** as many as 1 but no more than 2, 3, 4, or 5 residues. In an embodiment, 1, 2, or all of the highly conserved residues identified in **Figs. 4A-4B** or **Figs. 7A-7B** are present.

### Additional RuvC-like domains

In addition to the N-terminal RuvC-like domain, the Cas9 molecule or Cas9 polypeptide, e.g., an eaCas9 molecule or eaCas9 polypeptide, can comprise one or more additional RuvC-like domains. In an embodiment, the Cas9 molecule or Cas9 polypeptide can comprise two additional RuvC-like domains. Preferably, the additional RuvC-like domain is at least 5 amino acids in length and, e.g., less than 15 amino acids in length, e.g., 5 to 10 amino acids in length, e.g., 8 amino acids in length.

An additional RuvC-like domain can comprise an amino acid sequence:
I-X1-X2-E-X3-A-R-E (SEQ ID NO:12), wherein
X1 is V or H,
X2 is I, L or V (e.g., I or V); and
X3 is M or T.

In an embodiment, the additional RuvC-like domain comprises the amino acid sequence:
I-V-X2-E-M-A-R-E (SEQ ID NO:13), wherein
X2 is I, L or V (e.g., I or V) (e.g., the eaCas9 molecule or eaCas9 polypeptide can comprise an additional RuvC-like domain shown in **Fig. 2A-2G** or **Figs. 7A-7B** (depicted as B)).

An additional RuvC-like domain can comprise an amino acid sequence:
H-H-A-X1-D-A-X2-X3 (SEQ ID NO: 14), wherein
X1 is H or L;
X2 is R or V; and
X3 is E or V.

In an embodiment, the additional RuvC-like domain comprises the amino acid sequence: H-H-A-H-D-A-Y-L (SEQ ID NO:15).

In an embodiment, the additional RuvC-like domain differs from a sequence of SEQ ID NO: 12, 13, 14 or 15 by as many as 1 but no more than 2, 3, 4, or 5 residues.

In some embodiments, the sequence flanking the N-terminal RuvC-like domain is a sequences of formula V:
K-X1'-Y-X2'-X3'-X4'-Z-T-D-X9'-Y, (SEQ ID NO: 16).
wherein
X1' is selected from K and P,
X2' is selected from V, L, I, and F (e.g., V, I and L);
X3' is selected from G, A and S (e.g., G),
X4' is selected from L, I, V and F (e.g., L);
X9' is selected from D, E, N and Q; and
Z is an N-terminal RuvC-like domain, e.g., as described above.

### HNH-like domains

In an embodiment, an HNH-like domain cleaves a single stranded complementary domain, e.g., a complementary strand of a double stranded nucleic acid molecule. In an embodiment, an HNH-like domain is at least 15, 20, 25 amino acids in length but not more than 40, 35 or 30 amino acids in length, e.g., 20 to 35 amino acids in length, e.g., 25 to 30 amino acids in length. Exemplary HNH-like domains are described below.

In an embodiment, an eaCas9 molecule or eaCas9 polypeptide comprises an HNH-like domain having an amino acid sequence of formula VI:
X1-X2-X3-H-X4-X5-P-X6-X7-X8-X9-X10-X11-X12-X13-X14-X15-N-X16-X17-X18-X19-X20-X21-X22-X23-N (SEQ ID NO: 17), wherein
X1 is selected from D, E, Q and N (e.g., D and E);
X2 is selected from L, I, R, Q, V, M and K;
X3 is selected from D and E;
X4 is selected from I, V, T, A and L (e.g., A, I and V);
X5 is selected from V, Y, I, L, F and W (e.g., V, I and L);
X6 is selected from Q, H, R, K, Y, I, L, F and W;
X7 is selected from S, A, D, T and K (e.g., S and A);
X8 is selected from F, L, V, K, Y, M, I, R, A, E, D and Q (e.g., F);
X9 is selected from L, R, T, I, V, S, C, Y, K, F and G;
X10 is selected from K, Q, Y, T, F, L, W, M, A, E, G, and S;
X11 is selected from D, S, N, R, L and T (e.g., D);
X12 is selected from D, N and S;
X13 is selected from S, A, T, G and R (e.g., S);
X14 is selected from I, L, F, S, R, Y, Q, W, D, K and H (e.g., I, L and F);
X15 is selected from D, S, I, N, E, A, H, F, L, Q, M, G, Y and V;
X16 is selected from K, L, R, M, T and F (e.g., L, R and K);
X17 is selected from V, L, I, A and T;
X18 is selected from L, I, V and A (e.g., L and I);
X19 is selected from T, V, C, E, S and A (e.g., T and V);
X20 is selected from R, F, T, W, E, L, N, C, K, V, S, Q, I, Y, H and A;
X21 is selected from S, P, R, K, N, A, H, Q, G and L;
X22 is selected from D, G, T, N, S, K, A, I, E, L, Q, R and Y; and
X23 is selected from K, V, A, E, Y, I, C, L, S, T, G, K, M, D and F.

In an embodiment, a HNH-like domain differs from a sequence of SEQ ID NO: 17 by at least one but no more than, 2, 3, 4, or 5 residues.

In an embodiment, the HNH-like domain is cleavage competent.

In an embodiment, the HNH-like domain is cleavage incompetent.

In an embodiment, an eaCas9 molecule or eaCas9 polypeptide comprises an HNH-like domain comprising an amino acid sequence of formula VII:
X1-X2-X3-H-X4-X5-P-X6-S-X8-X9-X10-D-D-S-X14-X15-N-K-V-L-X19-X20-X21-X22-X23-N (SEQ ID NO: 18),
wherein
X1 is selected from D and E;
X2 is selected from L, I, R, Q, V, M and K;
X3 is selected from D and E;
X4 is selected from I, V, T, A and L (e.g., A, I and V);
X5 is selected from V, Y, I, L, F and W (e.g., V, I and L);
X6 is selected from Q, H, R, K, Y, I, L, F and W;
X8 is selected from F, L, V, K, Y, M, I, R, A, E, D and Q (e.g., F);
X9 is selected from L, R, T, I, V, S, C, Y, K, F and G;
X10 is selected from K, Q, Y, T, F, L, W, M, A, E, G, and S;
X14 is selected from I, L, F, S, R, Y, Q, W, D, K and H (e.g., I, L and F);
X15 is selected from D, S, I, N, E, A, H, F, L, Q, M, G, Y and V;
X19 is selected from T, V, C, E, S and A (e.g., T and V);
X20 is selected from R, F, T, W, E, L, N, C, K, V, S, Q, I, Y, H and A;
X21 is selected from S, P, R, K, N, A, H, Q, G and L;
X22 is selected from D, G, T, N, S, K, A, I, E, L, Q, R and Y; and
X23 is selected from K, V, A, E, Y, I, C, L, S, T, G, K, M, D and F.

In an embodiment, the HNH-like domain differs from a sequence of SEQ ID NO: 18 by 1, 2, 3, 4, or 5 residues.

In an embodiment, an eaCas9 molecule or eaCas9 polypeptide comprises an HNH-like domain comprising an amino acid sequence of formula VII:
X1-V-X3-H-I-V-P-X6-S-X8-X9-X10-D-D-S-X14-X15-N-K-V-L-T-X20-X21-X22-X23-N (SEQ ID NO:19),
wherein
X1 is selected from D and E;
X3 is selected from D and E;
X6 is selected from Q, H, R, K, Y, I, L and W;
X8 is selected from F, L, V, K, Y, M, I, R, A, E, D and Q (e.g., F);
X9 is selected from L, R, T, I, V, S, C, Y, K, F and G;
X10 is selected from K, Q, Y, T, F, L, W, M, A, E, G, and S;
X14 is selected from I, L, F, S, R, Y, Q, W, D, K and H (e.g., I, L and F);
X15 is selected from D, S, I, N, E, A, H, F, L, Q, M, G, Y and V;
X20 is selected from R, F, T, W, E, L, N, C, K, V, S, Q, I, Y, H and A;
X21 is selected from S, P, R, K, N, A, H, Q, G and L;
X22 is selected from D, G, T, N, S, K, A, I, E, L, Q, R and Y; and
X23 is selected from K, V, A, E, Y, I, C, L, S, T, G, K, M, D and F.

In an embodiment, the HNH-like domain differs from a sequence of SEQ ID NO: 19 by 1, 2, 3, 4, or 5 residues.

In an embodiment, an eaCas9 molecule or eaCas9 polypeptide comprises an HNH-like domain having an amino acid sequence of formula VIII:
D-X2-D-H-I-X5-P-Q-X7-F-X9-X10-D-X12-S-I-D-N-X16-V-L-X19-X20-S-X22-X23-N (SEQ ID NO:20),
wherein
X2 is selected from I and V;
X5 is selected from I and V;
X7 is selected from A and S;
X9 is selected from I and L;
X10 is selected from K and T;
X12 is selected from D and N;
X16 is selected from R, K and L; X19 is selected from T and V;
X20 is selected from S and R;
X22 is selected from K, D and A; and
X23 is selected from E, K, G and N (e.g., the eaCas9 molecule or eaCas9 polypeptide can comprise an HNH-like domain as described herein).

In an embodiment, the HNH-like domain differs from a sequence of SEQ ID NO: 20 by as many as 1 but no more than 2, 3, 4, or 5 residues.

In an embodiment, an eaCas9 molecule or eaCas9 polypeptide comprises the amino acid sequence of formula IX:
L-Y-Y-L-Q-N-G-X1'-D-M-Y-X2'-X3'-X4'-X5'-L-D-I-X6'-X7'-L-S-X8'-Y-Z-N-R-X9'-K-X10'-D-X11'-V-P (SEQ ID NO: 21),
wherein
X1' is selected from K and R;
X2' is selected from V and T;
X3' is selected from G and D;
X4' is selected from E, Q and D;
X5' is selected from E and D;
X6' is selected from D, N and H;
X7' is selected from Y, R and N;
X8' is selected from Q, D and N; X9' is selected from G and E;
X10' is selected from S and G;
X11' is selected from D and N; and
Z is an HNH-like domain, e.g., as described above.

In an embodiment, the eaCas9 molecule or eaCas9 polypeptide comprises an amino acid sequence that differs from a sequence of SEQ ID NO:21 by as many as 1 but no more than 2, 3, 4, or 5 residues.

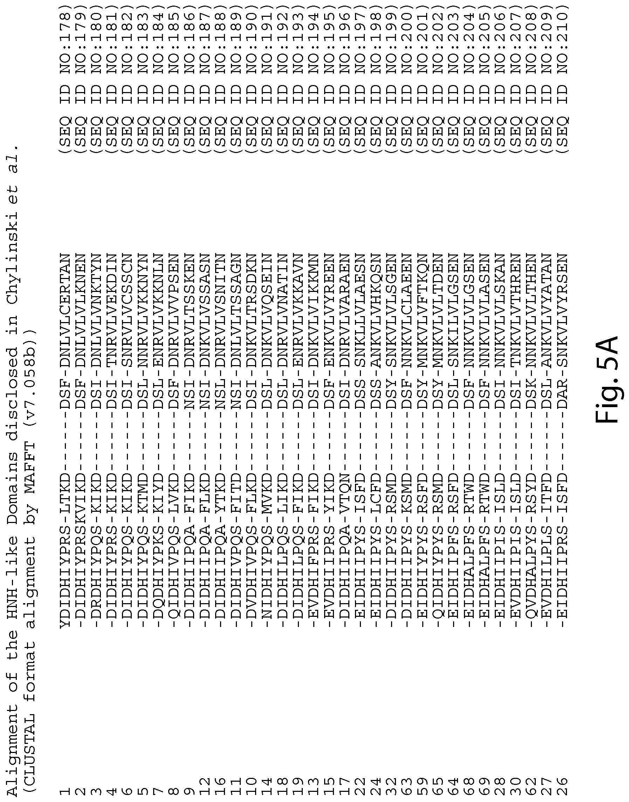

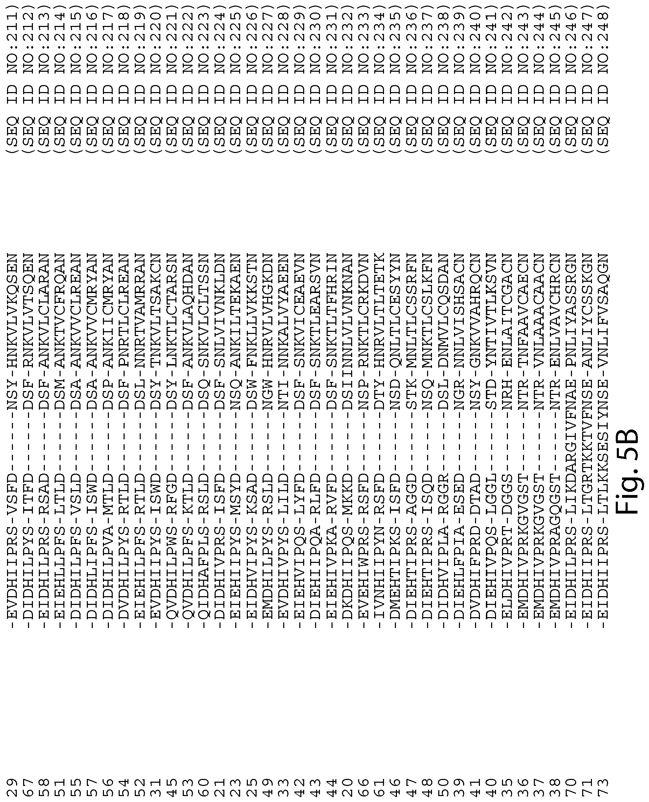

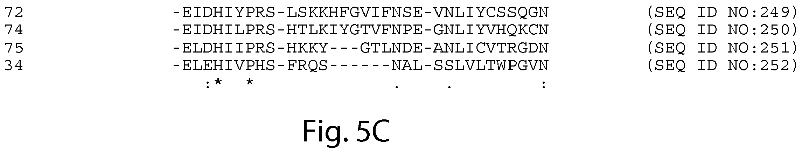

In an embodiment, the HNH-like domain differs from a sequence of an HNH-like domain disclosed herein, e.g., in **Figs. 5A-5C** or **Figs. 7A-7B****,** as many as 1 but no more than 2, 3, 4, or 5 residues. In an embodiment, 1 or both of the highly conserved residues identified in **Figs. 5A-5C** or **Figs. 7A-7B** are present.

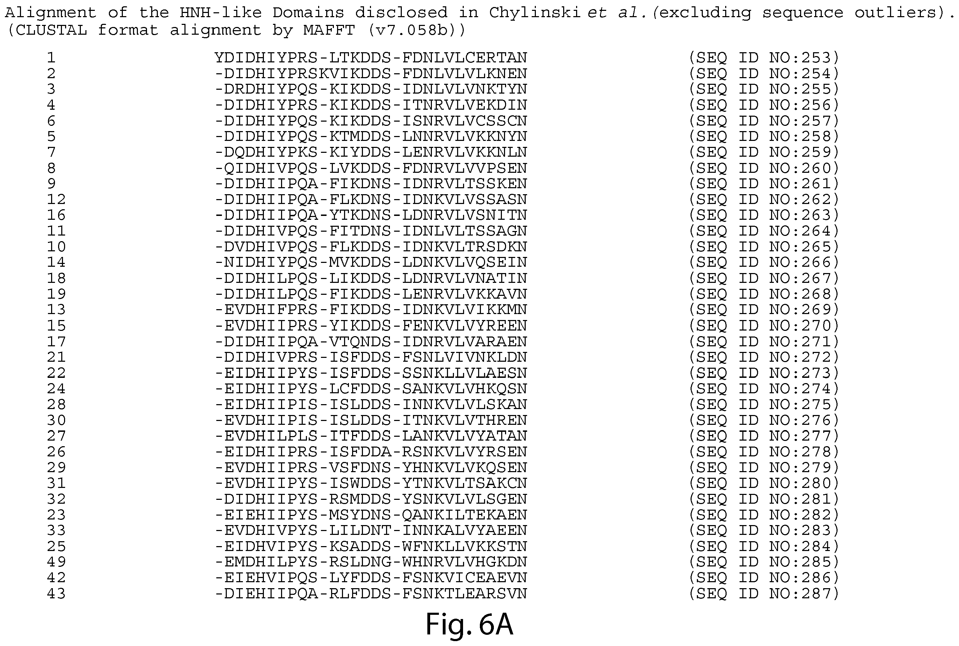

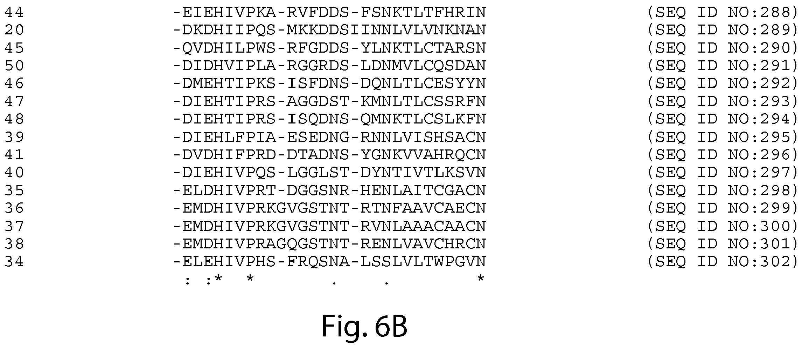

In an embodiment, the HNH -like domain differs from a sequence of an HNH-like domain disclosed herein, e.g., in **Figs. 6A-6B** or **Figs. 7A-7B****,** as many as 1 but no more than 2, 3, 4, or 5 residues. In an embodiment, 1, 2, all 3 of the highly conserved residues identified in **Figs. 6A-6B** or **Figs. 7A-7B** are present.

### Cas9 Activities

### Nuclease and Helicase Activities

In an embodiment, the Cas9 molecule or Cas9 polypeptide is capable of cleaving a target nucleic acid molecule. Typically wild type Cas9 molecules cleave both strands of a target nucleic acid molecule. Cas9 molecules and Cas9 polypeptides can be engineered to alter nuclease cleavage (or other properties), e.g., to provide a Cas9 molecule or Cas9 peolypeptide which is a nickase, or which lacks the ability to cleave target nucleic acid. A Cas9 molecule or Cas9 polypeptide that is capable of cleaving a target nucleic acid molecule is referred to herein as an eaCas9 (an enzymatically active Cas9) molecule or eaCas9 polypeptide. In an embodiment, an eaCas9 molecule or eaCas9 polypeptide, comprises one or more of the following activities:
a nickase activity, i.e., the ability to cleave a single strand, e.g., the non-complementary strand or the complementary strand, of a nucleic acid molecule;
a double stranded nuclease activity, i.e., the ability to cleave both strands of a double stranded nucleic acid and create a double stranded break, which in an embodiment is the presence of two nickase activities;
an endonuclease activity;
an exonuclease activity; and
a helicase activity, i.e., the ability to unwind the helical structure of a double stranded nucleic acid.

In an embodiment, an enzymatically active Cas9 or an eaCas9 molecule or an eacas9 polypeptide cleaves both DNA strands and results in a double stranded break. In an embodiment, an eaCas9 molecule or eaCas9 polypeptide cleaves only one strand, e.g., the strand to which the gRNA hybridizes to, or the strand complementary to the strand the gRNA hybridizes with. In an embodiment, an eaCas9 molecule or eaCas9 polypeptide comprises cleavage activity associated with an HNH-like domain. In an embodiment, an eaCas9 molecule or eaCas9 polypeptide comprises cleavage activity associated with an N-terminal RuvC-like domain. In an embodiment, an eaCas9 molecule or eaCas9 polypeptide comprises cleavage activity associated with an HNH-like domain and cleavage activity associated with an N-terminal RuvC-like domain. In an embodiment, an eaCas9 molecule or eaCas9 polypeptide comprises an active, or cleavage competent, HNH-like domain and an inactive, or cleavage incompetent, N-terminal RuvC-like domain. In an embodiment, an eaCas9 molecule or eaCas9 polypeptide comprises an inactive, or cleavage incompetent, HNH-like domain and an active, or cleavage competent, N-terminal RuvC-like domain. Some Cas9 molecules or Cas9 polypeptides have the ability to interact with a gRNA molecule, and in conjunction with the gRNA molecule localize to a core target domain, but are incapable of cleaving the target nucleic acid, or incapable of cleaving at efficient rates. Cas9 molecules having no, or no substantial, cleavage activity are referred to herein as an eiCas9 molecule or eiCas9 polypeptide. For example, an eiCas9 molecule or eiCas9 polypeptide can lack cleavage activity or have substantially less, e.g., less than 20, 10, 5, 1 or 0.1 % of the cleavage activity of a reference Cas9 molecule or eiCas9 polypeptide, as measured by an assay described herein.

### Targeting and PAMs

A Cas9 molecule or Cas9 polypeptide, is a polypeptide that can interact with a guide RNA (gRNA) molecule and, in concert with the gRNA molecule, localizes to a site which comprises a target domain and PAM sequence.

In an embodiment, the ability of an eaCas9 molecule or eaCas9 polypeptide to interact with and cleave a target nucleic acid is PAM sequence dependent. A PAM sequence is a sequence in the target nucleic acid. In an embodiment, cleavage of the target nucleic acid occurs upstream from the PAM sequence. EaCas9 molecules from different bacterial species can recognize different sequence motifs (e.g., PAM sequences). In an embodiment, an eaCas9 molecule of *S. pyogenes* recognizes the sequence motif NGG and directs cleavage of a target nucleic acid sequence 1 to 10, e.g., 3 to 5, base pairs upstream from that sequence. See, e.g., Mali et al., SCIENCE 2013; 339(6121): 823-826. In an embodiment, an eaCas9 molecule of *S. thermophilus* recognizes the sequence motif NGGNG and NNAGAAW (W = A or T) and directs cleavage of a core target nucleic acid sequence 1 to 10, e.g., 3 to 5, base pairs upstream from these sequences. See, e.g., Horvath et al., SCIENCE 2010; 327(5962):167-170, and Deveau et al., J BACTERIOL 2008; 190(4): 1390-1400. In an embodiment, an eaCas9 molecule of *S. mutans* recognizes the sequence motif NGG and/or NAAR (R = A or G) and directs cleavage of a core target nucleic acid sequence 1 to 10, e.g., 3 to 5 base pairs, upstream from this sequence. See, e.g., Deveau et al., J BACTERIOL 2008; 190(4): 1390-1400. In an embodiment, an eaCas9 molecule of *S. aureus* recognizes the sequence motif NNGRR (R = A or G) and directs cleavage of a target nucleic acid sequence 1 to 10, e.g., 3 to 5, base pairs upstream from that sequence. In an embodiment, an eaCas9 molecule of *S. aureus* recognizes the sequence motif NNGRRN (R = A or G) and directs cleavage of a target nucleic acid sequence 1 to 10, e.g., 3 to 5, base pairs upstream from that sequence. In an embodiment, an eaCas9 molecule of *S. aureus* recognizes the sequence motif NNGRRT (R = A or G) and directs cleavage of a target nucleic acid sequence 1 to 10, e.g., 3 to 5, base pairs upstream from that sequence. In an embodiment, an eaCas9 molecule of *S. aureus* recognizes the sequence motif NNGRRV (R = A or G, V = A, G or C) and directs cleavage of a target nucleic acid sequence 1 to 10, e.g., 3 to 5, base pairs upstream from that sequence. In an embodiment, an eaCas9 molecule of *Neisseria meningitidis* recognizes the sequence motif NNNNGATT or NNNGCTT and directs cleavage of a target nucleic acid sequence 1 to 10, e.g., 3 to 5, base pairs upstream from that sequence. See, e.g., Hou et al., PNAS Early Edition 2013, 1-6. The ability of a Cas9 molecule to recognize a PAM sequence can be determined, e.g., using a transformation assay described in Jinek et al., SCIENCE 2012 337:816. In the aforementioned embodiments, N can be any nucleotide residue, e.g., any of A, G, C or T.

As is discussed herein, Cas9 molecules can be engineered to alter the PAM specificity of the Cas9 molecule.

Exemplary naturally occurring Cas9 molecules are described in Chylinski et al., RNA BIOLOGY 2013 10:5, 727-737. Such Cas9 molecules include Cas9 molecules of a cluster 1 bacterial family, cluster 2 bacterial family, cluster 3 bacterial family, cluster 4 bacterial family, cluster 5 bacterial family, cluster 6 bacterial family, a cluster 7 bacterial family, a cluster 8 bacterial family, a cluster 9 bacterial family, a cluster 10 bacterial family, a cluster 11 bacterial family, a cluster 12 bacterial family, a cluster 13 bacterial family, a cluster 14 bacterial family, a cluster 15 bacterial family, a cluster 16 bacterial family, a cluster 17 bacterial family, a cluster 18 bacterial family, a cluster 19 bacterial family, a cluster 20 bacterial family, a cluster 21 bacterial family, a cluster 22 bacterial family, a cluster 23 bacterial family, a cluster 24 bacterial family, a cluster 25 bacterial family, a cluster 26 bacterial family, a cluster 27 bacterial family, a cluster 28 bacterial family, a cluster 29 bacterial family, a cluster 30 bacterial family, a cluster 31 bacterial family, a cluster 32 bacterial family, a cluster 33 bacterial family, a cluster 34 bacterial family, a cluster 35 bacterial family, a cluster 36 bacterial family, a cluster 37 bacterial family, a cluster 38 bacterial family, a cluster 39 bacterial family, a cluster 40 bacterial family, a cluster 41 bacterial family, a cluster 42 bacterial family, a cluster 43 bacterial family, a cluster 44 bacterial family, a cluster 45 bacterial family, a cluster 46 bacterial family, a cluster 47 bacterial family, a cluster 48 bacterial family, a cluster 49 bacterial family, a cluster 50 bacterial family, a cluster 51 bacterial family, a cluster 52 bacterial family, a cluster 53 bacterial family, a cluster 54 bacterial family, a cluster 55 bacterial family, a cluster 56 bacterial family, a cluster 57 bacterial family, a cluster 58 bacterial family, a cluster 59 bacterial family, a cluster 60 bacterial family, a cluster 61 bacterial family, a cluster 62 bacterial family, a cluster 63 bacterial family, a cluster 64 bacterial family, a cluster 65 bacterial family, a cluster 66 bacterial family, a cluster 67 bacterial family, a cluster 68 bacterial family, a cluster 69 bacterial family, a cluster 70 bacterial family, a cluster 71 bacterial family, a cluster 72 bacterial family, a cluster 73 bacterial family, a cluster 74 bacterial family, a cluster 75 bacterial family, a cluster 76 bacterial family, a cluster 77 bacterial family, or a cluster 78 bacterial family.

Exemplary naturally occurring Cas9 molecules include a Cas9 molecule of a cluster 1 bacterial family. Examples include a Cas9 molecule of: *S. pyogenes* (e.g., strain SF370, MGAS10270, MGAS10750, MGAS2096, MGAS315, MGAS5005, MGAS6180, MGAS9429, NZ131 and SSI-1), *S. thermophilus* (e.g., strain LMD-9), *S. pseudoporcinus* (e.g., strain SPIN 20026), *S. mutans* (e.g., strain UA159, NN2025), *S. macacae* (e.g., strain NCTC11558), *S. gallolyticus* (e.g., strain UCN34, ATCC BAA-2069), *S. equines* (e.g., strain ATCC 9812, MGCS 124), *S. dysdalactiae* (e.g., strain GGS 124), *S. bovis* (e.g., strain ATCC 700338), *S. anginosus* (e.g., strain F0211), *S. agalactiae* (e.g., strain NEM316, A909), *Listeria monocytogenes* (e.g., strain F6854), *Listeria innocua* (*L. innocua,* e.g., strain Clip11262), *Enterococcus italicus* (e.g., strain DSM 15952), or *Enterococcus faecium* (e.g., strain 1,231,408). Additional exemplary Cas9 molecules are a Cas9 molecule of *Neisseria meningitidis* (Hou et al., PNAS Early Edition 2013, 1-6 and a *S. aureus* cas9 molecule.

In an embodiment, a Cas9 molecule or Cas9 polypeptide, e.g., an eaCas9 molecule or eaCas9 polypeptide, comprises an amino acid sequence:
having 60%, 65%, 70%, 75%, 80%, 85%, 90%, 95%, 96%, 97%, 98% or 99% homology with;
differs at no more than, 2, 5, 10, 15, 20, 30, or 40% of the amino acid residues when compared with;
differs by at least 1, 2, 5, 10 or 20 amino acids, but by no more than 100, 80, 70, 60, 50, 40 or 30 amino acids from; or
is identical to any Cas9 molecule sequence described herein, or a naturally occurring Cas9 molecule sequence, e.g., a Cas9 molecule from a species listed herein or described in Chylinski et al., RNA BIOLOGY 2013 10:5, 727-737; Hou et al., PNAS Early Edition 2013, 1-6; SEQ ID NO:1-4. In an embodiment, the Cas9 molecule or Cas9 polypeptide comprises one or more of the following activities: a nickase activity; a double stranded cleavage activity (e.g., an endonuclease and/or exonuclease activity); a helicase activity; or the ability, together with a gRNA molecule, to localize to a target nucleic acid.

In an embodiment, a Cas9 molecule or Cas9 polypeptide comprises any of the amino acid sequence of the consensus sequence of **Figs. 2A-2G****,** wherein "*" indicates any amino acid found in the corresponding position in the amino acid sequence of a Cas9 molecule or Cas9 polypeptide of *S. pyogenes, S. thermophilus, S. mutans* and *L*. *innocua,* and "-" indicates any amino acid. In an embodiment, a Cas9 molecule differs from the sequence of the consensus sequence of **Figs. 2A-2G** by at least 1, but no more than 2, 3, 4, 5, 6, 7, 8, 9, or 10 amino acid residues. In an embodiment, a Cas9 molecule or Cas9 polypeptide comprises the amino acid sequence of SEQ ID NO:7 of **Figs. 7A-7B****,** wherein "*" indicates any amino acid found in the corresponding position in the amino acid sequence of a Cas9 molecule or Cas9 polypeptide of *S. pyogenes, or N. meningitidis,* "-" indicates any amino acid, and "-" indicates any amino acid or absent. In an embodiment, a Cas9 molecule or Cas9 polypeptide differs from the sequence of SEQ ID NO:6 or 7 disclosed in **Figs. 7A-7B** by at least 1, but no more than 2, 3, 4, 5, 6, 7, 8, 9, or 10 amino acid residues.

A comparison of the sequence of a number of Cas9 molecules indicate that certain regions are conserved. These are identified below as:
region 1 (residues1 to 180, or in the case of region 1'residues 120 to 180)
region 2 (residues360 to 480);
region 3 (residues 660 to 720);
region 4 (residues 817 to 900); and
region 5 (residues 900 to 960);

In an embodiment, a Cas9 molecule or Cas9 polypeptide comprises regions 1-5, together with sufficient additional Cas9 molecule sequence to provide a biologically active molecule, e.g., a Cas9 molecule having at least one activity described herein. In an embodiment, each of regions 1-5, independently, have 50%, 60%, 70%, 80%, 85%, 90%, 95%, 96%, 97%, 98% or 99% homology with the corresponding residues of a Cas9 molecule or Cas9 polypeptide described herein, e.g., a sequence from **Figs. 2A-2G** or from **Figs. 7A-****7B.**

In an embodiment, a Cas9 molecule or Cas9 polypeptide, e.g., an eaCas9 molecule or eaCas9 polypeptide, comprises an amino acid sequence referred to as region 1:
having 50%, 60%, 70%, 80%, 85%, 90%, 95%, 96%, 97%, 98% or 99% homology with amino acids 1-180 (the numbering is according to the motif sequence in **Figs. 2A-2G****;** 52% of residues in the four Cas9 sequences in **Figs. 2A-2G** are conserved) of the amino acid sequence of Cas9 of *S. pyogenes;*
differs by at least 1, 2, 5, 10 or 20 amino acids but by no more than 90, 80, 70, 60, 50, 40 or 30 amino acids from amino acids 1-180 of the amino acid sequence of Cas9 of *S. pyogenes, S. thermophilus, S. mutans* or *Listeria innocua;* or
is identical to 1-180 of the amino acid sequence of Cas9 *o*f *S. pyogenes, S. thermophilus, S. mutans* or *L. innocua.*

In an embodiment, a Cas9 molecule or Cas9 polypeptide, e.g., an eaCas9 molecule or eaCas9 polypeptide, comprises an amino acid sequence referred to as region 1':
having 55%, 60%, 65%, 70%, 75%, 80%, 85%, 90%, 95%, 96%, 97%, 98% or 99% homology with amino acids 120-180 (55% of residues in the four Cas9 sequences in **Figs. 2A-2G** are conserved) of the amino acid sequence of Cas9 of *S. pyogenes, S. thermophilus, S. mutans* or *L*. *innocua;*
differs by at least 1, 2, or 5 amino acids but by no more than 35, 30, 25, 20 or 10 amino acids from amino acids 120-180 of the amino acid sequence of Cas9 of *S. pyogenes, S. thermophilus, S. mutans* or *L. innocua* ; or
is identical to 120-180 of the amino acid sequence of Cas9 of *S. pyogenes, S. thermophilus, S. mutans* or *L. innocua.*

In an embodiment, a Cas9 molecule or Cas9 polypeptide, e.g., an eaCas9 molecule or eaCas9 polypeptide, comprises an amino acid sequence referred to as region 2:
having 50%, 55%, 60%, 65%, 70%, 75%, 80%, 85%, 90%, 95%, 96%, 97%, 98% or 99% homology with amino acids 360-480 (52% of residues in the four Cas9 sequences in **Figs. 2A-2G** are conserved) of the amino acid sequence of Cas9 of *S. pyogenes, S. thermophilus, S. mutans* or *L. innocua;*
differs by at least 1, 2, or 5 amino acids but by no more than 35, 30, 25, 20 or 10 amino acids from amino acids 360-480 of the amino acid sequence of Cas9 of *S. pyogenes, S. thermophilus, S. mutans* or *L. innocua;* or
is identical to 360-480 of the amino acid sequence of Cas9 of *S. pyogenes, S. thermophilus, S. mutans* or *L. innocua.*

In an embodiment, a Cas9 molecule or Cas9 polypeptide, e.g., an eaCas9 molecule or eaCas9 polypeptide, comprises an amino acid sequence referred to as region 3:
having 55%, 60%, 65%, 70%, 75%, 80%, 85%, 90%, 95%, 96%, 97%, 98%, or 99% homology with amino acids 660-720 (56% of residues in the four Cas9 sequences in **Figs. 2A-2G** are conserved) of the amino acid sequence of Cas9 of *S. pyogenes, S. thermophilus, S. mutans* or *L*. *innocua;*
differs by at least 1, 2, or 5 amino acids but by no more than 35, 30, 25, 20 or 10 amino acids from amino acids 660-720 of the amino acid sequence of Cas9 of *S. pyogenes, S. thermophilus, S. mutans* or *L. innocua;* or
is identical to 660-720 of the amino acid sequence of Cas9 of *S. pyogenes, S. thermophilus, S. mutans* or *L. innocua.*

In an embodiment, a Cas9 molecule or Cas9 polypeptide, e.g., an eaCas9 molecule or eaCas9 polypeptide, comprises an amino acid sequence referred to as region 4:
having 50%, 55%, 60%, 65%, 70%, 75%, 80%, 85%, 90%, 95%, 96%, 97%, 98%, or 99% homology with amino acids 817-900 (55% of residues in the four Cas9 sequences in **Figs. 2A-2G** are conserved) of the amino acid sequence of Cas9 of *S. pyogenes, S. thermophilus, S. mutans* or *L. innocua;*
differs by at least 1, 2, or 5 amino acids but by no more than 35, 30, 25, 20 or 10 amino acids from amino acids 817-900 of the amino acid sequence of Cas9 of *S. pyogenes, S. thermophilus, S. mutans* or *L. innocua;* or
is identical to 817-900 of the amino acid sequence of Cas9 of *S. pyogenes, S. thermophilus, S. mutans* or *L. innocua.*

In an embodiment, a Cas9 molecule or Cas9 polypeptide, e.g., an eaCas9 molecule or eaCas9 polypeptide, comprises an amino acid sequence referred to as region 5:
having 50%, 55%, 60%, 65%, 70%, 75%, 80%, 85%, 90%, 95%, 96%, 97%, 98%, or 99% homology with amino acids 900-960 (60% of residues in the four Cas9 sequences in **Figs. 2A-2G** are conserved) of the amino acid sequence of Cas9 of *S. pyogenes, S. thermophilus, S. mutans* or *L. innocua;*
differs by at least 1, 2, or 5 amino acids but by no more than 35, 30, 25, 20 or 10 amino acids from amino acids 900-960 of the amino acid sequence of Cas9 of *S. pyogenes, S. thermophilus, S. mutans* or *L. innocua;* or
is identical to 900-960 of the amino acid sequence of Cas9 of *S. pyogenes, S. thermophilus, S. mutans* or *L. innocua.*

### Engineered or Altered Cas9 Molecules and Cas9 Polypeptides

Cas9 molecules and Cas9 polypeptides described herein, e.g., naturally occurring Cas9 molecules can possess any of a number of properties, including: nickase activity, nuclease activity (e.g., endonuclease and/or exonuclease activity); helicase activity; the ability to associate functionally with a gRNA molecule; and the ability to target (or localize to) a site on a nucleic acid (e.g., PAM recognition and specificity). In an embodiment, a Cas9 molecule or Cas9 polypeptide can include all or a subset of these properties. In typical embodiments, a Cas9 molecule or Cas9 polypeptide have the ability to interact with a gRNA molecule and, in concert with the gRNA molecule, localize to a site in a nucleic acid. Other activities, e.g., PAM specificity, cleavage activity, or helicase activity can vary more widely in Cas9 molecules and Cas9 polypeptide.

Cas9 molecules include engineered Cas9 molecules and engineered Cas9 polypeptides (engineered, as used in this context, means merely that the Cas9 molecule or Cas9 polypeptide differs from a reference sequences, and implies no process or origin limitation). An engineered Cas9 molecule or Cas9 polypeptide can comprise altered enzymatic properties, e.g., altered nuclease activity, (as compared with a naturally occurring or other reference Cas9 molecule) or altered helicase activity. As discussed herein, an engineered Cas9 molecule or Cas9 polypeptide can have nickase activity (as opposed to double strand nuclease activity). In an embodiment an engineered Cas9 molecule or Cas9 polypeptide can have an alteration that alters its size, e.g., a deletion of amino acid sequence that reduces its size, e.g., without significant effect on one or more, or any Cas9 activity. In an embodiment, an engineered Cas9 molecule or Cas9 polypeptide can comprise an alteration that affects PAM recognition. E.g., an engineered Cas9 molecule can be altered to recognize a PAM sequence other than that recognized by the endogenous wild-type PI domain. In an embodiment, a Cas9 molecule or Cas9 polypeptide can differ in sequence from a naturally occurring Cas9 molecule but not have significant alteration in one or more Cas9 activities.

Cas9 molecules or Cas9 polypeptides with desired properties can be made in a number of ways, e.g., by alteration of a parental, e.g., naturally occurring Cas9 molecules or Cas9 polypeptides to provide an altered Cas9 molecule or Cas9 polypeptides having a desired property. For example, one or more mutations or differences relative to a parental Cas9 molecule, e.g., a naturally occurring or engineered Cas9 molecule, can be introduced. Such mutations and differences comprise: substitutions (e.g., conservative substitutions or substitutions of non-essential amino acids); insertions; or deletions. In an embodiment, a Cas9 molecule or Cas9 polypeptide can comprises one or more mutations or differences, e.g., at least 1, 2, 3, 4, 5, 10, 15, 20, 30, 40 or 50 mutations, but less than 200, 100, or 80 mutations relative to a reference, e.g., a parental, Cas9 molecule.

In an embodiment, a mutation or mutations do not have a substantial effect on a Cas9 activity, e.g. a Cas9 activity described herein. In an embodiment, a mutation or mutations have a substantial effect on a Cas9 activity, e.g. a Cas9 activity described herein.

### Non-Cleaving and Modified-Cleavage Cas9 Molecules and Cas9 Polypeptides

In an embodiment, a Cas9 molecule or Cas9 polypeptide comprises a cleavage property that differs from naturally occurring Cas9 molecules, e.g., that differs from the naturally occurring Cas9 molecule having the closest homology. For example, a Cas9 molecule or Cas9 polypeptide can differ from naturally occurring Cas9 molecules, e.g., a Cas9 molecule of *S. pyogenes,* as follows: its ability to modulate, e.g., decreased or increased, cleavage of a double stranded nucleic acid (endonuclease and/or exonuclease activity), e.g., as compared to a naturally occurring Cas9 molecule (e.g., a Cas9 molecule of *S. pyogenes);* its ability to modulate, e.g., decreased or increased, cleavage of a single strand of a nucleic acid, e.g., a non-complementary strand of a nucleic acid molecule or a complementary strand of a nucleic acid molecule (nickase activity), e.g., as compared to a naturally occurring Cas9 molecule (e.g., a Cas9 molecule of *S. pyogenes);* or the ability to cleave a nucleic acid molecule, e.g., a double stranded or single stranded nucleic acid molecule, can be eliminated.

### Modified Cleavage eaCas9 Molecules and eaCas9 Polypeptides

In an embodiment, an eaCas9 molecule or eaCas9 polypeptide comprises one or more of the following activities: cleavage activity associated with an N-terminal RuvC-like domain; cleavage activity associated with an HNH-like domain; cleavage activity associated with an HNH-like domain and cleavage activity associated with an N-terminal RuvC-like domain.

In an embodiment, an eaCas9 molecule or eaCas9 polypeptide comprises an active, or cleavage competent, HNH-like domain (e.g., an HNH-like domain described herein, e.g., SEQ ID NO: 17, SEQ ID NO: 18, SEQ ID NO: 19, SEQ ID NO: 20 or SEQ ID NO: 21) and an inactive, or cleavage incompetent, N-terminal RuvC-like domain. An exemplary inactive, or cleavage incompetent N-terminal RuvC-like domain can have a mutation of an aspartic acid in an N-terminal RuvC-like domain, e.g., an aspartic acid at position 9 of the consensus sequence disclosed in **Figs. 2A-2G** or an aspartic acid at position 10 of SEQ ID NO: 7, e.g., can be substituted with an alanine. In an embodiment, the eaCas9 molecule or eaCas9 polypeptide differs from wild type in the N-terminal RuvC-like domain and does not cleave the target nucleic acid, or cleaves with significantly less efficiency, e.g., less than 20, 10, 5, 1 or .1 % of the cleavage activity of a reference Cas9 molecule, e.g., as measured by an assay described herein. The reference Cas9 molecule can by a naturally occurring unmodified Cas9 molecule, e.g., a naturally occurring Cas9 molecule such as a Cas9 molecule of *S. pyogenes,* or *S. thermophilus.* In an embodiment, the reference Cas9 molecule is the naturally occurring Cas9 molecule having the closest sequence identity or homology.

In an embodiment, an eaCas9 molecule or eaCas9 polypeptide comprises an inactive, or cleavage incompetent, HNH domain and an active, or cleavage competent, N-terminal RuvC-like domain (e.g., a RuvC-like domain described herein, e.g., SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, or SEQ ID NO: 16). Exemplary inactive, or cleavage incompetent HNH-like domains can have a mutation at one or more of: a histidine in an HNH-like domain, e.g., a histidine shown at position 856 of the consensus sequence disclosed in **Figs. 2A-2G****,** e.g., can be substituted with an alanine; and one or more asparagines in an HNH-like domain, e.g., an asparagine shown at position 870 of the consensus sequence disclosed in **Figs. 2A-2G** and/or at position 879 of the consensus sequence disclosed in **Figs. 2A-2G****,** e.g., can be substituted with an alanine. In an embodiment, the eaCas9 differs from wild type in the HNH-like domain and does not cleave the target nucleic acid, or cleaves with significantly less efficiency, e.g., less than 20, 10, 5, 1 or 0.1% of the cleavage activity of a reference Cas9 molecule, e.g., as measured by an assay described herein. The reference Cas9 molecule can by a naturally occurring unmodified Cas9 molecule, e.g., a naturally occurring Cas9 molecule such as a Cas9 molecule of *S. pyogenes,* or *S. thermophilus.* In an embodiment, the reference Cas9 molecule is the naturally occurring Cas9 molecule having the closest sequence identity or homology.

### Alterations in the Ability to Cleave One or Both Strands of a Target Nucleic Acid

In an embodiment, exemplary Cas9 activities comprise one or more of PAM specificity, cleavage activity, and helicase activity. A mutation(s) can be present, e.g., in one or more RuvC-like domain, e.g., an N-terminal RuvC-like domain; an HNH-like domain; a region outside the RuvC-like domains and the HNH-like domain. In some embodiments, a mutation(s) is present in a RuvC-like domain, e.g., an N-terminal RuvC-like domain. In some embodiments, a mutation(s) is present in an HNH-like domain. In some embodiments, mutations are present in both a RuvC-like domain, e.g., an N-terminal RuvC-like domain and an HNH-like domain.

Exemplary mutations that may be made in the RuvC domain or HNH domain with reference to the *S. pyogenes* sequence include: D10A, E762A, H840A, N854A, N863A and/or D986A.

In an embodiment, a Cas9 molecule or Cas9 polypeptide is an eiCas9 molecule or eiCas9 polypeptide comprising one or more differences in a RuvC domain and/or in an HNH domain as compared to a reference Cas9 molecule, and the eiCas9 molecule or eiCas9 polypeptide does not cleave a nucleic acid, or cleaves with significantly less efficiency than does wildype, e.g., when compared with wild type in a cleavage assay, e.g., as described herein, cuts with less than 50, 25, 10, or 1% of a reference Cas9 molecule, as measured by an assay described herein.

Whether or not a particular sequence, e.g., a substitution, may affect one or more activity, such as targeting activity, cleavage activity, etc., can be evaluated or predicted, e.g., by evaluating whether the mutation is conservative or by the method described in Section IV. In an embodiment, a "non-essential" amino acid residue, as used in the context of a Cas9 molecule, is a residue that can be altered from the wild-type sequence of a Cas9 molecule, e.g., a naturally occurring Cas9 molecule, e.g., an eaCas9 molecule, without abolishing or more preferably, without substantially altering a Cas9 activity (e.g., cleavage activity), whereas changing an "essential" amino acid residue results in a substantial loss of activity (e.g., cleavage activity).

In an embodiment, a Cas9 molecule or Cas9 polypeptide comprises a cleavage property that differs from naturally occurring Cas9 molecules, e.g., that differs from the naturally occurring Cas9 molecule having the closest homology. For example, a Cas9 molecule or Cas9 polypeptide can differ from naturally occurring Cas9 molecules, e.g., a Cas9 molecule of *S aureus, S. pyogenes,* or *C. jejuni* as follows: its ability to modulate, e.g., decreased or increased, cleavage of a double stranded break (endonuclease and/or exonuclease activity), e.g., as compared to a naturally occurring Cas9 molecule (e.g., a Cas9 molecule of *S aureus, S. pyogenes,* or *C. jejuni);* its ability to modulate, e.g., decreased or increased, cleavage of a single strand of a nucleic acid, e.g., a non-complimentary strand of a nucleic acid molecule or a complementary strand of a nucleic acid molecule (nickase activity), e.g., as compared to a naturally occurring Cas9 molecule (e.g., a Cas9 molecule of *S aureus, S. pyogenes,* or *C. jejuni*); or the ability to cleave a nucleic acid molecule, e.g., a double stranded or single stranded nucleic acid molecule, can be eliminated.

In an embodiment, the altered Cas9 molecule or Cas9 polypeptide is an eaCas9 molecule or eaCas9 polypeptide comprising one or more of the following activities: cleavage activity associated with a RuvC domain; cleavage activity associated with an HNH domain; cleavage activity associated with an HNH domain and cleavage activity associated with a RuvC domain.

In an embodiment, the altered Cas9 molecule or Cas9 polypeptide is an eiCas9 molecule or eiCas9 polypeptide which does not cleave a nucleic acid molecule (either double stranded or single stranded nucleic acid molecules) or cleaves a nucleic acid molecule with significantly less efficiency, e.g., less than 20, 10, 5, 1 or 0.1% of the cleavage activity of a reference Cas9 molecule, e.g., as measured by an assay described herein. The reference Cas9 molecule can be a naturally occurring unmodified Cas9 molecule, e.g., a naturally occurring Cas9 molecule such as a Cas9 molecule of *S. pyogenes, S. thermophilus, S. aureus, C. jejuni* or *N. meningitidis.* In an embodiment, the reference Cas9 molecule is the naturally occurring Cas9 molecule having the closest sequence identity or homology. In an embodiment, the eiCas9 molecule or eiCas9 polypeptide lacks substantial cleavage activity associated with a RuvC domain and cleavage activity associated with an HNH domain.

In an embodiment, the altered Cas9 molecule or Cas9 polypeptide is is an eaCas9 molecule or eaCas9 polypeptide is comprising the fixed amino acid residues of *S. pyogenes* shown in the consensus sequence disclosed in **Figs. 2A-2G****,** and has one or more amino acids that differ from the amino acid sequence of *S. pyogenes* (e.g., has a substitution) at one or more residue (e.g., 2, 3, 5, 10, 15, 20, 30, 50, 70, 80, 90, 100, 200 amino acid residues) represented by an "-" in the consensus sequence disclosed in **Figs. 2A-2G** or SEQ ID NO:7.

In an embodiment, the altered Cas9 molecule or Cas9 polypeptide comprises a sequence in which:
the sequence corresponding to the fixed sequence of the consensus sequence disclosed in **Figs. 2A-2G** differs at no more than 1, 2, 3, 4, 5, 10, 15, or 20% of the fixed residues in the consensus sequence disclosed in **Figs. 2A-2G****;**
the sequence corresponding to the residues identified by "*" in the consensus sequence disclosed in **Figs. 2A-2G** differ at no more than 1, 2, 3, 4, 5, 10, 15, 20, 25, 30, 35, or 40% of the "*" residues from the corresponding sequence of naturally occurring Cas9 molecule, e.g., an *S. pyogenes* Cas9 molecule; and,
the sequence corresponding to the residues identified by "-" in the consensus sequence disclosed in **Figs. 2A-2G** differ at no more than 5, 10, 15, 20, 25, 30, 35, 40, 45, 55, or 60% of the "-" residues from the corresponding sequence of naturally occurring Cas9 molecule, e.g., an *S. pyogenes* Cas9 molecule.

In an embodiment, the altered Cas9 molecule or Cas9 polypeptide is an eaCas9 molecule or eaCas9 polypeptide comprising the fixed amino acid residues of *S. thermophilus* shown in the consensus sequence disclosed in **Figs. 2A-2G****,** and has one or more amino acids that differ from the amino acid sequence of *S. thermophilus* (e.g., has a substitution) at one or more residue (e.g., 2, 3, 5, 10, 15, 20, 30, 50, 70, 80, 90, 100, 200 amino acid residues) represented by an "-" in the consensus sequence disclosed in **Figs. 2A-2G****.** In an embodiment

In an embodiment, the altered Cas9 molecule or Cas9 polypeptide comprises a sequence in which:
the sequence corresponding to the fixed sequence of the consensus sequence disclosed in **Figs. 2A-2G** differs at no more than 1, 2, 3, 4, 5, 10, 15, or 20% of the fixed residues in the consensus sequence disclosed in **Figs. 2A-2G****;**
the sequence corresponding to the residues identified by "*" in the consensus sequence disclosed in **Figs. 2A-2G** differ at no more than 1, 2, 3, 4, 5, 10, 15, 20, 25, 30, 35, or 40% of the "*" residues from the corresponding sequence of naturally occurring Cas9 molecule, e.g., an *S. thermophilus* Cas9 molecule; and,
the sequence corresponding to the residues identified by "-" in the consensus sequence disclosed in **Figs. 2A-2G** differ at no more than 5, 10, 15, 20, 25, 30, 35, 40, 45, 55, or 60% of the "-" residues from the corresponding sequence of naturally occurring Cas9 molecule, e.g., an *S. thermophilus* Cas9 molecule.

In an embodiment, the altered Cas9 molecule or Cas9 polypeptide is an eaCas9 molecule or eaCas9 polypeptide comprising the fixed amino acid residues of *S. mutans* shown in the consensus sequence disclosed in **Figs. 2A-2G****,** and has one or more amino acids that differ from the amino acid sequence of *S. mutans* (e.g., has a substitution) at one or more residue (e.g., 2, 3, 5, 10, 15, 20, 30, 50, 70, 80, 90, 100, 200 amino acid residues) represented by an "-" in the consensus sequence disclosed in **Figs. 2A-2G****.**

In an embodiment, the altered Cas9 molecule or Cas9 polypeptide comprises a sequence in which:
the sequence corresponding to the fixed sequence of the consensus sequence disclosed in **Figs. 2A-2G** differs at no more than 1, 2, 3, 4, 5, 10, 15, or 20% of the fixed residues in the consensus sequence disclosed in **Figs. 2A-2G****;**
the sequence corresponding to the residues identified by "*" in the consensus sequence disclosed in **Figs. 2A-2G** differ at no more than 1, 2, 3, 4, 5, 10, 15, 20, 25, 30, 35, or 40% of the "*" residues from the corresponding sequence of naturally occurring Cas9 molecule, e.g., an *S. mutans* Cas9 molecule; and,
the sequence corresponding to the residues identified by "-" in the consensus sequence disclosed in **Figs. 2A-2G** differ at no more than 5, 10, 15, 20, 25, 30, 35, 40, 45, 55, or 60% of the "-" residues from the corresponding sequence of naturally occurring Cas9 molecule, e.g., an *S. mutans* Cas9 molecule.

In an embodiment, the altered Cas9 molecule or Cas9 polypeptide is an eaCas9 molecule or eaCas9 polypeptide comprising the fixed amino acid residues of *L*. *innocula* shown in the consensus sequence disclosed in **Figs. 2A-2G****,** and has one or more amino acids that differ from the amino acid sequence of *L*. *innocula* (e.g., has a substitution) at one or more residue (e.g., 2, 3, 5, 10, 15, 20, 30, 50, 70, 80, 90, 100, 200 amino acid residues) represented by an "-"in the consensus sequence disclosed in **Figs. 2A-2G****.**

In an embodiment, the altered Cas9 molecule or Cas9 polypeptide comprises a sequence in which:
the sequence corresponding to the fixed sequence of the consensus sequence disclosed in **Figs. 2A-2G** differs at no more than 1, 2, 3, 4, 5, 10, 15, or 20% of the fixed residues in the consensus sequence disclosed in **Figs. 2A-2G****;**
the sequence corresponding to the residues identified by "*" in the consensus sequence disclosed in **Figs. 2A-2G** differ at no more than 1, 2, 3, 4, 5, 10, 15, 20, 25, 30, 35, or 40% of the "*" residues from the corresponding sequence of naturally occurring Cas9 molecule, e.g., an *L. innocula* Cas9 molecule; and,
the sequence corresponding to the residues identified by "-" in the consensus sequence disclosed in **Figs. 2A-2G** differ at no more than 5, 10, 15, 20, 25, 30, 35, 40, 45, 55, or 60% of the "-" residues from the corresponding sequence of naturally occurring Cas9 molecule, e.g., an *L. innocula* Cas9 molecule.

In an embodiment, the altered Cas9 molecule or Cas9 polypeptide, e.g., an eaCas9 molecule or eaCas9 polypeptide, can be a fusion, e.g., of two of more different Cas9 molecules, e.g., of two or more naturally occurring Cas9 molecules of different species. For example, a fragment of a naturally occurring Cas9 molecule of one species can be fused to a fragment of a Cas9 molecule of a second species. As an example, a fragment of a Cas9 molecule of *S. pyogenes* comprising an N-terminal RuvC-like domain can be fused to a fragment of a Cas9 molecule of a species other than *S. pyogenes* (e.g., *S. thermophilus*) comprising an HNH-like domain.

### Cas9 Molecules or Cas9 polypeptides with Altered PAM Recognition or No PAM Recognition

Naturally occurring Cas9 molecules can recognize specific PAM sequences, for example, the PAM recognition sequences described above for *S. pyogenes, S. thermophiles, S. mutans, S. aureus* and *N. meningitidis.*

In an embodiment, a Cas9 molecule or Cas9 polypeptide has the same PAM specificities as a naturally occurring Cas9 molecule. In another embodiment, a Cas9 molecule or Cas9 polypeptide has a PAM specificity not associated with a naturally occurring Cas9 molecule, or a PAM specificity not associated with the naturally occurring Cas9 molecule to which it has the closest sequence homology. For example, a naturally occurring Cas9 molecule or Cas9 polypeptide can be altered, e.g., to alter PAM recognition, e.g., to alter the PAM sequence that the Cas9 molecule recognizes to decrease off target sites and/or improve specificity; or eliminate a PAM recognition requirement. In an embodiment, a Cas9 molecule or Cas9 polypeptide can be altered, e.g., to increase length of PAM recognition sequence and/or improve Cas9 specificity to high level of identity (e.g., 98%, 99% or 100% match between gRNA and a PAM sequence), to decrease off target sites and increase specificity. In an embodiment, the length of the PAM recognition sequence is at least 4, 5, 6, 7, 8, 9, 10 or 15 amino acids in length. In an embodiment, the Cas9 specificity requires at least 90%, 95%, 96%, 97%, 98%, 99% or more homology between the gRNA and the PAM sequence. Cas9 molecules or Cas9 polypeptides that recognize different PAM sequences and/or have reduced off-target activity can be generated using directed evolution. Exemplary methods and systems that can be used for directed evolution of Cas9 molecules are described, e.g., in Esvelt et al. NATURE 2011, 472(7344): 499-503. Candidate Cas9 molecules can be evaluated, e.g., by methods described in Section IV.

Alterations of the PI domain, which mediates PAM recognition, are discussed below.

### Synthetic Cas9 Molecules and Cas9 Polypeptides with Altered PI Domains

Current genome-editing methods are limited in the diversity of target sequences that can be targeted by the PAM sequence that is recognized by the Cas9 molecule utilized. A synthetic Cas9 molecule (or Syn-Cas9 molecule), or synthetic Cas9 polypeptide (or Syn-Cas9 polypeptide), as that term is used herein, refers to a Cas9 molecule or Cas9 polypeptide that comprises a Cas9 core domain from one bacterial species and a functional altered PI domain, i.e., a PI domain other than that naturally associated with the Cas9 core domain, e.g., from a different bacterial species.

In an embodiment, the altered PI domain recognizes a PAM sequence that is different from the PAM sequence recognized by the naturally-occurring Cas9 from which the Cas9 core domain is derived. In an embodiment, the altered PI domain recognizes the same PAM sequence recognized by the naturally-occurring Cas9 from which the Cas9 core domain is derived, but with different affinity or specificity. A Syn-Cas9 molecule or Syn-Cas9 polypetide can be, respectively, a Syn-eaCas9 molecule or Syn-eaCas9 polypeptide or a Syn-eiCas9 molecule Syn-eiCas9 polypeptide.

An exemplary Syn-Cas9 molecule or Syn-Cas9 polypetide comprises:
a) a Cas9 core domain, e.g., a Cas9 core domain from **Table 25** or **26,** e.g., a *S*. *aureus, S. pyogenes,* or *C. jejuni* Cas9 core domain; and
b) an altered PI domain from a species X Cas9 sequence selected from **Tables 28** and **29.**

In an embodiment, the RKR motif (the PAM binding motif) of said altered PI domain comprises: differences at 1, 2, or 3 amino acid residues; a difference in amino acid sequence at the first, second, or third position; differences in amino acid sequence at the first and second positions, the first and third positions, or the second and third positions; as compared with the sequence of the RKR motif of the native or endogenous PI domain associated with the Cas9 core domain.

In an embodiment, the Cas9 core domain comprises the Cas9 core domain from a species X Cas9 from **Table 25** and said altered PI domain comprises a PI domain from a species Y Cas9 from **Table 25.**

In an embodiment, the RKR motif of the species X Cas9 is other than the RKR motif of the species Y Cas9.

In an embodiment, the RKR motif of the altered PI domain is selected from XXY, XNG, and XNQ.

In an embodiment, the altered PI domain has at least 60, 70, 80, 90, 95, or 100% homology with the amino acid sequence of a naturally occurring PI domain of said species Y from **Table 25.**

In an embodiment, the altered PI domain differs by no more than 50, 40, 30, 25, 20, 15, 10, 5, 4, 3, 2, or 1 amino acid residue from the amino acid sequence of a naturally occurring PI domain of said second species from **Table 25.**

In an embodiment, the Cas9 core domain comprises a *S*. *aureus* core domain and altered PI domain comprises: an *A*. *denitrificans* PI domain; a *C. jejuni* PI domain; a *H. mustelae* PI domain; or an altered PI domain of species X PI domain, wherein species X is selected from **Table 29.**

In an embodiment, the Cas9 core domain comprises a *S. pyogenes* core domain and the altered PI domain comprises: an *A*. *denitrificans* PI domain; a *C. jejuni* PI domain; a *H. mustelae* PI domain; or an altered PI domain of species X PI domain, wherein species X is selected from **Table 29.**

In an embodiment, the Cas9 core domain comprises a *C. jejuni* core domain and the altered PI domain comprises: an *A*. *denitrificans* PI domain; a *H. mustelae* PI domain; or an altered PI domain of species X PI domain, wherein species X is selected from **Table 29.**

In an embodiment, the Cas9 molecule or Cas9 polypeptide further comprises a linker disposed between said Cas9 core domain and said altered PI domain.

In an embodiment, the linker comprises: a linker described elsewhere herein disposed between the Cas9 core domain and the heterologous PI domain. Suitable linkers are further described in Section V.

Exemplary altered PI domains for use in Syn-Cas9 molecules are described in **Tables 28** and **29.** The sequences for the 83 Cas9 orthologs referenced in **Tables 28** and **29** are provided in **Table 25. Table 27** provides the Cas9 orthologs with known PAM sequences and the corresponding RKR motif.

In an embodiment, a Syn-Cas9 molecule or Syn-Cas9 polypeptide may also be size-optimized, e.g., the Syn-Cas9 molecule or Syn-Cas9 polypeptide comprises one or more deletions, and optionally one or more linkers disposed between the amino acid residues flanking the deletions. In an embodiment, a Syn-Cas9 molecule or Syn-Cas9 polypeptide comprises a REC deletion.

### Size-Optimized Cas9 Molecules and Cas9 Polypeptides

Engineered Cas9 molecules and engineered Cas9 polypeptides described herein include a Cas9 molecule or Cas9 polypeptide comprising a deletion that reduces the size of the molecule while still retaining desired Cas9 properties, e.g., essentially native conformation, Cas9 nuclease activity, and/or target nucleic acid molecule recognition. Provided herein are Cas9 molecules or Cas9 polypeptides comprising one or more deletions and optionally one or more linkers, wherein a linker is disposed between the amino acid residues that flank the deletion. Methods for identifying suitable deletions in a reference Cas9 molecule, methods for generating Cas9 molecules with a deletion and a linker, and methods for using such Cas9 molecules will be apparent to one of ordinary skill in the art upon review of this document.

A Cas9 molecule, e.g., a *S. aureus, S. pyogenes,* or *C. jejuni,* Cas9 molecule, having a deletion is smaller, e.g., has reduced number of amino acids, than the corresponding naturally-occurring Cas9 molecule. The smaller size of the Cas9 molecules allows increased flexibility for delivery methods, and thereby increases utility for genome-editing. A Cas9 molecule or Cas9 polypeptide can comprise one or more deletions that do not substantially affect or decrease the activity of the resultant Cas9 molecules or Cas9 polypeptides described herein. Activities that are retained in the Cas9 molecules or Cas9 polypeptides comprising a deletion as described herein include one or more of the following:
a nickase activity, i.e., the ability to cleave a single strand, e.g., the non-complementary strand or the complementary strand, of a nucleic acid molecule; a double stranded nuclease activity, i.e., the ability to cleave both strands of a double stranded nucleic acid and create a double stranded break, which in an embodiment is the presence of two nickase activities;
an endonuclease activity;
an exonuclease activity;
a helicase activity, i.e., the ability to unwind the helical structure of a double stranded nucleic acid;
and recognition activity of a nucleic acid molecule, e.g., a target nucleic acid or a gRNA.

Activity of the Cas9 molecules or Cas9 polypeptides described herein can be assessed using the activity assays described herein or in the art.

### Identifying regions suitable for deletion

Suitable regions of Cas9 molecules for deletion can be identified by a variety of methods. Naturally-occurring orthologous Cas9 molecules from various bacterial species, e.g., any one of those listed in **Table 25,** can be modeled onto the crystal structure of *S. pyogenes* Cas9 (Nishimasu et al., Cell, 156:935-949, 2014) to examine the level of conservation across the selected Cas9 orthologs with respect to the three-dimensional conformation of the protein. Less conserved or unconserved regions that are spatially located distant from regions involved in Cas9 activity, e.g., interface with the target nucleic acid molecule and/or gRNA, represent regions or domains are candidates for deletion without substantially affecting or decreasing Cas9 activity.

### REC-Optimized Cas9 Molecules and Cas9 Polypeptides

A REC-optimized Cas9 molecule, or a REC-optimized Cas9 polypeptide, as that term is used herein, refers to a Cas9 molecule or Cas9 polypeptide that comprises a deletion in one or both of the REC2 domain and the RE1_{CT} domain (collectively a REC deletion), wherein the deletion comprises at least 10% of the amino acid residues in the cognate domain. A REC-optimized Cas9 molecule or Cas9 polypeptide can be an eaCas9 molecule or eaCas9 polypetide, or an eiCas9 molecule or eiCas9 polypeptide. An exemplary REC-optimized Cas9 molecule or REC-optimized Cas9 polypeptide comprises:
a) a deletion selected from:
   i) a REC2 deletion;
   ii) a REC1_{CT} deletion; or
   iii) a REC1_{SUB} deletion.

Optionally, a linker is disposed between the amino acid residues that flank the deletion. In an embodiment, a Cas9 molecule or Cas9 polypeptide includes only one deletion, or only two deletions. A Cas9 molecule or Cas9 polypeptide can comprise a REC2 deletion and a REC1_{CT} deletion. A Cas9 molecule or Cas9 polypeptide can comprise a REC2 deletion and a REC1_{SUB} deletion.

Generally, the deletion will contain at least 10% of the amino acids in the cognate domain, e.g., a REC2 deletion will include at least 10% of the amino acids in the REC2 domain. A deletion can comprise: at least 10, 20, 30, 40, 50, 60, 70, 80, or 90% of the amino acid residues of its cognate domain; all of the amino acid residues of its cognate domain; an amino acid residue outside its cognate domain; a plurality of amino acid residues outside its cognate domain; the amino acid residue immediately N terminal to its cognate domain; the amino acid residue immediately C terminal to its cognate domain; the amino acid residue immediately N terminal to its cognate and the amino acid residue immediately C terminal to its cognate domain; a plurality of, e.g., up to 5, 10, 15, or 20, amino acid residues N terminal to its cognate domain; a plurality of, e.g., up to 5, 10, 15, or 20, amino acid residues C terminal to its cognate domain; a plurality of, e.g., up to 5, 10, 15, or 20, amino acid residues N terminal to to its cognate domain and a plurality of e.g., up to 5, 10, 15, or 20, amino acid residues C terminal to its cognate domain.

In an embodiment, a deletion does not extend beyond: its cognate domain; the N terminal amino acid residue of its cognate domain; the C terminal amino acid residue of its cognate domain.

A REC-optimized Cas9 molecule or REC-optimized Cas9 polypeptide can include a linker disposed between the amino acid residues that flank the deletion. Any linkers known in the art that maintain the conformation or native fold of the Cas9 molecule (thereby retaining Cas9 activity) can be used between the amino acid resides that flank a REC deletion in a REC-optimized Cas9 molecule or REC-optimized Cas9 polypeptide. Linkers for use in generating recombinant proteins, e.g., multi-domain proteins, are known in the art (Chen et al., Adv Drug Delivery Rev, 65:1357-69, 2013).

In an embodiment, a REC-optimized Cas9 molecule or REC-optimized Cas9 polypeptide comprises an amino acid sequence that, other than any REC deletion and associated linker, has at least 50, 55, 60, 65, 70, 75, 80, 85, 90, 95, 99, or 100% homology with the amino acid sequence of a naturally occurring Cas 9, e.g., a Cas9 molecule described in **Table 25,** e.g., a *S*. *aureus* Cas9 molecule, a *S. pyogenes* Cas9 molecule, or a *C. jejuni* Cas9 molecule.

In an embodiment, a a REC-optimized Cas9 molecule or REC-optimized Cas9 polypeptide comprises an amino acid sequence that, other than any REC deletion and associated linker, differs by no more than 1, 2, 3, 4, 5, 6, 7, 8, 9, 10, 15, 20, or 25, amino acid residues from the amino acid sequence of a naturally occurring Cas 9, e.g., a Cas9 molecule described in **Table 25,** e.g., a *S. aureus* Cas9 molecule, a *S. pyogenes* Cas9 molecule, or a C. *jejuni* Cas9 molecule.

In an embodiment, a REC-optimized Cas9 molecule or REC-optimized Cas9 polypeptide comprises an amino acid sequence that, other than any REC deletion and associate linker, differs by no more than 1, 2, 3, 4, 5, 6, 7, 8, 9, 10, 15, 20, or 25% of the, amino acid residues from the amino acid sequence of a naturally occurring Cas 9, e.g., a Cas9 molecule described in **Table 25,** e.g., a *S*. *aureus* Cas9 molecule, a *S. pyogenes* Cas9 molecule, or a C. *jejuni* Cas9 molecule.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. Methods of alignment of sequences for comparison are well known in the art. Optimal alignment of sequences for comparison can be conducted, e.g., by the local homology algorithm of Smith and Waterman, (1970) Adv. Appl. Math. 2:482c, by the homology alignment algorithm of Needleman and Wunsch, (1970) J. Mol. Biol. 48:443, by the search for similarity method of Pearson and Lipman, (1988) Proc. Nat'l. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Brent et al., (2003) Current Protocols in Molecular Biology).

Two examples of algorithms that are suitable for determining percent sequence identity and sequence similarity are the BLAST and BLAST 2.0 algorithms, which are described in Altschul et al., (1977) Nuc. Acids Res. 25:3389-3402; and Altschul et al., (1990) J. Mol. Biol. 215:403-410, respectively. Software for performing BLAST analyses is publicly available through the National Center for Biotechnology Information.

The percent identity between two amino acid sequences can also be determined using the algorithm of E. Meyers and W. Miller, (1988) Comput. Appl. Biosci. 4:11-17) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (1970) J. Mol. Biol. 48:444-453) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1, 2, 3, 4, 5, or 6.

Sequence information for exemplary REC deletions are provided for 83 naturally-occurring Cas9 orthologs in **Table 25.**

The amino acid sequences of exemplary Cas9 molecules from different bacterial species are shown below.

**Table 25. Amino Acid Sequence of Cas9 Orthologs h**

| | | REC2 | | | REC1_{CT} | | | Rec_{sub} | | |
|---|---|---|---|---|---|---|---|---|---|---|
| **Species / Composite ID** | **Amino acid sequence** | start (AA pos) | stop (AA pos) | # AA delete d(n) | start (AA pos) | stop (AA pos) | # AA delete d(n) | start (AA pos) | stop (AA pos) | # AA delete d(n) |
| Staphylococcus Aureus tr\|J7RUA5\|J7RUA5_STAAU | SEQ ID NO: 304 | 126 | 166 | 41 | 296 | 352 | 57 | 296 | 352 | 57 |
| Streptococcus Pyogenes sp\|Q99ZW2\|CAS9_STRP1 | SEQ ID NO: 305 | 176 | 314 | 139 | 511 | 592 | 82 | 511 | 592 | 82 |
| Campylobacter jejuni NCTC 11168 gi\|218563121\|ref\|YP_00234490 0.1 | SEQ ID NO: 306 | 137 | 181 | 45 | 316 | 360 | 45 | 316 | 360 | 45 |
| Bacteroides fragilis NCTC 9343 gi\|60683389\|ref\|YP_213533.1\| | SEQ ID NO: 307 | 148 | 339 | 192 | 524 | 617 | 84 | 524 | 617 | 84 |
| Bifidobacterium bifidum S17 gi\|310286728\|ref\|YP_00393798 6. | SEQ ID NO: 308 | 173 | 335 | 163 | 516 | 607 | 87 | 516 | 607 | 87 |
| Veillonella atypica ACS-134-V-Col7a gi\|303229466\|ref\|ZP_07316256. 1 | SEQ ID NO: 309 | 185 | 339 | 155 | 574 | 663 | 79 | 574 | 663 | 79 |
| Lactobacillus rhamnosus GG gi\|258509199\|ref\|YP_00317195 0.1 | SEQ ID NO: 310 | 169 | 320 | 152 | 559 | 645 | 78 | 559 | 645 | 78 |
| Filifactor alocis ATCC 35896 gi\|374307738\|ref\|YP_00505416 9.1 | SEQ ID NO: 311 | 166 | 314 | 149 | 508 | 592 | 76 | 508 | 592 | 76 |
| Oenococcus kitaharae DSM 17330 gi\|366983953\|gb\|EHN59352.1\| | SEQ ID NO: 312 | 169 | 317 | 149 | 555 | 639 | 80 | 555 | 639 | 80 |
| Fructobacillus fructosus KCTC 3544 gi\|339625081\|ref\|ZP_08660870. 1 | SEQ ID NO: 313 | 168 | 314 | 147 | 488 | 571 | 76 | 488 | 571 | 76 |
| Catenibacterium mitsuokai DSM 15897 gi\|224543312\|ref\|ZP_03683851. 1 | SEQ ID NO: 314 | 173 | 318 | 146 | 511 | 594 | 78 | 511 | 594 | 78 |
| Finegoldia magna ATCC 29328 gi\|169823755\|ref\|YP_00169136 6.1 | SEQ ID NO: 315 | 168 | 313 | 146 | 452 | 534 | 77 | 452 | 534 | 77 |
| CoriobacteriumglomeransPW 2 gi\|328956315\|ref\|YP_00437364 8.1 | SEQ ID NO: 316 | 175 | 318 | 144 | 511 | 592 | 82 | 511 | 592 | 82 |
| Eubacterium yurii ATCC 43715 gi\|306821691\|ref\|ZP_07455288. 1 | SEQ ID NO: 317 | 169 | 310 | 142 | 552 | 633 | 76 | 552 | 633 | 76 |
| Peptoniphilus duerdenii ATCC BAA-1640 gi\|304438954\|ref\|ZP_07398877. 1 | SEQ ID NO: 318 | 171 | 311 | 141 | 535 | 615 | 76 | 535 | 615 | 76 |
| Acidaminococcus sp. D21 gi\|227824983\|ref\|ZP_03989815. 1 | SEQ ID NO: 319 | 167 | 306 | 140 | 511 | 591 | 75 | 511 | 591 | 75 |
| Lactobacillus farciminis KCTC 3681 gi\|336394882\|ref\|ZP_08576281. 1 | SEQ ID NO: 320 | 171 | 310 | 140 | 542 | 621 | 85 | 542 | 621 | 85 |
| Streptococcus sanguinis SK49 gi\|422884106\|ref\|ZP_16930555. 1 | SEQ ID NO: 321 | 185 | 324 | 140 | 411 | 490 | 85 | 411 | 490 | 85 |
| Coprococcus catus GD-7 gi\|291520705\|emb\|CBK78998.1 \| | SEQ ID NO: 322 | 172 | 310 | 139 | 556 | 634 | 76 | 556 | 634 | 76 |
| Streptococcus mutans UA159 gi\|24379809\|ref\|NP_721764.1\| | SEQ ID NO: 323 | 176 | 314 | 139 | 392 | 470 | 84 | 392 | 470 | 84 |
| Streptococcus pyogenes M1 GAS gi\|13622193\|gb\|AAK33936.1\| | SEQ ID NO: 324 | 176 | 314 | 139 | 523 | 600 | 82 | 523 | 600 | 82 |
| Streptococcus thermophilus LMD-9 gi\|116628213\|ref\|YP_820832.1\| | SEQ ID NO: 325 | 176 | 314 | 139 | 481 | 558 | 81 | 481 | 558 | 81 |
| Fusobacteriumnucleatum ATCC49256 gi\|34762592\|ref\|ZP_00143587.1 \| | SEQ ID NO: 326 | 171 | 308 | 138 | 537 | 614 | 76 | 537 | 614 | 76 |
| Planococcus antarcticus DSM 14505 gi\|389815359\|ref\|ZP_10206685. 1 | SEQ ID NO: 327 | 162 | 299 | 138 | 538 | 614 | 94 | 538 | 614 | 94 |
| Treponema denticola ATCC 35405 gi\|42525843\|ref\|NP_970941.1\| | SEQ ID NO: 328 | 169 | 305 | 137 | 524 | 600 | 81 | 524 | 600 | 81 |
| Solobacterium moorei F0204 gi\|320528778\|ref\|ZP_08029929. 1 | SEQ ID NO: 329 | 179 | 314 | 136 | 544 | 619 | 77 | 544 | 619 | 77 |
| Staphylococcus pseudintermedius ED99 gi\|323463801\|gb\|ADX75954.1\| | SEQ ID NO: 330 | 164 | 299 | 136 | 531 | 606 | 92 | 531 | 606 | 92 |
| Flavobacterium branchiophilum FL-15 gi\|347536497\|ref\|YP_00484392 2.1 | SEQ ID NO: 331 | 162 | 286 | 125 | 538 | 613 | 63 | 538 | 613 | 63 |
| Ignavibacterium album JCM 16511 gi\|385811609\|ref\|YP_00584800 5.1 | SEQ ID NO: 332 | 223 | 329 | 107 | 357 | 432 | 90 | 357 | 432 | 90 |
| Bergeyella zoohelcum ATCC 43767 gi\|423317190\|ref\|ZP_17295095. 1 | SEQ ID NO: 333 | 165 | 261 | 97 | 529 | 604 | 56 | 529 | 604 | 56 |
| Nitrobacter hamburgensis X14 gi\|92109262\|ref\|YP_571550.1\| | SEQ ID NO: 334 | 169 | 253 | 85 | 536 | 611 | 48 | 536 | 611 | 48 |
| Odoribacter laneus YIT 12061 gi\|374384763\|ref\|ZP_09642280. 1 | SEQ ID NO: 335 | 164 | 242 | 79 | 535 | 610 | 63 | 535 | 610 | 63 |
| Legionella pneumophila str. Paris gi\|54296138\|ref\|YP_122507.1\| | SEQ ID NO: 336 | 164 | 239 | 76 | 402 | 476 | 67 | 402 | 476 | 67 |
| Bacteroides sp. 20 3 gi\|301311869\|ref\|ZP_07217791. 1 | SEQ ID NO: 337 | 198 | 269 | 72 | 530 | 604 | 83 | 530 | 604 | 83 |
| Akkermansia muciniphila ATCC BAA-835 gi\|87736489\|ref\|YP_00187860 1. | SEQ ID NO: 338 | 136 | 202 | 67 | 348 | 418 | 62 | 348 | 418 | 62 |
| Prevotella sp. C561 gi\|345885718\|ref\|ZP_08837074. 1 | SEQ ID NO: 339 | 184 | 250 | 67 | 357 | 425 | 78 | 357 | 425 | 78 |
| Wolinella succinogenes DSM 1740 gi\|34557932\|ref\|NP_907747.1\| | SEQ ID NO: 340 | 157 | 218 | 36 | 401 | 468 | 60 | 401 | 468 | 60 |
| Alicyclobacillus hesperidum URH17-3-68 gi\|403744858\|ref\|ZP_10953934. 1 | SEQ ID NO: 341 | 142 | 196 | 55 | 416 | 482 | 61 | 416 | 482 | 61 |
| Caenispirillum salinarum AK4 gi\|427429481\|ref\|ZP_18919511. 1 | SEQ ID NO: 342 | 161 | 214 | 54 | 330 | 393 | 68 | 330 | 393 | 68 |
| Eubacterium rectale ATCC 33656 gi\|238924075\|ref\|YP_00293759 1.1 | SEQ ID NO: 343 | 133 | 185 | 53 | 322 | 384 | 60 | 322 | 384 | 60 |
| Mycoplasma synoviae 53 gi\|71894592\|ref\|YP_278700.1\| | SEQ ID NO: 344 | 187 | 239 | 53 | 319 | 381 | 80 | 319 | 381 | 80 |
| Porphyromonas sp. oral taxon 279 str. F0450 gi\|402847315\|ref\|ZP_10895610. 1 | SEQ ID NO: 345 | 150 | 202 | 53 | 309 | 371 | 60 | 309 | 371 | 60 |
| Streptococcus thermophilus LMD-9 gi\|116627542\|ref\|YP_820161.1\| | SEQ ID NO: 346 | 127 | 178 | 139 | 424 | 486 | 81 | 424 | 486 | 81 |
| Roseburia inulinivorans DSM 16841 gi\|225377804\|ref\|ZP_03755025. 1 | SEQ ID NO: 347 | 154 | 204 | 51 | 318 | 380 | 69 | 318 | 380 | 69 |
| Methylosinus trichosporium OB3b gi\|296446027\|ref\|ZP_06887976. 1 | SEQ ID NO: 348 | 144 | 193 | 50 | 426 | 488 | 64 | 426 | 488 | 64 |
| Ruminococcus albus 8 gi\|325677756\|reftZP_08157403. 1 | SEQ ID NO: 349 | 139 | 187 | 49 | 351 | 412 | 55 | 351 | 412 | 55 |
| Bifidobacterium longum DJO10A gi\|189440764\|ref\|YP_00195584 5. | SEQ ID NO: 350 | 183 | 230 | 48 | 370 | 431 | 44 | 370 | 431 | 44 |
| Enterococcus faecalis TX0012 gi\|315149830\|gb\|EFT93846.1\| | SEQ ID NO: 351 | 123 | 170 | 48 | 327 | 387 | 60 | 327 | 387 | 60 |
| Mycoplasma mobile 163K gi\|47458868\|ref\|YP_015730.1\| | SEQ ID NO: 352 | 179 | 226 | 48 | 314 | 374 | 79 | 314 | 374 | 79 |
| Actinomyces coleocanis DSM 15436 gi\|227494853\|ref\|ZP_03925169. 1 | SEQ ID NO: 353 | 147 | 193 | 47 | 358 | 418 | 40 | 358 | 418 | 40 |
| Dinoroseobacter shibae DFL 12 gi\|159042956\|ref\|YP_00153175 0.1 | SEQ ID NO: 354 | 138 | 184 | 47 | 338 | 398 | 48 | 338 | 398 | 48 |
| Actinomyces sp. oral taxon 180 str. F0310 gi\|315605738\|ref\|ZP_07880770. 1 | SEQ ID NO: 355 | 183 | 228 | 46 | 349 | 409 | 40 | 349 | 409 | 40 |
| Alcanivorax sp. W11-5 gi\|407803669\|ref\|ZP_11150502. 1 | SEQ ID NO: 356 | 139 | 183 | 45 | 344 | 404 | 61 | 344 | 404 | 61 |
| Aminomonas paucivorans DSM 12260 gi\|312879015\|ref\|ZP_07738815. 1 | SEQ ID NO: 357 | 134 | 178 | 45 | 341 | 401 | 63 | 341 | 401 | 63 |
| Mycoplasma canis PG 14 gi\|384393286\|gb\|EIE39736.1\| | SEQ ID NO: 358 | 139 | 183 | 45 | 319 | 379 | 76 | 319 | 379 | 76 |
| Lactobacillus coryniformis KCTC 3535 gi\|336393381\|ref\|ZP_08574780. 1 | SEQ ID NO: 359 | 141 | 184 | 44 | 328 | 387 | 61 | 328 | 387 | 61 |
| Elusimicrobium minutum Pei191 gi\|187250660\|ref\|YP_00187514 2.1 | SEQ ID NO: 360 | 177 | 219 | 43 | 322 | 381 | 47 | 322 | 381 | 47 |
| Neisseria meningitidis Z2491 gi\|218767588\|ref\|YP_00234210 0.1 | SEQ ID NO: 361 | 147 | 189 | 43 | 360 | 419 | 61 | 360 | 419 | 61 |
| Pasteurella multocida str. Pm70 gi\|15602992\|ref\|NP_246064.1\| | SEQ ID NO: 362 | 139 | 181 | 43 | 319 | 378 | 61 | 319 | 378 | 61 |
| Rhodovulum sp. PH10 gi\|402849997\|ref\|ZP_10898214. 1 | SEQ ID NO: 363 | 141 | 183 | 43 | 319 | 378 | 48 | 319 | 378 | 48 |
| Eubacterium dolichum DSM 3991 gi\|160915782\|ref\|ZP_02077990. 1 | SEQ ID NO: 364 | 131 | 172 | 42 | 303 | 361 | 59 | 303 | 361 | 59 |
| Nitratifractor salsuginis DSM 16511 gi\|319957206\|ref\|YP_00416846 9.1 | SEQ ID NO: 365 | 143 | 184 | 42 | 347 | 404 | 61 | 347 | 404 | 61 |
| Rhodospirillum rubrum ATCC 11170 gi\|83591793\|ref\|YP_425545.1\| | SEQ ID NO: 366 | 139 | 180 | 42 | 314 | 371 | 55 | 314 | 371 | 55 |
| Clostridium cellulolyticum H10 gi\|220930482\|ref\|YP_00250739 1.1 | SEQ ID NO: 367 | 137 | 176 | 40 | 320 | 376 | 61 | 320 | 376 | 61 |
| Helicobacter mustelae 12198 gi\|291276265\|ref\|YP_00351603 7.1 | SEQ ID NO: 368 | 148 | 187 | 40 | 298 | 354 | 48 | 298 | 354 | 48 |
| Ilyobacter polytropus DSM 2926 gi\|310780384\|ref\|YP_00396871 6.1 | SEQ ID NO: 369 | 134 | 173 | 40 | 462 | 517 | 63 | 462 | 517 | 63 |
| Sphaerochaeta globus str. Buddy gi\|325972003\|ref\|YP_00424819 4.1 | SEQ ID NO: 370 | 163 | 202 | 40 | 335 | 389 | 45 | 335 | 389 | 45 |
| Staphylococcus lugdunensis M23590 gi\|315659848\|ref\|ZP_07912707. 1 | SEQ ID NO: 371 | 128 | 167 | 40 | 337 | 391 | 57 | 337 | 391 | 57 |
| Treponema sp. JC4 gi\|384109266\|ref\|ZP_10010146. 1 | SEQ ID NO: 372 | 144 | 183 | 40 | 328 | 382 | 63 | 328 | 382 | 63 |
| uncultured delta proteobacterium HF007007E19 gi\|297182908\|gb\|ADI19058.1\| | SEQ ID NO: 373 | 154 | 193 | 40 | 313 | 365 | 55 | 313 | 365 | 55 |
| Alicycliphilus denitrificans K601 gi\|330822845\|ref\|YP_00438614 8.1 | SEQ ID NO: 374 | 140 | 178 | 39 | 317 | 366 | 48 | 317 | 366 | 48 |
| Azospirillum sp. B510 gi\|288957741\|ref\|YP-00344808 2.1 | SEQ ID NO: 375 | 205 | 243 | 39 | 342 | 389 | 46 | 342 | 389 | 46 |
| Bradyrhizobium sp. BTAil gi\|148255343\|ref\|YP_00123992 8.1 | SEQ ID NO: 376 | 143 | 181 | 39 | 323 | 370 | 48 | 323 | 370 | 48 |
| Parvibaculum lavamentivorans DS-1 gi\|154250555\|ref\|YP_00141137 9.1 | SEQ ID NO: 377 | 138 | 176 | 39 | 327 | 374 | 58 | 327 | 374 | 58 |
| Prevotella timonensis CRIS 5C-B1 gi\|282880052\|ref\|ZP_06288774. 1 | SEQ ID NO: 378 | 170 | 208 | 39 | 328 | 375 | 61 | 328 | 375 | 61 |
| Bacillus smithii 7 3 47FAA gi\|365156657\|ref\|ZP_09352959. 1 | SEQ ID NO: 379 | 134 | 171 | 38 | 401 | 448 | 63 | 401 | 448 | 63 |
| Cand. Puniceispirillum marinum IMCC1322 gi\|294086111\|ref\|YP_00355287 1.1 | SEQ ID NO: 380 | 135 | 172 | 38 | 344 | 391 | 53 | 344 | 391 | 53 |
| Barnesiella intestinihominis YIT 11860 gi\|404487228\|ref\|ZP_11022414. 1 | SEQ ID NO: 381 | 140 | 176 | 37 | 371 | 417 | 60 | 371 | 417 | 60 |
| Ralstonia syzygii R24 gi\|344171927\|emb\|CCA84553.1 \| | SEQ ID NO: 382 | 140 | 176 | 37 | 395 | 440 | 50 | 395 | 440 | 50 |
| Wolinella succinogenes DSM 1740 gi\|34557790\|ref\|NP_907605.1\| | SEQ ID NO: 383 | 145 | 180 | 36 | 348 | 392 | 60 | 348 | 392 | 60 |
| Mycoplasma gallisepticum str. F gi\|284931710\|gb\|ADC31648.1\| | SEQ ID NO: 384 | 144 | 177 | 34 | 373 | 416 | 71 | 373 | 416 | 71 |
| Acidothermus cellulolyticus 11B gi\|117929158\|ref\|YP_873709.1\| | SEQ ID NO: 385 | 150 | 182 | 33 | 341 | 380 | 58 | 341 | 380 | 58 |
| Mycoplasma ovipneumoniae SC01 gi\|363542550\|ref\|ZP_09312133. 1 | SEQ ID NO: 386 | 156 | 184 | 29 | 381 | 420 | 62 | 381 | 420 | 62 |

**Table 26. Amino Acid Sequence of Cas9 Core Domains**

| **Strain Name** | **Cas9 Start (AA pos)** | **Cas9 Stop (AA pos)** |
|---|---|---|
| | Start and Stop numbers refer to the sequence in **Table 25** | |
| Staphylococcus Aureus | 1 | 772 |
| Streptococcus Pyogenes | 1 | 1099 |
| Campulobacter Jejuni | 1 | 741 |

**Table 27. Identified PAM sequences and corresponding RKR motifs.**

| **Strain Name** | **PAM sequence (NA)** | **RKR motif (AA)** |
|---|---|---|
| Streptococcus pyogenes | NGG | RKR |
| Streptococcus mutans | NGG | RKR |
| Streptococcus thermophilus A | NGGNG | RYR |
| Treponema denticola | NAAAAN | VAK |
| Streptococcus thermophilus B | NNAAAAW | IYK |
| Campylobacter jejuni | NNNNACA | NLK |
| Pasteurella multocida | GNNNCNNA | KDG |
| Neisseria meningitidis | NNNNGATT or | IGK |
| Staphylococcus aureus | NNGRRV (R = A or G; V = A, G or C) | NDK |
| | NNGRRT (R = A or G) | |

PI domains are provided in **Tables 28** and **29.**

**Table 28. Altered PI Domains**

| **Strain Name** | **PI Start (AA pos)** | **PI Stop (AA pos)** | **Length of PI (AA)** | **RKR motif (AA)** |
|---|---|---|---|---|
| | **Start and Stop numbers refer to the sequences in Table 25** | | | |
| Alicycliphilus denitrificans K601 | 837 | 1029 | 193 | --Y |
| Campylobacter jejuni NCTC 11168 | 741 | 984 | 244 | -NG |
| Helicobacter mustelae 12198 | 771 | 1024 | 254 | -NQ |

**Table 29. Other Altered PI Domains**

| **Strain Name** | **PI Start (AA pos)** | **PI Stop (AA pos)** | **Length of PI (AA)** | **RKR motif (AA)** |
|---|---|---|---|---|
| | **Start and Stop numbers refer to the sequences in Table 25** | | | |
| Akkermansia muciniphila ATCC BAA-835 | 871 | 1101 | 231 | ALK |
| Ralstonia syzygii R24 | 821 | 1062 | 242 | APY |
| Cand. Puniceispirillum marinum IMCC1322 | 815 | 1035 | 221 | AYK |
| Fructobacillus fructosus KCTC 3544 | 1074 | 1323 | 250 | DGN |
| Eubacterium yurii ATCC 43715 | 1107 | 1391 | 285 | DGY |
| Eubacterium dolichum DSM 3991 | 779 | 1096 | 318 | DKK |
| Dinoroseobacter shibae DFL 12 | 851 | 1079 | 229 | DPI |
| Clostridium cellulolyticum H10 | 767 | 1021 | 255 | EGK |
| Pasteurella multocida str. Pm70 | 815 | 1056 | 242 | ENN |
| Mycoplasma canis PG 14 | 907 | 1233 | 327 | EPK |
| Porphyromonas sp. oral taxon 279 str. F0450 | 935 | 1197 | 263 | EPT |
| Filifactor alocis ATCC 35896 | 1094 | 1365 | 272 | EVD |
| Aminomonas paucivorans DSM 12260 | 801 | 1052 | 252 | EVY |
| Wolinella succinogenes DSM 1740 | 1034 | 1409 | 376 | EYK |
| Oenococcus kitaharae DSM 17330 | 1119 | 1389 | 271 | GAL |
| CoriobacteriumglomeransPW2 | 1126 | 1384 | 259 | GDR |
| Peptoniphilus duerdenii ATCC BAA-1640 | 1091 | 1364 | 274 | GDS |
| Bifidobacterium bifidum S17 | 1138 | 1420 | 283 | GGL |
| Alicyclobacillus hesperidum URH17-3-68 | 876 | 1146 | 271 | GGR |
| Roseburia inulinivorans DSM 16841 | 895 | 1152 | 258 | GGT |
| Actinomyces coleocanis DSM 15436 | 843 | 1105 | 263 | GKK |
| Odoribacter laneus YIT 12061 | 1103 | 1498 | 396 | GKV |
| Coprococcus catus GD-7 | 1063 | 1338 | 276 | GNQ |
| Enterococcus faecalis TX0012 | 829 | 1150 | 322 | GRK |
| Bacillus smithii 7 3 47FAA | 809 | 1088 | 280 | GSK |
| Legionella pneumophila str. Paris | 1021 | 1372 | 352 | GTM |
| Bacteroides fragilis NCTC 9343 | 1140 | 1436 | 297 | IPV |
| Mycoplasma ovipneumoniae SC01 | 923 | 1265 | 343 | IRI |
| Actinomyces sp. oral taxon 180 str. F0310 | 895 | 1181 | 287 | KEK |
| Treponema sp. JC4 | 832 | 1062 | 231 | KIS |
| Fusobacteriumnucleatum ATCC49256 | 1073 | 1374 | 302 | KKV |
| Lactobacillus farciminis KCTC 3681 | 1101 | 1356 | 256 | KKV |
| Nitratifractor salsuginis DSM 16511 | 840 | 1132 | 293 | KMR |
| Lactobacillus coryniformis KCTC 3535 | 850 | 1119 | 270 | KNK |
| Mycoplasma mobile 163K | 916 | 1236 | 321 | KNY |
| Flavobacterium branchiophilum FL-15 | 1182 | 1473 | 292 | KQK |
| Prevotella timonensis CRIS 5C-B1 | 957 | 1218 | 262 | KQQ |
| Methylosinus trichosporium OB3b | 830 | 1082 | 253 | KRP |
| Prevotella sp. C561 | 1099 | 1424 | 326 | KRY |
| Mycoplasma gallisepticum str. F | 911 | 1269 | 359 | KTA |
| Lactobacillus rhamnosus GG | 1077 | 1363 | 287 | KYG |
| Wolinella succinogenes DSM 1740 | 811 | 1059 | 249 | LPN |
| Streptococcus thermophilus LMD-9 | 1099 | 1388 | 290 | MLA |
| Treponema denticola ATCC 35405 | 1092 | 1395 | 304 | NDS |
| Bergeyella zoohelcum ATCC 43767 | 1098 | 1415 | 318 | NEK |
| Veillonella atypica ACS-134-V-Col7a | 1107 | 1398 | 292 | NGF |
| Neisseria meningitidis Z2491 | 835 | 1082 | 248 | NHN |
| Ignavibacterium album JCM 16511 | 1296 | 1688 | 393 | NKK |
| Ruminococcus albus 8 | 853 | 1156 | 304 | NNF |
| Streptococcus thermophilus LMD-9 | 811 | 1121 | 311 | NNK |
| Barnesiella intestinihominis YIT 11860 | 871 | 1153 | 283 | NPV |
| Azospirillum sp. B510 | 911 | 1168 | 258 | PFH |
| Rhodospirillum rubrum ATCC 11170 | 863 | 1173 | 311 | PRG |
| Planococcus antarcticus DSM 14505 | 1087 | 1333 | 247 | PYY |
| Staphylococcus pseudintermedius ED99 | 1073 | 1334 | 262 | QIV |
| Alcanivorax sp. W11-5 | 843 | 1113 | 271 | RIE |
| Bradyrhizobium sp. BTAil | 811 | 1064 | 254 | RIY |
| Streptococcus pyogenes M1 GAS | 1099 | 1368 | 270 | RKR |
| Streptococcus mutans UA159 | 1078 | 1345 | 268 | RKR |
| Streptococcus Pyogenes | 1099 | 1368 | 270 | RKR |
| Bacteroides sp. 20 3 | 1147 | 1517 | 371 | RNI |
| S. aureus | 772 | 1053 | 282 | RNK |
| Solobacterium moorei F0204 | 1062 | 1327 | 266 | RSG |
| Finegoldia magna ATCC 29328 | 1081 | 1348 | 268 | RTE |
| uncultured delta proteobacterium HF0070 07E19 | 770 | 1011 | 242 | SGG |
| Acidaminococcus sp. D21 | 1064 | 1358 | 295 | SIG |
| Eubacterium rectale ATCC 33656 | 824 | 1114 | 291 | SKK |
| Caenispirillum salinarum AK4 | 1048 | 1442 | 395 | SLV |
| Acidothermus cellulolyticus 11B | 830 | 1138 | 309 | SPS |
| Catenibacterium mitsuokai DSM 15897 | 1068 | 1329 | 262 | SPT |
| Parvibaculum lavamentivorans DS-1 | 827 | 1037 | 211 | TGN |
| Staphylococcus lugdunensis M23590 | 772 | 1054 | 283 | TKK |
| Streptococcus sanguinis SK49 | 1123 | 1421 | 299 | TRM |
| Elusimicrobium minutum Pei191 | 910 | 1195 | 286 | TTG |
| Nitrobacter hamburgensis X14 | 914 | 1166 | 253 | VAY |
| Mycoplasma synoviae 53 | 991 | 1314 | 324 | VGF |
| Sphaerochaeta globus str. Buddy | 877 | 1179 | 303 | VKG |
| Ilyobacter polytropus DSM 2926 | 837 | 1092 | 256 | VNG |
| Rhodovulum sp. PH10 | 821 | 1059 | 239 | VPY |
| Bifidobacterium longum DJO10A | 904 | 1187 | 284 | VRK |

**Amino acid sequences described in Table 25:**
SEQ ID NO: 304 
MKRNYILGLDIGITSVGYGIIDYETRDVIDAGVRLFKEANVENNEGRRSKRGARRLKRRRRH
RIQRVKKLLFDYNLLTDHSELSGINPYEARVKGLSQKLSEEEFSAALLHLAKRRGVHNVNEV
EEDTGNELSTKEQISRNSKALEEKYVAELQLERLKKDGEVRGSINRFKTSDYVKEAKQLLKV
QKAYHQLDQSFIDTYIDLLETRRTYYEGPGEGSPFGWKDIKEWYEMLMGHCTYFPEELRSVK
YAYNADLYNALNDLNNLVITRDENEKLEYYEKFQIIENVFKQKKKPTLKQIAKEILVNEEDI
KGYRVTSTGKPEFTNLKVYHDIKDITARKEIIENAELLDQIAKILTIYQSSEDIQEELTNLN
SELTQEEIEQISNLKGYTGTHNLSLKAINLILDELWHTNDNQIAIFNRLKLVPKKVDLSQQK
EIPTTLVDDFILSPVVKRSFIQSIKVINAIIKKYGLPNDIIIELAREKNSKDAQKMINEMQK
RNRQTNERIEEIIRTTGKENAKYLIEKIKLHDMQEGKCLYSLEAIPLEDLLNNPFNYEVDHI
IPRSVSFDNSFNNKVLVKQEENSKKGNRTPFQYLSSSDSKISYETFKKHILNLAKGKGRISK
TKKEYLLEERDINRFSVQKDFINRNLVDTRYATRGLMNLLRSYFRVNNLDVKVKSINGGFTS
FLRRKWKFKKERNKGYKHHAEDALIIANADFIFKEWKKLDKAKKVMENQMFEEKQAESMPEI
ETEQEYKEIFITPHQIKHIKDFKDYKYSHRVDKKPNRELINDTLYSTRKDDKGNTLIVNNLN
 
GLYDKDNDKLKKLINKSPEKLLMYHHDPQTYQKLKLIMEQYGDEKNPLYKYYEETGNYLTKY
SKKDNGPVIKKIKYYGNKLNAHLDITDDYPNSRNKVVKLSLKPYRFDVYLDNGVYKFVTVKN
LDVIKKENYYEVNSKCYEEAKKLKKISNQAEFIASFYNNDLIKINGELYRVIGVNNDLLNRI
EVNMIDITYREYLENMNDKRPPRIIKTIASKTQSIKKYSTDILGNLYEVKSKKHPQIIKKG
SEQ ID NO: 305
SEQ ID NO: 306 
MARILAFDIGISSIGWAFSENDELKDCGVRIFTKVENPKTGESLALPRRLARSARKRLARRK
ARLNHLKHLIANEFKLNYEDYQSFDESLAKAYKGSLISPYELRFRALNELLSKQDFARVILH
IAKRRGYDDIKNSDDKEKGAILKAIKQNEEKLANYQSVGEYLYKEYFQKFKENSKEFTNVRN
KKESYERCIAQSFLKDELKLIFKKQREFGFSFSKKFEEEVLSVAFYKRALKDFSHLVGNCSF
FTDEKRAPKNSPLAFMFVALTRIINLLNNLKNTEGILYTKDDLNALLNEVLKNGTLTYKQTK
KLLGLSDDYEFKGEKGTYFIEFKKYKEFIKALGEHNLSQDDLNEIAKDITLIKDEIKLKKAL
AKYDLNQNQIDSLSKLEFKDHLNISFKALKLVTPLMLEGKKYDEACNELNLKVAINEDKKDF
LPAFNETYYKDEVTNPVVLRAIKEYRKVLNALLKKYGKVHKINIELAREVGKNHSQRAKIEK
EQNENYKAKKDAELECEKLGLKINSKNILKLRLFKEQKEFCAYSGEKIKISDLQDEKMLEID
HIYPYSRSFDDSYMNKVLVFTKQNQEKLNQTPFEAFGNDSAKWQKIEVLAKNLPTKKQKRIL
DKNYKDKEQKNFKDRNLNDTRYIARLVLNYTKDYLDFLPLSDDENTKLNDTQKGSKVHVEAK
SGMLTSALRHTWGFSAKDRNNHLHHAIDAVIIAYANNSIVKAFSDFKKEQESNSAELYAKKI
SELDYKNKRKFFEPFSGFRQKVLDKIDEIFVSKPERKKPSGALHEETFRKEEEFYQSYGGKE
GVLKALELGKIRKVNGKIVKNGDMFRVDIFKHKKTNKFYAVPIYTMDFALKVLPNKAVARSK
KGEIKDWILMDENYEFCFSLYKDSLILIQTKDMQEPEFVYYNAFTSSTVSLIVSKHDNKFET
LSKNQKILFKNANEKEVIAKSIGIQNLKVFEKYIVSALGEVTKAEFRQREDFKK
SEQ ID NO: 307 
MKRILGLDLGTNSIGWALVNEAENKDERSSIVKLGVRVNPLTVDELTNFEKGKSITTNADRT
LKRGMRRNLQRYKLRRETLTEVLKEHKLITEDTILSENGNRTTFETYRLRAKAVTEEISLEE
 
FARVLLMINKKRGYKSSRKAKGVEEGTLIDGMDIARELYNNNLTPGELCLQLLDAGKKFLPD
FYRSDLQNELDRIWEKQKEYYPEILTDVLKEELRGKKRDAVWAICAKYFVWKENYTEWNKEK
GKTEQQEREHKLEGIYSKRKRDEAKRENLQWRVNGLKEKLSLEQLVIVFQEMNTQINNSSGY
LGAISDRSKELYFNKQTVGQYQMEMLDKNPNASLRNMVFYRQDYLDEFNMLWEKQAVYHKEL
TEELKKEIRDIIIFYQRRLKSQKGLIGFCEFESRQIEVDIDGKKKIKTVGNRVISRSSPLFQ
EFKIWQILNNIEVTVVGKKRKRRKLKENYSALFEELNDAEQLELNGSRRLCQEEKELLAQEL
FIRDKMTKSEVLKLLFDNPQELDLNFKTIDGNKTGYALFQAYSKMIEMSGHEPVDFKKPVEK
VVEYIKAVFDLLNWNTDILGFNSNEELDNQPYYKLWHLLYSFEGDNTPTGNGRLIQKMTELY
GFEKEYATILANVSFQDDYGSLSAKAIHKILPHLKEGNRYDVACVYAGYRHSESSLTREEIA
NKVLKDRLMLLPKNSLHNPVVEKILNQMVNVINVIIDIYGKPDEIRVELARELKKNAKEREE
LTKSIAQTTKAHEEYKTLLQTEFGLTNVSRTDILRYKLYKELESCGYKTLYSNTYISREKLF
SKEFDIEHIIPQARLFDDSFSNKTLEARSVNIEKGNKTAYDFVKEKFGESGADNSLEHYLNN
IEDLFKSGKISKTKYNKLKMAEQDIPDGFIERDLRNTQYIAKKALSMLNEISHRVVATSGSV
TDKLREDWQLIDVMKELNWEKYKALGLVEYFEDRDGRQIGRIKDWTKRNDHRHHAMDALTVA
FTKDVFIQYFNNKNASLDPNANEHAIKNKYFQNGRAIAPMPLREFRAEAKKHLENTLISIKA
KNKVITGNINKTRKKGGVNKNMQQTPRGQLHLETIYGSGKQYLTKEEKVNASFDMRKIGTVS
KSAYRDALLKRLYENDNDPKKAFAGKNSLDKQPIWLDKEQMRKVPEKVKIVTLEAIYTIRKE
ISPDLKVDKVIDVGVRKILIDRLNEYGNDAKKAFSNLDKNPIWLNKEKGISIKRVTISGISN
AQSLHVKKDKDGKPILDENGRNIPVDFVNTGNNHHVAVYYRPVIDKRGQLVVDEAGNPKYEL
EEVVVSFFEAVTRANLGLPIIDKDYKTTEGWQFLFSMKQNEYFVFPNEKTGFNPKEIDLLDV
ENYGLISPNLFRVQKFSLKNYVFRHHLETTIKDTSSILRGITWIDFRSSKGLDTIVKVRVNH
IGQIVSVGEY
SEQ ID NO: 308 
MSRKNYVDDYAISLDIGNASVGWSAFTPNYRLVRAKGHELIGVRLFDPADTAESRRMARTTR
RRYSRRRWRLRLLDALFDQALSEIDPSFLARRKYSWVHPDDENNADCWYGSVLFDSNEQDKR
FYEKYPTIYHLRKALMEDDSQHDIREIYLAIHHMVKYRGNFLVEGTLESSNAFKEDELLKLL
GRITRYEMSEGEQNSDIEQDDENKLVAPANGQLADALCATRGSRSMRVDNALEALSAVNDLS
REQRAIVKAIFAGLEGNKLDLAKIFVSKEFSSENKKILGIYFNKSDYEEKCVQIVDSGLLDD
EEREFLDRMQGQYNAIALKQLLGRSTSVSDSKCASYDAHRANWNLIKLQLRTKENEKDINEN
YGILVGWKIDSGQRKSVRGESAYENMRKKANVFFKKMIETSDLSETDKNRLIHDIEEDKLFP
IQRDSDNGVIPHQLHQNELKQIIKKQGKYYPFLLDAFEKDGKQINKIEGLLTFRVPYFVGPL
VVPEDLQKSDNSENHWMVRKKKGEITPWNFDEMVDKDASGRKFIERLVGTDSYLLGEPTLPK
NSLLYQEYEVLNELNNVRLSVRTGNHWNDKRRMRLGREEKTLLCQRLFMKGQTVTKRTAENL
LRKEYGRTYELSGLSDESKFTSSLSTYGKMCRIFGEKYVNEHRDLMEKIVELQTVFEDKETL
LHQLRQLEGISEADCALLVNTHYTGWGRLSRKLLTTKAGECKISDDFAPRKHSIIEIMRAED
RNLMEIITDKQLGFSDWIEQENLGAENGSSLMEVVDDLRVSPKVKRGIIQSIRLIDDISKAV
GKRPSRIFLELADDIQPSGRTISRKSRLQDLYRNANLGKEFKGIADELNACSDKDLQDDRLF
LYYTQLGKDMYTGEELDLDRLSSAYDIDHIIPQAVTQNDSIDNRVLVARAENARKTDSFTYM
PQIADRMRNFWQILLDNGLISRVKFERLTRQNEFSEREKERFVQRSLVETRQIMKNVATLMR
QRYGNSAAVIGLNAELTKEMHRYLGFSHKNRDINDYHHAQDALCVGIAGQFAANRGFFADGE
VSDGAQNSYNQYLRDYLRGYREKLSAEDRKQGRAFGFIVGSMRSQDEQKRVNPRTGEVVWSE
EDKDYLRKVMNYRKMLVTQKVGDDFGALYDETRYAATDPKGIKGIPFDGAKQDTSLYGGFSS
AKPAYAVLIESKGKTRLVNVTMQEYSLLGDRPSDDELRKVLAKKKSEYAKANILLRHVPKMQ
LIRYGGGLMVIKSAGELNNAQQLWLPYEEYCYFDDLSQGKGSLEKDDLKKLLDSILGSVQCL
YPWHRFTEEELADLHVAFDKLPEDEKKNVITGIVSALHADAKTANLSIVGMTGSWRRMNNKS
GYTFSDEDEFIFQSPSGLFEKRVTVGELKRKAKKEVNSKYRTNEKRLPTLSGASQP
SEQ ID NO: 309 
METQTSNQLITSHLKDYPKQDYFVGLDIGTNSVGWAVTNTSYELLKFHSHKMWGSRLFEEGE
SAVTRRGFRSMRRRLERRKLRLKLLEELFADAMAQVDSTFFIRLHESKYHYEDKTTGHSSKH
 
ILFIDEDYTDQDYFTEYPTIYHLRKDLMENGTDDIRKLFLAVHHILKYRGNFLYEGATFNSN
AFTFEDVLKQALVNITFNCFDTNSAISSISNILMESGKTKSDKAKAIERLVDTYTVFDEVNT
PDKPQKEQVKEDKKTLKAFANLVLGLSANLIDLFGSVEDIDDDLKKLQIVGDTYDEKRDELA
KVWGDEIHIIDDCKSVYDAIILMSIKEPGLTISQSKVKAFDKHKEDLVILKSLLKLDRNVYN
EMFKSDKKGLHNYVHYIKQGRTEETSCSREDFYKYTKKIVEGLADSKDKEYILNEIELQTLL
PLQRIKDNGVIPYQLHLEELKVILDKCGPKFPFLHTVSDGFSVTEKLIKMLEFRIPYYVGPL
NTHHNIDNGGFSWAVRKQAGRVTPWNFEEKIDREKSAAAFIKNLTNKCTYLFGEDVLPKSSL
LYSEFMLLNELNNVRIDGKALAQGVKQHLIDSIFKQDHKKMTKNRIELFLKDNNYITKKHKP
EITGLDGEIKNDLTSYRDMVRILGNNFDVSMAEDIITDITIFGESKKMLRQTLRNKFGSQLN
DETIKKLSKLRYRDWGRLSKKLLKGIDGCDKAGNGAPKTIIELMRNDSYNLMEILGDKFSFM
ECIEEENAKLAQGQVVNPHDIIDELALSPAVKRAVWQALRIVDEVAHIKKALPSRIFVEVAR
TNKSEKKKKDSRQKRLSDLYSAIKKDDVLQSGLQDKEFGALKSGLANYDDAALRSKKLYLYY
TQMGRCAYTGNIIDLNQLNTDNYDIDHIYPRSLTKDDSFDNLVLCERTANAKKSDIYPIDNR
IQTKQKPFWAFLKHQGLISERKYERLTRIAPLTADDLSGFIARQLVETNQSVKATTTLLRRL
YPDIDVVFVKAENVSDFRHNNNFIKVRSLNHHHAKDAYLNIVVGNVYHEKFTRNFRLFFKK
NGANRTYNLAKMFNYDVICTNAQDGKAWDVKTSMNTVKKMMASNDVRVTRRLLEQSGALADA
TIYKASVAAKAKDGAYIGMKTKYSVFADVTKYGGMTKIKNAYSIIVQYTGKKGEEIKEIVPL
PIYLINRNATDIELIDYVKSVIPKAKDISIKYRKLCINQLVKVNGFYYYLGGKTNDKIYIDN
AIELVVPHDIATYIKLLDKYDLLRKENKTLKASSITTSIYNINTSTVVSLNKVGIDVFDYFM
SKLRTPLYMKMKGNKVDELSSTGRSKFIKMTLEEQSIYLLEVLNLLTNSKTTFDVKPLGITG
SRSTIGVKIHNLDEFKIINESITGLYSNEVTIV
SEQ ID NO: 310 
MTKLNQPYGIGLDIGSNSIGFAVVDANSHLLRLKGETAIGARLFREGQSAADRRGSRTTRRR
LSRTRWRLSFLRDFFAPHITKIDPDFFLRQKYSEISPKDKDRFKYEKRLFNDRTDAEFYEDY
PSMYHLRLHLMTHTHKADPREIFLAIHHILKSRGHFLTPGAAKDFNTDKVDLEDIFPALTEA
YAQVYPDLELTFDLAKADDFKAKLLDEQATPSDTQKALVNLLLSSDGEKEIVKKRKQVLTEF
AKAITGLKTKFNLALGTEVDEADASNWQFSMGQLDDKWSNIETSMTDQGTEIFEQIQELYRA
RLLNGIVPAGMSLSQAKVADYGQHKEDLELFKTYLKKLNDHELAKTIRGLYDRYINGDDAKP
FLREDFVKALTKEVTAHPNEVSEQLLNRMGQANFMLKQRTKANGAIPIQLQQRELDQIIANQ
SKYYDWLAAPNPVEAHRWKMPYQLDELLNFHIPYYVGPLITPKQQAESGENVFAWMVRKDPS
GNITPYNFDEKVDREASANTFIQRMKTTDTYLIGEDVLPKQSLLYQKYEVLNELNNVRINNE
CLGTDQKQRLIREVFERHSSVTIKQVADNLVAHGDFARRPEIRGLADEKRFLSSLSTYHQLK
EILHEAIDDPTKLLDIENIITWSTVFEDHTIFETKLAEIEWLDPKKINELSGIRYRGWGQFS
RKLLDGLKLGNGHTVIQELMLSNHNLMQILADETLKETMTELNQDKLKTDDIEDVINDAYTS
PSNKKALRQVLRVVEDIKHAANGQDPSWLFIETADGTGTAGKRTQSRQKQIQTVYANAAQEL
IDSAVRGELEDKIADKASFTDRLVLYFMQGGRDIYTGAPLNIDQLSHYDIDHILPQSLIKDD
SLDNRVLVNATINREKNNVFASTLFAGKMKATWRKWHEAGLISGRKLRNLMLRPDEIDKFAK
GFVARQLVETRQIIKLTEQIAAAQYPNTKIIAVKAGLSHQLREELDFPKNRDVNHYHHAFDA
FLAARIGTYLLKRYPKLAPFFTYGEFAKVDVKKFREFNFIGALTHAKKNIIAKDTGEIVWDK
ERDIRELDRIYNFKRMLITHEVYFETADLFKQTIYAAKDSKERGGSKQLIPKKQGYPTQVYG
GYTQESGSYNALVRVAEADTTAYQVIKISAQNASKIASANLKSREKGKQLLNEIVVKQLAKR
RKNWKPSANSFKIVIPRFGMGTLFQNAKYGLFMVNSDTYYRNYQELWLSRENQKLLKKLFSI
KYEKTQMNHDALQVYKAIIDQVEKFFKLYDINQFRAKLSDAIERFEKLPINTDGNKIGKTET
LRQILIGLQANGTRSNVKNLGIKTDLGLLQVGSGIKLDKDTQIVYQSPSGLFKRRIPLADL
SEQ ID NO: 311 
MTKEYYLGLDVGTNSVGWAVTDSQYNLCKFKKKDMWGIRLFESANTAKDRRLQRGNRRRLER
KKQRIDLLQEIFSPEICKIDPTFFIRLNESRLHLEDKSNDFKYPLFIEKDYSDIEYYKEFPT
IFHLRKHLIESEEKQDIRLIYLALHNIIKTRGHFLIDGDLQSAKQLRPILDTFLLSLQEEQN
LSVSLSENQKDEYEEILKNRSIAKSEKVKKLKNLFEISDELEKEEKKAQSAVIENFCKFIVG
 
NKGDVCKFLRVSKEELEIDSFSFSEGKYEDDIVKNLEEKVPEKVYLFEQMKAMYDWNILVDI
LETEEYISFAKVKQYEKHKTNLRLLRDIILKYCTKDEYNRMFNDEKEAGSYTAYVGKLKKNN
KKYWIEKKRNPEEFYKSLGKLLDKIEPLKEDLEVLTMMIEECKNHTLLPIQKNKDNGVIPHQ
VHEVELKKILENAKKYYSFLTETDKDGYSVVQKIESIFRFRIPYYVGPLSTRHQEKGSNVWM
VRKPGREDRIYPWNMEEIIDFEKSNENFITRMTNKCTYLIGEDVLPKHSLLYSKYMVLNELN
NVKVRGKKLPTSLKQKVFEDLFENKSKVTGKNLLEYLQIQDKDIQIDDLSGFDKDFKTSLKS
YLDFKKQIFGEEIEKESIQNMIEDIIKWITIYGNDKEMLKRVIRANYSNQLTEEQMKKITGF
QYSGWGNFSKMFLKGISGSDVSTGETFDIITAMWETDNNLMQILSKKFTFMDNVEDFNSGKV
GKIDKITYDSTVKEMFLSPENKRAVWQTIQVAEEIKKVMGCEPKKIFIEMARGGEKVKKRTK
SRKAQLLELYAACEEDCRELIKEIEDRDERDFNSMKLFLYYTQFGKCMYSGDDIDINELIRG
NSKWDRDHIYPQSKIKDDSIDNLVLVNKTYNAKKSNELLSEDIQKKMHSFWLSLLNKKLITK
SKYDRLTRKGDFTDEELSGFIARQLVETRQSTKAIADIFKQIYSSEVVYVKSSLVSDFRKKP
LNYLKSRRVNDYHHAKDAYLNIVVGNVYNKKFTSNPIQWMKKNRDTNYSLNKVFEHDVVING
EVIWEKCTYHEDTNTYDGGTLDRIRKIVERDNILYTEYAYCEKGELFNATIQNKNGNSTVSL
KKGLDVKKYGGYFSANTSYFSLIEFEDKKGDRARHIIGVPIYIANMLEHSPSAFLEYCEQKG
YQNVRILVEKIKKNSLLIINGYPLRIRGENEVDTSFKRAIQLKLDQKNYELVRNIEKFLEKY
VEKKGNYPIDENRDHITHEKMNQLYEVLLSKMKKFNKKGMADPSDRIEKSKPKFIKLEDLID
KINVINKMLNLLRCDNDTKADLSLIELPKNAGSFVVKKNTIGKSKIILVNQSVTGLYENRRE
L
SEQ ID NO: 312 
MARDYSVGLDIGTSSVGWAAIDNKYHLIRAKSKNLIGVRLFDSAVTAEKRRGYRTTRRRLSR
RHWRLRLLNDIFAGPLTDFGDENFLARLKYSWVHPQDQSNQAHFAAGLLFDSKEQDKDFYRK
YPTIYHLRLALMNDDQKHDLREVYLAIHHLVKYRGHFLIEGDVKADSAFDVHTFADAIQRYA
ESNNSDENLLGKIDEKKLSAALTDKHGSKSQRAETAETAFDILDLQSKKQIQAILKSVVGNQ
ANLMAIFGLDSSAISKDEQKNYKFSFDDADIDEKIADSEALLSDTEFEFLCDLKAAFDGLTL
KMLLGDDKTVSAAMVRRFNEHQKDWEYIKSHIRNAKNAGNGLYEKSKKFDGINAAYLALQSD
NEDDRKKAKKIFQDEISSADIPDDVKADFLKKIDDDQFLPIQRTKNNGTIPHQLHRNELEQI
IEKQGIYYPFLKDTYQENSHELNKITALINFRVPYYVGPLVEEEQKIADDGKNIPDPTNHWM
VRKSNDTITPWNLSQVVDLDKSGRRFIERLTGTDTYLIGEPTLPKNSLLYQKFDVLQELNNI
RVSGRRLDIRAKQDAFEHLFKVQKTVSATNLKDFLVQAGYISEDTQIEGLADVNGKNFNNAL
TTYNYLVSVLGREFVENPSNEELLEEITELQTVFEDKKVLRRQLDQLDGLSDHNREKLSRKH
YTGWGRISKKLLTTKIVQNADKIDNQTFDVPRMNQSIIDTLYNTKMNLMEIINNAEDDFGVR
AWIDKQNTTDGDEQDVYSLIDELAGPKEIKRGIVQSFRILDDITKAVGYAPKRVYLEFARKT
QESHLTNSRKNQLSTLLKNAGLSELVTQVSQYDAAALQNDRLYLYFLQQGKDMYSGEKLNLD
NLSNYDIDHIIPQAYTKDNSLDNRVLVSNITNRRKSDSSNYLPALIDKMRPFWSVLSKQGLL
SKHKFANLTRTRDFDDMEKERFIARSLVETRQIIKNVASLIDSHFGGETKAVAIRSSLTADM
RRYVDIPKNRDINDYHHAFDALLFSTVGQYTENSGLMKKGQLSDSAGNQYNRYIKEWIHAAR
LNAQSQRVNPFGFVVGSMRNAAPGKLNPETGEITPEENADWSIADLDYLHKVMNFRKITVTR
RLKDQKGQLYDESRYPSVLHDAKSKASINFDKHKPVDLYGGFSSAKPAYAALIKFKNKFRLV
NVLRQWTYSDKNSEDYILEQIRGKYPKAEMVLSHIPYGQLVKKDGALVTISSATELHNFEQL
WLPLADYKLINTLLKTKEDNLVDILHNRLDLPEMTIESAFYKAFDSILSFAFNRYALHQNAL
VKLQAHRDDFNALNYEDKQQTLERILDALHASPASSDLKKINLSSGFGRLFSPSHFTLADTD
EFIFQSVTGLFSTQKTVAQLYQETK
SEQ ID NO: 313 
MVYDVGLDIGTGSVGWVALDENGKLARAKGKNLVGVRLFDTAQTAADRRGFRTTRRRLSRRK
WRLRLLDELFSAEINEIDSSFFQRLKYSYVHPKDEENKAHYYGGYLFPTEEETKKFHRSYPT
IYHLRQELMAQPNKRFDIREIYLAIHHLVKYRGHFLSSQEKITIGSTYNPEDLANAIEVYAD
EKGLSWELNNPEQLTEIISGEAGYGLNKSMKADEALKLFEFDNNQDKVAIKTLLAGLTGNQI
DFAKLFGKDISDKDEAKLWKLKLDDEALEEKSQTILSQLTDEEIELFHAVVQAYDGFVLIGL
 
LNGADSVSAAMVQLYDQHREDRKLLKSLAQKAGLKHKRFSEIYEQLALATDEATIKNGISTA
RELVEESNLSKEVKEDTLRRLDENEFLPKQRTKANSVIPHQLHLAELQKILQNQGQYYPFLL
DTFEKEDGQDNKIEELLRFRIPYYVGPLVTKKDVEHAGGDADNHWVERNEGFEKSRVTPWNF
DKVFNRDKAARDFIERLTGNDTYLIGEKTLPQNSLRYQLFTVLNELNNVRVNGKKFDSKTKA
DLINDLFKARKTVSLSALKDYLKAQGKGDVTITGLADESKFNSSLSSYNDLKKTFDAEYLEN
EDNQETLEKIIEIQTVFEDSKIASRELSKLPLDDDQVKKLSQTHYTGWGRLSEKLLDSKIID
ERGQKVSILDKLKSTSQNFMSIINNDKYGVQAWITEQNTGSSKLTFDEKVNELTTSPANKRG
IKQSFAVLNDIKKAMKEEPRRVYLEFAREDQTSVRSVPRYNQLKEKYQSKSLSEEAKVLKKT
LDGNKNKMSDDRYFLYFQQQGKDMYTGRPINFERLSQDYDIDHIIPQAFTKDDSLDNRVLVS
RPENARKSDSFAYTDEVQKQDGSLWTSLLKSGFINRKKYERLTKAGKYLDGQKTGFIARQLV
ETRQIIKNVASLIEGEYENSKAVAIRSEITADMRLLVGIKKHREINSFHHAFDALLITAAGQ
YMQNRYPDRDSTNVYNEFDRYTNDYLKNLRQLSSRDEVRRLKSFGFVVGTMRKGNEDWSEEN
TSYLRKVMMFKNILTTKKTEKDRGPLNKETIFSPKSGKKLIPLNSKRSDTALYGGYSNVYSA
YMTLVRANGKNLLIKIPISIANQIEVGNLKINDYIVNNPAIKKFEKILISKLPLGQLVNEDG
NLIYLASNEYRHNAKQLWLSTTDADKIASISENSSDEELLEAYDILTSENVKNRFPFFKKDI
DKLSQVRDEFLDSDKRIAVIQTILRGLQIDAAYQAPVKIISKKVSDWHKLQQSGGIKLSDNS
EMIYQSATGIFETRVKISDLL
SEQ ID NO: 314 
IVDYCIGLDLGTGSVGWAVVDMNHRLMKRNGKHLWGSRLFSNAETAANRRASRSIRRRYNKR
RERIRLLRAILQDMVLEKDPTFFIRLEHTSFLDEEDKAKYLGTDYKDNYNLFIDEDFNDYTY
YHKYPTIYHLRKALCESTEKADPRLIYLALHHIVKYRGNFLYEGQKFNMDASNIEDKLSDIF
TQFTSFNNIPYEDDEKKNLEILEILKKPLSKKAKVDEVMTLIAPEKDYKSAFKELVTGIAGN
KMNVTKMILCEPIKQGDSEIKLKFSDSNYDDQFSEVEKDLGEYVEFVDALHNVYSWVELQTI
MGATHTDNASISEAMVSRYNKHHDDLKLLKDCIKNNVPNKYFDMFRNDSEKSKGYYNYINRP
SKAPVDEFYKYVKKCIEKVDTPEAKQILNDIELENFLLKQNSRTNGSVPYQMQLDEMIKIID
NQAEYYPILKEKREQLLSILTFRIPYYFGPLNETSEHAWIKRLEGKENQRILPWNYQDIVDV
DATAEGFIKRMRSYCTYFPDEEVLPKNSLIVSKYEVYNELNKIRVDDKLLEVDVKNDIYNEL
FMKNKTVTEKKLKNWLVNNQCCSKDAEIKGFQKENQFSTSLTPWIDFTNIFGKIDQSNFDLI
ENIIYDLTVFEDKKIMKRRLKKKYALPDDKVKQILKLKYKDWSRLSKKLLDGIVADNRFGSS
VTVLDVLEMSRLNLMEIINDKDLGYAQMIEEATSCPEDGKFTYEEVERLAGSPALKRGIWQS
LQIVEEITKVMKCRPKYIYIEFERSEEAKERTESKIKKLENVYKDLDEQTKKEYKSVLEELK
GFDNTKKISSDSLFLYFTQLGKCMYSGKKLDIDSLDKYQIDHIVPQSLVKDDSFDNRVLVVP
SENQRKLDDLVVPFDIRDKMYRFWKLLFDHELISPKKFYSLIKTEYTERDEERFINRQLVET
RQITKNVTQIIEDHYSTTKVAAIRANLSHEFRVKNHIYKNRDINDYHHAHDAYIVALIGGFM
RDRYPNMHDSKAVYSEYMKMFRKNKNDQKRWKDGFVINSMNYPYEVDGKLIWNPDLINEIKK
CFYYKDCYCTTKLDQKSGQLFNLTVLSNDAHADKGVTKAVVPVNKNRSDVHKYGGFSGLQYT
IVAIEGQKKKGKKTELVKKISGVPLHLKAASINEKINYIEEKEGLSDVRIIKDNIPVNQMIE
MDGGEYLLTSPTEYVNARQLVLNEKQCALIADIYNAIYKQDYDNLDDILMIQLYIELTNKMK
VLYPAYRGIAEKFESMNENYVVISKEEKANIIKQMLIVMHRGPQNGNIVYDDFKISDRIGRL
KTKNHNLNNIVFISQSPTGIYTKKYKL
SEQ ID NO: 315 
MKSEKKYYIGLDVGTNSVGWAVTDEFYNILRAKGKDLWGVRLFEKADTAANTRIFRSGRRRN
DRKGMRLQILREIFEDEIKKVDKDFYDRLDESKFWAEDKKVSGKYSLFNDKNFSDKQYFEKF
PTIFHLRKYLMEEHGKVDIRYYFLAINQMMKRRGHFLIDGQISHVTDDKPLKEQLILLINDL
LKIELEEELMDSIFEILADVNEKRTDKKNNLKELIKGQDFNKQEGNILNSIFESIVTGKAKI
KNIISDEDILEKIKEDNKEDFVLTGDSYEENLQYFEEVLQENITLFNTLKSTYDFLILQSIL
KGKSTLSDAQVERYDEHKKDLEILKKVIKKYDEDGKLFKQVFKEDNGNGYVSYIGYYLNKNK
KITAKKKISNIEFTKYVKGILEKQCDCEDEDVKYLLGKIEQENFLLKQISSINSVIPHQIHL
FELDKILENLAKNYPSFNNKKEEFTKIEKIRKTFTFRIPYYVGPLNDYHKNNGGNAWIFRNK
 
GEKIRPWNFEKIVDLHKSEEEFIKRMLNQCTYLPEETVLPKSSILYSEYMVLNELNNLRING
KPLDTDVKLKLIEELFKKKTKVTLKSIRDYMVRNNFADKEDFDNSEKNLEIASNMKSYIDFN
NILEDKFDVEMVEDLIEKITIHTGNKKLLKKYIEETYPDLSSSQIQKIINLKYKDWGRLSRK
LLDGIKGTKKETEKTDTVINFLRNSSDNLMQIIGSQNYSFNEYIDKLRKKYIPQEISYEVVE
NLYVSPSVKKMIWQVIRVTEEITKVMGYDPDKIFIEMAKSEEEKKTTISRKNKLLDLYKAIK
KDERDSQYEKLLTGLNKLDDSDLRSRKLYLYYTQMGRDMYTGEKIDLDKLFDSTHYDKDHII
PQSMKKDDSIINNLVLVNKNANQTTKGNIYPVPSSIRNNPKIYNYWKYLMEKEFISKEKYNR
LIRNTPLTNEELGGFINRQLVETRQSTKAIKELFEKFYQKSKIIPVKASLASDLRKDMNTLK
SREVNDLHHAHDAFLNIVAGDVWNREFTSNPINYVKENREGDKVKYSLSKDFTRPRKSKGKV
IWTPEKGRKLIVDTLNKPSVLISNESHVKKGELFNATIAGKKDYKKGKIYLPLKKDDRLQDV
SKYGGYKAINGAFFFLVEHTKSKKRIRSIELFPLHLLSKFYEDKNTVLDYAINVLQLQDPKI
IIDKINYRTEIIIDNFSYLISTKSNDGSITVKPNEQMYWRVDEISNLKKIENKYKKDAILTE
EDRKIMESYIDKIYQQFKAGKYKNRRTTDTIIEKYEIIDLDTLDNKQLYQLLVAFISLSYKT
SNNAVDFTVIGLGTECGKPRITNLPDNTYLVYKSITGIYEKRIRIK
SEQ ID NO: 316 
MKLRGIEDDYSIGLDMGTSSVGWAVTDERGTLAHFKRKPTWGSRLFREAQTAAVARMPRGQR
RRYVRRRWRLDLLQKLFEQQMEQADPDFFIRLRQSRLLRDDRAEEHADYRWPLFNDCKFTER
DYYQRFPTIYHVRSWLMETDEQADIRLIYLALHNIVKHRGNFLREGQSLSAKSARPDEALNH
LRETLRVWSSERGFECSIADNGSILAMLTHPDLSPSDRRKKIAPLFDVKSDDAAADKKLGIA
LAGAVIGLKTEFKNIFGDFPCEDSSIYLSNDEAVDAVRSACPDDCAELFDRLCEVYSAYVLQ
GLLSYAPGQTISANMVEKYRRYGEDLALLKKLVKIYAPDQYRMFFSGATYPGTGIYDAAQAR
GYTKYNLGPKKSEYKPSESMQYDDFRKAVEKLFAKTDARADERYRMMMDRFDKQQFLRRLKT
SDNGSIYHQLHLEELKAIVENQGRFYPFLKRDADKLVSLVSFRIPYYVGPLSTRNARTDQHG
ENRFAWSERKPGMQDEPIFPWNWESIIDRSKSAEKFILRMTGMCTYLQQEPVLPKSSLLYEE
FCVLNELNGAHWSIDGDDEHRFDAADREGIIEELFRRKRTVSYGDVAGWMERERNQIGAHVC
GGQGEKGFESKLGSYIFFCKDVFKVERLEQSDYPMIERIILWNTLFEDRKILSQRLKEEYGS
RLSAEQIKTICKKRFTGWGRLSEKFLTGITVQVDEDSVSIMDVLREGCPVSGKRGRAMVMME
ILRDEELGFQKKVDDFNRAFFAENAQALGVNELPGSPAVRRSLNQSIRIVDEIASIAGKAPA
NIFIEVTRDEDPKKKGRRTKRRYNDLKDALEAFKKEDPELWRELCETAPNDMDERLSLYFMQ
RGKCLYSGRAIDIHQLSNAGIYEVDHIIPRTYVKDDSLENKALVYREENQRKTDMLLIDPEI
RRRMSGYWRMLHEAKLIGDKKFRNLLRSRIDDKALKGFIARQLVETGQMVKLVRSLLEARYP
ETNIISVKASISHDLRTAAELVKCREANDFHHAHDAFLACRVGLFIQKRHPCVYENPIGLSQ
VVRNYVRQQADIFKRCRTIPGSSGFIVNSFMTSGFDKETGEIFKDDWDAEAEVEGIRRSLNF
RQCFISRMPFEDHGVFWDATIYSPRAKKTAALPLKQGLNPSRYGSFSREQFAYFFIYKARNP
RKEQTLFEFAQVPVRLSAQIRQDENALERYARELAKDQGLEFIRIERSKILKNQLIEIDGDR
LCITGKEEVRNACELAFAQDEMRVIRMLVSEKPVSRECVISLFNRILLHGDQASRRLSKQLK
LALLSEAFSEASDNVQRNVVLGLIAIFNGSTNMVNLSDIGGSKFAGNVRIKYKKELASPKVN
VHLIDQSVTGMFERRTKIGL
SEQ ID NO: 317 
MENKQYYIGLDVGTNSVGWAVTDTSYNLLRAKGKDMWGARLFEKANTAAERRTKRTSRRRSE
REKARKAMLKELFADEINRVDPSFFIRLEESKFFLDDRSENNRQRYTLFNDATFTDKDYYEK
YKTIFHLRSALINSDEKFDVRLVFLAILNLFSHRGHFLNASLKGDGDIQGMDVFYNDLVESC
EYFEIELPRITNIDNFEKILSQKGKSRTKILEELSEELSISKKDKSKYNLIKLISGLEASVV
ELYNIEDIQDENKKIKIGFRESDYEESSLKVKEIIGDEYFDLVERAKSVHDMGLLSNIIGNS
KYLCEARVEAYENHHKDLLKIKELLKKYDKKAYNDMFRKMTDKNYSAYVGSVNSNIAKERRS
VDKRKIEDLYKYIEDTALKNIPDDNKDKIEILEKIKLGEFLKKQLTASNGVIPNQLQSRELR
AILKKAENYLPFLKEKGEKNLTVSEMIIQLFEFQIPYYVGPLDKNPKKDNKANSWAKIKQGG
RILPWNFEDKVDVKGSRKEFIEKMVRKCTYISDEHTLPKQSLLYEKFMVLNEINNIKIDGEK
ISVEAKQKIYNDLFVKGKKVSQKDIKKELISLNIMDKDSVLSGTDTVCNAYLSSIGKFTGVF
 
KEEINKQSIVDMIEDIIFLKTVYGDEKRFVKEEIVEKYGDEIDKDKIKRILGFKFSNWGNLS
KSFLELEGADVGTGEVRSIIQSLWETNFNLMELLSSRFTYMDELEKRVKKLEKPLSEWTIED
LDDMYLSSPVKRMIWQSMKIVDEIQTVIGYAPKRIFVEMTRSEGEKVRTKSRKDRLKELYNG
IKEDSKQWVKELDSKDESYFRSKKMYLYYLQKGRCMYSGEVIELDKLMDDNLYDIDHIYPRS
FVKDDSLDNLVLVKKEINNRKQNDPITPQIQASCQGFWKILHDQGFMSNEKYSRLTRKTQEF
SDEEKLSFINRQIVETGQATKCMAQILQKSMGEDVDVVFSKARLVSEFRHKFELFKSRLIND
FHHANDAYLNIVVGNSYFVKFTRNPANFIKDARKNPDNPVYKYHMDRFFERDVKSKSEVAWI
GQSEGNSGTIVIVKKTMAKNSPLITKKVEEGHGSITKETIVGVKEIKFGRNKVEKADKTPKK
PNLQAYRPIKTSDERLCNILRYGGRTSISISGYCLVEYVKKRKTIRSLEAIPVYLGRKDSLS
EEKLLNYFRYNLNDGGKDSVSDIRLCLPFISTNSLVKIDGYLYYLGGKNDDRIQLYNAYQLK
MKKEEVEYIRKIEKAVSMSKFDEIDREKNPVLTEEKNIELYNKIQDKFENTVFSKRMSLVKY
NKKDLSFGDFLKNKKSKFEEIDLEKQCKVLYNIIFNLSNLKEVDLSDIGGSKSTGKCRCKKN
ITNYKEFKLIQQSITGLYSCEKDLMTI
SEQ ID NO: 318 
MKNLKEYYIGLDIGTASVGWAVTDESYNIPKFNGKKMWGVRLFDDAKTAEERRTQRGSRRRL
NRRKERINLLQDLFATEISKVDPNFFLRLDNSDLYREDKDEKLKSKYTLFNDKDFKDRDYHK
KYPTIHHLIMDLIEDEGKKDIRLLYLACHYLLKNRGHFIFEGQKFDTKNSFDKSINDLKIHL
RDEYNIDLEFNNEDLIEIITDTTLNKTNKKKELKNIVGDTKFLKAISAIMIGSSQKLVDLFE
DGEFEETTVKSVDFSTTAFDDKYSEYEEALGDTISLLNILKSIYDSSILENLLKDADKSKDG
NKYISKAFVKKFNKHGKDLKTLKRIIKKYLPSEYANIFRNKSINDNYVAYTKSNITSNKRTK
ASKFTKQEDFYKFIKKHLDTIKETKLNSSENEDLKLIDEMLTDIEFKTFIPKLKSSDNGVIP
YQLKLMELKKILDNQSKYYDFLNESDEYGTVKDKVESIMEFRIPYYVGPLNPDSKYAWIKRE
NTKITPWNFKDIVDLDSSREEFIDRLIGRCTYLKEEKVLPKASLIYNEFMVLNELNNLKLNE
FLITEEMKKAIFEELFKTKKKVTLKAVSNLLKKEFNLTGDILLSGTDGDFKQGLNSYIDFKN
IIGDKVDRDDYRIKIEEIIKLIVLYEDDKTYLKKKIKSAYKNDFTDDEIKKIAALNYKDWGR
LSKRFLTGIEGVDKTTGEKGSIIYFMREYNLNLMELMSGHYTFTEEVEKLNPVENRELCYEM
VDELYLSPSVKRMLWQSLRVVDEIKRIIGKDPKKIFIEMARAKEAKNSRKESRKNKLLEFYK
FGKKAFINEIGEERYNYLLNEINSEEESKFRWDNLYLYYTQLGRCMYSLEPIDLADLKSNNI
YDQDHIYPKSKIYDDSLENRVLVKKNLNHEKGNQYPIPEKVLNKNAYGFWKILFDKGLIGQK
KYTRLTRRTPFEERELAEFIERQIVETRQATKETANLLKNICQDSEIVYSKAENASRFRQEF
DIIKCRTVNDLHHMHDAYLNIVVGNVYNTKFTKNPLNFIKDKDNVRSYNLENMFKYDVVRGS
YTAWIADDSEGNVKAATIKKVKRELEGKNYRFTRMSYIGTGGLYDQNLMRKGKGQIPQKENT
NKSNIEKYGGYNKASSAYFALIESDGKAGRERTLETIPIMVYNQEKYGNTEAVDKYLKDNLE
LQDPKILKDKIKINSLIKLDGFLYNIKGKTGDSLSIAGSVQLIVNKEEQKLIKKMDKFLVKK
KDNKDIKVTSFDNIKEEELIKLYKTLSDKLNNGIYSNKRNNQAKNISEALDKFKEISIEEKI
DVLNQIILLFQSYNNGCNLKSIGLSAKTGVVFIPKKLNYKECKLINQSITGLFENEVDLLNL
SEQ ID NO: 319 
MGKMYYLGLDIGTNSVGYAVTDPSYHLLKFKGEPMWGAHVFAAGNQSAERRSFRTSRRRLDR
RQQRVKLVQEIFAPVISPIDPRFFIRLHESALWRDDVAETDKHIFFNDPTYTDKEYYSDYPT
IHHLIVDLMESSEKHDPRLVYLAVAWLVAHRGHFLNEVDKDNIGDVLSFDAFYPEFLAFLSD
NGVSPWVCESKALQATLLSRNSVNDKYKALKSLIFGSQKPEDNFDANISEDGLIQLLAGKKV
KVNKLFPQESNDASFTLNDKEDAIEEILGTLTPDECEWIAHIRRLFDWAIMKHALKDGRTIS
ESKVKLYEQHHHDLTQLKYFVKTYLAKEYDDIFRNVDSETTKNYVAYSYHVKEVKGTLPKNK
ATQEEFCKYVLGKVKNIECSEADKVDFDEMIQRLTDNSFMPKQVSGENRVIPYQLYYYELKT
ILNKAASYLPFLTQCGKDAISNQDKLLSIMTFRIPYFVGPLRKDNSEHAWLERKAGKIYPWN
FNDKVDLDKSEEAFIRRMTNTCTYYPGEDVLPLDSLIYEKFMILNEINNIRIDGYPISVDVK
QQVFGLFEKKRRVTVKDIQNLLLSLGALDKHGKLTGIDTTIHSNYNTYHHFKSLMERGVLTR
DDVERIVERMTYSDDTKRVRLWLNNNYGTLTADDVKHISRLRKHDFGRLSKMFLTGLKGVHK
ETGERASILDFMWNTNDNLMQLLSECYTFSDEITKLQEAYYAKAQLSLNDFLDSMYISNAVK
 
RPIYRTLAVVNDIRKACGTAPKRIFIEMARDGESKKKRSVTRREQIKNLYRSIRKDFQQEVD
FLEKILENKSDGQLQSDALYLYFAQLGRDMYTGDPIKLEHIKDQSFYNIDHIYPQSMVKDDS
LDNKVLVQSEINGEKSSRYPLDAAIRNKMKPLWDAYYNHGLISLKKYQRLTRSTPFTDDEKW
DFINRQLVETRQSTKALAILLKRKFPDTEIVYSKAGLSSDFRHEFGLVKSRNINDLHHAKDA
FLAIVTGNVYHERFNRRWFMVNQPYSVKTKTLFTHSIKNGNFVAWNGEEDLGRIVKMLKQNK
NTIHFTRFSFDRKEGLFDIQPLKASTGLVPRKAGLDVVKYGGYDKSTAAYYLLVRFTLEDKK
TQHKLMMIPVEGLYKARIDHDKEFLTDYAQTTISEILQKDKQKVINIMFPMGTRHIKLNSMI
SIDGFYLSIGGKSSKGKSVLCHAMVPLIVPHKIECYIKAMESFARKFKENNKLRIVEKFDKI
TVEDNLNLYELFLQKLQHNPYNKFFSTQFDVLTNGRSTFTKLSPEEQVQTLLNILSIFKTCR
SSGCDLKSINGSAQAARIMISADLTGLSKKYSDIRLVEQSASGLFVSKSQNLLEYL
SEQ ID NO: 320 
MTKKEQPYNIGLDIGTSSVGWAVTNDNYDLLNIKKKNLWGVRLFEEAQTAKETRLNRSTRRR
YRRRKNRINWLNEIFSEELAKTDPSFLIRLQNSWVSKKDPDRKRDKYNLFIDGPYTDKEYYR
EFPTIFHLRKELILNKDKADIRLIYLALHNILKYRGNFTYEHQKFNISNLNNNLSKELIELN
QQLIKYDISFPDDCDWNHISDILIGRGNATQKSSNILKDFTLDKETKKLLKEVINLILGNVA
HLNTIFKTSLTKDEEKLNFSGKDIESKLDDLDSILDDDQFTVLDAANRIYSTITLNEILNGE
SYFSMAKVNQYENHAIDLCKLRDMWHTTKNEEAVEQSRQAYDDYINKPKYGTKELYTSLKKF
LKVALPTNLAKEAEEKISKGTYLVKPRNSENGVVPYQLNKIEMEKIIDNQSQYYPFLKENKE
KLLSILSFRIPYYVGPLQSAEKNPFAWMERKSNGHARPWNFDEIVDREKSSNKFIRRMTVTD
SYLVGEPVLPKNSLIYQRYEVLNELNNIRITENLKTNPIGSRLTVETKQRIYNELFKKYKKV
TVKKLTKWLIAQGYYKNPILIGLSQKDEFNSTLTTYLDMKKIFGSSFMEDNKNYDQIEELIE
WLTIFEDKQILNEKLHSSKYSYTPDQIKKISNMRYKGWGRLSKKILMDITTETNTPQLLQLS
NYSILDLMWATNNNFISIMSNDKYDFKNYIENHNLNKNEDQNISDLVNDIHVSPALKRGITQ
SIKIVQEIVKFMGHAPKHIFIEVTRETKKSEITTSREKRIKRLQSKLLNKANDFKPQLREYL
VPNKKIQEELKKHKNDLSSERIMLYFLQNGKSLYSEESLNINKLSDYQVDHILPRTYIPDDS
LENKALVLAKENQRKADDLLLNSNVIDRNLERWTYMLNNNMIGLKKFKNLTRRVITDKDKLG
FIHRQLVQTSQMVKGVANILDNMYKNQGTTCIQARANLSTAFRKALSGQDDTYHFKHPELVK
NRNVNDFHHAQDAYLASFLGTYRLRRFPTNEMLLMNGEYNKFYGQVKELYSKKKKLPDSRKN
GFIISPLVNGTTQYDRNTGEIIWNVGFRDKILKIFNYHQCNVTRKTEIKTGQFYDQTIYSPK
NPKYKKLIAQKKDMDPNIYGGFSGDNKSSITIVKIDNNKIKPVAIPIRLINDLKDKKTLQNW
LEENVKHKKSIQIIKNNVPIGQIIYSKKVGLLSLNSDREVANRQQLILPPEHSALLRLLQIP
DEDLDQILAFYDKNILVEILQELITKMKKFYPFYKGEREFLIANIENFNQATTSEKVNSLEE
LITLLHANSTSAHLIFNNIEKKAFGRKTHGLTLNNTDFIYQSVTGLYETRIHIE
SEQ ID NO: 321 
MTKFNKNYSIGLDIGVSSVGYAVVTEDYRVPAFKFKVLGNTEKEKIKKNLIGSTTFVSAQPA
KGTRVFRVNRRRIDRRNHRITYLRDIFQKEIEKVDKNFYRRLDESFRVLGDKSEDLQIKQPF
FGDKELETAYHKKYPTIYHLRKHLADADKNSPVADIREVYMAISHILKYRGHFLTLDKINPN
NINMQNSWIDFIESCQEVFDLEISDESKNIADIFKSSENRQEKVKKILPYFQQELLKKDSI
FKQLLQLLFGLKTKFKDCFELEEEPDLNFSKENYDENLENFLGSLEEDFSDVFAKLKVLRDT
ILLSGMLTYTGATHARFSATMVERYEEHRKDLQRFKFFIKQNLSEQDYLDIFGRKTQNGFDV
DKETKGYVGYITNKMVLTNPQKQKTIQQNFYDYISGKITGIEGAEYFLNKISDGTFLRKLRT
SDNGAIPNQIHAYELEKIIERQGKDYPFLLENKDKLLSILTFKIPYYVGPLAKGSNSRFAWI
KRATSSDILDDNDEDTRNGKIRPWNYQKLINMDETRDAFITNLIGNDIILLNEKVLPKRSLI
YEEVMLQNELTRVKYKDKYGKAHFFDSELRQNIINGLFKNNSKRVNAKSLIKYLSDNHKDLN
AIEIVSGVEKGKSFNSTLKTYNDLKTIFSEELLDSEIYQKELEEIIKVITVFDDKKSIKNYL
TKFFGHLEILDEEKINQLSKLRYSGWGRYSAKLLLDIRDEDTGFNLLQFLRNDEENRNLTKL
ISDNTLSFEPKIKDIQSKSTIEDDIFDEIKKLAGSPAIKRGILNSIKIVDELVQIIGYPPHN
IVIEMARENMTTEEGQKKAKTRKTKLESALKNIENSLLENGKVPHSDEQLQSEKLYLYYLQN
GKDMYTLDKTGSPAPLYLDQLDQYEVDHIIPYSFLPIDSIDNKVLTHRENNQQKLNNIPDKE
 
TVANMKPFWEKLYNAKLISQTKYQRLTTSERTPDGVLTESMKAGFIERQLVETRQIIKHVAR
ILDNRFSDTKIITLKSQLITNFRNTFHIAKIRELNDYHHAHDAYLAVVVGQTLLKVYPKLAP
ELIYGHHAHFNRHEENKATLRKHLYSNIMRFFNNPDSKVSKDIWDCNRDLPIIKDVIYNSQI
NFVKRTMIKKGAFYNQNPVGKFNKQLAANNRYPLKTKALCLDTSIYGGYGPMNSALSIIIIA
ERFNEKKGKIETVKEFHDIFIIDYEKFNNNPFQFLNDTSENGFLKKNNINRVLGFYRIPKYS
LMQKIDGTRMLFESKSNLHKATQFKLTKTQNELFFHMKRLLTKSNLMDLKSKSAIKESQNFI
LKHKEEFDNISNQLSAFSQKMLGNTTSLKNLIKGYNERKIKEIDIRDETIKYFYDNFIKMFS
FVKSGAPKDINDFFDNKCTVARMRPKPDKKLLNATLIHQSITGLYETRIDLSKLGED
SEQ ID NO: 322 
MKQEYFLGLDMGTGSLGWAVTDSTYQVMRKHGKALWGTRLFESASTAEERRMFRTARRRLDR
RNWRIQVLQEIFSEEISKVDPGFFLRMKESKYYPEDKRDAEGNCPELPYALFVDDNYTDKNY
HKDYPTIYHLRKMLMETTEIPDIRLVYLVLHHMMKHRGHFLLSGDISQIKEFKSTFEQLIQN
IQDEELEWHISLDDAAIQFVEHVLKDRNLTRSTKKSRLIKQLNAKSACEKAILNLLSGGTVK
LSDIFNNKELDESERPKVSFADSGYDDYIGIVEAELAEQYYIIASAKAVYDWSVLVEILGNS
VSISEAKIKVYQKHQADLKTLKKIVRQYMTKEDYKRVFVDTEEKLNNYSAYIGMTKKNGKKV
DLKSKQCTQADFYDFLKKNVIKVIDHKEITQEIESEIEKENFLPKQVTKDNGVIPYQVHDYE
LKKILDNLGTRMPFIKENAEKIQQLFEFRIPYYVGPLNRVDDGKDGKFTWSVRKSDARIYPW
NFTEVIDVEASAEKFIRRMTNKCTYLVGEDVLPKDSLVYSKFMVLNELNNLRLNGEKISVEL
KQRIYEELFCKYRKVTRKKLERYLVIEGIAKKGVEITGIDGDFKASLTAYHDFKERLTDVQL
SQRAKEAIVLNVVLFGDDKKLLKQRLSKMYPNLTTGQLKGICSLSYQGWGRLSKTFLEEITV
PAPGTGEVWNIMTALWQTNDNLMQLLSRNYGFTNEVEEFNTLKKETDLSYKTVDELYVSPAV
KRQIWQTLKVVKEIQKVMGNAPKRVFVEMAREKQEGKRSDSRKKQLVELYRACKNEERDWIT
ELNAQSDQQLRSDKLFLYYIQKGRCMYSGETIQLDELWDNTKYDIDHIYPQSKTMDDSLNNR
VLVKKNYNAIKSDTYPLSLDIQKKMMSFWKMLQQQGFITKEKYVRLVRSDELSADELAGFIE
RQIVETRQSTKAVATILKEALPDTEIVYVKAGNVSNFRQTYELLKVREMNDLHHAKDAYLNI
VVGNAYFVKFTKNAAWFIRNNPGRSYNLKRMFEFDIERSGEIAWKAGNKGSIVTVKKVMQKN
NILVTRKAYEVKGGLFDQQIMKKGKGQVPIKGNDERLADIEKYGGYNKAAGTYFMLVKSLDK
KGKEIRTIEFVPLYLKNQIEINHESAIQYLAQERGLNSPEILLSKIKIDTLFKVDGFKMWLS
GRTGNQLIFKGANQLILSHQEAAILKGVVKYVNRKNENKDAKLSERDGMTEEKLLQLYDTFL
DKLSNTVYSIRLSAQIKTLTEKRAKFIGLSNEDQCIVLNEILHMFQCQSGSANLKLIGGPGS
AGILVMNNNITACKQISVINQSPTGIYEKEIDLIKL
SEQ ID NO: 323 
MKKPYSIGLDIGTNSVGWAVVTDDYKVPAKKMKVLGNTDKSHIEKNLLGALLFDSGNTAEDR
RLKRTARRRYTRRRNRILYLQEIFSEEMGKVDDSFFHRLEDSFLVTEDKRGERHPIFGNLEE
EVKYHENFPTIYHLRQYLADNPEKVDLRLVYLALAHIIKFRGHFLIEGKFDTRNNDVQRLFQ
EFLAVYDNTFENSSLQEQNVQVEEILTDKISKSAKKDRVLKLFPNEKSNGRFAEFLKLIVGN
QADFKKHFELEEKAPLQFSKDTYEEELEVLLAQIGDNYAELFLSAKKLYDSILLSGILTVTD
VGTKAPLSASMIQRYNEHQMDLAQLKQFIRQKLSDKYNEVFSDVSKDGYAGYIDGKTNQEAF
YKYLKGLLNKIEGSGYFLDKIEREDFLRKQRTFDNGSIPHQIHLQEMRAIIRRQAEFYPFLA
DNQDRIEKLLTFRIPYYVGPLARGKSDFAWLSRKSADKITPWNFDEIVDKESSAEAFINRMT
NYDLYLPNQKVLPKHSLLYEKFTVYNELTKVKYKTEQGKTAFFDANMKQEIFDGVFKVYRKV
TKDKLMDFLEKEFDEFRIVDLTGLDKENKVFNASYGTYHDLCKILDKDFLDNSKNEKILEDI
VLTLTLFEDREMIRKRLENYSDLLTKEQVKKLERRHYTGWGRLSAELIHGIRNKESRKTILD
YLIDDGNSNRNFMQLINDDALSFKEEIAKAQVIGETDNLNQVVSDIAGSPAIKKGILQSLKI
VDELVKIMGHQPENIVVEMARENQFTNQGRRNSQQRLKGLTDSIKEFGSQILKEHPVENSQL
QNDRLFLYYLQNGRDMYTGEELDIDYLSQYDIDHIIPQAFIKDNSIDNRVLTSSKENRGKSD
DVPSKDVVRKMKSYWSKLLSAKLITQRKFDNLTKAERGGLTDDDKAGFIKRQLVETRQITKH
VARILDERFNTETDENNKKIRQVKIVTLKSNLVSNFRKEFELYKVREINDYHHAHDAYLNAV
IGKALLGVYPQLEPEFVYGDYPHFHGHKENKATAKKFFYSNIMNFFKKDDVRTDKNGEIIWK
 
KDEHISNIKKVLSYPQVNIVKKVEEQTGGFSKESILPKGNSDKLIPRKTKKFYWDTKKYGGF
DSPIVAYSILVIADIEKGKSKKLKTVKALVGVTIMEKMTFERDPVAFLERKGYRNVQEENII
KLPKYSLFKLENGRKRLLASARELQKGNEIVLPNHLGTLLYHAKNIHKVDEPKHLDYVDKHK
DEFKELLDVVSNFSKKYTLAEGNLEKIKELYAQNNGEDLKELASSFINLLTFTAIGAPATFK
FFDKNIDRKRYTSTTEILNATLIHQSITGLYETRIDLNKLGGD
SEQ ID NO: 324
SEQ ID NO: 325 
MTKPYSIGLDIGTNSVGWAVTTDNYKVPSKKMKVLGNTSKKYIKKNLLGVLLFDSGITAEGR
RLKRTARRRYTRRRNRILYLQEIFSTEMATLDDAFFQRLDDSFLVPDDKRDSKYPIFGNLVE
EKAYHDEFPTIYHLRKYLADSTKKADLRLVYLALAHMIKYRGHFLIEGEFNSKNNDIQKNFQ
DFLDTYNAIFESDLSLENSKQLEEIVKDKISKLEKKDRILKLFPGEKNSGIFSEFLKLIVGN
QADFRKCFNLDEKASLHFSKESYDEDLETLLGYIGDDYSDVFLKAKKLYDAILLSGFLTVTD
NETEAPLSSAMIKRYNEHKEDLALLKEYIRNISLKTYNEVFKDDTKNGYAGYIDGKTNQEDF
YVYLKKLLAEFEGADYFLEKIDREDFLRKQRTFDNGSIPYQIHLQEMRAILDKQAKFYPFLA
KNKERIEKILTFRIPYYVGPLARGNSDFAWSIRKRNEKITPWNFEDVIDKESSAEAFINRMT
SFDLYLPEEKVLPKHSLLYETFNVYNELTKVRFIAESMRDYQFLDSKQKKDIVRLYFKDKRK
VTDKDIIEYLHAIYGYDGIELKGIEKQFNSSLSTYHDLLNIINDKEFLDDSSNEAIIEEIIH
TLTIFEDREMIKQRLSKFENIFDKSVLKKLSRRHYTGWGKLSAKLINGIRDEKSGNTILDYL
IDDGISNRNFMQLIHDDALSFKKKIQKAQIIGDEDKGNIKEVVKSLPGSPAIKKGILQSIKI
VDELVKVMGGRKPESIVVEMARENQYTNQGKSNSQQRLKRLEKSLKELGSKILKENIPAKLS
KIDNNALQNDRLYLYYLQNGKDMYTGDDLDIDRLSNYDIDHIIPQAFLKDNSIDNKVLVSSA
SNRGKSDDVPSLEVVKKRKTFWYQLLKSKLISQRKFDNLTKAERGGLSPEDKAGFIQRQLVE
TRQITKHVARLLDEKFNNKKDENNRAVRTVKIITLKSTLVSQFRKDFELYKVREINDFHHAH
DAYLNAVVASALLKKYPKLEPEFVYGDYPKYNSFRERKSATEKVYFYSNIMNIFKKSISLAD
GRVIERPLIEVNEETGESVWNKESDLATVRRVLSYPQVNVVKKVEEQNHGLDRGKPKGLFNA
NLSSKPKPNSNENLVGAKEYLDPKKYGGYAGISNSFTVLVKGTIEKGAKKKITNVLEFQGIS
 
ILDRINYRKDKLNFLLEKGYKDIELIIELPKYSLFELSDGSRRMLASILSTNNKRGEIHKGN
QIFLSQKFVKLLYHAKRISNTINENHRKYVENHKKEFEELFYYILEFNENYVGAKKNGKLLN
SAFQSWQNHSIDELCSSFIGPTGSERKGLFELTSRGSAADFEFLGVKIPRYRDYTPSSLLKD
ATLIHQSVTGLYETRIDLAKLGEG
SEQ ID NO: 326 
MKKQKFSDYYLGFDIGTNSVGWCVTDLDYNVLRFNKKDMWGSRLFDEAKTAAERRVQRNSRR
RLKRRKWRLNLLEEIFSDEIMKIDSNFFRRLKESSLWLEDKNSKEKFTLFNDDNYKDYDFYK
QYPTIFHLRDELIKNPEKKDIRLIYLALHSIFKSRGHFLFEGQNLKEIKNFETLYNNLISFL
EDNGINKSIDKDNIEKLEKIICDSGKGLKDKEKEFKGIFNSDKQLVAIFKLSVGSSVSLNDL
FDTDEYKKEEVEKEKISFREQIYEDDKPIYYSILGEKIELLDIAKSFYDFMVLNNILSDSNY
ISEAKVKLYEEHKKDLKNLKYIIRKYNKENYDKLFKDKNENNYPAYIGLNKEKDKKEVVEKS
RLKIDDLIKVIKGYLPKPERIEEKDKTIFNEILNKIELKTILPKQRISDNGTLPYQIHEVEL
EKILENQSKYYDFLNYEENGVSTKDKLLKTFKFRIPYYVGPLNSYHKDKGGNSWIVRKEEGK
ILPWNFEQKVDIEKSAEEFIKRMTNKCTYLNGEDVIPKDSFLYSEYIILNELNKVQVNDEFL
NEENKRKIIDELFKENKKVSEKKFKEYLLVNQIANRTVELKGIKDSFNSNYVSYIKFKDIFG
EKLNLDIYKEISEKSILWKCLYGDDKKIFEKKIKNEYGDILNKDEIKKINSFKFNTWGRLSE
KLLTGIEFINLETGECYSSVMEALRRTNYNLMELLSSKFTLQESIDNENKEMNEVSYRDLIE
ESYVSPSLKRAILQTLKIYEEIKKITGRVPKKVFIEMARGGDESMKNKKIPARQEQLKKLYD
SCGNDIANFSIDIKEMKNSLSSYDNNSLRQKKLYLYYLQFGKCMYTGREIDLDRLLQNNDTY
DIDHIYPRSKVIKDDSFDNLVLVLKNENAEKSNEYPVKKEIQEKMKSFWRFLKEKNFISDEK
YKRLTGKDDFELRGFMARQLVNVRQTTKEVGKILQQIEPEIKIVYSKAEIASSFREMFDFIK
VRELNDTHHAKDAYLNIVAGNVYNTKFTEKPYRYLQEIKENYDVKKIYNYDIKNAWDKENSL
EIVKKNMEKNTVNITRFIKEEKGELFNLNPIKKGETSNEIISIKPKLYDGKDNKLNEKYGYY
TSLKAAYFIYVEHEKKNKKVKTFERITRIDSTLIKNEKNLIKYLVSQKKLLNPKIIKKIYKE
QTLIIDSYPYTFTGVDSNKKVELKNKKQLYLEKKYEQILKNALKFVEDNQGETEENYKFIYL
KKRNNNEKNETIDAVKERYNIEFNEMYDKFLEKLSSKDYKNYINNKLYTNFLNSKEKFKKLK
LWEKSLILREFLKIFNKNTYGKYEIKDSQTKEKLFSFPEDTGRIRLGQSSLGNNKELLEESV
TGLFVKKIKL
SEQ ID NO: 327 
MKNYTIGLDIGVASVGWVCIDENYKILNYNNRHAFGVHEFESAESAAGRRLKRGMRRRYNRR
KKRLQLLQSLFDSYITDSGFFSKTDSQHFWKNNNEFENRSLTEVLSSLRISSRKYPTIYHLR
SDLIESNKKMDLRLVYLALHNLVKYRGHFLQEGNWSEAASAEGMDDQLLELVTRYAELENLS
PLDLSESQWKAAETLLLNRNLTKTDQSKELTAMFGKEYEPFCKLVAGLGVSLHQLFPSSEQA
LAYKETKTKVQLSNENVEEVMELLLEEESALLEAVQPFYQQVVLYELLKGETYVAKAKVSAF
KQYQKDMASLKNLLDKTFGEKVYRSYFISDKNSQREYQKSHKVEVLCKLDQFNKEAKFAETF
YKDLKKLLEDKSKTSIGTTEKDEMLRIIKAIDSNQFLQKQKGIQNAAIPHQNSLYEAEKILR
NQQAHYPFITTEWIEKVKQILAFRIPYYIGPLVKDTTQSPFSWVERKGDAPITPWNFDEQID
KAASAEAFISRMRKTCTYLKGQEVLPKSSLTYERFEVLNELNGIQLRTTGAESDFRHRLSYE
MKCWIIDNVFKQYKTVSTKRLLQELKKSPYADELYDEHTGEIKEVFGTQKENAFATSLSGYI
SMKSILGAVVDDNPAMTEELIYWIAVFEDREILHLKIQEKYPSITDVQRQKLALVKLPGWGR
FSRLLIDGLPLDEQGQSVLDHMEQYSSVFMEVLKNKGFGLEKKIQKMNQHQVDGTKKIRYED
IEELAGSPALKRGIWRSVKIVEELVSIFGEPANIVLEVAREDGEKKRTKSRKDQWEELTKTT
LKNDPDLKSFIGEIKSQGDQRFNEQRFWLYVTQQGKCLYTGKALDIQNLSMYEVDHILPQNF
VKDDSLDNLALVMPEANQRKNQVGQNKMPLEIIEANQQYAMRTLWERLHELKLISSGKLGRL
KKPSFDEVDKDKFIARQLVETRQIIKHVRDLLDERFSKSDIHLVKAGIVSKFRRFSEIPKIR
DYNNKHHAMDALFAAALIQSILGKYGKNFLAFDLSKKDRQKQWRSVKGSNKEFFLFKNFGNL
RLQSPVTGEEVSGVEYMKHVYFELPWQTTKMTQTGDGMFYKESIFSPKVKQAKYVSPKTEKF
VHDEVKNHSICLVEFTFMKKEKEVQETKFIDLKVIEHHQFLKEPESQLAKFLAEKETNSPII
HARIIRTIPKYQKIWIEHFPYYFISTRELHNARQFEISYELMEKVKQLSERSSVEELKIVFG
 
LLIDQMNDNYPIYTKSSIQDRVQKFVDTQLYDFKSFEIGFEELKKAVAANAQRSDTFGSRIS
KKPKPEEVAIGYESITGLKYRKPRSVVGTKR
SEQ ID NO: 328 
MKKEIKDYFLGLDVGTGSVGWAVTDTDYKLLKANRKDLWGMRCFETAETAEVRRLHRGARRR
IERRKKRIKLLQELFSQEIAKTDEGFFQRMKESPFYAEDKTILQENTLFNDKDFADKTYHKA
YPTINHLIKAWIENKVKPDPRLLYLACHNIIKKRGHFLFEGDFDSENQFDTSIQALFEYLRE
DMEVDIDADSQKVKEILKDSSLKNSEKQSRLNKILGLKPSDKQKKAITNLISGNKINFADLY
DNPDLKDAEKNSISFSKDDFDALSDDLASILGDSFELLLKAKAVYNCSVLSKVIGDEQYLSF
AKVKIYEKHKTDLTKLKNVIKKHFPKDYKKVFGYNKNEKNNNNYSGYVGVCKTKSKKLIINN
SVNQEDFYKFLKTILSAKSEIKEVNDILTEIETGTFLPKQISKSNAEIPYQLRKMELEKILS
NAEKHFSFLKQKDEKGLSHSEKIIMLLTFKIPYYIGPINDNHKKFFPDRCWVVKKEKSPSGK
TTPWNFFDHIDKEKTAEAFITSRTNFCTYLVGESVLPKSSLLYSEYTVLNEINNLQIIIDGK
NICDIKLKQKIYEDLFKKYKKITQKQISTFIKHEGICNKTDEVIILGIDKECTSSLKSYIEL
KNIFGKQVDEISTKNMLEEIIRWATIYDEGEGKTILKTKIKAEYGKYCSDEQIKKILNLKFS
GWGRLSRKFLETVTSEMPGFSEPVNIITAMRETQNNLMELLSSEFTFTENIKKINSGFEDAE
KQFSYDGLVKPLFLSPSVKKMLWQTLKLVKEISHITQAPPKKIFIEMAKGAELEPARTKTRL
KILQDLYNNCKNDADAFSSEIKDLSGKIENEDNLRLRSDKLYLYYTQLGKCMYCGKPIEIGH
VFDTSNYDIDHIYPQSKIKDDSISNRVLVCSSCNKNKEDKYPLKSEIQSKQRGFWNFLQRNN
FISLEKLNRLTRATPISDDETAKFIARQLVETRQATKVAAKVLEKMFPETKIVYSKAETVSM
FRNKFDIVKCREINDFHHAHDAYLNIVVGNVYNTKFTNNPWNFIKEKRDNPKIADTYNYYKV
FDYDVKRNNITAWEKGKTIITVKDMLKRNTPIYTRQAACKKGELFNQTIMKKGLGQHPLKKE
GPFSNISKYGGYNKVSAAYYTLIEYEEKGNKIRSLETIPLYLVKDIQKDQDVLKSYLTDLLG
KKEFKILVPKIKINSLLKINGFPCHITGKTNDSFLLRPAVQFCCSNNEVLYFKKIIRFSEIR
SQREKIGKTISPYEDLSFRSYIKENLWKKTKNDEIGEKEFYDLLQKKNLEIYDMLLTKHKDT
IYKKRPNSATIDILVKGKEKFKSLIIENQFEVILEILKLFSATRNVSDLQHIGGSKYSGVAK
IGNKISSLDNCILIYQSITGIFEKRIDLLKV
SEQ ID NO: 329 
MEGQMKNNGNNLQQGNYYLGLDVGTSSVGWAVTDTDYNVLKFRGKSMWGARLFDEASTAEER
RTHRGNRRRLARRKYRLLLLEQLFEKEIRKIDDNFFVRLHESNLWADDKSKPSKFLLFNDTN
FTDKDYLKKYPTIYHLRSDLIHNSTEHDIRLVFLALHHLIKYRGHFIYDNSANGDVKTLDEA
VSDFEEYLNENDIEFNIENKKEFINVLSDKHLTKKEKKISLKKLYGDITDSENINISVLIEM
LSGSSISLSNLFKDIEFDGKQNLSLDSDIEETLNDVVDILGDNIDLLIHAKEVYDIAVLTSS
LGKHKYLCDAKVELFEKNKKDLMILKKYIKKNHPEDYKKIFSSPTEKKNYAAYSQTNSKNVC
SQEEFCLFIKPYIRDMVKSENEDEVRIAKEVEDKSFLTKLKGTNNSVVPYQIHERELNQILK
NIVAYLPFMNDEQEDISVVDKIKLIFKFKIPYYVGPLNTKSTRSWVYRSDEKIYPWNFSNVI
DLDKTAHEFMNRLIGRCTYTNDPVLPMDSLLYSKYNVLNEINPIKVNGKAIPVEVKQAIYTD
LFENSKKKVTRKSIYIYLLKNGYIEKEDIVSGIDIEIKSKLKSHHDFTQIVQENKCTPEEIE
RIIKGILVYSDDKSMLRRWLKNNIKGLSENDVKYLAKLNYKEWGRLSKTLLTDIYTINPEDG
EACSILDIMWNTNATLMEILSNEKYQFKQNIENYKAENYDEKQNLHEELDDMYISPAARRSI
WQALRIVDEIVDIKKSAPKKIFIEMAREKKSAMKKKRTESRKDTLLELYKSCKSQADGFYDE
ELFEKLSNESNSRLRRDQLYLYYTQMGRSMYTGKRIDFDKLINDKNTYDIDHIYPRSKIKDD
SITNRVLVEKDINGEKTDIYPISEDIRQKMQPFWKILKEKGLINEEKYKRLTRNYELTDEEL
SSFVARQLVETQQSTKALATLLKKEYPSAKIVYSKAGNVSEFRNRKDKELPKFREINDLHHA
KDAYLNIVVGNVYDTKFTEKFFNNIRNENYSLKRVFDFSVPGAWDAKGSTFNTIKKYMAKNN
PIIAFAPYEVKGELFDQQIVPKGKGQFPIKQGKDIEKYGGYNKLSSAFLFAVEYKGKKARER
SLETVYIKDVELYLQDPIKYCESVLGLKEPQIIKPKILMGSLFSINNKKLVVTGRSGKQYVC
HHIYQLSINDEDSQYLKNIAKYLQEEPDGNIERQNILNITSVNNIKLFDVLCTKFNSNTYEI
ILNSLKNDVNEGREKFSELDILEQCNILLQLLKAFKCNRESSNLEKLNNKKQAGVIVIPHLF
TKCSVFKVIHQSITGLFEKEMDLLK
SEQ ID NO: 330 
MGRKPYILSLDIGTGSVGYACMDKGFNVLKYHDKDALGVYLFDGALTAQERRQFRTSRRRKN
RRIKRLGLLQELLAPLVQNPNFYQFQRQFAWKNDNMDFKNKSLSEVLSFLGYESKKYPTIYH
LQEALLLKDEKFDPELIYMALYHLVKYRGHFLFDHLKIENLTNNDNMHDFVELIETYENLNN
IKLNLDYEKTKVIYEILKDNEMTKNDRAKRVKNMEKKLEQFSIMLLGLKFNEGKLFNHADNA
EELKGANQSHTFADNYEENLTPFLTVEQSEFIERANKIYLSLTLQDILKGKKSMAMSKVAAY
DKFRNELKQVKDIVYKADSTRTQFKKIFVSSKKSLKQYDATPNDQTFSSLCLFDQYLIRPKK
QYSLLIKELKKIIPQDSELYFEAENDTLLKVLNTTDNASIPMQINLYEAETILRNQQKYHAE
ITDEMIEKVLSLIQFRIPYYVGPLVNDHTASKFGWMERKSNESIKPWNFDEVVDRSKSATQF
IRRMTNKCSYLINEDVLPKNSLLYQEMEVLNELNATQIRLQTDPKNRKYRMMPQIKLFAVEH
IFKKYKTVSHSKFLEIMLNSNHRENFMNHGEKLSIFGTQDDKKFASKLSSYQDMTKIFGDIE
GKRAQIEEIIQWITIFEDKKILVQKLKECYPELTSKQINQLKKLNYSGWGRLSEKLLTHAYQ
GHSIIELLRHSDENFMEILTNDVYGFQNFIKEENQVQSNKIQHQDIANLTTSPALKKGIWST
IKLVRELTSIFGEPEKIIMEFATEDQQKGKKQKSRKQLWDDNIKKNKLKSVDEYKYIIDVAN
KLNNEQLQQEKLWLYLSQNGKCMYSGQSIDLDALLSPNATKHYEVDHIFPRSFIKDDSIDNK
VLVIKKMNQTKGDQVPLQFIQQPYERIAYWKSLNKAGLISDSKLHKLMKPEFTAMDKEGFIQ
RQLVETRQISVHVRDFLKEEYPNTKVIPMKAKMVSEFRKKFDIPKIRQMNDAHHAIDAYLNG
VVYHGAQLAYPNVDLFDFNFKWEKVREKWKALGEFNTKQKSRELFFFKKLEKMEVSQGERLI
SKIKLDMNHFKINYSRKLANIPQQFYNQTAVSPKTAELKYESNKSNEVVYKGLTPYQTYVVA
IKSVNKKGKEKMEYQMIDHYVFDFYKFQNGNEKELALYLAQRENKDEVLDAQIVYSLNKGDL
LYINNHPCYFVSRKEVINAKQFELTVEQQLSLYNVMNNKETNVEKLLIEYDFIAEKVINEYH
HYLNSKLKEKRVRTFFSESNQTHEDFIKALDELFKVVTASATRSDKIGSRKNSMTHRAFLGK
GKDVKIAYTSISGLKTTKPKSLFKLAESRNEL
SEQ ID NO: 331 
MAKILGLDLGTNSIGWAVVERENIDFSLIDKGVRIFSEGVKSEKGIESSRAAERTGYRSARK
IKYRRKLRKYETLKVLSLNRMCPLSIEEVEEWKKSGFKDYPLNPEFLKWLSTDEESNVNPYF
FRDRASKHKVSLFELGRAFYHIAQRRGFLSNRLDQSAEGILEEHCPKIEAIVEDLISIDEIS
TNITDYFFETGILDSNEKNGYAKDLDEGDKKLVSLYKSLLAILKKNESDFENCKSEIIERLN
KKDVLGKVKGKIKDISQAMLDGNYKTLGQYFYSLYSKEKIRNQYTSREEHYLSEFITICKVQ
GIDQINEEEKINEKKFDGLAKDLYKAIFFQRPLKSQKGLIGKCSFEKSKSRCAISHPDFEEY
RMWTYLNTIKIGTQSDKKLRFLTQDEKLKLVPKFYRKNDFNFDVLAKELIEKGSSFGFYKSS
KKNDFFYWFNYKPTDTVAACQVAASLKNAIGEDWKTKSFKYQTINSNKEQVSRTVDYKDLWH
LLTVATSDVYLYEFAIDKLGLDEKNAKAFSKTKLKKDFASLSLSAINKILPYLKEGLLYSHA
VFVANIENIVDENIWKDEKQRDYIKTQISEIIENYTLEKSRFEIINGLLKEYKSENEDGKRV
YYSKEAEQSFENDLKKKLVLFYKSNEIENKEQQETIFNELLPIFIQQLKDYEFIKIQRLDQK
VLIFLKGKNETGQIFCTEEKGTAEEKEKKIKNRLKKLYHPSDIEKFKKKIIKDEFGNEKIVL
GSPLTPSIKNPMAMRALHQLRKVLNALILEGQIDEKTIIHIEMARELNDANKRKGIQDYQND
NKKFREDAIKEIKKLYFEDCKKEVEPTEDDILRYQLWMEQNRSEIYEEGKNISICDIIGSNP
AYDIEHTIPRSRSQDNSQMNKTLCSQRFNREVKKQSMPIELNNHLEILPRIAHWKEEADNLT
REIEIISRSIKAAATKEIKDKKIRRRHYLTLKRDYLQGKYDRFIWEEPKVGFKNSQIPDTGI
ITKYAQAYLKSYFKKVESVKGGMVAEFRKIWGIQESFIDENGMKHYKVKDRSKHTHHTIDAI
TIACMTKEKYDVLAHAWTLEDQQNKKEARSIIEASKPWKTFKEDLLKIEEEILVSHYTPDNV
KKQAKKIVRVRGKKQFVAEVERDVNGKAVPKKAASGKTIYKLDGEGKKLPRLQQGDTIRGSL
HQDSIYGAIKNPLNTDEIKYVIRKDLESIKGSDVESIVDEVVKEKIKEAIANKVLLLSSNAQ
QKNKLVGTVWMNEEKRIAINKVRIYANSVKNPLHIKEHSLLSKSKHVHKQKVYGQNDENYAM
AIYELDGKRDFELINIFNLAKLIKQGQGFYPLHKKKEIKGKIVFVPIEKRNKRDVVLKRGQQ
VVFYDKEVENPKDISEIVDFKGRIYIIEGLSIQRIVRPSGKVDEYGVIMLRYFKEARKADDI
KQDNFKPDGVFKLGENKPTRKMNHQFTAFVEGIDFKVLPSGKFEKI
SEQ ID NO: 332 
MEFKKVLGLDIGTNSIGCALLSLPKSIQDYGKGGRLEWLTSRVIPLDADYMKAFIDGKNGLP
QVITPAGKRRQKRGSRRLKHRYKLRRSRLIRVFKTLNWLPEDFPLDNPKRIKETISTEGKFS
FRISDYVPISDESYREFYREFGYPENEIEQVIEEINFRRKTKGKNKNPMIKLLPEDWVVYYL
RKKALIKPTTKEELIRIIYLFNQRRGFKSSRKDLTETAILDYDEFAKRLAEKEKYSAENYET
KFVSITKVKEVVELKTDGRKGKKRFKVILEDSRIEPYEIERKEKPDWEGKEYTFLVTQKLEK
GKFKQNKPDLPKEEDWALCTTALDNRMGSKHPGEFFFDELLKAFKEKRGYKIRQYPVNRWRY
KKELEFIWTKQCQLNPELNNLNINKEILRKLATVLYPSQSKFFGPKIKEFENSDVLHIISED
IIYYQRDLKSQKSLISECRYEKRKGIDGEIYGLKCIPKSSPLYQEFRIWQDIHNIKVIRKES
EVNGKKKINIDETQLYINENIKEKLFELFNSKDSLSEKDILELISLNIINSGIKISKKEEET
THRINLFANRKELKGNETKSRYRKVFKKLGFDGEYILNHPSKLNRLWHSDYSNDYADKEKTE
KSILSSLGWKNRNGKWEKSKNYDVFNLPLEVAKAIANLPPLKKEYGSYSALAIRKMLVVMRD
GKYWQHPDQIAKDQENTSLMLFDKNLIQLTNNQRKVLNKYLLTLAEVQKRSTLIKQKLNEIE
HNPYKLELVSDQDLEKQVLKSFLEKKNESDYLKGLKTYQAGYLIYGKHSEKDVPIVNSPDEL
GEYIRKKLPNNSLRNPIVEQVIRETIFIVRDVWKSFGIIDEIHIELGRELKNNSEERKKTSE
SQEKNFQEKERARKLLKELLNSSNFEHYDENGNKIFSSFTVNPNPDSPLDIEKFRIWKNQSG
LTDEELNKKLKDEKIPTEIEVKKYILWLTQKCRSPYTGKIIPLSKLFDSNVYEIEHIIPRSK
MKNDSTNNLVICELGVNKAKGDRLAANFISESNGKCKFGEVEYTLLKYGDYLQYCKDTFKYQ
KAKYKNLLATEPPEDFIERQINDTRYIGRKLAELLTPVVKDSKNIIFTIGSITSELKITWGL
NGVWKDILRPRFKRLESIINKKLIFQDEDDPNKYHFDLSINPQLDKEGLKRLDHRHHALDAT
IIAATTREHVRYLNSLNAADNDEEKREYFLSLCNHKIRDFKLPWENFTSEVKSKLLSCVVSY
KESKPILSDPFNKYLKWEYKNGKWQKVFAIQIKNDRWKAVRRSMFKEPIGTVWIKKIKEVSL
KEAIKIQAIWEEVKNDPVRKKKEKYIYDDYAQKVIAKIVQELGLSSSMRKQDDEKLNKFINE
AKVSAGVNKNLNTTNKTIYNLEGRFYEKIKVAEYVLYKAKRMPLNKKEYIEKLSLQKMFNDL
PNFILEKSILDNYPEILKELESDNKYIIEPHKKNNPVNRLLLEHILEYHNNPKEAFSTEGLE
KLNKKAINKIGKPIKYITRLDGDINEEEIFRGAVFETDKGSNVYFVMYENNQTKDREFLKPN
PSISVLKAIEHKNKIDFFAPNRLGFSRIILSPGDLVYVPTNDQYVLIKDNSSNETIINWDDN
EFISNRIYQVKKFTGNSCYFLKNDIASLILSYSASNGVGEFGSQNISEYSVDDPPIRIKDVC
IKIRVDRLGNVRPL
SEQ ID NO: 333 
MKHILGLDLGTNSIGWALIERNIEEKYGKIIGMGSRIVPMGAELSKFEQGQAQTKNADRRTN
RGARRLNKRYKQRRNKLIYILQKLDMLPSQIKLKEDFSDPNKIDKITILPISKKQEQLTAFD
LVSLRVKALTEKVGLEDLGKIIYKYNQLRGYAGGSLEPEKEDIFDEEQSKDKKNKSFIAFSK
IVFLGEPQEEIFKNKKLNRRAIIVETEEGNFEGSTFLENIKVGDSLELLINISASKSGDTIT
IKLPNKTNWRKKMENIENQLKEKSKEMGREFYISEFLLELLKENRWAKIRNNTILRARYESE
FEAIWNEQVKHYPFLENLDKKTLIEIVSFIFPGEKESQKKYRELGLEKGLKYIIKNQVVFYQ
RELKDQSHLISDCRYEPNEKAIAKSHPVFQEYKVWEQINKLIVNTKIEAGTNRKGEKKYKYI
DRPIPTALKEWIFEELQNKKEITFSAIFKKLKAEFDLREGIDFLNGMSPKDKLKGNETKLQL
QKSLGELWDVLGLDSINRQIELWNILYNEKGNEYDLTSDRTSKVLEFINKYGNNIVDDNAEE
TAIRISKIKFARAYSSLSLKAVERILPLVRAGKYFNNDFSQQLQSKILKLLNENVEDPFAKA
AQTYLDNNQSVLSEGGVGNSIATILVYDKHTAKEYSHDELYKSYKEINLLKQGDLRNPLVEQ
IINEALVLIRDIWKNYGIKPNEIRVELARDLKNSAKERATIHKRNKDNQTINNKIKETLVKN
KKELSLANIEKVKLWEAQRHLSPYTGQPIPLSDLFDKEKYDVDHIIPISRYFDDSFTNKVIS
EKSVNQEKANRTAMEYFEVGSLKYSIFTKEQFIAHVNEYFSGVKRKNLLATSIPEDPVQRQI
KDTQYIAIRVKEELNKIVGNENVKTTTGSITDYLRNHWGLTDKFKLLLKERYEALLESEKFL
EAEYDNYKKDFDSRKKEYEEKEVLFEEQELTREEFIKEYKENYIRYKKNKLIIKGWSKRIDH
RHHAIDALIVACTEPAHIKRLNDLNKVLQDWLVEHKSEFMPNFEGSNSELLEEILSLPENER
TEIFTQIEKFRAIEMPWKGFPEQVEQKLKEIIISHKPKDKLLLQYNKAGDRQIKLRGQLHEG
TLYGISQGKEAYRIPLTKFGGSKFATEKNIQKIVSPFLSGFIANHLKEYNNKKEEAFSAEGI
MDLNNKLAQYRNEKGELKPHTPISTVKIYYKDPSKNKKKKDEEDLSLQKLDREKAFNEKLYV
 
KTGDNYLFAVLEGEIKTKKTSQIKRLYDIISFFDATNFLKEEFRNAPDKKTFDKDLLFRQYF
EERNKAKLLFTLKQGDFVYLPNENEEVILDKESPLYNQYWGDLKERGKNIYVVQKFSKKQIY
FIKHTIADIIKKDVEFGSQNCYETVEGRSIKENCFKLEIDRLGNIVKVIKR
SEQ ID NO: 334 
MHVEIDFPHFSRGDSHLAMNKNEILRGSSVLYRLGLDLGSNSLGWFVTHLEKRGDRHEPVAL
GPGGVRIFPDGRDPQSGTSNAVDRRMARGARKRRDRFVERRKELIAALIKYNLLPDDARERR
ALEVLDPYALRKTALTDTLPAHHVGRALFHLNQRRGFQSNRKTDSKQSEDGAIKQAASRLAT
DKGNETLGVFFADMHLRKSYEDRQTAIRAELVRLGKDHLTGNARKKIWAKVRKRLFGDEVLP
RADAPHGVRARATITGTKASYDYYPTRDMLRDEFNAIWAGQSAHHATITDEARTEIEHIIFY
QRPLKPAIVGKCTLDPATRPFKEDPEGYRAPWSHPLAQRFRILSEARNLEIRDTGKGSRRLT
KEQSDLVVAALLANREVKFDKLRTLLKLPAEARFNLESDRRAALDGDQTAARLSDKKGFNKA
WRGFPPERQIAIVARLEETEDENELIAWLEKECALDGAAAARVANTTLPDGHCRLGLRAIKK
IVPIMQDGLDEDGVAGAGYHIAAKRAGYDHAKLPTGEQLGRLPYYGQWLQDAVVGSGDARDQ
KEKQYGQFPNPTVHIGLGQLRRVVNDLIDKYGPPTEISIEFTRALKLSEQQKAERQREQRRN
QDKNKARAEELAKFGRPANPRNLLKMRLWEELAHDPLDRKCVYTGEQISIERLLSDEVDIDH
ILPVAMTLDDSPANKIICMRYANRHKRKQTPSEAFGSSPTLQGHRYNWDDIAARATGLPRNK
RWRFDANAREEFDKRGGFLARQLNETGWLARLAKQYLGAVTDPNQIWVVPGRLTSMLRGKWG
LNGLLPSDNYAGVQDKAEEFLASTDDMEFSGVKNRADHRHHAIDGLVTALTDRSLLWKMANA
YDEEHEKFVIEPPWPTMRDDLKAALEKMVVSHKPDHGIEGKLHEDSAYGFVKPLDATGLKEE
EAGNLVYRKAIESLNENEVDRIRDIQLRTIVRDHVNVEKTKGVALADALRQLQAPSDDYPQF
KHGLRHVRILKKEKGDYLVPIANRASGVAYKAYSAGENFCVEVFETAGGKWDGEAVRRFDAN
KKNAGPKIAHAPQWRDANEGAKLVMRIHKGDLIRLDHEGRARIMVVHRLDAAAGRFKLADHN
ETGNLDKRHATNNDIDPFRWLMASYNTLKKLAAVPVRVDELGRVWRVMPN
SEQ ID NO: 335 
METTLGIDLGTNSIGLALVDQEEHQILYSGVRIFPEGINKDTIGLGEKEESRNATRRAKRQM
RRQYFRKKLRKAKLLELLIAYDMCPLKPEDVRRWKNWDKQQKSTVRQFPDTPAFREWLKQNP
YELRKQAVTEDVTRPELGRILYQMIQRRGFLSSRKGKEEGKIFTGKDRMVGIDETRKNLQKQ
TLGAYLYDIAPKNGEKYRFRTERVRARYTLRDMYIREFEIIWQRQAGHLGLAHEQATRKKNI
FLEGSATNVRNSKLITHLQAKYGRGHVLIEDTRITVTFQLPLKEVLGGKIEIEEEQLKFKSN
ESVLFWQRPLRSQKSLLSKCVFEGRNFYDPVHQKWIIAGPTPAPLSHPEFEEFRAYQFINNI
IYGKNEHLTAIQREAVFELMCTESKDFNFEKIPKHLKLFEKFNFDDTTKVPACTTISQLRKL
FPHPVWEEKREEIWHCFYFYDDNTLLFEKLQKDYALQTNDLEKIKKIRLSESYGNVSLKAIR
RINPYLKKGYAYSTAVLLGGIRNSFGKRFEYFKEYEPEIEKAVCRILKEKNAEGEVIRKIKD
YLVHNRFGFAKNDRAFQKLYHHSQAITTQAQKERLPETGNLRNPIVQQGLNELRRTVNKLLA
TCREKYGPSFKFDHIHVEMGRELRSSKTEREKQSRQIRENEKKNEAAKVKLAEYGLKAYRDN
IQKYLLYKEIEEKGGTVCCPYTGKTLNISHTLGSDNSVQIEHIIPYSISLDDSLANKTLCDA
TFNREKGELTPYDFYQKDPSPEKWGASSWEEIEDRAFRLLPYAKAQRFIRRKPQESNEFISR
QLNDTRYISKKAVEYLSAICSDVKAFPGQLTAELRHLWGLNNILQSAPDITFPLPVSATENH
REYYVITNEQNEVIRLFPKQGETPRTEKGELLLTGEVERKVFRCKGMQEFQTDVSDGKYWRR
IKLSSSVTWSPLFAPKPISADGQIVLKGRIEKGVFVCNQLKQKLKTGLPDGSYWISLPVISQ
TFKEGESVNNSKLTSQQVQLFGRVREGIFRCHNYQCPASGADGNFWCTLDTDTAQPAFTPIK
NAPPGVGGGQIILTGDVDDKGIFHADDDLHYELPASLPKGKYYGIFTVESCDPTLIPIELSA
PKTSKGENLIEGNIWVDEHTGEVRFDPKKNREDQRHHAIDAIVIALSSQSLFQRLSTYNARR
ENKKRGLDSTEHFPSPWPGFAQDVRQSVVPLLVSYKQNPKTLCKISKTLYKDGKKIHSCGNA
VRGQLHKETVYGQRTAPGATEKSYHIRKDIRELKTSKHIGKVVDITIRQMLLKHLQENYHID
ITQEFNIPSNAFFKEGVYRIFLPNKHGEPVPIKKIRMKEELGNAERLKDNINQYVNPRNNHH
VMIYQDADGNLKEEIVSFWSVIERQNQGQPIYQLPREGRNIVSILQINDTFLIGLKEEEPEV
YRNDLSTLSKHLYRVQKLSGMYYTFRHHLASTLNNEREEFRIQSLEAWKRANPVKVQIDEIG
RITFLNGPLC
SEQ ID NO: 336 
MESSQILSPIGIDLGGKFTGVCLSHLEAFAELPNHANTKYSVILIDHNNFQLSQAQRRATRH
RVRNKKRNQFVKRVALQLFQHILSRDLNAKEETALCHYLNNRGYTYVDTDLDEYIKDETTIN
LLKELLPSESEHNFIDWFLQKMQSSEFRKILVSKVEEKKDDKELKNAVKNIKNFITGFEKNS
VEGHRHRKVYFENIKSDITKDNQLDSIKKKIPSVCLSNLLGHLSNLQWKNLHRYLAKNPKQF
DEQTFGNEFLRMLKNFRHLKGSQESLAVRNLIQQLEQSQDYISILEKTPPEITIPPYEARTN
TGMEKDQSLLLNPEKLNNLYPNWRNLIPGIIDAHPFLEKDLEHTKLRDRKRIISPSKQDEKR
DSYILQRYLDLNKKIDKFKIKKQLSFLGQGKQLPANLIETQKEMETHFNSSLVSVLIQIASA
YNKEREDAAQGIWFDNAFSLCELSNINPPRKQKILPLLVGAILSEDFINNKDKWAKFKIFWN
THKIGRTSLKSKCKEIEEARKNSGNAFKIDYEEALNHPEHSNNKALIKIIQTIPDIIQAIQS
HLGHNDSQALIYHNPFSLSQLYTILETKRDGFHKNCVAVTCENYWRSQKTEIDPEISYASRL
PADSVRPFDGVLARMMQRLAYEIAMAKWEQIKHIPDNSSLLIPIYLEQNRFEFEESFKKIKG
SSSDKTLEQAIEKQNIQWEEKFQRIINASMNICPYKGASIGGQGEIDHIYPRSLSKKHFGVI
FNSEVNLIYCSSQGNREKKEEHYLLEHLSPLYLKHQFGTDNVSDIKNFISQNVANIKKYISF
HLLTPEQQKAARHALFLDYDDEAFKTITKFLMSQQKARVNGTQKFLGKQIMEFLSTLADSKQ
LQLEFSIKQITAEEVHDHRELLSKQEPKLVKSRQQSFPSHAIDATLTMSIGLKEFPQFSQEL
DNSWFINHLMPDEVHLNPVRSKEKYNKPNISSTPLFKDSLYAERFIPVWVKGETFAIGFSEK
DLFEIKPSNKEKLFTLLKTYSTKNPGESLQELQAKSKAKWLYFPINKTLALEFLHHYFHKEI
VTPDDTTVCHFINSLRYYTKKESITVKILKEPMPVLSVKFESSKKNVLGSFKHTIALPATKD
WERLFNHPNFLALKANPAPNPKEFNEFIRKYFLSDNNPNSDIPNNGHNIKPQKHKAVRKVFS
LPVIPGNAGTMMRIRRKDNKGQPLYQLQTIDDTPSMGIQINEDRLVKQEVLMDAYKTRNLST
IDGINNSEGQAYATFDNWLTLPVSTFKPEIIKLEMKPHSKTRRYIRITQSLADFIKTIDEAL
MIKPSDSIDDPLNMPNEIVCKNKLFGNELKPRDGKMKIVSTGKIVTYEFESDSTPQWIQTLY
VTQLKKQP
SEQ ID NO: 337 
MKKIVGLDLGTNSIGWALINAYINKEHLYGIEACGSRIIPMDAAILGNFDKGNSISQTADRT
SYRGIRRLRERHLLRRERLHRILDLLGFLPKHYSDSLNRYGKFLNDIECKLPWVKDETGSYK
FIFQESFKEMLANFTEHHPILIANNKKVPYDWTIYYLRKKALTQKISKEELAWILLNFNQKR
GYYQLRGEEEETPNKLVEYYSLKVEKVEDSGERKGKDTWYNVHLENGMIYRRTSNIPLDWEG
KTKEFIVTTDLEADGSPKKDKEGNIKRSFRAPKDDDWTLIKKKTEADIDKIKMTVGAYIYDT
LLQKPDQKIRGKLVRTIERKYYKNELYQILKTQSEFHEELRDKQLYIACLNELYPNNEPRRN
SISTRDFCHLFIEDIIFYQRPLKSKKSLIDNCPYEENRYIDKESGEIKHASIKCIAKSHPLY
QEFRLWQFIVNLRIYRKETDVDVTQELLPTEADYVTLFEWLNEKKEIDQKAFFKYPPFGFKK
TTSNYRWNYVEDKPYPCNETHAQIIARLGKAHIPKAFLSKEKEETLWHILYSIEDKQEIEKA
LHSFANKNNLSEEFIEQFKNFPPFKKEYGSYSAKAIKKLLPLMRMGKYWSIENIDNGTRIRI
NKIIDGEYDENIRERVRQKAINLTDITHFRALPLWLACYLVYDRHSEVKDIVKWKTPKDIDL
YLKSFKQHSLRNPIVEQVITETLRTVRDIWQQVGHIDEIHIELGREMKNPADKRARMSQQMI
KNENTNLRIKALLTEFLNPEFGIENVRPYSPSQQDLLRIYEEGVLNSILELPEDIGIILGKF
NQTDTLKRPTRSEILRYKLWLEQKYRSPYTGEMIPLSKLFTPAYEIEHIIPQSRYFDDSLSN
KVICESEINKLKDRSLGYEFIKNHHGEKVELAFDKPVEVLSVEAYEKLVHESYSHNRSKMKK
LLMEDIPDQFIERQLNDSRYISKVVKSLLSNIVREENEQEAISKNVIPCTGGITDRLKKDWG
INDVWNKIVLPRFIRLNELTESTRFTSINTNNTMIPSMPLELQKGFNKKRIDHRHHAMDAII
IACANRNIVNYLNNVSASKNTKITRRDLQTLLCHKDKTDNNGNYKWVIDKPWETFTQDTLTA
LQKITVSFKQNLRVINKTTNHYQHYENGKKIVSNQSKGDSWAIRKSMHKETVHGEVNLRMIK
TVSFNEALKKPQAIVEMDLKKKILAMLELGYDTKRIKNYFEENKDTWQDINPSKIKVYYFTK
ETKDRYFAVRKPIDTSFDKKKIKESITDTGIQQIMLRHLETKDNDPTLAFSPDGIDEMNRNI
LILNKGKKHQPIYKVRVYEKAEKFTVGQKGNKRTKFVEAAKGTNLFFAIYETEEIDKDTKKV
IRKRSYSTIPLNVVIERQKQGLSSAPEDENGNLPKYILSPNDLVYVPTQEEINKGEVVMPID
 
RDRIYKMVDSSGITANFIPASTANLIFALPKATAEIYCNGENCIQNEYGIGSPQSKNQKAIT
GEMVKEICFPIKVDRLGNIIQVGSCILTN
SEQ ID NO: 338 
MSRSLTFSFDIGYASIGWAVIASASHDDADPSVCGCGTVLFPKDDCQAFKRREYRRLRRNIR
SRRVRIERIGRLLVQAQIITPEMKETSGHPAPFYLASEALKGHRTLAPIELWHVLRWYAHNR
GYDNNASWSNSLSEDGGNGEDTERVKHAQDLMDKHGTATMAETICRELKLEEGKADAPMEVS
TPAYKNLNTAFPRLIVEKEVRRILELSAPLIPGLTAEIIELIAQHHPLTTEQRGVLLQHGIK
LARRYRGSLLFGQLIPRFDNRIISRCPVTWAQVYEAELKKGNSEQSARERAEKLSKVPTANC
PEFYEYRMARILCNIRADGEPLSAEIRRELMNQARQEGKLTKASLEKAISSRLGKETETNVS
NYFTLHPDSEEALYLNPAVEVLQRSGIGQILSPSVYRIAANRLRRGKSVTPNYLLNLLKSRG
ESGEALEKKIEKESKKKEADYADTPLKPKYATGRAPYARTVLKKVVEEILDGEDPTRPARGE
AHPDGELKAHDGCLYCLLDTDSSVNQHQKERRLDTMTNNHLVRHRMLILDRLLKDLIQDFAD
GQKDRISRVCVEVGKELTTFSAMDSKKIQRELTLRQKSHTDAVNRLKRKLPGKALSANLIRK
CRIAMDMNWTCPFTGATYGDHELENLELEHIVPHSFRQSNALSSLVLTWPGVNRMKGQRTGY
DFVEQEQENPVPDKPNLHICSLNNYRELVEKLDDKKGHEDDRRRKKKRKALLMVRGLSHKHQ
SQNHEAMKEIGMTEGMMTQSSHLMKLACKSIKTSLPDAHIDMIPGAVTAEVRKAWDVFGVFK
ELCPEAADPDSGKILKENLRSLTHLHHALDACVLGLIPYIIPAHHNGLLRRVLAMRRIPEKL
IPQVRPVANQRHYVLNDDGRMMLRDLSASLKENIREQLMEQRVIQHVPADMGGALLKETMQR
VLSVDGSGEDAMVSLSKKKDGKKEKNQVKASKLVGVFPEGPSKLKALKAAIEIDGNYGVALD
PKPVVIRHIKVFKRIMALKEQNGGKPVRILKKGMLIHLTSSKDPKHAGVWRIESIQDSKGGV
KLDLQRAHCAVPKNKTHECNWREVDLISLLKKYQMKRYPTSYTGTPR
SEQ ID NO: 339 
MTQKVLGLDLGTNSIGSAVRNLDLSDDLQWQLEFFSSDIFRSSVNKESNGREYSLAAQRSAH
RRSRGLNEVRRRRLWATLNLLIKHGFCPMSSESLMRWCTYDKRKGLFREYPIDDKDFNAWIL
LDFNGDGRPDYSSPYQLRRELVTRQFDFEQPIERYKLGRALYHIAQHRGFKSSKGETLSQQE
TNSKPSSTDEIPDVAGAMKASEEKLSKGLSTYMKEHNLLTVGAAFAQLEDEGVRVRNNNDYR
AIRSQFQHEIETIFKFQQGLSVESELYERLISEKKNVGTIFYKRPLRSQRGNVGKCTLERSK
PRCAIGHPLFEKFRAWTLINNIKVRMSVDTLDEQLPMKLRLDLYNECFLAFVRTEFKFEDIR
KYLEKRLGIHFSYNDKTINYKDSTSVAGCPITARFRKMLGEEWESFRVEGQKERQAHSKNNI
SFHRVSYSIEDIWHFCYDAEEPEAVLAFAQETLRLERKKAEELVRIWSAMPQGYAMLSQKAI
RNINKILMLGLKYSDAVILAKVPELVDVSDEELLSIAKDYYLVEAQVNYDKRINSIVNGLIA
KYKSVSEEYRFADHNYEYLLDESDEKDIIRQIENSLGARRWSLMDANEQTDILQKVRDRYQD
FFRSHERKFVESPKLGESFENYLTKKFPMVEREQWKKLYHPSQITIYRPVSVGKDRSVLRLG
NPDIGAIKNPTVLRVLNTLRRRVNQLLDDGVISPDETRVVVETARELNDANRKWALDTYNRI
RHDENEKIKKILEEFYPKRDGISTDDIDKARYVIDQREVDYFTGSKTYNKDIKKYKFWLEQG
GQCMYTGRTINLSNLFDPNAFDIEHTIPESLSFDSSDMNLTLCDAHYNRFIKKNHIPTDMPN
YDKAITIDGKEYPAITSQLQRWVERVERLNRNVEYWKGQARRAQNKDRKDQCMREMHLWKME
LEYWKKKLERFTVTEVTDGFKNSQLVDTRVITRHAVLYLKSIFPHVDVQRGDVTAKFRKILG
IQSVDEKKDRSLHSHHAIDATTLTIIPVSAKRDRMLELFAKIEEINKMLSFSGSEDRTGLIQ
ELEGLKNKLQMEVKVCRIGHNVSEIGTFINDNIIVNHHIKNQALTPVRRRLRKKGYIVGGVD
NPRWQTGDALRGEIHKASYYGAITQFAKDDEGKVLMKEGRPQVNPTIKFVIRRELKYKKSAA
DSGFASWDDLGKAIVDKELFALMKGQFPAETSFKDACEQGIYMIKKGKNGMPDIKLHHIRHV
RCEAPQSGLKIKEQTYKSEKEYKRYFYAAVGDLYAMCCYTNGKIREFRIYSLYDVSCHRKSD
IEDIPEFITDKKGNRLMLDYKLRTGDMILLYKDNPAELYDLDNVNLSRRLYKINRFESQSNL
VLMTHHLSTSKERGRSLGKTVDYQNLPESIRSSVKSLNFLIMGENRDFVIKNGKIIFNHR
SEQ ID NO: 340 
MLVSPISVDLGGKNTGFFSFTDSLDNSQSGTVIYDESFVLSQVGRRSKRHSKRNNLRNKLVK
RLFLLILQEHHGLSIDVLPDEIRGLFNKRGYTYAGFELDEKKKDALESDTLKEFLSEKLQSI
 
DRDSDVEDFLNQIASNAESFKDYKKGFEAVFASATHSPNKKLELKDELKSEYGENAKELLAG
LRVTKEILDEFDKQENQGNLPRAKYFEELGEYIATNEKVKSFFDSNSLKLTDMTKLIGNISN
YQLKELRRYFNDKEMEKGDIWIPNKLHKITERFVRSWHPKNDADRQRRAELMKDLKSKEIME
LLTTTEPVMTIPPYDDMNNRGAVKCQTLRLNEEYLDKHLPNWRDIAKRLNHGKFNDDLADST
VKGYSEDSTLLHRLLDTSKEIDIYELRGKKPNELLVKTLGQSDANRLYGFAQNYYELIRQKV
RAGIWVPVKNKDDSLNLEDNSNMLKRCNHNPPHKKNQIHNLVAGILGVKLDEAKFAEFEKEL
WSAKVGNKKLSAYCKNIEELRKTHGNTFKIDIEELRKKDPAELSKEEKAKLRLTDDVILNEW
SQKIANFFDIDDKHRQRFNNLFSMAQLHTVIDTPRSGFSSTCKRCTAENRFRSETAFYNDET
GEFHKKATATCQRLPADTQRPFSGKIERYIDKLGYELAKIKAKELEGMEAKEIKVPIILEQN
AFEYEESLRKSKTGSNDRVINSKKDRDGKKLAKAKENAEDRLKDKDKRIKAFSSGICPYCGD
TIGDDGEIDHILPRSHTLKIYGTVFNPEGNLIYVHQKCNQAKADSIYKLSDIKAGVSAQWIE
EQVANIKGYKTFSVLSAEQQKAFRYALFLQNDNEAYKKVVDWLRTDQSARVNGTQKYLAKKI
QEKLTKMLPNKHLSFEFILADATEVSELRRQYARQNPLLAKAEKQAPSSHAIDAVMAFVARY
QKVFKDGTPPNADEVAKLAMLDSWNPASNEPLTKGLSTNQKIEKMIKSGDYGQKNMREVFGK
SIFGENAIGERYKPIVVQEGGYYIGYPATVKKGYELKNCKVVTSKNDIAKLEKIIKNQDLIS
LKENQYIKIFSINKQTISELSNRYFNMNYKNLVERDKEIVGLLEFIVENCRYYTKKVDVKFA
PKYIHETKYPFYDDWRRFDEAWRYLQENQNKTSSKDRFVIDKSSLNEYYQPDKNEYKLDVDT
QPIWDDFCRWYFLDRYKTANDKKSIRIKARKTFSLLAESGVQGKVFRAKRKIPTGYAYQALP
MDNNVIAGDYANILLEANSKTLSLVPKSGISIEKQLDKKLDVIKKTDVRGLAIDNNSFFNAD
FDTHGIRLIVENTSVKVGNFPISAIDKSAKRMIFRALFEKEKGKRKKKTTISFKESGPVQDY
LKVFLKKIVKIQLRTDGSISNIVVRKNAADFTLSFRSEHIQKLLK
SEQ ID NO: 341 
MAYRLGLDIGITSVGWAVVALEKDESGLKPVRIQDLGVRIFDKAEDSKTGASLALPRREARS
ARRRTRRRRHRLWRVKRLLEQHGILSMEQIEALYAQRTSSPDVYALRVAGLDRCLIAEEIAR
VLIHIAHRRGFQSNRKSEIKDSDAGKLLKAVQENENLMQSKGYRTVAEMLVSEATKTDAEGK
LVHGKKHGYVSNVRNKAGEYRHTVSRQAIVDEVRKIFAAQRALGNDVMSEELEDSYLKILCS
QRNFDDGPGGDSPYGHGSVSPDGVRQSIYERMVGSCTFETGEKRAPRSSYSFERFQLLTKVV
NLRIYRQQEDGGRYPCELTQTERARVIDCAYEQTKITYGKLRKLLDMKDTESFAGLTYGLNR
SRNKTEDTVFVEMKFYHEVRKALQRAGVFIQDLSIETLDQIGWILSVWKSDDNRRKKLSTLG
LSDNVIEELLPLNGSKFGHLSLKAIRKILPFLEDGYSYDVACELAGYQFQGKTEYVKQRLLP
PLGEGEVTNPVVRRALSQAIKVVNAVIRKHGSPESIHIELARELSKNLDERRKIEKAQKENQ
KNNEQIKDEIREILGSAHVTGRDIVKYKLFKQQQEFCMYSGEKLDVTRLFEPGYAEVDHIIP
YGISFDDSYDNKVLVKTEQNRQKGNRTPLEYLRDKPEQKAKFIALVESIPLSQKKKNHLLMD
KRAIDLEQEGFRERNLSDTRYITRALMNHIQAWLLFDETASTRSKRVVCVNGAVTAYMRARW
GLTKDRDAGDKHHAADAVVVACIGDSLIQRVTKYDKFKRNALADRNRYVQQVSKSEGITQYV
DKETGEVFTWESFDERKFLPNEPLEPWPFFRDELLARLSDDPSKNIRAIGLLTYSETEQIDP
IFVSRMPTRKVTGAAHKETIRSPRIVKVDDNKGTEIQVVVSKVALTELKLTKDGEIKDYFRP
EDDPRLYNTLRERLVQFGGDAKAAFKEPVYKISKDGSVRTPVRKVKIQEKLTLGVPVHGGRG
IAENGGMVRIDVFAKGGKYYFVPIYVADVLKRELPNRLATAHKPYSEWRVVDDSYQFKFSLY
PNDAVMIKPSREVDITYKDRKEPVGCRIMYFVSANIASASISLRTHDNSGELEGLGIQGLEV
FEKYVVGPLGDTHPVYKERRMPFRVERKMN
SEQ ID NO: 342 
MPVLSPLSPNAAQGRRRWSLALDIGEGSIGWAVAEVDAEGRVLQLTGTGVTLFPSAWSNENG
TYVAHGAADRAVRGQQQRHDSRRRRLAGLARLCAPVLERSPEDLKDLTRTPPKADPRAIFFL
RADAARRPLDGPELFRVLHHMAAHRGIRLAELQEVDPPPESDADDAAPAATEDEDGTRRAAA
DERAFRRLMAEHMHRHGTQPTCGEIMAGRLRETPAGAQPVTRARDGLRVGGGVAVPTRALIE
QEFDAIRAIQAPRHPDLPWDSLRRLVLDQAPIAVPPATPCLFLEELRRRGETFQGRTITREA
IDRGLTVDPLIQALRIRETVGNLRLHERITEPDGRQRYVPRAMPELGLSHGELTAPERDTLV
RALMHDPDGLAAKDGRIPYTRLRKLIGYDNSPVCFAQERDTSGGGITVNPTDPLMARWIDGW
 
VDLPLKARSLYVRDVVARGADSAALARLLAEGAHGVPPVAAAAVPAATAAILESDIMQPGRY
SVCPWAAEAILDAWANAPTEGFYDVTRGLFGFAPGEIVLEDLRRARGALLAHLPRTMAAART
PNRAAQQRGPLPAYESVIPSQLITSLRRAHKGRAADWSAADPEERNPFLRTWTGNAATDHIL
NQVRKTANEVITKYGNRRGWDPLPSRITVELAREAKHGVIRRNEIAKENRENEGRRKKESAA
LDTFCQDNTVSWQAGGLPKERAALRLRLAQRQEFFCPYCAERPKLRATDLFSPAETEIDHVI
ERRMGGDGPDNLVLAHKDCNNAKGKKTPHEHAGDLLDSPALAALWQGWRKENADRLKGKGHK
ARTPREDKDFMDRVGWRFEEDARAKAEENQERRGRRMLHDTARATRLARLYLAAAVMPEDPA
EIGAPPVETPPSPEDPTGYTAIYRTISRVQPVNGSVTHMLRQRLLQRDKNRDYQTHHAEDAC
LLLLAGPAVVQAFNTEAAQHGADAPDDRPVDLMPTSDAYHQQRRARALGRVPLATVDAALAD
IVMPESDRQDPETGRVHWRLTRAGRGLKRRIDDLTRNCVILSRPRRPSETGTPGALHNATHY
GRREITVDGRTDTVVTQRMNARDLVALLDNAKIVPAARLDAAAPGDTILKEICTEIADRHDR
VVDPEGTHARRWISARLAALVPAHAEAVARDIAELADLDALADADRTPEQEARRSALRQSPY
LGRAISAKKADGRARAREQEILTRALLDPHWGPRGLRHLIMREARAPSLVRIRANKTDAFGR
PVPDAAVWVKTDGNAVSQLWRLTSVVTDDGRRIPLPKPIEKRIEISNLEYARLNGLDEGAGV
TGNNAPPRPLRQDIDRLTPLWRDHGTAPGGYLGTAVGELEDKARSALRGKAMRQTLTDAGIT
AEAGWRLDSEGAVCDLEVAKGDTVKKDGKTYKVGVITQGIFGMPVDAAGSAPRTPEDCEKFE
EQYGIKPWKAKGIPLA
SEQ ID NO: 343 
MNYTEKEKLFMKYILALDIGIASVGWAILDKESETVIEAGSNIFPEASAADNQLRRDMRGAK
RNNRRLKTRINDFIKLWENNNLSIPQFKSTEIVGLKVRAITEEITLDELYLILYSYLKHRGI
SYLEDALDDTVSGSSAYANGLKLNAKELETHYPCEIQQERLNTIGKYRGQSQIINENGEVLD
LSNVFTIGAYRKEIQRVFEIQKKYHPELTDEFCDGYMLIFNRKRKYYEGPGNEKSRTDYGRF
TTKLDANGNYITEDNIFEKLIGKCSVYPDELRAAAASYTAQEYNVLNDLNNLTINGRKLEEN
EKHEIVERIKSSNTINMRKIISDCMGENIDDFAGARIDKSGKEIFHKFEVYNKMRKALLEIG
IDISNYSREELDEIGYIMTINTDKEAMMEAFQKSWIDLSDDVKQCLINMRKTNGALFNKWQS
FSLKIMNELIPEMYAQPKEQMTLLTEMGVTKGTQEEFAGLKYIPVDVVSEDIFNPVVRRSVR
ISFKILNAVLKKYKALDTIVIEMPRDRNSEEQKKRINDSQKLNEKEMEYIEKKLAVTYGIKL
SPSDFSSQKQLSLKLKLWNEQDGICLYSGKTIDPNDIINNPQLFEIDHIIPRSISFDDARSN
KVLVYRSENQKKGNQTPYYYLTHSHSEWSFEQYKATVMNLSKKKEYAISRKKIQNLLYSEDI
TKMDVLKGFINRNINDTSYASRLVLNTIQNFFMANEADTKVKVIKGSYTHQMRCNLKLDKNR
DESYSHHAVDAMLIGYSELGYEAYHKLQGEFIDFETGEILRKDMWDENMSDEVYADYLYGKK
WANIRNEVVKAEKNVKYWHYVMRKSNRGLCNQTIRGTREYDGKQYKINKLDIRTKEGIKVFA
KLAFSKKDSDRERLLVYLNDRRTFDDLCKIYEDYSDAANPFVQYEKETGDIIRKYSKKHNGP
RIDKLKYKDGEVGACIDISHKYGFEKGSKKVILESLVPYRMDVYYKEENHSYYLVGVKQSDI
KFEKGRNVIDEEAYARILVNEKMIQPGQSRADLENLGFKFKLSFYKNDIIEYEKDGKIYTER
LVSRTMPKQRNYIETKPIDKAKFEKQNLVGLGKTKFIKKYRYDILGNKYSCSEEKFTSFC
SEQ ID NO: 344 
MLRLYCANNLVLNNVQNLWKYLLLLIFDKKIIFLFKIKVILIRRYMENNNKEKIVIGFDLGV
ASVGWSIVNAETKEVIDLGVRLFSEPEKADYRRAKRTTRRLLRRKKFKREKFHKLILKNAEI
FGLQSRNEILNVYKDQSSKYRNILKLKINALKEEIKPSELVWILRDYLQNRGYFYKNEKLTD
EFVSNSFPSKKLHEHYEKYGFFRGSVKLDNKLDNKKDKAKEKDEEEESDAKKESEELIFSNK
QWINEIVKVFENQSYLTESFKEEYLKLFNYVRPFNKGPGSKNSRTAYGVFSTDIDPETNKFK
DYSNIWDKTIGKCSLFEEEIRAPKNLPSALIFNLQNEICTIKNEFTEFKNWWLNAEQKSEIL
KFVFTELFNWKDKKYSDKKFNKNLQDKIKKYLLNFALENFNLNEEILKNRDLENDTVLGLKG
VKYYEKSNATADAALEFSSLKPLYVFIKFLKEKKLDLNYLLGLENTEILYFLDSIYLAISYS
SDLKERNEWFKKLLKELYPKIKNNNLEIIENVEDIFEITDQEKFESFSKTHSLSREAFNHII
PLLLSNNEGKNYESLKHSNEELKKRTEKAELKAQQNQKYLKDNFLKEALVPLSVKTSVLQAI
KIFNQIIKNFGKKYEISQVVIEMARELTKPNLEKLLNNATNSNIKILKEKLDQTEKFDDFTK
KKFIDKIENSVVFRNKLFLWFEQDRKDPYTQLDIKINEIEDETEIDHVIPYSKSADDSWFNK
 
LLVKKSTNQLKKNKTVWEYYQNESDPEAKWNKFVAWAKRIYLVQKSDKESKDNSEKNSIFKN
KKPNLKFKNITKKLFDPYKDLGFLARNLNDTRYATKVFRDQLNNYSKHHSKDDENKLFKVVC
MNGSITSFLRKSMWRKNEEQVYRFNFWKKDRDQFFHHAVDASIIAIFSLLTKTLYNKLRVYE
SYDVQRREDGVYLINKETGEVKKADKDYWKDQHNFLKIRENAIEIKNVLNNVDFQNQVRYSR
KANTKLNTQLFNETLYGVKEFENNFYKLEKVNLFSRKDLRKFILEDLNEESEKNKKNENGSR
KRILTEKYIVDEILQILENEEFKDSKSDINALNKYMDSLPSKFSEFFSQDFINKCKKENSLI
LTFDAIKHNDPKKVIKIKNLKFFREDATLKNKQAVHKDSKNQIKSFYESYKCVGFIWLKNKN
DLEESIFVPINSRVIHFGDKDKDIFDFDSYNKEKLLNEINLKRPENKKFNSINEIEFVKFVK
PGALLLNFENQQIYYISTLESSSLRAKIKLLNKMDKGKAVSMKKITNPDEYKIIEHVNPLGI
NLNWTKKLENNN
SEQ ID NO: 345 
MLMSKHVLGLDLGVGSIGWCLIALDAQGDPAEILGMGSRVVPLNNATKAIEAFNAGAAFTAS
QERTARRTMRRGFARYQLRRYRLRRELEKVGMLPDAALIQLPLLELWELRERAATAGRRLTL
PELGRVLCHINQKRGYRHVKSDAAAIVGDEGEKKKDSNSAYLAGIRANDEKLQAEHKTVGQY
FAEQLRQNQSESPTGGISYRIKDQIFSRQCYIDEYDQIMAVQRVHYPDILTDEFIRMLRDEV
IFMQRPLKSCKHLVSLCEFEKQERVMRVQQDDGKGGWQLVERRVKFGPKVAPKSSPLFQLCC
IYEAVNNIRLTRPNGSPCDITPEERAKIVAHLQSSASLSFAALKKLLKEKALIADQLTSKSG
LKGNSTRVALASALQPYPQYHHLLDMELETRMMTVQLTDEETGEVTEREVAVVTDSYVRKPL
YRLWHILYSIEEREAMRRALITQLGMKEEDLDGGLLDQLYRLDFVKPGYGNKSAKFICKLLP
QLQQGLGYSEACAAVGYRHSNSPTSEEITERTLLEKIPLLQRNELRQPLVEKILNQMINLVN
ALKAEYGIDEVRVELARELKMSREERERMARNNKDREERNKGVAAKIRECGLYPTKPRIQKY
MLWKEAGRQCLYCGRSIEEEQCLREGGMEVEHIIPKSVLYDDSYGNKTCACRRCNKEKGNRT
ALEYIRAKGREAEYMKRINDLLKEKKISYSKHQRLRWLKEDIPSDFLERQLRLTQYISRQAM
AILQQGIRRVSASEGGVTARLRSLWGYGKILHTLNLDRYDSMGETERVSREGEATEELHITN
WSKRMDHRHHAIDALVVACTRQSYIQRLNRLSSEFGREDKKKEDQEAQEQQATETGRLSNLE
RWLTQRPHFSVRTVSDKVAEILISYRPGQRVVTRGRNIYRKKMADGREVSCVQRGVLVPRGE
LMEASFYGKILSQGRVRIVKRYPLHDLKGEVVDPHLRELITTYNQELKSREKGAPIPPLCLD
KDKKQEVRSVRCYAKTLSLDKAIPMCFDEKGEPTAFVKSASNHHLALYRTPKGKLVESIVTF
WDAVDRARYGIPLVITHPREVMEQVLQRGDIPEQVLSLLPPSDWVFVDSLQQDEMVVIGLSD
EELQRALEAQNYRKISEHLYRVQKMSSSYYVFRYHLETSVADDKNTSGRIPKFHRVQSLKAY
EERNIRKVRVDLLGRISLL
SEQ ID NO: 346 
MSDLVLGLDIGIGSVGVGILNKVTGEIIHKNSRIFPAAQAENNLVRRTNRQGRRLARRKKHR
RVRLNRLFEESGLITDFTKISINLNPYQLRVKGLTDELSNEELFIALKNMVKHRGISYLDDA
SDDGNSSVGDYAQIVKENSKQLETKTPGQIQLERYQTYGQLRGDFTVEKDGKKHRLINVFPT
SAYRSEALRILQTQQEFNPQITDEFINRYLEILTGKRKYYHGPGNEKSRTDYGRYRTSGETL
DNIFGILIGKCTFYPDEFRAAKASYTAQEFNLLNDLNNLTVPTETKKLSKEQKNQIINYVKN
EKAMGPAKLFKYIAKLLSCDVADIKGYRIDKSGKAEIHTFEAYRKMKTLETLDIEQMDRETL
DKLAYVLTLNTEREGIQEALEHEFADGSFSQKQVDELVQFRKANSSIFGKGWHNFSVKLMME
LIPELYETSEEQMTILTRLGKQKTTSSSNKTKYIDEKLLTEEIYNPVVAKSVRQAIKIVNAA
IKEYGDFDNIVIEMARETNEDDEKKAIQKIQKANKDEKDAAMLKAANQYNGKAELPHSVFHG
HKQLATKIRLWHQQGERCLYTGKTISIHDLINNSNQFEVDHILPLSITFDDSLANKVLVYAT
ANQEKGQRTPYQALDSMDDAWSFRELKAFVRESKTLSNKKKEYLLTEEDISKFDVRKKFIER
NLVDTRYASRVVLNALQEHFRAHKIDTKVSVVRGQFTSQLRRHWGIEKTRDTYHHHAVDALI
IAASSQLNLWKKQKNTLVSYSEDQLLDIETGELISDDEYKESVFKAPYQHFVDTLKSKEFED
SILFSYQVDSKFNRKISDATIYATRQAKVGKDKADETYVLGKIKDIYTQDGYDAFMKIYKKD
KSKFLMYRHDPQTFEKVIEPILENYPNKQINEKGKEVPCNPFLKYKEEHGYIRKYSKKGNGP
EIKSLKYYDSKLGNHIDITPKDSNNKVVLQSVSPWRADVYFNKTTGKYEILGLKYADLQFEK
GTGTYKISQEKYNDIKKKEGVDSDSEFKFTLYKNDLLLVKDTETKEQQLFRFLSRTMPKQKH
 
YVELKPYDKQKFEGGEALIKVLGNVANSGQCKKGLGKSNISIYKVRTDVLGNQHIIKNEGDK
PKLDF
SEQ ID NO: 347 
MNAEHGKEGLLIMEENFQYRIGLDIGITSVGWAVLQNNSQDEPVRITDLGVRIFDVAENPKN
GDALAAPRRDARTTRRRLRRRRHRLERIKFLLQENGLIEMDSFMERYYKGNLPDVYQLRYEG
LDRKLKDEELAQVLIHIAKHRGFRSTRKAETKEKEGGAVLKATTENQKIMQEKGYRTVGEML
YLDEAFHTECLWNEKGYVLTPRNRPDDYKHTILRSMLVEEVHAIFAAQRAHGNQKATEGLEE
AYVEIMTSQRSFDMGPGLQPDGKPSPYAMEGFGDRVGKCTFEKDEYRAPKATYTAELFVALQ
KINHTKLIDEFGTGRFFSEEERKTIIGLLLSSKELKYGTIRKKLNIDPSLKFNSLNYSAKKE
GETEEERVLDTEKAKFASMFWTYEYSKCLKDRTEEMPVGEKADLFDRIGEILTAYKNDDSRS
SRLKELGLSGEEIDGLLDLSPAKYQRVSLKAMRKMQPYLEDGLIYDKACEAAGYDFRALNDG
NKKHLLKGEEINAIVNDITNPVVKRSVSQTIKVINAIIQKYGSPQAVNIELAREMSKNFQDR
TNLEKEMKKRQQENERAKQQIIELGKQNPTGQDILKYRLWNDQGGYCLYSGKKIPLEELFDG
GYDIDHILPYSITFDDSYRNKVLVTAQENRQKGNRTPYEYFGADEKRWEDYEASVRLLVRDY
KKQQKLLKKNFTEEERKEFKERNLNDTKYITRVVYNMIRQNLELEPFNHPEKKKQVWAVNGA
VTSYLRKRWGLMQKDRSTDRHHAMDAVVIACCTDGMIHKISRYMQGRELAYSRNFKFPDEET
GEILNRDNFTREQWDEKFGVKVPLPWNSFRDELDIRLLNEDPKNFLLTHADVQRELDYPGWM
YGEEESPIEEGRYINYIRPLFVSRMPNHKVTGSAHDATIRSARDYETRGVVITKVPLTDLKL
NKDNEIEGYYDKDSDRLLYQALVRQLLLHGNDGKKAFAEDFHKPKADGTEGPVVRKVKIEKK
QTSGVMVRGGTGIAANGEMVRIDVFRENGKYYFVPVYTADVVRKVLPNRAATHTKPYSEWRV
MDDANFVFSLYSRDLIHVKSKKDIKTNLVNGGLLLQKEIFAYYTGADIATASIAGFANDSNF
KFRGLGIQSLEIFEKCQVDILGNISVVRHENRQEFH
SEQ ID NO: 348 
MRVLGLDAGIASLGWALIEIEESNRGELSQGTIIGAGTWMFDAPEEKTQAGAKLKSEQRRTF
RGQRRVVRRRRQRMNEVRRILHSHGLLPSSDRDALKQPGLDPWRIRAEALDRLLGPVELAVA
LGHIARHRGFKSNSKGAKTNDPADDTSKMKRAVNETREKLARFGSAAKMLVEDESFVLRQTP
TKNGASEIVRRFRNREGDYSRSLLRDDLAAEMRALFTAQARFQSAIATADLQTAFTKAAFFQ
RPLQDSEKLVGPCPFEVDEKRAPKRGYSFELFRFLSRLNHVTLRDGKQERTLTRDELALAAA
DFGAAAKVSFTALRKKLKLPETTVFVGVKADEESKLDVVARSGKAAEGTARLRSVIVDALGE
LAWGALLCSPEKLDKIAEVISFRSDIGRISEGLAQAGCNAPLVDALTAAASDGRFDPFTGAG
HISSKAARNILSGLRQGMTYDKACCAADYDHTASRERGAFDVGGHGREALKRILQEERISRE
LVGSPTARKALIESIKQVKAIVERYGVPDRIHVELARDVGKSIEEREEITRGIEKRNRQKDK
LRGLFEKEVGRPPQDGARGKEELLRFELWSEQMGRCLYTDDYISPSQLVATDDAVQVDHILP
WSRFADDSYANKTLCMAKANQDKKGRTPYEWFKAEKTDTEWDAFIVRVEALADMKGFKKRNY
KLRNAEEAAAKFRNRNLNDTRWACRLLAEALKQLYPKGEKDKDGKERRRVFSRPGALTDRLR
RAWGLQWMKKSTKGDRIPDDRHHALDAIVIAATTESLLQRATREVQEIEDKGLHYDLVKNVT
PPWPGFREQAVEAVEKVFVARAERRRARGKAHDATIRHIAVREGEQRVYERRKVAELKLADL
DRVKDAERNARLIEKLRNWIEAGSPKDDPPLSPKGDPIFKVRLVTKSKVNIALDTGNPKRPG
TVDRGEMARVDVFRKASKKGKYEYYLVPIYPHDIATMKTPPIRAVQAYKPEDEWPEMDSSYE
FCWSLVPMTYLQVISSKGEIFEGYYRGMNRSVGAIQLSAHSNSSDVVQGIGARTLTEFKKFN
VDRFGRKHEVERELRTWRGETWRGKAYI
SEQ ID NO: 349 
MGNYYLGLDVGIGSIGWAVINIEKKRIEDFNVRIFKSGEIQEKNRNSRASQQCRRSRGLRRL
YRRKSHRKLRLKNYLSIIGLTTSEKIDYYYETADNNVIQLRNKGLSEKLTPEEIAACLIHIC
NNRGYKDFYEVNVEDIEDPDERNEYKEEHDSIVLISNLMNEGGYCTPAEMICNCREFDEPNS
VYRKFHNSAASKNHYLITRHMLVKEVDLILENQSKYYGILDDKTIAKIKDIIFAQRDFEIGP
GKNERFRRFTGYLDSIGKCQFFKDQERGSRFTVIADIYAFVNVLSQYTYTNNRGESVFDTSF
ANDLINSALKNGSMDKRELKAIAKSYHIDISDKNSDTSLTKCFKYIKVVKPLFEKYGYDWDK
 
LIENYTDTDNNVLNRIGIVLSQAQTPKRRREKLKALNIGLDDGLINELTKLKLSGTANVSYK
YMQGSIEAFCEGDLYGKYQAKFNKEIPDIDENAKPQKLPPFKNEDDCEFFKNPVVFRSINET
RKLINAIIDKYGYPAAVNIETADELNKTFEDRAIDTKRNNDNQKENDRIVKEIIECIKCDEV
HARHLIEKYKLWEAQEGKCLYSGETITKEDMLRDKDKLFEVDHIVPYSLILDNTINNKALVY
AEENQKKGQRTPLMYMNEAQAADYRVRVNTMFKSKKCSKKKYQYLMLPDLNDQELLGGWRSR
NLNDTRYICKYLVNYLRKNLRFDRSYESSDEDDLKIRDHYRVFPVKSRFTSMFRRWWLNEKT
WGRYDKAELKKLTYLDHAADAIIIANCRPEYVVLAGEKLKLNKMYHQAGKRITPEYEQSKKA
CIDNLYKLFRMDRRTAEKLLSGHGRLTPIIPNLSEEVDKRLWDKNIYEQFWKDDKDKKSCEE
LYRENVASLYKGDPKFASSLSMPVISLKPDHKYRGTITGEEAIRVKEIDGKLIKLKRKSISE
ITAESINSIYTDDKILIDSLKTIFEQADYKDVGDYLKKTNQHFFTTSSGKRVNKVTVIEKVP
SRWLRKEIDDNNFSLLNDSSYYCIELYKDSKGDNNLQGIAMSDIVHDRKTKKLYLKPDFNYP
DDYYTHVMYIFPGDYLRIKSTSKKSGEQLKFEGYFISVKNVNENSFRFISDNKPCAKDKRVS
ITKKDIVIKLAVDLMGKVQGENNGKGISCGEPLSLLKEKN
SEQ ID NO: 350 
MLSRQLLGASHLARPVSYSYNVQDNDVHCSYGERCFMRGKRYRIGIDVGLNSVGLAAVEVSD
ENSPVRLLNAQSVIHDGGVDPQKNKEAITRKNMSGVARRTRRMRRRKRERLHKLDMLLGKFG
YPVIEPESLDKPFEEWHVRAELATRYIEDDELRRESISIALRHMARHRGWRNPYRQVDSLIS
DNPYSKQYGELKEKAKAYNDDATAAEEESTPAQLVVAMLDAGYAEAPRLRWRTGSKKPDAEG
YLPVRLMQEDNANELKQIFRVQRVPADEWKPLFRSVFYAVSPKGSAEQRVGQDPLAPEQARA
LKASLAFQEYRIANVITNLRIKDASAELRKLTVDEKQSIYDQLVSPSSEDITWSDLCDFLGF
KRSQLKGVGSLTEDGEERISSRPPRLTSVQRIYESDNKIRKPLVAWWKSASDNEHEAMIRLL
SNTVDIDKVREDVAYASAIEFIDGLDDDALTKLDSVDLPSGRAAYSVETLQKLTRQMLTTDD
DLHEARKTLFNVTDSWRPPADPIGEPLGNPSVDRVLKNVNRYLMNCQQRWGNPVSVNIEHVR
SSFSSVAFARKDKREYEKNNEKRSIFRSSLSEQLRADEQMEKVRESDLRRLEAIQRQNGQCL
YCGRTITFRTCEMDHIVPRKGVGSTNTRTNFAAVCAECNRMKSNTPFAIWARSEDAQTRGVS
LAEAKKRVTMFTFNPKSYAPREVKAFKQAVIARLQQTEDDAAIDNRSIESVAWMADELHRRI
DWYFNAKQYVNSASIDDAEAETMKTTVSVFQGRVTASARRAAGIEGKIHFIGQQSKTRLDRR
HHAVDASVIAMMNTAAAQTLMERESLRESQRLIGLMPGERSWKEYPYEGTSRYESFHLWLDN
MDVLLELLNDALDNDRIAVMQSQRYVLGNSIAHDATIHPLEKVPLGSAMSADLIRRASTPAL
WCALTRLPDYDEKEGLPEDSHREIRVHDTRYSADDEMGFFASQAAQIAVQEGSADIGSAIHH
ARVYRCWKTNAKGVRKYFYGMIRVFQTDLLRACHDDLFTVPLPPQSISMRYGEPRVVQALQS
GNAQYLGSLVVGDEIEMDFSSLDVDGQIGEYLQFFSQFSGGNLAWKHWVVDGFFNQTQLRIR
PRYLAAEGLAKAFSDDVVPDGVQKIVTKQGWLPPVNTASKTAVRIVRRNAFGEPRLSSAHHM
PCSWQWRHE
SEQ ID NO: 351 
MYSIGLDLGISSVGWSVIDERTGNVIDLGVRLFSAKNSEKNLERRTNRGGRRLIRRKTNRLK
DAKKILAAVGFYEDKSLKNSCPYQLRVKGLTEPLSRGEIYKVTLHILKKRGISYLDEVDTEA
AKESQDYKEQVRKNAQLLTKYTPGQIQLQRLKENNRVKTGINAQGNYQLNVFKVSAYANELA
TILKTQQAFYPNELTDDWIALFVQPGIAEEAGLIYRKRPYYHGPGNEANNSPYGRWSDFQKT
GEPATNIFDKLIGKDFQGELRASGLSLSAQQYNLLNDLTNLKIDGEVPLSSEQKEYILTELM
TKEFTRFGVNDVVKLLGVKKERLSGWRLDKKGKPEIHTLKGYRNWRKIFAEAGIDLATLPTE
TIDCLAKVLTLNTEREGIENTLAFELPELSESVKLLVLDRYKELSQSISTQSWHRFSLKTLH
LLIPELMNATSEQNTLLEQFQLKSDVRKRYSEYKKLPTKDVLAEIYNPTVNKTVSQAFKVID
ALLVKYGKEQIRYITIEMPRDDNEEDEKKRIKELHAKNSQRKNDSQSYFMQKSGWSQEKFQT
TIQKNRRFLAKLLYYYEQDGICAYTGLPISPELLVSDSTEIDHIIPISISLDDSINNKVLVL
SKANQVKGQQTPYDAWMDGSFKKINGKFSNWDDYQKWVESRHFSHKKENNLLETRNIFDSEQ
VEKFLARNLNDTRYASRLVLNTLQSFFTNQETKVRVVNGSFTHTLRKKWGADLDKTRETHHH
HAVDATLCAVTSFVKVSRYHYAVKEETGEKVMREIDFETGEIVNEMSYWEFKKSKKYERKTY
QVKWPNFREQLKPVNLHPRIKFSHQVDRKANRKLSDATIYSVREKTEVKTLKSGKQKITTDE
 
YTIGKIKDIYTLDGWEAFKKKQDKLLMKDLDEKTYERLLSIAETTPDFQEVEEKNGKVKRVK
RSPFAVYCEENDIPAIQKYAKKNNGPLIRSLKYYDGKLNKHINITKDSQGRPVEKTKNGRKV
TLQSLKPYRYDIYQDLETKAYYTVQLYYSDLRFVEGKYGITEKEYMKKVAEQTKGQVVRFCF
SLQKNDGLEIEWKDSQRYDVRFYNFQSANSINFKGLEQEMMPAENQFKQKPYNNGAINLNIA
KYGKEGKKLRKFNTDILGKKHYLFYEKEPKNIIK
SEQ ID NO: 352 
MYFYKNKENKLNKKVVLGLDLGIASVGWCLTDISQKEDNKFPIILHGVRLFETVDDSDDKLL
NETRRKKRGQRRRNRRLFTRKRDFIKYLIDNNIIELEFDKNPKILVRNFIEKYINPFSKNLE
LKYKSVTNLPIGFHNLRKAAINEKYKLDKSELIVLLYFYLSLRGAFFDNPEDTKSKEMNKNE
IEIFDKNESIKNAEFPIDKIIEFYKISGKIRSTINLKFGHQDYLKEIKQVFEKQNIDFMNYE
KFAMEEKSFFSRIRNYSEGPGNEKSFSKYGLYANENGNPELIINEKGQKIYTKIFKTLWESK
IGKCSYDKKLYRAPKNSFSAKVFDITNKLTDWKHKNEYISERLKRKILLSRFLNKDSKSAVE
KILKEENIKFENLSEIAYNKDDNKINLPIINAYHSLTTIFKKHLINFENYLISNENDLSKLM
SFYKQQSEKLFVPNEKGSYEINQNNNVLHIFDAISNILNKFSTIQDRIRILEGYFEFSNLKK
DVKSSEIYSEIAKLREFSGTSSLSFGAYYKFIPNLISEGSKNYSTISYEEKALQNQKNNFSH
SNLFEKTWVEDLIASPTVKRSLRQTMNLLKEIFKYSEKNNLEIEKIVVEVTRSSNNKHERKK
IEGINKYRKEKYEELKKVYDLPNENTTLLKKLWLLRQQQGYDAYSLRKIEANDVINKPWNYD
IDHIVPRSISFDDSFSNLVIVNKLDNAKKSNDLSAKQFIEKIYGIEKLKEAKENWGNWYLRN
ANGKAFNDKGKFIKLYTIDNLDEFDNSDFINRNLSDTSYITNALVNHLTFSNSKYKYSVVSV
NGKQTSNLRNQIAFVGIKNNKETEREWKRPEGFKSINSNDFLIREEGKNDVKDDVLIKDRSF
NGHHAEDAYFITIISQYFRSFKRIERLNVNYRKETRELDDLEKNNIKFKEKASFDNFLLINA
LDELNEKLNQMRFSRMVITKKNTQLFNETLYSGKYDKGKNTIKKVEKLNLLDNRTDKIKKIE
EFFDEDKLKENELTKLHIFNHDKNLYETLKIIWNEVKIEIKNKNLNEKNYFKYFVNKKLQEG
KISFNEWVPILDNDFKIIRKIRYIKFSSEEKETDEIIFSQSNFLKIDQRQNFSFHNTLYWVQ
IWVYKNQKDQYCFISIDARNSKFEKDEIKINYEKLKTQKEKLQIINEEPILKINKGDLFENE
EKELFYIVGRDEKPQKLEIKYILGKKIKDQKQIQKPVKKYFPNWKKVNLTYMGEIFKK
SEQ ID NO: 353 
MDNKNYRIGIDVGLNSIGFCAVEVDQHDTPLGFLNLSVYRHDAGIDPNGKKTNTTRLAMSGV
ARRTRRLFRKRKRRLAALDRFIEAQGWTLPDHADYKDPYTPWLVRAELAQTPIRDENDLHEK
LAIAVRHIARHRGWRSPWVPVRSLHVEQPPSDQYLALKERVEAKTLLQMPEGATPAEMVVAL
DLSVDVNLRPKNREKTDTRPENKKPGFLGGKLMQSDNANELRKIAKIQGLDDALLRELIELV
FAADSPKGASGELVGYDVLPGQHGKRRAEKAHPAFQRYRIASIVSNLRIRHLGSGADERLDV
ETQKRVFEYLLNAKPTADITWSDVAEEIGVERNLLMGTATQTADGERASAKPPVDVTNVAFA
TCKIKPLKEWWLNADYEARCVMVSALSHAEKLTEGTAAEVEVAEFLQNLSDEDNEKLDSFSL
PIGRAAYSVDSLERLTKRMIENGEDLFEARVNEFGVSEDWRPPAEPIGARVGNPAVDRVLKA
VNRYLMAAEAEWGAPLSVNIEHVREGFISKRQAVEIDRENQKRYQRNQAVRSQIADHINATS
GVRGSDVTRYLAIQRQNGECLYCGTAITFVNSEMDHIVPRAGLGSTNTRDNLVATCERCNKS
KSNKPFAVWAAECGIPGVSVAEALKRVDFWIADGFASSKEHRELQKGVKDRLKRKVSDPEID
NRSMESVAWMARELAHRVQYYFDEKHTGTKVRVFRGSLTSAARKASGFESRVNFIGGNGKTR
LDRRHHAMDAATVAMLRNSVAKTLVLRGNIRASERAIGAAETWKSFRGENVADRQIFESWSE
NMRVLVEKFNLALYNDEVSIFSSLRLQLGNGKAHDDTITKLQMHKVGDAWSLTEIDRASTPA
LWCALTRQPDFTWKDGLPANEDRTIIVNGTHYGPLDKVGIFGKAAASLLVRGGSVDIGSAIH
HARIYRIAGKKPTYGMVRVFAPDLLRYRNEDLFNVELPPQSVSMRYAEPKVREAIREGKAEY
LGWLVVGDELLLDLSSETSGQIAELQQDFPGTTHWTVAGFFSPSRLRLRPVYLAQEGLGEDV
SEGSKSIIAGQGWRPAVNKVFGSAMPEVIRRDGLGRKRRFSYSGLPVSWQG
SEQ ID NO: 354 
MRLGLDIGTSSIGWWLYETDGAGSDARITGVVDGGVRIFSDGRDPKSGASLAVDRRAARAMR
RRRDRYLRRRATLMKVLAETGLMPADPAEAKALEALDPFALRAAGLDEPLPLPHLGRALFHL
 
NQRRGFKSNRKTDRGDNESGKIKDATARLDMEMMANGARTYGEFLHKRRQKATDPRHVPSVR
TRLSIANRGGPDGKEEAGYDFYPDRRHLEEEFHKLWAAQGAHHPELTETLRDLLFEKIFFQR
PLKEPEVGLCLFSGHHGVPPKDPRLPKAHPLTQRRVLYETVNQLRVTADGREARPLTREERD
QVIHALDNKKPTKSLSSMVLKLPALAKVLKLRDGERFTLETGVRDAIACDPLRASPAHPDRF
GPRWSILDADAQWEVISRIRRVQSDAEHAALVDWLTEAHGLDRAHAEATAHAPLPDGYGRLG
LTATTRILYQLTADVVTYADAVKACGWHHSDGRTGECFDRLPYYGEVLERHVIPGSYHPDDD
DITRFGRITNPTVHIGLNQLRRLVNRIIETHGKPHQIVVELARDLKKSEEQKRADIKRIRDT
TEAAKKRSEKLEELEIEDNGRNRMLLRLWEDLNPDDAMRRFCPYTGTRISAAMIFDGSCDVD
HILPYSRTLDDSFPNRTLCLREANRQKRNQTPWQAWGDTPHWHAIAANLKNLPENKRWRFAP
DAMTRFEGENGFLDRALKDTQYLARISRSYLDTLFTKGGHVWVVPGRFTEMLRRHWGLNSLL
SDAGRGAVKAKNRTDHRHHAIDAAVIAATDPGLLNRISRAAGQGEAAGQSAELIARDTPPPW
EGFRDDLRVRLDRIIVSHRADHGRIDHAARKQGRDSTAGQLHQETAYSIVDDIHVASRTDLL
SLKPAQLLDEPGRSGQVRDPQLRKALRVATGGKTGKDFENALRYFASKPGPYQAIRRVRIIK
PLQAQARVPVPAQDPIKAYQGGSNHLFEIWRLPDGEIEAQVITSFEAHTLEGEKRPHPAAKR
LLRVHKGDMVALERDGRRVVGHVQKMDIANGLFIVPHNEANADTRNNDKSDPFKWIQIGARP
AIASGIRRVSVDEIGRLRDGGTRPI
SEQ ID NO: 355 
MLHCIAVIRVPPSEEPGFFETHADSCALCHHGCMTYAANDKAIRYRVGIDVGLRSIGFCAVE
VDDEDHPIRILNSVVHVHDAGTGGPGETESLRKRSGVAARARRRGRAEKQRLKKLDVLLEEL
GWGVSSNELLDSHAPWHIRKRLVSEYIEDETERRQCLSVAMAHIARHRGWRNSFSKVDTLLL
EQAPSDRMQGLKERVEDRTGLQFSEEVTQGELVATLLEHDGDVTIRGFVRKGGKATKVHGVL
EGKYMQSDLVAELRQICRTQRVSETTFEKLVLSIFHSKEPAPSAARQRERVGLDELQLALDP
AAKQPRAERAHPAFQKFKVVATLANMRIREQSAGERSLTSEELNRVARYLLNHTESESPTWD
DVARKLEVPRHRLRGSSRASLETGGGLTYPPVDDTTVRVMSAEVDWLADWWDCANDESRGHM
IDAISNGCGSEPDDVEDEEVNELISSATAEDMLKLELLAKKLPSGRVAYSLKTLREVTAAIL
ETGDDLSQAITRLYGVDPGWVPTPAPIEAPVGNPSVDRVLKQVARWLKFASKRWGVPQTVNI
EHTREGLKSASLLEEERERWERFEARREIRQKEMYKRLGISGPFRRSDQVRYEILDLQDCAC
LYCGNEINFQTFEVDHIIPRVDASSDSRRTNLAAVCHSCNSAKGGLAFGQWVKRGDCPSGVS
LENAIKRVRSWSKDRLGLTEKAMGKRKSEVISRLKTEMPYEEFDGRSMESVAWMAIELKKRI
EGYFNSDRPEGCAAVQVNAYSGRLTACARRAAHVDKRVRLIRLKGDDGHHKNRFDRRNHAMD
ALVIALMTPAIARTIAVREDRREAQQLTRAFESWKNFLGSEERMQDRWESWIGDVEYACDRL
NELIDADKIPVTENLRLRNSGKLHADQPESLKKARRGSKRPRPQRYVLGDALPADVINRVTD
PGLWTALVRAPGFDSQLGLPADLNRGLKLRGKRISADFPIDYFPTDSPALAVQGGYVGLEFH
HARLYRIIGPKEKVKYALLRVCAIDLCGIDCDDLFEVELKPSSISMRTADAKLKEAMGNGSA
KQIGWLVLGDEIQIDPTKFPKQSIGKFLKECGPVSSWRVSALDTPSKITLKPRLLSNEPLLK
TSRVGGHESDLVVAECVEKIMKKTGWVVEINALCQSGLIRVIRRNALGEVRTSPKSGLPISL
NLR
SEQ ID NO: 356 
MRYRVGLDLGTASVGAAVFSMDEQGNPMELIWHYERLFSEPLVPDMGQLKPKKAARRLARQQ
RRQIDRRASRLRRIAIVSRRLGIAPGRNDSGVHGNDVPTLRAMAVNERIELGQLRAVLLRMG
KKRGYGGTFKAVRKVGEAGEVASGASRLEEEMVALASVQNKDSVTVGEYLAARVEHGLPSKL
KVAANNEYYAPEYALFRQYLGLPAIKGRPDCLPNMYALRHQIEHEFERIWATQSQFHDVMKD
HGVKEEIRNAIFFQRPLKSPADKVGRCSLQTNLPRAPRAQIAAQNFRIEKQMADLRWGMGRR
AEMLNDHQKAVIRELLNQQKELSFRKIYKELERAGCPGPEGKGLNMDRAALGGRDDLSGNTT
LAAWRKLGLEDRWQELDEVTQIQVINFLADLGSPEQLDTDDWSCRFMGKNGRPRNFSDEFVA
FMNELRMTDGFDRLSKMGFEGGRSSYSIKALKALTEWMIAPHWRETPETHRVDEEAAIRECY
PESLATPAQGGRQSKLEPPPLTGNEVVDVALRQVRHTINMMIDDLGSVPAQIVVEMAREMKG
GVTRRNDIEKQNKRFASERKKAAQSIEENGKTPTPARILRYQLWIEQGHQCPYCESNISLEQ
ALSGAYTNFEHILPRTLTQIGRKRSELVLAHRECNDEKGNRTPYQAFGHDDRRWRIVEQRAN
 
ALPKKSSRKTRLLLLKDFEGEALTDESIDEFADRQLHESSWLAKVTTQWLSSLGSDVYVSRG
SLTAELRRRWGLDTVIPQVRFESGMPVVDEEGAEITPEEFEKFRLQWEGHRVTREMRTDRRP
DKRIDHRHHLVDAIVTALTSRSLYQQYAKAWKVADEKQRHGRVDVKVELPMPILTIRDIALE
AVRSVRISHKPDRYPDGRFFEATAYGIAQRLDERSGEKVDWLVSRKSLTDLAPEKKSIDVDK
VRANISRIVGEAIRLHISNIFEKRVSKGMTPQQALREPIEFQGNILRKVRCFYSKADDCVRI
EHSSRRGHHYKMLLNDGFAYMEVPCKEGILYGVPNLVRPSEAVGIKRAPESGDFIRFYKGDT
VKNIKTGRVYTIKQILGDGGGKLILTPVTETKPADLLSAKWGRLKVGGRNIHLLRLCAE
SEQ ID NO: 357 
MIGEHVRGGCLFDDHWTPNWGAFRLPNTVRTFTKAENPKDGSSLAEPRRQARGLRRRLRRKT
QRLEDLRRLLAKEGVLSLSDLETLFRETPAKDPYQLRAEGLDRPLSFPEWVRVLYHITKHRG
FQSNRRNPVEDGQERSRQEEEGKLLSGVGENERLLREGGYRTAGEMLARDPKFQDHRRNRAG
DYSHTLSRSLLLEEARRLFQSQRTLGNPHASSNLEEAFLHLVAFQNPFASGEDIRNKAGHCS
LEPDQIRAPRRSASAETFMLLQKTGNLRLIHRRTGEERPLTDKEREQIHLLAWKQEKVTHKT
LRRHLEIPEEWLFTGLPYHRSGDKAEEKLFVHLAGIHEIRKALDKGPDPAVWDTLRSRRDLL
DSIADTLTFYKNEDEILPRLESLGLSPENARALAPLSFSGTAHLSLSALGKLLPHLEEGKSY
TQARADAGYAAPPPDRHPKLPPLEEADWRNPVVFRALTQTRKVVNALVRRYGPPWCIHLETA
RELSQPAKVRRRIETEQQANEKKKQQAEREFLDIVGTAPGPGDLLKMRLWREQGGFCPYCEE
YLNPTRLAEPGYAEMDHILPYSRSLDNGWHNRVLVHGKDNRDKGNRTPFEAFGGDTARWDRL
VAWVQASHLSAPKKRNLLREDFGEEAERELKDRNLTDTRFITKTAATLLRDRLTFHPEAPKD
PVMTLNGRLTAFLRKQWGLHKNRKNGDLHHALDAAVLAVASRSFVYRLSSHNAAWGELPRGR
EAENGFSLPYPAFRSEVLARLCPTREEILLRLDQGGVGYDEAFRNGLRPVFVSRAPSRRLRG
KAHMETLRSPKWKDHPEGPRTASRIPLKDLNLEKLERMVGKDRDRKLYEALRERLAAFGGNG
KKAFVAPFRKPCRSGEGPLVRSLRIFDSGYSGVELRDGGEVYAVADHESMVRVDVYAKKNRF
YLVPVYVADVARGIVKNRAIVAHKSEEEWDLVDGSFDFRFSLFPGDLVEIEKKDGAYLGYYK
SCHRGDGRLLLDRHDRMPRESDCGTFYVSTRKDVLSMSKYQVDPLGEIRLVGSEKPPFVL
SEQ ID NO: 358 
MEKKRKVTLGFDLGIASVGWAIVDSETNQVYKLGSRLFDAPDTNLERRTQRGTRRLLRRRKY
RNQKFYNLVKRTEVFGLSSREAIENRFRELSIKYPNIIELKTKALSQEVCPDEIAWILHDYL
KNRGYFYDEKETKEDFDQQTVESMPSYKLNEFYKKYGYFKGALSQPTESEMKDNKDLKEAFF
FDFSNKEWLKEINYFFNVQKNILSETFIEEFKKIFSFTRDISKGPGSDNMPSPYGIFGEFGD
NGQGGRYEHIWDKNIGKCSIFTNEQRAPKYLPSALIFNFLNELANIRLYSTDKKNIQPLWKL
SSVDKLNILLNLFNLPISEKKKKLTSTNINDIVKKESIKSIMISVEDIDMIKDEWAGKEPNV
YGVGLSGLNIEESAKENKFKFQDLKILNVLINLLDNVGIKFEFKDRNDIIKNLELLDNLYLF
LIYQKESNNKDSSIDLFIAKNESLNIENLKLKLKEFLLGAGNEFENHNSKTHSLSKKAIDEI
LPKLLDNNEGWNLEAIKNYDEEIKSQIEDNSSLMAKQDKKYLNDNFLKDAILPPNVKVTFQQ
AILIFNKIIQKFSKDFEIDKVVIELAREMTQDQENDALKGIAKAQKSKKSLVEERLEANNID
KSVFNDKYEKLIYKIFLWISQDFKDPYTGAQISVNEIVNNKVEIDHIIPYSLCFDDSSANKV
LVHKQSNQEKSNSLPYEYIKQGHSGWNWDEFTKYVKRVFVNNVDSILSKKERLKKSENLLTA
SYDGYDKLGFLARNLNDTRYATILFRDQLNNYAEHHLIDNKKMFKVIAMNGAVTSFIRKNMS
YDNKLRLKDRSDFSHHAYDAAIIALFSNKTKTLYNLIDPSLNGIISKRSEGYWVIEDRYTGE
IKELKKEDWTSIKNNVQARKIAKEIEEYLIDLDDEVFFSRKTKRKTNRQLYNETIYGIATKT
DEDGITNYYKKEKFSILDDKDIYLRLLREREKFVINQSNPEVIDQIIEIIESYGKENNIPSR
DEAINIKYTKNKINYNLYLKQYMRSLTKSLDQFSEEFINQMIANKTFVLYNPTKNTTRKIKF
LRLVNDVKINDIRKNQVINKFNGKNNEPKAFYENINSLGAIVFKNSANNFKTLSINTQIAIF
GDKNWDIEDFKTYNMEKIEKYKEIYGIDKTYNFHSFIFPGTILLDKQNKEFYYISSIQTVRD
IIEIKFLNKIEFKDENKNQDTSKTPKRLMFGIKSIMNNYEQVDISPFGINKKIFE
SEQ ID NO: 359 
MGYRIGLDVGITSTGYAVLKTDKNGLPYKILTLDSVIYPRAENPQTGASLAEPRRIKRGLRR
RTRRTKFRKQRTQQLFIHSGLLSKPEIEQILATPQAKYSVYELRVAGLDRRLTNSELFRVLY
FFIGHRGFKSNRKAELNPENEADKKQMGQLLNSIEEIRKAIAEKGYRTVGELYLKDPKYNDH
KRNKGYIDGYLSTPNRQMLVDEIKQILDKQRELGNEKLTDEFYATYLLGDENRAGIFQAQRD
FDEGPGAGPYAGDQIKKMVGKDIFEPTEDRAAKATYTFQYFNLLQKMTSLNYQNTTGDTWHT
LNGLDRQAIIDAVFAKAEKPTKTYKPTDFGELRKLLKLPDDARFNLVNYGSLQTQKEIETVE
KKTRFVDFKAYHDLVKVLPEEMWQSRQLLDHIGTALTLYSSDKRRRRYFAEELNLPAELIEK
LLPLNFSKFGHLSIKSMQNIIPYLEMGQVYSEATTNTGYDFRKKQISKDTIREEITNPVVRR
AVTKTIKIVEQIIRRYGKPDGINIELARELGRNFKERGDIQKRQDKNRQTNDKIAAELTELG
IPVNGQNIIRYKLHKEQNGVDPYTGDQIPFERAFSEGYEVDHIIPYSISWDDSYTNKVLTSA
KCNREKGNRIPMVYLANNEQRLNALTNIADNIIRNSRKRQKLLKQKLSDEELKDWKQRNIND
TRFITRVLYNYFRQAIEFNPELEKKQRVLPLNGEVTSKIRSRWGFLKVREDGDLHHAIDATV
IAAITPKFIQQVTKYSQHQEVKNNQALWHDAEIKDAEYAAEAQRMDADLFNKIFNGFPLPWP
EFLDELLARISDNPVEMMKSRSWNTYTPIEIAKLKPVFVVRLANHKISGPAHLDTIRSAKLF
DEKGIVLSRVSITKLKINKKGQVATGDGIYDPENSNNGDKVVYSAIRQALEAHNGSGELAFP
DGYLEYVDHGTKKLVRKVRVAKKVSLPVRLKNKAAADNGSMVRIDVFNTGKKFVFVPIYIKD
TVEQVLPNKAIARGKSLWYQITESDQFCFSLYPGDMVHIESKTGIKPKYSNKENNTSVVPIK
NFYGYFDGADIATASILVRAHDSSYTARSIGIAGLLKFEKYQVDYFGRYHKVHEKKRQLFVK
RDE
SEQ ID NO: 360 
MQKNINTKQNHIYIKQAQKIKEKLGDKPYRIGLDLGVGSIGFAIVSMEENDGNVLLPKEIIM
VGSRIFKASAGAADRKLSRGQRNNHRHTRERMRYLWKVLAEQKLALPVPADLDRKENSSEGE
TSAKRFLGDVLQKDIYELRVKSLDERLSLQELGYVLYHIAGHRGSSAIRTFENDSEEAQKEN
TENKKIAGNIKRLMAKKNYRTYGEYLYKEFFENKEKHKREKISNAANNHKFSPTRDLVIKEA
EAILKKQAGKDGFHKELTEEYIEKLTKAIGYESEKLIPESGFCPYLKDEKRLPASHKLNEER
RLWETLNNARYSDPIVDIVTGEITGYYEKQFTKEQKQKLFDYLLTGSELTPAQTKKLLGLKN
TNFEDIILQGRDKKAQKIKGYKLIKLESMPFWARLSEAQQDSFLYDWNSCPDEKLLTEKLSN
EYHLTEEEIDNAFNEIVLSSSYAPLGKSAMLIILEKIKNDLSYTEAVEEALKEGKLTKEKQA
IKDRLPYYGAVLQESTQKIIAKGFSPQFKDKGYKTPHTNKYELEYGRIANPVVHQTLNELRK
LVNEIIDILGKKPCEIGLETARELKKSAEDRSKLSREQNDNESNRNRIYEIYIRPQQQVIIT
RRENPRNYILKFELLEEQKSQCPFCGGQISPNDIINNQADIEHLFPIAESEDNGRNNLVISH
SACNADKAKRSPWAAFASAAKDSKYDYNRILSNVKENIPHKAWRFNQGAFEKFIENKPMAAR
FKTDNSYISKVAHKYLACLFEKPNIICVKGSLTAQLRMAWGLQGLMIPFAKQLITEKESESF
NKDVNSNKKIRLDNRHHALDAIVIAYASRGYGNLLNKMAGKDYKINYSERNWLSKILLPPNN
IVWENIDADLESFESSVKTALKNAFISVKHDHSDNGELVKGTMYKIFYSERGYTLTTYKKLS
ALKLTDPQKKKTPKDFLETALLKFKGRESEMKNEKIKSAIENNKRLFDVIQDNLEKAKKLLE
EENEKSKAEGKKEKNINDASIYQKAISLSGDKYVQLSKKEPGKFFAISKPTPTTTGYGYDTG
DSLCVDLYYDNKGKLCGEIIRKIDAQQKNPLKYKEQGFTLFERIYGGDILEVDFDIHSDKNS
FRNNTGSAPENRVFIKVGTFTEITNNNIQIWFGNIIKSTGGQDDSFTINSMQQYNPRKLILS
SCGFIKYRSPILKNKEG
SEQ ID NO: 361 
MAAFKPNPINYILGLDIGIASVGWAMVEIDEDENPICLIDLGVRVFERAEVPKTGDSLAMAR
RLARSVRRLTRRRAHRLLRARRLLKREGVLQAADFDENGLIKSLPNTPWQLRAAALDRKLTP
LEWSAVLLHLIKHRGYLSQRKNEGETADKELGALLKGVADNAHALQTGDFRTPAELALNKFE
KESGHIRNQRGDYSHTFSRKDLQAELILLFEKQKEFGNPHVSGGLKEGIETLLMTQRPALSG
DAVQKMLGHCTFEPAEPKAAKNTYTAERFIWLTKLNNLRILEQGSERPLTDTERATLMDEPY
RKSKLTYAQARKLLGLEDTAFFKGLRYGKDNAEASTLMEMKAYHAISRALEKEGLKDKKSPL
NLSPELQDEIGTAFSLFKTDEDITGRLKDRIQPEILEALLKHISFDKFVQISLKALRRIVPL
MEQGKRYDEACAEIYGDHYGKKNTEEKIYLPPIPADEIRNPVVLRALSQARKVINGVVRRYG
 
SPARIHIETAREVGKSFKDRKEIEKRQEENRKDREKAAAKFREYFPNFVGEPKSKDILKLRL
YEQQHGKCLYSGKEINLGRLNEKGYVEIDHALPFSRTWDDSFNNKVLVLGSENQNKGNQTPY
EYFNGKDNSREWQEFKARVETSRFPRSKKQRILLQKFDEDGFKERNLNDTRYVNRFLCQFVA
DRMRLTGKGKKRVFASNGQITNLLRGFWGLRKVRAENDRHHALDAVVVACSTVAMQQKITRF
VRYKEMNAFDGKTIDKETGEVLHQKTHFPQPWEFFAQEVMIRVFGKPDGKPEFEEADTPEKL
RTLLAEKLSSRPEAVHEYVTPLFVSRAPNRKMSGQGHMETVKSAKRLDEGVSVLRVPLTQLK
LKDLEKMVNREREPKLYEALKARLEAHKDDPAKAFAEPFYKYDKAGNRTQQVKAVRVEQVQK
TGVWVRNHNGIADNATMVRVDVFEKGDKYYLVPIYSWQVAKGILPDRAVVQGKDEEDWQLID
DSFNFKFSLHPNDLVEVITKKARMFGYFASCHRGTGNINIRIHDLDHKIGKNGILEGIGVKT
ALSFQKYQIDELGKEIRPCRLKKRPPVR
SEQ ID NO: 362 
MQTTNLSYILGLDLGIASVGWAVVEINENEDPIGLIDVGVRIFERAEVPKTGESLALSRRLA
RSTRRLIRRRAHRLLLAKRFLKREGILSTIDLEKGLPNQAWELRVAGLERRLSAIEWGAVLL
HLIKHRGYLSKRKNESQTNNKELGALLSGVAQNHQLLQSDDYRTPAELALKKFAKEEGHIRN
QRGAYTHTFNRLDLLAELNLLFAQQHQFGNPHCKEHIQQYMTELLMWQKPALSGEAILKMLG
KCTHEKNEFKAAKHTYSAERFVWLTKLNNLRILEDGAERALNEEERQLLINHPYEKSKLTYA
QVRKLLGLSEQAIFKHLRYSKENAESATFMELKAWHAIRKALENQGLKDTWQDLAKKPDLLD
EIGTAFSLYKTDEDIQQYLTNKVPNSVINALLVSLNFDKFIELSLKSLRKILPLMEQGKRYD
QACREIYGHHYGEANQKTSQLLPAIPAQEIRNPVVLRTLSQARKVINAIIRQYGSPARVHIE
TGRELGKSFKERREIQKQQEDNRTKRESAVQKFKELFSDFSSEPKSKDILKFRLYEQQHGKC
LYSGKEINIHRLNEKGYVEIDHALPFSRTWDDSFNNKVLVLASENQNKGNQTPYEWLQGKIN
SERWKNFVALVLGSQCSAAKKQRLLTQVIDDNKFIDRNLNDTRYIARFLSNYIQENLLLVGK
NKKNVFTPNGQITALLRSRWGLIKARENNNRHHALDAIVVACATPSMQQKITRFIRFKEVHP
YKIENRYEMVDQESGEIISPHFPEPWAYFRQEVNIRVFDNHPDTVLKEMLPDRPQANHQFVQ
PLFVSRAPTRKMSGQGHMETIKSAKRLAEGISVLRIPLTQLKPNLLENMVNKEREPALYAGL
KARLAEFNQDPAKAFATPFYKQGGQQVKAIRVEQVQKSGVLVRENNGVADNASIVRTDVFIK
NNKFFLVPIYTWQVAKGILPNKAIVAHKNEDEWEEMDEGAKFKFSLFPNDLVELKTKKEYFF
GYYIGLDRATGNISLKEHDGEISKGKDGVYRVGVKLALSFEKYQVDELGKNRQICRPQQRQP
VR
SEO ID NO: 363 
MGIRFAFDLGTNSIGWAVWRTGPGVFGEDTAASLDGSGVLIFKDGRNPKDGQSLATMRRVPR
QSRKRRDRFVLRRRDLLAALRKAGLFPVDVEEGRRLAATDPYHLRAKALDESLTPHEMGRVI
FHLNQRRGFRSNRKADRQDREKGKIAEGSKRLAETLAATNCRTLGEFLWSRHRGTPRTRSPT
RIRMEGEGAKALYAFYPTREMVRAEFERLWTAQSRFAPDLLTPERHEEIAGILFRQRDLAPP
KIGCCTFEPSERRLPRALPSVEARGIYERLAHLRITTGPVSDRGLTRPERDVLASALLAGKS
LTFKAVRKTLKILPHALVNFEEAGEKGLDGALTAKLLSKPDHYGAAWHGLSFAEKDTFVGKL
LDEADEERLIRRLVTENRLSEDAARRCASIPLADGYGRLGRTANTEILAALVEETDETGTVV
TYAEAVRRAGERTGRNWHHSDERDGVILDRLPYYGEILQRHVVPGSGEPEEKNEAARWGRLA
NPTVHIGLNQLRKVVNRLIAAHGRPDQIVVELARELKLNREQKERLDRENRKNREENERRTA
ILAEHGQRDTAENKIRLRLFEEQARANAGIALCPYTGRAIGIAELFTSEVEIDHILPVSLTL
DDSLANRVLCRREANREKRRQTPFQAFGATPAWNDIVARAAKLPPNKRWRFDPAALERFERE
GGFLGRQLNETKYLSRLAKIYLGKICDPDRVYVTPGTLTGLLRARWGLNSILSDSNFKNRSD
HRHHAVDAVVIGVLTRGMIQRIAHDAARAEDQDLDRVFRDVPVPFEDFRDHVRERVSTITVA
VKPEHGKGGALHEDTSYGLVPDTDPNAALGNLVVRKPIRSLTAGEVDRVRDRALRARLGALA
APFRDESGRVRDAKGLAQALEAFGAENGIRRVRILKPDASVVTIADRRTGVPYRAVAPGENH
HVDIVQMRDGSWRGFAASVFEVNRPGWRPEWEVKKLGGKLVMRLHKGDMVELSDKDGQRRVK
VVQQIEISANRVRLSPHNDGGKLQDRHADADDPFRWDLATIPLLKDRGCVAVRVDPIGVVTL
RRSNV
SEQ ID NO: 364 
MMEVFMGRLVLGLDIGITSVGFGIIDLDESEIVDYGVRLFKEGTAAENETRRTKRGGRRLKR
RRVTRREDMLHLLKQAGIISTSFHPLNNPYDVRVKGLNERLNGEELATALLHLCKHRGSSVE
TIEDDEAKAKEAGETKKVLSMNDQLLKSGKYVCEIQKERLRTNGHIRGHENNFKTRAYVDEA
FQILSHQDLSNELKSAIITIISRKRMYYDGPGGPLSPTPYGRYTYFGQKEPIDLIEKMRGKC
SLFPNEPRAPKLAYSAELFNLLNDLNNLSIEGEKLTSEQKAMILKIVHEKGKITPKQLAKEV
GVSLEQIRGFRIDTKGSPLLSELTGYKMIREVLEKSNDEHLEDHVFYDEIAEILTKTKDIEG
RKKQISELSSDLNEESVHQLAGLTKFTAYHSLSFKALRLINEEMLKTELNQMQSITLFGLKQ
NNELSVKGMKNIQADDTAILSPVAKRAQRETFKVVNRLREIYGEFDSIVVEMAREKNSEEQR
KAIRERQKFFEMRNKQVADIIGDDRKINAKLREKLVLYQEQDGKTAYSLEPIDLKLLIDDPN
AYEVDHIIPISISLDDSITNKVLVTHRENQEKGNLTPISAFVKGRFTKGSLAQYKAYCLKLK
EKNIKTNKGYRKKVEQYLLNENDIYKYDIQKEFINRNLVDTSYASRVVLNTLTTYFKQNEIP
TKVFTVKGSLTNAFRRKINLKKDRDEDYGHHAIDALIIASMPKMRLLSTIFSRYKIEDIYDE
STGEVFSSGDDSMYYDDRYFAFIASLKAIKVRKFSHKIDTKPNRSVADETIYSTRVIDGKEK
VVKKYKDIYDPKFTALAEDILNNAYQEKYLMALHDPQTFDQIVKVVNYYFEEMSKSEKYFTK
DKKGRIKISGMNPLSLYRDEHGMLKKYSKKGDGPAITQMKYFDGVLGNHIDISAHYQVRDKK
VVLQQISPYRTDFYYSKENGYKFVTIRYKDVRWSEKKKKYVIDQQDYAMKKAEKKIDDTYEF
QFSMHRDELIGITKAEGEALIYPDETWHNFNFFFHAGETPEILKFTATNNDKSNKIEVKPIH
CYCKMRLMPTISKKIVRIDKYATDVVGNLYKVKKNTLKFEFD
SEQ ID NO: 365 
MKKILGVDLGITSFGYAILQETGKDLYRCLDNSVVMRNNPYDEKSGESSQSIRSTQKSMRRL
IEKRKKRIRCVAQTMERYGILDYSETMKINDPKNNPIKNRWQLRAVDAWKRPLSPQELFAIF
AHMAKHRGYKSIATEDLIYELELELGLNDPEKESEKKADERRQVYNALRHLEELRKKYGGET
IAQTIHRAVEAGDLRSYRNHDDYEKMIRREDIEEEIEKVLLRQAELGALGLPEEQVSELIDE
LKACITDQEMPTIDESLFGKCTFYKDELAAPAYSYLYDLYRLYKKLADLNIDGYEVTQEDRE
KVIEWVEKKIAQGKNLKKITHKDLRKILGLAPEQKIFGVEDERIVKGKKEPRTFVPFFFLAD
IAKFKELFASIQKHPDALQIFRELAEILQRSKTPQEALDRLRALMAGKGIDTDDRELLELFK
NKRSGTRELSHRYILEALPLFLEGYDEKEVQRILGFDDREDYSRYPKSLRHLHLREGNLFEK
EENPINNHAVKSLASWALGLIADLSWRYGPFDEIILETTRDALPEKIRKEIDKAMREREKAL
DKIIGKYKKEFPSIDKRLARKIQLWERQKGLDLYSGKVINLSQLLDGSADIEHIVPQSLGGL
STDYNTIVTLKSVNAAKGNRLPGDWLAGNPDYRERIGMLSEKGLIDWKKRKNLLAQSLDEIY
TENTHSKGIRATSYLEALVAQVLKRYYPFPDPELRKNGIGVRMIPGKVTSKTRSLLGIKSKS
RETNFHHAEDALILSTLTRGWQNRLHRMLRDNYGKSEAELKELWKKYMPHIEGLTLADYIDE
AFRRFMSKGEESLFYRDMFDTIRSISYWVDKKPLSASSHKETVYSSRHEVPTLRKNILEAFD
SLNVIKDRHKLTTEEFMKRYDKEIRQKLWLHRIGNTNDESYRAVEERATQIAQILTRYQLMD
AQNDKEIDEKFQQALKELITSPIEVTGKLLRKMRFVYDKLNAMQIDRGLVETDKNMLGIHIS
KGPNEKLIFRRMDVNNAHELQKERSGILCYLNEMLFIFNKKGLIHYGCLRSYLEKGQGSKYI
ALFNPRFPANPKAQPSKFTSDSKIKQVGIGSATGIIKAHLDLDGHVRSYEVFGTLPEGSIEW
FKEESGYGRVEDDPHH
SEQ ID NO: 366 
MRPIEPWILGLDIGTDSLGWAVFSCEEKGPPTAKELLGGGVRLFDSGRDAKDHTSRQAERGA
FRRARRQTRTWPWRRDRLIALFQAAGLTPPAAETRQIALALRREAVSRPLAPDALWAALLHL
AHHRGFRSNRIDKRERAAAKALAKAKPAKATAKATAPAKEADDEAGFWEGAEAALRQRMAAS
GAPTVGALLADDLDRGQPVRMRYNQSDRDGVVAPTRALIAEELAEIVARQSSAYPGLDWPAV
TRLVLDQRPLRSKGAGPCAFLPGEDRALRALPTVQDFIIRQTLANLRLPSTSADEPRPLTDE
EHAKALALLSTARFVEWPALRRALGLKRGVKFTAETERNGAKQAARGTAGNLTEAILAPLIP
GWSGWDLDRKDRVFSDLWAARQDRSALLALIGDPRGPTRVTEDETAEAVADAIQIVLPTGRA
SLSAKAARAIAQAMAPGIGYDEAVTLALGLHHSHRPRQERLARLPYYAALPDVGLDGDPVG
PPPAEDDGAAAEAYYGRIGNISVHIALNETRKIVNALLHRHGPILRLVMVETTRELKAGADE
 
RKRMIAEQAERERENAEIDVELRKSDRWMANARERRQRVRLARRQNNLCPYTSTPIGHADLL
GDAYDIDHVIPLARGGRDSLDNMVLCQSDANKTKGDKTPWEAFHDKPGWIAQRDDFLARLDP
QTAKALAWRFADDAGERVARKSAEDEDQGFLPRQLTDTGYIARVALRYLSLVTNEPNAVVAT
NGRLTGLLRLAWDITPGPAPRDLLPTPRDALRDDTAARRFLDGLTPPPLAKAVEGAVQARLA
ALGRSRVADAGLADALGLTLASLGGGGKNRADHRHHFIDAAMIAVTTRGLINQINQASGAGR
ILDLRKWPRTNFEPPYPTFRAEVMKQWDHIHPSIRPAHRDGGSLHAATVFGVRNRPDARVLV
QRKPVEKLFLDANAKPLPADKIAEIIDGFASPRMAKRFKALLARYQAAHPEVPPALAALAVA
RDPAFGPRGMTANTVIAGRSDGDGEDAGLITPFRANPKAAVRTMGNAVYEVWEIQVKGRPRW
THRVLTRFDRTQPAPPPPPENARLVMRLRRGDLVYWPLESGDRLFLVKKMAVDGRLALWPAR
LATGKATALYAQLSCPNINLNGDQGYCVQSAEGIRKEKIRTTSCTALGRLRLSKKAT
SEQ ID NO: 367 
MKYTLGLDVGIASVGWAVIDKDNNKIIDLGVRCFDKAEESKTGESLATARRIARGMRRRISR
RSQRLRLVKKLFVQYEIIKDSSEFNRIFDTSRDGWKDPWELRYNALSRILKPYELVQVLTHI
TKRRGFKSNRKEDLSTTKEGVVITSIKNNSEMLRTKNYRTIGEMIFMETPENSNKRNKVDEY
IHTIAREDLLNEIKYIFSIQRKLGSPFVTEKLEHDFLNIWEFQRPFASGDSILSKVGKCTLL
KEELRAPTSCYTSEYFGLLQSINNLVLVEDNNTLTLNNDQRAKIIEYAHFKNEIKYSEIRKL
LDIEPEILFKAHNLTHKNPSGNNESKKFYEMKSYHKLKSTLPTDIWGKLHSNKESLDNLFYC
LTVYKNDNEIKDYLQANNLDYLIEYIAKLPTFNKFKHLSLVAMKRIIPFMEKGYKYSDACNM
AELDFTGSSKLEKCNKLTVEPIIENVTNPVVIRALTQARKVINAIIQKYGLPYMVNIELARE
AGMTRQDRDNLKKEHENNRKAREKISDLIRQNGRVASGLDILKWRLWEDQGGRCAYSGKPIP
VCDLLNDSLTQIDHIYPYSRSMDDSYMNKVLVLTDENQNKRSYTPYEVWGSTEKWEDFEARI
YSMHLPQSKEKRLLNRNFITKDLDSFISRNLNDTRYISRFLKNYIESYLQFSNDSPKSCVVC
VNGQCTAQLRSRWGLNKNREESDLHHALDAAVIACADRKIIKEITNYYNERENHNYKVKYPL
PWHSFRQDLMETLAGVFISRAPRRKITGPAHDETIRSPKHFNKGLTSVKIPLTTVTLEKLET
MVKNTKGGISDKAVYNVLKNRLIEHNNKPLKAFAEKIYKPLKNGTNGAIIRSIRVETPSYTG
VFRNEGKGISDNSLMVRVDVFKKKDKYYLVPIYVAHMIKKELPSKAIVPLKPESQWELIDST
HEFLFSLYQNDYLVIKTKKGITEGYYRSCHRGTGSLSLMPHFANNKNVKIDIGVRTAISIEK
YNVDILGNKSIVKGEPRRGMEKYNSFKSN
SEQ ID NO: 368 
MIRTLGIDIGIASIGWAVIEGEYTDKGLENKEIVASGVRVFTKAENPKNKESLALPRTLARS
ARRRNARKKGRIQQVKHYLSKALGLDLECFVQGEKLATLFQTSKDFLSPWELRERALYRVLD
KEELARVILHIAKRRGYDDITYGVEDNDSGKIKKAIAENSKRIKEEQCKTIGEMMYKLYFQK
SLNVRNKKESYNRCVGRSELREELKTIFQIQQELKSPWVNEELIYKLLGNPDAQSKQEREGL
IFYQRPLKGFGDKIGKCSHIKKGENSPYRACKHAPSAEEFVALTKSINFLKNLTNRHGLCFS
QEDMCVYLGKILQEAQKNEKGLTYSKLKLLLDLPSDFEFLGLDYSGKNPEKAVFLSLPSTFK
LNKITQDRKTQDKIANILGANKDWEAILKELESLQLSKEQIQTIKDAKLNFSKHINLSLEAL
YHLLPLMREGKRYDEGVEILQERGIFSKPQPKNRQLLPPLSELAKEESYFDIPNPVLRRALS
EFRKVVNALLEKYGGFHYFHIELTRDVCKAKSARMQLEKINKKNKSENDAASQLLEVLGLPN
TYNNRLKCKLWKQQEEYCLYSGEKITIDHLKDQRALQIDHAFPLSRSLDDSQSNKVLCLTSS
NQEKSNKTPYEWLGSDEKKWDMYVGRVYSSNFSPSKKRKLTQKNFKERNEEDFLARNLVDTG
YIGRVTKEYIKHSLSFLPLPDGKKEHIRIISGSMTSTMRSFWGVQEKNRDHHLHHAQDAIII
ACIEPSMIQKYTTYLKDKETHRLKSHQKAQILREGDHKLSLRWPMSNFKDKIQESIQNIIPS
HHVSHKVTGELHQETVRTKEFYYQAFGGEEGVKKALKFGKIREINQGIVDNGAMVRVDIFKS
KDKGKFYAVPIYTYDFAIGKLPNKAIVQGKKNGIIKDWLEMDENYEFCFSLFKNDCIKIQTK
EMQEAVLAIYKSTNSAKATIELEHLSKYALKNEDEEKMFTDTDKEKNKTMTRESCGIQGLKV
FQKVKLSVLGEVLEHKPRNRQNIALKTTPKHV
SEQ ID NO: 369 
MKYSIGLDIGIASVGWSVINKDKERIEDMGVRIFQKAENPKDGSSLASSRREKRGSRRRNRR
KKHRLDRIKNILCESGLVKKNEIEKIYKNAYLKSPWELRAKSLEAKISNKEIAQILLHIAKR
RGFKSFRKTDRNADDTGKLLSGIQENKKIMEEKGYLTIGDMVAKDPKFNTHVRNKAGSYLFS
FSRKLLEDEVRKIQAKQKELGNTHFTDDVLEKYIEVFNSQRNFDEGPSKPSPYYSEIGQIAK
MIGNCTFESSEKRTAKNTWSGERFVFLQKLNNFRIVGLSGKRPLTEEERDIVEKEVYLKKEV
RYEKLRKILYLKEEERFGDLNYSKDEKQDKKTEKTKFISLIGNYTIKKLNLSEKLKSEIEED
KSKLDKIIEILTFNKSDKTIESNLKKLELSREDIEILLSEEFSGTLNLSLKAIKKILPYLEK
GLSYNEACEKADYDYKNNGIKFKRGELLPVVDKDLIANPVVLRAISQTRKVVNAIIRKYGTP
HTIHVEVARDLAKSYDDRQTIIKENKKRELENEKTKKFISEEFGIKNVKGKLLLKYRLYQEQ
EGRCAYSRKELSLSEVILDESMTDIDHIIPYSRSMDDSYSNKVLVLSGENRKKSNLLPKEYF
DRQGRDWDTFVLNVKAMKIHPRKKSNLLKEKFTREDNKDWKSRALNDTRYISRFVANYLENA
LEYRDDSPKKRVFMIPGQLTAQLRARWRLNKVRENGDLHHALDAAVVAVTDQKAINNISNIS
RYKELKNCKDVIPSIEYHADEETGEVYFEEVKDTRFPMPWSGFDLELQKRLESENPREEFYN
LLSDKRYLGWFNYEEGFIEKLRPVFVSRMPNRGVKGQAHQETIRSSKKISNQIAVSKKPLNS
IKLKDLEKMQGRDTDRKLYEALKNRLEEYDDKPEKAFAEPFYKPTNSGKRGPLVRGIKVEEK
QNVGVYVNGGQASNGSMVRIDVFRKNGKFYTVPIYVHQTLLKELPNRAINGKPYKDWDLIDG
SFEFLYSFYPNDLIEIEFGKSKSIKNDNKLTKTEIPEVNLSEVLGYYRGMDTSTGAATIDTQ
DGKIQMRIGIKTVKNIKKYQVDVLGNVYKVKREKRQTF
SEQ ID NO: 370 
MSKKVSRRYEEQAQEICQRLGSRPYSIGLDLGVGSIGVAVAAYDPIKKQPSDLVFVSSRIFI
PSTGAAERRQKRGQRNSLRHRANRLKFLWKLLAERNLMLSYSEQDVPDPARLRFEDAVVRAN
PYELRLKGLNEQLTLSELGYALYHIANHRGSSSVRTFLDEEKSSDDKKLEEQQAMTEQLAKE
KGISTFIEVLTAFNTNGLIGYRNSESVKSKGVPVPTRDIISNEIDVLLQTQKQFYQEILSDE
YCDRIVSAILFENEKIVPEAGCCPYFPDEKKLPRCHFLNEERRLWEAINNARIKMPMQEGAA
KRYQSASFSDEQRHILFHIARSGTDITPKLVQKEFPALKTSIIVLQGKEKAIQKIAGFRFRR
LEEKSFWKRLSEEQKDDFFSAWTNTPDDKRLSKYLMKHLLLTENEVVDALKTVSLIGDYGPI
GKTATQLLMKHLEDGLTYTEALERGMETGEFQELSVWEQQSLLPYYGQILTGSTQALMGKYW
HSAFKEKRDSEGFFKPNTNSDEEKYGRIANPVVHQTLNELRKLMNELITILGAKPQEITVEL
ARELKVGAEKREDIIKQQTKQEKEAVLAYSKYCEPNNLDKRYIERFRLLEDQAFVCPYCLEH
ISVADIAAGRADVDHIFPRDDTADNSYGNKVVAHRQCNDIKGKRTPYAAFSNTSAWGPIMHY
LDETPGMWRKRRKFETNEEEYAKYLQSKGFVSRFESDNSYIAKAAKEYLRCLFNPNNVTAVG
SLKGMETSILRKAWNLQGIDDLLGSRHWSKDADTSPTMRKNRDDNRHHGLDAIVALYCSRSL
VQMINTMSEQGKRAVEIEAMIPIPGYASEPNLSFEAQRELFRKKILEFMDLHAFVSMKTDND
ANGALLKDTVYSILGADTQGEDLVFVVKKKIKDIGVKIGDYEEVASAIRGRITDKQPKWYPM
EMKDKIEQLQSKNEAALQKYKESLVQAAAVLEESNRKLIESGKKPIQLSEKTISKKALELVG
GYYYLISNNKRTKTFVVKEPSNEVKGFAFDTGSNLCLDFYHDAQGKLCGEIIRKIQAMNPSY
KPAYMKQGYSLYVRLYQGDVCELRASDLTEAESNLAKTTHVRLPNAKPGRTFVIIITFTEMG
SGYQIYFSNLAKSKKGQDTSFTLTTIKNYDVRKVQLSSAGLVRYVSPLLVDKIEKDEVALCG
E
SEQ ID NO: 371 
MNQKFILGLDIGITSVGYGLIDYETKNIIDAGVRLFPEANVENNEGRRSKRGSRRLKRRRIH
RLERVKKLLEDYNLLDQSQIPQSTNPYAIRVKGLSEALSKDELVIALLHIAKRRGIHKIDVI
DSNDDVGNELSTKEQLNKNSKLLKDKFVCQIQLERMNEGQVRGEKNRFKTADIIKEIIQLLN
VQKNFHQLDENFINKYIELVEMRREYFEGPGKGSPYGWEGDPKAWYETLMGHCTYFPDELRS
VKYAYSADLFNALNDLNNLVIQRDGLSKLEYHEKYHIIENVFKQKKKPTLKQIANEINVNPE
DIKGYRITKSGKPQFTEFKLYHDLKSVLFDQSILENEDVLDQIAEILTIYQDKDSIKSKLTE
LDILLNEEDKENIAQLTGYTGTHRLSLKCIRLVLEEQWYSSRNQMEIFTHLNIKPKKINLTA
ANKIPKAMIDEFILSPVVKRTFGQAINLINKIIEKYGVPEDIIIELARENNSKDKQKFINEM
QKKNENTRKRINEIIGKYGNQNAKRLVEKIRLHDEQEGKCLYSLESIPLEDLLNNPNHYEVD
 
HIIPRSVSFDNSYHNKVLVKQSENSKKSNLTPYQYFNSGKSKLSYNQFKQHILNLSKSQDRI
SKKKKEYLLEERDINKFEVQKEFINRNLVDTRYATRELTNYLKAYFSANNMNVKVKTINGSF
TDYLRKVWKFKKERNHGYKHHAEDALIIANADFLFKENKKLKAVNSVLEKPEIESKQLDIQV
DSEDNYSEMFIIPKQVQDIKDFRNFKYSHRVDKKPNRQLINDTLYSTRKKDNSTYIVQTIKD
IYAKDNTTLKKQFDKSPEKFLMYQHDPRTFEKLEVIMKQYANEKNPLAKYHEETGEYLTKYS
KKNNGPIVKSLKYIGNKLGSHLDVTHQFKSSTKKLVKLSIKPYRFDVYLTDKGYKFITISYL
DVLKKDNYYYIPEQKYDKLKLGKAIDKNAKFIASFYKNDLIKLDGEIYKIIGVNSDTRNMIE
LDLPDIRYKEYCELNNIKGEPRIKKTIGKKVNSIEKLTTDVLGNVFTNTQYTKPQLLFKRGN
SEQ ID NO: 372 
MIMKLEKWRLGLDLGTNSIGWSVFSLDKDNSVQDLIDMGVRIFSDGRDPKTKEPLAVARRTA
RSQRKLIYRRKLRRKQVFKFLQEQGLFPKTKEECMTLKSLNPYELRIKALDEKLEPYELGRA
LFNLAVRRGFKSNRKDGSREEVSEKKSPDEIKTQADMQTHLEKAIKENGCRTITEFLYKNQG
ENGGIRFAPGRMTYYPTRKMYEEEFNLIRSKQEKYYPQVDWDDIYKAIFYQRPLKPQQRGYC
IYENDKERTFKAMPCSQKLRILQDIGNLAYYEGGSKKRVELNDNQDKVLYELLNSKDKVTFD
QMRKALCLADSNSFNLEENRDFLIGNPTAVKMRSKNRFGKLWDEIPLEEQDLIIETIITADE
DDAVYEVIKKYDLTQEQRDFIVKNTILQSGTSMLCKEVSEKLVKRLEEIADLKYHEAVESLG
YKFADQTVEKYDLLPYYGKVLPGSTMEIDLSAPETNPEKHYGKISNPTVHVALNQTRVVVNA
LIKEYGKPSQIAIELSRDLKNNVEKKAEIARKQNQRAKENIAINDTISALYHTAFPGKSFYP
NRNDRMKYRLWSELGLGNKCIYCGKGISGAELFTKEIEIEHILPFSRTLLDAESNLTVAHSS
CNAFKAERSPFEAFGTNPSGYSWQEIIQRANQLKNTSKKNKFSPNAMDSFEKDSSFIARQLS
DNQYIAKAALRYLKCLVENPSDVWTTNGSMTKLLRDKWEMDSILCRKFTEKEVALLGLKPEQ
IGNYKKNRFDHRHHAIDAVVIGLTDRSMVQKLATKNSHKGNRIEIPEFPILRSDLIEKVKNI
VVSFKPDHGAEGKLSKETLLGKIKLHGKETFVCRENIVSLSEKNLDDIVDEIKSKVKDYVAK
HKGQKIEAVLSDFSKENGIKKVRCVNRVQTPIEITSGKISRYLSPEDYFAAVIWEIPGEKKT
FKAQYIRRNEVEKNSKGLNVVKPAVLENGKPHPAAKQVCLLHKDDYLEFSDKGKMYFCRIAG
YAATNNKLDIRPVYAVSYCADWINSTNETMLTGYWKPTPTQNWVSVNVLFDKQKARLVTVSP
IGRVFRK
SEQ ID NO: 373 
MSSKAIDSLEQLDLFKPQEYTLGLDLGIKSIGWAILSGERIANAGVYLFETAEELNSTGNKL
ISKAAERGRKRRIRRMLDRKARRGRHIRYLLEREGLPTDELEEVVVHQSNRTLWDVRAEAVE
RKLTKQELAAVLFHLVRHRGYFPNTKKLPPDDESDSADEEQGKINRATSRLREELKASDCKT
IGQFLAQNRDRQRNREGDYSNLMARKLVFEEALQILAFQRKQGHELSKDFEKTYLDVLMGQR
SGRSPKLGNCSLIPSELRAPSSAPSTEWFKFLQNLGNLQISNAYREEWSIDAPRRAQIIDAC
SQRSTSSYWQIRRDFQIPDEYRFNLVNYERRDPDVDLQEYLQQQERKTLANFRNWKQLEKII
GTGHPIQTLDEAARLITLIKDDEKLSDQLADLLPEASDKAITQLCELDFTTAAKISLEAMYR
ILPHMNQGMGFFDACQQESLPEIGVPPAGDRVPPFDEMYNPVVNRVLSQSRKLINAVIDEYG
MPAKIRVELARDLGKGRELRERIKLDQLDKSKQNDQRAEDFRAEFQQAPRGDQSLRYRLWKE
QNCTCPYSGRMIPVNSVLSEDTQIDHILPISQSFDNSLSNKVLCFTEENAQKSNRTPFEYLD
AADFQRLEAISGNWPEAKRNKLLHKSFGKVAEEWKSRALNDTRYLTSALADHLRHHLPDSKI
QTVNGRITGYLRKQWGLEKDRDKHTHHAVDAIVVACTTPAIVQQVTLYHQDIRRYKKLGEKR
PTPWPETFRQDVLDVEEEIFITRQPKKVSGGIQTKDTLRKHRSKPDRQRVALTKVKLADLER
LVEKDASNRNLYEHLKQCLEESGDQPTKAFKAPFYMPSGPEAKQRPILSKVTLLREKPEPPK
QLTELSGGRRYDSMAQGRLDIYRYKPGGKRKDEYRVVLQRMIDLMRGEENVHVFQKGVPYDQ
GPEIEQNYTFLFSLYFDDLVEFQRSADSEVIRGYYRTFNIANGQLKISTYLEGRQDFDFFGA
NRLAHFAKVQVNLLGKVIK
SEQ ID NO: 374 
MRSLRYRLALDLGSTSLGWALFRLDACNRPTAVIKAGVRIFSDGRNPKDGSSLAVTRRAARA
MRRRRDRLLKRKTRMQAKLVEHGFFPADAGKRKALEQLNPYALRAKGLQEALLPGEFARALF
 
HINQRRGFKSNRKTDKKDNDSGVLKKAIGQLRQQMAEQGSRTVGEYLWTRLQQGQGVRARYR
EKPYTTEEGKKRIDKSYDLYIDRAMIEQEFDALWAAQAAFNPTLFHEAARADLKDTLLHQRP
LRPVKPGRCTLLPEEERAPLALPSTQRFRIHQEVNHLRLLDENLREVALTLAQRDAVVTALE
TKAKLSFEQIRKLLKLSGSVQFNLEDAKRTELKGNATSAALARKELFGAAWSGFDEALQDEI
VWQLVTEEGEGALIAWLQTHTGVDEARAQAIVDVSLPEGYGNLSRKALARIVPALRAAVITY
DKAVQAAGFDHHSQLGFEYDASEVEDLVHPETGEIRSVFKQLPYYGKALQRHVAFGSGKPED
PDEKRYGKIANPTVHIGLNQVRMVVNALIRRYGRPTEVVIELARDLKQSREQKVEAQRRQAD
NQRRNARIRRSIAEVLGIGEERVRGSDIQKWICWEELSFDAADRRCPYSGVQISAAMLLSDE
VEVEHILPFSKTLDDSLNNRTVAMRQANRIKRNRTPWDARAEFEAQGWSYEDILQRAERMPL
RKRYRFAPDGYERWLGDDKDFLARALNDTRYLSRVAAEYLRLVCPGTRVIPGQLTALLRGKF
GLNDVLGLDGEKNRNDHRHHAVDACVIGVTDQGLMQRFATASAQARGDGLTRLVDGMPMPWP
TYRDHVERAVRHIWVSHRPDHGFEGAMMEETSYGIRKDGSIKQRRKADGSAGREISNLIRIH
EATQPLRHGVSADGQPLAYKGYVGGSNYCIEITVNDKGKWEGEVISTFRAYGVVRAGGMGRL
RNPHEGQNGRKLIMRLVIGDSVRLEVDGAERTMRIVKISGSNGQIFMAPIHEANVDARNTDK
QDAFTYTSKYAGSLQKAKTRRVTISPIGEVRDPGFKG
SEQ ID NO: 375 
MARPAFRAPRREHVNGWTPDPHRISKPFFILVSWHLLSRVVIDSSSGCFPGTSRDHTDKFAE
WECAVQPYRLSFDLGTNSIGWGLLNLDRQGKPREIRALGSRIFSDGRDPQDKASLAVARRLA
RQMRRRRDRYLTRRTRLMGALVRFGLMPADPAARKRLEVAVDPYLARERATRERLEPFEIGR
ALFHLNQRRGYKPVRTATKPDEEAGKVKEAVERLEAAIAAAGAPTLGAWFAWRKTRGETLRA
RLAGKGKEAAYPFYPARRMLEAEFDTLWAEQARHHPDLLTAEAREILRHRIFHQRPLKPPPV
GRCTLYPDDGRAPRALPSAQRLRLFQELASLRVIHLDLSERPLTPAERDRIVAFVQGRPPKA
GRKPGKVQKSVPFEKLRGLLELPPGTGFSLESDKRPELLGDETGARIAPAFGPGWTALPLEE
QDALVELLLTEAEPERAIAALTARWALDEATAAKLAGATLPDFHGRYGRRAVAELLPVLERE
TRGDPDGRVRPIRLDEAVKLLRGGKDHSDFSREGALLDALPYYGAVLERHVAFGTGNPADPE
EKRVGRVANPTVHIALNQLRHLVNAILARHGRPEEIVIELARDLKRSAEDRRREDKRQADNQ
KRNEERKRLILSLGERPTPRNLLKLRLWEEQGPVENRRCPYSGETISMRMLLSEQVDIDHIL
PFSVSLDDSAANKVVCLREANRIKRNRSPWEAFGHDSERWAGILARAEALPKNKRWRFAPDA
LEKLEGEGGLRARHLNDTRHLSRLAVEYLRCVCPKVRVSPGRLTALLRRRWGIDAILAEADG
PPPEVPAETLDPSPAEKNRADHRHHALDAVVIGCIDRSMVQRVQLAAASAEREAAAREDNIR
RVLEGFKEEPWDGFRAELERRARTIVVSHRPEHGIGGALHKETAYGPVDPPEEGFNLVVRKP
IDGLSKDEINSVRDPRLRRALIDRLAIRRRDANDPATALAKAAEDLAAQPASRGIRRVRVLK
KESNPIRVEHGGNPSGPRSGGPFHKLLLAGEVHHVDVALRADGRRWVGHWVTLFEAHGGRGA
DGAAAPPRLGDGERFLMRLHKGDCLKLEHKGRVRVMQVVKLEPSSNSVVVVEPHQVKTDRSK
HVKISCDQLRARGARRVTVDPLGRVRVHAPGARVGIGGDAGRTAMEPAEDIS
SEQ ID NO: 376 
MKRTSLRAYRLGVDLGANSLGWFVVWLDDHGQPEGLGPGGVRIFPDGRNPQSKQSNAAGRRL
ARSARRRRDRYLQRRGKLMGLLVKHGLMPADEPARKRLECLDPYGLRAKALDEVLPLHHVGR
ALFHLNQRRGLFANRAIEQGDKDASAIKAAAGRLQTSMQACGARTLGEFLNRRHQLRATVRA
RSPVGGDVQARYEFYPTRAMVDAEFEAIWAAQAPHHPTMTAEAHDTIREAIFSQRAMKRPSI
GKCSLDPATSQDDVDGFRCAWSHPLAQRFRIWQDVRNLAVVETGPTSSRLGKEDQDKVARAL
LQTDQLSFDEIRGLLGLPSDARFNLESDRRDHLKGDATGAILSARRHFGPAWHDRSLDRQID
IVALLESALDEAAIIASLGTTHSLDEAAAQRALSALLPDGYCRLGLRAIKRVLPLMEAGRTY
AEAASAAGYDHALLPGGKLSPTGYLPYYGQWLQNDVVGSDDERDTNERRWGRLPNPTVHIGI
GQLRRVVNELIRWHGPPAEITVELTRDLKLSPRRLAELEREQAENQRKNDKRTSLLRKLGLP
ASTHNLLKLRLWDEQGDVASECPYTGEAIGLERLVSDDVDIDHLIPFSISWDDSAANKVVCM
RYANREKGNRTPFEAFGHRQGRPYDWADIAERAARLPRGKRWRFGPGARAQFEELGDFQARL
LNETSWLARVAKQYLAAVTHPHRIHVLPGRLTALLRATWELNDLLPGSDDRAAKSRKDHRHH
AIDALVAALTDQALLRRMANAHDDTRRKIEVLLPWPTFRIDLETRLKAMLVSHKPDHGLQAR
 
LHEDTAYGTVEHPETEDGANLVYRKTFVDISEKEIDRIRDRRLRDLVRAHVAGERQQGKTLK
AAVLSFAQRRDIAGHPNGIRHVRLTKSIKPDYLVPIRDKAGRIYKSYNAGENAFVDILQAES
GRWIARATTVFQANQANESHDAPAAQPIMRVFKGDMLRIDHAGAEKFVKIVRLSPSNNLLYL
VEHHQAGVFQTRHDDPEDSFRWLFASFDKLREWNAELVRIDTLGQPWRRKRGLETGSEDATR
IGWTRPKKWP
SEQ ID NO: 377 
MERIFGFDIGTTSIGFSVIDYSSTQSAGNIQRLGVRIFPEARDPDGTPLNQQRRQKRMMRRQ
LRRRRIRRKALNETLHEAGFLPAYGSADWPVVMADEPYELRRRGLEEGLSAYEFGRAIYHLA
QHRHFKGRELEESDTPDPDVDDEKEAANERAATLKALKNEQTTLGAWLARRPPSDRKRGIHA
HRNVVAEEFERLWEVQSKFHPALKSEEMRARISDTIFAQRPVFWRKNTLGECRFMPGEPLCP
KGSWLSQQRRMLEKLNNLAIAGGNARPLDAEERDAILSKLQQQASMSWPGVRSALKALYKQR
GEPGAEKSLKFNLELGGESKLLGNALEAKLADMFGPDWPAHPRKQEIRHAVHERLWAADYGE
TPDKKRVIILSEKDRKAHREAAANSFVADFGITGEQAAQLQALKLPTGWEPYSIPALNLFLA
ELEKGERFGALVNGPDWEGWRRTNFPHRNQPTGEILDKLPSPASKEERERISQLRNPTVVRT
QNELRKVVNNLIGLYGKPDRIRIEVGRDVGKSKREREEIQSGIRRNEKQRKKATEDLIKNGI
ANPSRDDVEKWILWKEGQERCPYTGDQIGFNALFREGRYEVEHIWPRSRSFDNSPRNKTLCR
KDVNIEKGNRMPFEAFGHDEDRWSAIQIRLQGMVSAKGGTGMSPGKVKRFLAKTMPEDFAAR
QLNDTRYAAKQILAQLKRLWPDMGPEAPVKVEAVTGQVTAQLRKLWTLNNILADDGEKTRAD
HRHHAIDALTVACTHPGMTNKLSRYWQLRDDPRAEKPALTPPWDTIRADAEKAVSEIVVSHR
VRKKVSGPLHKETTYGDTGTDIKTKSGTYRQFVTRKKIESLSKGELDEIRDPRIKEIVAAHV
AGRGGDPKKAFPPYPCVSPGGPEIRKVRLTSKQQLNLMAQTGNGYADLGSNHHIAIYRLPDG
KADFEIVSLFDASRRLAQRNPIVQRTRADGASFVMSLAAGEAIMIPEGSKKGIWIVQGVWAS
GQVVLERDTDADHSTTTRPMPNPILKDDAKKVSIDPIGRVRPSND
SEQ ID NO: 378 
MNKRILGLDTGTNSLGWAVVDWDEHAQSYELIKYGDVIFQEGVKIEKGIESSKAAERSGYKA
IRKQYFRRRLRKIQVLKVLVKYHLCPYLSDDDLRQWHLQKQYPKSDELMLWQRTSDEEGKNP
YYDRHRCLHEKLDLTVEADRYTLGRALYHLTQRRGFLSNRLDTSADNKEDGVVKSGISQLST
EMEEAGCEYLGDYFYKLYDAQGNKVRIRQRYTDRNKHYQHEFDAICEKQELSSELIEDLQRA
IFFQLPLKSQRHGVGRCTFERGKPRCADSHPDYEEFRMLCFVNNIQVKGPHDLELRPLTYEE
REKIEPLFFRKSKPNFDFEDIAKALAGKKNYAWIHDKEERAYKFNYRMTQGVPGCPTIAQLK
SIFGDDWKTGIAETYTLIQKKNGSKSLQEMVDDVWNVLYSFSSVEKLKEFAHHKLQLDEESA
EKFAKIKLSHSFAALSLKAIRKFLPFLRKGMYYTHASFFANIPTIVGKEIWNKEQNRKYIME
NVGELVFNYQPKHREVQGTIEMLIKDFLANNFELPAGATDKLYHPSMIETYPNAQRNEFGIL
QLGSPRTNAIRNPMAMRSLHILRRVVNQLLKESIIDENTEVHVEYARELNDANKRRAIADRQ
KEQDKQHKKYGDEIRKLYKEETGKDIEPTQTDVLKFQLWEEQNHHCLYTGEQIGITDFIGSN
PKFDIEHTIPQSVGGDSTQMNLTLCDNRFNREVKKAKLPTELANHEEILTRIEPWKNKYEQL
VKERDKQRTFAGMDKAVKDIRIQKRHKLQMEIDYWRGKYERFTMTEVPEGFSRRQGTGIGLI
SRYAGLYLKSLFHQADSRNKSNVYVVKGVATAEFRKMWGLQSEYEKKCRDNHSHHCMDAITI
ACIGKREYDLMAEYYRMEETFKQGRGSKPKFSKPWATFTEDVLNIYKNLLVVHDTPNNMPKH
TKKYVQTSIGKVLAQGDTARGSLHLDTYYGAIERDGEIRYVVRRPLSSFTKPEELENIVDET
VKRTIKEAIADKNFKQAIAEPIYMNEEKGILIKKVRCFAKSVKQPINIRQHRDLSKKEYKQQ
YHVMNENNYLLAIYEGLVKNKVVREFEIVSYIEAAKYYKRSQDRNIFSSIVPTHSTKYGLPL
KTKLLMGQLVLMFEENPDEIQVDNTKDLVKRLYKVVGIEKDGRIKFKYHQEARKEGLPIFST
PYKNNDDYAPIFRQSINNINILVDGIDFTIDILGKVTLKE
SEQ ID NO: 379 
MNYKMGLDIGIASVGWAVINLDLKRIEDLGVRIFDKAEHPQNGESLALPRRIARSARRRLRR
RKHRLERIRRLLVSENVLTKEEMNLLFKQKKQIDVWQLRVDALERKLNNDELARVLLHLAKR
RGFKSNRKSERNSKESSEFLKNIEENQSILAQYRSVGEMIVKDSKFAYHKRNKLDSYSNMIA
 
RDDLEREIKLIFEKQREFNNPVCTERLEEKYLNIWSSQRPFASKEDIEKKVGFCTFEPKEKR
APKATYTFQSFIVWEHINKLRLVSPDETRALTEIERNLLYKQAFSKNKMTYYDIRKLLNLSD
DIHFKGLLYDPKSSLKQIENIRFLELDSYHKIRKCIENVYGKDGIRMFNETDIDTFGYALTI
FKDDEDIVAYLQNEYITKNGKRVSNLANKVYDKSLIDELLNLSFSKFAHLSMKAIRNILPYM
EQGEIYSKACELAGYNFTGPKKKEKALLLPVIPNIANPVVMRALTQSRKVVNAIIKKYGSPV
SIHIELARDLSHSFDERKKIQKDQTENRKKNETAIKQLIEYELTKNPTGLDIVKFKLWSEQQ
GRCMYSLKPIELERLLEPGYVEVDHILPYSRSLDDSYANKVLVLTKENREKGNHTPVEYLGL
GSERWKKFEKFVLANKQFSKKKKQNLLRLRYEETEEKEFKERNLNDTRYISKFFANFIKEHL
KFADGDGGQKVYTINGKITAHLRSRWDFNKNREESDLHHAVDAVIVACATQGMIKKITEFYK
AREQNKESAKKKEPIFPQPWPHFADELKARLSKFPQESIEAFALGNYDRKKLESLRPVFVSR
MPKRSVTGAAHQETLRRCVGIDEQSGKIQTAVKTKLSDIKLDKDGHFPMYQKESDPRTYEAI
RQRLLEHNNDPKKAFQEPLYKPKKNGEPGPVIRTVKIIDTKNKVVHLDGSKTVAYNSNIVRT
DVFEKDGKYYCVPVYTMDIMKGTLPNKAIEANKPYSEWKEMTEEYTFQFSLFPNDLVRIVLP
REKTIKTSTNEEIIIKDIFAYYKTIDSATGGLELISHDRNFSLRGVGSKTLKRFEKYQVDVL
GNIHKVKGEKRVGLAAPTNQKKGKTVDSLQSVSD
SEQ ID NO: 380 
MRRLGLDLGTNSIGWCLLDLGDDGEPVSIFRTGARIFSDGRDPKSLGSLKATRREARLTRRR
RDRFIQRQKNLINALVKYGLMPADEIQRQALAYKDPYPIRKKALDEAIDPYEMGRAIFHINQ
RRGFKSNRKSADNEAGVVKQSIADLEMKLGEAGARTIGEFLADRQATNDTVRARRLSGTNAL
YEFYPDRYMLEQEFDTLWAKQAAFNPSLYIEAARERLKEIVFFQRKLKPQEVGRCIFLSDED
RISKALPSFQRFRIYQELSNLAWIDHDGVAHRITASLALRDHLFDELEHKKKLTFKAMRAIL
RKQGVVDYPVGFNLESDNRDHLIGNLTSCIMRDAKKMIGSAWDRLDEEEQDSFILMLQDDQK
GDDEVRSILTQQYGLSDDVAEDCLDVRLPDGHGSLSKKAIDRILPVLRDQGLIYYDAVKEAG
LGEANLYDPYAALSDKLDYYGKALAGHVMGASGKFEDSDEKRYGTISNPTVHIALNQVRAVV
NELIRLHGKPDEVVIEIGRDLPMGADGKRELERFQKEGRAKNERARDELKKLGHIDSRESRQ
KFQLWEQLAKEPVDRCCPFTGKMMSISDLFSDKVEIEHLLPFSLTLDDSMANKTVCFRQANR
DKGNRAPFDAFGNSPAGYDWQEILGRSQNLPYAKRWRFLPDAMKRFEADGGFLERQLNDTRY
ISRYTTEYISTIIPKNKIWVVTGRLTSLLRGFWGLNSILRGHNTDDGTPAKKSRDDHRHHAI
DAIVVGMTSRGLLQKVSKAARRSEDLDLTRLFEGRIDPWDGFRDEVKKHIDAIIVSHRPRKK
SQGALHNDTAYGIVEHAENGASTVVHRVPITSLGKQSDIEKVRDPLIKSALLNETAGLSGKS
FENAVQKWCADNSIKSLRIVETVSIIPITDKEGVAYKGYKGDGNAYMDIYQDPTSSKWKGEI
VSRFDANQKGFIPSWQSQFPTARLIMRLRINDLLKLQDGEIEEIYRVQRLSGSKILMAPHTE
ANVDARDRDKNDTFKLTSKSPGKLQSASARKVHISPTGLIREG
SEQ ID NO: 381 
MKNILGLDLGLSSIGWSVIRENSEEQELVAMGSRVVSLTAAELSSFTQGNGVSINSQRTQKR
TQRKGYDRYQLRRTLLRNKLDTLGMLPDDSLSYLPKLQLWGLRAKAVTQRIELNELGRVLLH
LNQKRGYKSIKSDFSGDKKITDYVKTVKTRYDELKEMRLTIGELFFRRLTENAFFRCKEQVY
PRQAYVEEFDCIMNCQRKFYPDILTDETIRCIRDEIIYYQRPLKSCKYLVSRCEFEKRFYLN
AAGKKTEAGPKVSPRTSPLFQVCRLWESINNIVVKDRRNEIVFISAEQRAALFDFLNTHEKL
KGSDLLKLLGLSKTYGYRLGEQFKTGIQGNKTRVEIERALGNYPDKKRLLQFNLQEESSSMV
NTETGEIIPMISLSFEQEPLYRLWHVLYSIDDREQLQSVLRQKFGIDDDEVLERLSAIDLVK
AGFGNKSSKAIRRILPFLQLGMNYAEACEAAGYNHSNNYTKAENEARALLDRLPAIKKNELR
QPVVEKILNQMVNVVNALMEKYGRFDEIRVELARELKQSKEERSNTYKSINKNQRENEQIAK
RIVEYGVPTRSRIQKYKMWEESKHCCIYCGQPVDVGDFLRGFDVEVEHIIPKSLYFDDSFAN
KVCSCRSCNKEKNNRTAYDYMKSKGEKALSDYVERVNTMYTNNQISKTKWQNLLTPVDKISI
DFIDRQLRESQYIARKAKEILTSICYNVTATSGSVTSFLRHVWGWDTVLHDLNFDRYKKVGL
TEVIEVNHRGSVIRREQIKDWSKRFDHRHHAIDALTIACTKQAYIQRLNNLRAEEGPDFNKM
SLERYIQSQPHFSVAQVREAVDRILVSFRAGKRAVTPGKRYIRKNRKRISVQSVLIPRGALS
EESVYGVIHVWEKDEQGHVIQKQRAVMKYPITSINREMLDKEKVVDKRIHRILSGRLAQYND
 
NPKEAFAKPVYIDKECRIPIRTVRCFAKPAINTLVPLKKDDKGNPVAWVNPGNNHHVAIYRD
EDGKYKERTVTFWEAVDRCRVGIPAIVTQPDTIWDNILQRNDISENVLESLPDVKWQFVLSL
QQNEMFILGMNEEDYRYAMDQQDYALLNKYLYRVQKLSKSDYSFRYHTETSVEDKYDGKPNL
KLSMQMGKLKRVSIKSLLGLNPHKVHISVLGEIKEIS
SEQ ID NO: 382 
MAEKQHRWGLDIGTNSIGWAVIALIEGRPAGLVATGSRIFSDGRNPKDGSSLAVERRGPRQM
RRRRDRYLRRRDRFMQALINVGLMPGDAAARKALVTENPYVLRQRGLDQALTLPEFGRALFH
LNQRRGFQSNRKTDRATAKESGKVKNAIAAFRAGMGNARTVGEALARRLEDGRPVRARMVGQ
GKDEHYELYIAREWIAQEFDALWASQQRFHAEVLADAARDRLRAILLFQRKLLPVPVGKCFL
EPNQPRVAAALPSAQRFRLMQELNHLRVMTLADKRERPLSFQERNDLLAQLVARPKCGFDML
RKIVFGANKEAYRFTIESERRKELKGCDTAAKLAKVNALGTRWQALSLDEQDRLVCLLLDGE
NDAVLADALREHYGLTDAQIDTLLGLSFEDGHMRLGRSALLRVLDALESGRDEQGLPLSYDK
AVVAAGYPAHTADLENGERDALPYYGELLWRYTQDAPTAKNDAERKFGKIANPTVHIGLNQL
RKLVNALIQRYGKPAQIVVELARNLKAGLEEKERIKKQQTANLERNERIRQKLQDAGVPDNR
ENRLRMRLFEELGQGNGLGTPCIYSGRQISLQRLFSNDVQVDHILPFSKTLDDSFANKVLAQ
HDANRYKGNRGPFEAFGANRDGYAWDDIRARAAVLPRNKRNRFAETAMQDWLHNETDFLARQ
LTDTAYLSRVARQYLTAICSKDDVYVSPGRLTAMLRAKWGLNRVLDGVMEEQGRPAVKNRDD
HRHHAIDAVVIGATDRAMLQQVATLAARAREQDAERLIGDMPTPWPNFLEDVRAAVARCVVS
HKPDHGPEGGLHNDTAYGIVAGPFEDGRYRVRHRVSLFDLKPGDLSNVRCDAPLQAELEPIF
EQDDARAREVALTALAERYRQRKVWLEELMSVLPIRPRGEDGKTLPDSAPYKAYKGDSNYCY
ELFINERGRWDGELISTFRANQAAYRRFRNDPARFRRYTAGGRPLLMRLCINDYIAVGTAAE
RTIFRVVKMSENKITLAEHFEGGTLKQRDADKDDPFKYLTKSPGALRDLGARRIFVDLIGRV
LDPGIKGD
SEQ ID NO: 383 
MIERILGVDLGISSLGWAIVEYDKDDEAANRIIDCGVRLFTAAETPKKKESPNKARREARGI
RRVLNRRRVRMNMIKKLFLRAGLIQDVDLDGEGGMFYSKANRADVWELRHDGLYRLLKGDEL
ARVLIHIAKHRGYKFIGDDEADEESGKVKKAGVVLRQNFEAAGCRTVGEWLWRERGANGKKR
NKHGDYEISIHRDLLVEEVEAIFVAQQEMRSTIATDALKAAYREIAFFVRPMQRIEKMVGHC
TYFPEERRAPKSAPTAEKFIAISKFFSTVIIDNEGWEQKIIERKTLEELLDFAVSREKVEFR
HLRKFLDLSDNEIFKGLHYKGKPKTAKKREATLFDPNEPTELEFDKVEAEKKAWISLRGAAK
LREALGNEFYGRFVALGKHADEATKILTYYKDEGQKRRELTKLPLEAEMVERLVKIGFSDFL
KLSLKAIRDILPAMESGARYDEAVLMLGVPHKEKSAILPPLNKTDIDILNPTVIRAFAQFRK
VANALVRKYGAFDRVHFELAREINTKGEIEDIKESQRKNEKERKEAADWIAETSFQVPLTRK
NILKKRLYIQQDGRCAYTGDVIELERLFDEGYCEIDHILPRSRSADDSFANKVLCLARANQQ
KTDRTPYEWFGHDAARWNAFETRTSAPSNRVRTGKGKIDRLLKKNFDENSEMAFKDRNLNDT
RYMARAIKTYCEQYWVFKNSHTKAPVQVRSGKLTSVLRYQWGLESKDRESHTHHAVDAIIIA
FSTQGMVQKLSEYYRFKETHREKERPKLAVPLANFRDAVEEATRIENTETVKEGVEVKRLLI
SRPPRARVTGQAHEQTAKPYPRIKQVKNKKKWRLAPIDEEKFESFKADRVASANQKNFYETS
TIPRVDVYHKKGKFHLVPIYLHEMVLNELPNLSLGTNPEAMDENFFKFSIFKDDLISIQTQG
TPKKPAKIIMGYFKNMHGANMVLSSINNSPCEGFTCTPVSMDKKHKDKCKLCPEENRIAGRC
LQGFLDYWSQEGLRPPRKEFECDQGVKFALDVKKYQIDPLGYYYEVKQEKRLGTIPQMRSAK
KLVKK
SEQ ID NO: 384 
MNNSIKSKPEVTIGLDLGVGSVGWAIVDNETNIIHHLGSRLFSQAKTAEDRRSFRGVRRLIR
RRKYKLKRFVNLIWKYNSYFGFKNKEDILNNYQEQQKLHNTVLNLKSEALNAKIDPKALSWI
LHDYLKNRGHFYEDNRDFNVYPTKELAKYFDKYGYYKGIIDSKEDNDNKLEEELTKYKFSNK
HWLEEVKKVLSNQTGLPEKFKEEYESLFSYVRNYSEGPGSINSVSPYGIYHLDEKEGKVVQK
YNNIWDKTIGKCNIFPDEYRAPKNSPIAMIFNEINELSTIRSYSIYLTGWFINQEFKKAYLN
 
KLLDLLIKTNGEKPIDARQFKKLREETIAESIGKETLKDVENEEKLEKEDHKWKLKGLKLNT
NGKIQYNDLSSLAKFVHKLKQHLKLDFLLEDQYATLDKINFLQSLFVYLGKHLRYSNRVDSA
NLKEFSDSNKLFERILQKQKDGLFKLFEQTDKDDEKILAQTHSLSTKAMLLAITRMTNLDND
EDNQKNNDKGWNFEAIKNFDQKFIDITKKNNNLSLKQNKRYLDDRFINDAILSPGVKRILRE
ATKVFNAILKQFSEEYDVTKVVIELARELSEEKELENTKNYKKLIKKNGDKISEGLKALGIS
EDEIKDILKSPTKSYKFLLWLQQDHIDPYSLKEIAFDDIFTKTEKFEIDHIIPYSISFDDSS
SNKLLVLAESNQAKSNQTPYEFISSGNAGIKWEDYEAYCRKFKDGDSSLLDSTQRSKKFAKM
MKTDTSSKYDIGFLARNLNDTRYATIVFRDALEDYANNHLVEDKPMFKVVCINGSVTSFLRK
NFDDSSYAKKDRDKNIHHAVDASIISIFSNETKTLFNQLTQFADYKLFKNTDGSWKKIDPKT
GVVTEVTDENWKQIRVRNQVSEIAKVIEKYIQDSNIERKARYSRKIENKTNISLFNDTVYSA
KKVGYEDQIKRKNLKTLDIHESAKENKNSKVKRQFVYRKLVNVSLLNNDKLADLFAEKEDIL
MYRANPWVINLAEQIFNEYTENKKIKSQNVFEKYMLDLTKEFPEKFSEFLVKSMLRNKTAII
YDDKKNIVHRIKRLKMLSSELKENKLSNVIIRSKNQSGTKLSYQDTINSLALMIMRSIDPTA
KKQYIRVPLNTLNLHLGDHDFDLHNMDAYLKKPKFVKYLKANEIGDEYKPWRVLTSGTLLIH
KKDKKLMYISSFQNLNDVIEIKNLIETEYKENDDSDSKKKKKANRFLMTLSTILNDYILLDA
KDNFDILGLSKNRIDEILNSKLGLDKIVK
SEQ ID NO: 385 
MGGSEVGTVPVTWRLGVDVGERSIGLAAVSYEEDKPKEILAAVSWIHDGGVGDERSGASRLA
LRGMARRARRLRRFRRARLRDLDMLLSELGWTPLPDKNVSPVDAWLARKRLAEEYVVDETER
RRLLGYAVSHMARHRGWRNPWTTIKDLKNLPQPSDSWERTRESLEARYSVSLEPGTVGQWAG
YLLQRAPGIRLNPTQQSAGRRAELSNATAFETRLRQEDVLWELRCIADVQGLPEDVVSNVID
AVFCQKRPSVPAERIGRDPLDPSQLRASRACLEFQEYRIVAAVANLRIRDGSGSRPLSLEER
NAVIEALLAQTERSLTWSDIALEILKLPNESDLTSVPEEDGPSSLAYSQFAPFDETSARIAE
FIAKNRRKIPTFAQWWQEQDRTSRSDLVAALADNSIAGEEEQELLVHLPDAELEALEGLALP
SGRVAYSRLTLSGLTRVMRDDGVDVHNARKTCFGVDDNWRPPLPALHEATGHPVVDRNLAIL
RKFLSSATMRWGPPQSIVVELARGASESRERQAEEEAARRAHRKANDRIRAELRASGLSDPS
PADLVRARLLELYDCHCMYCGAPISWENSELDHIVPRTDGGSNRHENLAITCGACNKEKGRR
PFASWAETSNRVQLRDVIDRVQKLKYSGNMYWTRDEFSRYKKSVVARLKRRTSDPEVIQSIE
STGYAAVALRDRLLSYGEKNGVAQVAVFRGGVTAEARRWLDISIERLFSRVAIFAQSTSTKR
LDRRHHAVDAVVLTTLTPGVAKTLADARSRRVSAEFWRRPSDVNRHSTEEPQSPAYRQWKES
CSGLGDLLISTAARDSIAVAAPLRLRPTGALHEETLRAFSEHTVGAAWKGAELRRIVEPEVY
AAFLALTDPGGRFLKVSPSEDVLPADENRHIVLSDRVLGPRDRVKLFPDDRGSIRVRGGAAY
IASFHHARVFRWGSSHSPSFALLRVSLADLAVAGLLRDGVDVFTAELPPWTPAWRYASIALV
KAVESGDAKQVGWLVPGDELDFGPEGVTTAAGDLSMFLKYFPERHWVVTGFEDDKRINLKPA
FLSAEQAEVLRTERSDRPDTLTEAGEILAQFFPRCWRATVAKVLCHPGLTVIRRTALGQPRW
RRGHLPYSWRPWSADPWSGGTP
SEQ ID NO: 386

MHNKKNITIGFDLGIASIGWAIIDSTTSKILDWGTRTFEERKTANERRAFRSTRRNIRRKAY
RNQRFINLILKYKDLFELKNISDIQRANKKDTENYEKIISFFTEIYKKCAAKHSNILEVKVK
ALDSKIEKLDLIWILHDYLENRGFFYDLEEENVADKYEGIEHPSILLYDFFKKNGFFKSNSS
IPKDLGGYSFSNLQWVNEIKKLFEVQEINPEFSEKFLNLFTSVRDYAKGPGSEHSASEYGIF
QKDEKGKVFKKYDNIWDKTIGKCSFFVEENRSPVNYPSYEIFNLLNQLINLSTDLKTTNKKI
WQLSSNDRNELLDELLKVKEKAKIISISLKKNEIKKIILKDFGFEKSDIDDQDTIEGRKIIK
EEPTTKLEVTKHLLATIYSHSSDSNWININNILEFLPYLDAICIILDREKSRGQDEVLKKLT
EKNIFEVLKIDREKQLDFVKSIFSNTKFNFKKIGNFSLKAIREFLPKMFEQNKNSEYLKWKD
EEIRRKWEEQKSKLGKTDKKTKYLNPRIFQDEIISPGTKNTFEQAVLVLNQIIKKYSKENII
DAIIIESPREKNDKKTIEEIKKRNKKGKGKTLEKLFQILNLENKGYKLSDLETKPAKLLDRL
RFYHQQDGIDLYTLDKINIDQLINGSQKYEIEHIIPYSMSYDNSQANKILTEKAENLKKGKL
IASEYIKRNGDEFYNKYYEKAKELFINKYKKNKKLDSYVDLDEDSAKNRFRFLTLQDYDEFQ

VEFLARNLNDTRYSTKLFYHALVEHFENNEFFTYIDENSSKHKVKISTIKGHVTKYFRAKPV
QKNNGPNENLNNNKPEKIEKNRENNEHHAVDAAIVAIIGNKNPQIANLLTLADNKTDKKFLL
HDENYKENIETGELVKIPKFEVDKLAKVEDLKKIIQEKYEEAKKHTAIKFSRKTRTILNGGL
SDETLYGFKYDEKEDKYFKIIKKKLVTSKNEELKKYFENPFGKKADGKSEYTVLMAQSHLSE
FNKLKEIFEKYNGFSNKTGNAFVEYMNDLALKEPTLKAEIESAKSVEKLLYYNFKPSDQFTY
HDNINNKSFKRFYKNIRIIEYKSIPIKFKILSKHDGGKSFKDTLFSLYSLVYKVYENGKESY
KSIPVTSQMRNFGIDEFDFLDENLYNKEKLDIYKSDFAKPIPVNCKPVFVLKKGSILKKKSL
DIDDFKETKETEEGNYYFISTISKRFNRDTAYGLKPLKLSVVKPVAEPSTNPIFKEYIPIHL
DELGNEYPVKIKEHTDDEKLMCTIK

### Nucleic Acids Encoding Cas9 Molecules

Nucleic acids encoding the Cas9 molecules or Cas9 polypeptides, e.g., an eaCas9 molecule or eaCas9 polypeptide are provided herein.

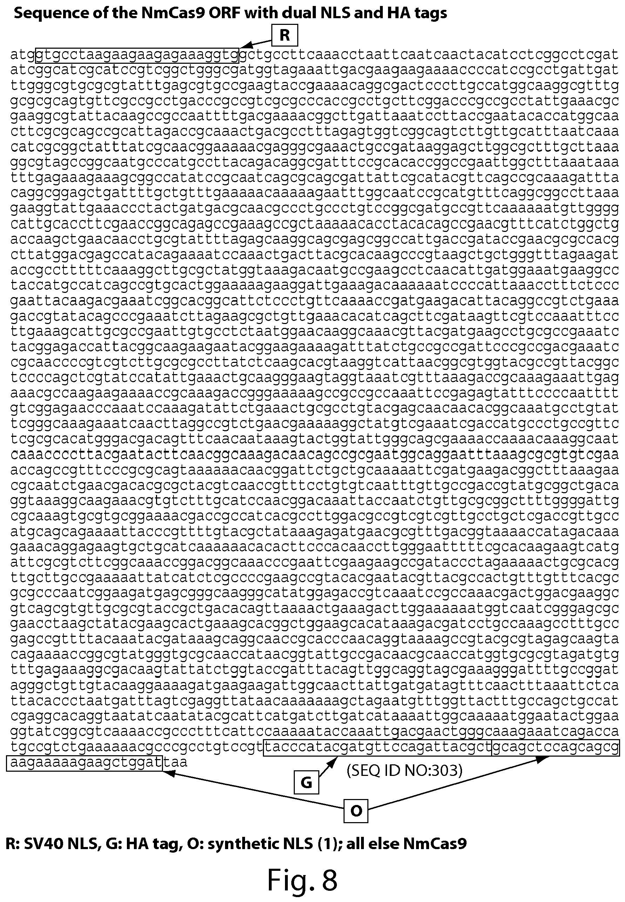

Exemplary nucleic acids encoding Cas9 molecules are described in Cong et al., SCIENCE 2013, 399(6121):819-823; Wang et al., CELL 2013, 153(4):910-918; Mali et al., SCIENCE 2013, 399(6121):823-826; Jinek et al., SCIENCE 2012, 337(6096):816-821. Another exemplary nucleic acid encoding a Cas9 molecule or Cas9 polypeptide is shown in black in **Fig. 8****.**

In an embodiment, a nucleic acid encoding a Cas9 molecule or Cas9 polypeptide can be a synthetic nucleic acid sequence. For example, the synthetic nucleic acid molecule can be chemically modified, e.g., as described in Section VIII. In an embodiment, the Cas9 mRNA has one or more (e.g., all of the following properties: it is capped, polyadenylated, substituted with 5-methylcytidine and/or pseudouridine.

In addition, or alternatively, the synthetic nucleic acid sequence can be codon optimized, e.g., at least one non-common codon or less-common codon has been replaced by a common codon. For example, the synthetic nucleic acid can direct the synthesis of an optimized messenger mRNA, e.g., optimized for expression in a mammalian expression system, e.g., described herein.

In addition, or alternatively, a nucleic acid encoding a Cas9 molecule or Cas9 polypeptide may comprise a nuclear localization sequence (NLS). Nuclear localization sequences are known in the art.

Provided below is an exemplary codon optimized nucleic acid sequence encoding a Cas9 molecule of *S. pyogenes.*

ATGGATAAAA AGTACAGCAT CGGGCTGGAC ATCGGTACAA ACTCAGTGGG
GTGGGCCGTG ATTACGGACG AGTACAAGGT ACCCTCCAAA AAATTTAAAG
TGCTGGGTAA CACGGACAGA CACTCTATAA AGAAAAATCT TATTGGAGCC
TTGCTGTTCG ACTCAGGCGA GACAGCCGAA GCCACAAGGT TGAAGCGGAC

CGCCAGGAGG CGGTATACCA GGAGAAAGAA CCGCATATGC TACCTGCAAG
AAATCTTCAG TAACGAGATG GCAAAGGTTG ACGATAGCTT TTTCCATCGC
CTGGAAGAAT CCTTTCTTGT TGAGGAAGAC AAGAAGCACG AACGGCACCC
CATCTTTGGC AATATTGTCG ACGAAGTGGC ATATCACGAA AAGTACCCGA
CTATCTACCA CCTCAGGAAG AAGCTGGTGG ACTCTACCGA TAAGGCGGAC
CTCAGACTTA TTTATTTGGC ACTCGCCCAC ATGATTAAAT TTAGAGGACA
TTTCTTGATC GAGGGCGACC TGAACCCGGA CAACAGTGAC GTCGATAAGC
TGTTCATCCA ACTTGTGCAG ACCTACAATC AACTGTTCGA AGAAAACCCT
ATAAATGCTT CAGGAGTCGA CGCTAAAGCA ATCCTGTCCG CGCGCCTCTC
AAAATCTAGA AGACTTGAGA ATCTGATTGC TCAGTTGCCC GGGGAAAAGA
AAAATGGATT GTTTGGCAAC CTGATCGCCC TCAGTCTCGG ACTGACCCCA
AATTTCAAAA GTAACTTCGA CCTGGCCGAA GACGCTAAGC TCCAGCTGTC
CAAGGACACA TACGATGACG ACCTCGACAA TCTGCTGGCC CAGATTGGGG
ATCAGTACGC CGATCTCTTT TTGGCAGCAA AGAACCTGTC CGACGCCATC
CTGTTGAGCG ATATCTTGAG AGTGAACACC GAAATTACTA AAGCACCCCT
TAGCGCATCT ATGATCAAGC GGTACGACGA GCATCATCAG GATCTGACCC
TGCTGAAGGC TCTTGTGAGG CAACAGCTCC CCGAAAAATA CAAGGAAATC
TTCTTTGACC AGAGCAAAAA CGGCTACGCT GGCTATATAG ATGGTGGGGC
CAGTCAGGAG GAATTCTATA AATTCATCAA GCCCATTCTC GAGAAAATGG
ACGGCACAGA GGAGTTGCTG GTCAAACTTA ACAGGGAGGA CCTGCTGCGG
AAGCAGCGGA CCTTTGACAA CGGGTCTATC CCCCACCAGA TTCATCTGGG
CGAACTGCAC GCAATCCTGA GGAGGCAGGA GGATTTTTAT CCTTTTCTTA
AAGATAACCG CGAGAAAATA GAAAAGATTC TTACATTCAG GATCCCGTAC
TACGTGGGAC CTCTCGCCCG GGGCAATTCA CGGTTTGCCT GGATGACAAG
GAAGTCAGAG GAGACTATTA CACCTTGGAA CTTCGAAGAA GTGGTGGACA
AGGGTGCATC TGCCCAGTCT TTCATCGAGC GGATGACAAA TTTTGACAAG
AACCTCCCTA ATGAGAAGGT GCTGCCCAAA CATTCTCTGC TCTACGAGTA
CTTTACCGTC TACAATGAAC TGACTAAAGT CAAGTACGTC ACCGAGGGAA
TGAGGAAGCC GGCATTCCTT AGTGGAGAAC AGAAGAAGGC GATTGTAGAC
CTGTTGTTCA AGACCAACAG GAAGGTGACT GTGAAGCAAC TTAAAGAAGA
CTACTTTAAG AAGATCGAAT GTTTTGACAG TGTGGAAATT TCAGGGGTTG
AAGACCGCTT CAATGCGTCA TTGGGGACTT ACCATGATCT TCTCAAGATC
ATAAAGGACA AAGACTTCCT GGACAACGAA GAAAATGAGG ATATTCTCGA
AGACATCGTC CTCACCCTGA CCCTGTTCGA AGACAGGGAA ATGATAGAAG
AGCGCTTGAA AACCTATGCC CACCTCTTCG ACGATAAAGT TATGAAGCAG
CTGAAGCGCA GGAGATACAC AGGATGGGGA AGATTGTCAA GGAAGCTGAT
CAATGGAATT AGGGATAAAC AGAGTGGCAA GACCATACTG GATTTCCTCA
AATCTGATGG CTTCGCCAAT AGGAACTTCA TGCAACTGAT TCACGATGAC
TCTCTTACCT TCAAGGAGGA CATTCAAAAG GCTCAGGTGA GCGGGCAGGG
AGACTCCCTT CATGAACACA TCGCGAATTT GGCAGGTTCC CCCGCTATTA
AAAAGGGCAT CCTTCAAACT GTCAAGGTGG TGGATGAATT GGTCAAGGTA
ATGGGCAGAC ATAAGCCAGA AAATATTGTG ATCGAGATGG CCCGCGAAAA
CCAGACCACA CAGAAGGGCC AGAAAAATAG TAGAGAGCGG ATGAAGAGGA
TCGAGGAGGG CATCAAAGAG CTGGGATCTC AGATTCTCAA AGAACACCCC
GTAGAAAACA CACAGCTGCA GAACGAAAAA TTGTACTTGT ACTATCTGCA
GAACGGCAGA GACATGTACG TCGACCAAGA ACTTGATATT AATAGACTGT
CCGACTATGA CGTAGACCAT ATCGTGCCCC AGTCCTTCCT GAAGGACGAC
TCCATTGATA ACAAAGTCTT GACAAGAAGC GACAAGAACA GGGGTAAAAG
TGATAATGTG CCTAGCGAGG AGGTGGTGAA AAAAATGAAG AACTACTGGC
GACAGCTGCT TAATGCAAAG CTCATTACAC AACGGAAGTT CGATAATCTG
ACGAAAGCAG AGAGAGGTGG CTTGTCTGAG TTGGACAAGG CAGGGTTTAT

TAAGCGGCAG CTGGTGGAAA CTAGGCAGAT CACAAAGCAC GTGGCGCAGA
TTTTGGACAG CCGGATGAAC ACAAAATACG ACGAAAATGA TAAACTGATA
CGAGAGGTCA AAGTTATCAC GCTGAAAAGC AAGCTGGTGT CCGATTTTCG
GAAAGACTTC CAGTTCTACA AAGTTCGCGA GATTAATAAC TACCATCATG
CTCACGATGC GTACCTGAAC GCTGTTGTCG GGACCGCCTT GATAAAGAAG
TACCCAAAGC TGGAATCCGA GTTCGTATAC GGGGATTACA AAGTGTACGA
TGTGAGGAAA ATGATAGCCA AGTCCGAGCA GGAGATTGGA AAGGCCACAG
CTAAGTACTT CTTTTATTCT AACATCATGA ATTTTTTTAA GACGGAAATT
ACCCTGGCCA ACGGAGAGAT CAGAAAGCGG CCCCTTATAG AGACAAATGG
TGAAACAGGT GAAATCGTCT GGGATAAGGG CAGGGATTTC GCTACTGTGA
GGAAGGTGCT GAGTATGCCA CAGGTAAATA TCGTGAAAAA AACCGAAGTA
CAGACCGGAG GATTTTCCAA GGAAAGCATT TTGCCTAAAA GAAACTCAGA
CAAGCTCATC GCCCGCAAGA AAGATTGGGA CCCTAAGAAA TACGGGGGAT
TTGACTCACC CACCGTAGCC TATTCTGTGC TGGTGGTAGC TAAGGTGGAA
AAAGGAAAGT CTAAGAAGCT GAAGTCCGTG AAGGAACTCT TGGGAATCAC
TATCATGGAA AGATCATCCT TTGAAAAGAA CCCTATCGAT TTCCTGGAGG
CTAAGGGTTA CAAGGAGGTC AAGAAAGACC TCATCATTAA ACTGCCAAAA
TACTCTCTCT TCGAGCTGGA AAATGGCAGG AAGAGAATGT TGGCCAGCGC
CGGAGAGCTG CAAAAGGGAA ACGAGCTTGC TCTGCCCTCC AAATATGTTA
ATTTTCTCTA TCTCGCTTCC CACTATGAAA AGCTGAAAGG GTCTCCCGAA
GATAACGAGC AGAAGCAGCT GTTCGTCGAA CAGCACAAGC ACTATCTGGA
TGAAATAATC GAACAAATAA GCGAGTTCAG CAAAAGGGTT ATCCTGGCGG
ATGCTAATTT GGACAAAGTA CTGTCTGCTT ATAACAAGCA CCGGGATAAG
CCTATTAGGG AACAAGCCGA GAATATAATT CACCTCTTTA CACTCACGAA
TCTCGGAGCC CCCGCCGCCT TCAAATACTT TGATACGACT ATCGACCGGA
AACGGTATAC CAGTACCAAA GAGGTCCTCG ATGCCACCCT CATCCACCAG
TCAATTACTG GCCTGTACGA AACACGGATC GACCTCTCTC AACTGGGCGG
CGACTAG
(SEQ ID NO: 22)

Provided below is the corresponding amino acid sequence of a *S. pyogenes* Cas9 molecule.
























*
(SEQ ID NO: 23)

Provided below is an exemplary codon optimized nucleic acid sequence encoding a Cas9 molecule *o*f *N. meningitidis.*

ATGGCCGCCTTCAAGCCCAACCCCATCAACTACATCCTGGGCCTGGACATCGGCATCGCCAG
CGTGGGCTGGGCCATGGTGGAGATCGACGAGGACGAGAACCCCATCTGCCTGATCGACCTGG
GTGTGCGCGTGTTCGAGCGCGCTGAGGTGCCCAAGACTGGTGACAGTCTGGCTATGGCTCGC
CGGCTTGCTCGCTCTGTTCGGCGCCTTACTCGCCGGCGCGCTCACCGCCTTCTGCGCGCTCG
CCGCCTGCTGAAGCGCGAGGGTGTGCTGCAGGCTGCCGACTTCGACGAGAACGGCCTGATCA
AGAGCCTGCCCAACACTCCTTGGCAGCTGCGCGCTGCCGCTCTGGACCGCAAGCTGACTCCT
CTGGAGTGGAGCGCCGTGCTGCTGCACCTGATCAAGCACCGCGGCTACCTGAGCCAGCGCAA
GAACGAGGGCGAGACCGCCGACAAGGAGCTGGGTGCTCTGCTGAAGGGCGTGGCCGACAACG
CCCACGCCCTGCAGACTGGTGACTTCCGCACTCCTGCTGAGCTGGCCCTGAACAAGTTCGAG
AAGGAGAGCGGCCACATCCGCAACCAGCGCGGCGACTACAGCCACACCTTCAGCCGCAAGGA
CCTGCAGGCCGAGCTGATCCTGCTGTTCGAGAAGCAGAAGGAGTTCGGCAACCCCCACGTGA
GCGGCGGCCTGAAGGAGGGCATCGAGACCCTGCTGATGACCCAGCGCCCCGCCCTGAGCGGC
GACGCCGTGCAGAAGATGCTGGGCCACTGCACCTTCGAGCCAGCCGAGCCCAAGGCCGCCAA
GAACACCTACACCGCCGAGCGCTTCATCTGGCTGACCAAGCTGAACAACCTGCGCATCCTGG
AGCAGGGCAGCGAGCGCCCCCTGACCGACACCGAGCGCGCCACCCTGATGGACGAGCCCTAC
CGCAAGAGCAAGCTGACCTACGCCCAGGCCCGCAAGCTGCTGGGTCTGGAGGACACCGCCTT
CTTCAAGGGCCTGCGCTACGGCAAGGACAACGCCGAGGCCAGCACCCTGATGGAGATGAAGG
CCTACCACGCCATCAGCCGCGCCCTGGAGAAGGAGGGCCTGAAGGACAAGAAGAGTCCTCTG
AACCTGAGCCCCGAGCTGCAGGACGAGATCGGCACCGCCTTCAGCCTGTTCAAGACCGACGA
GGACATCACCGGCCGCCTGAAGGACCGCATCCAGCCCGAGATCCTGGAGGCCCTGCTGAAGC
ACATCAGCTTCGACAAGTTCGTGCAGATCAGCCTGAAGGCCCTGCGCCGCATCGTGCCCCTG
ATGGAGCAGGGCAAGCGCTACGACGAGGCCTGCGCCGAGATCTACGGCGACCACTACGGCAA
GAAGAACACCGAGGAGAAGATCTACCTGCCTCCTATCCCCGCCGACGAGATCCGCAACCCCG
TGGTGCTGCGCGCCCTGAGCCAGGCCCGCAAGGTGATCAACGGCGTGGTGCGCCGCTACGGC
AGCCCCGCCCGCATCCACATCGAGACCGCCCGCGAGGTGGGCAAGAGCTTCAAGGACCGCAA
GGAGATCGAGAAGCGCCAGGAGGAGAACCGCAAGGACCGCGAGAAGGCCGCCGCCAAGTTCC
GCGAGTACTTCCCCAACTTCGTGGGCGAGCCCAAGAGCAAGGACATCCTGAAGCTGCGCCTG
TACGAGCAGCAGCACGGCAAGTGCCTGTACAGCGGCAAGGAGATCAACCTGGGCCGCCTGAA
CGAGAAGGGCTACGTGGAGATCGACCACGCCCTGCCCTTCAGCCGCACCTGGGACGACAGCT
TCAACAACAAGGTGCTGGTGCTGGGCAGCGAGAACCAGAACAAGGGCAACCAGACCCCCTAC
GAGTACTTCAACGGCAAGGACAACAGCCGCGAGTGGCAGGAGTTCAAGGCCCGCGTGGAGAC
CAGCCGCTTCCCCCGCAGCAAGAAGCAGCGCATCCTGCTGCAGAAGTTCGACGAGGACGGCT
TCAAGGAGCGCAACCTGAACGACACCCGCTACGTGAACCGCTTCCTGTGCCAGTTCGTGGCC
GACCGCATGCGCCTGACCGGCAAGGGCAAGAAGCGCGTGTTCGCCAGCAACGGCCAGATCAC
CAACCTGCTGCGCGGCTTCTGGGGCCTGCGCAAGGTGCGCGCCGAGAACGACCGCCACCACG
CCCTGGACGCCGTGGTGGTGGCCTGCAGCACCGTGGCCATGCAGCAGAAGATCACCCGCTTC
GTGCGCTACAAGGAGATGAACGCCTTCGACGGTAAAACCATCGACAAGGAGACCGGCGAGGT
GCTGCACCAGAAGACCCACTTCCCCCAGCCCTGGGAGTTCTTCGCCCAGGAGGTGATGATCC
GCGTGTTCGGCAAGCCCGACGGCAAGCCCGAGTTCGAGGAGGCCGACACCCCCGAGAAGCTG
CGCACCCTGCTGGCCGAGAAGCTGAGCAGCCGCCCTGAGGCCGTGCACGAGTACGTGACTCC

TCTGTTCGTGAGCCGCGCCCCCAACCGCAAGATGAGCGGTCAGGGTCACATGGAGACCGTGA
AGAGCGCCAAGCGCCTGGACGAGGGCGTGAGCGTGCTGCGTGCCCCTGACCCAGCTGAAG
CTGAAGGACCTGGAGAAGATGGTGAACCGCGAGCGCGAGCCCAAGCTGTACGAGGCCCTGAA
GGCCCGCCTGGAGGCCCACAAGGACGACCCCGCCAAGGCCTTCGCCGAGCCCTTCTACAAGT
ACGACAAGGCCGGCAACCGCACCCAGCAGGTGAAGGCCGTGCGCGTGGAGCAGGTGCAGAAG
ACCGGCGTGTGGGTGCGCAACCACAACGGCATCGCCGACAACGCCACCATGGTGCGCGTGGA
CGTGTTCGAGAAGGGCGACAAGTACTACCTGGTGCCCATCTACAGCTGGCAGGTGGCCAAGG
GCATCCTGCCCGACCGCGCCGTGGTGCAGGGCAAGGACGAGGAGGACTGGCAGCTGATCGAC
GACAGCTTCAACTTCAAGTTCAGCCTGCACCCCAACGACCTGGTGGAGGTGATCACCAAGAA
GGCCCGCATGTTCGGCTACTTCGCCAGCTGCCACCGCGGCACCGGCAACATCAACATCCGCA
TCCACGACCTGGACCACAAGATCGGCAAGAACGGCATCCTGGAGGGCATCGGCGTGAAGACC
GCCCTGAGCTTCCAGAAGTACCAGATCGACGAGCTGGGCAAGGAGATCCGCCCCTGCCGCCT
GAAGAAGCGCCCTCCTGTGCGCTAA
(SEQ ID NO: 24)

Provided below is the corresponding amino acid sequence of a *N. meningitidis* Cas9 molecule.

MAAFKPNPINYILGLDIGIASVGWAMVEIDEDENPICLIDLGVRVFERAEVPKTGDSLAMAR
RLARSVRRLTRRRAHRLLRARRLLKREGVLQAADFDENGLIKSLPNTPWQLRAAALDRKLTP
LEWSAVLLHLIKHRGYLSQRKNEGETADKELGALLKGVADNAHALQTGDFRTPAELALNKFE
KESGHIRNQRGDYSHTFSRKDLQAELILLFEKQKEFGNPHVSGGLKEGIETLLMTQRPALSG
DAVQKMLGHCTFEPAEPKAAKNTYTAERFIWLTKLNNLRILEQGSERPLTDTERATLMDEPY
RKSKLTYAQARKLLGLEDTAFFKGLRYGKDNAEASTLMEMKAYHAISRALEKEGLKDKKSPL
NLSPELQDEIGTAFSLFKTDEDITGRLKDRIQPEILEALLKHISFDKFVQISLKALRRIVPL
MEQGKRYDEACAEIYGDHYGKKNTEEKIYLPPIPADEIRNPVVLRALSQARKVINGVVRRYG
SPARIHIETAREVGKSFKDRKEIEKRQEENRKDREKAAAKFREYFPNFVGEPKSKDILKLRL
YEQQHGKCLYSGKEINLGRLNEKGYVEIDHALPFSRTWDDSFNNKVLVLGSENQNKGNQTPY
EYFNGKDNSREWQEFKARVETSRFPRSKKQRILLQKFDEDGFKERNLNDTRYVNRFLCQFVA
DRMRLTGKGKKRVFASNGQITNLLRGFWGLRKVRAENDRHHALDAVVVACSTVAMQQKITRF
VRYKEMNAFDGKTIDKETGEVLHQKTHFPQPWEFFAQEVMIRVFGKPDGKPEFEEADTPEKL
RTLLAEKLSSRPEAVHEYVTPLFVSRAPNRKMSGQGHMETVKSAKRLDEGVSVLRVPLTQLK
LKDLEKMVNREREPKLYEALKARLEAHKDDPAKAFAEPFYKYDKAGNRTQQVKAVRVEQVQK
TGVWVRNHNGIADNATMVRVDVFEKGDKYYLVPIYSWQVAKGILPDRAVVQGKDEEDWQLID
DSFNFKFSLHPNDLVEVITKKARMFGYFASCHRGTGNINIRIHDLDHKIGKNGILEGIGVKT
ALSFQKYQIDELGKEIRPCRLKKRPPVR*
(SEQ ID NO: 25)

Provided below is an amino acid sequence of a *S*. *aureus* Cas9 molecule.

MKRNYILGLDIGITSVGYGIIDYETRDVIDAGVRLFKEANVENNEGRRSKRGARRLKRRRRH
RIQRVKKLLFDYNLLTDHSELSGINPYEARVKGLSQKLSEEEFSAALLHLAKRRGVHNVNEV
EEDTGNELSTKEQISRNSKALEEKYVAELQLERLKKDGEVRGSINRFKTSDYVKEAKQLLKV
QKAYHQLDQSFIDTYIDLLETRRTYYEGPGEGSPFGWKDIKEWYEMLMGHCTYFPEELRSVK
YAYNADLYNALNDLNNLVITRDENEKLEYYEKFQIIENVFKQKKKPTLKQIAKEILVNEEDI
KGYRVTSTGKPEFTNLKVYHDIKDITARKEIIENAELLDQIAKILTIYQSSEDIQEELTNLN
SELTQEEIEQISNLKGYTGTHNLSLKAINLILDELWHTNDNQIAIFNRLKLVPKKVDLSQQK
EIPTTLVDDFILSPVVKRSFIQSIKVINAIIKKYGLPNDIIIELAREKNSKDAQKMINEMQK
RNRQTNERIEEIIRTTGKENAKYLIEKIKLHDMQEGKCLYSLEAIPLEDLLNNPFNYEVDHI
IPRSVSFDNSFNNKVLVKQEENSKKGNRTPFQYLSSSDSKISYETFKKHILNLAKGKGRISK
TKKEYLLEERDINRFSVQKDFINRNLVDTRYATRGLMNLLRSYFRVNNLDVKVKSINGGFTS

FLRRKWKFKKERNKGYKHHAEDALIIANADFIFKEWKKLDKAKKVMENQMFEEKQAESMPEI
ETEQEYKEIFITPHQIKHIKDFKDYKYSHRVDKKPNRELINDTLYSTRKDDKGNTLIVNNLN
GLYDKDNDKLKKLINKSPEKLLMYHHDPQTYQKLKLIMEQYGDEKNPLYKYYEETGNYLTKY
SKKDNGPVIKKIKYYGNKLNAHLDITDDYPNSRNKVVKLSLKPYRFDVYLDNGVYKFVTVKN
LDVIKKENYYEVNSKCYEEAKKLKKISNQAEFIASFYNNDLIKINGELYRVIGVNNDLLNRI
EVNMIDITYREYLENMNDKRPPRIIKTIASKTQSIKKYSTDILGNLYEVKSKKHPQIIKKG*
(SEQ ID NO: 26)

Provided below is an exemplary codon optimized nucleic acid sequence encoding a Cas9 molecule of *S. aureus* Cas9.

ATGAAAAGGAACTACATTCTGGGGCTGGACATCGGGATTACAAGCGTGGGGTATGGATTAT
TGACTATGAAACAAGGGACGTGATCGACGCAGGCGTCAGACTGTTCAAGGAGGCCAACGTGG
AAAACAATGAGGGACGGAGAAGCAAGAGGGGAGCCAGGCGCCTGAAACGACGGAGAAGGCAC
AGAATCCAGAGGGTGAAGAAACTGCTGTTCGATTACAACCTGCTGACCGACCATTCTGAGCT
GAGTGGAATTAATCCTTATGAAGCCAGGGTGAAAGGCCTGAGTCAGAAGCTGTCAGAGGAAG
AGTTTTCCGCAGCTCTGCTGCACCTGGCTAAGCGCCGAGGAGTGCATAACGTCAATGAGGTG
GAAGAGGACACCGGCAACGAGCTGTCTACAAAGGAACAGATCTCACGCAATAGCAAAGCTCT
GGAAGAGAAGTATGTCGCAGAGCTGCAGCTGGAACGGCTGAAGAAAGATGGCGAGGTGAGAG
GGTCAATTAATAGGTTCAAGACAAGCGACTACGTCAAAGAAGCCAAGCAGCTGCTGAAAGTG
CAGAAGGCTTACCACCAGCTGGATCAGAGCTTCATCGATACTTATATCGACCTGCTGGAGAC
TCGGAGAACCTACTATGAGGGACCAGGAGAAGGGAGCCCCTTCGGATGGAAAGACATCAAGG
AATGGTACGAGATGCTGATGGGACATTGCACCTATTTTCCAGAAGAGCTGAGAAGCGTCAAG
TACGCTTATAACGCAGATCTGTACAACGCCCTGAATGACCTGAACAACCTGGTCATCACCAG
GGATGAAAACGAGAAACTGGAATACTATGAGAAGTTCCAGATCATCGAAAACGTGTTTAAGC
AGAAGAAAAAGCCTACACTGAAACAGATTGCTAAGGAGATCCTGGTCAACGAAGAGGACATC
AAGGGCTACCGGGTGACAAGCACTGGAAAACCAGAGTTCACCAATCTGAAAGTGTATCACGA
TATTAAGGACATCACAGCACGGAAAGAAATCATTGAGAACGCCGAACTGCTGGATCAGATTG
CTAAGATCCTGACTATCTACCAGAGCTCCGAGGACATCCAGGAAGAGCTGACTAACCTGAAC
AGCGAGCTGACCCAGGAAGAGATCGAACAGATTAGTAATCTGAAGGGGTACACCGGAACACA
CAACCTGTCCCTGAAAGCTATCAATCTGATTCTGGATGAGCTGTGGCATACAAACGACAATC
AGATTGCAATCTTTAACCGGCTGAAGCTGGTCCCAAAAAAGGTGGACCTGAGTCAGCAGAAA
GAGATCCCAACCACACTGGTGGACGATTTCATTCTGTCACCCGTGGTCAAGCGGAGCTTCAT
CCAGAGCATCAAAGTGATCAACGCCATCATCAAGAAGTACGGCCTGCCCAATGATATCATTA
TCGAGCTGGCTAGGGAGAAGAACAGCAAGGACGCACAGAAGATGATCAATGAGATGCAGAAA
CGAAACCGGCAGACCAATGAACGCATTGAAGAGATTATCCGAACTACCGGGAAAGAGAACGC
AAAGTACCTGATTGAAAAAATCAAGCTGCACGATATGCAGGAGGGAAAGTGTCTGTATTCTC
TGGAGGCCATCCCCCTGGAGGACCTGCTGAACAATCCATTCAACTACGAGGTCGATCATATT
ATCCCCAGAAGCGTGTCCTTCGACAATTCCTTTAACAACAAGGTGCTGGTCAAGCAGGAAGA
GAACTCTAAAAAGGGCAATAGGACTCCTTTCCAGTACCTGTCTAGTTCAGATTCCAAGATCT
CTTACGAAACCTTTAAAAAGCACATTCTGAATCTGGCCAAAGGAAAGGGCCGCATCAGCAAG
ACCAAAAAGGAGTACCTGCTGGAAGAGCGGGACATCAACAGATTCTCCGTCCAGAAGGATTT
TATTAACCGGAATCTGGTGGACACAAGATACGCTACTCGCGGCCTGATGAATCTGCTGCGAT
CCTATTTCCGGGTGAACAATCTGGATGTGAAAGTCAAGTCCATCAACGGCGGGTTCACATCT
TTTCTGAGGCGCAAATGGAAGTTTAAAAAGGAGCGCAACAAAGGGTACAAGCACCATGCCGA
AGATGCTCTGATTATCGCAAATGCCGACTTCATCTTTAAGGAGTGGAAAAAGCTGGACAAAG
CCAAGAAAGTGATGGAGAACCAGATGTTCGAAGAGAAGCAGGCCGAATCTATGCCCGAAATC
GAGACAGAACAGGAGTACAAGGAGATTTTCATCACTCCTCACCAGATCAAGCATATCAAGGA
TTTCAAGGACTACAAGTACTCTCACCGGGTGGATAAAAAGCCCAACAGAGAGCTGATCAATG
ACACCCTGTATAGTACAAGAAAAGACGATAAGGGGAATACCCTGATTGTGAACAATCTGAAC
GGACTGTACGACAAAGATAATGACAAGCTGAAAAAGCTGATCAACAAAAGTCCCGAGAAGCT

GCTGATGTACCACCATGATCCTCAGACATATCAGAAACTGAAGCTGATTATGGAGCAGTACG
GCGACGAGAAGAACCCACTGTATAAGTACTATGAAGAGACTGGGAACTACCTGACCAAGTAT
AGCAAAAAGGATAATGGCCCCGTGATCAAGAAGATCAAGTACTATGGGAACAAGCTGAATGC
CCATCTGGACATCACAGACGATTACCCTAACAGTCGCAACAAGGTGGTCAAGCTGTCACTGA
AGCCATACAGATTCGATGTCTATCTGGACAACGGCGTGTATAAATTTGTGACTGTCAAGAAT
CTGGATGTCATCAAAAAGGAGAACTACTATGAAGTGAATAGCAAGTGCTACGAAGAGGCTAA
AAAGCTGAAAAAGATTAGCAACCAGGCAGAGTTCATCGCCTCCTTTTACAACAACGACCTGA
TTAAGATCAATGGCGAACTGTATAGGGTCATCGGGGTGAACAATGATCTGCTGAACCGCATT
GAAGTGAATATGATTGACATCACTTACCGAGAGTATCTGGAAAACATGAATGATAAGCGCCC
CCCTCGAATTATCAAAACAATTGCCTCTAAGACTCAGAGTATCAAAAAGTACTCAACCGACA
TTCTGGGAAACCTGTATGAGGTGAAGAGCAAAAAGCACCCTCAGATTATCAAAAAGGGC
(SEQ ID NO: 39)

If any of the above Cas9 sequences (e.g., a eiCas9) are fused with a transcription repressor at the C-terminus, it is understood that the stop codon will be removed.

### Other Cas Molecules and Cas9 Polypeptides

Various types of Cas molecules or Cas9 polypeptides can be used to practice the inventions disclosed herein. In some embodiments, Cas molecules of Type II Cas systems are used. In other embodiments, Cas molecules of other Cas systems are used. For example, Type I or Type III Cas molecules may be used. Exemplary Cas molecules (and Cas systems) are described, e.g., in Haft et al., PLoS COMPUTATIONAL BIOLOGY 2005, 1(6): e60 and Makarova et al., NATURE REVIEW MICROBIOLOGY 2011, 9:467-477, the contents of both references are incorporated herein by reference in their entirety. Exemplary Cas molecules (and Cas systems) are also shown in **Table 30.**

| **Table 30: Cas Systems** | | | | | |
|---|---|---|---|---|---|
| **Gene name^{‡}** | **System type or subtype** | **Name from Haft *et al.*^{§}** | **Structure of encoded protein (PDB accessions)^{¶}** | **Families (and superfamily) of encoded protein^{#**}** | **Representatives** |
| *cas1* | • Type I | *cas1* | 3GOD, 3LFX and 2YZS | COG1518 | SERP2463, SPy1047 *and ygbT* |
| | • Type II | | | | |
| | • Type III | | | | |
| *cas2* | • Type I | *cas2* | 2IVY, 2I8E and 3EXC | COG1343 and COG3512 | SERP2462, SPy1048, SPy1723 (N-terminal domain) and *ygbF* |
| | • Type II | | | | |
| | • Type III | | | | |
| *cas3'* | • Type 1^{‡‡} | *cas3* | NA | COG1203 | APE1232 and *ygcB* |
| *cas3"* | • Subtype I-A | NA | NA | COG2254 | APE1231 and BH0336 |
| | • Subtype I-B | | | | |
| *cas4* | • Subtype I-A | *cas4* and *csa1* | NA | COG1468 | APE1239 and BH0340 |
| | • Subtype I-B | | | | |
| | • Subtype I-C | | | | |
| | • Subtype I-D | | | | |
| | • Subtype II-B | | | | |
| *cas5* | • Subtype I-A | *cas5a, cas5d, cas5e, cas5h, cas5p, cas5t* and *cmx5* | 3KG4 | COG1688 (RAMP) | APE1234, BH0337, *devS* and *ygcI* |
| | • Subtype I-B | | | | |
| | • Subtype I-C | | | | |
| | • Subtype I-E | | | | |
| *cas6* | • Subtype I-A | *cas6* and *cmx6* | 3I4H | COG1583 and COG5551 (RAMP) | PF1131 and slr7014 |
| | • Subtype I-B | | | | |
| | • Subtype I-D | | | | |
| | • Subtype III- | | | | |
| | A• Subtype III-B | | | | |
| *cas6e* | • Subtype I-E | *cse3* | 1WJ9 | (RAMP) | *ygcH* |
| *cas6f* | • Subtype I-F | *csy4* | 2XLJ | (RAMP) | y1727 |
| *cas7* | • Subtype I-A | *csa2, csd2, cse4, csh2, csp1* and *cst2* | NA | COG1857 and COG3649 (RAMP) | *devR* and *ygcJ* |
| | • Subtype I-B | | | | |
| | • Subtype I-C | | | | |
| | • Subtype I-E | | | | |
| *cas8a1* | • Subtype I-A^{‡‡} | *cmx1, cst1, csx8, csx13* and CXXC-CXXC | NA | BH0338-like | LA3191^{§§} and PG2018^{§§} |
| *cas8a2* | • Subtype I-A^{‡‡} | *csa4* and *csx9* | NA | PH0918 | AF0070, AF1873, MJ0385, PF0637, PH0918 and SS01401 |
| *cas8b* | • Subtype I-B^{‡‡} | *csh1* and TM1802 | NA | BH0338-like | MTH1090 and TM1802 |
| *cas8c* | • Subtype I-C^{‡‡} | *csd1* and *csp2* | NA | BH0338-like | BH0338 |
| *cas9* | • Type II^{‡‡} | *csn1* and *csx12* | NA | COG3513 | FTN_0757 and SPy1046 |
| *cas10* | • Type III^{‡‡} | *cmr2, csm1* and *csx11* | NA | COG1353 | MTH326, Rv2823c^{§§} and TM1794^{§§} |
| *cas10d* | • Subtype I-D^{‡‡} | *csc3* | NA | COG1353 | slr7011 |
| *csy1* | • Subtype IF^{‡‡} | *csy1* | NA | y1724-like | y1724 |
| *csy2* | • Subtype I-F | *csy2* | NA | (RAMP) | y1725 |
| *csy3* | • Subtype I-F | *csy3* | NA | (RAMP) | y1726 |
| *cse1* | • Subtype I-E^{‡‡} | *cse1* | NA | YgcL-like | *ygcL* |
| *cse2* | • Subtype I-E | *cse2* | 2ZCA | YgcK-like | *ygcK* |
| *csc1* | • Subtype I-D | *csc1* | NA | alr1563-like (RAMP) | alr1563 |
| *csc2* | • Subtype I-D | *csc1* and *csc2* | NA | COG1337 (RAMP) | slr7012 |
| *csa5* | • Subtype I-A | *csa5* | NA | AF1870 | AF1870, MJ0380, PF0643 and SS01398 |
| *csn2* | • Subtype II-A | *csn2* | NA | SPy1049-like | SPy1049 |
| *csm2* | • Subtype III-A^{‡‡} | *csm2* | NA | COG1421 | MTH1081 and SERP2460 |
| *csm3* | • Subtype III-A | *csc2* and *csm3* | NA | COG1337 (RAMP) | MTH1080 and SERP2459 |
| *csm4* | • Subtype III-A | *csm4* | NA | COG1567 (RAMP) | MTH1079 and SERP2458 |
| *csm5* | • Subtype III-A | *csm5* | NA | COG1332 (RAMP) | MTH1078 and SERP2457 |
| *csm6* | • Subtype III-A | APE2256 and *csm6* | 2WTE | COG1517 | APE2256 and SSO1445 |
| *cmr1* | • Subtype III-B | *cmr1* | NA | COG1367 (RAMP) | PF1130 |
| *cmr3* | • Subtype III-B | *cmr3* | NA | COG1769 (RAMP) | PF1128 |
| *cmr4* | • Subtype III-B | *cmr4* | NA | COG1336 (RAMP) | PF1126 |
| *cmr5* | • Subtype III-B^{‡‡} | *cmr5* | 2ZOP and 2OEB | COG3337 | MTH324 and PF1125 |
| *cmr6* | • Subtype III-B | *cmr6* | NA | COG1604 (RAMP) | PF1124 |
| *csb1* | • Subtype I-U | GSU0053 | NA | (RAMP) | Balac_1306 and GSU0053 |
| *csb2* | • Subtype I-U^{§§} | NA | NA | (RAMP) | Balac_1305 and GSU0054 |
| *csb3* | • Subtype I-U | NA | NA | (RAMP) | Balac_1303^{§§} |
| *csxl7* | • Subtype I-U | NA | NA | NA | Btus_2683 |
| *csx14* | • Subtype I-U | NA | NA | NA | GSU0052 |
| *csx10* | • Subtype I-U | *csx10* | NA | (RAMP) | Caur_2274 |
| *csx16* | • Subtype III-U | VVA1548 | NA | NA | VVA1548 |
| *csaX* | • Subtype III-U | *csaX* | NA | NA | SSO1438 |
| *csx3* | • Subtype III-U | *csx3* | NA | NA | AF1864 |
| *csx1* | • Subtype III-U | *csa3, csx1, csx2,* DXTHG, NE0113 and TIGR02710 | 1XMX and 2I71 | COG1517 and COG4006 | MJ1666, NE0113, PF1127 and TM1812 |
| *csx15* | • Unknown | NA | NA | TTE2665 | TTE2665 |
| *csf1* | • Type U | *csf1* | NA | NA | AFE_1038 |
| *csf2* | • Type U | *csf2* | NA | (RAMP) | AFE_1039 |
| *csf3* | • Type U | *csf3* | NA | (RAMP) | AFE_1040 |
| *csf4* | • Type U | *csf4* | NA | NA | AFE_1037 |

### IV. Functional Analysis of Candidate Molecules

Candidate Cas9 molecules, candidate gRNA molecules, candidate Cas9 molecule/gRNA molecule complexes, can be evaluated by art-known methods or as described herein. For example, exemplary methods for evaluating the endonuclease activity of Cas9 molecule are described, e.g., in Jinek et al., SCIENCE 2012, 337(6096):816-821.

### Binding and Cleavage Assay: Testing the endonuclease activity of Cas9 molecule

The ability of a Cas9 molecule/gRNA molecule complex to bind to and cleave a target nucleic acid can be evaluated in a plasmid cleavage assay. In this assay, synthetic or *in vitro-*transcribed gRNA molecule is pre-annealed prior to the reaction by heating to 95°C and slowly cooling down to room temperature. Native or restriction digest-linearized plasmid DNA (300 ng (∼8 nM)) is incubated for 60 min at 37°C with purified Cas9 protein molecule (50-500 nM) and gRNA (50-500 nM, 1:1) in a Cas9 plasmid cleavage buffer (20 mM HEPES pH 7.5, 150 mM KCl, 0.5 mM DTT, 0.1 mM EDTA) with or without 10 mM MgCl₂. The reactions are stopped with 5X DNA loading buffer (30% glycerol, 1.2% SDS, 250 mM EDTA), resolved by a 0.8 or 1% agarose gel electrophoresis and visualized by ethidium bromide staining. The resulting cleavage products indicate whether the Cas9 molecule cleaves both DNA strands, or only one of the two strands. For example, linear DNA products indicate the cleavage of both DNA strands. Nicked open circular products indicate that only one of the two strands is cleaved.

Alternatively, the ability of a Cas9 molecule/gRNA molecule complex to bind to and cleave a target nucleic acid can be evaluated in an oligonucleotide DNA cleavage assay. In this assay, DNA oligonucleotides (10 pmol) are radiolabeled by incubating with 5 units T4 polynucleotide kinase and -3-6 pmol (∼20-40 mCi) [γ-32P]-ATP in IX T4 polynucleotide kinase reaction buffer at 37°C for 30 min, in a 50 µL reaction. After heat inactivation (65°C for 20 min), reactions are purified through a column to remove unincorporated label. Duplex substrates (100 nM) are generated by annealing labeled oligonucleotides with equimolar amounts of unlabeled complementary oligonucleotide at 95°C for 3 min, followed by slow cooling to room temperature. For cleavage assays, gRNA molecules are annealed by heating to 95°C for 30 s, followed by slow cooling to room temperature. Cas9 (500 nM final concentration) is pre-incubated with the annealed gRNA molecules (500 nM) in cleavage assay buffer (20 mM HEPES pH 7.5, 100 mM KCl, 5 mM MgCl2, 1 mM DTT, 5% glycerol) in a total volume of 9 µl. Reactions are initiated by the addition of 1 µl target DNA (10 nM) and incubated for 1 h at 37°C. Reactions are quenched by the addition of 20 µl of loading dye (5 mM EDTA, 0.025% SDS, 5% glycerol in formamide) and heated to 95°C for 5 min. Cleavage products are resolved on 12% denaturing polyacrylamide gels containing 7 M urea and visualized by phosphorimaging. The resulting cleavage products indicate that whether the complementary strand, the non-complementary strand, or both, are cleaved.

One or both of these assays can be used to evaluate the suitability of a candidate gRNA molecule or candidate Cas9 molecule.

### Binding Assay: Testing the binding of Cas9 molecule to target DNA

Exemplary methods for evaluating the binding of Cas9 molecule to target DNA are described, e.g., in Jinek et al., SCIENCE 2012; 337(6096):816-821.

For example, in an electrophoretic mobility shift assay, target DNA duplexes are formed by mixing of each strand (10 nmol) in deionized water, heating to 95°C for 3 min and slow cooling to room temperature. All DNAs are purified on 8% native gels containing IX TBE. DNA bands are visualized by UV shadowing, excised, and eluted by soaking gel pieces in DEPC-treated H₂O. Eluted DNA is ethanol precipitated and dissolved in DEPC-treated H₂O. DNA samples are 5' end labeled with [γ-32P]-ATP using T4 polynucleotide kinase for 30 min at 37°C. Polynucleotide kinase is heat denatured at 65°C for 20 min, and unincorporated radiolabel is removed using a column. Binding assays are performed in buffer containing 20 mM HEPES pH 7.5, 100 mM KCl, 5 mM MgCl₂, 1 mM DTT and 10% glycerol in a total volume of 10 µl. Cas9 protein molecule is programmed with equimolar amounts of pre-annealed gRNA molecule and titrated from 100 pM to 1 µM. Radiolabeled DNA is added to a final concentration of 20 pM. Samples are incubated for 1 h at 37°C and resolved at 4°C on an 8% native polyacrylamide gel containing IX TBE and 5 mM MgCl₂. Gels are dried and DNA visualized by phosphorimaging.

### Differential Scanning Flourimetry (DSF)

The thermostability of Cas9-gRNA ribonucleoprotein (RNP) complexes can be measured via DSF. This technique measures the thermostability of a protein, which can increase under favorable conditions such as the addition of a binding RNA molecule, e.g., a gRNA.

The assay is performed using two different protocols, one to test the best stoichiometric ratio of gRNA:Cas9 protein and another to determine the best solution conditions for RNP formation.

To determine the best solution to form RNP complexes, a 2uM solution of Cas9 in water+10x SYPRO Orange® (Life Techonologies cat#S-6650) and dispensed into a 384 well plate. An equimolar amount of gRNA diluted in solutions with varied pH and salt is then added. After incubating at room temperature for 10' and brief centrifugation to remove any bubbles,a Bio-Rad CFX384™ Real-Time System C1000 Touch™ Thermal Cycler with the Bio-Rad CFX Manager software is used to run a gradient from 20°C to 90°C with a 1° increase in temperature every 10seconds.

The second assay consists of mixing various concentrations of gRNA with 2uM Cas9 in optimal buffer from assay 1 above and incubating at RT for 10' in a 384 well plate. An equal volume of optimal buffer + 10x SYPRO Orange® (Life Techonologies cat#S-6650) is added and the plate sealed with Microseal® B adhesive (MSB-1001). Following brief centrifugation to remove any bubbles, a Bio-Rad CFX384™ Real-Time System C1000 Touch™ Thermal Cycler with the Bio-Rad CFX Manager software is used to run a gradient from 20°C to 90°C with a 1° increase in temperature every 10seconds.

### V. Genome Editing Approaches

Mutations in the *MYOC* gene may be corrected using one of the approaches or pathways described herein, e.g., using HDR and/or NHEJ. In an embodiment, a mutation or a mutational hotspot in the *MYOC* gene is corrected by homology directed repair (HDR) using a template nucleic acid (see Section V.1).

Also described herein are methods for targeted knockout of one or both alleles of the *MYOC* gene using NHEJ (see Section V.2). In another embodiment, methods are provided for targeted knockdown of the *MYOC* gene (see Section V.3).

### V.1 HDR Repair and Template Nucleic Acids

As described herein, nuclease-induced homology directed repair (HDR) can be used to alter a target sequence and correct (e.g., repair or edit) a mutation in the genome. While not wishing to be bound by theory, it is believed that alteration of the target sequence occurs by homology-directed repair (HDR) with a donor template or template nucleic acid. For example, the donor template or the template nucleic acid provides for alteration of the target sequence. It is contemplated that a plasmid donor can be used as a template for homologous recombination. It is further contemplated that a single stranded donor template can be used as a template for alteration of the target sequence by alternate methods of homology directed repair (e.g., single strand annealing) between the target sequence and the donor template. Donor template-effected alteration of a target sequence depends on cleavage by a Cas9 molecule. Cleavage by Cas9 can comprise a double strand break or two single strand breaks.

Mutations that can be corrected by HDR using a template nucleic acid include point mutations, mutation hotspots or sequence insertions. In an embodiment, a point mutation or a mutation hotspot (e.g., a mutation hotspot of less than about 30 bp, e.g., less than 25, 20, 15, 10 or 5 bp) can be corrected by either a single double-strand break or two single strand breaks. In an embodiment, a mutation hotspot (e.g., a mutation hotspot greater than about 30 bp, e.g., more than 35, 40, 45, 50, 75, 100, 150, 200, 250, 300, 400 or 500 bp) or an insertion can be corrected by (1) a single double-strand break, (2) two single strand breaks, (3) two double stranded breaks with a break occurring on each side of the target sequence, or (4) four single stranded breaks with a pair of single stranded breaks occurring on each side of the target sequence.

Mutations in the *MYOC* gene that can be corrected (e.g., altered) by HDR with a template nucleic acid include point mutations at T377R, P370L, I477N and/or mutational hotspots at amino acids 423-437, amino acids 246-252, or amino acids 477-502.

### Double strand break mediated correction

In an embodiment, double strand cleavage is effected by a Cas9 molecule having cleavage activity associated with an HNH-like domain and cleavage activity associated with anRuvC-like domain, e.g., an N-terminal RuvC-like domain, e.g., a wild type Cas9. Such embodiments require only a single gRNA.

### Single strand break mediated correction

In other embodiments, two single strand breaks, or nicks, are effected by a Cas9 molecule having nickase activity, e.g., cleavage activity associated with an HNH-like domain or cleavage activity associated with an N-terminal RuvC-like domain. Such embodiments require two gRNAs, one for placement of each single strand break. In an embodiment, the Cas9 molecule having nickase activity cleaves the strand to which the gRNA hybridizes, but not the strand that is complementary to the strand to which the gRNA hybridizes. In an embodiment, the Cas9 molecule having nickase activity does not cleave the strand to which the gRNA hybridizes, but rather cleaves the strand that is complementary to the strand to which the gRNA hybridizes.

In an embodiment, the nickase has HNH activity, e.g., a Cas9 molecule having the RuvC activity inactivated, e.g., a Cas9 molecule having a mutation at D10, e.g., the D10A mutation. D10A inactivates RuvC; therefore, the Cas9 nickase has (only) HNH activity and will cut on the strand to which the gRNA hybridizes (the complementary strand, which does not have the NGG PAM on it). In other embodiments, a Cas9 molecule having an H840, e.g., an H840A, mutation can be used as a nickase. H840A inactivates HNH; therefore, the Cas9 nickase has (only) RuvC activity and cuts on the non-complementary strand (the strand that has the NGG PAM and whose sequence is identical to the gRNA). In other embodiments, a Cas9 molecule having an N863, e.g., an N863A mutation, can be used as a nickase. N863A inactivates HNH therefore the Cas9 nickase has (only) RuvC activity and cuts on the non-complementary strand (the strand that has the NGG PAM and whose sequence is identical to the gRNA).

In an embodiment, in which a nickase and two gRNAs are used to position two single strand nicks, one nick is on the + strand and one nick is on the - strand of the target nucleic acid. The PAMs are outwardly facing. The gRNAs can be selected such that the gRNAs are separated by, from about 0-50, 0-100, or 0-200 nucleotides. In an embodiment, there is no overlap between the target sequence that is complementary to the targeting domains of the two gRNAs. In an embodiment, the gRNAs do not overlap and are separated by as much as 50, 100, or 200 nucleotides. In an embodiment, the use of two gRNAs can increase specificity, e.g., by decreasing off-target binding (Ran et al., Cell 2013; 154(6):1380-1389).

In an embodiment, a single nick can be used to induce HDR. It is contemplated herein that a single nick can be used to increase the ratio of HR to NHEJ at a given cleavage site.

### Placement of double strand or single strand breaks relative to the target position

The double strand break or single strand break in one of the strands should be sufficiently close to the target position such that correction occurs. In an embodiment, the distance is not more than 50, 100, 200, 300, 350 or 400 nucleotides. While not wishing to be bound by theory, it is believed that the break should be sufficiently close to the target sequence such that the break is within the region that is subject to exonuclease-mediated removal during end resection. If the distance between the target sequence and a break is too great, the mutation may not be included in the end resection and, therefore, may not be corrected, as donor sequence may only be used to correct sequence within the end resection region.

In an embodiment, in which a gRNA (unimolecular (or chimeric) or modular gRNA) and Cas9 nuclease induce a double strand break for the purpose of inducing HDR-mediated correction, the cleavage site is between 0-200 bp (e.g., 0 to 175, 0 to 150, 0 to 125, 0 to 100, 0 to 75, 0 to 50, 0 to 25, 25 to 200, 25 to 175, 25 to 150, 25 to 125, 25 to 100, 25 to 75, 25 to 50, 50 to 200, 50 to 175, 50 to 150, 50 to 125, 50 to 100, 50 to 75, 75 to 200, 75 to 175, 75 to 150, 75 to 125, 75 to 100 bp) away from the target position. In an embodiment, the cleavage site is between 0-100 bp (e.g., 0 to 75, 0 to 50, 0 to 25, 25 to 100, 25 to 75, 25 to 50, 50 to 100, 50 to 75 or 75 to 100 bp) away from the target position.

In an embodiment, in which two gRNAs (independently, unimolecular (or chimeric) or modular gRNA) complexing with Cas9 nickases induce two single strand breaks for the purpose of inducing HDR-mediated correction, the closer nick is between 0-200 bp (e.g., 0 to 175, 0 to 150, 0 to 125, 0 to 100, 0 to 75, 0 to 50, 0 to 25, 25 to 200, 25 to 175, 25 to 150, 25 to 125, 25 to 100, 25 to 75, 25 to 50, 50 to 200, 50 to 175, 50 to 150, 50 to 125, 50 to 100, 50 to 75, 75 to 200, 75 to 175, 75 to 150, 75 to 125, 75 to 100 bp) away from the target position and the two nicks will ideally be within 25-55 bp of each other (e.g., 25 to 50, 25 to 45, 25 to 40, 25 to 35, 25 to 30, 30 to 55, 30 to 50, 30 to 45, 30 to 40, 30 to 35, 35 to 55, 35 to 50, 35 to 45, 35 to 40, 40 to 55, 40 to 50, 40 to 45 bp) and no more than 100 bp away from each other (e.g., no more than 90, 80, 70, 60, 50, 40, 30, 20, 10 or 5 bp away from each other). In an embodiment, the cleavage site is between 0-100 bp (e.g., 0 to 75, 0 to 50, 0 to 25, 25 to 100, 25 to 75, 25 to 50, 50 to 100, 50 to 75 or 75 to 100 bp) away from the target position.

In one embodiment, two gRNAs, e.g., independently, unimolecular (or chimeric) or modular gRNA, are configured to position a double-strand break on both sides of a target position. In an alternate embodiment, three gRNAs, e.g., independently, unimolecular (or chimeric) or modular gRNA, are configured to position a double strand break (i.e., one gRNA complexes with a cas9 nuclease) and two single strand breaks or paired single stranded breaks (i.e., two gRNAs complex with Cas9 nickases) on either side of the target position. In another embodiment, four gRNAs, e.g., independently, unimolecular (or chimeric) or modular gRNA, are configured to generate two pairs of single stranded breaks (i.e., two pairs of two gRNAs complex with Cas9 nickases) on either side of the target position. The double strand break(s) or the closer of the two single strand nicks in a pair will ideally be within 0-500 bp of the target position (e.g., no more than 450, 400, 350, 300, 250, 200, 150, 100, 50 or 25 bp from the target position). When nickases are used, the two nicks in a pair are within 25-55 bp of each other (e.g., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50 , 45 to 50, 35 to 45, or 40 to 45 bp) and no more than 100 bp away from each other (e.g., no more than 90, 80, 70, 60, 50, 40, 30, 20 or 10 bp). In an embodiment, the gRNAs are configured to place a single strand break on either side of the target position. In an embodiment, the gRNAs are configured to place a single strand break on the same side (either 5' or 3') of the target position.

Regardless of whether a break is a double strand or a single strand break, the gRNA should be configured to avoid unwanted target chromosome elements, such as repeated elements, e.g., an *Alu* repeat, in the target domain. In addition, a break, whether a double strand or a single strand break, should be sufficiently distant from any sequence that should not be altered. For example, cleavage sites positioned within introns should be sufficiently distant from any intron/exon border, or naturally occurring splice signal, to avoid alteration of the exonic sequence or unwanted splicing events.

### Length of the homology arms

The homology arm should extend at least as far as the region in which end resection may occur, e.g., in order to allow the resected single stranded overhang to find a complementary region within the donor template. The overall length could be limited by parameters such as plasmid size or viral packaging limits. In an embodiment, a homology arm does not extend into repeated elements, e.g., Alu repeats, LINE repeats.

Exemplary homology arm lengths include a least 50, 100, 250, 500, 750 or 1000 nucleotides.

Target position, as used herein, refers to a site on a target nucleic acid (e.g., the chromosome) that is modified by a Cas9 molecule-dependent process. For example, the target position can be a modified Cas9 molecule cleavage of the target nucleic acid and template nucleic acid directed modification, e.g., correction, of the target position. In an embodiment, a target position can be a site between two nucleotides, e.g., adjacent nucleotides, on the target nucleic acid into which one or more nucleotides is added. The target position may comprise one or more nucleotides that are altered, e.g., corrected, by a template nucleic acid. In an embodiment, the target position is within a target sequence (e.g., the sequence to which the gRNA binds). In an embodiment, a target position is upstream or downstream of a target sequence (e.g., the sequence to which the gRNA binds).

A template nucleic acid, as that term is used herein, refers to a nucleic acid sequence which can be used in conjunction with a Cas9 molecule and a gRNA molecule to alter the structure of a target position. In an embodiment, the target nucleic acid is modified to have the some or all of the sequence of the template nucleic acid, typically at or near cleavage site(s). In an embodiment, the template nucleic acid is single stranded. In an alternate embodiment, the template nucleic acid is double stranded. In an embodiment, the template nucleic acid is DNA, e.g., double stranded DNA. In an alternate embodiment, the template nucleic acid is single stranded DNA. In an embodiment, the template nucleic acid is encoded on the same vector backbone, e.g. AAV genome, plasmid DNA, as the Cas9 and gRNA. In an embodiment, the template nucleic acid is excised from a vector backbone *in vivo,* e.g., it is flanked by gRNA recognition sequences.

In an embodiment, the template nucleic acid alters the structure of the target position by participating in a homology directed repair event. In an embodiment, the template nucleic acid alters the sequence of the target position. In an embodiment, the template nucleic acid results in the incorporation of a modified, or non-naturally occurring base into the target nucleic acid.

Typically, the template sequence undergoes a breakage mediated or catalyzed recombination with the target sequence. In an embodiment, the template nucleic acid includes sequence that corresponds to a site on the target sequence that is cleaved by an eaCas9 mediated cleavage event. In an embodiment, the template nucleic acid includes sequence that corresponds to both, a first site on the target sequence that is cleaved in a first Cas9 mediated event, and a second site on the target sequence that is cleaved in a second Cas9 mediated event.

In an embodiment, the template nucleic acid can include sequence which results in an alteration in the coding sequence of a translated sequence, e.g., one which results in the substitution of one amino acid for another in a protein product, e.g., transforming a mutant allele into a wild type allele, transforming a wild type allele into a mutant allele, and/or introducing a stop codon, insertion of an amino acid residue, deletion of an amino acid residue, or a nonsense mutation.

In another embodiment, the template nucleic acid can include sequence which results in an alteration in a non-coding sequence, e.g., an alteration in an exon or in a 5' or 3' non-translated or non-transcribed region. Such alterations include an alteration in a control element, e.g., a promoter, enhancer, and an alteration in a cis-acting or trans-acting control element.

A template nucleic acid having homology with a target position in the *MYOC* gene can be used to alter the structure of a target sequence. The template sequence can be used to alter an unwanted structure, e.g., an unwanted or mutant nucleotide.

A template nucleic acid comprises the following components:
[5' homology arm]-[replacement sequence]-[3' homology arm].

The homology arms provide for recombination into the chromosome, thus replacing the undesired element, e.g., a mutation or signature, with the replacement sequence. In an embodiment, the homology arms flank the most distal cleavage sites.

In an embodiment, the 3' end of the 5' homology arm is the position next to the 5' end of the replacement sequence. In an embodiment, the 5' homology arm can extend at least 10, 20, 30, 40, 50, 100, 200, 300, 400, 500, 600, 700, 800, 900, 1000, 1500, or 2000 nucleotides 5' from the 5' end of the replacement sequence.

In an embodiment, the 5' end of the 3' homology arm is the position next to the 3' end of the replacement sequence. In an embodiment, the 3' homology arm can extend at least 10, 20, 30, 40, 50, 100, 200, 300, 400, 500, 600, 700, 800, 900, 1000, 1500, or 2000 nucleotides 3' from the 3' end of the replacement sequence.

### Exemplary Template Nucleic Acids

Exemplary template nucleic acids (also referred to herein as donor constructs) to correction a mutation, e.g., P370L, in the *MYOC* gene, are provided.

Suitable sequence for the 5' homology arm for a template nucleic acid to correct a P370L mutation in the *MYOC* gene can include the following sequence or a portion thereof:

TTGCACCACTGCACTCCAGCCTAGGTAACAGTGCAAGACCCTGTCTCAAAAAATAATTATTT
TCATGTTTATTATATTAAAATGATGTATGAAATATGTGACTCATCAGGGCTTGAAAAACTTT
GTTGTATGGAGATTATTCTTATGAGTTGATTTTTCTCTCCTACCTTATAGTAATGAAATA
AACCAGGCATGAAAGTCACAATAAGTAATACAATGAACACCCATGGGTCCCTGCCCAGCTTA
AGTAGAATATTACAAATGCAGTTGAAGCCCTCTGTGCAACTTTCATCCTTACAACTGATACT
GAGTGAATTGTACTTTAAATATTTTATAGCTCCCACTCCCATGCATGCCCCTCAGTGATAGC
AATAATTGTCAATAACATGAAACACAGATTGATCATATAGCATTTACCATATATTTACTCTA
TACCAAGCACTTAACATATATAATTACATTTAAAATTTACAACAGCCCTACTACCCAAAACA
CTATTAGTATCCCCTTTTACAAATGCGATAACTGAGGCGTAGAGAGCTAAGTAACTTACTGA
AAGTCACACAGCCAGCGGGTGGTAGAGCCTAGCTTTAAACCCAGACGATTTGTCTCCAGGGC

TGTCACATCTACTGGCTCTGCCAAGCTTCCGCATGATCATTGTCTGTGTTTGGAAAGATTAT
GGATTAAGTGGTGCTTCGTTTTCTTTTCTGAATTTACCAGGATGTGGAGAACTAGTTTGGGT
AGGAGAGCCTCTCACGCTGAGAACAGCAGAAACAATTACTGGCAAGTATGGTGTGGATGC
GAGACCCCAAGCCCACCTACCCCTACACCCAGGAGACCACGTGGAGAATCGACACAGTTGGC
ACGGATGTCCGCCAGGTTTTTGAGTATGACCTCATCAGCCAGTTTATGCAGGGCTACCCTTC
TAAGGTTCACATACTGCCTAGGCCACTGGAAAGCACGGGTGCTGTGGTGTACTCGGGGAGCC
TCTATTTCCAGGGCGCTGAGTCCAGAACTGTCATAAGATATGAGCTGAATACCGAGACAGTG
AAGGCTGAGAAGGAAATCCCTGGAGCTGGCTACCACGGACAGTTCC (SEQ ID NO:
8856)

Suitable sequence for the 3' homology arm for a template nucleic acid to correct P370L mutation in the *MYOC* gene can include the following sequence or a portion thereof:

GTATTCTTGGGGTGGCTACACGGACATTGACTTGGCTGTGGATGAAGCAGGCCTCTGGGTCA
TTTACAGCACCGATGAGGCCAAAGGTGCCATTGTCCTCTCCAAACTGAACCCAGAGAATCTG
GAACTCGAACAAACCTGGGAGACAAACATCCGTAAGCAGTCAGTCGCCAATGCCTTCATCAT
CTGTGGCACCTTGTACACCGTCAGCAGCTACACCTCAGCAGATGCTACCGTCAACTTTGCTT
ATGACACAGGCACAGGTATCAGCAAGACCCTGACCATCCCATTCAAGAACCGCTATAAGTAC
AGCAGCATGATTGACTACAACCCCCTGGAGAAGAAGCTCTTTGCCTGGGACAACTTGAACAT
GGTCACTTATGACATCAAGCTCTCCAAGATGTGAAAAGCCTCCAAGCTGTACAGGCAATGGC
AGAAGGAGATGCTCAGGGCTCCTGGGGGAGCAGGCTGAAGGGAGAGCCAGCCAGCCAGGGC
CCAGGCAGCTTTGACTGCTTTCCAAGTTTTCATTAATCCAGAAGGATGAACATGGTCACCAT
CTAACTATTCAGGAATTGTAGTCTGAGGGCGTAGACAATTTCATATAATAAATATCCTTTAT
CTTCTGTCAGCATTTATGGGATGTTTAATGACATAGTTCAAGTTTTCTTGTGATTTGGGGCA
AAAGCTGTAAGGCATAATAGTTTCTTCCTGAAAACCATTGCTCTTGCATGTTACATGGTTAC
CACAAGCCACAATAAAAAGCATAACTTCTAAAGGAAGCAGAATAGCTCCTCTGGCCAGCATC
GAATATAAGTAAGATGCATTTACTACAGTTGGCTTCTAATGCTTCAGATAGAATACAGTTGG
GTCTCACATAACCCTTTACATTGTGAAATAAAATTTTCTTACCCAACGTTCTCTTCCTTGAA
CTTTGTGGGAATCTTTGCTTAAGAGAAGGATATAGATTCCAACCATCAGGTAATTCCTTCAG
GTTGGGAGATGTGATTGCAGGATGTTAAAGGTGGTGTGTGTGTGTGTGTGTGTGTGTGTAAC
TGAGAGGCTTGTGCCTGGTTTTGAGGTGCTGCCCAGGATGACGCCAA (SEQ ID NO:
8857)

In an embodiment, the replacement sequence comprises or consists of a cytosine (C) residue.

In an embodiment, to correct P370L in the *MYOC* gene, the homology arms, e.g., the 5' and 3' homology arms, may each comprise about 1000 base pairs (bp) of sequence flanking the most distal gRNAs (e.g., 1100bp of sequence on either side of the mutation). The 5' homology arm is shown as bold sequence, codon 370 is shown as underlined sequence, the inserted base to correct the P370L mutation is shown as boxed sequence, and the 3' homology arm is shown with no emphasis sequence.

TTGCACCACTGCACTCCAGCCTAGGTAACAGTGCAAGACCCTGTCTCAAAAAATAATTATTT
TCATGTTTATTATATTAAAATGATGTATGAAATATGTGACTCATCAGGGCTTGAAAAACTTT
GTTGTATGGAGATTATTCTTATGAGTTGATTTTTCTCTCCTACCTTATAGTAATGAAATA
AACCAGGCATGAAAGTCACAATAAGTAATACAATGAACACCCATGGGTCCCTGCCCAGCTTA
AGTAGAATATTACAAATGCAGTTGAAGCCCTCTGTGCAACTTTCATCCTTACAACTGATACT
GAGTGAATTGTACTTTAAATATTTTATAGCTCCCACTCCCATGCATGCCCCTCAGTGATAGC
AATAATTGTCAATAACATGAAACACAGATTGATCATATAGCATTTACCATATATTTACTCTA
TACCAAGCACTTAACATATATAATTACATTTAAAATTTACAACAGCCCTACTACCCAAAACA
CTATTAGTATCCCCTTTTACAAATGCGATAACTGAGGCGTAGAGAGCTAAGTAACTTACTGA
AAGTCACACAGCCAGCGGGTGGTAGAGCCTAGCTTTAAACCCAGACGATTTGTCTCCAGGGC
TGTCACATCTACTGGCTCTGCCAAGCTTCCGCATGATCATTGTCTGTGTTTGGAAAGATTAT
GGATTAAGTGGTGCTTCGTTTTCTTTTCTGAATTTACCAGGATGTGGAGAACTAGTTTGGGT
AGGAGAGCCTCTCACGCTGAGAACAGCAGAAACAATTACTGGCAAGTATGGTGTGTGGATGC
GAGACCCCAAGCCCACCTACCCCTACACCCAGGAGACCACGTGGAGAATCGACACAGTTGGC
ACGGATGTCCGCCAGGTTTTTGAGTATGACCTCATCAGCCAGTTTATGCAGGGCTACCCTTC
TAAGGTTCACATACTGCCTAGGCCACTGGAAAGCACGGGTGCTGTGGTGTACTCGGGGAGCC
TCTATTTCCAGGGCGCTGAGTCCAGAACTGTCATAAGATATGAGCTGAATACCGAGACAGTG
AAGGCTGAGAAGGAAATCCCTGGAGCTGGCTACCACGGACAGTTCCCGTATTCTTGGGGTGG
CTACACGGACATTGACTTGGCTGTGGATGAAGCAGGCCTCTGGGTCATTTACAGCACCGATG
AGGCCAAAGGTGCCATTGTCCTCTCCAAACTGAACCCAGAGAATCTGGAACTCGAACAAACC
TGGGAGACAAACATCCGTAAGCAGTCAGTCGCCAATGCCTTCATCATCTGTGGCACCTTGTA
CACCGTCAGCAGCTACACCTCAGCAGATGCTACCGTCAACTTTGCTTATGACACAGGCACAG
GTATCAGCAAGACCCTGACCATCCCATTCAAGAACCGCTATAAGTACAGCAGCATGATTGAC
TACAACCCCCTGGAGAAGAAGCTCTTTGCCTGGGACAACTTGAACATGGTCACTTATGACAT
CAAGCTCTCCAAGATGTGAAAAGCCTCCAAGCTGTACAGGCAATGGCAGAAGGAGATGCTCA
GGGCTCCTGGGGGGAGCAGGCTGAAGGGAGAGCCAGCCAGCCAGGGCCCAGGCAGCTTTGAC
TGCTTTCCAAGTTTTCATTAATCCAGAAGGATGAACATGGTCACCATCTAACTATTCAGGAA
TTGTAGTCTGAGGGCGTAGACAATTTCATATAATAAATATCCTTTATCTTCTGTCAGCATTT
ATGGGATGTTTAATGACATAGTTCAAGTTTTCTTGTGATTTGGGGCAAAAGCTGTAAGGCAT
AATAGTTTCTTCCTGAAAACCATTGCTCTTGCATGTTACATGGTTACCACAAGCCACAATAA

AAAGCATAACTTCTAAAGGAAGCAGAATAGCTCCTCTGGCCAGCATCGAATATAAGTAAGAT
GCATTTACTACAGTTGGCTTCTAATGCTTCAGATAGAATACAGTTGGGTCTCACATAACCCT
TTACATTGTGAAATAAAATTTTCTTACCCAACGTTCTCTTCCTTGAACTTTGTGGGAATCTT
TGCTTAAGAGAAGGATATAGATTCCAACCATCAGGTAATTCCTTCAGGTTGGGAGATGTGAT
TGCAGGATGTTAAAGGTGGTGTGTGTGTGTGTGTGTGTGTGTGTAACTGAGAGGCTTGTGCC
TGGTTTTGAGGTGCTGCCCAGGATGACGCCAA
(Template Construct 1; SEQ ID NO: 8858)

As described below in **Table 24,** shorter homology arms, e.g., 5' and/or 3' homology arms may be used.

It is contemplated herein that one or both homology arms may be shortened to avoid including certain sequence repeat elements, e.g., Alu repeats, LINE elements. For example, a 5' homology arm may be shortened to avoid a sequence repeat element. In other embodiments, a 3' homology arm may be shortened to avoid a sequence repeat element. In some embodiments, both the 5' and the 3' homology arms may be shortened to avoid including certain sequence repeat elements.

In an embodiment, to correct P370L in the *MYOC* gene, the 5' homology arm may be shortened less than 600 nucleotides, e.g., approximately 550 nucleotides, e.g., 450 nucleotides, to avoid inclusion of a LINE repeat element in the 5' homology arm. An exemplary 5' homology arm is shown as bold sequence, codon 370 is shown as underlined sequence, the inserted base to correct the P370L mutation is shown as non-bold and boxed sequence, and an exemplary 3' homology arm is shown with no emphasis.

AAGCTTCCGCATGATCATTGTCTGTGTTTGGAAAGATTATGGATTAAGTGGTGCTTCGTTTT
CTTTTCTGAATTTACCAGGATGTGGAGAACTAGTTTGGGTAGGAGAGCCTCTCACGCTGAGA
ACAGCAGAAACAATTACTGGCAAGTATGGTGTGGATGCGAGACCCCAAGCCCACCTACCC
CTACACCCAGGAGACCACGTGGAGAATCGACACAGTTGGCACGGATGTCCGCCAGGTTTTTG
AGTATGACCTCATCAGCCAGTTTATGCAGGGCTACCCTTCTAAGGTTCACATACTGCCTAGG
CCACTGGAAAGCACGGGTGCTGTGGTGTACTCGGGGAGCCTCTATTTCCAGGGCGCTGAGTC
CAGAACTGTCATAAGATATGAGCTGAATACCGAGACAGTGAAGGCTGAGAAGGAAATCCCTG
GAGCTGGCTACCACGGACAGTTCCCGTATTCTTGGGGTGGCTACACGGACATTGACTTGGCT
GTGGATGAAGCAGGCCTCTGGGTCATTTACAGCACCGATGAGGCCAAAGGTGCCATTGTCCT
CTCCAAACTGAACCCAGAGAATCTGGAACTCGAACAAACCTGGGAGACAAACATCCGTAAGC
AGTCAGTCGCCAATGCCTTCATCATCTGTGGCACCTTGTACACCGTCAGCAGCTACACCTCA
GCAGATGCTACCGTCAACTTTGCTTATGACACAGGCACAGGTATCAGCAAGACCCTGACCAT
CCCATTCAAGAACCGCTATAAGTACAGCAGCATGATTGACTACAACCCCCTGGAGAAGAAGC

TCTTTGCCTGGGACAACTTGAACATGGTCACTTATGACATCAAGCTCTCCAAGATGTGAAAA
GCCTCCAAGCTGTACAGGCAATGGCAGAAGGAGATGCTCAGGGCTCCTGGGGGAGCAGGCT
GAAGGGAGAGCCAGCCAGCCAGGGCCCAGGCAGCTTTGACTGCTTTCCAAGTTTTCATTAAT
CCAGAAGGATGAACATGGTCACCATCTAACTATTCAGGAATTGTAGTCTGAGGGCGTAGACA
ATTTCATATAATAAATATCCTTTATCTTCTGTCAGCATTTATGGGATGTTTAATGACATAGT
TCAAGTTTTCTTGTGATTTGGGGCAAAAGCTGTAAGGCATAATAGTTTCTTCCTGAAAACCA
TTGCTCTTGCATGTTACATGGTTACCACAAGCCACAATAAAAAGCATAACTTCTAAAGGAAG
CAGAATAGCTCCTCTGGCCAGCATCGAATATAAGTAAGATGCATTTACTACAGTTGGCTTCT
AATGCTTCAGATAGAATACAGTTGGGTCTCACATAACCCTTTACATTGTGAAATAAAATTTT
CTTACCCAACGTTCTCTTCCTTGAACTTTGTGGGAATCTTTGCTTAAGAGAAGGATATAGAT
TCCAACCATCAGGTAATTCCTTCAGGTTGGGAGATGTGATTGCAGGATGTTAAAGGTGGTGT
GTGTGTGTGTGTGTGTGTGTGTGTAACTGAGAGGCTTGTGCCTGGTTTTGAGGTGCTGCCCAGG
ATGACGCCAA
(Template Construct 2; SEQ ID NO: 8859)

It is contemplated herein that, in an embodiment, template nucleic acids for correcting a mutation may designed for use as a single-stranded oligonucleotide (ssODN). When using a ssODN, 5' and 3' homology arms may range up to about 200 base pairs (bp) in length, e.g., at least 25, 50, 75, 100, 125, 150, 175, or 200 bp in length. Longer homology arms are also contemplated for ssODNs as improvements in oligonucleotide synthesis continue to be made.

Exemplary template nucleic acids to correct a mutation, e.g., I477N or mutations in the mutational hotspot 477-502 region, in the *MYOC* gene, are provided.

Suitable sequence for the 5' homology arm for a template nucleic acid to correct an I477N mutation or mutations in the mutational hotspot 477-502 region in the *MYOC* gene can include the following sequence or a portion thereof:

GAACACCCATGGGTCCCTGCCCAGCTTAAGTAGAATATTACAAATGCAGTTGAAGCCCTCTG
TGCAACTTTCATCCTTACAACTGATACTGAGTGAATTGTACTTTAAATATTTTATAGCTCCC
ACTCCCATGCATGCCCCTCAGTGATAGCAATAATTGTCAATAACATGAAACACAGATTGATC
ATATAGCATTTACCATATATTTACTCTATACCAAGCACTTAACATATATAATTACATTTAAA
ATTTACAACAGCCCTACTACCCAAAACACTATTAGTATCCCCTTTTACAAATGCGATAACTG
AGGCGTAGAGAGCTAAGTAACTTACTGAAAGTCACACAGCCAGCGGGTGGTAGAGCCTAGCT
TTAAACCCAGACGATTTGTCTCCAGGGCTGTCACATCTACTGGCTCTGCCAAGCTTCCGCAT
GATCATTGTCTGTGTTTGGAAAGATTATGGATTAAGTGGTGCTTCGTTTTCTTTTCTGAATT
TACCAGGATGTGGAGAACTAGTTTGGGTAGGAGAGCCTCTCACGCTGAGAACAGCAGAAACA

ATTACTGGCAAGTATGGTGTGTGGATGCGAGACCCCAAGCCCACCTACCCCTACACCCAGGA
GACCACGTGGAGAATCGACACAGTTGGCACGGATGTCCGCCAGGTTTTTGAGTATGACCTCA
TCAGCCAGTTTATGCAGGGCTACCCTTCTAAGGTTCACATACTGCCTAGGCCACTGGAAAGC
ACGGGTGCTGTGGTGTACTCGGGGAGCCTCTATTTCCAGGGCGCTGAGTCCAGAACTGTCAT
AAGATATGAGCTGAATACCGAGACAGTGAAGGCTGAGAAGGAAATCCCTGGAGCTGGCTACC
ACGGACAGTTCCCGTATTCTTGGGGTGGCTACACGGACATTGACTTGGCTGTGGATGAAGCA
GGCCTCTGGGTCATTTACAGCACCGATGAGGCCAAAGGTGCCATTGTCCTCTCCAAACTGAA
CCCAGAGAATCTGGAACTCGAACAAACCTGGGAGACAAACATCCGTAAGCAGTCAGTCGCCA
ATGCCTTCATCATCTGTGGCACCTTGTACACCGTCAGCAGCTACACCTCAGCAGATGCTACC
GTCAACTTTGCTTATGACACAGGCACAGGTATCAGCAAGACCCTGACCATCCCATTCAAGAA
CCGCTATAAGTACAGCAGCATGA (SEQ ID NO: 8860)

Suitable sequence for the 3' homology arm for a template nucleic acid to correct an I477N mutation or mutations in the mutational hotspot 477-502 region in the *MYOC* gene can include the following sequence or a portion thereof:

AAGATGTGAAAAGCCTCCAAGCTGTACAGGCAATGGCAGAAGGAGATGCTCAGGGCTCCTGG
GGGGAGCAGGCTGAAGGAGAGCCAGCCAGCCAGGGCCCAGGCAGCTTTGACTGCTTTCCAA
GTTTTCATTAATCCAGAAGGATGAACATGGTCACCATCTAACTATTCAGGAATTGTAGTCTG
AGGGCGTAGACAATTTCATATAATAAATATCCTTTATCTTCTGTCAGCATTTATGGGATGTT
TAATGACATAGTTCAAGTTTTCTTGTGATTTGGGGCAAAAGCTGTAAGGCATAATAGTTTCT
TCCTGAAAACCATTGCTCTTGCATGTTACATGGTTACCACAAGCCACAATAAAAAGCATAAC
TTCTAAAGGAAGCAGAATAGCTCCTCTGGCCAGCATCGAATATAAGTAAGATGCATTTACTA
CAGTTGGCTTCTAATGCTTCAGATAGAATACAGTTGGGTCTCACATAACCCTTTACATTGTG
AAATAAAATTTTCTTACCCAACGTTCTCTTCCTTGAACTTTGTGGGAATCTTTGCTTAAGAG
AAGGATATAGATTCCAACCATCAGGTAATTCCTTCAGGTTGGGAGATGTGATTGCAGGATGT
TAAAGGTGGTGTGTGTGTGTGTGTGTGTGTGTAACTGAGAGGCTTGTGCCTGGTTTTGAG
GTGCTGCCCAGGATGACGCCAAGCAAATAGCAGCATCCACACTTTCCCACCTCCATCTCCTG
GTGCTCTCGGCACTACCGGAGCAATCTTTCCATCTCTCCCCTGAACCCACCCTCTATTCACC
CTAACTCCACTTCAGTTTGCTTTTGATTTTTTTTTTTTTTTTTTTTTTTTTTTGAGATGGAG
TCTCGCTCTGTCACCCAGGCTGGAGTGCAGTGGCACGATCTCGGCTCACTGCAAGTTCCGCC
TCCCAGGTTCACACCATTCTCCTGCCTCAGCCTCCCAAGTAGCTGGGACTACAGGCGCCTGC
CACCACGCCTGGCTAATTTTTTTTTTTTTCCAGTGAAGATGGGGTTTCACCATGTTAGCCAGG
ATGGTCTCGATCTCCTGACCTTGTCATCCACCCACCTTGGCCTCCCAAAGTGCTGGGATTAC
AGGCGTGAGCCACCACGCCCAGCCCCTCCACTTCAGTTTTTATCTGTCATCAGGGGTATGAA
TTTTATAAGCCACAACCTCAGG (SEQ ID NO: 8861)

In an embodiment, when correcting the I477N mutation, the replacement sequence comprises or consists of a thymine (T) residue.

In an embodiment, to correct I477N in the *MYOC* gene, the homology arms, e.g., the 5' and 3' homology arms, may each comprise about 1000 base pairs (bp) of sequence flanking the most distal gRNAs (e.g., 1200bp of sequence on either side of the mutation). The 5' homology arm is shown as bold sequence, codon 477 is shown as underlined sequence, the inserted base to correct the I477N mutation is shown as boxed sequence, and the 3' homology arm is shown as no emphasis sequence.

GAACACCCATGGGTCCCTGCCCAGCTTAAGTAGAATATTACAAATGCAGTTGAAGCCCTCTG
TGCAACTTTCATCCTTACAACTGATACTGAGTGAATTGTACTTTAAATATTTTATAGCTCCC
ACTCCCATGCATGCCCCTCAGTGATAGCAATAATTGTCAATAACATGAAACACAGATTGATC
ATATAGCATTTACCATATATTTACTCTATACCAAGCACTTAACATATATAATTACATTTAAA
ATTTACAACAGCCCTACTACCCAAAACACTATTAGTATCCCCTTTTACAAATGCGATAACTG
AGGCGTAGAGAGCTAAGTAACTTACTGAAAGTCACACAGCCAGCGGGTGGTAGAGCCTAGCT
TTAAACCCAGACGATTTGTCTCCAGGGCTGTCACATCTACTGGCTCTGCCAAGCTTCCGCAT
GATCATTGTCTGTGTTTGGAAAGATTATGGATTAAGTGGTGCTTCGTTTTCTTTTCTGAATT
TACCAGGATGTGGAGAACTAGTTTGGGTAGGAGAGCCTCTCACGCTGAGAACAGCAGAAACA
ATTACTGGCAAGTATGGTGTGTGGATGCGAGACCCCAAGCCCACCTACCCCTACACCCAGGA
GACCACGTGGAGAATCGACACAGTTGGCACGGATGTCCGCCAGGTTTTGAGTATGACCTCA
TCAGCCAGTTTATGCAGGGCTACCCTTCTAAGGTTCACATACTGCCTAGGCCACTGGAAAGC
ACGGGTGCTGTGGTGTACTCGGGGAGCCTCTATTTCCAGGGCGCTGAGTCCAGAACTGTCAT
AAGATATGAGCTGAATACCGAGACAGTGAAGGCTGAGAAGGAAATCCCTGGAGCTGGCTACC
ACGGACAGTTCCCGTATTCTTGGGGTGGCTACACGGACATTGACTTGGCTGTGGATGAAGCA
GGCCTCTGGGTCATTTACAGCACCGATGAGGCCAAAGGTGCCATTGTCCTCTCCAAACTGAA
CCCAGAGAATCTGGAACTCGAACAAACCTGGGAGACAAACATCCGTAAGCAGTCAGTCGCCA
ATGCCTTCATCATCTGTGGCACCTTGTACACCGTCAGCAGCTACACCTCAGCAGATGCTACC
GTCAACTTTGCTTATGACACAGGCACAGGTATCAGCAAGACCCTGACCATCCCATTCAAGAA
CCGCTATAAGTACAGCAGCATGATTATTGACTACAACCCCCTGGAGAAGAAGCTCTTTGCCT
GGGACAACTTGAACATGGTCACTTATGACATCAAGCTCTCCAAGATGTGAAAAGCCTCCAAG
CTGTACAGGCAATGGCAGAAGGAGATGCTCAGGGCTCCTGGGGGAGCAGGCTGAAGGGAGA
GCCAGCCAGCCAGGGCCCAGGCAGCTTTGACTGCTTTCCAAGTTTTCATTAATCCAGAAGGA
TGAACATGGTCACCATCTAACTATTCAGGAATTGTAGTCTGAGGGCGTAGACAATTTCATAT
AATAAATATCCTTTATCTTCTGTCAGCATTTATGGGATGTTTAATGACATAGTTCAAGTTTT
CTTGTGATTTGGGGCAAAAGCTGTAAGGCATAATAGTTTCTTCCTGAAAACCATTGCTCTTG

CATGTTACATGGTTACCACAAGCCACAATAAAAAGCATAACTTCTAAAGGAAGCAGAATAGC
TCCTCTGGCCAGCATCGAATATAAGTAAGATGCATTTACTACAGTTGGCTTCTAATGCTTCA
GATAGAATACAGTTGGGTCTCACATAACCCTTTACATTGTGAAATAAAATTTTCTTACCCAA
CGTTCTCTTCCTTGAACTTTGTGGGAATCTTTGCTTAAGAGAAGGATATAGATTCCAACCAT
CAGGTAATTCCTTCAGGTTGGGAGATGTGATTGCAGGATGTTAAAGGTGGTGTGTGTGTGTG
TGTGTGTGTGTGTAACTGAGAGGCTTGTGCCTGGTTTTGAGGTGCTGCCCAGGATGACGCCA
AGCAAATAGCAGCATCCACACTTTCCCACCTCCATCTCCTGGTGCTCTCGGCACTACCGGAG
CAATCTTTCCATCTCTCCCCTGAACCCACCCTCTATTCACCCTAACTCCACTTCAGTTTGCT
TTTGATTTTTTTTTTTTTTTTTTTTTTTTTTTGAGATGGAGTCTCGCTCTGTCACCCAGGCT
GGAGTGCAGTGGCACGATCTCGGCTCACTGCAAGTTCCGCCTCCCAGGTTCACACCATTCTC
CTGCCTCAGCCTCCCAAGTAGCTGGGACTACAGGCGCCTGCCACCACGCCTGGCTAATTTTT
TTTTTTTCCAGTGAAGATGGGGTTTCACCATGTTAGCCAGGATGGTCTCGATCTCCTGACCT
TGTCATCCACCCACCTTGGCCTCCCAAAGTGCTGGGATTACAGGCGTGAGCCACCACGCCCA
GCCCCTCCACTTCAGTTTTATCTGTCATCAGGGGTATGAATTTTATAAGCCACAACCTCAG
G

### (Template Construct 3; SEQ ID NO: 8862)

In an embodiment, when correcting the mutational hotspot 477-502 region, the replacement sequence comprises or consists of:

TTATTGACTACAACCCCCTGGAGAAGAAGCTCTTTGCCTGGGACAACTTGAACATGGTCACT
TATGACATCAAGCTCTCC (SEQ ID NO: 8863).

It is contemplated herein that one or both homology arms may be shortened to avoid including certain sequence repeat elements, e.g., Alu repeats, LINE elements. For example, a 5' homology arm may be shortened to avoid a sequence repeat element. In other embodiments, a 3' homology arm may be shortened to avoid a sequence repeat element. In some embodiments, both the 5' and the 3' homology arms may be shortened to avoid including certain sequence repeat elements.

It is contemplated herein that, in an embodiment, template nucleic acids for correcting a mutation may designed for use as a single-stranded oligonucleotide (ssODN). When using a ssODN, 5' and 3' homology arms may range up to about 200 base pairs (bp) in length, e.g., at least 25, 50, 75, 100, 125, 150, 175, or 200 bp in length. Longer homology arms are also contemplated for ssODNs as improvements in oligonucleotide synthesis continue to be made.

Exemplary template nucleic acids to correct a mutational hotspot 477-502 region, in the *MYOC* gene, are provided.

In an embodiment, to correct the mutational hotspot 477-502 region in the *MYOC* gene, the homology arms, e.g., the 5' and 3' homology arms, may each comprise about 1000 base pairs (bp) of sequence flanking the most distal gRNAs (e.g., 1200bp of sequence on either side of the mutation). The 5' homology arm is shown as bold sequence, the inserted nucleotides to correct the mutational hotspot 477-502 region is shown as boxed sequence, and the 3' homology arm is shown as no emphasis sequence.

GAACACCCATGGGTCCCTGCCCAGCTTAAGTAGAATATTACAAATGCAGTTGAAGCCCTCTG
TGCAACTTTCATCCTTACAACTGATACTGAGTGAATTGTACTTTAAATATTTTATAGCTCCC
ACTCCCATGCATGCCCCTCAGTGATAGCAATAATTGTCAATAACATGAAACACAGATTGATC
ATATAGCATTTACCATATATTTACTCTATACCAAGCACTTAACATATATAATTACATTTAAA
ATTTACAACAGCCCTACTACCCAAAACACTATTAGTATCCCCTTTTACAAATGCGATAACTG
AGGCGTAGAGAGCTAAGTAACTTACTGAAAGTCACACAGCCAGCGGGTGGTAGAGCCTAGCT
TTAAACCCAGACGATTTGTCTCCAGGGCTGTCACATCTACTGGCTCTGCCAAGCTTCCGCAT
GATCATTGTCTGTGTTTGGAAAGATTATGGATTAAGTGGTGCTTCGTTTTCTTTTCTGAATT
TACCAGGATGTGGAGAACTAGTTTGGGTAGGAGAGCCTCTCACGCTGAGAACAGCAGAAACA
ATTACTGGCAAGTATGGTGTGGATGCGAGACCCCAAGCCCACCTACCCCTACACCCAGGA
GACCACGTGGAGAATCGACACAGTTGGCACGGATGTCCGCCAGGTTTTTGAGTATGACCTCA
TCAGCCAGTTTATGCAGGGCTACCCTTCTAAGGTTCACATACTGCCTAGGCCACTGGAAAGC
ACGGGTGCTGTGGTGTACTCGGGGAGCCTCTATTTCCAGGGCGCTGAGTCCAGAACTGTCAT
AAGATATGAGCTGAATACCGAGACAGTGAAGGCTGAGAAGGAAATCCCTGGAGCTGGCTACC
ACGGACAGTTCCCGTATTCTTGGGGTGGCTACACGGACATTGACTTGGCTGTGGATGAAGCA
GGCCTCTGGGTCATTTACAGCACCGATGAGGCCAAAGGTGCCATTGTCCTCTCCAAACTGAA
CCCAGAGAATCTGGAACTCGAACAAACCTGGGAGACAAACATCCGTAAGCAGTCAGTCGCCA
ATGCCTTCATCATCTGTGGCACCTTGTACACCGTCAGCAGCTACACCTCAGCAGATGCTACC
GTCAACTTTGCTTATGACACAGGCACAGGTATCAGCAAGACCCTGACCATCCCATTCAAGAA
CCGCTATAAGTACAGCAGCATGATTATTGACTACAACCCCCTGGAGAAGAAGCTCTTTGCCT
GGGACAACTTGAACATGGTCACTTATGACATCAAGCTCTCCAAGATGTGAAAAGCCTCCAAG
CTGTACAGGCAATGGCAGAAGGAGATGCTCAGGGCTCCTGGGGGAGCAGGCTGAAGGGAGA
GCCAGCCAGCCAGGGCCCAGGCAGCTTTGACTGCTTTCCAAGTTTTCATTAATCCAGAAGGA
TGAACATGGTCACCATCTAACTATTCAGGAATTGTAGTCTGAGGGCGTAGACAATTTCATAT
AATAAATATCCTTTATCTTCTGTCAGCATTTATGGGATGTTTAATGACATAGTTCAAGTTTT
CTTGTGATTTGGGGCAAAAGCTGTAAGGCATAATAGTTTCTTCCTGAAAACCATTGCTCTTG
CATGTTACATGGTTACCACAAGCCACAATAAAAAGCATAACTTCTAAAGGAAGCAGAATAGC
TCCTCTGGCCAGCATCGAATATAAGTAAGATGCATTTACTACAGTTGGCTTCTAATGCTTCA

GATAGAATACAGTTGGGTCTCACATAACCCTTTACATTGTGAAATAAAATTTTCTTACCCAA
CGTTCTCTTCCTTGAACTTTGTGGGAATCTTTGCTTAAGAGAAGGATATAGATTCCAACCAT
CAGGTAATTCCTTCAGGTTGGGAGATGTGATTGCAGGATGTTAAAGGTGGTGTGTGTGTG
TGTGTGTGTGTGTAACTGAGAGGCTTGTGCCTGGTTTTGAGGTGCTGCCCAGGATGACGCCA
AGCAAATAGCAGCATCCACACTTTCCCACCTCCATCTCCTGGTGCTCTCGGCACTACCGGAG
CAATCTTTCCATCTCTCCCCTGAACCCACCCTCTATTCACCCTAACTCCACTTCAGTTTGCT
TTTGATTTTTTTTTTTTTTTTTTTTTTTTTTGAGATGGAGTCTCGCTCTGTCACCCAGGCT
GGAGTGCAGTGGCACGATCTCGGCTCACTGCAAGTTCCGCCTCCCAGGTTCACACCATTCTC
CTGCCTCAGCCTCCCAAGTAGCTGGGACTACAGGCGCCTGCCACCACGCCTGGCTAATTTTT
TTTTTTTCCAGTGAAGATGGGGTTTCACCATGTTAGCCAGGATGGTCTCGATCTCCTGACCT
TGTCATCCACCCACCTTGGCCTCCCAAAGTGCTGGGATTACAGGCGTGAGCCACCACGCCCA
GCCCCTCCACTTCAGTTTTATCTGTCATCAGGGGTATGAATTTTATAAGCCACAACCTCAG
G

### (Template Construct 3; SEQ ID NO: 8864)

**Table 24** below provides exemplary template nucleic acids. In an embodiment, the template nucleic acid includes the 5' homology arm and the 3' homology arm of a row from **Table 24.** In other embodiments, a 5' homology arm from the first column can be combined with a 3' homology arm from **Table 24.** In each embodiment, a combination of the 5' and 3' homology arms include a replacement sequence, which may be selected from cytosine (C), thymine (T) and

TTATTGACTACAACCCCCTGGAGAAGAAGCTCTTTGCCTGGGACAACTTGAACATGGTCACT
TATGACATCAAGCTCTCCAA (SEQ ID NO: 8865).

**Table 24**

| 5' homology arm (the number of nucleotides from SEQ ID NO: 5'H, beginning at the 3' end of SEQ ID NO: 5'H) | Replacement Sequence: C or T | 3' homology arm (the number of nucleotides from SEQ ID NO: 3'H, beginning at the 5' end of SEQ ID NO: 3'H) |
|---|---|---|
| 10 or more | | 10 or more |
| 20 or more | | 20 or more |
| 50 or more | | 50 or more |
| 100 or more | | 100 or more |
| 150 or more | | 150 or more |
| 200 or more | | 200 or more |
| 250 or more | | 250 or more |
| 300 or more | | 300 or more |
| 350 or more | | 350 or more |
| 400 or more | | 400 or more |
| 450 or more | | 450 or more |
| 500 or more | | 500 or more |
| 550 or more | | 550 or more |
| 600 or more | | 600 or more |
| 650 or more | | 650 or more |
| 700 or more | | 700 or more |
| 750 or more | | 750 or more |
| 800 or more | | 800 or more |
| 850 or more | | 850 or more |
| 900 or more | | 900 or more |
| 1000 or more | | 1000 or more |
| 1100 or more | | 1100 or more |
| 1200 or more | | 1200 or more |
| 1300 or more | | 1300 or more |
| 1400 or more | | 1400 or more |
| 1500 or more | | 1500 or more |
| 1600 or more | | 1600 or more |
| 1700 or more | | 1700 or more |
| 1800 or more | | 1800 or more |
| 1900 or more | | 1900 or more |
| 1200 or more | | 1200 or more |
| At least 50 but not long enough to include a repeated element. | | At least 50 but not long enough to include a repeated element. |
| At least 100 but not long enough to include a repeated element. | | At least 100 but not long enough to include a repeated element. |
| At least 150 but not long enough to include a repeated element. | | At least 150 but not long enough to include a repeated element. |
| 5 to 100 nucleotides | | 5 to 100 nucleotides |
| 10 to 150 nucleotides | | 10 to 150 nucleotides |
| 20 to 150 nucleotides | | 20 to 150 nucleotides |
| Template Construct No. 1 | | |
| Template Construct No. 2 | | |
| Template Construct No. 3 | | |

It is contemplated herein that one or both homology arms may be shortened to avoid including certain sequence repeat elements, e.g., Alu repeats, LINE elements. For example, a 5' homology arm may be shortened to avoid a sequence repeat element. In other embodiments, a 3' homology arm may be shortened to avoid a sequence repeat element. In some embodiments, both the 5' and the 3' homology arms may be shortened to avoid including certain sequence repeat elements.

It is contemplated herein that, in an embodiment, template nucleic acids for correcting a mutation may designed for use as a single-stranded oligonucleotide (ssODN). When using a ssODN, 5' and 3' homology arms may range up to about 200 base pairs (bp) in length, e.g., at least 25, 50, 75, 100, 125, 150, 175, or 200 bp in length. Longer homology arms are also contemplated for ssODNs as improvements in oligonucleotide synthesis continue to be made. It is contemplated herein that, in an embodiment, Cas9 could potentially cleave donor constructs either prior to or following homology directed repair (e.g., homologous recombination), resulting in a possible non-homologous-end-joining event and further DNA sequence mutation at the chromosomal locus of interest. Therefore, to avoid cleavage of the donor sequence before and/or after Cas9-mediated homology directed repair, alternate versions of the donor sequence may be used where silent mutations are introduced. These silent mutations may disrupt Cas9 binding and cleavage, but not disrupt the amino acid sequence of the repaired gene.

In an embodiment, a single or dual nickase eaCas9 is used to cleave the target DNA near the site of the mutation, or signature, to be modified, e.g., replaced. While not wishing to be bound by theory, in an embodiment, it is believed that the Cas9 mediated break induces HDR with the template nucleic acid to replace the target DNA sequence with the template sequence.

### V.2 NHEJ Approaches for Gene Targeting

As described herein, nuclease-induced non-homologous end-joining (NHEJ) can be used to target gene-specific knockouts. Nuclease-induced NHEJ can also be used to remove (e.g., delete) sequence insertions in a gene of interest.

While not wishing to be bound by theory, it is believed that, in an embodiment, the genomic alterations associated with the methods described herein rely on nuclease-induced NHEJ and the error-prone nature of the NHEJ repair pathway. NHEJ repairs a double-strand break in the DNA by joining together the two ends; however, generally, the original sequence is restored only if two compatible ends, exactly as they were formed by the double-strand break, are perfectly ligated. The DNA ends of the double-strand break are frequently the subject of enzymatic processing, resulting in the addition or removal of nucleotides, at one or both strands, prior to rejoining of the ends. This results in the presence of insertion and/or deletion (indel) mutations in the DNA sequence at the site of the NHEJ repair. Two-thirds of these mutations typically alter the reading frame and, therefore, produce a non-functional protein. Additionally, mutations that maintain the reading frame, but which insert or delete a significant amount of sequence, can destroy functionality of the protein. This is locus dependent as mutations in critical functional domains are likely less tolerable than mutations in non-critical regions of the protein.

The indel mutations generated by NHEJ are unpredictable in nature; however, at a given break site certain indel sequences are favored and are over represented in the population, likely due to small regions of microhomology. The lengths of deletions can vary widely; most commonly in the 1-50 bp range, but they can easily reach greater than 100-200 bp. Insertions tend to be shorter and often include short duplications of the sequence immediately surrounding the break site. However, it is possible to obtain large insertions, and in these cases, the inserted sequence has often been traced to other regions of the genome or to plasmid DNA present in the cells.

Because NHEJ is a mutagenic process, it can also be used to delete small sequence motifs as long as the generation of a specific final sequence is not required. If a double-strand break is targeted near to a short target sequence, the deletion mutations caused by the NHEJ repair often span, and therefore remove, the unwanted nucleotides. For the deletion of larger DNA segments, introducing two double-strand breaks, one on each side of the sequence, can result in NHEJ between the ends with removal of the entire intervening sequence. Both of these approaches can be used to delete specific DNA sequences; however, the error-prone nature of NHEJ may still produce indel mutations at the site of repair.

Both double strand cleaving eaCas9 molecules and single strand, or nickase, eaCas9 molecules can be used in the methods and compositions described herein to generate NHEJ-mediated indels. NHEJ-mediated indels targeted to the gene, e.g., a coding region, e.g., an early coding region of a gene of interest can be used to knockout (i.e., eliminate expression of) a gene of interest. For example, early coding region of a gene of interest includes sequence immediately following a transcription start site, within a first exon of the coding sequence, or within 500 bp of the transcription start site (e.g., less than 500, 450, 400, 350, 300, 250, 200, 150, 100 or 50 bp).

### Placement of double strand or single strand breaks relative to the target position

In an embodiment, in which a gRNA and Cas9 nuclease generate a double strand break for the purpose of inducing NHEJ-mediated indels, a gRNA, e.g., a unimolecular (or chimeric) or modular gRNA molecule, is configured to position one double-strand break in close proximity to a nucleotide of the target position. In an embodiment, the cleavage site is between 0-30 bp away from the target position (e.g., less than 30, 25, 20, 15, 10, 9, 8, 7, 6, 5, 4, 3, 2 or 1 bp from the target position).

In an embodiment, in which two gRNAs complexing with Cas9 nickases induce two single strand breaks for the purpose of inducing NHEJ-mediated indels, two gRNAs, e.g., independently, unimolecular (or chimeric) or modular gRNA, are configured to position two single-strand breaks to provide for NHEJ repair a nucleotide of the target position. In an embodiment, the gRNAs are configured to position cuts at the same position, or within a few nucleotides of one another, on different strands, essentially mimicking a double strand break. In an embodiment, the closer nick is between 0-30 bp away from the target position (e.g., less than 30, 25, 20, 15, 10, 9, 8, 7, 6, 5, 4, 3, 2 or 1 bp from the target position), and the two nicks are within 25-55 bp of each other (e.g., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50 , 45 to 50, 35 to 45, or 40 to 45 bp) and no more than 100 bp away from each other (e.g., no more than 90, 80, 70, 60, 50, 40, 30, 20 or 10 bp). In an embodiment, the gRNAs are configured to place a single strand break on either side of a nucleotide of the target position.

Both double strand cleaving eaCas9 molecules and single strand, or nickase, eaCas9 molecules can be used in the methods and compositions described herein to generate breaks both sides of a target position. Double strand or paired single strand breaks may be generated on both sides of a target position to remove the nucleic acid sequence between the two cuts (e.g., the region between the two breaks in deleted). In one embodiment, two gRNAs, e.g., independently, unimolecular (or chimeric) or modular gRNA, are configured to position a double-strand break on both sides of a target position. In an alternate embodiment, three gRNAs, e.g., independently, unimolecular (or chimeric) or modular gRNA, are configured to position a double strand break (i.e., one gRNA complexes with a cas9 nuclease) and two single strand breaks or paired single stranded breaks (i.e., two gRNAs complex with Cas9 nickases) on either side of the target position. In another embodiment, four gRNAs, e.g., independently, unimolecular (or chimeric) or modular gRNA, are configured to generate two pairs of single stranded breaks (i.e., two pairs of two gRNAs complex with Cas9 nickases) on either side of the target position. The double strand break(s) or the closer of the two single strand nicks in a pair will ideally be within 0-500 bp of the target position (e.g., no more than 450, 400, 350, 300, 250, 200, 150, 100, 50 or 25 bp from the target position). When nickases are used, the two nicks in a pair are within 25-55 bp of each other (e.g., between 25 to 50, 25 to 45, 25 to 40, 25 to 35, 25 to 30, 50 to 55, 45 to 55, 40 to 55, 35 to 55, 30 to 55, 30 to 50, 35 to 50, 40 to 50 , 45 to 50, 35 to 45, or 40 to 45 bp) and no more than 100 bp away from each other (e.g., no more than 90, 80, 70, 60, 50, 40, 30, 20 or 10 bp).

### V.3 Targeted Knockdown

Unlike CRISPR/Cas-mediated gene knockout, which permanently eliminates expression by mutating the gene at the DNA level, CRISPR/Cas knockdown allows for temporary reduction of gene expression through the use of artificial transcription factors. Mutating key residues in both DNA cleavage domains of the Cas9 protein (e.g. the D10A and H840A mutations) results in the generation of a catalytically inactive Cas9 (eiCas9 which is also known as dead Cas9 or dCas9) molecule. A catalytically inactive Cas9 complexes with a gRNA and localizes to the DNA sequence specified by that gRNA's targeting domain, however, it does not cleave the target DNA. Fusion of the dCas9 to an effector domain, e.g., a transcription repression domain, enables recruitment of the effector to any DNA site specified by the gRNA. Although an enzymatically inactive (eiCas9) Cas9 molecule itself can block transcription when recruited to early regions in the coding sequence, more robust repression can be achieved by fusing a transcriptional repression domain (for example KRAB, SID or ERD) to the Cas9 molecule and recruiting it to the promoter region of a gene. It is likely that targeting DNAseI hypersensitive regions of the promoter may yield more efficient gene repression or activation because these regions are more likely to be accessible to the Cas9 protein and are also more likely to harbor sites for endogenous transcription factors. Especially for gene repression, it is contemplated herein that blocking the binding site of an endogenous transcription factor would aid in downregulating gene expression. In an embodiment, one or more eiCas9 molecules may be used to block binding of one or more endogenous transcription factors. In another embodiment, an eiCas9 molecule can be fused to a chromatin modifying protein. Altering chromatin status can result in decreased expression of the target gene. In an embodiment, one or more eiCas9 molecules may be used to block binding of one or more endogenous transcription factors. In another embodiment, an eiCas9 molecule can be fused to a chromatin modifying protein. Altering chromatin status can result in decreased expression of the target gene. One or more eiCas9 molecules fused to one or more chromatin modifying proteins may be used to alter chromatin status.

In an embodiment, a gRNA molecule can be targeted to a known transcription response elements (e.g., promoters, enhancers, etc.), a known upstream activating sequences (UAS), and/or sequences of unknown or known function that are suspected of being able to control expression of the target DNA.

CRISPR/Cas-mediated gene knockdown can be used to reduce expression of an unwanted allele or transcript. Contemplated herein are scenarios wherein permanent destruction of the gene is not ideal. In these scenarios, site-specific repression may be used to temporarily reduce or eliminate expression. It is also contemplated herein that the off-target effects of a Cas-repressor may be less severe than those of a Cas-nuclease as a nuclease can cleave any DNA sequence and cause mutations whereas a Cas-repressor may only have an effect if it targets the promoter region of an actively transcribed gene. However, while nuclease-mediated knockout is permanent, repression may only persist as long as the Cas-repressor is present in the cells. Once the repressor is no longer present, it is likely that endogenous transcription factors and gene regulatory elements would restore expression to its natural state.

### V.4 Single-Strand Annealing

Single strand annealing (SSA) is another DNA repair process that repairs a double-strand break between two repeat sequences present in a target nucleic acid. Repeat sequences utilized by the SSA pathway are generally greater than 30 nucleotides in length. Resection at the break ends occurs to reveal repeat sequences on both strands of the target nucleic acid. After resection, single strand overhangs containing the repeat sequences are coated with RPA protein to prevent the repeats sequences from inappropriate annealing, e.g., to themselves. RAD52 binds to and each of the repeat sequences on the overhangs and aligns the sequences to enable the annealing of the complementary repeat sequences. After annealing, the single-strand flaps of the overhangs are cleaved. New DNA synthesis fills in any gaps, and ligation restores the DNA duplex. As a result of the processing, the DNA sequence between the two repeats is deleted. The length of the deletion can depend on many factors including the location of the two repeats utilized, and the pathway or processivity of the resection.

In contrast to HDR pathways, SSA does not require a template nucleic acid to alter or correct a target nucleic acid sequence. Instead, the complementary repeat sequence is utilized.

### V. 5 Other DNA Repair Pathways

### SSBR (single strand break repair)

Single-stranded breaks (SSB) in the genome are repaired by the SSBR pathway, which is a distinct mechanism from the DSB repair mechanisms discussed above. The SSBR pathway has four major stages: SSB detection, DNA end processing, DNA gap filling, and DNA ligation. A more detailed explanation is given in Caldecott, Nature Reviews Genetics 9, 619-631 (August 2008), and a summary is given here.

In the first stage, when a SSB forms, PARP1 and/or PARP2 recognize the break and recruit repair machinery. The binding and activity of PARP1 at DNA breaks is transient and it seems to accelerate SSBr by promoting the focal accumulation or stability of SSBr protein complexes at the lesion. Arguably the most important of these SSBr proteins is XRCC1, which functions as a molecular scaffold that interacts with, stabilizes, and stimulates multiple enzymatic components of the SSBr process including the protein responsible for cleaning the DNA 3' and 5' ends. For instance, XRCC1 interacts with several proteins (DNA polymerase beta, PNK, and three nucleases, APE1, APTX, and APLF) that promote end processing. APE1 has endonuclease activity. APLF exhibits endonuclease and 3' to 5' exonuclease activities. APTX has endonuclease and 3' to 5' exonuclease activity.

This end processing is an important stage of SSBR since the 3'- and/or 5'-termini of most, if not all, SSBs are 'damaged'. End processing generally involves restoring a damaged 3'-end to a hydroxylated state and and/or a damaged 5' end to a phosphate moiety, so that the ends become ligation-competent. Enzymes that can process damaged 3' termini include PNKP, APE1, and TDP1. Enzymes that can process damaged 5' termini include PNKP, DNA polymerase beta, and APTX. LIG3 (DNA ligase III) can also participate in end processing. Once the ends are cleaned, gap filling can occur.

At the DNA gap filling stage, the proteins typically present are PARP1, DNA polymerase beta, XRCC1, FEN1 (flap endonculease 1), DNA polymerase delta/epsilon, PCNA, and LIG1. There are two ways of gap filling, the short patch repair and the long patch repair. Short patch repair involves the insertion of a single nucleotide that is missing. At some SSBs, "gap filling" might continue displacing two or more nucleotides (displacement of up to 12 bases have been reported). FEN1 is an endonuclease that removes the displaced 5'-residues. Multiple DNA polymerases, including Pol *β*, are involved in the repair of SSBs, with the choice of DNA polymerase influenced by the source and type of SSB.

In the fourth stage, a DNA ligase such as LIG1 (Ligase I) or LIG3 (Ligase III) catalyzes joining of the ends. Short patch repair uses Ligase III and long patch repair uses Ligase I.

Sometimes, SSBR is replication-coupled. This pathway can involve one or more of CtIP, MRN, ERCC1, and FEN1. Additional factors that may promote SSBR include: aPARP, PARP1, PARP2, PARG, XRCC1, DNA polymerase b, DNA polymerase d, DNA polymerase e, PCNA, LIG1, PNK, PNKP, APE1, APTX, APLF, TDP1, LIG3, FEN1, CtIP, MRN, and ERCC1.

### MMR (mismatch repair)

Cells contain three excision repair pathways: MMR, BER, and NER. The excision repair pathways hace a common feature in that they typically recognize a lesion on one strand of the DNA, then exo/endonucleaseases remove the lesion and leave a 1-30 nucleotide gap that is sub-sequentially filled in by DNA polymerase and finally sealed with ligase. A more complete picture is given in Li, Cell Research (2008) 18:85-98, and a summary is provided here.

Mismatch repair (MMR) operates on mispaired DNA bases.

The MSH2/6 or MSH2/3 complexes both have ATPases activity that plays an important role in mismatch recognition and the initiation of repair. MSH2/6 preferentially recognizes base-base mismatches and identifies mispairs of 1 or 2 nucleotides, while MSH2/3 preferentially recognizes larger ID mispairs.

hMLH1 heterodimerizes with hPMS2 to form hMutL *α* which possesses an ATPase activity and is important for multiple steps of MMR. It possesses a PCNA/replication factor C (RFC)-dependent endonuclease activity which plays an important role in 3' nick-directed MMR involving EXO1. (EXO1 is a participant in both HR and MMR.) It regulates termination of mismatch-provoked excision. Ligase I is the relevant ligase for this pathway. Additional factors that may promote MMR include: EXO1, MSH2, MSH3, MSH6, MLH1, PMS2, MLH3, DNA Pol d, RPA, HMGB1, RFC, and DNA ligase I.

### Base excision repair (BER)

The base excision repair (BER) pathway is active throughout the cell cycle; it is responsible primarily for removing small, non-helix-distorting base lesions from the genome. In contrast, the related Nucleotide Excision Repair pathway (discussed in the next section) repairs bulky helix-distorting lesions. A more detailed explanation is given in Caldecott, Nature Reviews Genetics 9, 619-631 (August 2008), and a summary is given here.

Upon DNA base damage, base excision repair (BER) is initiated and the process can be simplified into five major steps: (a) removal of the damaged DNA base; (b) incision of the subsequent a basic site; (c) clean-up of the DNA ends; (d) insertion of the correct nucleotide into the repair gap; and (e) ligation of the remaining nick in the DNA backbone. These last steps are similar to the SSBR.

In the first step, a damage-specific DNA glycosylase excises the damaged base through cleavage of the N-glycosidic bond linking the base to the sugar phosphate backbone. Then AP endonuclease-1 (APE1) or bifunctional DNA glycosylases with an associated lyase activity incised the phosphodiester backbone to create a DNA single strand break (SSB). The third step of BER involves cleaning-up of the DNA ends. The fourth step in BER is conducted by Pol β that adds a new complementary nucleotide into the repair gap and in the final step XRCC1/Ligase III seals the remaining nick in the DNA backbone. This completes the short-patch BER pathway in which the majority (∼80%) of damaged DNA bases are repaired. However, if the 5' -ends in step 3 are resistant to end processing activity, following one nucleotide insertion by Pol β there is then a polymerase switch to the replicative DNA polymerases, Pol δ/ε, which then add ∼2-8 more nucleotides into the DNA repair gap. This creates a 5' -flap structure, which is recognized and excised by flap endonuclease-1 (FEN-1) in association with the processivity factor proliferating cell nuclear antigen (PCNA). DNA ligase I then seals the remaining nick in the DNA backbone and completes long-patch BER. Additional factors that may promote the BER pathway include: DNA glycosylase, APE1, Polb, Pold, Pole, XRCC1, Ligase III, FEN-1, PCNA, RECQL4, WRN, MYH, PNKP, and APTX.

### Nucleotide excision repair (NER)

Nucleotide excision repair (NER) is an important excision mechanism that removes bulky helix-distorting lesions from DNA. Additional details about NER are given in Marteijn et al., Nature Reviews Molecular Cell Biology 15, 465-481 (2014), and a summary is given here. NER a broad pathway encompassing two smaller pathways: global genomic NER (GG-NER) and transcription coupled repair NER (TC-NER). GG-NER and TC-NER use different factors for recognizing DNA damage. However, they utilize the same machinery for lesion incision, repair, and ligation.

Once damage is recognized, the cell removes a short single-stranded DNA segment that contains the lesion. Endonucleases XPF/ERCC1 and XPG (encoded by ERCC5) remove the lesion by cutting the damaged strand on either side of the lesion, resulting in a single-strand gap of 22-30 nucleotides. Next, the cell performs DNA gap filling synthesis and ligation. Involved in this process are: PCNA, RFC, DNA Pol δ, DNA Pol ε or DNA Pol κ, and DNA ligase I or XRCC1/Ligase III. Replicating cells tend to use DNA pol ε and DNA ligase I, while non-replicating cells tend to use DNA Pol δ, DNA Pol κ, and the XRCC1/ Ligase III complex to perform the ligation step.

NER can involve the following factors: XPA-G, POLH, XPF, ERCC1, XPA-G, and LIG1. Transcription-coupled NER (TC-NER) can involve the following factors: CSA, CSB, XPB, XPD, XPG, ERCC1, and TTDA. Additional factors that may promote the NER repair pathway include XPA-G, POLH, XPF, ERCC1, XPA-G, LIG1, CSA, CSB, XPA, XPB, XPC, XPD, XPF, XPG, TTDA, UVSSA, USP7, CETN2, RAD23B, UV-DDB, CAK subcomplex, RPA, and PCNA.

### Interstrand Crosslink (ICL)

A dedicated pathway called the ICL repair pathway repairs interstrand crosslinks. Interstrand crosslinks, or covalent crosslinks between bases in different DNA strand, can occur during replication or transcription. ICL repair involves the coordination of multiple repair processes, in particular, nucleolytic activity, translesion synthesis (TLS), and HDR. Nucleases are recruited to excise the ICL on either side of the crosslinked bases, while TLS and HDR are coordinated to repair the cut strands. ICL repair can involve the following factors: endonucleases, e.g., XPF and RAD51C, endonucleases such as RAD51, translesion polymerases, e.g., DNA polymerase zeta and Rev1), and the Fanconi anemia (FA) proteins, e.g., FancJ.

### Other pathways

Several other DNA repair pathways exist in mammals.

Translesion synthesis (TLS) is a pathway for repairing a single stranded break left after a defective replication event and involves translesion polymerases, e.g., DNA pol and Rev1..

Error-free postreplication repair (PRR) is another pathway for repairing a single stranded break left after a defective replication event.

### V.6 Examples of gRNAs in Genome Editing Methods

gRNA molecules as described herein can be used with Cas9 molecules that generate a double strand break or a single strand break to alter the sequence of a target nucleic acid, e.g., a target position or target genetic signature. gRNA molecules useful in these methods are described below.

In an embodiment, the gRNA, e.g., a chimeric gRNA, is configured such that it comprises one or more of the following properties;
a) it can position, e.g., when targeting a Cas9 molecule that makes double strand breaks, a double strand break (i) within 50, 100, 150, 200, 250, 300, 350, 400, 450, or 500 nucleotides of a target position, or (ii) sufficiently close that the target position is within the region of end resection;
b) it has a targeting domain of at least 16 nucleotides, e.g., a targeting domain of (i) 16, (ii), 17, (iii) 18, (iv) 19, (v) 20, (vi) 21, (vii) 22, (viii) 23, (ix) 24, (x) 25, or (xi) 26 nucleotides; and
c)
   (i) the proximal and tail domain, when taken together, comprise at least 15, 18, 20, 25, 30, 31, 35, 40, 45, 49, 50, or 53 nucleotides, e.g., at least 15, 18, 20, 25, 30, 31, 35, 40, 45, 49, 50, or 53 nucleotides from a naturally occurring *S*. *pyogenes, S. thermophilus, S. aureus,* or *N. meningitidis* tail and proximal domain, or a sequence that differs by no more than 1, 2, 3, 4, 5; 6, 7, 8, 9 or 10 nucleotides therefrom;
   (ii) there are at least 15, 18, 20, 25, 30, 31, 35, 40, 45, 49, 50, or 53 nucleotides 3' to the last nucleotide of the second complementarity domain, e.g., at least 15, 18, 20, 25, 30, 31, 35, 40, 45, 49, 50, or 53 nucleotides from the corresponding sequence of a naturally occurring *S. pyogenes, S. thermophilus, S. aureus,* or *N. meningitidis* gRNA, or a sequence that differs by no more than 1, 2, 3, 4, 5; 6, 7, 8, 9 or 10 nucleotides therefrom;
   (iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, e.g., at least 16, 19, 21, 26, 31, 32, 36, 41, 46, 50, 51, or 54 nucleotides from the corresponding sequence of a naturally occurring *S. pyogenes, S. thermophilus, S. aureus,* or *N. meningitidis* gRNA, or a sequence that differs by no more than 1, 2, 3, 4, 5; 6, 7, 8, 9 or 10 nucleotides therefrom;
   (iv) the tail domain is at least 10, 15, 20, 25, 30, 35 or 40 nucleotides in length, e.g., it comprises at least 10, 15, 20, 25, 30, 35 or 40 nucleotides from a naturally occurring *S. pyogenes, S. thermophilus, S. aureus,* or *N. meningitidis* tail domain, or a sequence that differs by no more than 1, 2, 3, 4, 5; 6, 7, 8, 9 or 10 nucleotides therefrom; or
   (v) the tail domain comprises 15, 20, 25, 30, 35, 40 nucleotides or all of the corresponding portions of a naturally occurring tail domain, e.g., a naturally occurring *S. pyogenes, S. thermophilus, S. aureus,* or *N. meningitidis* tail domain.

In an embodiment, the gRNA is configured such that it comprises properties: a and b(i).

In an embodiment, the gRNA is configured such that it comprises properties: a and b(ii).

In an embodiment, the gRNA is configured such that it comprises properties: a and b(iii).

In an embodiment, the gRNA is configured such that it comprises properties: a and b(iv).

In an embodiment, the gRNA is configured such that it comprises properties: a and b(v).

In an embodiment, the gRNA is configured such that it comprises properties: a and b(vi).

In an embodiment, the gRNA is configured such that it comprises properties: a and b(vii).

In an embodiment, the gRNA is configured such that it comprises properties: a and b(viii).

In an embodiment, the gRNA is configured such that it comprises properties: a and b(ix).

In an embodiment, the gRNA is configured such that it comprises properties: a and b(x).

In an embodiment, the gRNA is configured such that it comprises properties: a and b(xi).

In an embodiment, the gRNA is configured such that it comprises properties: a and c.

In an embodiment, the gRNA is configured such that in comprises properties: a, b, and c.

In an embodiment, the gRNA is configured such that in comprises properties: a(i), b(i), and c(i).

In an embodiment, the gRNA is configured such that in comprises properties: a(i), b(i), and c(ii).

In an embodiment, the gRNA is configured such that in comprises properties: a(i), b(ii), and c(i).

In an embodiment, the gRNA is configured such that in comprises properties: a(i), b(ii), and c(ii).

In an embodiment, the gRNA is configured such that in comprises properties: a(i), b(iii), and c(i).

In an embodiment, the gRNA is configured such that in comprises properties: a(i), b(iii), and c(ii).

In an embodiment, the gRNA is configured such that in comprises properties: a(i), b(iv), and c(i).

In an embodiment, the gRNA is configured such that in comprises properties: a(i), b(iv), and c(ii).

In an embodiment, the gRNA is configured such that in comprises properties: a(i), b(v), and c(i).

In an embodiment, the gRNA is configured such that in comprises properties: a(i), b(v), and c(ii).

In an embodiment, the gRNA is configured such that in comprises properties: a(i), b(vi), and c(i).

In an embodiment, the gRNA is configured such that in comprises properties: a(i), b(vi), and c(ii).

In an embodiment, the gRNA is configured such that in comprises properties: a(i), b(vii), and c(i).

In an embodiment, the gRNA is configured such that in comprises properties: a(i), b(vii), and c(ii).

In an embodiment, the gRNA is configured such that in comprises properties: a(i), b(viii), and c(i).

In an embodiment, the gRNA is configured such that in comprises properties: a(i), b(viii), and c(ii).

In an embodiment, the gRNA is configured such that in comprises properties: a(i), b(ix), and c(i).

In an embodiment, the gRNA is configured such that in comprises properties: a(i), b(ix), and c(ii).

In an embodiment, the gRNA is configured such that in comprises properties: a(i), b(x), and c(i).

In an embodiment, the gRNA is configured such that in comprises properties: a(i), b(x), and c(ii).

In an embodiment, the gRNA is configured such that in comprises properties: a(i), b(xi), and c(i).

In an embodiment, the gRNA is configured such that in comprises properties: a(i), b(xi), and c(ii).

In an embodiment, the gRNA, e.g., a chimeric gRNA, is configured such that it comprises one or more of the following properties;
a) one or both of the gRNAs can position, e.g., when targeting a Cas9 molecule that makes single strand breaks, a single strand break within (i) 50, 100, 150, 200, 250, 300, 350, 400, 450, or 500 nucleotides of a target position, or (ii) sufficiently close that the target position is within the region of end resection;
b) one or both have a targeting domain of at least 16 nucleotides, e.g., a targeting domain of (i) 16, (ii), 17, (iii) 18, (iv) 19, (v) 20, (vi) 21, (vii) 22, (viii) 23, (ix) 24, (x) 25, or (xi) 26 nucleotides; and
c)
   (i) the proximal and tail domain, when taken together, comprise at least 15, 18, 20, 25, 30, 31, 35, 40, 45, 49, 50, or 53 nucleotides, e.g., at least 15, 18, 20, 25, 30, 31, 35, 40, 45, 49, 50, or 53 nucleotides from a naturally occurring *S*. *pyogenes, S. thermophilus, S. aureus,* or *N. meningitidis* tail and proximal domain, or a sequence that differs by no more than 1, 2, 3, 4, 5; 6, 7, 8, 9 or 10 nucleotides therefrom;
   (ii) there are at least 15, 18, 20, 25, 30, 31, 35, 40, 45, 49, 50, or 53 nucleotides 3' to the last nucleotide of the second complementarity domain, e.g., at least 15, 18, 20, 25, 30, 31, 35, 40, 45, 49, 50, or 53 nucleotides from the corresponding sequence of a naturally occurring *S. pyogenes, S. thermophilus, S. aureus,* or *N. meningitidis* gRNA, or a sequence that differs by no more than 1, 2, 3, 4, 5; 6, 7, 8, 9 or 10 nucleotides therefrom;
   (iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, e.g., at least 16, 19, 21, 26, 31, 32, 36, 41, 46, 50, 51, or 54 nucleotides from the corresponding sequence of a naturally occurring *S. pyogenes, S. thermophilus, S. aureus,* or *N. meningitidis* gRNA, or a sequence that differs by no more than 1, 2, 3, 4, 5; 6, 7, 8, 9 or 10 nucleotides therefrom;
   (iv) the tail domain is at least 10, 15, 20, 25, 30, 35 or 40 nucleotides in length, e.g., it comprises at least 10, 15, 20, 25, 30, 35 or 40 nucleotides from a naturally occurring *S. pyogenes, S. thermophilus, S. aureus,* or *N. meningitidis* tail domain, or a sequence that differs by no more than 1, 2, 3, 4, 5; 6, 7, 8, 9 or 10 nucleotides therefrom; or
   (v) the tail domain comprises 15, 20, 25, 30, 35, 40 nucleotides or all of the corresponding portions of a naturally occurring tail domain, e.g., a naturally occurring *S. pyogenes, S. thermophilus, S. aureus,* or *N. meningitidis* tail domain.

In an embodiment, the gRNA is configured such that it comprises properties: a and b(i).

In an embodiment, the gRNA is configured such that it comprises properties: a and b(ii).

In an embodiment, the gRNA is configured such that it comprises properties: a and b(iii).

In an embodiment, the gRNA is configured such that it comprises properties: a and b(iv).

In an embodiment, the gRNA is configured such that it comprises properties: a and b(v).

In an embodiment, the gRNA is configured such that it comprises properties: a and b(vi).

In an embodiment, the gRNA is configured such that it comprises properties: a and b(vii).

In an embodiment, the gRNA is configured such that it comprises properties: a and b(viii).

In an embodiment, the gRNA is configured such that it comprises properties: a and b(ix).

In an embodiment, the gRNA is configured such that it comprises properties: a and b(x).

In an embodiment, the gRNA is configured such that it comprises properties: a and b(xi).

In an embodiment, the gRNA is configured such that it comprises properties: a and c.

In an embodiment, the gRNA is configured such that in comprises properties: a, b, and c.

In an embodiment, the gRNA is configured such that in comprises properties: a(i), b(i), and c(i).

In an embodiment, the gRNA is configured such that in comprises properties: a(i), b(i), and c(ii).

In an embodiment, the gRNA is configured such that in comprises properties: a(i), b(ii), and c(i).

In an embodiment, the gRNA is configured such that in comprises properties: a(i), b(ii), and c(ii).

In an embodiment, the gRNA is configured such that in comprises properties: a(i), b(iii), and c(i).

In an embodiment, the gRNA is configured such that in comprises properties: a(i), b(iii), and c(ii).

In an embodiment, the gRNA is configured such that in comprises properties: a(i), b(iv), and c(i).

In an embodiment, the gRNA is configured such that in comprises properties: a(i), b(iv), and c(ii).

In an embodiment, the gRNA is configured such that in comprises properties: a(i), b(v), and c(i).

In an embodiment, the gRNA is configured such that in comprises properties: a(i), b(v), and c(ii).

In an embodiment, the gRNA is configured such that in comprises properties: a(i), b(vi), and c(i).

In an embodiment, the gRNA is configured such that in comprises properties: a(i), b(vi), and c(ii).

In an embodiment, the gRNA is configured such that in comprises properties: a(i), b(vii), and c(i).

In an embodiment, the gRNA is configured such that in comprises properties: a(i), b(vii), and c(ii).

In an embodiment, the gRNA is configured such that in comprises properties: a(i), b(viii), and c(i).

In an embodiment, the gRNA is configured such that in comprises properties: a(i), b(viii), and c(ii).

In an embodiment, the gRNA is configured such that in comprises properties: a(i), b(ix), and c(i).

In an embodiment, the gRNA is configured such that in comprises properties: a(i), b(ix), and c(ii).

In an embodiment, the gRNA is configured such that in comprises properties: a(i), b(x), and c(i).

In an embodiment, the gRNA is configured such that in comprises properties: a(i), b(x), and c(ii).

In an embodiment, the gRNA is configured such that in comprises properties: a(i), b(xi), and c(i).

In an embodiment, the gRNA is configured such that in comprises properties: a(i), b(xi), and c(ii).

In an embodiment, the gRNA is used with a Cas9 nickase molecule having HNH activity, e.g., a Cas9 molecule having the RuvC activity inactivated, e.g., a Cas9 molecule having a mutation at D10, e.g., the D10A mutation.

In an embodiment, the gRNA is used with a Cas9 nickase molecule having RuvC activity, e.g., a Cas9 molecule having the HNH activity inactivated, e.g., a Cas9 molecule having a mutation at 840, e.g., the H840A.

In an embodiment, the gRNAs are used with a Cas9 nickase molecule having RuvC activity, e.g., a Cas9 molecule having the HNH activity inactivated, e.g., a Cas9 molecule having a mutation at N863, e.g., the N863A mutation.

In an embodiment, a pair of gRNAs, e.g., a pair of chimeric gRNAs, comprising a first and a second gRNA, is configured such that they comprises one or more of the following properties;
a) one or both of the gRNAs can position, e.g., when targeting a Cas9 molecule that makes single strand breaks, a single strand break within (i) 50, 100, 150, 200, 250, 300, 350, 400, 450, or 500 nucleotides of a target position, or (ii) sufficiently close that the target position is within the region of end resection;
b) one or both have a targeting domain of at least 16 nucleotides, e.g., a targeting domain of (i) 16, (ii), 17, (iii) 18, (iv) 19, (v) 20, (vi) 21, (vii) 22, (viii) 23, (ix) 24, (x) 25, or (xi) 26 nucleotides;
c) for one or both:
   (i) the proximal and tail domain, when taken together, comprise at least 15, 18, 20, 25, 30, 31, 35, 40, 45, 49, 50, or 53 nucleotides, e.g., at least 15, 18, 20, 25, 30, 31, 35, 40, 45, 49, 50, or 53 nucleotides from a naturally occurring *S. pyogenes, S. thermophilus, S. aureus,* or *N. meningitidis* tail and proximal domain, or a sequence that differs by no more than 1, 2, 3, 4, 5; 6, 7, 8, 9 or 10 nucleotides therefrom;
   (ii) there are at least 15, 18, 20, 25, 30, 31, 35, 40, 45, 49, 50, or 53 nucleotides 3' to the last nucleotide of the second complementarity domain, e.g., at least 15, 18, 20, 25, 30, 31, 35, 40, 45, 49, 50, or 53 nucleotides from the corresponding sequence of a naturally occurring *S. pyogenes, S. thermophilus, S. aureus,* or *N. meningitidis* gRNA, or a sequence that differs by no more than 1, 2, 3, 4, 5; 6, 7, 8, 9 or 10 nucleotides therefrom;
   (iii) there are at least 16, 19, 21, 26, 31, 32, 36, 41, 46, 50, 51, or 54 nucleotides 3' to the last nucleotide of the second complementarity domain that is complementary to its corresponding nucleotide of the first complementarity domain, e.g., at least 16, 19, 21, 26, 31, 32, 36, 41, 46, 50, 51, or 54 nucleotides from the corresponding sequence of a naturally occurring *S. pyogenes, S. thermophilus, S. aureus,* or *N. meningitidis* gRNA, or a sequence that differs by no more than 1, 2, 3, 4, 5; 6, 7, 8, 9 or 10 nucleotides therefrom;
   (iv) the tail domain is at least 10, 15, 20, 25, 30, 35 or 40 nucleotides in length, e.g., it comprises at least 10, 15, 20, 25, 30, 35 or 40 nucleotides from a naturally occurring *S. pyogenes, S. thermophilus, S. aureus,* or *N. meningitidis* tail domain; or, or a sequence that differs by no more than 1, 2, 3, 4, 5; 6, 7, 8, 9 or 10 nucleotides therefrom; or
   (v) the tail domain comprises 15, 20, 25, 30, 35, 40 nucleotides or all of the corresponding portions of a naturally occurring tail domain, e.g., a naturally occurring *S. pyogenes, S. thermophilus, S. aureus,* or *N. meningitidis* tail domain;
d) the gRNAs are configured such that, when hybridized to target nucleic acid, they are separated by 0-50, 0-100, 0-200, at least 10, at least 20, at least 30 or at least 50 nucleotides;
e) the breaks made by the first gRNA and second gRNA are on different strands; and
f) the PAMs are facing outwards.

In an embodiment, one or both of the gRNAs is configured such that it comprises properties: a and b(i).

In an embodiment, one or both of the gRNAs is configured such that it comprises properties: a and b(ii).

In an embodiment, one or both of the gRNAs is configured such that it comprises properties: a and b(iii).

In an embodiment, one or both of the gRNAs is configured such that it comprises properties: a and b(iv).

In an embodiment, one or both of the gRNAs is configured such that it comprises properties: a and b(v).

In an embodiment, one or both of the gRNAs is configured such that it comprises properties: a and b(vi).

In an embodiment, one or both of the gRNAs is configured such that it comprises properties: a and b(vii).

In an embodiment, one or both of the gRNAs is configured such that it comprises properties: a and b(viii).

In an embodiment, one or both of the gRNAs is configured such that it comprises properties: a and b(ix).

In an embodiment, one or both of the gRNAs is configured such that it comprises properties: a and b(x).

In an embodiment, one or both of the gRNAs is configured such that it comprises properties: a and b(xi).

In an embodiment, one or both of the gRNAs configured such that it comprises properties: a and c.

In an embodiment, one or both of the gRNAs is configured such that it comprises properties: a, b, and c.

In an embodiment, one or both of the gRNAs is configured such that it comprises properties: a(i), b(i), and c(i).

In an embodiment, one or both of the gRNAs is configured such that it comprises properties: a(i), b(i), and c(ii).

In an embodiment, one or both of the gRNAs is configured such that it comprises properties: a(i), b(i), c, and d.

In an embodiment, one or both of the gRNAs is configured such that it comprises properties: a(i), b(i), c, and e.

In an embodiment, one or both of the gRNAs is configured such that it comprises properties: a(i), b(i), c, d, and e.

In an embodiment, one or both of the gRNAs is configured such that it comprises properties: a(i), b(ii), and c(i).

In an embodiment, one or both of the gRNAs is configured such that it comprises properties: a(i), b(ii), and c(ii).

In an embodiment, one or both of the gRNAs is configured such that it comprises properties: a(i), b(ii), c, and d.

In an embodiment, one or both of the gRNAs is configured such that it comprises properties: a(i), b(ii), c, and e.

In an embodiment, one or both of the gRNAs is configured such that it comprises properties: a(i), b(ii), c, d, and e.

In an embodiment, one or both of the gRNAs is configured such that it comprises properties: a(i), b(iii), and c(i).

In an embodiment, one or both of the gRNAs is configured such that it comprises properties: a(i), b(iii), and c(ii).

In an embodiment, one or both of the gRNAs is configured such that it comprises properties: a(i), b(iii), c, and d.

In an embodiment, one or both of the gRNAs is configured such that it comprises properties: a(i), b(iii), c, and e.

In an embodiment, one or both of the gRNAs is configured such that it comprises properties: a(i), b(iii), c, d, and e.

In an embodiment, one or both of the gRNAs is configured such that it comprises properties: a(i), b(iv), and c(i).

In an embodiment, one or both of the gRNAs is configured such that it comprises properties: a(i), b(iv), and c(ii).

In an embodiment, one or both of the gRNAs is configured such that it comprises properties: a(i), b(iv), c, and d.

In an embodiment, one or both of the gRNAs is configured such that it comprises properties: a(i), b(iv), c, and e.

In an embodiment, one or both of the gRNAs is configured such that it comprises properties: a(i), b(iv), c, d, and e.

In an embodiment, one or both of the gRNAs is configured such that it comprises properties: a(i), b(v), and c(i).

In an embodiment, one or both of the gRNAs is configured such that it comprises properties: a(i), b(v), and c(ii).

In an embodiment, one or both of the gRNAs is configured such that it comprises properties: a(i), b(v), c, and d.

In an embodiment, one or both of the gRNAs is configured such that it comprises properties: a(i), b(v), c, and e.

In an embodiment, one or both of the gRNAs is configured such that it comprises properties: a(i), b(v), c, d, and e.

In an embodiment, one or both of the gRNAs is configured such that it comprises properties: a(i), b(vi), and c(i).

In an embodiment, one or both of the gRNAs is configured such that it comprises properties: a(i), b(vi), and c(ii).

In an embodiment, one or both of the gRNAs is configured such that it comprises properties: a(i), b(vi), c, and d.

In an embodiment, one or both of the gRNAs is configured such that it comprises properties: a(i), b(vi), c, and e.

In an embodiment, one or both of the gRNAs is configured such that it comprises properties: a(i), b(vi), c, d, and e.

In an embodiment, one or both of the gRNAs is configured such that it comprises properties: a(i), b(vii), and c(i).

In an embodiment, one or both of the gRNAs is configured such that it comprises properties: a(i), b(vii), and c(ii).

In an embodiment, one or both of the gRNAs is configured such that it comprises properties: a(i), b(vii), c, and d.

In an embodiment, one or both of the gRNAs is configured such that it comprises properties: a(i), b(vii), c, and e.

In an embodiment, one or both of the gRNAs is configured such that it comprises properties: a(i), b(vii), c, d, and e.

In an embodiment, one or both of the gRNAs is configured such that it comprises properties: a(i), b(viii), and c(i).

In an embodiment, one or both of the gRNAs is configured such that it comprises properties: a(i), b(viii), and c(ii).

In an embodiment, one or both of the gRNAs is configured such that it comprises properties: a(i), b(viii), c, and d.

In an embodiment, one or both of the gRNAs is configured such that it comprises properties: a(i), b(viii), c, and e.

In an embodiment, one or both of the gRNAs is configured such that it comprises properties: a(i), b(viii), c, d, and e.

In an embodiment, one or both of the gRNAs is configured such that it comprises properties: a(i), b(ix), and c(i).

In an embodiment, one or both of the gRNAs is configured such that it comprises properties: a(i), b(ix), and c(ii).

In an embodiment, one or both of the gRNAs is configured such that it comprises properties: a(i), b(ix), c, and d.

In an embodiment, one or both of the gRNAs is configured such that it comprises properties: a(i), b(ix), c, and e.

In an embodiment, one or both of the gRNAs is configured such that it comprises properties: a(i), b(ix), c, d, and e.

In an embodiment, one or both of the gRNAs is configured such that it comprises properties: a(i), b(x), and c(i).

In an embodiment, one or both of the gRNAs is configured such that it comprises properties: a(i), b(x), and c(ii).

In an embodiment, one or both of the gRNAs is configured such that it comprises properties: a(i), b(x), c, and d.

In an embodiment, one or both of the gRNAs is configured such that it comprises properties: a(i), b(x), c, and e.

In an embodiment, one or both of the gRNAs is configured such that it comprises properties: a(i), b(x), c, d, and e.

In an embodiment, one or both of the gRNAs is configured such that it comprises properties: a(i), b(xi), and c(i).

In an embodiment, one or both of the gRNAs is configured such that it comprises properties: a(i), b(xi), and c(ii).

In an embodiment, one or both of the gRNAs is configured such that it comprises properties: a(i), b(xi), c, and d.

In an embodiment, one or both of the gRNAs is configured such that it comprises properties: a(i), b(xi), c, and e.

In an embodiment, one or both of the gRNAs is configured such that it comprises properties: a(i), b(xi), c, d, and e.

In an embodiment, the gRNAs are used with a Cas9 nickase molecule having HNH activity, e.g., a Cas9 molecule having the RuvC activity inactivated, e.g., a Cas9 molecule having a mutation at D10, e.g., the D10A mutation.

In an embodiment, the gRNAs are used with a Cas9 nickase molecule having RuvC activity, e.g., a Cas9 molecule having the HNH activity inactivated, e.g., a Cas9 molecule having a mutation at H840, e.g., the H840A mutation.
In an embodiment, the gRNAs are used with a Cas9 nickase molecule having RuvC activity, e.g., a Cas9 molecule having the HNH activity inactivated, e.g., a Cas9 molecule having a mutation at N863, e.g., the N863A mutation.

### VI. Target Cells

Cas9 molecules, gRNA molecules (e.g., a Cas9 molecule/gRNA molecule complex), and donor nucleic acids can be used to manipulate a cell, e.g., to edit a target nucleic acid, in a wide variety of cells.

In an embodiment, a cell is manipulated by editing (e.g., correcting) the *MYOC* target gene, e.g., as described herein. In an embodiment, the expression of the *MYOC* target gene is modulated, e.g., *in vivo.* In another embodiment, the expression of the *MYOC* target gene is modulated, e.g., *ex vivo.*

The Cas9 and gRNA molecules described herein can be delivered to a target cell. In an embodiment, the target cell is a cell from the eye, e.g., a trabecular meshwork cell, retinal pigment epithelial cell, a retinal cell, an iris cell, a ciliary body cell and/or the optic nerve. In an embodiment, the target cell is a trabecular meshwork cell. In an embodiment, the target cell is a retinal cell, e.g., a cell of the retinal pigment epithelium or a photoreceptor cell. In an embodiment, the target cell is a cone photoreceptor cell or cone cell, a rod photoreceptor cell or rod cell, or a macular cone photoreceptor cell. In an embodiment, cone photoreceptors in the macular are targeted, i.e., cone photoreceptors in the macular are the target cells.

In an embodiment, the target cell is removed from the subject, the mutation corrected *ex vivo,* and the cell returned to the subject. In an embodiment, a photoreceptor cell is removed from the subject, the mutation corrected *ex vivo,* and the photoreceptor cell is returned to the subject. In an embodiment, a cone photoreceptor cell is removed from the subject, the mutation corrected *ex vivo,* and the cone photoreceptor cell is returned to the subject. In an embodiment, a trabecular meshwork cell is removed from the subject, the mutation corrected *ex vivo,* and the trabecular meshwork cell is returned to the subject.

In an embodiment, the cells are induced pluripotent stem cells (iPS) cells or cells derived from iPS cells, e.g., iPS cells from the subject, modified to alter the gene and differentiated into trabecular meshwork cells, retinal progenitor cells or retinal cells, e.g., retinal photoreceptors, and injected into the eye of the subject, e.g., into the trabecular meshwork, or, e.g., subretinally, e.g., in the submacular region of the retina.

In an embodiment, the cells are targeted *in vivo,* e.g., by delivery of the components, e.g., a Cas9 molecule and gRNA molecules, to the target cells. In an embodiment, the target cells are trabecular meshwork cells, retinal pigment epithelium or photoreceptor cells. In an embodiment, AAV is used to transduce the target cells.

### VII. Delivery, Formulations and Routes of Administration

The components, e.g., a Cas9 molecule, gRNA molecule or template molecule, or all three, can be delivered, formulated or administered in a variety of forms, see, e.g., **Tables 31-32.** In an embodiment, one Cas9 molecule and two or more (e.g., 2, 3, 4, or more) different gRNA molecules are delivered, e.g., by an AAV vector. In an embodiment, the sequence encoding the Cas9 molecule and the sequence(s) encoding the two or more (e.g., 2, 3, 4, or more) different gRNA molecules are present on the same nucleic acid molecule, e.g., an AAV vector. When a Cas9 or gRNA component is encoded as DNA for delivery, the DNA will typically, but not necessarily, include a control region, e.g., comprising a promoter, to effect expression. Useful promoters for Cas9 molecule sequences include CMV, EFS, EF-1a, MSCV, PGK, CAG control promoters. In an embodiment, the promoter is a constitutive promoter. In another embodiment, the promoter is a tissue specific promoter. Useful promoters for gRNAs include H1, 7SK, tRNA and U6 promoters. Promoters with similar or dissimilar strengths can be selected to tune the expression of components. Sequences encoding a Cas9 molecule can comprise a nuclear localization signal (NLS), e.g., an SV40 NLS. In an embodiment, the sequence encoding a Cas9 molecule comprises at least two nuclear localization signals. In an embodiment a promoter for a Cas9 molecule or a gRNA molecule can be, independently, inducible, tissue specific, or cell specific.

**Table 31** provides examples of how the components can be formulated, delivered, or administered.

**Table 31**

| Elements | | | |
|---|---|---|---|
| Cas9 Molecule(s) | gRNA Molecule(s) | Donor Template Nucleic Acid | Comments |
| DNA | DNA | DNA | In this embodiment, a Cas9 molecule, typically an eaCas9 molecule, and a gRNA are transcribed from DNA. In this embodiment, they are encoded on separate molecules. In this embodiment, the donor template is provided as a separate DNA molecule. |
| DNA | DNA | | In this embodiment, a Cas9 molecule, typically an eaCas9 molecule, and a gRNA are transcribed from DNA. In this embodiment, they are encoded on separate molecules. In this embodiment, the donor template is provided on the same DNA molecule that encodes the gRNA. |
| DNA | | DNA | In this embodiment, a Cas9 molecule, typically an eaCas9 molecule, and a gRNA are transcribed from DNA, here from a single molecule. In this embodiment, the donor template is provided as a separate DNA molecule. |
| DNA | DNA | DNA | In this embodiment, a Cas9 molecule, typically an eaCas9 molecule, and a gRNA are transcribed from DNA. In this embodiment, they are encoded on separate molecules. In this embodiment, the donor template is provided on the same DNA molecule that encodes the Cas9. |
| | | | |
| DNA | RNA | DNA | In this embodiment, a Cas9 molecule, typically an eaCas9 molecule, is transcribed from DNA, and a gRNA is provided as in vitro transcribed or synthesized RNA. In this embodiment, the donor template is provided as a separate DNA molecule. |
| DNA | RNA | DNA | In this embodiment, a Cas9 molecule, typically an eaCas9 molecule, is transcribed from DNA, and a gRNA is provided as in vitro transcribed or synthesized RNA. In this embodiment, the donor template is provided on the same DNA molecule that encodes the Cas9. |
| | | | |
| mRNA | RNA | DNA | In this embodiment, a Cas9 molecule, typically an eaCas9 molecule, is translated from in vitro transcribed mRNA, and a gRNA is provided as in vitro transcribed or synthesized RNA. In this embodiment, the donor template is provided as a DNA molecule. |
| mRNA | DNA | DNA | In this embodiment, a Cas9 molecule, typically an eaCas9 molecule, is translated from in vitro transcribed mRNA, and a gRNA is transcribed from DNA. In this embodiment, the donor template is provided as a separate DNA molecule. |
| mRNA | DNA | | In this embodiment, a Cas9 molecule, typically an eaCas9 molecule, is translated from in vitro transcribed mRNA, and a gRNA is transcribed from DNA. In this embodiment, the donor template is provided on the same DNA molecule that encodes the gRNA. |
| Protein | DNA | DNA | In this embodiment, a Cas9 molecule, typically an eaCas9 molecule, is provided as a protein, and a gRNA is transcribed from DNA. In this embodiment, the donor template is provided as a separate DNA molecule. |
| Protein | DNA | | In this embodiment, a Cas9 molecule, typically an eaCas9 molecule, is provided as a protein, and a gRNA is transcribed from DNA. In this embodiment, the donor template is provided on the same DNA molecule that encodes the gRNA. |
| Protein | RNA | DNA | In this embodiment, an eaCas9 molecule is provided as a protein, and a gRNA is provided as transcribed or synthesized RNA. In this embodiment, the donor template is provided as a DNA molecule. |

**Table 32** summarizes various delivery methods for the components of a Cas system, e.g., the Cas9 molecule component and the gRNA molecule component, as described herein.

**Table 32**

| **Delivery Vector/Mode** | | **Delivery into Non-Dividing Cells** | **Duration of Expression** | **Genome Integration** | **Type of Molecule Delivered** |
|---|---|---|---|---|---|
| **Physical (eg, electroporation, particle gun, Calcium Phosphate transfection, cell compression or squeezing)** | | YES | Transient | NO | Nucleic Acids and Proteins |
| ***Viral*** | **Retrovirus** | NO | Stable | YES | RNA |
| | **Lentivirus** | YES | Stable | YES/NO with modifications | RNA |
| | **Adenovirus** | YES | Transient | NO | DNA |
| | **Adeno-Associated Virus (AAV)** | YES | Stable | NO | DNA |
| | **Vaccinia Virus** | YES | Very Transient | NO | DNA |
| | **Herpes Simplex Virus** | YES | Stable | NO | DNA |
| ***Non-Viral*** | **Cationic Liposomes** | YES | Transient | Depends on what is delivered | Nucleic Acids and Proteins |
| | **Polymeric Nanoparticles** | YES | Transient | Depends on what is delivered | Nucleic Acids and Proteins |
| ***Biological Non-Viral Delivery Vehicles*** | **Attenuated Bacteria** | YES | Transient | NO | Nucleic Acids |
| | **Engineered Bacteriophages** | YES | Transient | NO | Nucleic Acids |
| | **Mammalian Virus-like Particles** | YES | Transient | NO | Nucleic Acids |
| | **Biological liposomes: Erythrocyte Ghosts and Exosomes** | YES | Transient | NO | Nucleic Acids |

### DNA-based Delivery of a Cas9 molecule and/or one or more gRNA molecule

Nucleic acids encoding Cas9 molecules (e.g., eaCas9 molecules), gRNA molecules, a donor template nucleic acid, or any combination (e.g., two or all) thereof, can be administered to subjects or delivered into cells by art-known methods or as described herein. For example, Cas9-encoding and/or gRNA-encoding DNA can be delivered, e.g., by vectors (e.g., viral or non-viral vectors), non-vector based methods (e.g., using naked DNA or DNA complexes), or a combination thereof.

Nucleic acids encoding Cas9 molecules (e.g., eaCas9 molecules) and/or gRNA molecules can be conjugated to molecules promoting uptake by the target cells (e.g., the target cells described herein). Donor template molecules can be conjugated to molecules promoting uptake by the target cells (e.g., the target cells described herein).

In some embodiments, the Cas9- and/or gRNA-encoding DNA is delivered by a vector (e.g., viral vector/virus or plasmid).

A vector can comprise a sequence that encodes a Cas9 molecule and/or a gRNA molecule. A vector can also comprise a sequence encoding a signal peptide (*e.g.,* for nuclear localization, nucleolar localization, mitochondrial localization), fused, e.g., to a Cas9 molecule sequence. For example, ae vector can comprise a nuclear localization sequence (e.g., from SV40) fused to the sequence encoding the Cas9 molecule.

One or more regulatory/control elements, e.g., a promoter, an enhancer, an intron, a polyadenylation signal, a Kozak consensus sequence, internal ribosome entry sites (IRES), a 2A sequence, and splice acceptor or donor can be included in the vectors. In some embodiments, the promoter is recognized by RNA polymerase II (e.g., a CMV promoter). In other embodiments, the promoter is recognized by RNA polymerase III (e.g., a U6 promoter). In some embodiments, the promoter is a regulated promoter (e.g., inducible promoter). In other embodiments, the promoter is a constitutive promoter. In some embodiments, the promoter is a tissue specific promoter. In some embodiments, the promoter is a viral promoter. In other embodiments, the promoter is a non-viral promoter.

In some embodiments, the vector or delivery vehicle is a viral vector (e.g., for generation of recombinant viruses). In some embodiments, the virus is a DNA virus (e.g., dsDNA or ssDNA virus). In another embodiment, the virus is an RNA virus (e.g., an ssRNA virus). In some embodiments, the virus infects dividing cells. In other embodiments, the virus infects non-dividing cells. Exemplary viral vectors/viruses include, e.g., retroviruses, lentiviruses, adenovirus, adeno-associated virus (AAV), vaccinia viruses, poxviruses, and herpes simplex viruses.

In some embodiments, the virus infects dividing cells. In other embodiments, the virus infects non-dividing cells. In some embodiments, the virus infects both dividing and non-dividing cells. In some embodiments, the virus can integrate into the host genome. In some embodiments, the virus is engineered to have reduced immunity, e.g., in human. In some embodiments, the virus is replication-competent. In another embodiment, the virus is replication-defective, e.g., having one or more coding regions for the genes necessary for additional rounds of virion replication and/or packaging replaced with other genes or deleted. In some embodiments, the virus causes transient expression of the Cas9 molecule and/or the gRNA molecule. In other embodiments, the virus causes long-lasting, e.g., at least 1 week, 2 weeks, 1 month, 2 months, 3 months, 6 months, 9 months, 1 year, 2 years, or permanent expression, of the Cas9 molecule and/or the gRNA molecule. The packaging capacity of the viruses may vary, e.g., from at least about 4 kb to at least about 30 kb, e.g., at least about 5 kb, 10 kb, 15 kb, 20 kb, 25 kb, 30 kb, 35 kb, 40 kb, 45 kb, or 50 kb.

In an embodiment, the viral vector recognizes a specific cell type or tissue. For example, the viral vector can be pseudotyped with a different/alternative viral envelope glycoprotein; engineered with a cell type-specific receptor (e.g., genetic modification(s) of one or more viral envelope glycoproteins to incorporate a targeting ligand such as a peptide ligand, a single chain antibody, or a growth factor); and/or engineered to have a molecular bridge with dual specificities with one end recognizing a viral glycoprotein and the other end recognizing a moiety of the target cell surface (e.g., a ligand-receptor, monoclonal antibody, avidin-biotin and chemical conjugation).

Exemplary viral vectors/viruses include, e.g., retroviruses, lentiviruses, adenovirus, adeno-associated virus (AAV), vaccinia viruses, poxviruses, and herpes simplex viruses.

In some embodiments, the Cas9- and/or gRNA-encoding DNA is delivered by a recombinant retrovirus. In some embodiments, the retrovirus (e.g., Moloney murine leukemia virus) comprises a reverse transcriptase, e.g., that allows integration into the host genome. In some embodiments, the retrovirus is replication-competent. In other embodiments, the retrovirus is replication-defective, e.g., having one of more coding regions for the genes necessary for additional rounds of virion replication and packaging replaced with other genes, or deleted.

In some embodiments, the Cas9- and/or gRNA-encoding DNA is delivered by a recombinant lentivirus. For example, the lentivirus is replication-defective, e.g., does not comprise one or more genes required for viral replication.

In some embodiments, the Cas9- and/or gRNA-encoding DNA is delivered by a recombinant adenovirus. In some embodiments, the adenovirus is engineered to have reduced immunity in human.

In some embodiments, the Cas9- and/or gRNA-encoding DNA is delivered by a recombinant AAV. In some embodiments, the AAV does not incorporate its genome into that of a host cell, e.g., a target cell as describe herein. In some embodiments, the AAV can incorporate at least part of its genome into that of a host cell, e.g., a target cell as described herein. In some embodiments, the AAV is a self-complementary adeno-associated virus (scAAV), e.g., a scAAV that packages both strands which anneal together to form double stranded DNA. AAV serotypes that may be used in the disclosed methods, include AAV1, AAV2, modified AAV2 (e.g., modifications at Y444F, Y500F, Y730F and/or S662V), AAV3, modified AAV3 (e.g., modifications at Y705F, Y731F and/or T492V), AAV4, AAV5, AAV6, modified AAV6 (e.g., modifications at S663V and/or T492V), AAV8, AAV 8.2, AAV9, AAV rh 10, and pseudotyped AAV, such as AAV2/8, AAV2/5 and AAV2/6 can also be used in the disclosed methods. In an embodiment, an AAV capsid that can be used in the methods described herein is a capsid sequence from serotype AAV1, AAV2, AAV3, AAV4, AAV5, AAV6, AAV7, AAV8, AAV9, AAV.rh8, AAV.rh10, AAV.rh32/33, AAV.rh43, AAV.rh64R1, or AAV7m8.

In an embodiment, the Cas9- and/or gRNA-encoding DNA is delivered in a re-engineered AAV capsid, e.g., with 50% or greater, e.g., 60% or greater, 70% or greater, 80% or greater, 90% or greater, or 95% or greater, sequence homology with a capsid sequence from serotypes AAV1, AAV2, AAV3, AAV4, AAV5, AAV6, AAV7, AAV8, AAV9, AAV.rh8, AAV.rh10, AAV.rh32/33, AAV.rh43, or AAV.rh64R1.

In an embodiment, the Cas9- and/or gRNA-encoding DNA is delivered by a chimeric AAV capsid. Exemplary chimeric AAV capsids include, but are not limited to, AAV9i1, AAV2i8, AAV-DJ, AAV2G9, AAV2i8G9, or AAV8G9.

In an embodiment, the AAV is a self-complementary adeno-associated virus (scAAV), e.g., a scAAV that packages both strands which anneal together to form double stranded DNA.

In some embodiments, the Cas9- and/or gRNA-encoding DNA is delivered by a hybrid virus, e.g., a hybrid of one or more of the viruses described herein. In an embodiment, the hybrid virus is hybrid of an AAV (e.g., of any AAV serotype), with a Bocavirus, B19 virus, porcine AAV, goose AAV, feline AAV, canine AAV, or MVM.

A Packaging cell is used to form a virus particle that is capable of infecting a host or target cell. Such a cell includes a 293 cell, which can package adenovirus, and a ψ2 cell or a PA317 cell, which can package retrovirus. A viral vector used in gene therapy is usually generated by a producer cell line that packages a nucleic acid vector into a viral particle. The vector typically contains the minimal viral sequences required for packaging and subsequent integration into a host or target cell (if applicable), with other viral sequences being replaced by an expression cassette encoding the protein to be expressed. For example, an AAV vector used in gene therapy typically only possesses inverted terminal repeat (ITR) sequences from the AAV genome which are required for packaging and gene expression in the host or target cell. The missing viral functions can be supplied in trans by the packaging cell line and/or plasmid containing E2A, E4, and VA genes from adenovirus, and plasmid encoding *Rep* and *Cap* genes from AAV, as described in "Triple Transfection Protocol." Henceforth, the viral DNA is packaged in a cell line, which contains a helper plasmid encoding the other AAV genes, namely rep and cap, but lacking ITR sequences. In embodiment, the viral DNA is packaged in a producer cell line, which contains E1A and/or E1B genes from adenovirus. The cell line is also infected with adenovirus as a helper. The helper virus (e.g., adenovirus or HSV) or helper plasmid promotes replication of the AAV vector and expression of AAV genes from the helper plasmid with ITRs. The helper plasmid is not packaged in significant amounts due to a lack of ITR sequences. Contamination with adenovirus can be reduced by, e.g., heat treatment to which adenovirus is more sensitive than AAV.

In an embodiment, the viral vector has the ability of cell type and/or tissue type recognition. For example, the viral vector can be pseudotyped with a different/alternative viral envelope glycoprotein; engineered with a cell type-specific receptor (e.g., genetic modification of the viral envelope glycoproteins to incorporate targeting ligands such as a peptide ligand, a single chain antibody, a growth factor); and/or engineered to have a molecular bridge with dual specificities with one end recognizing a viral glycoprotein and the other end recognizing a moiety of the target cell surface (e.g., ligand-receptor, monoclonal antibody, avidin-biotin and chemical conjugation).

In an embodiment, the viral vector achieves cell type specific expression. For example, a tissue-specific promoter can be constructed to restrict expression of the transgene (Cas 9 and gRNA) in only the target cell. The specificity of the vector can also be mediated by microRNA-dependent control of transgene expression. In an embodiment, the viral vector has increased efficiency of fusion of the viral vector and a target cell membrane. For example, a fusion protein such as fusion-competent hemagglutin (HA) can be incorporated to increase viral uptake into cells. In an embodiment, the viral vector has the ability of nuclear localization. For example, a virus that requires the breakdown of the nuclear envelope (during cell division) and therefore will not infect a non-diving cell can be altered to incorporate a nuclear localization peptide in the matrix protein of the virus thereby enabling the transduction of non-proliferating cells.

In some embodiments, the Cas9- and/or gRNA-encoding DNA is delivered by a non-vector based method (e.g., using naked DNA or DNA complexes). For example, the DNA can be delivered, e.g., by organically modified silica or silicate (Ormosil), electroporation, transient cell compression or squeezing (e.g., as described in Lee, et al., Nano Lett 12: 6322-27), gene gun, sonoporation, magnetofection, lipid-mediated transfection, dendrimers, inorganic nanoparticles, calcium phosphates, or a combination thereof.

In an embodiment, delivery via electroporation comprises mixing the cells with the Cas9-and/or gRNA-encoding DNA in a cartridge, chamber or cuvette and applying one or more electrical impulses of defined duration and amplitude. In an embodiment, delivery via electroporation is performed using a system in which cells are mixed with the Cas9-and/or gRNA-encoding DNA in a vessel connected to a device (eg, a pump) which feeds the mixture into a cartridge, chamber or cuvette wherein one or more electrical impulses of defined duration and amplitude are applied, after which the cells are delivered to a second vessel.

In some embodiments, the Cas9- and/or gRNA-encoding DNA is delivered by a combination of a vector and a non-vector based method. In an embodiment, the donor template nucleic acid is delivered by a combination of a vector and a non-vector based method For example, a virosome comprises a liposome combined with an inactivated virus (e.g., HIV or influenza virus), which can result in more efficient gene transfer, e.g., in a respiratory epithelial cell than either a viral or a liposomal method alone.

In an embodiment, the delivery vehicle is a non-viral vector. In an embodiment, the non-viral vector is an inorganic nanoparticle (e.g., attached to the payload to the surface of the nanoparticle). Exemplary inorganic nanoparticles include, e.g., magnetic nanoparticles (e.g., Fe₃MnO₂), or silica. The outer surface of the nanoparticle can be conjugated with a positively charged polymer (e.g., polyethylenimine, polylysine, polyserine) which allows for attachment (e.g., conjugation or entrapment) of payload. In an embodiment, the non-viral vector is an organic nanoparticle (e.g., entrapment of the payload inside the nanoparticle). Exemplary organic nanoparticles include, e.g., SNALP liposomes that contain cationic lipids together with neutral helper lipids which are coated with polyethylene glycol (PEG) and protamine and nucleic acid complex coated with lipid coating.

Exemplary lipids for gene transfer are shown below in **Table 33.**

**Table 33: Lipids Used for Gene Transfer**

| **Lipid** | **Abbreviation** | **Feature** |
|---|---|---|
| 1,2-Dioleoyl-sn-glycero-3-phosphatidylcholine | DOPC | Helper |
| 1,2-Dioleoyl-sn-glycero-3-phosphatidylethanolamine | DOPE | Helper |
| Cholesterol | | Helper |
| N-[1-(2,3-Dioleyloxy)prophyl]*N*,*N*,*N*-trimethylammonium chloride | DOTMA | Cationic |
| 1,2-Dioleoyloxy-3-trimethylammonium-propane | DOTAP | Cationic |
| Dioctadecylamidoglycylspermine | DOGS | Cationic |
| *N*-(3-Aminopropyl)-*N*,*N*-dimethyl-2,3-bis(dodecyloxy)-1-propanaminium bromide | GAP-DLRIE | Cationic |
| Cetyltrimethylammonium bromide | CTAB | Cationic |
| 6-Lauroxyhexyl ornithinate | LHON | Cationic |
| 1-(2,3-Dioleoyloxypropyl)-2,4,6-trimethylpyridinium | 2Oc | Cationic |
| 2,3-Dioleyloxy-*N*-[2(sperminecarboxamido-ethyl]-*N*,*N*-dimethyl-1-propanaminium trifluoroacetate | DOSPA | Cationic |
| 1,2-Dioleyl-3-trimethylammonium-propane | DOPA | Cationic |
| *N*-(2-Hydroxyethyl)-*N*,*N*-dimethyl-2,3-bis(tetradecyloxy)-1-propanaminium bromide | MDRIE | Cationic |
| Dimyristooxypropyl dimethyl hydroxyethyl ammonium bromide | DMRI | Cationic |
| 3β-[*N*-(*N*',*N*'-Dimethylaminoethane)-carbamoyl]cholesterol | DC-Chol | Cationic |
| Bis-guanidium-tren-cholesterol | BGTC | Cationic |
| 1,3-Diodeoxy-2-(6-carboxy-spermyl)-propylamide | DOSPER | Cationic |
| Dimethyloctadecylammonium bromide | DDAB | Cationic |
| Dioctadecylamidoglicylspermidin | DSL | Cationic |
| rac-[(2,3-Dioctadecyloxypropyl)(2-hydroxyethyl)]-dimethylammonium chloride | CLIP-1 | Cationic |
| rac-[2(2,3-Dihexadecyloxypropyl-oxymethyloxy)ethyl]trimethylammonium bromide | CLIP-6 | Cationic |
| Ethyldimyristoylphosphatidylcholine | EDMPC | Cationic |
| 1,2-Distearyloxy-*N*,*N*-dimethyl-3-aminopropane | DSDMA | Cationic |
| 1,2-Dimyristoyl-trimethylammonium propane | DMTAP | Cationic |
| *O*,*O*'-Dimyristyl-N-lysyl aspartate | DMKE | Cationic |
| 1,2-Distearoyl-sn-glycero-3-ethylphosphocholine | DSEPC | Cationic |
| *N*-Palmitoyl D-erythro-sphingosyl carbamoyl-spermine | CCS | Cationic |
| *N*-*t*-Butyl-*N*0-tetradecyl-3-tetradecylaminopropionamidine | diC14-amidine | Cationic |
| Octadecenolyoxy[ethyl-2-heptadecenyl-3 hydroxyethyl] imidazolinium chloride | DOTIM | Cationic |
| *N*1-Cholesteryloxycarbonyl-3,7-diazanonane-1,9-diamine | CDAN | Cationic |
| 2-(3-[Bis(3-amino-propyl)-amino]propylamino)-*N-*ditetradecylcarbamoylme-ethyl-acetamide | RPR209120 | Cationic |
| 1,2-dilinoleyloxy-3-dimethylaminopropane | DLinDMA | Cationic |
| 2,2-dilinoleyl-4-dimethylaminoethyl-[1,3]-dioxolane | DLin-KC2-DMA | Cationic |
| dilinoleyl-methyl-4-dimethylaminobutyrate | DLin-MC3-DMA | Cationic |

Exemplary polymers for gene transfer are shown below in **Table 34.**

**Table 34: Polymers Used for Gene Transfer**

| **Polymer** | **Abbreviation** |
|---|---|
| Poly(ethylene)glycol | PEG |
| Polyethylenimine | PEI |
| Dithiobis(succinimidylpropionate) | DSP |
| Dimethyl-3,3'-dithiobispropionimidate | DTBP |
| Poly(ethylene imine) biscarbamate | PEIC |
| Poly(L-lysine) | PLL |
| Histidine modified PLL | |
| Poly(*N*-vinylpyrrolidone) | PVP |
| Poly(propylenimine) | PPI |
| Poly(amidoamine) | PAMAM |
| Poly(amido ethylenimine) | SS-PAEI |
| Triethylenetetramine | TETA |
| Poly(β-aminoester) | |
| Poly(4-hydroxy-L-proline ester) | PHP |
| Poly(allylamine) | |
| Poly(α-[4-aminobutyl]-L-glycolic acid) | PAGA |
| Poly(D,L-lactic-co-glycolic acid) | PLGA |
| Poly(*N*-ethyl-4-vinylpyridinium bromide) | |
| Poly(phosphazene)s | PPZ |
| Poly(phosphoester)s | PPE |
| Poly(phosphoramidate)s | PPA |
| Poly(*N*-2-hydroxypropylmethacrylamide) | pHPMA |
| Poly (2-(dimethylamino)ethyl methacrylate) | pDMAEMA |
| Poly(2-aminoethyl propylene phosphate) | PPE-EA |
| Chitosan | |
| Galactosylated chitosan | |
| *N*-Dodacylated chitosan | |
| Histone | |
| Collagen | |
| Dextran-spermine | D-SPM |

In an embodiment, the vehicle has targeting modifications to increase target cell update of nanoparticles and liposomes, e.g., cell specific antigens, monoclonal antibodies, single chain antibodies, aptamers, polymers, sugars and cell penetrating peptides. In an embodiment, the vehicle uses fusogenic and endosome-destabilizing peptides/polymers. In an embodiment, the vehicle undergoes acid-triggered conformational changes (e.g., to accelerate endosomal escape of the cargo). In an embodiment, a stimuli-cleavable polymer is used, e.g., for release in a cellular compartment. For example, disulfide-based cationic polymers that are cleaved in the reducing cellular environment can be used.

In an embodiment, the delivery vehicle is a biological non-viral delivery vehicle. In an embodiment, the vehicle is an attenuated bacterium (e.g., naturally or artificially engineered to be invasive but attenuated to prevent pathogenesis and expressing the transgene (e.g., *Listeria monocytogenes,* certain *Salmonella strains, Bifidobacterium longum,* and modified *Escherichia coli*), bacteria having nutritional and tissue-specific tropism to target specific tissues, bacteria having modified surface proteins to alter target tissue specificity). In an embodiment, the vehicle is a genetically modified bacteriophage (e.g., engineered phages having large packaging capacity, less immunogenic, containing mammalian plasmid maintenance sequences and having incorporated targeting ligands). In an embodiment, the vehicle is a mammalian virus-like particle. For example, modified viral particles can be generated (e.g., by purification of the "empty" particles followed by *ex vivo* assembly of the virus with the desired cargo). The vehicle can also be engineered to incorporate targeting ligands to alter target tissue specificity. In an embodiment, the vehicle is a biological liposome. For example, the biological liposome is a phospholipid-based particle derived from human cells (e.g., erythrocyte ghosts, which are red blood cells broken down into spherical structures derived from the subject (e.g., tissue targeting can be achieved by attachment of various tissue or cell-specific ligands), or secretory exosomes -subject (i.e., patient) derived membrane-bound nanovescicle (30 -100 nm) of endocytic origin (e.g., can be produced from various cell types and can therefore be taken up by cells without the need of for targeting ligands).

In an embodiment, one or more nucleic acid molecules (e.g., DNA molecules) other than the components of a Cas system, e.g., the Cas9 molecule component and/or the gRNA molecule component described herein, are delivered. In an embodiment, the nucleic acid molecule is delivered at the same time as one or more of the components of the Cas system are delivered. In an embodiment, the nucleic acid molecule is delivered before or after (e.g., less than about 30 minutes, 1 hour, 2 hours, 3 hours, 6 hours, 9 hours, 12 hours, 1 day, 2 days, 3 days, 1 week, 2 weeks, or 4 weeks) one or more of the components of the Cas system are delivered. In an embodiment, the nucleic acid molecule is delivered by a different means than one or more of the components of the Cas system, e.g., the Cas9 molecule component and/or the gRNA molecule component, are delivered. The nucleic acid molecule can be delivered by any of the delivery methods described herein. For example, the nucleic acid molecule can be delivered by a viral vector, e.g., an integration-deficient lentivirus, and the Cas9 molecule component and/or the gRNA molecule component can be delivered by electroporation, e.g., such that the toxicity caused by nucleic acids (e.g., DNAs) can be reduced. In an embodiment, the nucleic acid molecule encodes a therapeutic protein, e.g., a protein described herein. In an embodiment, the nucleic acid molecule encodes an RNA molecule, e.g., an RNA molecule described herein.

### Delivery of RNA encoding a Cas9 molecule

RNA encoding Cas9 molecules (e.g., eaCas9 molecules, eiCas9 molecules or eiCas9 fusion proteins) and/or gRNA molecules, can be delivered into cells, e.g., target cells described herein, by art-known methods or as described herein. For example, Cas9-encoding and/or gRNA-encoding RNA can be delivered, e.g., by microinjection, electroporation, transient cell compression or squeezing (e.g., as described in Lee, et al., Nano Lett 12: 6322-27), lipid-mediated transfection, peptide-mediated delivery, or a combination thereof. Cas9-encoding and/or gRNA-encoding RNA can be conjugated to molecules promoting uptake by the target cells (e.g., target cells described herein).

In an embodiment, delivery via electroporation comprises mixing the cells with the RNA encoding Cas9 molecules (e.g., eaCas9 molecules, eiCas9 molecules or eiCas9 fusion protiens) and/or gRNA molecules, with or without donor template nucleic acid molecules, in a cartridge, chamber or cuvette and applying one or more electrical impulses of defined duration and amplitude. In an embodiment, delivery via electroporation is performed using a system in which cells are mixed with the RNA encoding Cas9 molecules (e.g., eaCas9 molecules, eiCas9 molecules or eiCas9 fusion protiens) and/or gRNA molecules, with or without donor template nucleic acid molecules in a vessel connected to a device (eg, a pump) which feeds the mixture into a cartridge, chamber or cuvette wherein one or more electrical impulses of defined duration and amplitude are applied, after which the cells are delivered to a second vessel. Cas9-encoding and/or gRNA-encoding RNA can be conjugated to molecules to promote uptake by the target cells (e.g., target cells described herein).

### Delivery Cas9 molecule protein

Cas9 molecules (e.g., eaCas9 molecules, eiCas9 molecules or eiCas9 fusion proteins) can be delivered into cells by art-known methods or as described herein. For example, Cas9 protein molecules can be delivered, e.g., by microinjection, electroporation, transient cell compression or squeezing (e.g., as described in Lee, et al., Nano Lett 12: 6322-27), lipid-mediated transfection, peptide-mediated delivery, or a combination thereof. Delivery can be accompanied by DNA encoding a gRNA or by a gRNA. Cas9 protein can be conjugated to molecules promoting uptake by the target cells (e.g., target cells described herein).

In an embodiment, delivery via electroporation comprises mixing the cells with the Cas9 molecules (e.g., eaCas9 molecules, eiCas9 molecules or eiCas9 fusion protiens) and/or gRNA molecules, with or without donor nucleic acid, in a cartridge, chamber or cuvette and applying one or more electrical impulses of defined duration and amplitude. In an embodiment, delivery via electroporation is performed using a system in which cells are mixed with the Cas9 molecules (e.g., eaCas9 molecules, eiCas9 molecules or eiCas9 fusion protiens) and/or gRNA molecules, with or without donor nucleic acid in a vessel connected to a device (eg, a pump) which feeds the mixture into a cartridge, chamber or cuvette wherein one or more electrical impulses of defined duration and amplitude are applied, after which the cells are delivered to a second vessel. Cas9-encoding and/or gRNA-encoding RNA can be conjugated to molecules to promote uptake by the target cells (e.g., target cells described herein).

### Route of Administration

Systemic modes of administration include oral and parenteral routes. Parenteral routes include, by way of example, intravenous, intrarterial, intraosseous, intramuscular, intradermal, subcutaneous, intranasal and intraperitoneal routes. Components administered systemically may be modified or formulated to target the components to the eye.

Local modes of administration include, by way of example, intraocular, intraorbital, subconjuctival, intravitreal, subretinal or transscleral routes, as well as delivery directly into the trabecular meshwork.. In an embodiment, significantly smaller amounts of the components (compared with systemic approaches) may exert an effect when administered locally (for example, intravitreally) compared to when administered systemically (for example, intravenously). Local modes of administration can reduce or eliminate the incidence of potentially toxic side effects that may occur when therapeutically effective amounts of a component are administered systemically.

In an embodiment, components described herein are delivered subretinally, e.g., by subretinal injection. Subretinal injections may be made directly into the macular, e.g., submacular injection.

In an embodiment, components described herein are delivered by intravitreal injection. Intravitreal injection has a relatively low risk of retinal detachment risk. In an embodiment, nanoparticle or viral, e.g., AAV vector, e.g., an AAV2 vector, e.g., a modified AAV2 vector, is delivered intravitreally.

Methods for administration of agents to the eye are known in the medical arts and can be used to administer components described herein. Exemplary methods include intraocular injection (e.g., retrobulbar, subretinal, submacular, intravitreal and intrachoridal), iontophoresis, eye drops, and intraocular implantation (e.g., intravitreal, sub-Tenons and subconjunctival).

Administration may be provided as a periodic bolus (for example, subretinally, intravenously or intravitreally) or as continuous infusion from an internal reservoir (for example, from an implant disposed at an intra- or extra-ocular location (see, U.S. Pat. Nos. 5,443,505 and 5,766,242)) or from an external reservoir (for example, from an intravenous bag). Components may be administered locally, for example, by continuous release from a sustained release drug delivery device immobilized to an inner wall of the eye or via targeted transscleral controlled release into the choroid (see, for example, PCT/US00/00207, PCT/US02/14279, Ambati et al. (2000) INVEST. OPHTHALMOL. VIS. SCI.41:1181-1185, and Ambati et al. (2000) INVEST. OPHTHALMOL. VIS. SCI.41:1186-1191). A variety of devices suitable for administering components locally to the inside of the eye are known in the art. See, for example, U.S. Pat. Nos. 6,251,090, 6,299,895, 6,416,777, 6,413,540, and PCT/US00/28187.

In addition, components may be formulated to permit release over a prolonged period of time. A release system can include a matrix of a biodegradable material or a material which releases the incorporated components by diffusion. The components can be homogeneously or heterogeneously distributed within the release system. A variety of release systems may be useful, however, the choice of the appropriate system will depend upon rate of release required by a particular application. Both non-degradable and degradable release systems can be used. Suitable release systems include polymers and polymeric matrices, non-polymeric matrices, or inorganic and organic excipients and diluents such as, but not limited to, calcium carbonate and sugar (for example, trehalose). Release systems may be natural or synthetic. However, synthetic release systems are preferred because generally they are more reliable, more reproducible and produce more defined release profiles. The release system material can be selected so that components having different molecular weights are released by diffusion through or degradation of the material.

Representative synthetic, biodegradable polymers include, for example: polyamides such as poly(amino acids) and poly(peptides); polyesters such as poly(lactic acid), poly(glycolic acid), poly(lactic-co-glycolic acid), and poly(caprolactone); poly(anhydrides); polyorthoesters; polycarbonates; and chemical derivatives thereof (substitutions, additions of chemical groups, for example, alkyl, alkylene, hydroxylations, oxidations, and other modifications routinely made by those skilled in the art), copolymers and mixtures thereof. Representative synthetic, non-degradable polymers include, for example: polyethers such as poly(ethylene oxide), poly(ethylene glycol), and poly(tetramethylene oxide); vinyl polymers-polyacrylates and polymethacrylates such as methyl, ethyl, other alkyl, hydroxyethyl methacrylate, acrylic and methacrylic acids, and others such as poly(vinyl alcohol), poly(vinyl pyrolidone), and poly(vinyl acetate); poly(urethanes); cellulose and its derivatives such as alkyl, hydroxyalkyl, ethers, esters, nitrocellulose, and various cellulose acetates; polysiloxanes; and any chemical derivatives thereof (substitutions, additions of chemical groups, for example, alkyl, alkylene, hydroxylations, oxidations, and other modifications routinely made by those skilled in the art), copolymers and mixtures thereof.

Poly(lactide-co-glycolide) microsphere can also be used for intraocular injection. Typically the microspheres are composed of a polymer of lactic acid and glycolic acid, which are structured to form hollow spheres. The spheres can be approximately 15-30 microns in diameter and can be loaded with components described herein.

### Bi-Modal or Differential Delivery of Components

Separate delivery of the components of a Cas system, e.g., the Cas9 molecule component and the gRNA molecule component, and more particularly, delivery of the components by differing modes, can enhance performance, e.g., by improving tissue specificity and safety.

In an embodiment, the Cas9 molecule and the gRNA molecule are delivered by different modes, or as sometimes referred to herein as differential modes. Different or differential modes, as used herein, refer modes of delivery that confer different pharmacodynamic or pharmacokinetic properties on the subject component molecule, e.g., a Cas9 molecule, gRNA molecule, or template nucleic acid. For example, the modes of delivery can result in different tissue distribution, different half-life, or different temporal distribution, e.g., in a selected compartment, tissue, or organ.

Some modes of delivery, e.g., delivery by a nucleic acid vector that persists in a cell, or in progeny of a cell, e.g., by autonomous replication or insertion into cellular nucleic acid, result in more persistent expression of and presence of a component. Examples include viral, e.g., adeno- associated virus or lentivirus, delivery.

By way of example, the components, e.g., a Cas9 molecule and a gRNA molecule, can be delivered by modes that differ in terms of resulting half-life or persistent of the delivered component the body, or in a particular compartment, tissue or organ. In an embodiment, a gRNA molecule can be delivered by such modes. The Cas9 molecule component can be delivered by a mode which results in less persistence or less exposure to the body or a particular compartment or tissue or organ.

More generally, in an embodiment, a first mode of delivery is used to deliver a first component and a second mode of delivery is used to deliver a second component. The first mode of delivery confers a first pharmacodynamic or pharmacokinetic property. The first pharmacodynamic property can be, e.g., distribution, persistence, or exposure, of the component, or of a nucleic acid that encodes the component, in the body, a compartment, tissue or organ. The second mode of delivery confers a second pharmacodynamic or pharmacokinetic property. The second pharmacodynamic property can be, e.g., distribution, persistence, or exposure, of the component, or of a nucleic acid that encodes the component, in the body, a compartment, tissue or organ.

In an embodiment, the first pharmacodynamic or pharmacokinetic property, e.g., distribution, persistence or exposure, is more limited than the second pharmacodynamic or pharmacokinetic property.

In an embodiment, the first mode of delivery is selected to optimize, e.g., minimize, a pharmacodynamic or pharmacokinetic property, e.g., distribution, persistence or exposure.

In an embodiment, the second mode of delivery is selected to optimize, e.g., maximize, a pharmacodynamic or pharmcokinetic property, e.g., distribution, persistence or exposure.

In an embodiment, the first mode of delivery comprises the use of a relatively persistent element, e.g., a nucleic acid, e.g., a plasmid or viral vector, e.g., an AAV or lentivirus. As such vectors are relatively persistent product transcribed from them would be relatively persistent.

In an embodiment, the second mode of delivery comprises a relatively transient element, e.g., an RNA or protein.

In an embodiment, the first component comprises gRNA, and the delivery mode is relatively persistent, e.g., the gRNA is transcribed from a plasmid or viral vector, e.g., an AAV or lentivirus. Transcription of these genes would be of little physiological consequence because the genes do not encode for a protein product, and the gRNAs are incapable of acting in isolation. The second component, a Cas9 molecule, is delivered in a transient manner, for example as mRNA or as protein, ensuring that the full Cas9 molecule/gRNA molecule complex is only present and active for a short period of time.

Furthermore, the components can be delivered in different molecular form or with different delivery vectors that complement one another to enhance safety and tissue specificity.

Use of differential delivery modes can enhance performance, safety and efficacy. E.g., the likelihood of an eventual off-target modification can be reduced. Delivery of immunogenic components, e.g., Cas9 molecules, by less persistent modes can reduce immunogenicity, as peptides from the bacterially-derived Cas enzyme are displayed on the surface of the cell by MHC molecules. A two-part delivery system can alleviate these drawbacks.

Differential delivery modes can be used to deliver components to different, but overlapping target regions. The formation active complex is minimized outside the overlap of the target regions. Thus, in an embodiment, a first component, e.g., a gRNA molecule is delivered by a first delivery mode that results in a first spatial, e.g., tissue, distribution. A second component, e.g., a Cas9 molecule is delivered by a second delivery mode that results in a second spatial, e.g., tissue, distribution. In an embodiment, the first mode comprises a first element selected from a liposome, nanoparticle, e.g., polymeric nanoparticle, and a nucleic acid, e.g., viral vector. The second mode comprises a second element selected from the group. In an embodiment, the first mode of delivery comprises a first targeting element, e.g., a cell specific receptor or an antibody, and the second mode of delivery does not include that element. In embodiment, the second mode of delivery comprises a second targeting element, e.g., a second cell specific receptor or second antibody.

When the Cas9 molecule is delivered in a virus delivery vector, a liposome, or polymeric nanoparticle, there is the potential for delivery to and therapeutic activity in multiple tissues, when it may be desirable to only target a single tissue. A two-part delivery system can resolve this challenge and enhance tissue specificity. If the gRNA molecule and the Cas9 molecule are packaged in separated delivery vehicles with distinct but overlapping tissue tropism, the fully functional complex is only be formed in the tissue that is targeted by both vectors.

### Ex vivo delivery

In some embodiments, components described in **Table 31** are introduced into cells which are then introduced into the subject e.g., cells are removed from a subject, manipulated ex vivo and then introduced into the subject. Methods of introducing the components can include, e.g., any of the delivery methods described herein, e.g., any of the delivery methods described in **Table 32.**

### VIII. Modified Nucleosides, Nucleotides, and Nucleic Acids

Modified nucleosides and modified nucleotides can be present in nucleic acids, e.g., particularly gRNA, but also other forms of RNA, e.g., mRNA, RNAi, or siRNA. As described herein, "nucleoside" is defined as a compound containing a five-carbon sugar molecule (a pentose or ribose) or derivative thereof, and an organic base, purine or pyrimidine, or a derivative thereof. As described herein, "nucleotide" is defined as a nucleoside further comprising a phosphate group.

Modified nucleosides and nucleotides can include one or more of:
(i) alteration, e.g., replacement, of one or both of the non-linking phosphate oxygens and/or of one or more of the linking phosphate oxygens in the phosphodiester backbone linkage;
(ii) alteration, e.g., replacement, of a constituent of the ribose sugar, e.g., of the 2' hydroxyl on the ribose sugar;
(iii) wholesale replacement of the phosphate moiety with "dephospho" linkers;
(iv) modification or replacement of a naturally occurring nucleobase;
(v) replacement or modification of the ribose-phosphate backbone;
(vi) modification of the 3' end or 5' end of the oligonucleotide, e.g., removal, modification or replacement of a terminal phosphate group or conjugation of a moiety; and
(vii) modification of the sugar.

The modifications listed above can be combined to provide modified nucleosides and nucleotides that can have two, three, four, or more modifications. For example, a modified nucleoside or nucleotide can have a modified sugar and a modified nucleobase. In an embodiment, every base of a gRNA is modified, e.g., all bases have a modified phosphate group, e.g., all are phosphorothioate groups. In an embodiment, all, or substantially all, of the phosphate groups of a unimolecular or modular gRNA molecule are replaced with phosphorothioate groups.

In an embodiment, modified nucleotides, e.g., nucleotides having modifications as described herein, can be incorporated into a nucleic acid, e.g., a "modified nucleic acid." In some embodiments, the modified nucleic acids comprise one, two, three or more modified nucleotides. In some embodiments, at least 5% (e.g., at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100%) of the positions in a modified nucleic acid are a modified nucleotides.

Unmodified nucleic acids can be prone to degradation by, e.g., cellular nucleases. For example, nucleases can hydrolyze nucleic acid phosphodiester bonds. Accordingly, in one aspect the modified nucleic acids described herein can contain one or more modified nucleosides or nucleotides, e.g., to introduce stability toward nucleases.

In some embodiments, the modified nucleosides, modified nucleotides, and modified nucleic acids described herein can exhibit a reduced innate immune response when introduced into a population of cells, both *in vivo* and *ex vivo.* The term "innate immune response" includes a cellular response to exogenous nucleic acids, including single stranded nucleic acids, generally of viral or bacterial origin, which involves the induction of cytokine expression and release, particularly the interferons, and cell death. In some embodiments, the modified nucleosides, modified nucleotides, and modified nucleic acids described herein can disrupt binding of a major groove interacting partner with the nucleic acid. In some embodiments, the modified nucleosides, modified nucleotides, and modified nucleic acids described herein can exhibit a reduced innate immune response when introduced into a population of cells, both *in vivo* and *ex vivo,* and also disrupt binding of a major groove interacting partner with the nucleic acid.

### Definitions of Chemical Groups

As used herein, "alkyl" is meant to refer to a saturated hydrocarbon group which is straight-chained or branched. Example alkyl groups include methyl (Me), ethyl (Et), propyl (*e.g.,* n-propyl and isopropyl), butyl (*e.g.,* n-butyl, isobutyl, t-butyl), pentyl (*e.g.,* n-pentyl, isopentyl, neopentyl), and the like. An alkyl group can contain from 1 to about 20, from 2 to about 20, from 1 to about 12, from 1 to about 8, from 1 to about 6, from 1 to about 4, or from 1 to about 3 carbon atoms.

As used herein, "aryl" refers to monocyclic or polycyclic (*e.g.,* having 2, 3 or 4 fused rings) aromatic hydrocarbons such as, for example, phenyl, naphthyl, anthracenyl, phenanthrenyl, indanyl, indenyl, and the like. In some embodiments, aryl groups have from 6 to about 20 carbon atoms.

As used herein, "alkenyl" refers to an aliphatic group containing at least one double bond.

As used herein, "alkynyl" refers to a straight or branched hydrocarbon chain containing 2-12 carbon atoms and characterized in having one or more triple bonds. Examples of alkynyl groups include, but are not limited to, ethynyl, propargyl, and 3-hexynyl.

As used herein, "arylalkyl" or "aralkyl" refers to an alkyl moiety in which an alkyl hydrogen atom is replaced by an aryl group. Aralkyl includes groups in which more than one hydrogen atom has been replaced by an aryl group. Examples of "arylalkyl" or "aralkyl" include benzyl, 2-phenylethyl, 3-phenylpropyl, 9-fluorenyl, benzhydryl, and trityl groups.

As used herein, "cycloalkyl" refers to a cyclic, bicyclic, tricyclic, or polycyclic nonaromatic hydrocarbon groups having 3 to 12 carbons. Examples of cycloalkyl moieties include, but are not limited to, cyclopropyl, cyclopentyl, and cyclohexyl.

As used herein, "heterocyclyl" refers to a monovalent radical of a heterocyclic ring system. Representative heterocyclyls include, without limitation, tetrahydrofuranyl, tetrahydrothienyl, pyrrolidinyl, pyrrolidonyl, piperidinyl, pyrrolinyl, piperazinyl, dioxanyl, dioxolanyl, diazepinyl, oxazepinyl, thiazepinyl, and morpholinyl.

As used herein, "heteroaryl" refers to a monovalent radical of a heteroaromatic ring system. Examples of heteroaryl moieties include, but are not limited to, imidazolyl, oxazolyl, thiazolyl, triazolyl, pyrrolyl, furanyl, indolyl, thiophenyl pyrazolyl, pyridinyl, pyrazinyl, pyridazinyl, pyrimidinyl, indolizinyl, purinyl, naphthyridinyl, quinolyl, and pteridinyl.

### Phosphate Backbone Modifications

### The Phosphate Group

In some embodiments, the phosphate group of a modified nucleotide can be modified by replacing one or more of the oxygens with a different substituent. Further, the modified nucleotide, e.g., modified nucleotide present in a modified nucleic acid, can include the wholesale replacement of an unmodified phosphate moiety with a modified phosphate as described herein. In some embodiments, the modification of the phosphate backbone can include alterations that result in either an uncharged linker or a charged linker with unsymmetrical charge distribution.

Examples of modified phosphate groups include, phosphorothioate, phosphoroselenates, borano phosphates, borano phosphate esters, hydrogen phosphonates, phosphoroamidates, alkyl or aryl phosphonates and phosphotriesters. In some embodiments, one of the non-bridging phosphate oxygen atoms in the phosphate backbone moiety can be replaced by any of the following groups: sulfur (S), selenium (Se), BR₃ (wherein R can be, e.g., hydrogen, alkyl, or aryl), C (e.g., an alkyl group, an aryl group, and the like), H, NR₂ (wherein R can be, e.g., hydrogen, alkyl, or aryl), or OR (wherein R can be, e.g., alkyl or aryl). The phosphorous atom in an unmodified phosphate group is achiral. However, replacement of one of the non-bridging oxygens with one of the above atoms or groups of atoms can render the phosphorous atom chiral; that is to say that a phosphorous atom in a phosphate group modified in this way is a stereogenic center. The stereogenic phosphorous atom can possess either the "R" configuration (herein Rp) or the "S" configuration (herein Sp).

Phosphorodithioates have both non-bridging oxygens replaced by sulfur. The phosphorus center in the phosphorodithioates is achiral which precludes the formation of oligoribonucleotide diastereomers. In some embodiments, modifications to one or both non-bridging oxygens can also include the replacement of the non-bridging oxygens with a group independently selected from S, Se, B, C, H, N, and OR (R can be, e.g., alkyl or aryl).

The phosphate linker can also be modified by replacement of a bridging oxygen, (i.e., the oxygen that links the phosphate to the nucleoside), with nitrogen (bridged phosphoroamidates), sulfur (bridged phosphorothioates) and carbon (bridged methylenephosphonates). The replacement can occur at either linking oxygen or at both of the linking oxygens.

### Replacement of the Phosphate Group

The phosphate group can be replaced by non-phosphorus containing connectors. In some embodiments, the charge phosphate group can be replaced by a neutral moiety.

Examples of moieties which can replace the phosphate group can include, without limitation, e.g., methyl phosphonate, hydroxylamino, siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino.

### Replacement of the Ribophosphate Backbone

Scaffolds that can mimic nucleic acids can also be constructed wherein the phosphate linker and ribose sugar are replaced by nuclease resistant nucleoside or nucleotide surrogates. In some embodiments, the nucleobases can be tethered by a surrogate backbone. Examples can include, without limitation, the morpholino, cyclobutyl, pyrrolidine and peptide nucleic acid (PNA) nucleoside surrogates.

### Sugar Modifications

The modified nucleosides and modified nucleotides can include one or more modifications to the sugar group. For example, the 2' hydroxyl group (OH) can be modified or replaced with a number of different "oxy" or "deoxy" substituents. In some embodiments, modifications to the 2' hydroxyl group can enhance the stability of the nucleic acid since the hydroxyl can no longer be deprotonated to form a 2'-alkoxide ion. The 2'-alkoxide can catalyze degradation by intramolecular nucleophilic attack on the linker phosphorus atom.

Examples of "oxy"-2' hydroxyl group modifications can include alkoxy or aryloxy (OR, wherein "R" can be, e.g., alkyl, cycloalkyl, aryl, aralkyl, heteroaryl or a sugar); polyethyleneglycols (PEG), O(CH₂CH₂O)ₙCH₂CH₂OR wherein R can be, e.g., H or optionally substituted alkyl, and n can be an integer from 0 to 20 (e.g., from 0 to 4, from 0 to 8, from 0 to 10, from 0 to 16, from 1 to 4, from 1 to 8, from 1 to 10, from 1 to 16, from 1 to 20, from 2 to 4, from 2 to 8, from 2 to 10, from 2 to 16, from 2 to 20, from 4 to 8, from 4 to 10, from 4 to 16, and from 4 to 20). In some embodiments, the "oxy"-2' hydroxyl group modification can include "locked" nucleic acids (LNA) in which the 2' hydroxyl can be connected, e.g., by a C₁₋₆ alkylene or C₁₋₆ heteroalkylene bridge, to the 4' carbon of the same ribose sugar, where exemplary bridges can include methylene, propylene, ether, or amino bridges; O-amino (wherein amino can be, e.g., NH₂; alkylamino, dialkylamino, heterocyclyl, arylamino, diarylamino, heteroarylamino, or diheteroarylamino, ethylenediamine, or polyamino) and aminoalkoxy, O(CH₂)ₙ-amino, (wherein amino can be, e.g., NH₂; alkylamino, dialkylamino, heterocyclyl, arylamino, diarylamino, heteroarylamino, or diheteroarylamino, ethylenediamine, or polyamino). In some embodiments, the "oxy"-2' hydroxyl group modification can include the methoxyethyl group (MOE), (OCH₂CH₂OCH₃, e.g., a PEG derivative).

"Deoxy" modifications can include hydrogen (i.e. deoxyribose sugars, e.g., at the overhang portions of partially ds RNA); halo (e.g., bromo, chloro, fluoro, or iodo); amino (wherein amino can be, e.g., NH₂; alkylamino, dialkylamino, heterocyclyl, arylamino, diarylamino, heteroarylamino, diheteroarylamino, or amino acid); NH(CH₂CH₂NH)ₙCH₂CH₂-amino (wherein amino can be, e.g., as described herein), - NHC(O)R (wherein R can be, e.g., alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with e.g., an amino as described herein.

The sugar group can also contain one or more carbons that possess the opposite stereochemical configuration than that of the corresponding carbon in ribose. Thus, a modified nucleic acid can include nucleotides containing e.g., arabinose, as the sugar. The nucleotide "monomer" can have an alpha linkage at the 1' position on the sugar, e.g., alpha-nucleosides. The modified nucleic acids can also include "abasic" sugars, which lack a nucleobase at C-1'. These abasic sugars can also be further modified at one or more of the constituent sugar atoms. The modified nucleic acids can also include one or more sugars that are in the L form, e.g. L-nucleosides.

Generally, RNA includes the sugar group ribose, which is a 5-membered ring having an oxygen. Exemplary modified nucleosides and modified nucleotides can include, without limitation, replacement of the oxygen in ribose (e.g., with sulfur (S), selenium (Se), or alkylene, such as, e.g., methylene or ethylene); addition of a double bond (e.g., to replace ribose with cyclopentenyl or cyclohexenyl); ring contraction of ribose (e.g., to form a 4-membered ring of cyclobutane or oxetane); ring expansion of ribose (e.g., to form a 6- or 7-membered ring having an additional carbon or heteroatom, such as for example, anhydrohexitol, altritol, mannitol, cyclohexanyl, cyclohexenyl, and morpholino that also has a phosphoramidate backbone). In some embodiments, the modified nucleotides can include multicyclic forms (e.g., tricyclo; and "unlocked" forms, such as glycol nucleic acid (GNA) (e.g., R-GNA or S-GNA, where ribose is replaced by glycol units attached to phosphodiester bonds), threose nucleic acid (TNA, where ribose is replaced with α-L-threofuranosyl-(3'→2')).

### Modifications on the Nucleobase

The modified nucleosides and modified nucleotides described herein, which can be incorporated into a modified nucleic acid, can include a modified nucleobase. Examples of nucleobases include, but are not limited to, adenine (A), guanine (G), cytosine (C), and uracil (U). These nucleobases can be modified or wholly replaced to provide modified nucleosides and modified nucleotides that can be incorporated into modified nucleic acids. The nucleobase of the nucleotide can be independently selected from a purine, a pyrimidine, a purine or pyrimidine analog. In some embodiments, the nucleobase can include, for example, naturally-occurring and synthetic derivatives of a base.

### Uracil

In some embodiments, the modified nucleobase is a modified uracil. Exemplary nucleobases and nucleosides having a modified uracil include without limitation pseudouridine (ψ), pyridin-4-one ribonucleoside, 5-aza-uridine, 6-aza-uridine, 2-thio-5-aza-uridine, 2-thio-uridine (s2U), 4-thio-uridine (s4U), 4-thio-pseudouridine, 2-thio-pseudouridine, 5-hydroxy-uridine (ho⁵U), 5-aminoallyl-uridine, 5-halo-uridine (e.g., 5-iodo-uridine or 5-bromo-uridine), 3-methyl-uridine (m³U), 5-methoxy-uridine (mo⁵U), uridine 5-oxyacetic acid (cmo⁵U), uridine 5-oxyacetic acid methyl ester (mcmo⁵U), 5-carboxymethyl-uridine (cm⁵U), 1-carboxymethyl-pseudouridine, 5-carboxyhydroxymethyl-uridine (chm⁵U), 5-carboxyhydroxymethyl-uridine methyl ester (mchm⁵U), 5-methoxycarbonylmethyl-uridine (mcm⁵U), 5-methoxycarbonylmethyl-2-thio-uridine (mcm⁵s2U), 5-aminomethyl-2-thio-uridine (nm5s2U), 5-methylaminomethyl-uridine (mnm⁵U), 5-methylaminomethyl-2-thio-uridine (mnm⁵s2U), 5-methylaminomethyl-2-seleno-uridine (mnm⁵se²U), 5-carbamoylmethyl-uridine (ncm⁵U), 5-carboxymethylaminomethyl-uridine (cmnm⁵U), 5-carboxymethylaminomethyl-2-thio-uridine (cmnm⁵s2U), 5-propynyl-uridine, 1-propynyl-pseudouridine, 5-taurinomethyl-uridine (τcm⁵U), 1-taurinomethyl-pseudouridine, 5-taurinomethyl-2-thio-uridine(τm⁵s2U), 1-taurinomethyl-4-thio-pseudouridine, 5-methyl-uridine (m⁵U, i.e., having the nucleobase deoxythymine), 1-methyl-pseudouridine (m¹ψ), 5-methyl-2-thio-uridine (m⁵s2U), 1-methyl-4-thio-pseudouridine (m¹s⁴ψ), 4-thio-1-methyl-pseudouridine, 3-methyl-pseudouridine (m³ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m⁵D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp³U), 1-methyl-3-(3-amino-3-carboxypropyl)pseudouridine (acp³ψ), 5-(isopentenylaminomethyl)uridine (inm⁵U), 5-(isopentenylaminomethyl)-2-thio-uridine (inm⁵s2U), α-thio-uridine, 2'-O-methyl-uridine (Um), 5,2'-O-dimethyl-uridine (m⁵Um), 2'-O-methyl-pseudouridine (ψm), 2-thio-2'-O-methyl-uridine (s2Um), 5-methoxycarbonylmethyl-2'-O-methyl-uridine (mcm⁵Um), 5-carbamoylmethyl-2'-O-methyl-uridine (ncm ⁵Um), 5-carboxymethylaminomethyl-2'-O-methyl-uridine (cmnm ⁵Um), 3,2'-O-dimethyl-uridine (m³Um), 5-(isopentenylaminomethyl)-2'-O-methyl-uridine (inm ⁵Um), 1-thio-uridine, deoxythymidine, 2'-F-ara-uridine, 2'-F-uridine, 2'-OH-ara-uridine, 5-(2-carbomethoxyvinyl) uridine, 5-[3-(1-E-propenylamino)uridine, pyrazolo[3,4-d]pyrimidines, xanthine, and hypoxanthine.

### Cytosine

In some embodiments, the modified nucleobase is a modified cytosine. Exemplary nucleobases and nucleosides having a modified cytosine include without limitation 5-aza-cytidine, 6-aza-cytidine, pseudoisocytidine, 3-methyl-cytidine (m³C), N4-acetyl-cytidine (act), 5-formyl-cytidine (f⁵C), N4-methyl-cytidine (m⁴C), 5-methyl-cytidine (m⁵C), 5-halo-cytidine (e.g., 5-iodo-cytidine), 5-hydroxymethyl-cytidine (hm⁵C), 1-methyl-pseudoisocytidine, pyrrolo-cytidine, pyrrolo-pseudoisocytidine, 2-thio-cytidine (s2C), 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, lysidine (k²C), α-thio-cytidine, 2'-O-methyl-cytidine (Cm), 5,2'-O-dimethyl-cytidine (m⁵Cm), N4-acetyl-2'-O-methyl-cytidine (ac⁴Cm), N4,2'-O-dimethyl-cytidine (m⁴Cm), 5-formyl-2'-O-methyl-cytidine (f⁵Cm), N4,N4,2'-O-trimethyl-cytidine (m⁴₂Cm), 1-thio-cytidine, 2'-F-ara-cytidine, 2'-F-cytidine, and 2'-OH-ara-cytidine.

### Adenine

In some embodiments, the modified nucleobase is a modified adenine. Exemplary nucleobases and nucleosides having a modified adenine include without limitation 2-amino-purine, 2,6-diaminopurine, 2-amino-6-halo-purine (e.g., 2-amino-6-chloro-purine), 6-halo-purine (e.g., 6-chloro-purine), 2-amino-6-methyl-purine, 8-azido-adenosine, 7-deaza-adenosine, 7-deaza-8-aza-adenosine, 7-deaza-2-amino-purine, 7-deaza-8-aza-2-amino-purine, 7-deaza-2,6-diaminopurine, 7-deaza-8-aza-2,6-diaminopurine, 1-methyl-adenosine (m¹A), 2-methyl-adenosine (m²A), N6-methyl-adenosine (m⁶A), 2-methylthio-N6-methyl-adenosine (ms2m⁶A), N6-isopentenyl-adenosine (i⁶A), 2-methylthio-N6-isopentenyl-adenosine (ms²i⁶A), N6-(cis-hydroxyisopentenyl)adenosine (io6A), 2-methylthio-N6-(cis-hydroxyisopentenyl)adenosine (ms2io⁶A), N6-glycinylcarbamoyl-adenosine (g6A), N6-threonylcarbamoyl-adenosine (t⁶A), N6-methyl-N6-threonylcarbamoyl-adenosine (m⁶t⁶A), 2-methylthio-N6-threonylcarbamoyl-adenosine (ms²g⁶A), N6,N6-dimethyl-adenosine (m⁶₂A), N6-hydroxynorvalylcarbamoyl-adenosine (hn⁶A), 2-methylthio-N6-hydroxynorvalylcarbamoyl-adenosine (ms2hn⁶A), N6-acetyl-adenosine (ac⁶A), 7-methyl-adenosine, 2-methylthio-adenosine, 2-methoxy-adenosine, α-thio-adenosine, 2'-O-methyl-adenosine (Am), N⁶,2'-O-dimethyl-adenosine (m⁶Am), N⁶-Methyl-2'-deoxyadenosine, N6,N6,2'-O-trimethyl-adenosine (m⁶₂Am), 1,2'-O-dimethyl-adenosine (m¹Am), 2'-O-ribosyladenosine (phosphate) (Ar(p)), 2-amino-N6-methyl-purine, 1-thio-adenosine, 8-azido-adenosine, 2'-F-ara-adenosine, 2'-F-adenosine, 2'-OH-ara-adenosine, and N6-(19-amino-pentaoxanonadecyl)-adenosine.

### Guanine

In some embodiments, the modified nucleobase is a modified guanine. Exemplary nucleobases and nucleosides having a modified guanine include without limitation inosine (I), 1-methyl-inosine (m¹I), wyosine (imG), methylwyosine (mimG), 4-demethyl-wyosine (imG-14), isowyosine (imG2), wybutosine (yW), peroxywybutosine (o₂yW), hydroxywybutosine (OHyW), undermodified hydroxywybutosine (OHyW*), 7-deaza-guanosine, queuosine (Q), epoxyqueuosine (oQ), galactosyl-queuosine (galQ), mannosyl-queuosine (manQ), 7-cyano-7-deaza-guanosine (preQ₀), 7-aminomethyl-7-deaza-guanosine (preQ₁), archaeosine (G⁺), 7-deaza-8-aza-guanosine, 6-thio-guanosine, 6-thio-7-deaza-guanosine, 6-thio-7-deaza-8-aza-guanosine, 7-methyl-guanosine (m⁷G), 6-thio-7-methyl-guanosine, 7-methyl-inosine, 6-methoxy-guanosine, 1-methyl-guanosine (m'G), N2-methyl-guanosine (m²G), N2,N2-dimethyl-guanosine (m²₂G), N2,7-dimethyl-guanosine (m²,7G), N2, N2,7-dimethyl-guanosine (m²,2,7G), 8-oxo-guanosine, 7-methyl-8-oxo-guanosine, 1-methyl-6-thio-guanosine, N2-methyl-6-thio-guanosine, N2,N2-dimethyl-6-thio-guanosine, α-thio-guanosine, 2'-O-methyl-guanosine (Gm), N2-methyl-2'-O-methyl-guanosine (m²Gm), N2,N2-dimethyl-2'-O-methyl-guanosine (m²₂Gm), 1-methyl-2'-O-methyl-guanosine (m'Gm), N2,7-dimethyl-2'-O-methyl-guanosine (m²,7Gm), 2'-O-methyl-inosine (Im), 1,2'-O-dimethyl-inosine (m'Im), O⁶-phenyl-2'-deoxyinosine, 2'-O-ribosylguanosine (phosphate) (Gr(p)), 1-thio-guanosine, O⁶-methyl-guanosine, O⁶-Methyl-2'-deoxyguanosine, 2'-F-ara-guanosine, and 2'-F-guanosine.

### Exemplary Modified gRNAs

In some embodiments, the modified nucleic acids can be modified gRNAs. It is to be understood that any of the gRNAs described herein can be modified in accordance with this section, including any gRNA that comprises a targeting domain from **Tables 1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E,** or **23A-23B.**

As discussed above, transiently expressed or delivered nucleic acids can be prone to degradation by, e.g., cellular nucleases. Accordingly, in one aspect the modified gRNAs described herein can contain one or more modified nucleosides or nucleotides which introduce stability toward nucleases. While not wishing to be bound by theory it is also believed that certain modified gRNAs described herein can exhibit a reduced innate immune response when introduced into a population of cells, particularly the cells of the present invention. As noted above, the term "innate immune response" includes a cellular response to exogenous nucleic acids, including single stranded nucleic acids, generally of viral or bacterial origin, which involves the induction of cytokine expression and release, particularly the interferons, and cell death.

While some of the exemplary modification discussed in this section may be included at any position within the gRNA sequence, in some embodiments, a gRNA comprises a modification at or near its 5' end (e.g., within 1-10, 1-5, or 1-2 nucleotides of its 5' end). In some embodiments, a gRNA comprises a modification at or near its 3' end (e.g., within 1-10, 1-5, or 1-2 nucleotides of its 3' end). In some embodiments, a gRNA comprises both a modification at or near its 5' end and a modification at or near its 3' end.

In an embodiment, the 5' end of a gRNA is modified by the inclusion of a eukaryotic mRNA cap structure or cap analog (e.g., a *G(5')ppp(5')G* cap analog, a *m7G(5')ppp(5')G* cap analog, or a *3'-O-Me-m7G(5')ppp(5')G* anti reverse cap analog (ARCA)). The cap or cap analog can be included during either chemical synthesis or *in vitro* transcription of the gRNA.

In an embodiment, an *in vitro* transcribed gRNA is modified by treatment with a phosphatase (e.g., calf intestinal alkaline phosphatase) to remove the 5' triphosphate group.

In an embodiment, the 3' end of a gRNA is modified by the addition of one or more (e.g., 25-200) adenine (A) residues. The polyA tract can be contained in the nucleic acid (e.g., plasmid, PCR product, viral genome) encoding the gRNA, or can be added to the gRNA during chemical synthesis, or following *in vitro* transcription using a polyadenosine polymerase (e.g., *E. coli* Poly(A)Polymerase).

In an embodiment, *in vitro* transcribed gRNA contains both a 5' cap structure or cap analog and a 3' polyA tract. In an embodiment, an *in vitro* transcribed gRNA is modified by treatment with a phosphatase (e.g., calf intestinal alkaline phosphatase) to remove the 5' triphosphate group and comprises a 3' polyA tract.

In some embodiments, gRNAs can be modified at a 3' terminal U ribose. For example, the two terminal hydroxyl groups of the U ribose can be oxidized to aldehyde groups and a concomitant opening of the ribose ring to afford a modified nucleoside as shown below: 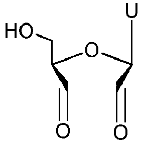 wherein "U" can be an unmodified or modified uridine.

In another embodiment, the 3' terminal U can be modified with a 2'3' cyclic phosphate as shown below: 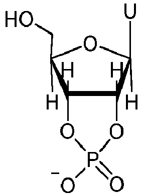 wherein "U" can be an unmodified or modified uridine.

In some embodiments, the gRNA molecules may contain 3' nucleotides which can be stabilized against degradation, e.g., by incorporating one or more of the modified nucleotides described herein. In this embodiment, e.g., uridines can be replaced with modified uridines, e.g., 5-(2-amino)propyl uridine, and 5-bromo uridine, or with any of the modified uridines described herein; adenosines and guanosines can be replaced with modified adenosines and guanosines, e.g., with modifications at the 8-position, e.g., 8-bromo guanosine, or with any of the modified adenosines or guanosines described herein.

In some embodiments, sugar-modified ribonucleotides can be incorporated into the gRNA, e.g., wherein the 2' OH-group is replaced by a group selected from H, -OR, -R (wherein R can be, e.g., alkyl, cycloalkyl, aryl, aralkyl, heteroaryl or sugar), halo, -SH, -SR (wherein R can be, e.g., alkyl, cycloalkyl, aryl, aralkyl, heteroaryl or sugar), amino (wherein amino can be, e.g., NH₂; alkylamino, dialkylamino, heterocyclyl, arylamino, diarylamino, heteroarylamino, diheteroarylamino, or amino acid); or cyano (-CN). In some embodiments, the phosphate backbone can be modified as described herein, e.g., with a phosphothioate group. In some embodiments, one or more of the nucleotides of the gRNA can each independently be a modified or unmodified nucleotide including, but not limited to 2'-sugar modified, such as, 2'-O-methyl, 2'-O-methoxyethyl, or 2'-Fluoro modified including, e.g., 2'-F or 2'-O-methyl, adenosine (A), 2'-F or 2'-O-methyl, cytidine (C), 2'-F or 2'-O-methyl, uridine (U), 2'-F or 2'-O-methyl, thymidine (T), 2'-F or 2'-O-methyl, guanosine (G), 2'-O-methoxyethyl-5-methyluridine (Teo), 2'-O-methoxyethyladenosine (Aeo), 2'-O-methoxyethyl-5-methylcytidine (m5Ceo), and any combinations thereof.

In some embodiments, a gRNA can include "locked" nucleic acids (LNA) in which the 2' OH-group can be connected, e.g., by a C1-6 alkylene or Cl-6 heteroalkylene bridge, to the 4' carbon of the same ribose sugar, where exemplary bridges can include methylene, propylene, ether, or amino bridges; O-amino (wherein amino can be, e.g., NH₂; alkylamino, dialkylamino, heterocyclyl, arylamino, diarylamino, heteroarylamino, or diheteroarylamino, ethylenediamine, or polyamino) and aminoalkoxy or O(CH₂)ₙ-amino (wherein amino can be, e.g., NH₂; alkylamino, dialkylamino, heterocyclyl, arylamino, diarylamino, heteroarylamino, or diheteroarylamino, ethylenediamine, or polyamino).

In some embodiments, a gRNA can include a modified nucleotide which is multicyclic (e.g., tricyclo; and "unlocked" forms, such as glycol nucleic acid (GNA) (e.g., R-GNA or S-GNA, where ribose is replaced by glycol units attached to phosphodiester bonds), or threose nucleic acid (TNA, where ribose is replaced with α-L-threofuranosyl-(3'→2')).

Generally, gRNA molecules include the sugar group ribose, which is a 5-membered ring having an oxygen. Exemplary modified gRNAs can include, without limitation, replacement of the oxygen in ribose (e.g., with sulfur (S), selenium (Se), or alkylene, such as, e.g., methylene or ethylene); addition of a double bond (e.g., to replace ribose with cyclopentenyl or cyclohexenyl); ring contraction of ribose (e.g., to form a 4-membered ring of cyclobutane or oxetane); ring expansion of ribose (e.g., to form a 6- or 7-membered ring having an additional carbon or heteroatom, such as for example, anhydrohexitol, altritol, mannitol, cyclohexanyl, cyclohexenyl, and morpholino that also has a phosphoramidate backbone). Although the majority of sugar analog alterations are localized to the 2' position, other sites are amenable to modification, including the 4' position. In an embodiment, a gRNA comprises a 4'-S, 4'-Se or a 4'-C-aminomethyl-2'-O-Me modification.

In some embodiments, deaza nucleotides, e.g., 7-deaza-adenosine, can be incorporated into the gRNA. In some embodiments, O- and N-alkylated nucleotides, e.g., N6-methyl adenosine, can be incorporated into the gRNA. In some embodiments, one or more or all of the nucleotides in a gRNA molecule are deoxynucleotides.

### miRNA binding sites

microRNAs (or miRNAs) are naturally occurring cellular 19-25 nucleotide long noncoding RNAs. They bind to nucleic acid molecules having an appropriate miRNA binding site, e.g., in the 3' UTR of an mRNA, and down-regulate gene expression. While not wishing to be bound by theory it is believed that the down regulation is either by reducing nucleic acid molecule stability or by inhibiting translation. An RNA species disclosed herein, e.g., an mRNA encoding Cas9 can comprise an miRNA binding site, e.g., in its 3'UTR. The miRNA binding site can be selected to promote down regulation of expression is a selected cell type. By way of example, the incorporation of a binding site for miR-122, a microRNA abundant in liver, can inhibit the expression of the gene of interest in the liver.

### EXAMPLES

The following Examples are merely illustrative and are not intended to limit the scope or content of the invention in any way.

### Example 1: Evaluation of candidate guide RNAs (gRNAs)

The suitability of candidate gRNAs can be evaluated as described in this example. Although described for a chimeric gRNA, the approach can also be used to evaluate modular gRNAs.

### Cloning gRNAs into Vectors

For each gRNA, a pair of overlapping oligonucleotides is designed and obtained. Oligonucleotides are annealed and ligated into a digested vector backbone containing an upstream U6 promoter and the remaining sequence of a long chimeric gRNA. Plasmid is sequence-verified and prepped to generate sufficient amounts of transfection-quality DNA. Alternate promoters maybe used to drive *in vivo* transcription (e.g. HI promoter) or for *in vitro* transcription (e.g., a T7 promoter).

### Cloning gRNAs in linear dsDNA molecule (STITCHR)

For each gRNA, a single oligonucleotide is designed and obtained. The U6 promoter and the gRNA scaffold (e.g. including everything except the targeting domain, e.g., including sequences derived from the crRNA and tracrRNA, e.g., including a first complementarity domain; a linking domain; a second complementarity domain; a proximal domain; and a tail domain) are separately PCR amplified and purified as dsDNA molecules. The gRNA-specific oligonucleotide is used in a PCR reaction to stitch together the U6 and the gRNA scaffold, linked by the targeting domain specified in the oligonucleotide. Resulting dsDNA molecule (STITCHR product) is purified for transfection. Alternate promoters may be used to drive in vivo transcription (e.g., HI promoter) or for in vitro transcription (e.g., T7 promoter). Any gRNA scaffold may be used to create gRNAs compatible with Cas9s from any bacterial species.

### Initial gRNA Screen

Each gRNA to be tested is transfected, along with a plasmid expressing Cas9 and a small amount of a GFP-expressing plasmid into human cells. In preliminary experiments, these cells can be immortalized human cell lines such as 293T, K562 or U2OS. Alternatively, primary human cells may be used. In this case, cells may be relevant to the eventual therapeutic cell target (for example, photoreceptor cells). The use of primary cells similar to the potential therapeutic target cell population may provide important information on gene targeting rates in the context of endogenous chromatin and gene expression.

Transfection may be performed using lipid transfection (such as Lipofectamine or Fugene) or by electroporation (such as Lonza Nucleofection). Following transfection, GFP expression can be determined either by fluorescence microscopy or by flow cytometry to confirm consistent and high levels of transfection. These preliminary transfections can comprise different gRNAs and different targeting approaches (17-mers, 20-mers, nuclease, dual-nickase, etc.) to determine which gRNAs/combinations of gRNAs give the greatest activity.

Efficiency of cleavage with each gRNA may be assessed by measuring NHEJ-induced indel formation at the target locus by a T7E1-type assay or by sequencing. Alternatively, other mismatch-sensitive enzymes, such as Cell/Surveyor nuclease, may also be used.

For the T7E1 assay, PCR amplicons are approximately 500-700bp with the intended cut site placed asymmetrically in the amplicon. Following amplification, purification and size-verification of PCR products, DNA is denatured and re-hybridized by heating to 95°C and then slowly cooling. Hybridized PCR products are then digested with T7 Endonuclease I (or other mismatch-sensitive enzyme) which recognizes and cleaves non-perfectly matched DNA. If indels are present in the original template DNA, when the amplicons are denatured and re-annealed, this results in the hybridization of DNA strands harboring different indels and therefore lead to double-stranded DNA that is not perfectly matched. Digestion products may be visualized by gel electrophoresis or by capillary electrophoresis. The fraction of DNA that is cleaved (density of cleavage products divided by the density of cleaved and uncleaved) may be used to estimate a percent NHEJ using the following equation: %NHEJ = (1-(1-fraction cleaved)^{½}). The T7E1 assay is sensitive down to about 2-5% NHEJ.

Sequencing may be used instead of, or in addition to, the T7E1 assay. For Sanger sequencing, purified PCR amplicons are cloned into a plasmid backbone, transformed, miniprepped and sequenced with a single primer. Sanger sequencing may be used for determining the exact nature of indels after determining the NHEJ rate by T7E1.

Sequencing may also be performed using next generation sequencing techniques. When using next generation sequencing, amplicons may be 300-500bp with the intended cut site placed asymmetrically. Following PCR, next generation sequencing adapters and barcodes (for example Illumina multiplex adapters and indexes) may be added to the ends of the amplicon, e.g., for use in high throughput sequencing (for example on an Illumina MiSeq). This method allows for detection of very low NHEJ rates.

### Example 2: Assessment of Gene Targeting by NHEJ

The gRNAs that induce the greatest levels of NHEJ in initial tests can be selected for further evaluation of gene targeting efficiency. In this case, cells are derived from disease subjects and, therefore, harbor the relevant mutation.

Following transfection (usually 2-3 days post-transfection,) genomic DNA may be isolated from a bulk population of transfected cells and PCR may be used to amplify the target region. Following PCR, gene targeting efficiency to generate the desired mutations (either knockout of a target gene or removal of a target sequence motif) may be determined by sequencing. For Sanger sequencing, PCR amplicons may be 500-700bp long. For next generation sequencing, PCR amplicons may be 300-500bp long. If the goal is to knockout gene function, sequencing may be used to assess what percent of alleles have undergone NHEJ-induced indels that result in a frameshift or large deletion or insertion that would be expected to destroy gene function. If the goal is to remove a specific sequence motif, sequencing may be used to assess what percent of alleles have undergone NHEJ-induced deletions that span this sequence.

### Example 3: Assessment of Gene Targeting by HDR

The gRNAs that induce the greatest levels of NHEJ in initial tests can be selected for further evaluation of gene targeting efficiency. In this case, cells are derived from disease subjects and, therefore, harbor the relevant mutation.

Following transfection (usually 2-3 days post-transfection,) genomic DNA may be isolated from a bulk population of transfected cells and PCR may be used to amplify the target region. Following PCR, gene targeting efficiency can be determined by several methods.

Determination of gene targeting frequency involves measuring the percentage of alleles that have undergone homologous directed repair (HDR) with the donor template and which therefore have incorporated desired correction. If the desired HDR event creates or destroys a restriction enzyme site, the frequency of gene targeting may be determined by a RFLP assay. If no restriction site is created or destroyed, sequencing may be used to determine gene targeting frequency. If a RFLP assay is used, sequencing may still be used to verify the desired HDR event and ensure that no other mutations are present. At least one of the primers is placed in the endogenous gene sequence outside of the region included in the homology arms, which prevents amplification of donor template still present in the cells. Therefore, the length of the homology arms present in the donor template may affect the length of the PCR amplicon. PCR amplicons can either span the entire donor region (both primers placed outside the homology arms) or they can span only part of the donor region and a single junction between donor and endogenous DNA (one internal and one external primer). If the amplicons span less than entire donor region, two different PCRs should be used to amplify and sequence both the 5' and the 3' junction.

If the PCR amplicon is short (less than 600bp) it is possible to use next generation sequencing. Following PCR, next generation sequencing adapters and barcodes (for example Illumina multiplex adapters and indexes) may be added to the ends of the amplicon, e.g., for use in high throughput sequencing (for example on an Illumina MiSeq). This method allows for detection of very low gene targeting rates.

If the PCR amplicon is too long for next generation sequencing, Sanger sequencing can be performed. For Sanger sequencing, purified PCR amplicons will be cloned into a plasmid backbone (for example, TOPO cloned using the LifeTech Zero Blunt® TOPO® cloning kit), transformed, miniprepped and sequenced.

### Incorporation by Reference

All publications, patents, and patent applications mentioned herein are hereby incorporated by reference in their entirety as if each individual publication, patent or patent application was specifically and individually indicated to be incorporated by reference. In case of conflict, the present application, including any definitions herein, will control.

### Equivalents

Those skilled in the art will recognize, or be able to ascertain using no more than routine experimentation, many equivalents to the specific embodiments of the invention described herein. Such equivalents are intended to be encompassed by the following claims. The invention is now defined by the following clauses:
1. A gRNA molecule comprising a targeting domain which is complementary with a target domain from the *MYOC* gene.
2. The gRNA molecule of clause 1, wherein said targeting domain is configured to provide a cleavage event selected from a double strand break and a single strand break, within 500, 400, 300, 200, 100, 50, 25, or 10 nucleotides of a POAG target point position, a POAG target hotspot mutation, or a POAG target knockout position.
3. The gRNA molecule of clause 1, wherein said targeting domain is configured to target an enzymatically inactive Cas9 (eiCas9) or an eiCas9 fusion protein, sufficiently close to a POAG knockdown target position to reduce, decrease or repress expression of the *MYOC* gene.
4. The gRNA molecule of any of clauses 1-3, wherein said targeting domain is configured to target the promoter region of the *MYOC* gene.
5. The gRNA molecule of any of clauses 1-4, wherein said targeting domain comprises a sequence that is the same as, or differs by no more than 3 nucleotides from, a targeting domain sequence from any of Tables 1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E, or 23A-23B.
6. The gRNA molecule of any of clauses 1-5, wherein said targeting domain comprises a sequence that is the same as a targeting domain sequence from any of Tables 1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E, or 23A-23B.
7. The gRNA molecule of any of clauses 1, 2, or 4-6, wherein said targeting domain is selected from those in Tables 1A-1E, 21A-21D, 22A-22E, or 23A-23B.
8. The gRNA molecule of any of clauses 1, 2, or 4-6, wherein said targeting domain is selected from those in Tables 2A-2E, 18A-18D, 19A-19E, or 20A-20D.
9. The gRNA molecule of any of clauses 1, 2, or 4-6, wherein said targeting domain is selected from those in Tables 3A-3E, 12A-12D, 13A-13E, 14A-14C, 15A-15D, 16A-16E, or 17A-17B.
10. The gRNA molecule of any of clauses 1, 2, or 4-6, wherein said targeting domain is selected from those in Tables 4A-4E, 6A-6E, 7A-7G, or 8A-8E.
11. The gRNA molecule of any of clauses 1 or 3-6, wherein said targeting domain is selected from those in Tables 5A-5F, 9A-9E, 10A-10G, or 11A-11E.
12. The gRNA molecule of clause 2, wherein the POAG target point position comprises a mutation at P370 in the *MYOC* gene, or wherein the POAG target hotspot mutation comprises a mutational hotspot between amino acid sequence positions 246-252, 368-380, 368-370 plus 377-380, 364-380, 347-380, 423-437, or 477-502 in the *MYOC* gene.
13. The gRNA molecule of any of clauses 1-12, wherein said gRNA is a modular gRNA molecule.
14. The gRNA molecule of any of clauses 1-12, wherein said gRNA is a chimeric gRNA molecule.
15. The gRNA molecule of any of clauses 1-14, wherein said targeting domain is 16 nucleotides or more in length.
16. The gRNA molecule of any of clauses 1-15, wherein said targeting domain is 17 nucleotides in length.
17. The gRNA molecule of any of clauses 1-16, wherein said targeting domain is 18 nucleotides in length.
18. The gRNA molecule of any of clauses 1-17, wherein said targeting domain is 19 nucleotides in length.
19. The gRNA molecule of any of clauses 1-18, wherein said targeting domain is 20 nucleotides in length.
20. The gRNA molecule of any of clauses 1-19, wherein said targeting domain is 21 nucleotides in length.
21. The gRNA molecule of any of clauses 1-20, wherein said targeting domain is 22 nucleotides in length.
22. The gRNA molecule of any of clauses 1-21, wherein said targeting domain is 23 nucleotides in length.
23. The gRNA molecule of any of clauses 1-22, wherein said targeting domain is 24 nucleotides in length.
24. The gRNA molecule of any of clauses 1-23, wherein said targeting domain is 25 nucleotides in length.
25. The gRNA molecule of any of clauses 1-24, wherein said targeting domain is 26 nucleotides in length.
26. The gRNA molecule of any of clauses 1-25, comprising from 5' to 3':
   a targeting domain;
   a first complementarity domain;
   a linking domain;
   a second complementarity domain;
   a proximal domain; and
   a tail domain.
27. The gRNA molecule of any of clauses 1-26, comprising:
   a linking domain of no more than 25 nucleotides in length;
   a proximal and tail domain, that taken together, are at least 20 nucleotides in length;
   a targeting domain of 17 or 18 nucleotides in length.
28. The gRNA molecule of any of clauses 1-27, comprising:
   a linking domain of no more than 25 nucleotides in length;
   a proximal and tail domain, that taken together, are at least 25 nucleotides in length;
   a targeting domain of 17 or 18 nucleotides in length.
29. The gRNA molecule of any of clauses 1-28, comprising:
   a linking domain of no more than 25 nucleotides in length;
   a proximal and tail domain, that taken together, are at least 30 nucleotides in length;
   a targeting domain of 17 nucleotides in length.
30. The gRNA molecule of any of clauses 1-29, comprising:
   a linking domain of no more than 25 nucleotides in length;
   a proximal and tail domain, that taken together, are at least 40 nucleotides in length;
   a targeting domain of 17 nucleotides in length.
31. A nucleic acid that comprises: (a) sequence that encodes a gRNA molecule comprising a targeting domain that is complementary with a POAG target domain in *MYOC* gene.
32. The nucleic acid of clause 31, wherein said gRNA molecule is a gRNA molecule of any of clauses 1-30.
33. The nucleic acid of clause 31 or 32, wherein said targeting domain is configured to provide a cleavage event selected from a double strand break and a single strand break, within 500, 400, 300, 200, 100, 50, 25, or 10 nucleotides of the POAG target point position, a POAG target hotspot mutation, or a POAG target knockout position.
34. The nucleic acid of clause 31 or 32, wherein said targeting domain is configured to target an enzymatically inactive Cas9 (eiCas9) or an eiCas9 fusion protein, sufficiently close to a POAG knockdown target position to reduce, decrease or repress expression of the *MYOC* gene.
35. The nucleic acid of any of clauses 31-34, wherein said targeting domain is configured to target the promoter region of the *MYOC* gene.
36. The nucleic acid of any of clauses 31-35, wherein said targeting domain comprises a sequence that is the same as, or differs by no more than 3 nucleotides from, a targeting domain sequence from any of Tables 1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E, or 23A-23B.
37. The nucleic acid of any of clauses 31-36, wherein said targeting domain comprises a sequence that is the same as a targeting domain sequence from any of Tables 1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E, or 23A-23B.
38. The nucleic acid of any of clauses 31-37, wherein said gRNA is a modular gRNA molecule.
39. The nucleic acid of any of clauses 31-37, wherein said gRNA is a chimeric gRNA molecule.
40. The nucleic acid of any of clauses 31-39, wherein said targeting domain is 16 nucleotides or more in length.
41. The nucleic acid of any of clauses 31-40, wherein said targeting domain is 17 nucleotides in length.
42. The nucleic acid of any of clauses 31-41, wherein said targeting domain is 18 nucleotides in length.
43. The nucleic acid of any of clauses 31-42, wherein said targeting domain is 19 nucleotides in length.
44. The nucleic acid of any of clauses 31-43, wherein said targeting domain is 20 nucleotides in length.
45. The nucleic acid of any of clauses 31-44, wherein said targeting domain is 21 nucleotides in length.
46. The nucleic acid of any of clauses 31-45, wherein said targeting domain is 22 nucleotides in length.
47. The nucleic acid of any of clauses 31-46, wherein said targeting domain is 23 nucleotides in length.
48. The nucleic acid of any of clauses 31-47, wherein said targeting domain is 24 nucleotides in length.
49. The nucleic acid of any of clauses 31-48, wherein said targeting domain is 25 nucleotides in length.
50. The nucleic acid of any of clauses 31-49, wherein said targeting domain is 26 nucleotides in length.
51. The nucleic acid of any of clauses 31-50, comprising from 5' to 3':
   a targeting domain;
   a first complementarity domain;
   a linking domain;
   a second complementarity domain;
   a proximal domain; and
   a tail domain.
52. The nucleic acid of any of clauses 31-51, comprising:
   a linking domain of no more than 25 nucleotides in length;
   a proximal and tail domain, that taken together, are at least 20 nucleotides in length;
   a targeting domain of 17 or 18 nucleotides in length.
53. The nucleic acid of any of clauses 31-52, comprising:
   a linking domain of no more than 25 nucleotides in length;
   a proximal and tail domain, that taken together, are at least 25 nucleotides in length;
   a targeting domain of 17 or 18 nucleotides in length.
54. The nucleic acid of any of clauses 31-53, comprising:
   a linking domain of no more than 25 nucleotides in length;
   a proximal and tail domain, that taken together, are at least 30 nucleotides in length;
   a targeting domain of 17 nucleotides in length.
55. The nucleic acid of any of clauses 31-54, comprising:
   a linking domain of no more than 25 nucleotides in length;
   a proximal and tail domain, that taken together, are at least 40 nucleotides in length;
   a targeting domain of 17 nucleotides in length.
56. The nucleic acid of any of clauses 31-55, further comprising: (b) sequence that encodes a Cas9 molecule.
57. The nucleic acid of clause 56, wherein said Cas9 molecule is an eaCas9 molecule.
58. The nucleic acid of clause 57, wherein said eaCas9 molecule comprises a nickase molecule.
59. The nucleic acid of clause 57 or 58, wherein said eaCas9 molecule forms a double strand break in a target nucleic acid.
60. The nucleic acid of clause 57 or 58, wherein said eaCas9 molecule forms a single strand break in a target nucleic acid.
61. The nucleic acid of clause 60, wherein said single strand break is formed in the strand of the target nucleic acid to which the targeting domain of said gRNA molecule is complementary.
62. The nucleic acid of clause 60, wherein said single strand break is formed in the strand of the target nucleic acid other than the strand to which to which the targeting domain of said gRNA is complementary.
63. The nucleic acid of clause 57 or 58, wherein said eaCas9 molecule comprises HNH-like domain cleavage activity but has no, or no significant, N-terminal RuvC-like domain cleavage activity.
64. The nucleic acid of clause 57, 58, or 63, wherein said eaCas9 molecule is an HNH-like domain nickase.
65. The nucleic acid of clause 57, 58, or 63, or 64, wherein said eaCas9 molecule comprises a mutation at D10.
66. The nucleic acid of clause 57 or 58, wherein said eaCas9 molecule comprises N-terminal RuvC-like domain cleavage activity but has no, or no significant, HNH-like domain cleavage activity.
67. The nucleic acid of clause 57, 58, or 66, wherein said eaCas9 molecule is an N-terminal RuvC-like domain nickase.
68. The nucleic acid of clause 57, 58, 66, or 67, wherein said eaCas9 molecule comprises a mutation at H840 or N863.
69. The nucleic acid of clause 56, wherein said Cas9 molecule is an eiCas9 molecule.
70. The nucleic acid of clause 56 or 69, wherein said Cas9 molecule is an eiCas9-fusion protein molecule.
71. The nucleic acid of clause 70, wherein the eiCas9 fusion protein molecule is an eiCas9-transcription repressor domain fusion or eiCas9-chromatin modifying protein fusion.
72. The nucleic acid of any of clauses 31-71, further comprising: (c) a sequence that encodes a second gRNA molecule described herein having a targeting domain that is complementary to a second target domain of the *MYOC* gene.
73. The nucleic acid of clause 72, wherein said second gRNA molecule is a gRNA molecule of any of clauses 1-30.
74. The nucleic acid of clause 72 or 73, wherein said targeting domain of said second gRNA is configured to provide a cleavage event selected from a double strand break and a single strand break, within 500, 400, 300, 200, 100, 50, 25, or 10 nucleotides of the POAG target point position, a POAG target hotspot mutation, or a POAG target knockout position.
75. The nucleic acid of clause 72 or 73, wherein said targeting domain of said second gRNA is configured to target an enzymatically inactive Cas9 (eiCas9) or an eiCas9 fusion protein, sufficiently close to a POAG knockdown target position to reduce, decrease or repress expression of the *MYOC* gene.
76. The nucleic acid of any of clauses 72-75, wherein said targeting domain of said second gRNA is configured to target the promoter region of the *MYOC* gene.
77. The nucleic acid of clause 72-76, wherein said targeting domain of said second gRNA comprises a sequence that is the same as, or differs by no more than 3 nucleotides from, a targeting domain sequence from any of Tables 1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E, or 23A-23B.
78. The nucleic acid of any of clauses 72-77, wherein said targeting domain of said second gRNA comprises a sequence that is the same as a targeting domain sequence from any of Tables 1A-1E, 2A-2E, 3A-3E, 4A-4E, 5A-5F, 6A-6E, 7A-7G, 8A-8E, 9A-9E, 10A-10G, 11A-11E, 12A-12D, 13A-13E, 14A-14C, 15A-15D, 16A-16E, 17A-17B, 18A-18D, 19A-19E, 20A-20D, 21A-21D, 22A-22E, or 23A-23B.
79. The nucleic acid of any of clauses 72-78, wherein said second gRNA molecule is a modular gRNA molecule.
80. The nucleic acid of any of clauses 72-78, wherein said second gRNA molecule is a chimeric gRNA molecule.
81. The nucleic acid of any of clauses 72-80, wherein said targeting domain is 16 nucleotides or more in length.
82. The nucleic acid of any of clauses 72-81, wherein said targeting domain is 17 nucleotides in length.
83. The nucleic acid of any of clauses 72-82, wherein said targeting domain is 18 nucleotides in length.
84. The nucleic acid of any of clauses 72-83, wherein said targeting domain is 19 nucleotides in length.
85. The nucleic acid of any of clauses 72-84, wherein said targeting domain is 20 nucleotides in length.
86. The nucleic acid of any of clauses 72-85, wherein said targeting domain is 21 nucleotides in length.
87. The nucleic acid of any of clauses 72-86, wherein said targeting domain is 22 nucleotides in length.
88. The nucleic acid of any of clauses 72-87, wherein said targeting domain is 23 nucleotides in length.
89. The nucleic acid of any of clauses 72-88, wherein said targeting domain is 24 nucleotides in length.
90. The nucleic acid of any of clauses 72-89, wherein said targeting domain is 25 nucleotides in length.
91. The nucleic acid of any of clauses 72-90, wherein said targeting domain is 26 nucleotides in length.
92. The nucleic acid of any of clauses 72-91, wherein said second gRNA molecule comprises from 5' to 3':
   a targeting domain;
   a first complementarity domain;
   a linking domain;
   a second complementarity domain;
   a proximal domain; and
   a tail domain.
93. The nucleic acid of any of clauses 72-92, wherein said second gRNA molecule comprises:
   a linking domain of no more than 25 nucleotides in length;
   a proximal and tail domain, that taken together, are at least 20 nucleotides in length;
   a targeting domain of 17 or 18 nucleotides in length.
94. The nucleic acid of any of clauses 72-93, wherein said second molecule gRNA molecule comprises:
   a linking domain of no more than 25 nucleotides in length;
   a proximal and tail domain, that taken together, are at least 25 nucleotides in length;
   a targeting domain of 17 or 18 nucleotides in length.
95. The nucleic acid of any of clauses 72-94, wherein said second gRNA molecule comprises:
   a linking domain of no more than 25 nucleotides in length;
   a proximal and tail domain, that taken together, are at least 30 nucleotides in length;
   a targeting domain of 17 nucleotides in length.
96. The nucleic acid of any of clauses 72-95, wherein said second gRNA molecule comprises:
   a linking domain of no more than 25 nucleotides in length;
   a proximal and tail domain, that taken together, are at least 40 nucleotides in length;
   a targeting domain of 17 nucleotides in length.
97. The nucleic acid of any of clauses 72-96, further comprising a third gRNA molecule.
98. The nucleic acid of clause 97, further comprising a fourth gRNA molecule.
99. The nucleic acid of any of clauses 31-71, wherein said nucleic acid does not comprise (c) a sequence that encodes a second gRNA molecule.
100. The nucleic acid of any of clauses 56-99, wherein each of (a) and (b) is present on the same nucleic acid molecule.
101. The nucleic acid of clause 100, wherein said nucleic acid molecule is an AAV vector.
102. The nucleic acid of any of clauses 56-99, wherein: (a) is present on a first nucleic acid molecule; and (b) is present on a second nucleic acid molecule.
103. The nucleic acid of clause 102, wherein said first and second nucleic acid molecules are AAV vectors.
104. The nucleic acid of any of clauses 72-98 or 100-103, wherein each of (a) and (c) is present on the same nucleic acid molecule.
105. The nucleic acid of clause 104, wherein said nucleic acid molecule is an AAV vector.
106. The nucleic acid of any of clauses 72-98 or 100-103, wherein: (a) is present on a first nucleic acid molecule; and (c) is present on a second nucleic acid molecule.
107. The nucleic acid of clause 106, wherein said first and second nucleic acid molecules are AAV vectors.
108. The nucleic acid of any of clauses 72-98, 100, 101, 104, or 105, wherein each of (a), (b), and (c) are present on the same nucleic acid molecule.
109. The nucleic acid of clause 108, wherein said nucleic acid molecule is an AAV vector.
110. The nucleic acid of any of clauses 72-98 or 100-107, wherein:
   one of (a), (b), and (c) is encoded on a first nucleic acid molecule; and
   and a second and third of (a), (b), and (c) is encoded on a second nucleic acid molecule.
111. The nucleic acid of clause 110, wherein said first and second nucleic acid molecules are AAV vectors.
112. The nucleic acid of any of clauses 72-98, 102, 103, 106, 107, 110, or 111, wherein:
   (a) is present on a first nucleic acid molecule; and (b) and (c) are present on a second nucleic acid molecule.
113. The nucleic acid of clause 112, wherein said first and second nucleic acid molecules are AAV vectors.
114. The nucleic acid of any of clauses 72-98, 102-105, 110, or 111, wherein: (b) is present on a first nucleic acid molecule; and (a) and (c) are present on a second nucleic acid molecule.
115. The nucleic acid of clause 114, wherein said first and second nucleic acid molecules are AAV vectors.
116. The nucleic acid of any of clauses 72-98, 100, 101, 106, 107, 110, or 111, wherein:
   (c) is present on a first nucleic acid molecule; and (b) and (a) are present on a second nucleic acid molecule.
117. The nucleic acid of clause 116, wherein said first and second nucleic acid molecules are AAV vectors.
118. The nucleic acid of any of clauses 102, 106, 110, 112, 114 or 116, wherein said first nucleic acid molecule is other than an AAV vector and said second nucleic acid molecule is an AAV vector.
119. The nucleic acid of any of clauses 31-118, wherein said nucleic acid comprises a promoter operably linked to the sequence that encodes said gRNA molecule of (a).
120. The nucleic acid of clauses 72-98 or 100-119, wherein said nucleic acid comprises a second promoter operably linked to the sequence that encodes the second gRNA molecule of (c).
121. The nucleic acid of clause 120, wherein the promoter and second promoter differ from one another.
122. The nucleic acid of clause 120, wherein the promoter and second promoter are the same.
123. The nucleic acid of any of clauses 56-122, wherein said nucleic acid comprises a promoter operably linked to the sequence that encodes the Cas9 molecule of (b).
124. A composition comprising the (a) gRNA molecule of any of clauses 1-30.
125. The composition of clause 124, further comprising (b) a Cas9 molecule of any of clauses 56-71.
126. The composition of any of clauses 124 or 125, further comprising (c) a second gRNA molecule of any of clauses 72-96.
127. The composition of clause 126, further comprising a third gRNA molecule.
128. The composition of clause 127, further comprising a fourth gRNA molecule.
129. A method of altering a cell comprising contacting said cell with:
   (a) a gRNA of any of clauses 1-30;
   (b) a Cas9 molecule of any of clauses 56-71;
   optionally, (c) a second gRNA molecule of any of clauses 72-96; and
   optionally, (d) a template nucleic acid.
130. The method of clause 129, further comprising a third gRNA molecule.
131. The method of clause 130, further comprising a fourth gRNA molecule.
132. The method of any of clauses 129-131, comprising contacting said cell with (a), (b), (c) and optionally (d).
133. The method of any of clauses 129-132, wherein said cell is from a subject suffering from POAG.
134. The method of any of clauses 129-133, wherein said cell is from a subject having a mutation at a POAG target position of the *MYOC* gene.
135. The method of any of clauses 129-134, wherein said cell is an ocular cell.
136. The method of any of clauses 129-134, wherein said cell is a trabecular meshwork cell.
137. The method of any of clauses 129-134, wherein said cell is a retinal pigment cell.
138. The method of any of clauses 129-137, wherein said contacting step is performed *ex vivo.*
139. The method of any of clauses 129-138, wherein said contacted cell is returned to said subject's body.
140. The method of any of clauses 129-137, wherein said contacting step is performed *in vivo.*
141. The method of any of clauses 129-140, comprising acquiring knowledge of the presence of the POAG target position mutation in said cell.
142. The method of clause 141, comprising acquiring knowledge of the presence of the POAG target position mutation in said cell by sequencing a portion of the *MYOC* gene.
143. The method of any of clauses 129-142, comprising correcting a POAG target position mutation.
144. The method of any of clauses 129-143, wherein the contacting step comprises contacting said cell with a nucleic acid that encodes at least one of (a), (b), (c) and (d).
145. The method of any of clauses 129-144, wherein the contacting step comprises contacting the cell with a nucleic acid of any of clauses 31-123.
146. The method of any of clauses 129-145, wherein the contacting step comprises delivering to said cell said Cas9 molecule of (b) and a nucleic acid which encodes and (a) and optionally (c) and/or (d).
147. The method of any of clauses 129-145, wherein the contacting step comprises delivering to said cell said Cas9 molecule of (b), said gRNA molecule of (a) and optionally said second gRNA molecule of (c).
148. The method of any of clauses 129-145, wherein the contacting step comprises delivering to said cell said gRNA molecule of (a), optionally said second gRNA molecule of (c) and a nucleic acid that encodes the Cas9 molecule of (b).
149. A method of treating a subject, comprising contacting a subject (or a cell from said subject) with:
   (a) a gRNA of any of clauses 1-30;
   (b) a Cas9 molecule of any of clauses 56-71;
   optionally, (c) a second gRNA of any of clauses 72-96; and
   optionally, (d) a template nucleic acid.
150. The method of clause 149, further comprising a third gRNA molecule.
151. The method of clause 150, further comprising a fourth gRNA molecule.
152. The method of any of clauses 149-151, further comprising contacting said subject with (a), (b), (c) and optionally (d).
153. The method of any of clauses 149-152, wherein said subject is suffering from POAG.
154. The method of any of clauses 149-153, wherein said subject has a mutation at the POAG target position of the *MYOC* gene.
155. The method of any of clauses 149-154, comprising acquiring knowledge of the presence of the POAG target position mutation in said subject.
156. The method of clause 155, comprising acquiring knowledge of the presence of the POAG target position mutation in said subject by sequencing a portion of the *MYOC* gene.
157. The method of any of clauses 149-156, comprising correcting the POAG target position mutation in the *MYOC* gene.
158. The method of any of clauses 149-157, wherein a cell of said subject is contacted *ex vivo* with (a), (b), and optionally (c) and/or (d).
159. The method of clause 158, wherein said cell is returned to the subject's body.
160. The method of any of clauses 149-159, wherein treatment comprises introducing a cell into said subject's body, wherein said cell is contacted *ex vivo* with (a), (b), and optionally (c) and/or (d).
161. The method of any of clauses 149-157, wherein said contacting step is performed *in vivo.*
162. The method of clause 149-157 or 161, wherein said contacting step comprises subretinal delivery.
163. The method of clause 149-157 or 161, wherein said contacting step comprises subretinal injection.
164. The method of clause 149-157 or 161, wherein said contacting step comprises intravitreal delivery.
165. The method of clause 149-157 or 161, wherein said contacting step comprises intravitreal injection.
166. The method of any of clauses 149-165, wherein the contacting step comprises contacting said subject with a nucleic acid that encodes at least one of (a), (b), and (c).
167. The method of any of clauses 149-166, wherein the contacting step comprises contacting said subject with a nucleic acid of any of any of clauses 31-123.
168. The method of any of clauses 149-167, wherein the contacting step comprises delivering to said subject said Cas9 molecule of (b) and a nucleic acid which encodes and (a) and optionally (c), and optionally (d).
169. The method of any of clauses 149-167, wherein the contacting step comprises delivering to said subject said Cas9 molecule of (b), said gRNA of (a) and optionally said second gRNA of (c), and optionally said template nucleic acid of (d).
170. The method of any of clauses 149-167, wherein the contacting step comprises delivering to said subject said gRNA of (a), optionally said second gRNA of (c) and a nucleic acid that encodes the Cas9 molecule of (b).
171. A reaction mixture comprising a gRNA, a nucleic acid, or a composition described herein, and a cell from a subject having POAG, or a subject having a mutation at a POAG target position of the *MYOC* gene.
172. A kit comprising, (a) gRNA molecule of any of clauses 1-30, or a nucleic acid that encodes said gRNA, and one or more of the following:
   (b) a Cas9 molecule of any of clauses 56-71;
   (c) a second gRNA molecule of any of clauses 72-96;
   (d) a template nucleic acid; and
   (e) a nucleic acid that encodes one or more of (b) and (c).
173. The kit of clause 172, comprising nucleic acid that encodes one or more of (a), (b) (c) and (d).
174. The kit of clause 173, further comprising a third gRNA molecule targeting a POAG target position.
175. The kit of clause 174, further comprising a fourth gRNA molecule targeting a POAG target position.
176. A gRNA molecule of any of clauses 1-30 for use in treating POAG in a subject.
177. The gRNA molecule of clause 176, wherein the gRNA molecule is used in combination with (b) a Cas9 molecule of any of clauses 56-71.
178. The gRNA molecule of clause 176 or 177, wherein the gRNA molecule is used in combination with (c) a second gRNA molecule of any of clauses 72-96.
179. Use of a gRNA molecule of any of clauses 1-30 in the manufacture of a medicament for treating POAG in a subject.
180. The use of clause 179, wherein the medicament further comprises (b) a Cas9 molecule of any of clause 56-71.
181. The use of clause 179 or 180, wherein the medicament further comprises (c) a second gRNA molecule of any of clause 72-96.
182. A composition of any of clauses 124-128 for use in treating POAG in a subject.

## Claims

1. A gRNA molecule comprising a first targeting domain which is complementary with a first target domain from the *MYOC* gene, wherein said first target domain is located within 500 bp of the start codon of the *MYOC* gene, wherein said targeting domain is configured to provide a double strand break in the presence a Cas9 molecule, and wherein said double strand break results in knockout of the *MYOC* gene.

2. The gRNA molecule of claim 1, wherein said target domain is in the *MYOC* gene coding region.

3. The gRNA molecule of claim 1 or 2, wherein said first targeting domain comprises or consists of a nucleotide sequence set forth in any of Tables 4, 6, 7, or 8.

4. The gRNA molecule of any of claims 1 to 3, wherein said first target domain is located within 400 bp, within 300 bp, within 200 bp, or within 100 bp of the transcription start site of the *MYOC* gene.

5. The gRNA molecule of claim 4, wherein said first target domain is located within 100 bp of the transcription start site of the *MYOC* gene; optionally, wherein said first targeting domain comprises or consists of the nucleotide sequence of myoC-94 (SEQ ID NO:499), myoC-92 (SEQ ID NO:498), myoC-93, myoC-95 (SEQ ID NO:500), myoC-130 (SEQ ID NO:525), myoC-131 (SEQ ID NO:526), myoC-170 (SEQ ID NO:556), myoC-171 (SEQ ID NO:557), myoC-172 (SEQ ID NO:558), myoC-173 (SEQ ID NO:559), myoC-174 (SEQ ID NO:560), myoC-244 (SEQ ID NO:630), myoC-245 (SEQ ID NO:631), myoC-246 (SEQ ID NO:632), myoC-247 (SEQ ID NO:633), myoC-248 (SEQ ID NO:634), myoC-249 (SEQ ID NO:635), myoC-1595 (SEQ ID NO:1879), myoC-1597 (SEQ ID NO:1880), myoC-1600 (SEQ ID NO:1881), myoC-3263 (SEQ ID NO:3009), myoC-3264 (SEQ ID NO:3010), myoC-3265 (SEQ ID NO:3011), myoC-3266 (SEQ ID NO:3012), myoC-3597 (SEQ ID NO:3343), myoC-3598 (SEQ ID NO:3344), myoC-3599 (SEQ ID NO:3345), myoC-3600 (SEQ ID NO:3346), myoC-3601 (SEQ ID NO:3347), myoC-3602 (SEQ ID NO:3348), myoC-3711 (SEQ ID NO:3457), myoC-3712 (SEQ ID NO:3458), myoC-3713 (SEQ ID NO:3459), myoC-3714 (SEQ ID NO:3460), myoC-3715 (SEQ ID NO:3461), myoC-3716 (SEQ ID NO:3462), myoC-3771 (SEQ ID NO:3517), myoC-3772 (SEQ ID NO:3518), myoC-3773 (SEQ ID NO:3519), myoC-3774 (SEQ ID NO:3520), myoC-3775 (SEQ ID NO:3521), myoC-3776 (SEQ ID NO:3522), myoC-3777 (SEQ ID NO:3523), myoC-3778 (SEQ ID NO:3524), myoC-3779 (SEQ ID NO:3525), myoC-3780 (SEQ ID NO:3526), myoC-3781 (SEQ ID NO:3527), myoC-3782 (SEQ ID NO:3528), myoC-91 (SEQ ID NO:477), myoC-129 (SEQ ID NO:488), myoC-169 (SEQ ID NO:555), myoC-243 (SEQ ID NO:629), myoC-1592 (SEQ ID NO:1876), myoC-1593 (SEQ ID NO:1877), myoC-1594 (SEQ ID NO:1878), myoC-3158 (SEQ ID NO:2904), myoC-3160 (SEQ ID NO:2906), myoC-3603 (SEQ ID NO:3349), myoC-3604 (SEQ ID NO:3350), myoC-3605 (SEQ ID NO:3351), myoC-3606 (SEQ ID NO:3352), myoC-3607 (SEQ ID NO:3353), myoC-3608 (SEQ ID NO:3354), myoC-5354 (SEQ ID NO:5100), myoC-5365 (SEQ ID NO:5111), myoC-1592 (SEQ ID NO:1876), myoC-3158 (SEQ ID NO:2904), and myoC-3160 (SEQ ID NO:2906); or
wherein said first target domain is located within 200 bp of the transcription start site of the *MYOC* gene; optionally, wherein said first targeting domain comprises or consists of the nucleotide sequence of myoC-94 (SEQ ID NO:499), myoC-92 (SEQ ID NO:498), myoC-93, myoC-95 (SEQ ID NO:500), myoC-130 (SEQ ID NO:525), myoC-131 (SEQ ID NO:526), myoC-170 (SEQ ID NO:556), myoC-171 (SEQ ID NO:557), myoC-172 (SEQ ID NO:558), myoC-173 (SEQ ID NO:559), myoC-174 (SEQ ID NO:560), myoC-244 (SEQ ID NO:630), myoC-245 (SEQ ID NO:631), myoC-246 (SEQ ID NO:632), myoC-247 (SEQ ID NO:633), myoC-248 (SEQ ID NO:634), myoC-249 (SEQ ID NO:635), myoC-1595 (SEQ ID NO:1879), myoC-1597 (SEQ ID NO:1880), myoC-1600 (SEQ ID NO:1881), myoC-3263 (SEQ ID NO:3009), myoC-3264 (SEQ ID NO:3010), myoC-3265 (SEQ ID NO:3011), myoC-3266 (SEQ ID NO:3012), myoC-3597 (SEQ ID NO:3343), myoC-3598 (SEQ ID NO:3344), myoC-3599 (SEQ ID NO:3345), myoC-3600 (SEQ ID NO:3346), myoC-3601 (SEQ ID NO:3347), myoC-3602 (SEQ ID NO:3348), myoC-3711 (SEQ ID NO:3457), myoC-3712 (SEQ ID NO:3458), myoC-3713 (SEQ ID NO:3459), myoC-3714 (SEQ ID NO:3460), myoC-3715 (SEQ ID NO:3461), myoC-3716 (SEQ ID NO:3462), myoC-3771 (SEQ ID NO:3517), myoC-3772 (SEQ ID NO:3518), myoC-3773 (SEQ ID NO:3519), myoC-3774 (SEQ ID NO:3520), myoC-3775 (SEQ ID NO:3521), myoC-3776 (SEQ ID NO:3522), myoC-3777 (SEQ ID NO:3523), myoC-3778 (SEQ ID NO:3524), myoC-3779 (SEQ ID NO:3525), myoC-3780 (SEQ ID NO:3526), myoC-3781 (SEQ ID NO:3527), myoC-3782 (SEQ ID NO:3528), myoC-91 (SEQ ID NO:477), myoC-129 (SEQ ID NO:488), myoC-169 (SEQ ID NO:555), myoC-243 (SEQ ID NO:629), myoC-1592 (SEQ ID NO:1876), myoC-1593 (SEQ ID NO:1877), myoC-1594 (SEQ ID NO:1878), myoC-3158 (SEQ ID NO:2904), myoC-3160 (SEQ ID NO:2906), myoC-3603 (SEQ ID NO:3349), myoC-3604 (SEQ ID NO:3350), myoC-3605 (SEQ ID NO:3351), myoC-3606 (SEQ ID NO:3352), myoC-3607 (SEQ ID NO:3353), myoC-3608 (SEQ ID NO:3354), myoC-5354 (SEQ ID NO:5100), myoC-5365 (SEQ ID NO:5111), myoC-1592 (SEQ ID NO:1876), myoC-3158 (SEQ ID NO:2904), myoC-3160 (SEQ ID NO:2906), myoC-96 (SEQ ID NO:501), myoC-132 (SEQ ID NO:527), myoC-175 (SEQ ID NO:561), myoC-176 (SEQ ID NO:562), myoC-177 (SEQ ID NO:563), myoC-178 (SEQ ID NO:564), myoC-251 (SEQ ID NO:637), myoC-252 (SEQ ID NO:638), myoC-253 (SEQ ID NO:639), myoC-1601 (SEQ ID NO:1882), myoC-1603 (SEQ ID NO:1883), myoC-1604 (SEQ ID NO:1884), and myoC-1605 (SEQ ID NO:1885); or
wherein said first target domain is located within 300 bp of the transcription start site of the *MYOC* gene; optionally, wherein said first targeting domain comprises or consists of the nucleotide sequence of myoC-94 (SEQ ID NO:499), myoC-92 (SEQ ID NO:498), myoC-93, myoC-95 (SEQ ID NO:500), myoC-130 (SEQ ID NO:525), myoC-131 (SEQ ID NO:526), myoC-170 (SEQ ID NO:556), myoC-171 (SEQ ID NO:557), myoC-172 (SEQ ID NO:558), myoC-173 (SEQ ID NO:559), myoC-174 (SEQ ID NO:560), myoC-244 (SEQ ID NO:630), myoC-245 (SEQ ID NO:631), myoC-246 (SEQ ID NO:632), myoC-247 (SEQ ID NO:633), myoC-248 (SEQ ID NO:634), myoC-249 (SEQ ID NO:635), myoC-1595 (SEQ ID NO:1879), myoC-1597 (SEQ ID NO:1880), myoC-1600 (SEQ ID NO:1881), myoC-3263 (SEQ ID NO:3009), myoC-3264 (SEQ ID NO:3010), myoC-3265 (SEQ ID NO:3011), myoC-3266 (SEQ ID NO:3012), myoC-3597 (SEQ ID NO:3343), myoC-3598 (SEQ ID NO:3344), myoC-3599 (SEQ ID NO:3345), myoC-3600 (SEQ ID NO:3346), myoC-3601 (SEQ ID NO:3347), myoC-3602 (SEQ ID NO:3348), myoC-3711 (SEQ ID NO:3457), myoC-3712 (SEQ ID NO:3458), myoC-3713 (SEQ ID NO:3459), myoC-3714 (SEQ ID NO:3460), myoC-3715 (SEQ ID NO:3461), myoC-3716 (SEQ ID NO:3462), myoC-3771 (SEQ ID NO:3517), myoC-3772 (SEQ ID NO:3518), myoC-3773 (SEQ ID NO:3519), myoC-3774 (SEQ ID NO:3520), myoC-3775 (SEQ ID NO:3521), myoC-3776 (SEQ ID NO:3522), myoC-3777 (SEQ ID NO:3523), myoC-3778 (SEQ ID NO:3524), myoC-3779 (SEQ ID NO:3525), myoC-3780 (SEQ ID NO:3526), myoC-3781 (SEQ ID NO:3527), myoC-3782 (SEQ ID NO:3528), myoC-91 (SEQ ID NO:477), myoC-129 (SEQ ID NO:488), myoC-169 (SEQ ID NO:555), myoC-243 (SEQ ID NO:629), myoC-1592 (SEQ ID NO:1876), myoC-1593 (SEQ ID NO:1877), myoC-1594 (SEQ ID NO:1878), myoC-3158 (SEQ ID NO:2904), myoC-3160 (SEQ ID NO:2906), myoC-3603 (SEQ ID NO:3349), myoC-3604 (SEQ ID NO:3350), myoC-3605 (SEQ ID NO:3351), myoC-3606 (SEQ ID NO:3352), myoC-3607 (SEQ ID NO:3353), myoC-3608 (SEQ ID NO:3354), myoC-5354 (SEQ ID NO:5100), myoC-5365 (SEQ ID NO:5111), myoC-1592 (SEQ ID NO:1876), myoC-3158 (SEQ ID NO:2904), myoC-3160 (SEQ ID NO:2906), myoC-96 (SEQ ID NO:501), myoC-132 (SEQ ID NO:527), myoC-175 (SEQ ID NO:561), myoC-176 (SEQ ID NO:562), myoC-177 (SEQ ID NO:563), myoC-178 (SEQ ID NO:564), myoC-251 (SEQ ID NO:637), myoC-252 (SEQ ID NO:638), myoC-253 (SEQ ID NO:639), myoC-1601 (SEQ ID NO:1882), myoC-1603 (SEQ ID NO:1883), myoC-1604 (SEQ ID NO:1884), myoC-1605 (SEQ ID NO:1885), myoC-179 (SEQ ID NO:565), myoC-180 (SEQ ID NO:566), myoC-181 (SEQ ID NO:567), myoC-254 (SEQ ID NO:640), myoC-255 (SEQ ID NO:641), myoC-256 (SEQ ID NO:642), myoC-3500 (SEQ ID NO:3246), myoC-3568 (SEQ ID NO:3314), myoC-3717 (SEQ ID NO:3463), and myoC-5357 (SEQ ID NO:5103).

6. The gRNA molecule of any of claims 1-5, wherein said gRNA is a modular or chimeric gRNA molecule; or
wherein said first targeting domain is 16 nucleotides or more in length.

7. The gRNA molecule of any of claims 1-5, comprising from 5' to 3':
the first targeting domain;
a first complementarity domain;
a linking domain;
a second complementarity domain;
a proximal domain; and
a tail domain.

8. A composition comprising a gRNA molecule of any of claims 1-7, and, optionally, a Cas9 molecule.

9. The composition of claim 8, further comprising a second gRNA molecule comprising a second targeting domain that is complementary with a second target domain from the *MYOC* gene, preferably, wherein said second targeting domain comprises or consists of a nucleotide sequence set forth in any of Tables 4, 6, 7, or 8, wherein the sequence of said second targeting domain is different than the sequence of the said first targeting domain.

10. A nucleic acid comprising (a) a sequence encoding the first gRNA molecule of any of claims 1-7; and optionally further comprising (b) a sequence that encodes a Cas9 molecule; and/or further comprising (c) a sequence that encodes a second gRNA molecule comprising a second targeting domain that is complementary with a second target domain from the *MYOC* gene.

11. A viral vector comprising a nucleic acid of claim 10; and optionally the viral vector is an AAV vector.

12. The gRNA molecule of any of claims 1-7, a composition of claim 9, a nucleic acid of claim 10, or a vector of claim 11, for use in a method of knocking out a *MYOC* gene in a cell comprising contacting the cell with said gRNA, composition, nucleic acid or AAV vector.

13. The gRNA, composition, nucleic acid or vector for the use of claim 12, wherein said knock out of the *MYOC* gene reduces expression of the *MYOC* gene product.

14. The gRNA, composition, nucleic acid or vector for the use of any of claims 12 or 13, wherein the cell is a cell from the eye; and optionally wherein the cell from the eye is a trabecular meshwork cell.

15. The gRNA, composition, nucleic acid or vector for the use of any of claims 12 to 14, wherein the *MYOC* gene being knocked out comprises a mutation in a nucleotide sequence encoding an amino acid located in a mutational hotspot between positions 423-437 of the *MYOC* gene product.
